# EUROPEAN PATENT SPECIFICATION

(11) **EP 2 406 371 B1**
(45) Date of publication and mention of the grant of the patent: **09.05.2018**
(21) Application number: 10709359.3
(22) Date of filing: 12.03.2010
(51) Int. Cl.: C12N 5/079, G01N 33/50, C12Q 1/37

(54) **CELLS USEFUL FOR IMMUNO-BASED BOTULINUM TOXIN SEROTYPE A ACTIVITY ASSAYS**
FÜR BOTULINUMTOXIN-SEROTYP-A-AKTIVITÄTSTESTS AUF IMMUNBASIS GEEIGNETE ZELLEN
CELLULES UTILES POUR DES ESSAIS D'ACTIVITÉ DE LA TOXINE BOTULINIQUE DE SÉROTYPE A BASÉS SUR L'IMMUNOLOGIE

(30) Priority: 13.03.2009 US 160199 P
(43) Date of publication of application: 18.01.2012
(60) Divisional application: 18150275.8
(73) Proprietor: ALLERGAN, INC., Irvine, CA 92612 (US)
(72) Inventor: JACKY, Birgitte, P.S., Orange CA 92866 (US); WANG, Joanne, Irvine CA 92620 (US); ZHU, Hong, San Diego CA 92130 (US); HODGES, D., Dianne, Tustin CA 92780 (US); FERNANDEZ-SALAS, Ester, Fullerton CA 92831 (US)
(74) Representative: Hoffmann Eitle
(86) International application number: PCT/US2010/027242
(87) International publication number: WO 2010/105234

(56) References cited:
- WO-A1-2009/114748
- MASUMOTO N ET AL: "Involvement of SNAP-25 in TRH-induced exocytosis in pituitary GH4C1 cells" JOURNAL OF ENDOCRINOLOGY, vol. 153, no. 1, 1997, pages R5-R10, XP008124119 ISSN: 0022-0795
- YOWLER BRIAN C ET AL: "Botulinum neurotoxin A activity is dependent upon the presence of specific gangliosides in neuroblastoma cells expressing synaptotagmin I." THE JOURNAL OF BIOLOGICAL CHEMISTRY 6 SEP 2002 LNKD- PUBMED:12089155, vol. 277, no. 36, 6 September 2002 (2002-09-06), pages 32815-32819, XP002590839 ISSN: 0021-9258
- PELLETT SABINE ET AL: "A neuronal cell-based botulinum neurotoxin assay for highly sensitive and specific detection of neutralizing serum antibodies." FEBS LETTERS 16 OCT 2007 LNKD- PUBMED:17889852, vol. 581, no. 25, 16 October 2007 (2007-10-16), pages 4803-4808, XP22289797 ISSN: 0014-5793
- MARINI PATRIZIA ET AL: "SiMa, a new neuroblastoma cell line combining poor prognostic cytogenetic markers with high adrenergic differentiation", CANCER GENETICS AND CYTOGENETICS, ELSEVIER SCIENCE PUBLISHING, NEW YORK, NY, US, vol. 112, 1 January 1999 (1999-01-01), pages 161-164, XP003027695, ISSN: 0165-4608
- ESTER FERNÁNDEZ-SALAS ET AL: "Botulinum Neurotoxin Serotype a Specific Cell-Based Potency Assay to Replace the Mouse Bioassay", PLOS ONE, vol. 7, no. 11, 21 November 2012 (2012-11-21), page e49516, XP055070996, DOI: 10.1371/journal.pone.0049516

## Description

This patent application claims priority pursuant to 35 U.S.C. § 119(e) to U. S. Provisional Patent Application Serial No. 61/160,199 filed March 13, 2009.

The sequences discosed in the present specification are contained in the Sequence Listing submitted with the present specification which is hereby incorporated by reference in its entirety.

The ability of Clostridial toxins, such as, e.g., Botulinum neurotoxins (BoNTs), BoNT/A, BoNT/B, BoNT/C1, BoNT/D, BoNT/E, BoNT/F and BoNT/G, and Tetanus neurotoxin (TeNT), to inhibit neuronal transmission are being exploited in a wide variety of therapeutic and cosmetic applications, see e.g., William J. Lipham, Cosmetic and Clinical Applications of Botulinum Toxin (Slack, Inc., 2004). Clostridial toxins commercially available as pharmaceutical compositions include, BoNT/A preparations, such as, e.g., BOTOX® (Allergan, Inc., Irvine, CA), DYSPORT®/RELOXIN®, (Ipsen Ltd., Slough, England), PURTOX® (Mentor Corp., Santa Barbara, CA), XEOMIN® (Merz Pharmaceuticals, GmbH., Frankfurt, Germany), NEURONOX® (Medy-Tox, Inc., Ochang-myeon, South Korea), BTX-A (Biogen-tech Ltd., University, Yantai, Shandong, China); and BoNT/B preparations, such as, e.g., MYOBLOC®/NEUROBLOC® (Solstice Neurosciences, Inc., South San Francisco, CA). As an example, BOTOX® is currently approved in one or more countries for the following indications: achalasia, adult spasticity, anal fissure, back pain, blepharospasm, bruxism, cervical dystonia, essential tremor, glabellar lines or hyperkinetic facial lines, headache, hemifacial spasm, hyperactivity of bladder, hyperhidrosis, juvenile cerebral palsy, multiple sclerosis, myoclonic disorders, nasal labial lines, spasmodic dysphonia, strabismus and VII nerve disorder.

At present the mouse LD₅₀ bioassay, a lethality test, remains the "gold standard" test used by all pharmaceutical manufacturers to express the potency of their preparations. S. S. Arnon et al., JAMA 285: 1059-1070 (2001). In fact, the units on the pharmaceutical preparations' labels are mouse LD₅₀ units and the number of animals needed to produce statistically useful LD₅₀ data is large. The advantage of the mouse LD₅₀ bioassay is that it measures all the steps necessary for botulinum toxin uptake (e.g., toxin binding to a cell surface receptor, internalization of the toxin-receptor complex, light chain translocation into the cytoplasm, light chain cleavage of substrate), instead of merely determining the activity for only part of this intoxication process, such as, e.g., in vitro assays that only measure light chain enzymatic activity. Unfortunately, the mouse LD₅₀ bioassay suffers from many drawbacks including high operational cost due to the large numbers of laboratory animals required, a lack of specificity since all BoNT serotypes will cause the same measurable end-point, and the potential for inaccuracy unless large animal groups are used. In addition, animal rights groups have exerted pressure on regulatory agencies in the United States (FDA/NICEATM/ICCVAM) and Europe (MHRA and EDQM), and on pharmaceutical companies manufacturing botulinum neurotoxin products to reduce animal testing and more importantly replace the mouse LD₅₀ bioassay for product release. The regulatory agencies are engaging pharmaceutical companies to apply the three "Rs" principle to the potency testing of botulinum neurotoxins: Reduce, Refine, Replace. D. Straughan, Progress in Applying the Three Rs to the Potency Testing of Botulinum Toxin Type A, Altern. Lab. Anim. 34(3): 305-313 (2006). In recent years, several steps have been already taken to reduce and refine the mouse LD₅₀ bioassay in order to standardize the protocol and produce more consistent data using fewer animals per assay.

Thus, a simple, reliable, validated and governmental agency acceptable botulinum toxin activity assay that can evaluate the integrity of all the steps necessary in botulinum toxin uptake would be of significant value because such a non-animal based assay would alleviate the need for animal testing and all the disadvantages, costs and ethical concerns associated with this type of animal-based assay. Companion patent application Ester Fernandez-Salas, et al., *Immuno-Based Botulinum Toxin Serotype A Activity Assays,* U.S. Patent Application Serial No: 12/403,531, provides novel compositions, cells, and methods for assaying the activity of a BoNT/A useful for various industries, such as, e.g., the pharmaceutical and food industries. Such compositions, cells, and methods do not use live animals or tissues taken from live animals, but can evaluate all the steps necessary for neurotoxin action, namely, binding and cellular uptake of toxin, translocation into the cell cytosol, and protease activity. Sabine Pellett et al. (581(25) FEBS Letters, 4803-4808 (2007)) disclose a sensitive assay to determine sensitivity to BoNT/A using primary rat spinal cord cells.

One of the necessary components for the methods disclosed in U.S. Patent Application Serial No: 12/403,531 is the use of cells from an established cell line that are susceptible to low levels of BoNT/A intoxication because the amount of BoNT/A contained within a commercially-available pharmaceutical composition is very low. For example, approximately 4-5 ng of a BoNT/A complex is contained in the pharmaceutical composition sold as BOTOX®. Thus, there is a need to identify and create established cell lines comprising cells that are susceptible to BoNT/A intoxication when only very low levels of the neurotoxin is present in the sample. The present specification provides novel cells and cell compositions. that are susceptible to BoNT/A intoxication when only very low amounts of BoNT/A is present and thus allow assaying of commercially-available pharmaceutical composition comprising BoNT/A.

### BRIEF DESCRIPTION OF THE DRAWINGS

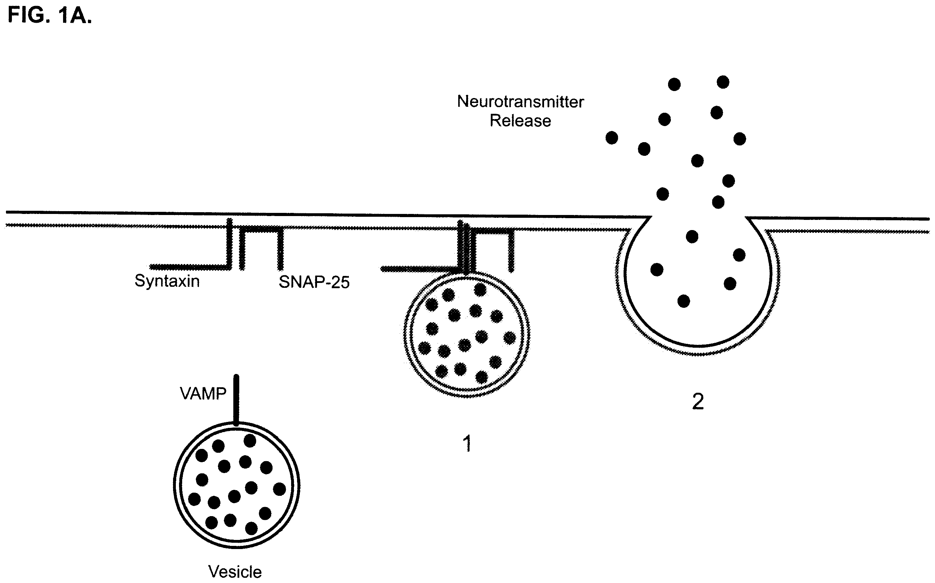

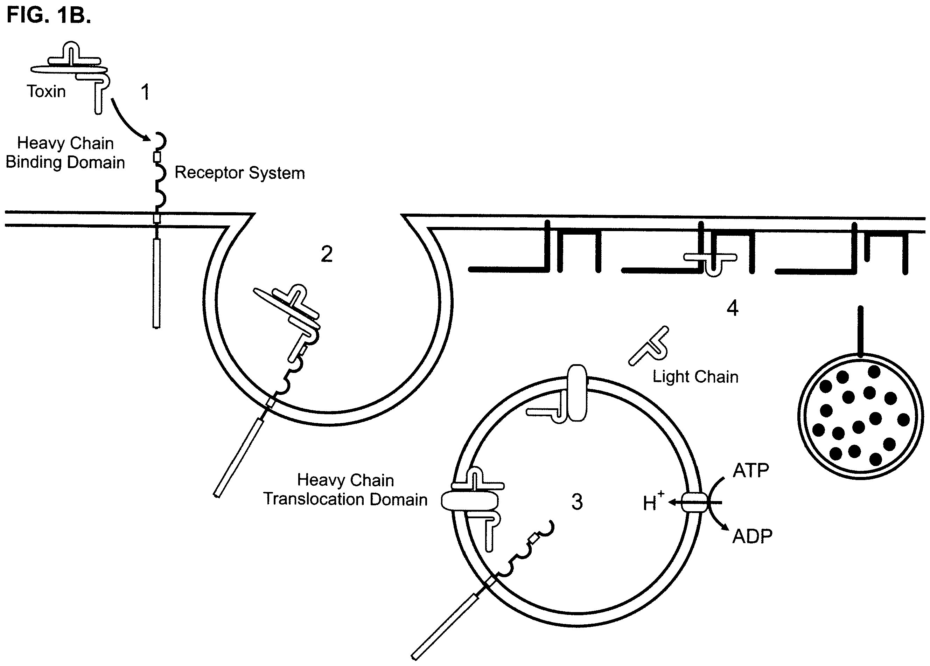

**FIG. 1** shows a schematic of the current paradigm of neurotransmitter release and Clostridial toxin intoxication in a central and peripheral neuron. FIG. 1A shows a schematic for the neurotransmitter release mechanism of a central and peripheral neuron. The release process can be described as comprising two steps: 1) vesicle docking, where the vesicle-bound SNARE protein of a vesicle containing neurotransmitter molecules associates with the membrane-bound SNARE proteins located at the plasma membrane; and 2) neurotransmitter release, where the vesicle fuses with the plasma membrane and the neurotransmitter molecules are exocytosed. FIG. 1B shows a schematic of the intoxication mechanism for tetanus and botulinum toxin activity in a central and peripheral neuron. This intoxication process can be described as comprising four steps: 1) receptor binding, where Clostridial toxin binding to a Clostridial receptor complex and initiates the intoxication process; 2) complex internalization, where after toxin binding, a vesicle containing a toxin/receptor system complex is endocytosed into the cell; 3) light chain translocation, where multiple events are thought to occur, including changes in the internal pH of the vesicle, formation of a channel pore comprising the H_{N} domain of Clostridial toxin heavy chain, separation of the Clostridial toxin light chain from the heavy chain, and release of the light chain and 4) enzymatic target modification, where the light chain of Clostridial toxin proteolytically cleaves its target SNARE substrates, such as, *e.g*., SNAP-25, VAMP or Syntaxin, thereby preventing vesicle docking and neurotransmitter release.

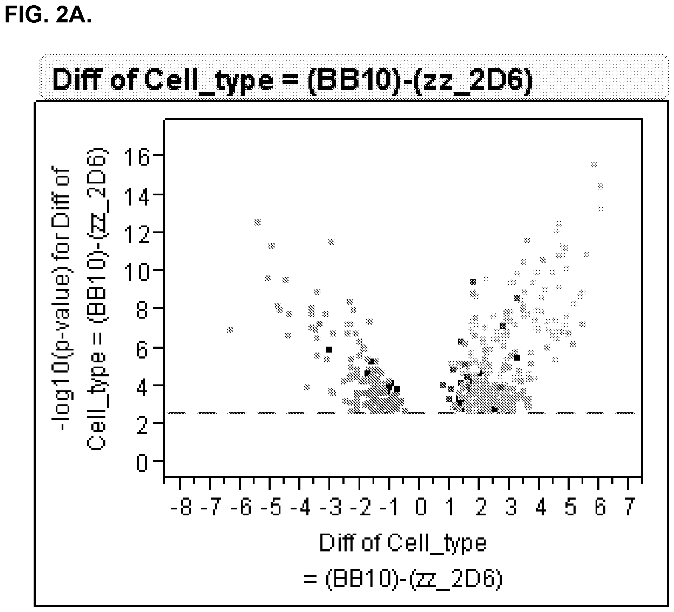

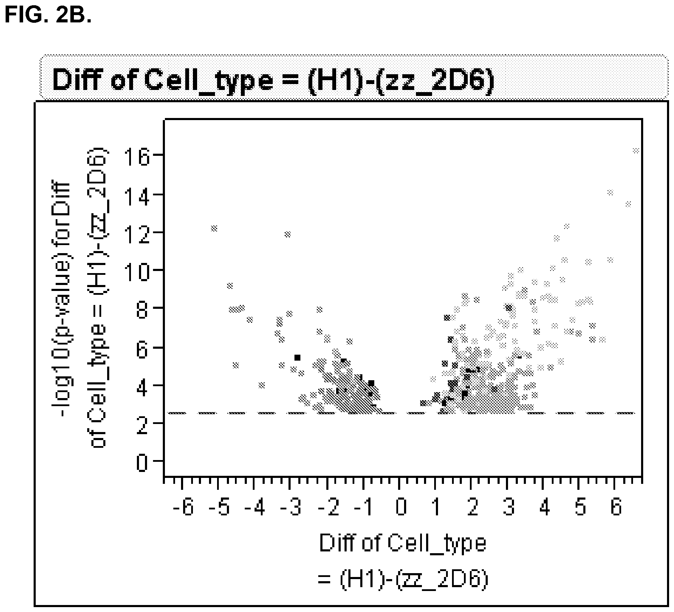

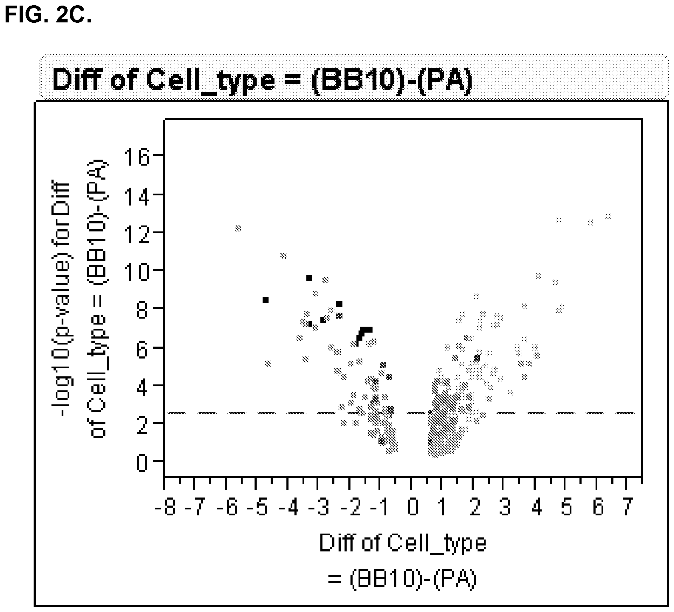

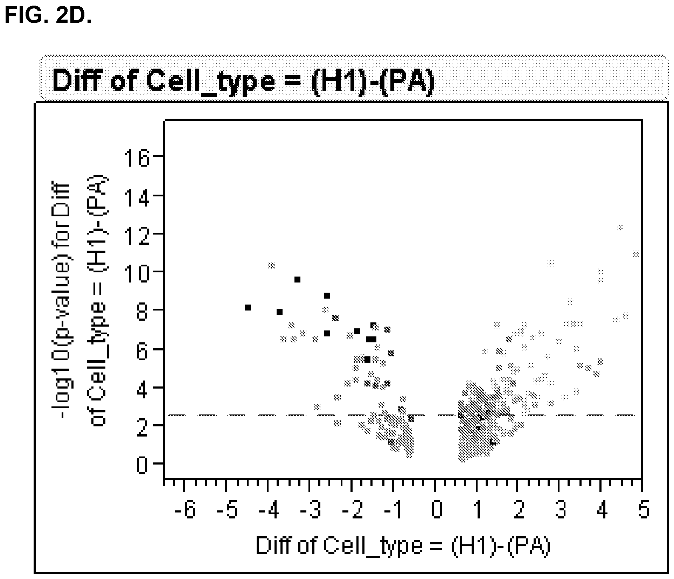

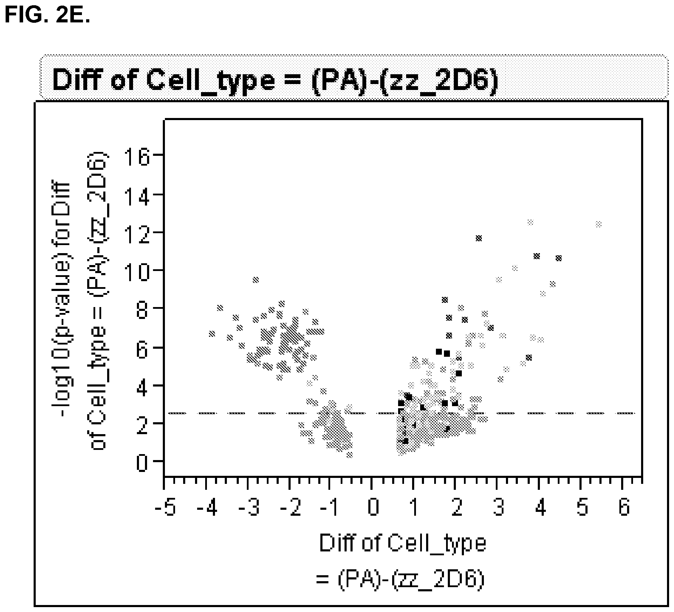

**FIG. 2** shows volcano plots of genes that are more than 4-fold (log₂(4) = 2) up or down regulated in BB10 and H1 cell lines as compared to the 2D6 cell line. FIG. 2A shows the genes that are up regulated in the BB10 cell line as compared to the 2D6 cell line. FIG2B shows the genes that are up regulated in the H1 cell line as compared to the 2D6 cell line. FIG2C shows the genes that are up regulated in the BB10 cell line as compared to the parental SiMa cell line (PA). FIG2D shows the genes that are up regulated in the H1 cell line as compared to the parental SiMa cell line (PA). FIG2E shows the genes that are up regulated in the parental SiMa cell line (PA) as compared to the 2D6 cell line. FIG. 2F shows the genes that are down regulated in the BB10 cell line as compared to the 2D6 cell line. FIG2G shows the genes that are down regulated in the H1 cell line as compared to the 2D6 cell line. FIG2H shows the genes that are down regulated in the parental SiMa cell line (PA) as compared to the2D6 cell line. Each dot represents a probe set. The dashed line marks the 95 % confidence interval, p ≤ 0.05. Most of the genes that are over expressed in BB10 and H1 compared to 2D6 (A and B) are also over or under-expressed in BB10 compared to PA (C), in H1 compared to PA (D), and in PA compared to 2D6 (E), however a number of them are less than 4-fold over-expressed and/or do not pass the 95 % confidence interval.

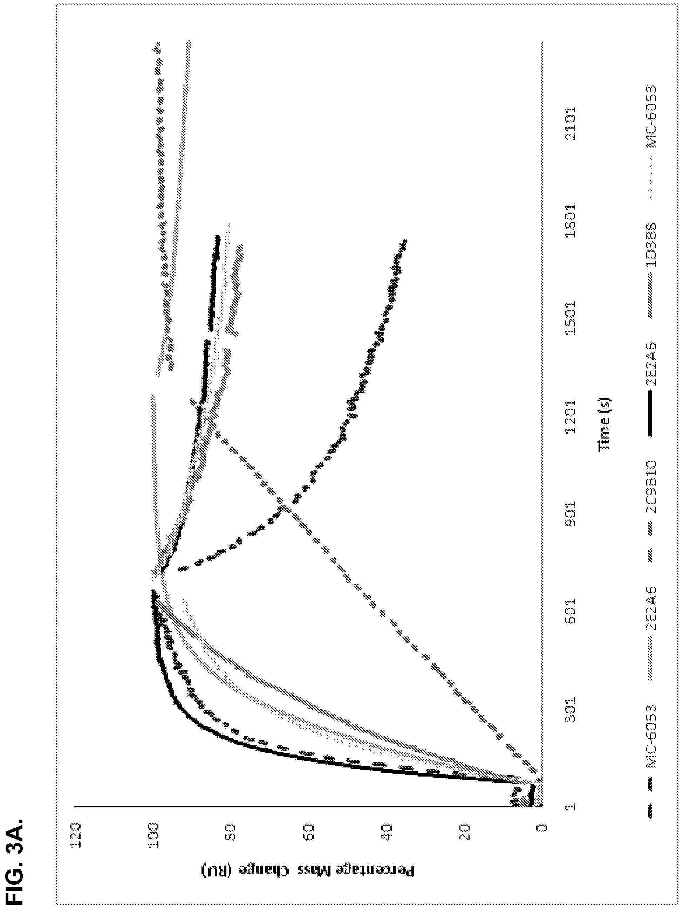

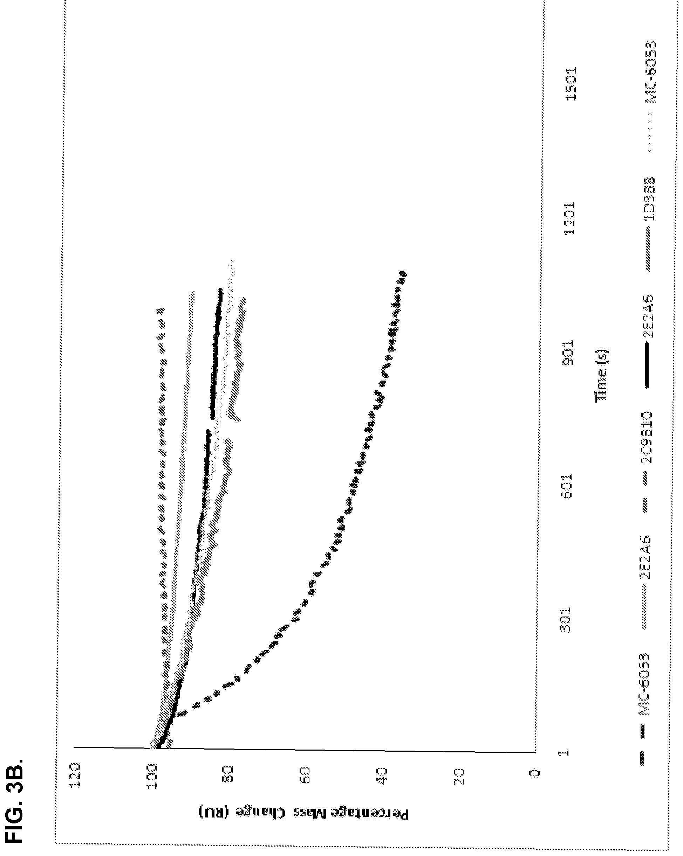

**FIG. 3** shows normalized BIAcore SPR curves of 7.8 nM of the antibodies 2E2A6, 1D3B8, 3C1A5 and 2C9B10 and commercial MC-6050 and MC-6053. FIG. 3A shows the normalized data for the on-rate of each antibody. FIG. 3B shows the normalized data for the off-rate of each antibody.

### DETAILED DESCRIPTION

The present specification provides novel cells and cell compositions that are susceptible to BoNT/A intoxication to allow assaying of BoNT/A and BoNT/A compositions such as, *e.g*., commercially-available pharmaceutical composition comprising BoNT/A as specified in the claim. The cell and cell compositions disclosed in the present specification are useful to conduct methods that can detect picomolar quantities of BoNT/A in a sample, such as the methods disclosed in, *e.g*., Ester Fernandez-Salas, et al., *Immuno-Based Botulinum Toxin Serotype A Activity Assays,* U.S. Patent Application Serial No: 12/403,531. The cells and cell compositions, and their use in methods of detecting BoNT/A activity reduce the need for animal toxicity studies, yet serve to analyze the multiple steps of BoNT/A intoxication, namely, binding and cellular uptake of toxin, translocation into the cell cytosol, and protease activity. As discussed further below, the novel cells and cell compositions of the present specification can be used in assays that analyze crude and bulk samples as well as highly purified di-chain toxins and formulated toxin products and further are amenable to automated high throughput assay formats.

Thus, aspects of the present specification provide a cell composition comprising cells from a clonal cell line that are susceptible to BoNT/A intoxication.

Other aspects of the present specification provide a method of detecting BoNT/A activity using the clonal cell lines disclosed in the present specification.

Clostridia toxins produced by *Clostridium botulinum, Clostridium tetani, Clostridium baratii* and *Clostridium butyricum* are the most widely used in therapeutic and cosmetic treatments of humans and other mammals. Strains of *C*. *botulinum* produce seven antigenically-distinct serotypes of botulinum toxins (BoNTs), which have been identified by investigating botulism outbreaks in man (BoNT/A, BoNT/B, BoNT/E and BoNT/F), animals (BoNT/C1 and BoNT/D), or isolated from soil (BoNT/G). While all seven botulinum toxin serotypes have similar structure and biological properties, each also displays heterogeneous characteristics, such as, *e.g*., different pharmacological properties. In contrast, tetanus toxin (TeNT) is produced by a uniform group of *C*. *tetani.* Two other species of Clostridia, *C*. *baratii* and *C*. *butyricum,* also produce toxins similar to BoNT/F and BoNT/E, respectively.

Clostridial toxins are each translated as a single chain polypeptide of approximately 150 kDa that is subsequently cleaved by proteolytic scission within a disulfide loop by a naturally-occurring protease, such as, e.g., an endogenous Clostridial toxin protease or a naturally-occurring protease produced in the environment. This posttranslational processing yields a di-chain molecule comprising an approximately 50 kDa light chain (LC) and an approximately 100 kDa heavy chain (HC) held together by a single disulfide bond and noncovalent interactions. Each mature di-chain molecule comprises three functionally distinct domains: 1) an enzymatic domain located in the LC that includes a metalloprotease region containing a zinc-dependent endopeptidase activity which specifically targets core components of the neurotransmitter release apparatus; 2) a translocation domain contained within the amino-terminal half of the HC (H_{N}) that facilitates release of the LC from intracellular vesicles into the cytoplasm of the target cell; and 3) a binding domain found within the carboxyl-terminal half of the HC (H_{C}) that determines the binding activity and binding specificity of the toxin to the receptor complex located at the surface of the target cell.

The binding, translocation and enzymatic activity of these three functional domains are all necessary for toxicity. While all details of this process are not yet precisely known, the overall cellular intoxication mechanism whereby Clostridial toxins enter a neuron and inhibit neurotransmitter release is similar, regardless of serotype or subtype. Although the applicants have no wish to be limited by the following description, the intoxication mechanism can be described as comprising at least four steps: 1) receptor binding, 2) complex internalization, 3) light chain translocation, and 4) enzymatic target modification (FIG. 1). The process is initiated when the H_{C} binding domain of a Clostridial toxin binds to a toxin-specific receptor system located on the plasma membrane surface of a target cell. The binding specificity of a receptor complex is thought to be achieved, in part, by specific combinations of gangliosides and protein receptors that appear to distinctly comprise each Clostridial toxin receptor complex. Once bound, the toxin/receptor complexes are internalized by endocytosis and the internalized vesicles are sorted to specific intracellular routes. The translocation step appears to be triggered by the acidification of the vesicle compartment. This process seems to initiate important pH-dependent structural rearrangements that increase hydrophobicity, promote pore formation, and facilitate separation of the heavy and light chains of the toxin. Once separated, the light chain endopeptidase of the toxin is released from the intracellular vesicle into the cytosol where it appears to specifically target core components of the neurotransmitter release apparatus. These core proteins, vesicle-associated membrane protein (VAMP)/synaptobrevin, synaptosomal-associated protein of 25 kDa (SNAP-25) and Syntaxin, are necessary for synaptic vesicle docking and fusion at the nerve terminal and constitute members of the soluble N-ethylmaleimide-sensitive factor-attachment protein-receptor (SNARE) family. BoNT/A and BoNT/E cleave SNAP-25 in the carboxyl terminal region, releasing a nine or twenty six amino acid fragment, respectively, and BoNT/C1 also cleaves SNAP-25 near the carboxyl terminus releasing an eight amino acid fragment. The botulinum serotypes BoNT/B, BoNT/D, BoNT/F and BoNT/G, and tetanus toxin, act on the conserved central portion of VAMP, and release the amino terminal portion of VAMP into the cytosol. BoNT/C1 cleaves syntaxin at a single site near the cytosolic membrane surface. The selective proteolysis of synaptic SNAREs accounts for the block of neurotransmitter release caused by Clostridial toxins in vivo. The SNARE protein targets of Clostridial toxins are common to exocytosis in a variety of non-neuronal types; in these cells, as in neurons, light chain peptidase activity inhibits exocytosis, see, e.g., Yann Humeau et al., How Botulinum and Tetanus Neurotoxins Block Neurotransmitter Release, 82(5) Biochimie. 427-446 (2000); Kathryn Turton et al., Botulinum and Tetanus Neurotoxins: Structure, Function and Therapeutic Utility, 27(11) Trends Biochem. Sci. 552-558. (2002); Giovanna Lalli et al., The Journey of Tetanus and Botulinum Neurotoxins in Neurons, 11(9) Trends Microbiol. 431-437, (2003).

Aspects of the present disclosure comprise, in part, a cell from an established cell line. As used herein, the term "cell" refers to any eukaryotic cell susceptible to BoNT/A intoxication by a BoNT/A or any eukaryotic cell that can uptake a BoNT/A, as further defined in the claim. The term cell encompasses cells from a variety of organisms, such as, e.g., murine, rat, porcine, bovine, equine, primate and human cells; from a variety of cell types such as, e.g., neuronal and non-neuronal; and can be isolated from or part of a heterogeneous cell population, tissue or organism. As used herein, the term "established cell line" is synonymous with "immortal cell line," or "transformed cell line" and refers to a cell culture of cells selected for indefinite propagation from a cell population derived from an organism, tissue, or organ source. By definition, an established cell line excludes a cell culture of primary cells. As used herein, the term "primary cells" are cells harvested directly from fresh tissues or organs and do not have the potential to propagate indefinitely. An established cell line can comprise a heterogeneous population of cells or a uniform population of cells. An established cell line derived from a single cell is referred to as a clonal cell line. An established cell line can be one whose cells endogenously express all component necessary for the cells to undergo the overall cellular mechanism whereby a BoNT/A proteolytically cleaves a SNAP-25 substrate and encompasses the binding of a BoNT/A to a BoNT/A receptor, the internalization of the neurotoxin/receptor complex, the translocation of the BoNT/A light chain from an intracellular vesicle into the cytoplasm and the proteolytic cleavage of a SNAP-25. Alternatively, an established cell line can be one whose cells have had introduced from an exogenous source at least one component necessary for the cells to undergo the overall cellular mechanism whereby a BoNT/A proteolytically cleaves a SNAP-25 substrate and encompasses the binding of a BoNT/A to a BoNT/A receptor, the internalization of the neurotoxin/receptor complex, the translocation of the BoNT/A light chain from an intracellular vesicle into the cytoplasm and the proteolytic cleavage of a SNAP-25. Also referred to as a genetically-engineered cell line, cells from such an established cell line may, *e.g*., express an exogenous FGFR2, an exogenous FGFR3, an exogenous SV2, an exogenous SNAP-25, or any combination thereof. As used herein, the term "established clonal cell line" or "clonal cell line" refers to a cell culture of cells selected from a heterogenous population of cell types comprising an established cell line, which can also be referred to as a parental cell line. Thus, as a non-limiting example, the cells from the A6, A7, A9, A10, A11, B5, B10, C5, C11, C12, D7, D11, E11, F10, H1, H3, H8, H10, 1E3, 2B9, 2E4, 3B8, 3D5, 3G10, 4B5, 4C8, 5F3, AC9, AF4, BB3, BB10, BE3, BF8, CC11, CD6, CE6, CG8, CG10, DC4, DD10, DE7, YF5, and H1 1.4 clonal cell lines were selected from the heterogenous population of cell types comprising the SiMa cell line (or parental SiMa cell line) DSMZ ACC 164.

Aspects of the present disclosure comprise, in part, a cell from an established clonal cell line susceptible to BoNT/A intoxication cells of the invention are defined in the claim. As used herein, the terms "cell(s) susceptible to BoNT/A intoxication," "cell(s) susceptible to BoNT/A intoxication by a BoNT/A," or "cell(s) from an established clonal cell line susceptible to BoNT/A intoxication by a BoNT/A" refer to cell(s) that can undergo the overall cellular mechanism whereby a BoNT/A proteolytically cleaves a SNAP-25 substrate and encompasses the binding of a BoNT/A to a BoNT/A receptor, the internalization of the neurotoxin/receptor complex, the translocation of the BoNT/A light chain from an intracellular vesicle into the cytoplasm and the proteolytic cleavage of a SNAP-25. By definition, cell(s) susceptible to BoNT/A intoxication must express, or be engineered to express, at least one BoNT/A receptor and at least one SNAP-25 substrate. Non-limiting examples of a cell or cells from an established clonal cell line susceptible to BoNT/A intoxication, include A6, A7, A9, A10, A11, B5, B10, C5, C11, C12, D7, D11, E11, F10, H1, H3, H8, H10, 1E3, 2B9, 2D2, 2D6, 2E4, 3B8, 3D5, 3G10, 4B5, 4C8, 4D3, 5C10, 5F3, AC9, AF4, BB3, BB10, BE3, BF8, CC11, CD6, CE6, CG8, CG10, DC4, DD10, DE7, DF5, YB7, YF5, and H1 1.4 clonal cell lines. As used herein, the terms "cell(s) that can uptake BoNT/A" or "cell(s) comprising an established clonal cell line that can uptake BoNT/A" refer to cells that can undergo the overall cellular mechanism whereby a BoNT/A proteolytically cleaves a SNAP-25 substrate and encompasses the binding of a BoNT/A to a BoNT/A receptor, the internalization of the neurotoxin/receptor complex, the translocation of the BoNT/A light chain from an intracellular vesicle into the cytoplasm and the proteolytic cleavage of a SNAP-25. By definition, cell(s) that can uptake BoNT/A must express, or be engineered to express, at least one BoNT/A receptor and at least one SNAP-25 substrate. Non-limiting examples of a cell or cells from an established clonal cell line that can uptake BoNT/A, include A6, A7, A9, A10, A11, B5, B10, C5, C11, C12, D7, D11, E11. F10, H1, H3, H8, H10, 1E3, 2B9, 2D2, 2D6, 2E4, 3B8, 3D5, 3G10, 4B5, 4C8, 4D3, 5C10, 5F3, AC9, AF4, BB3, BB10, BE3, BF8, CC11, CD6, CE6, CG8, CG10, DC4, DD10, DE7, DF5, YB7, YF5, and H1 1.4 clonal cell lines. Cell lines comprising cell(s) from an established clonal cell line susceptible to BoNT/A intoxication or cell(s) that can uptake BoNT/A are referred to as a "responder cell line."

Aspects of the present disclosure comprise, in part, cells from an established clonal cell line more susceptible to BoNT/A intoxication. As used herein, the terms "cell(s) more susceptible to BoNT/A intoxication," "cell(s) more susceptible to BoNT/A intoxication by a BoNT/A," or "cell(s) from an established clonal cell line more susceptible to BoNT/A intoxication by a BoNT/A" refer to cell(s) that undergo the overall cellular mechanism whereby a BoNT/A proteolytically cleaves a SNAP-25 substrate to a greater degree as compared to a cell(s) comprising a parental SiMa cell line, such as, e.g., parental SiMa cell line DSMZ ACC 164. Non-limiting examples of a cell or cells from an established clonal cell line more susceptible to BoNT/A intoxication, include a cell or cells from a A10, H1, 2E4, 3B8, 3D5, 5F3, AF4, BB3, BB10, DC4, or H1 1.4 clonal cell lines. As used herein, the terms "cell(s) that uptake BoNT/A more" or "cell(s) comprising an established clonal cell line that uptake BoNT/A more" refer to cells that undergo the overall cellular mechanism whereby a BoNT/A proteolytically cleaves a SNAP-25 substrate to a greater degree as compared to a cell(s) comprising a parental SiMa cell line, such as, e.g., parental SiMa cell line DSMZ ACC 164. Non-limiting examples of a cell or cells from an established clonal cell line that can uptake BoNT/A more, include a cell or cells from a A10, H1, 2E4, 3B8, 3D5, 5F3, AF4, BB3, BB10, DC4, or H1 1.4 clonal cell lines. Cell lines comprising cell(s) from an established clonal cell line more susceptible to BoNT/A intoxication or cell(s) that can uptake BoNT/A more are referred to as a "high responder cell line."

Aspects of the present disclosure comprise, in part, cells from an established clonal cell line less susceptible to BoNT/A intoxication. As used herein, the terms "cell(s) less susceptible to BoNT/A intoxication," "cell(s) less susceptible to BoNT/A intoxication by a BoNT/A," or "cell(s) from an established clonal cell line less susceptible to BoNT/A intoxication by a BoNT/A" refer to cell(s) that undergo the overall cellular mechanism whereby a BoNT/A proteolytically cleaves a SNAP-25 substrate to a lesser degree as compared to a cell(s) comprising a parental SiMa cell line, such as, e.g., parental SiMa cell line DSMZ ACC 164. Non-limiting examples of a cell or cells from an established clonal cell line less susceptible to BoNT/A intoxication, include A7, B10, D11, H10, 2D2, 2D6, 4D3, 5C10, DF5, and YB7 clonal cell lines. As used herein, the terms "cell(s) that uptake BoNT/A less" or "cell(s) comprising an established clonal cell line that uptake BoNT/A less" refer to cells that undergo the overall cellular mechanism whereby a BoNT/A proteolytically cleaves a SNAP-25 substrate to a lesser degree as compared to a cell(s) comprising a parental SiMa cell line, such as, e.g., parental SiMa cell line DSMZ ACC 164. Non-limiting examples of a cell or cells from an established clonal cell line that can uptake BoNT/A less, include A7, B10, D11, H10, 2D2, 2D6, 4D3, 5C10, DF5, and YB7 clonal cell lines. Cell lines comprising cell(s) from an established clonal cell line less susceptible to BoNT/A intoxication or cell(s) that can uptake BoNT/A less are referred to as a "low responder cell line."

Aspects of the present disclosure comprise, in part, a cell from an established clonal cell line not susceptible to BoNT/A intoxication. As used herein, the terms "cell(s) not susceptible to BoNT/A intoxication," "cell(s) not susceptible to BoNT/A intoxication by a BoNT/A," or "cell(s) from an established clonal cell line not susceptible to BoNT/A intoxication by a BoNT/A" refer to cell(s) that cannot undergo the overall cellular mechanism whereby a BoNT/A proteolytically cleaves a SNAP-25 substrate. As used herein, the terms "cell(s) that cannot uptake BoNT/A" or "cell(s) comprising an established clonal cell line that cannot uptake BoNT/A" refer to cells that cannot undergo the overall cellular mechanism whereby a BoNT/A proteolytically cleaves a SNAP-25 substrate. Cell lines comprising cell(s) from an established clonal cell line not susceptible to BoNT/A intoxication or cell(s) that cannot uptake BoNT/A are referred to as a "non-responder cell line."

Thus in an embodiment, cells from an established clonal cell line are susceptible to BoNT/A intoxication. In aspects of this embodiment, cells from an established clonal cell line are susceptible to BoNT/A intoxication by, e.g., about 500 pM or less, about 400 pM or less, about 300 pM or less, about 200 pM or less, or about 100 pM or less of a BoNT/A. In other aspects of this embodiment, cells from an established clonal cell line are susceptible to BoNT/A intoxication by, e.g., about 90 pM or less, about 80 pM or less, about 70 pM or less, about 60 pM or less, about 50 pM or less, about 40 pM or less, about 30 pM or less, about 20 pM or less, or about 10 pM or less of a BoNT/A. In still other aspects, cells from an established clonal cell line are susceptible to BoNT/A intoxication by, e.g., about 9 pM or less, about 8 pM or less, about 7 pM or less, about 6 pM or less, about 5 pM or less, about 4 pM or less, about 3 pM or less, about 2 pM or less, or about 1 pM or less of a BoNT/A. In yet other aspects, cells from an established clonal cell line are susceptible to BoNT/A intoxication by, e.g., about 0.9 pM or less, about 0.8 pM or less, about 0.7 pM or less, about 0.6 pM or less, about 0.5 pM or less, about 0.4 pM or less, about 0.3 pM or less, about 0.2 pM, or about 0.1 pM or less of a BoNT/A. As used herein, the term "about" when qualifying a value of a stated item, number, percentage, or term refers to a range of plus or minus ten percent of the value of the stated item, percentage, parameter, or term.

In another embodiment, cells comprising an established clonal cell line can uptake a BoNT/A. In aspects of this embodiment, cells comprising an established clonal cell line can uptake, e.g., about 500 pM or less, about 400 pM or less, about 300 pM or less, about 200 pM or less, or about 100 pM or less of a BoNT/A. In other aspects of this embodiment, cells comprising an established clonal cell line possess the ability to uptake about 90 pM or less, about 80 pM or less, about 70 pM or less, about 60 pM or less, about 50 pM or less, about 40 pM or less, about 30 pM or less, about 20 pM or less, or about 10 pM or less of a BoNT/A. In still other aspects, cells comprising an established clonal cell line possess the ability to uptake about 9 pM or less, about 8 pM or less, about 7 pM or less, about 6 pM or less, about 5 pM or less, about 4 pM or less, about 3 pM or less, about 2 pM or less, or about 1 pM or less of a BoNT/A. In yet other aspects, cells comprising an established clonal cell line possess the ability to uptake about 0.9 pM or less, about 0.8 pM or less, about 0.7 pM or less, about 0.6 pM or less, about 0.5 pM or less, about 0.4 pM or less, about 0.3 pM or less, about 0.2 pM or less, or about 0.1 pM or less of a BoNT/A. In other aspects, cells comprising an established clonal cell line can uptake BoNT/A from, *e.g*., about 0.01 pM to about 100 pM, about 0.01 pM to about 75 pM, about 0.01 pM to about 50 pM, about 0.01 pM to about 25 pM, about 0.01 pM to about 20 pM, about 0.01 pM to about 15 pM, about 0.01 pM to about 10 pM, about 0.01 pM to about 5 pM, about 0.001 pM to about 100 pM, about 0.001 pM to about 75 pM, about 0.001 pM to about 50 pM, about 0.001 pM to about 25 pM, about 0.001 pM to about 20 pM, about 0.001 pM to about 15 pM, about 0.001 pM to about 10 pM, or about 0.001 pM to about 5 pM of BoNT/A.

In another embodiment, cells from an established clonal cell line are not susceptible to BoNT/A intoxication. In aspects of this embodiment, cells from an established clonal cell line are not susceptible to BoNT/A intoxication by, *e.g*., about 500 pM or less, about 400 pM or less, about 300 pM or less, about 200 pM or less, or about 100 pM or less of a BoNT/A. In other aspects of this embodiment, cells from an established clonal cell line are susceptible to BoNT/A intoxication by, *e.g*., about 90 pM or less, about 80 pM or less, about 70 pM or less, about 60 pM or less, about 50 pM or less, about 40 pM or less, about 30 pM or less, about 20 pM or less A, or about 10 pM or less of a BoNT/A. In still other aspects, cells from an established clonal cell line are not susceptible to BoNT/A intoxication by, *e.g*., about 9 pM or less, about 8 pM or less, about 7 pM or less, about 6 pM or less, about 5 pM or less, about 4 pM or less, about 3 pM or less, about 2 pM or less, or about 1 pM or less of a BoNT/A. In yet other aspects, cells from an established clonal cell line are not susceptible to BoNT/A intoxication by, *e.g*., about 0.9 pM or less, about 0.8 pM or less, about 0.7 pM or less, about 0.6 pM or less, about 0.5 pM or less, about 0.4 pM or less, about 0.3 pM or less, about 0.2 pM, or about 0.1 pM or less of a BoNT/A. In other aspects, cells from an established clonal cell line are not susceptible to BoNT/A intoxication from, *e.g*., about 0.01 pM to about 100 pM, about 0.01 pM to about 75 pM, about 0.01 pM to about 50 pM, about 0.01 pM to about 25 pM, about 0.01 pM to about 20 pM, about 0.01 pM to about 15 pM, about 0.01 pM to about 10 pM, about 0.01 pM to about 5 pM, about 0.001 pM to about 100 pM, about 0.001 pM to about 75 pM, about 0.001 pM to about 50 pM, about 0.001 pM to about 25 pM, about 0.001 pM to about 20 pM, about 0.001 pM to about 15 pM, about 0.001 pM to about 10 pM, or about 0.001 pM to about 5 pM of BoNT/A.

Aspects of the present disclosure comprise, in part, cells from an established clonal cell line susceptible to BoNT/A intoxication that are more stable than cells from a parental SiMa cell line, such as, *e.g*., parental SiMa cell line DSMZ ACC 164. As used herein, the term "stable" refers to cells from an established clonal cell line for a particular passage number that exhibit a relative EC₅₀, sensitivity, efficacy, well-defined upper asymptote, and/or a well-defined dose-response curve for BoNT/A activity that is similar to the values for relative EC₅₀, sensitivity, efficacy, well-defined upper asymptote, and/or a well-defined dose-response curve exhibited by cells from a parental SiMa cell line, such as, *e.g*., parental SiMa cell line DSMZ ACC 164, at the same or similar passage number, where the same assay conditions and the same BoNT/A (or molecule) are used in both assays.

Thus in an embodiment, cells from an established clonal cell line are more stable as compared to a parental SiMa cell line. In an aspect of this embodiment, cells from an established clonal cell line are more stable as compared to the parental SiMa cell line DSMZ ACC 164. In other aspects of this embodiment, cells from an established clonal cell line are more stable for, *e.g*., at least 5 more passages, at least 10 more passages, at least 15 more passages, at least 20 more passages, at least 25 more passages, or at least 30 more passages, as compared to a parental SiMa cell line. In yet other aspects of this embodiment, cells from an established clonal cell line are more stable for, *e.g*., at least 5 more passages, at least 10 more passages, at least 15 more passages, at least 20 more passages, at least 25 more passages, or at least 30 more passages, as compared to a parental SiMa cell line DSMZ ACC 164.

Aspects of the present disclosure comprise, in part, cells from an established clonal cell line susceptible to BoNT/A intoxication that are stable over a plurality of cell passages. As used herein, the term "stable" refers to cells from an established clonal cell line for a particular passage number that exhibit a relative EC₅₀, sensitivity, efficacy, well-defined upper asymptote, and/or a well-defined dose-response curve for BoNT/A activity that is similar to the values for relative EC₅₀, sensitivity, efficacy, well-defined upper asymptote, and/or a well-defined dose-response curve exhibited by cells from the same established clonal cell line, but from a prior passage or passages, where the same assay conditions and the same BoNT/A (or molecule) are used in both assays.

Cells from an established cell line disclosed in the present specification can exhibit consistent sensitivity to BoNT/A activity over a plurality of cell passages. As used herein, the term "sensitivity to BoNT/A activity" refers to the lowest dose that an assay can measure consistently above the signal detected by a non-treatment control or background signal.

Thus, in an embodiment, cells from the established clonal cell line exhibit a sensitivity for BoNT/A activity for any given passages that is *e.g*., 100 pM or less, about 80 pM or less, about 70 pM or less, about 60 pM or less, about 50 pM or less, about 40 pM or less, about 30 pM or less, about 20 pM or less, about 10 pM or less, about 1 pM or less, about 0.9 pM or less, about 0.8 pM or less, about 0.7 pM or less, about 0.6 pM or less, about 0.5 pM or less, about 0.4 pM or less, about 0.3 pM or less, about 0.2 pM or less, or about 0.1 pM or less of a BoNT/A. In aspects of this embodiment, cells from the established clonal cell line exhibit a sensitivity for BoNT/A activity for any given passages that is, *e.g*., about 0.01 pM to about 100 pM, about 0.01 pM to about 75 pM, about 0.01 pM to about 50 pM, about 0.01 pM to about 25 pM, about 0.01 pM to about 20 pM, about 0.01 pM to about 15 pM, about 0.01 pM to about 10 pM, about 0.01 pM to about 5 pM, about 0.001 pM to about 100 pM, about 0.001 pM to about 75 pM, about 0.001 pM to about 50 pM, about 0.001 pM to about 25 pM, about 0.001 pM to about 20 pM, about 0.001 pM to about 15 pM, about 0.001 pM to about 10 pM, or about 0.001 pM to about 5 pM of BoNT/A.

In another embodiment, cells from the established clonal cell line exhibit a sensitivity for BoNT/A activity that is about 100 pM or less, about 75 pM or less, about 50 pM or less, about 25 pM or less, less about 20 pM or less, about 15 pM or less, about 10 pM or less, or about 1 pM or less for, *e.g*., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, or 50 or more cell passages. In other aspects of this embodiment, cells from the established clonal cell line exhibit a sensitivity for BoNT/A activity that is about 100 pM or less, about 75 pM or less, about 50 pM or less, about 25 pM or less, less about 20 pM or less, about 15 pM or less, about 10 pM or less, or about 1 pM or less for, *e.g*., about 15 to about 60 passages, about 20 to about 60 passages, about 25 to about 60 passages, about 30 to about 60 passages, about 35 to about 60 passages, about 40 to about 60 passages, about 45 to about 60 passages, about 50 to about 60 passages, about 15 to about 50 passages, about 20 to about 50 passages, about 25 to about 50 passages, about 30 to about 50 passages, about 35 to about 50 passages, about 40 to about 50 passages, about 15 to about 40 passages, about 20 to about 40 passages, about 25 to about 40 passages, or about 30 to about 40 passages.

Cells from an established cell line disclosed in the present specification can exhibit a consistent relative efficacy of BoNT/A uptake, BoNT/A activity, or BoNT/A intoxication over a plurality of cell passages. As used herein, the term "relative efficacy" refers to how well the upper asymptote for the BoNT/A activity detected in the current assay run compares to the upper asymptote for the BoNT/A activity detected in a reference standard, a reference molecule, or a reference passage number used on that assay. As used herein, the term "signal to noise ratio for the upper asymptote" refers to the signal detected in an assay at the upper limit of detection divided by the signal detected by a non-treatment control or background signal. The upper limit of detection is the highest dose that an assay can measure consistently before saturation of the signal occurs.

Thus, in an embodiment, cells from an established cell line disclosed in the present specification can exhibit a well defined upper asymptote over a plurality of cell passages and maintain a signal to noise ratio that is consistent and adequate for the assay. In aspects of this embodiment, cells from an established cell line disclosed in the present specification must have a signal to noise ratio for the upper asymptote for BoNT/A activity of, *e.g*., at least 3:1, at least 4:1, at least 5:1, at least 6:1, at least 7:1, at least 8:1, at least 9:1, at least 10:1, at least 15:1, at least 20:1, at least 25:1, at least 30:1, at least 35:1, at least 40:1, at least 45:1, at least 50:1, at least 60:1, at least 70:1, at least 80:1, at least 90:1, or at least 100:1, at least 150:1, at least 200:1, at least 250:1, at least 300:1, at least 350:1, at least 400:1, at least 450:1, at least 500:1, at least 550:1, or at least 600:1, over, *e.g*., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, or 50 or more cell passages. In other aspects of this embodiment, cells from an established cell line disclosed in the present specification must have a signal to noise ratio for the upper asymptote for BoNT/A activity of, e.g., at least 3:1, at least 4:1, at least 5:1, at least 6:1, at least 7:1, at least 8:1, at least 9:1, at least 10:1, at least 15:1, at least 20:1, at least 25:1, at least 30:1, at least 35:1, at least 40:1, at least 45:1, at least 50:1, at least 60:1, at least 70:1, at least 80:1, at least 90:1, or at least 100:1, at least 150:1, at least 200:1, at least 250:1, at least 300:1, at least 350:1, at least 400:1, at least 450:1, at least 500:1, at least 550:1, or at least 600:1, over, e.g., about 15 to about 60 passages, about 20 to about 60 passages, about 25 to about 60 passages, about 30 to about 60 passages, about 35 to about 60 passages, about 40 to about 60 passages, about 45 to about 60 passages, about 50 to about 60 passages, about 15 to about 50 passages, about 20 to about 50 passages, about 25 to about 50 passages, about 30 to about 50 passages, about 35 to about 50 passages, about 40 to about 50 passages, about 15 to about 40 passages, about 20 to about 40 passages, about 25 to about 40 passages, or about 30 to about 40 passages.

Cells from an established cell line disclosed in the present specification can exhibit a well defined dose-response curve for BoNT/A activity over a plurality of cell passages. As used herein, the term "dose-response curve" refers to the how well the raw data fits the statistical model of choice for that assay. As a non-limiting example, a sigmoidal curve with a four parameter logistics fit is a dose-response curve for an enzymatic activity assay, such as, *e.g*., a potency assay. As another non-limiting example, a ligand binding with one site saturation fit is a dose-response curve for a ligand/antibody binding assay.

Thus, in an embodiment, cells from an established cell line disclosed in the present specification exhibit a well defined dose-response curve for BoNT/A activity over a plurality of cell passages. In aspects of this embodiment, cells from an established cell line disclosed in the present specification exhibit a well defined dose-response curve for BoNT/A activity over, *e.g*., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, or 50 or more cell passages. In other aspects of this embodiment, cells from an established cell line disclosed in the present specification exhibit a well defined dose-response curve for BoNT/A activity over, *e.g*., about 15 to about 60 passages, about 20 to about 60 passages, about 25 to about 60 passages, about 30 to about 60 passages, about 35 to about 60 passages, about 40 to about 60 passages, about 45 to about 60 passages, about 50 to about 60 passages, about 15 to about 50 passages, about 20 to about 50 passages, about 25 to about 50 passages, about 30 to about 50 passages, about 35 to about 50 passages, about 40 to about 50 passages, about 15 to about 40 passages, about 20 to about 40 passages, about 25 to about 40 passages, or about 30 to about 40 passages.

Cells from an established cell line disclosed in the present specification can exhibit a consistent relative EC₅₀ value for BoNT/A activity over a plurality of cell passages. As used herein, the term "relative EC₅₀" or "relative EC₅₀ value" refers to an EC₅₀ value of BoNT/A activity that is normalized against theEC₅₀ calculated for a reference standard, a reference molecule, or a reference passage number used on that assay.

Thus, in an embodiment, cells from an established clonal cell line exhibit a consistent relative EC₅₀ for BoNT/A activity over a plurality of cell passages. In aspects of this embodiment, cells from an established clonal cell line exhibit a consistent relative EC₅₀ for BoNT/A activity that is, *e.g*., about ± 10%, about ± 20%, about ± 30%, about ± 40%, about ± 50%, about 60%, about 70%, or about ± 75%, the relative EC₅₀ for BoNT/A activity over, *e.g*., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, or 50 or more cell passages. In other aspects of this embodiment, cells from an established clonal cell line exhibit a relative EC₅₀ for BoNT/A activity that is, *e.g*., about ± 10% to about 75%, about ± 10% to about 70%, about ± 10% to about 60%, about ± 10% to about 50%, about ± 10% to about 40%, about ± 10% to about 30%, or about ± 10% to about 20% the relative EC₅₀ for BoNT/A activity over, *e.g*., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, or 50 or more cell passages.

Aspects of the present disclosure comprise, in part, cells from an established clonal cell line susceptible to BoNT/A intoxication that exhibit at least a 1.5-fold difference in gene expression levels of one or more genes listed in Tables 5, 6, 7, or 8 as compared to the expression levels of these genes in cells from the 2D6 cell line. In other aspects of this embodiment, cells from an established clonal cell line susceptible to BoNT/A intoxication exhibit a difference in gene expression levels of one or more genes listed in Tables 5, 6, 7, or 8 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from the 2D6 cell line. In other aspects of this embodiment, cells from an established clonal cell line susceptible to BoNT/A intoxication exhibit a difference in gene expression levels of, *e.g.*, 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 5, 6, 7, or 8 as compared to the expression levels of these genes in cells from the 2D6 cell line. In yet other aspects, cells from an established clonal cell line susceptible to BoNT/A intoxication exhibit a difference in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 5, 6, 7, or 8 as compared to the expression levels of these genes in cells from the 2D6 cell line. The log ratio in Tables 5, 6, 7, and 8 represent log₂ values where 0.585 is log₂(1.5) which is a 1.5-fold difference, 1 is log₂(2) which is a 2-fold difference, 1.584 is log₂(3) which is a 3-fold difference, 2 is log₂(4) which is a 4-fold difference, 2.321 is log₂(5) which is a 5-fold difference, 2.584 is log₂(6) which is a 6-fold difference, 2.807 is log₂(7) which is a 7-fold difference, 3 is log₂(8) which is a 8-fold difference, 3.169 is log₂(9) which is a 9-fold difference, and 3.321 is log₂(10) which is a 10-fold difference.

In an embodiment, cells from an established clonal cell line susceptible to BoNT/A intoxication exhibit at least a 1.5-fold increase in gene expression levels of one or more genes listed in Tables 5 or 7 as compared to the expression levels of these genes in cells from the 2D6 cell line. In aspects of this embodiment, cells from an established clonal cell line susceptible to BoNT/A intoxication exhibit an increase in gene expression levels of one or more genes listed in Tables 5 or 7 of, *e.g*., at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from the 2D6 cell line. In other aspects of this embodiment,cells from an established clonal cell line susceptible to BoNT/A intoxication exhibit an increase in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 5 or 7 as compared to the expression levels of these genes in cells from the 2D6 cell line. In yet other aspects, cells from an established clonal cell line susceptible to BoNT/A intoxication exhibit an increase in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 5 or 7 as compared to the expression levels of these genes in cells from the 2D6 cell line.

In an embodiment, cells from an established clonal cell line susceptible to BoNT/A intoxication exhibit at least a 1.5-fold increase in gene expression levels of one or more genes selected from A2BP1, ACO1, ACOT7, ACOT9, ACTL6A, ACTL6B, ADRBK2, AGPS, AIDA, AKAP13, ALCAM, ALDH7A1, AMOTL1, ANAPC7, ANKRD13A, ANKRD54, ANKS1B, ANLN, ANP32E, ANXA6, APAF1, APBB2, APOO, ARF3, ARHGAP11A, ARHGAP24, ARHGAP24, ARHGAP6, ARHGEF3, ARHGEF6, ARL13B, ARL6, ARL6IP1, ASCL1, ASF1A, ASF1B, ASXL3, ATAD2, ATP6V1A, AURKA, AURKB, BARD1, BASP1, BAX, BCL11A, BCR, BNC2, BOK, BRCA1, BRCA2, BRD4, BRI3BP, BRIP1, BTBD3, BTG3, BUB1, BUB1B, BUB3, BVES, C10ORF58, C10ORF78, C11ORF75, C12ORF48, C12ORF49, C14ORF106, C14ORF143, C15ORF23, C15ORF42, C18ORF54, C1ORF112, C1ORF183, C1ORF43, C1QL1, C20ORF108, C20ORF20, C20ORF7, C20ORF72, C22ORF28, C22ORF29, C22ORF39, C3ORF14, C3ORF70, C4ORF46, C4ORF49, C5ORF32, C6ORF115, C9ORF100, CARHSP1, CBLB, CBWD1, CBX5, CCDC109B, CCDC117, CCDC15, CCDC21, CCDC3, CCDC34, CCDC52, CCDC86, CCDC99, CCNB1, CCNE2, CCNF, CCNYL1, CD24, CD47, CD9, CDC2, CDC25B, CDC42EP4, CDC45L, CDC5L, CDC6, CDCA2, CDCA4, CDCA5, CDCA7, CDCA8, CDH2, CDK2, CDK2AP1, CDK6, CDKAL1, CDKN2C, CDKN2D, CDO1, CDS2, CECR5, CELSR3, CENPF, CENPH), CENPI, CENPJ, CENPK, CENPL, CENPM, CENPN, CENPO, CEP135, CEP152, CEP55, CEP78, CEP97, CHAF1A, CHD6, CHEK1, CHEK2, CHMP4B, CHRNA3, CHRNA7, CHST15, CIT, CKAP2, CKAP2L, CKLF, CKS1B, CLDND1, CLSTN2, CMTM7, CNN3, CNOT4, CNP, CNTN1, CNTN4, COBLL1, COQ3, CPNE4, CPT1A, CPVL, CRISPLD1, CRTAC1, CRYBG3, CRYZ, CSE1L, CSRP2, CSRP2BP, CSTF1, CTDSPL2, CTNNBL1, CTSL2, CUGBP2, CXCR4, CYTSA, DACT1, DAZ1, DBF4B, DBH, DCLRE1B, DCPS, DDAH2, DDT, DEK, DENR, DEPDC1, DEPDC1B, DERA, DGCR14, DGKE, DHX15, DHX35, DIAPH1, DIAPH3, DKFZP434L187, DLEU2, DLGAP5, DLL3, DLX6, DNA2, DNM3, DNMT3B, DOCK10, DOK4, DOK5, DPF1, DPYD, DPYSL3, DRAM1, DRG1, DSCC1, DSN1, DTL, DTNBP1, DTYMK, DVL2, DYNLT3, DYRK4, E2F1, E2F2, E2F7, E2F8, EAF2, EBF1, ECT2, EFNA5, EFNB2, ELAVL4, ELOVL7, EME1, EMILIN2, EMILIN3, EML1, ENC1, EPB41L5, EPOR, ERCC6L, ESCO2, ESF1, ESPL1, ETS1, ETV1, EXO1, EXOC5, EXOSC6, EXPH5, EZH2, FAM101B, FAM105A, FAM110A, FAM114A1, FAM118A, FAM120C, FAM129B, FAM13A, FAM162B, FAM181B, FAM19A4, FAM19A5, FAM54A, FAM64A, FAM7A3, FAM83D, FANCG, FANCI, FANCL, FANCM, FARP1, FAT1, FBLN1, FBN1, FBP1, FBXO43, FBXO5, FEN1, FGFR2, FH, FIGNL1, FKBP5, FNDC5, FOXD1, FOXN3, FRMD6, FRZB, FSTL1, FZD2, FZD5, FZD8, G2E3, GABBR2, GAP43, GAP43, GAS2L3, GATM, GEMIN4, GFPT2, GFRA1, GFRA2, GGCX, GINS1, GINS3, GJC1, GLDC, GMNN, GNAI1, GNASAS, GNB4, GNG11, GNG12, GNG4, GPAM, GPN3, GPR125, GPR161, GPSM2, GPX3, GRP, GSG2, GSS, GSTCD, GTSE1, GTSF1, GXYLT1, H1F0, H2AFX, HAT1, HAUS6, HDAC8, HEG1, HES6, HGF, HIC2, HMG4L, HMGB1, HMGB2, HMGB3, HMGXB4, HMMR, HOOK3, HPS4, HRH3, HS3ST2, HS6ST2, HSPB11, HTATSF1, IDH2, IFI27L1, IQGAP1, ITGA6, ITGAV, ITGB5, ITPRIP, JARID2, KCNG3, KCNJ8, KCNN1, KCTD12, KDELC2, KHDRBS3, KIAA0101, KIAA0406, KIAA1211, KIAA1524, KIF11, KIF14, KIF16B, KIF18A, KIF23, KIFC1, KLF7, KLHL13, KLHL5, KNTC1, KPNA2, LBH, LGR5, LHFPL2, LIFR, LIG3, LMF2, LMNB1, LOC100127983, LOC100128844, LOC100288551, LOC340109, LOC344595, LOC440288, LOC642597, LOC728052, LPAR1, LRFN2, LRRC1, LSM4, LUM, MAB21L1, MAB21L2, MAGEH1, MAN1A1, MAN2A1, MAOA, MAP3K13, MAPK11, MAPK12, MAPKAPK3, MASTL, MBD2, MCAM, MCM10, MCM3, MCM4, MCM5, MCM6, MCM7, MCM8, MDC1, MDM1, MED20, MEIS1, MEIS2, MELK, MEST, MFAP2, MGAT5B, MINA, MKI67, MKL1, MLF1IP, MMD, MNS1, MPHOSPH9, MPP5, MPPED2, MRC2, MRPL35, MRPL49, MRS2, MSN, MTF2, MTHFD1, MTUS1, MVK, MYBL1, MYBL2, MYD88, MYO1B, MYO6, MYST1, NAAA, NAGA, NANP, NARG1, NASP, NAT11, NAT13, NCAPD3, NCAPG, NCAPG2, NCAPH, NCAPH2, NDN, NEDD1, NEDD9, NEIL3, NELL2, NFIB, NOC4L, NOS1, NR3C1, NRG3, NRM, NRXN1, NSMCE4A, NTAN1, NUCKS1, NUDT1, NUF2, NUP107, NUP37, NUP43, NUP50, NUP93, NUSAP1, NXPH1, NXT2, ODZ3, ODZ4, OIP5, ORC1L, ORC6L, OSBPL3, PAICS, PANK2, PARP3, PASK, PBK, PCDH17, PCDH8, PCGF5, PCNA, PDCL, PDE5A, PDLIM5, PDRG1, PEG3, PELI2, PELO, PEX13, PEX26, PFKFB3, PGAM5, PGM2, PHF19, PHF20, PHF21B, PHF5A, PHLPP1, PHYHIPL, PI4KA, PIP4K2A, PIR, PKMYT1, PLCL2, PLD5, PLEKHF2, PLK2, PLK4, PLS3, PLXNA2, PLXNA4, PLXNB2, PM20D2, POLA1, POLA2, POLE, POLE2, POLQ, POSTN, PPAP2B, PPAT, PPIF, PPIL5, PPM1F, PPP1R13B, PPP1R3C, PPP2R2B, PPP3CB, PRIM1, PRIM2, PRLHR, PRPS1, PRSS12, PSMA7, PSMC3IP, PSMD5, PSMD9, PSRC1, PTER, PTGES2, PTGFRN, PTGR1, PTPRE, PTPRG, PTPRK, PTPRM, PTTG1, PVRL3, PXMP4, PXN, RAB35, RAB3B, RAD18, RAD51, RAD51C, RALY, RAN, RANBP1, RANGAP1, RASEF, RBBP7, RBBP9, RBL1, RBPMS, RBX1, RECQL4, REEP1, RELB, RELL1, REXO2, RFC1, RFC2, RFC3, RFC4, RFC5, RGMA, RGS5, RILPL2, RIMBP2, RMI1, RNASEH2A, RNF182, RNF26, RNF34, RPA1, RPP30, RRM1, RRM2, RRP7A, RSRC1, S1PR3, SALL4, SAP30, SASS6, SBF2, SCD5, SCML1, SCML2, SDC1, SEC23A, SEC61A2, SELM, SEMA6A, SEMA6D, SESN3, SEZ6L, SFI1, SFRP1, SGOL2, SH3D19, SHCBP1, SHMT1, SHOX2, SIM1, SIVA1, SKA1, SKA2, SKA3, SKP2, SLC25A10, SLC26A2, SLC2A4RG, SLC2A8, SLC43A3, SLC44A5, SLC7A2, SLC8A1, SLCO3A1, SLIT3, SLITRK5, SLK, SLMO2, SMC1A, SMC2, SMC4, SMC6, SMO, SMYD5, SNAI2, SNAP29, SNCAIP, SNRPD3, SNX18, SNX5, SORD, SOX4, SPAG6, SPARC, SPATS2L, SPC24, SPC25, SPOCK1, SPSB4, SRGAP1, SRRD, SRRM4, SSH2, ST8SIA1, ST8SIA2, ST8SIA4, STARD4, STIL, STK17A, STMN3, STOM, STON2, STRA6, SUSD5, SUV39H1, SVIP, SYN2, SYNE2, SYNM, SYT17, SYTL4, TBC1D1, TBX3, TCF19, TCF7L2, TCFL5, TDRKH, TEAD3, TESC, TFDP1, TFDP2, TFPI2, TH1L, THOC4, TIFA, TIMP3, TLE3, TLE4, TMCC3, TMEM107, TMEM132C, TMEM151B, TMEM170B, TMEM178, TMEM194A, TMEM38B, TMEM48, TMEM56, TMEM98, TMPO, TMSB15B, TNFAIP8, TOB2, TOMM34, TPBG, TPTE, TPX2, TRIM29, TRIM36, TRIM68, TRIM9, TRIP13, TROAP, TSHZ3, TSPAN14, TSPAN4, TSPAN5, TTC28, TTC9, TUBA1B, TUBB, TWIST1, TXNRD1, TYMS, TYRO3, UBE2C, UBE2L3, UBE2V1, UBE3B, UCK1, UCP2, UFD1L, UHRF1, UNC119B, UNG, UQCC, USP1, USP48, UTP18, VAPB, VAV3, VPS29, VSTM2L, WDHD1, WDR51A, WDR53, WDR62, WDR67, WEE1, WEE1, WHSC1, WRAP53, WWC3, XRCC4, XRCC6, ZAK, ZFHX4, ZFP82, ZGPAT, ZNF215, ZNF217, ZNF238, ZNF253, ZNF280B, ZNF367, ZNF43, ZNF443, ZNF503, ZNF521, ZNF560, ZNF608, ZNF626, ZNF681, ZNF71, ZNF823, ZNF85, ZNF92, ZNF93, and/or ZWINT, as compared to the expression levels of these genes in cells from the 2D6 cell line.

In an aspect of this embodiment, cells from an established clonal cell line susceptible to BoNT/A intoxication exhibit at least a 1.5-fold increase in gene expression levels of one or more genes selected from A2BP1, ACOT9, ALCAM, ANLN, ARHGAP24, ARHGAP6, ARHGEF3, ARHGEF6, ASCL1, ASF1B, ATAD2, AURKA, AURKB, BARD1, BASP1, BNC2, BRCA2, BRIP1, BTG3, BUB1, BUB1B, BVES, C11ORF75, C12ORF48, C14ORF106, C15ORF42, C18ORF54, C1ORF112, C3ORF70, C4ORF49, CCDC109B, CCDC3, CCNB1, CCNE2, CD9, CDC2, CDC25B, CDC45L, CDC6, CDCA2, CDCA5, CDCA8, CDK2, CDKN2D, CENPF, CENPI, CENPL, CENPN, CEP55, CHEK1, CHEK2, CHRNA7, CIT, CKAP2L, CLSTN2, CNTN1, CNTN4, CPVL, CRYBG3, CSRP2, CTSL2, CUGBP2, CXCR4, DAZ1, DEPDC1, DEPDC1B, DIAPH3, DLGAP5, DNA2, DOK5, DPYD, DPYSL3, DSCC1, DSN1, DTL, DYNLT3, E2F1, E2F2, E2F7, E2F8, ECT2, EFNB2, ELOVL7, EME1, EMILIN2, EMILIN3, EML1, ENC1, ERCC6L, ESCO2, ESPL1, ETV1, EXO1, EXPH5, FAM101B, FAM114A1, FAM54A, FAM64A, FAM7A3, FAM83D, FANCI, FAT1, FBLN1, FBP1, FBXO43, FGFR2, FNDC5, FOXD1, FRMD6, FRZB, FZD5, GAS2L3, GFRA2, GINS1, GINS3, GNAI1, GNB4, GNG11, GNG12, GPSM2, GRP, GTSE1, GTSF1, HGF, HMMR, HS3ST2, ITGA6, ITPRIP, KCNG3, KCTD12, KDELC2, KIAA0101, KIAA1524, KIF11, KIF14, KIF18A, KIF23, KIFC1, LOC340109, LOC642597, LOC728052, LOC728052, LPAR1, LUM, MAB21L1, MAB21L2, MAOA, MCM10, MCM5, MELK, MINA, MKI67, MLF1IP, MPPED2, MRC2, MSN, MYBL1, MYBL2, MYO6, NCAPG, NCAPH, NDN, NEDD9, NEIL3, NR3C1, NRXN1, NUF2, NUSAP1, OIP5, ORC1L, OSBPL3, PBK, PCGF5, PDE5A, PEG3, PELO, PFKFB3, PHLPP1, PLD5, PLK2, PLK4, PLS3, POLA2, POLE2, POLQ, PPAP2B, PPP1R3C, PRLHR, PRSS12, PSRC1, PTGR1, PTPRE, PTPRK, PTPRM, PTTG1, PVRL3, RAD51, RBL1, RBPMS, RELL1, RFC5, RGMA, RGS5, RNF182, RRM2, S1PR3, SGOL2, SHCBP1, SHOX2, SIM1, SKA1, SLC43A3, SLC44A5, SLC7A2, SLITRK5, SMC2, SMC6, SPAG6, SPARC, SPC24, SPC25, ST8SIA4, STK17A, SUSD5, SYN2, SYNM, SYT17, TCF19, TESC, TFDP2, TFPI2, TIMP3, TLE3, TMEM132C, TMEM178, TNFAIP8, TPBG, TPTE, TPX2, TRIM29, TRIM36, TRIM68, TROAP, TWIST1, TYMS, UBE2C, UBE3B, UHRF1, WDHD1, WDR51A, WDR67, WEE1, ZFP82, ZNF367, ZNF521, and/or ZWINT, as compared to the expression levels of these genes in cells from the 2D6 cell line.

In another aspect of this embodiment, cells from an established clonal cell line susceptible to BoNT/A intoxication exhibit at least a 1.5-fold increase in gene expression levels of one or more genes selected from A2BP1, ALCAM, ANLN, ARHGAP24, ARHGAP6, ASCL1, ASF1B, ATAD2, AURKA, AURKB, BARD1, BASP1, BNC2, BRIP1, BUB1, BVES, C11ORF75, C12ORF48, C14ORF106, C18ORF54, C3ORF70, CCDC109B, CCDC3, CD9, CDC2, CDC45L, CDCA2, CDCA8, CDK2, CENPL, CEP55, CHEK2, CHRNA7, CKAP2L, CNTN1, CRYBG3, CUGBP2, CXCR4, DAZ1, DEPDC1, DIAPH3, DLGAP5, DOK5, DPYD, DTL, DYNLT3, E2F1, E2F7, E2F8, EFNB2, ELOVL7, EMILIN2, EML1, ENC1, ESPL1, ETV1, EXO1, EXPH5, FAM54A, FAM64A, FAM7A3, FAM83D, FANCI, FAT1, FBLN1, FBXO43, FGFR2, FZD5, GNAI1, GNB4, GNG11, GNG12, GPSM2, GRP, GTSE1, GTSF1, HGF, HMMR, ITGA6, ITPRIP, KCTD12, KDELC2, KIF11, KIF14, KIF18A, KIF23, KIFC1, LOC340109, LOC728052, LPAR1, LUM, MAB21L1, MAB21L2, MAOA, MCM10, MELK, MINA, MKI67, MSN, MYBL1, NCAPH, NDN, NEDD9, NEIL3, NRXN1, NUF2, NUSAP1, OIP5, ORC1L, OSBPL3, PBK, PCGF5, PEG3, PEG3, PHLPP1, PLD5, PLK2, PLK4, PLS3, POLE2, PPAP2B, PPP1R3C, PRLHR, PRSS12, PTPRE, PTPRK, PTPRM, RBPMS, RELL1, RGS5, RNF182, RRM2, S1PR3, SGOL2, SHCBP1, SHOX2, SIM1, SLC43A3, SLC44A5, SLC7A2, SLITRK5, SMC6, SPAG6, SPARC, SPC24, SPC25, ST8SIA4, STK17A, SYT17, TFPI2, TIMP3, TMEM132C, TMEM178, TPBG, TPTE, TPX2, TRIM36, TWIST1, UBE3B, and/or ZNF367, as compared to the expression levels of these genes in cells from the 2D6 cell line.

In yet another aspect of this embodiment, cells from an established clonal cell line susceptible to BoNT/A intoxication exhibit at least a 1.5-fold increase in gene expression levels of one or more genes selected from A2BP1, ARHGAP24, ARHGAP6, ASCL1, ASF1B, ATAD2, AURKA, BARD1, BASP1, BNC2, BUB1, BVES, C11ORF75, CCDC3, CHEK2, CHRNA7, CKAP2L, CNTN1, CRYBG3, CUGBP2, CUGBP2, CXCR4, DAZ1, DEPDC1, DIAPH3, DLGAP5, DOK5, DPYD, DTL, DYNLT3, E2F8, EFNB2, ELOVL7, ENC1, EXPH5, FAM7A3, FANCI, FAT1, FBLN1, FGFR2, GNAI1, GNB4, GNG11, GNG12, GRP, GTSE1, GTSF1, HMMR, ITGA6, ITPRIP, KCTD12, KDELC2, LOC728052, LPAR1, LUM, MAB21L1, MAB21L2, MAOA, MCM10, MELK, MINA, MKI67, MSN, MYBL1, NDN, NEDD9, NRXN1, OSBPL3, PEG3, PLK2, PLS3, PRLHR, PRSS12, PTPRK, PTPRM, RBPMS, RGS5, RNF182, RRM2, SGOL2, SIM1, SLC43A3, SLC44A5, SLC7A2, SMC6, SPAG6, SPARC, STK17A, TFPI2, TIMP3, TMEM132C, TMEM178, and/or TPTE, as compared to the expression levels of these genes in cells from the 2D6 cell line.

In another embodiment, cells from an established clonal cell line susceptible to BoNT/A intoxication exhibit at least a 1.5-fold increase in gene expression levels of one or more genes selected from ADAMTS9, ATAD2, C11ORF82, CDC45L, CNTN1, CNTN4, Cyclin A, Cyclin E, E2F1, E2F2, E2F7, ELOVL7, EME1, FGFR, FGFR2, KIAA1524, MELK, MYBL1, MYBL2, NDC80, NDN, ORC1L, PLS3, PRIMA1, RAD54L, RBL1, RBPMS, RRM2, S1PR3, SCLY, SLCIA3, SPC24, SPC25, ST8SIA4, TFDP1, TFP12, TK1, TMEM35, TTK, TWIST1, TYMS, TYK, and/or ZWINT, as compared to the expression levels of these genes in cells from the 2D6 cell line.

In yet another embodiment, cells from an established clonal cell line susceptible to BoNT/A intoxication exhibit at least a 1.5-fold increase in gene expression levels of one or more genes selected from AURKB, BIRC5, BRCA1, BRCA2, BRIP1,BUB1B, CD9, DLGAP3, DYNLT3, ENC1, FBLN1, FOXM1, Gβγ, GNAI1, GNG11, GNG12, GPSM2, GUCY1B3, HGF, ITGA6, JNK, KCNJ5, KIF18A, KITLG, MMD, MSN, MYRIP, NEK2, NR3C1, NXPH1, OSBPL3, PKMYT1, PTPRM, RAD51, RAD51AP1, SLC7A2, SLC43A3, SMC6, SNAI2, SNCAIP, SSH2, STK17A, SYNPO2, TOP2A, TPTE, TRAF4, TSPAN, TSPAN4, UBE3, UBE3B, and/or VAV3, as compared to the expression levels of these genes in cells from the 2D6 cell line. In still another embodiment, cells from an established clonal cell line susceptible to BoNT/A intoxication exhibit at least a 1.5-fold increase in gene expression levels of one or more genes selected from ALCAM, AURKA, CHEK, CIT, CSRP2, E2F, ECT2, EFNB2, ERK, ESPL1, GNAI, GPR161, HMMR, KIF4A, KIF14, KIF15, KIF22, KIF23, KIFC1, LPAR1, MK167, OIP5, PHLPP, PP1/PP2A, PPP1R3C, PRC1, PTTG1, RACGAP, RB, RGS5, SDC2, and/or TPX2, as compared to the expression levels of these genes in cells from the 2D6 cell line.

In another embodiment, cells from an established clonal cell line susceptible to BoNT/A intoxication exhibit at least a 1.5-fold increase in gene expression levels of one or more genes selected from ASCL1,HES6, MAPK, NMU, PEG3, PTPRK, PRLHR, PTPRK, SGOL2, SPARC, and/or ZNF217, as compared to the expression levels of these genes in cells from the 2D6 cell line. In yet another embodiment, cells from an established clonal cell line susceptible to BoNT/A intoxication exhibit at least a 1.5-fold increase in gene expression levels of one or more genes selected from ASF1B, BASP1, CHAF1A, NCAPH, PBK, PRAME, SMC2, UHRF1, and/or VRK1, as compared to the expression levels of these genes in cells from the 2D6 cell line. In still another embodiment, cells from an established clonal cell line susceptible to BoNT/A intoxication exhibit at least a 1.5-fold increase in gene expression levels of one or more genes selected from C14ORF106, CEP72, KIF20A, PCNA, PEX13, PFC5, POLQ, SPAG5, SYTL4, TROAP, and/or WDR51A, as compared to the expression levels of these genes in cells from the 2D6 cell line. In a further embodiment, cells from an established clonal cell line susceptible to BoNT/A intoxication exhibit at least a 1.5-fold increase in gene expression levels of one or more genes selected from ANLN, ARHGAP24, ASPM, BUB1, CCDC99, CEP55, CKAP2, DRAM, E2F8, PLXNA2, SLC16A10, UBE2C, UBE2S, and/or WDHD1, as compared to the expression levels of these genes in cells from the 2D6 cell line.

In another embodiment, cells from an established clonal cell line susceptible to BoNT/A intoxication exhibit at least a 1.5-fold increase in gene expression levels of one or more genes selected from ARHGEF3, CTSL2, DIAPH3, FBP1, KIF2C, KIF11, PFKFB3, and/or PLK4, as compared to the expression levels of these genes in cells from the 2D6 cell line. In yet another embodiment, cells from an established clonal cell line susceptible to BoNT/A intoxication exhibit at least a 1.5-fold increase in gene expression levels of one or more genes selected from ARHGEF3, CTSL2, DIAPH3, FBP1, KIF2C, KIF11, PFKFB3, and/or PLK4, as compared to the expression levels of these genes in cells from the 2D6 cell line. In still another embodiment, cells from an established clonal cell line susceptible to BoNT/A intoxication exhibit at least a 1.5-fold increase in gene expression levels of one or more genes selected from FNDC5, HSPC159, MAB21L2, SLITRK5, SYN2, and/or ZNF367, as compared to the expression levels of these genes in cells from the 2D6 cell line. In a further another embodiment, cells from an established clonal cell line susceptible to BoNT/A intoxication exhibit at least a 1.5-fold increase in gene expression levels of one or more genes selected from EXO1, KCTD12, MYO6, PHEBL1, SHCBP1, TPBG, and/or TUBB6, as compared to the expression levels of these genes in cells from the 2D6 cell line. In yet another embodiment, cells from an established clonal cell line susceptible to BoNT/A intoxication exhibit at least a 1.5-fold increase in gene expression levels of one or more genes selected from BTG3, GABRA5, TR1P10, and/or ZNF521, as compared to the expression levels of these genes in cells from the 2D6 cell line. In still another embodiment, cells from an established clonal cell line susceptible to BoNT/A intoxication exhibit at least a 1.5-fold increase in gene expression levels of one or more genes selected from BNC2, DPYD, EMILIN2, PPIL5, and/or TACC3, as compared to the expression levels of these genes in cells from the 2D6 cell line.

In another embodiment, cells from an established clonal cell line susceptible to BoNT/A intoxication exhibit at least a 1.5-fold difference in gene expression levels of one or more genes listed in Tables 14 or 16 as compared to the expression levels levels of these genes in cells from the 2D6 cell line. In an aspect of this embodiment, cells from an established clonal cell line susceptible to BoNT/A intoxication exhibit at least a 1.5-fold increase in gene expression levels of one or more genes selected from ACOT9, ADAMTS9, ARHGAP24, ARHGEF3, ASCL1, BARD1, BASP1, C3ORF70, C11ORF75, CCDC109B, CD9, CDCA7L, CDK2, CENPL, CLSTN2, CNTN1, CSRP2, CTSL2, CUGBP2, DEPDC1, DIAPH3, DOK5, DPYD, DYNLT3, EMILIN2, ETV1, FAM101B, FBLN1, FGFR2, FNDC5, GNAI1, GNB4, GNG11, GNG12, GPR177, GTSE1, HGF, KITLG, LPAR1, MAB21L2, MAOA, MCM10, MINA, MSN, MYO6, MYRIP, PAG1, PEG3, PLK2, POLA2, PPP1R3C, PRLHR, PRSS12, PTGR1, PTPRK, PVRL3, RAB32, RBPMS, SDC2, SGOL2, SLC43A3, SLC7A2, SMC2, SMC6, SPARC, SPC25, ST8SIA4, TCF7L1, TFPI2, TMEM35, TMEM178, TNFAIP8, TPTE, TRIP10, TWIST1, and/or ZNF521, as compared to the expression levels of these genes in cells from the 2D6 cell line. In another aspect of this embodiment, cells from an established clonal cell line susceptible to BoNT/A intoxication exhibit at least a 1.5-fold increase in gene expression levels of one or more genes selected from ADAMTS9, ARHGAP24, ASCL1, BARD1, BASP1, BVES, C11ORF75, CDCA7L, CNTN1, CUGBP2, DOK5, DPYD, DYNLT3, FBLN1, FGFR2, GNAI1, GNB4, GNG11, GNG12, GTSE1, GTSF1, ITPRIP, KDELC2, LOC728052, LPAR1, MAB21L2, MAOA, MINA, MSN, PEG3, PLK2, PRLHR, PRSS12, PTPRK, RBPMS, RNF182, SGOL2, SLC43A3, SLC44A5, SLC7A2, SMC6, SPARC, TFPI2, TMEM178, and/or TPTE, as compared to the expression levels of these genes in cells from the 2D6 cell line. In yet another aspect of this embodiment, cells from an established clonal cell line susceptible to BoNT/A intoxication exhibit at least a 1.5-fold increase in gene expression levels of one or more genes selected from ADAMTS9, ASCL1, BASP1, DOK5, DPYD, GNB4, GNG11, GTSF1, MAOA, MINA, MSN, PEG3, PLK2, PRSS12, RNF182, SLC44A5, SPARC, TFPI2, and/or TPTE, as compared to the expression levels of these genes in cells from the 2D6 cell line.

In yet another embodiment, cells from an established clonal cell line susceptible to BoNT/A intoxication exhibit at least a 1.5-fold increase in gene expression levels of one or more genes selected from ADAMTS9, ARHGAP24, ARHGEF3, ASCL1, BARD1, CD9, CDK2, CSRP2, CTSL2, DIAPH3, DOK5, DYNLT3, EMILIN2, ETV1, FBLN1, FGFR2, GNAI1, GNB4, GNG11, GNG12, HGF, KITLG, LPAR1, MCM10, MSN, PAG1, PEG3, PLK2, POLA2, PPP1R3C, PTPRK, RAB32, SDC2, SLC43A3, SLC7A2, SMC6, SPARC, SPC25, ST8SIA4, TCF7L1, TFPI2, TMEM35, TNFAIP8, TPTE, TRIP10, and/or TWIST1, as compared to the expression levels of these genes in cells from the 2D6 cell line. In an aspect of this embodiment, cells from an established clonal cell line susceptible to BoNT/A intoxication exhibit at least a 1.5-fold increase in gene expression levels of one or more genes selected from ADAMTS9, ARHGAP24, ASCL1, BARD1, DOK5, DYNLT3, FBLN1, FGFR2, GNAI1, GNB4, GNG11, GNG12, LPAR1, MSN, PEG3, PLK2, PTPRK, SLC43A3, SLC7A2, SMC6, SPARC, TFPI2, and/or TPTE, as compared to the expression levels of these genes in cells from the 2D6 cell line. In another aspect of this embodiment, cells from an established clonal cell line susceptible to BoNT/A intoxication exhibit at least a 1.5-fold increase in gene expression levels of one or more genes selected from ADAMTS9, ASCL1, DOK5, GNB4, GNG11, MSN, PEG3, PLK2, SPARC, TFPI2, and/or TPTE, as compared to the expression levels of these genes in cells from the 2D6 cell line.

In still another embodiment, cells from an established clonal cell line susceptible to BoNT/A intoxication exhibit at least a 1.5-fold increase in gene expression levels of one or more genes selected from ACOT9, BASP1, C11ORF75, CCDC109B, CDCA7L, CLSTN2, CNTN1, CUGBP2, DEPDC1, DPYD, FAM101B, FNDC5, GTSE1, MAOA, MINA, MYO6, MYRIP, PLK2, PRLHR, PVRL3, RBPMS, SGOL2, SMC2, TFP12, TMEM178, and/or ZNF521, as compared to the expression levels of these genes in cells from the 2D6 cell line. In an aspect of this embodiment, cells from an established clonal cell line susceptible to BoNT/A intoxication exhibit at least a 1.5-fold increase in gene expression levels of one or more genes selected from BASP1, C11ORF75, CDCA7L, CNTN1, CUGBP2, DPYD, GTSE1, MAOA, MINA, PLK2, PRLHR, RBPMS, SGOL2, TFPI2, and/or TMEM178, as compared to the expression levels of these genes in cells from the 2D6 cell line. In another aspect of this embodiment, cells from an established clonal cell line susceptible to BoNT/A intoxication exhibit at least a 1.5-fold increase in gene expression levels of one or more genes selected from BASP1, DPYD, MAOA, MINA, PLK2, and/or TFPI2, as compared to the expression levels of these genes in cells from the 2D6 cell line.

In a further embodiment, cells from an established clonal cell line susceptible to BoNT/A intoxication exhibit at least a 1.5-fold increase in gene expression levels of one or more genes selected from C3ORF70, MAB21L2, PRSS12, CENPL, GPR177, and/or PTGR1, as compared to the expression levels of these genes in cells from the 2D6 cell line. In an aspect of this embodiment, cells from an established clonal cell line susceptible to BoNT/A intoxication exhibit at least a 1.5-fold increase in gene expression levels of one or more genes selected from MAB21L2 and/or PRSS12, as compared to the expression levels of these genes in cells from the 2D6 cell line.

In another embodiment, cells from an established clonal cell line susceptible to BoNT/A intoxication exhibit at least a 1.5-fold decrease in gene expression levels of one or more genes listed in Tables 6 or 8 as compared to the expression levels of these genes in cells from a 2D6 cell line. In other aspects of this embodiment, cells from an established clonal cell line susceptible to BoNT/A intoxication exhibit a decrease in gene expression levels of one or more genes listed in Tables 6 or 8 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from a 2D6 cell line. In other aspects of this embodiment, cells from an established clonal cell line susceptible to BoNT/A intoxication exhibit a decrease in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 6 or 8 as compared to the expression levels of these genes in cells from the 2D6 cell line. In yet other aspects of this embodiment, cells from an established clonal cell line susceptible to BoNT/A intoxication exhibit an decrease in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 6 or 8 as compared to the expression levels of these genes in cells from the 2D6 cell line.

In another embodiment, cells from an established clonal cell line susceptible to BoNT/A intoxication exhibit at least a 1.5-fold decrease in gene expression levels of one or more genes selected from AATK, ABCA2, ABCC5, ABCC8, ABR, ABTB1, ACOT1, ACRBP, ACRV1, ACYP2, ADARB1, ADCY1, ADM, AFF1, AGAP4, AGPAT3, AGXT2L2, AHI1, AKAP9, AKIRIN1, AKT2, AMH, ANK2, ANK3, ANKHD1, ANKRD17, ANKRD50, ANKS1A, AQP1, ARF1, ARHGAP1, ARHGAP23, ARL17A, ASAM, ASPHD1, ATP2B3, ATP2B3, ATP6V0A1, ATP8B2, ATP9A, BACE1, BACH2, BLZF1, BPTF, BRUNOL4, BRUNOL5, BRWD1, BRWD2, BTBD18, BTN3A3, BZRAP1, C14ORF159, C15ORF24, C15ORF57, C16ORF52, C17ORF28, C1ORF21, C1ORF21, C1ORF50, C21ORF57, C21ORF59, C21ORF66, C2CD2, C2CD4A, C2ORF60, C2ORF68, C3ORF23, C4ORF41, C5ORF42, C6ORF154, C6ORF52, C7ORF28B, C7ORF54, C9ORF150, C9ORF68, CACNA1D, CALY, CAMK2B, CAMK2N2, CAP2, CAPN2, CARTPT, CBS, CCDC104, CCDC50, CCDC76, CD151, CD163L1, CD302, CDC42EP1, CDH10, CDH12, CDKN1C, CDKN2A, CDS1, CFC1, CHD5, CLCN3, CLDN12, CLIP4, CLMN, CNGA3, CNNM1, CNOT6L, COL6A1, COPA, CPNE8, CRKRS, CRTC1, CRYGS, CSTB, CTSK, CUL4A, CYGB, CYLD, CYP2E1, CYP3A5, CYTL1, D4S234E, DCLK1, DCTD, DEPDC6, DHX36, DIP2A, DIS3, DKFZP547G183, DNAJC22, DTNA, DTWD1, DUSP16, DUSP5, DVL3, EFNB3, EIF3C, ELFN2, ENOSF1, ERAP1, ERBB2, ERBB4, ERMAP, ETFDH, EXOSC6, FADS3, FAM120AOS, FAM150B, FAM165B, FAM184B, FAM30A, FAM46A, FAM66C, FAT4, FBRSL1, FBXW7, FGD5, FGF13, FLJ33630, FNBP1, FOXO6, FUBP3, GAL, GAL, GLT25D2, GNAS, GOLGA8A, GPR123, GPX7, GRIA2, GRIP2, GRM5, GUCY1A3, H2AFY, HCG 1776018, HCG 2022304, HCN4, HEATR1, HELQ, HERC4, HERPUD2, HEXDC, HGSNAT, HIST1H2AC, HIST1H2AE, HIST1H2BD, HIST1H2BK, HIST1H3I, HIST2H2BE, HIVEP3, HNRNPM, HNRNPR, HPCAL1, HPCAL4, HSPD1, IER3, IL10RB, IL17B, INTU, JMY, KAT2B, KATNB1, KBTBD11, KCNMA1, KCNQ2, KCNS2, KCTD13, KHDC1, KIAA0125, KIAA1370, KIAA1598, KIF1A, KIF5C, KIF5C, KISS1R, KLHDC1, LCORL, LGALS3BP, LOC100130097, LOC100130360, LOC100130522, LOC100272228, LOC284408, LOC399491, LOC401320, LOC641298, LOC642852, LOC90110, LOC94431, LONRF2, LRP2BP, LRRC37A2, LRRFIP1, LTBP3, LYPLAL1, LYRM5, MAP3K5, MAP7, MAP9, MCM3AP, MCM3APAS, MCTP1, MEOX2, METTL3, MFSD4, MGAT4A, MLXIP, MRPL1, MRPS33, MST1, MUC20, MXD1, MZF1, NAMPT, NAP1L3, NBPF1, NBPF10, NCOA7, NCRNA00171, NCRNA00182, NDRG1, NDUFA4L2, NDUFV3, NEBL, NELF, NHEDC2, NHEG1, NIPAL2, NIPAL3, NLRX1, NOL3, NSMAF, NUDT19, NUP153, NUP54, OLFM3, OR7D2, OSBP, PABPC1L, PABPC4, PACRGL, PAM, PAPPA, PAQR6, PARP12, PCBD2, PCNXL2, PDCD6, PDE4C, PDE9A, PDGFRB, PDIA2, PGAP1, PHF17, PHKA1, PHKA2, PHLDA2, PIGH, PION, PKD1, PLA2G4C, PLCB4, PLEKHH1, PLP2, PLRG1, PLXNC1, PNCK, PNMA3, POFUT2, POGK, PPAPDC1A, PPAPDC1B, PPID, PPIE, PPM1K, PPP1R2, PPP2R2C, PPT1, PRKACB, PRPH, PSMB7, PTCD1, PTGER2, PTGS1, PTN, PTPRD, PTPRN, RAB11FIP3, RAB6B, RAF1, RGAG4, RGS11, RGS8, RHBDL1, RHOQ, RHOU, RLF, RNASET2, RNF13, RNF149, RNF165, RNF207, RNF41, RPL37, RSL1D1, RUNDC3A, S100A6, SCMH1, SCN2A, SERP1, SETX, SFRS18, SFXN3, SGK3, SH3BP5, SH3GL2, SH3YL1, SHC2, SHC4, SIGIRR, SIK1, SLC12A7, SLC1A2, SLC1A6, SLC22A17, SLC35F3, SLC38A5, SLCO1A2, SMAGP, SMAP2, SNCA, SOBP, SORCS1, SORL1, SPINT2, SPIRE2, SPOCK2, SRR, ST8SIA3, STAR, STEAP3, STOX2, STX3, SYNJ1, SYT13, SYT5, TAF10, TANC2, TCEA1, THBS2, THSD4, TIMP1, TM2D1, TMEM111, TMEM151A, TMEM184C, TMEM41B, TMEM43, TMEM5, TMEM59L, TMIE, TNFRSF25, TNFRSF25, TOX4, TP53BP1, TPM3, TRA2A, TRIM33, TRIM73, TRIM8, TRIT1, TSR1, TTC17, TTC3, TTC39C, TUSC3, TXLNB, U2AF1, UBE2D3, UBE2G2, UBN2, UBXN6, UCN, UGP2, UNC80, UNQ1887, USP36, USPL1, VN1R1, VPS13C, VPS53, VPS8, WDFY3, WDR27, WDR85, WSB1, YJEFN3, YLPM1, ZDHHC11, ZER1, ZG16B, ZNF275, ZNF440, ZNF573, ZNF641, ZNF662, ZNF785, and/or ZNF814, as compared to the expression levels of these genes in cells from the 2D6 cell line.

In an aspect of this embodiment, cells from an established clonal cell line susceptible to BoNT/A intoxication exhibit at least a 1.5-fold decrease in gene expression levels of one or more genes selected from ACOT1, ADARB1, ADM, ANK2, AQP1, ASAM, C2CD4A, C3ORF23, CACNA1D, CAMK2B, CAP2, CARTPT, CD163L1, CDC42EP1, CDH10, CDKN1C, CDKN2A, CFC1, CLMN, CPNE8, CYGB, CYP2E1, CYTL1, DNAJC22, DUSP5, ENOSF1, ERAP1, FGF13, FOXO6, GAL, GNAS, GPX7, GRIA2, GRM5, HCG 1776018, HIST1H2AC, HIST1H2BD, HIST1H2BK, HIST2H2BE, IL17B, KIAA0125, KIAA1598, KISS1R, LRP2BP, MEOX2, NCRNA00182, NDRG1, NDUFA4L2, NIPAL2, NUDT19, PDE4C, PHKA2, PHLDA2, PTGER2, RGS11, S100A6, SCN2A, SHC4, SIGIRR, SLC1A2, SLC1A6, SLC35F3, SLC38A5, SORCS1, SYT13, SYT5, THBS2, TIMP1, and/or TMEM111, as compared to the expression levels of these genes in cells from the 2D6 cell line.

In another aspect of this embodiment, cells from an established clonal cell line susceptible to BoNT/A intoxication exhibit at least a 1.5-fold decrease in gene expression levels of one or more genes selected from ACOT1, ADARB1, ANK2, AQP1, C3ORF23, CAP2, CARTPT, CD163L1, CDC42EP1, CDKN2A, CFC1, CPNE8, CYGB, CYP2E1, CYTL1, FGF13, FOXO6, GNAS, GRIA2, GRM5, HIST1H2AC, IL17B, KIAA0125, KIAA1598, KISS1R, MEOX2, NDRG1, NDUFA4L2, NIPAL2, PDE4C, PHKA2, RGS11, SCN2A, SLC1A2, SLC35F3, SLC38A5, SYT13, and/or THBS2, as compared to the expression levels of these genes in cells from the 2D6 cell line.

In yet another aspect of this embodiment, cells from an established clonal cell line susceptible to BoNT/A intoxication exhibit at least a 1.5-fold decrease in gene expression levels of one or more genes selected from AQP1, CAP2, CARTPT, CD163L1, CDC42EP1, CFC1, CPNE8, CYGB, CYP2E1, CYTL1, FGF13, GNAS, GRIA2, GRM5, HIST1H2AC, IL17B, KIAA0125, MEOX2, NDUFA4L2, PDE4C, PHKA2, RGS11, SLC1A2, SLC35F3, SLC38A5, and/or SYT13, as compared to the expression levels of these genes in cells from the 2D6 cell line.

In another embodiment, cells from an established clonal cell line susceptible to BoNT/A intoxication exhibit at least a 1.5-fold decrease in gene expression levels of one or more genes selected from ANXA2, AQP1, ARHGAP9, CDH10, CDKN2A, CHPT1, CNTN2, ERAP1, and/or RGS11, as compared to the expression levels of these genes in cells from the 2D6 cell line. In yet another embodiment, cells from an established clonal cell line susceptible to BoNT/A intoxication exhibit at least a 1.5-fold decrease in gene expression levels of one or more genes selected from ABCC8, AELIM3, CAP2, IL17B, MEF2A, NEEBL, PHC, S100A6, SLC1A6, SMAD1, SMAD5, SMAD8, SYT13, and/or SYTL1, as compared to the expression levels of these genes in cells from the 2D6 cell line. In still another embodiment, cells from an established clonal cell line susceptible to BoNT/A intoxication exhibit at least a 1.5-fold decrease in gene expression levels of one or more genes selected from CSTB, GPCR, GRIM5, KISSR, SCN2A, SLC1A2, and/or THBS2, as compared to the expression levels of these genes in cells from the 2D6 cell line.

In another embodiment, cells from an established clonal cell line susceptible to BoNT/A intoxication exhibit at least a 1.5-fold decrease in gene expression levels of one or more genes selected from ADARB1, ADM, PTPPH, and/or SLCO1A2, as compared to the expression levels of these genes in cells from the 2D6 cell line. In yet another embodiment, cells from an established clonal cell line susceptible to BoNT/A intoxication exhibit at least a 1.5-fold decrease in gene expression levels of one or more genes selected from CNGA3, HIST1H3E, and/or PTGS1, as compared to the expression levels of these genes in cells from the 2D6 cell line. In still another embodiment, cells from an established clonal cell line susceptible to BoNT/A intoxication exhibit at least a 1.5-fold decrease in gene expression levels of one or more genes selected from HIST1H2BD and/or OSCAR as compared to the expression levels of these genes in cells from the 2D6 cell line. In a further embodiment, cells from an established clonal cell line susceptible to BoNT/A intoxication exhibit at least a 1.5-fold decrease in gene expression levels of one or more genes selected from CALY as compared to the expression levels of these genes in cells from the 2D6 cell line.

In another embodiment, cells from an established clonal cell line susceptible to BoNT/A intoxication exhibit at least a 1.5-fold decrease in gene expression levels of one or more genes selected from COL5A1 and/or MICAL2 as compared to the expression levels of these genes in cells from the 2D6 cell line. In yet another embodiment, cells from an established clonal cell line susceptible to BoNT/A intoxication exhibit at least a 1.5-fold decrease in gene expression levels of one or more genes selected from OLFML2A and/or SIGIRR as compared to the expression levels of these genes in cells from the 2D6 cell line. In still another embodiment, cells from an established clonal cell line susceptible to BoNT/A intoxication exhibit at least a 1.5-fold decrease in gene expression levels of one or more genes selected from HPCAL1 and/or LPAR5 as compared to the expression levels of these genes in cells from the 2D6 cell line. In a further embodiment, cells from an established clonal cell line susceptible to BoNT/A intoxication exhibit at least a 1.5-fold decrease in gene expression levels of one or more genes selected from HTR1E and/or SORCS1 as compared to the expression levels of these genes in cells from the 2D6 cell line.

In another embodiment, cells from an established clonal cell line susceptible to BoNT/A intoxication exhibit at least a 1.5-fold decrease in gene expression levels of one or more genes selected from ANK2, CPNE8, CREB5, IL17B, KIAA0125, LOC100289109, LOC144571, NPAS4, SLC1A2, SORCS1, THBS2, and/or ZNF814, as compared to the expression levels of these genes in cells from the 2D6 cell line.

Aspects of the present disclosure comprise, in part, cells from an established clonal cell line susceptible to BoNT/A intoxication that exhibit at least a 1.5-fold difference in gene expression levels of one or more genes listed in Tables 9, 10, 11, or 12 as compared to the expression levels of these genes in cells from a parental SiMa cell line. In aspects of this embodiment, cells from an established clonal cell line susceptible to BoNT/A intoxication exhibit at least a 1.5-fold difference in gene expression levels of one or more genes listed in Tables 9, 10, 11, or 12 as compared to the expression levels of these genes in cells from the parental SiMa cell line DSMZ ACC 164. In other aspects of this embodiment, cells from an established clonal cell line susceptible to BoNT/A intoxication exhibit a difference in gene expression levels of one or more genes listed in Tables 9, 10, 11, or 12 of, e.g., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from the parental SiMa cell line DSMZ ACC 164. In yet other aspects of this embodiment, cells from an established clonal cell line susceptible to BoNT/A intoxication exhibit a difference in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 9, 10, 11, or 12 as compared to the expression levels of these genes in cells from the parental SiMa cell line DSMZ ACC 164. In still other aspects of this embodiment, cells from an established clonal cell line susceptible to BoNT/A intoxication exhibit a difference in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 9, 10, 11, or 12 as compared to the expression levels of these genes in cells from the parental SiMa cell line DSMZ ACC 164. The log ratio in Tables 9, 10, 11, or 12 represent log₂ values where 0.585 is log₂(1.5) which is a 1.5-fold difference, 1 is log₂(2) which is a 2-fold difference, 1.584 is log₂(3) which is a 3-fold difference, 2 is log₂(4) which is a 4-fold difference, 2.321 is log₂(5) which is a 5-fold difference, 2.584 is log₂(6) which is a 6-fold difference, 2.807 is log₂(7) which is a 7-fold difference, 3 is log₂(8) which is a 8-fold difference, 3.169 is log₂(9) which is a 9-fold difference, and 3.321 is log₂(10) which is a 10-fold difference.

In an embodiment, cells from an established clonal cell line susceptible to BoNT/A intoxication exhibit at least a 1.5-fold difference in gene expression levels of one or more genes selected from ACOT9, ADAMTS9, ARHGAP24, ARHGEF3, ASCL1, BARD1, BASP1, C3ORF70, C11ORF75, CCDC109B, CD9, CDCA7L, CDK2, CENPL, CLSTN2, CNTN1, CSRP2, CTSL2, CUGBP2, DEPDC1, DIAPH3, DOK5, DPYD, DYNLT3, EMILIN2, ETV1, FAM101B, FBLN1, FGFR2, FNDC5, GNAI1, GNB4, GNG11, GNG12, GPR177, GTSE1, HGF, KITLG, LPAR1, MAB21L2, MAOA, MCM10, MINA, MSN, MYO6, MYRIP, PAG1, PEG3, PLK2, POLA2, PPP1R3C, PRLHR, PRSS12, PTGR1, PTPRK, PVRL3, RAB32, RBPMS, SDC2, SGOL2, SLC43A3, SLC7A2, SMC2, SMC6, SPARC, SPC25, ST8SIA4, TCF7L1, TFPI2, TMEM35, TMEM178, TNFAIP8, TPTE, TRIP10, TWIST1, and/or ZNF521, as compared to the expression levels of these genes in cells from the parental SiMa cell line DSMZ ACC 164. In another aspect of this embodiment, cells from an established clonal cell line susceptible to BoNT/A intoxication exhibit at least a 1.5-fold difference in gene expression levels of one or more genes selected from ADAMTS9, ARHGAP24, ASCL1, BARD1, BASP1, BVES, C11ORF75, CDCA7L, CNTN1, CUGBP2, DOK5, DPYD, DYNLT3, FBLN1, FGFR2, GNAI1, GNB4, GNG11, GNG12, GTSE1, GTSF1, ITPRIP, KDELC2, LOC728052, LPAR1, MAB21L2, MAOA, MINA, MSN, PEG3, PLK2, PRLHR, PRSS12, PTPRK, RBPMS, RNF182, SGOL2, SLC43A3, SLC44A5, SLC7A2, SMC6, SPARC, TFPI2, TMEM178, and/or TPTE, as compared to the expression levels of these genes in cells from the parental SiMa cell line DSMZ ACC 164. In yet another aspect of this embodiment, cells from an established clonal cell line susceptible to BoNT/A intoxication exhibit at least a 1.5-fold difference in gene expression levels of one or more genes selected from ADAMTS9, ASCL1, BASP1, DOK5, DPYD, GNB4, GNG11, GTSF1, MAOA, MINA, MSN, PEG3, PLK2, PRSS12, RNF182, SLC44A5, SPARC, TFPI2, and/or TPTE, as compared to the expression levels of these genes in cells from the parental SiMa cell line DSMZ ACC 164.

In another embodiment, cells from an established clonal cell line susceptible to BoNT/A intoxication exhibit at least a 1.5-fold increase in gene expression levels of one or more genes listed in Tables 9 or 11 as compared to the expression levels of these genes in cells from a parental SiMa cell line DSMZ ACC 164. In other aspects of this embodiment, cells from an established clonal cell line susceptible to BoNT/A intoxication exhibit an increase in gene expression levels of one or more genes listed in Tables 9 or 11 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from a parental SiMa cell line DSMZ ACC 164. In other aspects of this embodiment, cells from an established clonal cell line susceptible to BoNT/A intoxication exhibit an increase in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 9 or 11 as compared to the expression levels of these genes in cells from the parental SiMa cell line DSMZ ACC 164. In yet other aspects of this embodiment, cells from an established clonal cell line susceptible to BoNT/A intoxication exhibit an increase in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 9 or 11 as compared to the expression levels of these genes in cells from the parental SiMa cell line DSMZ ACC 164.

In an embodiment, cells from an established clonal cell line susceptible to BoNT/A intoxication exhibit at least a 1.5-fold increase in gene expression levels of one or more genes selected from A2BP1, ADRM1, AFF4, AGAP4, ALCAM, ANAPC7, ANKRD13D, ANKRD28, API5, ARF1, ARHGAP6, ARHGEF10, ARHGEF6, ARL17A, ASCL1, ASXL1, ASXL3, ATF7IP, B3GALNT2, BASP1, BLCAP, BNC2, C10ORF58, C12ORF49, C12ORF51, C1ORF43, C20ORF117, C20ORF7, CADM1, CADPS, CALCB, CAPN1, CAPRIN2, CBLB, CBLN2, CBWD1, CCDC150, CCDC3, CD9, CDC25B, CFDP1, CHRNA7, CIAPIN1, CLASP2, CNOT7, CPVL, CRYBG3, CSE1L, CTTNBP2NL, CUGBP2, CXCR4, DACT1, DAZ1, DBH, DCAF8, DENR, DHX35, DKFZP434L187, DLEU2, DLL1, DLL3, DYNC2H1, EAF2, EFNA5, EIF3B, EIF3C, ELOVL7, EML1, EML4, ENC1, EVL, EXOSC6, EXPH5, EZH2, FAM178A, FAM181B, FAM19A4, FAM7A3, FLJ10213, FUBP3, FUS, FZD5, GAP43, GFM1, GFRA2, GGA2, GNAI1, GNAS, GNB4, GNG11, GOLGA4, GPR125, GRM8, GRP, GSPT1, GSS, GSTCD, GULP1, HAUS2, HEG1, HNRNPL, HNRNPM, HOXA7, HOXD4, ID2, IDH3B, IL1A, INSM1, IREB2, ITGA6, ITGB5, KCTD12, KDELC2, KHDRBS3, KIAA0907, KIAA1267, KIF16B, KLC1, KLHL13, LBH, LMO4, LOC100128844, LOC340109, LOC641298, LOC647190, LOC728052, LOC728153, LRPPRC, LRRFIP2, LSM3, LUM, MAB21L1, MAB21L2, MAN2A1, MAOA, MARS, MDN1, MED13L, MED22, MGC24103, MINA, MKLN1, MLEC, MMD, MORF4L2, MPZL1, MSH6, MSI2, MSN, MTMR9, MYO1B, MYO6, N4BP2, NAAA, NDUFS8, NEDD9, NFIB, NKTR, NLN, NOS1, NR2C1, NUFIP2, NUPL1, OSBPL3, PAPD4, PCBP2, PCDH9, PCGF5, PCNX, PDLIM5, PDZRN3, PELI2, PFKFB3, PGP, PHF20, PLK2, PLXNA2, PLXNA4, PM20D2, POLE, POLQ, PPP2R2A, PPP2R3C, PRDX2, PSMA7, PSME4, PTGFRN, PTK2, PTPN1, PTPRE, PTPRG, QKI, RAB12, RAB35, RAB3GAP2, RAD23B, RAF1, RALGAPA1, RAN, RASEF, RBL1, RDH11, RELL1, REPS1, RGS5, RIMBP2, RNF182, RNF34, RNPEP, ROBO2, RPAIN, RPL35A, RPRD1A, S1PR3, SALL4, SBNO1, SCARB2, SDHA, SEMA6A, SEZ6L, SF1, SFRS8, SIM1, SIRT2, SKIL, SLC20A1, SLC44A5, SLC7A2, SLCO3A1, SLITRK5, SMAD4, SMARCC2, SMC3, SNRPB2, SNRPN, SNX5, SOX2, SPAG6, SPAG9, SPATS2L, SPON1, SR140, SSBP2, ST8SIA1, STMN3, STRA6, SYNCRIP, SYTL3, TAF15, TCF7L2, TDG, TERT, TFPI2, TGFBR1, TH1L, THOC4, TIMP3, TLE3, TMEM132C, TMEM178, TMEM181, TNRC6A, TPBG, TRA2A, TRIM29, TRIM36, TSHZ3, TXNRD1, U2AF1, UBE2O, UBE2V1, UBE2Z, UBXN2A, UCHL1, USP25, USP32, USP34, VPS29, XPO1, XPO7, ZFAND6, ZFHX4, ZFYVE16, ZGPAT, ZNF217, ZNF451, ZNF503, and/or ZNF664, as compared to the expression levels of these genes in cells from the parental SiMa cell line DSMZ ACC 164.

In an aspect of this embodiment, cells from an established clonal cell line susceptible to BoNT/A intoxication exhibit at least a 1.5-fold increase in gene expression levels of one or more genes selected from ALCAM, ARHGAP6, ARHGEF6, ASCL1, BNC2, CBLN2, CCDC150, CCDC3, CHRNA7, CRYBG3, CUGBP2, CXCR4, DAZ1, DKFZP434L187, DLL1, EFNA5, ELOVL7, EML1, EML4, ENC1, EXPH5, FAM7A3, FZD5, GFRA2, GNAI1, GNB4, GNG11, GRP, ID2, ITGA6, KCTD12, LOC100128844, LOC340109, LOC728052, LUM, MAB21L1, MAB21L2, MAOA, MGC24103, MSN, NEDD9, NFIB, OSBPL3, PAPD4, PCDH9, PDZRN3, PLK2, POLQ, PTGFRN, PTPRE, PTPRG, RAD23B, RGS5, RNF182, ROBO2, SIM1, SLC20A1, SLC44A5, SLITRK5, SPAG6, SPAG9, SR140, TFPI2, TIMP3, TMEM132C, TPBG, TRIM29, TRIM36, and/or ZFAND6, as compared to the expression levels of these genes in cells from the parental SiMa cell line DSMZ ACC 164.

In another aspect of this embodiment, cells from an established clonal cell line susceptible to BoNT/A intoxication exhibit at least a 1.5-fold increase in gene expression levels of one or more genes selected from ALCAM, ARHGAP6, ARHGEF6, CBLN2, CCDC3, CHRNA7, CRYBG3, CUGBP2, CXCR4, DAZ1, ELOVL7, EML1, EXPH5, FAM7A3, GNB4, GNG11, GRP, ITGA6, KCTD12, LOC340109, LUM, MAB21L1, MAB21L2, MGC24103, PCDH9, PLK2, POLQ, PTPRE, RGS5, RNF182, ROBO2, SIM1, SLC44A5, SLITRK5, SPAG6, TIMP3, and/or TMEM132C, as compared to the expression levels of these genes in cells from the parental SiMa cell line DSMZ ACC 164.

In yet another aspect of this embodiment, cells from an established clonal cell line susceptible to BoNT/A intoxication exhibit at least a 1.5-fold increase in gene expression levels of one or more genes selected from CBLN2, CCDC3, CHRNA7, CRYBG3, CXCR4, DAZ1, EXPH5, FAM7A3, GNB4, GRP, KCTD12, LUM, MGC24103, PCDH9, PLK2, POLQ, PTPRE, RGS5, ROBO2, SIM1, SLITRK5, TIMP3, and/or TMEM132C, as compared to the expression levels of these genes in cells from the parental SiMa cell line DSMZ ACC 164.

In another embodiment, cells from an established clonal cell line susceptible to BoNT/A intoxication exhibit at least a 1.5-fold increase in gene expression levels of one or more genes listed in Tables 14 or 16 as compared to the expression levels of these genes in cells from the parental SiMa cell line DSMZ ACC 164. In aspects of this embodiment, cells from an established clonal cell line susceptible to BoNT/A intoxication exhibit at least a 1.5-fold increase in gene expression levels of one or more genes listed in Tables 14 or 16 as compared to the expression levels of these genes in cells from a parental SiMa cell line DSMZ ACC 164. In other aspects of this embodiment, cells from an established clonal cell line susceptible to BoNT/A intoxication exhibit an increase in gene expression levels of one or more genes listed in Tables 14 or 16 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from a parental SiMa cell line DSMZ ACC 164. In other aspects of this embodiment, cells from an established clonal cell line susceptible to BoNT/A intoxication exhibit an increase in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 14 or 16 as compared to the expression levels of these genes in cells from the parental SiMa cell line DSMZ ACC 164. In yet other aspects of this embodiment, cells from an established clonal cell line susceptible to BoNT/A intoxication exhibit an increase in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 14 or 16 as compared to the expression levels of these genes in cells from the parental SiMa cell line DSMZ ACC 164.

In aspects of this embodiment, cells from an established clonal cell line susceptible to BoNT/A intoxication exhibit at least a 1.5-fold increase in gene expression levels of one or more genes selected from ACOT9, ADAMTS9, ARHGAP24, ARHGEF3, ASCL1, BARD1, BASP1, C3ORF70, C11ORF75, CCDC109B, CD9, CDCA7L, CDK2, CENPL, CLSTN2, CNTN1, CSRP2, CTSL2, CUGBP2, DEPDC1, DIAPH3, DOK5, DPYD, DYNLT3, EMILIN2, ETV1, FAM101B, FBLN1, FGFR2, FNDC5, GNAI1, GNB4, GNG11, GNG12, GPR177, GTSE1, HGF, KITLG, LPAR1, MAB21L2, MAOA, MCM10, MINA, MSN, MYO6, MYRIP, PAG1, PEG3, PLK2, POLA2, PPP1R3C, PRLHR, PRSS12, PTGR1, PTPRK, PVRL3, RAB32, RBPMS, SDC2, SGOL2, SLC43A3, SLC7A2, SMC2, SMC6, SPARC, SPC25, ST8SIA4, TCF7L1, TFPI2, TMEM35, TMEM178, TNFAIP8, TPTE, TRIP10, TWIST1, and/or ZNF521, as compared to the expression levels of these genes in cells from the parental SiMa cell line DSMZ ACC 164. In another aspect of this embodiment, cells from an established clonal cell line susceptible to BoNT/A intoxication exhibit at least a 1.5-fold increase in gene expression levels of one or more genes selected from ADAMTS9, ARHGAP24, ASCL1, BARD1, BASP1, BVES, C11ORF75, CDCA7L, CNTN1, CUGBP2, DOK5, DPYD, DYNLT3, FBLN1, FGFR2, GNAI1, GNB4, GNG11, GNG12, GTSE1, GTSF1, ITPRIP, KDELC2, LOC728052, LPAR1, MAB21L2, MAOA, MINA, MSN, PEG3, PLK2, PRLHR, PRSS12, PTPRK, RBPMS, RNF182, SGOL2, SLC43A3, SLC44A5, SLC7A2, SMC6, SPARC, TFPI2, TMEM178, and/or TPTE, as compared to the expression levels of these genes in cells from the parental SiMa cell line DSMZ ACC 164. In yet another aspect of this embodiment, cells from an established clonal cell line susceptible to BoNT/A intoxication exhibit at least a 1.5-fold increase in gene expression levels of one or more genes selected from ADAMTS9, ASCL1, BASP1, DOK5, DPYD, GNB4, GNG11, GTSF1, MAOA, MINA, MSN, PEG3, PLK2, PRSS12, RNF182, SLC44A5, SPARC, TFPI2, and/or TPTE, as compared to the expression levels of these genes in cells from the parental SiMa cell line DSMZ ACC 164.

In yet another embodiment, cells from an established clonal cell line susceptible to BoNT/A intoxication exhibit at least a 1.5-fold increase in gene expression levels of one or more genes selected from ADAMTS9, ARHGAP24, ARHGEF3, ASCL1, BARD1, CD9, CDK2, CSRP2, CTSL2, DIAPH3, DOK5, DYNLT3, EMILIN2, ETV1, FBLN1, FGFR2, GNAI1, GNB4, GNG11, GNG12, HGF, KITLG, LPAR1, MCM10, MSN, PAG1, PEG3, PLK2, POLA2, PPP1R3C, PTPRK, RAB32, SDC2, SLC43A3, SLC7A2, SMC6, SPARC, SPC25, ST8SIA4, TCF7L1, TFPI2, TMEM35, TNFAIP8, TPTE, TRIP10, and/or TWIST1, as compared to the expression levels of these genes in cells from the parental SiMa cell line DSMZ ACC 164. In an aspect of this embodiment, cells from an established clonal cell line susceptible to BoNT/A intoxication exhibit at least a 1.5-fold increase in gene expression levels of one or more genes selected from ADAMTS9, ARHGAP24, ASCL1, BARD1, DOK5, DYNLT3, FBLN1, FGFR2, GNAI1, GNB4, GNG11, GNG12, LPAR1, MSN, PEG3, PLK2, PTPRK, SLC43A3, SLC7A2, SMC6, SPARC, TFPI2, and/or TPTE, as compared to the expression levels of these genes in cells from the parental SiMa cell line DSMZ ACC 164. In another aspect of this embodiment, cells from an established clonal cell line susceptible to BoNT/A intoxication exhibit at least a 1.5-fold increase in gene expression levels of one or more genes selected from ADAMTS9, ASCL1, DOK5, GNB4, GNG11, MSN, PEG3, PLK2, SPARC, TFPI2, and/or TPTE, as compared to the expression levels of these genes in cells from the parental SiMa cell line DSMZ ACC 164.

In still another embodiment, cells from an established clonal cell line susceptible to BoNT/A intoxication exhibit at least a 1.5-fold increase in gene expression levels of one or more genes selected from ACOT9, BASP1, C11ORF75, CCDC109B, CDCA7L, CLSTN2, CNTN1, CUGBP2, DEPDC1, DPYD, FAM101B, FNDC5, GTSE1, MAOA, MINA, MYO6, MYRIP, PLK2, PRLHR, PVRL3, RBPMS, SGOL2, SMC2, TFP12, TMEM178, and/or ZNF521, as compared to the expression level of these genes in cells from the parental SiMa cell line DSMZ ACC 164. In an aspect of this embodiment, cells from an established clonal cell line susceptible to BoNT/A intoxication exhibit at least a 1.5-fold increase in gene expression levels of one or more genes selected from BASP1, C11ORF75, CDCA7L, CNTN1, CUGBP2, DPYD, GTSE1, MAOA, MINA, PLK2, PRLHR, RBPMS, SGOL2, TFPI2, and/or TMEM178, as compared to the expression levels of these genes in cells from the parental SiMa cell line DSMZ ACC 164. In another aspect of this embodiment, cells from an established clonal cell line susceptible to BoNT/A intoxication exhibit at least a 1.5-fold increase in gene expression levels of one or more genes selected from BASP1, DPYD, MAOA, MINA, PLK2, and/or TFPI2, as compared to the expression levels of these genes in cells from the parental SiMa cell line DSMZ ACC 164.

In a further embodiment, cells from an established clonal cell line susceptible to BoNT/A intoxication exhibit at least a 1.5-fold increase in gene expression levels of one or more genes selected from C3ORF70, MAB21L2, PRSS12, CENPL, GPR177, and/or PTGR1, as compared to the expression levels of these genes in cells from the parental SiMa cell line DSMZ ACC 164. In an aspect of this embodiment, cells from an established clonal cell line susceptible to BoNT/A intoxication exhibit at least a 1.5-fold increase in gene expression levels of one or more genes selected from MAB21L2 and/or PRSS12, as compared to the expression levels of these genes in cells from the parental SiMa cell line DSMZ ACC 164.

In another embodiment, cells from an established clonal cell line susceptible to BoNT/A intoxication exhibit at least a 1.5-fold decrease in gene expression levels of one or more genes listed in Tables 10 or 12 as compared to the expression levels of these genes in cells from a parental SiMa cell line. In aspects of this embodiment, cells from an established clonal cell line susceptible to BoNT/A intoxication exhibit at least a 1.5-fold decrease in gene expression levels of one or more genes listed in Tables 10 or 12 as compared to the expression levels of these genes in cells from the parental SiMa cell line DSMZ ACC 164. In other aspects of this embodiment, cells from an established clonal cell line susceptible to BoNT/A intoxication exhibit a decrease in gene expression levels of one or more genes listed in Tables 10 or 12 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from the parental SiMa cell line DSMZ ACC 164. In other aspects cells from an established clonal cell line susceptible to BoNT/A intoxication exhibit a decrease in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 10 or 12 as compared to the expression levels of these genes in cells from the parental SiMa cell line DSMZ ACC 164. In yet other aspects cells from an established clonal cell line susceptible to BoNT/A intoxication exhibit an decrease in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 10 or 12 as compared to the expression levels of these genes in cells from the parental SiMa cell line DSMZ ACC 164.

In an embodiment, cells from an established clonal cell line susceptible to BoNT/A intoxication exhibit at least a 1.5-fold decrease in gene expression levels of one or more genes selected from 1-Mar, ABCG1, ABTB1, ACOT1, ACSL1, ACVR1, ACVR2A, ACYP2, ADAMTS1, ADAMTS3, ADAMTSL1, ADARB1, ADCY1, ADD2, ADO, AFF3, AKAP12, ALDH1A2, ALKBH1, ALLC, AMN1, ANKH, ANKRA2, ANKRD50, ANXA5, AP3B2, ARMC10, ARMCX5, ASAM, ASB13, ASPHD1, ATG5, ATL3, ATP2B3, ATP2C1, ATRNL1, B3GALTL, BACE1, BACE2, BBS10, BBS7, BCAP29, BCL2, BCORL1, BRUNOL4, BRUNOL6, BRWD1, BTN3A3, C10ORF10, C10ORF104, C11ORF57, C11ORF70, C14ORF167, C15ORF39, C16ORF52, C17ORF69, C18ORF18, C1GALT1C1, C1ORF21, C1ORF25, C1ORF97, C1RL, , C21ORF57, C2CD2, C2CD4A, C2ORF67, C2ORF68, C3ORF23, C4ORF12, C4ORF39, C4ORF41, C5ORF42, C6ORF120, C9ORF150, CADM2, CAMK2D, CAMK2N1, CAMK2N2, CAP2, CAPN2, CARTPT, CCDC126, CCDC40, CCDC50, CCNY, CD248, CD302, CDC2L6, CDC37L1, CDC42EP3, CDH12, CDKN1C, CDKN2A, CDS1, CFC1, CHMP1B, CLCN3, CLDN12, CLMN, CLN8, CNGA3, CNNM1, CNOT6L, COL27A1, COL5A1, COL6A1, COL6A2, COL6A3, COX18, COX5B, CRYGD, CRYZL1, CTPS2, CTSC, CYB5R2, CYGB, CYLD, CYP2E1, CYP2U1, CYTL1, CYYR1, D4S234E, DAB1, DBT, DCAF10, DCLK1, DCTD, DDAH1, DHRSX, DISP1, DKFZP434I0714, DKK1, DNAJC12, DNAJC24, DNASE1L1, DNER, DOPEY2, DRAM2, DSCAM, DSCR3, DTX3L, DUSP16, DUSP22, ECEL1, ECHDC3, EEF1D, EEF2K, EFEMP2, EFNB3, EMID1, ENOX2, ERAP1, ERBB2, ERBB4, ETFDH, ETS2, EYA1, FAM13C, FAM162B, FAM165B, FAM172A, FAM175A, FAM190A, FAM26F, FAM46A, FAM49A, FAM71E1, FAM76A, FAM85A, FBXL5, FBXW7, FCRLB, FGD5, FGF1, FGF13, FGF19, FGF3, FGF7, FIP1L1, FKTN, FLJ10038, FLJ35220, FLJ35390, FLJ37798, FLJ39051, FOXO6, FSTL1, FUCA2, GAL, GART, GAS5, GDPD5, GLCE, GLI2, GLIS1, GLIS3, GLT25D2, GNA14, GNAS, GPR123, GPX7, GRM5, GTF2H5, GUCY1A3, H2AFJ, HCG 1776018, HDAC4, HEBP2, HELQ, HERPUD2, HHLA3, HIST1H2AC, HIST1H2BD, HIST1H2BK, HIST2H2BE, HMGCLL1, HNRNPR, HPCAL1, HPDL, HPS3, HSBP1L1, HSPA1A, HTATIP2, ICAM2, IFNAR1, IGFBP5, IGFBP7, IGSF5, IL10RB, IL13RA2, IL17D, IL20RA, IL7, IMMP2L, INSR, IRS1, ITGB1BP1, JMY, JRKL, KAT2B, KBTBD11, KCMF1, KCNMA1, KCNQ2, KCNQ5, KCTD18, KDM1A, KDSR, KHDC1, KIAA1109, KIAA1324, KIAA1598, KIAA1804, KIRREL3, KLHDC1, KRCC1, LGALS3, LGALS3BP, LIFR, LINGO1, LIPT1, LMAN2L, LMCD1, LOC100129195, LOC100129884, LOC100130522, LOC100130856, LOC100132167, LOC100216479, LOC100272217, LOC100287039, LOC151146, LOC153682, LOC220930, LOC254128, LOC255167, LOC283588, LOC285286, LOC285550, LOC285878, LOC286052, LOC339290, LOC401321, LOC645513, LOC80154, LOC90246, LOC93622, LOC94431, LPAR3, LPHN2, LRCH2, LRP2BP, LYRM1, LYRM5, MAGI2, MANSC1, MAP3K13, MAP3K5, MAP9, MAPKAP1, MBD5, MBLAC2, MBNL1, MCTP1, MED6, MEGF11, MEOX2, MET, METT5D1, MFSD4, MFSD6, MFSD9, MGAT4A, MORC3, MREG, MRPS18C, MRPS33, MST1, MTMR3, MTUS2, MXRA7, N4BP3, NAP1L3, NCAM1, NCAM2, NCOA7, NCRNA00081, NCRNA00117, NCRNA00171, NDUFA4L2, NDUFV3, NEAT1, NEBL, NEIL2, NETO1, NFATC4, NHEDC2, NIPAL3, NLRX1, NNAT, NOTCH4, NPW, NPY, NR1H3, NR2F1, NT5E, NUDT19, NUDT6, OGFRL1, OLFM3, OMA1, OPRM1, OPTN, P4HTM, PABPC4L, PABPC5, PAK1, PAM, PAPOLG, PAPPA, PBX1, PCDHB10, PCDHB16, PCNX, PCNXL2, PDCD2, PDE4DIP, PDGFRB, PEX12, PEX6, PHKA1, PHLDB3, PIBF1, PID1, PIGH, PIGP, PIK3CB, PIWIL2, PKNOX1, PKNOX2, PLA2G12A, PLCB1, PLEKHA2, PLEKHA3, PLIN2, PLRG1, POLR3GL, POU6F1, PPAPDC1A, PPOX, PPP1R14A, PPP2R5C, PPP3CA, PRAME, PRKACB, PRMT2, PRPH, PSD3, PSTK, PTGER2, PTGES, PTN, PTP4A3, PTPRD, PTPRN2, PTPRR, QPCT, RAB4A, RAB6B, RAC2, RAI2, RCAN1, RCC1, RDH13, RFPL1S, RG9MTD2, RGAG4, RHBDD1, RHBDF2, RHOU, RNF13, RNF41, RNLS, RPL31, RPL37, RPRD1A, RRN3, RSL1D1, RSPH3, SAP30, SAV1, SCG5, SCN5A, SERTAD4, SGMS1, SH3BGR, SH3GL2, SH3KBP1, SH3YL1, SIAE, SIGIRR, SIK1, SIK3, SIX2, SLC12A7, SLC16A14, SLC22A17, SLC22A5, SLC25A12, SLC25A4, SLC35F3, SLC6A15, SLIT1, SMARCA2, SMEK2, SNAP91, SNCA, SOCS5, SORCS2, SPATA17, SPATA7, SPIN3, SPINT1, SPINT2, SPOCK2, SPRED1, SSPN, ST8SIA3, STAC2, STAR, STEAP3, STOX2, STX12, STXBP5L, SUCLG2, SYNJ1, SYNPR, SYT13, TAF12, TAF1B, TBC1D12, TBC1D15, TCEAL2, TEX264, THAP2, TM2D1, TMCC1, TMEM182, TMEM184C, TMEM196, TMEM45B, TMEM5, TMEM59L, TMEM65, TMIE, TNC, TNFRSF10D, TNFSF4, TOX, TRAPPC9, TRHDE, TRIM61, TRIM69, TSPAN7, TSPAN9, TTC23, TTC39C, TUSC3, TXLNB, UBE2D3, UBE2W, UCN, UNC5A, UTP23, VPS37A, VSTM2A, WASF3, WDFY3, WDTC1, XPR1, XYLT1, ZBTB41, ZC3H12B, ZNF148, ZNF185, ZNF22, ZNF23, ZNF25, ZNF250, ZNF280D, ZNF285A, ZNF295, ZNF346, ZNF528, ZNF585A, ZNF610, ZNF641, ZNF662, ZNF677, and/or ZNF862, as compared to the expression levels of these genes in cells from the parental SiMa cell line DSMZ ACC 164.

In an aspect of this embodiment, cells from an established clonal cell line susceptible to BoNT/A intoxication exhibit at least a 1.5-fold decrease in gene expression levels of one or more genes selected from ACOT1, ADARB1, ASAM, BACE2, C11ORF70, C2CD4A, C3ORF23, CADM2, CAP2, CARTPT, CDKN1C, CFC1, CNGA3, COL5A1, CYGB, CYP2E1, CYTL1, DSCAM, ECEL1, ECHDC3, FAM26F, FAM49A, FGF1, FGF13, FGF19, FLJ39051, FUCA2, GAL, GPX7, GRM5, HCG 1776018, HHLA3, HIST1H2AC, HIST1H2BD, HSPA1A, HTATIP2, ICAM2, IGFBP5, IGFBP7, IL13RA2, KCNMA1, KCNQ5, KIAA1598, KRCC1, LMCD1, LOC100216479, LOC254128, LOC339290, LPAR3, MEGF11, MEOX2, NCAM2, NDUFA4L2, NEAT1, NNAT, NPW, NPY, PABPC4L, PAPPA, PID1, PPAPDC1A, PRAME, PRKACB, PTGER2, PTN, PTP4A3, RAC2, SLC35F3, SYT13, TCEAL2, THAP2, TMIE, TNFRSF10D, TRHDE, TXLNB, and/or ZNF662, as compared to the expression levels of these genes in cells from the parental SiMa cell line DSMZ ACC 164.

In another aspect of this embodiment, cells from an established clonal cell line susceptible to BoNT/A intoxication exhibit at least a 1.5-fold decrease in gene expression levels of one or more genes selected from ACOT1, ASAM, BACE2, C11ORF70, C2CD4A, C3ORF23, CADM2, CAP2, CARTPT, CFC1, CNGA3, CYGB, CYP2E1, CYTL1, DSCAM, ECEL1, ECHDC3, FGF1, FGF13, FUCA2, GAL, GPX7, HSPA1A, HTATIP2, ICAM2, IGFBP5, IGFBP7, KIAA1598, KRCC1, LMCD1, LOC100216479, LOC254128, LOC339290, LPAR3, MEOX2, NDUFA4L2, NEAT1, NNAT, NPW, NPY, PAPPA, PID1, PPAPDC1A, PRAME, PTN, SLC35F3, SYT13, TCEAL2, TMIE, TNFRSF10D, TRHDE, and/or ZNF662, as compared to the expression levels of these genes in cells from the parental SiMa cell line DSMZ ACC 164.

In yet another aspect of this embodiment, cells from an established clonal cell line susceptible to BoNT/A intoxication exhibit at least a 1.5-fold decrease in gene expression levels of one or more genes selected from C11ORF70, CADM2, CAP2, CARTPT, CNGA3, CYGB, CYP2E1, CYTL1, DSCAM, FGF13, FUCA2, GAL, HSPA1A, HTATIP2, ICAM2, IGFBP5, IGFBP7, LMCD1, LOC100216479, MEOX2, NDUFA4L2, NEAT1, NNAT, NPW, PID1, PRAME, SLC35F3, SYT13, TCEAL2, TMIE, TNFRSF10D, and/or ZNF662, as compared to the expression levels of these genes in cells from the parental SiMa cell line DSMZ ACC 164.

In another embodiment, cells from an established clonal cell line susceptible to BoNT/A intoxication exhibit at least a 1.5-fold increase in gene expression levels of one or more genes listed in Tables 5 or 7 as compared to the expression levels of these genes in cells from the 2D6 cell line, and at least a 1.5-fold increase in gene expression levels of one or more genes listed in in Tables 9 or 11 as compared to the expression levels of these genes in cells from a parental SiMa cell line. In aspects of this embodiment, cells from an established clonal cell line susceptible to BoNT/A intoxication exhibit at least a 1.5-fold increase in gene expression levels of one or more genes listed in Tables 5 or 7 as compared to the expression levels of these genes in cells from the 2D6 cell line, and at least a 1.5-fold increase in gene expression levels of one or more genes listed in in Tables 9 or 11 as compared to the expression levels of these genes in cells from the parental SiMa cell line DSMZ ACC 164. In other aspects of this embodiment, cells from an established clonal cell line susceptible to BoNT/A intoxication exhibit an increase in gene expression levels of one or more genes listed in Tables 5 or 7 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least 7.0-fold, or at least 8.0-fold as compared to the expression levels of these genes in cells from the 2D6 cell line, and exhibit an increase in gene expression levels of one or more genes listed in Tables 9 or 11 of, e*.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least 7.0-fold, or at least 8.0-fold as compared to the expression levels of these genes in cells from the parental SiMa cell line DSMZ ACC 164. In other aspects of this embodiment, cells from an established clonal cell line susceptible to BoNT/A intoxication exhibit an increase in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 5 or 7 as compared to the expression levels of these genes in cells from the 2D6 cell line, and listed in Tables 9 or 11 as compared to the expression levels of these genes in cells from the parental SiMa cell line DSMZ ACC 164. In yet other aspects cells from an established clonal cell line susceptible to BoNT/A intoxication exhibit an increase in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 5 or 7 as compared to the expression levels of these genes in cells from the 2D6 cell line, and listed in Tables 9 or 11 as compared to the expression levels of these genes in cells from the parental SiMa cell line DSMZ ACC 164.

In another embodiment, cells from an established clonal cell line susceptible to BoNT/A intoxication exhibit at least a 1.5-fold increase in gene expression levels of one or more genes selected from A2BP1, ALCAM, ANAPC7, ARF1, ARHGAP6, ARHGEF6, ARL17A, ASCL1, ASXL3, BASP1, BNC2, C10ORF58, C12ORF49, C1ORF43, C20ORF7, CBLB, CBWD1, CCDC3, CD9, CDC25B, CHRNA7, CPVL, CRYBG3, CSE1L, CUGBP2, CXCR4, DACT1, DAZ1, DBH, DHX35, DKFZP434L187, DLEU2, DLL3, EAF2, EFNA5, EIF3C, ELOVL7, EML1, ENC1, EXOSC6, EXPH5, EZH2, FAM181B, FAM19A4, FAM7A3, FUBP3, FZD5, GAP43, GFRA2, GNAI1, GNB4, GNG11, GPR125, GRP, GSS, ITGA6, ITGA6, ITGB5, KCTD12, KDELC2, KHDRBS3, KIF16B, KLHL13, LBH, LOC100128844, LOC340109, LOC641298, LOC728052, LUM, MAB21L1, MAN2A1, MAOA, MINA, MMD, MSN, MYO1B, MYO6, NAAA, NEDD9, NOS1, OSBPL3, PCGF5, PELI2, PFKFB3, PHF20, PLK2, PLS3, PLXNA2, PLXNA4, PM20D2, POLE, PSMA7, PTGFRN, PTPRE, PTPRG, RAB35, RAF1, RAN, RASEF, RBL1, RELL1, RGS5, RIMBP2, RNF182, RNF34, S1PR3, SALL4, SEMA6A, SEZ6L, SIM1, SLC44A5, SLC7A2, SLCO3A1, SLITRK5, SPAG6, SPATS2L, ST8SIA1, STMN3, STRA6, TCF7L2, TFPI2, TH1L, THOC4, TIMP3, TLE3, TMEM132C, TMEM178, TPBG, TRA2A, TRIM29, TRIM36, TSHZ3, TXNRD1, U2AF1, UBE2V1, VPS29, ZFHX4, ZGPAT, ZNF217, and/or ZNF503, as compared to the expression levels of these genes in cells from both the 2D6 cell line and the parental SiMa cell line DSMZ ACC 164.

In an aspect of this embodiment, cells from an established clonal cell line susceptible to BoNT/A intoxication exhibit at least a 1.5-fold increase in gene expression levels of one or more genes selected from ALCAM, ARHGAP6, ARHGEF6, ASCL1, BNC2, CCDC3, CHRNA7, CRYBG3, CUGBP2, CXCR4, DAZ1, EFNA5, ELOVL7, EML1, ENC1, EXPH5, FAM7A3, FZD5, GFRA2, GNAI1, GNB4, GNG11, GRP, ITGA6, KCTD12, LOC100128844, LOC340109, LOC728052, LUM, MAB21L1, MAB21L2, MAOA, MSN, NEDD9, OSBPL3, PLK2, PTGFRN, PTPRE, RGS5, RNF182, SIM1, SLC44A5, SLITRK5, SPAG6, TFPI2, TIMP3, TMEM132C, TPBG, TRIM29, and/or TRIM36, as compared to the expression levels of these genes in cells from both the 2D6 cell line and the parental SiMa cell line DSMZ ACC 164.

In another aspect of this embodiment, cells from an established clonal cell line susceptible to BoNT/A intoxication exhibit at least a 1.5-fold increase in gene expression levels of one or more genes selected from ALCAM, ARHGAP6, ARHGEF6, CCDC3, CHRNA7, CRYBG3, CUGBP2, CXCR4, DAZ1, ELOVL7, EML1, EXPH5, FAM7A3, GNB4, GNG11, GRP, ITGA6, KCTD12, LOC340109, LUM, MAB21L1, MAB21L2, PLK2, PTPRE, RGS5, RNF182, SIM1, SLC44A5, SLITRK5, SPAG6, TIMP3, and/or TMEM132C, as compared to the expression levels of these genes in cells from both the 2D6 cell line and the parental SiMa cell line DSMZ ACC 164.

In yet another aspect of this embodiment, cells from an established clonal cell line susceptible to BoNT/A intoxication exhibit at least a 1.5-fold increase in gene expression levels of one or more genes selected from CCDC3, CHRNA7, CRYBG3, CXCR4, DAZ1, EXPH5, FAM7A3, GNB4, GRP, KCTD12, LUM, PLK2, PTPRE, RGS5, SIM1, SLITRK5, TIMP3, and/or TMEM132C, as compared to the expression levels of these genes in cells from both the 2D6 cell line and the parental SiMa cell line DSMZ ACC 164.

In another embodiment, cells from an established clonal cell line susceptible to BoNT/A intoxication exhibit at least a 1.5-fold increase in gene expression levels of one or more genes selected from A2BP1, ALCAM, ARF1, ARL17A, ASCL1, BASP1, CD9, CUGBP2, EAF2, EIF3C, FAM181B, FUBP3, GNAI1, GNB4, GNG11, KDELC2, KHDRBS3, KLHL13, LOC641298, LOC728052, MAB21L2, MAOA, MINA, MSN, MYO6, PLK2, PLS3, RAF1, RELL1, RNF182, SEZ6L, SEZ6L, SIM1, SLC44A5, SLC7A2, SLCO3A1, SLITRK5, SPATS2L, TCF7L2, TFPI2, TMEM178, TRA2A, U2AF1, and/or ZNF217, as compared to the expression levels of these genes in cells from both the 2D6 cell line and the parental SiMa cell line DSMZ ACC 164.

In an aspect of this embodiment, cells from an established clonal cell line susceptible to BoNT/A intoxication exhibit at least a 1.5-fold increase in gene expression levels of one or more genes selected from A2BP1, ALCAM, ARF1, ASCL1, BASP1, CD9, CUGBP2, EIF3C, FUBP3, GNAI1, GNB4, GNG11, KDELC2, KHDRBS3, LOC641298, LOC728052, MAB21L2, MAOA, MINA, MSN, MYO6, PLK2, PLS3, RELL1, RNF182, SIM1, SLC44A5, SLC7A2, SLCO3A1, SLITRK5, TCF7L2, TFPI2, TMEM178, TRA2A, and/or ZNF217, as compared to the expression levels of these genes in cells from both the 2D6 cell line and the parental SiMa cell line DSMZ ACC 164.

In another aspect of this embodiment, cells from an established clonal cell line susceptible to BoNT/A intoxication exhibit at least a 1.5-fold increase in gene expression levels of one or more genes selected from ASCL1, CUGBP2, GNAI1, GNB4, GNG11, LOC728052, MAB21L2, MAOA, MSN, PLK2, RNF182, SIM1, SLC44A5, SLITRK5, and/or TFPI2, as compared to the expression levels of these genes in cells from both the 2D6 cell line and the parental SiMa cell line DSMZ ACC 164.

In yet another aspect of this embodiment, cells from an established clonal cell line susceptible to BoNT/A intoxication exhibit at least a 1.5-fold increase in gene expression levels of one or more genes selected from GNB4, GNG11, PLK2, RNF182, SIM1, SLC44A5, and/or SLITRK5, as compared to the expression levels of these genes in cells from both the 2D6 cell line and the parental SiMa cell line DSMZ ACC 164.

In still another aspect of this embodiment, cells from an established clonal cell line susceptible to BoNT/A intoxication exhibit at least a 1.5-fold increase in gene expression levels of one or more genes selected from GNB4, PLK2, SIM1, and/or SLITRK5, as compared to the expression levels of these genes in cells from both the 2D6 cell line and the parental SiMa cell line DSMZ ACC 164.

In another embodiment, cells from an established clonal cell line susceptible to BoNT/A intoxication exhibit at least a 1.5-fold decrease in gene expression levels of one or more genes listed in Tables 6 or 8 as compared to the expression levels of these genes in cells from the 2D6 cell line, and at least a 1.5-fold decrease in gene expression levels of one or more genes listed in in Tables 10 or 12 as compared to the expression levels of these genes in cells from a parental SiMa cell line. In aspects of this embodiment, cells from an established clonal cell line susceptible to BoNT/A intoxication exhibit at least a 1.5-fold decrease in gene expression levels of one or more genes listed in Tables 6 or 8 as compared to the expression levels of these genes in cells from the 2D6 cell line, and at least a 1.5-fold decrease in gene expression levels of one or more genes listed in in Tables 10 or 12 as compared to the expression levels of these genes in cells from the parental SiMa cell line DSMZ ACC 164. In other aspects of this embodiment, cells from an established clonal cell line susceptible to BoNT/A intoxication exhibit a decrease in gene expression levels of one or more genes listed in Tables 6 or 8 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least 7.0-fold, or at least 8.0-fold as compared to the expression levels of these genes in cells from the 2D6 cell line, and a decrease in gene expression levels of one or more genes listed in Tables 10 or 12 of, *e.g*., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least 7.0-fold, or at least 8.0-fold as compared to the expression levels of these genes in cells from the parental SiMa cell line DSMZ ACC 164. In other aspects of this embodiment, cells from an established clonal cell line susceptible to BoNT/A intoxication exhibit a decrease in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 6 or 8 as compared to the expression levels of these genes in cells from the 2D6 cell line, and listed in Tables 10 or 12 as compared to the expression levels of these genes in cells from the parental SiMa cell line DSMZ ACC 164. In yet other aspects cells from an established clonal cell line susceptible to BoNT/A intoxication exhibit a decrease in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 6 or 8 as compared to the expression levels of these genes in cells from the 2D6 cell line, and listed in Tables 10 or 12 as compared to the expression levels of these genes in cells from the parental SiMa cell line DSMZ ACC 164.

In an embodiment, cells from an established clonal cell line susceptible to BoNT/A intoxication exhibit at least a 1.5-fold decrease in gene expression levels of one or more genes selected from ABTB1, ACOT1, ACYP2, ADARB1, ADCY1, ANKRD50, ASAM, ASPHD1, ATP2B3, BACE1, BRUNOL4, BRWD1, BTN3A3, C16ORF52, C1ORF21, C21ORF57, C2CD2, C2CD4A, C3ORF23, C9ORF150, CAMK2N2, CAP2, CAPN2, CARTPT, CD302, CDH12, CDKN2A, CDS1, CFC1, CLCN3, CLDN12, CLMN, CNGA3, CNNM1, CNOT6L, COL6A1, CYGB, CYLD, CYP2E1, CYTL1, D4S234E, DCLK1, DCTD, DUSP16, EFNB3, ERAP1, ERBB2, ERBB4, ETFDH, FAM162B, FAM165B, FAM46A, FBXW7, FGD5, FGF13, FOXO6, FSTL1, GAL, GLT25D2, GNAS, GPR123, GPX7, GRM5, GUCY1A3, HCG 1776018, HELQ, HERPUD2, HIST1H2AC, HIST1H2BD, HIST1H2BK, HIST2H2BE, HNRNPR, HPCAL1, IL10RB, JMY, KAT2B, KBTBD11, KCNMA1, KCNQ2, KHDC1, KIAA1598, KLHDC1, LGALS3BP, LIFR, LOC100130522, LOC94431, LRP2BP, LYRM5, MAP3K5, MAP9, MCTP1, MEOX2, MFSD4, MGAT4A, MRPS33, MST1, NAP1L3, NCOA7, NCRNA00171, NDUFA4L2, NDUFV3, NEBL, NHEDC2, NIPAL3, NLRX1, NUDT19, OLFM3, PAM, PAPPA, PCNXL2, PDGFRB, PHKA1, PIGH, PLRG1, PLRG1, PPAPDC1A, PRKACB, PRPH, PTGER2, PTN, PTPRD, RAB6B, RGAG4, RHOU, RNF13, RNF41, RSL1D1, SH3GL2, SH3YL1, SIGIRR, SIK1, SLC12A7, SLC22A17, SLC35F3, SNCA, SPINT2, SPOCK2, ST8SIA3, STAR, STEAP3, STOX2, SYNJ1, SYT13, TM2D1, TMEM184C, TMEM5, TMEM59L, TMIE, TTC39C, TUSC3, TXLNB, UCN, WDFY3, WDFY3, ZNF641, and/or ZNF662, as compared to the expression levels of these genes in cells from both the 2D6 cell line and the parental SiMa cell line DSMZ ACC 164.

In an aspect of this embodiment, cells from an established clonal cell line susceptible to BoNT/A intoxication exhibit at least a 1.5-fold decrease in gene expression levels of one or more genes selected from ACOT1, ADARB1, ASAM, C2CD4A, C3ORF23, CAP2, CARTPT, CFC1, CNGA3, CYGB, CYP2E1, CYTL1, FGF13, GAL, GPX7, GRM5, HCG 1776018, HIST1H2AC, HIST1H2BD, KCNMA1, KIAA1598, MEOX2, NDUFA4L2, PPAPDC1A, PRKACB, PTGER2, PTN, SLC35F3, SYT13, TMIE, TXLNB, and/or ZNF662, as compared to the expression levels of these genes in cells from both the 2D6 cell line and the parental SiMa cell line DSMZ ACC 164.

In another aspect of this embodiment, cells from an established clonal cell line susceptible to BoNT/A intoxication exhibit at least a 1.5-fold decrease in gene expression levels of one or more genes selected from ACOT1, ASAM, C2CD4A, C3ORF23, CAP2, CARTPT, CFC1, CNGA3, CYGB, CYP2E1, CYTL1, FGF13, GAL, GPX7, KIAA1598, MEOX2, NDUFA4L2, PPAPDC1A, PTN, SLC35F3, SYT13, TMIE, and/or ZNF662, as compared to the expression levels of these genes in cells from both the 2D6 cell line and the parental SiMa cell line DSMZ ACC 164.

In yet another aspect of this embodiment, cells from an established clonal cell line susceptible to BoNT/A intoxication exhibit at least a 1.5-fold decrease in gene expression levels of one or more genes selected from CAP2, CARTPT, CNGA3, CYGB, CYP2E1, CYTL1, FGF13, GAL, MEOX2, NDUFA4L2, SLC35F3, SYT13, TMIE, and/or ZNF662, as compared to the expression levels of these genes in cells from both the 2D6 cell line and the parental SiMa cell line DSMZ ACC 164.

In another embodiment, cells from an established clonal cell line susceptible to BoNT/A intoxication exhibit at least a 1.5-fold decrease in gene expression levels of one or more genes selected from CAP2, CDKN2A, CNGA3, FAM162B, FGF13, FOXO6, FSTL1, KCNMA1, KHDC1, LIFR, MCTP1, MEOX2, PPAPDC1A, PTN, SYT13, TMIE, and/or ZNF662, as compared to the expression levels of these genes in cells from both the 2D6 cell line and the parental SiMa cell line DSMZ ACC 164.

In an aspect of this embodiment, cells from an established clonal cell line susceptible to BoNT/A intoxication exhibit at least a 1.5-fold decrease in gene expression levels of one or more genes selected from CAP2, CDKN2A, CNGA3, FGF13, FOXO6, FSTL1, KCNMA1, KHDC1, MCTP1, MEOX2, PPAPDC1A, PTN, SYT13, TMIE, and/or ZNF662, as compared to the expression levels of these genes in cells from both the 2D6 cell line and the parental SiMa cell line DSMZ ACC 164.

In another aspect of this embodiment, cells from an established clonal cell line susceptible to BoNT/A intoxication exhibit at least a 1.5-fold decrease in gene expression levels of one or more genes selected from CNGA3, FGF13, KCNMA1, MEOX2, PPAPDC1A, PTN, SYT13, TMIE, and/or ZNF662, as compared to the expression levels of these genes in cells from both the 2D6 cell line and the parental SiMa cell line DSMZ ACC 164.

In yet another aspect of this embodiment, cells from an established clonal cell line susceptible to BoNT/A intoxication exhibit at least a 1.5-fold decrease in gene expression levels of one or more genes selected from CNGA3, FGF13, MEOX2, PPAPDC1A, PTN, SYT13, TMIE, and/or ZNF662, as compared to the expression levels of these genes in cells from both the 2D6 cell line and the parental SiMa cell line DSMZ ACC 164.

In still another aspect of this embodiment, cells from an established clonal cell line susceptible to BoNT/A intoxication exhibit at least a 1.5-fold decrease in gene expression levels of one or more genes selected from CNGA3, FGF13, MEOX2, SYT13, TMIE, and/or ZNF662, as compared to the expression levels of these genes in cells from both the 2D6 cell line and the parental SiMa cell line DSMZ ACC 164.

In another embodiment, cells from an established clonal cell line susceptible to BoNT/A intoxication exhibit at least a 1.5-fold difference in gene expression levels of one or more genes listed in Tables 14 or 16 as compared to the expression levels of these genes in cells from both the 2D6 cell line and the parental SiMa cell line DSMZ ACC 164. In aspects of this embodiment, cells from an established clonal cell line susceptible to BoNT/A intoxication exhibit at least a 1.5-fold difference in gene expression levels of one or more genes listed in Tables 14 or 16 as compared to the expression levels of these genes in cells from both the 2D6 cell line and the parental SiMa cell line DSMZ ACC 164. In other aspects of this embodiment, cells from an established clonal cell line susceptible to BoNT/A intoxication exhibit a difference in gene expression levels of one or more genes listed in Tables 14 or 16 of, e.g., at least a 1.5-fold, at least a 2.0-fold, at least a 2.5-fold, at least a 3.0-fold, at least a 3.5-fold, at least a 4.0-fold, at least a 4.5-fold, at least a 5.0-fold, at least a 5.5-fold, at least a 6.0-fold, at least a 7.0-fold, or at least a 8.0-fold as compared to the expression levels of these genes in cells from both the 2D6 cell line and the parental SiMa cell line DSMZ ACC 164. In other aspects of this embodiment, cells from an established clonal cell line susceptible to BoNT/A intoxication exhibit an increase in gene expression levels of, *e.g*., 2 or more genes, 3 or more genes, 4 or more genes, 5 or more genes, 6 or more genes, 7 or more genes, 8 or more genes, 9 or more genes, 10 or more genes, 20 or more genes, 30 or more genes, 40 or more genes, 50 or more genes, 60 or more genes, 70 or more genes, 80 or more genes, 90 or more genes, or 100 or more genes listed in Tables 14 or 16 as compared to the expression levels of these genes in cells from both the 2D6 cell line and the parental SiMa cell line DSMZ ACC 164. In yet other aspects cells from an established clonal cell line susceptible to BoNT/A intoxication exhibit an increase in gene expression levels of, *e.g*., about 5 genes to about 100 genes, about 10 genes to about 100 genes, about 15 genes to about 100 genes, about 20 genes to about 100 genes, about 25 genes to about 100 genes, about 5 genes to about 75 genes, about 10 genes to about 75 genes, about 15 genes to about 75 genes, about 20 genes to about 75 genes, about 25 genes to about 75 genes, about 5 genes to about 50 genes, about 10 genes to about 50 genes, about 15 genes to about 50 genes, about 20 genes to about 50 genes, or about 25 genes to about 50 genes listed in Tables 14 or 16 as compared to the expression levels of these genes in cells from both the 2D6 cell line and the parental SiMa cell line DSMZ ACC 164.

In an embodiment, cells from an established clonal cell line susceptible to BoNT/A intoxication exhibit at least a 1.5-fold difference in gene expression levels of one or more genes selected from ACOT9, ADAMTS9, ARHGAP24, ARHGEF3, ASCL1, BARD1, BASP1, C3ORF70, C11ORF75, CCDC109B, CD9, CDCA7L, CDK2, CENPL, CLSTN2, CNTN1, CSRP2, CTSL2, CUGBP2, DEPDC1, DIAPH3, DOK5, DPYD, DYNLT3, EMILIN2, ETV1, FAM101B, FBLN1, FGFR2, FNDC5, GNAI1, GNB4, GNG11, GNG12, GPR177, GTSE1, HGF, KITLG, LPAR1, MAB21L2, MAOA, MCM10, MINA, MSN, MYO6, MYRIP, PAG1, PEG3, PLK2, POLA2, PPP1R3C, PRLHR, PRSS12, PTGR1, PTPRK, PVRL3, RAB32, RBPMS, SDC2, SGOL2, SLC43A3, SLC7A2, SMC2, SMC6, SPARC, SPC25, ST8SIA4, TCF7L1, TFPI2, TMEM35, TMEM178, TNFAIP8, TPTE, TRIP10, TWIST1, and/or ZNF521, as compared to the expression levels of these genes in cells from both the 2D6 cell line and the parental SiMa cell line DSMZ ACC 164. In another aspect of this embodiment, cells from an established clonal cell line susceptible to BoNT/A intoxication exhibit at least a 1.5-fold difference in gene expression levels of one or more genes selected from ADAMTS9, ARHGAP24, ASCL1, BARD1, BASP1, BVES, C11ORF75, CDCA7L, CNTN1, CUGBP2, DOK5, DPYD, DYNLT3, FBLN1, FGFR2, GNAI1, GNB4, GNG11, GNG12, GTSE1, GTSF1, ITPRIP, KDELC2, LOC728052, LPAR1, MAB21L2, MAOA, MINA, MSN, PEG3, PLK2, PRLHR, PRSS12, PTPRK, RBPMS, RNF182, SGOL2, SLC43A3, SLC44A5, SLC7A2, SMC6, SPARC, TFPI2, TMEM178, and/or TPTE, as compared to the expression levels of these genes in cells from both the 2D6 cell line and the parental SiMa cell line DSMZ ACC 164. In yet another aspect of this embodiment, cells from an established clonal cell line susceptible to BoNT/A intoxication exhibit at least a 1.5-fold difference in gene expression levels of one or more genes selected from ADAMTS9, ASCL1, BASP1, DOK5, DPYD, GNB4, GNG11, GTSF1, MAOA, MINA, MSN, PEG3, PLK2, PRSS12, RNF182, SLC44A5, SPARC, TFPI2, and/or TPTE, as compared to the expression levels of these genes in cells from both the 2D6 cell line and the parental SiMa cell line DSMZ ACC 164.

In another embodiment, cells from an established clonal cell line susceptible to BoNT/A intoxication exhibit at least a 1.5-fold increase in gene expression levels of one or more genes selected from ADAMTS9, ARHGAP24, ARHGEF3, ASCL1, BARD1, CD9, CDK2, CSRP2, CTSL2, DIAPH3, DOK5, DYNLT3, EMILIN2, ETV1, FBLN1, FGFR2, GNAI1, GNB4, GNG11, GNG12, HGF, KITLG, LPAR1, MCM10, MSN, PAG1, PEG3, PLK2, POLA2, PPP1R3C, PTPRK, RAB32, SDC2, SLC43A3, SLC7A2, SMC6, SPARC, SPC25, ST8SIA4, TCF7L1, TFPI2, TMEM35, TNFAIP8, TPTE, TRIP10, and/or TWIST1, as compared to the expression levels of these genes in cells from both the 2D6 cell line and the parental SiMa cell line DSMZ ACC 164. In an aspect of this embodiment, cells from an established clonal cell line susceptible to BoNT/A intoxication exhibit at least a 1.5-fold increase in gene expression levels of one or more genes selected from ADAMTS9, ARHGAP24, ASCL1, BARD1, DOK5, DYNLT3, FBLN1, FGFR2, GNAI1, GNB4, GNG11, GNG12, LPAR1, MSN, PEG3, PLK2, PTPRK, SLC43A3, SLC7A2, SMC6, SPARC, TFPI2, and/or TPTE, as compared to the expression levels of these genes in cells from both the 2D6 cell line and the parental SiMa cell line DSMZ ACC 164. In another aspect of this embodiment, cells from an established clonal cell line susceptible to BoNT/A intoxication exhibit at least a 1.5-fold increase in gene expression levels of one or more genes selected from ADAMTS9, ASCL1, DOK5, GNB4, GNG11, MSN, PEG3, PLK2, SPARC, TFPI2, and/or TPTE, as compared to the expression levels of these genes in cells from both the 2D6 cell line and the parental SiMa cell line DSMZ ACC 164.

In yet another embodiment, cells from an established clonal cell line susceptible to BoNT/A intoxication exhibit at least a 1.5-fold increase in gene expression levels of one or more genes selected from ACOT9, BASP1, C11ORF75, CCDC109B, CDCA7L, CLSTN2, CNTN1, CUGBP2, DEPDC1, DPYD, FAM101B, FNDC5, GTSE1, MAOA, MINA, MYO6, MYRIP, PLK2, PRLHR, PVRL3, RBPMS, SGOL2, SMC2, TFP12, TMEM178, and/or ZNF521, as compared to the expression levels of these genes in cells from both the 2D6 cell line and the parental SiMa cell line DSMZ ACC 164. In an aspect of this embodiment, cells from an established clonal cell line susceptible to BoNT/A intoxication exhibit at least a 1.5-fold increase in gene expression levels of one or more genes selected from BASP1, C11ORF75, CDCA7L, CNTN1, CUGBP2, DPYD, GTSE1, MAOA, MINA, PLK2, PRLHR, RBPMS, SGOL2, TFPI2, and/or TMEM178, as compared to the expression levels of these genes in cells from both the 2D6 cell line and the parental SiMa cell line DSMZ ACC 164. In another aspect of this embodiment, cells from an established clonal cell line susceptible to BoNT/A intoxication exhibit at least a 1.5-fold increase in gene expression levels of one or more genes selected from BASP1, DPYD, MAOA, MINA, PLK2, and/or TFPI2, as compared to the expression levels of these genes in cells from both the 2D6 cell line and the parental SiMa cell line DSMZ ACC 164.

In a still another embodiment, cells from an established clonal cell line susceptible to BoNT/A intoxication exhibit at least a 1.5-fold increase in gene expression levels of one or more genes selected from C3ORF70, MAB21L2, PRSS12, CENPL, GPR177, and/or PTGR1, as compared to the expression levels of these genes in cells from both the 2D6 cell line and the parental SiMa cell line DSMZ ACC 164. In an aspect of this embodiment, cells from an established clonal cell line susceptible to BoNT/A intoxication exhibit at least a 1.5-fold increase in gene expression levels of one or more genes selected from MAB21L2 and/or PRSS12, as compared to the expression levels of these genes in cells from both the 2D6 cell line and the parental SiMa cell line DSMZ ACC 164.

Aspects of the present disclosure comprise, in part, cells from an established clonal cell line susceptible to BoNT/A intoxication that exhibit an equivalent or lower EC₅₀ for BoNT/A activity relative to the EC₅₀ for BoNT/A activity of cells from a parental SiMa cell line. In an aspect of this embodiment, cells from an established clonal cell line susceptible to BoNT/A intoxication exhibit an equivalent or lower EC₅₀ for BoNT/A activity relative to the EC₅₀ for BoNT/A activity of cells from the parental SiMa cell line DSMZ ACC 164. In an aspect of this embodiment, cells from an established clonal cell line susceptible to BoNT/A intoxication exhibit an equivalent EC₅₀ for BoNT/A activity relative to the EC₅₀ for BoNT/A activity of cells from the parental SiMa cell line DSMZ ACC 164. In aspects of this embodiment, cells from an established clonal cell line susceptible to BoNT/A intoxication exhibit a lower EC₅₀ for BoNT/A activity of, e.g., at least 10%, at least 20%, at least 30%, at least 40%, at least 50%, at least 60%, at least 70%, at least 80%, at least 90%, or at least 100% relative to the EC₅₀ for BoNT/A activity of cells from the parental SiMa cell line DSMZ ACC 164. In other aspects of this embodiment, cells from an established clonal cell line susceptible to BoNT/A intoxication exhibit a lower EC₅₀ for BoNT/A activity of, e.g., at least 110%, at least 120%, at least 130%, at least 140%, at least 150%, at least 160%, at least 170%, at least 180%, at least 190%, or at least 200% relative to the EC₅₀ for BoNT/A activity of cells from the parental SiMa cell line DSMZ ACC 164. In yet other aspects of this embodiment, cells from an established clonal cell line susceptible to BoNT/A intoxication exhibit a lower EC₅₀ for BoNT/A activity of, e.g., at least 225%, at least 250%, at least 275%, at least 300%, at least 325%, at least 350%, at least 375%, at least 400%, at least 425%, at least 450%, at least 475%, or at least 500% relative to the EC₅₀ for BoNT/A activity of cells from the parental SiMa cell line DSMZ ACC 164. Cells of this invention from an established clonal cell line susceptible to BoNT/A intoxication exhibit an EC₅₀ for BoNT/A activity of, *e.g.*, 2.2 pM or less, 2.1 pM or less, 2.0 pM or less, 1.9 pM or less, 1.8 pM or less, 1.7 pM or less, 1.6 pM or less, 1.5 pM or less, 1.4 pM or less, 1.3 pM or less, 1.2 pM or less, 1.1 pM or less, 1.0 pM or less, 0.9 pM or less, 0.8 pM or less, 0.7 pM or less, 0.6 pM or less, 0.5 pM or less, 0.4 pM or less, 0.3 pM or less, 0.2 pM or less, or 0.1 pM or less.

Aspects of the present disclosure comprise, in part, a BoNT/A. As used herein, the term "BoNT/A" is synonymous with "botulinum neurotoxin serotype A" or "botulinum neurotoxin type A" and refers to both a naturally-occurring BoNT/A or a non-naturally occurring BoNT/As thereof, and includes BoNT/A complex comprising the about 150 kDa BoNT/A neurotoxin and associated non-toxin associated proteins (NAPs), as well as the about 150 kDa BoNT/A neurotoxin alone. Non-limiting examples of BoNT/A complexes include, *e.g*., the 900-kDa BoNT/A complex, the 500-kDa BoNT/A complex, the 300-kDa BoNT/A complex. Non-limiting examples of the about 150 kDa BoNT/A neurotoxin include, *e.g*., SEQ ID NO: 1, SEQ ID NO: 2, SEQ ID NO: 3, SEQ ID NO: 4.

As used herein, the term "naturally occurring BoNT/A" refers to any BoNT/A produced by a naturally-occurring process, including, without limitation, BoNT/A isoforms produced from a posttranslational modification, an alternatively-spliced transcript, or a spontaneous mutation, and BoNT/A subtypes, such as, *e.g*., a BoNT/A1 subtype, BoNT/A2 subtype, BoNT/A3 subtype, BoNT/A4 subtype, and BoNT/A5 subtype. A naturally occurring BoNT/A includes, without limitation, SEQ ID NO: 1, SEQ ID NO: 2, SEQ ID NO: 3, SEQ ID NO: 4, or one that substitutes, deletes or adds, *e.g*., 1 or more, 2 or more, 3 or more, 4 or more, 5 or more, 6 or more, 7 or more, 8 or more, 9 or more, 10 or more, 20 or more, 30 or more, 40 or more, 50 or more, or 100 amino acids from SEQ ID NO: 1, SEQ ID NO: 2, SEQ ID NO: 3, or SEQ ID NO: 4. Commercially available pharmaceutical compositions of a naturally-occurring BoNT/A includes, without limitation, BOTOX® (Allergan, Inc., Irvine, CA), DYSPORT®/RELOXIN®, (Ipsen Ltd., Slough, England), PURTOX® (Mentor Corp., Santa Barbara, CA), XEOMIN® (Merz Pharmaceuticals, GmbH., Frankfurt, Germany), NEURONOX® (Medy-Tox, Inc., Ochang-myeon, South Korea), BTX-A.

As used herein, the term "non-naturally occurring BoNT/A" refers to any BoNT/A whose structure was modified with the aid of human manipulation, including, without limitation, a BoNT/A with an altered amino acid sequence produced by genetic engineering using random mutagenesis or rational design and a BoNT/A produced by in vitro chemical synthesis. Non-limiting examples of non-naturally occurring BoNT/As are described in, *e.g*., Steward, L.E. et al., *Post*-*translational Modifications and Clostridial Neurotoxins*, U.S. Patent 7,223,577; Dolly, J.O. et al., *Activatable Clostridial Toxins*, U.S. Patent No. 7,419,676; Steward, L.E. et al., *Clostridial Neurotoxin Compositions and Modified Clostridial Neurotoxins*, US 2004/0220386; Steward, L.E. et al., *Modified Clostridial Toxins With Enhanced Targeting Capabilities For Endogenous Clostridial Toxin Receptor Systems*, U.S. Patent Publication No. 2008/0096248; Steward, L.E. et al., *Modified Clostridial Toxins With Altered Targeting Capabilities For Clostridial Toxin Target Cells*, U.S. Patent Publication No. 2008/0161543; Steward, L.E. et al., *Modified Clostridial Toxins With Enhanced Translocation Capabilities and Altered Targeting Activity For Clostridial Toxin Target* Cells, U.S. Patent Publication No. 2008/0241881.

Thus in an embodiment, the BoNT/A activity being detected is from a naturally occurring BoNT/A. In aspects of this embodiment, the BoNT/A activity being detected is from a BoNT/A isoform or a BoNT/A subtype. In aspects of this embodiment, the BoNT/A activity being detected is from the BoNT/A of SEQ ID NO: 1, SEQ ID NO: 2, SEQ ID NO: 3, or SEQ ID NO: 4. In other aspects of this embodiment, the BoNT/A activity being detected is from a BoNT/A having, e.g., at least 70%, at least 75%, at least 80%, at least 85%, at least 90%, or at least 95% amino acid identity with SEQ ID NO: 1, SEQ ID NO: 2, SEQ ID NO: 3, or SEQ ID NO: 4. In other aspects of this embodiment, the BoNT/A activity being detected is from BOTOX®, DYSPORT®/RELOXIN®, PURTOX®, XEOMIN®, NEURONOX®, or BTX-A.

In another embodiment, the BoNT/A activity being detected is from a non-naturally occurring BoNT/A. In other aspects of this embodiment, the BoNT/A activity being detected is from a non-naturally occurring BoNT/A variant having, e.g., at least 70%, at least 75%, at least 80%, at least 85%, at least 90%, or at least 95% amino acid identity with SEQ ID NO: 1, SEQ ID NO: 2, SEQ ID NO: 3, or SEQ ID NO: 4. In other aspects of this embodiment, the BoNT/A activity being detected is from a non-naturally occurring BoNT/A variant having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more non-contiguous amino acid substitutions, deletions, or additions relative to SEQ ID NO: 1, SEQ ID NO: 2, SEQ ID NO: 3, or SEQ ID NO: 4. In yet other aspects of this embodiment, the BoNT/A activity being detected is from a non-naturally occurring BoNT/A variant having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more contiguous amino acid substitutions, deletions, or additions relative to SEQ ID NO: 1, SEQ ID NO: 2, SEQ ID NO: 3, or SEQ ID NO: 4.

Aspects of the present disclosure comprise, in part, a SNAP-25. As used herein, the term "SNAP-25" refers to a naturally-occurring SNAP-25 or a non-naturally occurring SNAP-25 which is preferentially cleaved by a BoNT/A. As used herein, the term "preferentially cleaved" refers to that the cleavage rate of a BoNT/A substrate by a BoNT/A that is at least one order of magnitude higher than the cleavage rate of any other substrate by BoNT/A. In aspects of this embodiment, the cleavage rate of a BoNT/A substrate by a BoNT/A that is at least two orders of magnitude higher, at least three orders of magnitude higher, at least four orders of magnitude higher, or at least five orders of magnitude higher than the cleavage rate of any other substrate by BoNT/A.

As used herein, the term "naturally occurring SNAP-25" refers to any SNAP-25 produced by a naturally-occurring process, including, without limitation, SNAP-25 isoforms produced from a post-translational modification, an alternatively-spliced transcript, or a spontaneous mutation, and SNAP-25 subtypes. A naturally occurring SNAP-25 includes, without limitation, SEQ ID NO. 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24, or one that substitutes, deletes or adds, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more amino acids from SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

As used herein, the term "non-naturatty occurring SNAP-25" refers to any SNAP-25 whose structure was modified with the aid of human manipulation, including, without limitation, a SNAP-25 produced by genetic engineering using random mutagenesis or rational design and a SNAP-25 produced by in vitro chemical synthesis. Non-limiting examples of non-naturally occurring SNAP-25s are described in, *e.g*., Steward, L.E. et al., *FRET Protease Assays for Clostridial Toxins*, U.S. Patent 7,332,567; Fernandez-Salas et al., *Lipohilic Dye-based FRET Assays for Clostridial Toxin Activity*, U.S. Patent Publication 2008/0160561. A non-naturally occurring SNAP-25 may substitute, delete or add, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more amino acids from SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

Thus in an embodiment, a SNAP-25 is a naturally occurring SNAP-25. In aspects of this embodiment, the SNAP-25 is a SNAP-25 isoform or a SNAP-25 subtype. In aspects of this embodiment, the naturally occurring SNAP-25 is the naturally occurring SNAP-25 of SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. In other aspects of this embodiment, the SNAP-25 is a naturally occurring SNAP-25 having, e.g., at least 70% amino acid identity, at least 75%, at least 80%, at least 85%, at least 90%, or at least 95% amino acid identity with SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

In another embodiment, a SNAP-25 is a non-naturally occurring SNAP-25. In other aspects of this embodiment, the SNAP-25 is a non-naturally occurring SNAP-25 having, e.g., at least 70%, at least 75%, at least 80%, at least 85%, at least 90%, or at least 95% amino acid identity with SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. In other aspects of this embodiment, the SNAP-25 is a non-naturally occurring SNAP-25 having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more non-contiguous amino acid substitutions, deletions, or additions relative to SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. In yet other aspects of this embodiment, the SNAP-25 is a non-naturally occurring SNAP-25 having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more contiguous amino acid substitutions, deletions, or additions relative to SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

A SNAP-25 can be an endogenous SNAP-25 or an exogenous SNAP-25. As used herein, the term "endogenous SNAP-25" refers to a SNAP-25 naturally present in the cell because it is naturally encoded within the cell's genome, such that the cell inherently expresses the SNAP-25 without the need of an external source of SNAP-25 or an external source of genetic material encoding a SNAP-25. The expression of an endogenous SNAP-25 may be with or without environmental stimulation such as, e.g., cell differentiation. By definition, an endogenous SNAP-25 can only be a naturally-occurring SNAP-25 or variants thereof. For example, the following established cell lines express an endogenous SNAP-25: BE(2)-M17, Kelly, LA1-55n, N1E-115, N4TG3, N18, Neuro-2a, NG108-15, PC12, SH-SY5Y, SiMa, and SK-N-BE(2)-C.

As used herein, the term "exogenous SNAP-25" refers to a SNAP-25 expressed in a cell through the introduction of an external source of SNAP-25 or an external source of genetic material encoding a SNAP-25 by human manipulation. The expression of an exogenous SNAP-25 may be with or without environmental stimulation such as, e.g., cell differentiation. As a non-limiting example, cells from an established clonal cell line can express an exogenous SNAP-25 by transient or stably transfection of a SNAP-25 coding sequence. As another non-limiting example, cells from an established clonal cell line can express an exogenous SNAP-25 by protein transfection of a SNAP-25. An exogenous SNAP-25 can be a naturally-occurring SNAP-25 or variants thereof, or a non-naturally occurring SNAP-25 or variants thereof.

Thus in an embodiment, cells from an established clonal cell line express an endogenous SNAP-25. In aspects of this embodiment, the endogenous SNAP-25 expressed by cells from an established clonal cell line is a naturally-occurring SNAP-25. In other aspects of this embodiment, the endogenous SNAP-25 expressed by cells from an established clonal cell line is SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. In yet other aspects of this embodiment, the endogenous SNAP-25 expressed by cells from an established clonal cell line is a naturally occurring SNAP-25, such as, e.g., a SNAP-25 isoform or a SNAP-25 subtype. In other aspects of this embodiment, the endogenous SNAP-25 expressed by cells from an established clonal cell line is a naturally occurring SNAP-25 having, e.g., at least 70% amino acid identity, at least 75%, at least 80%, at least 85%, at least 90%, or at least 95% amino acid identity with SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

In another embodiment, cells from an established clonal cell line are transiently or stably engineered to express an exogenous SNAP-25. In an aspect of this embodiment, cells from an established clonal cell line are transiently or stably engineered to express a naturally-occurring SNAP-25. In other aspects of this embodiment, cells from an established clonal cell line are transiently or stably engineered to express the naturally-occurring SNAP-25 of SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. In yet other aspects of this embodiment, cells from an established clonal cell line are transiently or stably engineered to express a naturally occurring SNAP-25, such as, e.g., a SNAP-25 isoform or a SNAP-25 subtype. In still other aspects of this embodiment, cells from an established clonal cell line are transiently or stably engineered to express a naturally occurring SNAP-25 having, e.g., at least 70% amino acid identity, at least 75%, at least 80%, at least 85%, at least 90%, or at least 95% amino acid identity with SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

In another aspect of the embodiment, cells from an established clonal cell line are transiently or stably engineered to express a non-naturally occurring SNAP-25. In other aspects of this embodiment, cells from an established clonal cell line are transiently or stably engineered to express a non-naturally occurring SNAP-25 having, e.g., at least 70%, at least 75%, at least 80%, at least 85%, at least 90%, or at least 95% amino acid identity with SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. In other aspects of this embodiment, cells from an established clonal cell line are transiently or stably engineered to express a non-naturally occurring SNAP-25 having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more non-contiguous amino acid substitutions, deletions, or additions relative to SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. In yet other aspects of this embodiment, cells from an established clonal cell line are transiently or stably engineered to express a non-naturally occurring SNAP-25 having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more contiguous amino acid substitutions, deletions, or additions relative to SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

Assays that detect the cleavage of a BoNT/A substrate after exposure to a BoNT/A can be used to assess whether a cell is expressing an endogenous or an exogenous SNAP-25. In these assays, generation of a SNAP-25 cleavage-product would be detected in cells expressing a SNAP-25 after BoNT/A treatment. Non-limiting examples of specific Western blot analysis, as well as well-characterized reagents, conditions and protocols are readily available from commercial vendors that include, without limitation, Amersham Biosciences, Piscataway, NJ; Bio-Rad Laboratories, Hercules, CA; Pierce Biotechnology, Inc., Rockford, IL; Promega Corporation, Madison, WI, and Stratagene, Inc., La Jolla, CA. It is understood that these and similar assays for SNAP-25 cleavage can be useful in identifying cells expressing an endogenous or an exogenous SNAP-25.

As non-limiting examples, Western blot analysis using an antibody that recognizes BoNT/A SNAP-25-cleaved product or both the cleaved and uncleaved forms of SNAP-25 can be used to assay for uptake of BoNT/A. Examples of α-SNAP-25 antibodies useful for these assays include, without limitation, α-SNAP-25 mouse monoclonal antibody SMI-81 (Sternberger Monoclonals Inc., Lutherville, MD), mouse α-SNAP-25 monoclonal antibody CI 71.1 (Synaptic Systems, Goettingen, Germany), α-SNAP-25 mouse monoclonal antibody CI 71.2 (Synaptic Systems, Goettingen, Germany), α-SNAP-25 mouse monoclonal antibody SP12 (Abcam, Cambridge, MA), α-SNAP-25 rabbit polyclonal antiserum (Synaptic Systems, Goettingen, Germany), α-SNAP-25 rabbit polyclonal antiserum (Abcam, Cambridge, MA), and α-SNAP-25 rabbit polyclonal antiserum S9684 (Sigma, St Louis, MO).

Aspects of the present disclosure comprise, in part, a BoNT/A receptor. As used herein, the term "BoNT/A receptor" refers to either a naturally-occurring BoNT/A receptor or a non-naturally occurring BoNT/A receptor which preferentially interacts with BoNT/A in a manner that elicits a BoNT/A intoxication response. As used herein, the term "preferentially interacts" refers to that the equilibrium dissociation constant (KD) of BoNT/A for a BoNT/A receptor is at least one order of magnitude less than that of BoNT/A for any other receptor. The equilibrium dissociation constant, a specific type of equilibrium constant that measures the propensity of an BoNT/A-BoNT/A receptor complex to separate (dissociate) reversibly into its component molecules, namely the BoNT/A and the BoNT/A receptor complex. The disassociation constant is defined as KD=[L][R]/[C], where [L] equals the molar concentration of BoNT/A, [R] is the molar concentration of a BoNT/A receptor, and [C] is the molar concentration of the BoNT/A-BoNT/A receptor complex, and where all concentrations are of such components when the system is at equilibrium. The smaller the dissociation constant, the more tightly bound the BoNT/A is to its receptor, or the higher the binding affinity between BoNT/A and BoNT/A receptor. In aspects of this embodiment, the disassociation constant of BoNT/A for a BoNT/A receptor is at least two orders of magnitude less, at least three orders of magnitude less, at least four orders of magnitude less, or at least five orders of magnitude less than that of BoNT/A for any other receptor. In other aspects of this embodiment, the binding affinity of a BoNT/A that preferentially interacts with a BoNT/A receptor can have an equilibrium disassociation constant of, *e.g*., of 500 nM or less, 400 nM or less, 300 nM or less, 200 nM, or less 100 nM or less. In other aspects of this embodiment, the binding affinity of a BoNT/A that preferentially interacts with a BoNT/A receptor can have an equilibrium disassociation constant of, *e.g*., of 90 nM or less, 80 nM or less, 70 nM or less, 60 nM or less, 50 nM or less, 40 nM or less, 30 nM or less, 20 nM, or less 10 nM or less. As used herein, the term "elicits a BoNT/A intoxication response" refers to the ability of a BoNT/A receptor to interact with a BoNT/A to form a neurotoxin/receptor complex and the subsequent internalization of that complex into the cell cytoplasm.

As used herein, the term "naturally occurring BoNT/A receptor" refers to any BoNT/A receptor produced by a naturally-occurring process, including, without limitation, BoNT/A receptor isoforms produced from a post-translational modification, an alternatively-spliced transcript, or a spontaneous mutation, and BoNT/A receptor subtypes. A naturally occurring BoNT/A receptor includes, without limitation, a fibroblast growth factor receptor 2 (FGFR2), a fibroblast growth factor receptor 3 (FGFR3), a synaptic vesicle glycoprotein 2 (SV2), and a complex ganglioside like GT1b, such as those described in Ester Fernandez-Salas, et al., *Botulinum Toxin Screening Assays*, U.S. Patent Publication 2008/0003240; Ester Fernandez-Salas, et al., *Botulinum Toxin Screening Assays*, U.S. Patent Publication 2008/0182799; Min Dong et al., SV2 is the Protein Receptor for Botulinum Neurotoxin A, Science (2006); S. Mahrhold et al, The Synaptic Vesicle Protein 2C Mediates the Uptake of Botulinum Neurotoxin A into Phrenic Nerves, 580(8) FEBS Lett. 2011-2014 (2006), each of which is hereby incorporated by reference in its entirety. A naturally occurring FGFR2 includes, without limitation, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, and SEQ ID NO: 70, or one that substitutes, deletes or adds, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more amino acids from SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, and SEQ ID NO: 70. A naturally occurring FGFR3 includes, without limitation, SEQ ID NO: 25, SEQ ID NO: 26, and SEQ ID NO: 27, or one that substitutes, deletes or adds, *e.g*., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more amino acids from SEQ ID NO: 25, SEQ ID NO: 26, and SEQ ID NO: 27. A naturally occurring SV2 includes, without limitation, SEQ ID NO: 28, SEQ ID NO: 29, SEQ ID NO: 30, and SEQ ID NO: 31, or one that substitutes, deletes or adds, *e.g*., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more amino acids from SEQ ID NO: 28, SEQ ID NO: 29, SEQ ID NO: 30, and SEQ ID NO: 31.

As used herein, the term "non-naturally occurring BoNT/A receptor variant" refers to any BoNT/A receptor produced with the aid of human manipulation or design, including, without limitation, a BoNT/A receptor produced by genetic engineering using random mutagenesis or rational design and a BoNT/A receptor produced by chemical synthesis. Non-limiting examples of non-naturally occurring BoNT/A variants include, *e.g*., conservative BoNT/A receptor variants, non-conservative BoNT/A receptor variants, BoNT/A receptor chimeric variants and active BoNT/A receptor fragments.

As used herein, the term "non-naturally occurring BoNT/A receptor" refers to any BoNT/A receptor whose structure was modified with the aid of human manipulation, including, without limitation, a BoNT/A receptor produced by genetic engineering using random mutagenesis or rational design and a BoNT/A receptor produced by in vitro chemical synthesis. Non-limiting examples of non-naturally occurring BoNT/A receptors are described in, *e.g*., Ester Fernandez-Salas, et al., *Botulinum Toxin Screening Assays*, U.S. Patent Publication 2008/0003240; Ester Fernandez-Salas, et al., *Botulinum Toxin Screening Assays*, U.S. Patent Publication 2008/0182799, each of which is hereby incorporated by reference in its entirety. A non-naturally occurring BoNT/A receptor may substitute, delete or add, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more amino acids from SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70.

Thus in an embodiment, a BoNT/A receptor is a naturally occurring BoNT/A receptor such as, *e.g*., FGFR2, FGFR3 or SV2. In aspects of this embodiment, the BoNT/A receptor is a BoNT/A receptor isoform or a BoNT/A receptor subtype. In aspects of this embodiment, the naturally occurring BoNT/A receptor is the naturally occurring BoNT/A receptor of SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70. In other aspects of this embodiment, the BoNT/A receptor is a naturally occurring BoNT/A receptor having, e.g., at least 70% amino acid identity, at least 75%, at least 80%, at least 85%, at least 90%, or at least 95% amino acid identity with SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70.

In another embodiment, a BoNT/A receptor is a non-naturally occurring BoNT/A receptor, such as, *e.g*., a genetically-engineered FGFR2, a genetically-engineered FGFR3, or a genetically-engineered SV2. In other aspects of this embodiment, the BoNT/A receptor is a non-naturally occurring BoNT/A receptor having, e.g., at least 70%, at least 75%, at least 80%, at least 85%, at least 90%, or at least 95% amino acid identity with SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70. In other aspects of this embodiment, the BoNT/A receptor is a non-naturally occurring BoNT/A receptor having, *e.g*., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more non-contiguous amino acid substitutions, deletions, or additions relative to SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70. In yet other aspects of this embodiment, the BoNT/A receptor is a non-naturally occurring BoNT/A receptor having, *e.g*., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more contiguous amino acid substitutions, deletions, or additions relative to SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70.

A BoNT/A receptor can be an endogenous BoNT/A receptor or an exogenous BoNT/A receptor. As used herein, the term "endogenous BoNT/A receptor" refers to a BoNT/A receptor naturally present in the cell because it is naturally encoded within the cell's genome, such that the cell inherently expresses the BoNT/A receptor without the need an external source of BoNT/A receptor or an external source of genetic material encoding a BoNT/A receptor. Expression of an endogenous BoNT/A receptor may be with or without environmental stimulation such as e.g., cell differentiation or promoter activation. For example, the following established clonal cell lines express at least one endogenous BoNT/A receptor: BE(2)-M17, Kelly, LA1-55n, N1E-115, N4TG3, N18, Neuro-2a, NG108-15, PC12, SH-SY5Y, SiMa, and SK-N-BE(2)-C. An endogenous BoNT/A receptor can only be a naturally-occurring BoNT/A receptor or naturally-occurring variants thereof.

As used herein, the term "exogenous BoNT/A receptor" refers to a BoNT/A receptor expressed in a cell through the introduction of an external source of BoNT/A receptor or an external source of genetic material encoding a BoNT/A receptor by human manipulation. The expression of an exogenous BoNT/A receptor may be with or without environmental stimulation such as, *e.g*., cell differentiation or promoter activation. As a non-limiting example, cells from an established clonal cell line can express one or more exogenous BoNT/A receptors by transient or stably transfection of a polynucleotide molecule encoding a BoNT/A receptor, such as, *e.g*., a FGFR2, a FGFR3, or a SV2. As another non-limiting example, cells from an established clonal cell line can express one or more exogenous BoNT/A receptors by protein transfection of the BoNT/A receptors, such as, *e.g*., a FGFR2, a FGFR3, or a SV2. An exogenous BoNT/A receptor can be a naturally-occurring BoNT/A receptor or naturally occurring variants thereof, or non-naturally occurring BoNT/A receptor or non-naturally occurring variants thereof.

Thus in an embodiment, cells from an established clonal cell line express an endogenous BoNT/A receptor. In aspects of this embodiment, the endogenous BoNT/A receptor expressed by cells from an established clonal cell line is a naturally-occurring BoNT/A receptor. In other aspects of this embodiment, the endogenous BoNT/A receptor expressed by cells from an established clonal cell line is SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70. In yet aspects of this embodiment, the endogenous BoNT/A receptor expressed by cells from an established clonal cell line is a naturally occurring BoNT/A receptor, such as, *e.g*., a BoNT/A receptor isoform or a BoNT/A receptor subtype. In other aspects of this embodiment, the endogenous BoNT/A receptor expressed by cells from an established clonal cell line is a naturally occurring BoNT/A receptor having, e.g., at least 70% amino acid identity, at least 75%, at least 80%, at least 85%, at least 90%, or at least 95% amino acid identity with SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70.

In another embodiment, cells from an established clonal cell line are transiently or stably engineered to express an exogenous BoNT/A receptor. In an aspect of this embodiment, cells from an established clonal cell line are transiently or stably engineered to express a naturally-occurring BoNT/A receptor. In other aspects of this embodiment, cells from an established clonal cell line are transiently or stably engineered to express the naturally-occurring BoNT/A receptor of SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70. In yet other aspects of this embodiment, cells from an established clonal cell line are transiently or stably engineered to express a naturally occurring BoNT/A receptor, such as, *e.g*., a BoNT/A receptor isoform or a BoNT/A receptor subtype. In still other aspects of this embodiment, cells from an established clonal cell line are transiently or stably engineered to express a naturally occurring BoNT/A receptor having, e.g., at least 70% amino acid identity, at least 75%, at least 80%, at least 85%, at least 90%, or at least 95% amino acid identity with SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70.

In another aspect of the embodiment, cells from an established clonal cell line are transiently or stably engineered to express a non-naturally occurring BoNT/A receptor. In other aspects of this embodiment, cells from an established clonal cell line are transiently or stably engineered to express a non-naturally occurring BoNT/A receptor having, e.g., at least 70%, at least 75%, at least 80%, at least 85%, at least 90%, or at least 95% amino acid identity with SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70. In other aspects of this embodiment, cells from an established clonal cell line are transiently or stably engineered to express a non-naturally occurring BoNT/A receptor having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more non-contiguous amino acid substitutions, deletions, or additions relative to SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70. In yet other aspects of this embodiment, cells from an established clonal cell line are transiently or stably engineered to express a non-naturally occurring BoNT/A receptor having, e.g., 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more contiguous amino acid substitutions, deletions, or additions relative to SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, or SEQ ID NO: 70

In another embodiment, cells from an established clonal cell line are transiently or stably engineered to express an exogenous FGFR2, an exogenous FGFR3, an exogenous SV2, or any combination thereof. In aspects of this embodiment, cells from an established clonal cell line are transiently or stably engineered to express a naturally-occurring FGFR2, a naturally-occurring FGFR3, a naturally-occurring SV2, or any combination thereof. In yet other aspects of this embodiment, cells from an established clonal cell line are transiently or stably engineered to express a non-naturally-occurring FGFR2, a non-naturally-occurring FGFR3, a non-naturally-occurring SV2, or any combination thereof. In still other aspects of this embodiment, cells from an established clonal cell line are transiently or stably engineered to express either a naturally-occurring FGFR2 or a non-naturally-occurring FGFR2, a naturally-occurring FGFR3 or a non-naturally-occurring FGFR3, a naturally-occurring SV2 or a non-naturally-occurring SV2, or any combination thereof.

Cells that express one or more endogenous or exogenous BoNT/A receptors can be identified by routine methods including direct and indirect assays for toxin uptake. Assays that determine BoNT/A binding or uptake properties can be used to assess whether a cell is expressing a BoNT/A receptor. Such assays include, without limitation, cross-linking assays using labeled BoNT/A, such as, *e.g*., [¹²⁵I] BoNT/A, see, *e.g*., Noriko Yokosawa et al., Binding of Clostridium botulinum type C neurotoxin to different neuroblastoma cell lines, 57(1) Infect. Immun. 272-277 (1989); Noriko Yokosawa et al., Binding of botulinum type CI, D and E neurotoxins to neuronal cell lines and synaptosomes, 29(2) Toxicon 261-264 (1991); and Tei-ichi Nishiki et al., Identification of protein receptor for Clostridium botulinum type B neurotoxin in rat brain synaptosomes, 269(14) J. Biol. Chem. 10498-10503 (1994). Other non-limiting assays include immunocytochemical assays that detect BoNT/A binding using labeled or unlabeled antibodies, see, e.g., Atsushi Nishikawa et al., The receptor and transporter for internalization of Clostridium botulinum type C progenitor toxin into HT-29 cells, 319(2) Biochem. Biophys. Res. Commun. 327-333 (2004) and immunoprecipitation assays, see, *e.g*., Yukako Fujinaga et al., Molecular characterization of binding subcomponents of Clostridium botulinum type C progenitor toxin for intestinal epithelial cells and erythrocytes, 150(Pt 5) Microbiology 1529-1538 (2004), that detect bound toxin using labeled or unlabeled antibodies. Antibodies useful for these assays include, without limitation, antibodies selected against BoNT/A, antibodies selected against a BoNT/A receptor, such as, e.g., FGFR2, FGFR3, or SV2, and/or antibodies selected against a ganglioside, such as, e.g., GD1a, GD1b, GD3, GQ1b, or GT1b. If the antibody is labeled, the binding of the molecule can be detected by various means, including Western blot analysis, direct microscopic observation of the cellular location of the antibody, measurement of cell or substrate-bound antibody following a wash step, flow cytometry, electrophoresis or capillary electrophoresis, employing techniques well-known to those of skill in the art. If the antibody is unlabeled, one may employ a labeled secondary antibody for indirect detection of the bound molecule, and detection can proceed as for a labeled antibody. It is understood that these and similar assays that determine BoNT/A uptake properties or characteristics can be useful in identifying cells expressing endogenous or exogenous BoNT/A receptors.

Assays that monitor the release of a molecule after exposure to BoNT/A can also be used to assess whether a cell is expressing one or more endogenous or exogenous BoNT/A receptors. In these assays, inhibition of the molecule's release would occur in cells expressing a BoNT/A receptor after BoNT/A treatment. Well known assays include methods that measure inhibition of radio-labeled catecholamine release from neurons, such as, e.g., [3H] noradrenaline or [3H] dopamine release, see e.g., A Fassio et al., Evidence for calcium-dependent vesicular transmitter release insensitive to tetanus toxin and botulinum toxin type F, 90(3) Neuroscience 893-902 (1999); and Sara Stigliani et al., The sensitivity of catecholamine release to botulinum toxin C1 and E suggests selective targeting of vesicles set into the readily releasable pool, 85(2) J. Neurochem. 409-421 (2003), or measures catecholamine release using a fluorometric procedure, see, e.g., Anton de Paiva et al., A role for the interchain disulfide or its participating thiols in the internalization of botulinum neurotoxin A revealed by a toxin derivative that binds to ecto-acceptors and inhibits transmitter release intracellularly, 268(28) J. Biol. Chem. 20838-20844 (1993); Gary W. Lawrence et al., Distinct exocytotic responses of intact and permeabilised chromaffin cells after cleavage of the 25-kDa synaptosomal-associated protein (SNAP-25) or synaptobrevin by botulinum toxin A or B, 236(3) Eur. J. Biochem. 877-886 (1996); and Patrick Foran et al., Botulinum neurotoxin C1 cleaves both syntaxin and SNAP-25 in intact and permeabilized chromaffin cells: correlation with its blockade of catecholamine release, 35(8) Biochemistry 2630-2636 (1996). Other non-limiting examples include assays that measure inhibition of hormone release from endocrine cells, such as, e.g., anterior pituitary cells or ovarian cells. It is understood that these and similar assays for molecule release can be useful in identifying cells expressing endogenous or exogenous or BoNT/A receptors.

Assays that detect the cleavage of a BoNT/A substrate after exposure to a BoNT/A can also be used to assess whether a cell is expressing one or more endogenous or exogenous BoNT/A receptors. In these assays, generation of a BoNT/A substrate cleavage-product, or disappearance of the intact BoNT/A substrate, would be detected in cells expressing a BoNT/A receptor after BoNT/A treatment. Non-limiting examples of specific Western blot analysis, as well as well-characterized reagents, conditions and protocols are readily available from commercial vendors that include, without limitation, Amersham Biosciences, Piscataway, NJ; Bio-Rad Laboratories, Hercules, CA; Pierce Biotechnology, Inc., Rockford, IL; Promega Corporation, Madison, WI, and Stratagene, Inc., La Jolla, CA. It is understood that these and similar assays for BoNT/A substrate cleavage can be useful in identifying cells expressing endogenous or exogenous BoNT/A receptors.

As non-limiting examples, Western blot analysis using an antibody that recognizes BoNT/A SNAP-25-cleaved product or both the cleaved and uncleaved forms of SNAP-25 can be used to assay for uptake of BoNT/A. Examples of α-SNAP-25 antibodies useful for these assays include, without limitation, SMI-81 α-SNAP-25 mouse monoclonal antibody (Sternberger Monoclonals Inc., Lutherville, MD), CI 71.1 mouse α-SNAP-25 monoclonal antibody (Synaptic Systems, Goettingen, Germany), CI 71.2 α-SNAP-25 mouse monoclonal antibody (Synaptic Systems, Goettingen, Germany), SP12 α-SNAP-25 mouse monoclonal antibody (Abcam, Cambridge, MA), α-SNAP-25 rabbit polyclonal antiserum (Synaptic Systems, Goettingen, Germany), α-SNAP-25 rabbit polyclonal antiserum S9684 (Sigma, St. Louis, MO), and α-SNAP-25 rabbit polyclonal antiserum (Abcam, Cambridge, MA).

Aspects of the present disclosure provide cells that through genetic manipulation or recombinant engineering are made to express an exogenous SNAP-25 and/or one or more exogenous BoNT/A receptors. Cells useful to express an exogenous SNAP-25 and/or one or more exogenous BoNT/A receptors through genetic manipulation or recombinant engineering include neuronal cells and non-neuronal cells that may or may not express an endogenous SNAP-25 and/or one or more endogenous BoNT/A receptors. It is further understood that such genetically manipulated or recombinantly engineered cells may express an exogenous SNAP-25 and one or more exogenous BoNT/A receptors under control of a constitutive, tissue-specific, cell-specific or inducible promoter element, enhancer element or both. It is understood that any cell is useful as long as the cell can be genetically manipulated or recombinantly engineered to expresses an exogenous SNAP-25 and/or one or more exogenous BoNT/A receptors and is capable of undergoing BoNT/A intoxication.

Methods useful for introducing into a cell an exogenous polynucleotide molecule encoding a component necessary for the cells to undergo the overall cellular mechanism whereby a BoNT/A proteolytically cleaves a SNAP-25 substrate, such as, *e.g*., a SNAP-25, a FGFR2, a FGFR3, or a SV2, include, without limitation, chemical-mediated delivery methods, such as, *e.g*., calcium phosphate-mediated, diethyl-aminoethyl (DEAE) dextran-mediated, lipid-mediated, polyethyleneimine (PEI)-mediated, polylysine-mediated and polybrene-mediated; physical-mediated delivery methods, such as, *e.g.*, biolistic particle delivery, microinjection, protoplast fusion and electroporation; and viral-mediated delivery methods, such as, *e.g*., retroviral-mediated transfection, see *e*.*g*., Introducing Cloned Genes into Cultured Mammalian Cells, pp. 16.1-16.62 (Sambrook & Russell, eds., Molecular Cloning A Laboratory Manual, Vol. 3, 3rd ed. 2001); Alessia Colosimo et al., Transfer and Expression of Foreign Genes in Mammalian Cells, 29(2) Biotechniques 314-318, 320-322, 324 (2000); Philip Washbourne & A. Kimberley McAllister, Techniques for Gene Transfer into Neurons, 12(5) Curr. Opin. Neurobiol. 566-573 (2002); and Current Protocols in Molecular Biology, John Wiley and Sons, pp 9.16.4-9.16.11 (2000). One skilled in the art understands that selection of a specific method to introduce a polynucleotide molecule into a cell will depend, in part, on whether the cell will transiently or stably contain a component necessary for the cells to undergo the overall cellular mechanism whereby a BoNT/A proteolytically cleaves a SNAP-25 substrate. Non-limiting examples of polynucleotide molecule encoding a component necessary for the cells to undergo the overall cellular mechanism whereby a BoNT/A proteolytically cleaves a SNAP-25 substrate as follows: FGFR2 polynucleotide molecule of SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, or SEQ ID NO: 138; FGFR3 polynucleotide molecule of SEQ ID NO: 139, SEQ ID NO: 140, or SEQ ID NO: 141; SV2 polynucleotide molecule of SEQ ID NO: 142, SEQ ID NO: 143, or SEQ ID NO: 144; and SNAP-25 polynucleotide molecule of SEQ ID NO: 145, or SEQ ID NO: 146.

Chemical-mediated delivery methods are well-known to a person of ordinary skill in the art and are described in, *e.g*., Martin Jordan & Florian Worm, Transfection of Adherent and Suspended Cells by Calcium Phosphate, 33(2) Methods 136-143 (2004); Chun Zhang et al., Polyethylenimine Strategies for Plasmid Delivery to Brain-Derived Cells, 33(2) Methods 144-150 (2004), each of which is hereby incorporated by reference in its entirety. Such chemical-mediated delivery methods can be prepared by standard procedures and are commercially available, see, *e*.*g*., CellPhect Transfection Kit (Amersham Biosciences, Piscataway, NJ); Mammalian Transfection Kit, Calcium phosphate and DEAE Dextran, (Stratagene, Inc., La Jolla, CA); Lipofectamine™ Transfection Reagent (Invitrogen, Inc., Carlsbad, CA); ExGen 500 Transfection kit (Fermentas, Inc., Hanover, MD), and SuperFect and Effectene Transfection Kits (Qiagen, Inc., Valencia, CA).

Physical-mediated delivery methods are well-known to a person of ordinary skill in the art and are described in, *e.g.*, Jeike E. Biewenga et al., Plasmid-Mediated Gene Transfer in Neurons using the Biolistics Technique, 71(1) J. Neurosci. Methods. 67-75 (1997); John O'Brien & Sarah C. R. Lummis, Biolistic and Diolistic Transfection: Using the Gene Gun to Deliver DNA and Lipophilic Dyes into Mammalian Cells, 33(2) Methods 121-125 (2004); M. Golzio et al., In Vitro and In Vivo Electric Field-Mediated Permeabilization, Gene Transfer, and Expression, 33(2) Methods 126-135 (2004); and Oliver Greschet al., New Non-Viral Method for Gene Transfer into Primary Cells, 33(2) Methods 151-163 (2004).

Viral-mediated delivery methods are well-known to a person of ordinary skill in the art and are described in, *e.g*., Chooi M. Lai et al., Adenovirus and Adeno-Associated Virus Vectors, 21(12) DNA Cell Biol. 895-913 (2002); Ilya Frolov et al., Alphavirus-Based Expression Vectors: Strategies and Applications, 93(21) Proc. Natl. Acad. Sci. U. S. A. 11371-11377 (1996); Roland Wolkowicz et al., Lentiviral Vectors for the Delivery of DNA into Mammalian Cells, 246 Methods Mol. Biol. 391-411 (2004); A. Huser & C. Hofmann, Baculovirus Vectors: Novel Mammalian Cell Gene-Delivery Vehicles and Their Applications, 3(1) Am. J. Pharmacogenomics 53-63 (2003); Tiziana Tonini et al., Transient Production of Retroviral- and Lentiviral-Based Vectors for the Transduction of Mammalian Cells, 285 Methods Mol. Biol. 141-148 (2004); Manfred Gossen & Hermann Bujard, *Tight Control of Gene Expression in Eukaryotic Cells by Tetracycline-Responsive Promoters*, U.S. Patent No. 5.464.758; Hermann Bujard & Manfred Gossen, *Methods for Regulating Gene Expression*, U.S. Patent No. 5,814,618; David S. Hogness, *Polynucleotides Encoding Insect Steroid Hormone Receptor Polypeptides and Cells Transformed With Same*, U.S. Patent No. 5,514,578; David S. Hogness, *Polynucleotide Encoding Insect Ecdysone Receptor*, U.S. Patent 6,245,531; Elisabetta Vegeto et al., *Progesterone Receptor Having C*. *Terminal Hormone Binding Domain Truncations*, U.S. Patent No. 5,364,791: Elisabetta Vegeto et al., *Mutated Steroid Hormone Receptors*, *Methods for Their Use and Molecular Switch for Gene Therapy*, U.S. Patent No. 5,874,534. Such viral-mediated delivery methods can be prepared by standard procedures and are commercially available, see, e.g., VIRAPOWER™ Adenoviral Expression System (Invitrogen, Inc., Carlsbad, CA) and VIRAPOWER™ Adenoviral Expression System Instruction Manual 25-0543 version A, Invitrogen, Inc., (Jul. 15, 2002); and ADEASY™ Adenoviral Vector System (Stratagene, Inc., La Jolla, CA) and ADEASY™ Adenoviral Vector System Instruction Manual 064004f, Stratagene, Inc. Furthermore, such viral delivery systems can be prepared by standard methods and are commercially available, see, e.g., BD™ Tet-Off and Tet-On Gene Expression Systems (BD Biosciences-Clonetech, Palo Alto, CA) and BD™ Tet-Off and Tet-On Gene Expression Systems User Manual, PT3001-1, BD Biosciences Clonetech, (Mar. 14, 2003), GeneSwitch™ System (Invitrogen, Inc., Carlsbad, CA) and GENESWITCH™ System A Mifepristone-Regulated Expression System for Mammalian Cells version D, 25-0313, Invitrogen, Inc., (Nov. 4, 2002); VIRAPOWER™ Lentiviral Expression System (Invitrogen, Inc., Carlsbad, CA) and VIRAPOWER™ Lentiviral Expression System Instruction Manual 25-0501 version E, Invitrogen, Inc., (Dec. 8, 2003); and COMPLETE CONTROL® Retroviral Inducible Mammalian Expression System (Stratagene, La Jolla, CA) and COMPLETE CONTROL® Retroviral Inducible Mammalian Expression System Instruction Manual, 064005e.

As mentioned above, an exogenous component necessary for the cells to undergo the overall cellular mechanism whereby a BoNT/A proteolytically cleaves a SNAP-25 substrate, such as, *e.g*., a SNAP-25, a FGFR2, a FGFR3, or a SV2 disclosed in the present specification can be introduced into a cell. Any and all methods useful for introducing such an exogenous component with a delivery agent into a cell population can be useful with the proviso that this method introduce the exogenous component disclosed in the present specification in at least 50% of the cells within a given cell population. Thus, aspects of this embodiment can include a cell population in which, e.g., at least 50%, at least 60%, at least 70%, at least 80%, or at least 90% of the given cell population contains an exogenous component necessary for the cells to undergo the overall cellular mechanism whereby a BoNT/A proteolytically cleaves a SNAP-25 substrate, such as, *e.g*., a SNAP-25, a FGFR2, a FGFR3, or a SV2 disclosed in the present specification. As used herein, the term "delivery agent" refers to any molecule that enables or enhances internalization of a covalently-linked, non-covalently-linked or in any other manner associated with a polypeptide into a cell. Thus, the term "delivery agent" encompasses, without limitation, proteins, peptides, peptidomimetics, small molecules, polynucleotide molecules, liposomes, lipids, viruses, retroviruses and cells that, without limitation, transport a covalently or non-covalently linked molecule to the cell membrane, cell cytoplasm or nucleus. It further is understood that the term "delivery agent" encompasses molecules that are internalized by any mechanism, including delivery agents which function via receptor mediated endocytosis and those which are independent of receptor mediated endocytosis.

A delivery agent can also be an agent that enables or enhances cellular uptake of a covalently linked component, like FGFR2, FGFR3, SV2, or SNAP-25, such as, *e.g*., by chemical conjugation or by genetically produced fusion proteins. Methods that covalently link delivery agents and methods of using such agents are described in, e.g., Steven F. Dowdy, *Protein Transduction System and Methods of Use Thereof*, International Publication No WO 00/34308; Gérard Chassaing & Alain Prochiantz, *Peptides which can be Used as Vectors for the Intracellular Addressing of Active Molecules*, U.S. Patent No. 6,080,724; Alan Frankel et al., *Fusion Protein Comprising TAT-derived Transport Moiert*, U.S. Patent No. 5,674,980; Alan Frankel et al., *TAT*-*derived Transport Polypeptide Conjugates*, U.S. Patent No. 5,747,641; Alan Frankel et al., *TAT-derived Transport Polypeptides and Fusion Proteins*, U.S. Patent No. 5,804,604; Peter F. J. O'Hare et al., *Use of Transport Proteins*, U.S. Patent No. 6,734,167; Yao-Zhong Lin & Jack J. Hawiger, *Method for Importing Biologically Active Molecules into Cells*, U.S. Patent No. 5,807,746; Yao-Zhong Lin & Jack J. Hawiger, *Method for Importing Biologically Active Molecules into Cells*, U.S. Patent No. 6,043,339; Yao-Zhong Lin et al., *Sequence and Method for Genetic Engineering of Proteins with Cell Membrane Translocating Activity*, U.S. Patent No. 6,248,558; Yao-Zhong Lin et al., Sequence and Method for Genetic *Engineering of Proteins with Cell Membrane Translocating Activity*, U.S. Patent No. 6,432,680; Jack J. Hawiger et al., *Method for Importing Biologically Active Molecules into Cells*, U.S. Patent No. 6,495,518; Yao-Zhong Lin et al., *Sequence and Method for Genetic Engineering of Proteins with Cell Membrane Translocating Activity*, U.S. Patent No. 6,780,843; Jonathan B. Rothbard & Paul A Wender, *Method and Composition for Enhancing Transport Across Biological Membranes*, U.S. Patent No. 6,306,993; Jonathan B. Rothbard & Paul A Wender, *Method and Composition for Enhancing Transport Across Biological Membranes*, U.S. Patent No. 6,495,663; and Pamela B. Davis et al., *Fusion Proteins for Protein Delivery*, U.S. Patent No. 6,287,817.

A delivery agent can also be an agent that enables or enhances cellular uptake of a non-covalently associated component, like FGFR2, FGFR3, SV2c, or SNAP-25. Methods that function in the absence of covalent linkage and methods of using such agents are described in, e.g., Gilles Divita et al, *Peptide-Mediated Transfection Agents and Methods of Use*, U.S. Patent No. 6,841,535; Philip L Feigner and Olivier Zelphati, *Intracellular Protein Delivery Compositions and Methods of Use*, U.S. Patent Publication No. 2003/0008813; and Michael Karas, *Intracellular Delivery of Small Molecules*, *Proteins and Nucleic Acids*, U.S. Patent Publication 2004/0209797. Such peptide delivery agents can be prepared and used by standard methods and are commercially available, see, e.g. the CHARIOT™ Reagent (Active Motif, Carlsbad, CA); BIO-PORTER® Reagent (Gene Therapy Systems, Inc., San Diego, CA), BIO TREK™ Protein Delivery Reagent (Stratagene, La Jolla, CA), and PRO-JECT™ Protein Transfection Reagent (Pierce Biotechnology Inc., Rockford, IL).

Aspects of the present disclosure can also be described as follows:
1. An established clonal cell line comprising a cell susceptible to intoxication by BoNT/A.
2. The established clonal cell line of 1, wherein the clonal cell line is selected from a parental SiMa cell line.
3. The established clonal cell line of 1 or 2, wherein the cell is susceptible to BoNT/A intoxication by about 100 pM or less, by about 50 pM or less, by about 10 pM or less, by about 5 pM or less, by about 1 pM or less, by about 0.5 pM or less, or by about 0.1 pM or less of a BoNT/A.
4. The established clonal cell line of 1 or 2, wherein susceptibility to BoNT/A intoxication is measured before differentiation of the cell.
5. The established clonal cell line of 2, wherein in the undifferentiated state, the cell exhibits at least a 1.5-fold increase in gene expression of one or more genes relative to the expression of the one or more genes in a cell from a 2D6 cell line, the genes being taken from Table 5, or wherein the cell exhibits at least a 1.5-fold decrease in gene expression of one or more genes relative to the expression of the one or more genes in a cell from a 2D6 cell line, the genes being taken from Table 6.
6. The established clonal cell line of 2, wherein in the differentiated state, the cell exhibits at least a 1.5-fold increase in gene expression of one or more genes relative to the expression of the one or more genes in a cell from a 2D6 cell line, the genes being taken from Table 9, or wherein the cell exhibits at least a 1.5-fold decrease in gene expression of one or more genes relative to the expression of the one or more genes in a cell from a 2D6 cell line, the genes being taken from Table 10.
7. The established clonal cell line of 2, wherein in the undifferentiated state, the cell exhibits at least a 1.5-fold increase in gene expression of one or more genes relative to the expression of the one or more genes in a cell from a parental SiMa cell line, the genes being taken from Table 7, or wherein the cell exhibits at least a 1.5-fold decrease in gene expression of one or more genes relative to the expression of the one or more genes in a cell from a parental SiMa cell line, the genes being taken from Table 8.
8. The established clonal cell line of 2, wherein in the differentiated state, the cell exhibits at least a 1.5-fold increase in gene expression of one or more genes relative to the expression of the one or more genes in a cell from a parental SiMa cell line, the genes being taken from Table 11, or wherein the cell exhibits at least a 1.5-fold decrease in gene expression of one or more genes relative to the expression of the one or more genes in a cell from a parental SiMa cell line, the genes being taken from Table 12.
9. The established clonal cell line of 1 or 2, wherein the cell exhibits a sensitivity for BoNT/A activity that is 100 pM or less or about 25 pM for about 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, 50 or more cell passage, 55 or more cell passage, or 60 or more cell passage.
10. The established clonal cell line of 1 or 2, wherein the cell from an established clonal cell line exhibits a well defined signal to noise ratio for the upper asymptote for BoNT/A activity of at least 3:1, at least 4:1, at least 5:1, at least 6:1, at least 7:1, at least 8:1, at least 9:1, at least 10:1, over, e.g., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, 50 or more cell passage, 55 or more cell passage, or 60 or more cell passage.
11. An established clonal cell line comprising a cell susceptible to intoxication by BoNT/A produced from a parental SiMa cell line, wherein the cell is susceptible to BoNT/A intoxication by about 100 pM or less, by about 50 pM or less, by about 10 pM or less, by about 5 pM or less, by about 1 pM or less, by about 0.5 pM or less, or by about 0.1 pM or less of a BoNT/A.
12. An established clonal cell line comprising cells susceptible to intoxication by BoNT/A,
   wherein the clonal cell line is selected from a parental SiMa cell line; and
   wherein the the clonal cell line comprises cells susceptible to BoNT/A intoxication by about 100 pM or less of a BoNT/A.
13. The established clonal cell line of 12, wherein the parental SiMa cell line is the parental SiMa cell line DSMZ ACC 164.
14. The established clonal cell line of 12, wherein the cell exhibits a sensitivity for BoNT/A activity that is 100 pM or less or about 25 pM for about 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, 50 or more cell passage, 55 or more cell passage, or 60 or more cell passage.
15. The established clonal cell line of 12, wherein the cell from an established clonal cell line exhibits a well defined signal to noise ratio for the upper asymptote for BoNT/A activity of at least 3:1, at least 4:1, at least 5:1, at least 6:1, at least 7:1, at least 8:1, at least 9:1, at least 10:1, over, e.g., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, 50 or more cell passage, 55 or more cell passage, or 60 or more cell passage.
16. The established clonal cell line of 12, wherein susceptibility to BoNT/A intoxication is measured before differentiation of the cell or after differentiation of the cell.
17. An established clonal cell line comprising cells susceptible to intoxication by BoNT/A,
   wherein the cells exhibits at least a 1.5-fold difference in gene expression levels of three or more genes as compared to the expression levels of these genes in cells from the 2D6 cell line;
   wherein the gene expression levels are selected from th group ADAMTS9, ASCL1, BASP1, DOK5, DPYD, GNB4, GNG11, GTSF1, MAOA, MINA, MSN, PEG3, PLK2, PRSS12, RNF182, SLC44A5, SPARC, TFPI2, and TPTE; and
   wherein the the clonal cell line comprises cells susceptible to BoNT/A intoxication by about 100 pM or less of a BoNT/A.
18. The established clonal cell line of 17, wherein the cells exhibits at least a 2.0-fold difference in gene expression levels of one or more genes as compared to the expression levels of these genes in cells from the 2D6 cell line, at least a 3.0-fold difference in gene expression levels of one or more genes as compared to the expression levels of these genes in cells from the 2D6 cell line, or at least a 4.0-fold difference in gene expression levels of one or more genes as compared to the expression levels of these genes in cells from the 2D6 cell line.
19. The established clonal cell line of 17, wherein the gene expression levels are measured before differentiation of the cell or after differentiation of the cell.
20. The established clonal cell line of 17, wherein the cell exhibits a sensitivity for BoNT/A activity that is 100 pM or less or about 25 pM for about 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, 50 or more cell passage, 55 or more cell passage, or 60 or more cell passage.
21. The established clonal cell line of 17, wherein the cell from an established clonal cell line exhibits a well defined signal to noise ratio for the upper asymptote for BoNT/A activity of at least 3:1, at least 4:1, at least 5:1, at least 6:1, at least 7:1, at least 8:1, at least 9:1, at least 10:1, over, e.g., 5 or more cell passages, 10 or more cell passages, 15 or more cell passages, 20 or more cell passages, 25 or more cell passages, 30 or more cell passages, 35 or more cell passages, 40 or more cell passages, 45 or more cell passages, 50 or more cell passage, 55 or more cell passage, or 60 or more cell passage.

### EXAMPLES

### Example I

### Screening for Cell Lines Comprising Cells Susceptible to BoNT/A Intoxication

The following example illustrates how to identify clonal cells from a parental established cell line that are susceptible to BoNT/A intoxication or have neurotoxin uptake capacity.

### 1. Isolation of clonal cell lines.

Companion patent application Ester Fernandez-Salas, et al., *Immuno-Based Botulinum Toxin Serotype A Activity Assays,* U.S. Patent Application Serial No: 12/403,531 identified several established cell lines useful for conducting the disclosed immuno-based methods of detecting BoNT/A activity, including, e.g., LA1-55n (ECACC 06041203), N18 (ECACC 88112301), Neuro-2a (ATCC CCL-131), PC12 (ATCC CRL-1721), SH-SY5Y (ATCC CRL-2266), and SiMa (DSMZ ACC 164). During characterization of the SiMa cell line, it was discovered that the cells comprising this established cell line comprised at least five different cellular phenotypes. To determine whether any one of these phenotypically-distinct cell types was responsible for the susceptibility of this cell line to BoNT/A intoxication, two different limited-dilution screens were conducted to obtain single colony isolates for each phenotypically-distinct cell type.

In both screens, a suitable density of cells from a SiMa stock were grown in RPMI 1640 medium, having 10% fetal bovine serum, 1% Penicillin-Streptomycin, 2 mM L-Glutamine, contained in a T175 Collagen IV coated flask. After the second passage, the cells were trypsin-treated to produce a cell suspension and the cell concentration was determined. About 4.0 x 10⁶ cells from this cell suspension was transferred into a 50 mL tube and the cells were dissociated into single cells by repeated vigorous expulsion through an 18.5 gauge needle using a 10 mL syringe. Cells from this disassociated single-cell suspension were then diluted to a concentration of 0.2 x 10⁶ cells/mL by adding 15 mL of fresh growth medium, and 2.5 µL of this dilution was added to 50 mL of fresh growth medium to obtain a concentration of 10 cells/mL. From this final dilution stock, 100 µL of growth medium was added to each well of a 96-well Collagen IV coated plates (final average density of one cell/well), and the cells were grown undisturbed in a 37 °C incubator under 5% carbon dioxide for four weeks. A total of nineteen 96-well plates were setup for analysis. Plates were microscopically examined periodically to assess colony growth. After four weeks, each well was microscopically examined to identify growing colonies, and for each growing colony identified, 100 µL of fresh growth medium was added to each well and the cells were grown undisturbed in a 37 °C incubator under 5% carbon dioxide for two weeks. After two additional weeks of growth, the growing single colonies were trypsin-treated and transferred to a new 96-well plate for continued growth. Once colonies grew to about 1,000 cells, based on visual inspection, the cells were trypsin-treated and each cell-suspension was transferred into a new well from a 24-well Collagen IV-coated plate. The cells were grown in a 37 °C incubator under 5% carbon dioxide with fresh growth medium being replenished every 2-3 days, if needed. The cells were grown until the culture reached approximately 60% confluence or greater, at which point the cells were trypsin-treated and each cell-suspension was transferred into a 25 cm² Collagen IV-coated flask. The cells were grown in a 37 °C incubator under 5% carbon dioxide with fresh growth medium being replenished every 2-3 days, if needed. Once the cells in the flask reached 70-80% confluence, they were frozen and stored in liquid nitrogen until the clonal cell lines were tested to determine their susceptibility to BoNT/A intoxication. Of the 1,824 colony isolates initially setup from both screens, 130 clonal cell lines were selected based on viability and growth criteria and expanded for subsequent screening procedures.

### 2. Primary screen for BoNT/A intoxication susceptibility of cells from a clonal cell line using a BoNT/A complex.

One way to determine whether cells from a clonal cell line were susceptible to BoNT/A intoxication was to conduct a primary screen using a CELLTITER-GLO® luminescent cell viability assay (Promega, Madison, WI) for normalizing the cell number in each well and an immuno-based method for determining BoNT/A activity as described in Ester Fernandez-Salas, et al., *Immuno-Based Botulinum Toxin Serotype A Activity Assays,* U.S. Patent Application Serial No: 12/403,531, which is hereby incorporated by reference in its entirety. Two separate 96-well plates were set up, one for the BoNT/A activity assay and the other for the cell viability assay.

To conduct the BoNT/A activity assay, a lysate was prepared from cells treated with BoNT/A by plating cells from each isolated clonal cell line into a well from a poly-D-lysine 96-well plate (BD Biosciences, Bedford, MA). The wells contained 0.1 mL of a serum-free medium comprising Minimum Essential Medium, 2 mM GlutaMAX™ I with Earle's salts, 1 x B27 supplement, 1 x N2 supplement, 0.1 mM Non-Essential Amino Acids, 10 mM HEPES and 25 µg/mL GT1b (302-011-M005, Alexis Biochemicals, San Diego, CA). These cells were incubated in a 37 °C incubator under 5% carbon dioxide until the cells differentiated, as assessed by standard and routine morphological criteria, such as growth arrest and neurite extension (approximately 3 days). The media from the differentiated cells was aspirated from each well and replaced with fresh media containing 1 nM of a BoNT/A complex. After a 24 hrs incubation, the cells were washed by aspirating the growth media and rinsing each well with 200 µL of 1 x PBS. To harvest the cells, 1 x PBS was aspirated, the cells lysed by adding 30 µl of Lysis Buffer comprising 20 mM Tris-HCl (pH 7,5), 150 mM NaCl, 1 mM EDTA, 1 mM EGTA, 1% Triton X-100 to each well, and the plate incubated on a shaker rotating at 500 rpm for 30 minutes at 4 °C. The plate was centrifuged at 4000 rpm for 20 minutes at 4 °C to pellet cellular debris and the supernatant was transferred to a capture antibody coated 96-well plate to perform the detection step.

To prepare an α-SNAP-25 capture antibody solution, the α-SNAP-25 mouse monoclonal antibody contained in the ascites from hybridoma cell line 2E2A6 (Example VII) was purified using a standard Protein A purification protocol.

To prepare an α-SNAP-25 detection antibody solution, α-SNAP-25 rabbit polyclonal antibody S9684 (Sigma, St. Louis, MO) was conjugated to Ruthenium(II)-tris-bipyridine-(4-methysulfonate) NHS ester labeling reagent (Meso Scale Discovery, Gaithersburg, MD) according to the manufacturer's instructions (Meso Scale Discovery, Gaithersburg, MD). The conjugation reaction was performed by adding 30 µL of distilled water reconstituted MSD SULFO-TAG™ stock solution to 200 µL of 2 mg/mL α-SNAP-25 polyclonal antibodies and incubating the reaction at room temperature for 2 hours in the dark. The labeled antibodies were purified using a standard spin column protocol and the protein concentration determined using a standard colorimetric protein assay. The absorbance of the α-SNAP-25 antibody/MSD SULFO-TAG™ conjugate was measured at 455 nm using a spectrophotometer to determine the concentration in moles per liter. The detection antibody solution was stored at 4 °C until needed.

To prepare an α-SNAP-25 solid phase support comprising an α-SNAP-25 capture antibody, approximately 5 µL of the appropriate α-SNAP-25 monoclonal antibody solution (20 µg/mL in 1 x PBS) is added to each well of a 96-well MSD High Bind plate and the solution is allowed to air dry in a biological safety cabinet for 2-3 hours in order to liquid evaporate the solution. The capture antibody-bound wells were sealed and stored at 4 °C until needed.

To detect the presence of a cleaved SNAP-25 product by ECL sandwich ELISA, the wells from an α-SNAP-25 solid phase support were then blocked by adding 150 µL of Blocking Buffer comprising 2% Amersham Blocking Reagent (GE Life Sciences, Piscataway, NJ) and 10% goat serum (VWR, West Chester, PA) at room temperature for 2 hours. The Blocking Buffer was aspirated, 25 µL of a lysate from cells treated with BoNT/A was added to each well and the plates were incubated at 4 °C for overnight. Plate wells were washed three times by aspirating the cell lysate and rinsing each well three times with 200 µL 1 x PBS, 0.1% TWEEN-20® (polyoxyethylene (20) sorbitan monolaureate). After washing, 25 µl of 5 µg/mL α-SNAP-25 detection antibody solution comprising 2% Amersham Blocking Reagent in 1 x PBS, 0.1% TWEEN-20® (polyoxyethylene (20) sorbitan monolaureate) was added to each well, sealed, and incubated at room temperature for 1 hour with shaking. After α-SNAP-25 detection antibody incubation, the wells were washed three times with 200 µL 1 x PBS, 0.1% TWEEN-20® (polyoxyethylene (20) sorbitan monolaureate). After washing 150 µL of 1 x Read Buffer (Meso Scale Discovery, Gaithersburg, MD) was added to each well and the plates were read using a SECTOR™ Imager 6000 Image Reader (Meso Scale Discovery, Gaithersburg, MD). The raw data was collected using the ECL imager.

To conduct the luminescent cell viability assay, cells from each isolated clonal cell line were plated and differentiated as described above. The media from the differentiated cells was aspirated from each well and replaced with 100 µl of the reagent mixture comprising the CELLTITER-GLO® substrate and CELLTITER-GLO® buffer according to the manufacturer's instructions. The plate was then protected from light and mixed for two minutes on an orbital shaker. The solution from the 96-well cell plate with clear bottom was transferred to a white luminometer plate and after 10 minutes incubation was read on the luminometer.

These results indicated that the cell viability readings ranged from 9,838 to 10,423,188 and BoNT/A activity signals ranged from 84 to 27,242 (Table 1). In addition, a ratio of BoNT/A activity over cell viability was calculated to normalize the data, although there was not always a 1:1 ratio of viability and activity. Seven clonal cell lines exhibited activity/viability ratios of about 0.2 x 10⁻² or greater: H1, A7, A11, F10, A9, A10, and C11 (Table 1). Nine clonal cell lines exhibited activity/viability ratios of about 0.125 x 10⁻² to about 0.150 x 10⁻²: H3, H8, E11, H10, A6, C5, C12, D11, and B5 (Table 1). Two cell lines exhibited activity/viability ratios of about 0.100 x 10⁻²: D7 and B10 (Table 1). Based on these activity/viability ratios in conjunction with the cell viability and BoNT/A activity signal measurements, these 18 clonal cell lines were selected for further testing by conducting a secondary screen using a BoNT/A activity assay in order to generate a dose-response curve to BoNT/A.

| **Table 1. Primary Screen for Clonal Cell Lines Susceptible to BoNT/A Intoxication Using Viability and Activity Signals** | | | | | | | |
|---|---|---|---|---|---|---|---|
| **Well** | **Cell Viability Reading (RLU)** | **BoNT/A Activity Signal (RLU)** | **Activity/Viabi lity Ratio** | **Well** | **Cell Viability Reading (RLU)** | **BoNT/A Activity Signal (RLU)** | **Activity/Viabil ity Ratio** |
| A1 | 2634417 | 2425 | 0.092 x 10⁻² | A7 | 10132753 | 27242 | 0.269 x 10⁻² |
| B1 | 731808 | 198 | 0.027 x 10⁻² | B7 | 2977371 | 1642 | 0.055 x 10⁻² |
| C1 | 4258160 | 1641 | 0.039 x 10⁻² | C7 | 5746577 | 4878 | 0.085 x 10⁻² |
| D1 | 6554606 | 4885 | 0.075 x 10⁻² | D7 | 5861338 | 6044 | 0.103 x 10⁻² |
| E1 | 2374483 | 897 | 0.038 x 10⁻² | E7 | 2819910 | 1399 | 0.050 x 10⁻² |
| F1 | 2801444 | 1842 | 0.066 x 10⁻² | F7 | 23523 | 84 | 0.357 x 10⁻² |
| G1 | 5094510 | 4579 | 0.090 x 10⁻² | G7 | 6232344 | 4031 | 0.065 x 10⁻² |
| H1 | 3808699 | 10861 | 0.285 x 10⁻² | H7 | 4916756 | 2759 | 0.056 x 10⁻² |
| A2 | 9593106 | 7090 | 0.074 x 10⁻² | A8 | 5945833 | 5611 | 0.094 x 10⁻² |
| B2 | 5374966 | 5673 | 0.106 x 10⁻² | B8 | 4591002 | 2128 | 0.046 x 10⁻² |
| C2 | 4170158 | 2972 | 0.071 x 10⁻² | C8 | 5648241 | 8758 | 0.155 x 10⁻² |
| D2 | 31283 | 88 | 0.281 x 10⁻² | D8 | 4207887 | 3360 | 0.080 x 10⁻² |
| E2 | 5706902 | 3743 | 0.066 x 10⁻² | E8 | 5894699 | 3844 | 0.065 x 10⁻² |
| F2 | 4884798 | 4568 | 0.094 x 10⁻² | F8 | 5585487 | 4651 | 0.083 x 10⁻² |
| G2 | 3552045 | 2190 | 0.062 x 10⁻² | G8 | 4758800 | 4545 | 0.096 x 10⁻² |
| H2 | 3743690 | 3421 | 0.091 x 10⁻² | H8 | 5439137 | 8017 | 0.147 x 10⁻² |
| A3 | 15372 | 89 | 0.579 x 10⁻² | A9 | 10423188 | 20706 | 0.199 x 10⁻² |
| B3 | 5381672 | 1551 | 0.029 x 10⁻² | B9 | 6493893 | 4444 | 0.068 x 10⁻² |
| C3 | 4270534 | 3959 | 0.093 x 10⁻² | C9 | 5742047 | 5788 | 0.101 x 10⁻² |
| D3 | 48702 | 97 | 0.199 x 10⁻² | D9 | 3424405 | 1848 | 0.054 x 10⁻² |
| E3 | 4707349 | 2671 | 0.057 x 10⁻² | E9 | 5771943 | 8064 | 0.140 x 10⁻² |
| F3 | 3053132 | 1539 | 0.050 x 10⁻² | F9 | 24524 | 88 | 0.359 x 10⁻² |
| G3 | 5518292 | 4195 | 0.076 x 10⁻² | G9 | 7291510 | 6674 | 0.092 x 10⁻² |
| H3 | 4156632 | 6339 | 0.153 x 10⁻² | H9 | 4169883 | 6048 | 0.145 x 10⁻² |
| A4 | 19793 | 89 | 0.450 x 10⁻² | A10 | 9050811 | 17617 | 0.195 x 10⁻² |
| B4 | 6224766 | 4299 | 0.069 x 10⁻² | B10 | 5590113 | 5175 | 0.093 x 10⁻² |
| C4 | 3212859 | 3202 | 0.100 x 10⁻² | C10 | 2325807 | 1713 | 0.074 x 10⁻² |
| D4 | 4376180 | 2367 | 0.054 x 10⁻² | D10 | 2644116 | 2589 | 0.098 x 10⁻² |
| E4 | 3931793 | 3975 | 0.101 x 10⁻² | E10 | 4210945 | 2666 | 0.063 x 10⁻² |
| F4 | 1150134 | 526 | 0.046 x 10⁻² | F10 | 2932749 | 6390 | 0.218 x 10⁻² |
| G4 | 19023 | 94 | 0.494 x 10⁻² | G10 | 3980127 | 3114 | 0.078 x 10⁻² |
| H4 | 3368425 | 2645 | 0.079 x 10⁻² | H10 | 4519035 | 6187 | 0.137 x 10⁻² |
| A5 | 8231689 | 7809 | 0.095 x 10⁻² | A11 | 9870025 | 24690 | 0.250 x 10⁻² |
| B5 | 5332004 | 6545 | 0.123 x 10⁻² | B11 | 4973892 | 2235 | 0.045 x 10⁻² |
| C5 | 3869441 | 5042 | 0.130 x 10⁻² | C11 | 3326448 | 6405 | 0.193 x 10⁻² |
| D5 | 2554300 | 1161 | 0.045 x 10⁻² | D11 | 5747961 | 7169 | 0.125 x 10⁻² |
| E5 | 3781403 | 764 | 0.020 x 10⁻² | E11 | 5831546 | 8432 | 0.145 x 10⁻² |
| F5 | 4608801 | 3720 | 0.081 x 10⁻² | F11 | 5291803 | 4317 | 0.082 x 10⁻² |
| G5 | 7105403 | 7330 | 0.103 x 10⁻² | G11 | 3499587 | 2790 | 0.080 x 10⁻² |
| H5 | 5585607 | 7544 | 0.135 x 10⁻² | H11 | 3225750 | 4518 | 0.140 x 10⁻² |
| A6 | 7698708 | 10152 | 0.132 x 10⁻² | A12 | 9838 | 89 | 0.905 x 10⁻² |
| B6 | 4706560 | 3196 | 0.068 x 10⁻² | B12 | 3728293 | 2016 | 0.054 x 10⁻² |
| C6 | 5866251 | 6145 | 0.105 x 10⁻² | C12 | 4086862 | 5308 | 0.130 x 10⁻² |
| D6 | 2285787 | 768 | 0.034 x 10⁻² | D12 | 5044526 | 3860 | 0.077 x 10⁻² |
| E6 | 2471979 | 1038 | 0.042 x 10⁻² | E12 | 4787913 | 2719 | 0.057 x 10⁻² |
| F6 | 10958 | 91 | 0.830 x 10⁻² | F12 | 5101695 | 2585 | 0.051 x 10⁻² |
| G6 | 19590 | 91 | 0.465 x 10⁻² | G12 | 2887390 | 1622 | 0.056 x 10⁻² |
| H6 | 2057687 | 2564 | 0.125 x 10⁻² | H12 | 2829307 | 5105 | 0.180 x 10⁻² |

To conduct BoNT/A activity assay in order to generate a dose-response curve to BoNT/A, cells from each isolated clonal cell line were seeded in a poly-D-lysine 96-well plate and differentiated as described above. Of the top 18 clonal cell lines selected for further testing, only 8 remained viable, the remaining 10 clonal cell lines failed to regrow and were lost. The media from the differentiated cells was aspirated from each well and replaced with fresh media containing either 0 (untreated sample), 0.03 pM, 0.1 pM, 0.3 pM, 0.9 pM, 2.8 pM, 8.3 pM, and 25 pM of a BoNT/A complex. After a 24 hrs treatment, the cells were washed, incubated for an additional two days in serum-free medium without toxin to allow for the cleavage of the SNAP-25 substrate, and harvested as described above. The supernatant was transferred to a capture antibody coated 96-well plate to perform the detection step.

The α-SNAP-25 capture antibody solution, the α-SNAP-25 detection antibody solution, and the α-SNAP-25 solid phase support were prepared as described above. Detection of the presence of cleaved SNAP-25 product by ECL sandwich ELISA analysis was performed as described above. The raw data obtained from the ECL imager was then transferred to SigmaPlot v. 9.0 and a 4-parameter logistics fit was used to define the dose-response curves. There were no constraints used for the 4-parameter logistic function when plotting the data. Graphical reports were generated using the following analysis: R2 (correlation coefficient), a (Max for data set), b (hillslope), and X0 ± SE (EC₅₀ value ± standard error).

These results indicate that of the 8 clonal cell lines tested, only two were more susceptible to BoNT/A intoxication when compared to cells comprising the parental SiMa cell line (Table 2). For example, cells comprising the parental SiMa cell line exhibited an EC₅₀ activity for BoNT/A of 2.20 pM, whereas cells comprising the H1 and A10 cell lines exhibited an EC₅₀ activity for BoNT/A of 1.76 pM or less. Conversely, cells comprising the H10 and D11 cell line exhibited an EC₅₀ activity for BoNT/A that was higher than that observed for cells comprising the parental SiMa cell fine, but less than 5.0 pM. It should be noted that any clonal cell line exhibiting an EC₅₀ activity for BoNT/A that is 5.0 pM or less (100 U/mL or less) is useful for an immuno-based method for determining BoNT/A activity described in Ester Fernandez-Salas, et al., *Immuno-Based Botulinum Toxin Serotype A Activity Assays,* U.S. Patent Application Serial No: 12/403,531.

| **Table 2. Secondary Screen for Clonal Cell Lines Susceptible to BoNT/A Intoxication** | | | |
|---|---|---|---|
| **Clone** | **BoNT/A Activity** | | |
| | **EC₅₀ (pM)** | **Signal_{MIN} (RLU)** | **Signal_{MAX} (RLU)** |
| SiMa | 2.20 | 123 | 47,611 |
| H1 | 0.77 | 193 | 125,252 |
| H3 | ND | ND | ND |
| B5 | >25.0 | 115 | 22,277 |
| C5 | ND | ND | ND |
| A6 | ND | ND | ND |
| A7 | 5.59 | 153 | 100,412 |
| D7 | ND | ND | ND |
| H8 | ND | ND | ND |
| A9 | ND | ND | ND |
| A10 | 1.76 | 207 | 113,029 |
| B10 | 6.35 | 147 | 101,935 |
| F10 | >25.0 | 111 | 32.663 |
| H10 | 2.80 | 171 | 120,975 |
| A11 | ND | ND | ND |
| C11 | ND | ND | ND |
| D11 | 4.4 | 1.84 | 105,527 |
| E11 | ND | ND | ND |
| C12 | ND | ND | ND |
| ND = Not determined. | | | |

### 3. Primary screen for BoNT/A intoxication susceptibility of cells from a clonal cell line using a formulated BoNT/A pharmaceutical product

Another way to determine whether cells from a clonal cell line were susceptibility to BoNT/A intoxication was to conduct a primary screen by generating a dose-response curve to BoNT/A and calculate the EC₅₀ value using an immuno-based method for determining BoNT/A activity as described in Ester Fernandez-Salas, et al., *Immuno-Based Botulinum Toxin Serotype A Activity Assays,* U.S. Patent Application Serial No: 12/403,531.

Initially six of 35 clonal cell line isolates were examined using a full dose response to BoNT/A. To prepare a lysate from cells treated with BoNT/A, cells from six clonal cell line were seeded in a poly-D-lysine 96-well plate and differentiated as described above in Section 2 except that the serum-free medium comprised 60 µg/mL GT1b. The clonal cell lines used were 3D8, YB8, 1D4, 2D6, 1E11, 2F5. The H1 and H10 clonal cell lines identified above, along with the parental SiMa cell line, were used as references to identify cell lines highly susceptible to BoNT/A intoxication. The media from the differentiated cells was aspirated from each well and replaced with fresh media containing either 0 (untreated sample), 0.98 U/mL, 1.9 U/mL, 3.91 U/mL, 7.81 U/mL, 15.6 U/mL, 31.3 U/mL, 62.5 U/mL, 125 U/mL, 250 U/mL, or 500 U/mL of a formulated BoNT/A pharmaceutical product. After a 24 hrs treatment, the cells were washed, incubated for an additional two days in serum-free medium without toxin to allow for the cleavage of the SNAP-25 substrate. To harvest the cells, 1 x PBS was aspirated, the cells lysed by adding 30 µl of Lysis Buffer comprising 20 mM Tris-HCl (pH 7,5), 150 mM NaCl, 1 mM EDTA, 1 mM EGTA, 1% Triton X-100 to each well, and the plate incubated on a shaker rotating at 500 rpm for 30 minutes at 4 °C. The plate was centrifuged at 4000 rpm for 20 minutes at 4 °C to pellet cellular debris. The protein concentration was determined using standard methods. The supernatant was transferred to a capture antibody coated 96-well plate to perform the detection step of the immuno-based method.

The α-SNAP-25 capture antibody solution, the α-SNAP-25 detection antibody solution, and the α-SNAP-25 solid phase support were prepared as described above in Section 2. Detection of the presence of SNAP-25 cleavage product by ECL sandwich ELISA analysis was performed, collected data was analyzed and the EC₅₀ calculated as described above in Section 2, except that SigmaPlot v. 10 was used.

These results show that clonal cell line H1 comprises cells that are more susceptible to BoNT/A than the parental SIMa cell line (Table 3). In addition, results indicate that although clonal cell lines H10, 1D4, 1E11, 2D6, 2F5, 3D8, and YB8 all comprise cells that are less susceptible to BoNT/A than the parental SiMa cell line, all have an EC₅₀ of BoNT/A activity that is below 30 U/mL (Table 3). It should be noted that any clonal cell line exhibiting an EC₅₀ activity for BoNT/A that is 100 U/mL or less (5.0 pM or less) is useful for an immuno-based method for determining BoNT/A activity described in Ester Fernandez-Salas, et al., *Immuno-Based Botulinum Toxin Serotype A Activity Assays,* U.S. Patent Application Serial No: 12/403,531.

| **Table 3. Primary Screen for Clonal Cell Lines Susceptible to BoNT/A Intoxication** | | | |
|---|---|---|---|
| **Clone** | **BoNT/A Activity** | | |
| | **EC₅₀ (U/mL)** | **Signal_{MIN} (RLU)** | **Signal_{MAX} (RLU)** |
| SiMa | 10.8 ± 1.0 | 10,519 | 253,484 |
| H1 | 10.2 ± 0.6 | 13,996 | 276,982 |
| H10 | 22.2 ± 2.0 | 5,379 | 296,168 |
| 1D4 | 22.4 ± 0.8 | 6,064 | 239,096 |
| 1E11 | 14.2 ± 1.7 | 8,698 | 276,323 |
| 2D6 | 27.3 ± 1.8 | 3,347 | 179,454 |
| 2F5 | 15.8 ± 0.9 | 7,007 | 265,461 |
| 3D8 | 13.4 ± 0.6 | 11,658 | 266,279 |
| YB8 | 19.7 ± 1.3 | 8,477 | 293,559 |

This initial primary screen using a dose-response curve with the 10 different BoNT/A concentrations identified three concentrations that were useful for a shortened assay using 3 different BoNT/A concentrations, with one concentration being slightly higher than the lower asymptote (8 U/mL), one concentration near the EC₅₀ (25 U/mL), and one concentration representing about 80% of the upper asymptote (80 U/mL). In these subsequent primary screens the remaining 29 clonal cell line isolates were tested as described above except that the cells were treated with either 0 (untreated sample), 8 U/mL, 25 U/mL, or 80 U/mL of a formulated BoNT/A pharmaceutical product. The clonal cell lines tested were 1E3, 2B9, 2D2, 2E4, 3B8, 3D5, 3G10, 4B5, 4C8, 4D3, 5C10, 5F3, AC9, AF4, BB3, BB10, BE3, BF8, CC11, CD6, CE6, CG8, CG10, DC4, DD10, DE7, DF5, YB7, and YF5. In addition, the parental SiMa was used as positive control.

These results show that clonal cell lines 2E4, 3B8, 3D5, 5F3, AF4, BB3, BB10, and DC4 all comprise cells that are more susceptible to BoNT/A than the parental SiMa cell line (Table 4). In addition, these results indicate that clonal cell lines 1E3, 2B9, 3G10, 4B5, 4C8, AC9, BE3, BF8, CC11, CD6, CE6, CG8, CG10, DD10, DE7, and YF5 all comprise cells that are of similar susceptibility to BoNT/A to that of the parental SiMa cell line (Table 4). Lastly, these results reveal that clonal cell lines 2D2, 4D3, 5C10, DF5, and YB7 all comprise cells that are less susceptible to BoNT/A than the parental SiMa cell line (Table 4).

| **Table 4. Primary Screen for Clonal Cell Lines Susceptible to BoNT/A Intoxication** | | | | | | |
|---|---|---|---|---|---|---|
| **Clone** | **8 U/mL BoNT/A** | | **25 U/mL BoNT/A** | | **80 U/mL BoNT/A** | |
| | **Activity Signal (RLU)** | **Fold Difference** | **Activity Signal (RLU)** | **Fold Difference** | **Activity Signal (RLU)** | **Fold Difference** |
| SiMa | 75811/ 80562* | - | 138952/ 175685* | - | 173979/ 225768* | - |
| 1E3 | 71040 | 0.9 | 128771 | 0.9 | 155107 | 0.9 |
| 2B9 | 66962 | 0.9 | 135006 | 1.0 | 166871 | 1.0 |
| 2D2 | 60224* | 0.8 | 136947* | 0.8 | 199786* | 0.9 |
| 2E4 | 103680 | 1.4 | 217980 | 1.6 | 270463 | 1.6 |
| 3B8 | 123521 | 1.6 | 227367 | 1.6 | 249218 | 1.4 |
| 3D5 | 113532 | 1.5 | 201683 | 1.5 | 270578 | 1.6 |
| 3G10 | 83049 | 1.1 | 130930 | 0.9 | 175540 | 1.0 |
| 4B5 | 74344 | 1.0 | 137604 | 1.0 | 177363 | 1.0 |
| 4C8 | 87047 | 1.1 | 144395 | 1.0 | 185115 | 1.1 |
| 4D3 | 38063 | 0.5 | 80180 | 0.6 | 101724 | 0.6 |
| 5C10 | 16024* | 0.2 | 49030 | 0.3 | 116396* | 0.6 |
| 5F3 | 98263* | 1.7 | 182902* | 1.4 | 212275* | 1.5 |
| AC9 | 60016* | 1.0 | 127030* | 1.0 | 180384* | 1.4 |
| AF4 | 115460 | 1.5 | 179901 | 1.3 | 190867 | 1.1 |
| BB3 | 74668 | 1.3 | 120966 | 1.4 | 170177 | 1.4 |
| BB10 | 152748 | 2.0 | 232406 | 1.7 | 266555 | 1.5 |
| BE3 | 100849 | 1.0 | 199487 | 0.9 | 236415 | 1.0 |
| BF8 | 55763* | 1.0 | 111746* | 1.0 | 153074* | 1.3 |
| CC11 | 54421* | 1.0 | 123576* | 1.0 | 155337* | 1.2 |
| CD6 | 74970 | 1.0 | 151862 | 1.1 | 183377 | 1.1 |
| CE6 | 70019 | 0.9 | 146292 | 1.1 | 208184 | 1.2 |
| CG8 | 81790* | 1.0 | 159098* | 1.0 | 189209* | 1.3 |
| CG10 | 69503 | 0.9 | 141079 | 1.0 | 185801 | 1.1 |
| DC4 | 112085 | 1.5 | 163319 | 1.2 | 169352 | 1.0 |
| DD10 | 116325 | 1.5 | 205445 | 1.5 | 223473 | 1.3 |
| DE7 | 60994* | 0.9 | 131237* | 0.9 | 174487* | 0.9 |
| DF5 | 34,165* | 0.5 | 104,696* | 0.7 | 179,314* | 0.9 |
| YB7 | 50433 | 0.7 | 103707 | 0.7 | 143423 | 0.8 |
| YF5 | 65848 | 0.9 | 122487 | 0.9 | 146263 | 0.4 |
| * Two separate screens were done. In the first, the signal detected for the SiMa cell line was 75811 RLU and this value was used to determine the fold difference for clonal cell lines tested in this screen (numbers with no asterisk). For the second screen, the signal detected for the SiMa cell line was 80562 RLU and this value was used to determine the fold difference for clonal cell lines tested in this screen (numbers with asterisk). | | | | | | |

### Example II

### Stability Analysis of Clonal Cell Lines

The following example illustrates how to characterize the stability of cells from an established clonal cell line that are susceptible to BoNT/A intoxication or have neurotoxin uptake capacity.

During characterization of the SiMa cell line, it was determined that the parental SiMa cell line became unstable in terms of a significant loss of sensitivity to BoNT/A. For example, prior to passage 13, cells comprising the parental SiMa cell line routinely exhibited an EC₅₀ for BoNT/A activity that was under 5.0 pM. However, after passages about 14 to about 20 passages, cells comprising the parental SiMa cell exhibited an EC₅₀ for BoNT/A activity that was routinely over 25.0 pM. One reason for this dramatic reduction in the EC₅₀ value may be because the SiMa cell line is heterogeneous in nature since it comprises at least five different cell types. Over time, one of these cell types could overtake the culture to the exclusion of the other four. If the overtaking cell type was less susceptible to BoNT/A intoxication, or not susceptible at all, then as this cell type became the dominant one in the cell line the EC₅₀ for BoNT/A activity in this overtaken SiMa cell line would decrease. Thus, because the established clonal cell lines were derived from only one of these cell types, then it follows that the clonal cell line would exhibit greater stability in terms of maintaining an EC₅₀ for BoNT/A activity that was routinely under 5.0 pM.

To determine whether the cells from the established clonal cell lines showed increased stability relative to the cells comprising the parental SIMa cell line, the EC₅₀ for BoNT/A activity was determined for clonal cell lines BB10, H1, H10, and the parental SiMa cell line at different passage numbers using an immuno-based method for determining BoNT/A activity. The media from the differentiated cells was aspirated from each well and replaced with fresh media containing either 0 (untreated sample), 0.98 U/mL, 1.9 U/mL, 3.91 U/mL, 7.81 U/mL, 15.6 U/mL, 31.3 U/mL, 62.5 U/mL, 125 U/mL, 250 U/mL, or 500 U/mL of a formulated BoNT/A pharmaceutical product. After a 24 hrs treatment, the cells were washed, incubated for an additional two days in serum-free medium without toxin to allow for the cleavage of the SNAP-25 substrate, and harvested as described above. The supernatant was transferred to a capture antibody coated 96-well plate to perform the detection step.

Similarly, the preparation of the α-SNAP-25 capture antibody solution, α-SNAP-25 detection antibody solution, and the solid phase support comprising an α-SNAP-25 capture antibody were as described in Section 3. Lastly, detection of the presence of SNAP-25 cleavage product by ECL sandwich ELISA was performed, collected data was analyzed, and the EC₅₀ calculated as described above in Section 2, except that PLA v. 2 was used for the 4-parameter logistics fit.

The results from this stability experiments indicated that BB10, H1 and H10 cell lines all exhibited increase stability as compared to the parental SiMa cell line. For example, where cells from the parental SiMa cell line exhibited an EC₅₀ for BoNT/A activity that was over 500 U/mL (or 25 pM) after passages about 14 to about 20 passages, cells comprising the BB10, H1 and H10 cell lines all exhibited an EC₅₀ for BoNT/A activity that was below 10 U/mL (or 0.5 pM) after passage about 30.

### Example III

### Gene Expression Profile of Cell Lines Comprising Cells Susceptible to BoNT/A Intoxication

The following example illustrates how to characterize cells from an established clonal cell line that are susceptible to BoNT/A intoxication or have neurotoxin uptake capacity by gene expression profiling.

To determine the changes in genome-wide gene transcription levels in cells susceptible to BoNT/A intoxication, gene expression analysis was performed on cells from H1, BB10, 2D6 and the parental SiMa cell line, in both differentiated and undifferentiated states using GENECHIP® U133 Plus 2 microarray technology (Affymetrix, Inc., Santa Clara, CA) according to the manufacturer's instructions, see, *e.g.,* GENECHIP® Expression Analysis Technical Manual, 702232, Rev. 2 (2006) which is hereby incorporated by reference in its entirety.

To prepare differentiated cells for these cell lines, a suitable density of cells from the H1, BB10, 2D6, and SiMa cell lines were seeded in separate T175 tissue culture flasks containing 50 mL of a serum-free medium comprising Enhanced Minimum Essential Medium, 10 mM HEPES, 1 mM sodium pyruvate, 0.1 mM Non-Essential Amino Acids, 1 x B27 supplement, 1 x N2 supplement, and 60 µg/mL GT1b (302-011-M005, Alexis Biochemicals, San Diego, CA). These cells were incubated in a 37 °C incubator under 5% carbon dioxide until the cells differentiated, as assessed by standard and routine morphological criteria, such as growth arrest and neurite extension, and attained 80% confluence (approximately 3 days). The cells were then washed with 50 mL of 1 x PBS, trypsin-treated for 5 min at room temperature, and then 15 mL of growth medium was added to the flask to inactivate the trypsin. The cell suspension was transferred to a 50 mL tube and centrifuge at 200 RCF for 5 min at room temperature to pellet the cells. The cell pellet was washed once in 50 mL of 1 x PBS, centrifuge at 200 RCF for 5 min at room temperature to pellet the cells, and the supernatant was removed. The cell pellet was quick frozen on dry ice and stored at -80 °C until needed.

To prepare undifferentiated cells for these cell lines, a suitable density of cells from the H1, BB10, 2D6, and SiMa cell lines were seeded in separate T175 tissue culture flasks containing 50 mL of growth medium comprising RPMI 1640, 10% fetal bovine serum, 10 mM HEPES, 1 mM sodium pyruvate, 0.1 mM Non-Essential Amino Acids, and 60 µg/mL GT1b. These cells were incubated in a 37 °C incubator under 5% carbon dioxide until the cells attained 80% confluence (approximately 3 days). The cells were then processed as described above and the cell pellet was quick frozen on dry ice and stored at -80 °C until needed.

To prepare total RNA, cells for each cell line were lysed and total RNA isolated and purified using a RNA isolation kit (QIAGEN, Valencia, CA) according to the manufacturer's protocols. DNA was digested using an on-column DNase I procedure (QIAGEN, Valencia, CA). RNA was quantified photospectrometrically at 260 nm and purity assessed by the A260A280 ratio using a spectrophotometer. RNA integrity was assessed using a Bioanalyser 2100 (Agilent Technologies, Inc., Santa Clara, CA). Extracted total RNA aliquots were snap-frozen in liquid nitrogen and stored at -80 °C.

To prepare cDNA probes for the gene microarray chips, about 1-15 µg was reversed transcribed using a T7-Oligo(dT) promoter primer in the first strand cDNA synthesis reaction. Following RNase H-mediated second strand cDNA synthesis, the double-stranded cDNA was purified and served as a template for subsequent in vitro transcription reaction. The in vitro transcription reaction was carried out in the presence of T7 RNA polymerase and a biotinylated nucleotide analog/ribonucleotide mix for complementary RNA (cRNA) amplification and biotin labeling. The biotinylated cRNA targets were then cleaned up, fragmented, and hybridized to microchip expression arrays for 17 hours at 65 °C according to the manufacturer's instructions, see, *e.g.,* GENECHIP® Expression Analysis Technical Manual, 702232, Rev. 2 (2006) which is hereby incorporated by reference in its entirety. To identify substantial treatment-related up- or down-regulated gene expression differences compared to control cell lines, greater than 1.5-fold differences (log₂ 0.58) were considered significant, and only those genes that displayed consistent changes in expression in the triplicate analysis were considered (Tables 5-12).

Expression profiling for cell lines H1, BB10 and 2D6 analyzed in the undifferentiated state revealed that 1,323 genes were identified as having their expression levels increase by 1.5-fold (log₂ 0.58) or more in the H1 and BB10 cell lines as compared to the gene expression levels obtained from the 2D6 cell line (Table 5). Of these, 686 genes exhibited an increase in expression levels in the H1 and BB10 cell lines by 2-fold (log₂ 1.0) or more as compared to the expression level for these genes determined from the 2D6 cell line; and 231 genes exhibited an increase in expression levels in the H1 and BB10 cell lines by 4-fold (log₂ 2.0) or more as compared to the expression level for these genes determined from the 2D6 cell line. With respect to decreased expression, 1,438 genes were identified as having their expression levels decreased by 1.5-fold (log₂ 0.58) or more in the H1 and BB10 cell lines as compared to the gene expression levels obtained from the 2D6 cell line (Table 6). Of these, 668 genes exhibited a decrease in expression levels in the H1 and BB10 cell lines by 2-fold (log₂ 1.0) or more as compared to the expression level for these genes determined from the 2D6 cell line; and 107 genes exhibited a decrease in expression levels in the H1 and BB10 cell lines by 4-fold (log₂ 2.0) or more as compared to the expression level for these genes determined from the 2D6 cell line.

Expression profiling for cell lines H1, BB10 and 2D6 analyzed in the differentiated state revealed that 2,556 genes were identified as having their expression levels increased by 1.5-fold (log₂ 0.58) or more in the H1 and BB10 cell lines as compared to the gene expression levels obtained from the 2D6 cell line (Table 7). Of these, 1,634 genes exhibited an increase in expression levels in the H1 and BB10 cell lines by 2-fold (log₂ 1.0) or more as compared to the expression level for these genes determined from the 2D6 cell line; and 696 genes exhibited an increase in expression levels in the H1 and BB10 cell lines by 4-fold (log₂ 2.0) or more as compared to the expression level for these genes determined from the 2D6 cell line. With respect to decreased expression, 2,087 genes were identified as having their expression levels decreased by 1.5-fold (log₂ 0.58) or more in the H1 and BB10 cell lines as compared to the gene expression levels obtained from the 2D6 cell line (Table 8). Of these, 1,215 genes exhibited a decrease in expression levels in the H1 and BB10 cell lines by 2-fold (log₂ 1.0) or more as compared to the expression level for these genes determined from the 2D6 cell line; and 280 genes exhibited a decrease in expression levels in the H1 and BB10 cell lines by 4-fold (log₂ 2.0) or more as compared to the expression level for these genes determined from the 2D6 cell line.

Expression profiling for cell lines H1, BB10 and SiMa analyzed in the undifferentiated state revealed that 1,232 genes were identified as having their expression levels increase by 1.5-fold (log₂ 0.58) or more in the H1 and BB10 cell lines as compared to the gene expression levels obtained from the SiMa parental cell line (Table 9). Of these, 764 genes exhibited an increase in expression levels in the H1 and BB10 cell lines by 2-fold (log₂ 1.0) or more as compared to the expression level for these genes determined from the SiMa parental cell line; and 228 genes exhibited an increase in expression levels in H1 and BB10 cell lines by 4-fold (log₂ 2.0) or more as compared to the expression level for these genes determined from the SiMa parental cell line. With respect to decreased expression, 1,265 genes were identified as having their expression levels decreased by 1.5-fold (log₂ 0.58) or more in the H1 and BB10 cell lines as compared to the gene expression levels obtained from the SiMa parental cell line (Table 10). Of these, 648 genes exhibited a decrease in expression levels in the H1 and BB10 cell lines by 2-fold (log₂ 1.0) or more as compared to the expression level for these genes determined from the SiMa parental cell line; and 189 genes exhibited a decrease in expression levels in H1 and BB10 cell lines by 4-fold (log₂ 2.0) or more as compared to the expression level for these genes determined from the SiMa parental cell line.

Expression profiling for cell lines H1, BB10 and SiMa analyzed in the differentiated state revealed that 756 genes were identified as having their expression levels increase by 1.5-fold (log₂ 0.58) or more in the H1 and BB10 cell lines as compared to the gene expression levels obtained from the SiMa parental cell line (Table 11). Of these, 472 genes exhibited an increase in expression levels in the H1 and BB10 cell lines by 2-fold (log₂ 1.0) or more as compared to the expression level for these genes determined from the SiMa parental cell line; and 150 genes exhibited an increase in expression levels in H1 and BB10 cell lines by 4-fold (log₂ 2.0) or more as compared to the expression level for these genes determined from the SiMa parental cell line. With respect to decreased expression, 912 genes were identified as having their expression levels decreased by 1.5-fold (log₂ 0.58) or more in the H1 and BB10 cell lines as compared to the gene expression levels obtained from the SiMa parental cell line (Table 12). Of these, 411 genes exhibited a decrease in expression levels in the H1 and BB10 cell lines by 2-fold (log₂ 1.0) or more as compared to the expression level for these genes determined from the SiMa parental cell line; and 108 genes exhibited a decrease in expression levels in H1 and BB10 cell lines by 4-fold (log₂ 2.0) or more as compared to the expression level for these genes determined from the SiMa parental cell line.

### Example IV

### Pathway Analysis of Gene Expression Profiles from Clonal Cell Lines

The following example illustrates how to characterize gene expression profiles from clonal cells from an established clonal cell line that are susceptible to BoNT/A intoxication or have neurotoxin uptake capacity in order to identify biological networks or pathways associated with BoNT/A intoxication.

An Ingenuity Pathway Analysis (IPA) Core Analysis (Ingenuity Systems Inc., Redwood City, CA) was performed to characterize the RNA expression phenotype of two independently derived BoNT/A very sensitive single clone cell lines, BB10 and H1, compared to a related but much less BoNT/A sensitive single clone cell line, 2D6. The analysis was done to identify the specific gene expression phenotype of a BoNT/A sensitive cell line. The result can also be used to predict and potentially identify proteins that function to enhance BoNT/A uptake.

An Ingenuity Pathway Analysis (IPA) Core Analysis was performed that identified biologically relevant networks to BoNT/A intoxication based on connectivity among the genes from an IPA library and the genes exhibiting significant changes as determined from the expression profiling discussed above. For this analysis, all genes that exhibited an increased or decrease in expression levels in the H1 and BB10 cell lines by 4-fold (log₂ 2.0) or more as compared to the expression level for these genes determined from the 2D6 cell line were used, in conjunction with, selected genes with known or suspected relevance to BoNT/A intoxication, hereafter referred to as the Profile genes. The significance of the association to a given network was measured and ranked by the ratio of the number of Profile genes that mapped to the specific network divided by the total number of genes that map to that network. Fisher's exact test was used to calculate p-scores, which represent the association between the Profile genes and the network, and based on the p-scores the genes from the Profile genes were used to rank the networks. The p-scores were derived from p-values. If there are *n* genes in the network and *f* of them are Profile genes, then the p-value is the probability of finding *f* or more Profile genes in a set of *n* genes randomly selected from the Global Molecular Network. Since significant p-values are quite low (*e.g.,* 1 x 10⁻⁸), a p-score was defined as the component of the p-value; p-score = -log10 (p-value). The identified networks were overlaid with differentially expressed genes which had more than 1.5-fold differential expression and a p-value ≤ 0.001 either before or after differentiation.

Using IPA Core Analysis the differentially expressed Profile genes were connected to 26 Networks. Nineteen of these included 10 or more Profile genes (Table 13). Among these nineteen networks three overall networks groups, named A, B and C were identified (Table 13). Within the networks in these three groups there were overlapping genes and the expression for more than three genes was increased after differentiation. Group A genes include the FGFR2 receptor which has been shown to function in the BoNT/A intoxication process as a receptor for BoNT/A and are as follows: genes exhibiting a 1.5-fold or more decrease in expression levels in the H1 and BB10 cell lines as compared to their expression in cells from the 2D6 cell line, ANXA2, AQP1, ARHGAP9, CDH10, CDKN2A, CHPT1, CNTN2, ERAP1, and RGS11; genes exhibiting a 1.5-fold or more increase in expression levels in the H1 and BB10 cell lines as compared to their expression in cells from the 2D6 cell line, ADAMTS9, ATAD2, C11ORF82, CDC45L, CNTN1, CNTN4, Cyclin A, Cyclin E, E2F1, E2F2, E2F7, ELOVL7, EME1, FGFR, FGFR2, KIAA1524, MELK, MYBL1, MYBL2, NDC80, NDN, ORC1L, PLS3, PRIMA1, RAD54L, RBL1, RBPMS, RRM2, S1PR3, SCLY, SLCIA3, SPC24, SPC25, ST8SIA4, TFDP1, TFP12, TK1, TMEM35, TTK, TWIST1, TYMS, TYK, and ZWINT.

Group B genes include the EGFR, a receptor which is regulated by NGF, which has been shown to increase BoNT/A uptake in cell culture experiments and are as follows: genes exhibiting a 1.5-fold or more decrease in expression levels in the H1 and BB10 cell lines as compared to their expression in cells from the 2D6 cell line, ABCC8, AELIM3, CAP2, IL17B, MEF2A, NEEBL, PHC, S100A6, SLC1A6, SMAD1, SMAD5, SMAD8, SYT13, and SYTL1; genes exhibiting a 1.5-fold or more increase in expression levels in the H1 and BB10 cell lines as compared to their expression in cells from the 2D6 cell line, AURKB, BIRC5, BRCA1, BRCA2, BRIP1,BUB1B, CD9, DLGAP3, DYNLT3, ENC1, FBLN1, FOXM1, Gβγ, GNAI1, GNG11, GNG12, GPSM2, GUCY1B3, HGF, ITGA6, JNK, KCNJ5, KIF18A, KITLG, MMD, MSN, MYRIP, NEK2, NR3C1, NXPH1, OSBPL3, PKMYT1, PTPRM, RAD51, RAD51AP1, SLC7A2, SLC43A3, SMC6, SNAI2, SNCAIP, SSH2, STK17A, SYNPO2, TOP2A, TPTE, TRAF4, TSPAN, TSPAN4, UBE3, UBE3B, and VAV3.

Group C genes include a number of microtubule motor proteins of the kinesin family that may function in the intracellular trafficking of BoNT/A and are as follows: genes exhibiting a 1.5-fold or more decrease in expression levels in the H1 and BB10 cell lines as compared to their expression in cells from the 2D6 cell line, CSTB, GPCR, GRIM5, KISSR, SCN2A, SLC1A2, and THBS2; genes exhibiting a 1.5-fold or more increase in expression levels in the H1 and BB10 cell lines as compared to their expression in cells from the 2D6 cell line, ALCAM, AURKA, CHEK, CIT, CSRP2, E2F, ECT2, EFNB2, ERK, ESPL1, GNAI, GPR161, HMMR, KIF4A, KIF14, KIF15, KIF22, KIF23, KIFC1, LPAR1, MK167, OIP5, PHLPP, PP1/PP2A, PPP1R3C, PRC1, PTTG1, RACGAP, RB, RGS5, SDC2, and TPX2.

The remaining networks identified did not share common genes between any other network. For Network 3, genes exhibiting a 1.5-fold or more decrease in expression levels in the H1 and BB10 cell lines as compared to their expression in cells from the 2D6 cell line, ADARB1, ADM, PTPPH, and SLCO1A2; genes exhibiting a 1.5-fold or more increase in expression levels in the H1 and BB10 cell lines as compared to their expression in cells from the 2D6 cell line, ASCL1,HES6, MAPK, NMU, PEG3, PTPRK, PRLHR, PTPRK, SGOL2, SPARC, and ZNF217. For Network 6, genes exhibiting a 1.5-fold or more decrease in expression levels in the H1 and BB10 cell lines as compared to their expression in cells from the 2D6 cell line, CNGA3, HIST1H3E and PTGS1; genes exhibiting a 1.5-fold or more increase in expression levels in the H1 and BB10 cell lines as compared to their expression in cells from the 2D6 cell line, ASF1B, BASP1, CHAF1A, NCAPH, PBK, PRAME, SMC2, UHRF1, and VRK1. For Network 9, genes exhibiting a 1.5-fold or more decrease in expression levels in the H1 and BB10 cell lines as compared to their expression in cells from the 2D6 cell line, HIST1H2BD and OSCAR; genes exhibiting a 1.5-fold or more increase in expression levels in the H1 and BB10 cell lines as compared to their expression in cells from the 2D6 cell line, C14ORF106, CEP72, KIF20A, PCNA, PEX13, PFC5, POLQ, SPAG5, SYTL4, TROAP, and WDR51A. For Network 12, genes exhibiting a 1.5-fold or more increase in expression levels in the H1 and BB10 cell lines as compared to their expression in cells from the 2D6 cell line, ANLN, ARHGAP24, ASPM, BUB1, CCDC99, CEP55, CKAP2, DRAM, E2F8, PLXNA2, SLC16A10, UBE2C, UBE2S, and WDHD1. For Network 13, genes exhibiting a 1.5-fold or more decrease in expression levels in the H1 and BB10 cell lines as compared to their expression in cells from the 2D6 cell line, CALY; genes exhibiting a 1.5-fold or more increase in expression levels in the H1 and BB10 cell lines as compared to their expression in cells from the 2D6 cell line, ARHGEF3, CTSL2, DIAPH3, FBP1, KIF2C, KIF11, PFKFB3, and PLK4. For Network 14, genes exhibiting a 1.5-fold or more decrease in expression levels in the H1 and BB10 cell lines as compared to their expression in cells from the 2D6 cell line, COL5A1 and MICAL2; genes exhibiting a 1.5-fold or more increase in expression levels in the H1 and BB10 cell lines as compared to their expression in cells from the 2D6 cell line, FNDC5, HSPC159, MAB21L2, SLITRK5, SYN2, and ZNF367. For Network 15, genes exhibiting a 1.5-fold or more decrease in expression levels in the H1 and BB10 cell lines as compared to their expression in cells from the 2D6 cell line, OLFML2A and SIGIRR; genes exhibiting a 1.5-fold or more increase in expression levels in the H1 and BB10 cell lines as compared to their expression in cells from the 2D6 cell line, EXO1, KCTD12, MYO6, PHEBL1, SHCBP1, TPBG, and TUBB6. For Network 17, genes exhibiting a 1.5-fold or more decrease in expression levels in the H1 and BB10 cell lines as compared to their expression in cells from the 2D6 cell line, HPCAL1 and LPAR5; genes exhibiting a 1.5-fold or more increase in expression levels in the H1 and BB10 cell lines as compared to their expression in cells from the 2D6 cell line, BTG3, GABRA5, TR1P10, and ZNF521. For Network 19, genes exhibiting a 1.5-fold or more decrease in expression levels in the H1 and BB10 cell lines as compared to their expression in cells from the 2D6 cell line, HTR1E and SORCS1; genes exhibiting a 1.5-fold or more increase in expression levels in the H1 and BB10 cell lines as compared to their expression in cells from the 2D6 cell line, BNC2, DPYD, EMILIN2, PPIL5, and TACC3.

| **Table 13. IPA Core Analysis of Profile Genes** | | | | | | | | |
|---|---|---|---|---|---|---|---|---|
| **Group** | **Network** | **Proteins in Network** | **pvalue** | **Profile Gene Number** | **Expression Undifferentiated State** | | **Expression Differentiated State** | |
| | | | | | **Decreased** | **Increased** | **Decreased** | **Increased** |
| A | 1 | ADAMTS9, ATAD2, CDC45L, CDKN2A, CNTN1, CNTN2, Cyclin A, Cyclin E, DIRAS3, E2F1, E2F2, E2F7, FGFR2, HDAC, MYBL1, MYBL2, NDC80, NDN, NFkB, ORC1L, PLS3, RAD54L, RBL1, RRM2, S1PR3, SPC24, SPC25, TFDP1, TFPI2, TK1, TNFRSF10D, TTK, TYMS, TYK, ZWINT | 51 | 30 | CDKN2A | ADAMTS9, CNTN1, HDAC, NDN, PLS3, S1PR3, TFPI2 | CDKN2A, CNTN2, | ADAMTS9, ATAD2, CDC45L, CNTN1, Cyclin A, Cyclin E, E2F1, E2F2, E2F7, FGFR2, MYBL1, MYBL2, NDC80, NDN, ORC1L, PLS3, RAD54L, RBL1, RRM2, S1PR3, SPC24, SPC25, TFDP1, TFPI2, TK1, TTK, TYMS, TYK, ZWINT |
| C | 2 | ALCAM, ANK2, ANK3, AURKA, DOK5, E2F, ERK, ESPL1, GDF15, GFRA2, HMMR, ITGA9, KIF15, KIF22, KISS1R, KPNA, KPNB, LGI1, MAFB, MAP2K1, MAP2K2, MKI67, NRG3, OIP5, PHKA2, PHLPP, POSTN, PP1/PP2A, PP2A, PPP1R3C, PPP2R2B, PPP2R2C, PTTG1, RAF1, RB, SCN2A, TPX2 | 46 | 28 | ANK2, ANK3, PHKA2, PPP2R2C, SCN2A | ALCAM, DOK5, ERK, GFRA2, PP1/PP2A, PP2A, PPP1R3C, PPP2R2B | KISSR, SCN2A | ALCAM, AURKA, E2F, ERK, ESPL1, HMMR, KIF15, KIF22, MKI67, OIP5, PHLPP, PP1/PP2A, PPP1R3C, PTTG1, RB, TPX2 |
| - | 3 | ADARB 1, ADM, AKAP12, ALP, ASCL1, BHLHB2, CRH, DLL1, FRZB, FSH, GRIA2, GRIP2, hCG, HES6, HTATIP2, IL13RA2, MAPK, NMU, PDGF BB, PDLIM3, PEG3, PLAT, PPFIA4, PRLHR, PTPRH, PTPRK, SGOL2, SLCO1A2, SMAD6, SPARC, STC1, TEAD4, TGFB, VEGF, ZNF217 | 46 | 28 | ADARB 1, GRIP2, SLCO1A2 | ASCL1, DLL1, FRZB, MAPK, PEG3, PLAT, PRLHR, SMAD6, SPARC, ZNF217 | ADARB 1, ADM, PTPPH, SLCO1A2 | ASCL1, HES6, MAPK, NMU, PEG3, PRLHR, PTPRK, SGOL2, SPARC, ZNF217 |
| C | 4 | AKT, ACTN1, ACTN2, ACTN3, ATF5, ATM/ATR, BDKRB2, CHEK1, CHEK2, CIT, CSRP2, CSTB, CTSH, ECT2, EFNB2, ELMO1, GNAI, GABBR2, GPCR, GPR161, GRM5, ICAM2, KIF4A, KIF14, KIF23, KIFC1, LPAR1, MMP, PDGFA PDGFB, PRC1, RAC, RAC2, RACGAP1, RGS5, SDC2, SLC1A2, THBS2 | 43 | 27 | CSTB, GPCR, GRIM5, SLC1A2 | GNAI, GPR161, LPAR1, RGS5 | CSTB, GPCR, GRIM5, SLC1A2, THBS2 | CIT, CSRP2, ECT2, EFNB2, CHEK, GNAI, GPR161, KIF4A, KIF14, KIF23, KIFC1, LPAR1, PRC1, RACGAP, RGS5, SDC2, |
| B | 5 | ABLIM3, F-ACTF, G-ACT, CD9, CXCR4, DTNA, ENC1, FNBP1, Gβγ, GNAI1, GNG11, GNG12, GPSM2, ITG, | 38 | 25 | SYT13, S100A6 | CD9, CXCR4, ENC1, Gβγ, GNAI1, GNG11, GNG12, ITGA6, JNK, MSN, MYRIP, STK17A | AELIM3, S100A6, SYT13, SYTL1 | CD9, ENC1, Gβγ, GNAI1, GNG11, GNG12, GPSM2, ITGA6, JNK, KCNJ4, MSN, MYRIP, |
| | | ITA5, ITGA6, JNK, KCNJ4, MLC, MSN, MYRIP, NOX, NET1, NRXN1, NXPH1, PI3K, RHO, S100A6, STK17A, SYT13, SYTL1, TRAF4, TSPAN, TSPAN3, TSPAN4, VAV3 | | | | | | NXPH1, STK17A, TRAF4, TSPAN, TSPAN4, VAV3 |
| - | 6 | ADCY, ADCY1, AP1, ASF1B, ATP2B4, BASP1, BCL11B, CAM, CAMK2B, CHAF1A, CNGA3, CYP2E1, DACH1, HIST1H3E, HISH3, HRH3, HSP90, IL1, IL12, INS, INFA, ISG20, KCNN2, LDL, MMP14, NCAPH, PBK, PDGF, PIAS1, PRAME, PTGS1, SEC31A, SMC2, UHRF1, VRK1 | 36 | 24 | CAMK2B | BASP1, HRH3 | CNGA3, HIST1H3E, PTGS1 | ASF1B, BASP1, CHAF1A, NCAPH, PBK, PRAME, SMC2, UHRF1, VRK1 |
| B | 7 | BARD1, BRCA1, BRCA2, BRIP1, BUB1B, CNA, CAP2, CK2, DLGAP5, FAT, GUCY1B3, HSP70, KITLG, MEF2A, NEK2, NFAT, NR3C1, NF1, PHLDA2, PKC(s), RAD51, RAD51AP1, TOP2, SAMD4A, SLC1A6, SMC6, SNAI2, SNCAIP, | 32 | 24 | CAP2, CNA, PHLDA2, PKC(s) | NR3C1, SMC6, SNCAIP, TCF7L2 | CAP2, MEF2A, PKC(s), SLC1A6 | BRCA1, BRCA2, BRIP1, BUB1B, DLGAP3, GUCY1B3, NEK2, KITLG, NR3C1, RAD51, RAD51AP1, SMC6, SNAI2, SNCAIP, UBE3, UBE3B |
| | | STAT5a, STAT5b, TCF7L2, UBE3, UBE3B, UBN, XPO1 | | | | | | |
| B | 8 | CBP, AURKB, BIRC5, CACNA1D, CBP/p300, CD3, CREB, CREB5, DBH, EGR1, ERK1, ERK2, FBLN1, FBN1, FOXM1, HGF, INCENP, KCNMA1, LGALS1, MAOA, MEK, NDRG1, NID1, P38 MAPK, PKA, PKMYT1, PRKAC, PRKACB, RAF, RAP1, RAS, RGMA, RSK, SMAD1, SMAD5, SMAD8, SYNPO2, TOP2A | 30 | 21 | KCNMA1, NDRG1, RAS | ERK1, ERK2, MAOA, P38 MAPK, RGMA | NDRG1, SMAD1, SMAD5, SMAD8 | AURKB, BIRC5, ERK1, ERK2, FBLN1, FOXM1, HGF, MAOA, P38 MAPK, PKMYT1, SYNPO2, TOP2A |
| - | 9 | C14ORF106, CDKN1A, CEP72, DHX8, FKBPL, HIST1H2BD, HNF4A, KIF20A, KRT18, MTHFS, OSCAR, PCGF5, PCNA, PELO, PEX13, PFC5, POLD4, POLK, POLL, POLM, POLQ, POLS, R3HDM1, RASL12, RFC5, SLC33A1, SPAG5, STAT4, SULT1A1, SULT1C2, SYTL4, TCEAL3, TROAP, VEZT, WDR51A, | 19 | 15 | - | PCGF5 | HIST1H2BD, OSCAR | C14ORF106, CEP72, KIF20A, PCNA, PEX13, PFC5, POLQ, SPAG5, SYTL4, TROAP, WDR51A |
| | | ZBTB16 | | | | | | |
| A | 10 | ARHGAP9, CDH10, CDS1, CDS2, CHPT1, CNTN4, ELOVL7, EME1, FGFR, GAS6, HAND1, IFI202B, ISL1, KIAA1524, KRT74, MIRN31, MUS81, MYF6, MYOD1, NFYB, OTX1, PRIMA1, QKI, RBM9, RBPMS, RORA, SHOX2, SLC39A8, SOX2, SOX15, ST8SIA4, STAT3, TWIST1, VPS39, ZFHX3 | 18 | 15 | CHPT1 | CNTN4, ELOVL7, HAND1, ODS2, SOX2, TWIST1 | ARHGAP9, CDH10 | CNTN4, ELOVL7, EME1, FGFR, KIAA1524, PRIMA1 , RBPMS, ST8SIA4, TWIST1 |
| B | 11 | ALDH3A2, B3GAL T2, BAMBI, CNN2, CTNNAL1, CTNNB1, CTSC, CUGBP2, DYNLT3, IL13, KIF18A, LIN7C, MFI2, MPP5, MRC2, NDST1, PDGF-CC, PHLDA2, PLAU, PPM1J, PPP1CA, PTPN14, PTPRM, SLC16A3, SLC26A2, SLC43A3, SLC7A2, SSH2, STK17A, TAX1BP3, TCF7L1, TGFB1, TPTE, TSPAN8 | 18 | 15 | PHLDA2 | BAMBI, CUGBP2, DYNLT3, SLC7A2, SLC43A3, STK17A, TPTE | - | DYNLT3, KIF18A, PTPRM, SLC7A2, SLC43A3, SSH2, STK17A, TPTE |
| - | 12 | AHR, AHRR, ANLN, APH1B, ARHGAP24, ASPM, BUB1, CCDC99, | 17 | 15 | HS3ST1 | PDZRN3, PVRL3 | - | ANLN, ARHGAP24, ASPM, BUB1, CCDC99, |
| | | CEP55, CKAP2, DRAM, E2F8, HS3ST1, JPH1, MIRN124-1, NMT2, PDZRN3, PLXNA2, PMM1, PPP1R13L, PVRL3, RFFL, S100A2, SLC16A10, TP53, UBE2A, UBE2C, UBE2D2, UBE2S, UBE2V1, UBE2V2, UBL3, VPS37C, WDHD1 | | | | | | CEP55, CKAP2, DRAM, E2F8, PLXNA2, SLC16A10, UBE2C, UBE2S, WDHD1 |
| - | 13 | ARHGEF3, CALY, CSH2, CTSL2, DIAPH3, ECEL1, FBP1, FFAR2, FRAT2, GSK3B, HSD17B4, IL6, IL19, KIF11, KIF2C, KNG1, LARGE, LEFTY2, LGR5, LIF, LPAR3, MPZ, MSLN, ORM2, PFKFB3, PLK4, PMP22, PRPH, PRSS8, PTGER3, SCN7A, SCNN1B, SLC4A11, TAL1 | 17 | 14 | CALY | ARHGEF3, GSK3B, RMP22 | CALY | ARHGEF3, CTSL2, DIAPH3, FBP1, KIF2C, KIF11, PFKFB3, PLK4 |
| - | 14 | AGT, AGTRAP, Beta ARK, CADPS, CD160, COL5A1, CPNE8, FNDC5, HSPC159, INA, ITGA7, LIMA1, LPP, MAB21L2, | 15 | 13 | CPNE3 | CADPS, FNDC5, MAB21L2, SLITRK5, ST8SIA2 | COL5A1, MICAL2 | FNDC5, HSPC159, MAB21L2, SLITRK5, SYN2, ZNF367 |
| | | MAPK1, MERTK, MICAL2, MIRN294, MIRN185, MIRN352, NTN1, PLCG1, PLCG2, PTGFR, RASGEF1B, RBM4B, RSU1, SKAP1, SLITRK5, SPRED1, ST8SIA2, SYN2, TGFB3, TNS1, ZNF367 | | | | | | |
| - | 15 | ACT1, BAG5, CPVL, EXO1, GIPC1, GJC1, GPR37, HSPA5, HSPA1A, HSPBP1, IkBKB, KCTD12, MAP3K3, MAP3K14, MEKK3/NIK, MIRN30B, MYO6, OLFML2A, PELI3, RHEBL1, RNF126, RPL10A, RPN1, SEC16A, SHC1, SHCBP1, SIGIRR, TJP3, TPBG, TRAF6, TRAF2-TRAF5, TRAF2-TRAF5, TRAF6, TUBB6, TUBB2B | 15 | 13 | - | KCTD12, TPBG | OLFML2A, SIGIRR | EXO1, KCTD12, MYO6, PHEBL1, SHCBP1, TPBG, TUBB6 |
| B | 16 | ABCC8, BACE2, CLASP1, COLEC12, CXCR7, DACT1, ENTPD5, FGD6, FMO1, GSTT1, IL17B, KRT34, KYNU, LAMP3, | 15 | 13 | IL17B | DACT1, PCDH8 | ABCC8, IL17B, NEEBL | MMD, OSBPL3, SYNPO2 |
| | | LMCD1, MIRN101B, MMD, NEBL, NFRKB, OSBPL3, PCDH8, PEMT, RASAL2, SAMD4A, ST3GAL3, SYNGR3, SYNPO2, TNF, TNNC1, TXN2, YWHAG, ZNF267, ZYX | | | | | | |
| - | 17 | ARPP-19, BTG3, CBFA2T2, CBLN1, CNGA2, EBF1, EGF, EGFR, GABA, GABRA5, GABRB3, GABRD, GABRR1, HDAC9, HPCAL1, HTT, IDS, INSL3, LPAR5, OPRL1, PCDH9, PDE10A, PDE11A, PDE1A, PDE1B, PDE2A, PDE4C, PDE6H, PPAP2B, PPP1R1B, ROBO2, SH3D2C1, TRIP10, ZNF521 | 14 | 13 | HPCAL1 | PPAP2B | HPCAL 1, LPAR5 | BTG3, GABRA5, TRIP10, ZNF521 |
| A | 18 | ANXA2, AQP1, C11ORF82, CCNI, CDKN2A, DEFB104A, DEFB4, EGLN3, ERAP1, FAM129A, GNAO1, IFNG, IL1B, IL1F7, INCA, LOC729687, | 14 | 12 | AQP1, CDKN2A | MINA, PDE5A | ANXA2, CDKN2A, ERAP1, RGS11 | C11ORF82, MELK, MINA, PDE5A, SCLY, SLC1A3, TMEM35 |
| | | MELK, MINA, MIRN210, MPA2L, MYC, NNAT, PDE5A, RGS11, RNASE7, RT1-B, SCLY, SCUBE1, SLC11A1, SLC14A1, SLC1A3, ST18, TSH, TMEM35, TREM3 | | | | | | |
| - | 19 | BIRC8, BNC2, CASP5, CASP10, CASP14, CASP, CBLN2, DEDD2, DNASE1L1, DPYD, DSG3, EIF4E, EMILIN2, HTR1D, HTR1E, HTR1F, HTR3A, IFT57, KHDC1, MIRN20A, MTCH1, NGFR, PLXNA4, PPIL5, RB1, RP11-257K9.7, SEMA6D, SERPINA3K, SORCS1, STK25, SYT5, TACC3, TERT | 10 | 10 | - | EMILIN2, PLXNA4 | HTR1E, SORCS1 | BNC2, DPYD, EMILIN2, PPIL5, TACC3 |
| - | 20 | EGFL7, GFI1B | 2 | 1 | - | - | - | - |
| - | 21 | AGRN, PRSS12 | 2 | 1 | - | - | - | - |
| - | 22 | KCNB1, KCNG3 | 2 | 1 | - | - | - | - |
| - | 23 | NUP37, NUP43 | 2 | 1 | - | - | - | - |
| - | 24 | HS6ST1, HS6ST2 | 2 | 1 | - | - | - | - |
| - | 25 | FUCA1, FUCA2 | 2 | 1 | - | - | - | - |
| - | 26 | IQGAP, IQGAP3, MIRN339 | 2 | 1 | - | - | - | - |

### Example V

### Pathway Analysis of Gene Expression Profiles from Clonal Cell Lines

The following example illustrates how to characterize gene expression profiles from clonal cells from an established clonal cell line that are susceptible to BoNT/A intoxication or have neurotoxin uptake capacity in order to identify biological networks or pathways associated with BoNT/A intoxication.

Computer analysis was performed to characterize the RNA expression phenotype of two independently derived single clone cell lines, BB10 and H1, very sensitive to BoNT/A activity and a single clone cell line, 2D6, that was less sensitive BoNT/A activity. The results are useful in identifying clonal cell lines selected from the SiMa parental cell line that are useful to practice the methods disclosed in the present specification.

To identify the specific gene expression phenotype associated with a BoNT/A sensitive cell line, the data was analyzed using JMP Genomic (SAS Institute Inc., Cary, NC). To perform a JMP Genomics analysis, the RNA expression data was imported to JMP Genomics by creating two SAS files, designated "data" and "Experiment design." A basic expression workflow was performed, including a variance analysis and one-way analysis of variance (one-way ANOVA) to identify differences in mean expression values. The variance analysis was used to identify differences between the cell lines and the differentiation state of each cell line (differentiated or non-differentiated). The expression values of all four cell lines were compared pair wise, independent of differentiation and genes that were ≥ 4-fold over-expressed in the H1 cell line as compared to the 2D6 cell line, in the BB10 cell line as compared to the 2D6 cell line, and in SiMa parental cell line (PA) compared to the 2D6 cell line. The variance analysis treats all factors in the study as random effects, to find out what their contribution is to the proportion of variance explained (total variance = 100%). About half of the variance, 46.9% was assigned to differences among the different cell types, while 26% was assigned to differences before and after differentiation. Only 4.6% of the variance was assigned to differences among differentiated or non-differentiated cells types, meaning that the genes that were differentially expressed after differentiation were similar for all cell types examined, suggesting that the difference in BoNT/A sensitivity among the cell lines is independent of differentiation state. As such, the focus was placed on genes that are different among the different cells types irrespective of differentiation.

Based on the results from the variance analysis, the expression values of all cell lines were compared pair wise, independent of differentiation (FIG. 2). Only gene probes that were ≥ 4-fold over-expressed in the H1 cell line as compared to the 2D6 cell line, and in the BB10 cell line as compared to the 2D6 cell line are shown. The gene probes were plotted as expression value (Log₂) on the x-axis and p-value (-log₁₀(p-value)) on the y-axis. The red dashed line marks the 95% confidence interval. The genes probes that were ≥ 4-fold over-expressed in the H1 cell line as compared to the 2D6 cell line (FIG. 2A), and the BB10 cell line as compared to the 2D6 cell line (FIG. 2B), were also to a large extend among the genes that were over-expressed in the BB10 cell line as compared to the SiMa parental cell line (PA, FIG. 2C), in the H1 cell line as compared to the SiMa parental cell line (FIG. 2D), and in the SiMa parental cell line as compared to the 2D6 cell line (FIG. 2E). These data suggest that BoNT/A sensitivity was related to a gradual increase in expression of these genes. Based on this finding, the number of gene probes was further narrowed down to 119 by including only gene probes that were also over-expressed in the SiMa parental cell line compared to the 2D6 cell line, rationalizing that gene probes that were differential expressed across one more cell line would be even more likely to be important for BoNT/A sensitivity. As such, the JMP Genomic analysis resulted in the identification of 119 gene probes, designated the JMP probe set.

The JMP probe set was exported to Ingenuity Pathway Analysis (IPA) Core Analysis (Ingenuity Systems Inc., Redwood City, CA) to further characterize the JMP probe set. Using this analysis, biologically relevant networks to BoNT/A intoxication were identified based on connectivity among the genes from an IPA library and the gene probes contained in the JMP probe set. The significance of the association to a given network was measured and ranked by the ratio of the number of gene probes from the JMP probe set that mapped to the specific network divided by the total number of genes that map to that network. Fisher's exact test was used to calculate p-scores, and based on the p-scores the genes from the JMP probe set were used to rank the networks. The p-scores were derived from p-values. If there are *n* genes in the network and *f* of them are gene probes from the JMP probe set, then the p-value is the probability of finding *f* or more gene probes from the JMP probe set in a set of *n* genes randomly selected from the Global Molecular Network. Since significant p-values are quite low (e.g., 1 x 10⁻⁸), a p-score was defined as the component of the p-value; p-score = -log10 (p-value). The identified networks were overlaid with differentially expressed genes which had more than 1.5-fold differential expression and a p-value ≤ 0.001 either before or after differentiation. Using IPA Core Analysis, 111 of the 119 gene probes from the JMP probe set were mapped to networks. The eight unmapped probes most likely represent either a probe with an outdated nomenclature or one representing a gene without a known gene product. The 111 gene probes corresponded to 79 genes that were greater than 4.0-fold differentially expressed in the H1 and BB10 cell lines when compared to the 2D6 cell line and that were also greater than 4.0-fold differentially expressed in the SiMa parental cell lines when compared to the 2D6 cell line, when the p-value cut off was set to 0.05 (Table 14).

| **Table 14. Genes Identified using IPA Core Analysis** | | | | | |
|---|---|---|---|---|---|
| **Gene Name** | **Gene Symbol** | **Probe ID. No.** | **Location** | **Type** | **Log Ratio** |
| Achaete-scute complex homolog 1 | ASCL1 | 209985_s_at | Nucleus | Transcription regulator | 4.469 |
| Achaete-scute complex homolog 1 | ASCL1 | 209988_s_at | Nucleus | Transcription regulator | 2.774 |
| Achaete-scute complex homolog 1 | ASCL1 | 213768_s_at | Nucleus | Transcription regulator | 2.955 |
| Acyl-CoA thioesterase 9 | ACOT9 | 221641_s_at | Cytoplasm | Enzyme | 2.251 |
| ADAM metallopeptidase with thrombospondin type 1 motif, 9 | ADAMTS9 | 226814_at | Extracellular Space | Peptidase | 4.034 |
| Ankyrin 2, neuronal | ANK2 | 216195_at | Plasma Membrane | Other | -2.673 |
| Ankyrin 2, neuronal | ANK2 | 232606_at | Plasma Membrane | Other | -2.621 |
| Blood vessel epicardial substance | BVES | 228783_at | Plasma Membrane | Other | 3.174 |
| Brain abundant, membrane attached signal protein 1 | BASP1 | 202391_at | Plasma Membrane | Other | 4.549 |
| BRCA1 associated RING domain 1 | BARD1 | 205345_at | Nucleus | Transcription regulator | 3.44 |
| Calsyntenin 2 | CLSTN2 | 219414_at | Plasma Membrane | Transporter | 2.024 |
| cAMP responsive element binding protein 5 | CREB5 | 232555_at | Nucleus | Transcription | -2.67 |
| Cathepsin L2 | CTSL2 | 210074_at | Cytoplasm | Peptidase | 2.119 |
| CD9 molecule | CD9 | 201005_at | Plasma Membrane | Other | 2.887 |
| Cell division cycle associated 7-like | CDCA7L | 225081_s_at | Nucleus | Other | 3.737 |
| Centromere protein L | CENPL | 1554271_a_at | Unknown | Other | 2.582 |
| Chromosome 11 open reading frame 75 | C11ORF75 | 219806_s_at | Unknown | Other | 3.914 |
| Chromosome 3 open reading frame 70 | C3ORF70 | 242447_at | Unknown | Other | 2.705 |
| Coiled-coil domain containing 109B | CCDC109B | 218802_at | Unknown | Other | 2.881 |
| Contactin 1 | CNTN1 | 211203_s_at | Plasma Membrane | Enzyme | 3.684 |
| Contactin 1 | CNTN1 | 227202_at | Plasma Membrane | Enzyme | 3.184 |
| Contactin 1 | CNTN1 | 227209_at | Plasma Membrane | Enzyme | 3.188 |
| Copine VIII | CPNE8 | 228365_at | Unknown | Other | -3.355 |
| Copine VIII | CPNE8 | 241706_at | Unknown | Other | -2.736 |
| CUG triplet repeat, RNA binding protein 2 | CUGBP2 | 202157_s_at | Nucleus | Other | 3.588 |
| Cyclin-dependent kinase 2 | CDK2 | 204252_at | Nucleus | Kinase | 2.744 |
| Cysteine and glycine-rich protein 2 | CSRP2 | 207030_s_at | Nucleus | Other | 2.487 |
| Cysteine and glycine-rich protein 2 | CSRP2 | 211126_s_at | Nucleus | Other | 2.109 |
| DEP domain containing 1 | DEPDC1 | 220295_x_at | Unknown | Other | 2.795 |
| Diaphanous homolog 3 | DIAPH3 | 232596_at | Cytoplasm | Enzyme | 2.434 |
| Dihydropyrimidine dehydrogenase | DPYD | 204646_at | Cytoplasm | Enzyme | 4.248 |
| Docking protein 5 | DOK5 | 214844_s_at | Plasma Membrane | Other | 4.56 |
| Dynein, light chain, Tctex-type 3 | DYNLT3 | 203303_at | Cytoplasm | Other | 3.449 |
| Elastin microfibril interfacer 2 | EMILIN2 | 224374_s_at | Extracellular Space | Other | 2.603 |
| Ets variant 1 | ETV1 | 206501_x_at | Nucleus | Transcription regulator | 2.578 |
| Ets variant 1 | ETV1 | 217053_x_at | Nucleus | Transcription regulator | 2.499 |
| Ets variant 1 | ETV1 | 217061_s_at | Nucleus | Transcription regulator | 2.557 |
| Family with sequence similarity 101, member B | FAM101B | 226876_at | Unknown | Other | 2.399 |
| Fibroblast growth factor receptor 2 | FGFR2 | 203638_s_at | Plasma Membrane | Kinase | 2.306 |
| Fibroblast growth factor receptor 2 | FGFR2 | 203639_s_at | Plasma Membrane | Kinase | 3.239 |
| Fibroblast growth factor receptor 2 | FGFR2 | 208228_s_at | Plasma Membrane | Kinase | 2.151 |
| Fibronectin type III domain containing 5 | FNDC5 | 226096_at | Unknown | Other | 2.054 |
| Fibulin 1 | FBLN1 | 201787_at | Extracellular Space | Other | 2.801 |
| Fibulin 1 | FBLN1 | 202994_s_at | Extracellular Space | Other | 2.378 |
| Fibulin 1 | FBLN1 | 202995_s_at | Extracellular Space | Other | 3.733 |
| G protein-coupled receptor 177 | GPR177 | 221958_s_at | Unknown | Other | 2.389 |
| G protein-coupled receptor 177 | GPR177 | 228950_s_at | Unknown | Other | 2.622 |
| G-2 and S-phase expressed 1 | GTSE1 | 204317_at | Cytoplasm | Other | 3.334 |
| Gametocyte specific factor 1 | GTSF1 | 227711_at | Unknown | Other | 6.407 |
| Guanine nucleotide binding protein (G protein), alpha inhibiting activity polypeptide 1 | GNAI1 | 227692_at | Plasma Membrane | Enzyme | 3.123 |
| Guanine nucleotide binding protein (G protein), beta polypeptide 4 | GNB4 | 225710_at | Plasma Membrane | Enzyme | 6.066 |
| Guanine nucleotide binding protein (G protein), gamma 11 | GNG11 | 204115_at | Plasma Membrane | Enzyme | 4.251 |
| Guanine nucleotide binding protein (G protein), gamma 12 | GNG12 | 212294_at | Plasma Membrane | Enzyme | 2.315 |
| Guanine nucleotide binding protein (G protein), gamma 12 | GNG12 | 222834_s_at | Plasma Membrane | Enzyme | 3.403 |
| Hepatocyte growth factor (hepapoietin A) | HGF | 209960_at | Extracellular Space | Growth factor | 2.939 |
| Hypothetical protein LOC100289109 | LOC100289 109 | 216189_at | Unknown | Other | -3.256 |
| Hypothetical protein LOC144571 | LOC144571 | 1564139_at | Unknown | Other | -2.394 |
| Inositol 1,4,5-triphosphate receptor interacting protein | ITPRIP | 225582_at | Unknown | Other | 3.522 |
| Interleukin 17B | IL17B | 220273_at | Extracellular Space | Cytokine | -4.735 |
| KDEL (Lys-Asp-Glu-Leu) containing 2 | KDELC2 | 225128_at | Unknown | Other | 3.003 |
| KIAA0125 | KIAA0125 | 206478_at | Unknown | Other | -8.436 |
| KIT ligand | KITLG | 226534_at | Extracellular Space | Growth factor | 2.59 |
| LY6/PLAUR domain containing 6 | LYPD6 | 227764_at | Extracellular Space | Other | 2.64 |
| Lysophosphatidic acid receptor 1 | LPAR1 | 204036_at | Plasma Membrane | Receptor | 3.151 |
| MAB-21-like 2 | MAB21L2 | 210303_at | Unknown | Other | 3.817 |
| Minichromosome maintenance complex component 10 | MCM10 | 223570_at | Nucleus | Other | 2.249 |
| Moesin | MSN | 200600_at | Plasma Membrane | Other | 5.842 |
| Monoamine oxidase A | MAOA | 204388_s_at | Cytoplasm | Enzyme | 4.029 |
| Monoamine oxidase A | MAOA | 204389_at | Cytoplasm | Enzyme | 3.355 |
| Monoamine oxidase A | MAOA | 212741_at | Cytoplasm | Enzyme | 4.157 |
| MYC induced nuclear antigen | MINA | 213188_s_at | Nucleus | Other | 4.019 |
| MYC induced nuclear antigen | MINA | 213189_at | Nucleus | Other | 4.636 |
| Myosin VI | MYO6 | 203216_s_at | Cytoplasm | Other | 2.406 |
| Myosin VIIA and Rab interacting protein | MYRIP | 214156_at | Cytoplasm | Other | 2.805 |
| Neuronal PAS domain protein 4 | NPAS4 | 1554299_at | Nucleus | Transcription regulator | -2.515 |
| Paternally expressed 3 | PEG3 | 209242_at | Nucleus | Kinase | 5.896 |
| Paternally expressed 3 | PEG3 | 209243_s_at | Nucleus | Kinase | 4.556 |
| Phosphoprotein associated with glycosphingolipid microdomains 1 | PAG1 | 225626_at | Plasma Membrane | Other | 2.193 |
| Poliovirus receptor-related 3 | PVRL3 | 213325_at | Plasma Membrane | Other | 2.325 |
| Polo-like kinase 2 | PLK2 | 201939_at | Nucleus | Kinase | 4.93 |
| Polymerase (DNA directed), alpha 2 (70kD subunit) | POLA2 | 204441_s_at | Nucleus | Enzyme | 2.198 |
| Prolactin releasing hormone receptor | PRLHR | 231805_at | Plasma Membrane | Receptor | 3.19 |
| Prostaglandin reductase 1 | PTGR1 | 228824_s_at | Cytoplasm | Enzyme | 2.398 |
| Prostaglandin reductase 1 | PTGR1 | 231897_at | Cytoplasm | Enzyme | 2.33 |
| Protease, serine, 12 (neurotrypsin, motopsin) | PRSS12 | 205515_at | Extracellular Space | Peptidase | 5.146 |
| Protein phosphatase 1, regulatory (inhibitor) subunit 3C | PPP1R3C | 204284_at | Cytoplasm | Phosphatase | 2.827 |
| Protein tyrosine phosphatase, receptor type, K | PTPRK | 203038_at | Plasma Membrane | Phosphatase | 3.451 |
| RAB32, member RAS oncogene family | RAB32 | 204214_s_at | Cytoplasm | Other | 2.192 |
| RELT-like 1 | RELL1 | 226430_at | Unknown | Other | 2.88 |
| Rho GTPase activating protein 24 | ARHGAP24 | 223422_s_at | Cytoplasm | Other | 3.043 |
| Rho guanine nucleotide exchange factor (GEF) 3 | ARHGEF3 | 218501_at | Cytoplasm | Other | 2.473 |
| Ring finger protein 182 | RNF182 | 230720_at | Unknown | Other | 4.746 |
| RNA binding protein with multiple splicing | RBPMS | 209487_at | Unknown | Other | 2.329 |
| RNA binding protein with multiple splicing | RBPMS | 209488_s_at | Unknown | Other | 3.292 |
| Secreted protein, acidic, cysteine-rich (osteonectin) | SPARC | 200665_s_at | Extracellular Space | Other | 4.919 |
| Secreted protein, acidic, cysteine-rich (osteonectin) | SPARC | 212667_at | Extracellular Space | Other | 4.58 |
| Shugoshin-like 2 | SGOL2 | 230165_at | Nucleus | Other | 3.108 |
| Similar to hCG2031213 | LOC728052 | 1558795_at | Unknown | Other | 3.402 |
| Solute carrier family 1 (glial high affinity glutamate transporter), member 2 | SLC1A2 | 225491_at | Plasma Membrane | Transporter | -4.354 |
| Solute carrier family 43, member 3 | SLC43A3 | 210692_s_at | Extracellular | Other | 3.126 |
| Solute carrier family 43, member | SLC43A3 | 213113_s_at | Extracellular Space | Other | 2.804 |
| Solute carrier family 44, member | SLC44A5 | 1569112_at | Unknown | Other | 3.923 |
| 5 | | | | | |
| Solute carrier family 44, member 5 | SLC44A5 | 235763_at | Unknown | Other | 4.439 |
| Solute carrier family 7 (cationic amino acid transporter, y+ system), member 2 | SLC7A2 | 225516_at | Plasma Membrane | Transporter | 3.23 |
| Sortilin-related VPS10 domain containing receptor 1 | SORCS1 | 1556891_at | Plasma Membrane | Transporter | -2.424 |
| SPC25, NDC80 kinetochore complex component, homolog | SPC25 | 209891_at | Unknown | Other | 2.634 |
| ST8 alpha-N-acetyl-neuraminide alpha-2,8-sialyltransferase 4 | ST8SIA4 | 230836_at | Cytoplasm | Enzyme | 2.705 |
| Structural maintenance of chromosomes 2 | SMC2 | 204240_s_at | Nucleus | Transporter | 2.442 |
| Structural maintenance of chromosomes 6 | SMC6 | 218781_at | Nucleus | Other | 3.327 |
| Structural maintenance of chromosomes 6 | SMC6 | 236535_at | Nucleus | Other | 3.44 |
| Syndecan 2 | SDC2 | 212154_at | Plasma Membrane | Other | 2.367 |
| Syndecan 2 | SDC2 | 212158_at | Plasma Membrane | Other | 2.098 |
| Thrombospondin 2 | THBS2 | 203083_at | Extracellular Space | Other | -2.81 |
| Thyroid hormone receptor interactor 10 | TRIP10 | 202734_at | Cytoplasm | Other | 2.663 |
| Tissue factor pathway inhibitor 2 | TFPI2 | 209278_s_at | Extracellular Space | Other | 4.666 |
| Transcription factor 7-like 1 (T-cell specific, HMG-box) | TCF7L1 | 221016_s_at | Nucleus | Transcription regulator | 2.835 |
| Transmembrane phosphatase with tensin homology | TPTE | 220205_at | Plasma Membrane | Phosphatase | 6.04 |
| Transmembrane protein 178 | TMEM178 | 229302_at | Unknown | Other | 3.194 |
| Transmembrane protein 35 | TMEM35 | 219685_at | Unknown | Other | 2.879 |
| Tumor necrosis factor, alpha-induced protein 8 | TNFAIP8 | 210260_s_at | Cytoplasm | Other | 2.389 |
| Twist homolog 1 | TWIST1 | 213943_at | Nucleus | Transcription regulator | 2.573 |
| Zinc finger protein 521 | ZNF521 | 226676_at | Nucleus | Other | 2.382 |
| Zinc finger protein 521 | ZNF521 | 226677_at | Nucleus | Other | 2.943 |
| Zinc finger protein 814 | ZNF814 | 1556204_a_at | Unknown | Other | -2.505 |
| The log ratio represent log₂ values where 0.585 is log₂(1.5) which is a 1.5-fold difference, 1 is log₂(2) | | | | | |
| which is a 2-fold difference, 1.584 is log₂(3) which is a 3-fold difference, 2 is log₂(4) which is a 4-fold difference, 2.321 is log₂(5) which is a 5-fold difference, 2.584 is log₂(6) which is a 6-fold difference, 2.807 is log₂(7) which is a 7-fold difference, 3 is log₂(8) which is a 8-fold difference, 3.169 is log₂(9) which is a 9-fold difference, and 3.321 is log₂(10) which is a 10-fold difference. | | | | | |

Using IPA Core Analysis, 73 of the 79 genes were mapped to 5 different protein networks (Table 15). Based on overlapping gene mapping, 73 of the 79 genes that were up-regulated could be associated with two major protein network groups, named A and B (Table 15). Networks 1, 2 and 5 belong to Group A, and Networks 3 and 4 belong to Group B. Similarly, all down-regulated genes were all associated with one network, named C (Table 15).

Group A genes include the FGFR2 receptor which has been shown to function in the BoNT/A intoxication process as a receptor for BoNT/A and are as follows: genes exhibiting a 1.5-fold or more increase in expression levels in the H1 and BB10 cell lines as compared to their expression in cells from the 2D6 cell line, ADAMTS9, ARHGAP24, ARHGEF3, ASCL1, BARD1, CD9, CDK2, CSRP2, CTSL2, DIAPH3, DOK5, DYNLT3, EMILIN2, ETV1, FBLN1, FGFR2, GNAI1, GNB4, GNG11, GNG12, HGF, KITLG, LPAR1, MCM10, MSN, PAG1, PEG3, PLK2, POLA2, PPP1R3C, PTPRK, RAB32, SDC2, SLC43A3, SLC7A2, SMC6, SPARC, SPC25, ST8SIA4, TCF7L1, TFPI2, TMEM35, TNFAIP8, TPTE, TRIP10, and TWIST1.

Group B genes are as follows: genes exhibiting a 1.5-fold or more increase in expression levels in the H1 and BB10 cell lines as compared to their expression in cells from the 2D6 cell line, ACOT9, BASP1, C11ORF75, CCDC109B, CDCA7L, CLSTN2, CNTN1, CUGBP2, DEPDC1, DPYD, FAM101B, FNDC5, GTSE1, MAOA, MINA, MYO6, MYRIP, PLK2, PRLHR, PVRL3, RBPMS, SGOL2, SMC2, TFP12, TMEM178, and ZNF521.

Group C genes are as follows: genes exhibiting a 1.5-fold or more decrease in expression levels in the H1 and BB10 cell lines as compared to their expression in cells from the 2D6 cell line, ANK2, CPNE8, CREB5, IL17B, KIAA0125, LOC100289109, LOC144571, NPAS4, SLC1A2, SORCS1, THBS2, and/or ZNF814.

The remaining networks identified did not share common genes between any other network. Individually, however, genes exhibiting a 1.5-fold or more increase in expression levels in the H1 and BB10 cell lines as compared to their expression in cells from the 2D6 cell line were as follows: C3ORF70 (Network 6), MAB21L2 (Network 7), PRSS12 (Network 8), CENPL (Network 9), GPR177 (Network 10), and PTGR1 (Network 11).

| **Table 15. IPA Core Analysis of Genes** | | | | | | |
|---|---|---|---|---|---|---|
| **Network** | **Group** | **Proteins in Network** | **pvalue** | **JMP Gene Number** | **Expression Profile** | |
| | | | | | **Increased** | **Decreased** |
| 1 | A | ADAMTS9, AKT, ASCL1, CDK2, CSRP2, CTSL2, DOK5, E2F, FBLN1, FSH, GNAI1, GNG11, hCG, HGF, KITLG, LPAR1, MAPK, MCM10, MEK, MSN, NFKB, P38, MAPK, PAG1, PDGF BB, PEG3, PLK2, RAC, RAS, SDC2, SPARC, TFPI2, TGFβ, TNFAIP8, TRIP10, VEGF | 48 | 22 | ADAMTS9, ASCL1, CDK2, CSRP2, CTSL2, DOK5, FBLN1, GNAI1, GNG11, HGF, KITLG, LPAR1, MCM10, MSN, PAG1, PEG3, PLK2, SDC2, SPARC, TFPI2, TNFAIP8, TRIP10 | - |
| 2 | A | CASP3, Caspase 3/7, CCL6, DIAPH3, DYNLT3, EMILIN2, EPM2A, ETV1, EYA2, FAM3B, FASTK, GNAI1, GNB4, GNG10, GNG11, GNG12, GSTM2, HK1, IGFBP3, IL13, PLCE1, POLA2, PPP1R3C, PTPRK, PTPRZ1, RAB32, SLC43A3, SLC7A2, SRC, ST8SIA4, TNF, TPTE, TWIST1 | 35 | 17 | DIAPH3, DYNLT3, EMILIN2, ETV1, GNAI1, GNB4, GNG11, GNG12, POLA2, PPP1R3C, PTPRK, RAB32, SLC43A3, SLC7A2, ST8SIA4, TPTE, TWIST1 | - |
| 3 | B | ACOT9, AR, ATXN7, BASP1, CCNE2, CCNK, CLSTN2, CNTN1, CUGBP2, DGKA, FNDC5, GTSE1, IL4, MAOA, MSH2, NOVA1, NF1, PICK1, PLK2, PNRC1, POM121, PRLHR, PTP4A1, PTP4A2, PVRL1, PVRL3, PVRL4, RBPMS, SERPINB5, SMARCA4, SPN, TFP12, TP53, ZNF521 | 26 | 14 | ACOT9, BASP1, CLSTN2, CNTN1, CUGBP2, FNDC5, GTSE1, MAOA, PLK2, PRLHR, PVRL3, RBPMS, TFP12, ZNF521 | - |
| 4 | B | ARHGAP17, AXL, C11ORF75, CAPN6, CCDC109B, CDCA7L, DEPDC1, DPYD, EIF4E, FAM101B, GRB2, HNRNPF, HTATIP2, MAOA, MINA, MIR124, MIRLET7A1, MYC, MYO6, MYRIP, NCAPD3, NF2, PPP2R5A, PPP2R5D, PPP2R5E, RPS16, RPS23, SGOL2, SLC25A12, SMC2, SMC4, TMEM178, TUSC2, TXNIP, ZBTB16 | 24 | 13 | C11ORF75, CCDC109B, CDCA7L, DEPDC1, DPYD, FAM101B, MAOA, MINA, MYO6, MYRIP, SGOL2, SMC2, TMEM178 | - |
| 5 | A | ARHGAP24, ARHGEF, | 17 | 10 | ARHGAP24, | - |
| | | ARHGEF3, BARD1, CD9, CD53, CTNNB1, DUSP3, ERK, ERM, FGF9, FGF18, FGF21, FGF23, FGFR2, IL1, IL6, IL17RD, Integrinα6β1, KLB, LEF/TCF, LPAR1, MIRN297-2, PI3K, RAS homolog, RNAPOLII, SLC26A2, SMC6, SPC25, SREBF1, TACC1, TCF7L1, TMEM35, TPT1, WISP1 | | | ARHGEF3, BARD1, CD9, FGFR2, LPAR1, SMC6, SPC25, TCF7L1, TMEM35 | |
| 6 | - | C3ORF70, MIR31 | 2 | 1 | C3ORF70 | - |
| 7 | - | MAB21L2, MIRN294 | 2 | 1 | MAB21L2 | - |
| 8 | - | AGPN, PRSS12 | 2 | 1 | PRSS12 | - |
| 9 | - | CENPL, MIRN340 | 2 | 1 | CENPL | - |
| 10 | - | GPR177, MIRN324 | 2 | 1 | GPR177 | - |
| 11 | - | ADH, PTGR1 | 2 | 1 | PTGR1 | - |
| 12 | C | ANK2, ATP2A2, BDNF, CEBPG, CPNE8, CREB5, GRM3, GRM5, IGFBP2, IL17B, ITPR, ITPR1, K+, L1CAM, MIRN330, Neurotrophin, NFE2L1, NGFR, NME1, NPAS4, Pro-inflammatory Cytokine, PSEN1, SCN2A, SCN3A, SCNN1B, SLC1A2, SLC8A1, SORCS1, SORT1, TGFA, THBS2, TNF, TNFAIP6 | 22 | 8 | - | ANK2 CPNE8 CREB5, IL17B, KIAA0125, LOC100289109, LOC144571, NPAS4, SLC1A2 SORCS1, THBS2, ZNF814 |

Computer analysis was performed as in Example V, except that the list of probes reflects genes that were greater than 1.5-fold differential expressed in the H1 and the BB10 cell lines when compared to both the 2D6 and SiMa parental cell lines. When the p-value cut off was set to 0.05, this analysis identified 439 gene probes that could be classified to 369 genes (Table 16).

| **Table 16. Genes Identified using IPA Core Analysis** | | | | | |
|---|---|---|---|---|---|
| **Gene Name** | **Gene Symbol** | **Probe ID. No.** | **Location** | **Type** | **Log Ratio** |
| 6-phosphofructo-2-kinase/fructose-2,6-biphosphatase | PFKFB3 | 202464_s_at | Cytoplasm | kinase | 1.811 |
| achaete-scute complex homolog | ASCL1 | 209985_s_at | Nucleus | transcription regulator | 2.429 |
| achaete-scute complex homolog | ASCL1 | 209987_s_at | Nucleus | transcription regulator | 2.097 |
| achaete-scute complex homolog 1 | ASCL1 | 209988_s_at | Nucleus | transcription regulator | 1.446 |
| achaete-scute complex homolog 1 | ASCL1 | 213768_s_at | Nucleus | transcription regulator | 1.578 |
| activated leukocyte cell adhesion molecule | ALCAM | 1569362_at - | Plasma Membrane | other | 2.612 |
| activated leukocyte cell adhesion molecule | ALCAM | 201951_at | Plasma Membrane | other | 1.774 |
| activated leukocyte cell adhesion molecule | ALCAM | 201952_at | Plasma Membrane | other | 1.521 |
| acyl-CoA thioesterase 1 | ACOT1 | 202982_s_at | Cytoplasm | enzyme | -2.875 |
| acylphosphatase 2, muscle type | ACYP2 | 206833_s_at | unknown | enzyme | -1.15 |
| additional sex combs like 3 | ASXL3 | 233536_at | unknown | other | 1.572 |
| adenosine deaminase, RNA-specific, B1 | ADARB1 | 203865_s_at | Nucleus | enzyme | -2.474 |
| adenylate cyclase 1 | ADCY1 | 213245_at | Plasma Membrane | enzyme | -1.935 |
| adipocyte-specific adhesion molecule | ASAM | 228082_at | Plasma Membrane | other | -2.802 |
| ADP-ribosylation factor 1 | ARF1 | 1565651_at | Cytoplasm | enzyme | 1.063 |
| ADP-ribosylation factor-like 17A | ARL17A | 243899_at | unknown | other | 0.741 |
| anaphase promoting complex subunit 7 | ANAPC7 | 225554_s_at | unknown | other | 0.679 |
| ankyrin repeat and BTB (POZ) domain containing 1 | ABTB1 | 229164_s_at | Cytoplasm | translation regulator | -0.656 |
| ankyrin repeat domain 50 | ANKRD50 | 225731_at | unknown | other | -0.654 |
| ankyrin repeat domain 50 | ANKRD50 | 225735_at | unknown | other | -1.114 |
| aspartate beta-hydroxylase domain containing 1 | ASPHD1 | 214993 _at - | unknown | other | -0.763 |
| ataxin 2-binding protein 1 | A2BP1 | 1553422_s_at | Cytoplasm | other | 1.453 |
| ataxin 2-binding protein 1 | A2BP1 | 221217_s_at | Cytoplasm | other | 1.497 |
| ATPase, Ca++ transporting, plasma membrane 3 | ATP2B3 | 207026_s_at | Plasma Membrane | transporter | -1.248 |
| ATPase, Ca++ transporting, plasma membrane 3 | ATP2B3 | 242036_x_at | Plasma Membrane | transporter | -1.777 |
| basonuclin 2 | BNC2 | 220272_at | Nucleus | other | 2.466 |
| basonuclin 2 | BNC2 | 230722_at | Nucleus | other | 1.641 |
| βγcrystallin domain containing 3 | CRYBG3 | 214030_at | unknown | other | 4.74 |
| β-site APP-cleaving enzyme 1 | BACE1 | 217904_s_at | Cytoplasm | peptidase | -1.57 |
| brain abundant, membrane attached signal protein 1 | BASP1 | 202391_at | Plasma Membrane | other | 1.146 |
| bromodomain and WD repeat domain containing 1 | BRWD1 | 225446_at | Nucleus | transcription regulator | -1.018 |
| bruno-like 4, RNA binding protein | BRUNOL4 | 238966_at | Nucleus | translation regulator | -0.81 |
| butyrophilin, subfamily 3, member A3 | BTN3A3 | 38241_at | unknown | other | -1.108 |
| C2 calcium-dependent domain containing 2 | C2CD2 | 212875_s_at | unknown | other | -1.27 |
| C2 calcium-dependent domain containing 4A | C2CD4A | 241031_at | unknown | other | -2.615 |
| cadherin 12, type 2 (N-cadherin 2) | CDH12 | 207149_at | Plasma Membrane | other | -1.408 |
| calcium/calmodulin-dependent protein kinase II inhibitor 2 | CAMK2N2 | 230706_s_at | Nucleus | other | -0.692 |
| calmin (calponin-like, transmembrane) | CLMN | 213839_at | Cytoplasm | other | -1.911 |
| calpain 2, (m/ll) large subunit | CAPN2 | 208683_at | Cytoplasm | peptidase | -1.429 |
| CAP, adenylate cyclase-associated protein, 2 | CAP2 | 212551_at | Plasma Membrane | other | -1.974 |
| CAP, adenylate cyclase-associated protein, 2 | CAP2 | 212554_at | Plasma Membrane | other | -3.68 |
| carboxypeptidase, vitellogenic-like | CPVL | 208146_s_at | unknown | peptidase | 1.708 |
| CART prepropeptide | CARTPT | 206339_at | Extracellular Space | other | -4.676 |
| Cas-Br-M (murine) ecotropic retroviral transforming sequence b | CBLB | 209682_at | Nucleus | other | 1.032 |
| CCR4-NOT transcription complex, subunit 6-like | CNOT6L | 227119_at | Cytoplasm | other | -0.785 |
| CD302 molecule | CD302 | 203799 at | Plasma Membrane | receptor | -1.518 |
| CD9 molecule | CD9 | 201005 at | Plasma Membrane | other | 1.415 |
| CDP-diacylglycerol synthase (phosphatidate cytidylyltransferase) 1 | CDS1 | 226185_at | Cytoplasm | enzyme | -1.74 |
| cell division cycle 25 homolog B (S. pombe) | CDC25B | 201853_s_at | Nucleus | phosphatase | 1.453 |
| chemokine (C-X-C motif) receptor 4 | CXCR4 | 209201_x_at | Plasma Membrane | receptor | 4.135 |
| chemokine (C-X-C motif) receptor 4 | CXCR4 | 211919_s_at | Plasma Membrane | receptor | 3.957 |
| chemokine (C-X-C motif) receptor 4 | CXCR4 | 217028_at | Plasma Membrane | receptor | 3.176 |
| chloride channel 3 | CLCN3 | 201733 at | Plasma Membrane | ion channel | -0.908 |
| chloride channel 3 | CLCN3 | 201734 at | Plasma Membrane | ion channel | -0.987 |
| cholinergic receptor, nicotinic, alpha 7 | CHRNA7 | 210123_s_at | Plasma Membrane | receptor | 3.098 |
| chromosome 1 open reading frame 21 | C1ORF21 | 221272_s_at | unknown | other | -0.74 |
| chromosome 1 open reading frame 21 | C1ORF21 | 223127_s_at | unknown | other | -0.748 |
| chromosome 1 open reading frame 43 | C1ORF43 | 1555225_at | unknown | other | 1.604 |
| chromosome 10 open reading frame 58 | C10ORF58 | 224435_at | Extracellular Space | other | 0.918 |
| chromosome 12 open reading frame 49 | C12ORF49 | 218867_s_at | unknown | other | 0.692 |
| chromosome 16 open reading frame 52 | C16ORF52 | 230721_at | unknown | other | -0.87 |
| chromosome 20 open reading frame 7 | C20ORF7 | 227160_s_at | unknown | other | 0.624 |
| chromosome 21 open reading frame 57 | C21ORF57 | 227421_at | unknown | other | -1.086 |
| chromosome 21 open reading frame 57 | C21ORF57 | 239208_s_at | unknown | other | -0.657 |
| chromosome 3 open reading frame 23 | C3ORF23 | 1555905_a_at | Cytoplasm | other | -2.797 |
| chromosome 3 open reading frame 23 | C3ORF23 | 1555906_s_at | Cytoplasm | other | -1.502 |
| chromosome 3 open reading frame 23 | C3ORF23 | 241666_at | Cytoplasm | other | -1.017 |
| chromosome 9 open reading frame 150 | C9ORF150 | 227443_at | unknown | other | -1.773 |
| claudin 12 | CLDN12 | 223249_at | Plasma Membrane | other | -0.766 |
| COBW domain containing 1 | CBWD1 | 226193_x_at | unknown | other | 0.849 |
| coiled-coil domain containing 3 | CCDC3 | 223316_at | Cytoplasm | other | 3.357 |
| collagen, type VI, alpha 1 | COL6A1 | 213428_s_at | Extracellular Space | other | -1.638 |
| cripto, FRL-1, cryptic family 1 | CFC1 | 223753_s_at | Extracellular Space | other | -2.834 |
| cripto, FRL-1, cryptic family 1 | CFC1 | 236724_at | Extracellular Space | other | -1.636 |
| CSE1 chromosome segregation 1-like | CSE1L | 210765_at | Nucleus | transporter | 1.581 |
| CUG triplet repeat, RNA binding protein 2 | CUGBP2 | 202156_s_at | Nucleus | other | 1.318 |
| CUG triplet repeat, RNA binding protein 2 | CUGBP2 | 202157 s at | Nucleus | other | 2.156 |
| CUG triplet repeat, RNA binding protein 2 | CUGBP2 | 202158 s at | Nucleus | other | 2.539 |
| cyclic nucleotide gated channel alpha 3 | CNGA3 | 207261_at | Plasma Membrane | ion channel | -3.182 |
| cyclin M1 | CNNM1 | 220166 - at | Plasma Membrane | other | -1.214 |
| cyclin-dependent kinase inhibitor 2A (melanoma, p16, inhibits CDK4) | CDKN2A | 209644_x_at | Nucleus | transcription regulator | -1.277 |
| cylindromatosis (turban tumor syndrome) | CYLD | 213295_at | Nucleus | transcription regulator | -0.815 |
| cytochrome P450, family 2, subfamily E, polypeptide 1 | CYP2E1 | 1431_at | Cytoplasm | enzyme | -4.466 |
| cytochrome P450, family 2, subfamily E, polypeptide 1 | CYP2E1 | 209975 at | Cytoplasm | enzyme | -3.946 |
| cytochrome P450, family 2, subfamily E, polypeptide 1 | CYP2E1 | 209976_s_at | Cytoplasm | enzyme | -3.429 |
| cytoglobin | CYGB | 1553572_a_at | Cytoplasm | transporter | -4.921 |
| cytokine-like 1 | CYTL1 | 219837_s_at | Extracellular Space | cytokine | -6.33 |
| dapper, antagonist of beta-catenin, homolog 1 | DACT1 | 219179_at | Cytoplasm | other | 1.682 |
| dCMP deaminase | DCTD | 201572_x_at | unknown | enzyme | -0.649 |
| DEAH (Asp-Glu-Ala-His) box polypeptide 35 | DHX35 | 218579_s_at | unknown | enzyme | 0.735 |
| deleted in azoospermia 1 | DAZ1 | 207909_xat | Nucleus | translation regulator | 6.92 |
| deleted in azoospermia 1 | DAZ1 | 207912_s_at | Nucleus | translation regulator | 5.558 |
| deleted in azoospermia 1 | DAZ1 | 208281_x_at | Nucleus | translation regulator | 5.258 |
| deleted in azoospermia 1 | DAZ1 | 208282_x_at | Nucleus | translation regulator | 7.008 |
| deleted in azoospermia 1 | DAZ1 | 216351_x_at | Nucleus | translation regulator | 3.596 |
| deleted in azoospermia 1 | DAZ1 | 216922_x_at | Nucleus | translation regulator | 5.185 |
| deleted in lymphocytic leukemia 2 (non-protein coding) | DLEU2 | 216870_x_at | unknown | other | 1.233 |
| delta-like 3 (Drosophila) | DLL3 | 219537_x_at | Extracellular Space | other | 0.779 |
| DNA segment on chromosome 4 (unique) 234 expressed sequence | D4S234E | 209569_x_at | Cytoplasm | other | -1.247 |
| DNA segment on chromosome 4 (unique) 234 expressed sequence | D4S234E | 209570_s_at | Cytoplasm | other | -1.488 |
| DNA segment on chromosome 4 (unique) 234 expressed sequence | D4S234E | 213533_at | Cytoplasm | other | -1.507 |
| dopamine beta-hydroxylase (dopamine beta-monooxygenase) | DBH | 206450_at | Cytoplasm | enzyme | 1.311 |
| doublecortin-like kinase 1 | DCLK1 | 215303_at | Cytoplasm | kinase | -1.62 |
| dual specificity phosphatase 16 | DUSP16 | 224832_at | Nucleus | phosphatase | -0.918 |
| echinoderm microtubule associated protein like 1 | EML1 | 204797_s_at | Cytoplasm | other | 2.668 |
| ectodermal-neural cortex (with BTB-like domain) | ENC1 | 201340_s_at | Nucleus | peptidase | 2.445 |
| ectodermal-neural cortex (with BTB-like domain) | ENC1 | 201341_at | Nucleus | peptidase | 1.91 |
| electron-transferring-flavoprotein dehydrogenase | ETFDH | 33494_at | Cytoplasm | enzyme | -0.807 |
| ELL associated factor 2 | EAF2 | 219551_at | Nucleus | transcription regulator | 0.693 |
| ELOVL family member 7, elongation of long chain fatty acids | ELOVL7 | 227180_at | unknown | other | 2.644 |
| endoplasmic reticulum aminopeptidase 1 | ERAP1 | 214012 - a t | Extracellular Space | peptidase | -1.885 |
| enhancer of zeste homolog 2 | EZH2 | 203358_s_at | Nucleus | transcription regulator | 0.94 |
| ephrin-A5 | EFNA5 | 214036_at | Plasma Membrane | kinase | 2.041 |
| ephrin-A5 | EFNA5 | 227955_s_at | Plasma Membrane | kinase | 1.84 |
| ephrin-A5 | EFNA5 | 233814_at | Plasma Membrane | kinase | 1.805 |
| ephrin-B3 | EFNB3 | 205031_at | Plasma Membrane | kinase | -0.816 |
| eukaryotic translation initiation | EIF3C | 236700_at | Cytoplasm | translation | 1.378 |
| factor 3, subunit C | | | | regulator | |
| exophilin 5 | EXPH5 | 214734_at | unknown | other | 3.973 |
| exosome component 6 | EXOSC6 | 231916_at | Nucleus | other | 1.057 |
| family with sequence similarity 162, member B | FAM162B | 228875 at | unknown | other | -0.743 |
| family with sequence similarity 165, member B | FAM165B | 228239_at | Plasma Membrane | other | -0.822 |
| family with sequence similarity 181, member B | FAM181B | 231430_at | unknown | other | 0.794 |
| family with sequence similarity 19 (chemokine (C-C motif)-like), member A4 | FAM19A4 | 242348_at | Extracellular Space | other | 1.282 |
| family with sequence similarity 46, member A | FAM46A | 224973_at | unknown | other | -1.509 |
| family with sequence similarity 7, member A3 | FAM7A3 | 243356_at | unknown | other | 3.495 |
| far upstream element (FUSE) binding protein 3 | FUBP3 | 239193_at | Nucleus | transcription regulator | 1.192 |
| F-box and WD repeat domain containing 7 | FBXW7 | 229419_at | Nucleus | transcription regulator | -0.921 |
| fibroblast growth factor 13 | FGF13 | 205110_s_at | Extracellular Space | growth factor | -3.522 |
| follistatin-like 1 | FSTL1 | 208782_at | Extracellular Space | other | -1.275 |
| forkhead box O6 | FOXO6 | 239657_x_at | Nucleus | other | -1.512 |
| frizzled homolog 5 | FZD5 | 221245_s_at | Plasma Membrane | receptor | 2.347 |
| FYVE, RhoGEF and PH domain containing 5 | FGD5 | 226985_at | Cytoplasm | other | -1.952 |
| G protein-coupled receptor 123 | GPR123 | 239221_at | Plasma Membrane | receptor | -1.302 |
| G protein-coupled receptor 125 | GPR125 | 210473_s_at | Plasma Membrane | receptor | 0.714 |
| galanin prepropeptide | GAL | 207466_at | Extracellular Space | other | -1.434 |
| galanin prepropeptide | GAL | 214240_at | Extracellular Space | other | -3.128 |
| gastrin-releasing peptide | GRP | 206326_at | Extracellular Space | growth factor | 4.698 |
| GDNF family receptor alpha 2 | GFRA2 | 205721_at | Plasma Membrane | receptor | 1.847 |
| GDNF family receptor alpha 2 | GFRA2 | 205722_s_at | Plasma | receptor | 2.265 |
| | | | Membrane | | |
| glutamate receptor, metabotropic 5 | GRM5 | 214217 _at | Plasma Membrane | receptor | -2.231 |
| glutathione peroxidase 7 | GPX7 | 213170_at | Cytoplasm | enzyme | -2.692 |
| glutathione synthetase | GSS | 211630_s_at | Cytoplasm | enzyme | 0.599 |
| glycosyltransferase 25 domain containing 2 | GLT25D2 | 209883_at | unknown | other | -1.223 |
| GNAS complex locus | GNAS | 214157_at | Plasma Membrane | enzyme | -1.794 |
| growth associated protein 43 | GAP43 | 204471_at | Plasma Membrane | other | 0.869 |
| growth associated protein 43 | GAP43 | 216963_s_at | Plasma Membrane | other | 1.151 |
| guanine nucleotide binding protein (G protein), alpha inhibiting activity polypeptide 1 | GNAI1 | 209576_at | Plasma Membrane | enzyme | 2.225 |
| guanine nucleotide binding protein (G protein), alpha inhibiting activity polypeptide 1 | GNAI1 | 227692_at | Plasma Membrane | enzyme | 2.111 |
| guanine nucleotide binding protein (G protein), beta polypeptide 4 | GNB4 | 225710_at | Plasma Membrane | enzyme | 4.695 |
| guanine nucleotide binding protein (G protein), gamma 11 | GNG11 | 204115_at | Plasma Membrane | enzyme | 2.587 |
| guanylate cyclase 1, soluble, alpha 3 | GUCY1A3 | 221942_s_at | Cytoplasm | enzyme | -1.522 |
| guanylate cyclase 1, soluble, alpha 3 | GUCY1A3 | 229530_at | Cytoplasm | enzyme | -1.696 |
| helicase, POLQ-like | HELQ | 228736_at | Nucleus | enzyme | -1.28 |
| HERPUD family member 2 | HERPUD2 | 236170_x_at | unknown | other | -0.897 |
| heterogeneous nuclear ribonucleoprotein R | HNRNPR | 208765_s_at | Nucleus | other | -0.64 |
| hippocalcin-like 1 | HPCAL1 | 205462_s_at | Cytoplasm | other | -1.551 |
| hippocalcin-like 1 | HPCAL1 | 212552_at | Cytoplasm | other | -1.387 |
| histone cluster 1, H2ac | HIST1H2A C | 215071_s_at | Nucleus | other | -2.462 |
| histone cluster 1, H2bd | HIST1H2B D | 209911_x_at | Nucleus | other | -2.036 |
| histone cluster 1, H2bk | HIST1H2B K | 209806 at | Nucleus | other | -1.575 |
| histone cluster 2, H2be | HIST2H2B | 202708_s_at | Nucleus | other | -1.735 |
| | E | | | | |
| hypothetical LOC 100130522 | LOC10013 0522 | 230477_at | unknown | other | -0.928 |
| hypothetical LOC26082 | DKFZP434 L187 | 230861_at | unknown | other | 0.968 |
| hypothetical protein LOC100128844 | LOC10012 8844 | 229110_at | unknown | other | 2.106 |
| hypothetical protein LOC643401 | LOC34010 9 | 1557765_at | unknown | other | 2.848 |
| integrin, alpha 6 | ITGA6 | 201656_at | Plasma Membrane | other | 2.822 |
| integrin, alpha 6 | ITGA6 | 215177_s_at | Plasma Membrane | other | 2.024 |
| integrin, beta 5 | ITGB5 | 201125_s_at | Plasma Membrane | other | 0.913 |
| interleukin 10 receptor, beta | IL10RB | 209575_at | Plasma Membrane | transmembr ane receptor | -1.444 |
| junction mediating and regulatory protein, p53 cofactor | JMY | 226352_at | Nucleus | transcription regulator | -0.744 |
| K(lysine) acetyltransferase 2B | KAT2B | 203845_at | Nucleus | transcription regulator | -0.731 |
| KDEL (Lys-Asp-Glu-Leu) containing 2 | KDELC2 | 225128_at | unknown | other | 1.085 |
| kelch domain containing 1 | KLHDC1 | 1552733_at | unknown | other | -0.952 |
| kelch repeat and BTB (POZ) domain containing 11 | KBTBD11 | 204301_at | unknown | other | -0.733 |
| kelch-like 13 (Drosophila) | KLHL13 | 227875_at | unknown | other | 0.902 |
| KH domain containing, RNA binding, transduction associated 3 | KHDRBS3 | 209781_s_at | Nucleus | other | 1.082 |
| KH homology domain containing 1 | KHDC1 | 230055_at | unknown | other | -1.656 |
| KIAA1598 | KIAA1598 | 221802_s_at | unknown | other | -2.811 |
| kinesin family member 16B | KIF16B | 232083_at | Cytoplasm | other | 1.688 |
| lectin, galactoside-binding, soluble, 3 binding protein | LGALS3BP | 200923_at | Plasma Membrane | transmembr ane receptor | -1.417 |
| leukemia inhibitory factor receptor alpha | LIFR | 225575_at | Plasma Membrane | transmembr ane receptor | -0.941 |
| limb bud and heart development homolog (mouse) | LBH | 221011_s_at | Nucleus | transcription regulator | 0.788 |
| LRP2 binding protein | LRP2BP | 207797_s_at | unknown | other | -1.365 |
| lumican | LUM | 201744_s_at | Extracellular Space | other | 5.638 |
| LYR motif containing 5 | LYRM5 | 225469_at | unknown | other | -0.93 |
| mab-21-like 1 | MAB21L1 | 206163_at | unknown | other | 2.722 |
| mab-21-like 2 | MAB21L2 | 210302_s_at | unknown | other | 2.871 |
| mab-21-like 2 | MAB21L2 | 210303_at | unknown | other | 2.204 |
| macrophage stimulating 1 (hepatocyte growth factor-like) | MST1 | 216320_x_at | Extracellular Space | growth factor | -1.012 |
| major facilitator superfamily domain containing 4 | MFSD4 | 229254_at | unknown | other | -1.752 |
| mannosidase, alpha, class 2A, member 1 | MAN2A1 | 226538_at | Cytoplasm | enzyme | 0.864 |
| mannosyl (alpha-1,3-)-glycoprotein beta-1,4-N-acetylglucosaminyltransferase, isozyme A | MGAT4A | 226039_at | unknown | enzyme | -0.904 |
| mesenchyme homeobox 2 | MEOX2 | 206201_s_at | Nucleus | transcription regulator | -4.213 |
| microtubule-associated protein 9 | MAP9 | 220145_at | unknown | other | -0.7 |
| microtubule-associated protein 9 | MAP9 | 228423_at | unknown | other | -1.108 |
| microtubule-associated protein 9 | MAP9 | 235550_at | unknown | other | -0.863 |
| mitochondrial ribosomal protein S33 | MRPS33 | 218654_s_at | Cytoplasm | other | -0.609 |
| mitogen-activated protein kinase kinase kinase 5 | MAP3K5 | 203836_s_at | Cytoplasm | kinase | -1.159 |
| moesin | MSN | 200600 at | Plasma Membrane | other | 2.082 |
| monoamine oxidase A | MAOA | 204388_s_at | Cytoplasm | enzyme | 2.15 |
| monoamine oxidase A | MAOA | 204389_at | Cytoplasm | enzyme | 1.88 |
| monoamine oxidase A | MAOA | 212741_at | Cytoplasm | enzyme | 2.048 |
| monocyte to macrophage differentiation-associated | MMD | 244523_at | Plasma Membrane | other | 1.045 |
| multiple C2 domains, transmembrane 1 | MCTP1 | 220122_at | unknown | other | -1.597 |
| MYC induced nuclear antigen | MINA | 213188_s_at | Nucleus | other | 1.391 |
| MYC induced nuclear antigen | MINA | 213189_at | Nucleus | other | 1.641 |
| myosin IB | MYO1B | 212365_at | Cytoplasm | other | 0.71 |
| myosin VI | MYO6 | 203216_s_at | Cytoplasm | other | 1.104 |
| myosin VI | MYO6 | 210480_s_at | Cytoplasm | other | 1.41 |
| Na+/H+ exchanger domain containing 2 | NHEDC2 | 1564746_at | Plasma Membrane | other | -1.462 |
| N-acylethanolamine acid amidase | NAAA | 214765_s_at | Cytoplasm | enzyme | 0.911 |
| NADH dehydrogenase (ubiquinone) 1 alpha subcomplex, 4-like | NDUFA4L2 | 218484_at | unknown | enzyme | -3.507 |
| NADH dehydrogenase (ubiquinone) flavoprotein 3, 10kDa | NDUFV3 | 226616_s_at | Cytoplasm | enzyme | -0.875 |
| nebulette | NEBL | 203961_at | Cytoplasm | other | -1.359 |
| neural precursor cell expressed, developmentally down-regulated 9 | NEDD9 | 202150_s_at | Nucleus | other | 2.41 |
| NIPA-like domain containing 3 | NIPAL3 | 225876_at | unknown | other | -1.203 |
| nitric oxide synthase 1 (neuronal) | NOS1 | 239132_at | Cytoplasm | enzyme | 1.138 |
| nitric oxide synthase 1 (neuronal) | NOS1 | 240911_at | Cytoplasm | enzyme | 1.187 |
| NLR family member X1 | NLRX1 | 219680_at | unknown | other | -1.541 |
| non-protein coding RNA 171 | NCRNA001 71 | 215985_at | unknown | other | -0.692 |
| nuclear receptor coactivator 7 | NCOA7 | 225344_at | Nucleus | other | -0.842 |
| nucleosome assembly protein 1-like 3 | NAP 1 L3 | 204749_at | Nucleus | other | -1.53 |
| nudix (nucleoside diphosphate linked moiety X)-type motif 19 | NUDT19 | 235384_at | Cytoplasm | other | -1.685 |
| olfactomedin 3 | OLFM3 | 1554524_a_at | Cytoplasm | other | -0.927 |
| oxysterol binding protein-like 3 | OSBPL3 | 209626_s_at | Cytoplasm | other | 2.083 |
| pecanex-like 2 | PCNXL2 | 1554256_a_at | unknown | other | -0.728 |
| pellino homolog 2 | PELI2 | 219132_at | Cytoplasm | other | 0.732 |
| peptidase M20 domain containing 2 | PM20D2 | 225421_at | unknown | other | 1.6 |
| peptidylglycine alpha-amidating monooxygenase | PAM | 202336_s_at | Plasma Membrane | enzyme | -0.861 |
| peripherin | PRPH | 213847_at | Plasma Membrane | other | -1.417 |
| PHD finger protein 20 | PHF20 | 235389_at | Nucleus | other | 0.596 |
| phosphatidic acid phosphatase type 2 domain containing 1A | PPAPDC1 A | 236044_at | unknown | phosphatase | -2.621 |
| phosphatidylinositol glycan anchor biosynthesis, class H | PIGH | 209625_at | Cytoplasm | enzyme | -0.597 |
| phosphoinositide-interacting regulator of transient receptor potential channels | HCG 1776018 | 232887_at | unknown | other | -2.019 |
| phosphorylase kinase, alpha 1 (muscle) | PHKA1 | 229876_at | Cytoplasm | kinase | -1.2 |
| plastin 3 (T isoform) | PLS3 | 201215_at | Cytoplasm | other | 1.068 |
| platelet-derived growth factor receptor, beta polypeptide | PDGFRB | 202273_at | Plasma Membrane | kinase | -1.373 |
| pleiotrophin | PTN | 209466_x_at | Extracellular Space | growth factor | -1.426 |
| pleiotrophin | PTN | 211737_x_at | Extracellular Space | growth factor | -2.929 |
| pleiotropic regulator 1 | PLRG1 | 225194_at | Nucleus | transcription regulator | -0.701 |
| pleiotropic regulator 1 | PLRG1 | 227246_at | Nucleus | transcription regulator | -1.228 |
| plexin A2 | PLXNA2 | 213030_s_at | Plasma Membrane | other | 0.946 |
| plexin A4 | PLXNA4 | 232317_at | Plasma Membrane | receptor | 1.368 |
| polo-like kinase 2 | PLK2 | 201939_at | Nucleus | kinase | 3.606 |
| polycomb group ring finger 5 | PCGF5 | 227935_s_at | unknown | other | 0.791 |
| polymerase (DNA directed), epsilon | POLE | 216026_s_at | Nucleus | enzyme | 1.053 |
| potassium channel tetramerisation domain containing 12 | KCTD12 | 212188_at | unknown | ion channel | 3.396 |
| potassium channel tetramerisation domain containing 12 | KCTD12 | 212192_at | unknown | ion channel | 4.806 |
| potassium large conductance calcium-activated channel, subfamily M, alpha member 1 | KCNMA1 | 221584_s_at | Plasma Membrane | ion channel | -2.389 |
| potassium voltage-gated channel, KQT-like subfamily, member 2 | KCNQ2 | 205737_at | Plasma Membrane | ion channel | -1.246 |
| pregnancy-associated plasma protein A, pappalysin 1 | PAPPA | 201982_s_at | Extracellular Space | peptidase | -1.672 |
| prostaglandin E receptor 2 (subtype EP2), 53kDa | PTGER2 | 206631_at | Plasma Membrane | receptor | -2.328 |
| prostaglandin F2 receptor negative regulator | PTGFRN | 224937_at | Plasma Membrane | other | 2.301 |
| proteasome (prosome, macropain) subunit, alpha type, 7 | PSMA7 | 216088_s_at | Cytoplasm | peptidase | 0.658 |
| protein kinase, cAMP-dependent, catalytic, beta | PRKACB | 235780_at | Cytoplasm | kinase | -2.17 |
| protein tyrosine phosphatase, receptor type, D | PTPRD | 214043_at | Plasma Membrane | phosphatase | -1.16 |
| protein tyrosine phosphatase, receptor type, E | PTPRE | 221840_at | Plasma Membrane | phosphatase | 3.044 |
| protein tyrosine phosphatase, receptor type, G | PTPRG | 204944_at | Plasma Membrane | phosphatase | 1.237 |
| RAB35, member RAS oncogene family | RAB35 | 205461_at | Cytoplasm | enzyme | 0.737 |
| RAB6B, member RAS oncogene family | RAB6B | 225259_at | Cytoplasm | enzyme | -1.229 |
| Rac/Cdc42 guanine nucleotide exchange factor (GEF) 6 | ARHGEF6 | 209539_at | Cytoplasm | other | 2.719 |
| RAN, member RAS oncogene family | RAN | 200750_s_at | Nucleus | enzyme | 0.59 |
| RAS and EF-hand domain containing | RASEF | 1553986_at | unknown | other | 1.393 |
| ras homolog gene family, member U | RHOU | 223168_at | Cytoplasm | enzyme | -1.154 |
| regulator of G-protein signaling 5 | RGS5 | 1555725 a at | Plasma Membrane | other | 4.038 |
| regulator of G-protein signaling 5 | RGS5 | 209070 s at | Plasma Membrane | other | 3.729 |
| regulator of G-protein signaling 5 | RGS5 | 209071_s_at | Plasma Membrane | other | 3.468 |
| regulator of G-protein signaling 5 | RGS5 | 218353_at | Plasma Membrane | other | 3.65 |
| RELT-like 1 | RELL1 | 226430_at | unknown | other | 1.214 |
| retinoblastoma-like 1 (p107) | RBL1 | 1559307_s_at | Nucleus | other | 0.959 |
| retrotransposon gag domain containing 4 | RGAG4 | 227823_at | unknown | other | -1.775 |
| Rho GTPase activating protein 6 | ARHGAP6 | 206167_s_at | Cytoplasm | other | 2.809 |
| ribosomal L1 domain containing 1 | RSL1D1 | 213750_at | Cytoplasm | other | -0.816 |
| RIMS binding protein 2 | RIMBP2 | 238817_at | Plasma Membrane | other | 1.679 |
| ring finger protein 13 | RNF13 | 201780_s_at | Nucleus | other | -0.707 |
| ring finger protein 182 | RNF182 | 230720_at | unknown | other | 2.696 |
| ring finger protein 34 | RNF34 | 219035_s_at | Cytoplasm | enzyme | 0.85 |
| ring finger protein 41 | RNF41 | 201962_s_at | Cytoplasm | other | -0.591 |
| RNA polymerase I transcription factor homolog pseudogene 1 | LOC94431 | 216908_x_at | unknown | other | -0.674 |
| sal-like 4 | SALL4 | 229661_at | Nucleus | other | 1.586 |
| salt-inducible kinase 1 | SIK1 | 208078_s_at | Cytoplasm | kinase | -1.403 |
| seizure related 6 homolog (mouse)-like | SEZ6L | 207873_x_at | Plasma Membrane | other | 0.866 |
| seizure related 6 homolog (mouse)-like | SEZ6L | 211894_x_at | Plasma Membrane | other | 0.797 |
| sema domain, transmembrane domain (TM), and cytoplasmic domain, (semaphorin) | SEMA6A | 220454_s_at | Plasma Membrane | other | 1.356 |
| type, 2 peptidase inhibitor, Kunitz | SPINT2 | 210715_s_at | Extracellular Space | other | -1.889 |
| SH3 domain containing, Ysc84-like 1 | SH3YL1 | 204019_s_at | unknown | other | -0.859 |
| SH3-domain GRB2-like 2 | SH3GL2 | 205751_at | Plasma Membrane | enzyme | -1.605 |
| similar to hCG2031213 | LOC72805 2 | 1558795_at | unknown | other | 2.239 |
| similar to hCG2031213 | LOC72805 2 | 1558796_a_at | unknown | other | 1.887 |
| single immunoglobulin and toll-interleukin 1 receptor (TIR) domain | SIGIRR | 52940_at | Plasma Membrane | receptor | -1.42 |
| single-minded homolog 1 | SIM1 | 1556300_s_at | Nucleus | transcription regulator | 6.36 |
| single-minded homolog 1 | SIM1 | 206876_at | Nucleus | transcription regulator | 5.737 |
| SLIT and NTRK-like family, member 5 | SLITRK5 | 214930_at | unknown | other | 4.094 |
| SMG1 homolog, phosphatidylinositol 3-kinase-related kinase pseudogene | LOC64129 8 | 244766_at | unknown | other | 1.065 |
| solute carrier family 12 (potassium/chloride transporters), member 7 | SLC12A7 | 218066_at | Plasma Membrane | transporter | -1.258 |
| solute carrier family 22, member 17 | SLC22A17 | 218675_at | Plasma Membrane | transporter | -0.724 |
| solute carrier family 35, member F3 | SLC35F3 | 229065_at | unknown | other | -3.048 |
| solute carrier family 44, member 5 | SLC44A5 | 1569112_at | unknown | other | 2.738 |
| solute carrier family 44, member 5 | SLC44A5 | 235763_at | unknown | other | 1.845 |
| solute carrier family 7 (cationic amino acid transporter, y+ system), member 2 | SLC7A2 | 225516_at | Plasma Membrane | transporter | 1.892 |
| solute carrier organic anion transporter family, member 3A1 | SLC03A1 | 219229_at | Plasma Membrane | transporter | 1.622 |
| solute carrier organic anion transporter family, member 3A1 | SLC03A1 | 227367_at | Plasma Membrane | transporter | 1.045 |
| sparc/osteonectin, cwcv and kazal-like domains proteoglycan (testican) 2 | SPOCK2 | 202524_s_at | Extracellular Space | other | -0.83 |
| sperm associated antigen 6 | SPAG6 | 210032_s_at | Cytoplasm | other | 1.687 |
| sperm associated antigen 6 | SPAG6 | 210033_s_at | Cytoplasm | other | 2.774 |
| spermatogenesis associated, serine-rich 2-like | SPATS2L | 222154_s_at | unknown | other | 0.907 |
| sphingosine-1-phosphate receptor 3 | S1PR3 | 228176_at | Plasma Membrane | receptor | 1.9 |
| ST8 alpha-N-acetyl-neuraminide alpha-2,8-sialyltransferase 1 | ST8SIA1 | 210073_at | Cytoplasm | enzyme | 1.114 |
| ST8 alpha-N-acetyl-neuraminide alpha-2,8-sialyltransferase 3 | ST8SIA3 | 230262_at | Cytoplasm | enzyme | -1.039 |
| stathmin-like 3 | STMN3 | 222557_at | Nucleus | other | 0.788 |
| STEAP family member 3 | STEAP3 | 218424_s_at | Cytoplasm | transporter | -1.827 |
| steroidogenic acute regulatory protein | STAR | 204548_at | Cytoplasm | transporter | -1.093 |
| stimulated by retinoic acid gene 6 homolog | STRA6 | 1569334_at | Plasma Membrane | other | 1.359 |
| stimulated by retinoic acid gene 6 homolog | STRA6 | 1569335_a_at | Plasma Membrane | other | 1.167 |
| storkhead box 2 | STOX2 | 226822_at | unknown | other | -0.854 |
| synaptojanin 1 | SYNJ1 | 212990_at | Cytoplasm | phosphatase | -0.624 |
| synaptotagmin XIII | SYT13 | 226086_at | unknown | transporter | -4.754 |
| synuclein, alpha (non A4 component of amyloid precursor) | SNCA | 236081_at | Cytoplasm | other | -0.935 |
| taxilin beta | TXLNB | 227834_at | Cytoplasm | other | -2.226 |
| teashirt zinc finger homeobox 3 | TSHZ3 | 223392_s_at | Nucleus | transcription regulator | 1.472 |
| teashirt zinc finger homeobox 3 | TSHZ3 | 223393_s_at | Nucleus | transcription regulator | 0.962 |
| tetratricopeptide repeat domain 39C | TTC39C | 238480_at | unknown | other | -0.824 |
| TH1-like | TH1L | 220607_x_at | Nucleus | other | 1.027 |
| TH1-like | TH1L | 225006_x_at | Nucleus | other | 0.884 |
| TH1-like | TH1L | 225261_x_at | Nucleus | other | 0.883 |
| TH1-like | TH1L | 225865_x_at | Nucleus | other | 0.904 |
| thioredoxin reductase 1 | TXNRD1 | 201266_at | Cytoplasm | enzyme | 1.032 |
| THO complex 4 | THOC4 | 226319_s_at | Nucleus | transcription regulator | 0.996 |
| TIMP metallopeptidase inhibitor 3 | TIMP3 | 201147_s_at | Extracellular Space | other | 5.151 |
| TIMP metallopeptidase inhibitor 3 | TIMP3 | 201148_s_at | Extracellular Space | other | 3.637 |
| TIMP metallopeptidase inhibitor 3 | TIMP3 | 201149_s_at | Extracellular Space | other | 5.037 |
| TIMP metallopeptidase inhibitor 3 | TIMP3 | 201150_s_at | Extracellular Space | other | 4.312 |
| tissue factor pathway inhibitor 2 | TFPI2 | 209277_at | Extracellular Space | other | 1.592 |
| tissue factor pathway inhibitor 2 | TFPI2 | 209278_s_at | Extracellular Space | other | 2.326 |
| TM2 domain containing 1 | TM2D1 | 213882_at | Plasma Membrane | receptor | -0.769 |
| transcription factor 7-like 2 (T-cell specific, HMG-box) | TCF7L2 | 212761_at | Nucleus | transcription regulator | 1.005 |
| transcription factor 7-like 2 (T-cell specific, HMG-box) | TCF7L2 | 212762_s_at | Nucleus | transcription regulator | 1.224 |
| transcription factor 7-like 2 (T-cell specific, HMG-box) | TCF7L2 | 216035_x_at | Nucleus | transcription regulator | 1.072 |
| transcription factor 7-like 2 (T-cell specific, HMG-box) | TCF7L2 | 216037_x_at | Nucleus | transcription regulator | 0.81 |
| transcription factor 7-like 2 (T-cell specific, HMG-box) | TCF7L2 | 216511_s_at | Nucleus | transcription regulator | 1.003 |
| transducin-like enhancer of split 3 | TLE3 | 212770_at | Nucleus | other | 0.976 |
| transformer 2 alpha homolog | TRA2A | 213593_s_at | Nucleus | other | 1.12 |
| transmembrane inner ear | TMIE | 1553601_a_at | unknown | other | -3.334 |
| transmembrane protein 132C | TMEM132 C | 232313_at | unknown | other | 4.02 |
| transmembrane protein 178 | TMEM178 | 229302_at | unknown | other | 1.697 |
| transmembrane protein 184C | TMEM184 C | 219074_at | unknown | other | -0.779 |
| transmembrane protein 5 | TMEM5 | 204808_s_at | Plasma Membrane | other | -1.074 |
| transmembrane protein 59-like | TMEM59L | 219005_at | Cytoplasm | other | -0.845 |
| tripartite motif-containing 29 | TRIM29 | 211002_s_at | Cytoplasm | transcription regulator | 2.005 |
| tripartite motif-containing 36 | TRIM36 | 219736_at | Cytoplasm | other | 2.221 |
| trophoblast glycoprotein | TPBG | 203476_at | Plasma Membrane | other | 2.158 |
| tumor suppressor candidate 3 | TUSC3 | 232770_at | Extracellular Space | enzyme | -1.407 |
| U2 small nuclear RNA auxiliary factor 1 | U2AF1 | 232141_at - | Nucleus | other | 0.776 |
| ubiquitin-conjugating enzyme E2 variant 1 | UBE2V1 | 201003_x_at | Nucleus | transcription regulator | 0.663 |
| urocortin | UCN | 206072_at | Extracellular Space | other | -0.961 |
| vacuolar protein sorting 29 homolog | VPS29 | 223026_s_at | Cytoplasm | transporter | 0.805 |
| v-erb-a erythroblastic leukemia viral oncogene homolog 4 | ERBB4 | 206794_at - | Plasma Membrane | kinase | -1.537 |
| v-erb-a erythroblastic leukemia viral oncogene homolog 4 | ERBB4 | 214053_at - | Plasma Membrane | kinase | -1.708 |
| v-erb-b2 erythroblastic leukemia viral oncogene homolog 2, neuro/glioblastoma derived oncogene homolog | ERBB2 | 216836_s_at | Plasma Membrane | kinase | -0.698 |
| v-raf-1 murine leukemia viral oncogene homolog 1 | RAF1 | 1557675_at | Cytoplasm | kinase | 0.696 |
| WD repeat and FYVE domain containing 3 | WDFY3 | 212602_at | Cytoplasm | enzyme | -0.626 |
| WD repeat and FYVE domain containing 3 | WDFY3 | 212606_at | Cytoplasm | enzyme | -0.785 |
| zinc finger homeobox 4 | ZFHX4 | 219779_at | unknown | other | 1.36 |
| zinc finger homeobox 4 | ZFHX4 | 241700_at | unknown | other | 1.234 |
| zinc finger protein 217 | ZNF217 | 203739_at | Nucleus | transcription regulator | 1.096 |
| zinc finger protein 503 | ZNF503 | 227195_at | Nucleus | other | 1.373 |
| zinc finger protein 641 | ZNF641 | 226509_at | unknown | other | -1.427 |
| zinc finger protein 662 | ZNF662 | 228538_at | unknown | other | -3.352 |
| zinc finger, CCCH-type with G patch domain | ZGPAT | 221848_at | unknown | other | 0.991 |

Computer analysis was performed as in Example V, except that the list of probes reflects genes that were greater than 1.5-fold differential expressed in the H1 cell line and the BB10 cell line when compared to both the 2D6 and SiMa parental cell lines, and also a greater than 1.5-fold differential expressed in the SiMa parental cell line when compared to the 2D6 cell line. When the p-value cut off was set to 0.05, this analysis identified 94 gene probes that could be classified to 70 genes (Table 17).

| **Table 17. Genes Identified using IPA Core Analysis** | | | | | |
|---|---|---|---|---|---|
| **Gene Name** | **Gene Symbol** | **Probe ID. No.** | **Location** | **Type** | **Log Ratio** |
| achaete-scute complex homolog 1 | ASCL1 | 209985_s_at | Nucleus | transcription regulator | 2.429 |
| achaete-scute complex homolog 1 | ASCL1 | 209988_s_at | Nucleus | transcription regulator | 1.446 |
| achaete-scute complex homolog 1 | ASCL1 | 213768_s_at | Nucleus | transcription regulator | 1.578 |
| activated leukocyte cell adhesion molecule | ALCAM | 201952_at | Plasma Membrane | other | 1.521 |
| ADP-ribosylation factor 1 | ARF1 | 1565651_at | Cytoplasm | enzyme | 1.063 |
| ADP-ribosylation factor-like 17A | ARL17A | 243899_at | unknown | other | 0.741 |
| ataxin 2-binding protein 1 | A2BP1 | 1553422_s_at | Cytoplasm | other | 1.453 |
| ataxin 2-binding protein 1 | A2BP1 | 221217_s_at | Cytoplasm | other | 1.497 |
| brain abundant, membrane attached signal protein 1 | BASP1 | 202391_at | Plasma Membrane | other | 1.146 |
| CAP, adenylate cyclase-associated protein, 2 | CAP2 | 212551_at | Plasma Membrane | other | -1.974 |
| CD9 molecule | CD9 | 201005_at | Plasma Membrane | other | 1.415 |
| CUG triplet repeat, RNA binding protein 2 | CUGBP2 | 202157_s_at | Nucleus | other | 2.156 |
| cyclic nucleotide gated channel alpha 3 | CNGA3 | 207261_at | Plasma Membrane | ion channel | -3.182 |
| cyclin-dependent kinase inhibitor 2A (melanoma, p16, | CDKN2A | 209644_x_at | Nucleus | transcription regulator | -1.277 |
| inhibits CDK4) | | | | | |
| ELL associated factor 2 | EAF2 | 219551_at | Nucleus | transcription regulator | 0.693 |
| eukaryotic translation initiation factor 3, subunit C | EIF3C | 236700_at | Cytoplasm | translation regulator | 1.378 |
| family with sequence similarity 162, member B | FAM162B | 228875_at | unknown | other | -0.743 |
| family with sequence similarity 181, member B | FAM181B | 231430_at | unknown | other | 0.794 |
| far upstream element (FUSE) binding protein 3 | FUBP3 | 239193_at | Nucleus | transcription regulator | 1.192 |
| fibroblast growth factor 13 | FGF13 | 205110_s_at | Extracellular Space | growth factor | -3.522 |
| follistatin-like 1 | FSTL1 | 208782_at | Extracellular Space | other | -1.275 |
| forkhead box O6 | FOXO6 | 239657_x_at | Nucleus | other | -1.512 |
| guanine nucleotide binding protein (G protein), alpha inhibiting activity polypeptide 1 | GNAI1 | 227692_at | Plasma Membrane | enzyme | 2.111 |
| guanine nucleotide binding protein (G protein), beta polypeptide 4 | GNB4 | 225710_at | Plasma Membrane | enzyme | 4.695 |
| guanine nucleotide binding protein (G protein), gamma 11 | GNG11 | 204115_at | Plasma Membrane | enzyme | 2.587 |
| KDEL (Lys-Asp-Glu-Leu) containing 2 | KDELC2 | 225128_at | unknown | other | 1.085 |
| kelch-like 13 | KLHL13 | 227875_at | unknown | other | 0.902 |
| KH domain containing, RNA binding, transduction associated 3 | KHDRBS3 | 209781_s_at | Nucleus | other | 1.082 |
| KH homology domain containing 1 | KHDC1 | 230055_at | unknown | other | -1.656 |
| leukemia inhibitory factor receptor alpha | LIFR | 225575_at | Plasma Membrane | transmembra ne receptor | -0.941 |
| mab-21-like 2 | MAB21L2 | 210303_at | unknown | other | 2.204 |
| mesenchyme homeobox 2 | MEOX2 | 206201_s_at | Nucleus | transcription regulator | -4.213 |
| moesin | MSN | 200600_at | Plasma Membrane | other | 2.082 |
| monoamine oxidase A | MAOA | 204388_s_at | Cytoplasm | enzyme | 2.15 |
| monoamine oxidase A | MAOA | 204389_at | Cytoplasm | enzyme | 1.88 |
| monoamine oxidase A | MAOA | 212741_at | Cytoplasm | enzyme | 2.048 |
| multiple C2 domains, transmembrane 1 | MCTP1 | 220122_at | unknown | other | -1.597 |
| MYC induced nuclear antigen | MINA | 213188_s_at | Nucleus | other | 1.391 |
| MYC induced nuclear antigen | MINA | 213189_at | Nucleus | other | 1.641 |
| myosin VI | MYO6 | 203216_s_at | Cytoplasm | other | 1.104 |
| phosphatidic acid phosphatase type 2 domain containing | PPAPDC1A | 236044_at | unknown | phosphatase | -2.621 |
| plastin 3 (T isoform) | PLS3 | 201215_at | Cytoplasm | other | 1.068 |
| pleiotrophin | PTN | 209466_x_at | Extracellular Space | growth factor | -1.426 |
| pleiotrophin | PTN | 211737_x_at | Extracellular Space | growth factor | -2.929 |
| polo-like kinase 2 (Drosophila) | PLK2 | 201939_at | Nucleus | kinase | 3.606 |
| potassium large conductance calcium-activated channel, subfamily M, alpha member 1 | KCNMA1 | 221584 _s_at | Plasma Membrane | ion channel | -2.389 |
| RELT-like 1 | RELL1 | 226430_at | unknown | other | 1.214 |
| ring finger protein 182 | RNF182 | 230720_at | unknown | other | 2.696 |
| seizure related 6 homolog-like | SEZ6L | 207873_x_at | Plasma Membrane | other | 0.866 |
| seizure related 6 homolog (mouse)-like | SEZ6L | 211894_x_at | Plasma Membrane | other | 0.797 |
| similar to hCG2031213 | LOC728052 | 1558795_at | unknown | other | 2.239 |
| single-minded homolog 1 (Drosophila) | SIM1 | 1556300_s_at | Nucleus | transcription regulator | 6.36 |
| single-minded homolog 1 (Drosophila) | SIM1 | 206876_at | Nucleus | transcription regulator | 5.737 |
| SLIT and NTRK-like family, member 5 | SLITRK5 | 214930_at | unknown | other | 4.094 |
| SMG1 homolog, phosphatidylinositol 3-kinase-related kinase pseudogene | LOC641298 | 244766_at | unknown | other | 1.065 |
| solute carrier family 44, member 5 | SLC44A5 | 1569112_at | unknown | other | 2.738 |
| solute carrier family 44, member 5 | SLC44A5 | 235763_at | unknown | other | 1.845 |
| solute carrier family 7 (cationic amino acid | SLC7A2 | 225516_at | Plasma Membrane | transporter | 1.892 |
| transporter, y+ system), member 2 | | | | | |
| solute carrier organic anion transporter family, member 3A1 | SLCO3A1 | 219229_at | Plasma Membrane | transporter | 1.622 |
| spermatogenesis associated, serine-rich 2-like | SPATS2L | 222154_s_at | unknown | other | 0.907 |
| synaptotagmin XIII | SYT13 | 226086_at | unknown | transporter | -4.754 |
| tissue factor pathway inhibitor 2 | TFPI2 | 209278_s_at | Extracellular Space | other | 2.326 |
| transcription factor 7-like 2 (T-cell specific, HMG-box) | TCF7L2 | 212761_at | Nucleus | transcription regulator | 1.005 |
| transformer 2 alpha homolog | TRA2A | 213593_s_at | Nucleus | other | 1.12 |
| transmembrane inner ear | TMIE | 1553601_a_at | unknown | other | -3.334 |
| transmembrane protein 178 | TMEM178 | 229302_at | unknown | other | 1.697 |
| U2 small nuclear RNA auxiliary factor 1 | U2AF1 | 232141_at | Nucleus | other | 0.776 |
| v-raf-1 murine leukemia viral oncogene homolog 1 | RAF1 | 1557675_at | Cytoplasm | kinase | 0.696 |
| zinc finger protein 217 | ZNF217 | 203739_at | Nucleus | transcription regulator | 1.096 |
| zinc finger protein 662 | ZNF662 | 228538_at | unknown | other | -3.352 |
| The log ratio represent log₂ values where 0.585 is log₂(1.5) which is a 1.5-fold difference, 1 is log₂(2) which is a 2-fold difference, 1.584 is log₂(3) which is a 3-fold difference, 2 is log₂(4) which is a 4-fold difference, 2.321 is log₂(5) which is a 5-fold difference, 2.584 is log₂(6) which is a 6-fold difference, 2.807 is log₂(7) which is a 7-fold difference, 3 is log₂(8) which is a 8-fold difference, 3.169 is log₂(9) which is a 9-fold difference, and 3.321 is log₂(10) which is a 10-fold difference. | | | | | |

### Example VI

### Immuno-Based Method to Detect Picomolar Amounts of BoNT/A

The following example illustrates how to perform immuno-based methods of detecting BoNT/A activity that can detect picomolar amounts of the BoNT/A pharmaceutical product, such as, e.g., BOTOX® DYSPORT®/RELOXIN®, PURTOX®, XEOMIN®, NEURONOX®, or BTX-A.

### 1. Immuno-based method of detecting BoNT/A using ECL sandwich ELISA.

To prepare a lysate from cells treated with a BoNT/A, approximately 150,000 cells from an established clonal cell line was plated into the wells of 96-well tissue culture poly-D-lysine plates containing 100 µL of a serum-free medium containing Minimum Essential Medium, 2 mM GlutaMAX™ I with Earle's salts, 1 x B27 supplement, 1 x N2 supplement, 0.1 mM Non-Essential Amino Acids, 10 mM HEPES and 60 µg/mL GT1b. The cell lines used were BB10, H1, and SiMa. These cells were incubated in a 37 °C incubator under 5% carbon dioxide until the cells differentiated, as assessed by standard and routine morphological criteria, such as growth arrest and neurite extension (approximately 3 days). The media from the differentiated cells was aspirated from each well and replaced with fresh media containing one of the following: 1) SiMa cell line, 0 (untreated), 0.98 U/mL, 1.9 U/mL, 3.91 U/mL, 7.81 U/mL, 15.6 U/mL, 31.3 U/mL, 62.5 U/mL, 125 U/mL, 250 U/mL, or 500 U/mL of a BoNT/A pharmaceutical product reconstituted in a sodium chloride free medium; 2) BB10, 0 (untreated), 0.49 U/mL, 0.98 U/mL, 1.9 U/mL, 3.91 U/mL, 7.81 U/mL, 15.6 U/mL, 31.3 U/mL, 62.5 U/mL, 125 U/mL, 250 U/mL; 3) H1, 0 (untreated), 0.3 U/mL, 1 U/mL, 3 U/mL, 9 U/mL, 28 U/mL, 83 U/mL, and 250 U/mL. Because the BoNT/A pharmaceutical product contains sodium chloride, its addition to the culture medium resulted in a hypertonic media that was detrimental to cell viability. To circumvent the hypertonicity issue, 200 µL of MEM media made without sodium chloride was used to reconstitute the BoNT/A pharmaceutical product giving a final concentration of 25 pM BoNT/A (500 U/mL). The matrix was kept constant for all concentrations along the dose-response curve by adding sodium chloride in the media used to make the dilutions match the amount of excipients present at the highest concentration used (25 pM or 500 U/mL). After a 24 hr treatment, the cells were washed, and incubated for an additional two days without toxin. To harvest the cells, 1 x PBS was aspirated, the cells lysed by adding 75 µL of Lysis Buffer comprising 50 mM HEPES, 150 mM NaCl, 1.5 mM MgCl₂, 1 mM EGTA, 1% Triton X-100 to each well, and the plate incubated on a shaker rotating at 500 rpm for 30 minutes at 4 °C. The plate was centrifuged at 4000 rpm for 20 minutes at 4 °C to pellet cellular debris and the supernatant was transferred to a capture antibody coated 96-well plate to perform the detection step.

To prepare the α-SNAP-25 capture antibody solution, the α-SNAP-25 monoclonal antibody contained in the ascites from hybridoma cell line 2E2A6 (Example VII) was purified using a standard Protein A purification protocol. To prepare the α-SNAP-25 detection antibody solution, α-SNAP-25 rabbit polyclonal antibody S9684 (Sigma, St. Louis, MO) was conjugated to Ruthenium(II)-tris-bipyridine-(4-methysulfonate) NHS ester labeling reagent (Meso Scale Discovery, Gaithersburg, MD) according to the manufacturer's instructions (Meso Scale Discovery, Gaithersburg, MD). The conjugation reaction was performed by adding 30 µL of distilled water reconstituted MSD SULFO-TAG™ stock solution to 200 µL of 2 mg/mL α-SNAP-25 polyclonal antibodies and incubating the reaction at room temperature for 2 hours in the dark. The labeled antibodies were purified using a standard spin column protocol and the protein concentration determined using a standard colorimetric protein assay. The absorbance of the α-SNAP-25 antibody/MSD SULFO-TAG™ conjugate was measured at 455 nm using a spectrophotometer to determine the concentration in moles per liter. The detection antibody solution was stored at 4 °C until needed.

To prepare the solid phase support comprising the capture antibody that is specific for a SNAP-25 cleaved product, approximately 1 µL of α-SNAP-25 monoclonal antibody 2E2A6 solution (45 µg/mL in 1 x PBS, 750 µg/mL of BSA) was added to each well of a 96-well MSD High Bind plate (Meso Scale Discovery, Gaithersburg, MD) and the solution is allowed to air dry in a biological safety cabinet for 2-3 hours in order to liquid evaporate the solution, and then the plates were sealed and stored at 4 °C until needed. The dried capture antibody-bound wells were then blocked by adding 150 µL of Blocking Buffer comprising 1 x PBS, 0.05% Tween-20, 2 % ECL Blocking reagent (GE Healthcare-Amersham), and 1 % goat serum (Rockland Immunochemicals, Gilbertsville, PA) at room temperature for 2 hours with rotation at 600 rpm.

To detect the presence of a SNAP-25 cleavage product by ECL sandwich ELISA analysis, the Blocking Buffer from plates was aspirated, 25 µL of a lysate from cells treated with BoNT/A was added to each well and the plates were incubated at 4 °C for overnight. Plate wells were washed three times by aspirating the cell lysate and rinsing each well three times with 200 µL 1 x PBS, 0.1% TWEEN-20® (polyoxyethylene (20) sorbitan monolaureate). After washing, 25 µl of 5 pg/mL α-SNAP-25 detection antibody solution comprising 2% Amersham Blocking Reagent in 1 x PBS, 0.1% TWEEN-20® (polyoxyethylene (20) sorbitan monolaureate) was added to each well, the plate was sealed, and the sealed plate was incubated at room temperature for 1 hour with shaking. After α-SNAP-25 detection antibody incubation, the wells were washed three times with 250 µL 1 x PBS, 0.1% TWEEN-20® (polyoxyethylene (20) sorbitan monolaureate). After washing 150 µL of 1 x Read Buffer (Meso Scale Discovery, Gaithersburg, MD) was added to each well and the plates were read using a SECTOR™ Imager 6000 Image Reader (Meso Scale Discovery, Gaithersburg, MD). The raw data was then transferred to SigmaPlot v 10.0 and a 4-parameter logistics fit was used to define the dose-response curves. There were no constraints used for the 4-parameter logistic function when plotting the data. Graphical reports were generated using the following analysis: R2 (correlation coefficient), a (Max for data set), b (hillslope), and X0 ± SE (EC₅₀ value ± standard error).

These results indicated that on average the cells comprising the BB10 and H1 clonal cell lines were more susceptible to BoNT/A intoxication as compared to cells comprising the parental SiMa cell line. Cells from a BB10 cell line exhibited an EC₅₀ for BoNT/A activity of 7 U/mL, cells from a H1 cell line exhibited an EC₅₀ for BoNT/A activity of 8 U/mL, and cells from the parental SiMa cell line exhibited an EC₅₀ for BoNT/A activity of 13 U/mL. This method can also be performed in a multiplex fashion as described in Ester Fernandez-Salas, et al., *Immuno-Based Botulinum Toxin Serotype A Activity Assays,* U.S. Patent Application Serial No: 12/403,531.

### Example VII

### Transfection of BoNT/A receptor DNA into sensitive clonal cell lines further improves sensitivity of the clonal cell line for BoNT/A

The following example illustrates how to introduce a polynucleotide molecule encoding a BoNT/A receptor into cells from a clonal cell line to further improve susceptibility to BoNT/A intoxication or improve BoNT/A uptake capacity.

To introduce an exogenous BoNT/A receptor into cells comprising an established clonal cell line, a mammalian expression construct comprising a polynucleotide molecule of SEQ ID NO: 139 encoding the FGFR3 of SEQ ID NO: 25, was transfected into cells from a H1 clonal cell line by a cationic lipid method. A suitable density (about 5 x10⁶ cells) of cells from an established clonal cell line are plated in a 100 mm tissue culture dish containing 5 mL of complete culture media and grown in a 37 °C incubator under 5% carbon dioxide until the cells reached a density appropriate for transfection. A 3 mL transfection solution is prepared by adding 1.5 mL of OPTI-MEM® Reduced Serum Medium containing 60 µL of LIPOFECTAMINE® 2000 (Invitrogen, Carlsbad, CA) incubated at room temperature for 5 minutes to 1.5 mL of OPTI-MEM® Reduced Serum Medium containing 24 µg of an expression construct encoding the FGFR3 of SEQ ID NO: 25. This transfection mixture is incubated at room temperature for approximately 30 minutes. The complete media on the cells is replaced with the 3 mL transfection solution and the cells are incubated in a 37 °C incubator under 5% carbon dioxide for approximately 8 hours. Transfection media is replaced with 3 mL of fresh complete culture media and the cells are incubated in a 37 °C incubator under 5% carbon dioxide for approximately 24 hours. Media is replaced with 3 mL of fresh complete culture media containing approximately 1mM G418 (Invitrogen, Carlsbad, CA) for selection of transfected cells. Cells are incubated in a 37 °C incubator under 5% carbon dioxide for approximately 1 week, the old media is replaced with fresh complete culture media containing 0.5 mM G418 and the cells are plated onto one 96-well collagen IV coated plate at a plating density of 0.5 cells per well. Cell growth and viability was monitored by microscopic examination and after about 3 weeks single clones appeared in about 20% of the wells. These clones were grown for an additional 2 weeks and then transferred to duplicate 24-well collagen IV coated plates, one to maintain the clonal cell line and the other to test for BoNT/A susceptibility.

To determine the susceptibility of the cells comprising the clonal cell lines overexpressing a BoNT/A receptor, a Western blot analysis was done to determine sensitivity and an ECL sandwich ELISA was done to determine an EC₅₀ value. A total of eight clones were tested for the FGFR3 stably transfected H1 cells.

To prepare a cell lysate for the Western blot analysis, cells from the clonal cell line were plated into two wells in a poly-D-lysine coated 96-well plate in serum-free medium containing Minimum Essential Medium, 2 mM GLUTAMAX™ I with Earle's salts, 1 x B27 supplement, 1 x N2 supplement, 0.1 mM Non-Essential Amino Acids, 10 mM HEPES and 25 µg/mL GT1b (Alexis Biochemicals, Lausen, Switzerland). These cells were incubated in a 37 °C incubator under 5% carbon dioxide until the cells differentiated, as assessed by standard and routine morphological criteria, such as growth arrest and neurite extension (approximately 3 days). The media from the differentiated cells was aspirated from each well and replaced with fresh media containing either 0 (untreated), 0.5 nM or 0.25 nM of a BoNT/A complex. After a 6 hr treatment, the cells were washed, and incubated for an additional 18 hr without toxin. After the incubation, the cells were washed by aspirating the growth media and rinsing each well with 200 µl of 1 x PBS. To harvest the cells, the 1 x PBS was aspirated, the cells were lysed by adding 40 µL of 2 x SDS Loading Buffer and the plate was swirled for an even coating of Loading Buffer. The lysate was transferred to a new 96-well PCR plate (Axygen, Union City, CA) and the plate was heated to 95 °C for 5 min to complete cell lysis and denature proteins.

To detect for the presence of both uncleaved SNAP-25 substrate and cleaved SNAP-25 products, an aliquot from each harvested sample was analyzed by Western blot. In this analysis, a 12 µL aliquot of the harvested sample was separated by MOPS polyacrylamide gel electrophoresis using NUPAGE® Novex 12% Bis-Tris precast polyacrylamide gels (Invitrogen Inc., Carlsbad, CA) under denaturing, reducing conditions. Separated proteins were transferred from the gel onto nitrocellulose membranes (Bio-Rad Laboratories, Hercules, CA) by Western blotting using a "Transfer +4 cassettes & Cooler" transfer chamber (GE Healthcare, Piscataway, NJ). Membranes were blocked by incubating at room temperature for 1 hr with gentle agitation in a solution containing Tris-Buffered Saline (TBS) (25 mM 2-amino-2-hydroxymethyl-1,3-propanediol hydrochloric acid (Tris-HCl)(pH 7.4), 137 mM sodium chloride, 2.7 mM potassium chloride), 0.1% TWEEN-20® (polyoxyethylene (20) sorbitan monolaureate), 2% Bovine Serum Albumin (BSA), 5% nonfat dry milk. Blocked membranes were incubated at 4 °C for overnight in TBS, 0.1% TWEEN-20® (polyoxyethylene (20) sorbitan monolaureate), 2% BSA, and 5% nonfat dry milk containing a 1:5,000 dilution of α-SNAP-25 rabbit polyclonal antiserum S9684 (Sigma, St. Louis, MO) as the primary antibody. The α-SNAP-25 rabbit polyclonal antibodies can detect both the uncleaved SNAP-25 substrate and the SNAP-25 cleavage product, allowing for the assessment of overall SNAP-25 expression in each cell line and the percent of SNAP-25 cleaved after BoNT/A treatment as a parameter to assess the amount of BoNT/A uptake. Primary antibody probed blots were washed three times for 5 minutes each time in TBS, TWEEN-20® (polyoxyethylene (20) sorbitan monolaureate). Washed membranes were incubated at room temperature for 2 hours in TBS, 0.1% TWEEN-20® (polyoxyethylene (20) sorbitan monolaureate), 2% BSA, and 5% nonfat dry milk containing a 1:10,000 dilution of goat polyclonal anti-rabbit immunoglobulin G, heavy and light chains (IgG, H+L) antibody conjugated to horseradish peroxidase (Invitrogen, Inc., Carlsbad, CA) as a secondary antibody. Secondary antibody-probed blots were washed five times for 5 minutes each time in TBS, 0.1% TWEEN-20® (polyoxyethylene (20) sorbitan monolaureate). Signal detection of the labeled SNAP-25 products were visualized using the ECL Plus™ Western Blot Detection System (GE Healthcare, Amersham Biosciences, Piscataway, NJ) and the membrane was imaged and the percent of cleaved SNAP-25 products quantified with a Typhoon 9410 Variable Mode Imager and Imager Analysis software (GE Healthcare, Amersham Biosciences, Piscataway, NJ). The choice of pixel size (100 to 200 pixels) and PMT voltage settings (350 to 600, normally 400) depended on the individual blot. The volumes of the bands corresponding to either intact and cleaved SNAP-25 product were quantified using Image Quant TL and the total amount of SNAP-25 and the percentage of SNAP-25 cleaved were calculated based on the intensity of these two SNAP-25 bands.

Clone H1 1.4 had a high percentage of SNAP-25 cleavage and high levels of total SNAP25 and was selected for further testing. The selected clone was first grown on a 10 cm² dish and then in T175 flasks to ensure that enough cells were produced for both ECL sandwich ELISA assay and for freezing stocks. Frozen stocks were made with 1 x 10⁶ cells and 1 mL of Recovery Cell Culture Freezing Media (GIBCO-Invitrogen, #12648-010 Freezing media).

To prepare a cell lysate for the ECL sandwich ELISA, approximately 50,000 cells from H1 clonal cell line and from H1 1.4 clonal cell line overexpressing FGFR3 were plated and differentiated as described above. The media from the differentiated cells was aspirated from each well and replaced with fresh media containing either (untreated), 0.27 pM, 0.82 pM, 2.47 pM, 7.4 pM, 22.2 pM, 66.6 pM, or 200 pM of a BoNT/A complex of a BoNT/A complex. After a 6 hr treatment, the cells were washed, and incubated overnight without toxin. After a 24 hr treatment, the cells were washed by aspirating the BoNT/A containing media and rinsing each well with 200 µL of 1 x PBS. Washed cells were harvested by lysing in freshly prepared Triton X-100 Lysis Buffer (150 mM NaCl, 20 mM Tris-HCl (pH 7.5), 1 mM EDTA, 1 mM EGTA, 1% Triton X-100 plus protease inhibitors) at 4°C for 30 minutes with constant agitation. Lysed cells were centrifuged at 4000 rpm for 20 min at 4°C to pellet debris.

The α-SNAP-25 capture antibody solution, the α-SNAP-25 detection antibody solution, and the solid phase support comprising the capture antibody that is specific for a SNAP-25 cleaved product were prepared as described in Example V. To detect the presence of a cleaved SNAP-25 product by ECL sandwich ELISA, the plates were processed, and the data collected and analyzed, and the EC₅₀ calculated as described in Example VI. The EC₅₀ for H1 1.4 cells was estimated to about 1.7 pM, compared to an EC₅₀ about 8.7 pM for H1 cells indicating that the newly generated H1 1.4 cell line is about 5 times more sensitive than the H1 cell line for BoNT/A uptake (average of two independent experiments with similar results). The maximum signal for both cell lines was identical indicating similar efficacy towards SNAP25 cleavage. The increase in sensitivity is especially prominent at lower concentrations of BoNT/A, where the H1 1.4 cells are many times more sensitive to BoNT/A compared to H1 cells producing a very robust signal over background at the 0.27 pM concentration. Over-expression of FGFR3 was verified in a Western blot using α-FGFR3 antibodies sc-123 (Santa Cruz Biotechnologies, Santa Cruz, CA).

### Example VIII

### Development of α-SNAP-25 Monoclonal Antibodies that Selectively Bind a SNAP-25 Epitope Having a Free Carboxyl-terminus at the P₁ Residue of the BoNT/A Cleavage Site Scissile Bond

The following example illustrates how to make α-SNAP-25 monoclonal antibodies that can selectively bind to a SNAP-25 epitope having a carboxyl-terminus at the P₁ residue of the BoNT/A cleavage site scissile bond.

### 1. Generation of α-SNAP-25 monoclonal antibodies.

α-SNAP-25 monoclonal antibodies with higher binding affinity for a SNAP-25 cleavage product and/or α-SNAP-25 antibodies with a higher binding specificity for a SNAP-25 cleavage product were developed. In addition, because a permanent, stable and renewable source of α-SNAP-25 antibodies is preferable for an FDA-approved assay, screens to isolate monoclonal antibodies were undertaken. To develop monoclonal α-SNAP-25 antibodies the 13-residue peptide CDSNKTRIDEANQ_{COOH} (SEQ ID NO: 38) was designed as a SNAP-25 antigen having a carboxyl-terminus at the P₁ residue of the BoNT/A cleavage site scissile bond. This peptide comprises a flexible linker region and a N-terminal Cysteine residue for conjugation to KLH and amino acids 186-197 of human SNAP-25 (SEQ ID NO: 5) with a carboxylated C-terminal glutamine (SEQ ID NO: 38). The generation of monoclonal antibodies to well-chosen, unique peptide sequences provides control over epitope specificity, allowing the identification of a particular subpopulation of protein among a pool of closely related isoforms. Blast searches revealed that this peptide has high homology only to SNAP-25 and almost no possible cross-reactivity with other proteins in neuronal cells. The sequence was also carefully scrutinized by utilizing computer algorithms to determine hydropathy index, protein surface probability, regions of flexibility, and favorable secondary structure, followed by proper orientation and presentation of the chosen peptide sequence. The peptide was synthesized and conjugated to Keyhole Limpet Hemocyanin (KLH) to increase immunogenicity. Six Balb/c mice were immunized with this peptide, and after three immunizations in about eight weeks, the mice were bled for testing. The blood was allowed to clot by incubating at 4 °C for 60 minutes. The clotted blood was centrifuged at 10,000x g at 4 °C for 10 minutes to pellet the cellular debris. The resulting serum sample was dispensed into 50 µL aliquots and stored at -20 °C until needed.

A similar strategy based on other SNAP-25 antigens disclosed in the present specification is used to develop α-SNAP-25 monoclonal antibodies that can selectively bind to a SNAP-25 having a carboxyl-terminus at the P₁ residue of the BoNT/A cleavage site scissile bond. For example, the SNAP-25 antigen of SEQ ID NO: 45 can be conjugated to KLH instead of the SNAP-25 antigen of SEQ ID NO: 38. As another example, the amino acids 186-197 of human SNAP-25 from the SNAP-25 antigen of SEQ ID NO: 38 can be replaced with SEQ ID NO: 32, SEQ ID NO: 33, SEQ ID NO: 34, SEQ ID NO: 35, SEQ ID NO: 36, SEQ ID NO: 39, SEQ ID NO: 40, SEQ ID NO: 41, SEQ ID NO: 42, SEQ ID NO: 43, SEQ ID NO: 44, SEQ ID NO: 147, or SEQ ID NO: 148.

### 2. Screening for the presence of α-SNAP-25 monoclonal antibodies.

To determine the presence of an α-SNAP-25 monoclonal antibody that can selectively bind to a SNAP-25 antigen having a carboxyl-terminus at the P₁ residue of the BoNT/A cleavage site scissile bond, comparative ELISA and cell-based cleavage assay were performed using the extracted mouse serum. For comparative ELISA, two fusion proteins were constructed: BIRA-HIS TAG®-SNAP-25₁₃₄₋₁₉₇ of SEQ ID NO: 48 and the BIRA-HIS TAG®-SNAP-25₁₃₄₋₂₀₆ of SEQ ID NO: 49. BIRA-HIS TAG®-SNAP-25₁₃₄₋₁₉₇ comprised a naturally-biotinylated 16 amino acid BirA peptide of SEQ ID NO: 50 amino-terminally linked to a SNAP-25 peptide comprising amino acids 134-197 of SEQ ID NO: 5. BIRA-HIS TAG®-SNAP-25₁₃₄₋₂₀₆ comprised a naturally-biotinylated 16 amino acid BirA peptide of SEQ ID NO: 50 amino-terminally linked to a SNAP-25 peptide comprising amino acids 134-206 of SEQ ID NO: 5. These two substrates were suspended in 1 x PBS at a concentration of 10 µg/mL BIRA-HIS TAG®-SNAP-25₁₃₄₋₁₉₇ and the BIRA-HIS TAG®-SNAP-25₁₃₄₋₂₀₆. The BIRA-HIS TAG®-SNAP-25₁₃₄₋₁₉₇ and the BIRA-HIS TAG®-SNAP-25₁₃₄₋₂₀₆ were coated onto separate plates by adding approximately 100 µl of the appropriate Substrate Solution and incubating the plates at room temperature for one hour. Washed plates were incubated at 37 °C for one hour in 0.5% BSA in 1 x TBS containing a 1:10 to 1:100 dilution of an antibody-containing serum derived from one of the six immunized mice (Mouse 1, Mouse 2, Mouse 3, Mouse 4, Mouse 5, and Mouse 6). Primary antibody probed plates were washed four times for 5 minutes each time in 200 µl TBS, 0.1% TWEEN-20® (polyoxyethylene (20) sorbitan monolaureate). Washed plates were incubated at 37 °C for 1 hour in 1 x TBS containing a 1:10,000 dilution of goat polyclonal anti-mouse IgG antibody conjugated to Horseradish peroxidase (Pierce Biotechnology, Rockford, IL) as a secondary antibody. Secondary antibody-probed plates were washed four times in 200 µl TBS, 0.1% TWEEN-20® (polyoxyethylene (20) sorbitan monolaureate). Chromogenic detection of the labeled SNAP-25 products were visualized by chromogenic detection using ImmunoPure TMB substrate kit (Pierce Biotechnology, Rockford, IL). The development of a yellow color in the BIRA-HIS TAG®-SNAP-25₁₃₄₋₁₉₇ coated plates, but not the BIRA-HIS TAG®-SNAP-25₁₃₄₋₂₀₆ coated plates, indicated that the α-SNAP-25 antibody preferentially recognized the SNAP-25₁₉₇ cleavage product. The resulted indicated that of the six mice used for immunization, three mice (Mouse 2, Mouse 3, and Mouse 4) had higher titers and more specificity towards a SNAP-25 antigen having a carboxyl-terminus at the P₁ residue of the BoNT/A cleavage site scissile bond.

These results were confirmed using an ELISA light chain activity assay. A 96-well Reacti-Bind Streptavidin coated plates (Pierce Biotechnology, Rockford, IL) were prepared by adding approximately 100 µl of the following Substrate Solution: Rows A-C were coated with 100 µL of BIRA-HIS TAG®-SNAP-25₁₃₄₋₁₉₇ at twelve different concentrations; Rows D-H were coated with 100 µL of BIRA-HIS TAG®-SNAP-25₁₃₄₋₂₀₆ at 10 µg/mL. The plates were washed by aspirating the Substrate Solution and rinsing each well three times with 200 µl TBS, 0.1% TWEEN-20® (polyoxyethylene (20) sorbitan monolaureate). Dilutions of BoNT/A were pre-reduced at 37 °C for 20 minutes in BoNT/A Incubation Buffer (50 mM HEPES, pH 7.4, 1% fetal bovine serum, 10 µM ZnCl₂, 10 mM dithiothrietol) and 100 µl of the pre-reduced BoNT/A was added to the substrate-coated plates and incubated at 37 °C for 90 minutes. BoNT/A treated plates were washed by aspirating the BoNT/A Incubation Buffer and rinsing each plate three times with 200 µl TBS, 0.1% TWEEN-20® (polyoxyethylene (20) sorbitan monolaureate). Washed plates were incubated at 37 °C for one hour in 0.5% BSA in 1 x TBS containing a 1:10 to 1:100 dilution of the antibody-containing serum being tested. Primary antibody probed plates were washed four times for 5 minutes each time in 200 µl TBS, 0.1% TWEEN-20® (polyoxyethylene (20) sorbitan monolaureate). Washed plates were incubated at 37 °C for 1 hour in 1 x TBS containing a 1:10,000 dilution of goat polyclonal anti-mouse IgG antibody conjugated to Horseradish peroxidase (Pierce Biotechnology, Rockford, IL) as a secondary antibody. Secondary antibody-probed plates were washed four times in 200 µl TBS, 0.1% TWEEN-20® (polyoxyethylene (20) sorbitan monolaureate). Chromogenic detection of the labeled SNAP-25 products were visualized by chromogenic detection using ImmunoPure TMB substrate kit (Pierce Biotechnology, Rockford, IL). The development of a yellow color, which correlated with the presence of the SNAP-25₁₉₇ cleavage product was detected in BoNT/A treated samples, but not untreated controls, using antibody-containing serum derived from all six immunized mice (Mouse 1, Mouse 2, Mouse 3, Mouse 4, Mouse 5, and Mouse 6). Thus, the comparative ELISA analysis indicated that of the mice used for immunization, three mice (Mouse 2, Mouse 3, and Mouse 4) had higher titers and more specificity towards a SNAP-25 antigen having a carboxyl-terminus at the P₁ residue of the BoNT/A cleavage site scissile bond.

For cell-based cleavage assay, a suitable density of PC12 cells were plated into 60 mm² tissue culture plates containing 3 mL of an appropriate serum medium (Table 1). The cells were grown in a 37 °C incubator under 5% carbon dioxide until cells reached the appropriate density. A 500 µL transfection solution was prepared by adding 250 µL of OPTI-MEM® Reduced Serum Medium containing 15 µL of LIPOFECTAINE® 2000 (Invitrogen Inc., Carlsbad, CA) incubated at room temperature for 5 minutes to 250 µL of OPTI-MEM® Reduced Serum Medium containing 10 µg of a pQBI-25/GFP-BoNT/A-LC expression construct (SEQ ID NO: 51). The pQBI-25/GFP-BoNT/A-LC expression construct comprises a pQBI-25 expression vector (Qbiogene Inc., Carlsbad, CA) whose promoter elements are functionally linked to a polynucleotide encoding the GFP-BoNT/A light chain of SEQ ID NO: 52. This transfection mixture was incubated at room temperature for approximately 20 minutes. The media was replaced with fresh unsupplemented media and the 500 µL transfection solution was added to the cells. The cells were then incubated in a 37 °C incubator under 5% carbon dioxide for approximately 6 to 18 hours. The cells were washed and harvested as described in Example II. To detect for the presence of the cleaved SNAP-25₁₉₇ product, an aliquot from each harvested sample was analyzed by Western blot as described in Example II, except that the primary antibody used was a 1:1,000 dilution of the antibody-containing serum and the secondary antibody used was a 1:20,000 of mouse α-IgG Horseradish Peroxidase (Pierce Biotechnology, Rockford, IL). A single band corresponding to the SNAP-25₁₉₇ cleavage product was detected in BoNT/A treated samples, but not untreated controls, using antibody-containing serum derived from three mice (Mouse 2, Mouse 3, and Mouse 4). Thus, the cell-based cleavage assay indicated that of the mice used for immunization, three mice (Mouse 2, Mouse 3, and Mouse 4) had higher titers and more specificity towards a SNAP-25 antigen having a carboxyl-terminus at the P₁ residue of the BoNT/A cleavage site scissile bond.

### 3. Production of hybridomas.

To make hybridomas producing α-SNAP-25 monoclonal antibodies that can selectively bind to a SNAP-25 antigen having a carboxyl-terminus at the P₁ residue of the BoNT/A cleavage site scissile bond, the spleen from Mouse 2 was harvested three days subsequent to a final "booster" immunization and the spleen cells were fused with myeloma cells P3-X63 Ag8.653 using standard hybridoma protocols. These cells were plated into five 96-well plates and hybrids were selected using HAT medium. Within 8-14 days after fusion, the first screening of the approximately 480 parent clones was carried out using comparative ELISA with the BIRA-HIS TAG®-SNAP-25₁₃₄₋₁₉₇ and the BIRA-HIS TAG®-SNAP-25₁₃₄₋₂₀₆ peptides coated in two separate plates. The comparative ELISA provided a quick screen method to identify hybridomas producing antibodies specific for the cleaved SNAP-25₁₉₇. The top 18 clones were subjected to further screening using the cell-based cleavage assay described above and immunostaining of LC/A transfected cells. (Table 18).

| **Table 18. Analysis of Supernatants Containing α-SNAP-25 Monoclonal Antibody** | | | | | | |
|---|---|---|---|---|---|---|
| **Clone** | **Comparative ELISA** | | | | **Cell-Based Assay** | |
| | **OD SNAP-25₁₉₇** | **OD SNAP-25₂₀₆** | **Ratio_{197/206}** | **Ratio_{206/197}** | **SNAP-25₁₉₇** | **SNAP-25₂₀₆** |
| 1D3 | 1.805 | 0.225 | 8.02 | 0.13 | +++ | - |
| 1F12 | 0.365 | 0.093 | 3.92 | 0.25 | - | - |
| 1G10 | 0.590 | 0.137 | 4.31 | 0.23 | ++ | - |
| 1H1 | 0.335 | 0.121 | 2.77 | 0.36 | - | - |
| 1H8 | 0.310 | 0.302 | 1.03 | 0.97 | + | - |
| 2C9 | 0.139 | 0.274 | 0.51 | 1.97 | - | - |
| 2E2 | 0.892 | 0.036 | 24.78 | 0.04 | ++ | - |
| 2E4 | 0.228 | 0.069 | 3.30 | 0.30 | + | - |
| 2F11 | 1.095 | 1.781 | 0.61 | 1.63 | - | - |
| 3C1 | 1.268 | 0.053 | 23.92 | 0.04 | ++ | - |
| 3C3 | 0.809 | 0.052 | 15.56 | 0.06 | ++ | - |
| 3E1 | 0.086 | 0.155 | 0.55 | 1.80 | 0 | - |
| 3E8 | 2.048 | 0.053 | 38.64 | 0.03 | +++ | - |
| 3G2 | 0.053 | 0.158 | 0.34 | 2.98 | - | - |
| 4D1 | 0.106 | 0.218 | 0.49 | 2.06 | - | - |
| 4G6 | 0.061 | 0.159 | 0.38 | 2.61 | - | - |
| 5A5 | 0.251 | 0.106 | 2.37 | 0.42 | + | - |
| 5F11 | 0.243 | 0.061 | 3.98 | 0.25 | - | - |

Clones 1D3, 1G10, 2E2, 3C1, 3C3, and 3E8 were further cloned by limiting dilution because the conditioned media produced by these clones comprised α-SNAP-25 antibodies with a preferential binding specificity having a ratio_{197/206} of at least 4:1 for the SNAP-25₁₉₇ cleavage product relative to the SNAP-25₂₀₆ uncleaved substrate and detected the SNAP-25₁₉₇-cleavage product using the cell-based cleavage assay and the immunostaining of PC12 cells transfected with GFP-LC/A. Similarly clones 2C9, 2F11, 3G2, 4D1 and 4G6 were further cloned by limiting dilution because the conditioned media produced by these clones comprised α-SNAP-25 antibodies with a preferential binding specificity having a ratio_{206/197} of at least 1.5:1 for the SNAP-25₂₀₆ uncleaved substrate relative to the SNAP-25₁₉₇ cleavage product and detected the SNAP-25₂₀₆-uncleaved substrate using the cell-based cleavage assay. These single-cell derived clones were screened again using comparative ELISA, cell-based cleavage, and immunostaining to confirm their affinity and specificity, and the antibodies were isotyped using standard procedures. Ascites were produced from clones 1D3B8 (IgM.k), 1G10A12 (IgG3.k), 2C9B10 (IgG3.k), 2E2A6 (IgG3.k), 2F11B6 (IgM.k), 3C1A5 (IgG2a.k), and 3C3E2 (IgG2a.k). Clone 3E8 stopped producing antibodies during the cloning process and could not be further evaluated.

### 4. Evaluation of binding specificity of α-SNAP-25 monoclonal antibodies.

To evaluate binding specificity of an α-SNAP-25 monoclonal antibody that can selectively bind to a SNAP-25 antigen having a carboxyl-terminus at the P₁ residue of the BoNT/A cleavage site scissile bond, ascites from clones 1D3B8, 1G10A12, 2C9B10, 2E2A6, 2F11B6, 3C1A5, and 3C3E2 were used to detect SNAP-25 cleavage product using the cell-based activity assay, immunocytochemistry and immunoprecipitation.

For the cell-based activity assay, binding specificity was determined by analyzing the ability of α-SNAP-25 antibody-containing ascites to detect the uncleaved SNAP-25₂₀₆ substrate and the cleaved SNAP-25₁₉₇ product by Western blot analysis. A suitable density of PC12 cells were plated into 60 mm² tissue culture plates containing 3 mL of an appropriate serum medium, grown in a 37 °C incubator under 5% carbon dioxide until an appropriate cell density was reached, and transfected with the either a transfection solution lacking the pQBI-25/GFP-BoNT/A-LC expression construct (untransfected cells) or a transfection solution containing the pQBI-25/GFP-BoNT/A-LC expression construct (transfected cells) as described above. The cells were washed and harvested as described in Example I. To detect for the presence of both the uncleaved SNAP-25₂₀₆ substrate and the cleaved SNAP-25₁₉₇ product, an aliquot from each harvested sample was analyzed by Western blot as described in Example I, except that the primary antibody used was a 1:100 dilution of the α-SNAP-25 monoclonal antibody-containing ascites and the secondary antibody used was a 1:20,000 of α-mouse IgG conjugated to Horseradish Peroxidase (Pierce Biotechnology, Rockford, IL). In addition, three commercially available mouse α-SNAP-25 monoclonal antibodies were tested. SMI-81 (Sternberger Monoclonals Inc., Lutherville, MD), an α-SNAP-25 antibody the manufacturer indicates detects both the uncleaved SNAP-25₂₀₆ substrate and the cleaved SNAP-25₁₉₇ product, was used at a 15,000 dilution according to the manufacturer's recommendations. MC-6050 (Research & Diagnostic Antibodies, Las Vegas, NV), an α-SNAP-25 antibody the manufacturer indicates detects both the uncleaved SNAP-25₂₀₆ substrate and the cleaved SNAP-25₁₉₇ product, was used at a 1:100 dilution according to the manufacturer's recommendations. MC-6053 (Research & Diagnostic Antibodies, Las Vegas, NV), an α-SNAP-25 antibody the manufacturer indicates detects only the cleaved SNAP-25₁₉₇ product, was used at a 1:100 dilution according to the manufacturer's recommendations.

Table 19 indicates the α-SNAP-25 antibody-containing ascites that detected only the SNAP-25₁₉₇ cleavage product. The cell-based cleavage assay indicated that ascites produced from clones 1D3B8, 2C9B10, 2E2A6, 3C1A5, and 3C3E2 synthesize an α-SNAP-25 monoclonal antibody having high binding specificity for the SNAP-25₁₉₇ cleavage product that allows for the selective recognition of this cleavage product relative to the SNAP-25₂₀₆ uncleaved substrate. Commercial antibody SMI-81 detected the SNAP-25₂₀₆ uncleaved substrate, but only poorly recognized the SNAP-25₁₉₇ cleavage product (Table 19). Surprisingly, commercial antibody MC-6050 only detected the SNAP-25₂₀₆ uncleaved substrate, and failed to recognize the SNAP-25₁₉₇ cleavage product (Table 19). Even more surprisingly, commercial antibody MC-6050 only detected the SNAP-25₂₀₆ uncleaved substrate, and failed to recognize the SNAP-25₁₉₇ cleavage product, even though the manufacturer advertises that this antibody selectively detects the SNAP-25₁₉₇ cleavage product (Table 19). Thus, this analysis indicates that while 1D3B8, 2C9B10, 2E2A6, 3C1A5, and 3C3E2 exhibit suitable selectivity for the SNAP-25₁₉₇ cleavage product, 1G10A12 and 2F11B6 do not. In addition, commercial antibodies SMI-81, MC-6050 and MC-6053 all are unsuitable for the immuno-based methods disclosed in the present application because all failed to selectivity detect the SNAP-25₁₉₇ cleavage product.

For immunocytochemistry analysis, binding specificity was determined by analyzing the ability of α-SNAP-25 antibody-containing ascites to detect the uncleaved SNAP-25₂₀₆ substrate and the cleaved SNAP-25₁₉₇ product by immunostaining. *See e.g.,* Ester Fernandez-Salas et al., Plasma Membrane Localization Signals in the Light Chain of Botulinum Neurotoxin, Proc. Natl. Acad. Sci., U.S.A. 101(9): 3208-3213 (2004). A suitable density of PC12 cells were plated, grown, and transfected with either a transfection solution lacking the pQBI-25/GFP-BoNT/A-LC expression construct (untransfected cells) or a transfection solution containing the pQBI-25/GFP-BoNT/A-LC expression construct (transfected cells) as described above. The cells were washed in 1 x PBS and fixed in 5 mL of PAF at room temperature for 30 minutes. Fixed cells were washed in phosphate buffered saline, incubated in 5 mL of 0.5% Triton® X-100 (polyethylene glycol octylphenol ether) in 1 x PBS, washed in 1 x PBS, and permeabilized in 5 mL of methanol at -20 °C for six minutes. Permeabilized cells were blocked in 5 mL of 100 mM glycine at room temperature for 30 minutes, washed in 1 x PBS, and blocked in 5 mL of 0.5% BSA in 1 x PBS at room temperature for 30 minutes. Blocked cells were washed in 1 x PBS and incubated at room temperature for two hours in 0.5% BSA in 1 x PBS containing a 1:10 dilution of an ascites from a clonal hybridoma cell line being tested. Primary antibody probed cells were washed three times for 5 minutes each time in 1 x PBS. Washed cells were incubated at room temperature for 2 hours in 1 x PBS containing a 1:200 dilution of goat polyclonal anti-mouse immunoglobulin G, heavy and light chains (IgG, H+L) antibody conjugated to ALEXA® FLUOR 568 (Invitrogen Inc., Carlsbad, CA) as a secondary antibody. Secondary antibody-probed cells were washed three times for 5 minutes each time in 1 x PBS. Washed cells were prepared for microscopic examination by mounting in VECTASHIELD® Mounting Media (Vector Laboratories, Burlingame, CA) and coverslipped. Images of signal detection were obtained with a Leica confocal microscope using appropriate laser settings. Table 19 indicates that the α-SNAP-25 antibody-containing ascites that specifically detected the SNAP-25₁₉₇-cleavage product. The immunocytochemistry analysis indicated that ascites produced from clones 1D3B8, 2C9B10, 2E2A6, 3C1A5, and 3C3E2 synthesize an α-SNAP-25 monoclonal antibody having high binding specificity for the SNAP-25₁₉₇ cleavage product that allows for the preferential recognition of this cleavage product relative to the SNAP-25₂₀₆ uncleaved substrate.

For immunoprecipitation analysis, binding specificity was determined by analyzing the ability of Protein A (HiTrap™ Protein A HP Columns, GE Healthcare, Amersham, Piscataway, NJ), purified α-SNAP-25 monoclonal antibodies to precipitate the uncleaved SNAP-25₂₀₆ substrate and the cleaved SNAP-25₁₉₇ product. *See e.g.,* Chapter 8 Storing and Purifying Antibodies, pp. 309-311, Harlow & Lane, supra, 1998a. A suitable density of PC12 cells were plated, grown, and transfected with either a transfection solution containing a pQBI-25/GFP expression construct (control cells; SEQ ID NO: 53) or a transfection solution containing the pQBI-25/GFP-BoNT/A-LC expression construct (experimental cells) as described above. The pQBI-25/GFP expression construct comprises an expression vector whose promoter elements are functionally linked to a polynucleotide encoding GFP of SEQ ID NO: 54. After an overnight incubation, the cells were washed by aspirating the growth media and rinsing each well with 200 µL 1 x PBS. To harvest the cells, the PBS was aspirated, the cells were lysed by adding an Immunoprecipitation Lysis Buffer comprising 50 mM HEPES, 150 mM NaCl, 1.5 mM MgCl₂, 1 mM EGDT, 10% glycerol, 1% Triton® X-100 (polyethylene glycol octylphenol ether) and a 1 x COMPLETE™ Protease inhibitor cocktail (Roche Applied Biosciences, Indianapolis, IN) and incubating at 4 °C for one hour. The lysed cells were centrifuged at 3,000 x g at 4 °C for 10 minutes to remove cellular debris and the supernatant transferred to a clean tube and diluted to a protein concentration of approximately 1 mg/mL. Approximately 5 µg of purified monoclonal antibody was added to 0.5 mL of diluted supernatant and incubated at 4 °C for two hours. After primary antibody incubation, approximately 50 µl of immobilized Protein G (Pierce Biotechnology, Rockford, IL) was added to the diluted supernatant and incubated at 4 °C for one hour. The incubated supernatant was washed three times for 30 minutes each time by adding 0.5 mL of Immunoprecipitation Lysis Buffer, centrifuging at 300 x g at 4 °C for one minute to pellet the immobilized Protein G, and decanting the supernatant. After washing, the pellet was resuspended in 30 µL of 1 x SDS Loading Buffer and the sample was heated to 95 °C for 5 minutes. To detect for the presence of both the uncleaved SNAP-25₂₀₆ substrate and the cleaved SNAP-25₁₉₇ product, an aliquot from each harvested sample was analyzed by Western blot as described in Example I, except that the primary antibody used was a 1:1,000 dilution of the α-SNAP-25 polyclonal antibody serum (see Example IV) and the secondary antibody used was a 1:20,000 of rabbit α-IgG Horseradish Peroxidase (Pierce Biotechnology, Rockford, IL). Table 19 indicates the α-SNAP-25 antibody-containing ascites that specifically pulled down the SNAP-25₁₉₇-cleavage product by immunoprecipitation analysis. The immunoprecipitation analysis indicated that ascites produced from clones 2E2A6 and 3C1A5 synthesize an α-SNAP-25 monoclonal antibody having high binding specificity for the SNAP-25₁₉₇ cleavage product that allows for the preferential recognition of this cleavage product relative to the SNAP-25₂₀₆ uncleaved substrate.

| **Table 19. Analysis of Clone Ascites Containing α-SNAP-25 Monoclonal Antibody** | | | | | | |
|---|---|---|---|---|---|---|
| **Clone** | **Cell-Based Assay** | | **Immunocytochemistry** | | **Immunoprecipitation** | |
| | **SNAP-25₁₉₇** | **SNAP-25₂₀₆** | **SNAP-25₁₉₇** | **SNAP-25₂₀₆** | **SNAP-25₁₉₇** | **SNAP-25₂₀₆** |
| 1D3B8 | ++ | - | ++ | - | Not Tested | Not Tested |
| 1G10A12 | ++ | ++ | Not Tested | Not Tested | Not Tested | Not Tested |
| 2C9B10 | ++ | - | ++ | - | Not Tested | Not Tested |
| 2E2A6 | ++ | - | ++ | - | ++ | - |
| 2F11B6 | + | + | + | + | Not Tested | Not Tested |
| 3C1A5 | ++ | - | ++ | - | ++ | - |
| 3C3E2 | + | - | Not Tested | Not Tested | Not Tested | Not Tested |
| MC-6050 | - | + | Not Tested | Not Tested | Not Tested | Not Tested |
| MC-6053 | - | + | Not Tested | Not Tested | Not Tested | Not Tested |
| SMI-81 | -/+ | ++ | Not Tested | Not Tested | Not Tested | Not Tested |

### 5. Evaluation of binding affinity of α-SNAP-25 monoclonal antibodies.

To determine the binding affinity of an α-SNAP-25 monoclonal antibody showing high binding specificity for either the SNAP-25₁₉₇ cleavage product or the SNAP-25₂₀₆ uncleaved substrate, binding affinity assays were performed on a BIACORE 3000 instrument using carboxymethyl dextran (CM5) sensor chips (BIAcore, Inc., Piscataway, NJ). Runs were conducted at 25 °C with HBS-EP buffer comprising 10 mM HEPES (pH 7.4), 150 mM sodium chloride, 3 mM EDTA, 0.005% (v/v) surfactant P20 at a flow rate of 10 µl/min. SNAP-25 peptides comprising amino acids 134-197 of SEQ ID NO: 5 (SNAP-25₁₃₄₋₁₉₇) or amino acids 134-206 of SEQ ID NO: 5 (SNAP-25₁₃₄₋₂₀₆) were covalently attached to the surface of the CM5 sensor chips using standard amine coupling. Briefly, the CM5 chips were activated by a 7 minute injection of a mixture of 0.2 M 1-ethyl-3-(3-dimethylaminopropyl) carbodiimide and 0.05 M N-hydroxysuccimide; the SNAP-25 peptides were then injected in 10 mM sodium acetate (pH 4.0) for 20 min at a flow rate of 10 µL/min ; and unreacted succimide esters were blocked by a 7-min injection of 1 M ethanolamine hydrochloride, pH 8.5. The immobilized amount of SNAP-25₁₃₄₋₁₉₇ or SNAP-25₁₃₄₋₂₀₆ on the chip was reflected by a 100-150 increase in response units (about 0.10-0.15 ng/mm²). Antibody samples comprising either ascites or purified monoclonal antibodies produced from clones 1D3B8, 2C9B10, 2E2A6, 3C1A5, and 3C3E2, as well as, commercially available α-SNAP-25 antibodies were passed over the surface of the CM5 chips allowing an association time of 10 min and a dissociation time of 20 min. The surfaces were regenerated between runs by a 1 minute injection of 10 mM glycine-HCl (pH 2.5) at a flow rate of 15 µL/min. Sensorgram curves were fitted to a 1:1 kinetic binding model with the BIAevaluation 3.0 software.

The results indicate that both 2E2A6 and 3C1A5 were highly specific for cleaved SNAP-25₁₉₇ product over SNAP-25 uncleaved substrate (Table 20). When compared to the binding affinities of MC-6050 and MC-6053, 1D3B6 had an approximately 10-fold higher equilibrium disassociation constant for the SNAP-25 cleavage product relative to these commercial antibodies (Table 20). Interestingly, 2E2A6 had only a slightly lower equilibrium disassociation constant for the SNAP-25 cleavage product relative to these commercial antibodies (0.405 nM versus 0.497 and 0.508)(Table 20). As neither of these commercial α-SNAP-25 antibodies selectively recognized the SNAP-25 cleavage product (Table 19), an equilibrium disassociation constant lower than about 0.5 nM appears, in part, critical to achieve such selectivity. Similarly, when compared to the binding affinities of MC-6050 and MC-6053, 2E2A6 had an about at least one-fold slower off rate/dissociation constant (6.74 x 10⁻⁵ versus 8.82 x 10⁻⁴ s⁻¹ and 1.18 x 10⁻³ s⁻¹) (Table 20). This further suggests that an off rate/dissociation constant lower than about 8.82 x 10⁻⁴ appears, in part, critical to achieve selective binding for the SNAP-25 cleavage product. This result is consistent with 1 D3B8, which had an off rate/dissociation constant of 5.78 x 10⁻⁵ s⁻¹ (Table 20).

| **Table 20. Analysis of Binding Affinity α-SNAP-25 Monoclonal Antibodies** | | | | |
|---|---|---|---|---|
| **SPR Parameter** | **1D3B8** | | **2E2A6** | |
| | **SNAP-25₁₉₇** | **SNAP-25₂₀₆^{a}** | **SNAP-25₁₉₇** | **SNAP-25₂₀₆^{b}** |
| Ka (M⁻¹ s⁻¹) | 1.06 x 10⁶ | - | 1.70 x 10⁶ (1.66 x 10⁵) | - (-) |
| Kd (s⁻¹) | 5.78 x 10⁻⁵ | - | 1.53 x 10⁻⁴ (6.74 x 10⁻) | - (-) |
| KD (nM) | 0.050 | - | 0.090 (0.405) | (-) |
| **SPR Parameter** | **3C1A5** | | **2C9B10** | |
| | **SNAP-25₁₉₇** | **SNAP-25₂₀₆^{c}** | **SNAP-25₁₉₇** | **SNAP-25₂₀₆^{d}** |
| Ka (M⁻¹s⁻¹) | 2.17 x 10⁵ | - | 1.15 x 10⁴ | - |
| Kd (s⁻¹) | 2.88 x 10⁻⁴ | - | 3.11 x 10⁻⁴ | - |
| KD (nM) | 1.33 | - | 27.1 | - |
| **SPR Parameter** | **MC-6050** | | **MC-6053** | |
| | **SNAP-25₁₉₇** | **SNAP-25₂₀₆** | **SNAP-25₁₉₇** | **SNAP-25₂₀₆** |
| Ka (M⁻¹ s⁻¹) | 1.78 x 10⁶ | 3.06 x 10² | 2.32 x 10⁶ | 1.06 x 10² |
| Kd (s⁻¹) | 8.82 x 10⁻⁴ | 6.07 x 10⁻³ | 1.18 x 10⁻³ | 2.56 x 10⁻⁵ |
| KD (nM) | 0.497 | 19,800 | 0.508 | 240 |
| **^{a}** No binding was observed when up to 125 nM of α-SNAP-25 monoclonal antibody 1D3B8 was passed over the surface of the CM5 sensor chip after a 10 minute association time. | | | | |
| **^{b}** No binding was observed when up to 10 µM of α-SNAP-25 monoclonal antibody 2E2A6 was passed over the surface of the CM5 sensor chip after a 10 minute association time. | | | | |
| **^{c}** No binding was observed when up to 100 nM of α-SNAP-25 monoclonal antibody 3C1A5 was passed over the surface of the CM5 sensor chip after a 10 minute association time. | | | | |
| **^{d}** No binding was observed when up to 100 nM of α-SNAP-25 monoclonal antibody 2C9B10 was passed over the surface of the CM5 sensor chip after a 10 minute association time. | | | | |

To compare the six different antibodies, the on-rate (ka) and off-rate (kd) for each was normalized using a program from the BIA evaluation 4.1 software. For comparison of the on-rates, the data were first individually trimmed by deleting the re-generation portion and the injection spikes, and then normalized to a 0 to 100 scale. For comparison of the off-rate, the data were normalized to the injection stop/top point. This analysis showed that 2C9B10 had a much slower on-rate than the other antibodies (FIG. 3A), and that MC-6053 has a much faster off-rate (dissociation) that the other antibodies (FIG. 3B). The fast off-rate of MC-6053 indicates that this antibody will not perform well in the methods disclosed in the present specification because this antibody will have difficulty staying bound to the substrate antigen during the washing steps.

### 6. Sequencing of the epitope from isolated α-SNAP-25 monoclonal antibodies.

To determine the epitope of an isolated α-SNAP-25 monoclonal antibody that can selectively bind to a SNAP-25 antigen having a carboxyl-terminus at the P₁ residue of the BoNT/A cleavage site scissile bond, the polynucleotide molecule encoding the variable heavy (V_{H}) and variable light (V_{L}) chains of the α-SNAP-25 monoclonal antibody produced by hybridomas 1D3B8, 2C9B10, 2E2A6, 3C1A5 and 3C3E2 were sequenced. mRNA was extracted and purified from each hybridoma using standard protocols and reversed transcribed into cDNA using either an oligo dT anti-sense primer or a gene-specific (murine IgG1 CH and kappa CL) anti-sense primer. Specific murine and human constant domain primers were used to amplify the cDNA by PCR after cDNA production to determine the isotype of the antibody. Degenerate V_{H} and V_{L} primers were used to amplify the variable domains from the cDNA. For 5'RACE, a homopolymeric dCTP tail was added to the 3' end of the cDNA. The heavy and light chains were then amplified with an oligo dG sense primer and a gene specific (CH/KC) anti-sense primer. PCR products included the sequence of the signal peptide, variable domains and constant domains up to the anti-sense primer. The PCR products were gel purified to remove small fragments, and cloned into a blunt or TA vector for sequencing. Five independent clones for each chain were sequenced and alignments of V_{H} and VL chains and consensus sequences were determined. Methods used to determine the V_{H} and V_{L} amino acid sequences are described in, *e.g.,* Roger A Sabbadini, et al., *Novel Bioactive Lipid Derivatives and Methods of Making and Using Same,* U.S. Patent Publication 2007/0281320: and Peter Amersdorfer, et al., Molecular Characterization of Murine Humoral Immune Response to Botulinum Neurotoxin Type A Binding Domain as Assessed by Using Phage Antibody Libraries, 65(9) Infect. Immun. 3743-3752. In addition, commercial services are available to sequence the variable heavy (V_{H}) and variable light (V_{L}) chains of an antibody and identify the CDR regions, see, *e.g.,* Fusion Antibodies Ltd., Northern Ireland.

The polynucleotide sequence comprising the V_{H} and V_{L} chains of the α-SNAP-25 monoclonal antibody produced by the hybridomas disclosed in the present specification is as follows: 1D3B8 V_{H} (SEQ ID NO: 71), 2C9B10 V_{H} (SEQ ID NO: 73), 2E2A6 V_{H} (SEQ ID NO: 75), 3C1A5 V_{H} (SEQ ID NO. 77), 3C3E2 V_{H} variant 1 (SEQ ID NO: 79), 3C3E2 V_{H} variant 2 (SEQ ID NO: 81), 3C3E2 V_{H} variant 3 (SEQ ID NO: 149), 1D3B8 V_{L} (SEQ ID NO: 83), 2C9B10 V_{L} (SEQ ID NO: 85), 2E2A6 V_{L} (SEQ ID NO: 87), 3C1A5 V_{L} (SEQ ID NO: 89), and 3C3E2 V_{L} (SEQ 10 NO: 91). The amino acid sequence comprising the V_{H} and V_{L} chains of the α-SNAP-25 monoclonal antibody produced by the hybridomas disclosed in the present specification is as follows: 1D3B8 V_{H} (SEQ ID NO: 72), 2C9B10 V_{H} (SEQ ID NO: 74), 2E2A6 V_{H} (SEQ ID NO: 76), 3C1A5 V_{H} (SEQ ID NO: 78), 3C3E2 V_{H} variant 1 (SEQ ID NO: 80), 3C3E2 V_{H} variant 2 (SEQ ID NO: 82); 3C3E2 V_{H} variant 2 (SEQ ID NO: 150), 1D3B8 V_{L} (SEQ ID NO: 84), 2C9B10 V_{L} (SEQ ID NO: 86), 2E2A6 V_{L} (SEQ ID NO: 88), 3C1A5 V_{L} (SEQ ID NO: 90), and 3C3E2 V_{L} (SEQ ID NO: 92). The amino acid sequences comprising the V_{H} and V_{L} CDR domains of the α-SNAP-25 monoclonal antibody produced by the hybridomas 1D3B8, 2C9B10, 2E2A6, 3C1A5, and 3C3E2 are given in Table 21.

| **Table 21. CDR Sequences of V_{H} and V_{L} domains from α-SNAP-25 Monoclonal Antibodies** | | | |
|---|---|---|---|
| **CDR** | **Sequence** | **Identified In** | **SEQ ID NO:** |
| V_{H} CDR 1 | TFTDHSIH | 2E2A6 | 93 |
| | | 2C9B10 | |
| | | 3C1A5 | |
| V_{H} CDR 1 | TFTNYVIH | 3C3E2 | 94 |
| V_{H} CDR 1 | IFTDHALH | 1D3B8 | 95 |
| V_{H} CDR 2 | YIFPGNGNIEYNDKFKG | 2E2A6 | 96 |
| V_{H} CDR 2 | YLFPGNGNFEYNEKFKG | 2C9B10 3C1A5 | 97 |
| V_{H} CDR 2 | YINPYNDGSKYNEKFKG | 3C3E2 | 98 |
| V_{H} CDR 2 | YIFPGNGNIEYNEKFKG | 1D3B8 | 99 |
| V_{H} CDR 3 | KRMGY | 2E2A6 3C1A5 | 100 |
| V_{H} CDR 3 | KKMDY | 2C9B10 1D3B8 | 101 |
| V_{H} CDR 3 | ARMDY | 3C3E2var1 | 102 |
| V_{H} CDR 3 | ARMGY | 3C3E2var2 | 151 |
| V_{H} CDR 3 | ARHLANTYYYFDY | 3C3E2var3 | 152 |
| V_{L} CDR 1 | RSSQSIVHSNGNTYLE | 1D3B8 | 103 |
| V_{L} CDR 1 | RTTENIYSYFV | 2C9B10 | 104 |
| V_{L} CDR 1 | KSSQSLLYTNGKTYLT | 2E2A6 | 105 |
| V_{L} CDR 1 | KSSQSLLNTNGKTYLT | 3C1A5 | 106 |
| V_{L} CDR 1 | RASQNIGNYLH | 3C3E2 | 107 |
| V_{L} CDR 2 | KVSNRFS | 1D3B8 | 108 |
| V_{L} CDR 2 | NAKSLAE | 2C9B10 | 109 |
| V_{L} CDR 2 | LVSELDS | 2E2A6 | 110 |
| V_{L} CDR 2 | LVSKLDS | 3C1A5 | 111 |
| V_{L} CDR 2 | YASQSIS | 3C3E2 | 112 |
| V_{L} CDR 3 | FQGSHVPPT | 1D3B8 | 113 |
| V_{L} CDR 3 | QHHYGTPYT | 2C9B10 | 114 |
| V_{L} CDR 3 | LQSAHFPFT | 2E2A6 | 115 |
| V_{L} CDR 3 | LQSSHFPFT | 3C1A5 | 116 |
| V_{L} CDR 3 | QQSDTWPLT | 3C3E2 | 117 |

Non-limiting examples of amino acid sequences comprising V_{H} CDR domain variants of the α-SNAP-25 monoclonal antibody produced by the hybridomas disclosed in the present specification include V_{H} CDR1 variant SEQ ID NO: 118 for 1D3B8; V_{H} CDR1 variant SEQ ID NO: 119 for 2C9B10, 2E2A6 and 3C1A5 V_{H}; V_{H} CDR1 variant SEQ ID NO: 120 for 3C1A5 V_{H} and 3C3E2 variant 3; V_{H} CDR2 variant SEQ ID NO: 121 for 1D3B8 and 2E2A6; V_{H} CDR2 variant SEQ ID NO: 122 for 2C9B10 and 3C1A5 V_{H}; V_{H} CDR2 variant SEQ ID NO: 123 for 3C1A5 V_{H} and 3C3E2 variant 3; V_{H} CDR3 variant MDY for 1D3B8 and 2C9B10; V_{H} CDR3 variant MGY for 2E2A6 and 3C1A5 V_{H}; and V_{H} CDR3 variant SEQ ID NO: 124 for 3C1A5 V_{H} and 3C3E2 variant 3. Non-limiting examples of amino acid sequences comprising V_{L} CDR domain variants of the α-SNAP-25 monoclonal antibody produced by the hybridomas disclosed in the present specification include V_{L} CDR1 variant SEQ ID NO: 125 for 1 D3B8; V_{L} CDR1 variant SEQ ID NO: 126 for 2C9B10; V_{L} CDR1 variant SEQ ID NO: 127 for 2E2A6; V_{L} CDR1 variant SEQ ID NO: 128 for 3C1A5; V_{L} CDR1 variant SEQ ID NO: 129 for 3C3E2; V_{L} CDR2 variant KVS for 1D3B8; V_{L} CDR2 variant NAK for 2C9B10; V_{L} CDR2 variant LVS for 2E2A6; V_{L} CDR2 variant YAT for 3C1A5; and V_{L} CDR2 variant YAS for 3C3E2.

### Example IX

### Development of α-SNAP-25 Polyclonal Antibodies that Selectively Bind a SNAP-25 Epitope Having a Free Carboxyl-terminus at the P₁ Residue of the BoNT/A Cleavage Site Scissile Bond

The following example illustrates how to make α-SNAP-25 polyclonal antibodies that can selectively bind to a SNAP-25 epitope having a carboxyl-terminus at the P₁ residue of the BoNT/A cleavage site scissile bond.

To develop α-SNAP-25 polyclonal antibodies that bind an epitope comprising a carboxyl-terminus at the P1 residue from the BoNT/A cleavage site scissile bond from a SNAP-25 cleavage product, the 10-residue peptide CGGGRIDEANQ (SEQ ID NO: 46) was designed as a SNAP-25 cleavage product antigen. This peptide comprising a N-terminal Cysteine residue for conjugation to KLH, a G-spacer flexible spacer (GGG) linked to amino acids 191-197 of human SNAP-25 (SEQ ID NO: 5) and has a carboxylated C-terminal glutamine. Blast searches revealed that this peptide has high homology only to SNAP-25 and almost no possible cross-reactivity with other proteins in neuronal cells. The sequence was also carefully scrutinized by utilizing computer algorithms to determine hydropathy index, protein surface probability, regions of flexibility, and favorable secondary structure, followed by proper orientation and presentation of the chosen peptide sequence. The peptide was synthesized and conjugated to Keyhole Limpet Hemocyanin (KLH) to increase immunogenicity. Before the animals were immunized, naïve rabbits were first screened against cell lysates from candidate cell lines in a Western blot in order to identify animals that had no immunoreactivity to the proteins present in the cell lysates. Two pre-screened rabbits were immunized with this peptide, and after three immunizations in about eight weeks, the rabbits were bled for testing. The blood was allowed to clot by incubating at 4 °C for 60 minutes. The clotted blood was centrifuged at 10,000x g at 4 °C for 10 minutes to pellet the cellular debris. The resulting serum sample was dispensed into 50 µL aliquots and stored at -20 °C until needed.

A similar strategy based on other SNAP-25 antigens disclosed in the present specification is used to develop α-SNAP-25 polyclonal antibodies that bind an epitope comprising a carboxyl-terminus at the P1 residue from the BoNT/A cleavage site scissile bond from a SNAP-25 cleavage product. For example, the SNAP-25 antigen of SEQ ID NO: 47 can be conjugated to KLH instead of the SNAP-25 antigen of SEQ ID NO: 46. As another example, the amino acids 191-197 of human SNAP-25 from the SNAP-25 antigen of SEQ ID NO: 38 can be replaced with SEQ ID NO: 33, SEQ ID NO: 34, SEQ ID NO: 35, SEQ ID NO: 36, SEQ ID NO: 37, SEQ ID NO: 39, SEQ ID NO: 40, SEQ ID NO: 41, SEQ ID NO: 42, SEQ ID NO: 43, or SEQ ID NO: 44.

### 2. Screening for the presence of α-SNAP-25 polyclonal antibodies.

To determine the presence of α-SNAP-25 polyclonal antibodies that can selectively bind to a SNAP-25 antigen having a carboxyl-terminus at the P₁ residue of the BoNT/A cleavage site scissile bond, comparative ELISA and cell-based cleavage assays were performed using the extracted rabbit serum as described in Example VIII. The serum from both rabbits contained α-SNAP-25 polyclonal antibodies that can selectively bind to a SNAP-25 antigen having a carboxyl-terminus at the P₁ residue of the BoNT/A cleavage site scissile bond. The α-SNAP-25 rabbit polyclonal antibodies were designated as NTP 22 and NTP 23.

### 3. Purification of α-SNAP-25 polyclonal antibodies.

To purify α-SNAP-25 polyclonal antibodies that can selectively bind to a SNAP-25 antigen having a carboxyl-terminus at the P₁ residue of the BoNT/A cleavage site scissile bond, NTP 22 and NTP 23 antibodies from rabbit serum were purified using affinity columns containing the SNAP-25 antigen of SEQ ID NO: 46.

### 4. Evaluation of binding specificity of α-SNAP-25 polyclonal antibodies.

To evaluate binding specificity of an α-SNAP-25 polyclonal antibody that can selectively bind to a SNAP-25 antigen having a carboxyl-terminus at the P₁ residue of the BoNT/A cleavage site scissile bond, purified NTP 22 and NTP 23 α-SNAP-25 polyclonal antibodies were used to detect cleavage product using the cell-based activity assay, immunnocytochemistry and immunoprecipitation as described in Example VIII. The cell-based cleavage assay, immunocytochemistry analysis and immunoprecipitation analysis all indicated that NTP 22 and NTP 23 α-SNAP-25 polyclonal antibodies did not cross-react with uncleaved SNAP-25. Thus both NTP 22 and NTP 23 have high binding specificity for the SNAP-25₁₉₇ cleavage product that allows for the preferential recognition of this cleavage product relative to the SNAP-25₂₀₆ uncleaved substrate. Affinity for the antigens can be determined using SPR in the BiAcore as described in Example VIII.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

**Table 5. Increased Gene Expression in Cell Lines H1 and BB10 vs. Cell Line 2D6 in Undifferentiated State Using Microarray Analysis**

| **Gene Symbol** | **Probe Set ID** | **Representative Public ID** | **RefSeq Transcript ID** | **Entrez Gene ID** | **Log2 Ratio** | **pvalue** |
|---|---|---|---|---|---|---|
| PLS3 | 201215_at | NM_005032 | NM_005032 | 5358 | 6.56 | 2.91E-12 |
| H99792 | 225710_at | H99792 | --- | --- | 6.46 | 1.81E-07 |
| DAZ1 | 208282_x_at | NM_020363 | NM_00100537 5 | 1617 | 6.45 | 4.63E-04 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| TPTE | 220205_at | NM_013315 | NM_013315 | 7179 | 6.44 | 3.16E-08 |
| | | | NM_199259 | | | |
| | | | NM_199260 | | | |
| | | | NM_199261 | | | |
| DAZ1 | 207909_x_at | U21663 | NM_00100537 5 | 1617 | 6.20 | 2.48E-04 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| MSN | 200600_at | NM_002444 | NM_002444 | 4478 | 6.11 | 2.92E-09 |
| CXCR4 | 217028_at | AJ224869 | NM_00100854 0 | 7852 | 5.95 | 3.23E-04 |
| | | | NM_003467 | | | |
| NDN | 209550_at | U35139 | NM_002487 | 4692 | 5.81 | 3.18E-06 |
| FLJ32942 | 227711_at | BG150433 | NM_144594 | 121355 | 5.73 | 8.08E-09 |
| AI197932 | 229498_at | AI197932 | --- | --- | 5.59 | 3.21E-08 |
| PEG3 | 209242_at | AL042588 | NM_006210 | 5178 | 5.58 | 1.13E-07 |
| TIMP3 | 201149_s_at | U67195 | NM_000362 | 7078 | 5.57 | 3.00E-05 |
| NEDD9 | 202149_at | AL136139 | NM_006403 | 4739 | 5.49 | 3.70E-09 |
| | | | NM_182966 | | | |
| TIMP3 | 201150_s_at | NM_000362 | NM_000362 | 7078 | 5.40 | 1.53E-03 |
| PRSS12 | 205515_at | NM_003619 | NM_003619 | 8492 | 5.37 | 1.39E-06 |
| LUM | 201744_s_at | NM_002345 | NM_002345 | 4060 | 5.37 | 1.04E-06 |
| RGS5 | 1555725_a_at | AF493929 | NM_003617 | 8490 | 5.22 | 2.23E-07 |
| TFPI2 | 209278_s_at | L27624 | NM_006528 | 7980 | 5.22 | 1.95E-05 |
| CXCR4 | 211919_s_at | AF348491 | NM_00100854 0 | 7852 | 5.18 | 9.56E-04 |
| | | | N M 003467 | | | |
| RGS5 | 218353_at | NM_025226 | NM_003617 | 8490 | 5.15 | 1.82E-07 |
| ASCL1 | 209985_s_at | BE797438 | NM_004316 | 429 | 5.09 | 8.38E-06 |
| KCTD12 | 212192_at | A1718937 | NM_138444 | 115207 | 5.04 | 6.32E-06 |
| SPARC | 200665_s_at | NM_003118 | NM_003118 | 6678 | 4.97 | 2.45E-06 |
| CXCR4 | 209201_x_at | L01639 | NM_00100854 0 | 7852 | 4.97 | 9.51E-04 |
| | | | NM_003467 | | | |
| TIMP3 | 201147_s_at | BF347089 | NM_000362 | 7078 | 4.95 | 1.56E-04 |
| DAZ1 | 207912_s_at | NM_004081 | NM_00100537 5 | 1617 | 4.94 | 1.79E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| MINA | 213189_at | BE966695 | NM_032778 | 84864 | 4.92 | 1.50E-08 |
| | | | NM_153182 | | | |
| CUGBP2 | 202157_s_at | U69546 | NM_006561 | 10659 | 4.91 | 1.20E-06 |
| BF000203 | 236385_at | BF000203 | --- | --- | 4.87 | 6.43E-06 |
| GRP | 206326_at | NM_002091 | NM_002091 | 2922 | 4.82 | 1.83E-06 |
| RGS5 | 209070_s_at | AI183997 | NM_003617 | 8490 | 4.78 | 1.01E-07 |
| AI183997 | 233858_at | AU144918 | --- | --- | 4.72 | 1.79E-05 |
| RNF182 | 230720_at | AI884906 | NM_152737 | 221687 | 4.72 | 4.74E-07 |
| PEG3 | 209243_s_at | AF208967 | NM_006210 | 5178 | 4.69 | 8.13E-06 |
| RAM2 | 225081_s_at | AK022955 | NM_018719 | 55536 | 4.52 | 1.18E-04 |
| DKFZp667G211 | 214030_at | BE501352 | XM_376254 | 131544 | 4.52 | 6.37E-10 |
| RGS5 | 209071_s_at | AF159570 | NM_003617 | 8490 | 4.50 | 1.74E-07 |
| VIP | 206577_at | NM_003381 | NM_003381 | 7432 | 4.44 | 2.77E-04 |
| | | | NM_194435 | | | |
| PRSS12 | 213802_at | AI810767 | NM_003619 | 8492 | 4.41 | 1.46E-03 |
| MGC34032 | 235763_at | AA001450 | NM_152697 | 204962 | 4.40 | 3.84E-08 |
| DAZ1 | 208281_x_at | NM_020364 | NM_00100537 5 | 1617 | 4.39 | 5.53E-04 |
| DAZ3 | | | | 57054 | | |
| DAZ2 | | | NM_00100578 | 57055 | | |
| DAZ4 | | | 5 | 57135 | | |
| | | | NM_00100578 6 | | | |
| | | | NM_004081 | | | |
| | | | NM_020363 | | | |
| | | | NM_020364 | | | |
| | | | NM_020420 | | | |
| DAZ1 | 216922_x_at | AF271088 | NM_00100537 5 | 1617 | 4.35 | 6.79E-04 |
| DAZ3 | | | | 57054 | | |
| DAZ2 | | | NM_00100578 | 57055 | | |
| DAZ4 | | | 5 | 57135 | | |
| | | | NM_00100578 6 | | | |
| | | | NM_004081 | | | |
| | | | NM_020363 | | | |
| | | | NM_020364 | | | |
| | | | NM_020420 | | | |
| SPARC | 212667_at | AL575922 | NM_003118 | 6678 | 4.30 | 4.26E-05 |
| MAB21L2 | 210302_s_at | AF262032 | NM_006439 | 10586 | 4.28 | 1.96E-08 |
| CCDC3 | 223316_at | AL136562 | NM_031455 | 83643 | 4.21 | 1.24E-06 |
| SLAC2-B | 214734_at | AB014524 | NM_015065 | 23086 | 4.20 | 1.64E-03 |
| ARHGAP6 | 206167_s_at | NM_001174 | NM_001174 | 395 | 4.19 | 1.68E-05 |
| | | | NM_006125 | | | |
| | | | NM_013422 | | | |
| | | | NM_013423 | | | |
| | | | NM_013427 | | | |
| BASP1 | 202391_at | NM_006317 | NM_006317 | 10409 | 4.18 | 3.67E-08 |
| SIM1 | 206876_at | AL121948 | NM_005068 | 6492 | 4.16 | 4.38E-05 |
| SMC6L1 | 236535_at | AW069285 | NM_024624 | 79677 | 4.09 | 2.41E-07 |
| CUGBP2 | 202158_s_at | NM_006561 | NM_006561 | 10659 | 4.09 | 8.95E-04 |
| FAT | 201579_at | NM_005245 | NM_005245 | 2195 | 4.08 | 5.01E-03 |
| GNG11 | 204115_at | NM_004126 | NM_004126 | 2791 | 4.07 | 6.53E-04 |
| ITGA6 | 201656_at | NM_000210 | NM_000210 | 3655 | 3.98 | 4.23E-09 |
| ACATE2 | 221641_s_at | AF241787 | NM_012332 | 23597 | 3.97 | 4.71E-05 |
| TCTE1L | 203303_at | NM_006520 | NM_006520 | 6990 | 3.96 | 2.11E-07 |
| NEDD9 | 202150_s_at | U64317 | NM_006403 | 4739 | 3.94 | 1.90E-06 |
| | | | NM_182966 | | | |
| DOK5 | 214844_s_at | AL050069 | NM_018431 | 55816 | 3.83 | 1.64E-05 |
| | | | NM_177959 | | | |
| EDG2 | 204036_at | AW269335 | NM_001401 | 1902 | 3.82 | 2.36E-08 |
| | | | NM_057159 | | | |
| MAOA | 204388_s_at | NM_000240 | NM_000240 | 4128 | 3.80 | 8.51E-05 |
| SPAG6 | 210033_s_at | AF079363 | NM_012443 | 9576 | 3.78 | 6.66E-06 |
| | | | NM_172242 | | | |
| BF476109 | 1556300_s_at | BF476109 | --- | --- | 3.78 | 7.77E-06 |
| PTPRK | 203038_at | NM_002844 | NM_002844 | 5796 | 3.75 | 1.34E-03 |
| GFRA2 | 205722_s_at | NM_001495 | NM_001495 | 2675 | 3.74 | 7.15E-06 |
| PTPRE | 221840_at | AA775177 | NM_006504 | 5791 | 3.72 | 2.12E-06 |
| | | | NM_130435 | | | |
| ADAMTS9 | 226814_at | AI431730 | NM_020249 | 56999 | 3.70 | 9.69E-05 |
| | | | NM_182920 | | | |
| | | | NM_182921 | | | |
| MINA | 213188_s_at | AI823896 | NM_032778 | 84864 | 3.69 | 3.36E-05 |
| | | | NM_153182 | | | |
| PLK2 | 201939_at | NM_006622 | NM_006622 | 10769 | 3.69 | 1.39E-06 |
| KIAA1754 | 225582_at | AA425726 | NM_033397 | 85450 | 3.67 | 2.03E-04 |
| FN5 | 219806_s_at | NM_020179 | NM_020179 | 56935 | 3.65 | 2.97E-04 |
| SMC6L1 | 218781_at | NM_024624 | NM_024624 | 79677 | 3.64 | 1.72E-08 |
| EML1 | 204797_s_at | NM_004434 | NM_00100870 7 | 2009 | 3.64 | 6.58E-05 |
| | | | NM_004434 | | | |
| ADAMTS9 | 1562275_at | AL832835 | NM_020249 | 56999 | 3.58 | 1.72E-06 |
| | | | NM_182920 | | | |
| | | | NM_182921 | | | |
| LOC441157 | 1558795_at | AL833240 | XM_499037 | 441157 | 3.56 | 7.55E-06 |
| MAOA | 212741_at | AA923354 | NM_000240 | 4128 | 3.55 | 6.62E-04 |
| TRIM29 | 211002_s_at | AF230389 | NM_012101 | 23650 | 3.51 | 2.23E-07 |
| | | | NM_058193 | | | |
| MAOA | 204389_at | NM_000240 | NM_000240 | 4128 | 3.50 | 4.16E-07 |
| MGC34032 | 1569112_at | AW020413 | NM_152697 | 204962 | 3.50 | 9.78E-06 |
| ELOVL7 | 227180_at | AW138767 | XM_371740 | 79993 | 3.45 | 6.72E-04 |
| TFPI2 | 209277_at | AL574096 | NM_006528 | 7980 | 3.43 | 6.92E-06 |
| ASCL1 | 213768_s_at | AW950513 | NM_004316 | 429 | 3.36 | 5.80E-07 |
| CHRNA7 | 210123_s_at | U62436 | NM_000746 | 1139 | 3.33 | 4.22E-06 |
| CHRFAM7A | | | NM_139320 | 89832 | | |
| | | | NM_148911 | | | |
| ASCL1 | 209987_s_at | BC002341 | NM_004316 | 429 | 3.33 | 1.82E-08 |
| FRZB | 203697_at | U91903 | NM_001463 | 2487 | 3.31 | 1.02E-05 |
| SLC43A3 | 210692_s_at | BC003163 | NM_014096 | 29015 | 3.31 | 7.81E-05 |
| | | | NM_017611 | | | |
| | | | NM_199329 | | | |
| LOC253012 | 242601_at | AA600175 | NM_198151 | 253012 | 3.30 | 2.75E-04 |
| CD9 | 201005_at | NM_001769 | NM_001769 | 928 | 3.26 | 3.31E-04 |
| SMAD6 | 207069_s_at | NM_005585 | NM_005585 | 4091 | 3.26 | 6.45E-06 |
| PCGF5 | 226326_at | AI798098 | NM_032373 | 84333 | 3.26 | 7.00E-07 |
| MAB21L2 | 210303_at | AF262032 | NM_006439 | 10586 | 3.26 | 6.74E-05 |
| SLC7A2 | 225516_at | AA876372 | NM_00100853 9 | 6542 | 3.22 | 1.59E-06 |
| | | | NM_003046 | | | |
| AL109791 | 233932_at | AL109791 | --- | --- | 3.20 | 1.60E-03 |
| MGC33926 | 229302_at | AA058832 | NM_152390 | 130733 | 3.20 | 1.16E-03 |
| KCTD12 | 212188_at | AA551075 | NM_138444 | 115207 | 3.19 | 7.60E-05 |
| MAFB | 218559_s_at | NM_005461 | NM_005461 | 9935 | 3.16 | 7.22E-03 |
| BNC2 | 220272_at | NM_017637 | NM_017637 | 54796 | 3.14 | 3.14E-03 |
| ASCL1 | 209988_s_at | BC001638 | NM_004316 | 429 | 3.14 | 4.77E-08 |
| CBLN2 | 242301_at | R60224 | NM_182511 | 147381 | 3.14 | 3.35E-03 |
| ALCAM | 240655_at | BE502785 | NM_001627 | 214 | 3.13 | 2.14E-04 |
| AF131795 | 233139_at | AF131795 | --- | --- | 3.12 | 8.04E-03 |
| AI656180 | 242447_at | AI656180 | --- | --- | 3.12 | 3.69E-05 |
| LOC402524 | 242206_at | AW590588 | XM_379850 | 402524 | 3.10 | 5.25E-04 |
| EDG3 | 228176_at | AA534817 | NM_005226 | 1903 | 3.10 | 4.81E-04 |
| KDELC2 | 225128_at | AL548941 | NM_153705 | 143888 | 3.09 | 2.21E-04 |
| NEGR1 | 243357_at | AA115106 | NM_173808 | 257194 | 3.09 | 1.42E-05 |
| DACT1 | 219179_at | NM_016651 | NM_016651 | 51339 | 3.09 | 2.14E-04 |
| DAZ1 | 216351_x_at | AF248483 | | 1617 | 3.06 | 4.06E-04 |
| DAZ3 | | | NM_00100537 | 57054 | | |
| DAZ2 | | | 5 | 57055 | | |
| DAZ4 | | | NM_00100578 5 | 57135 | | |
| | | | NM_00100578 6 | | | |
| | | | NM_004081 | | | |
| | | | NM_020363 | | | |
| | | | NM_020364 | | | |
| | | | NM_020420 | | | |
| KIAA0527 | 214954_at | BF977837 | XM_171054 | 26032 | 3.03 | 2.02E-04 |
| TPBG | 203476_at | NM_006670 | NM_006670 | 7162 | 3.02 | 2.88E-05 |
| LGR5 | 213880_at | AL524520 | NM_003667 | 8549 | 3.02 | 2.16E-03 |
| FLJ23091 | 228950_s_at | AL534095 | NM_00100229 2 | 79971 | 3.01 | 2.38E-03 |
| | | | NM_024911 | | | |
| PLXNA4 | 232317_at | AB046770 | XM_039393 | 57671 | 2.99 | 6.60E-07 |
| | | | XM_379927 | | | |
| FLJ20647 | 218802_at | NM_017918 | NM_017918 | 55013 | 2.99 | 7.56E-03 |
| CNTN4 | 229084_at | R42166 | NM_175607 | 152330 | 2.96 | 2.55E-06 |
| | | | NM_175612 | | | |
| | | | NM_175613 | | | |
| RBPMS | 209488_s_at | D84109 | NM_00100871 0 | 11030 | 2.96 | 5.32E-03 |
| | | | NM_00100871 1 | | | |
| | | | NM_00100871 2 | | | |
| | | | NM_006867 | | | |
| E2F7 | 228033_at | AI341146 | NM_203394 | 144455 | 2.94 | 1.26E-03 |
| TRIM29 | 202504_at | NM_012101 | NM_012101 NM_058193 | 23650 | 2.93 | 6.42E-08 |
| N26620 | 227503_at | N26620 | --- | --- | 2.93 | 1.14E-04 |
| TIMP3 | 201148_s_at | AW338933 | NM_000362 | 7078 | 2.91 | 1.23E-03 |
| C2orf31 | 221245_s_at | NM_030804 | --- | 81561 | 2.91 | 2.25E-05 |
| DKFZp7610201 | 232313_at | AL122107 | XM_044062 | 92293 | 2.91 | 1.59E-06 |
| BC041965 | 1560411_at | BC041965 | --- | --- | 2.91 | 8.58E-04 |
| GNAI1 | 227692_at | AU153866 | NM_002069 | 2770 | 2.90 | 2.00E-06 |
| GPR10 | 231805_at | AL563031 | NM_004248 | 2834 | 2.90 | 1.19E-04 |
| LGR5 | 210393_at | AF062006 | NM_003667 | 8549 | 2.89 | 4.97E-05 |
| ENC1 | 201340_s_at | AF010314 | NM_003633 | 8507 | 2.89 | 4.72E-05 |
| LOC51760 | 205613_at | NM_016524 | NM_016524 | 51760 | 2.88 | 9.54E-03 |
| GNG12 | 222834_s_at | N32508 | NM_018841 | 55970 | 2.88 | 1.98E-04 |
| BARD1 | 205345_at | NM_000465 | NM_000465 | 580 | 2.87 | 6.84E-03 |
| A2BP1 | 1553422_s_at | NM_145892 | NM_018723 | 54715 | 2.86 | 4.30E-03 |
| | | | NM_145891 | | | |
| | | | NM_145892 | | | |
| | | | NM_145893 | | | |
| PPP1R3C | 204284_at | N26005 | NM_005398 | 5507 | 2.84 | 1.93E-05 |
| AF086027 | 1559697_a_at | AF086027 | --- | --- | 2.83 | 1.45E-03 |
| PMP22 | 210139_s_at | L03203 | NM_000304 | 5376 | 2.83 | 1.89E-04 |
| | | | NM_153321 | | | |
| | | | NM_153322 | | | |
| GFRA2 | 205721_at | U97145 | NM_001495 | 2675 | 2.83 | 4.80E-04 |
| PFKFB3 | 202464_s_at | NM_004566 | NM_004566 | 5209 | 2.82 | 3.61E-03 |
| BF222929 | 242346_x_at | BF222929 | --- | --- | 2.82 | 2.87E-04 |
| SLC43A3 | 213113_s_at | AI630178 | NM_014096 | 29015 | 2.80 | 1.02E-06 |
| | | | NM_017611 | | | |
| | | | NM_199329 | | | |
| PEX5L | 1562473_at | BC037943 | NM_016559 | 51555 | 2.79 | 1.76E-04 |
| PELO | 226731_at | AA156873 | | 53918 | 2.76 | 4.84E-03 |
| | | | NM_015946 | | | |
| FBN1 | 202765_s_at | AI264196 | NM_000138 | 2200 | 2.76 | 2.17E-03 |
| PLEKHE1 | 212719_at | AB011178 | NM_194449 | 23239 | 2.75 | 5.65E-03 |
| MYRIP | 214156_at | AL050090 | NM_015460 | 25924 | 2.75 | 2.44E-06 |
| BNC2 | 235723_at | AA843242 | NM_017637 | 54796 | 2.74 | 2.24E-03 |
| CPVL | 208146_s_at | NM_031311 | NM_019029 | 54504 | 2.73 | 2.87E-03 |
| | | | NM_031311 | | | |
| PLXNA4 | 228104_at | AL117427 | XM_039393 | 57671 | 2.72 | 4.31E-04 |
| | | | XM_379927 | | | |
| BF940761 | 229427_at | BF940761 | --- | --- | 2.69 | 4.80E-03 |
| ARHGEF6 | 209539_at | D25304 | NM_004840 | 9459 | 2.68 | 2.81E-04 |
| H17657 | 241444_at | H17657 | --- | --- | 2.67 | 2.17E-04 |
| GNAI1 | 209576_at | AL049933 | NM_002069 | 2770 | 2.65 | 1.06E-07 |
| LOC441157 | 1558796_a_at | AL833240 | XM_499037 | 441157 | 2.65 | 1.11E-03 |
| CADPS | 1568603_at | AI912173 | NM_003716 | 8618 | 2.64 | 4.31E-04 |
| | | | NM_183393 | | | |
| | | | NM_183394 | | | |
| POSTN | 210809_s_at | D13665 | NM_006475 | 10631 | 2.63 | 1.47E-03 |
| PTPRR | 210675_s_at | U77917 | NM_002849 | 5801 | 2.60 | 4.53E-03 |
| | | | NM_130846 | | | |
| MGC24103 | 232568_at | AU145658 | --- | 158295 | 2.59 | 3.96E-04 |
| STK17A | 202693_s_at | AW194730 | NM_004760 | 9263 | 2.59 | 1.11E-04 |
| LOC440738 | 221697_at | AF276659 | NM_001004342 | 440738 | 2.58 | 1.85E-03 |
| MYO6 | 203215_s_at | AA877789 | NM_004999 | 4646 | 2.56 | 7.95E-03 |
| CADPS | 1568604_a_at | AI912173 | NM_003716 | 8618 | 2.56 | 2.70E-07 |
| | | | NM_183393 | | | |
| | | | NM_183394 | | | |
| NR3C1 | 211671_s_at | U01351 | NM_000176 | 2908 | 2.56 | 2.36E-09 |
| FRZB | 203698_s_at | NM_001463 | NM_001463 | 2487 | 2.56 | 3.80E-04 |
| FLJ23091 | 221958_s_at | AA775681 | NM_00100229 2 | 79971 | 2.54 | 5.86E-03 |
| | | | NM_024911 | | | |
| ADAMTS9 | 1554697_at | AF488803 | NM_020249 | 56999 | 2.54 | 1.99E-04 |
| | | | NM_182920 | | | |
| | | | NM_182921 | | | |
| AW130600 | 226865_at | AW130600 | --- | --- | 2.53 | 4.86E-04 |
| ST8SIA4 | 230836_at | AI422986 | NM_005668 | 7903 | 2.50 | 7.33E-03 |
| | | | NM_175052 | | | |
| CNTN1 | 211203_s_at | U07820 | NM_001843 | 1272 | 2.49 | 1.34E-04 |
| | | | NM_175038 | | | |
| EMILIN2 | 224374_s_at | AF270513 | NM_032048 | 84034 | 2.48 | 1.13E-04 |
| HGF | 210997_at | M77227 | NM_000601 | 3082 | 2.46 | 4.58E-03 |
| | | | NM_00101093 1 | | | |
| | | | NM_00101093 2 | | | |
| | | | NM_00101093 3 | | | |
| | | | NM_00101093 4 | | | |
| AI582818 | 229053_at | AI582818 | --- | --- | 2.44 | 6.88E-03 |
| LOC253981 | 226430_at | AI394438 | --- | 253981 | 2.44 | 3.85E-03 |
| GNG12 | 212294_at | BG111761 | NM_018841 | 55970 | 2.42 | 1.06E-06 |
| C6orf148 | 220513_at | NM_030568 | NM_030568 | 80759 | 2.41 | 5.89E-03 |
| OSBPL3 | 209626_s_at | AI202969 | NM_015550 | 26031 | 2.40 | 9.56E-04 |
| | | | NM_145320 | | | |
| | | | NM_145321 | | | |
| | | | NM_145322 | | | |
| | | | NM_145323 | | | |
| | | | NM_145324 | | | |
| SEMA6D | 233882_s_at | AK022831 | NM_020858 | 80031 | 2.39 | 1.02E-03 |
| | | | NM_024966 | | | |
| | | | NM_153616 | | | |
| | | | NM_153617 | | | |
| | | | NM_153618 | | | |
| | | | NM_153619 | | | |
| PCDH8 | 206935_at | NM_002590 | NM_002590 | 5100 | 2.37 | 6.17E-07 |
| | | | NM_032949 | | | |
| MAB21L1 | 206163_at | NM_005584 | NM_005584 | 4081 | 2.36 | 4.98E-03 |
| TSC | 218872_at | NM_017899 | NM_017899 | 54997 | 2.36 | 1.01E-05 |
| PPP2R2B | 213849_s_at | AA974416 | NM_004576 | 5521 | 2.35 | 2.05E-06 |
| | | | NM_181674 | | | |
| | | | NM_181675 | | | |
| | | | NM_181676 | | | |
| | | | NM_181677 | | | |
| | | | NM_181678 | | | |
| PDE5A | 227088_at | BF221547 | NM_001083 | 8654 | 2.35 | 2.27E-04 |
| | | | NM_033430 | | | |
| | | | NM_033431 | | | |
| | | | NM_033437 | | | |
| ZNF521 | 226676_at | AK021452 | NM_015461 | 25925 | 2.34 | 1.55E-03 |
| PVRL3 | 213325_at | AA129716 | NM_015480 | 25945 | 2.34 | 3.70E-06 |
| ENC1 | 201341_at | NM_003633 | NM_003633 | 8507 | 2.33 | 3.06E-05 |
| RGMA | 223468_s_at | AL136826 | NM_020211 | 56963 | 2.33 | 1.24E-07 |
| ST8SIA2 | 239537_at | AW589904 | NM_006011 | 8128 | 2.32 | 8.94E-07 |
| FGFR2 | 203639_s_at | M80634 | NM_000141 | 2263 | 2.29 | 4.06E-03 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| BAMBI | 203304_at | NM_012342 | NM_012342 | 25805 | 2.29 | 2.34E-08 |
| ARHGAP6 | 1563386_at | AL079282 | NM_001174 | 395 | 2.27 | 2.07E-03 |
| | | | NM_006125 | | | |
| | | | NM_013422 | | | |
| | | | NM_013423 | | | |
| | | | NM_013427 | | | |
| PLCL2 | 213309_at | AL117515 | NM_015184 | 23228 | 2.27 | 1.92E-03 |
| A2BP1 | 221217_s_at | NM_018723 | NM_018723 | 54715 | 2.26 | 5.56E-03 |
| | | | NM_145891 | | | |
| | | | NM_145892 | | | |
| | | | NM_145893 | | | |
| LOC340109 | 1557765_at | BC039509 | XM_379322 | 340109 | 2.25 | 2.28E-05 |
| SLC39A8 | 209267_s_at | AB040120 | NM_022154 | 64116 | 2.25 | 4.91E-03 |
| ITGA6 | 215177_s_at | AV733308 | NM_000210 | 3655 | 2.23 | 2.05E-03 |
| CNTN1 | 227209_at | AI091445 | NM_001843 | 1272 | 2.23 | 4.31E-03 |
| | | | NM_175038 | | | |
| EGLN3 | 219232_s_at | NM_022073 | NM_022073 | 112399 | 2.23 | 7.56E-03 |
| ZFHX4 | 219779_at | NM_024721 | NM_024721 | 79776 | 2.22 | 1.39E-03 |
| AF086027 | 1559696_at | AF086027 | --- | --- | 2.21 | 1.05E-03 |
| AL050204 | 213929_at | AL050204 | --- | --- | 2.20 | 2.15E-03 |
| TPTE | 233879_at | AL137389 | NM_013315 | 7179 | 2.20 | 2.76E-03 |
| | | | NM_199259 | | | |
| | | | NM_199260 | | | |
| | | | NM_199261 | | | |
| TMEM35 | 219685_at | NM_021637 | NM_021637 | 59353 | 2.20 | 8.17E-03 |
| MSX2 | 205555_s_at | D31771 | NM_002449 | 4488 | 2.20 | 6.02E-04 |
| GPR51 | 211679_x_at | AF095784 | NM_005458 | 9568 | 2.19 | 5.89E-03 |
| BE856929 | 239349_at | BE856929 | --- | --- | 2.17 | 2.00E-03 |
| SYN2 | 210247_at | AW139618 | NM_003178 | 6854 | 2.15 | 5.68E-03 |
| | | | NM_133625 | | | |
| COLEC12 | 221019_s_at | NM_030781 | NM_030781 | 81035 | 2.15 | 7.66E-03 |
| | | | NM_130386 | | | |
| TCF7L1 | 221016_s_at | NM_031283 | NM_031283 | 83439 | 2.14 | 1.38E-03 |
| NRXN1 | 228547_at | BF509242 | NM_004801 | 9378 | 2.13 | 8.92E-03 |
| | | | NM_138735 | | | |
| CRH | 205630_at | NM_000756 | NM_000756 | 1392 | 2.13 | 1.12E-03 |
| GPR161 | 230369_at | AI743151 | NM_007369 | 23432 | 2.12 | 1.61E-04 |
| | | | NM_153832 | | | |
| ZNF217 | 203739_at | NM_006526 | NM_006526 | 7764 | 2.12 | 3.18E-04 |
| NEDD9 | 233223_at | AK000850 | NM_006403 | 4739 | 2.11 | 4.23E-04 |
| | | | NM_182966 | | | |
| RASEF | 235144_at | AA826324 | NM_152573 | 158158 | 2.09 | 2.92E-03 |
| PLAT | 201860_s_at | NM_000930 | NM_000930 | 5327 | 2.09 | 6.29E-04 |
| | | | NM_000931 | | | |
| | | | NM_033011 | | | |
| GPR51 | 209990_s_at | AF056085 | NM_005458 | 9568 | 2.05 | 1.33E-05 |
| AL161982 | 1563494_at | AL161982 | --- | --- | 2.05 | 1.21E-03 |
| SEMA6D | 226492_at | AL036088 | NM_020858 | 80031 | 2.05 | 1.22E-04 |
| | | | NM_024966 | | | |
| | | | NM_153616 | | | |
| | | | NM_153617 | | | |
| | | | NM_153618 | | | |
| | | | NM_153619 | | | |
| ORF1-FL49 | 224707_at | AL522667 | NM_032412 | 84418 | 2.04 | 2.79E-05 |
| NID | 202007_at | BF940043 | NM_002508 | 4811 | 2.04 | 2.24E-03 |
| PCGF5 | 229194_at | AL045882 | NM_032373 | 84333 | 2.02 | 2.51E-04 |
| TCF7L2 | 216035_x_at | AV721430 | NM_030756 | 6934 | 2.02 | 2.84E-07 |
| AA156721 | 201952_at | AA156721 | --- | --- | 2.02 | 1.05E-06 |
| FBN1 | 202766_s_at | NM_000138 | NM_000138 | 2200 | 2.02 | 7.81E-04 |
| DBH | 206450_at | NM_000787 | NM_000787 | 1621 | 2.01 | 2.59E-03 |
| PTPRR | 206084_at | NM_002849 | NM_002849 | 5801 | 2.01 | 4.19E-03 |
| | | | NM_130846 | | | |
| CNTN1 | 227202_at | AW072790 | NM_001843 | 1272 | 2.00 | 1.32E-03 |
| | | | NM_175038 | | | |
| ARHGEF3 | 218501_at | NM_019555 | NM_019555 | 50650 | 2.00 | 4.42E-05 |
| SLITRK5 | 214930_at | AW449813 | NM_015567 | 26050 | 2.00 | 7.82E-06 |
| ETV1 | 206501_x_at | NM_004956 | NM_004956 | 2115 | 1.99 | 3.21E-03 |
| SPAG6 | 210032_s_at | AI651156 | NM_012443 | 9576 | 1.99 | 2.72E-04 |
| | | | NM_172242 | | | |
| LCRISP1 | 223475_at | AF142573 | NM_031461 | 83690 | 1.99 | 3.27E-03 |
| FGFR2 | 211401_s_at | AB030078 | NM_000141 | 2263 | 1.99 | 1.83E-04 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| FLJ30058 | 238047_at | AA405456 | NM_144967 | 158763 | 1.99 | 3.10E-03 |
| BF434771 | 1568598_at | BF434771 | XM_498494 | 440000 | 1.98 | 2.21E-03 |
| TCF7L2 | 216511_s_at | AJ270770 | NM_030756 | 6934 | 1.97 | 7.59E-05 |
| MYO6 | 203216_s_at | NM_004999 | NM_004999 | 4646 | 1.96 | 8.88E-05 |
| ATP2B4 | 212136_at | AW517686 | NM_00100139 6 | 493 | 1.96 | 7.81E-03 |
| | | | NM_001684 | | | |
| RAB32 | 204214_s_at | NM_006834 | NM_006834 | 10981 | 1.96 | 3.53E-03 |
| AI703256 | 231336_at | AI703256 | --- | --- | 1.95 | 4.67E-04 |
| FLJ40773 | 1563933_a_at | AK091691 | NM_152666 | 200150 | 1.95 | 1.13E-03 |
| LOC92689 | 213455_at | W87466 | NM_138389 | 92689 | 1.95 | 5.71E-04 |
| TCF7L2 | 212761_at | AI949687 | NM_030756 | 6934 | 1.94 | 5.52E-05 |
| TDRD7 | 213361_at | AW129593 | NM_014290 | 23424 | 1.93 | 4.13E-03 |
| GFPT2 | 205100_at | NM_005110 | NM_005110 | 9945 | 1.92 | 3.68E-04 |
| FGFR2 | 203638_s_at | NM_022969 | NM_000141 | 2263 | 1.92 | 3.46E-03 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| ALCAM | 201951_at | BF242905 | NM_001627 | 214 | 1.91 | 6.46E-06 |
| ETV1 | 217053_x_at | X87175 | NM_004956 | 2115 | 1.91 | 7.09E-03 |
| NR3C1 | 201865_x_at | AI432196 | NM_000176 | 2908 | 1.90 | 1.19E-05 |
| PDLIM5 | 203242_s_at | BG054550 | NM_00101151 3 | 10611 | 1.89 | 7.00E-05 |
| | | | NM_00101151 4 | | | |
| | | | NM_00101151 5 | | | |
| | | | NM_00101151 6 | | | |
| | | | NM_006457 | | | |
| ST8SIA2 | 221285_at | NM_006011 | NM_006011 | 8128 | 1.89 | 8.94E-04 |
| TWIST1 | 213943_at | X99268 | NM_000474 | 7291 | 1.88 | 3.85E-04 |
| TLE4 | 216997_x_at | AL358975 | NM_007005 | 7091 | 1.88 | 7.83E-03 |
| GPR161 | 214104_at | AI703188 | NM_007369 | 23432 | 1.88 | 5.85E-03 |
| | | | NM_153832 | | | |
| EDG2 | 204037_at | BF055366 | NM_001401 | 1902 | 1.87 | 9.88E-08 |
| | | | NM_057159 | | | |
| PDLIM5 | 216804_s_at | AK027217 | NM_00101151 3 | 10611 | 1.87 | 2.39E-05 |
| | | | NM_00101151 4 | | | |
| | | | NM_00101151 5 | | | |
| | | | NM_00101151 6 | | | |
| | | | NM_006457 | | | |
| R39126 | 230932_at | R39126 | --- | --- | 1.87 | 2.27E-04 |
| AI377043 | 230722_at | AI377043 | --- | --- | 1.87 | 1.53E-03 |
| W93046 | 230068_s_at | W93046 | --- | --- | 1.86 | 2.03E-03 |
| DMN | 212730_at | AK026420 | NM_015286 | 23336 | 1.86 | 2.15E-03 |
| | | | NM_145728 | | | |
| ARHGAP24 | 223422_s_at | AI743534 | NM_031305 | 83478 | 1.86 | 1.60E-03 |
| CCNE2 | 211814_s_at | AF112857 | NM_004702 | 9134 | 1.85 | 5.31E-03 |
| | | | NM_057735 | | | |
| | | | NM_057749 | | | |
| NRXN1 | 209914_s_at | AW149405 | NM_004801 | 9378 | 1.84 | 2.88E-03 |
| | | | NM_138735 | | | |
| ETV1 | 217061_s_at | AC004857 | NM_004956 | 2115 | 1.83 | 4.93E-04 |
| FAM19A5 | 229459_at | AV723914 | NM_015381 | 25817 | 1.82 | 1.07E-06 |
| ITPR1 | 240052_at | AA648993 | NM_002222 | 3708 | 1.82 | 7.08E-04 |
| ZAK | 222757_s_at | AB030034 | NM_016653 | 51776 | 1.81 | 3.74E-04 |
| | | | NM_133646 | | | |
| FGFR2 | 208228_s_at | M87771 | NM_000141 | 2263 | 1.81 | 1.44E-03 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| ZNF545 | 1555793_a_at | AL834267 | NM_133466 | 284406 | 1.80 | 4.60E-04 |
| AW024890 | 229942_at | AW024890 | --- | --- | 1.79 | 6.14E-05 |
| AI733341 | 240911_at | AI733341 | --- | --- | 1.79 | 7.62E-04 |
| PPAP2B | 209355_s_at | AB000889 | NM_003713 | 8613 | 1.79 | 4.28E-04 |
| | | | NM_177414 | | | |
| TBX3 | 222917_s_at | U69556 | NM_005996 | 6926 | 1.79 | 9.78E-06 |
| | | | NM_016569 | | | |
| TRIP10 | 202734_at | NM_004240 | NM_004240 | 9322 | 1.79 | 8.00E-03 |
| TCF7L2 | 212762_s_at | AI375916 | NM_030756 | 6934 | 1.79 | 3.86E-07 |
| PELO | 214660_at | X68742 | NM_015946 | 53918 | 1.78 | 2.39E-03 |
| GPR51 | 217077_s_at | AF095723 | NM_005458 | 9568 | 1.78 | 3.16E-04 |
| FBLN1 | 202994_s_at | Z95331 | NM_001996 | 2192 | 1.77 | 2.59E-03 |
| | | | NM_006485 | | | |
| | | | NM_006486 | | | |
| | | | NM_006487 | | | |
| DOK5 | 1554863_s_at | BC008992 | NM_018431 | 55816 | 1.77 | 3.36E-03 |
| | | | NM_177959 | | | |
| NRXN1 | 209915_s_at | AB035356 | NM_004801 | 9378 | 1.76 | 7.05E-03 |
| | | | NM_138735 | | | |
| FLJ25067 | 243507_s_at | AV715251 | NM_152504 | 149840 | 1.76 | 6.53E-07 |
| DNAPTP6 | 222154_s_at | AK002064 | NM_015535 | 26010 | 1.76 | 4.90E-04 |
| MGC21654 | 1556429_a_at | BC026969 | NM_145647 | 93594 | 1.76 | 6.54E-03 |
| CPNE4 | 228796_at | BE645967 | NM_130808 | 131034 | 1.75 | 4.64E-04 |
| POSTN | 1555778_a_at | AY140646 | NM_006475 | 10631 | 1.75 | 4.56E-05 |
| ATOH8 | 228890_at | BF434995 | NM_032827 | 84913 | 1.75 | 7.05E-04 |
| ALCAM | 1569362_at | BC041127 | NM_001627 | 214 | 1.75 | 9.54E-03 |
| HTATSF1 | 202601_s_at | AI373539 | NM_014500 | 27336 | 1.75 | 5.33E-05 |
| MYD88 | 209124_at | U70451 | NM_002468 | 4615 | 1.74 | 3.20E-04 |
| PPAP2B | 212230_at | AV725664 | NM_003713 | 8613 | 1.74 | 1.11E-05 |
| | | | NM_177414 | | | |
| ZNF253 | 242919_at | AV683221 | NM_021047 | 114977 | 1.73 | 1.13E-03 |
| LOC114977 | | | | 56242 | | |
| DCLRE1B | 219490_s_at | NM_022836 | NM_022836 | 64858 | 1.73 | 3.54E-03 |
| TBX3 | 229576_s_at | N29712 | NM_005996 | 6926 | 1.72 | 6.02E-07 |
| | | | NM_016569 | | | |
| KHDRBS3 | 230249_at | AI650382 | NM_006558 | 10656 | 1.72 | 3.44E-04 |
| FLJ39609 | 1555980_a_at | CA447406 | XM_379692 | 284598 | 1.72 | 9.03E-03 |
| BF110268 | 238178_at | BF110268 | --- | --- | 1.71 | 1.80E-04 |
| KLF7 | 238482_at | AL120562 | NM_003709 | 8609 | 1.71 | 1.93E-03 |
| ELL2 | 226099_at | AI924426 | NM_012081 | 22936 | 1.71 | 8.96E-04 |
| KHDRBS3 | 209781_s_at | AF069681 | NM_006558 | 10656 | 1.71 | 3.55E-05 |
| TRIM37 | 1569114_at | BC011742 | NM_00100520 7 | 4591 | 1.71 | 3.38E-03 |
| | | | NM_015294 | | | |
| DLL3 | 222898_s_at | BE350882 | NM_016941 | 10683 | 1.70 | 5.19E-07 |
| | | | NM_203486 | | | |
| SNAI2 | 213139_at | AI572079 | NM_003068 | 6591 | 1.70 | 2.74E-03 |
| FAM19A5 | 229655_at | N66656 | NM_015381 | 25817 | 1.70 | 4.35E-05 |
| ZNF560 | 1553276_at | NM_152476 | NM_152476 | 147741 | 1.70 | 2.56E-04 |
| TBX3 | 219682_s_at | NM_016569 | NM_005996 | 6926 | 1.69 | 1.06E-06 |
| | | | NM_016569 | | | |
| C20orf23 | 232083_at | AB046810 | NM_024704 | 55614 | 1.69 | 8.35E-06 |
| AF131796 | 231916_at | AF131796 | --- | --- | 1.69 | 2.15E-03 |
| NSE1 | 229546_at | AI378035 | NM_145175 | 151354 | 1.69 | 2.85E-08 |
| CXorf34 | 205238_at | NM_024917 | NM_024917 | 79979 | 1.68 | 6.46E-03 |
| CRH | 205629_s_at | BC002599 | NM_000756 | 1392 | 1.68 | 5.48E-03 |
| NR3C1 | 216321_s_at | X03348 | NM_000176 | 2908 | 1.68 | 5.45E-07 |
| PTGFRN | 224937_at | BF311866 | NM_020440 | 5738 | 1.67 | 6.89E-04 |
| ARG99 | 226322_at | BF109231 | NM_031920 | 83857 | 1.67 | 4.14E-03 |
| | | | NM_175861 | | | |
| NXN | 243503_at | AI632740 | NM_022463 | 64359 | 1.67 | 2.57E-04 |
| STK17A | 202695_s_at | NM_004760 | NM_004760 | 9263 | 1.66 | 2.52E-03 |
| C11orf8 | 205413_at | NM_001584 | NM_001584 | 744 | 1.66 | 1.85E-05 |
| PI15 | 229947_at | AI088609 | NM_015886 | 51050 | 1.66 | 1.62E-04 |
| NXT2 | 209629_s_at | AF201942 | NM_018698 | 55916 | 1.65 | 3.43E-04 |
| CDC25B | 201853_s_at | NM_021873 | NM_004358 | 994 | 1.65 | 4.80E-05 |
| | | | NM_021872 | | | |
| | | | NM_021873 | | | |
| | | | NM_021874 | | | |
| | | | NM_212530 | | | |
| TPST2 | 204079_at | NM_003595 | NM_00100856 6 | 8459 | 1.65 | 2.97E-04 |
| | | | NM_003595 | | | |
| LOC344595 | 235606_at | AA417117 | XM_298151 | 344595 | 1.64 | 2.58E-05 |
| H22448 | 242530_at | H22448 | --- | --- | 1.64 | 2.20E-04 |
| BF981643 | 214720_x_at | BF981643 | NM_144710 | 151011 | 1.64 | 1.45E-04 |
| | | | NM_178584 | | | |
| LOC93349 | 214791_at | AK023116 | NM_138402 | 93349 | 1.64 | 9.50E-05 |
| DOK4 | 209690_s_at | BF591163 | NM_018110 | 55715 | 1.64 | 7.06E-06 |
| LZTS1 | 47550_at | N21184 | NM_021020 | 11178 | 1.63 | 3.08E-04 |
| TBX3 | 225544_at | AI806338 | NM_005996 | 6926 | 1.63 | 5.24E-06 |
| | | | NM_016569 | | | |
| FSTL1 | 208782_at | BC000055 | NM_007085 | 11167 | 1.63 | 2.00E-03 |
| EMILIN3 | 228307_at | AL137580 | NM_052846 | 90187 | 1.63 | 2.86E-03 |
| ATP2B4 | 212135_s_at | AW517686 | NM_00100139 6 | 493 | 1.63 | 4.64E-03 |
| | | | NM_001684 | | | |
| AL833029 | 1559444_at | AL833029 | --- | --- | 1.63 | 2.28E-03 |
| PDLIM5 | 203243_s_at | NM_006457 | NM_00101151 3 | 10611 | 1.62 | 2.91E-05 |
| | | | NM_00101151 4 | | | |
| | | | NM_00101151 5 | | | |
| | | | NM_00101151 6 | | | |
| | | | NM_006457 | | | |
| TBC1D19 | 220260_at | NM_018317 | NM_018317 | 55296 | 1.62 | 6.08E-05 |
| FZD5 | 206136_at | NM_003468 | NM_003468 | 7855 | 1.62 | 1.51E-03 |
| PCDH9 | 219737_s_at | AI524125 | NM_020403 | 5101 | 1.62 | 3.72E-03 |
| | | | NM_203487 | | | |
| PDLIM5 | 212412_at | AV715767 | NM_00101151 3 | 10611 | 1.61 | 7.77E-04 |
| | | | NM_00101151 4 | | | |
| | | | NM_00101151 5 | | | |
| | | | NM_00101151 6 | | | |
| | | | NM_006457 | | | |
| ZNF537 | 223393_s_at | AL136805 | NM_020856 | 57616 | 1.61 | 2.15E-03 |
| AW052186 | 239942_at | AW052186 | --- | --- | 1.61 | 5.57E-03 |
| GPR51 | 209991_x_at | AF069755 | NM_005458 | 9568 | 1.60 | 1.65E-06 |
| BF509125 | 232198_at | BF509125 | --- | --- | 1.60 | 4.75E-03 |
| C14orf31 | 225464_at | N30138 | NM_152330 | 122786 | 1.60 | 3.92E-03 |
| PARVA | 217890_s_at | NM_018222 | NM_018222 | 55742 | 1.59 | 6.06E-03 |
| CBLB | 227900_at | AV701750 | NM-170662 | 868 | 1.59 | 2.60E-03 |
| EB-1 | 227441_s_at | AW005572 | NM_020140 | 56899 | 1.59 | 2.08E-04 |
| | | | NM_152788 | | | |
| | | | NM_181670 | | | |
| NAGA | 1555041_a_at | M29276 | NM_000262 | 4668 | 1.59 | 2.23E-06 |
| KIAA1713 | 233536_at | AI202664 | XM_290811 | 80816 | 1.59 | 9.66E-04 |
| HOMER3 | 215489_x_at | AI871287 | NM_004838 | 9454 | 1.58 | 2.88E-04 |
| NFIB | 233304_at | AU145782 | NM_005596 | 4781 | 1.58 | 6.95E-03 |
| SOX5 | 238009_at | AI446064 | NM_006940 | 6660 | 1.58 | 9.49E-06 |
| | | | NM_152989 | | | |
| | | | NM_178010 | | | |
| TCF7L2 | 216037_x_at | AA664011 | NM_030756 | 6934 | 1.57 | 2.43E-03 |
| LOC152573 | 229485_x_at | AI735586 | XM_496688 | 152573 | 1.57 | 6.34E-05 |
| DCBLD2 | 213865_at | AI378788 | NM_080927 | 131566 | 1.57 | 1.25E-04 |
| NS5ATP13TP2 | 1558613_at | BC040647 | NM_178507 | 220323 | 1.56 | 8.59E-03 |
| SAMHD1 | 204502_at | NM_015474 | NM_015474 | 25939 | 1.56 | 9.98E-05 |
| N50083 | 229110_at | N50083 | --- | --- | 1.56 | 4.18E-04 |
| LRRN3 | 209840_s_at | AI221950 | NM_018334 | 54674 | 1.55 | 9.99E-07 |
| CADPS | 233950_at | AK000873 | NM_003716 | 8618 | 1.55 | 1.09E-04 |
| | | | NM_183393 | | | |
| | | | NM_183394 | | | |
| ELL2 | 226982_at | AI745624 | NM_012081 | 22936 | 1.55 | 3.35E-03 |
| HRH3 | 220447_at | NM_007232 | NM_007232 | 11255 | 1.54 | 6.15E-04 |
| LRRN3 | 209841_s_at | AL442092 | NM_018334 | 54674 | 1.54 | 6.25E-07 |
| PLK4 | 211088_s_at | Z25433 | NM_014264 | 10733 | 1.53 | 1.15E-03 |
| NOS1 | 239132_at | BE299830 | NM_000620 | 4842 | 1.53 | 1.65E-03 |
| PLEKHF2 | 218640_s_at | NM_024613 | NM_024613 | 79666 | 1.53 | 1.36E-03 |
| MGC72104 | 243689_s_at | AI681945 | NM_207350 | 284802 | 1.52 | 1.75E-04 |
| NOS1 | 207309_at | NM_000620 | NM_000620 | 4842 | 1.52 | 2.45E-04 |
| C1orf43 | 1555225_at | BC008306 | NM_015449 | 25912 | 1.52 | 2.71E-03 |
| | | | NM_138740 | | | |
| GPR161 | 206972_s_at | NM_007369 | NM_007369 | 23432 | 1.52 | 4.09E-03 |
| | | | NM_153832 | | | |
| ODZ3 | 219523_s_at | NM_018104 | XM_371717 | 55714 | 1.51 | 2.29E-04 |
| DOK4 | 209691_s_at | BC003541 | NM_018110 | 55715 | 1.50 | 1.43E-04 |
| TRIM68 | 219405_at | NM_018073 | NM_018073 | 55128 | 1.50 | 2.70E-05 |
| DPYSL3 | 201431_s_at | NM_001387 | NM_001387 | 1809 | 1.50 | 7.17E-03 |
| RBL1 | 1559307_s_at | BG387892 | NM_002895 | 5933 | 1.50 | 2.27E-03 |
| | | | NM_183404 | | | |
| MYO6 | 210480_s_at | U90236 | NM_004999 | 4646 | 1.50 | 1.85E-05 |
| SYNGR1 | 210613_s_at | BC000731 | NM_004711 | 9145 | 1.50 | 8.09E-03 |
| | | | NM_145731 | | | |
| | | | NM_145738 | | | |
| CDO1 | 204154_at | NM_001801 | NM_001801 | 1036 | 1.50 | 3.73E-04 |
| NXT2 | 209628_at | AK023289 | NM_018698 | 55916 | 1.49 | 1.70E-03 |
| ACO1 | 207071_s_at | NM_002197 | NM_002197 | 48 | 1.49 | 5.20E-03 |
| STOM | 201060_x_at | AI537887 | NM_004099 | 2040 | 1.49 | 3.02E-04 |
| | | | NM_198194 | | | |
| ZNF503 | 227195_at | AA603467 | NM_032772 | 84858 | 1.49 | 8.67E-07 |
| CBX5 | 242069_at | BE568225 | NM_012117 | 23468 | 1.48 | 3.23E-04 |
| PPAP2B | 212226_s_at | AA628586 | NM_003713 | 8613 | 1.48 | 2.29E-03 |
| | | | NM_177414 | | | |
| C20orf102 | 226973_at | AA206763 | NM_080607 | 128434 | 1.47 | 1.33E-04 |
| CDKN2C | 211792_s_at | U17074 | NM_001262 | 1031 | 1.47 | 7.95E-03 |
| | | | NM_078626 | | | |
| PDZRN3 | 212915_at | AL569804 | NM_015009 | 23024 | 1.47 | 5.49E-04 |
| CRYGD | 207532_at | NM_006891 | NM_006891 | 1421 | 1.46 | 3.28E-03 |
| AW207243 | 238617_at | AW207243 | --- | --- | 1.46 | 3.92E-09 |
| EIF5A2 | 235296_at | BG500474 | NM_020390 | 56648 | 1.46 | 5.19E-09 |
| ANKRD5 | 220144_s_at | NM_022096 | NM_022096 | 63926 | 1.45 | 8.13E-04 |
| | | | NM_198798 | | | |
| UNQ470 | 228403_at | AL541276 | NM_198573 | 375704 | 1.45 | 3.48E-03 |
| KCNJ8 | 205303_at | BF514158 | NM_004982 | 3764 | 1.45 | 5.86E-05 |
| ID1 | 208937_s_at | D13889 | NM_002165 | 3397 | 1.45 | 1.41E-04 |
| | | | NM_181353 | | | |
| SALL4 | 229661_at | NM_020436 | NM_020436 | 57167 | 1.44 | 2.91E-05 |
| DPYSL3 | 201430_s_at | W72516 | NM_001387 | 1809 | 1.44 | 2.58E-03 |
| MSX2 | 210319_x_at | D89377 | NM_002449 | 4488 | 1.44 | 1.48E-03 |
| FNDC5 | 226096_at | AI760132 | NM_153756 | 252995 | 1.44 | 7.61E-04 |
| ZNF239 | 206261_at | NM_005674 | NM_005674 | 8187 | 1.44 | 6.22E-05 |
| COBLL1 | 203642_s_at | NM_014900 | NM_014900 | 22837 | 1.44 | 6.55E-03 |
| KIAA1524 | 1553810_a_at | NM_020890 | NM_020890 | 57650 | 1.44 | 8.59E-03 |
| AL038824 | 213676_at | AL038824 | XM_496810 | 441151 | 1.44 | 6.78E-04 |
| ARL6 | 235320_at | AL138043 | NM_032146 | 84100 | 1.44 | 4.10E-07 |
| | | | NM_177976 | | | |
| MGC15937 | 227794_at | BF432254 | NM_080661 | 92292 | 1.43 | 3.16E-03 |
| BE671123 | 235798_at | BE671123 | --- | --- | 1.43 | 4.96E-06 |
| ARL6 | 223735_at | AL136815 | NM_032146 | 84100 | 1.43 | 7.57E-05 |
| | | | NM_177976 | | | |
| LOC221810 | 221911_at | BE881590 | --- | 221810 | 1.43 | 2.72E-03 |
| SEZ6L | 207873_x_at | NM_021115 | NM_021115 | 23544 | 1.42 | 2.45E-05 |
| DOK4 | 207747_s_at | NM_018110 | NM_018110 | 55715 | 1.42 | 3.44E-05 |
| SLC1A1 | 206396_at | NM_004170 | NM_004170 | 6505 | 1.42 | 4.65E-03 |
| NSE1 | 225667_s_at | AI601101 | NM_145175 | 151354 | 1.42 | 1.15E-03 |
| ARHGAP24 | 230803_s_at | AI761947 | NM_031305 | 83478 | 1.42 | 3.92E-03 |
| C6orf152 | 242006_at | AI379143 | NMJ81714 | 167691 | 1.42 | 6.09E-03 |
| LOC132671 | 229331_at | AI559300 | NM_145263 | 132671 | 1.41 | 1.82E-03 |
| TH | 208291_s_at | NM_000360 | NM_000360 | 7054 | 1.40 | 7.49E-04 |
| | | | NM_199292 | | | |
| | | | NM_199293 | | | |
| RBL1 | 1555004_a_at | BC032247 | NM_002895 | 5933 | 1.40 | 5.16E-03 |
| | | | NM_183404 | | | |
| SFRP1 | 202037_s_at | NM_003012 | NM_003012 | 6422 | 1.40 | 5.74E-05 |
| CTNNA1 | 1558214_s_at | BG330076 | NM_001903 | 1495 | 1.40 | 5.25E-07 |
| CPT1A | 210688_s_at | BC000185 | NM_001876 | 1374 | 1.40 | 1.43E-03 |
| AI917901 | 243140_at | AI917901 | --- | --- | 1.40 | 1.50E-04 |
| HES1 | 203394_s_at | BE973687 | NM_005524 | 3280 | 1.40 | 4.39E-06 |
| GALNT7 | 222587_s_at | BF699855 | NM_017423 | 51809 | 1.40 | 1.79E-03 |
| MGC33846 | 231430_at | AW205640 | NM_175885 | 220382 | 1.39 | 1.42E-03 |
| SEZ6L | 211894_x_at | AB041736 | NM_021115 | 23544 | 1.39 | 9.95E-07 |
| CHML | 206079_at | NM_001821 | NM_001821 | 1122 | 1.39 | 8.74E-03 |
| EGLN3 | 222847_s_at | AI378406 | NM_022073 | 112399 | 1.39 | 3.90E-03 |
| MDM1 | 213761_at | AW664850 | NM_017440 | 56890 | 1.38 | 1.18E-04 |
| | | | NM_020128 | | | |
| SFRP1 | 202035_s_at | AI332407 | NM_003012 | 6422 | 1.38 | 3.39E-03 |
| CBLN1 | 205747_at | NM_004352 | NM_004352 | 869 | 1.38 | 1.13E-03 |
| PDLIM5 | 211681_s_at | AF116705 | NM_00101151 3 | 10611 | 1.38 | 1.41E-04 |
| | | | NM_00101151 4 | | | |
| | | | NM_00101151 5 | | | |
| | | | NM_00101151 6 | | | |
| | | | NM_006457 | | | |
| NQO1 | 210519_s_at | BC000906 | NM_000903 | 1728 | 1.38 | 4.39E-03 |
| ST8SIA1 | 210073_at | L32867 | NM_003034 | 6489 | 1.38 | 9.29E-04 |
| AL359594 | 1566867_at | AL359594 | --- | --- | 1.38 | 4.68E-03 |
| DLEU2 | 216870_x_at | AF264787 | NM_006021 | 8847 | 1.38 | 2.48E-04 |
| BF966021 | 212698_s_at | BF966021 | NM_144710 | 151011 | 1.37 | 4.75E-04 |
| | | | NM_178584 | | | |
| C14orf125 | 226510_at | BF435286 | XM_113763 | 25938 | 1.37 | 5.78E-03 |
| | | | XM_370759 | | | |
| TRIM37 | 1554001_at | BC036012 | NM_00100520 7 | 4591 | 1.37 | 1.10E-05 |
| | | | NM_015294 | | | |
| TLE3 | 228340_at | BE967118 | NM_005078 | 7090 | 1.36 | 2.10E-03 |
| SFRP1 | 202036_s_at | AF017987 | NM_003012 | 6422 | 1.36 | 1.13E-03 |
| C5orf13 | 222344_at | AW972765 | NM_004772 | 9315 | 1.36 | 3.74E-03 |
| SLC8A3 | 1562403_a_at | AK096553 | NM_033262 | 6547 | 1.36 | 1.60E-04 |
| | | | NM_058240 | | | |
| | | | NM_182932 | | | |
| | | | NM_182933 | | | |
| | | | NM_182936 | | | |
| | | | NM_183002 | | | |
| FGFR2 | 208234_x_at | NM_022976 | NM_000141 | 2263 | 1.36 | 2.95E-03 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| DLL3 | 219537_x_at | NM_016941 | NM_016941 | 10683 | 1.35 | 4.38E-05 |
| | | | NM_203486 | | | |
| CUGBP2 | 202156_s_at | N36839 | NM_006561 | 10659 | 1.35 | 2.49E-03 |
| APBB2 | 213419_at | U62325 | NM_173075 | 323 | 1.35 | 5.44E-04 |
| DOCK10 | 219279_at | NM_017718 | NM_014689 | 55619 | 1.34 | 5.78E-05 |
| MAPK12 | 206106_at | AL022328 | NM_002969 | 6300 | 1.34 | 2.33E-06 |
| TLE3 | 212770_at | AW873621 | NM_005078 | 7090 | 1.34 | 8.20E-04 |
| BE670307 | 227955_s_at | BE670307 | --- | --- | 1.34 | 1.89E-05 |
| HGF | 210998_s_at | M77227 | NM_000601 | 3082 | 1.34 | 9.86E-03 |
| | | | NM_00101093 1 | | | |
| | | | NM_00101093 2 | | | |
| | | | NM_001 01093 3 | | | |
| | | | NM_001 01093 4 | | | |
| FLJ11259 | 218627_at | NM_018370 | NM_018370 | 55332 | 1.33 | 4.22E-03 |
| AI826125 | 229866_at | AI826125 | --- | --- | 1.33 | 4.02E-04 |
| SEZ6L | 240709_at | AW204757 | NM_021115 | 23544 | 1.33 | 5.75E-03 |
| RAB27B | 228708_at | BF438386 | NM_004163 | 5874 | 1.33 | 9.45E-03 |
| MAP3K1 | 225927_at | AA541479 | XM_042066 | 4214 | 1.33 | 1.90E-03 |
| AL040051 | 225481_at | AL040051 | --- | --- | 1.33 | 5.84E-04 |
| BE967311 | 226225_at | BE967311 | --- | --- | 1.32 | 2.96E-05 |
| TLE4 | 204872_at | NM_007005 | NM_007005 | 7091 | 1.32 | 4.50E-03 |
| SNCAIP | 219511_s_at | NM_005460 | NM_005460 | 9627 | 1.32 | 3.58E-04 |
| KIAA0376 | 212480_at | AB002374 | NM_015330 | 23384 | 1.32 | 2.79E-05 |
| LZTS1 | 219042_at | NM_021020 | NM_021020 | 11178 | 1.32 | 3.07E-04 |
| KIAA0318 | 238817_at | BF063412 | NM_015347 | 23504 | 1.32 | 5.00E-04 |
| RNF34 | 219035_s_at | NM_025126 | NM_025126 | 80196 | 1.32 | 1.40E-03 |
| | | | NM_194271 | | | |
| STXBP4 | 244404_at | AI376134 | NM_178509 | 252983 | 1.31 | 3.35E-05 |
| AU144956 | 232501_at | AU144956 | --- | --- | 1.31 | 6.46E-06 |
| FLJ11127 | 219694_at | NM_019018 | NM_019018 | 54491 | 1.31 | 1.32E-03 |
| MITF | 226066_at | AL117653 | NM_000248 | 4286 | 1.31 | 1.07E-03 |
| | | | NM_006722 | | | |
| | | | NM_198158 | | | |
| | | | NM_198159 | | | |
| | | | NM_198177 | | | |
| | | | NM_198178 | | | |
| ETS1 | 224833_at | BE218980 | NM_005238 | 2113 | 1.31 | 2.75E-03 |
| NANOS1 | 228523_at | AW970089 | NM_00100955 3 | 340719 | 1.31 | 1.30E-03 |
| | | | NM_199461 | | | |
| FAM19A5 | 237094_at | AI953086 | NM_015381 | 25817 | 1.31 | 2.32E-07 |
| LBH | 221011_s_at | NM_030915 | NM_030915 | 81606 | 1.31 | 1.22E-04 |
| PEX26 | 219180_s_at | AI817074 | NM_017929 | 55670 | 1.30 | 3.74E-03 |
| GALNT7 | 218313_s_at | NM_017423 | NM_017423 | 51809 | 1.30 | 1.80E-04 |
| C2orf23 | 204365_s_at | NM_022912 | NM_022912 | 65055 | 1.30 | 5.58E-03 |
| AL049709 | 216548_x_at | AL049709 | --- | --- | 1.30 | 5.30E-04 |
| MGC34032 | 1552782_at | AK091400 | NM_152697 | 204962 | 1.30 | 1.97E-03 |
| DLL1 | 224215_s_at | AF196571 | NM_005618 | 28514 | 1.30 | 1.62E-04 |
| TMEM20 | 236219_at | AI452512 | NM_153226 | 159371 | 1.30 | 3.29E-03 |
| AW305300 | 238962_at | AW305300 | --- | --- | 1.30 | 4.31E-04 |
| C20orf23 | 219570_at | NM_024704 | NM_024704 | 55614 | 1.29 | 1.18E-03 |
| DRIM | 209725_at | AF072718 | NM_014503 | 27340 | 1.29 | 1.99E-03 |
| CBLB | 209682_at | U26710 | NM_170662 | 868 | 1.29 | 3.69E-03 |
| STRA6 | 1569335_a_at | BC015881 | NM_022369 | 64220 | 1.29 | 9.19E-04 |
| CRTAC1 | 221204_s_at | NM_018058 | NM_018058 | 55118 | 1.28 | 5.56E-04 |
| AF070602 | 216214_at | AF070602 | --- | --- | 1.28 | 5.02E-03 |
| NAGA | 202943_s_at | M38083 | NM_000262 | 4668 | 1.28 | 1.26E-03 |
| AK025909 | 233814_at | AK025909 | --- | --- | 1.27 | 1.16E-04 |
| AI141426 | 229898_at | AI141426 | --- | --- | 1.27 | 6.87E-05 |
| SLC8A1 | 241752_at | AA094434 | NM_021097 | 6546 | 1.27 | 6.10E-04 |
| INSIG1 | 201625_s_at | BE300521 | NM_005542 | 3638 | 1.26 | 8.33E-03 |
| | | | NM_198336 | | | |
| | | | NM_198337 | | | |
| IQGAP1 | 210840_s_at | D29640 | NM_003870 | 8826 | 1.26 | 2.60E-04 |
| GAP43 | 204471_at | NM_002045 | NM_002045 | 2596 | 1.26 | 5.02E-04 |
| PDLIM5 | 213684_s_at | BF671400 | NM_00101151 3 | 10611 | 1.26 | 9.47E-04 |
| | | | NM_00101151 4 | | | |
| | | | NM_00101151 5 | | | |
| | | | NM_00101151 6 | | | |
| | | | NM_006457 | | | |
| GAP43 | 216963_s_at | AF279774 | NM_002045 | 2596 | 1.26 | 5.66E-03 |
| KLHL5 | 226001_at | AK002174 | NM_00100707 5 | 51088 | 1.26 | 3.67E-04 |
| | | | NM_199039 | | | |
| RGS4 | 204339_s_at | BC000737 | NM_005613 | 5999 | 1.25 | 4.16E-04 |
| AF279774 | 216967_at | AF279774 | --- | --- | 1.25 | 9.39E-03 |
| PTER | 218967_s_at | NM_030664 | NM_00100148 4 | 9317 | 1.25 | 1.22E-03 |
| | | | NM_030664 | | | |
| TLE3 | 212769_at | AI567426 | NM_005078 | 7090 | 1.25 | 3.78E-03 |
| TLE3 | 206472_s_at | NM_005078 | NM_005078 | 7090 | 1.25 | 4.37E-03 |
| RGS4 | 204338_s_at | NM_005613 | NM_005613 | 5999 | 1.25 | 3.77E-04 |
| CPT1A | 203634_s_at | NM_001876 | NM_001876 | 1374 | 1.25 | 1.93E-03 |
| ASAHL | 227135_at | AI436803 | NM_014435 | 27163 | 1.25 | 9.20E-06 |
| LOC344595 | 239466_at | AA463827 | XM_298151 | 344595 | 1.25 | 4.65E-05 |
| TH1L | 225006_x_at | AJ238379 | NM_016397 | 51497 | 1.24 | 9.26E-03 |
| | | | NM_198976 | | | |
| SEZ6L | 216047_x_at | AL050253 | NM_021115 | 23544 | 1.24 | 1.75E-05 |
| NSE1 | 234331_s_at | AK025063 | NM_145175 | 151354 | 1.24 | 1.19E-04 |
| LRRIQ2 | 219886_at | NM_024548 | NM_024548 | 79598 | 1.24 | 1.44E-03 |
| GALNT10 | 207357_s_at | NM_017540 | NM_017540 | 55568 | 1.24 | 2.18E-03 |
| | | | NM_198321 | | | |
| BF815640 | 238517_at | BF815640 | --- | --- | 1.23 | 4.48E-03 |
| SLC30A9 | 229500_at | AI609256 | NM_006345 | 10463 | 1.23 | 2.32E-03 |
| LOC63929 | 227910_at | AI635379 | NM_022098 | 63929 | 1.23 | 1.48E-04 |
| CSE1L | 210765_at | AF053640 | NM_001316 | 1434 | 1.23 | 7.45E-03 |
| | | | NM_177436 | | | |
| BCL2L11 | 1558143_a_at | AK027160 | NM_006538 | 10018 | 1.23 | 6.27E-03 |
| | | | NM_138621 | | | |
| | | | NM_138622 | | | |
| | | | NM_138623 | | | |
| | | | NM_138624 | | | |
| | | | NM_138625 | | | |
| | | | NM_138626 | | | |
| | | | NM_138627 | | | |
| | | | NM_207002 | | | |
| | | | NM_207003 | | | |
| TM4SF14 | 221002_s_at | NM_030927 | NM_030927 | 81619 | 1.22 | 4.04E-03 |
| SLIT3 | 203813_s_at | NM_003062 | NM_003062 | 6586 | 1.22 | 1.58E-06 |
| RAPGEF5 | 204681_s_at | NM_012294 | XM_499059 | 9771 | 1.22 | 6.58E-04 |
| ZNF184 | 213452_at | AI811577 | NM_007149 | 7738 | 1.22 | 9.16E-03 |
| PCYT2 | 209577_at | BC000351 | NM_002861 | 5833 | 1.22 | 2.05E-03 |
| WDR51A | 234749_s_at | AL117629 | NM_015426 | 25886 | 1.22 | 2.87E-03 |
| MAN2A1 | 226538_at | AV700323 | NM_002372 | 4124 | 1.21 | 3.05E-03 |
| TLE4 | 233575_s_at | AA705845 | NM_007005 | 7091 | 1.21 | 2.34E-04 |
| AW665447 | 230113_at | AW665447 | --- | --- | 1.21 | 3.86E-03 |
| SAP30 | 204899_s_at | BF247098 | NM_003864 | 8819 | 1.21 | 2.33E-03 |
| LOC221810 | 221910_at | BF131965 | --- | 221810 | 1.21 | 8.18E-03 |
| HOMER3 | 204647_at | NM_004838 | NM_004838 | 9454 | 1.20 | 4.61E-03 |
| SLC1A1 | 213664_at | AW235061 | NM_004170 | 6505 | 1.20 | 5.98E-03 |
| C20orf108 | 224690_at | BG432350 | NM_080821 | 116151 | 1.20 | 8.73E-03 |
| ZNF238 | 207164_s_at | NM_006352 | NM_006352 | 10472 | 1.20 | 7.96E-03 |
| | | | NM_205768 | | | |
| PIR | 207469_s_at | NM_003662 | NM_003662 | 8544 | 1.20 | 1.53E-05 |
| FLRT3 | 219250_s_at | NM_013281 | NM_013281 | 23767 | 1.19 | 7.10E-04 |
| | | | NM_198391 | | | |
| SLIT3 | 203812_at | AB011538 | NM_003062 | 6586 | 1.19 | 1.40E-03 |
| TGIF | 203313_s_at | NM_003244 | NM_003244 | 7050 | 1.19 | 5.40E-05 |
| | | | NM_170695 | | | |
| | | | NM_173207 | | | |
| | | | NM_173208 | | | |
| | | | NM_173209 | | | |
| | | | NM_173210 | | | |
| | | | NM_173211 | | | |
| | | | NM_174886 | | | |
| CDKN2C | 204159_at | NM_001262 | NM_001262 | 1031 | 1.19 | 8.59E-05 |
| | | | NM_078626 | | | |
| MTUS1 | 212095_s_at | BE552421 | NM_00100192 4 | 57509 | 1.19 | 2.48E-04 |
| | | | NM_00100192 5 | | | |
| | | | NM_00100192 7 | | | |
| | | | NM_00100193 1 | | | |
| | | | NM_020749 | | | |
| SCARA3 | 223842_s_at | AB007830 | NM_016240 | 51435 | 1.19 | 3.33E-04 |
| | | | NM_182826 | | | |
| HTATSF1 | 202602_s_at | NM_014500 | NM_014500 | 27336 | 1.19 | 2.42E-05 |
| TRAM2 | 202369_s_at | NM_012288 | NM_012288 | 9697 | 1.18 | 7.27E-03 |
| PTGFRN | 224950_at | BF476250 | NM_020440 | 5738 | 1.18 | 2.73E-03 |
| DRD2 | 216938_x_at | S69899 | NM_000795 | 1813 | 1.18 | 2.02E-03 |
| | | | NM_016574 | | | |
| TOP3B | 215781_s_at | D87012 | NM_003935 | 8940 | 1.18 | 2.21E-03 |
| ACY1L2 | 225421_at | AI654133 | NM_001010852 | 135293 | 1.18 | 7.34E-04 |
| BC025350 | 1569418_at | BC025350 | --- | --- | 1.18 | 1.04E-06 |
| KIAA1706 | 225630_at | AB051493 | NM_030636 | 80820 | 1.18 | 1.55E-04 |
| AW874669 | 239834_at | AW874669 | --- | --- | 1.18 | 7.57E-04 |
| CPT1A | 203633_at | BF001714 | NM_001876 | 1374 | 1.17 | 1.23E-03 |
| C2orf23 | 204364_s_at | BE535746 | NM_022912 | 65055 | 1.17 | 2.59E-04 |
| SMO | 218629_at | NM_005631 | NM_005631 | 6608 | 1.17 | 3.37E-05 |
| RPH3A | 205230_at | NM_014954 | NM_014954 | 22895 | 1.17 | 6.35E-03 |
| VIL2 | 208621_s_at | BF663141 | NM_003379 | 7430 | 1.17 | 8.04E-04 |
| SEC15L2 | 225900_at | AW294630 | XM_039570 | 23233 | 1.17 | 7.12E-03 |
| MCC | 206132_at | NM_002387 | NM_002387 | 4163 | 1.17 | 5.91E-04 |
| C20orf108 | 224693_at | AI133137 | NM_080821 | 116151 | 1.16 | 2.04E-05 |
| RFC5 | 203209_at | BC001866 | NM_007370 | 5985 | 1.16 | 9.74E-03 |
| | | | NM_181578 | | | |
| MAPK11 | 211499_s_at | U92268 | NM_002751 | 5600 | 1.16 | 3.08E-04 |
| | | | NM_138993 | | | |
| EB-1 | 227439_at | AI806510 | NM_020140 | 56899 | 1.16 | 9.25E-08 |
| | | | NM_152788 | | | |
| | | | NM_181670 | | | |
| FBXL7 | 213249_at | AU145127 | NM_012304 | 23194 | 1.16 | 5.25E-07 |
| TTC9 | 213172_at | AW235608 | XM_027236 | 23508 | 1.16 | 7.95E-04 |
| SLC40A1 | 223044_at | AL136944 | NM_014585 | 30061 | 1.16 | 6.08E-04 |
| AI452715 | 229817_at | AI452715 | --- | --- | 1.16 | 3.41E-03 |
| MAN2A1 | 235103_at | AA029155 | NM_002372 | 4124 | 1.16 | 5.37E-04 |
| VIL2 | 217234_s_at | AF199015 | NM_003379 | 7430 | 1.16 | 3.24E-03 |
| SEZ6L | 213609_s_at | AB023144 | NM_021115 | 23544 | 1.15 | 2.65E-06 |
| TLX2 | 207410_s_at | NM_016170 | NM_001534 | 3196 | 1.15 | 3.55E-05 |
| | | | NM_016170 | | | |
| CGI-96 | 202937_x_at | AL022316 | NM_015703 | 27341 | 1.15 | 2.55E-03 |
| KCNJ8 | 205304_s_at | NM_004982 | NM_004982 | 3764 | 1.14 | 5.54E-03 |
| ZNF537 | 223392_s_at | BF510588 | NM_020856 | 57616 | 1.14 | 9.47E-06 |
| DHX35 | 234728_s_at | AL023803 | NM_021931 | 60625 | 1.14 | 6.62E-06 |
| STRA6 | 1569334_at | BC015881 | NM_022369 | 64220 | 1.14 | 5.40E-03 |
| EAF2 | 219551_at | NM_018456 | NM_018456 | 55840 | 1.14 | 9.77E-04 |
| ITPR1 | 203710_at | NM_002222 | NM_002222 | 3708 | 1.14 | 1.07E-03 |
| LIFR | 225575_at | AI680541 | NM_002310 | 3977 | 1.13 | 4.19E-06 |
| GJA7 | 208460_at | NM_005497 | NM_005497 | 10052 | 1.13 | 4.63E-03 |
| MBD2 | 214397_at | AI827820 | NM_003927 | 8932 | 1.13 | 1.61E-03 |
| | | | NM_015832 | | | |
| ANKRD13 | 224810_s_at | AL569476 | NM_033121 | 88455 | 1.13 | 2.65E-04 |
| HDAC8 | 223908_at | AF212246 | NM_018486 | 55869 | 1.13 | 1.96E-04 |
| STN2 | 227461_at | AA632295 | NM_033104 | 85439 | 1.12 | 4.18E-03 |
| BE464799 | 214036_at | BE464799 | --- | --- | 1.12 | 1.37E-04 |
| SOX2 | 228038_at | AI669815 | NM_003106 | 6657 | 1.12 | 1.75E-04 |
| GTF2E1 | 205930_at | NM_005513 | NM_005513 | 2960 | 1.12 | 1.67E-03 |
| H10661 | 242317 _at | H10661 | --- | --- | 1.12 | 3.16E-04 |
| HSZFP36 | 229732_at | AI417785 | XM_375568 | 55552 | 1.12 | 2.16E-03 |
| RBBP9 | 221440_s_at | NM_006606 | NM_006606 | 10741 | 1.12 | 9.04E-04 |
| | | | NM_153328 | | | |
| DNAJB6 | 209015_s_at | BC002446 | NM_005494 | 10049 | 1.12 | 2.51E-03 |
| | | | NM_058246 | | | |
| KNTC1 | 206316_s_at | NM_014708 | NM_014708 | 9735 | 1.12 | 7.31E-03 |
| FUT11 | 238551_at | BF541967 | NM_173540 | 170384 | 1.12 | 1.14E-03 |
| MAP2K6 | 205698_s_at | NM_002758 | NM_002758 | 5608 | 1.11 | 8.00E-03 |
| | | | NM_031988 | | | |
| NRM | 225592_at | D81048 | NM_007243 | 11270 | 1.11 | 8.62E-03 |
| STMN3 | 222557_at | AL353715 | NM_015894 | 50861 | 1.11 | 1.13E-03 |
| INSIG1 | 201626_at | BG292233 | NM_005542 | 3638 | 1.11 | 1.47E-03 |
| | | | NM_198336 | | | |
| | | | NM_198337 | | | |
| SH3MD2 | 225589_at | AB040927 | NM_020870 | 57630 | 1.11 | 3.78E-04 |
| BCL2L11 | 225606_at | AI949179 | NM_006538 | 10018 | 1.11 | 4.77E-07 |
| | | | NM_138621 | | | |
| | | | NM_138622 | | | |
| | | | NM_138623 | | | |
| | | | NM_138624 | | | |
| | | | NM_138625 | | | |
| | | | NM_138626 | | | |
| | | | NM_138627 | | | |
| | | | NM_207002 | | | |
| | | | NM_207003 | | | |
| TYRO3 | 211432_s_at | U05682 | NM_006293 | 7301 | 1.11 | 7.76E-03 |
| APAF1 | 204859_s_at | NM_013229 | NM_001160 | 317 | 1.11 | 8.54E-03 |
| | | | NM_013229 | | | |
| | | | NM_181861 | | | |
| | | | NM_181868 | | | |
| | | | NM_181869 | | | |
| LRRC1 | 218816_at | NM_018214 | NM_018214 | 55227 | 1.11 | 4.89E-03 |
| | | | NM_025168 | | | |
| DNM3 | 209839_at | AL136712 | NM_015569 | 26052 | 1.11 | 5.67E-06 |
| MAN2A1 | 205105_at | NM_002372 | NM_002372 | 4124 | 1.11 | 6.81E-03 |
| FLJ23322 | 220707_s_at | NM_024955 | NM_024955 | 80020 | 1.10 | 2.15E-03 |
| NDRG2 | 214279_s_at | W74452 | NM_016250 | 57447 | 1.10 | 5.44E-03 |
| | | | NM_201535 | | | |
| | | | NM_201536 | | | |
| | | | NM_201537 | | | |
| | | | NM_201538 | | | |
| | | | NM_201539 | | | |
| | | | NM_201540 | | | |
| | | | NM_201541 | | | |
| SCAM-1 | 207788_s_at | NM_005775 | NM_005775 | 10174 | 1.10 | 9.08E-03 |
| LMO1 | 206718_at | NM_002315 | NM_002315 | 4004 | 1.10 | 5.27E-04 |
| ITPR1 | 216944_s_at | U23850 | NM_002222 | 3708 | 1.10 | 8.15E-03 |
| PCNXL2 | 39650_s_at | AB007895 | NM_014801 | 80003 | 1.10 | 9.56E-05 |
| | | | NM_024938 | | | |
| RBBP9 | 232751_at | AL121893 | NM_006606 | 10741 | 1.10 | 8.94E-04 |
| | | | NM_153328 | | | |
| AP3B1 | 203142_s_at | NM_003664 | NM_003664 | 8546 | 1.09 | 2.19E-03 |
| SNAP29 | 222597_at | AI346639 | NM_004782 | 9342 | 1.09 | 1.46E-04 |
| C20orf161 | 1553960_at | CA447177 | NM_033421 | 90203 | 1.09 | 2.01E-03 |
| | | | NM_152897 | | | |
| LOC283464 | 232021_at | AI864273 | XM_290597 | 283464 | 1.09 | 1.32E-03 |
| AI589594 | 238694_at | AI589594 | --- | --- | 1.09 | 2.79E-05 |
| SOX2 | 213721_at | L07335 | NM_003106 | 6657 | 1.08 | 7.40E-04 |
| ST3GAL6 | 210942_s_at | AB022918 | NM_006100 | 10402 | 1.08 | 2.47E-04 |
| BG231758 | 227615_at | BG231758 | --- | --- | 1.08 | 5.95E-08 |
| FLJ33814 | 225644_at | BF060776 | NM_173510 | 150275 | 1.08 | 2.63E-06 |
| TYMS | 1554696_s_at | AB077208 | NM_001071 | 7298 | 1.08 | 7.83E-03 |
| BOK | 223349_s_at | BE614255 | NM_032515 | 666 | 1.08 | 3.19E-06 |
| C1QL1 | 205575_at | NM_006688 | NM_006688 | 10882 | 1.08 | 1.06E-04 |
| GMPS | 236648_at | AI684467 | NM_003875 | 8833 | 1.08 | 6.96E-04 |
| AI733583 | 243235_at | AI733583 | --- | --- | 1.08 | 6.94E-03 |
| BE066500 | 239729_at | BE066500 | --- | --- | 1.08 | 8.69E-04 |
| ASAHL | 232072_at | AK025371 | NM_014435 | 27163 | 1.08 | 3.73E-04 |
| SNX5 | 229981_at | AA131508 | NM_014426 | 27131 | 1.08 | 1.66E-05 |
| | | | NM_152227 | | | |
| CYBA | 203028_s_at | NM_000101 | NM_000101 | 1535 | 1.07 | 5.76E-04 |
| PTER | 222798_at | BF112019 | NM_00100148 4 | 9317 | 1.07 | 2.48E-03 |
| | | | NM_030664 | | | |
| LOC440820 | 226602_s_at | T30183 | XM_496519 | 440820 | 1.07 | 2.20E-05 |
| AA214369 | 238545_at | AA214369 | --- | --- | 1.07 | 3.48E-03 |
| ELAVL4 | 241424_at | BF111117 | NM_021952 | 1996 | 1.07 | 4.96E-03 |
| C6orf189 | 228875_at | AI540210 | --- | 221303 | 1.07 | 1.85E-04 |
| PARG1 | 203910_at | NM_004815 | NM_004815 | 9411 | 1.07 | 1.74E-04 |
| OLFML3 | 218162_at | NM_020190 | NM_020190 | 56944 | 1.07 | 4.22E-03 |
| PI4K2B | 222631_at | AI862887 | NM_018323 | 55300 | 1.07 | 2.03E-03 |
| PHYHIPL | 226623_at | AI829726 | NM_032439 | 84457 | 1.07 | 5.85E-05 |
| AA156933 | 236141_at | AA156933 | --- | --- | 1.07 | 9.86E-04 |
| AI346432 | 229132_at | AI346432 | --- | --- | 1.07 | 3.94E-04 |
| AI919519 | 235092_at | AI919519 | --- | --- | 1.07 | 8.75E-03 |
| ELK3 | 221773_at | AW575374 | NM_005230 | 2004 | 1.07 | 2.45E-03 |
| LPPR4 | 213496_at | AW592563 | NM_014839 | 9890 | 1.07 | 1.23E-05 |
| EZH2 | 203358_s_at | NM_004456 | NM_004456 | 2146 | 1.06 | 5.13E-03 |
| | | | NM_152998 | | | |
| NS5ATP13TP2 | 225510_at | BG033561 | NM_178507 | 220323 | 1.06 | 1.40E-04 |
| KIAA1102 | 212325_at | AK027231 | NM_014988 | 22998 | 1.06 | 1.82E-09 |
| ZNF505 | 208119_s_at | NM_031218 | NM_00100412 6 | 81931 | 1.06 | 5.75E-03 |
| | | | NM_031218 | | | |
| C20orf40 | 231200_at | BG222606 | NM_144703 | 149986 | 1.06 | 1.73E-03 |
| SLC8A1 | 238546_at | BF344961 | NM_021097 | 6546 | 1.06 | 6.63E-03 |
| PREX1 | 224909_s_at | BF308645 | NM_020820 | 57580 | 1.06 | 7.16E-03 |
| MAK3 | 222393_s_at | AU149868 | NM_025146 | 80218 | 1.06 | 1.11E-05 |
| TMPO | 209753_s_at | BG391171 | NM_003276 | 7112 | 1.06 | 3.02E-03 |
| ADORA2A | 205013_s_at | NM_000675 | NM_000675 | 135 | 1.06 | 9.97E-03 |
| MGC3040 | 223523_at | BC000568 | NM_023943 | 66000 | 1.06 | 7.88E-07 |
| AI700633 | 212812_at | AI700633 | --- | --- | 1.06 | 8.55E-03 |
| TWSG1 | 219201_s_at | NM_020648 | NM_020648 | 57045 | 1.06 | 9.70E-04 |
| PPP3CB | 209817_at | M29550 | NM_021132 | 5532 | 1.06 | 2.71E-06 |
| MAP3K13 | 206249_at | NM_004721 | NM_004721 | 9175 | 1.06 | 3.52E-03 |
| SKP2 | 210567_s_at | BC001441 | NM_005983 | 6502 | 1.06 | 8.10E-04 |
| | | | NM_032637 | | | |
| SYN2 | 210315_at | AF077737 | NM_003178 | 6854 | 1.05 | 9.08E-03 |
| | | | NM_133625 | | | |
| CGI-96 | 33307_at | AL022316 | NM_015703 | 27341 | 1.05 | 7.16E-04 |
| GNG12 | 1555240_s_at | AF493879 | NM_018841 | 55970 | 1.05 | 6.35E-04 |
| CUTL2 | 213920_at | AB006631 | NM_015267 | 23316 | 1.04 | 1.63E-03 |
| FLRT3 | 222853_at | N71923 | NM_013281 | 23767 | 1.04 | 9.61E-07 |
| | | | NM_198391 | | | |
| FLJ13842 | 219877_at | NM_024645 | NM_024645 | 79698 | 1.04 | 6.38E-03 |
| ADRBK2 | 228771_at | AI651212 | NM_005160 | 157 | 1.04 | 7.18E-05 |
| SEC61A2 | 228747_at | AI694646 | NM_018144 | 55176 | 1.04 | 4.56E-03 |
| ST3GAL6 | 213355_at | AI989567 | NM_006100 | 10402 | 1.04 | 1.38E-04 |
| BF434228 | 235850_at | BF434228 | --- | --- | 1.04 | 4.97E-03 |
| PCGF5 | 227935_s_at | AA522681 | NM_032373 | 84333 | 1.04 | 8.11E-04 |
| MYST1 | 214885_at | AL050395 | NM_032188 | 84148 | 1.04 | 5.82E-03 |
| PEX13 | 1558163_at | BC040953 | NM_002618 | 5194 | 1.03 | 3.87E-03 |
| INSIG1 | 201627_s_at | NM_005542 | NM_005542 | 3638 | 1.03 | 2.57E-06 |
| | | | NM_198336 | | | |
| | | | NM_198337 | | | |
| NELL2 | 203413_at | NM_006159 | NM_006159 | 4753 | 1.03 | 1.75E-03 |
| RND3 | 212724_at | BG054844 | NM_005168 | 390 | 1.03 | 1.50E-03 |
| TFDP2 | 203588_s_at | BG034328 | NM_006286 | 7029 | 1.03 | 9.43E-03 |
| CTNNBL1 | 221021_s_at | NM_030877 | NM_030877 | 56259 | 1.03 | 3.20E-03 |
| CHML | 1565951_s_at | AJ293392 | NM_001821 | 1122 | 1.03 | 8.52E-03 |
| ITPR1 | 211323_s_at | L38019 | NM_002222 | 3708 | 1.02 | 3.21E-05 |
| DKFZP564O123 | 202536_at | AK002165 | NM_014043 | 25978 | 1.02 | 9.94E-05 |
| CKLF | 219161_s_at | NM_016951 | NM_016326 | 51192 | 1.02 | 8.63E-03 |
| | | | NM_016951 | | | |
| | | | NM_181640 | | | |
| | | | NM_181641 | | | |
| IQGAP1 | 213446_s_at | AI679073 | NM_003870 | 8826 | 1.02 | 1.01E-03 |
| C11orf8 | 215692_s_at | BE645386 | NM_001584 | 744 | 1.02 | 1.49E-03 |
| AL559474 | 235918_x_at | AL559474 | --- | --- | 1.02 | 2.13E-03 |
| ADRBK2 | 204184_s_at | NM_005160 | NM_005160 | 157 | 1.02 | 3.30E-04 |
| LOC152185 | 234995_at | AA668779 | NM_144718 | 152185 | 1.02 | 9.02E-04 |
| XRCC4 | 205071_x_at | AB017445 | NM_003401 | 7518 | 1.02 | 3.71E-03 |
| | | | NM_022406 | | | |
| | | | NM_022550 | | | |
| GPR125 | 210473_s_at | M37712 | NM_145290 | 166647 | 1.01 | 1.54E-08 |
| SLCO3A1 | 219229_at | NM_013272 | NM_013272 | 28232 | 1.01 | 7.19E-04 |
| POLE | 216026_s_at | AL080203 | NM_006231 | 5426 | 1.01 | 8.50E-03 |
| TMOD3 | 223078_s_at | AF177171 | NM_014547 | 29766 | 1.01 | 2.98E-05 |
| SKP2 | 203626_s_at | NM_005983 | NM_005983 | 6502 | 1.01 | 9.59E-08 |
| | | | NM_032637 | | | |
| RBPMS | | 207836_s_at NM_006867 | NM_00100871 0 | 11030 | 1.00 | 5.05E-03 |
| | | | NM_00100871 1 | | | |
| | | | NM_00100871 2 | | | |
| | | | NM_006867 | | | |
| PHTF2 | 217097_s_at | AC004990 | NM_020432 | 57157 | 1.00 | 4.53E-03 |
| TM4SF14 | 223314_at | BF025955 | NM_030927 | 81619 | 1.00 | 2.72E-03 |
| SDC2 | 212157_at | BE048514 | NM_002998 | 6383 | 1.00 | 6.35E-03 |
| TP53I3 | 210609_s_at | BC0004 74 | NM_004881 | 9540 | 1.00 | 8.78E-04 |
| | | | NM_147184 | | | |
| AL049385 | 232297_at | AL049385 | | --- | 1.00 | 1.36E-05 |
| MTUS1 | | 212096_s_at AL096842 | NM_00100192 4 | 57509 | 1.00 | 1.43E-04 |
| | | | NM_00100192 5 | | | |
| | | | NM_00100192 7 | | | |
| | | | NM_00100193 1 | | | |
| | | | NM_020749 | | | |
| KLF7 | 1555420_a_at | BC012919 | NM_003709 | 8609 | 1.00 | 1.21E-04 |
| ZNF555 | 1556187_at | AF052118 | NM_152791 | 148254 | 0.99 | 7.62E-03 |
| GPAM | 225424_at | AB046780 | NM_020918 | 57678 | 0.99 | 5.06E-03 |
| FLJ13273 | 1554518_at | BC032942 | NM_024751 | 79807 | 0.99 | 2.86E-04 |
| SAP30 | 204900_x_at | NM_003864 | NM_003864 | 8819 | 0.99 | 3.10E-03 |
| FBXL10 | 229855_at | AA779208 | NM_00100536 6 | 84678 | 0.99 | 1.80E-03 |
| | | | NM_032590 | | | |
| NEUROD4 | 221318_at | NM_021191 | NM_021191 | 58158 | 0.99 | 2.89E-03 |
| FLJ12649 | 219626_at | NM_024597 | XM_291344 | 79649 | 0.99 | 9.37E-05 |
| TMPO | 209754_s_at | AF113682 | NM_003276 | 7112 | 0.99 | 6.52E-03 |
| RASL11B | 219142_at | NM_023940 | NM_023940 | 65997 | 0.99 | 4.29E-03 |
| ASAHL | 214765_s_at | AK024677 | NM_014435 | 27163 | 0.98 | 1.03E-05 |
| ZNF238 | 212774_at | AJ223321 | NM_006352 | 10472 | 0.98 | 6.70E-05 |
| | | | NM_205768 | | | |
| ITPR2 | 202662_s_at | NM_002223 | NM_002223 | 3709 | 0.98 | 8.48E-03 |
| SEZ6L | 231650_s_at | BE672217 | NM_021115 | 23544 | 0.98 | 1.12E-04 |
| C10orf47 | 229801_at | AI640157 | NM_153256 | 254427 | 0.98 | 7.31E-03 |
| SNAG1 | 226683_at | AU146771 | NM_052870 | 112574 | 0.98 | 1.70E-04 |
| FGF11 | 227271_at | AU151265 | NM_004112 | 2256 | 0.98 | 9.49E-03 |
| DDT | 202929_s_at | NM_001355 | NM_001355 | 1652 | 0.98 | 3.16E-04 |
| KLHL5 | | 233866_at AK001836 | NM_00100707 5 | 51088 | 0.97 | 7.05E-03 |
| | | | NM_199039 | | | |
| PVRL3 | 241970_at | C14898 | NM_015480 | 25945 | 0.97 | 5.02E-03 |
| SEMA5A | 205405_at | NM_003966 | NM_003966 | 9037 | 0.97 | 2.27E-03 |
| RFC1 | 209085_x_at | L14922 | NM_002913 | 5981 | 0.97 | 6.24E-03 |
| TRIM36 | 219736_at | NM_018700 | NM_018700 | 55521 | 0.97 | 4.06E-03 |
| FLJ23322 | 231846_at | AK026975 | NM_024955 | 80020 | 0.97 | 3.27E-03 |
| IMP-1 | 223689_at | AF198254 | NM_006546 | 10642 | 0.97 | 1.78E-04 |
| MDFI | 205375_at | NM_005586 | NM_005586 | 4188 | 0.97 | 7.23E-03 |
| BE878277 | 212829_at | BE878277 | --- | --- | 0.97 | 1.51E-04 |
| FLJ14451 | 227430_at | AI969773 | NM_032786 | 84872 | 0.97 | 8.15E-03 |
| NARG1 | 226998_at | AL556909 | NM_057175 | 80155 | 0.96 | 1.89E-06 |
| TMEM38B | 222736_s_at | BC000049 | NM_018112 | 55151 | 0.96 | 3.02E-05 |
| LAMP2 | 203041_s_at | J04183 | NM_002294 | 3920 | 0.96 | 1.13E-04 |
| | | | NM_013995 | | | |
| FANCL | 218397_at | NM_018062 | NM_018062 | 55120 | 0.96 | 3.05E-03 |
| APOBEC3G | 204205_at | NM_021822 | NM_021822 | 60489 | 0.96 | 7.76E-03 |
| NM_024853 | 220533_at | NM_024853 | --- | --- | 0.96 | 3.50E-03 |
| UNG | 202330_s_at | NM_003362 | NM_003362 | 7374 | 0.96 | 2.31E-03 |
| | | | NM_080911 | | | |
| ACTL6A | 202666_s_at | NM_004301 | NM_004301 | 86 | 0.96 | 1.28E-03 |
| | | | NM_177989 | | | |
| | | | NM_178042 | | | |
| N71074 | 238078_at | N71074 | --- | --- | 0.96 | 7.93E-04 |
| FLJ40432 | 228810_at | AW135279 | NM_152523 | 151195 | 0.96 | 4.18E-06 |
| KIAA1102 | 212327_at | AK026815 | NM_014988 | 22998 | 0.96 | 1.66E-05 |
| C12orf2 | 225946_at | BG484552 | NM_007211 | 11228 | 0.96 | 4.33E-06 |
| FLJ11539 | 219791_s_at | NM_024748 | XM_496724 | 79804 | 0.96 | 4.56E-04 |
| MNS1 | 219703_at | NM_018365 | NM_018365 | 55329 | 0.96 | 6.11E-04 |
| SLC8A1 | 235518_at | AI741439 | NM_021097 | 6546 | 0.95 | 4.16E-03 |
| PSMD9 | 209334_s_at | BC002383 | NM_002813 | 5715 | 0.95 | 5.08E-06 |
| ACY1L2 | 225431_x_at | BE779764 | NM_001010852 | 135293 | 0.95 | 7.73E-04 |
| SOD2 | 215223_s_at | W46388 | NM_000636 | 6648 | 0.95 | 3.07E-03 |
| PPP1R3D | 204554_at | AL109928 | NM_006242 | 5509 | 0.95 | 2.56E-03 |
| BF056892 | 229024_at | BF056892 | --- | --- | 0.95 | 1.67E-04 |
| CHD6 | 225031_at | AL031667 | NM_032221 | 84181 | 0.95 | 4.37E-05 |
| MGC7036 | 227983_at | AI810244 | NM_145058 | 196383 | 0.95 | 2.13E-04 |
| CCBE1 | 243805_at | BG035826 | NM_133459 | 147372 | 0.95 | 1.68E-03 |
| AL110237 | 232959_at | AL110237 | --- | --- | 0.95 | 9.01E-04 |
| LOC134111 | 226612_at | H17038 | XM_059689 | 134111 | 0.94 | 3.88E-03 |
| ELAVL4 | 234904_x_at | S73887 | NM_021952 | 1996 | 0.94 | 1.21E-04 |
| PANK2 | 238856_s_at | BG108346 | NM_024960 | 80025 | 0.94 | 3.01E-05 |
| | | | NM_153637 | | | |
| | | | NM_153638 | | | |
| | | | NM_153639 | | | |
| | | | NM_153640 | | | |
| | | | NM_153641 | | | |
| CBX5 | 230752_at | AI638063 | NM_012117 | 23468 | 0.94 | 6.48E-04 |
| NDUFA6 | 202000_at | BC002772 | NM_002490 | 4700 | 0.94 | 5.54E-04 |
| HSPC117 | 200042_at | NM_014306 | NM_014306 | 51493 | 0.94 | 6.59E-07 |
| TLE4 | 235765_at | AI742932 | NM_007005 | 7091 | 0.94 | 9.75E-04 |
| SDC4 | 202071_at | NM_002999 | NM_002999 | 6385 | 0.94 | 8.24E-05 |
| CD47 | 211075_s_at | Z25521 | NM_001777 | 961 | 0.94 | 1.46E-03 |
| | | | NM_198793 | | | |
| EGFR | 224999_at | BE878463 | NM_005228 | 1956 | 0.93 | 4.74E-03 |
| | | | NM_201282 | | | |
| | | | NM_201283 | | | |
| | | | NM_201284 | | | |
| TLX2 | 211049_at | BC006356 | NM_001534 | 3196 | 0.93 | 6.56E-04 |
| | | | NM_016170 | | | |
| VIL2 | 208622_s_at | AA670344 | NM_003379 | 7430 | 0.93 | 3.33E-03 |
| JPH1 | 1553533_at | NM_020647 | NM_020647 | 56704 | 0.93 | 2.57E-03 |
| TEAD3 | 209454_s_at | AF142482 | NM_003214 | 7005 | 0.93 | 5.72E-03 |
| FKBP5 | 204560_at | NM_004117 | NM_004117 | 2289 | 0.93 | 2.37E-03 |
| SEC61A2 | 219499_at | NM_018144 | NM_018144 | 55176 | 0.93 | 9.99E-03 |
| PGM2 | 225366_at | AI652855 | NM_018290 | 55276 | 0.93 | 6.09E-04 |
| P4HA1 | 207543_s_at | NM_000917 | NM_000917 | 5033 | 0.93 | 2.61E-04 |
| SEC23A | 204344_s_at | NM_006364 | NM_006364 | 10484 | 0.93 | 3.24E-03 |
| KIAA1102 | 212328_at | AB029025 | NM_014988 | 22998 | 0.93 | 1.29E-05 |
| RANBP1 | 202483_s_at | NM_002882 | NM_002882 | 5902 | 0.93 | 7.82E-03 |
| STARD4 | 226390_at | AA628398 | NM_139164 | 134429 | 0.93 | 1.59E-04 |
| B4GALT6 | 206232_s_at | NM_004775 | NM_004775 | 9331 | 0.93 | 2.63E-03 |
| AHI1 | 220842_at | NM_017651 | NM_017651 | 54806 | 0.92 | 6.04E-03 |
| MCM4 | 212142_at | AI936566 | NM_005914 | 4173 | 0.92 | 7.62E-04 |
| | | | NM_182746 | | | |
| CHD6 | 225026_at | BF572029 | NM_032221 | 84181 | 0.92 | 3.17E-03 |
| HMGB2 | 208808_s_at | BC000903 | NM_002129 | 3148 | 0.92 | 7.83E-03 |
| DNMT3B | 220668_s_at | NM_006892 | NM_006892 | 1789 | 0.92 | 2.56E-03 |
| | | | NM_175848 | | | |
| | | | NM_175849 | | | |
| | | | NM_175850 | | | |
| AI095542 | 235759_at | AI095542 | --- | --- | 0.92 | 4.36E-03 |
| SLC25A1 | 210010_s_at | U25147 | NM_005984 | 6576 | 0.92 | 2.10E-03 |
| ELAVL4 | 238073_at | R55745 | NM_021952 | 1996 | 0.92 | 1.01E-03 |
| BG149337 | 229814_at | BG149337 | --- | --- | 0.92 | 2.56E-03 |
| R43103 | 243642_x_at | R43103 | --- | --- | 0.92 | 3.67E-03 |
| NEDD1 | 1552417_a_at | NM_152905 | NM_152905 | 121441 | 0.91 | 2.33E-03 |
| MTUS1 | 212093_s_at | AI695017 | NM_00100192 4 | 57509 | 0.91 | 4.60E-04 |
| | | | NM_00100192 5 | | | |
| | | | NM_00100192 7 | | | |
| | | | NM_00100193 1 | | | |
| | | | NM_020749 | | | |
| ALG10 | 1552306_at | BC033730 | NM_032834 | 84920 | 0.91 | 7.39E-04 |
| ELAVL4 | 206051_at | NM_021952 | NM_021952 | 1996 | 0.91 | 1.99E-03 |
| MLF1 | 204783_at | AI911434 | NM_022443 | 4291 | 0.91 | 1.31E-04 |
| SLC2A8 | 218985_at | NM_014580 | NM_014580 | 29988 | 0.91 | 6.78E-03 |
| ITPR2 | 202660_at | AA834576 | NM_002223 | 3709 | 0.91 | 9.15E-03 |
| CISH | 223377_x_at | AF035947 | NM_013324 | 1154 | 0.91 | 4.28E-03 |
| | | | NM_145071 | | | |
| C3orf4 | 1554149_at | BC013610 | NM_019895 | 56650 | 0.91 | 2.11E-05 |
| LOC134111 | 226618_at | AW572911 | XM_059689 | 134111 | 0.91 | 5.63E-04 |
| BE857972 | 243781_at | BE857972 | --- | --- | 0.91 | 1.20E-03 |
| Cep70 | 224150_s_at | AF289495 | NM_024491 | 80321 | 0.91 | 4.27E-03 |
| PP2447 | 232706_s_at | AW009248 | NM_025204 | 80305 | 0.90 | 4.10E-03 |
| RGS4 | 204337_at | AL514445 | NM_005613 | 5999 | 0.90 | 1.42E-03 |
| KLF7 | 204334_at | AA488672 | NM_003709 | 8609 | 0.90 | 9.17E-04 |
| SLCO3A1 | 227367_at | AW976431 | NM_013272 | 28232 | 0.90 | 4.34E-03 |
| FZD2 | 210220_at | L37882 | NM_001466 | 2535 | 0.90 | 2.77E-04 |
| SESTD1 | 226763_at | AW409611 | NM_178123 | 91404 | 0.90 | 9.00E-04 |
| DHX35 | 218579_s_at | NM_021931 | NM_021931 | 60625 | 0.90 | 2.42E-05 |
| PDLIM1 | 208690_s_at | BC000915 | NM_020992 | 9124 | 0.90 | 6.24E-04 |
| BICD1 | 1554020_at | BC010091 | NM_00100339 | 636 | 0.90 | 3.41E-06 |
| | | | 8 | | | |
| | | | NM_001714 | | | |
| PXMP2 | 219076_s_at | NM_018663 | NM_018663 | 5827 | 0.90 | 6.61E-03 |
| SPOCK | 202363_at | AF231124 | NM_004598 | 6695 | 0.90 | 5.89E-03 |
| AA581439 | 244650_at | AA581439 | --- | --- | 0.89 | 5.83E-03 |
| BC003667 | 222487_s_at | BC003667 | --- | --- | 0.89 | 8.10E-03 |
| NFYB | 218128_at | AU151875 | NM_006166 | 4801 | 0.89 | 2.19E-03 |
| SYNJ2 | 212828_at | AA191573 | NM_003898 | 8871 | 0.89 | 6.92E-03 |
| ITGB5 | 201124_at | AL048423 | NM_002213 | 3693 | 0.89 | 4.74E-03 |
| SLC39A8 | 219869_s_at | NM_022154 | NM_022154 | 64116 | 0.89 | 1.30E-03 |
| AI333006 | 240432_x_at | AI333006 | --- | --- | 0.89 | 2.24E-03 |
| BRD4 | 202103_at | AI991631 | NM_014299 | 23476 | 0.89 | 3.39E-03 |
| | | | NM_058243 | | | |
| MCM8 | 233560_x_at | AA370141 | NM_032485 | 84515 | 0.89 | 2.33E-03 |
| | | | NM_182802 | | | |
| PKIA | 226864_at | BF245954 | NM_006823 | 5569 | 0.89 | 4.86E-04 |
| | | | NM_181839 | | | |
| FAM43A | 227410_at | AW264102 | NM_153690 | 131583 | 0.89 | 4.81E-03 |
| ZMYM1 | 220206_at | NM_024772 | NM_024772 | 79830 | 0.89 | 5.27E-03 |
| SLK | 206875_s_at | NM_014720 | NM_014720 | 9748 | 0.89 | 9.22E-06 |
| EPB41L5 | 225855_at | AB046768 | NM_020909 | 57669 | 0.89 | 8.39E-04 |
| RAD18 | 223417_at | AF169796 | NM_020165 | 56852 | 0.89 | 2.04E-04 |
| MEIS2 | 207480_s_at | NM_020149 | NM_002399 | 4212 | 0.89 | 4.23E-04 |
| | | | NM_020149 | | | |
| | | | NM_170674 | | | |
| | | | NM_170675 | | | |
| | | | NM_170676 | | | |
| | | | NM_170677 | | | |
| | | | NM_172315 | | | |
| | | | NM_172316 | | | |
| NFYA | 204107_at | BF445142 | NM_002505 | 4800 | 0.88 | 3.28E-06 |
| | | | NM_021705 | | | |
| SREBF2 | 201248_s_at | NM_004599 | NM_004599 | 6721 | 0.88 | 4.75E-03 |
| FLJ31842 | 237515_at | AA054642 | NM_152487 | 148534 | 0.88 | 8.21E-04 |
| ZFD25 | 205739_x_at | NM_016220 | --- | 51427 | 0.88 | 8.79E-03 |
| DLEU2 | 215629_s_at | AA905286 | NM_006021 | 8847 | 0.88 | 6.43E-04 |
| ZNF85 | 206572_x_at | NM_003429 | NM_003429 | 7639 | 0.88 | 6.85E-06 |
| PCDH17 | 228863_at | N69091 | NM_014459 | 27253 | 0.88 | 6.70E-03 |
| PTPRG | 204944_at | NM_002841 | NM_002841 | 5793 | 0.88 | 3.65E-03 |
| AL833487 | 1562245_a_at | AL833487 | --- | --- | 0.88 | 8.59E-03 |
| PCDH17 | 205656_at | NM_014459 | NM_014459 | 27253 | 0.88 | 6.19E-03 |
| ANAPC7 | 225554_s_at | AA131793 | NM_016238 | 51434 | 0.88 | 8.37E-04 |
| BE504215 | 228563_at | BE504215 | --- | --- | 0.88 | 1.17E-03 |
| MLLT3 | 204917_s_at | AV756536 | NM_004529 | 4300 | 0.88 | 5.47E-03 |
| CD47 | 213857_s_at | BG230614 | NM_001777 | 961 | 0.88 | 3.54E-04 |
| | | | NM_198793 | | | |
| PTK7 | 1555324_at | BC046109 | NM_002821 | 5754 | 0.87 | 3.45E-03 |
| | | | NM_152880 | | | |
| | | | NM_152881 | | | |
| | | | NM_152882 | | | |
| | | | NM_152883 | | | |
| EP300 | 1562921_at | BC040700 | NM_001429 | 2033 | 0.87 | 3.55E-03 |
| VPS29 | 223026_s_at | AF201946 | NM_016226 | 51699 | 0.87 | 9.51E-03 |
| | | | NM_057180 | | | |
| EBF | 227646_at | BG435302 | NM_024007 | 1879 | 0.87 | 6.32E-04 |
| GPC6 | 223730_at | AF111178 | NM_005708 | 10082 | 0.87 | 7.72E-03 |
| PHF5A | 225309_at | AL008582 | NM_032758 | 84844 | 0.87 | 1.93E-03 |
| FLJ31842 | 234980_at | A1004375 | NM_152487 | 148534 | 0.87 | 3.12E-04 |
| AW953794 | 230968_at | AW953794 | --- | --- | 0.87 | 3.09E-03 |
| AI888657 | 244189_at | AI888657 | --- | --- | 0.87 | 1.23E-03 |
| FLJ21415 | 218867_s_at | NM_024738 | NM_024738 | 79794 | 0.87 | 2.65E-03 |
| MAK3 | 217745_s_at | NM_025146 | NM_025146 | 80218 | 0.87 | 5.22E-04 |
| SEC23A | 212887_at | AI753659 | NM_006364 | 10484 | 0.86 | 6.69E-03 |
| PAX5 | 221969_at | BF510692 | NM_016734 | 5079 | 0.86 | 4.37E-03 |
| UNC84B | 212144_at | AL021707 | NM_015374 | 25777 | 0.86 | 9.44E-03 |
| PRIM2A | 205628_at | NM_000947 | NM_000947 | 5558 | 0.86 | 1.80E-03 |
| ISL1 | 206104_at | NM_002202 | NM_002202 | 3670 | 0.86 | 2.89E-03 |
| NAGA | 202944_at | NM_000262 | NM_000262 | 4668 | 0.86 | 1.12E-04 |
| SCD4 | 224901_at | AL571375 | NM_024906 | 79966 | 0.86 | 2.89E-04 |
| PHYH | 203335_at | NM_006214 | NM_006214 | 5264 | 0.86 | 3.64E-05 |
| HMGCS1 | 205822_s_at | NM_002130 | NM_002130 | 3157 | 0.86 | 5.71E-04 |
| GALNAC4S-6S⁻ | 203066_at | NM_014863 | NM_015892 | 51363 | 0.86 | 1.55E-04 |
| DKFZp762A217 | 235775_at | AI765006 | NM_152588 | 160335 | 0.86 | 2.87E-03 |
| ADRBK2 | 204183_s_at | AI478542 | NM_005160 | 157 | 0.85 | 7.45E-05 |
| BC002942 | 31837_at | U62317 | NM_033200 | 91289 | 0.85 | 2.32E-03 |
| KIAA1853 | 236151_at | BF315452 | NM_194286 | 84530 | 0.85 | 8.93E-03 |
| C10orf58 | 228155_at | BF512388 | NM_032333 | 84293 | 0.85 | 4.55E-08 |
| KIAA0286 | 212619_at | AW205215 | NM_015257 | 23306 | 0.85 | 2.40E-03 |
| AU146017 | 234082_at | AU146017 | --- | --- | 0.85 | 5.56E-03 |
| GPR85 | 219898_at | NM_018970 | NM_018970 | 54329 | 0.85 | 2.12E-03 |
| KIAA1145 | 235146_at | N51717 | NM_020698 | 57458 | 0.85 | 4.62E-05 |
| CSTF1 | 202190_at | NM_001324 | NM_001324 | 1477 | 0.85 | 2.75E-03 |
| BF510533 | 228107_at | BF510533 | --- | --- | 0.85 | 7.13E-04 |
| PANK2 | 218809_at | NM_024960 | NM_024960 | 80025 | 0.85 | 5.93E-04 |
| | | | NM_153637 | | | |
| | | | NM_153638 | | | |
| | | | NM_153639 | | | |
| | | | NM_153640 | | | |
| | | | NM_153641 | | | |
| DHX37 | 223364_s_at | BC004463 | NM_032656 | 57647 | 0.85 | 1.99E-03 |
| HMGB3 | 203744_at | NM_005342 | NM_005342 | 3149 | 0.85 | 6.52E-03 |
| BNIP3 | 201848_s_at | U15174 | NM_004052 | 664 | 0.85 | 1.65E-03 |
| LOC284244 | 214162_at | AF070541 | --- | 284244 | 0.85 | 2.94E-03 |
| SV2C | 216086_at | AB028977 | XM_043493 | 22987 | 0.85 | 3.24E-03 |
| MTP18 | 223172_s_at | AF060924 | NM_00100370 4 | 51537 | 0.85 | 6.41E-03 |
| | | | NM_016498 | | | |
| SNAP29 | 218327_s_at | NM_004782 | NM_004782 | 9342 | 0.85 | 4.67E-03 |
| NFYA | 204109_s_at | NM_002505 | NM_002505 | 4800 | 0.85 | 4.40E-03 |
| | | | NM_021705 | | | |
| FLJ37858 | 227354_at | BF589359 | NM_001007549 | 441357 | 0.84 | 6.04E-03 |
| R58282 | 238557_at | R58282 | --- | --- | 0.84 | 5.38E-03 |
| CDH2 | 203441_s_at | NM_001792 | NM_001792 | 1000 | 0.84 | 1.55E-03 |
| C20orf45 | 217851_s_at | NM_016045 | NM_016045 | 51012 | 0.84 | 1.50E-03 |
| MYO1B | 212364_at | BF432550 | NM_012223 | 4430 | 0.84 | 1.02E-03 |
| PAICS | 201014_s_at | NM_006452 | NM_006452 | 10606 | 0.84 | 4.23E-04 |
| ZNF505 | 215758_x_at | AC007204 | NM_00100412 | 81931 | 0.84 | 1.32E-04 |
| | | | 6 | | | |
| | | | NM_031218 | | | |
| SEMA6A | 215028_at | AB002438 | NM_020796 | 57556 | 0.84 | 1.21E-04 |
| SLIT2 | 209897_s_at | AF055585 | NM_004787 | 9353 | 0.84 | 3.51E-03 |
| CELSR3 | 40020_at | AB011536 | NM_001407 | 1951 | 0.84 | 1.82E-06 |
| DIRAS2 | 219619_at | NM_017594 | NM_017594 | 54769 | 0.84 | 9.62E-03 |
| TOB2 | 222243_s_at | AB051450 | NM_016272 | 10766 | 0.84 | 3.84E-03 |
| HAND1 | 220138_at | NM_004821 | NM_004821 | 9421 | 0.84 | 8.36E-04 |
| RAD18 | 224200_s_at | AB035274 | NM_020165 | 56852 | 0.83 | 3.81E-03 |
| LRIG3 | 226908_at | AI627704 | NM_153377 | 121227 | 0.83 | 9.44E-03 |
| AI150000 | 226671_at | AI150000 | --- | --- | 0.83 | 7.72E-04 |
| EIF5A2 | 235289_at | AV747725 | NM_020390 | 56648 | 0.83 | 2.77E-03 |
| CDK2AP1 | 201938_at | NM_004642 | NM_004642 | 8099 | 0.83 | 8.12E-03 |
| MLF1 | 204784_s_at | NM_022443 | NM_022443 | 4291 | 0.83 | 2.50E-03 |
| MPP5 | 219321_at | NM_022474 | NM_022474 | 64398 | 0.83 | 1.84E-03 |
| TXNRD1 | 201266_at | NM_003330 | NM_003330 | 7296 | 0.83 | 6.73E-05 |
| | | | NM_182729 | | | |
| | | | NM_182742 | | | |
| | | | NM_182743 | | | |
| C10orf58 | 224435_at | BC005871 | NM_032333 | 84293 | 0.82 | 5.15E-06 |
| EVE1 | 225162_at | BG285417 | NM_001009555 | 152503 | 0.82 | 5.43E-03 |
| RALY | 201271_s_at | NM_016732 | NM_007367 | 22913 | 0.82 | 1.71E-03 |
| | | | NM_016732 | | | |
| SEC10L1 | 228418_at | BF509391 | NM_006544 | 10640 | 0.82 | 6.79E-06 |
| MFAP2 | 203417_at | NM_017459 | NM_002403 | 4237 | 0.82 | 9.64E-05 |
| | | | NM_017459 | | | |
| MYO1B | 212365_at | BF215996 | NM_012223 | 4430 | 0.82 | 2.49E-04 |
| ACAA1 | 214274_s_at | AI860341 | NM_001607 | 30 | 0.82 | 5.44E-03 |
| KIAA0701 | 1554292_a_at | BC014891 | NM_00100694 7 | 23074 | 0.81 | 5.60E-03 |
| | | | NM_015054 | | | |
| AK022598 | 232662_x_at | AK022598 | --- | --- | 0.81 | 5.55E-03 |
| PLXNA2 | 213030_s_at | AI688418 | NM_025179 | 5362 | 0.81 | 6.66E-03 |
| BE501881 | 227565_at | BE501881 | --- | --- | 0.81 | 2.21E-03 |
| BF447112 | 236192_at | BF447112 | --- | --- | 0.81 | 2.02E-03 |
| LOC85028 | 228990_at | AI339426 | XM_378848 | 85028 | 0.81 | 4.87E-04 |
| BF511170 | 227332_at | BF511170 | --- | --- | 0.81 | 3.67E-03 |
| KLF3 | 219657_s_at | NM_016531 | NM_016531 | 51274 | 0.81 | 3.64E-03 |
| GRIA1 | 209793_at | AL567302 | NM_000827 | 2890 | 0.81 | 3.68E-03 |
| SUHW2 | 229360_at | N22886 | | 140883 | 0.81 | 8.50E-03 |
| | | | NM_080764 | | | |
| LOC401022 | 242042_s_at | AA406052 | --- | 401022 | 0.81 | 5.97E-04 |
| C3orf4 | 208925_at | AF161522 | NM_019895 | 56650 | 0.81 | 1.42E-08 |
| MRPL40 | 203152_at | NM_003776 | NM_003776 | 64976 | 0.81 | 6.67E-03 |
| PVT1 | 1558290_a_at | BG200951 | XM_372058 | 441378 | 0.81 | 2.88E-03 |
| LOC441378 | | | XM_499128 | 5820 | | |
| KELCHL | 221837_at | BG325646 | NM_032775 | 84861 | 0.81 | 3.12E-04 |
| CELSR3 | 205165_at | NM_001407 | NM_001407 | 1951 | 0.81 | 1.43E-04 |
| RAB35 | 205461_at | NM_006861 | NM_006861 | 11021 | 0.81 | 6.64E-03 |
| MGC70857 | 226710_at | AI199072 | NM_001001795 | 414919 | 0.81 | 3.76E-05 |
| AA446953 | 241749_at | AA446953 | XM_294592 | 347273 | 0.81 | 2.10E-04 |
| PSMD5 | 203447_at | AU157008 | NM_005047 | 5711 | 0.80 | 8.55E-04 |
| NUP50 | 222583_s_at | AF116624 | NM_007172 | 10762 | 0.80 | 2.54E-04 |
| | | | NM_153645 | | | |
| | | | NM_153684 | | | |
| ARL61P6 | 225707_at | AL581082 | NM_152522 | 151188 | 0.80 | 4.86E-03 |
| H1F0 | 208886_at | BC000145 | NM_005318 | 3005 | 0.80 | 2.95E-04 |
| RQCD1 | 1554080_at | BC007102 | NM_005444 | 9125 | 0.80 | 5.80E-03 |
| MVK | 215649_s_at | AF217536 | NM_000431 | 4598 | 0.80 | 4.39E-04 |
| CDC5L | 209055_s_at | AW268817 | NM_001253 | 988 | 0.80 | 9.38E-03 |
| LGR4 | 218326_s_at | NM_018490 | NM_018490 | 55366 | 0.80 | 1.35E-04 |
| C19orf32 | 226597_at | AI348159 | NM_138393 | 92840 | 0.80 | 6.56E-03 |
| HPS4 | 218402_s_at | NM_022081 | NM_022081 | 89781 | 0.80 | 8.60E-04 |
| | | | NM_152840 | | | |
| | | | NM_152841 | | | |
| | | | NM_152842 | | | |
| | | | NM_152843 | | | |
| MGC18216 | 243358_at | BF347362 | --- | 145815 | 0.79 | 8.83E-03 |
| ZNF43 | 222136_x_at | AK022905 | NM_003423 | 7594 | 0.79 | 1.19E-06 |
| DRG1 | 236537_at | AI760332 | NM_004147 | 4733 | 0.79 | 9.05E-03 |
| SLC2A4RG | 218494_s_at | NM_020062 | NM_020062 | 56731 | 0.79 | 1.26E-04 |
| CHRNA3 | 211772_x_at | BC006114 | NM_000743 | 1136 | 0.79 | 5.89E-03 |
| UBE2L3 | 200683_s_at | BE964689 | NM_003347 | 7332 | 0.79 | 2.55E-04 |
| | | | NM_198157 | | | |
| NUP43 | 238474_at | BF978064 | NM_024647 | 348995 | 0.79 | 6.09E-03 |
| | | | NM_198887 | | | |
| EML5 | 1568777_at | BM677635 | NM_183387 | 161436 | 0.79 | 2.74E-03 |
| N24703 | 238637_at | N24703 | --- | --- | 0.79 | 1.03E-03 |
| CHRNA3 | 211587_x_at | M37981 | NM_000743 | 1136 | 0.79 | 2.09E-03 |
| ZNF20 | 213916_at | AU154474 | NM_021143 | 7568 | 0.79 | 2.24E-03 |
| VAMP1 | 213326_at | AU150319 | NM_014231 | 6843 | 0.79 | 2.99E-03 |
| | | | NM_016830 | | | |
| | | | NM_199245 | | | |
| TMEM38B | 222735_at | AW452608 | NM_018112 | 55151 | 0.79 | 1.36E-05 |
| MAPKAPK3 | 202788_at | NM_004635 | NM_004635 | 7867 | 0.79 | 7.86E-03 |
| GNB4 | 223488_s_at | BC000873 | NM_021629 | 59345 | 0.79 | 5.46E-03 |
| EPOR | 209962_at | M34986 | NM_000121 | 2057 | 0.78 | 2.19E-04 |
| C6orf115 | 223361_at | AF116682 | XM_371848 | 58527 | 0.78 | 1.07E-03 |
| AK057923 | 1556364_at | AK057923 | XM_498935 | 440960 | 0.78 | 4.56E-03 |
| PGM2 | 225367_at | BF512139 | NM_018290 | 55276 | 0.78 | 3.75E-04 |
| CECR5 | 218592_s_at | NM_017829 | NM_017829 | 27440 | 0.78 | 4.74E-03 |
| | | | NM_033070 | | | |
| CHRNA3 | 210221_at | BC000513 | NM_000743 | 1136 | 0.78 | 2.56E-05 |
| STMN3 | 218207_s_at | NM_015894 | NM_015894 | 50861 | 0.78 | 8.10E-04 |
| UCP2 | 208997_s_at | U82819 | NM_003355 | 7351 | 0.78 | 3.02E-04 |
| SFRS8 | 202773_s_at | AI023864 | NM_004592 | 6433 | 0.78 | 4.72E-03 |
| | | | NM_152235 | | | |
| FIGNL1 | 1552921_a_at | NM_022116 | NM_022116 | 63979 | 0.78 | 5.97E-03 |
| MEST | 202016_at | NM_002402 | NM_002402 | 4232 | 0.78 | 9.96E-03 |
| | | | NM_177524 | | | |
| | | | NM_177525 | | | |
| C20orf45 | 222441_x_at | BF032213 | NM_016045 | 51012 | 0.78 | 2.01E-05 |
| PLCXD2 | 235230_at | AW170015 | NM_153268 | 257068 | 0.78 | 7.04E-03 |
| UCK1 | 223142_s_at | AF237290 | NM_031432 | 83549 | 0.78 | 3.34E-05 |
| SNAP29 | 239084_at | BE896490 | NM_004782 | 9342 | 0.78 | 7.09E-03 |
| SNRPD3 | 202567_at | NM_004175 | NM_004175 | 6634 | 0.78 | 8.05E-03 |
| GABPB2 | 204618_s_at | NM_005254 | NM_002041 | 2553 | 0.78 | 4.11E-04 |
| | | | NM_005254 | | | |
| | | | NM_016654 | | | |
| | | | NM_016655 | | | |
| | | | NM_181427 | | | |
| RNF10 | 208632_at | AL578551 | NM_014868 | 9921 | 0.77 | 7.94E-04 |
| RFC1 | 208021_s_at | NM_002913 | NM_002913 | 5981 | 0.77 | 9.34E-03 |
| PAPPA | 240295_at | AL045014 | NM_002581 | 5069 | 0.77 | 2.94E-03 |
| TM4SF9 | 225387_at | AA059445 | NM_005723 | 10098 | 0.77 | 1.49E-03 |
| PDCL | 204448_s_at | AF031463 | NM_005388 | 5082 | 0.77 | 8.07E-03 |
| GNAZ | 204993_at | NM_002073 | NM_002073 | 2781 | 0.77 | 3.40E-03 |
| STK39 | 202786_at | NM_013233 | NM_013233 | 27347 | 0.77 | 5.82E-03 |
| UBE2L3 | 200684_s_at | AI819709 | NM_003347 | 7332 | 0.77 | 4.66E-06 |
| | | | NM_198157 | | | |
| BZRP | 202096_s_at | NM_000714 | NM_000714 | 706 | 0.77 | 2.65E-03 |
| | | | NM_007311 | | | |
| ELK3 | 206127_at | NM_005230 | NM_005230 | 2004 | 0.77 | 2.08E-03 |
| ZDHHC13 | 219296_at | NM_019028 | NM_00100148 3 | 54503 | 0.77 | 9.19E-04 |
| | | | NM_019028 | | | |
| TIMP2 | 231579_s_at | BE968786 | NM_003255 | 7077 | 0.77 | 2.80E-03 |
| C8orf13 | 226614_s_at | BE856336 | NM_053279 | 83648 | 0.77 | 3.12E-03 |
| BAX | 208478_s_at | NM_004324 | NM_004324 | 581 | 0.77 | 5.20E-03 |
| | | | NM_138761 | | | |
| | | | NM_138762 | | | |
| | | | NM_138763 | | | |
| | | | NM_138764 | | | |
| | | | NM_138765 | | | |
| TCF8 | 239952_at | AI743662 | NM_030751 | 6935 | 0.77 | 2.84E-04 |
| LOC128977 | 227086_at | AA194312 | NM_173793 | 128977 | 0.77 | 3.39E-03 |
| PAICS | 201013_s_at | AA902652 | NM_006452 | 10606 | 0.76 | 3.48E-03 |
| AKAP8 | 203848_at | AL050160 | NM_005858 | 10270 | 0.76 | 6.90E-05 |
| TWSG1 | 225406_at | AA195009 | NM_020648 | 57045 | 0.76 | 1.41E-03 |
| PDK1 | 206686_at | NM_002610 | NM_002610 | 5163 | 0.76 | 4.33E-04 |
| TIMP2 | 224560_at | BF107565 | NM_003255 | 7077 | 0.76 | 7.63E-03 |
| CDS2 | 212862_at | AL568982 | NM_003818 | 8760 | 0.76 | 2.78E-05 |
| 384D8-2 | 205086_s_at | NM_014551 | NM_014551 | 29781 | 0.76 | 4.69E-04 |
| | | | NM_152299 | | | |
| DTNBP1 | 223445_at | AF061734 | NM_032122 | 84062 | 0.76 | 9.22E-04 |
| | | | NM_183040 | | | |
| | | | NM_183041 | | | |
| BG492376 | 1557961_s_at | BG492376 | --- | --- | 0.76 | 3.56E-03 |
| ARL6IP6 | 225711_at | AA654338 | NM_152522 | 151188 | 0.76 | 8.52E-03 |
| LRP10 | 201412_at | NM_014045 | NM_014045 | 26020 | 0.75 | 8.53E-03 |
| G22P1 | 200792_at | NM_001469 | NM_001469 | 2547 | 0.75 | 1.40E-05 |
| SDCBP | 200958_s_at | NM_005625 | NM_00100706 7 | 6386 | 0.75 | 8.95E-04 |
| | | | NM_00100706 8 | | | |
| | | | NM_00100706 9 | | | |
| | | | NM_00100707 0 | | | |
| | | | NM_005625 | | | |
| PELI2 | 219132_at | NM_021255 | NM_021255 | 57161 | 0.75 | 4.32E-04 |
| NFYB | 218129_s_at | NM_006166 | NM_006166 | 4801 | 0.75 | 6.84E-03 |
| BTBD3 | 202946_s_at | NM_014962 | NM_014962 | 22903 | 0.75 | 2.02E-04 |
| | | | NM_181443 | | | |
| CSE1L | 201111_at | AF053641 | NM_001316 | 1434 | 0.75 | 9.19E-03 |
| | | | NM_177436 | | | |
| CAMKK1 | 223460_at | AL136576 | NM_032294 | 84254 | 0.75 | 6.46E-03 |
| | | | NM_172206 | | | |
| | | | NM_172207 | | | |
| TFDP2 | 244043_at | AI049624 | NM_006286 | 7029 | 0.75 | 1.03E-03 |
| AU154030 | 242141_at | AU154030 | --- | --- | 0.75 | 6.48E-03 |
| C20orf20 | 218586_at | NM_018270 | NM_018270 | 55257 | 0.75 | 4.07E-04 |
| PPAT | 209434_s_at | U00238 | NM_002703 | 5471 | 0.75 | 4.18E-04 |
| FH | 203032_s_at | AI363836 | NM_000143 | 2271 | 0.75 | 3.75E-04 |
| USP28 | 231837_at | AB040948 | NM_020886 | 57646 | 0.75 | 4.33E-04 |
| COVA1 | 32042_at | S72904 | NM_006375 | 10495 | 0.75 | 1.36E-03 |
| | | | NM_182314 | | | |
| KIAA1432 | 226221_at | AL138104 | XM_039698 | 57589 | 0.75 | 1.64E-03 |
| ZNF43 | 206695_x_at | NM_003423 | NM_003423 | 7594 | 0.75 | 2.70E-03 |
| MICAL-L1 | 55081_at | W46406 | NM_033386 | 85377 | 0.75 | 4.38E-03 |
| HPRP8BP | 215905_s_at | AL157420 | NM_004814 | 9410 | 0.74 | 1.02E-04 |
| MAC30 | 214283_at | AU150186 | NM_014573 | 27346 | 0.74 | 8.38E-06 |
| C10orf78 | 238794_at | N23586 | NM_00100275 9 | 119392 | 0.74 | 7.02E-03 |
| | | | NM_145247 | | | |
| Cep70 | 219036_at | NM_024491 | NM_024491 | 80321 | 0.74 | 1.04E-03 |
| CDKAL1 | 214877_at | BE794663 | NM_017774 | 54901 | 0.74 | 7.46E-04 |
| CUGBP2 | 227178_at | AI652861 | NM_006561 | 10659 | 0.74 | 1.87E-03 |
| SQLE | 213562_s_at | BF979497 | NM_003129 | 6713 | 0.74 | 1.93E-03 |
| RABL3 | 226089_at | AI742028 | NM_173825 | 285282 | 0.74 | 2.21E-03 |
| TRAM2 | 202368_s_at | AI986461 | NM_012288 | 9697 | 0.74 | 4.19E-03 |
| CD47 | 227259_at | BF439618 | NM_001777 | 961 | 0.74 | 6.22E-03 |
| | | | NM_198793 | | | |
| T87730 | 242500_at | T87730 | --- | --- | 0.74 | 5.92E-05 |
| DGCR2 | 214198_s_at | AU150824 | NM_005137 | 9993 | 0.74 | 5.08E-05 |
| GIT2 | 209876_at | AF124491 | NM_014776 | 9815 | 0.74 | 1.42E-03 |
| | | | NM_057169 | | | |
| | | | NM_057170 | | | |
| | | | NM_139201 | | | |
| MKKS | 222530_s_at | AF275813 | NM_018848 | 8195 | 0.74 | 8.92E-03 |
| | | | NM_170784 | | | |
| NFKBIE | 203927_at | NM_004556 | NM_004556 | 4794 | 0.73 | 7.87E-03 |
| PTCH | 209815_at | BG054916 | NM_000264 | 5727 | 0.73 | 4.15E-07 |
| SORT1 | 212807_s_at | BF447105 | NM_002959 | 6272 | 0.73 | 6.06E-04 |
| SKP2 | 203625_x_at | BG105365 | NM_005983 | 6502 | 0.73 | 2.38E-03 |
| | | | NM_032637 | | | |
| B3GNT1 | 219326_s_at | NM_006577 | NM_006577 | 10678 | 0.73 | 3.66E-03 |
| | | | NM_033252 | | | |
| TST | 209605_at | D87292 | NM_003312 | 7263 | 0.73 | 3.44E-03 |
| RQCD1 | 1553510_s_at | NM_005444 | NM_005444 | 9125 | 0.73 | 7.31E-03 |
| LOC440928 | 235147_at | R56118 | XM_498917 | 440928 | 0.73 | 8.09E-05 |
| EPB41L5 | 220977_x_at | NM_020909 | NM_020909 | 57669 | 0.73 | 9.69E-03 |
| B4GALT6 | 206233_at | AF097159 | NM_004775 | 9331 | 0.73 | 3.61E-04 |
| MTHFD1 | 202309_at | NM_005956 | NM_005956 | 4522 | 0.73 | 5.58E-03 |
| SUHW2 | 230789_at | AI015954 | NM_080764 | 140883 | 0.73 | 4.75E-03 |
| TCF4 | 228837_at | BE857360 | NM_003199 | 6925 | 0.73 | 1.23E-05 |
| C20orf22 | 224742_at | BF570412 | NM_015600 | 26090 | 0.73 | 5.04E-04 |
| EIF4ENIF1 | 218626_at | NM_019843 | NM_019843 | 56478 | 0.73 | 3.09E-06 |
| ZNF141 | 206931_at | NM_003441 | NM_003441 | 7700 | 0.73 | 1.09E-03 |
| ZNF278 | 209494_s_at | AI807017 | NM_014323 | 23598 | 0.73 | 1.80E-03 |
| | | | NM_032050 | | | |
| | | | NM_032051 | | | |
| | | | NM_032052 | | | |
| RNF26 | 224947_at | AL559247 | NM_032015 | 79102 | 0.73 | 9.69E-04 |
| GNG4 | 1555765_a_at | AF493872 | NM_004485 | 2786 | 0.73 | 2.53E-03 |
| BCR | 202315_s_at | NM_004327 | NM_004327 | 613 | 0.72 | 5.57E-03 |
| | | | NM_021574 | | | |
| FPGT | 205140_at | NM_003838 | NM_003838 | 8790 | 0.72 | 4.20E-05 |
| FLJ13273 | 235387_at | AA740875 | NM_024751 | 79807 | 0.72 | 2.00E-06 |
| PRIM2A | 215708_s_at | AL121975 | NM_000947 | 5558 | 0.72 | 3.15E-03 |
| TOMM22 | 217960_s_at | NM_020243 | NM_020243 | 56993 | 0.72 | 3.02E-03 |
| CDH2 | 203440_at | M34064 | NM_001792 | 1000 | 0.72 | 1.27E-04 |
| DENR | 234915_s_at | BC000925 | NM_003677 | 8562 | 0.72 | 5.52E-06 |
| KIAA1280 | 219520_s_at | NM_018458 | NM_015691 | 55841 | 0.72 | 1.98E-03 |
| CDC5L | 209056_s_at | NM_001253 | NM_001253 | 988 | 0.72 | 5.21E-03 |
| MYH9 | 211926_s_at | AI827941 | NM_002473 | 4627 | 0.72 | 5.89E-03 |
| SLC25A13 | 203775_at | NM_014251 | NM_014251 | 10165 | 0.72 | 5.47E-03 |
| SEMA6A | 220454_s_at | NM_020796 | NM_020796 | 57556 | 0.72 | 5.20E-04 |
| SOX2 | 213722_at | AW007161 | NM_003106 | 6657 | 0.72 | 3.52E-04 |
| RP2 | 205191_at | NM_006915 | NM_006915 | 6102 | 0.72 | 1.30E-03 |
| AI291804 | 242856_at | AI291804 | --- | --- | 0.72 | 6.53E-03 |
| SLC26A2 | 224963_at | AK025078 | NM_000112 | 1836 | 0.72 | 3.23E-03 |
| GRIA1 | 211520_s_at | M64752 | NM_000827 | 2890 | 0.71 | 6.86E-04 |
| SCD | 211162_x_at | AF116616 | NM_005063 | 6319 | 0.71 | 8.56E-03 |
| TM4SF6 | 209109_s_at | U84895 | NM_003270 | 7105 | 0.71 | 1.07E-04 |
| CYB5-M | 201634_s_at | NM_030579 | NM_030579 | 80777 | 0.71 | 8.39E-03 |
| AW025579 | 213429_at | AW025579 | --- | --- | 0.71 | 1.53E-04 |
| CDW92 | 224595_at | AK022549 | NM_022109 | 23446 | 0.71 | 4.42E-03 |
| | | | NM_080546 | | | |
| KIAA1853 | 230010_at | AA608629 | NM_194286 | 84530 | 0.71 | 1.11E-03 |
| GCLM | 203925_at | NM_002061 | NM_002061 | 2730 | 0.71 | 2.48E-03 |
| TLK2 | 228627_at | AL041560 | NM_006852 | 11011 | 0.71 | 1.99E-03 |
| RNF144 | 204040_at | NM_014746 | NM_014746 | 9781 | 0.71 | 3.67E-03 |
| PAXIP1L | 212825_at | AI357401 | NM_007349 | 22976 | 0.71 | 7.70E-03 |
| PHOX2B | 207009_at | NM_003924 | NM_003924 | 8929 | 0.71 | 1.10E-05 |
| FAM19A2 | 241399_at | AI142028 | NM_178539 | 338811 | 0.71 | 3.12E-03 |
| RSN | 210716_s_at | M97501 | NM_002956 | 6249 | 0.71 | 1.47E-03 |
| | | | NM_198240 | | | |
| RB1 | 203132_at | NM_000321 | NM_000321 | 5925 | 0.71 | 9.52E-03 |
| AA603344 | 235964_x_at | AA603344 | --- | --- | 0.71 | 5.50E-03 |
| ZNF278 | 211392_s_at | AF242522 | NM_014323 | 23598 | 0.71 | 9.10E-03 |
| | | | NM_032050 | | | |
| | | | NM_032051 | | | |
| | | | NM_032052 | | | |
| DEK | 200934_at | NM_003472 | NM_003472 | 7913 | 0.71 | 7.14E-03 |
| GPC6 | 227059_at | AI651255 | NM_005708 | 10082 | 0.71 | 1.48E-03 |
| KBTBD4 | 223765_s_at | AF151086 | NM_016506 | 55709 | 0.71 | 2.77E-04 |
| | | | NM_018095 | | | |
| AA521504 | 235952_at | AA521504 | --- | --- | 0.71 | 7.68E-03 |
| LOC163404 | 231397_at | AF131783 | XM_375754 | 163404 | 0.71 | 9.06E-03 |
| CHRNB4 | 207516_at | NM_000750 | NM_000750 | 1143 | 0.70 | 2.92E-04 |
| UBE2L3 | 200676_s_at | NM_003347 | NM_003347 | 7332 | 0.70 | 3.80E-07 |
| | | | NM_198157 | | | |
| MAGEH1 | 218573_at | NM_014061 | NM_014061 | 28986 | 0.70 | 8.04E-07 |
| PKM2 | 201251_at | NM_002654 | NM_002654 | 5315 | 0.70 | 5.34E-03 |
| | | | NM_182470 | | | |
| | | | NM_182471 | | | |
| DVL2 | 57532_at | AW016304 | NM_004422 | 1856 | 0.70 | 2.07E-03 |
| AMMECR1 | 1553219_a_at | NM_015365 | NM_015365 | 9949 | 0.70 | 2.11E-03 |
| SLA/LP | 235516_at | AI038867 | NM_016955 | 51091 | 0.70 | 3.09E-03 |
| | | | NM_153825 | | | |
| PEPD | 202108_at | NM_000285 | NM_000285 | 5184 | 0.70 | 7.53E-04 |
| LDHA | 200650_s_at | NM_005566 | NM_005566 | 3939 | 0.70 | 3.92E-03 |
| MVK | 204056_s_at | NM_000431 | NM_000431 | 4598 | 0.70 | 3.92E-03 |
| RAB22A | 218360_at | NM_020673 | NM_020673 | 57403 | 0.70 | 2.55E-03 |
| MAP3K71P1 | 203901_at | NM_006116 | NM_006116 | 10454 | 0.70 | 4.29E-03 |
| | | | NM_153497 | | | |
| HMGCR | 202540_s_at | NM_000859 | NM_000859 | 3156 | 0.70 | 6.44E-04 |
| AGPS | 225113_at | BF688144 | NM_003659 | 8540 | 0.70 | 1.13E-03 |
| ZNF305 | 240181_at | AI939511 | NM_014724 | 9753 | 0.70 | 2.45E-03 |
| SATB2 | 213435_at | AB028957 | NM_015265 | 23314 | 0.70 | 6.66E-03 |
| HPS4 | 54037_at | AL041451 | NM_022081 | 89781 | 0.70 | 1.86E-05 |
| | | | NM_152840 | | | |
| | | | NM_152841 | | | |
| | | | NM_152842 | | | |
| | | | NM_152843 | | | |
| B4GALT4 | 210540_s_at | BC004523 | NM_003778 | 8702 | 0.70 | 4.75E-03 |
| | | | NM_212543 | | | |
| ZNF555 | 1556188_a_at | AF052118 | NM_152791 | 148254 | 0.70 | 9.31E-03 |
| NEDD1 | 234984_at | AA236927 | NM_152905 | 121441 | 0.70 | 9.72E-03 |
| KIAA1648 | 213058_at | AL033538 | --- | 284900 | 0.70 | 4.59E-03 |
| MITF | 207233_s_at | NM_000248 | NM_000248 | 4286 | 0.69 | 9.18E-03 |
| | | | NM_006722 | | | |
| | | | NM_198158 | | | |
| | | | NM_198159 | | | |
| | | | NM_198177 | | | |
| | | | NM_198178 | | | |
| TM4SF9 | 225388_at | AI928507 | NM_005723 | 10098 | 0.69 | 1.96E-05 |
| GGA2 | 214190_x_at | AI799984 | NM_015044 | 23062 | 0.69 | 5.04E-04 |
| | | | NM_138640 | | | |
| FLJ10199 | 218815_s_at | NM_018022 | NM_018022 | 55092 | 0.69 | 5.48E-03 |
| SAMHD1 | 234987_at | AV715309 | NM_015474 | 25939 | 0.69 | 8.34E-03 |
| IDH3B | 210418_s_at | AF023265 | NM_006899 | 3420 | 0.69 | 9.34E-05 |
| | | | NM_174855 | | | |
| | | | NM_174856 | | | |
| BNIP3 | 201849_at | NM_004052 | NM_004052 | 664 | 0.69 | 3.88E-03 |
| CANP | 1557129_a_at | AA960844 | NM_198947 | 374393 | 0.69 | 8.06E-03 |
| GNG4 | 205184_at | NM_004485 | NM_004485 | 2786 | 0.69 | 4.91E-03 |
| G3BP | 201514_s_at | NM_005754 | NM_005754 | 10146 | 0.69 | 7.45E-05 |
| | | | NM_198395 | | | |
| B3GNT1 | 222870_s_at | AF288208 | NM_006577 | 10678 | 0.69 | 3.54E-03 |
| | | | NM_033252 | | | |
| CENTB2 | 212476_at | D26069 | NM_012287 | 23527 | 0.69 | 1.34E-03 |
| LBR | 201795_at | NM_002296 | NM_002296 | 3930 | 0.69 | 3.92E-04 |
| | | | NM_194442 | | | |
| EB-1 | 227440_at | AW005572 | NM_020140 | 56899 | 0.69 | 8.38E-06 |
| | | | NM_152788 | | | |
| | | | NM 181670 | | | |
| LANCL1 | 202019_s_at | AI935255 | NM_006055 | 10314 | 0.69 | 4.49E-03 |
| AI857788 | 227126_at | AI857788 | --- | --- | 0.69 | 9.75E-03 |
| GPC1 | 202756_s_at | NM_002081 | NM_002081 | 2817 | 0.69 | 1.42E-05 |
| RBM14 | 204178_s_at | NM_006328 | NM_006328 | 10432 | 0.69 | 2.67E-03 |
| CRKL | 212180_at | AK000311 | NM_005207 | 1399 | 0.68 | 3.46E-07 |
| SMC1L1 | 201589_at | D80000 | NM_006306 | 8243 | 0.68 | 1.70E-03 |
| NFIB | 213032_at | AI186739 | NM_005596 | 4781 | 0.68 | 4.95E-03 |
| RBM14 | 1555639_a_at | AF315633 | NM_006328 | 10432 | 0.68 | 5.28E-04 |
| IDH3B | 210014_x_at | AF023266 | NM_006899 | 3420 | 0.68 | 2.88E-04 |
| | | | NM_174855 | | | |
| | | | NM_174856 | | | |
| SC4MOL | 209146_at | AV704962 | NM_006745 | 6307 | 0.68 | 1.06E-05 |
| SLC25A15 | 222705_s_at | BC002702 | NM_014252 | 10166 | 0.68 | 5.67E-05 |
| ESD | 228162_at | AA193515 | NM_001984 | 2098 | 0.68 | 7.72E-03 |
| PPAT | 209433_s_at | AI457120 | NM_002703 | 5471 | 0.68 | 1.12E-03 |
| ROD1 | 224618_at | BF059136 | NM_005156 | 9991 | 0.68 | 6.34E-03 |
| ATP7A | 205197_s_at | BE567813 | NM_000052 | 538 | 0.68 | 6.71E-03 |
| AK022438 | 233508_at | AK022438 | --- | --- | 0.68 | 3.52E-03 |
| BG337831 | 238065_at | BG337831 | --- | --- | 0.68 | 8.60E-03 |
| CDS2 | 212864_at | Y16521 | NM_003818 | 8760 | 0.68 | 1.15E-04 |
| MGC34646 | 224996_at | N30209 | NM_173519 | 157807 | 0.68 | 1.11E-04 |
| RECK | 205407_at | NM_021111 | NM_021111 | 8434 | 0.67 | 8.99E-03 |
| MCAM | 211042_x_at | BC006329 | NM_006500 | 4162 | 0.67 | 2.11E-04 |
| LOC128977 | 1553974_at | BC030758 | NM_173793 | 128977 | 0.67 | 2.13E-04 |
| SNX5 | 222417_s_at | AF121855 | NM_014426 | 27131 | 0.67 | 7.63E-04 |
| | | | NM_152227 | | | |
| C20orf22 | 228123_s_at | AW303865 | NM_015600 | 26090 | 0.67 | 2.89E-04 |
| PHF20 | 235389_at | BG168139 | NM_016436 | 51230 | 0.67 | 9.09E-03 |
| NM_014838 | 204799_at | NM_014838 | --- | --- | 0.67 | 1.84E-04 |
| Cep70 | 1554489_a_at | BC016050 | NM_024491 | 80321 | 0.67 | 5.84E-03 |
| GCAT | 205164_at | NM_014291 | NM_014291 | 23464 | 0.67 | 9.67E-03 |
| MGC20255 | 225257_at | AA534536 | NM_052848 | 90324 | 0.67 | 2.25E-04 |
| KIAA1715 | 225717_at | AI814587 | NM_030650 | 80856 | 0.67 | 2.65E-03 |
| SH3MD1 | 224817_at | W93554 | NM_014631 | 9644 | 0.67 | 3.37E-04 |
| NFYA | 204108_at | AL031778 | NM_002505 | 4800 | 0.67 | 6.22E-03 |
| | | | NM_021705 | | | |
| MGC10854 | 223456_s_at | BC004285 | NM_032300 | 84260 | 0.67 | 2.49E-03 |
| DNAJC13 | 212467_at | AB014578 | NM_173823 | 23317 | 0.67 | 4.58E-05 |
| SEMA6A | 223449_at | AF225425 | NM_020796 | 57556 | 0.67 | 7.98E-03 |
| BC017932 | 1569283_at | BC017932 | XM_495940 | 440122 | 0.67 | 1.88E-03 |
| CDW92 | 224596_at | AI634866 | NM_022109 | 23446 | 0.67 | 6.95E-03 |
| | | | NM_080546 | | | |
| HMGCS1 | 221750_at | BG035985 | NM_002130 | 3157 | 0.67 | 5.27E-04 |
| IDH3B | 201509_at | NM_006899 | NM_006899 | 3420 | 0.66 | 3.23E-04 |
| | | | NM_174855 | | | |
| | | | NM_174856 | | | |
| CECR6 | 224393_s_at | AF307451 | NM_031890 | 27439 | 0.66 | 3.23E-05 |
| IPO11 | 222659_at | AK001696 | NM_016338 | 51194 | 0.66 | 3.34E-03 |
| ACTL6B | 206013_s_at | NM_016188 | NM_016188 | 51412 | 0.66 | 5.47E-03 |
| D15Wsu75e | 212527_at | BF057059 | XM_039495 | 27351 | 0.66 | 1.32E-04 |
| AL134573 | 228208_x_at | AL134573 | --- | --- | 0.66 | 1.64E-03 |
| MLLT3 | 204918_s_at | NM_004529 | NM_004529 | 4300 | 0.66 | 8.51 E-03 |
| GGA2 | 208913_at | AA868560 | NM_015044 | 23062 | 0.66 | 2.22E-03 |
| | | | NM_138640 | | | |
| ROD1 | 224617_at | AI735576 | NM_005156 | 9991 | 0.66 | 3.70E-03 |
| SNN | 218032_at | AF070673 | NM_003498 | 8303 | 0.66 | 5.14E-03 |
| CD47 | 213055_at | BF693956 | NM_001777 | 961 | 0.66 | 2.63E-03 |
| | | | NM_198793 | | | |
| ENO1 | 217294_s_at | U88968 | NM_001428 | 2023 | 0.66 | 2.43E-04 |
| SESN3 | 235150_at | AW376955 | NM_144665 | 143686 | 0.66 | 7.70E-04 |
| CRY1 | 209674_at | D83702 | NM_004075 | 1407 | 0.66 | 2.50E-03 |
| NM_018243 | 201308_s_at | NM_018243 | NM_018243 | 55752 | 0.66 | 3.72E-03 |
| CBX5 | 241875_at | AA204654 | NM_012117 | 23468 | 0.66 | 1.75E-03 |
| ZFYVE16 | 203651_at | NM_014733 | NM_014733 | 9765 | 0.66 | 1.76E-05 |
| NRF1 | 204652_s_at | NM_005011 | NM_005011 | 4899 | 0.66 | 2.20E-03 |
| GALNT2 | 217788_s_at | NM_004481 | NM_004481 | 2590 | 0.66 | 6.79E-06 |
| SNX5 | 217792_at | NM_014426 | NM_014426 | 27131 | 0.66 | 1.46E-03 |
| | | | NM_152227 | | | |
| ALK | 208212_s_at | NM_004304 | NM_004304 | 238 | 0.65 | 2.47E-03 |
| WDR44 | 219297_at | NM_019045 | NM_019045 | 54521 | 0.65 | 3.28E-04 |
| ELMOD2 | 1553928_at | NM_153702 | NM_153702 | 255520 | 0.65 | 1.79E-04 |
| VAPB | 202550_s_at | NM_004738 | NM_004738 | 9217 | 0.65 | 3.71E-05 |
| LOC129285 | 1553955_at | AY134855 | NM_152994 | 129285 | 0.65 | 3.87E-03 |
| MPHOSPH9 | 206205_at | NM_022782 | NM_022782 | 10198 | 0.65 | 7.66E-05 |
| SCML2 | 206147_x_at | NM_006089 | NM_006089 | 10389 | 0.65 | 1.04E-03 |
| HS6ST2 | 230030_at | AI767756 | NM_147174 | 90161 | 0.65 | 3.43E-03 |
| | | | NM_147175 | | | |
| NUP43 | 219007_at | NM_024647 | NM_024647 | 348995 | 0.65 | 6.58E-03 |
| | | | NM_198887 | | | |
| R12027 | 241836_x_at | R12027 | --- | --- | 0.65 | 6.67E-03 |
| H16725 | 239138_at | H16725 | --- | | 0.65 | 7.65E-03 |
| AGPS | 205401_at | NM_003659 | NM_003659 | 8540 | 0.65 | 1.27E-03 |
| DKFZP564M082 | 204218_at | NM_014042 | NM_014042 | 25906 | 0.65 | 2.63E-03 |
| MCART1 | 214126_at | N39314 | NM_033412 | 92014 | 0.65 | 6.73E-04 |
| NUP50 | 218295_s_at | NM_007172 | NM_007172 | 10762 | 0.65 | 4.08E-04 |
| | | | NM_153645 | | | |
| | | | NM_153684 | | | |
| PDK1 | 226452_at | AU146532 | NM_002610 | 5163 | 0.65 | 7.55E-03 |
| GPR125 | 1555122_at | BC026009 | NM_145290 | 166647 | 0.65 | 1.16E-03 |
| KIAA0692 | 212200_at | AK025933 | --- | 23141 | 0.65 | 3.35E-04 |
| ZADH1 | 230774_at | BE465894 | NM_152444 | 145482 | 0.65 | 1.11E-03 |
| CD24 | 209772_s_at | X69397 | NM_013230 | 934 | 0.65 | 3.11E-04 |
| KIAA1715 | 225718_at | AL133768 | NM_030650 | 80856 | 0.65 | 2.15E-03 |
| MANEA | 222805_at | AI587307 | NM_024641 | 79694 | 0.65 | 3.26E-04 |
| C10orf86 | 219067_s_at | NM_017615 | NM_017615 | 54780 | 0.65 | 6.76E-03 |
| AL524175 | 225479_at | AL524175 | --- | --- | 0.64 | 4.37E-05 |
| MICAL-L1 | 221779_at | BC001090 | NM_033386 | 85377 | 0.64 | 1.27E-03 |
| AI708256 | 229342_at | AI708256 | --- | --- | 0.64 | 1.31E-05 |
| G3BP | 201503_at | BG500067 | NM_005754 | 10146 | 0.64 | 3.53E-03 |
| | | | NM_198395 | | | |
| PLCXD1 | 222795_s_at | BE675241 | NM_018390 | 55344 | 0.64 | 2.51E-03 |
| CD47 | 226016_at | AL118798 | NM_001777 | 961 | 0.64 | 1.22E-07 |
| | | | NM_198793 | | | |
| RAB8B | 219210_s_at | NM_016530 | NM_016530 | 51762 | 0.64 | 2.35E-04 |
| RNF150 | 227657_at | AA722069 | NM_020724 | 57484 | 0.64 | 5.51E-03 |
| TEX261 | 212083_at | BG170659 | NM_144582 | 113419 | 0.64 | 6.37E-03 |
| BE893995 | 234983_at | BE893995 | --- | --- | 0.64 | 1.28E-03 |
| MGC3731 | 222057_at | AW002578 | NM_024313 | 79159 | 0.64 | 8.73E-03 |
| LOC284801 | 225762_x_at | AL531683 | XM_378973 | 284801 | 0.64 | 3.26E-03 |
| SLC25A17 | 211754_s_at | BC005957 | NM_006358 | 10478 | 0.64 | 9.76E-04 |
| KIAA0692 | 212201_at | AW274877 | --- | 23141 | 0.64 | 2.57E-03 |
| PCDHB2 | 231725_at | NM_018936 | NM_018936 | 56133 | 0.64 | 1.47E-03 |
| ATP1A1 | 220948_s_at | NM_000701 | NM_000701 | 476 | 0.64 | 2.01E-03 |
| | | | NM_00100158 6 | | | |
| CHKB | 204193_at | NM_005198 | NM_005198 | 1120 | 0.64 | 3.36E-03 |
| | | | NM_152253 | | | |
| MPHOSPH9 | 215731_s_at | X98258 | NM_022782 | 10198 | 0.64 | 3.26E-03 |
| KIAA0877 | 1560916_a_at | BI461155 | XM_371891 | 23333 | 0.64 | 1.49E-03 |
| | | | XM_374422 | | | |
| MAC30 | 212279_at | BE779865 | NM_014573 | 27346 | 0.64 | 1.07E-03 |
| RPA1 | 201529_s_at | NM_002945 | NM_002945 | 6117 | 0.64 | 4.43E-03 |
| HADHSC | 211569_s_at | AF001903 | NM_005327 | 3033 | 0.64 | 4.68E-03 |
| HNRPC | 227110_at | AA126793 | NM_004500 | 3183 | 0.64 | 8.86E-04 |
| | | | NM_031314 | | | |
| C2orf12 | 225269_s_at | BE384529 | --- | 192137 | 0.64 | 5.65E-05 |
| GSK3B | 209945_s_at | BC000251 | NM_002093 | 2932 | 0.64 | 8.48E-04 |
| RCN3 | 61734_at | AI797684 | NM_020650 | 57333 | 0.64 | 1.45E-03 |
| MANEA | 219003_s_at | NM_024641 | NM_024641 | 79694 | 0.63 | 1.47E-04 |
| LOC339448 | 238010_at | BF970340 | NM_207356 | 339448 | 0.63 | 6.74E-03 |
| U1SNRNPBP | 205300_s_at | NM_022717 | NM_007020 | 11066 | 0.63 | 7.93E-03 |
| | | | NM_022717 | | | |
| | | | NM_180699 | | | |
| | | | NM_180703 | | | |
| AB014540 | 209307_at | AB014540 | --- | --- | 0.63 | 5.07E-03 |
| MKL1 | 212748_at | AB037859 | NM_020831 | 57591 | 0.63 | 9.09E-04 |
| NBS1 | 217299_s_at | AK001017 | NM_002485 | 4683 | 0.63 | 5.38E-03 |
| ACSL3 | 201662_s_at | D89053 | NM_004457 | 2181 | 0.63 | 3.55E-04 |
| | | | NM_203372 | | | |
| GALNT2 | 223991_s_at | AF130059 | NM_004481 | 2590 | 0.63 | 9.09E-04 |
| FBXL10 | 226215_s_at | AI989996 | NM_00100536 6 | 84678 | 0.63 | 6.47E-03 |
| | | | NM_032590 | | | |
| C1orf48 | 230592_at | BE501789 | NM_015471 | 25936 | 0.63 | 6.21E-03 |
| FSCN1 | 210933_s_at | BC004908 | NM_003088 | 6624 | 0.63 | 7.38E-03 |
| ALMS1 | 214221_at | A1825212 | NM_015120 | 7840 | 0.63 | 6.34E-03 |
| MGC3040 | 223524_s_at | BC000568 | NM_023943 | 66000 | 0.63 | 8.05E-03 |
| WDR5 | 223308_s_at | AL521101 | NM_017588 | 11091 | 0.63 | 1.08E-03 |
| | | | NM_052821 | | | |
| RBMS1 | 203748_x_at | NM_016839 | NM_002897 | 5937 | 0.63 | 4.92E-04 |
| | | | NM_016836 | | | |
| | | | NM_016839 | | | |
| ACTR6 | 218395_at | NM_022496 | NM_022496 | 64431 | 0.63 | 1.97E-06 |
| TOMM22 | 222474_s_at | AB041906 | NM_020243 | 56993 | 0.63 | 2.85E-03 |
| LOC129138 | 231847_at | Z97630 | NM_138797 | 129138 | 0.63 | 1.02E-04 |
| LOC338799 | 226369_at | AW138760 | --- | 338799 | 0.63 | 3.36E-03 |
| SLC2A4RG | 222650_s_at | BE898559 | NM_020062 | 56731 | 0.63 | 7.06E-04 |
| SCOC | 224786_at | AL133580 | NM_032547 | 60592 | 0.63 | 1.37E-05 |
| MGC3731 | 219324_at | NM_024313 | NM_024313 | 79159 | 0.62 | 1.54E-03 |
| C10orf6 | 203481_at | AI655902 | NM_018121 | 55719 | 0.62 | 2.18E-04 |
| NUP133 | 233421_s_at | AU146738 | NM_018230 | 55746 | 0.62 | 7.97E-03 |
| CSRP2BP | 228543_at | AA780252 | NM_020536 | 57325 | 0.62 | 6.49E-04 |
| | | | NM_177926 | | | |
| HSPC129 | 1555106_a_at | BC035744 | NM_016396 | 51496 | 0.62 | 1.80E-03 |
| MGC5139 | 202365_at | BC004815 | --- | 84747 | 0.62 | 1.02E-04 |
| HIC2 | 212964_at | AI912206 | NM_015094 | 23119 | 0.62 | 1.32E-04 |
| FCHO2 | 228220_at | AI627666 | NM_138782 | 115548 | 0.62 | 4.50E-03 |
| CDK5RAP1 | 218315_s_at | NM_016408 | NM_016082 | 51654 | 0.62 | 7.08E-05 |
| | | | NM_016408 | | | |
| FH | 214170_x_at | AA669797 | NM_000143 | 2271 | 0.62 | 9.05E-03 |
| ENTPD6 | 201704_at | NM_001247 | NM_001247 | 955 | 0.62 | 1.44E-03 |
| NUP50 | 213682_at | AL036344 | NM_007172 | 10762 | 0.62 | 2.73E-03 |
| | | | NM_153645 | | | |
| | | | NM_153684 | | | |
| SNX5 | 229980_s_at | AA131508 | NM_014426 | 27131 | 0.62 | 1.66E-04 |
| | | | NM_152227 | | | |
| B4GALT6 | 235333_at | BG503479 | NM_004775 | 9331 | 0.62 | 4.18E-03 |
| LOC150383 | 227590_at | BE501980 | NM_00100891 7 | 150383 | 0.62 | 1.10E-03 |
| | | | NM_207327 | | | |
| SYNGR1 | 213854_at | BF511590 | NM_004711 | 9145 | 0.62 | 3.40E-03 |
| | | | NM_145731 | | | |
| | | | NM_145738 | | | |
| DZIP3 | 207231_at | NM_014648 | NM_014648 | 9666 | 0.62 | 5.60E-05 |
| CSRP2BP | 228544_s_at | AA780252 | NM_020536 | 57325 | 0.62 | 8.59E-03 |
| | | | NM_177926 | | | |
| SCC-112 | 212138_at | AK021757 | NM_015200 | 23244 | 0.62 | 2.22E-06 |
| TEAD1 | 224955_at | AI590088 | NM_021961 | 7003 | 0.62 | 1.84E-03 |
| DYRK4 | 212954_at | AF263541 | NM_003845 | 8798 | 0.62 | 2.89E-04 |
| RAPH1 | 243012_at | BF196252 | NM_025252 | 65059 | 0.62 | 5.72E-03 |
| | | | NM_203365 | | | |
| | | | NM_213589 | | | |
| SC5DL | 211423_s_at | D85181 | NM_006918 | 6309 | 0.61 | 1.75E-03 |
| BUB3 | 201457_x_at | AF081496 | NM_00100779 3 | 9184 | 0.61 | 5.81E-03 |
| | | | NM_004725 | | | |
| TMEM38B | 218772_x_at | NM_018112 | NM_018112 | 55151 | 0.61 | 1.91E-03 |
| TPARL | 218095_s_at | NM_018475 | NM_018475 | 55858 | 0.61 | 1.44E-03 |
| HRPT2 | 235196_at | AA447464 | NM_024529 | 79577 | 0.61 | 2.37E-03 |
| KIAA0406 | 212898_at | AB007866 | NM_014657 | 9675 | 0.61 | 1.44E-03 |
| FBXO30 | 226541_at | AI808182 | NM_032145 | 84085 | 0.61 | 1.59E-03 |
| EPOR | 209963_s_at | M34986 | NM_000121 | 2057 | 0.61 | 8.17E-03 |
| AP3B1 | 203141_s_at | AW058575 | NM_003664 | 8546 | 0.61 | 1.23E-04 |
| FLJ33008 | 228954_at | AW452620 | NM_152449 | 145748 | 0.61 | 3.98E-04 |
| FLJ21908 | 1557984_s_at | BI464019 | NM_024604 | 79657 | 0.61 | 1.58E-03 |
| ALS2CR2 | 223266_at | AB038950 | NM_018571 | 55437 | 0.61 | 6.51E-05 |
| RPP30 | 203436_at | NM_006413 | NM_006413 | 10556 | 0.61 | 4.85E-03 |
| LOC388272 | 226608_at | AW157311 | NM_001001436 | 388272 | 0.61 | 7.08E-03 |
| AI421677 | 242964_at | AI421677 | --- | --- | 0.61 | 2.28E-04 |
| HSDL1 | 223248_at | AK025626 | NM_031463 | 83693 | 0.61 | 3.31E-04 |
| GLT25D1 | 222644_s_at | AI924150 | NM_024656 | 79709 | 0.61 | 3.50E-03 |
| CBX5 | 209715_at | L07515 | NM_012117 | 23468 | 0.61 | 7.77E-03 |
| SCOC | 223341_s_at | AF330205 | NM_032547 | 60592 | 0.61 | 7.55E-03 |
| FLJ22531 | 204922_at | NM_024650 | NM_024650 | 79703 | 0.61 | 4.70E-03 |
| TMOD3 | 220800_s_at | NM_014547 | NM_014547 | 29766 | 0.61 | 1.32E-03 |
| ZFP161 | 209724_s_at | AL534416 | NM_003409 | 7541 | 0.61 | 7.01E-05 |
| AL563613 | 226532_at | AL563613 | --- | --- | 0.61 | 3.89E-03 |
| SESN3 | 242899_at | N78412 | NM_144665 | 143686 | 0.61 | 2.87E-03 |
| PGM2 | 223738_s_at | AL136705 | NM_018290 | 55276 | 0.61 | 9.76E-05 |
| ORC4L | 203352_at | NM_002552 | NM_002552 | 5000 | 0.60 | 3.05E-03 |
| | | | NM_181741 | | | |
| | | | NM_181742 | | | |
| PANK3 | 218433_at | NM_024594 | NM_024594 | 79646 | 0.60 | 9.11E-03 |
| MGC13170 | 224468_s_at | BC006151 | NM_199249 | 84798 | 0.60 | 2.79E-04 |
| | | | NM_199250 | | | |
| HABP4 | 209818_s_at | AF241831 | NM_014282 | 22927 | 0.60 | 5.02E-04 |
| RBMS1 | 225265_at | AI580100 | NM_002897 | 5937 | 0.60 | 1.37E-03 |
| | | | NM_016836 | | | |
| | | | NM_016839 | | | |
| SESN3 | 225123_at | BE883841 | NM_144665 | 143686 | 0.60 | 3.28E-05 |
| RG9MTD1 | 223267_at | AF226052 | NM_017819 | 54931 | 0.60 | 4.25E-05 |
| SEMA6A | 225660_at | W92748 | NM_020796 | 57556 | 0.60 | 3.33E-03 |
| PPM1F | 37384_at | D86995 | NM_014634 | 9647 | 0.60 | 9.35E-04 |
| BMP2K | 219546_at | NM_017593 | NM_017593 | 55589 | 0.60 | 7.82E-04 |
| | | | NM_198892 | | | |
| SCD | 211708_s_at | BC005807 | NM_005063 | 6319 | 0.60 | 3.69E-03 |
| LOC286505 | 226421_at | AA707320 | --- | 286505 | 0.60 | 6.02E-03 |
| TOMM70A | 201512_s_at | BC003633 | NM_014820 | 9868 | 0.60 | 8.65E-03 |
| ANXA11 | 206200_s_at | NM_001157 | NM_001157 | 311 | 0.60 | 1.86E-03 |
| | | | NM_145868 | | | |
| | | | NM_145869 | | | |
| AW971254 | 222369_at | AW971254 | --- | --- | 0.60 | 7.78E-04 |
| AI754928 | 226347_at | AI754928 | --- | --- | 0.60 | 4.04E-03 |
| NHLRC2 | 235356_at | AW297204 | NM_198514 | 374354 | 0.60 | 4.48E-03 |
| HDAC1 | 201209_at | NM_004964 | NM_004964 | 3065 | 0.60 | 2.74E-05 |
| MGC14141 | 225568_at | BE728983 | NM_032928 | 85014 | 0.60 | 4.04E-04 |
| NIT2 | 218557_at | NM_020202 | NM_020202 | 56954 | 0.59 | 8.84E-03 |
| SREBF2 | 201247_at | BE513151 | NM_004599 | 6721 | 0.59 | 7.04E-03 |
| MKKS | 218138_at | NM_018848 | NM_018848 | 8195 | 0.59 | 1.60E-04 |
| | | | NM_170784 | | | |
| EPIM | 213434_at | H95263 | NM_001980 | 2054 | 0.59 | 3.73E-03 |
| | | | NM_194356 | | | |
| NDRG3 | 221082_s_at | NM_022477 | NM_022477 | 57446 | 0.59 | 7.59E-04 |
| | | | NM_032013 | | | |
| TIGD7 | 224365_s_at | AF251050 | NM_033208 | 91151 | 0.59 | 8.93E-03 |
| R68654 | 236971_at | R68654 | --- | --- | 0.59 | 5.73E-03 |
| BE675101 | 236384_at | BE675101 | --- | --- | 0.59 | 2.31E-04 |
| MAC30 | 212281_s_at | BF038366 | NM_014573 | 27346 | 0.59 | 1.68E-04 |
| FLJ12806 | 220199_s_at | NM_022831 | NM_022831 | 64853 | 0.59 | 2.17E-05 |
| ASF1A | 203427_at | NM_014034 | NM_014034 | 25842 | 0.59 | 1.25E-04 |
| CTCF | 202521_at | NM_006565 | NM_006565 | 10664 | 0.59 | 4.94E-05 |
| PSCD3 | 225147_at | AL521959 | NM_004227 | 9265 | 0.59 | 9.96E-03 |
| IQSEC3 | 213834_at | AB029033 | XM_370667 | 440073 | 0.59 | 2.90E-04 |
| | | | XM_495902 | | | |
| MPHOSPH9 | 221965_at | AI990326 | NM_022782 | 10198 | 0.58 | 9.17E-03 |
| DNCI2 | 236698_at | AI823600 | NM_001378 | 1781 | 0.58 | 1.70E-03 |
| ZNRF3 | 226360_at | AK022809 | XM_290972 | 84133 | 0.58 | 2.94E-03 |
| OSBPL11 | 222586_s_at | AI884890 | NM_022776 | 114885 | 0.58 | 2.49E-03 |
| NIPSNAP3A | 224436_s_at | BC005935 | NM_015469 | 25934 | 0.58 | 3.38E-03 |
| NBS1 | 202906_s_at | AF049895 | NM_002485 | 4683 | 0.58 | 4.86E-03 |
| MGC3162 | 218860_at | NM_024078 | NM_024078 | 79050 | 0.58 | 3.07E-04 |
| FLJ13149 | 219016_at | NM_021826 | NM_021826 | 60493 | 0.58 | 8.95E-03 |
| DIAPH1 | 213514_s_at | AU158818 | NM_005219 | 1729 | 0.58 | 7.31E-03 |
| IGSF4 | 209031_at | AL519710 | NM_014333 | 23705 | 0.58 | 2.03E-03 |
| ATP6V1A | 201971_s_at | NM_001690 | NM_001690 | 523 | 0.58 | 4.36E-04 |
| FLJ25952 | 225365_at | BG249221 | NM_153251 | 253832 | 0.58 | 2.88E-03 |

**Table 6. Decreased Gene Expression in Cell Lines H1 and BB10 vs. Cell Line 2D6 in Undifferentiated State Using Microarray Analysis**

| **Gene Symbol** | **Probe Set ID** | **Representativ e Public ID** | **RefSeq Transcript ID** | **Entrez Gene ID** | **Log2 Ratio** | **pvalue** |
|---|---|---|---|---|---|---|
| KIAA0125 | 206478_at | NM_014792 | NM_014792 | 9834 | -7.57 | 6.75E-06 |
| CART | 206339_at | NM_004291 | NM_004291 | 9607 | -6.35 | 6.05E-07 |
| CPNE8 | 228365_at | AI765180 | NM_153634 | 144402 | -5.76 | 5.62E-09 |
| MEOX2 | 206201_s_at | NM_005924 | NM_005924 | 4223 | -5.64 | 6.26E-06 |
| CYTL1 | 219837_s_at | NM_018659 | NM_018659 | 54360 | -5.64 | 1.55E-10 |
| CYGB | 226632_at | AL513673 | NM_134268 | 114757 | -5.63 | 1.66E-03 |
| FGF13 | 205110_s_at | NM_004114 | NM_004114 | 2258 | -5.18 | 1.42E-06 |
| | | | NM_033642 | | | |
| CPNE8 | 241706_at | AA431782 | NM_153634 | 144402 | -4.70 | 2.99E-10 |
| CYGB | 1553572_a_at | NM_134268 | NM_134268 | 114757 | -4.40 | 5.68E-05 |
| BC036254 | 1557443_s_at | BC036254 | --- | --- | -4.26 | 3.10E-05 |
| SLC1A2 | 225491_at | AL157452 | NM_004171 | 6506 | -4.14 | 7.08E-13 |
| CAP2 | 212554_at | N90755 | NM_006366 | 10486 | -4.08 | 3.14E-07 |
| AL353759 | 215071_s_at | AL353759 | --- | --- | -3.91 | 1.82E-05 |
| GNAS | 217057_s_at | AF107846 | NM_000516 | 2778 | -3.79 | 5.67E-04 |
| | | | NM_016592 | | | |
| | | | NM_080425 | | | |
| | | | NM_080426 | | | |
| CFC1 | 223753_s_at | AF312769 | NM_032545 | 55997 | -3.75 | 5.58E-06 |
| GRM5 | 214217_at | D60132 | NM_000842 | 2915 | -3.73 | 3.90E-09 |
| CYP2E1 | 1431_at | J02843 | NM_000773 | 1571 | -3.66 | 1.03E-03 |
| LOC56901 | 218484_at | NM_020142 | NM_020142 | 56901 | -3.66 | 1.04E-04 |
| PAPPA | 224942_at | BG434272 | NM_002581 | 5069 | -3.64 | 2.15E-04 |
| AQP1 | 209047_at | AL518391 | NM_000385 | 358 | -3.61 | 1.21E-05 |
| | | | NM_198098 | | | |
| BG325630 | 234973_at | BG325630 | --- | --- | -3.57 | 9.05E-04 |
| CDKN2A | 209644_x_at | U38945 | NM_000077 | 1029 | -3.30 | 4.00E-06 |
| | | | NM_058195 | | | |
| | | | NM_058197 | | | |
| CYP2E1 | 209975_at | AF182276 | NM_000773 | 1571 | -3.28 | 1.91E-03 |
| BF432956 | 229057_at | BF432956 | --- | --- | -3.21 | 2.21E-06 |
| ANK3 | 237839_at | BF433975 | NM_001149 | 288 | -3.19 | 4.55E-03 |
| | | | NM_020987 | | | |
| AU144437 | 232887_at | AU144437 | --- | --- | -3.17 | 9.97E-06 |
| LOC158572 | 243017_at | AA032156 | XM_379705 | 158572 | -3.12 | 5.87E-07 |
| SAMD4 | 212845_at | AB028976 | NM_015589 | 23034 | -3.12 | 2.46E-03 |
| M160 | 223655_at | AF264014 | NM_174941 | 283316 | -3.12 | 3.65E-05 |
| SYT13 | 226086_at | AB037848 | NM_020826 | 57586 | -3.11 | 1.85E-05 |
| AI093492 | 237435_at | AI093492 | --- | --- | -3.03 | 2.14E-03 |
| CYP2E1 | 209976_s_at | AF182276 | NM_000773 | 1571 | -3.01 | 9.74E-04 |
| N39597 | 1555993_at | N39597 | --- | --- | -2.99 | 1.61E-04 |
| ASAM | 226834_at | BG112263 | NM_024769 | 79827 | -2.98 | 6.28E-03 |
| KIAA1598 | 221802_s_at | AU157109 | NM_018330 | 57698 | -2.94 | 8.29E-05 |
| CDC42EP1 | 204693_at | NM_007061 | NM_007061 | 11135 | -2.90 | 4.19E-03 |
| | | | NM_152243 | | | |
| N92599 | 241421_at | N92599 | --- | --- | -2.86 | 2.63E-05 |
| GDF15 | 221577_x_at | AF003934 | NM_004864 | 9518 | -2.84 | 2.08E-03 |
| AI341823 | 239657_x_at | AI341823 | --- | --- | -2.82 | 2.19E-05 |
| AW665665 | 237187_at | AW665665 | --- | --- | -2.78 | 1.83E-03 |
| SLC35F3 | 229065_at | BF968270 | NM_173508 | 148641 | -2.76 | 7.38E-06 |
| AA151917 | 230503_at | AA151917 | --- | --- | -2.73 | 3.30E-05 |
| PAPPA | 201982_s_at | NM_002581 | NM_002581 | 5069 | -2.70 | 2.76E-06 |
| IL17B | 220273_at | NM_014443 | NM_014443 | 27190 | -2.67 | 2.02E-04 |
| KIAA0125 | 220377_at | NM_014151 | | 9834 | -2.65 | 1.07E-05 |
| | | | NM_014792 | | | |
| ANK2 | 232606_at | AK021894 | NM_001148 | 287 | -2.65 | 4.00E-08 |
| | | | NM_020977 | | | |
| AK021495 | 215571_at | AK021495 | --- | --- | -2.65 | 9.41E-04 |
| ANK3 | 239726_at | AI743588 | NM_001149 | 288 | -2.64 | 4.93E-04 |
| | | | NM_020987 | | | |
| ANK2 | 1561895_at | BC030740 | NM_001148 | 287 | -2.63 | 1.24E-04 |
| | | | NM_020977 | | | |
| PAPPA | 224940_s_at | BF107618 | NM_002581 | 5069 | -2.63 | 4.08E-04 |
| PAPPA | 228128_x_at | AI110886 | NM_002581 | 5069 | -2.60 | 6.58E-04 |
| AW274468 | 242299_at | AW274468 | --- | --- | -2.54 | 9.96E-08 |
| GRIA2 | 205358_at | NM_000826 | NM_000826 | 2891 | -2.54 | 3.09E-03 |
| PAPPA | 201981_at | AA148534 | NM_002581 | 5069 | -2.51 | 2.17E-04 |
| ANK2 | 216195_at | AF131823 | NM_001148 | 287 | -2.51 | 1.40E-06 |
| | | | NM_020977 | | | |
| PAPPA | 224941_at | BF107618 | NM_002581 | 5069 | -2.50 | 1.61E-03 |
| LOC283404 | 1558195_at | BG204230 | --- | 283404 | -2.49 | 1.03E-07 |
| GRIP2 | 216481_at | AF052177 | XM_042936 | 80852 | -2.44 | 1.03E-04 |
| BF000047 | 235736_at | BF000047 | --- | --- | -2.43 | 2.70E-05 |
| FGF1 | 1552721_a_at | NM_033136 | NM_000800 | 2246 | -2.43 | 2.69E-04 |
| | | | NM_033136 | | | |
| | | | NM_033137 | | | |
| GRIA2 | 236538_at | BE219628 | NM_000826 | 2891 | -2.40 | 8.42E-04 |
| BM977131 | 1557478_at | BM977131 | --- | --- | -2.38 | 9.09E-05 |
| BHLHB2 | 201170_s_at | NM_003670 | NM_003670 | 8553 | -2.37 | 5.35E-03 |
| CAP2 | 212551_at | NM_006366 | NM_006366 | 10486 | -2.36 | 1.47E-03 |
| PTN | 211737_x_at | BC005916 | NM_002825 | 5764 | -2.35 | 1.99E-06 |
| AI051127 | 231478_at | AI051127 | --- | --- | -2.34 | 3.52E-03 |
| PHLDA2 | 209803_s_at | AF001294 | NM_003311 | 7262 | -2.32 | 1.79E-05 |
| RGS11 | 206107_at | NM_003834 | NM_003834 | 8786 | -2.31 | 1.17E-03 |
| | | | NM_183337 | | | |
| EGR1 | 201694_s_at | NM_001964 | NM_001964 | 1958 | -2.30 | 5.17E-03 |
| DKK1 | 204602_at | NM_012242 | NM_012242 | 22943 | -2.29 | 9.68E-04 |
| ZAP128 | 202982_s_at | NM_006821 | NM_006821 | 10965 | -2.29 | 2.38E-03 |
| AK096401 | 1556204_a_at | AK096401 | XM_375664 | 400721 | -2.28 | 4.02E-05 |
| AI459194 | 227404_s_at | AI459194 | --- | --- | -2.28 | 8.03E-03 |
| DKFZp313N062 | 1555905_a_at | AI147556 | NM_173826 | 285343 | -2.26 | 6.00E-04 |
| LOC51315 | 222585_x_at | BE326678 | NM_016618 | 51315 | -2.26 | 7.70E-05 |
| HIST2H2BE | 202708_s_at | NM_003528 | NM_003528 | 8349 | -2.25 | 5.90E-05 |
| BC035768 | 1559987_at | BC035768 | --- | --- | -2.24 | 2.82E-04 |
| ERBB4 | 206794_at | NM_005235 | NM_005235 | 2066 | -2.24 | 7.92E-04 |
| IL7 | 206693_at | NM_000880 | NM_000880 | 3574 | -2.20 | 1.47E-04 |
| AA601031 | 228919_at | AA601031 | --- | --- | -2.18 | 1.30E-03 |
| HS3ST1 | 205466_s_at | NM_005114 | NM_005114 | 9957 | -2.18 | 6.77E-04 |
| ANK3 | 215314_at | AU146646 | NM_001149 | 288 | -2.18 | 3.85E-04 |
| | | | NM_020987 | | | |
| LOC144571 | 1564139_at | AK056852 | --- | 144571 | -2.17 | 1.36E-03 |
| ENOSF1 | 213645_at | AF305057 | NM_017512 | 55556 | -2.16 | 1.79E-06 |
| RASGEF1B | 230233_at | BF110534 | NM_152545 | 153020 | -2.16 | 7.76E-03 |
| CREB5 | 205931_s_at | NM_004904 | NM_004904 | 9586 | -2.15 | 5.67E-03 |
| | | | NM_182898 | | | |
| | | | NM_182899 | | | |
| CG018 | 213375_s_at | N80918 | NM_052818 | 90634 | -2.14 | 7.06E-03 |
| GAL | 214240_at | AL556409 | NM_015973 | 51083 | -2.12 | 7.95E-03 |
| ANK2 | 202921_s_at | NM_001148 | NM_001148 | 287 | -2.11 | 3.33E-05 |
| | | | NM_020977 | | | |
| CAMK2B | 209956_s_at | U23460 | NM_001220 | 816 | -2.10 | 8.15E-06 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| PHKA2 | 1560358_at | BC038597 | NM_000292 | 5256 | -2.08 | 2.64E-05 |
| CACNA1D | 210108_at | BE550599 | NM_000720 | 776 | -2.08 | 2.44E-03 |
| AW954199 | 239331_at | AW954199 | --- | --- | -2.08 | 1.68E-07 |
| LOC150759 | 221973_at | AI983904 | XM_498456 | 150759 | -2.05 | 4.18E-04 |
| | | | XM_499585 | | | |
| ADCY1 | 235049_at | AA021120 | NM_021116 | 107 | -2.05 | 1.26E-03 |
| AI435590 | 237193_s_at | AI435590 | --- | --- | -2.02 | 9.66E-04 |
| AQP1 | 207542_s_at | NM_000385 | NM_000385 | 358 | -2.02 | 1.79E-03 |
| | | | NM_198098 | | | |
| BE219849 | 240180_at | BE219849 | --- | --- | -2.00 | 1.75E-03 |
| BF111884 | 230491_at | BF111884 | --- | --- | -2.00 | 8.24E-07 |
| KIAA0251 | 1558430_at | R08650 | NM_015027 | 23042 | -2.00 | 5.90E-03 |
| AI810266 | 235456_at | AI810266 | --- | --- | -2.00 | 2.25E-07 |
| PPP3CA | 1562467_at | AK097085 | NM_000944 | 5530 | -1.97 | 1.35E-03 |
| SYNPR | 230303_at | H11380 | NM_144642 | 132204 | -1.96 | 6.12E-03 |
| ADCY1 | 213245_at | AL120173 | NM_021116 | 107 | -1.96 | 8.54E-05 |
| BCAR3 | 239908_at | AA496799 | NM_003567 | 8412 | -1.96 | 2.42E-03 |
| BC025319 | 1568974_at | BC025319 | --- | --- | -1.95 | 6.55E-05 |
| DDEF1 | 1557685_at | AK054840 | NM_018482 | 50807 | -1.94 | 5.02E-04 |
| NEURL | 230767_at | BF448300 | NM_004210 | 9148 | -1.94 | 4.29E-04 |
| STAR | 204548_at | NM_000349 | NM_000349 | 6770 | -1.93 | 5.97E-05 |
| | | | NM_00100724 3 | | | |
| NAG8 | 210109_at | AF191492 | NM_014411 | 27099 | -1.92 | 7.56E-03 |
| LOC55831 | 215250_at | AU147317 | NM_018447 | 55831 | -1.92 | 4.21E-04 |
| AI927692 | 228528_at | AI927692 | --- | --- | -1.91 | 1.93E-03 |
| BC041941 | 1559597_at | BC041941 | --- | --- | -1.90 | 9.44E-04 |
| CLDN10 | 1556687_a_at | BE465772 | NM_006984 | 9071 | -1.90 | 2.11E-03 |
| | | | NM_182848 | | | |
| AI800713 | 235122_at | AI800713 | --- | --- | -1.88 | 8.12E-04 |
| ADARB1 | 203865_s_at | NM_015833 | NM_001112 | 104 | -1.87 | 6.53E-04 |
| | | | NM_015833 | | | |
| | | | NM_015834 | | | |
| LOC92162 | 229452_at | AL544576 | NM_203411 | 92162 | -1.86 | 1.23E-03 |
| AL049252 | 216527_at | AL049252 | --- | --- | -1.85 | 4.62E-05 |
| ARC | 210090_at | AF193421 | NM_015193 | 23237 | -1.85 | 1.06E-04 |
| FYN | 1559101_at | AK090692 | NM_002037 | 2534 | -1.85 | 6.72E-04 |
| | | | NM_153047 | | | |
| | | | NM_153048 | | | |
| AAK1 | 214956_at | AF090101 | NM_014911 | 22848 | -1.85 | 2.35E-03 |
| FARP1 | 227996_at | BF725250 | NM_00100171 5 | 10160 | -1.85 | 2.09E-03 |
| | | | NM_005766 | | | |
| PTGER2 | 206631_at | NM_000956 | NM_000956 | 5732 | -1.84 | 5.50E-04 |
| LOC360030 | 232744_x_at | BG485129 | --- | 360030 | -1.84 | 1.99E-03 |
| TCEAL2 | 211276_at | AF063606 | NM_080390 | 140597 | -1.84 | 4.69E-04 |
| SIGIRR | 218921_at | NM_021805 | NM_021805 | 59307 | -1.83 | 2.45E-03 |
| RAB7 | 239405_at | A1022632 | NM_004637 | 7879 | -1.83 | 1.40E-04 |
| CAMK2B | 210404_x_at | AF078803 | NM_001220 | 816 | -1.82 | 2.11E-06 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| ERBB4 | 214053_at | AW772192 | NM_005235 | 2066 | -1.82 | 9.63E-05 |
| FLJ13955 | 227001_at | AI096706 | NM_024759 | 79815 | -1.81 | 1.39E-03 |
| AI697701 | 243666_at | AI697701 | --- | --- | -1.81 | 1.63E-04 |
| AK024567 | 234597_at | AK024567 | --- | --- | -1.81 | 4.51E-07 |
| RHOQ | 1559582_at | BC033251 | NM_012249 | 23433 | -1.81 | 2.02E-03 |
| ATP2B3 | 207026_s_at | NM_021949 | NM_00100134 4 | 492 | -1.81 | 3.94E-03 |
| | | | NM_021949 | | | |
| BF055351 | 231205_at | BF055351 | --- | --- | -1.79 | 3.90E-03 |
| ACYP2 | 206833_s_at | NM_001108 | NM_138448 | 98 | -1.79 | 1.40E-04 |
| CAMK2B | 211483_x_at | AF081924 | NM_001220 | 816 | -1.78 | 7.39E-06 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| DUSP5 | 209457_at | U16996 | NM_004419 | 1847 | -1.77 | 7.59E-04 |
| NLF1 | 241031_at | BE218239 | NM_207322 | 145741 | -1.77 | 1.06E-06 |
| DTNA | 240125_at | AI697494 | NM_001390 | 1837 | -1.77 | 2.09E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| FLJ12671 | 233728_at | AU148213 | NM_030980 | 81875 | -1.77 | 1.42E-03 |
| TM4SF2 | 234245_at | AL162055 | NM_004615 | 7102 | -1.76 | 3.16E-03 |
| MGC13040 | 224463_s_at | BC006128 | NM_032930 | 85016 | -1.76 | 9.67E-03 |
| IGFBP5 | 211959_at | AW007532 | NM_000599 | 3488 | -1.76 | 5.39E-03 |
| COL9A2 | 213622_at | AI733465 | NM_001852 | 1298 | -1.76 | 2.66E-03 |
| AB088847 | 1555547_at | AB088847 | --- | --- | -1.76 | 4.05E-06 |
| UGCGL2 | 1558467_a_at | BE156619 | NM_020121 | 55757 | -1.75 | 2.63E-03 |
| GPX7 | 213170_at | AA406605 | NM_015696 | 2882 | -1.75 | 1.66E-03 |
| RLF | 241438_at | AI742686 | NM_012421 | 6018 | -1.75 | 3.53E-03 |
| DUSP16 | 1558739_at | R30807 | NM_030640 | 80824 | -1.74 | 1.87E-04 |
| SORCS1 | 1556891_at | BE328250 | NM_052918 | 114815 | -1.74 | 2.33E-03 |
| ANK2 | 202920_at | BF726212 | NM_001148 | 287 | -1.73 | 1.72E-04 |
| | | | NM_020977 | | | |
| AL119491 | 244292_at | AL119491 | --- | --- | -1.73 | 2.74E-03 |
| ATP2B3 | 242036_x_at | H09073 | NM_00100134 4 | 492 | -1.72 | 2.83E-03 |
| | | | NM_021949 | | | |
| DRD1IP | 219896_at | NM_015722 | NM_015722 | 50632 | -1.72 | 4.50E-04 |
| KCNQ2 | 205737_at | NM_004518 | NM_004518 | 3785 | -1.72 | 4.02E-06 |
| | | | NM_172106 | | | |
| | | | NM_172107 | | | |
| | | | NM_172108 | | | |
| | | | NM_172109 | | | |
| LOC51315 | 233329_s_at | AK025986 | NM_016618 | 51315 | -1.71 | 4.22E-05 |
| PNCK | 229411_at | AI986390 | NM_198452 | 139728 | -1.71 | 9.68E-05 |
| AA007336 | 237586_at | AA007336 | --- | --- | -1.71 | 5.37E-04 |
| AI681941 | 237663_at | AI681941 | --- | --- | -1.71 | 9.56E-04 |
| SRPK2 | 242755_at | BE672140 | NM_182691 | 6733 | -1.71 | 3.64E-05 |
| | | | NM_182692 | | | |
| C9orf3 | 232783_at | AI147867 | NM_032823 | 84909 | -1.70 | 4.59E-03 |
| RGAG4 | 227823_at | BE348679 | XM_291322 | 340526 | -1.70 | 6.60E-05 |
| AK098781 | 1560814_a_at | AK098781 | --- | --- | -1.69 | 7.73E-04 |
| ERBB4 | 233498_at | AK024204 | NM_005235 | 2066 | -1.69 | 1.49E-03 |
| C9orf10 | 241242_at | BE503118 | NM_014612 | 23196 | -1.68 | 1.66E-03 |
| BF342356 | 238934_at | BF342356 | --- | --- | -1.67 | 6.33E-06 |
| IER3 | 201631_s_at | NM_003897 | NM_003897 | 8870 | -1.67 | 2.10E-06 |
| | | | NM_052815 | | | |
| AU147091 | 232818_at | AU147091 | XM_097622 | 149297 | -1.67 | 1.82E-05 |
| SNX27 | 244349_at | AI807658 | NM_030918 | 81609 | -1.66 | 4.72E-04 |
| ACSL4 | 1559663_at | AF090916 | NM_004458 | 2182 | -1.66 | 4.29E-04 |
| | | | NM_022977 | | | |
| AA705933 | 239866_at | AA705933 | --- | --- | -1.66 | 1.19E-04 |
| PRR3 | 244204_at | W87300 | NM_025263 | 80742 | -1.66 | 4.53E-03 |
| TMP21 | 239851_at | AW629289 | NM_006827 | 10972 | -1.66 | 5.43E-03 |
| DDHD1 | 231165_at | BE857355 | NM_030637 | 80821 | -1.65 | 5.85E-07 |
| BRD4 | 232551_at | AA521443 | NM_014299 | 23476 | -1.65 | 1.19E-03 |
| | | | NM_058243 | | | |
| LRRC5 | 234148_at | AK025238 | NM_018103 | 55144 | -1.65 | 6.30E-04 |
| BE674309 | 230913_at | BE674309 | --- | --- | -1.64 | 9.40E-04 |
| AI052447 | 236796_at | A1052447 | --- | --- | -1.64 | 1.69E-05 |
| BF513468 | 241505_at | BF513468 | --- | --- | -1.63 | 7.53E-03 |
| KIAA0754 | 215268_at | AW663712 | --- | 23056 | -1.62 | 1.40E-05 |
| FLJ10378 | 242153_at | AW264125 | NM_018078 | 55132 | -1.62 | 6.22E-03 |
| | | | NM_032239 | | | |
| | | | NM_178043 | | | |
| AGPAT3 | 223182_s_at | AI337300 | NM_020132 | 56894 | -1.62 | 7.22E-06 |
| C9orf150 | 216769_x_at | AK025180 | NM_203403 | 286343 | -1.62 | 6.30E-03 |
| LOC286411 | 1557133_at | AV753446 | --- | 286411 | -1.62 | 1.50E-03 |
| HNRPD | 213359_at | W74620 | NM_00100381 0 | 3184 | -1.61 | 9.97E-03 |
| | | | NM_002138 | | | |
| | | | NM_031369 | | | |
| | | | NM_031370 | | | |
| CKMT1 | 1561155_at | W76548 | NM_020990 | 1159 | -1.61 | 1.10E-03 |
| BF433341 | 235977_at | BF433341 | --- | --- | -1.61 | 3.15E-03 |
| PRKACB | 202742_s_at | NM_002731 | NM_002731 | 5567 | -1.61 | 2.42E-03 |
| | | | NM_182948 | | | |
| | | | NM_207578 | | | |
| FLJ45880 | 228538_at | BE671164 | NM_207404 | 389114 | -1.59 | 3.25E-03 |
| CRYGS | 223643_at | AI762135 | NM_017541 | 1427 | -1.58 | 2.85E-05 |
| BACH2 | 221234_s_at | NM_021813 | NM_021813 | 60468 | -1.58 | 2.32E-05 |
| AW150212 | 1556038_at | AW150212 | --- | --- | -1.58 | 7.16E-03 |
| CDH12 | 207149_at | L33477 | NM_004061 | 1010 | -1.58 | 7.69E-03 |
| AB062488 | 1555014_x_at | AB062488 | --- | --- | -1.58 | 1.71E-03 |
| RHOU | 223168_at | AL096776 | NM_021205 | 58480 | -1.57 | 2.07E-07 |
| C21orf66 | 239407_at | AI793248 | NM_013329 | 94104 | -1.57 | 4.80E-03 |
| | | | NM_016631 | | | |
| | | | NM_058191 | | | |
| | | | NM_145328 | | | |
| ENOSF1 | 204143_s_at | NM_017512 | NM_017512 | 55556 | -1.56 | 4.33E-04 |
| BE894882 | 235681_at | BE894882 | --- | --- | -1.55 | 4.33E-05 |
| AW606588 | 239008_at | AW606588 | --- | --- | -1.54 | 5.81E-03 |
| SYTL1 | 227134_at | AI341537 | NM_032872 | 84958 | -1.54 | 6.41E-03 |
| AK025360 | 216173_at | AK025360 | --- | --- | -1.54 | 2.23E-04 |
| DKFZP564D166 | 231799_at | AK021886 | XM_371074 | 26115 | -1.54 | 7.37E-03 |
| LGALS3BP | 200923_at | NM_005567 | NM_005567 | 3959 | -1.54 | 5.18E-09 |
| AI768374 | 228661_s_at | AI768374 | --- | --- | -1.54 | 1.48E-03 |
| AA639707 | 243837_x_at | AA639707 | --- | --- | -1.54 | 3.39E-03 |
| AI821995 | 240201_at | AI821995 | --- | --- | -1.54 | 1.51E-04 |
| PHGDHL1 | 241932_at | AI073803 | NM_177967 | 337867 | -1.53 | 2.06E-03 |
| LOC51315 | 218303_x_at | NM_016618 | NM_016618 | 51315 | -1.53 | 2.32E-05 |
| C10orf10 | 209183_s_at | AL136653 | NM_007021 | 11067 | -1.52 | 3.47E-03 |
| AI817960 | 239649_at | A1817960 | --- | --- | -1.52 | 9.05E-05 |
| CTBP2 | 215377_at | AK024129 | NM_001329 | 1488 | -1.52 | 3.37E-04 |
| | | | NM_022802 | | | |
| AI828075 | 230795_at | AI828075 | --- | --- | -1.51 | 3.70E-05 |
| ARHGAP20 | 228368_at | AI936560 | NM_020809 | 57569 | -1.51 | 1.86E-03 |
| BC020585 | 1568817_at | BC020585 | --- | --- | -1.51 | 6.55E-04 |
| NDRG1 | 200632_s_at | NM_006096 | NM_006096 | 10397 | -1.51 | 1.79E-04 |
| SLICK | 234103_at | AU145191 | NM_198503 | 343450 | -1.51 | 8.70E-03 |
| PROM1 | 204304_s_at | NM_006017 | NM_006017 | 8842 | -1.51 | 1.53E-04 |
| THADA | 233692_at | AK024928 | NM_022065 | 63892 | -1.51 | 8.55E-05 |
| | | | NM_198554 | | | |
| FBXL13 | 243057_at | AI923673 | NM_145032 | 222235 | -1.50 | 2.27E-03 |
| LOC54103 | 222150_s_at | AK026747 | XM_379881 | 54103 | -1.50 | 3.51E-04 |
| | | | XM_499340 | | | |
| BE552011 | 230208_at | BE552011 | --- | --- | -1.50 | 4.21E-03 |
| MGC45477 | 1554250_s_at | BC033812 | --- | 387277 | -1.50 | 3.37E-04 |
| AI521273 | 241702_at | AI521273 | --- | --- | -1.49 | 2.58E-03 |
| AL833239 | 1560082_at | AL833239 | --- | --- | -1.49 | 2.21E-03 |
| BACE1 | 217904_s_at | NM_012104 | NM_012104 | 23621 | -1.48 | 3.27E-05 |
| | | | NM_138971 | | | |
| | | | NM_138972 | | | |
| | | | NM_138973 | | | |
| PRSS11 | 201185_at | NM_002775 | NM_002775 | 5654 | -1.48 | 7.56E-04 |
| PTPRH | 208300_at | NM_002842 | NM_002842 | 5794 | -1.48 | 2.27E-03 |
| C9orf150 | 227443_at | AI972386 | NM_203403 | 286343 | -1.48 | 1.85E-03 |
| FLJ10159 | 236602_at | AA833813 | --- | 55084 | -1.48 | 1.23E-06 |
| GLT25D2 | 209883_at | AF288389 | NM_015101 | 23127 | -1.47 | 9.47E-04 |
| LOC133308 | 229491_at | BF433180 | NM_178833 | 133308 | -1.47 | 2.89E-03 |
| KIAA1276 | 233823_at | AB033102 | XM_039169 | 27146 | -1.46 | 3.60E-04 |
| GNRHR | 1568661_at | BC039430 | NM_000406 | 2798 | -1.46 | 8.63E-03 |
| RAB6B | 221792_at | AW118072 | NM_016577 | 51560 | -1.46 | 2.71E-03 |
| FLJ12666 | 215029_at | AL117451 | NM_024595 | 79647 | -1.45 | 1.46E-04 |
| LOC23117 | 238342_at | BF677084 | XM_290670 | 23117 | -1.45 | 1.20E-05 |
| BG430958 | 1559455_at | BG430958 | --- | --- | -1.44 | 7.89E-04 |
| C6orf148 | 237347_at | AL039379 | NM_030568 | 80759 | -1.44 | 3.82E-04 |
| MAST4 | 225613_at | AI096389 | XM_291141 | 23227 | -1.44 | 4.89E-03 |
| AL832439 | 1560661_x_at | AL832439 | --- | --- | -1.44 | 3.31E-04 |
| AA824624 | 239531_at | AA824624 | --- | --- | -1.44 | 1.73E-05 |
| AU146893 | 229858_at | AU146893 | --- | --- | -1.44 | 1.74E-03 |
| NDFIP2 | 1568807_a_at | AI301081 | NM_019080 | 54602 | -1.43 | 4.05E-06 |
| SFRS1 | 239384_at | R18746 | NM_006924 | 6426 | -1.43 | 8.25E-03 |
| STMN2 | 203001_s_at | NM_007029 | NM_007029 | 11075 | -1.43 | 6.36E-03 |
| PTP4A3 | 209695_at | BC003105 | NM_007079 | 11156 | -1.43 | 3.11E-05 |
| | | | NM_032611 | | | |
| BE671117 | 243114_at | BE671117 | --- | --- | -1.43 | 3.37E-04 |
| AK021551 | 232589_at | AK021551 | --- | --- | -1.43 | 2.58E-03 |
| SRP54 | 1557505_a_at | AI076351 | NM_003136 | 6729 | -1.42 | 1.37E-03 |
| KIAA1641 | 220940_at | NM_025190 | NM_020970 | 57730 | -1.42 | 1.31E-04 |
| AW591282 | 231374_at | AW591282 | --- | --- | -1.42 | 5.06E-03 |
| MCM3AP | 215581_s_at | AK022303 | NM_003906 | 8888 | -1.42 | 7.91E-04 |
| TPD52 | 239862_at | W89022 | NM_005079 | 7163 | -1.42 | 1.59E-04 |
| MGC15523 | 1563226_at | BC020925 | NM_138570 | 124565 | -1.42 | 3.66E-04 |
| CEBPD | 203973_s_at | NM_005195 | NM_005195 | 1052 | -1.42 | 2.21E-03 |
| LOC133308 | 1564746_at | BC009732 | NM_178833 | 133308 | -1.41 | 9.96E-05 |
| AI041215 | 242912_at | AI041215 | --- | --- | -1.41 | 1.41E-03 |
| BTN3A3 | 204820_s_at | NM_006994 | NM_006994 | 10384 | -1.41 | 3.26E-03 |
| BTN3A2 | | | NM_007047 | 11118 | | |
| | | | NM_197974 | | | |
| HIAT1 | 242374_at | AA747563 | NM_033055 | 64645 | -1.41 | 1.51E-03 |
| KIAA0500 | 213839_at | AW028110 | --- | 57237 | -1.40 | 2.08E-04 |
| C9orf41 | 1564690_at | BC022435 | NM_152420 | 138199 | -1.40 | 2.81E-04 |
| AL832439 | 1560659_at | AL832439 | --- | --- | -1.40 | 4.57E-05 |
| ABR | 214671_s_at | L19704 | NM_001092 | 29 | -1.40 | 1.63E-04 |
| | | | NM_021962 | | | |
| BE219187 | 237594_at | BE219187 | --- | --- | -1.40 | 1.72E-03 |
| HLCS | 207833_s_at | NM_000411 | NM_000411 | 3141 | -1.39 | 7.33E-03 |
| NFYC | 1558782_a_at | BG390627 | NM_014223 | 4802 | -1.39 | 2.59E-03 |
| SNF1LK | 208078_s_at | NM_030751 | NM_173354 | 150094 | -1.39 | 3.83E-03 |
| C9orf150 | 233112_at | AW301393 | NM_203403 | 286343 | -1.39 | 1.07E-03 |
| SCFD1 | 243525_at | AA808051 | NM_016106 | 23256 | -1.39 | 5.74E-04 |
| | | | NM_182835 | | | |
| AA936428 | 244674_at | AA936428 | --- | --- | -1.39 | 1.59E-03 |
| DKFZp761N111 | 229254_at | BE550027 | NM_181644 | 148808 | -1.39 | 3.32E-06 |
| AI691075 | 229728_at | AI691075 | --- | --- | -1.38 | 1.16E-04 |
| FLJ11710 | 244646_at | AW972881 | --- | 79904 | -1.38 | 7.97E-03 |
| AGPAT3 | 223183_at | AI928403 | NM_20132 | 56894 | -1.38 | 6.88E-04 |
| BC037911 | 1568981_at | BC037911 | --- | --- | -1.38 | 1.58E-05 |
| RHOU | 223169_s_at | AB051826 | NM_021205 | 58480 | -1.38 | 6.23E-03 |
| AW172407 | 240908_at | AW172407 | XM_373053 | 391733 | -1.38 | 1.17E-04 |
| HIST1H2BK | 209806_at | BC000893 | | 85236 | -1.38 | 1.12E-04 |
| | | | NM_080593 | | | |
| BE046461 | 215303_at | BE046461 | --- | --- | -1.38 | 7.47E-04 |
| AA973100 | 230725_at | AA973100 | --- | --- | -1.38 | 8.35E-07 |
| SRP54 | 1557504_at | A1076351 | NM_003136 | 6729 | -1.38 | 5.06E-03 |
| FLJ13197 | 219871_at | NM_024614 | NM_024614 | 79667 | -1.37 | 8.97E-03 |
| OR7D2 | 1562337_at | AK095468 | NM_175883 | 162998 | -1.37 | 2.08E-04 |
| TRIM32 | 1557315_a_at | AL134420 | NM_012210 | 22954 | -1.37 | 2.53E-03 |
| AI394334 | 232696_at | AI394334 | --- | --- | -1.37 | 2.28E-03 |
| BIRC6 | 242068_at | AA608834 | NM_016252 | 57448 | -1.37 | 7.28E-03 |
| ETFA | 239830_at | AW850555 | NM_000126 | 2108 | -1.37 | 4.23E-04 |
| PDGFRB | 202273_at | NM_002609 | NM_002609 | 5159 | -1.37 | 1.64E-04 |
| DDEF1 | 232952_at | AU146493 | NM_018482 | 50807 | -1.37 | 3.89E-03 |
| AI912571 | 228333_at | AI912571 | --- | --- | -1.36 | 9.03E-04 |
| AW150212 | 1556039_s_at | AW150212 | --- | --- | -1.36 | 2.10E-04 |
| SLC16A14 | 238029_s_at | R15072 | NM_152527 | 151473 | -1.36 | 3.15E-04 |
| COL6A1 | 212091_s_at | AI141603 | NM_001848 | 1291 | -1.35 | 4.35E-04 |
| TMEM1 | 215269_at | AI922538 | NM_00100172 3 | 7109 | -1.35 | 7.34E-04 |
| | | | NM_003274 | | | |
| AA262583 | 238534_at | AA262583 | --- | --- | -1.35 | 3.27E-03 |
| AL037414 | 230055_at | AL037414 | --- | --- | -1.35 | 3.69E-04 |
| KIF1A | 203849_s_at | BG473130 | NM_004321 | 547 | -1.35 | 3.10E-03 |
| KIAA1904 | 1559072_a_at | BC032083 | NM_052906 | 114794 | -1.34 | 4.03E-03 |
| ACSL4 | 1557418_at | W95007 | NM_004458 | 2182 | -1.34 | 4.22E-04 |
| | | | NM_022977 | | | |
| ATP1B4 | 243737_at | AI082610 | NM_012069 | 23439 | -1.34 | 4.77E-03 |
| MAP2K5 | 244298_at | AA702999 | NM_002757 | 5607 | -1.34 | 3.13E-05 |
| | | | NM_145160 | | | |
| | | | NM_145161 | | | |
| | | | NM_145162 | | | |
| GAL | 207466_at | NM_015973 | NM_015973 | 51083 | -1.33 | 8.78E-03 |
| DOC2A | 205744_at | NM_003586 | NM_003586 | 8448 | -1.33 | 3.98E-07 |
| AV703843 | 1560775_at | AV703843 | --- | --- | -1.33 | 4.77E-03 |
| PCTK3 | 214797_s_at | BC000281 | NM_002596 | 5129 | -1.33 | 9.10E-04 |
| | | | NM_212502 | | | |
| | | | NM_212503 | | | |
| DKFZp761O011 | 207797_s_at | NM_018409 | NM_018409 | 55805 | -1.33 | 6.16E-03 |
| RAC2 | 207419_s_at | NM_002872 | NM_002872 | 5880 | -1.33 | 4.07E-03 |
| STMN2 | 203000_at | BF967657 | NM_007029 | 11075 | -1.32 | 8.36E-03 |
| D4S234E | 209569_x_at | NM_014392 | NM_014392 | 27065 | -1.32 | 1.21E-06 |
| AV709727 | 225996_at | AV709727 | --- | --- | -1.32 | 2.28E-03 |
| C19orf4 | 219005_at | NM_012109 | NM_012109 | 25789 | -1.32 | 6.30E-06 |
| AI276956 | 230766_at | AI276956 | --- | --- | -1.32 | 5.11E-04 |
| C13orf22 | 1562238_at | AL832776 | NM_005800 | 10208 | -1.32 | 1.46E-03 |
| CRYZL1 | 243091_at | BF435599 | NM_005111 | 9946 | -1.32 | 7.89E-03 |
| | | | NM_145311 | | | |
| | | | NM_145858 | | | |
| HR | 241355_at | BF528433 | NM_005144 | 55806 | -1.32 | 3.52E-03 |
| | | | NM_018411 | | | |
| LOC285513 | 1564077_at | AK074440 | NM_198281 | 285513 | -1.32 | 8.81E-03 |
| AI973085 | 239765_at | AI973085 | --- | --- | -1.32 | 9.72E-03 |
| DJ462O23.2 | 225876_at | T84558 | NM_020448 | 57185 | -1.31 | 3.81E-03 |
| PKD1 | 216949_s_at | L39891 | NM_000296 | 5310 | -1.31 | 4.56E-03 |
| | | | NM_00100994 4 | | | |
| MAD | 226275_at | AI188653 | NM_002357 | 4084 | -1.31 | 3.40E-03 |
| MSI2 | 243579_at | BF029215 | NM_138962 | 124540 | -1.31 | 4.03E-03 |
| | | | NM_170721 | | | |
| UGCGL2 | 1558466_at | BE156619 | NM_020121 | 55757 | -1.31 | 7.77E-04 |
| DDEF1 | 238193_at | W87434 | NM_018482 | 50807 | -1.31 | 6.32E-04 |
| KIAA1277 | 221886_at | AL037701 | NM_015689 | 27147 | -1.31 | 3.41E-07 |
| AI056683 | 228171_s_at | AI056683 | --- | --- | -1.31 | 8.22E-04 |
| FYN | 243006_at | BG222258 | NM_002037 | 2534 | -1.31 | 1.31E-04 |
| | | | NM_153047 | | | |
| | | | NM_153048 | | | |
| GART | 236969_at | AI961651 | NM_000819 | 2618 | -1.30 | 9.66E-06 |
| | | | NM_175085 | | | |
| FLJ32130 | 242272_at | AI375066 | NM_152458 | 146540 | -1.30 | 2.75E-04 |
| C6orf198 | 227834_at | AL589605 | XM_371849 | 167838 | -1.30 | 9.19E-03 |
| DTNA | 210736_x_at | U46746 | NM_001390 | 1837 | -1.30 | 5.47E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| SERP1 | 235475_at | AI580135 | NM_014445 | 27230 | -1.30 | 1.39E-03 |
| C21orf25 | 212875_s_at | AP001745 | NM_199050 | 25966 | -1.30 | 5.16E-05 |
| MGC12488 | 211386_at | BC005372 | --- | 84786 | -1.30 | 2.63E-03 |
| BM989131 | 1558075_at | BM989131 | --- | --- | -1.30 | 1.86E-04 |
| ENOSF1 | 204142_at | NM_017512 | NM_017512 | 55556 | -1.30 | 6.73E-04 |
| AW014345 | 236229_at | AW014345 | --- | --- | -1.29 | 1.23E-05 |
| PKD1 | 202328_s_at | NM_000296 | NM_000296 | 5310 | -1.29 | 5.93E-03 |
| | | | NM_00100994 4 | | | |
| KIAA1276 | 227505_at | N64630 | XM_039169 | 27146 | -1.29 | 9.12E-04 |
| AL390170 | 213904_at | AL390170 | --- | --- | -1.29 | 4.93E-04 |
| BE672499 | 230962_at | BE672499 | --- | --- | -1.28 | 1.31E-05 |
| FLJ20753 | 229918_at | AB046860 | XM_371082 | 55036 | -1.28 | 1.20E-05 |
| SIGIRR | 52940_at | AA085764 | NM_021805 | 59307 | -1.28 | 3.70E-04 |
| S100A6 | 217728_at | NM_014624 | NM_014624 | 6277 | -1.28 | 5.27E-04 |
| UCN | 206072_at | NM_003353 | NM_003353 | 7349 | -1.28 | 1.49E-03 |
| INSL3 | 214572_s_at | NM_005543 | NM_005543 | 3640 | -1.28 | 3.79E-03 |
| TBX3 | 243234_at | BG055137 | NM_005996 | 6926 | -1.28 | 1.16E-04 |
| | | | NM_016569 | | | |
| LOC349136 | 242452_at | AI683911 | NM_198285 | 349136 | -1.28 | 4.80E-04 |
| LOC440457 | 206088_at | NM_014834 | XM_496239 | 440457 | -1.28 | 1.40E-03 |
| LONP | 221833_at | AI971258 | NM_031490 | 83752 | -1.27 | 6.14E-03 |
| LOC158301 | 239193_at | BF060981 | --- | 158301 | -1.27 | 2.55E-04 |
| AW449624 | 239379_at | AW449624 | --- | --- | -1.27 | 2.70E-03 |
| KCTD13 | 45653_at | AW026481 | NM_178863 | 253980 | -1.27 | 2.70E-06 |
| ST7L | 216123_x_at | AK024158 | NM_017744 | 54879 | -1.27 | 1.64E-04 |
| | | | NM_138727 | | | |
| | | | NM_138728 | | | |
| | | | NM_138729 | | | |
| | | | NM_198327 | | | |
| | | | NM_198328 | | | |
| AA835417 | 243007 _at | AA835417 | --- | --- | -1.27 | 1.46E-03 |
| FLJ40342 | 1558854_a_at | BG616498 | NM_152347 | 124989 | -1.27 | 5.60E-05 |
| UBE2D3 | 233303_at | AL110175 | NM_003340 | 7323 | -1.26 | 6.81E-05 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| ZNF365 | 206448_at | NM_014951 | NM_199450 | 22891 | -1.26 | 1.40E-03 |
| | | | NM_199451 | | | |
| | | | NM_199452 | | | |
| CDKN1C | 213182_x_at | R78668 | NM_000076 | 1028 | -1.26 | 1.79E-03 |
| FLJ32130 | 1554769_at | BC040642 | NM_152458 | 146540 | -1.26 | 3.10E-04 |
| ICAM2 | 213620_s_at | AA126728 | NM_000873 | 3384 | -1.26 | 5.57E-03 |
| KIAA0256 | 216278_at | AL109705 | NM_014701 | 9728 | -1.26 | 1.61E-03 |
| BF432875 | 235782_at | BF432875 | XM _497668 | 388620 | -1.26 | 4.13E-04 |
| PRKACB | 202741_at | AA130247 | NM_002731 | 5567 | -1.25 | 3.74E-04 |
| | | | NM_182948 | | | |
| | | | NM_207578 | | | |
| BG548427 | 235547 _at | BG548427 | --- | --- | -1.25 | 2.88E-03 |
| MCF2L | 35147 _at | AB002360 | NM_024979 | 23263 | -1.25 | 1.29E-04 |
| AGPAT3 | 223184_s_at | BC004219 | NM_020132 | 56894 | -1.25 | 7.98E-04 |
| HSD17B1 | 205829_at | NM_000413 | NM_000413 | 3292 | -1.25 | 2.09E-03 |
| AF086013 | 1556543_at | AF086013 | --- | --- | -1.25 | 7.87E-04 |
| FAH | 202862_at | NM_000137 | NM_000137 | 2184 | -1.25 | 3.32E-03 |
| FLJ23754 | 1553248_at | NM_152675 | NM_152675 | 201252 | -1.25 | 8.02E-03 |
| RAB6B | 225259_at | AI805050 | NM_016577 | 51560 | -1.25 | 8.67E-05 |
| ZNF148 | 243559_at | BF515306 | NM_021964 | 7707 | -1.25 | 2.25E-03 |
| HIAT1 | 230868_at | BF433103 | NM_033055 | 64645 | -1.24 | 2.21E-03 |
| GAD2 | 243265_at | AW444497 | NM_000818 | 2572 | -1.24 | 4.66E-03 |
| KCTD13 | 221889_at | AW026481 | NM_178863 | 253980 | -1.24 | 1.81E-04 |
| PAN3 | 239597_at | AA993566 | NM_175854 | 255967 | -1.24 | 8.71E-03 |
| RPIP8 | 206196_s_at | NM_006695 | NM_006695 | 10900 | -1.24 | 4.99E-03 |
| FBXO22 | 215854_at | AU146050 | NM_012170 | 26263 | -1.24 | 8.97E-04 |
| | | | NM_147188 | | | |
| AI733281 | 239994_at | AI733281 | --- | --- | -1.24 | 8.71E-03 |
| COL6A1 | 213428_s_at | AA292373 | NM_001848 | 1291 | -1.23 | 1.41E-05 |
| PHC3 | 215521_at | AK023029 | NM_024947 | 80012 | -1.23 | 2.90E-05 |
| BF431902 | 228469_at | BF431902 | --- | --- | -1.23 | 3.29E-03 |
| SLC7A1 | 215401_at | AU147698 | NM_003045 | 6541 | -1.23 | 4.72E-05 |
| ABCC5 | 226363_at | BE550362 | NM_005688 | 10057 | -1.23 | 1.08E-04 |
| WDR9 | 225446_at | AI638279 | NM_00100724 6 | 54014 | -1.22 | 4.64E-03 |
| | | | NM_018963 | | | |
| | | | NM_033656 | | | |
| C21orf18 | 213989_x_at | AB004853 | NM_00100725 8 | 54093 | -1.22 | 4.28E-04 |
| | | | NM_00100725 9 | | | |
| | | | NM_00100726 0 | | | |
| | | | NM_00100726 1 | | | |
| | | | NM_00100726 2 | | | |
| | | | NM_017438 | | | |
| FLJ11267 | 220216_at | NM_019607 | NM_019607 | 56260 | -1.22 | 2.57E-04 |
| BE217890 | 240046_at | BE217890 | --- | --- | -1.22 | 1.64E-03 |
| UBE2D3 | 242403_at | AI459177 | NM_003340 | 7323 | -1.22 | 7.24E-05 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| AI697662 | 231465_at | AI697662 | --- | --- | -1.22 | 6.05E-06 |
| WDR9 | 1553227_s_at | NM_018963 | NM_00100724 6 | 54014 | -1.22 | 9.76E-0.3 |
| | | | NM_018963 | | | |
| | | | NM_033656 | | | |
| AI985021 | 238918_at | AI985021 | --- | --- | -1.22 | 3.05E-03 |
| LOC387904 | 243760_at | AI739231 | XM_370711 | 387904 | -1.21 | 8.97E-04 |
| GFRA3 | 214479_at | NM_001496 | NM_001496 | 2676 | -1.21 | 3.99E-03 |
| BF196060 | 236521_at | BF196060 | --- | --- | -1.21 | 8.75E-04 |
| CDKN1C | 219534_x_at | NM_000076 | NM_000076 | 1028 | -1.21 | 3.80E-04 |
| FLJ10613 | 46947_at | T87245 | NM_019067 | 54552 | -1.21 | 6.49E-03 |
| BG288330 | 235028_at | BG288330 | --- | --- | -1.20 | 3.05E-03 |
| PPP3CA | 236545_at | AA532718 | NM_000944 | 5530 | -1.20 | 2.51E-03 |
| EXT1 | 1564378_a_at | AK025101 | NM_000127 | 2131 | -1.20 | 9.41E-04 |
| AI862542 | 235696_at | AI862542 | --- | --- | -1.20 | 3.39E-04 |
| D4S234E | 213533_at | M98528 | NM_014392 | 27065 | -1.20 | 2.09E-05 |
| AL353951 | 232601_at | AL353951 | XM_498488 | 439987 | -1.20 | 9.24E-03 |
| OKFZp434O052 | 1557879_at | BF110506 | NM_194302 | 255101 | -1.20 | 1.41E-03 |
| MCTP1 | 220122_at | NM_024717 | NM_00100279 6 | 79772 | -1.20 | 2.83E-05 |
| | | | NM_024717 | | | |
| VN1R1 | 221412_at | NM_020633 | NM_020633 | 57191 | -1.20 | 1.29E-03 |
| H29132 | 241658_at | H29132 | --- | --- | -1.20 | 2.35E-03 |
| MGC13057 | 232773_at | AI932618 | NM_032321 | 84281 | -1.20 | 2.72E-03 |
| LOC51619 | 244046_at | BF939235 | NM_015983 | 51619 | -1.20 | 5.06E-03 |
| HNRPR | 232004_at | AK001846 | NM_005826 | 10236 | -1.20 | 5.41E-04 |
| U80764 | 222128_at | U80764 | --- | --- | -1.20 | 1.17E-04 |
| RAB6IP2 | 1557738_at | BC041344 | NM_015064 | 23085 | -1.19 | 2.25E-03 |
| | | | NM_178037 | | | |
| | | | NM_178038 | | | |
| | | | NM_178039 | | | |
| | | | NM_178040 | | | |
| LOC255326 | 239151_at | BG427809 | XM_497224 | 255326 | -1.19 | 4.72E-04 |
| CTBP2 | 233315_at | AK024947 | NM_001329 | 1488 | -1.19 | 1.31E-03 |
| | | | NM_022802 | | | |
| MCF2L | 212935_at | AB002360 | NM_024979 | 23263 | -1.19 | 4.90E-05 |
| BF514509 | 239049_at | BF514509 | --- | --- | -1.19 | 6.48E-03 |
| AA678564 | 238620_at | AA678564 | --- | --- | -1.19 | 9.54E-03 |
| AW364675 | 239066_at | AW364675 | --- | --- | -1.19 | 6.72E-04 |
| BE856960 | 242712_x_at | BE856960 | --- | --- | -1.18 | 8.35E-03 |
| TYMS | 217690_at | BG281679 | NM_001071 | 7298 | -1.18 | 3.35E-03 |
| AL530703 | 231882_at | AL530703 | XM_498570 | 440156 | -1.18 | 1.80E-06 |
| SH3BGR | 204979_s_at | NM_007341 | NM_00100171 3 | 6450 | -1.18 | 6.25E-05 |
| | | | NM_007341 | | | |
| KIF5C | 1557089_at | CA449408 | XM_377774 | 3800 | -1.18 | 8.52E-03 |
| AP1GBP1 | 243667_at | AW977986 | NM_007247 | 11276 | -1.18 | 2.85E-04 |
| | | | NM_080550 | | | |
| DCAMKL1 | 205399_at | NM_004734 | NM_004734 | 9201 | -1.18 | 8.71E-03 |
| JAG2 | 32137_at | Y14330 | NM_002226 | 3714 | -1.18 | 9.67E-03 |
| | | | NM_145159 | | | |
| TNFRSF25 | 219423_x_at | NM_003790 | NM_003790 | 8718 | -1.18 | 9.97E-05 |
| | | | NM_148965 | | | |
| | | | NM_148966 | | | |
| | | | NM_148967 | | | |
| | | | NM_148968 | | | |
| | | | NM_148969 | | | |
| | | | NM_148970 | | | |
| | | | NM_148971 | | | |
| | | | NM_148972 | | | |
| | | | NM_148973 | | | |
| | | | NM_148974 | | | |
| R99939 | 239245_at | R99939 | XM_498488 | 439987 | -1.18 | 2.96E-03 |
| KLF9 | 203542_s_at | AI690205 | NM_001206 | 687 | -1.18 | 9.74E-05 |
| ZC3HDC1 | 218543_s_at | NM_022750 | NM_022750 | 64761 | -1.17 | 7.23E-04 |
| IDS | 1559136_s_at | AU140213 | NM_000202 | 3423 | -1.17 | 9.45E-03 |
| | | | NM_006123 | | | |
| CDKN1C | 213348_at | N33167 | NM_000076 | 1028 | -1.17 | 3.74E-04 |
| MLLT10 | 238257_at | N64035 | NM_00100956 9 | 8028 | -1.17 | 8.79E-03 |
| | | | NM_004641 | | | |
| LRRN6A | 227933_at | AI193252 | NM_032808 | 84894 | -1.17 | 5.79E-05 |
| IL10RB | 209575_at | BC001903 | NM_000628 | 3588 | -1.17 | 2.48E-04 |
| KIF1A | 225482_at | AL533416 | NM_004321 | 547 | -1.17 | 1.50E-03 |
| FAM46A | 221766_s_at | AW246673 | NM_017633 | 55603 | -1.17 | 3.00E-04 |
| D4S234E | 209570_s_at | BC001745 | NM_014392 | 27065 | -1.17 | 3.84E-06 |
| PDCD2 | 228420_at | AW590850 | NM_002598 | 5134 | -1.17 | 2.71E-03 |
| | | | NM_144781 | | | |
| DKFZp313N062 | 1555906_s_at | AI147556 | NM_173826 | 285343 | -1.17 | 1.60E-05 |
| TIMP1 | 201666_at | NM_003254 | NM_003254 | 7076 | -1.17 | 1.45E-03 |
| LOC401074 | 1559826_a_at | BC039495 | XM_376247 | 401074 | -1.16 | 6.12E-03 |
| AW300500 | 228907_at | AW300500 | --- | --- | -1.16 | 9.31E-03 |
| BACH2 | 227173_s_at | AW450901 | NM_021813 | 60468 | -1.16 | 1.95E-04 |
| FLJ11800 | 220575_at | NM_024974 | NM_024974 | 80039 | -1.15 | 8.14E-05 |
| AU146999 | 215986_at | AU146999 | --- | --- | -1.15 | 4.17E-05 |
| FLJ10404 | 243047_at | AV698751 | NM_019057 | 54540 | -1.15 | 3.32E-06 |
| AI744451 | 81811_at | AI744451 | XM_373053 | 391733 | -1.15 | 6.74E-04 |
| UBE2D3 | 240383_at | AI239832 | NM_003340 | 7323 | -1.15 | 1.23E-03 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| YEATS2 | 236462_at | AA742310 | NM_018023 | 55689 | -1.15 | 9.25E-03 |
| LTBP3 | 219922_s_at | NM_021070 | NM_021070 | 4054 | -1.15 | 3.83E-05 |
| IREB2 | 1563130_a_at | AL109710 | NM_004136 | 3658 | -1.15 | 6.76E-04 |
| CRYGS | 223644_s_at | AF161703 | NM_017541 | 1427 | -1.15 | 2.33E-03 |
| BE326951 | 243562_at | BE326951 | --- | --- | -1.15 | 3.70E-03 |
| KIAA0217 | 241965_at | BF589232 | NM_015155 | 23185 | -1.15 | 6.15E-04 |
| AK024175 | 222152_at | AK024175 | XM_373053 | 391733 | -1.15 | 6.88E-04 |
| DNAJB5 | 207453_s_at | NM_012266 | NM_012266 | 25822 | -1.14 | 8.99E-05 |
| AI798680 | 227943_at | AI798680 | --- | --- | -1.14 | 1.60E-03 |
| VPS13C | 235023_at | AA828371 | NM_017684 | 54832 | -1.14 | 2.37E-03 |
| LAF4 | 232286_at | AA572675 | NM_002285 | 3899 | -1.14 | 5.79E-03 |
| AA021559 | 244815_at | AA021559 | --- | --- | -1.14 | 1.83E-03 |
| AI051572 | 243310_at | AI051572 | --- | --- | -1.14 | 4.12E-04 |
| PAK3 | 214078_at | AF070581 | NM_002578 | 5063 | -1.14 | 2.48E-05 |
| RNF19 | 230599_at | AI681558 | NM_015435 | 25897 | -1.14 | 5.16E-03 |
| | | | NM_183419 | | | |
| SH3GL2 | 205751_at | NM_003026 | NM_003026 | 6456 | -1.14 | 7.99E-04 |
| AA779679 | 227805_at | AA779679 | --- | --- | -1.13 | 3.80E-04 |
| BC009525 | 1568813_at | BC009525 | --- | --- | -1.13 | 2.50E-03 |
| AA682539 | 235575_at | AA682539 | --- | --- | -1.13 | 2.54E-03 |
| PSMB7 | 244801_at | AI248671 | NM_002799 | 5695 | -1.13 | 3.90E-06 |
| FARP1 | 239246_at | AW102941 | NM_00100171 5 | 10160 | -1.13 | 2.54E-03 |
| | | | NM_005766 | | | |
| SF3B14 | 1561923_a_at | AF147425 | NM_016047 | 51639 | -1.13 | 1.53E-03 |
| KATNB1 | 203163_at | NM_005886 | NM_005886 | 10300 | -1.13 | 6.37E-05 |
| FLJ10246 | 220458_at | NM_018038 | NM_018038 | 55104 | -1.13 | 1.74E-03 |
| BE738279 | 242546_at | BE738279 | XM_498570 | 440156 | -1.13 | 1.10E-07 |
| ABR | 212895_s_at | AL527773 | NM_001092 | 29 | -1.13 | 2.07E-03 |
| | | | NM_021962 | | | |
| AI631833 | 227396_at | AI631833 | --- | --- | -1.12 | 5.31E-03 |
| AI424872 | 81737_at | AI424872 | XM_497487 | 441749 | -1.12 | 1.92E-03 |
| AK027225 | 222145_at | AK027225 | XM_497487 | 441749 | -1.12 | 2.62E-04 |
| BIRC1 | 204860_s_at | AI817801 | NM_004536 | 4671 | -1.12 | 3.81E-03 |
| SLC12A7 | 218066_at | NM_006598 | NM_006598 | 10723 | -1.12 | 2.90E-03 |
| TRIO | 209010_s_at | AI797657 | NM_007118 | 7204 | -1.12 | 7.12E-05 |
| AV699781 | 239577_at | AV699781 | --- | --- | -1.12 | 1.61E-03 |
| AB037813 | 231969_at | AB037813 | --- | --- | -1.12 | 9.92E-03 |
| ABCC3 | 208161_s_at | NM_020037 | NM_003786 | 8714 | -1.12 | 5.49E-03 |
| | | | NM_020037 | | | |
| | | | NM_020038 | | | |
| ATP2B2 | 204685_s_at | R52647 | NM_00100133 1 | 491 | -1.12 | 3.74E-05 |
| | | | NM_001683 | | | |
| FGF7 | 230918_at | BE856598 | NM_002009 | 2252 | -1.12 | 7.41E-03 |
| MRAS | 225185_at | BF343625 | NM_012219 | 22808 | -1.12 | 4.78E-04 |
| LOC255326 | 231106_at | AI684591 | XM_497224 | 255326 | -1.11 | 2.25E-04 |
| BTN3A3 | 204821_at | NM_006994 | NM_006994 | 10384 | -1.11 | 3.77E-04 |
| | | | NM_197974 | | | |
| PPP2R5C | 1557718_at | AL834350 | NM_002719 | 5527 | -1.11 | 2.73E-03 |
| | | | NM_178586 | | | |
| | | | NM_178587 | | | |
| | | | NM_178588 | | | |
| BE467577 | 228251_at | BE467577 | --- | --- | -1.11 | 3.53E-03 |
| BM983749 | 1558686_at | BM983749 | --- | --- | -1.11 | 5.76E-04 |
| AK022031 | 1556713_at | AK022031 | --- | --- | -1.11 | 3.57E-03 |
| PPP3R1 | 1565811_at | BC033945 | NM_000945 | 5534 | -1.11 | 1.10E-03 |
| AI907083 | 222380_s_at | AI907083 | XM_373053 | 391733 | -1.11 | 5.00E-03 |
| DNAH5 | 243938_x_at | AI872645 | NM_001369 | 1767 | -1.11 | 1.58E-03 |
| PRPH | 213847_at | NM_006262 | NM_006262 | 5630 | -1.10 | 3.67E-03 |
| AI796536 | 229810_at | AI796536 | --- | --- | -1.10 | 1.23E-04 |
| R3HDM | 210714_at | BC001217 | NM_015361 | 23518 | -1.10 | 5.50E-03 |
| AA412065 | 242492_at | AA412065 | --- | --- | -1.10 | 1.37E-03 |
| SCN2A2 | 206381_at | NM_021007 | NM_021007 | 6326 | -1.10 | 5.85E-03 |
| AU 157716 | 227051_at | AU157716 | --- | --- | -1.10 | 3.37E-03 |
| LOC339047 | 214682_at | AK023376 | XM_290671 | 339047 | -1.10 | 9.05E-04 |
| CNOT4 | 1559347_at | BI599409 | NM_00100822 5 | 4850 | -1.10 | 5.45E-03 |
| | | | NM_013316 | | | |
| POFUT2 | 209578_s_at | BC000626 | NM_015227 | 23275 | -1.10 | 5.47E-04 |
| | | | NM_133634 | | | |
| | | | NM_133635 | | | |
| PTN | 209466_x_at | M57399 | NM_002825 | 5764 | -1.10 | 2.17E-04 |
| BF001773 | 241906_at | BF001773 | --- | --- | -1.10 | 3.71E-03 |
| TM4SF8 | 232632_at | AU145719 | NM_005724 | 10099 | -1.10 | 3.31E-04 |
| | | | NM_198902 | | | |
| CKLFSF8 | 234562_x_at | AK000115 | NM_178868 | 152189 | -1.10 | 5.38E-04 |
| PCSK7 | 232521_at | AK027156 | NM_00100152 2 | 6876 | -1.10 | 8.49E-03 |
| TAGLN | | | | 9159 | | |
| | | | NM_003186 | | | |
| | | | NM_004716 | | | |
| HNRPD | 236000_s_at | AA863112 | NM_00100381 0 | 3184 | -1.10 | 1.74E-03 |
| | | | NM_002138 | | | |
| | | | NM_031369 | | | |
| | | | NM_031370 | | | |
| FARP1 | 235501_at | AW961576 | NM_00100171 5 | 10160 | -1.10 | 2.99E-03 |
| | | | NM_005766 | | | |
| LOC255326 | 242393_x_at | AV708982 | XM_497224 | 255326 | -1.10 | 5.74E-03 |
| IBTK | 215086_at | AB037838 | NM_015525 | 25998 | -1.10 | 8.89E-03 |
| DTNA | 211493_x_at | U46744 | NM_001390 | 1837 | -1.10 | 3.03E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| DTNA | 227084_at | AW339310 | NM_001390 | 1837 | -1.10 | 3.15E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| LOC54103 | 213142_x_at | AV700415 | XM_379881 | 54103 | -1.10 | 8.79E-03 |
| | | | XM_499340 | | | |
| AA088430 | 242556_at | AA088430 | --- | --- | -1.10 | 2.33E-04 |
| RP42 | 242428_at | N58513 | NM_020640 | 54165 | -1.09 | 9.11E-03 |
| KIAA1731 | 1565830_at | AL833615 | XM_374922 | 85459 | -1.09 | 3.43E-03 |
| LOC284408 | 1558809_s_at | AK094324 | --- | 284408 | -1.09 | 6.98E-05 |
| LOC255326 | 233271_at | AU145563 | XM_497224 | 255326 | -1.09 | 6.71E-05 |
| AW290940 | 226773_at | AW290940 | --- | --- | -1.09 | 4.20E-05 |
| TAGLN2 | 200916_at | NM_003564 | NM_003564 | 8407 | -1.09 | 2.34E-03 |
| HIST1H2BD | 209911_x_at | BC002842 | | 3017 | -1.09 | 2.10E-03 |
| | | | NM_021063 | | | |
| | | | NM_138720 | | | |
| RSNL2 | 242710_at | AI791820 | NM_024692 | 79745 | -1.09 | 1.82E-03 |
| AL833123 | 1559496_at | AL833123 | --- | --- | -1.09 | 6.13E-04 |
| AF138859 | 233037_at | AF138859 | --- | --- | -1.09 | 4.93E-04 |
| DUSP16 | 1558740_s_at | R30807 | NM_030640 | 80824 | -1.09 | 5.64E-04 |
| NSUN6 | 242239_at | AW970888 | NM_182543 | 221078 | -1.09 | 1.80E-03 |
| SLC39A14 | 1561886_a_at | R00975 | NM_015359 | 23516 | -1.09 | 3.24E-03 |
| FMNL2 | 1556656_at | BF477401 | NM_00100441 7 | 114793 | -1.09 | 8.64E-03 |
| | | | NM_00100442 1 | | | |
| | | | NM_00100442 2 | | | |
| | | | NM_052905 | | | |
| MYO15B | 219173_at | NM_024957 | XR_000222 | 80022 | -1.09 | 7.64E-03 |
| MTSS1 | 232757_at | AV705679 | NM_014751 | 9788 | -1.09 | 4.23E-03 |
| BCL6B | 243825_at | T79768 | NM_181844 | 255877 | -1.09 | 2.13E-04 |
| AK023394 | 232107_at | AK023394 | --- | --- | -1.09 | 4.94E-04 |
| CDKN1C | 216894_x_at | D64137 | NM_000076 | 1028 | -1.09 | 8.38E-05 |
| PGAP1 | 220576_at | NM_024989 | NM_024989 | 80055 | -1.09 | 4.32E-03 |
| ATP8B2 | 226771_at | AB032963 | NM_00100585 5 | 57198 | -1.09 | 7.06E-06 |
| | | | NM_020452 | | | |
| PDE5A | 1556257_at | BC037908 | NM_001083 | 8654 | -1.08 | 5.89E-03 |
| | | | NM_033430 | | | |
| | | | NM_033431 | | | |
| | | | NM_033437 | | | |
| STK17A | 241410_at | AI949081 | NM_004760 | 9263 | -1.08 | 7.78E-03 |
| FBLP-1 | 1555480_a_at | AF459643 | NM_017556 | 54751 | -1.08 | 4.80E-04 |
| NDUFV3 | 226616_s_at | NM_021075 | NM_00100150 3 | 4731 | -1.08 | 7.94E-07 |
| | | | NM_021075 | | | |
| PHACTR1 | 1561642_at | AF085859 | NM_030948 | 221692 | -1.08 | 6.92E-03 |
| PAK3 | 236277_at | H23551 | NM_002578 | 5063 | -1.08 | 7.40E-05 |
| TP53BP1 | 1569098_s_at | BU508386 | NM_005657 | 7158 | -1.08 | 1.66E-05 |
| FNBP1 | 230389_at | BE046511 | NM_015033 | 23048 | -1.08 | 1.40E-03 |
| AK021973 | 234098_at | AK021973 | --- | --- | -1.08 | 9.97E-03 |
| N70015 | 236510_at | N70015 | --- | --- | -1.08 | 8.69E-04 |
| PPP2R2C | 223574_x_at | AF086924 | NM_020416 | 5522 | -1.08 | 2.02E-05 |
| | | | NM_181876 | | | |
| JMY | 226352_at | BF447037 | NM_152405 | 133746 | -1.08 | 4.14E-03 |
| MYT1L | 1554633_a_at | AF036943 | NM_015025 | 23040 | -1.08 | 2.26E-03 |
| AVO3 | 237330_at | AA603494 | XM_380173 | 253260 | -1.08 | 2.23E-03 |
| PTPN2 | 240843_at | N53696 | NM_002828 | 5771 | -1.07 | 1.44E-04 |
| | | | NM_080422 | | | |
| | | | NM_080423 | | | |
| LUC7L2 | 242024_at | T90999 | NM_016019 | 51631 | -1.07 | 8.65E-03 |
| AI631888 | 235532_at | AI631888 | --- | --- | -1.07 | 1.86E-08 |
| PDE9A | 205593_s_at | NM_002606 | NM_00100156 7 | 5152 | -1.07 | 1.16E-05 |
| | | | NM_00100156 8 | | | |
| | | | NM_00100156 9 | | | |
| | | | NM_00100157 0 | | | |
| | | | NM_00100157 1 | | | |
| | | | NM_00100157 2 | | | |
| | | | NM_00100157 3 | | | |
| | | | NM_00100157 4 | | | |
| | | | NM_00100157 5 | | | |
| | | | NM_00100157 6 | | | |
| | | | NM_00100157 7 | | | |
| | | | NM_00100157 8 | | | |
| | | | NM_00100157 9 | | | |
| | | | NM_00100158 0 | | | |
| | | | NM_00100158 1 | | | |
| | | | NM_00100158 2 | | | |
| LOC92154 | 1555894_s_at | AA829283 | NM_138383 | 92154 | -1.07 | 1.06E-03 |
| CUL4A | 232466_at | AU155661 | NM_00100889 5 | 8451 | -1.07 | 1.04E-04 |
| | | | NM_003589 | | | |
| FLJ21106 | 219980_at | NM_025097 | NM_025097 | 80167 | -1.07 | 3.33E-03 |
| GSPT1 | 240452_at | AA580082 | NM_002094 | 2935 | -1.07 | 1.19E-03 |
| PCAF | 203845_at | AV727449 | NM_003884 | 8850 | -1.07 | 9.18E-05 |
| TIGD4 | 232998_at | AK024235 | NM_145720 | 201798 | -1.07 | 1.44E-03 |
| PCNXL2 | 1554256_a_at | BC008300 | NM_014801 | 80003 | -1.06 | 3.23E-03 |
| | | | NM_024938 | | | |
| AI950451 | 240971_x_at | AI950451 | --- | --- | -1.06 | 6.35E-03 |
| LOC57228 | 209679_s_at | BC003379 | NM_020467 | 57228 | -1.06 | 5.22E-04 |
| ARFGEF1 | 236742_at | AA132172 | NM_006421 | 10565 | -1.06 | 8.57E-04 |
| FLJ14966 | 243611_at | BG231646 | NM_032867 | 84953 | -1.06 | 3.24E-03 |
| AK097571 | 1556261_a_at | AK097571 | --- | --- | -1.06 | 3.95E-03 |
| FLJ22729 | 238295_at | N22706 | NM_024683 | 79736 | -1.06 | 4.49E-05 |
| BICD1 | 242052_at | AW979272 | NM_00100339 8 | 636 | -1.06 | 1.94E-03 |
| | | | NM_001714 | | | |
| AA468422 | 239780_at | AA468422 | --- | --- | -1.06 | 5.66E-04 |
| BF476403 | 230218_at | BF476403 | --- | --- | -1.06 | 9.08E-04 |
| BIRC1 | 238446_at | AI970117 | NM_004536 | 441081 | -1.06 | 3.30E-03 |
| LOC441081 | | | XM_496754 | 4671 | | |
| T78074 | 236386_at | T78074 | XM_496203 | 440423 | -1.06 | 3.85E-03 |
| P15RS | 231502_at | BF591615 | NM_018170 | 55197 | -1.06 | 4.06E-03 |
| JMJD1C | 228793_at | BF002296 | NM_004241 | 221037 | -1.06 | 2.41E-05 |
| TRIM33 | 214815_at | AU136587 | NM_015906 | 51592 | -1.05 | 9.87E-04 |
| | | | NM_033020 | | | |
| WDR42A | 233637_at | AU146915 | NM_015726 | 50717 | -1.05 | 1.13E-03 |
| AA757630 | 236724_at | AA757630 | --- | --- | -1.05 | 7.93E-03 |
| NM_014152 | 220694_at | NM_014152 | --- | --- | -1.05 | 4.06E-04 |
| AW665538 | 244532_x_at | AW665538 | XM_496692 | 441014 | -1.05 | 2.86E-03 |
| MYST4 | 215597_x_at | AU143799 | NM_012330 | 23522 | -1.05 | 4.35E-04 |
| CAPZA1 | 238407_at | AI792880 | NM_006135 | 829 | -1.05 | 2.34E-07 |
| KIAA1276 | 235288_at | AI627532 | XM_039169 | 27146 | -1.05 | 5.52E-05 |
| CNNM1 | 220166_at | NM_020348 | NM_020348 | 26507 | -1.05 | 2.70E-05 |
| RPH3AL | 221614_s_at | BC005153 | NM_006987 | 9501 | -1.04 | 8.30E-04 |
| MGC13098 | 60815_at | AA601208 | XM_379817 | 84820 | -1.04 | 3.22E-05 |
| | | | XM_499280 | | | |
| AI003508 | 244114_x_at | AI003508 | XM_496690 | 401131 | -1.04 | 2.52E-03 |
| RAC2 | 213603_s_at | BE138888 | NM_002872 | 5880 | -1.04 | 2.21E-03 |
| PLXNC1 | 206471_s_at | NM_005761 | NM_005761 | 10154 | -1.04 | 5.22E-03 |
| C19orf30 | 223913_s_at | AB058892 | NM_174947 | 284424 | -1.04 | 1.45E-03 |
| BF002625 | 229530_at | BF002625 | --- | --- | -1.04 | 2.72E-03 |
| ATP6V0A1 | 1559375_s_at | AV739195 | NM_005177 | 535 | -1.04 | 7.96E-03 |
| BACE1 | 224335_s_at | AB050436 | NM_012104 | 23621 | -1.04 | 1.07E-03 |
| | | | NM_138971 | | | |
| | | | NM_138972 | | | |
| | | | NM_138973 | | | |
| MGC9850 | 224857_s_at | BE378670 | NM_152705 | 219404 | -1.04 | 1.09E-03 |
| BC035958 | 1569540_at | BC035958 | --- | --- | -1.04 | 3.88E-03 |
| FLJ32130 | 1554770_x_at | BC040642 | NM_152458 | 146540 | -1.04 | 1.31E-05 |
| GFRA3 | 229936_at | AA694259 | NM_001496 | 2676 | -1.04 | 1.40E-03 |
| FKBP11 | 228308_at | AI452444 | NM_016594 | 51303 | -1.04 | 8.54E-03 |
| AL137290 | 233724_at | AL137290 | --- | --- | -1.04 | 4.79E-04 |
| FRAS1 | 226145_s_at | AL157471 | NM_020875 | 80144 | -1.04 | 3.23E-04 |
| | | | NM_025074 | | | |
| | | | NM_032863 | | | |
| | | | NM_206841 | | | |
| AU145394 | 215370_at | AU145394 | XM_496203 | 440423 | -1.04 | 3.51E-06 |
| BRUNOL6 | 227775_at | BE467313 | NM_052840 | 60677 | -1.04 | 1.58E-03 |
| METAP2 | 240769_at | AI733395 | NM_006838 | 10988 | -1.04 | 8.99E-03 |
| NDP52 | 238560_at | AI684710 | NM_005831 | 10241 | -1.04 | 2.82E-07 |
| AW150065 | 217653_x_at | AW150065 | --- | --- | -1.04 | 2.30E-04 |
| C21orf57 | 227421_at | BF026326 | NM_00100611 4 | 54059 | -1.04 | 4.05E-06 |
| | | | NM_058181 | | | |
| CDKL3 | 1561190_at | AF087989 | NM_016508 | 51265 | -1.03 | 1.48E-04 |
| CLN8 | 219341_at | NM_018941 | NM_018941 | 2055 | -1.03 | 2.97E-03 |
| CACNA1B | 235781_at | AA448208 | NM_000718 | 774 | -1.03 | 6.51E-04 |
| CPEB4 | 242384_at | AI452469 | NM_030627 | 80315 | -1.03 | 8.14E-03 |
| BCAN | 223632_s_at | AI739071 | NM_021948 | 63827 | -1.03 | 2.54E-03 |
| | | | NM_198427 | | | |
| KIAA0746 | 212314_at | AB018289 | NM_015187 | 23231 | -1.03 | 5.88E-05 |
| PGAP1 | 233479_at | AU148008 | NM_024989 | 80055 | -1.03 | 2.96E-03 |
| C1orf50 | 219406_at | NM_024097 | NM_024097 | 79078 | -1.03 | 7.42E-04 |
| AF130084 | 1569538_at | AF130084 | --- | --- | -1.03 | 5.41E-03 |
| PCF11 | 239926_at | AI675753 | NM_015885 | 51585 | -1.03 | 2.46E-03 |
| FNBP1 | 230086_at | AA937109 | NM_015033 | 23048 | -1.03 | 1.57E-04 |
| AW117322 | 239415_at | AW117322 | XM_374094 | 389237 | -1.03 | 5.77E-03 |
| BC033530 | 1560209_at | BC033530 | --- | --- | -1.03 | 9.56E-05 |
| AA888777 | 242740_at | AA888777 | --- | --- | -1.03 | 1.32E-04 |
| SFRS1 | 226419_s_at | AA046439 | NM_006924 | 6426 | -1.03 | 8.12E-03 |
| HNRPD | 229434_at | AA865357 | NM_00100381 0 | 3184 | -1.03 | 1.17E-04 |
| | | | NM_002138 | | | |
| | | | NM_031369 | | | |
| | | | NM_031370 | | | |
| LOC222070 | 51774_s_at | AW014299 | --- | 222070 | -1.03 | 7.32E-05 |
| PHKA1 | 229876_at | BE503584 | NM_002637 | 5255 | -1.02 | 1.73E-05 |
| NPY | 206001_at | NM_000905 | NM_000905 | 4852 | -1.02 | 5.51E-03 |
| CD302 | 203799_at | NM_014880 | NM_014880 | 9936 | -1.02 | 5.85E-04 |
| UBE3A | 1560741_at | AL832250 | | 7337 | -1.02 | 1.28E-03 |
| | | | NM_000462 | | | |
| | | | NM_130838 | | | |
| | | | NM_130839 | | | |
| AI582773 | 214373_at | AI582773 | XM_498956 | 440996 | -1.02 | 5.09E-03 |
| C21orf66 | 240105_at | AI021902 | NM_013329 | 94104 | -1.02 | 3.11E-04 |
| | | | NM_016631 | | | |
| | | | NM_058191 | | | |
| | | | NM_145328 | | | |
| ICA1 | 204002_s_at | NM_022307 | NM_004968 | 3382 | -1.02 | 8.76E-05 |
| | | | NM_022307 | | | |
| | | | NM_022308 | | | |
| CAPN2 | 208683_at | M23254 | NM_001748 | 824 | -1.02 | 7.34E-04 |
| CYLD | 213295_at | AA555096 | NM_015247 | 1540 | -1.02 | 8.54E-04 |
| AK021477 | 232710_at | AK021477 | --- | --- | -1.02 | 9.52E-03 |
| AV658469 | 244699_at | AV658469 | --- | --- | -1.02 | 6.27E-04 |
| GUCY1A3 | 221942_s_at | AI719730 | NM_000856 | 2982 | -1.02 | 1.00E-03 |
| FLJ10287 | 232489_at | AK001149 | NM_019083 | 54482 | -1.02 | 1.54E-03 |
| PAQR6 | 219236_at | NM_024897 | NM_024897 | 79957 | -1.02 | 6.43E-04 |
| | | | NM_198406 | | | |
| PELI1 | 232304_at | AK026714 | NM_020651 | 57162 | -1.01 | 4.26E-04 |
| AU152763 | 232939_at | AU152763 | --- | --- | -1.01 | 1.50E-03 |
| AK021423 | 232890_at | AK021423 | --- | --- | -1.01 | 8.93E-05 |
| SORL1 | 203509_at | NM_003105 | NM_003105 | 6653 | -1.01 | 7.27E-03 |
| PPIE | 210502_s_at | AF042386 | NM_006112 | 10450 | -1.01 | 7.51E-03 |
| | | | NM_203456 | | | |
| | | | NM_203457 | | | |
| FNBP1 | 239453_at | AA262084 | NM_015033 | 23048 | -1.01 | 1.33E-05 |
| C9orf126 | 233246_at | AI523450 | NM_173690 | 286205 | -1.01 | 4.78E-03 |
| FAM13A1 | 232628_at | AI740629 | NM_014883 | 10144 | -1.01 | 2.51E-03 |
| DDX31 | 236210_at | AW628575 | NM_022779 | 64794 | -1.01 | 3.89E-03 |
| | | | NM_138620 | | | |
| CRABP2 | 202575_at | NM_001878 | NM_001878 | 1382 | -1.01 | 3.16E-03 |
| KIAA0746 | 212311_at | AA522514 | NM_015187 | 23231 | -1.01 | 2.25E-03 |
| PDE4C | 206791_s_at | BF511742 | NM_000923 | 5143 | -1.01 | 1.92E-03 |
| BE669703 | 229541_at | BE669703 | --- | --- | -1.01 | 8.81E-05 |
| MLL | 212079_s_at | AA715041 | NM_005933 | 4297 | -1.01 | 8.80E-04 |
| ZDHHC11 | 221646_s_at | AF267859 | NM_024786 | 79844 | -1.01 | 2.90E-04 |
| BC038589 | 1569311_at | BC038589 | --- | --- | -1.01 | 9.76E-04 |
| C21orf66 | 1555125_at | BC030539 | NM_013329 | 94104 | -1.00 | 1.94E-03 |
| | | | NM_016631 | | | |
| | | | NM_058191 | | | |
| | | | NM_145328 | | | |
| AK024879 | 216751_at | AK024879 | --- | --- | -1.00 | 8.95E-06 |
| EFNB3 | 205031_at | NM_001406 | NM_001406 | 1949 | -1.00 | 1.02E-03 |
| AW188311 | 243281_at | AW188311 | --- | --- | -1.00 | 1.13E-03 |
| N95363 | 213183_s_at | N95363 | --- | --- | -1.00 | 2.68E-05 |
| RAB6IP2 | 232565_at | AK025052 | NM_015064 | 23085 | -1.00 | 3.56E-03 |
| | | | NM_178037 | | | |
| | | | NM_178038 | | | |
| | | | NM_178039 | | | |
| | | | NM_178040 | | | |
| RASA4 | 208534_s_at | NM_006989 | NM_006989 | 10156 | -1.00 | 1.13E-03 |
| FLJ21767 | | | XM_376602 | 401331 | | |
| | | | XM_379820 | | | |
| DCTD | 210137_s_at | BC001286 | NM_001921 | 1635 | -1.00 | 2.80E-03 |
| EIF3S9 | 236274_at | AW129967 | NM_003751 | 8662 | -1.00 | 7.13E-04 |
| | | | NM_182712 | | | |
| PTP4A3 | 206574_s_at | NM_007079 | NM_007079 | 11156 | -1.00 | 8.68E-03 |
| | | | NM_032611 | | | |
| ARIH1 | 1558710_at | BI791845 | NM_005744 | 25820 | -1.00 | 2.71E-03 |
| BF507816 | 240517_at | BF507816 | --- | --- | -1.00 | 3.52E-04 |
| C10orf42 | 243335_at | BE764796 | NM_138357 | 90550 | -0.99 | 5.78E-03 |
| LNK | 203320_at | NM_005475 | NM_005475 | 10019 | -0.99 | 9.93E-05 |
| OPTN | 202073_at | AV757675 | NM_00100821 1 | 10133 | -0.99 | 1.43E-03 |
| | | | NM_00100821 2 | | | |
| | | | NM_00100821 3 | | | |
| | | | NM_021980 | | | |
| AK022461 | 232472_at | AK022461 | --- | --- | -0.99 | 8.48E-04 |
| GRK4 | 241989_at | AW269179 | NM_00100405 6 | 2868 | -0.99 | 1.24E-04 |
| | | | NM_00100405 7 | | | |
| | | | NM_005307 | | | |
| | | | NM_182982 | | | |
| LOC200030 | 242191_at | AI701905 | NM_183372 | 200030 | -0.99 | 5.68E-07 |
| LOC400781 | | | XM_371384 | 400781 | | |
| AG1 | | | XM_378897 | 440673 | | |
| | | | XM_496394 | | | |
| RAB3-GAP150 | 240234_at | AI216539 | NM_012414 | 25782 | -0.99 | 3.46E-03 |
| DHX57 | 239311_at | AI367034 | NM_144995 | 90957 | -0.99 | 3.00E-03 |
| | | | NM_145646 | | | |
| | | | NM_198963 | | | |
| LRRN5 | 216167_at | AK024867 | NM_006338 | 10446 | -0.99 | 3.96E-03 |
| | | | NM_201630 | | | |
| GART | 230097_at | AI207338 | NM_000819 | 2618 | -0.99 | 1.29E-03 |
| | | | NM_175085 | | | |
| KIAA2024 | 1566166_at | AL833423 | NM_172070 | 130507 | -0.99 | 2.85E-03 |
| MLL3 | 244010_at | AI057455 | NM_021230 | 58508 | -0.99 | 1.91E-03 |
| | | | NM_170606 | | | |
| GSDML | 215659_at | AK025174 | NM_018530 | 55876 | -0.99 | 4.78E-03 |
| DSCAM | 237268_at | BE503065 | NM_001389 | 1826 | -0.99 | 1.16E-03 |
| | | | NM_206887 | | | |
| AK057701 | 1558515_at | AK057701 | --- | --- | -0.99 | 6.69E-03 |
| MGC11332 | 213393_at | AI767210 | NM_032718 | 84804 | -0.99 | 7.13E-04 |
| ISYNA1 | 222240_s_at | AL137749 | NM_016368 | 51477 | -0.99 | 2.38E-04 |
| CLTC | 220855_at | NM_014127 | NM_004859 | 1213 | -0.99 | 7.98E-03 |
| COL6A2 | 209156_s_at | AY029208 | NM_001849 | 1292 | -0.98 | 2.04E-03 |
| | | | NM_058174 | | | |
| | | | NM_058175 | | | |
| SUV420H1 | 242646_at | AA702946 | NM_016028 | 51111 | -0.98 | 1.65E-04 |
| | | | NM_017635 | | | |
| KIAA1223 | 225735_at | AL110131 | XM_048747 | 57182 | -0.98 | 5.36E-05 |
| DNAJC12 | 218976_at | NM_021800 | NM_021800 | 56521 | -0.98 | 1.57E-06 |
| | | | NM_201262 | | | |
| RHBDL1 | 207383_s_at | NM_003961 | NM_003961 | 9028 | -0.98 | 6.79E-03 |
| UBE2E1 | 236062_at | AI742722 | NM_003341 | 7324 | -0.98 | 5.85E-03 |
| | | | NM_182666 | | | |
| HEL308 | 228736_at | AW084661 | NM_133636 | 113510 | -0.98 | 1.68E-03 |
| H28915 | 244359_s_at | H28915 | XM_496692 | 441014 | -0.98 | 5.93E-03 |
| BRUNOL5 | 232416_at | AL390161 | NM_021938 | 60680 | -0.98 | 4.62E-03 |
| RNF12 | 242121_at | AW973232 | NM_016120 | 51132 | -0.98 | 6.21E-03 |
| | | | NM_183353 | | | |
| SFRS15 | 222310_at | AA648521 | NM_020706 | 57466 | -0.98 | 2.65E-04 |
| ACSL4 | 1557419_a_at | W95007 | NM_004458 | 2182 | -0.98 | 4.91E-03 |
| | | | NM_022977 | | | |
| SPOCK2 | 202524_s_at | NM_014767 | NM_014767 | 9806 | -0.98 | 6.88E-05 |
| KIAA0999 | 242920_at | AW590838 | NM_025164 | 23387 | -0.97 | 3.05E-03 |
| KCNK10 | 220727_at | NM_021161 | NM_021161 | 54207 | -0.97 | 9.90E-03 |
| | | | NM_138317 | | | |
| | | | NM_138318 | | | |
| AATK | 205986_at | NM_004920 | --- | 9625 | -0.97 | 2.81E-05 |
| SERTAD4 | 230660_at | AU146709 | NM_019605 | 56256 | -0.97 | 5.49E-03 |
| CDC2L5 | 242693_at | AW664859 | NM_003718 | 8621 | -0.97 | 6.26E-03 |
| | | | NM_031267 | | | |
| NM_025120 | 220728_at | NM_025120 | --- | --- | -0.97 | 5.32E-03 |
| SMG1 | 244766_at | BG180003 | NM_014006 | 23049 | -0.97 | 2.50E-03 |
| KIAA0220 | | | NM_015092 | 283846 | | |
| LOC440345 | | | XM_496125 | 440345 | | |
| DNAJB5 | 212817_at | AK023253 | NM_012266 | 25822 | -0.97 | 1.18E-03 |
| HIST3H2A | 221582_at | BC001193 | NM_033445 | 92815 | -0.97 | 2.09E-03 |
| SORL1 | 212560_at | AV728268 | NM_003105 | 6653 | -0.97 | 4.84E-03 |
| BBP | 213882_at | AA012917 | NM_032027 | 83941 | -0.97 | 7.60E-03 |
| UGCGL2 | 218801_at | NM_020121 | NM_020121 | 55757 | -0.96 | 1.53E-03 |
| TLE1 | 228284_at | BE302305 | NM_005077 | 7088 | -0.96 | 2.82E-06 |
| CRYZL1 | 1552347_at | NM_145311 | NM_005111 | 9946 | -0.96 | 8.59E-03 |
| | | | NM_145311 | | | |
| | | | NM_145858 | | | |
| KIAA1223 | 225731_at | BF196876 | XM_048747 | 57182 | -0.96 | 1.92E-04 |
| CTNND1 | 238884_at | AW195351 | NM_001331 | 1500 | -0.96 | 7.20E-03 |
| FLJ10980 | 225327 _at | AB037791 | NM_019600 | 56204 | -0.96 | 4.11E-04 |
| LOC255783 | 227325_at | AW172584 | --- | 255783 | -0.96 | 6.35E-03 |
| SLICK | 244455_at | AI732637 | NM_198503 | 343450 | -0.96 | 5.42E-04 |
| MCM3AP | 212269_s_at | AJ010089 | NM_003906 | 8888 | -0.96 | 2.03E-05 |
| EMR3 | 1561042_at | AF086249 | NM_032571 | 84658 | -0.96 | 1.76E-03 |
| | | | NM_152939 | | | |
| OCIAD2 | 225314_at | BG291649 | NM_152398 | 132299 | -0.96 | 6.40E-05 |
| BACE1 | 222463_s_at | AF190725 | NM_012104 | 23621 | -0.96 | 5.33E-04 |
| | | | NM_138971 | | | |
| | | | NM_138972 | | | |
| | | | NM_138973 | | | |
| DNAJC12 | 223721_s_at | AF176013 | NM_021800 | 56521 | -0.95 | 9.38E-05 |
| | | | NM_201262 | | | |
| CCNJ | 1557830_at | AW063658 | NM_019084 | 54619 | -0.95 | 1.55E-03 |
| KLF12 | 243089_at | AA551114 | NM_007249 | 11278 | -0.95 | 3.83E-03 |
| | | | NM_016285 | | | |
| RSL1D1 | 212019_at | AK025446 | NM_015659 | 26156 | -0.95 | 2.34E-04 |
| AK022004 | 233003_at | AK022004 | --- | --- | -0.95 | 3.09E-03 |
| FTO | 215385_at | AK022473 | XM_051200 | 79068 | -0.95 | 4.76E-03 |
| AKAP10 | 236007_at | AU147278 | NM_007202 | 11216 | -0.95 | 8.23E-04 |
| PDXK | 202671_s_at | NM_003681 | NM_003681 | 8566 | -0.95 | 3.67E-06 |
| TRIT1 | 218617_at | NM_017646 | NM_017646 | 54802 | -0.95 | 3.17E-07 |
| MYT1L | 210016_at | BF223003 | NM_015025 | 23040 | -0.95 | 8.33E-05 |
| AA573901 | 227783_at | AA573901 | --- | --- | -0.95 | 5.00E-04 |
| AA804267 | 243185_at | AA804267 | --- | --- | -0.95 | 9.14E-03 |
| AW593330 | 237064_x_at | AW593330 | --- | --- | -0.95 | 2.04E-03 |
| HERC1 | 237632_at | AA765387 | NM_003922 | 8925 | -0.95 | 3.34E-04 |
| INPP1 | 202794_at | NM_002194 | NM_002194 | 3628 | -0.95 | 1.43E-04 |
| LOC169834 | 230876_at | AI827906 | XM_095965 | 169834 | -0.95 | 1.26E-04 |
| C21orf5 | 205248_at | NM_005128 | NM_005128 | 9980 | -0.95 | 3.75E-04 |
| FBXW7 | 222729_at | BE551877 | NM_018315 | 55294 | -0.95 | 4.83E-05 |
| | | | NM_033632 | | | |
| NFASC | 214799_at | A1821777 | NM_00100538 7 | 23114 | -0.95 | 1.20E-03 |
| | | | NM_00100538 8 | | | |
| | | | NM_00100538 9 | | | |
| | | | NM_015090 | | | |
| LOC90639 | 235533_at | AW021673 | --- | 90639 | -0.95 | 6.72E-03 |
| BRUNOL5 | 230497_at | BE503640 | NM_021938 | 60680 | -0.95 | 1.48E-06 |
| CHPT1 | 1559739_at | AK025141 | NM_020244 | 56994 | -0.95 | 1.25E-05 |
| APOC1 | 204416_x_at | NM_001645 | NM_001645 | 341 | -0.95 | 8.52E-03 |
| TPD52 | 228818_at | BF110792 | NM_005079 | 7163 | -0.94 | 2.91E-03 |
| HBXAP | 215786_at | AK022170 | NM_016578 | 51773 | -0.94 | 8.66E-03 |
| ATXN1 | 203232_s_at | NM_000332 | NM_000332 | 6310 | -0.94 | 1.93E-03 |
| PDXK | 218019_s_at | NM_021941 | NM_003681 | 8566 | -0.94 | 1.93E-04 |
| FBXW7 | 218751_s_at | NM_018315 | NM_018315 NM_033632 | 55294 | -0.94 | 8.91E-05 |
| ANKRD11 | 228866_at | BF514864 | NM_013275 | 29123 | -0.94 | 9.50E-03 |
| LOC149297 | 1558113_at | BM545032 | XM_097622 | 149297 | -0.94 | 3.48E-03 |
| FLJ20758 | 228512_at | AW138833 | NM_017952 | 55037 | -0.94 | 8.02E-05 |
| BE552208 | 229359_at | BE552208 | --- | --- | -0.94 | 3.55E-05 |
| PDE4DIP | 244511_at | AV700591 | NM_00100281 0 | 9659 | -0.94 | 2.74E-03 |
| | | | NM_00100281 1 | | | |
| | | | NM_00100281 2 | | | |
| | | | NM_014644 | | | |
| | | | NM_022359 | | | |
| MGC9850 | 224874_at | N32181 | NM_152705 | 219404 | -0.94 | 2.65E-04 |
| DUSP8 | 206374_at | NM_004420 | NM_004420 | 1850 | -0.94 | 1.10E-05 |
| LOC400729 | 243021_at | AI344101 | XM_378824 | 400729 | -0.94 | 5.94E-03 |
| LOC440745 | | | XM_498840 | 440745 | | |
| AI939586 | 237930_at | AI939586 | --- | --- | -0.94 | 6.80E-03 |
| PHF17 | 225820_at | AV646599 | NM_024900 | 79960 | -0.94 | 5.13E-03 |
| | | | NM_199320 | | | |
| LOC222070 | 58900_at | AW025284 | --- | 222070 | -0.93 | 6.09E-04 |
| APOE | 203381_s_at | N33009 | NM_000041 | 348 | -0.93 | 7.96E-03 |
| AF086134 | 1561754_at | AF086134 | XM_498989 | 441057 | -0.93 | 4.00E-03 |
| AMH | 206516_at | NM_000479 | NM_000479 | 268 | -0.93 | 3.43E-04 |
| SYT15 | 1560879_a_at | AI971263 | NM_031912 | 83849 | -0.93 | 1.08E-03 |
| | | | NM_181519 | | | |
| KCNJ12 | 207110_at | NM_021012 | NM_021012 | 3768 | -0.93 | 5.28E-03 |
| ARHGEF4 | 205109_s_at | NM_015320 | NM_015320 | 50649 | -0.93 | 2.32E-03 |
| | | | NM_032995 | | | |
| PPM1B | 232580_x_at | AL117553 | NM_002706 | 5495 | -0.93 | 7.12E-04 |
| | | | NM_177968 | | | |
| | | | NM_177969 | | | |
| ZNF587 | 60794_f_at | AI400621 | NM_032828 | 84914 | -0.93 | 6.69E-06 |
| HIPK2 | 224016_at | AF111850 | NM_022740 | 28996 | -0.93 | 8.37E-03 |
| LOC133926 | 235628_x_at | BG025030 | --- | 133926 | -0.93 | 4.71E-03 |
| AI681025 | 230220_at | AI681025 | --- | --- | -0.93 | 2.78E-04 |
| LOC64744 | 225282_at | AL137764 | NM_022733 | 64744 | -0.92 | 4.81E-05 |
| ICA1 | 214191_at | W67992 | NM_004968 | 3382 | -0.92 | 2.91E-04 |
| | | | NM_022307 | | | |
| | | | NM_022308 | | | |
| DKFZp451M211 | 239240_at | AI814116 | NM_182585 | 285023 | -0.92 | 9.61E-03 |
| SFRS15 | 222311_s_at | AA648521 | NM_020706 | 57466 | -0.92 | 6.79E-03 |
| RNF111 | 241413_at | W80457 | NM_017610 | 54778 | -0.92 | 5.97E-04 |
| KATNB1 | 203162_s_at | NM_005886 | NM_005886 | 10300 | -0.92 | 3.97E-04 |
| PPM1K | 235061_at | AV706522 | NM_152542 | 152926 | -0.92 | 2.02E-03 |
| PHC3 | 233431_x_at | AU148142 | NM_024947 | 80012 | -0.92 | 1.61E-03 |
| TJP1 | 244476_at | R39769 | NM_003257 | 7082 | -0.91 | 9.46E-04 |
| | | | NM_175610 | | | |
| ZDHHC11 | 232417_x_at | AU150300 | NM_024786 | 79844 | -0.91 | 3.90E-03 |
| AKAP9 | 215483_at | AK000270 | NM_005751 | 10142 | -0.91 | 3.78E-03 |
| | | | NM_147166 | | | |
| | | | NM_147171 | | | |
| | | | NM_147185 | | | |
| PLRG1 | 227246_at | BF438014 | NM_002669 | 5356 | -0.91 | 4.23E-03 |
| AL390216 | 234317_s_at | AL390216 | --- | --- | -0.91 | 9.52E-03 |
| METAP2 | 244185_at | AA921841 | NM_006838 | 10988 | -0.91 | 3.64E-04 |
| PER1 | 36829_at | AF022991 | NM_002616 | 5187 | -0.91 | 3.45E-03 |
| FLJ44796 | 230757_at | AA195276 | NM_001001704 | 401209 | -0.91 | 3.23E-03 |
| R3HDM | 240503_at | AW274946 | NM_015361 | 23518 | -0.91 | 3.05E-03 |
| NME5 | 206197_at | NM_003551 | NM_003551 | 8382 | -0.91 | 1.53E-04 |
| MGC5566 | 220449_at | NM_024049 | --- | 79015 | -0.90 | 2.88E-03 |
| MGC13098 | 222208_s_at | W07700 | XM_379817 | 84820 | -0.90 | 1.93E-05 |
| | | | XM_499280 | | | |
| W90010 | 238271_x_at | W90010 | --- | --- | -0.90 | 3.98E-03 |
| DCTD | 201572_x_at | NM_001921 | NM_001921 | 1635 | -0.90 | 3.11E-03 |
| MGC42174 | 244304_at | AW129650 | NM_152383 | 129563 | -0.90 | 8.32E-03 |
| DJ328E19.C1. 1 | 214693_x_at | BE732345 | NM_015383 | 200030 | -0.90 | 2.29E-04 |
| | | | NM_173638 | 25832 | | |
| LOC200030 | | | NM_183372 | 284565 | | |
| MGC8902 | | | XM_371384 | 401967 | | |
| LOC401967 | | | XM_496394 | 440673 | | |
| AG1 | | | XM_496406 | | | |
| AA714835 | 1556646_at | AA714835 | --- | --- | -0.90 | 8.70E-04 |
| PPID | 204186_s_at | AI014573 | NM_005038 | 5481 | -0.90 | 1.20E-03 |
| ST18 | 206135_at | NM_014682 | NM_014682 | 9705 | -0.90 | 1.28E-03 |
| ZNF275 | 225382_at | U82670 | XM_372267 | 10838 | -0.90 | 3.80E-03 |
| W80446 | 231013_at | W80446 | --- | --- | -0.90 | 2.17E-03 |
| AI200555 | 228275_at | AI200555 | --- | --- | -0.90 | 1.34E-05 |
| CXorf43 | 1553646_at | NM_144657 | NM_144657 | 139324 | -0.90 | 5.46E-04 |
| PAPD4 | 222282_at | AV761453 | NM_173797 | 167153 | -0.90 | 7.86E-03 |
| AI015847 | 1568838_at | AI015847 | --- | --- | -0.90 | 3.96E-03 |
| CTGLF1 | 1565620_at | BG621044 | NM_133446 | 119016 | -0.90 | 3.42E-03 |
| KIAA1838 | 241152_at | BE466903 | NM_032448 | 84498 | -0.90 | 1.43E-03 |
| SLC22A17 | 218675_at | NM_020372 | NM_016609 | 51310 | -0.89 | 2.14E-04 |
| | | | NM_020372 | | | |
| FLJ21159 | 220145_at | NM_024826 | NM_024826 | 79884 | -0.89 | 6.77E-03 |
| TRIP12 | 244659_at | AL120025 | NM_004238 | 9320 | -0.89 | 1.07E-03 |
| FAM46A | 224973_at | AL078599 | NM_017633 | 55603 | -0.89 | 5.88E-03 |
| PPP2R2C | 228010_at | AI669212 | NM_020416 | 5522 | -0.89 | 1.10E-03 |
| | | | NM_181876 | | | |
| MAST4 | 210958_s_at | BC003646 | XM_291141 | 23227 | -0.89 | 1.00E-03 |
| KIDINS220 | 1557246_at | AA992480 | NM_020738 | 57498 | -0.89 | 4.66E-03 |
| NOB1P | 223018_at | BC000050 | NM_014062 | 28987 | -0.89 | 6.87E-03 |
| AI469960 | 230653_at | AI469960 | --- | --- | -0.89 | 4.89E-03 |
| HCG8 | 215985_at | X92110 | --- | 80869 | -0.89 | 5.08E-03 |
| BRUNOL4 | 238966_at | AA678985 | NM_020180 | 56853 | -0.89 | 1.12E-05 |
| C21orf51 | 228239_at | AA148789 | NM_058182 | 54065 | -0.89 | 8.47E-04 |
| MST1 | 216320_x_at | U37055 | NM_020998 | 4485 | -0.89 | 7.59E-03 |
| DVL3 | 201908_at | NM_004423 | NM_004423 | 1857 | -0.89 | 2.80E-03 |
| NR2F1 | 209506_s_at | BC004154 | NM_005654 | 7025 | -0.89 | 2.53E-05 |
| STK19 | 243719_at | AI973206 | | 8859 | -0.89 | 3.23E-03 |
| | | | NM_004197 | | | |
| | | | NM_032454 | | | |
| KLF9 | 203543_s_at | NM_001206 | NM_001206 | 687 | -0.89 | 1.00E-03 |
| AA830545 | 235730_at | AA830545 | --- | --- | -0.89 | 1.23E-03 |
| AI949265 | 229156_s_at | AI949265 | XM_499089 | 441301 | -0.89 | 2.40E-04 |
| SHANK2 | 213307_at | AF131790 | NM_012309 | 22941 | -0.88 | 1.79E-04 |
| | | | NM_133266 | | | |
| AI802997 | 235211_at | AI802997 | --- | --- | -0.88 | 8.15E-06 |
| GPR123 | 239221_at | AI884909 | NM_032422 | 84435 | -0.88 | 8.91E-04 |
| ABCA2 | 212772_s_at | AL162060 | NM_001606 | 20 | -0.88 | 4.48E-04 |
| | | | NM_212533 | | | |
| AL049377 | 1562684_at | AL049377 | --- | --- | -0.88 | 5.29E-05 |
| SAT2 | 225272_at | AA128261 | NM_133491 | 112483 | -0.88 | 1.49E-03 |
| ERMAP | 219905_at | NM_018538 | NM_018538 | 114625 | -0.88 | 1.01E-03 |
| C20orf103 | 219463_at | NM_012261 | NM_012261 | 24141 | -0.88 | 2.58E-03 |
| CDS1 | 226185_at | AK026697 | NM_001263 | 1040 | -0.88 | 1.82E-04 |
| AI459283 | 231220_at | AI459283 | --- | --- | -0.88 | 1.08E-03 |
| DKFZp313N062 | 226688_at | AW003508 | NM_173826 | 285343 | -0.88 | 1.19E-03 |
| AW235215 | 238913_at | AW235215 | --- | --- | -0.87 | 7.73E-03 |
| SLC27A2 | 205768_s_at | NM_003645 | NM_003645 | 11001 | -0.87 | 3.01E-03 |
| AA975422 | 242829_x_at | AA975422 | --- | --- | -0.87 | 5.23E-03 |
| DEPDC6 | 218858_at | NM_022783 | NM_022783 | 64798 | -0.87 | 7.47E-04 |
| CDC2L5 | 210965_x_at | BC001274 | NM_003718 | 8621 | -0.87 | 1.03E-03 |
| | | | NM_031267 | | | |
| AHSA2 | 212980_at | AL050376 | NM_152392 | 130872 | -0.87 | 8.94E-03 |
| AK056212 | 1557215_at | AK056212 | --- | --- | -0.87 | 4.38E-04 |
| BF114733 | 236703_at | BF114733 | --- | --- | -0.87 | 8.70E-03 |
| AW628665 | 235555_at | AW628665 | --- | --- | -0.87 | 4.04E-04 |
| FXR1 | 229519_at | H48840 | NM_005087 | 8087 | -0.86 | 7.99E-03 |
| AF5Q31 | 232865_at | N59653 | NM_014423 | 27125 | -0.86 | 2.34E-03 |
| TAF15 | 241885_at | BF431050 | NM_003487 | 8148 | -0.86 | 2.03E-03 |
| | | | NM_139215 | | | |
| NM_001048 | 213921_at | NM_001048 | --- | --- | -0.86 | 1.92E-03 |
| PFAAP5 | 214753_at | AW084068 | NM_014887 | 10443 | -0.86 | 1.88E-03 |
| FRMD4A | 1560031_at | R19413 | NM_018027 | 55691 | -0.86 | 1.21E-04 |
| NELF | 221214_s_at | NM_015537 | NM_015537 | 26012 | -0.86 | 5.19E-05 |
| MGC11332 | 213403_at | BF223370 | NM_032718 | 84804 | -0.86 | 5.38E-03 |
| ETV3 | 1552423_at | NM_005240 | NM_005240 | 2117 | -0.86 | 4.99E-04 |
| TMF1 | 242243_at | AI767435 | NM_007114 | 7110 | -0.86 | 4.40E-03 |
| PIAS2 | 244633_at | AA404996 | NM_004671 | 9063 | -0.86 | 6.85E-03 |
| | | | NM_173206 | | | |
| SLC19A1 | 229489_at | AI681043 | NM_003056 | 6573 | -0.86 | 4.96E-03 |
| | | | NM_194255 | | | |
| ECE2 | 230706_s_at | A1084921 | NM_014693 | 9718 | -0.86 | 7.23E-04 |
| HNRPM | 238610_s_at | AI906424 | NM_005968 | 4670 | -0.86 | 2.13E-03 |
| | | | NM_031203 | | | |
| AW303888 | 1561346_at | AW303888 | --- | --- | -0.86 | 5.89E-07 |
| HNRPM | 233605_x_at | AK022050 | NM_005968 | 4670 | -0.86 | 3.84E-05 |
| | | | NM_031203 | | | |
| FBXO15 | 231472_at | BE464323 | NM_152676 | 201456 | -0.86 | 5.46E-03 |
| UBE2B | 239163_at | AW364833 | NM_003337 | 7320 | -0.86 | 1.91E-03 |
| BE467787 | 243835_at | BE467787 | --- | --- | -0.86 | 3.40E-04 |
| SORCS1 | 228194_s_at | AI675836 | NM_052918 | 114815 | -0.86 | 7.48E-05 |
| ZDHHC11 | 1552283_s_at | NM_024786 | NM_024786 | 79844 | -0.86 | 1.80E-04 |
| DEADC1 | 227213_at | AA706895 | NM_182503 | 134637 | -0.85 | 7.30E-04 |
| RRAGC | 218088_s_at | NM_022157 | NM_022157 | 64121 | -0.85 | 7.96E-03 |
| UBE3C | 242673_at | AA931284 | NM_014671 | 9690 | -0.85 | 2.73E-03 |
| MGC13098 | 224517_at | BC006435 | XM_379817 | 84820 | -0.85 | 2.71E-05 |
| | | | XM_499280 | | | |
| FLJ23825 | 1555866_a_at | BM980844 | NM_173620 | 284004 | -0.85 | 2.22E-03 |
| SLC16A1 | 1560579_s_at | BC023568 | NM_003051 | 6566 | -0.85 | 6.33E-04 |
| RSNL2 | 226425_at | AU144247 | NM_024692 | 79745 | -0.85 | 1.24E-03 |
| LOC283922 | 228688_at | AA843726 | XM_208908 | 283922 | -0.85 | 3.51E-04 |
| GOLGIN-67 | 213650_at | AW006438 | NM_015003 | 23015 | -0.85 | 2.66E-03 |
| | | | NM_181076 | | | |
| | | | NM_181077 | | | |
| FLJ37953 | 241825_at | AI265967 | NM_152382 | 129450 | -0.85 | 1.32E-03 |
| AV700050 | 228835_at | AV700050 | --- | --- | -0.85 | 2.40E-03 |
| KIAA1915 | 1569706_at | BC017579 | XM_055481 | 114803 | -0.85 | 6.38E-03 |
| CEPT1 | 1561884_at | AL833102 | NM_00100779 4 | 10390 | -0.85 | 1.37E-03 |
| | | | NM_006090 | | | |
| BF510762 | 230262_at | BF510762 | --- | --- | -0.85 | 2.02E-06 |
| TAGLN2 | 210978_s_at | BC002616 | NM_003564 | 8407 | -0.84 | 3.57E-03 |
| DUSP6 | 208893_s_at | BC005047 | NM_001946 | 1848 | -0.84 | 1.45E-04 |
| | | | NM_022652 | | | |
| AI811800 | 241846_at | AI811800 | --- | --- | -0.84 | 3.44E-03 |
| AA167167 | 243684_at | AA167167 | --- | --- | -0.84 | 3.92E-03 |
| CNOT6L | 227119_at | BF103856 | NM_144571 | 246175 | -0.84 | 2.79E-07 |
| ARIH1 | 242431_at | AI656728 | NM_005744 | 25820 | -0.84 | 2.27E-03 |
| BC037935 | 1569539_at | BC037935 | --- | --- | -0.84 | 6.55E-04 |
| DUSP6 | 208891_at | BC003143 | NM_001946 | 1848 | -0.84 | 3.81E-03 |
| | | | NM_022652 | | | |
| C15orf28 | 220710_at | NM_024970 | --- | 80035 | -0.84 | 9.94E-03 |
| AA935515 | 242790_at | AA935515 | --- | --- | -0.84 | 1.15E-03 |
| MSI2 | 243010_at | BE000929 | NM_138962 | 124540 | -0.84 | 1.62E-03 |
| | | | NM_170721 | | | |
| CXorf45 | 205583_s_at | NM_024810 | NM_024810 | 79868 | -0.84 | 2.89E-03 |
| PMS2L5 | 242201_at | AI418253 | NM_174930 | 5383 | -0.84 | 7.25E-04 |
| AW000942 | 240230_s_at | AW000942 | --- | --- | -0.84 | 6.90E-03 |
| C9orf94 | 229585_at | AI803088 | NM_152702 | 206938 | -0.84 | 1.26E-03 |
| FLJ20719 | 230712_at | AI634549 | XM_373827 | 55672 | -0.84 | 5.27E-03 |
| GA17 | 215190_at | AV717062 | NM_006360 | 10480 | -0.84 | 6.82E-03 |
| CD200 | 209582_s_at | H23979 | NM_00100419 6 | 4345 | -0.84 | 7.62E-03 |
| | | | NM_00100419 7 | | | |
| | | | NM_005944 | | | |
| AA928506 | 213750_at | AA928506 | --- | --- | -0.84 | 2.15E-04 |
| AHI1 | 221569_at | AL136797 | NM_017651 | 54806 | -0.84 | 2.48E-05 |
| RPIP8 | 213439_x_at | AI197870 | NM_006695 | 10900 | -0.84 | 1.33E-06 |
| AI929792 | 236081_at | AI929792 | --- | --- | -0.84 | 3.07E-04 |
| CTNNB1 | 242558_at | AW362945 | NM_001904 | 1499 | -0.84 | 1.09E-04 |
| ELOVL5 | 1566079_at | AL833001 | NM_021814 | 60481 | -0.83 | 1.76E-03 |
| WDR11 | 229694_at | BF062828 | NM_018117 | 55717 | -0.83 | 2.23E-03 |
| MAN2A2 | 219999_at | NM_018621 | NM_006122 | 4122 | -0.83 | 4.36E-03 |
| AA825721 | 242228_at | AA825721 | --- | --- | -0.83 | 8.71E-04 |
| RAB12 | 238714_at | AA504249 | XM_113967 | 201475 | -0.83 | 4.32E-04 |
| KCNMA1 | 221584_s_at | U11058 | NM_002247 | 3778 | -0.83 | 7.71E-04 |
| BE467087 | 231075_x_at | BE467087 | --- | --- | -0.83 | 4.80E-03 |
| FGD5 | 226985_at | AW269340 | NM_152536 | 152273 | -0.83 | 5.34E-03 |
| ProSAPiP1 | 204447_at | NM_014731 | NM_014731 | 9762 | -0.83 | 1.67E-04 |
| ZNF539 | 1559449_a_at | BF679633 | NM_203282 | 399655 | -0.83 | 2.80E-04 |
| NKX6-1 | 221366_at | NM_006168 | NM_006168 | 4825 | -0.83 | 7.76E-03 |
| FLJ22624 | 236560_at | BE218020 | NM_024808 | 79866 | -0.83 | 7.40E-03 |
| MGC72104 | 234949_at | AL117574 | NM_207350 | 284802 | -0.83 | 1.91E-03 |
| GRIK2 | 1563754_at | AJ252246 | NM_021956 | 2898 | -0.83 | 6.46E-03 |
| | | | NM_175768 | | | |
| AI018235 | 240314_at | AI018235 | --- | --- | -0.82 | 3.87E-03 |
| ZNF518 | 242343_x_at | H57111 | NM_014803 | 9849 | -0.82 | 6.06E-03 |
| FNBP4 | 235101_at | AV683244 | NM_015308 | 23360 | -0.82 | 3.49E-03 |
| C21orf86 | 226995_at | AV705934 | NM_153454 | 257103 | -0.82 | 2.10E-03 |
| AW148862 | 235217_at | AW148862 | XM_380136 | 402582 | -0.82 | 8.18E-03 |
| AL559202 | 226458_at | AL559202 | --- | --- | -0.82 | 5.40E-05 |
| JMJD2C | 214861_at | AI341811 | NM_015061 | 23081 | -0.82 | 2.97E-03 |
| AOF2 | 212348_s_at | AB011173 | NM_00100999 9 | 23028 | -0.82 | 1.77E-05 |
| | | | NM_015013 | | | |
| TTC3 | 208663_s_at | AI652848 | NM_00100189 4 | 7267 | -0.82 | 7.37E-05 |
| | | | NM_003316 | | | |
| BE327722 | 239840_at | BE327722 | --- | --- | -0.82 | 1.21E-06 |
| AK021474 | 215892_at | AK021474 | --- | --- | -0.82 | 9.46E-05 |
| BF344235 | 242202_at | BF344235 | XM_379855 | 402530 | -0.82 | 5.35E-04 |
| YLPM1 | 1557065_at | AA927802 | XM_085151 | 56252 | -0.82 | 8.82E-03 |
| CaMKIINalpha | 228302_x_at | AW162846 | NM_018584 | 55450 | -0.81 | 1.09E-03 |
| AKT2 | 236664_at | AA448167 | NM_001626 | 208 | -0.81 | 3.20E-03 |
| PRKCD | 202545_at | NM_006254 | NM_006254 | 5580 | -0.81 | 2.09E-04 |
| | | | NM_212539 | | | |
| CYLD | 39582_at | AL050166 | NM_015247 | 1540 | -0.81 | 1.26E-03 |
| EIF4G1 | 208624_s_at | BE966878 | NM_004953 | 1981 | -0.81 | 7.60E-04 |
| | | | NM_182917 | | | |
| | | | NM_198241 | | | |
| | | | NM_198242 | | | |
| | | | NM_198244 | | | |
| ACVR2 | 244332_at | AW974077 | NM_001616 | 92 | -0.81 | 1.17E-06 |
| AI150690 | 228466_at | AI150690 | --- | --- | -0.81 | 4.49E-04 |
| AV700891 | 222303_at | AV700891 | --- | --- | -0.81 | 1.60E-03 |
| C20orf13 | 233719_s_at | AL121754 | NM_017714 | 55617 | -0.81 | 6.92E-04 |
| SMURF2 | 230099_at | AI139993 | NM_022739 | 64750 | -0.81 | 2.36E-03 |
| SHC2 | 213464_at | AV705938 | XM_375550 | 25759 | -0.81 | 4.62E-06 |
| AW167298 | 228116_at | AW167298 | XM_210856 | 283029 | -0.80 | 5.41E-03 |
| GPR56 | 206582_s_at | NM_005682 | NM_005682 | 9289 | -0.80 | 4.72E-03 |
| | | | NM_201524 | | | |
| | | | NM_201525 | | | |
| ATXN7 | 209964_s_at | AF032105 | NM_000333 | 6314 | -0.80 | 2.95E-03 |
| AK000995 | 215349_at | AK000995 | --- | --- | -0.80 | 4.02E-03 |
| AW086077 | 232800_at | AW086077 | --- | --- | -0.80 | 1.60E-03 |
| SLC37A1 | 218928_s_at | NM_018964 | NM_018964 | 54020 | -0.80 | 5.55E-03 |
| PCNXL2 | 229202_at | AI768826 | NM_014801 | 80003 | -0.80 | 2.18E-04 |
| | | | NM_024938 | | | |
| SCMH1 | 221216_s_at | NM_012236 | NM_012236 | 22955 | -0.80 | 5.20E-06 |
| FLJ31951 | 236322_at | AA830854 | NM_144726 | 153830 | -0.80 | 5.68E-03 |
| CXXC5 | 233955_x_at | AK001782 | NM_016463 | 51523 | -0.80 | 9.43E-05 |
| KIAA0220 | 231989_s_at | AC003007 | --- | 283846 | -0.80 | 5.47E-04 |
| POLR3C | 239383_at | AW023610 | NM_006468 | 10623 | -0.79 | 4.37E-03 |
| SLC9A3R2 | 209830_s_at | AF035771 | NM_004785 | 9351 | -0.79 | 1.09E-04 |
| CACNA1D | 207998_s_at | NM_000720 | NM_000720 | 776 | -0.79 | 2.71E-03 |
| AI417268 | 225028_at | AI417268 | --- | --- | -0.79 | 5.37E-03 |
| CTSB | 213274_s_at | AA020826 | NM_001908 | 1508 | -0.79 | 2.12E-04 |
| | | | NM_147780 | | | |
| | | | NM_147781 | | | |
| | | | NM_147782 | | | |
| | | | NM_147783 | | | |
| C9orf85 | 1557805_at | AF085969 | NM_182505 | 138241 | -0.79 | 4.15E-06 |
| | | | NM_198394 | | | |
| TNRC15 | 1560133_at | BC012484 | NM_015575 | 26058 | -0.79 | 1.04E-04 |
| ARSD | 225280_x_at | N51673 | NM_001669 | 414 | -0.79 | 8.39E-03 |
| | | | NM_009589 | | | |
| KLHDC1 | 1552733_at | NM_172193 | NM_172193 | 122773 | -0.79 | 8.55E-05 |
| PRO0478 | 220696_at | NM_014129 | --- | 29048 | -0.79 | 7.62E-04 |
| RAB40B | 204547_at | NM_006822 | NM_006822 | 10966 | -0.79 | 3.04E-04 |
| ETFDH | 33494_at | S69232 | NM_004453 | 2110 | -0.79 | 1.02E-03 |
| TGFBR1 | 236561_at | AV700621 | NM_004612 | 7046 | -0.79 | 6.33E-03 |
| GPR18 | 233554_at | AF339764 | NM_005292 | 2841 | -0.79 | 2.72E-04 |
| U2AF1 | 231904_at | AU122448 | NM_006758 | 7307 | -0.79 | 1.73E-03 |
| DKFZP434I0714 | 231954_at | AL137273 | --- | 54553 | -0.79 | 1.34E-03 |
| YAP | 244803_at | AI335191 | NM_018253 | 55249 | -0.79 | 7.84E-05 |
| | | | NM_139118 | | | |
| | | | NM_139119 | | | |
| | | | NM_139120 | | | |
| | | | NM_139121 | | | |
| LOC23117 | 238341_at | BF677084 | XM_290670 | 23117 | -0.79 | 1.63E-03 |
| CLASP2 | 212308_at | AL137636 | NM_015097 | 23122 | -0.78 | 1.67E-03 |
| C18orf17 | 238480_at | AI871745 | NM_153211 | 125488 | -0.78 | 8.79E-04 |
| LOC51066 | 206741_at | NM_015931 | NM_015931 | 51066 | -0.78 | 3.65E-05 |
| S100A13 | 202598_at | NM_005979 | XM_371380 | 6284 | -0.78 | 4.04E-03 |
| FLJ39653 | 1553145_at | BC010030 | NM_152684 | 202020 | -0.78 | 4.89E-03 |
| ANP32A | 235954_at | AA972597 | NM_006305 | 8125 | -0.78 | 9.06E-03 |
| TAF15 | 227884_at | AW296067 | NM_003487 | 8148 | -0.78 | 1.80E-03 |
| | | | NM_139215 | | | |
| HNRPR | 208765_s_at | NM_005826 | NM_005826 | 10236 | -0.78 | 8.21E-05 |
| AF194537 | 224549_x_at | AF194537 | --- | --- | -0.78 | 6.16E-03 |
| AF5Q31 | 1555436_a_at | BC025700 | NM_014423 | 27125 | -0.78 | 9.74E-05 |
| MGC47869 | 229888_at | AA861435 | NM_175874 | 144608 | -0.78 | 5.64E-03 |
| MEG3 | 235077_at | BF956762 | XR_000277 | 55384 | -0.78 | 2.12E-03 |
| DKFZp547A023 | 214731_at | AB037854 | NM_018704 | 55917 | -0.78 | 1.66E-03 |
| AI820796 | 217704_x_at | AI820796 | --- | --- | -0.78 | 1.61E-04 |
| CCNB1IP1 | 217988_at | NM_021178 | NM_021178 | 57820 | -0.77 | 9.59E-03 |
| | | | --- | | | |
| | | | NM_182851 | | | |
| | | | NM_182852 | | | |
| WDFY1 | 1562955_at | BC028181 | NM_020830 | 57590 | -0.77 | 2.91E-03 |
| GPLD1 | 238752_at | AA780295 | NM_001503 | 2822 | -0.77 | 2.12E-04 |
| | | | NM_177483 | | | |
| CTSB | 213275_x_at | W47179 | NM_001908 | 1508 | -0.77 | 2.40E-03 |
| | | | NM_147780 | | | |
| | | | NM_147781 | | | |
| | | | NM_147782 | | | |
| | | | NM_147783 | | | |
| PLA2G4B | 222256_s_at | AK000550 | NM_005090 | 8681 | -0.77 | 2.03E-03 |
| BE856980 | 230961_at | BE856980 | --- | --- | -0.77 | 2.41E-03 |
| ABTB1 | 229164_s_at | AI337369 | NM_032548 | 80325 | -0.77 | 2.25E-03 |
| | | | NM_172028 | | | |
| AI702720 | 242309_at | A1702720 | --- | --- | -0.77 | 2.84E-03 |
| MGC4707 | 238161_at | AA813859 | NM_00100367 6 | 79096 | -0.77 | 2.25E-03 |
| | | | NM_00100367 7 | | | |
| | | | NM_00100367 8 | | | |
| | | | NM_024113 | | | |
| CD200 | 209583_s_at | AF063591 | NM_00100419 6 | 4345 | -0.77 | 1.43E-03 |
| | | | NM_00100419 7 | | | |
| | | | NM_005944 | | | |
| GTF3C3 | 1555439_at | AF465407 | NM_012086 | 9330 | -0.77 | 3.57E-05 |
| PTPRD | 214043_at | BF062299 | NM_002839 | 5789 | -0.77 | 6.45E-05 |
| | | | NM_130391 | | | |
| | | | NM_130392 | | | |
| | | | NM_130393 | | | |
| MLR1 | 235970_at | AI807408 | NM_153686 | 254251 | -0.77 | 1.70E-03 |
| AI305170 | 235747_at | AI305170 | --- | --- | -0.77 | 8.11E-04 |
| PLXNC1 | 213241_at | AF035307 | NM_005761 | 10154 | -0.77 | 8.27E-03 |
| SP192 | 1569320_at | CA391618 | NM_021639 | 60313 | -0.77 | 2.71E-03 |
| LRCH3 | 1559490_at | AL832278 | NM_032773 | 84859 | -0.77 | 7.53E-03 |
| BC035170 | 1561079_at | BC035170 | --- | --- | -0.77 | 3.38E-03 |
| BC029480 | 1554447_at | BC029480 | --- | --- | -0.77 | 1.93E-03 |
| AI598222 | 235679_at | AI598222 | --- | --- | -0.77 | 4.88E-04 |
| SH3KBP1 | 223082_at | AF230904 | NM_031892 | 30011 | -0.77 | 9.30E-05 |
| TNFSF13B | 223502_s_at | AF134715 | NM_006573 | 10673 | -0.77 | 2.15E-06 |
| AP3B2 | 205678_at | NM_004644 | NM_004644 | 8120 | -0.76 | 1.23E-04 |
| MDS009 | 236649_at | AA907927 | NM_020234 | 56986 | -0.76 | 6.41E-04 |
| CLIPR-59 | 212358_at | AL117468 | NM_015526 | 25999 | -0.76 | 2.11E-03 |
| EDG4 | 206723_s_at | AF011466 | NM_004720 | 9170 | -0.76 | 2.87E-04 |
| RNASET2 | 217984_at | NM_003730 | NM_003730 | 8635 | -0.76 | 7.52E-03 |
| AI979338 | 229364_at | AI979338 | --- | --- | -0.76 | 5.81E-03 |
| KIAA1545 | 225704_at | AA527531 | XM_495939 | 57666 | -0.76 | 8.16E-03 |
| MGC29898 | 235259_at | AA398590 | NM_145048 | 133015 | -0.76 | 3.12E-03 |
| SIDT2 | 56256_at | AA150165 | NM_015996 | 51092 | -0.76 | 8.92E-03 |
| MGC20741 | 232826_at | AU144129 | NM_018561 | 25862 | -0.76 | 2.64E-03 |
| WBSCR19 | 232964_at | AL137266 | NM_175064 | 285955 | -0.76 | 3.56E-03 |
| ACTR2 | 234210_x_at | AK025051 | NM_00100538 6 | 10097 | -0.76 | 2.63E-03 |
| | | | NM_005722 | | | |
| LOC92249 | 212957_s_at | AU154785 | XM_499179 | 92249 | -0.76 | 1.06E-03 |
| JAK1 | 234193_at | AK025218 | NM_002227 | 3716 | -0.76 | 5.21E-03 |
| PRH1 | 243389_at | AW663570 | NM_006250 | 5554 | -0.76 | 3.39E-03 |
| AI523613 | 222358_x_at | AI523613 | --- | --- | -0.76 | 4.89E-06 |
| HSPA12A | 214434_at | AB007877 | XM_048898 | 259217 | -0.76 | 6.62E-03 |
| PIK3R2 | 1568629_s_at | BC033311 | NM_005027 | 5296 | -0.76 | 4.17E-03 |
| MEG3 | 212732_at | AI950273 | XR_000277 | 55384 | -0.76 | 2.60E-03 |
| AI743452 | 237031_at | AI743452 | --- | --- | -0.76 | 8.51E-03 |
| PVT1 | 222087_at | AW451806 | XM_372058 | 5820 | -0.76 | 1.17E-04 |
| HNRPD | 235999_at | AA863112 | NM_00100381 0 | 3184 | -0.75 | 2.35E-03 |
| | | | NM_002138 | | | |
| | | | NM_031369 | | | |
| | | | NM_031370 | | | |
| BG253162 | 242585_at | BG253162 | --- | --- | -0.75 | 7.96E-04 |
| LOC401320 | 238540_at | AA933883 | XM_379482 | 401320 | -0.75 | 9.69E-03 |
| | | | XM_380098 | | | |
| AK024681 | 231972_at | AK024681 | --- | --- | -0.75 | 5.89E-05 |
| FLJ14167 | 232289_at | BF237871 | --- | 92080 | -0.75 | 9.36E-03 |
| FLJ10996 | 244648_at | AA007283 | NM_019044 | 54520 | -0.75 | 2.90E-03 |
| TMEM25 | 226647_at | AL562445 | NM_032780 | 84866 | -0.75 | 1.43E-03 |
| BC003629 | 210230_at | BC003629 | --- | --- | -0.75 | 7.07E-03 |
| DUSP6 | 208892_s_at | BC003143 | NM_001946 | 1848 | -0.75 | 5.05E-03 |
| | | | NM_022652 | | | |
| H63 | 1552719_at | NM_138423 | NM_138423 | 113201 | -0.75 | 2.02E-03 |
| | | | NM_177974 | | | |
| CXorf45 | 205584_at | NM_024810 | NM_024810 | 79868 | -0.75 | 2.96E-03 |
| BG396868 | 1559957_a_at | BG396868 | --- | --- | -0.75 | 1.66E-04 |
| CLDN12 | 223249_at | AL136770 | NM_012129 | 9069 | -0.75 | 3.65E-04 |
| UBE2G2 | 209042_s_at | BC001738 | NM_003343 | 7327 | -0.75 | 1.16E-05 |
| | | | NM_182688 | | | |
| BTN3A1 | 209770_at | U90552 | NM_007048 | 11119 | -0.75 | 8.21E-03 |
| | | | NM_194441 | | | |
| MRPL1 | 223154_at | AF212225 | NM_020236 | 65008 | -0.75 | 1.63E-03 |
| SYNJ1 | 207594_s_at | NM_003895 | NM_003895 | 8867 | -0.75 | 9.53E-03 |
| | | | NM_203446 | | | |
| SORBS1 | 218087_s_at | NM_015385 | NM_006434 | 10580 | -0.75 | 9.17E-03 |
| | | | NM_015385 | | | |
| PORCN | 219483_s_at | NM_022825 | NM_022825 | 64840 | -0.75 | 3.19E-05 |
| | | | NM_203473 | | | |
| | | | NM_203474 | | | |
| | | | NM_203475 | | | |
| | | | NM_203476 | | | |
| ZRANB3 | 244092_at | AI670931 | NM_032143 | 84083 | -0.75 | 9.19E-03 |
| MRPS30 | 231207_at | AW263539 | NM_016640 | 10884 | -0.75 | 6.93E-04 |
| FLJ12666 | 217893_s_at | NM_024595 | NM_024595 | 79647 | -0.74 | 3.79E-03 |
| DKFZp761L141 | 238426_at | AV707703 | NM_152913 | 222865 | -0.74 | 8.47E-03 |
| AI814750 | 1565692_at | AI814750 | --- | --- | -0.74 | 2.10E-03 |
| H2AFY | 226840_at | AW291297 | NM_004893 | 9555 | -0.74 | 2.05E-04 |
| | | | NM_138609 | | | |
| | | | NM_138610 | | | |
| LOC388312 | 238678_at | AI094787 | XM_373704 | 388312 | -0.74 | 3.88E-03 |
| MCAM | 209087_x_at | AF089868 | NM_006500 | 4162 | -0.74 | 6.99E-03 |
| SH3YL1 | 204019_s_at | NM_015677 | NM_015677 | 26751 | -0.74 | 1.21E-03 |
| KCNH2 | 210036_s_at | AB044806 | NM_000238 | 3757 | -0.74 | 6.86E-05 |
| | | | NM_172056 | | | |
| | | | NM_172057 | | | |
| AW157450 | 235716_at | AW157450 | --- | --- | -0.74 | 1.29E-03 |
| LOC400099 | 230136_at | AI573252 | XM_378399 | 400099 | -0.74 | 3.10E-03 |
| ASB13 | 239610_at | BF509210 | NM_024701 | 79754 | -0.74 | 1.28E-03 |
| POU6F1 | 216330_s_at | L14482 | NM_002702 | 5463 | -0.74 | 2.20E-03 |
| SH2D3C | 1552667_a_at | NM_005489 | NM_005489 | 10044 | -0.74 | 7.58E-03 |
| | | | NM_170600 | | | |
| JAM2 | 219213_at | NM_021219 | NM_021219 | 58494 | -0.74 | 3.58E-05 |
| AW665096 | 214188_at | AW665096 | --- | --- | -0.74 | 2.49E-03 |
| RIOK3 | 215588_x_at | AK024958 | NM_003831 | 8780 | -0.73 | 2.49E-04 |
| | | | NM_145906 | | | |
| AI963605 | 230269_at | AI963605 | --- | --- | -0.73 | 8.73E-04 |
| BE217923 | 228686_at | BE217923 | --- | --- | -0.73 | 1.71E-03 |
| C6orf111 | 226412_at | N66397 | NM_032870 | 25957 | -0.73 | 9.78E-03 |
| BG109865 | 222459_at | BG 109865 | --- | --- | -0.73 | 1.83E-03 |
| SP3 | 232529_at | AU144413 | NM_003111 | 6670 | -0.73 | 2.50E-03 |
| RAB18 | 229398_at | A1769954 | NM_021252 | 22931 | -0.73 | 3.78E-03 |
| KIAA1324 | 243349_at | AI672868 | NM_020775 | 57535 | -0.73 | 8.70E-03 |
| CTSB | 227961_at | AA130998 | NM_001908 | 1508 | -0.73 | 7.63E-04 |
| | | | NM_147780 | | | |
| | | | NM_147781 | | | |
| | | | NM_147782 | | | |
| | | | NM_147783 | | | |
| AV733292 | 225444_at | AV733292 | --- | --- | -0.73 | 1.88E-04 |
| H73101 | 1556984_at | H73101 | --- | --- | -0.73 | 1.17E-03 |
| MGAT4A | 219797_at | NM_012214 | NM_012214 | 11320 | -0.73 | 2.44E-03 |
| EPOR | 396_f_at | X97671 | NM_000121 | 2057 | -0.73 | 4.18E-03 |
| LOC401394 | 231406_at | AW205664 | XM_379512 | 401394 | -0.73 | 2.54E-04 |
| GPR153 | 221902_at | AL567940 | NM_207370 | 387509 | -0.73 | 2.83E-03 |
| KIAA0241 | 1563452_at | AL833560 | NM_015060 | 23080 | -0.73 | 5.29E-04 |
| BF114745 | 235459_at | BF114745 | --- | --- | -0.73 | 8.34E-03 |
| AA553722 | 243594_x_at | AA553722 | --- | --- | -0.73 | 9.09E-04 |
| AL359605 | 227318_at | AL359605 | --- | --- | -0.73 | 3.65E-05 |
| SMA4 | 214850_at | X75940 | NM_021652 | 11039 | -0.73 | 1.38E-03 |
| ADFP | 209122_at | BC005127 | NM_001122 | 123 | -0.73 | 5.72E-04 |
| ETS2 | 201329_s_at | NM_005239 | NM_005239 | 2114 | -0.73 | 5.11E-03 |
| AV761014 | 217667_at | AV761014 | --- | --- | -0.73 | 4.49E-03 |
| LOC283174 | 229734_at | BF507379 | NM_001001873 | 283174 | -0.73 | 1.27E-04 |
| IGBP1 | 242337_at | AI347128 | NM_001551 | 3476 | -0.73 | 3.64E-03 |
| TMEM1 | 209412_at | U61500 | NM_00100172 3 | 7109 | -0.73 | 3.07E-03 |
| | | | NM_003274 | | | |
| MGEA5 | 223494_at | AF307332 | NM_012215 | 10724 | -0.73 | 4.98E-03 |
| PEX6 | 204545_at | NM_000287 | NM_000287 | 5190 | -0.73 | 9.95E-03 |
| MGC42174 | 242539_at | AW665509 | NM_152383 | 129563 | -0.73 | 6.73E-04 |
| KIF5C | 203129_s_at | BF059313 | XM_377774 | 3800 | -0.73 | 3.45E-04 |
| GPR56 | 212070_at | AL554008 | NM_005682 | 9289 | -0.72 | 1.51E-03 |
| | | | NM_201524 | | | |
| | | | NM_201525 | | | |
| AMOTL2 | 203002_at | NM_016201 | NM_016201 | 51421 | -0.72 | 5.43E-04 |
| LOC440360 | 236838_at | H11609 | XM_498641 | 440360 | -0.72 | 5.58E-03 |
| MYEF2 | 222153_at | AK023133 | NM_016132 | 50804 | -0.72 | 2.99E-03 |
| T82487 | 241759_at | T82487 | --- | --- | -0.72 | 2.18E-03 |
| CSTB | 201201_at | NM_000100 | NM_000100 | 1476 | -0.72 | 9.61E-04 |
| NGLY1 | 207492_at | NM_025105 | NM_018297 | 55768 | -0.72 | 9.68E-03 |
| DUSP8 | 238594_x_at | AI864441 | NM_004420 | 1850 | -0.72 | 6.31E-03 |
| LUZP1 | 1558173_a_at | AK093016 | NM_033631 | 7798 | -0.72 | 3.02E-04 |
| BRUNOL4 | 223654_s_at | BC004167 | NM_020180 | 56853 | -0.72 | 3.06E-04 |
| RGS8 | 234297_at | AL359941 | NM_033345 | 85397 | -0.72 | 8.24E-03 |
| AI950023 | 244433_at | AI950023 | --- | --- | -0.72 | 5.91E-03 |
| AA327293 | 1566284_at | AA327293 | --- | --- | -0.72 | 1.66E-03 |
| CA442342 | 1556097_at | CA442342 | --- | --- | -0.72 | 3.35E-03 |
| PDE8B | 243590_at | AA860184 | NM_003719 | 8622 | -0.72 | 9.18E-03 |
| ARHGAP1 | 241419_at | AA709154 | NM_004308 | 392 | -0.72 | 4.95E-03 |
| DKFZp547I014 | 207283_at | NM_020217 | NM_020217 | 56969 | -0.72 | 2.61E-03 |
| DNASE1L1 | 203912_s_at | NM_006730 | NM_00100993 2 | 1774 | -0.72 | 1.52E-03 |
| | | | NM_00100993 3 | | | |
| | | | NM_00100993 4 | | | |
| | | | NM_006730 | | | |
| ANKRD17 | 225852_at | BE463523 | NM_032217 | 26057 | -0.72 | 7.58E-06 |
| | | | NM_198889 | | | |
| AI827550 | 229606_at | AI827550 | --- | --- | -0.72 | 1.23E-04 |
| AI538880 | 1558250_s_at | AI538880 | --- | --- | -0.72 | 7.65E-05 |
| AI040738 | 240730_at | AI040738 | --- | --- | -0.72 | 7.11E-03 |
| FBXW7 | 229419_at | BF222826 | NM_018315 | 55294 | -0.72 | 5.69E-04 |
| | | | NM_033632 | | | |
| ABCG1 | 204567_s_at | NM_004915 | NM_004915 | 9619 | -0.72 | 3.49E-04 |
| | | | NM_016818 | | | |
| | | | NM_207174 | | | |
| | | | NM_207627 | | | |
| | | | NM_207628 | | | |
| | | | NM_207629 | | | |
| | | | NM_207630 | | | |
| TRA2A | 213593_s_at | AW978896 | NM_013293 | 29896 | -0.72 | 4.15E-03 |
| APC | 215310_at | AF038181 | NM_000038 | 324 | -0.72 | 3.73E-03 |
| CXXC5 | 222996_s_at | BC002490 | NM_016463 | 51523 | -0.71 | 8.07E-04 |
| CSNK1A1 | 235464_at | AA010757 | NM_001892 | 1452 | -0.71 | 6.52E-04 |
| USP7 | 230967_s_at | BF433061 | NM_003470 | 7874 | -0.71 | 1.69E-03 |
| TAS2R14 | 235762_at | AI458566 | NM_023922 | 50840 | -0.71 | 1.07E-03 |
| HNRPR | 208766_s_at | BC001449 | NM_005826 | 10236 | -0.71 | 5.99E-04 |
| CAPN1 | 232012_at | AV691296 | NM_005186 | 823 | -0.71 | 4.07E-03 |
| AI825987 | 235889_at | AI825987 | --- | --- | -0.71 | 1.70E-03 |
| FLJ23867 | 226706_at | BE301252 | --- | 200058 | -0.71 | 5.23E-03 |
| IGF2 | 202410_x_at | NM_000612 | NM_000612 | 3481 | -0.71 | 2.47E-03 |
| AI887898 | 228423_at | AI887898 | XM_374094 | 389237 | -0.71 | 2.25E-03 |
| MGC14801 | 224443_at | BC005997 | NM_032705 | 84791 | -0.71 | 1.76E-03 |
| C1orf50 | 62212_at | W37846 | NM_024097 | 79078 | -0.71 | 1.18E-03 |
| RLF | 204243_at | NM_012421 | NM_012421 | 6018 | -0.71 | 2.22E-04 |
| AW268719 | 236798_at | AW268719 | --- | --- | -0.71 | 1.17E-04 |
| AL080112 | 216858_x_at | AL080112 | --- | --- | -0.71 | 6.17E-04 |
| AI022648 | 242972_at | AI022648 | --- | --- | -0.71 | 3.82E-04 |
| AW072078 | 230959_at | AW072078 | XM_496690 | 401131 | -0.71 | 2.04E-05 |
| PHKA1 | 205450_at | NM_002637 | NM_002637 | 5255 | -0.71 | 2.60E-05 |
| AA694209 | 229066_at | AA694209 | --- | --- | -0.71 | 1.09E-04 |
| MAP1LC3B | 208786_s_at | AF183417 | NM_022818 | 81631 | -0.71 | 4.58E-03 |
| PABPC4 | 201064_s_at | NM_003819 | NM_003819 | 8761 | -0.71 | 2.02E-04 |
| IGF2 | 210881_s_at | M17863 | NM_000612 | 3481 | -0.70 | 3.36E-03 |
| C5orf16 | 1564031_a_at | AK054889 | NM_ 173828 | 285613 | -0.70 | 3.27E-03 |
| UBXD1 | 223012_at | AF272894 | NM_025241 | 80700 | -0.70 | 2.81E-03 |
| ANKRD28 | 229307_at | N32051 | NM_015199 | 23243 | -0.70 | 5.01E-03 |
| UNQ1912 | 226752_at | AI816071 | NM_ 198507 | 345757 | -0.70 | 3.53E-03 |
| ARHGAP1 | 202117_at | BG468434 | NM_004308 | 392 | -0.70 | 2.81E-03 |
| C21orf59 | 218123_at | NM_017835 | NM_017835 | 56683 | -0.70 | 6.33E-07 |
| | | | NM_021254 | | | |
| MONDOA | 225157_at | AW245631 | NM_014938 | 22877 | -0.70 | 1.12E-04 |
| NM_024900 | 218517_at | NM_024900 | | --- | -0.70 | 8.22E-04 |
| C6orf133 | 215558_at | AK001118 | NM_015255 | 23304 | -0.70 | 6.83E-03 |
| TRIM14 | 203147_s_at | BE962483 | NM_014788 | 9830 | -0.70 | 7.70E-03 |
| | | | NM_033219 | | | |
| | | | NM_033220 | | | |
| | | | NM_033221 | | | |
| NM_018678 | 220494_s_at | NM_018678 | --- | --- | -0.70 | 1.04E-03 |
| LOC144766 | 243986_at | BE409452 | XM_378419 | 144766 | -0.70 | 1.05E-03 |
| PPP3CA | 202429_s_at | AL353950 | NM_000944 | 5530 | -0.70 | 5.44E-04 |
| RNF41 | 201962_s_at | NM_005785 | NM_005785 | 10193 | -0.70 | 4.15E-04 |
| | | | NM_194358 | | | |
| | | | NM_194359 | | | |
| RPL35A | 215208_x_at | AK021571 | NM_000996 | 6165 | -0.70 | 5.45E-03 |
| CLSTN3 | 204375_at | NM_014718 | NM_014718 | 9746 | -0.70 | 4.42E-04 |
| PTK2 | 241387_at | AW276701 | NM_005607 | 5747 | -0.70 | 5.63E-04 |
| | | | NM_153831 | | | |
| SYNJ1 | 212990_at | AB020717 | NM_003895 | 8867 | -0.69 | 1.15E-06 |
| | | | NM_203446 | | | |
| KIAA1276 | 227504_s_at | N64630 | XM_039169 | 27146 | -0.69 | 7.28E-06 |
| MLR1 | 240592_at | AI939336 | NM_ 153686 | 254251 | -0.69 | 3.41E-04 |
| LMNA | 1554600_s_at | BC033088 | NM_005572 | 4000 | -0.69 | 9.76E-03 |
| | | | NM_ 170707 | | | |
| | | | NM_ 170708 | | | |
| ZNF207 | 1556035_s_at | AI201248 | NM_003457 | 7756 | -0.69 | 3.42E-03 |
| TM4SF2 | 202242_at | NM_004615 | NM_004615 | 7102 | -0.69 | 2.98E-03 |
| AGRN | 212285_s_at | AW008051 | NM_198576 | 375790 | -0.69 | 4.04E-03 |
| AF116695 | 224254_x_at | AF116695 | --- | --- | -0.69 | 4.32E-03 |
| FLJ20758 | 228020_at | BG055431 | NM_017952 | 55037 | -0.69 | 3.97E-05 |
| MYLIP | 223130_s_at | AF212221 | NM_013262 | 29116 | -0.69 | 8.41E-03 |
| PTPRD | 213362_at | N73931 | NM_002839 | 5789 | -0.69 | 4.67E-03 |
| | | | NM_ 130391 | | | |
| | | | NM_ 130392 | | | |
| | | | NM_ 130393 | | | |
| UNC5A | 236448_at | R37358 | XM_030300 | 90249 | -0.69 | 1.44E-05 |
| USP36 | 224979_s_at | AU154368 | NM_025090 | 57602 | -0.69 | 1.61E-03 |
| RBPMS2 | 228802_at | BE348466 | XM_496072 | 348093 | -0.69 | 1.05E-03 |
| SENP2 | 222523_at | BE622841 | NM_021627 | 59343 | -0.69 | 4.48E-03 |
| ATXN1 | 203231_s_at | AW235612 | NM_000332 | 6310 | -0.69 | 9.97E-03 |
| AA630955 | 242133_s_at | AA630955 | --- | --- | -0.69 | 1.82E-03 |
| LOC285458 | 242577_at | BF109197 | --- | 285458 | -0.69 | 9.98E-03 |
| ZNF573 | 217627_at | BE515346 | NM_ 152360 | 126231 | -0.69 | 5.61E-03 |
| APC | 216933_x_at | S67788 | NM_000038 | 324 | -0.69 | 1.03E-03 |
| WDFY3 | 212598_at | BE348236 | NM_014991 | 23001 | -0.69 | 1.37E-05 |
| | | | NM_178583 | | | |
| | | | NM_178585 | | | |
| FNBP1 | 244286_at | AI017983 | NM_015033 | 23048 | -0.69 | 3.21E-03 |
| DSCR3 | 203635_at | NM_006052 | NM_006052 | 10311 | -0.68 | 1.64E-05 |
| MONDOA | 202519_at | NM_014938 | NM_014938 | 22877 | -0.68 | 3.72E-04 |
| AI859990 | 244197_x_at | AI859990 | --- | --- | -0.68 | 1.47E-03 |
| C7orf19 | 1558426_x_at | BC016797 | NM_032831 | 80228 | -0.68 | 6.95E-04 |
| MGC20419 | 225642_at | AL581536 | NM_138417 | 112970 | -0.68 | 5.44E-03 |
| KIAA1644 | 221901_at | BF516072 | XM_376018 | 85352 | -0.68 | 3.43E-03 |
| AI146751 | 242565_x_at | AI146751 | --- | --- | -0.68 | 1.09E-03 |
| PHF17 | 225816_at | AW138134 | NM_024900 NM_199320 | 79960 | -0.68 | 8.15E-04 |
| NFASC | 213438_at | AA995925 | NM_00100538 7 | 23114 | -0.68 | 1.25E-03 |
| | | | NM_00100538 8 | | | |
| | | | NM_00100538 9 | | | |
| | | | NM_015090 | | | |
| AI332346 | 225445_at | AI332346 | --- | --- | -0.68 | 4.20E-03 |
| PEX6 | 238672_at | AW953952 | NM_000287 | 5190 | -0.68 | 1.21E-05 |
| PALM2-AKAP2 | 226694_at | BG540494 | NM_007203 NM_147150 | 445815 | -0.68 | 3.43E-04 |
| AI821721 | 241223_x_at | AI821721 | --- | --- | -0.68 | 5.48E-04 |
| BF197705 | 230401_at | BF197705 | --- | --- | -0.68 | 7.84E-03 |
| BG527339 | 226457_at | BG527339 | --- | --- | -0.68 | 2.91E-04 |
| LOC146174 | 230721_at | BF436957 | NM_173501 | 146174 | -0.68 | 1.72E-03 |
| C1orf21 | 223127_s_at | AW003850 | NM_030806 | 81563 | -0.68 | 6.33E-04 |
| FBX031 | 222352_at | BE465371 | NM_024735 | 79791 | -0.68 | 1.00E-04 |
| TAF15 | 227891_s_at | AU144462 | NM_003487 | 8148 | -0.68 | 9.77E-03 |
| | | | NM_139215 | | | |
| ZNF207 | 231848_x_at | AW192569 | NM_003457 | 7756 | -0.68 | 1.45E-03 |
| ZNF37A | 228711_at | BF059259 | NM_00100709 4 | 7587 | -0.68 | 5.10E-04 |
| | | | NM_003421 | | | |
| ATP11A | 1556055_at | U90905 | NM_015205 NM_032189 | 23250 | -0.68 | 4.26E-03 |
| TIM14 | 225358_at | AI003794 | NM_145261 | 131118 | -0.68 | 2.38E-03 |
| | | | NM_201259 | | | |
| | | | NM_201260 | | | |
| | | | NM_201261 | | | |
| COL5A1 | 212489_at | AI983428 | NM_000093 | 1289 | -0.68 | 8.47E-03 |
| MGC35274 | 226748_at | AI674731 | NM_153374 | 256586 | -0.68 | 1.32E-05 |
| ST7L | 238512_at | BF961733 | NM_017744 | 54879 | -0.67 | 2.66E-03 |
| | | | NM_138727 | | | |
| | | | NM_138728 | | | |
| | | | NM_138729 | | | |
| | | | NM_198327 | | | |
| | | | NM_198328 | | | |
| HUMPPA | 213230_at | AI422335 | NM_014603 | 30850 | -0.67 | 7.14E-03 |
| Cep164 | 204251_s_at | NM_014956 | NM_014956 | 22897 | -0.67 | 5.51E-03 |
| C14orf159 | 218298_s_at | NM_024952 | NM_024952 | 80017 | -0.67 | 2.50E-03 |
| MCTP1 | 235740_at | BG250585 | NM_00100279 6 | 79772 | -0.67 | 3.10E-03 |
| | | | NM_024717 | | | |
| ZNF451 | 215012_at | AU144775 | NM_015555 | 26036 | -0.67 | 3.81E-03 |
| MAPT | 225379_at | AA199717 | NM_005910 | 4137 | -0.67 | 6.84E-04 |
| | | | NM_016834 | | | |
| | | | NM_016835 | | | |
| | | | NM_016841 | | | |
| SMURF2 | 241900_at | AW195928 | NM_022739 | 64750 | -0.67 | 7.08E-03 |
| FOXP1 | 235444_at | AI417897 | NM_032682 | 27086 | -0.67 | 5.32E-04 |
| ZBED3 | 228402_at | AI679968 | NM_032367 | 84327 | -0.67 | 3.66E-03 |
| ABCC8 | 210246_s_at | AF087138 | NM_000352 | 6833 | -0.67 | 5.10E-03 |
| FLJ11016 | 219754_at | NM_018301 | NM_018301 | 55285 | -0.67 | 4.87E-03 |
| AI344332 | 227227_at | AI344332 | XM_373737 | 388397 | -0.67 | 6.03E-05 |
| HIS1 | 202814_s_at | AW193511 | NM_006460 | 10614 | -0.67 | 5.23E-04 |
| LOC401394 | 217529_at | BE547674 | XM_379512 | 401394 | -0.67 | 1.35E-04 |
| TMEM34 | 219074_at | NM_018241 | NM_018241 | 55751 | -0.67 | 2.81E-03 |
| PPID | 204185_x_at | NM_005038 | NM_005038 | 5481 | -0.67 | 2.92E-03 |
| SFRS21P | 232597_x_at | AK025132 | NM_004719 | 9169 | -0.67 | 3.06E-03 |
| SLC35E1 | 227518_at | AW051365 | NM_024881 | 79939 | -0.67 | 7.53E-03 |
| C14orf118 | 229514_at | AB032978 | NM_017926 | 55668 | -0.67 | 8.14E-04 |
| | | | NM_017972 | | | |
| KIAA0913 | 212359_s_at | W89120 | NM_015037 | 23053 | -0.67 | 7.72E-03 |
| C9orf85 | 238912_x_at | AA824363 | NM_182505 | 138241 | -0.67 | 1.86E-03 |
| | | | NM_198394 | | | |
| CaMKIINalpha | 218309_at | NM_018584 | NM_018584 | 55450 | -0.67 | 1.94E-07 |
| TAF1 | 227205_at | AW575233 | NM_004606 | 6872 | -0.67 | 3.47E-03 |
| | | | NM_138923 | | | |
| LOC144363 | 225469_at | AA015609 | NM_00100166C | 144363 | -0.66 | 4.42E-03 |
| TNKS | 233152_x_at | AL049979 | NM_003747 | 8658 | -0.66 | 2.23E-03 |
| C1orf52 | 228135_at | AA738437 | NM_198077 | 148423 | -0.66 | 3.10E-03 |
| BG502771 | 226397_s_at | BG502771 | --- | --- | -0.66 | 6.19E-05 |
| ELMO1 | 204513_s_at | NM_014800 | NM_014800 | 9844 | -0.66 | 6.47E-03 |
| | | | NM_130442 | | | |
| AI627803 | 236194_at | AI627803 | --- | --- | -0.66 | 2.79E-03 |
| AA604601 | 1570566_at | AA604601 | --- | --- | -0.66 | 1.29E-04 |
| RAMP1 | 204916_at | NM_005855 | NM_005855 | 10267 | -0.66 | 2.24E-06 |
| AL137655 | 231886_at | AL137655 | XM_373814 | 388572 | -0.66 | 1.31E-04 |
| PCDHB16 | 232099_at | AB046841 | | 57717 | -0.66 | 4.23E-03 |
| | | | NM_020957 | | | |
| KIAA0804 | 209553_at | BC001001 | NM_00100992 1 | 23355 | -0.66 | 2.61E-03 |
| | | | NM_015303 | | | |
| N63920 | 226179_at | N63920 | --- | --- | -0.66 | 3.55E-04 |
| MCPH1 | 219592_at | NM_024596 | NM_024596 | 79648 | -0.66 | 3.60E-03 |
| MLLT6 | 224784_at | BG024886 | NM_005937 | 4302 | -0.66 | 6.68E-04 |
| ARRDC1 | 226405_s_at | AK001822 | NM_152285 | 92714 | -0.66 | 2.73E-03 |
| RAB11FIP4 | 224482_s_at | BC006240 | NM_032932 | 84440 | -0.66 | 3.11E-03 |
| EFNA1 | 202023_at | NM_004428 | NM_004428 NM_182685 | 1942 | -0.66 | 2.72E-03 |
| C18orf17 | 1552307_a_at | NM_153211 | NM_153211 | 125488 | -0.66 | 6.53E-03 |
| MBTD1 | 207115_x_at | NM_017643 | NM_017643 | 54799 | -0.66 | 6.47E-03 |
| ALG3 | 207396_s_at | NM_005787 | NM_00100694 0 | 10195 | -0.66 | 8.88E-04 |
| | | | NM_00100694 1 | | | |
| | | | NM_00100694 2 | | | |
| | | | NM_005787 | | | |
| IFNAR1 | 225661_at | AA811138 | NM_000629 | 3454 | -0.66 | 2.66E-03 |
| FER1L4 | 222245_s_at | AF218012 | --- | 80307 | -0.66 | 3.17E-03 |
| DDX50 | 1568815_a_at | AA903184 | NM_024045 | 79009 | -0.66 | 4.02E-03 |
| ELK1 | 210376_x_at | M25269 | NM_005229 | 2002 | -0.66 | 8.45E-03 |
| FBX031 | 224162_s_at | BC002985 | NM_024735 | 79791 | -0.66 | 3.15E-03 |
| RNF13 | 201779_s_at | AF070558 | NM_007282 | 11342 | -0.66 | 1.55E-03 |
| | | | NM_183381 | | | |
| | | | NM_183382 | | | |
| | | | NM_183383 | | | |
| | | | NM_183384 | | | |
| LOC440345 | 238449_at | BG534511 | XM_496125 | 440345 | -0.66 | 1.28E-03 |
| CXXC5 | 224516_s_at | BC006428 | NM_016463 | 51523 | -0.66 | 1.05E-05 |
| MKNK2 | 223199_at | AA404592 | NM_017572 | 2872 | -0.66 | 2.26E-03 |
| | | | NM_199054 | | | |
| NFYC | 202215_s_at | NM_014223 | NM_014223 | 4802 | -0.66 | 4.41E-04 |
| RAB12 | 236019_at | AI076335 | XM_113967 | 201475 | -0.66 | 1.77E-03 |
| ETFDH | 205530_at | NM_004453 | NM_004453 | 2110 | -0.66 | 1.11E-03 |
| LOC153364 | 230298_at | AI692906 | NM_203406 | 153364 | -0.66 | 1.11E-04 |
| ELMO3 | 219411_at | NM_024712 | NM_024712 | 79767 | -0.66 | 2.82E-03 |
| SERTAD4 | 229674_at | AL035414 | NM_019605 | 56256 | -0.66 | 3.57E-04 |
| GPRASP1 | 204793_at | NM_014710 | NM_014710 | 9737 | -0.65 | 1.35E-03 |
| SMAD4 | 1565703_at | AL832789 | NM_005359 | 4089 | -0.65 | 4.45E-03 |
| ATP9A | 216129_at | AL117659 | XM_030577 | 10079 | -0.65 | 3.73E-03 |
| MGC21688 | 232806_s_at | AU158601 | NM_144635 | 131408 | -0.65 | 3.23E-03 |
| LOC90410 | 210312_s_at | BC002640 | NM_174887 | 90410 | -0.65 | 1.34E-04 |
| BE348430 | 230235_at | BE348430 | --- | --- | -0.65 | 1.62E-04 |
| UBE2G2 | 1557053_s_at | BC035653 | NM_003343 | 7327 | -0.65 | 1.15E-03 |
| | | | NM_182688 | | | |
| TRA2A | 229574_at | AI268231 | NM_013293 | 29896 | -0.65 | 1.49E-03 |
| MGC42174 | 238602_at | AL036541 | NM_152383 | 129563 | -0.65 | 9.27E-03 |
| QSCN6 | 201482_at | NM_002826 | NM_00100412 8 | 5768 | -0.65 | 6.49E-03 |
| | | | NM_002826 | | | |
| C15orf24 | 217898_at | NM_020154 | NM_020154 | 56851 | -0.65 | 6.93E-03 |
| SLCO1A2 | 211480_s_at | AF085224 | NM_005075 | 6579 | -0.65 | 3.46E-04 |
| | | | NM_021094 | | | |
| | | | NM_134431 | | | |
| ATP2C1 | 230387_at | AL038450 | NM_00100148 5 | 27032 | -0.65 | 6.34E-03 |
| | | | NM_00100148 6 | | | |
| | | | NM_00100148 7 | | | |
| | | | NM_014382 | | | |
| AF5Q31 | 232864_s_at | N59653 | NM_014423 | 27125 | -0.65 | 1.51E-03 |
| AL049975 | 234020_x_at | AL049975 | --- | --- | -0.64 | 1.81E-03 |
| AI634543 | 242208_at | AI634543 | --- | --- | -0.64 | 1.23E-03 |
| AI695743 | 244534_at | AI695743 | --- | --- | -0.64 | 9.29E-03 |
| CCNDBP1 | 223084_s_at | AF246144 | NM_012142 | 23582 | -0.64 | 1.19E-03 |
| | | | NM_037370 | | | |
| ZBED3 | 235109_at | AI887983 | NM_032367 | 84327 | -0.64 | 4.83E-03 |
| BACE1 | 222462_s_at | AI653425 | NM_012104 | 23621 | -0.64 | 2.24E-05 |
| | | | NM_138971 | | | |
| | | | NM_138972 | | | |
| | | | NM_138973 | | | |
| SERTAD4 | 235337_at | AI276403 | NM_019605 | 56256 | -0.64 | 9.08E-04 |
| ZNF596 | 240324_at | H50654 | NM_173539 | 169270 | -0.64 | 6.01E-04 |
| AA845825 | 217534_at | AA845825 | --- | --- | -0.64 | 1.19E-03 |
| BE551142 | 241091_at | BE551142 | XM_497668 | 388620 | -0.64 | 2.87E-03 |
| KIAA1181 | 224576_at | AK000752 | NM_020462 | 57222 | -0.64 | 9.10E-03 |
| JMJD1C | 221763_at | AI694023 | NM_004241 | 221037 | -0.64 | 1.29E-04 |
| PPP2R5B | 635_s_at | L42374 | NM_006244 | 5526 | -0.64 | 7.89E-04 |
| FLJ31438 | 228316_at | AA905470 | NM_152385 | 130162 | -0.64 | 3.25E-05 |
| AI524240 | 238863_x_at | AI524240 | --- | --- | -0.64 | 3.04E-05 |
| DJ971N18.2 | 240094_at | AL042660 | NM_021156 | 56255 | -0.64 | 7.59E-03 |
| TTC3 | 208661_s_at | AW510696 | NM_00100189 4 | 7267 | -0.64 | 1.95E-04 |
| | | | NM_003316 | | | |
| JMJD3 | 41386_i_at | AB002344 | XM_043272 | 23135 | -0.64 | 6.66E-03 |
| SEC6L1 | 228847_at | W69265 | NM_007277 | 11336 | -0.64 | 8.93E-03 |
| C21orf106 | 227199_at | AW027812 | NM_015151 | 23181 | -0.64 | 1.63E-03 |
| | | | NM_206889 | | | |
| | | | NM_206890 | | | |
| | | | NM_206891 | | | |
| KIAA1919 | 242851_at | BE964989 | NM_153369 | 91749 | -0.64 | 1.22E-03 |
| AI683552 | 217679_x_at | AI683552 | --- | --- | -0.64 | 3.76E-03 |
| ICA1 | 207949_s_at | NM_004968 | NM_004968 | 3382 | -0.64 | 3.58E-06 |
| | | | NM_022307 | | | |
| | | | NM_022308 | | | |
| AI885665 | 238360_s_at | AI885665 | --- | --- | -0.64 | 3.47E-04 |
| AW511239 | 229765_at | AW511239 | --- | --- | -0.64 | 7.38E-03 |
| BI496583 | 1557675_at | BI496583 | --- | --- | -0.63 | 3.19E-03 |
| LOC253982 | 214993_at | AF070642 | NM_181718 | 253982 | -0.63 | 1.41E-04 |
| | | | NM_198907 | | | |
| ASB13 | 218862_at | NM_024701 | NM_024701 | 79754 | -0.63 | 2.48E-03 |
| C18orf23 | 230143_at | BF433220 | NM_152470 | 147341 | -0.63 | 3.83E-03 |
| WDFY3 | 212602_at | AI806395 | NM_014991 | 23001 | -0.63 | 3.16E-04 |
| | | | NM_178583 | | | |
| | | | NM_178585 | | | |
| TMEM5 | 204808_s_at | NM_014254 | NM_014254 | 10329 | -0.63 | 4.54E-05 |
| SPTBN1 | 200671_s_at | N92501 | NM_003128 | 6711 | -0.63 | 4.18E-04 |
| | | | NM_178313 | | | |
| AI216567 | 236754_at | AI216567 | --- | --- | -0.63 | 2.90E-03 |
| PTPRN2 | 203030_s_at | AF007555 | NM_002847 | 5799 | -0.63 | 7.43E-03 |
| | | | NM_130842 | | | |
| | | | NM_130843 | | | |
| M17S2 | 1568857_a_at | BC012591 | NM_005899 | 4077 | -0.63 | 6.88E-03 |
| | | | NM_031858 | | | |
| | | | NM_031862 | | | |
| C6orf170 | 1553726_s_at | NM_152730 | NM_152730 | 221322 | -0.63 | 2.53E-04 |
| SON | 201085_s_at | AA664291 | NM_003103 | 6651 | -0.63 | 2.20E-03 |
| | | | NM_032195 | | | |
| | | | NM_058183 | | | |
| | | | NM_138925 | | | |
| | | | NM_138926 | | | |
| | | | NM_138927 | | | |
| AA102332 | 240172_at | AA102332 | --- | --- | -0.63 | 6.39E-03 |
| MGC15416 | 225861_at | AW001250 | NM_032371 | 84331 | -0.63 | 4.24E-04 |
| | | | NM_138418 | | | |
| AK024602 | 222207_x_at | AK024602 | --- | --- | -0.63 | 1.29E-03 |
| AW945538 | 239208_s_at | AW945538 | --- | --- | -0.63 | 2.53E-03 |
| AI140364 | 221740_x_at | AI140364 | XM_373737 | 388397 | -0.63 | 1.10E-04 |
| ARHGEF7 | 1562271_x_at | AL831814 | NM_003899 | 8874 | -0.63 | 5.76E-03 |
| | | | NM_145735 | | | |
| AG1 | 215434_x_at | AV684285 | XM_371384 | 440673 | -0.63 | 1.46E-03 |
| | | | XM_496394 | | | |
| BZRAP1 | 205839_s_at | NM_004758 | NM_004758 | 9256 | -0.63 | 3.66E-03 |
| C14orf149 | 227699_at | BF511003 | NM_144581 | 112849 | -0.63 | 5.42E-03 |
| U2AF1 | 232141_at | AU144161 | NM_006758 | 7307 | -0.63 | 2.12E-03 |
| CAMK2B | 213276_at | T15766 | NM_001220 | 816 | -0.63 | 7.05E-03 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| NRD1 | 229422 at | AA448346 | NM_002525 | 4898 | -0.63 | 5.72E-03 |
| C10orf18 | 244165_at | AI809511 | XM_374765 | 54906 | -0.62 | 9.92E-03 |
| TMPIT | 234084_x_at | AU147104 | NM_031925 | 83862 | -0.62 | 7.32E-04 |
| AI334297 | 225961_at | AI334297 | --- | --- | -0.62 | 9.96E-03 |
| MAPT | 203929_s_at | AI056359 | NM_005910 | 4137 | -0.62 | 6.17E-04 |
| | | | NM_016834 | | | |
| | | | NM_016835 | | | |
| | | | NM_016841 | | | |
| XRN1 | 242995_at | AW976347 | NM_019001 | 54464 | -0.62 | 5.64E-03 |
| NFIC | 226377_at | A1093722 | NM_005597 | 4782 | -0.62 | 1.82E-03 |
| | | | NM_205843 | | | |
| FBXO9 | 212991_at | AL137520 | NM_012347 | 26268 | -0.62 | 1.09E-05 |
| | | | NM_033480 | | | |
| | | | NM_033481 | | | |
| OSBPL6 | 223805_at | AF323728 | NM_032523 | 114880 | -0.62 | 1.05E-04 |
| | | | NM_145739 | | | |
| NRD1 | 242235_x_at | AW978721 | NM_002525 | 4898 | -0.62 | 6.13E-04 |
| ATXN7L1 | 227732_at | AB033044 | --- | 57485 | -0.62 | 5.06E-03 |
| PDPK1 | 32029_at | AC005591 | NM_002613 | 5170 | -0.62 | 8.86E-03 |
| | | | NM_031268 | | | |
| BF110411 | 221878_at | BF110411 | XM_371506 | 388969 | -0.62 | 4.02E-03 |
| SNX1 | 1559154_at | AK098001 | NM_003099 | 6642 | -0.62 | 2.16E-03 |
| | | | NM_148955 | | | |
| | | | NM_152826 | | | |
| LOC282965 | 230108_at | BF433475 | --- | 282965 | -0.62 | 7.42E-03 |
| AA045233 | 224686_x_at | AA045233 | XM_373737 | 388397 | -0.62 | 1.42E-04 |
| TIMM9 | 218316_at | NM_012460 | NM_012460 | 26520 | -0.62 | 9.93E-03 |
| PTPRN2 | 203029_s_at | NM_002847 | NM_002847 | 5799 | -0.62 | 1.78E-04 |
| | | | NM_130842 | | | |
| | | | NM_130843 | | | |
| MGC52110 | 226039_at | AW006441 | NM_00100821E | 493753 | -0.62 | 2.08E-04 |
| SCN5A | 207413_s_at | NM_000335 | NM_000335 | 6331 | -0.62 | 2.09E-03 |
| | | | NM_198056 | | | |
| ATF7 | 244473_at | R37637 | NM_006856 | 11016 | -0.62 | 6.77E-04 |
| AL162054 | 1559420_x_at | AL162054 | --- | --- | -0.62 | 2.91E-03 |
| INHA | 1569409_x_at | BC037812 | NM_002191 | 3623 | -0.62 | 9.67E-03 |
| CTTN | 214782_at | AU155105 | NM_005231 | 2017 | -0.62 | 3.85E-03 |
| | | | NM_138565 | | | |
| FLJ20758 | 228590_at | AA045257 | NM_017952 | 55037 | -0.62 | 1.29E-06 |
| DTX3 | 221835_at | N92708 | NM_178502 | 196403 | -0.61 | 6.82E-03 |
| AFG3L2 | 232919_at | AU147380 | NM_006796 | 10939 | -0.61 | 7.83E-03 |
| NR2F1 | 209505_at | AI951185 | NM_005654 | 7025 | -0.61 | 1.18E-04 |
| MYLIP | 228098_s_at | AW292746 | NM_013262 | 29116 | -0.61 | 9.86E-05 |
| FLJ00312 | 221971_x_at | BE672818 | XM_374801 | 399761 | -0.61 | 2.00E-05 |
| STAT3 | 235680_at | AI914925 | NM_003150 | 6774 | -0.61 | 7.44E-04 |
| | | | NM_139276 | | | |
| | | | NM_213662 | | | |
| PALM2-AKAP2 | 202759_s_at | BE879367 | NM_007203 | 445815 | -0.61 | 1.06E-05 |
| | | | NM_147150 | | | |
| AI692645 | 230206_at | AI692645 | --- | --- | -0.61 | 7.26E-04 |
| PPP3CA | 202457 _s_at | AA911231 | NM_000944 | 5530 | -0.61 | 1.76E-04 |
| POM121 | 212178_s_at | AK022555 | NM_172020 | 340318 | -0.61 | 6.29E-04 |
| LOC340318 | | | XM_290401 | 9883 | | |
| | | | XM_379877 | | | |
| AK024255 | 232569_at | AK024255 | --- | --- | -0.61 | 5.98E-03 |
| TRIM8 | 223132_s_at | AF220034 | NM_030912 | 81603 | -0.61 | 5.53E-05 |
| PDLIM4 | 211564_s_at | BC003096 | NM_003687 | 8572 | -0.61 | 4.83E-04 |
| SHFM3 | 221519_at | AF281859 | NM_022039 | 6468 | -0.61 | 8.52E-06 |
| RIC3 | 220282_at | NM_024557 | NM_024557 | 79608 | -0.61 | 3.26E-03 |
| AK024881 | 234723_x_at | AK024881 | --- | --- | -0.61 | 7.62E-03 |
| DKFZP434H132 | 204494_s_at | AW516789 | NM_015492 | 56905 | -0.61 | 1.22E-04 |
| SMA3 | 206565_x_at | NM_006780 | NM_006780 | 10571 | -0.61 | 8.06E-03 |
| C21orf108 | 212996_s_at | AI803485 | --- | 9875 | -0.61 | 8.23E-04 |
| MAP1LC3B | 208785_s_at | BE893893 | NM_022818 | 81631 | -0.61 | 2.42E-03 |
| PRKCZ | 202178_at | NM_002744 | NM_002744 | 5590 | -0.61 | 2.33E-03 |
| BICD1 | 214806_at | U90030 | NM_00100339 8 | 636 | -0.61 | 1.56E-03 |
| | | | NM_001714 | | | |
| MAP3K12 | 205447_s_at | BE222201 | NM_006301 | 7786 | -0.61 | 3.86E-03 |
| PSIP1 | 232363_at | AK024516 | NM_021144 | 11168 | -0.61 | 2.71E-03 |
| | | | NM_033222 | | | |
| HPCAL1 | 205462_s_at | NM_002149 | NM_002149 | 3241 | -0.61 | 1.40E-05 |
| | | | NM_134421 | | | |
| PALM2-AKAP2 | 202760_s_at | NM_007203 | NM_007203 | 445815 | -0.61 | 4.74E-03 |
| | | | NM_147150 | | | |
| LOC282997 | 222307_at | AI695595 | --- | 282997 | -0.61 | 8.47E-03 |
| BF339133 | 213296_at | BF339133 | --- | --- | -0.60 | 3.76E-03 |
| AI791303 | 238890_at | AI791303 | --- | --- | -0.60 | 2.71E-03 |
| KIAA1881 | 207730_x_at | NM_017932 | XM_375558 | | -0.60 | 1.50E-03 |
| AL355532 | 213845_at | AL355532 | --- | | -0.60 | 1.88E-04 |
| ELOVL5 | 214153_at | BE467941 | NM_021814 | | -0.60 | 2.39E-03 |
| FLJ38991 | 227442_at | BG283902 | NM_173827 | | -0.60 | 9.08E-05 |
| C3orf6 | 226713_at | AI247881 | NM_174908 | | -0.60 | 6.70E-03 |
| | | | NM_178335 | | | |
| FNTA | 230808_at | AA833870 | NM_002027 | | -0.60 | 3.58E-03 |
| MGC54289 | 225228_at | AI474054 | NM_178454 | | -0.60 | 6.20E-03 |
| KIAA1536 | 209002_s_at | BC003177 | NM_020898 | | -0.60 | 9.44E-03 |
| CDK5R1 | 204995_at | AL567411 | NM_003885 | | -0.60 | 4.65E-04 |
| SFXN3 | 1559993_at | AK091504 | NM_030971 | | -0.60 | 4.27E-03 |
| AU157441 | 226511_at | AU157441 | --- | | -0.60 | 3.90E-03 |
| FLJ23861 | 231252_at | AI680874 | NM_152519 | 151050 | -0.60 | 5.09E-03 |
| BE219067 | 243641_at | BE219067 | --- | --- | -0.60 | 2.03E-06 |
| BC042959 | 1556194_a_at | BC042959 | --- | --- | -0.60 | 5.58E-03 |
| PFAAP5 | 221899_at | AI809961 | NM_014887 | 10443 | -0.60 | 8.51E-03 |
| PPP2R2C | 223573_s_at | AW166283 | NM_020416 NM_181876 | 5522 | -0.60 | 4.24E-03 |
| CLASP2 | 212309_at | AV725315 | NM_015097 | 23122 | -0.60 | 3.17E-04 |
| INSR | 226450_at | AV703054 | NM_000208 | 3643 | -0.60 | 8.36E-06 |
| USP34 | 207365_x_at | NM_014709 | NM_014709 | 9736 | -0.60 | 8.70E-03 |
| RUNX1 | 236114_at | AI798118 | NM_00100189 0 | 861 | -0.59 | 5.87E-03 |
| | | | NM_001754 | | | |
| VPS13C | 218396_at | NM_017684 | NM_017684 | 54832 | -0.59 | 6.96E-03 |
| KPNA1 | 202057_at | BC002374 | NM_002264 | 3836 | -0.59 | 3.30E-04 |
| RPS6KL1 | 223534_s_at | BC004540 | NM_031464 | 83694 | -0.59 | 6.25E-04 |
| CNNM3 | 240339_at | AA829860 | NM_017623 | 26505 | -0.59 | 2.25E-03 |
| | | | NM_199078 | | | |
| AK022428 | 234731_at | AK022428 | --- | --- | -0.59 | 3.34E-04 |
| NT5C3 | 223298_s_at | AF312735 | NM_016489 | 51251 | -0.59 | 8.53E-04 |
| JMJD3 | 213146_at | AA521267 | XM_043272 | 23135 | -0.59 | 7.42E-03 |
| NM_018289 | 219794_at | NM_018289 | --- | --- | -0.59 | 4.18E-07 |
| FCMD | 205283_at | NM_006731 | NM_006731 | 2218 | -0.59 | 4.33E-03 |
| C18orf17 | 1570552_at | AF363068 | NM_153211 | 125488 | -0.59 | 2.11E-03 |
| FLJ23451 | 1569601_at | BF106979 | NM_024766 | 79823 | -0.59 | 1.13E-03 |
| BF514509 | 239050_s_at | BF514509 | --- | --- | -0.59 | 1.54E-03 |
| LOC442647 | 229157_at | AI949265 | XM_499544 | 442647 | -0.59 | 2.37E-03 |
| SLC24A5 | 228792_at | AU145100 | NM_205850 | 283652 | -0.59 | 1.36E-03 |
| AI796076 | 243339_at | AI796076 | --- | --- | -0.59 | 2.87E-03 |
| BE857570 | 235677_at | BE857570 | --- | --- | -0.59 | 2.57E-03 |
| RALGDS | 209051_s_at | AF295773 | NM_006266 | 5900 | -0.59 | 8.43E-03 |
| SND1 | 242506_at | AI138785 | NM_014390 | 27044 | -0.59 | 1.79E-03 |
| BM914560 | 1558220_at | BM914560 | XM_496669 | 440993 | -0.58 | 6.65E-03 |
| SEZ6L2 | 218720_x_at | NM_012410 | NM_012410 | 26470 | -0.58 | 3.65E-03 |
| | | | NM_201575 | | | |
| AW294192 | 228040_at | AW294192 | --- | --- | -0.58 | 3.57E-04 |
| PCNT2 | 233387_s_at | AK024009 | NM_006031 | 5116 | -0.58 | 5.43E-03 |
| CHST5 | 219182_at | NM_024533 | NM_012126 | 23563 | -0.58 | 9.75E-03 |
| | | | NM_024533 | | | |
| MGC15416 | 1553715_s_at | NM_032371 | NM_032371 | 84331 | -0.58 | 4.55E-03 |
| | | | NM_138418 | | | |
| ABAT | 209460_at | AF237813 | NM_000663 | 18 | -0.58 | 5.91E-03 |
| | | | NM_020686 | | | |
| C9orf112 | 225586_at | BF222775 | NM_138778 | 92715 | -0.58 | 3.08E-03 |
| DSCR3 | 217309_s_at | AJ001867 | NM_006052 | 10311 | -0.58 | 6.91E-03 |
| IER2 | 202081_at | NM_004907 | NM_004907 | 9592 | -0.58 | 6.30E-03 |
| MGC4796 | 223852_s_at | BC005169 | NM_032017 | 83931 | -0.58 | 7.87E-03 |
| CD151 | 204306_s_at | NM_004357 | NM_004357 | 977 | -0.58 | 1.98E-03 |
| | | | NM_139030 | | | |
| MACF1 | 208633_s_at | W61052 | NM_012090 | 23499 | -0.58 | 1.19E-03 |
| | | | NM_033044 | | | |
| MGC16279 | 65585_at | AA527515 | NM_032916 | 85002 | -0.58 | 2.75E-03 |
| ZBTB4 | 225629_s_at | AI669498 | NM_020899 | 57659 | -0.58 | 4.70E-05 |
| GRIPAP1 | 1557562_at | AI984118 | NM_020137 | 56850 | -0.58 | 3.18E-03 |
| | | | NM_207672 | | | |
| AU145662 | 233399_x_at | AU145662 | XM_499131 | 441383 | -0.58 | 1.90E-03 |
| SYNGAP1 | 230297_x_at | AW002361 | NM_006772 | 8831 | -0.58 | 5.57E-03 |
| MGC15407 | 224968_at | AL518311 | NM_080667 | 112942 | -0.58 | 3.50E-03 |

**Table 7. Increased Gene Expression in Cell Lines H1 and BB10 vs. Cell Line 2D6 in Differentiated State Using Microarray Analysis**

| **Gene Symbol** | **Probe Set ID** | **Representative Public ID** | **RefSeq Transcript ID** | **Entrez Gene** | **Log2 Ratio** | **pvalue** |
|---|---|---|---|---|---|---|
| PLS3 | 201215_at | NM_005032 | NM_005032 | 5358 | 6.46 | 2.18E-11 |
| PEG3 | 209242_at | AL042588 | NM_006210 | 5178 | 6.29 | 4.38E-07 |
| FLJ32942 | 227711_at | BG150433 | NM_144594 | 121355 | 6.29 | 3.26E-08 |
| MSN | 200600_at | NM_002444 | NM_002444 | 4478 | 6.24 | 7.65E-10 |
| DKFZp667G211 | 214030_at | BE501352 | XM_376254 | 131544 | 6.06 | 3.72E-05 |
| MCM10 | 220651_s_at | NM_018518 | NM_018518 | 55388 | 5.94 | 2.90E-06 |
| | | | NM_182751 | | | |
| TPTE | 220205_at | NM_013315 | NM_013315 | 7179 | 5.89 | 2.90E-07 |
| | | | NM_199259 | | | |
| | | | NM_199260 | | | |
| | | | NM_199261 | | | |
| DLG7 | 203764_at | NM_014750 | NM_014750 | 9787 | 5.79 | 1.58E-05 |
| PLK2 | 201939_at | NM_006622 | NM_006622 | 10769 | 5.72 | 1.74E-08 |
| HMMR | 209709_s_at | U29343 | NM_012484 | 3161 | 5.64 | 2.12E-07 |
| | | | NM_012485 | | | |
| BUB1 | 209642_at | AF043294 | NM_004336 | 699 | 5.53 | 9.46E-06 |
| RGS5 | 1555725_a_at | AF493929 | NM_003617 | 8490 | 5.49 | 1.14E-04 |
| NDN | 209550_at | U35139 | NM_002487 | 4692 | 5.42 | 1.66E-05 |
| FLJ34013 | 225710_at | H99792 | --- | --- | 5.39 | 6.18E-08 |
| FAT | 201579_at | NM_005245 | NM_005245 | 2195 | 5.36 | 1.20E-03 |
| RGS5 | 218353_at | NM_025226 | NM_003617 | 8490 | 5.29 | 4.83E-04 |
| KCTD12 | 212192_at | AI718937 | NM_138444 | 115207 | 5.28 | 1.26E-06 |
| TFPI2 | 209278_s_at | L27624 | NM_006528 | 7980 | 5.28 | 1.22E-05 |
| AI813331 | 229097_at | AI813331 | --- | --- | 5.23 | 3.44E-07 |
| GNG11 | 204115_at | NM_004126 | NM_004126 | 2791 | 5.21 | 7.49E-06 |
| AI197932 | 229498_at | AI197932 | --- | --- | 5.17 | 1.33E-07 |
| RGS5 | 209070_s_at | AI183997 | NM_003617 | 8490 | 5.14 | 3.20E-04 |
| MKI67 | 212021_s_at | AU132185 | NM_002417 | 4288 | 5.04 | 2.78E-05 |
| PRSS12 | 205515_at | NM_003619 | NM_003619 | 8492 | 5.01 | 3.19E-07 |
| MCM10 | 222962_s_at | AB042719 | NM_018518 | 55388 | 5.01 | 2.63E-07 |
| | | | NM_182751 | | | |
| BIRC5 | 210334_x_at | AB028869 | NM_001168 | 332 | 5.00 | 5.51E-05 |
| RAMP | 222680_s_at | AK001261 | NM_016448 | 51514 | 5.00 | 2.74E-05 |
| ASF1B | 218115_at | NM_018154 | NM_018154 | 55723 | 4.97 | 3.08E-05 |
| MKI67 | 212023_s_at | AU147044 | NM_002417 | 4288 | 4.97 | 7.09E-06 |
| MKI67 | 212020_s_at | AU152107 | NM_002417 | 4288 | 4.97 | 3.98E-07 |
| ATAD2 | 218782_s_at | NM_014109 | NM_014109 | 29028 | 4.96 | 7.16E-07 |
| BIRC5 | 202094_at | AA648913 | NM_001168 | 332 | 4.96 | 3.70E-05 |
| FLJ40629 | 229610_at | AW088063 | NM_152515 | 150468 | 4.96 | 2.93E-05 |
| EXO1 | 204603_at | NM_003686 | NM_003686 | 9156 | 4.90 | 1.18E-05 |
| | | | NM_006027 | | | |
| | | | NM_130398 | | | |
| DEPDC1 | 222958_s_at | AK000490 | NM_017779 | 55635 | 4.89 | 2.51E-07 |
| RGS5 | 209071_s_at | AF159570 | NM_003617 | 8490 | 4.87 | 1.25E-04 |
| RRM2 | 209773_s_at | BC001886 | NM_001034 | 6241 | 4.86 | 4.36E-05 |
| KIF14 | 236641_at | AW183154 | XM_375825 | 9928 | 4.86 | 1.11E-05 |
| OIP5 | 213599_at | BE045993 | NM_007280 | 11339 | 4.85 | 5.98E-08 |
| C10orf3 | 218542_at | NM_018131 | NM_018131 | 55165 | 4.85 | 4.79E-06 |
| BRRN1 | 212949_at | D38553 | NM_015341 | 23397 | 4.85 | 2.87E-06 |
| FLJ10719 | 223785_at | BC004277 | NM_018193 | 55215 | 4.84 | 6.89E-06 |
| CHEK2 | 210416_s_at | BC004207 | NM_00100573 5 | 11200 | 4.81 | 1.17E-03 |
| | | | NM_007194 | | | |
| | | | NM_145862 | | | |
| N31731 | 230165_at | N31731 | --- | --- | 4.80 | 2.02E-07 |
| BRIP1 | 235609_at | BF056791 | NM_032043 | 83990 | 4.80 | 3.95E-05 |
| MELK | 204825_at | NM_014791 | NM_014791 | 9833 | 4.80 | 2.52E-04 |
| KIF20A | 218755_at | NM_005733 | NM_005733 | 10112 | 4.71 | 2.34E-05 |
| SPARC | 200665_s_at | NM_003118 | NM_003118 | 6678 | 4.71 | 6.23E-06 |
| BUB1 | 215509_s_at | AL137654 | NM_004336 | 699 | 4.69 | 2.73E-06 |
| BASP1 | 202391_at | NM_006317 | NM_006317 | 10409 | 4.68 | 5.50E-07 |
| SULT1C2 | 1553321_a_at | NM_006588 | NM_006588 | 27233 | 4.66 | 9.28E-06 |
| GTSE1 | 204317_at | BF305380 | NM_016426 | 51512 | 4.64 | 2.33E-08 |
| DAZ1 | 208282_x_at | NM_020363 | NM_00100537 5 | 1617 | 4.64 | 4.44E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| CDC45L | 204126_s_at | NM_003504 | NM_003504 | 8318 | 4.62 | 2.60E-04 |
| PRSS12 | 213802_at | AI810767 | NM_003619 | 8492 | 4.61 | 1.09E-05 |
| BRIP1 | 221703_at | AF360549 | NM_032043 | 83990 | 4.60 | 7.21E-06 |
| RNF182 | 230720_at | AI884906 | NM_152737 | 221687 | 4.59 | 1.15E-07 |
| SLC1A3 | 202800_at | NM_004172 | NM_004172 | 6507 | 4.55 | 1.78E-06 |
| DAZ1 | 207912_s_at | NM_004081 | NM_00100537 5 | 1617 | 4.55 | 1.51E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| GTSE1 | 204315_s_at | AI340239 | NM_016426 | 51512 | 4.54 | 9.21E-06 |
| NEIL3 | 219502_at | NM_018248 | NM_018248 | 55247 | 4.54 | 8.88E-06 |
| SPARC | 212667_at | AL575922 | NM_003118 | 6678 | 4.53 | 5.72E-05 |
| FN5 | 219806_s_at | NM_020179 | NM_020179 | 56935 | 4.53 | 2.58E-05 |
| CENPA | 204962_s_at | NM_001809 | NM_001809 | 1058 | 4.53 | 1.46E-06 |
| MYBL1 | 213906_at | AW592266 | XM_034274 | 4603 | 4.52 | 2.33E-05 |
| MAOA | 212741_at | AA923354 | NM_000240 | 4128 | 4.52 | 9.33E-07 |
| HMMR | 207165_at | NM_012485 | NM_012484 | 3161 | 4.51 | 5.91E-06 |
| | | | NM_012485 | | | |
| ZNF367 | 229551_x_at | N62196 | NM_153695 | 195828 | 4.50 | 2.65E-05 |
| NMU | 206023_at | NM_006681 | NM_006681 | 10874 | 4.50 | 1.75E-04 |
| CDCA8 | 221520_s_at | BC001651 | NM_018101 | 55143 | 4.49 | 1.67E-06 |
| FLJ10156 | 221591_s_at | BC005004 | NM_019013 | 54478 | 4.48 | 1.61E-06 |
| SHCBP1 | 219493_at | NM_024745 | NM_024745 | 79801 | 4.47 | 1.26E-05 |
| DIAPH3 | 232596_at | AL354829 | NM_030932 | 81624 | 4.46 | 1.53E-06 |
| DAZ1 | 207909_x_at | U21663 | NM_00100537 5 | 1617 | 4.46 | 3.63E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| FOXM1 | 202580_x_at | NM_021953 | NM_021953 | 2305 | 4.44 | 1.30E-04 |
| | | | NM_202002 | | | |
| | | | NM_202003 | | | |
| MAB21L2 | 210302_s_at | AF262032 | NM_006439 | 10586 | 4.43 | 9.15E-04 |
| KIF18A | 221258_s_at | NM_031217 | NM_031217 | 81930 | 4.42 | 2.20E-07 |
| STK6 | 204092_s_at | NM_003600 | NM_003600 | 6790 | 4.41 | 1.58E-06 |
| | | | NM_198433 | | | |
| | | | N M_198434 | | | |
| | | | NM_198435 | | | |
| | | | NM_198436 | | | |
| | | | NM_198437 | | | |
| ANLN | 1552619_a_at | NM_018685 | NM_018685 | 54443 | 4.41 | 6.79E-05 |
| MINA | 213188_s_at | AI823896 | NM_032778 | 84864 | 4.41 | 1.40E-04 |
| | | | NM_153182 | | | |
| CENPE | 205046_at | NM_001813 | NM_001813 | 1062 | 4.40 | 5.00E-07 |
| FLJ20641 | 227928_at | AI224977 | NM_017915 | 55010 | 4.40 | 5.48E-07 |
| CDC2 | 203214_x_at | NM_001786 | NM_001786 | 983 | 4.37 | 1.70E-06 |
| | | | NM_033379 | | | |
| KIF4A | 218355_at | NM_012310 | NM_012310 | 24137 | 4.36 | 5.88E-08 |
| CDCA2 | 226661_at | T90295 | NM_152562 | 157313 | 4.34 | 2.67E-07 |
| BRCA1 | 211851_x_at | AF005068 | NM_007294 | 672 | 4.34 | 1.52E-05 |
| | | | NM_007295 | | | |
| | | | NM_007296 | | | |
| | | | NM_007297 | | | |
| | | | NM_007298 | | | |
| | | | NM_007299 | | | |
| | | | NM_007300 | | | |
| | | | NM_007301 | | | |
| | | | NM_007302 | | | |
| | | | NM_007303 | | | |
| | | | NM_007304 | | | |
| | | | NM_007305 | | | |
| | | | NM_007306 | | | |
| DEPDC1 | 235545_at | AI810054 | NM_017779 | 55635 | 4.32 | 1.48E-07 |
| MINA | 213189_at | BE966695 | NM_032778 | 84864 | 4.31 | 1.18E-06 |
| | | | NM_153182 | | | |
| CDKN3 | 209714_s_at | AF213033 | NM_005192 | 1033 | 4.31 | 8.89E-05 |
| PLK4 | 204887_s_at | NM_014264 | NM_014264 | 10733 | 4.29 | 1.64E-04 |
| RRM2 | 201890_at | BE966236 | NM_001034 | 6241 | 4.28 | 5.37E-05 |
| RAD51 | 205024_s_at | NM_002875 | NM_002875 | 5888 | 4.27 | 1.40E-05 |
| | | | NM_133487 | | | |
| AURKB | 209464_at | AB011446 | NM_004217 | 9212 | 4.27 | 2.23E-05 |
| TTK | 204822_at | NM_003318 | NM_003318 | 7272 | 4.27 | 7.61E-06 |
| POLE2 | 205909_at | NM_002692 | NM_002692 | 5427 | 4.27 | 9.18E-04 |
| OSBPL3 | 209627_s_at | AY008372 | NM_015550 | 26031 | 4.26 | 1.75E-05 |
| | | | NM_145320 | | | |
| | | | NM_145321 | | | |
| | | | NM_145322 | | | |
| | | | NM_145323 | | | |
| | | | NM_145324 | | | |
| PEG3 | 209243_s_at | AF208967 | NM_006210 | 5178 | 4.26 | 3.95E-05 |
| KIF11 | 204444_at | NM_004523 | NM_004523 | 3832 | 4.25 | 4.91E-04 |
| Spc24 | 235572_at | AI469788 | NM_182513 | 147841 | 4.25 | 7.74E-09 |
| FGFR2 | 203639_s_at | M80634 | NM_000141 | 2263 | 4.24 | 9.58E-06 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| CDCA1 | 223381_at | AF326731 | NM_031423 | 83540 | 4.22 | 1.90E-05 |
| | | | NM_145697 | | | |
| ANLN | 222608_s_at | AK023208 | NM_018685 | 54443 | 4.22 | 2.01E-04 |
| MAB21L1 | 206163_at | NM_005584 | NM_005584 | 4081 | 4.21 | 2.65E-05 |
| CDC6 | 203967_at | U77949 | NM_001254 | 990 | 4.20 | 1.21E-03 |
| ESPL1 | 38158_at | D79987 | NM_012291 | 9700 | 4.17 | 1.15E-06 |
| MCM5 | 201755_at | NM_006739 | NM_006739 | 4174 | 4.16 | 4.33E-04 |
| MGC57827 | 225834_at | AL135396 | NM_207418 | 389835 | 4.16 | 6.28E-06 |
| STK6 | 208079_s_at | NM_003158 | NM_003600 | 6790 | 4.16 | 1.80E-05 |
| | | | NM_198433 | | | |
| | | | NM_198434 | | | |
| | | | NM_198435 | | | |
| | | | NM_198436 | | | |
| | | | NM_198437 | | | |
| SLD5 | 211767_at | BC005995 | NM_032336 | 84296 | 4.16 | 3.72E-03 |
| RAMP | 218585_s_at | NM_016448 | NM_016448 | 51514 | 4.16 | 1.38E-04 |
| KNTC2 | 204162_at | NM_006101 | NM_006101 | 10403 | 4.14 | 1.03E-06 |
| DEPDC1 | 220295_x_at | NM_017779 | NM_017779 | 55635 | 4.14 | 6.26E-07 |
| KNSL7 | 219306_at | NM_020242 | NM_020242 | 56992 | 4.14 | 7.68E-06 |
| CDCA2 | 236957_at | AI248208 | NM_152562 | 157313 | 4.13 | 1.53E-05 |
| MLF11P | 229305_at | AA460299 | NM_024629 | 79682 | 4.13 | 2.13E-06 |
| CDC2 | 210559_s_at | D88357 | NM_001786 | 983 | 4.11 | 3.40E-06 |
| | | | NM_033379 | | | |
| CDKN3 | 1555758_a_at | AF213040 | NM_005192 | 1033 | 4.10 | 5.09E-07 |
| ATAD2 | 222740_at | AI925583 | NM_014109 | 29028 | 4.10 | 1.44E-05 |
| FAM54A | 228069_at | AL138828 | NM_138419 | 113115 | 4.10 | 8.67E-07 |
| CDK2 | 204252_at | M68520 | NM_001798 | 1017 | 4.10 | 1.46E-06 |
| | | | NM_052827 | | | |
| AI377043 | 230722_at | AI377043 | --- | --- | 4.10 | 2.84E-08 |
| CDC25C | 205167_s_at | NM_001790 | NM_001790 | 995 | 4.09 | 1.38E-05 |
| | | | NM_022809 | | | |
| GTSE1 | 204318_s_at | NM_016426 | NM_016426 | 51512 | 4.08 | 6.60E-06 |
| UHRF1 | 225655_at | AK025578 | NM_013282 | 29128 | 4.08 | 5.58E-05 |
| FLJ23311 | 219990_at | NM_024680 | NM_024680 | 79733 | 4.08 | 1.87E-05 |
| PBK | 219148_at | NM_018492 | NM_018492 | 55872 | 4.08 | 2.74E-06 |
| MGC34032 | 235763_at | AA001450 | NM_152697 | 204962 | 4.07 | 4.96E-06 |
| Spc25 | 209891_at | AF225416 | NM_020675 | 57405 | 4.07 | 2.08E-04 |
| ASPM | 219918_s_at | NM_018123 | NM_018136 | 259266 | 4.06 | 1.74E-06 |
| IQGAP3 | 229538_s_at | AW271106 | NM_178229 | 128239 | 4.04 | 3.66E-05 |
| UBE3B | 213822_s_at | BE856776 | NM_130466 | 89910 | 4.03 | 1.32E-06 |
| | | | NM_183414 | | | |
| | | | NM_183415 | | | |
| SMC2L1 | 204240_s_at | NM_006444 | NM_006444 | 10592 | 4.02 | 1.45E-04 |
| SYNPO2 | 225720_at | AW009747 | NM_133477 | 171024 | 4.02 | 2.27E-04 |
| FBLN1 | 202995_s_at | NM_006486 | NM_001996 | 2192 | 4.01 | 1.34E-06 |
| | | | NM_006485 | | | |
| | | | NM_006486 | | | |
| | | | NM_006487 | | | |
| ITGA6 | 201656_at | NM_000210 | NM_000210 | 3655 | 4.00 | 1.10E-06 |
| CDC6 | 203968_s_at | NM_001254 | NM_001254 | 990 | 4.00 | 3.34E-04 |
| TK1 | 1554408_a_at | BC007986 | NM_003258 | 7083 | 4.00 | 4.33E-07 |
| FLJ25416 | 228281_at | BF343258 | NM_145018 | 220042 | 3.99 | 1.36E-06 |
| ORC1L | 205085_at | NM_004153 | NM_004153 | 4998 | 3.98 | 1.89E-05 |
| CNTN1 | 211203_s_at | U07820 | NM_001843 | 1272 | 3.97 | 1.43E-05 |
| | | | NM_175038 | | | |
| NUSAP1 | 218039_at | NM_016359 | NM_016359 | 51203 | 3.97 | 2.83E-06 |
| | | | NM_018454 | | | |
| KIF23 | 204709_s_at | NM_004856 | NM_004856 | 9493 | 3.96 | 7.58E-05 |
| | | | NM_138555 | | | |
| FBLN1 | 201787_at | NM_001996 | NM_001996 | 2192 | 3.96 | 1.15E-04 |
| | | | NM_006485 | | | |
| | | | NM_006486 | | | |
| | | | NM_006487 | | | |
| RAD54L | 204558_at | NM_003579 | NM_003579 | 8438 | 3.95 | 6.59E-06 |
| CENPF | 209172_s_at | U30872 | NM_016343 | 1063 | 3.95 | 2.98E-08 |
| FGL1 | 205305_at | NM_004467 | NM_004467 | 2267 | 3.94 | 2.35E-05 |
| | | | NM_147203 | | | |
| | | | NM_201552 | | | |
| | | | NM_201553 | | | |
| ARHGAP24 | 223422_s_at | AI743534 | NM_031305 | 83478 | 3.94 | 2.71E-05 |
| DKFZp564A232 | 1556300_s_at | BF476109 | --- | --- | 3.93 | 1.17E-03 |
| PEPDC1 | 232278_s_at | AJ278112 | NM_017779 | 55635 | 3.93 | 3.60E-05 |
| GRP | 206326_at | NM_002091 | NM_002091 | 2922 | 3.92 | 5.54E-05 |
| MAOA | 204388_s_at | NM_000240 | NM_000240 | 4128 | 3.92 | 3.38E-07 |
| POLQ | 219510_at | NM_006596 | NM_006596 | 10721 | 3.91 | 8.49E-06 |
| | | | NM_199420 | | | |
| DKFZp762E1312 | 218726_at | NM_018410 | NM_018410 | 55355 | 3.91 | 7.86E-06 |
| GTSE1 | 215942_s_at | BF973178 | NM_016426 | 51512 | 3.90 | 9.11E-06 |
| TPX2 | 210052_s_at | AF098158 | NM_012112 | 22974 | 3.90 | 3.85E-06 |
| LOC440738 | 221697_at | AF276659 | NM_001004342 | 440738 | 3.90 | 2.27E-03 |
| LOC340109 | 1557765_at | BC039509 | XM_379322 | 340109 | 3.89 | 2.74E-05 |
| NUSAP1 | 219978_s_at | NM_018454 | NM_016359 | 51203 | 3.89 | 3.26E-06 |
| | | | NM_018454 | | | |
| LOC253981 | 226430_at | AI394438 | --- | 253981 | 3.88 | 6.06E-07 |
| ESPL1 | 204817_at | NM_012291 | NM_012291 | 9700 | 3.85 | 7.34E-05 |
| KIFC1 | 209680_s_at | BC000712 | XM_371813 | 3833 | 3.83 | 1.36E-06 |
| KCTD12 | 212188_at | AA551075 | NM_138444 | 115207 | 3.82 | 1.05E-05 |
| KIF14 | 206364_at | NM_014875 | XM_375825 | 9928 | 3.82 | 6.29E-07 |
| SGOL2 | 235425_at | AW965339 | NM_152524 | 151246 | 3.81 | 3.04E-05 |
| KIAA0186 | 206102_at | NM_021067 | XM_375911 | 9837 | 3.81 | 4.00E-06 |
| C20orf129 | 225687_at | BC001068 | NM_030919 | 81610 | 3.80 | 4.60E-05 |
| DNA2L | 213647_at | D42046 | XM_166103 | 1763 | 3.79 | 4.96E-07 |
| CCDC3 | 223316_at | AL136562 | NM_031455 | 83643 | 3.78 | 9.60E-05 |
| CIT | 212801_at | AI861788 | NM_007174 | 11113 | 3.78 | 9.25E-05 |
| BUB1B | 203755_at | NM_001211 | NM_001211 | 701 | 3.78 | 6.66E-07 |
| CDCA3 | 221436_s_at | NM_031299 | NM_031299 | 83461 | 3.77 | 1.69E-04 |
| MCM10 | 223570_at | AL136840 | NM_018518 | 55388 | 3.76 | 8.71E-06 |
| | | | NM_182751 | | | |
| C18orf24 | 217640_x_at | BF038461 | NM_145060 | 220134 | 3.76 | 7.58E-05 |
| EMILIN2 | 224374_s_at | AF270513 | NM_032048 | 84034 | 3.75 | 2.71E-05 |
| C18orf54 | 229442_at | BF059556 | NM_173529 | 162681 | 3.75 | 4.74E-08 |
| MAB21L2 | 210303_at | AF262032 | NM_006439 | 10586 | 3.73 | 4.97E-07 |
| BNC2 | 238478_at | H97386 | NM_017637 | 54796 | 3.73 | 6.40E-07 |
| AI926924 | 239253_at | AI926924 | --- | --- | 3.73 | 3.76E-04 |
| CDC2 | 203213_at | AL524035 | NM_001786 | 983 | 3.73 | 5.41E-06 |
| | | | NM_033379 | | | |
| Pfs2 | 221521_s_at | BC003186 | NM_016095 | 51659 | 3.73 | 2.20E-05 |
| MGC34032 | 1569112_at | AW020413 | NM_152697 | 204962 | 3.72 | 5.15E-06 |
| NEK2 | 204641_at | NM_002497 | NM_002497 | 4751 | 3.71 | 2.52E-05 |
| MGC45871 | 226876_at | AI961778 | NM_182705 | 359845 | 3.71 | 6.58E-06 |
| DPYD | 204646_at | NM_000110 | NM_000110 | 1806 | 3.70 | 1.06E-04 |
| BIRC5 | 202095_s_at | NM_001168 | NM_001168 | 332 | 3.70 | 1.51E-06 |
| ELOVL7 | 227180_at | AW138767 | XM_371740 | 79993 | 3.69 | 9.47E-05 |
| BF000203 | 236385_at | BF000203 | --- | --- | 3.69 | 1.75E-05 |
| STK17A | 202693_s_at | AW194730 | NM_004760 | 9263 | 3.68 | 3.33E-04 |
| CHEK1 | 205394_at | NM_001274 | NM_001274 | 1111 | 3.67 | 2.79E-04 |
| FLJ13912 | 45633_at | AI421812 | NM_022770 | 64785 | 3.67 | 3.76E-06 |
| FLJ10719 | 213008_at | BG403615 | NM_018193 | 55215 | 3.67 | 4.86E-06 |
| CENPF | 207828_s_at | NM_005196 | NM_016343 | 1063 | 3.65 | 1.58E-06 |
| E2F2 | 228361_at | AL561296 | NM_004091 | 1870 | 3.65 | 2.93E-06 |
| NEGR1 | 243357_at | AA115106 | NM_173808 | 257194 | 3.65 | 1.29E-06 |
| CENPA | 210821_x_at | BC002703 | NM_001809 | 1058 | 3.65 | 7.15E-06 |
| MGC40179 | 242560_at | AA579890 | NM_173472 | 115795 | 3.64 | 1.89E-04 |
| SPAG6 | 210033_s_at | AF079363 | NM_012443 | 9576 | 3.64 | 3.09E-03 |
| | | | NM_172242 | | | |
| SLAC2-B | 214734_at | AB014524 | NM_015065 | 23086 | 3.64 | 3.42E-04 |
| ASCL1 | 209985_s_at | BE797438 | NM_004316 | 429 | 3.63 | 3.48E-04 |
| ZNF521 | 226677_at | AF141339 | NM_015461 | 25925 | 3.62 | 6.14E-07 |
| NEK2 | 211080_s_at | Z25425 | NM_002497 | 4751 | 3.62 | 4.42E-05 |
| TK1 | 202338_at | NM_003258 | NM_003258 | 7083 | 3.62 | 1.03E-05 |
| CCNB1 | 214710_s_at | BE407516 | NM_031966 | 891 | 3.62 | 5.65E-07 |
| TCF7L1 | 221016_s_at | NM_031283 | NM_031283 | 83439 | 3.61 | 1.36E-03 |
| MYBL2 | 201710_at | NM_002466 | NM_002466 | 4605 | 3.60 | 5.06E-05 |
| BRCA2 | 208368_s_at | NM_000059 | NM_000059 | 675 | 3.60 | 1.84E-05 |
| TOP2A | 201292_at | AL561834 | NM_001067 | 7153 | 3.60 | 4.23E-06 |
| CCNE2 | 205034_at | NM_004702 | NM_004702 | 9134 | 3.60 | 1.96E-05 |
| | | | NM_057735 | | | |
| | | | NM_057749 | | | |
| AI937080 | 244033_at | AI937080 | --- | --- | 3.59 | 1.30E-04 |
| MKI67 | 212022_s_at | BF001806 | NM_002417 | 4288 | 3.59 | 3.34E-05 |
| dJ383J4.3 | 1554271_a_at | BC019022 | NM_033319 | 91687 | 3.57 | 3.09E-05 |
| SMC6L1 | 236535_at | AW069285 | NM_024624 | 79677 | 3.56 | 3.19E-05 |
| ECT2 | 219787_s_at | NM_018098 | NM_018098 | 1894 | 3.56 | 7.41E-08 |
| CDCA3 | 223307_at | BC002551 | NM_031299 | 83461 | 3.56 | 9.80E-05 |
| CDCA5 | 224753_at | BE614410 | NM_080668 | 113130 | 3.56 | 1.86E-05 |
| TRIP10 | 202734_at | NM_004240 | NM_004240 | 9322 | 3.55 | 1.98E-05 |
| C14orf106 | 226630_at | BF062175 | NM_018353 | 55320 | 3.55 | 5.62E-07 |
| TMEM35 | 219685_at | NM_021637 | NM_021637 | 59353 | 3.55 | 9.24E-05 |
| POLA2 | 204441_s_at | NM_002689 | NM_002689 | 23649 | 3.54 | 5.49E-06 |
| FLJ13912 | 218719_s_at | NM_022770 | NM_022770 | 64785 | 3.52 | 1.92E-06 |
| PDE5A | 227088_at | BF221547 | NM_001083 | 8654 | 3.52 | 7.25E-04 |
| | | | NM_033430 | | | |
| | | | NM_033431 | | | |
| | | | NM_033437 | | | |
| SIM1 | 206876_at | AL121948 | NM_005068 | 6492 | 3.52 | 9.26E-04 |
| WDR51A | 226355_at | AW001089 | NM_015426 | 25886 | 3.52 | 2.74E-05 |
| TFPI2 | 209277_at | AL574096 | NM_006528 | 7980 | 3.51 | 1.33E-04 |
| CUGBP2 | 202158_s_at | NM_006561 | NM_006561 | 10659 | 3.51 | 3.91E-03 |
| ATAD2 | 235266_at | AI139629 | NM_014109 | 29028 | 3.49 | 5.52E-05 |
| DOK5 | 1554863_s_at | BC008992 | NM_018431 | 55816 | 3.49 | 1.51E-03 |
| | | | NM_177959 | | | |
| ATAD2 | 228401_at | AI656807 | NM_014109 | 29028 | 3.49 | 2.96E-06 |
| WDHD1 | 204728_s_at | NM_007086 | NM_00100839 6 | 11169 | 3.48 | 1.11E-05 |
| | | | NM_007086 | | | |
| BC002493 | 223274_at | BC002493 | --- | --- | 3.47 | 3.08E-05 |
| BARD1 | 205345_at | NM_000465 | NM_000465 | 580 | 3.46 | 1.90E-03 |
| MGC45866 | 230021_at | AI638593 | NM_152259 | 90381 | 3.46 | 1.66E-05 |
| Cep152 | 239413_at | AI130715 | NM_014985 | 22995 | 3.46 | 2.08E-07 |
| DKFZp7610201 | 232313_at | AL122107 | XM_044062 | 92293 | 3.46 | 1.14E-04 |
| TOP2A | 201291_s_at | AU159942 | NM_001067 | 7153 | 3.45 | 8.58E-06 |
| TRAF4 | 202871_at | NM_004295 | NM_004295 | 9618 | 3.44 | 2.74E-06 |
| | | | NM_145751 | | | |
| RBPMS | 209488_s_at | D84109 | NM_00100871 0 | 11030 | 3.44 | 6.92E-04 |
| | | | NM_00100871 1 | | | |
| | | | NM_00100871 2 | | | |
| | | | NM_006867 | | | |
| CNTN1 | 227202_at | AW072790 | NM_001843 | 1272 | 3.44 | 1.89E-06 |
| | | | NM_175038 | | | |
| DCC1 | 219000_s_at | NM_024094 | NM_024094 | 79075 | 3.43 | 2.54E-04 |
| KIF2C | 211519_s_at | AY026505 | NM_006845 | 11004 | 3.42 | 9.85E-05 |
| SLC43A3 | 210692_s_at | BC003163 | NM_014096 | 29015 | 3.41 | 1.35E-04 |
| | | | NM_017611 | | | |
| | | | NM_199329 | | | |
| KIAA0101 | 202503_s_at | NM_014736 | NM_014736 | 9768 | 3.40 | 1.33E-05 |
| FBP1 | 209696_at | D26054 | NM_000507 | 2203 | 3.40 | 3.15E-04 |
| AA224205 | 238075_at | AA224205 | --- | --- | 3.39 | 1.33E-05 |
| ADAMTS9 | 226814_at | AI431730 | NM_020249 | 56999 | 3.38 | 2.69E-06 |
| | | | NM_182920 | | | |
| | | | NM_182921 | | | |
| FLJ10706 | 220840_s_at | NM_018186 | NM_018186 | 55732 | 3.38 | 4.66E-05 |
| HCAP-G | 218662_s_at | NM_022346 | NM_022346 | 64151 | 3.37 | 3.87E-06 |
| SLC7A2 | 225516_at | AA876372 | NM_00100853 9 | 6542 | 3.37 | 1.80E-08 |
| | | | NM_003046 | | | |
| BF477980 | 242775_at | BF477980 | --- | --- | 3.36 | 1.18E-04 |
| UBE2C | 202954_at | NM_007019 | NM_007019 | 11065 | 3.36 | 1.60E-06 |
| | | | NM_181799 | | | |
| | | | NM_181800 | | | |
| | | | NM_181801 | | | |
| | | | NM_181802 | | | |
| | | | NM_181803 | | | |
| CSRP2 | 207030_s_at | NM_001321 | NM_001321 | 1466 | 3.35 | 9.35E-07 |
| PRC1 | 218009_s_at | NM_003981 | NM_003981 | 9055 | 3.35 | 2.53E-06 |
| | | | NM_199413 | | | |
| | | | NM_199414 | | | |
| GNG12 | 222834_s_at | N32508 | NM_018841 | 55970 | 3.34 | 4.89E-06 |
| AB040957 | 231855_at | AB040957 | --- | --- | 3.34 | 2.91E-07 |
| DAZ1 | 208281_x_at | NM_020364 | NM_00100537 5 | 1617 | 3.34 | 4.52E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| MCM5 | 216237_s_at | AA807529 | NM_006739 | 4174 | 3.34 | 1.01E-04 |
| CCNA2 | 203418_at | NM_001237 | NM_001237 | 890 | 3.34 | 2.17E-04 |
| FLJ20105 | 219650_at | NM_017669 | NM_00100995 4 | 54821 | 3.34 | 1.12E-05 |
| | | | NM_017669 | | | |
| BF108778 | 235363_at | BF108778 | --- | --- | 3.33 | 1.13E-04 |
| BM039 | 222118_at | AK023669 | NM_018455 | 55839 | 3.33 | 1.47E-07 |
| SDC2 | 212154_at | AI380298 | NM_002998 | 6383 | 3.33 | 5.56E-04 |
| LOC440928 | 235147_at | R56118 | XM_498917 | 440928 | 3.33 | 1.32E-03 |
| LOC146909 | 222039_at | AA292789 | XM_085634 | 146909 | 3.31 | 2.57E-05 |
| RAD51AP1 | 204146_at | BE966146 | NM_006479 | 10635 | 3.31 | 4.07E-06 |
| SMC6L1 | 218781_at | NM_024624 | NM_024624 | 79677 | 3.30 | 2.52E-08 |
| ZWINT | 204026_s_at | NM_007057 | NM_00100541 3 | 11130 | 3.29 | 7.56E-06 |
| | | | NM_00100541 4 | | | |
| | | | NM_007057 | | | |
| | | | NM_032997 | | | |
| KIAA1754 | 225582_at | AA425726 | NM_033397 | 85450 | 3.29 | 3.22E-06 |
| CCNB1 | 228729_at | N90191 | NM_031966 | 891 | 3.29 | 3.59E-06 |
| PRAME | 204086_at | NM_006115 | NM_006115 | 23532 | 3.28 | 3.84E-04 |
| | | | NM_206953 | | | |
| | | | NM_206954 | | | |
| | | | NM_206955 | | | |
| | | | NM_206956 | | | |
| BRCA1 | 204531_s_at | NM_007295 | NM_007294 | 672 | 3.28 | 2.73E-04 |
| | | | NM_007295 | | | |
| | | | NM_007296 | | | |
| | | | NM_007297 | | | |
| | | | NM_007298 | | | |
| | | | NM_007299 | | | |
| | | | NM_007300 | | | |
| | | | NM_007301 | | | |
| | | | NM_007302 | | | |
| | | | NM_007303 | | | |
| | | | NM_007304 | | | |
| | | | NM_007305 | | | |
| | | | NM_007306 | | | |
| CNTN1 | 227209_at | AI091445 | NM_001843 | 1272 | 3.27 | 3.23E-05 |
| | | | NM_175038 | | | |
| SLC43A3 | 213113_s_at | AI630178 | NM_014096 | 29015 | 3.27 | 1.59E-06 |
| | | | NM_017611 | | | |
| | | | NM_199329 | | | |
| TIMP3 | 201147_s_at | BF347089 | NM_000362 | 7078 | 3.27 | 2.90E-04 |
| LTB4DH | 231897_at | AL135787 | NM_012212 | 22949 | 3.27 | 4.25E-05 |
| FLJ22624 | 219544_at | NM_024808 | NM_024808 | 79866 | 3.27 | 4.96E-04 |
| GNAI1 | 227692_at | AU153866 | NM_002069 | 2770 | 3.26 | 1.36E-05 |
| BC035780 | 1560258_a_at | BC035780 | --- | --- | 3.26 | 1.57E-07 |
| CCNB2 | 202705_at | NM_004701 | NM_004701 | 9133 | 3.24 | 2.06E-06 |
| PTTG1 | 203554_x_at | NM_004219 | NM_004219 | 9232 | 3.24 | 8.78E-06 |
| LOC157570 | 235588_at | AA740849 | XM_088331 | 157570 | 3.23 | 2.41E-04 |
| BG391951 | 236852_at | BG391951 | --- | --- | 3.22 | 1.77E-04 |
| MLF1IP | 218883_s_at | NM_024629 | NM_024629 | 79682 | 3.21 | 1.31E-05 |
| MGC24665 | 226456_at | AW138157 | NM_152308 | 116028 | 3.21 | 6.88E-05 |
| GNAI1 | 209576_at | AL049933 | NM_002069 | 2770 | 3.21 | 1.53E-06 |
| PLK4 | 204886_at | AL043646 | NM_014264 | 10733 | 3.20 | 2.80E-04 |
| GPSM2 | 205240_at | NM_013296 | NM_013296 | 29899 | 3.20 | 9.04E-07 |
| TROAP | 204649_at | NM_005480 | NM_005480 | 10024 | 3.19 | 2.23E-05 |
| SYNPO2 | 227662_at | AA541622 | NM_133477 | 171024 | 3.19 | 1.95E-03 |
| C14orf106 | 206500_s_at | NM_018353 | NM_018353 | 55320 | 3.19 | 5.08E-04 |
| HCAP-G | 218663_at | NM_022346 | NM_022346 | 64151 | 3.19 | 1.36E-04 |
| FLJ10719 | 213007_at | W74442 | NM_018193 | 55215 | 3.18 | 1.31E-05 |
| LTB4DH | 228824_s_at | BE566894 | NM_012212 | 22949 | 3.17 | 2.71E-05 |
| TRIM36 | 219736_at | NM_018700 | NM_018700 | 55521 | 3.17 | 4.38E-05 |
| HGF | 209960_at | X16323 | NM_000601 | 3082 | 3.17 | 6.34E-04 |
| | | | NM_00101093 1 | | | |
| | | | NM_00101093 2 | | | |
| | | | NM_00101093 3 | | | |
| | | | NM_00101093 4 | | | |
| CHAF1A | 203976_s_at | NM_005483 | NM_005483 | 10036 | 3.17 | 3.20E-04 |
| AK022040 | 233445_at | AK022040 | --- | --- | 3.17 | 1.50E-06 |
| AA938184 | 236312_at | AA938184 | --- | --- | 3.17 | 1.06E-04 |
| CTSL2 | 210074_at | AF070448 | NM_001333 | 1515 | 3.17 | 5.51E-04 |
| FKSG14 | 222848_at | BC005400 | NM_022145 | 64105 | 3.16 | 3.35E-06 |
| EME1 | 234465_at | AK021607 | NM_152463 | 146956 | 3.16 | 8.50E-06 |
| TCTE1L | 203303_at | NM_006520 | NM_006520 | 6990 | 3.15 | 2.23E-04 |
| SBLF | 213413_at | BG434174 | NM_006873 | 11037 | 3.15 | 3.72E-03 |
| MCM6 | 201930_at | NM_005915 | NM_005915 | 4175 | 3.15 | 3.87E-06 |
| LOC402524 | 242206_at | AW590588 | XM_379850 | 402524 | 3.14 | 1.94E-07 |
| INCENP | 242787_at | AI924134 | NM_020238 | 3619 | 3.14 | 7.83E-06 |
| LTB4DH | 228825_at | BE566894 | NM_012212 | 22949 | 3.12 | 1.25E-04 |
| FNDC5 | 226096_at | AI760132 | NM_153756 | 252995 | 3.12 | 3.00E-04 |
| P2RY5 | 218589_at | NM_005767 | NM_005767 | 10161 | 3.12 | 2.25E-03 |
| GABRA5 | 206456_at | NM_000810 | NM_000810 | 2558 | 3.11 | 3.01E-04 |
| E2F1 | 204947_at | NM_005225 | NM_005225 | 1869 | 3.11 | 1.27E-05 |
| E2F7 | 228033_at | AI341146 | NM_203394 | 144455 | 3.11 | 9.10E-05 |
| EDG2 | 204036_at | AW269335 | NM_001401 | 1902 | 3.11 | 9.74E-05 |
| | | | NM_057159 | | | |
| RFC5 | 203210_s_at | NM_007370 | NM_007370 | 5985 | 3.09 | 2.74E-05 |
| | | | NM_181578 | | | |
| BNC2 | 220272_at | NM_017637 | NM_017637 | 54796 | 3.09 | 7.83E-06 |
| DEPDC1B | 226980_at | AK001166 | NM_018369 | 55789 | 3.09 | 1.82E-06 |
| PPAP2B | 209355_s_at | AB000889 | NM_003713 | 8613 | 3.09 | 1.33E-03 |
| | | | NM_177414 | | | |
| RBL1 | 1555004_a_at | BC032247 | NM_002895 | 5933 | 3.09 | 1.25E-05 |
| | | | NM_183404 | | | |
| LOC132671 | 229331_at | AI559300 | NM_145263 | 132671 | 3.09 | 2.77E-04 |
| AI656180 | 242447_at | AI656180 | --- | --- | 3.09 | 6.33E-05 |
| KCNG3 | 1552897_a_at | NM_133329 | NM_133329 | 170850 | 3.09 | 1.62E-04 |
| | | | NM_172344 | | | |
| EFNB2 | 202668_at | BF001670 | NM_004093 | 1948 | 3.08 | 7.07E-04 |
| TACC3 | 218308_at | NM_006342 | NM_006342 | 10460 | 3.07 | 121E-05 |
| SHOX2 | 210135_s_at | AF022654 | NM_003030 | 6474 | 3.07 | 2.01E-03 |
| | | | NM_006884 | | | |
| TYMS | 1554696_s_at | AB077208 | NM_001071 | 7298 | 3.07 | 2.09E-06 |
| CDK2 | 211804_s_at | AB012305 | NM_001798 | 1017 | 3.06 | 3.25E-06 |
| | | | NM_052827 | | | |
| MGC33887 | 213644_at | AI979276 | NM_145036 | 201134 | 3.06 | 7.13E-03 |
| BVES | 228783_at | AA993518 | NM_007073 | 11149 | 3.06 | 3.06E-04 |
| | | | NM_147147 | | | |
| C20orf172 | 219512_at | NM_024918 | NM_024918 | 79980 | 3.05 | 1.03E-05 |
| OSBPL3 | 209626_s_at | AI202969 | NM_015550 | 26031 | 3.05 | 2.81E-04 |
| | | | NM_145320 | | | |
| | | | NM_145321 | | | |
| | | | NM_145322 | | | |
| | | | NM_145323 | | | |
| | | | NM_145324 | | | |
| CXCR4 | 211919_s_at | AF348491 | NM_00100854 0 | 7852 | 3.05 | 2.72E-03 |
| | | | NM_003467 | | | |
| GAS2L3 | 235709_at | H37811 | NM_174942 | 283431 | 3.04 | 8.83E-03 |
| CENPJ | 220885_s_at | NM_018451 | NM_018451 | 55835 | 3.04 | 5.23E-04 |
| TIMP3 | 201149_s_at | U67195 | NM_000362 | 7078 | 3.03 | 3.76E-05 |
| AW271106 | 229490_s_at | AW271106 | --- | --- | 3.03 | 3.24E-06 |
| SPAG5 | 203145_at | NM_006461 | NM_006461 | 10615 | 3.03 | 2.06E-06 |
| CXCR4 | 217028_at | AJ224869 | NM_00100854 0 | 7852 | 3.03 | 8.98E-04 |
| | | | NM_003467 | | | |
| PRIMA1 | 230087_at | AI823645 | NM_178004 | 145270 | 3.02 | 1.47E-05 |
| | | | NM_178013 | | | |
| DDA3 | 201896_s_at | BC001425 | NM_00100529 0 | 84722 | 3.02 | 5.61E-06 |
| | | | NM_032636 | | | |
| DAZ1 | 216922_x_at | AF271088 | NM_00100537 5 | 1617 | 3.02 | 8.33E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| MYRIP | 214156_at | AL050090 | NM_015460 | 25924 | 3.02 | 2.47E-05 |
| KITLG | 226534_at | AI446414 | NM_000899 | 4254 | 3.01 | 4.26E-04 |
| | | | NM_003994 | | | |
| GAS2L3 | 238756_at | AI860012 | NM_174942 | 283431 | 3.00 | 7.34E-05 |
| NRXN1 | 228547_at | BF509242 | NM_004801 | 9378 | 3.00 | 1.59E-03 |
| | | | NM_138735 | | | |
| ORC6L | 219105_x_at | NM_014321 | NM_014321 | 23594 | 3.00 | 2.00E-07 |
| CHEK1 | 205393_s_at | NM_001274 | NM_001274 | 1111 | 2.99 | 6.29E-05 |
| FLJ20641 | 220060_s_at | NM_017915 | NM_017915 | 55010 | 2.98 | 3.74E-07 |
| SLITRK5 | 214930_at | AW449813 | NM_015567 | 26050 | 2.98 | 2.02E-05 |
| BNC2 | 233036_at | AU146418 | NM_017637 | 54796 | 2.98 | 6.17E-03 |
| SYNPO2 | 225895_at | AI634580 | NM_133477 | 171024 | 2.98 | 8.59E-04 |
| UCP2 | 208998_at | U94592 | NM_003355 | 7351 | 2.97 | 3.77E-05 |
| ETV1 | 217061_s_at | AC004857 | NM_004956 | 2115 | 2.96 | 6.17E-07 |
| MNS1 | 219703_at | NM_018365 | NM_018365 | 55329 | 2.96 | 3.49E-04 |
| MGC11266 | 226118_at | BE326728 | NM_024322 | 79172 | 2.95 | 4.33E-06 |
| AW075105 | 228868_x_at | AW075105 | --- | --- | 2.95 | 2.52E-05 |
| LOC91431 | 228859_at | BF056790 | NM_138698 | 91431 | 2.95 | 1.95E-05 |
| OSAP | 223734_at | AF329088 | NM_032623 | 84709 | 2.95 | 4.92E-04 |
| ARHGAP11A | 204492_at | NM_014783 | NM_014783 | 9824 | 2.95 | 3.64E-08 |
| | | | NM_199357 | | | |
| C6orf173 | 226936_at | BG492359 | --- | --- | 2.95 | 2.93E-07 |
| HSPA2 | 211538_s_at | U56725 | NM_021979 | 3306 | 2.95 | 2.25E-03 |
| MYO6 | 203216_s_at | NM_004999 | NM_004999 | 4646 | 2.94 | 2.66E-07 |
| MAOA | 204389_at | NM_000240 | NM_000240 | 4128 | 2.94 | 4.60E-06 |
| ZNF521 | 226676_at | AK021452 | NM_015461 | 25925 | 2.94 | 8.51E-04 |
| ASPM | 232238_at | AK001380 | NM_018136 | 259266 | 2.94 | 4.78E-04 |
| ENC1 | 201340_s_at | AF010314 | NM_003633 | 8507 | 2.94 | 2.73E-05 |
| KDELC2 | 225128_at | AL548941 | NM_153705 | 143888 | 2.94 | 4.64E-05 |
| SYNPO2 | 225894_at | AL589603 | NM_133477 | 171024 | 2.93 | 8.98E-05 |
| ENC1 | 201341_at | NM_003633 | NM_003633 | 8507 | 2.93 | 2.30E-05 |
| LOC441157 | 1558796_a_at | AL833240 | XM_499037 | 441157 | 2.93 | 2.03E-03 |
| SDC2 | 212158_at | AL577322 | NM_002998 | 6383 | 2.92 | 7.40E-07 |
| TROAP | 1568596_a_at | AI199355 | NM_005480 | 10024 | 2.92 | 8.12E-06 |
| LOC253012 | 242601_at | AA600175 | NM_198151 | 253012 | 2.92 | 1.02E-03 |
| MGC24103 | 232568_at | AU145658 | --- | 158295 | 2.91 | 1.01E-02 |
| TFDP1 | 242939_at | AI950069 | NM_007111 | 7027 | 2.91 | 4.17E-05 |
| LOC441157 | 1558795_at | AL833240 | XM_499037 | 441157 | 2.90 | 7.52E-07 |
| C22orf18 | 218741_at | NM_024053 | NM_00100287 6 | 79019 | 2.90 | 2.43E-05 |
| | | | NM_024053 | | | |
| TYMS | 202589_at | NM_001071 | NM_001071 | 7298 | 2.89 | 1.61E-06 |
| CXCR4 | 209201_x_at | L01639 | NM_00100854 0 | 7852 | 2.88 | 5.99E-03 |
| | | | NM_003467 | | | |
| MGC45871 | 226905_at | BG036514 | NM_182705 | 359845 | 2.88 | 3.75E-04 |
| KNTC1 | 206316_s_at | NM_014708 | NM_014708 | 9735 | 2.87 | 5.79E-05 |
| PCNA | 201202_at | NM_002592 | NM_002592 | 5111 | 2.87 | 8.09E-06 |
| | | | NM_182649 | | | |
| NEUROD4 | 221318_at | NM_021191 | NM_021191 | 58158 | 2.86 | 1.12E-04 |
| AF15Q14 | 1552680_a_at | NM_020380 | NM_020380 | 57082 | 2.85 | 1.73E-04 |
| | | | NM_144508 | | | |
| | | | NM_170589 | | | |
| AA699443 | 241838_at | AA699443 | --- | --- | 2.85 | 9.18E-05 |
| CCNE1 | 213523_at | AI671049 | NM_001238 | 898 | 2.85 | 3.52E-04 |
| | | | NM_057182 | | | |
| ST8SIA4 | 230836_at | AI422986 | NM_005668 | 7903 | 2.85 | 4.72E-06 |
| | | | NM_175052 | | | |
| MGC33926 | 229302_at | AA058832 | NM_152390 | 130733 | 2.84 | 2.64E-05 |
| DDB2 | 203409_at | NM_000107 | NM_000107 | 1643 | 2.84 | 2.16E-03 |
| TWIST1 | 213943_at | X99268 | NM_000474 | 7291 | 2.83 | 1.05E-05 |
| DHRS6 | 218285_s_at | NM_020139 | NM_020139 | 56898 | 2.82 | 4.65E-05 |
| DHFR | 202533_s_at | BC003584 | NM_000791 | 1719 | 2.82 | 4.52E-05 |
| FLJ11029 | 228273_at | BG165011 | NM_018304 | 55771 | 2.82 | 6.55E-07 |
| RNASEH2A | 203022_at | NM_006397 | NM_006397 | 10535 | 2.82 | 9.96E-07 |
| DCLRE1B | 222889_at | AI703304 | NM_022836 | 64858 | 2.81 | 7.59E-05 |
| C13orf3 | 227165_at | AI829603 | NM_145061 | 221150 | 2.81 | 9.72E-05 |
| RBPMS | 209487_at | D84109 | NM_00100871 0 | 11030 | 2.81 | 2.78E-06 |
| | | | NM_00100871 1 | | | |
| | | | NM_00100871 2 | | | |
| | | | NM_006867 | | | |
| ETV1 | 217053_x_at | X87175 | NM_004956 | 2115 | 2.80 | 4.43E-07 |
| CHRNA7 | 210123_s_at | U62436 | NM_000746 | 1139 | 2.80 | 1.15E-04 |
| CHRFAM7A | | | NM_139320 | 89832 | | |
| | | | NM_148911 | | | |
| MARLIN1 | 238600_at | AW157571 | NM_144720 | 152789 | 2.80 | 1.18E-03 |
| PLCL2 | 213309_at | AL117515 | NM_015184 | 23228 | 2.80 | 1.93E-04 |
| Cep152 | 238535_at | AW955956 | NM_014985 | 22995 | 2.79 | 8.14E-06 |
| RAB32 | 204214_s_at | NM_006834 | NM_006834 | 10981 | 2.79 | 1.16E-03 |
| MAD2L1 | 1554768_a_at | AF394735 | NM_002358 | 4085 | 2.79 | 3.08E-06 |
| ITGA6 | 215177_s_at | AV733308 | NM_000210 | 3655 | 2.79 | 2.99E-04 |
| ENDOG | 204824_at | NM_004435 | NM_004435 | 2021 | 2.78 | 3.20E-04 |
| TFDP2 | 203588_s_at | BG034328 | NM_006286 | 7029 | 2.78 | 7.51E-05 |
| BTG3 | 1556213_a_at | BC028229 | NM_006806 | 10950 | 2.78 | 5.93E-04 |
| KIF2C | 209408_at | U63743 | NM_006845 | 11004 | 2.77 | 6.84E-05 |
| CD9 | 201005_at | NM_001769 | NM_001769 | 928 | 2.77 | 9.74E-06 |
| TMPO | 209754_s_at | AF113682 | NM_003276 | 7112 | 2.77 | 9.71E-06 |
| CDC25B | 201853_s_at | NM_021873 | NM_004358 | 994 | 2.77 | 2.17E-06 |
| | | | NM_021872 | | | |
| | | | NM_021873 | | | |
| | | | NM_021874 | | | |
| | | | NM_212530 | | | |
| TMPO | 209753_s_at | BG391171 | NM_003276 | 7112 | 2.76 | 7.36E-06 |
| AURKB | 239219_at | N55457 | NM_004217 | 9212 | 2.76 | 3.82E-04 |
| TIFA | 226117_at | AA195074 | NM_052864 | 92610 | 2.75 | 2.55E-05 |
| CDCA7 | 224428_s_at | AY029179 | NM_031942 | 83879 | 2.75 | 2.27E-06 |
| | | | NM_145810 | | | |
| CDT1 | 209832_s_at | AF321125 | NM_030928 | 81620 | 2.75 | 4.06E-04 |
| HS3ST2 | 219697_at | NM_006043 | NM_006043 | 9956 | 2.75 | 9.91E-05 |
| C18orf54 | 244324_at | BG283921 | NM_173529 | 162681 | 2.75 | 1.19E-05 |
| PTPRM | 1555579_s_at | BC029442 | NM_002845 | 5797 | 2.74 | 5.55E-05 |
| PLK1 | 202240_at | NM_005030 | NM_005030 | 5347 | 2.74 | 4.99E-06 |
| FBLN1 | 202994_s_at | Z95331 | NM_001996 | 2192 | 2.74 | 5.20E-04 |
| | | | NM_006485 | | | |
| | | | NM_006486 | | | |
| | | | NM_006487 | | | |
| RAD54B | 219494_at | NM_012415 | NM_012415 | 25788 | 2.74 | 3.14E-04 |
| | | | NM_134434 | | | |
| C14orf31 | 225464_at | N30138 | NM_152330 | 122786 | 2.74 | 6.69E-06 |
| EDG3 | 228176_at | AA534817 | NM_005226 | 1903 | 2.73 | 2.38E-04 |
| UBL3 | 243072_at | AI733828 | NM_007106 | 5412 | 2.72 | 2.94E-04 |
| ASCL1 | 213768_s_at | AW950513 | NM_004316 | 429 | 2.71 | 5.62E-05 |
| RHEBL1 | 1570253_a_at | BC014155 | NM_144593 | 121268 | 2.70 | 1.21E-05 |
| FLJ40773 | 1563933_a_at | AK091691 | NM_152666 | 200150 | 2.70 | 3.08E-04 |
| ALCAM | 240655_at | BE502785 | NM_001627 | 214 | 2.70 | 9.75E-04 |
| BRCA2 | 214727_at | X95152 | NM_000059 | 675 | 2.70 | 4.89E-03 |
| SCAP1 | 1560861_at | BG210619 | NM_003726 | 8631 | 2.70 | 9.27E-05 |
| CCNA2 | 213226_at | AI346350 | NM_001237 | 890 | 2.69 | 3.29E-05 |
| PKMYT1 | 204267_x_at | NM_004203 | NM_004203 | 9088 | 2.69 | 2.26E-05 |
| | | | NM_182687 | | | |
| VRK1 | 203856_at | NM_003384 | NM_003384 | 7443 | 2.69 | 6.68E-06 |
| ARHGEF3 | 218501_at | NM_019555 | NM_019555 | 50650 | 2.69 | 2.18E-04 |
| ASPM | 239002_at | AA748494 | NM_018136 | 259266 | 2.69 | 7.18E-03 |
| H17657 | 241444_at | H17657 | --- | --- | 2.69 | 3.92E-04 |
| TNFAIP8 | 210260_s_at | BC005352 | NM_014350 | 25816 | 2.68 | 5.03E-05 |
| Cep72 | 219531_at | NM_018140 | NM_018140 | 55722 | 2.68 | 5.02E-05 |
| FGFR2 | 203638_s_at | NM_022969 | NM_000141 | 2263 | 2.68 | 4.60E-05 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| CSRP2 | 211126_s_at | U46006 | NM_001321 | 1466 | 2.68 | 6.18E-07 |
| FAM49A | 208092_s_at | NM_030797 | NM_030797 | 81553 | 2.68 | 1.51E-03 |
| CHAF1A | 214426_x_at | BF062223 | NM_005483 | 10036 | 2.67 | 2.14E-07 |
| LOC387816 | 241360_at | BG413368 | XM_370664 | 387816 | 2.67 | 1.92E-05 |
| MTB | 219588_s_at | NM_017760 | NM_017760 | 54892 | 2.67 | 4.22E-05 |
| EML1 | 204797_s_at | NM_004434 | NM_00100870 7 | 2009 | 2.67 | 3.68E-03 |
| | | | NM_004434 | | | |
| MAD2L1 | 203362_s_at | NM_002358 | NM_002358 | 4085 | 2.67 | 1.55E-06 |
| RBL1 | 205296_at | AL365505 | NM_002895 | 5933 | 2.66 | 2.73E-05 |
| | | | NM_183404 | | | |
| SYNPO2 | 225721_at | AI658662 | NM_133477 | 171024 | 2.66 | 4.01E-04 |
| SYNE2 | 202761_s_at | NM_015180 | NM_015180 | 23224 | 2.66 | 1.44E-06 |
| | | | NM_182910 | | | |
| | | | NM_182912 | | | |
| | | | NM_182913 | | | |
| | | | NM_182914 | | | |
| RASSF2 | 203185_at | NM_014737 | NM_014737 | 9770 | 2.65 | 1.34E-04 |
| | | | NM_170773 | | | |
| | | | NM_170774 | | | |
| AI001880 | 242723_at | AI001880 | --- | --- | 2.65 | 9.66E-04 |
| PTPRK | 203038_at | NM_002844 | NM_002844 | 5796 | 2.65 | 5.22E-06 |
| ETV1 | 206501_x_at | NM_004956 | NM_004956 | 2115 | 2.65 | 2.26E-07 |
| WDHD1 | 216228_s_at | AK001538 | NM_00100839 6 | 11169 | 2.64 | 9.95E-04 |
| | | | NM_007086 | | | |
| ZNF505 | 1569241_a_at | BC020837 | NM_00100412 6 | 81931 | 2.64 | 6.40E-03 |
| | | | NM_031218 | | | |
| SESN3 | 235683_at | BF685808 | NM_144665 | 143686 | 2.63 | 1.07E-03 |
| BLM | 205733_at | NM_000057 | NM_000057 | 641 | 2.63 | 3.91E-03 |
| MYO6 | 210480_s_at | U90236 | NM_004999 | 4646 | 2.62 | 1.11E-06 |
| SIL | 205339_at | NM_003035 | NM_003035 | 6491 | 2.61 | 1.15E-04 |
| CENPH | 231772_x_at | AL572471 | NM_022909 | 64946 | 2.60 | 1.04E-05 |
| GUCY1B3 | 211555_s_at | AF020340 | NM_000857 | 2983 | 2.60 | 8.17E-05 |
| MLF1IP | 229304_s_at | AA460299 | NM_024629 | 79682 | 2.60 | 9.56E-06 |
| ASCL1 | 209987_s_at | BC002341 | NM_004316 | 429 | 2.60 | 2.04E-04 |
| BF111626 | 228559_at | BF111626 | --- | --- | 2.60 | 2.36E-05 |
| GAJ | 223700_at | AY028916 | NM_032117 | 84057 | 2.60 | 2.48E-05 |
| LOC494143 | 235117_at | AI191897 | NM_001008708 | 494143 | 2.59 | 9.30E-04 |
| BG252802 | 229641_at | BG252802 | --- | --- | 2.58 | 2.53E-05 |
| AA406603 | 230002_at | AA406603 | --- | --- | 2.58 | 3.50E-06 |
| AW024890 | 229942_at | AW024890 | --- | --- | 2.58 | 9.80E-04 |
| GPR10 | 231805_at | AL563031 | NM_004248 | 2834 | 2.58 | 2.33E-04 |
| Cep152 | 215170_s_at | AB020719 | NM_014985 | 22995 | 2.58 | 6.05E-06 |
| EB-1 | 227441_s_at | AW005572 | NM_020140 | 56899 | 2.57 | 1.57E-03 |
| | | | NM_152788 | | | |
| | | | NM_181670 | | | |
| TLE3 | 206472_s_at | NM_005078 | NM_005078 | 7090 | 2.57 | 3.99E-04 |
| WEE1 | 215711_s_at | AJ277546 | NM_003390 | 7465 | 2.56 | 8.25E-05 |
| BM039 | 219555_s_at | NM_018455 | NM_018455 | 55839 | 2.56 | 9.17E-08 |
| TMSNB | 205347_s_at | NM_021992 | NM_021992 | 11013 | 2.56 | 1.86E-04 |
| KIAA1524 | 1553810_a_at | NM_020890 | NM_020890 | 57650 | 2.55 | 1.25E-06 |
| ACATE2 | 221641_s_at | AF241787 | NM_012332 | 23597 | 2.54 | 6.19E-04 |
| AF15Q14 | 228323_at | BF248364 | NM_020380 | 57082 | 2.54 | 2.09E-04 |
| | | | NM_144508 | | | |
| | | | NM_170589 | | | |
| CDC7 | 204510_at | NM_003503 | NM_003503 | 8317 | 2.54 | 7.24E-04 |
| PTPRM | 203329_at | NM_002845 | NM_002845 | 5797 | 2.53 | 3.83E-04 |
| PLEKHE1 | 212719_at | AB011178 | NM_194449 | 23239 | 2.52 | 1.61E-05 |
| MYO6 | 203215_s_at | AA877789 | NM_004999 | 4646 | 2.51 | 2.63E-04 |
| AL035461 | 231827_at | AL035461 | --- | --- | 2.51 | 3.24E-04 |
| BF509125 | 232198_at | BF509125 | --- | --- | 2.51 | 3.41E-03 |
| TIMP3 | 201150_s_at | NM_000362 | NM_000362 | 7078 | 2.51 | 1.89E-05 |
| NRXN1 | 209915_s_at | AB035356 | NM_004801 | 9378 | 2.51 | 9.30E-04 |
| | | | NM_138735 | | | |
| LOC221810 | 221911_at | BE881590 | --- | 221810 | 2.51 | 4.94E-06 |
| SLC16A10 | 222939_s_at | N30257 | NM_018593 | 117247 | 2.51 | 2.79E-04 |
| PPAP2B | 212230_at | AV725664 | NM_003713 | 8613 | 2.50 | 1.17E-04 |
| | | | NM_177414 | | | |
| C14orf143 | 214264_s_at | AI656610 | NM_145231 | 90141 | 2.50 | 1.62E-05 |
| FLJ11259 | 218627_at | NM_018370 | NM_018370 | 55332 | 2.50 | 3.56E-04 |
| FAM46C | 226811_at | AL046017 | NM_017709 | 54855 | 2.50 | 6.18E-03 |
| CLSTN2 | 219414_at | NM_022131 | NM_022131 | 64084 | 2.50 | 2.54E-04 |
| OVOS | 228245_s_at | AW594320 | XM_495907 | 408186 | 2.49 | 1.90E-04 |
| LOC440080 | | | XM_495909 | 440080 | | |
| MCM8 | 224320_s_at | BC005170 | NM_032485 | 84515 | 2.49 | 1.61E-05 |
| | | | NM_182802 | | | |
| ARG99 | 226322_at | BF109231 | NM_031920 | 83857 | 2.49 | 6.26E-04 |
| | | | NM_175861 | | | |
| SUV39H1 | 218619_s_at | NM_003173 | NM_003173 | 6839 | 2.49 | 1.39E-03 |
| BG109249 | 1558750_a_at | BG109249 | --- | --- | 2.48 | 6.02E-05 |
| CKAP2 | 218252_at | NM_018204 | NM_018204 | 26586 | 2.48 | 9.73E-07 |
| MGC17791 | 227420_at | BF338045 | NM_152362 | 126282 | 2.48 | 2.52E-04 |
| AK025909 | 233814_at | AK025909 | --- | --- | 2.48 | 3.30E-04 |
| MGC20533 | 226308_at | AA099118 | NM_00101169 9 | 93323 | 2.48 | 8.23E-06 |
| | | | NM_033417 | | | |
| GATM | 216733_s_at | X86401 | NM_001482 | 2628 | 2.48 | 4.09E-04 |
| TPBG | 203476_at | NM_006670 | NM_006670 | 7162 | 2.47 | 4.81E-05 |
| PTER | 222798_at | BF112019 | NM_00100148 4 | 9317 | 2.47 | 8.26E-04 |
| | | | NM_030664 | | | |
| FGFR2 | 208228_s_at | M87771 | NM_000141 | 2263 | 2.47 | 2.00E-05 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| SMC4L1 | 201663_s_at | NM_005496 | NM_00100279 9 | 10051 | 2.47 | 1.52E-06 |
| | | | NM_00100280 0 | | | |
| | | | NM_005496 | | | |
| AW963185 | 241700_at | AW963185 | --- | --- | 2.46 | 2.26E-04 |
| RFC3 | 204128_s_at | NM_002915 | NM_002915 | 5983 | 2.46 | 3.05E-05 |
| | | | NM_181558 | | | |
| GLDC | 204836_at | NM_000170 | NM_000170 | 2731 | 2.46 | 4.48E-04 |
| BF432331 | 242519_at | BF432331 | --- | --- | 2.46 | 1.67E-04 |
| AW151660 | 239744_at | AW151660 | --- | --- | 2.46 | 4.79E-05 |
| HRH3 | 220447_at | NM_007232 | NM_007232 | 11255 | 2.46 | 1.62E-03 |
| VAV3 | 218807_at | NM_006113 | NM_006113 | 10451 | 2.46 | 6.79E-08 |
| MCM6 | 238977_at | R54683 | NM_005915 | 4175 | 2.45 | 1.80E-04 |
| KIF22 | 202183_s_at | NM_007317 | NM_007317 | 3835 | 2.44 | 1.23E-04 |
| HSPC150 | 223229_at | AB032931 | NM_014176 | 29089 | 2.44 | 1.16E-05 |
| QKI | 212636_at | AL031781 | NM_006775 | 9444 | 2.43 | 4.26E-03 |
| | | | NM_206853 | | | |
| | | | NM_206854 | | | |
| | | | NM_206855 | | | |
| MGC21654 | 1556429_a_at | BC026969 | NM_145647 | 93594 | 2.43 | 3.09E-06 |
| ASCL1 | 209988_s_at | BC001638 | NM_004316 | 429 | 2.43 | 1.00E-04 |
| FLJ37034 | 229622_at | H16258 | --- | 151176 | 2.42 | 4.51E-03 |
| FSHPRH1 | 207590_s_at | NM_006733 | NM_006733 | 2491 | 2.42 | 3.10E-03 |
| PPP1R3C | 204284_at | N26005 | NM_005398 | 5507 | 2.42 | 2.95E-04 |
| MASTL | 228468_at | BF108964 | NM_032844 | 84930 | 2.42 | 1.20E-05 |
| DPYSL3 | 201430_s_at | W72516 | NM_001387 | 1809 | 2.41 | 3.61E-06 |
| AW052186 | 239942_at | AW052186 | --- | --- | 2.41 | 9.52E-03 |
| RAM2 | 225081_s_at | AK022955 | NM_018719 | 55536 | 2.40 | 3.44E-03 |
| PCSK1 | 205825_at | NM_000439 | NM_000439 | 5122 | 2.40 | 1.58E-03 |
| TMPO | 203432_at | AW272611 | NM_003276 | 7112 | 2.40 | 5.20E-05 |
| CUGBP2 | 202157_s_at | U69546 | NM_006561 | 10659 | 2.40 | 6.84E-03 |
| C19orf14 | 215218_s_at | AC004144 | NM_173636 | 284403 | 2.38 | 1.41E-03 |
| TRIM68 | 219405_at | NM_018073 | NM_018073 | 55128 | 2.38 | 3.90E-03 |
| TEX15 | 221448_s_at | NM_031271 | NM_031271 | 56154 | 2.38 | 6.78E-03 |
| AU156421 | 233413_at | AU156421 | --- | --- | 2.37 | 1.88E-03 |
| CDKN2D | 210240_s_at | U20498 | NM_001800 | 1032 | 2.36 | 8.95E-04 |
| | | | NM_079421 | | | |
| PTGFRN | 224937_at | BF311866 | NM_020440 | 5738 | 2.36 | 4.21E-05 |
| DERA | 218102_at | NM_015954 | NM_015954 | 51071 | 2.36 | 4.21E-04 |
| VAV3 | 218806_s_at | AF118887 | NM_006113 | 10451 | 2.35 | 2.50E-04 |
| RASEF | 1553986_at | BC023566 | NM_152573 | 158158 | 2.34 | 4.65E-04 |
| TNFSF13B | 223502_s_at | AF134715 | NM_006573 | 10673 | 2.34 | 3.38E-03 |
| STN2 | 235852_at | BE379761 | NM_033104 | 85439 | 2.34 | 2.91E-03 |
| SLC4A11 | 223748_at | AF336127 | NM_032034 | 83959 | 2.34 | 7.89E-03 |
| C20orf72 | 225890_at | AI678096 | NM_052865 | 92667 | 2.33 | 7.53E-05 |
| CPVL | 208146_s_at | NM_031311 | NM_019029 | 54504 | 2.33 | 1.12E-03 |
| | | | NM_031311 | | | |
| HMGB2 | 208808_s_at | BC000903 | NM_002129 | 3148 | 2.33 | 5.57E-07 |
| FLJ13215 | 220466_at | NM_025004 | NM_025004 | 80071 | 2.32 | 1.61E-04 |
| PLK4 | 211088_s_at | Z25433 | NM_014264 | 10733 | 2.31 | 1.24E-05 |
| MRC2 | 37408_at | AB014609 | NM_006039 | 9902 | 2.31 | 4.86E-03 |
| ZNF488 | 229901_at | AI056483 | NM_153034 | 118738 | 2.31 | 3.12E-03 |
| TM4SF7 | 209264_s_at | AF054841 | NM_003271 | 7106 | 2.31 | 1.36E-04 |
| CCNF | 204827_s_at | U17105 | NM_001761 | 899 | 2.31 | 1.47E-04 |
| RECQL4 | 1553015_a_at | NM_004260 | NM_004260 | 9401 | 2.31 | 2.02E-04 |
| CDR2 | 209501_at | AL582414 | NM_001802 | 1039 | 2.31 | 1.41E-05 |
| HGF | 210997_at | M77227 | NM_000601 | 3082 | 2.30 | 3.55E-03 |
| | | | NM_00101093 1 | | | |
| | | | NM_00101093 2 | | | |
| | | | NM_00101093 3 | | | |
| | | | NM_00101093 4 | | | |
| WHSC1 | 209052_s_at | BF111870 | NM_007331 | 7468 | 2.30 | 2.39E-03 |
| | | | NM_014919 | | | |
| | | | NM_133330 | | | |
| | | | NM_133331 | | | |
| | | | NM_133332 | | | |
| | | | NM_133333 | | | |
| | | | NM_133334 | | | |
| | | | NM_133335 | | | |
| | | | NM_133336 | | | |
| CDCA7 | 230060_at | AI277642 | NM_031942 | 83879 | 2.30 | 9.28E-03 |
| | | | NM_145810 | | | |
| SNAI2 | 213139_at | AI572079 | NM_003068 | 6591 | 2.30 | 9.13E-04 |
| NM_004260 | 213520_at | NM_004260 | --- | --- | 2.30 | 1.47E-04 |
| DIRAS3 | 215506_s_at | AK021882 | NM_004675 | 9077 | 2.30 | 2.88E-03 |
| FLJ32363 | 229886_at | AA502768 | NM_198566 | 375444 | 2.30 | 4.42E-04 |
| KCNJ4 | 208359_s_at | NM_004981 | NM_004981 | 3761 | 2.30 | 6.70E-04 |
| | | | NM_152868 | | | |
| PLCXD2 | 235230_at | AW170015 | NM_153268 | 257068 | 2.29 | 2.14E-03 |
| GALNAC4S-6S | 203066_at | NM_014863 | NM_015892 | 51363 | 2.29 | 8.81E-05 |
| TREX2 | 207891_s_at | NM_017518 | --- | 11219 | 2.29 | 6.18E-05 |
| DHFR | 202534_x_at | NM_000791 | NM_000791 | 1719 | 2.29 | 3.70E-05 |
| dJ383J4.3 | 232065_x_at | N29457 | NM_033319 | 91687 | 2.29 | 1.75E-05 |
| ITGA9 | 227297_at | AI479176 | NM_002207 | 3680 | 2.29 | 1.72E-03 |
| NUP43 | 238474_at | BF978064 | NM_024647 | 348995 | 2.28 | 1.07E-03 |
| | | | NM_198887 | | | |
| SSB4 | 229929_at | AL563476 | NM_080862 | 92369 | 2.28 | 5.85E-04 |
| GNG12 | 212294_at | BG111761 | NM_018841 | 55970 | 2.28 | 2.93E-05 |
| POLD1 | 203422_at | NM_002691 | NM_002691 | 5424 | 2.27 | 8.87E-04 |
| SHMT1 | 209980_s_at | L23928 | NM_004169 | 6470 | 2.27 | 6.54E-05 |
| | | | NM_148918 | | | |
| DMN | 212730_at | AK026420 | NM_015286 | 23336 | 2.27 | 9.70E-05 |
| | | | NM_145728 | | | |
| EB-1 | 227439_at | AI806510 | NM_020140 | 56899 | 2.27 | 3.41E-05 |
| | | | NM_152788 | | | |
| | | | NM_181670 | | | |
| SGOL1 | 231938_at | AK024292 | NM_138484 | 151648 | 2.26 | 2.13E-07 |
| ARG99 | 226931_at | AU151239 | NM_031920 | 83857 | 2.26 | 3.38E-04 |
| | | | NM_175861 | | | |
| U79256 | 37577_at | U79256 | --- | --- | 2.26 | 3.97E-05 |
| HS6ST2 | 1552766_at | NM_147174 | NM_147174 | 90161 | 2.26 | 2.22E-04 |
| | | | NM_147175 | | | |
| AW305300 | 238962_at | AW305300 | --- | --- | 2.25 | 3.89E-05 |
| CCNF | 204826_at | NM_001761 | NM_001761 | 899 | 2.25 | 6.97E-06 |
| CNAP1 | 201774_s_at | AK022511 | NM_014865 | 9918 | 2.25 | 6.86E-05 |
| AA019836 | 241733_at | AA019836 | --- | --- | 2.25 | 3.30E-04 |
| SPAG6 | 210032_s_at | AI651156 | NM_012443 | 9576 | 2.24 | 1.37E-03 |
| | | | NM_172242 | | | |
| PEX13 | 1558163_at | BC040953 | NM_002618 | 5194 | 2.24 | 3.22E-04 |
| AW953794 | 230968_at | AW953794 | --- | --- | 2.24 | 1.52E-03 |
| TBPIP | 213951_s_at | BE964655 | NM_013290 | 29893 | 2.24 | 3.20E-05 |
| | | | NM_016556 | | | |
| SESN3 | 235150_at | AW376955 | NM_144665 | 143686 | 2.24 | 4.11E-05 |
| JPH1 | 229139_at | AI202201 | NM_020647 | 56704 | 2.24 | 3.22E-03 |
| CTSH | 202295_s_at | NM_004390 | NM_004390 | 1512 | 2.24 | 4.20E-03 |
| | | | NM_148979 | | | |
| NRG3 | 229233_at | H05240 | NM_001010848 | 10718 | 2.24 | 1.44E-03 |
| DPYSL3 | 201431_s_at | NM_001387 | NM_001387 | 1809 | 2.23 | 2.10E-06 |
| RACGAP1 | 222077_s_at | AU153848 | NM_013277 | 29127 | 2.23 | 3.69E-06 |
| BF222929 | 242346_x_at | BF222929 | --- | --- | 2.23 | 1.34E-03 |
| FBXO5 | 218875_s_at | NM_012177 | NM_012177 | 26271 | 2.23 | 3.85E-05 |
| LOC143381 | 227550_at | AW242720 | --- | 143381 | 2.23 | 7.49E-03 |
| KIAA0056 | 212789_at | AI796581 | NM_015261 | 23310 | 2.22 | 1.47E-05 |
| GSG2 | 223759_s_at | AB039834 | NM_031965 | 83903 | 2.22 | 7.72E-05 |
| ZNF537 | 223392_s_at | BF510588 | NM_020856 | 57616 | 2.22 | 4.48E-06 |
| ADAMTS9 | 1554697_at | AF488803 | NM_020249 | 56999 | 2.21 | 5.03E-04 |
| | | | NM_182920 | | | |
| | | | NM_182921 | | | |
| CHAF1A | 203975_s_at | BF000239 | NM_005483 | 10036 | 2.21 | 6.39E-04 |
| TEAD4 | 41037_at | U63824 | NM_003213 | 7004 | 2.21 | 6.00E-03 |
| | | | NM_201441 | | | |
| | | | NM_201443 | | | |
| PTER | 218967_s_at | NM_030664 | NM_00100148 4 | 9317 | 2.21 | 8.86E-03 |
| | | | NM_030664 | | | |
| ARHGAP20 | 228368_at | AI936560 | NM_020809 | 57569 | 2.20 | 2.69E-04 |
| GMNN | 218350_s_at | NM_015895 | NM_015895 | 51053 | 2.20 | 1.84E-05 |
| RAD18 | 224200_s_at | AB035274 | NM_020165 | 56852 | 2.20 | 8.40E-08 |
| CCNE2 | 211814_s_at | AF112857 | NM_004702 | 9134 | 2.20 | 8.82E-03 |
| | | | NM_057735 | | | |
| | | | NM_057749 | | | |
| RAD18 | 223417_at | AF169796 | NM_020165 | 56852 | 2.19 | 2.68E-06 |
| HGF | 210755_at | U46010 | NM_000601 | 3082 | 2.19 | 4.99E-03 |
| | | | NM_00101093 1 | | | |
| | | | NM_00101093 2 | | | |
| | | | NM_00101093 3 | | | |
| | | | NM_00101093 4 | | | |
| ALCAM | 1569362_at | BC041127 | NM_001627 | 214 | 2.19 | 3.28E-04 |
| RFC3 | 204127_at | BC000149 | NM_002915 | 5983 | 2.19 | 1.51E-04 |
| | | | NM_181558 | | | |
| KIF22 | 216969_s_at | AC002301 | NM_007317 | 3835 | 2.18 | 7.12E-04 |
| SALL4 | 229661_at | NM_020436 | NM_020436 | 57167 | 2.18 | 3.50E-03 |
| BNC2 | 235723_at | AA843242 | NM_017637 | 54796 | 2.18 | 6.54E-03 |
| VMP | 239293_at | R38624 | NM_080723 | 140767 | 2.18 | 7.44E-03 |
| AU154486 | 213253_at | AU154486 | --- | --- | 2.18 | 1.78E-04 |
| EBF | 229487_at | W73890 | NM_024007 | 1879 | 2.17 | 1.69E-03 |
| CDC25A | 204696_s_at | NM_001789 | NM_001789 | 993 | 2.17 | 7.52E-04 |
| | | | NM_201567 | | | |
| FLJ23091 | 221958_s_at | AA775681 | NM_00100229 2 | 79971 | 2.17 | 1.84E-06 |
| | | | NM_024911 | | | |
| MGC33846 | 231430_at | AW205640 | NM_175885 | 220382 | 2.17 | 4.57E-05 |
| BG028209 | 230521_at | BG028209 | --- | --- | 2.17 | 1.18E-04 |
| FLJ20920 | 218844_at | NM_025149 | NM_025149 | 80221 | 2.16 | 2.85E-04 |
| AF131796 | 231916_at | AF131796 | --- | --- | 2.16 | 7.81E-05 |
| RFC5 | 203209_at | BC001866 | NM_007370 | 5985 | 2.16 | 3.16E-06 |
| | | | NM_181578 | | | |
| ATP2B4 | 212136_at | AW517686 | NM_00100139 6 | 493 | 2.16 | 8.96E-03 |
| | | | NM_001684 | | | |
| AI653240 | 240247_at | AI653240 | XM_496059 | 440261 | 2.16 | 3.06E-03 |
| SNCAIP | 219511_s_at | NM_005460 | NM_005460 | 9627 | 2.15 | 6.77E-04 |
| DHFR | 48808_at | AI144299 | NM_000791 | 1719 | 2.14 | 6.65E-05 |
| KCNN2 | 220116_at | NM_021614 | NM_021614 | 3781 | 2.14 | 8.09E-03 |
| | | | NM_170775 | | | |
| AI144156 | 238670_at | AI144156 | --- | --- | 2.14 | 4.87E-06 |
| C15orf20 | 228252_at | AF108138 | NM_025049 | 80119 | 2.14 | 6.49E-04 |
| AA287223 | 238748_at | AA287223 | --- | --- | 2.13 | 7.76E-04 |
| CKS1B | 201897_s_at | NM_001826 | NM_001826 | 1163 | 2.13 | 2.30E-06 |
| RHEBL1 | 1553713_a_at | NM_144593 | NM_144593 | 121268 | 2.13 | 1.90E-05 |
| PVRL3 | 213325_at | AA129716 | NM_015480 | 25945 | 2.13 | 3.03E-03 |
| H2AFX | 212525_s_at | AA760862 | NM_002105 | 3014 | 2.13 | 2.86E-04 |
| XRCC4 | 205071_x_at | AB017445 | NM_003401 | 7518 | 2.13 | 6.87E-07 |
| | | | NM_022406 | | | |
| | | | NM_022550 | | | |
| STK17A | 202695_s_at | NM_004760 | NM_004760 | 9263 | 2.12 | 4.96E-04 |
| MCM2 | 202107_s_at | NM_004526 | NM_004526 | 4171 | 2.12 | 7.65E-06 |
| DHFR | 202532_s_at | BC000192 | NM_000791 | 1719 | 2.12 | 1.71E-04 |
| INCENP | 219769_at | NM_020238 | NM_020238 | 3619 | 2.12 | 7.09E-03 |
| CBLN1 | 205747_at | NM_004352 | NM_004352 | 869 | 2.11 | 1.51E-03 |
| ZNF545 | 1555793_a_at | AL834267 | NM_133466 | 284406 | 2.11 | 5.39E-07 |
| GGH | 203560_at | NM_003878 | NM_003878 | 8836 | 2.10 | 7.91E-06 |
| FLJ23091 | 228950_s_at | AL534095 | NM_00100229 2 | 79971 | 2.10 | 5.48E-05 |
| | | | NM_024911 | | | |
| TLE3 | 212769_at | AI567426 | NM_005078 | 7090 | 2.09 | 4.14E-03 |
| BE464799 | 214036_at | BE464799 | --- | --- | 2.09 | 9.39E-04 |
| ANP32E | 208103_s_at | NM_030920 | NM_030920 | 81611 | 2.09 | 1.57E-05 |
| NXPH1 | 232377_at | N62902 | NM_152745 | 30010 | 2.08 | 8.71E-05 |
| CDC25A | 1555772_a_at | AY137580 | NM_001789 | 993 | 2.08 | 1.17E-03 |
| | | | NM_201567 | | | |
| PPAP2B | 212226_s_at | AA628586 | NM_003713 | 8613 | 2.08 | 2.24E-03 |
| | | | NM_177414 | | | |
| FABP6 | 210445_at | U19869 | NM_001445 | 2172 | 2.08 | 2.58E-03 |
| T96523 | 237469_at | T96523 | --- | --- | 2.08 | 5.67E-03 |
| KIAA0286 | 212621_at | AB006624 | NM_015257 | 23306 | 2.08 | 1.03E-05 |
| DTYMK | 203270_at | NM_012145 | NM_012145 | 1841 | 2.08 | 4.74E-05 |
| NRM | 225592_at | D81048 | NM_007243 | 11270 | 2.08 | 4.53E-05 |
| FLJ25067 | 243507_s_at | AV715251 | NM_152504 | 149840 | 2.08 | 2.73E-04 |
| ZNF215 | 220214_at | NM_013250 | NM_013250 | 7762 | 2.08 | 6.34E-05 |
| SEMA6A | 220454_s_at | NM_020796 | NM_020796 | 57556 | 2.07 | 4.90E-04 |
| SEMA6A | 223449_at | AF225425 | NM_020796 | 57556 | 2.07 | 1.30E-03 |
| PPIL5 | 235113_at | AA742244 | NM_152329 | 122769 | 2.07 | 2.13E-05 |
| | | | NM_203466 | | | |
| | | | NM_203467 | | | |
| SMC4L1 | 201664_at | AL136877 | NM_00100279 9 | 10051 | 2.06 | 2.53E-06 |
| | | | NM_00100280 0 | | | |
| | | | NM_005496 | | | |
| MPHOSPH1 | 205235_s_at | NM_016195 | NM_016195 | 9585 | 2.06 | 3.99E-06 |
| SKP2 | 210567_s_at | BC001441 | NM_005983 | 6502 | 2.06 | 9.75E-05 |
| | | | NM_032637 | | | |
| ZNF537 | 223393_s_at | AL136805 | NM_020856 | 57616 | 2.06 | 1.76E-04 |
| PLEKHK1 | 230469_at | AW665138 | NM_145307 | 219790 | 2.06 | 6.70E-04 |
| ALCAM | 201951_at | BF242905 | NM_001627 | 214 | 2.06 | 1.36E-03 |
| AI733341 | 240911_at | AI733341 | --- | --- | 2.06 | 4.21E-04 |
| C14orf94 | 218383_at | NM_017815 | NM_017815 | 54930 | 2.05 | 4.04E-05 |
| HES6 | 226446_at | AW249678 | NM_018645 | 55502 | 2.05 | 1.20E-04 |
| EAF2 | 219551_at | NM_018456 | NM_018456 | 55840 | 2.05 | 1.74E-04 |
| GJA7 | 208460_at | NM_005497 | NM_005497 | 10052 | 2.05 | 2.33E-03 |
| PHF21B | 1560091_a_at | AK092243 | NM_138415 | 112885 | 2.04 | 6.65E-04 |
| SEMA6A | 225660_at | W92748 | NM_020796 | 57556 | 2.04 | 7.90E-04 |
| BF593263 | 228018_at | BF593263 | --- | --- | 2.03 | 4.92E-03 |
| FLJ20647 | 218802_at | NM_017918 | NM_017918 | 55013 | 2.03 | 1.70E-03 |
| C20orf58 | 228017_s_at | BF593263 | NM_152864 | 128414 | 2.03 | 1.08E-04 |
| NET1 | 201830_s_at | NM_005863 | NM_005863 | 10276 | 2.03 | 1.27E-03 |
| FNDC5 | 226097_at | AI686488 | NM_153756 | 252995 | 2.03 | 1.40E-03 |
| HSDL2 | 209513_s_at | BC004331 | NM_032303 | 84263 | 2.03 | 3.49E-05 |
| NRXN1 | 209914_s_at | AW149405 | NM_004801 | 9378 | 2.02 | 6.83E-03 |
| | | | NM_138735 | | | |
| CXorf34 | 205238_at | NM_024917 | NM_024917 | 79979 | 2.02 | 5.46E-03 |
| SYTL4 | 227703_s_at | AL391688 | NM_080737 | 94121 | 2.02 | 3.25E-04 |
| FGFR2 | 211401_s_at | AB030078 | NM_000141 | 2263 | 2.01 | 3.92E-04 |
| | | | NM_022969 | | | |
| | | | NM_022970 | | | |
| | | | NM_022971 | | | |
| | | | NM_022972 | | | |
| | | | NM_022973 | | | |
| | | | NM_022974 | | | |
| | | | NM_022975 | | | |
| | | | NM_022976 | | | |
| | | | NM_023028 | | | |
| | | | NM_023029 | | | |
| | | | NM_023030 | | | |
| | | | NM_023031 | | | |
| LOC440288 | 239792_at | N75594 | XM_496075 | 440288 | 2.01 | 5.78E-04 |
| TEX15 | 232760_at | AL133653 | NM_031271 | 56154 | 2.00 | 9.86E-04 |
| C6orf189 | 228875_at | AI540210 | --- | 221303 | 2.00 | 1.16E-05 |
| NSE1 | 229546_at | AI378035 | NM_145175 | 151354 | 2.00 | 2.29E-04 |
| KLHL13 | 227875_at | AB037730 | NM_033495 | 90293 | 2.00 | 3.06E-06 |
| ARHGAP24 | 230803_s_at | AI761947 | NM_031305 | 83478 | 2.00 | 2.48E-04 |
| RFC4 | 204023_at | NM_002916 | NM_002916 | 5984 | 1.99 | 1.39E-05 |
| | | | NM_181573 | | | |
| TFDP2 | 203589_s_at | NM_006286 | NM_006286 | 7029 | 1.99 | 1.73E-06 |
| THBS4 | 204776_at | NM_003248 | NM_003248 | 7060 | 1.99 | 4.42E-03 |
| EMILIN3 | 228307_at | AL137580 | NM_052846 | 90187 | 1.99 | 6.67E-03 |
| SESN3 | 242906_at | BE787063 | NM_144665 | 143686 | 1.99 | 2.42E-04 |
| TLE4 | 216997_x_at | AL358975 | NM_007005 | 7091 | 1.99 | 1.02E-02 |
| MYH11 | 227249_at | AI857685 | NM_002474 | 4629 | 1.99 | 3.80E-04 |
| | | | NM_022844 | | | |
| SMTN | 209427_at | AF064238 | NM_006932 | 6525 | 1.99 | 3.97E-03 |
| | | | NM_134269 | | | |
| | | | NM_134270 | | | |
| FLJ11127 | 219694_at | NM_019018 | NM_019018 | 54491 | 1.99 | 1.47E-03 |
| AA156721 | 201952_at | AA156721 | --- | --- | 1.98 | 1.86E-04 |
| LOC338773 | 227300_at | AL521682 | NM_181724 | 338773 | 1.98 | 1.37E-03 |
| SDC1 | 201286_at | Z48199 | NM_00100694 6 | 6382 | 1.98 | 1.62E-04 |
| | | | NM_002997 | | | |
| LYN | 202625_at | AI356412 | NM_002350 | 4067 | 1.98 | 1.63E-05 |
| TLE3 | 212770_at | AW873621 | NM_005078 | 7090 | 1.98 | 1.14E-04 |
| LOX | 204298_s_at | NM_002317 | NM_002317 | 4015 | 1.96 | 7.97E-04 |
| KIAA0286 | 212619_at | AW205215 | NM_015257 | 23306 | 1.96 | 2.89E-06 |
| LHFPL2 | 212658_at | N66633 | NM_005779 | 10184 | 1.96 | 3.29E-05 |
| C11orf8 | 205413_at | NM_001584 | NM_001584 | 744 | 1.96 | 1.73E-06 |
| Cep70 | 1554489_a_at | BC016050 | NM_024491 | 80321 | 1.96 | 2.05E-06 |
| GPR155 | 231166_at | AI733474 | NM_152529 | 151556 | 1.96 | 8.30E-05 |
| POLA | 204835_at | NM_016937 | NM_016937 | 5422 | 1.96 | 2.08E-04 |
| ZAK | 225665_at | AI129320 | NM_016653 | 51776 | 1.96 | 2.36E-06 |
| | | | NM_133646 | | | |
| DKFZP564K196 | 223170_at | AF132000 | NM_015544 | 26022 | 1.96 | 2.12E-05 |
| TPST2 | 204079_at | NM_003595 | NM_00100856 6 | 8459 | 1.95 | 1.43E-03 |
| | | | NM_003595 | | | |
| TRIP | 205598_at | NM_005879 | NM_005879 | 10293 | 1.95 | 1.00E-05 |
| TPTE | 233879_at | AL137389 | NM_013315 | 7179 | 1.94 | 3.31E-03 |
| | | | NM_199259 | | | |
| | | | NM_199260 | | | |
| | | | NM_199261 | | | |
| FLJ10408 | 220535_at | NM_018088 | NM_018088 | 55138 | 1.94 | 9.00E-03 |
| DNAPTP6 | 222154_s_at | AK002064 | NM_015535 | 26010 | 1.94 | 2.39E-07 |
| DKFZp434P055 | 232398_at | AK001064 | NM_173466 | 91531 | 1.93 | 5.72E-03 |
| FBXO5 | 234863_x_at | AK026197 | NM_012177 | 26271 | 1.93 | 2.02E-05 |
| STRA6 | 1569335_a_at | BC015881 | NM_022369 | 64220 | 1.93 | 3.41E-04 |
| PASK | 213534_s_at | D50925 | NM_015148 | 23178 | 1.93 | 6.33E-04 |
| DRF1 | 238508_at | BG026951 | NM_025104 | 80174 | 1.93 | 4.54E-04 |
| | | | NM_145663 | | | |
| R71596 | 229580_at | R71596 | --- | --- | 1.93 | 8.51E-03 |
| TTF2 | 204407 _at | AF080255 | NM_003594 | 8458 | 1.93 | 3.06E-03 |
| KIAA0635 | 206003_at | NM_014645 | NM_025009 | 9662 | 1.93 | 1.09E-05 |
| OLFML3 | 218162_at | NM_020190 | NM_020190 | 56944 | 1.93 | 7.36E-03 |
| GPSM2 | 221922_at | AW195581 | NM_013296 | 29899 | 1.92 | 1.45E-05 |
| AW022607 | 226806_s_at | AW022607 | --- | --- | 1.92 | 6.78E-03 |
| OPN3 | 219032_x_at | NM_014322 | NM_014322 | 23596 | 1.92 | 2.34E-03 |
| DTYMK | 1553983_at | AF258562 | NM_012145 | 1841 | 1.92 | 1.06E-04 |
| NY-REN-41 | 226287_at | AI458313 | NM_080654 | 91057 | 1.92 | 1.77E-04 |
| RGMA | 223468_s_at | AL136826 | NM_020211 | 56963 | 1.91 | 2.31E-03 |
| EBF | 232204_at | AF208502 | NM_024007 | 1879 | 1.91 | 4.36E-05 |
| C9orf140 | 225777_at | AW250904 | NM_178448 | 89958 | 1.91 | 8.55E-05 |
| HPCA | 205454_at | BC001777 | NM_002143 | 3208 | 1.91 | 2.09E-04 |
| RFC2 | 1053_at | M87338 | NM_002914 | 5982 | 1.91 | 2.20E-06 |
| | | | NM_181471 | | | |
| GUCY1B3 | 203817_at | W93728 | NM_000857 | 2983 | 1.91 | 1.10E-03 |
| SEZ6L | 207873_x_at | NM_021115 | NM_021115 | 23544 | 1.90 | 4.82E-07 |
| CUGBP2 | 202156_s_at | N36839 | NM_006561 | 10659 | 1.90 | 8.07E-04 |
| AF15Q14 | 1552682_a_at | NM_144508 | NM_020380 | 57082 | 1.90 | 2.18E-04 |
| | | | NM_144508 | | | |
| | | | NM_170589 | | | |
| CENPJ | 223513_at | AF139625 | NM_018451 | 55835 | 1.90 | 1.64E-05 |
| SEZ6L | 211894_x_at | AB041736 | NM_021115 | 23544 | 1.90 | 1.44E-05 |
| SDC1 | 201287_s_at | NM_002997 | NM_00100694 6 | 6382 | 1.90 | 6.29E-05 |
| | | | NM_002997 | | | |
| PLAUR | 210845_s_at | U08839 | NM_00100537 6 | 5329 | 1.90 | 5.08E-03 |
| | | | NM_00100537 7 | | | |
| | | | NM_002659 | | | |
| CBLN2 | 242301_at | R60224 | NM_182511 | 147381 | 1.90 | 4.25E-03 |
| DCLRE1B | 219490_s_at | NM_022836 | NM_022836 | 64858 | 1.89 | 2.44E-04 |
| ZFHX4 | 219779_at | NM_024721 | NM_024721 | 79776 | 1.89 | 6.77E-05 |
| CYBA | 203028_s_at | NM_000101 | NM_000101 | 1535 | 1.89 | 3.82E-05 |
| DOCK10 | 219279_at | NM_017718 | NM_014689 | 55619 | 1.88 | 9.11E-04 |
| MCM3 | 201555_at | NM_002388 | NM_002388 | 4172 | 1.88 | 1.06E-05 |
| ECT2 | 234992_x_at | BG170335 | NM_018098 | 1894 | 1.87 | 2.46E-04 |
| ADA | 204639_at | NM_000022 | NM_000022 | 100 | 1.87 | 8.91E-04 |
| SOX4 | 213668_s_at | AI989477 | NM_003107 | 6659 | 1.87 | 2.08E-04 |
| STN2 | 227461_at | AA632295 | NM_033104 | 85439 | 1.87 | 6.01E-04 |
| HS6ST2 | 230030_at | AI767756 | NM_147174 | 90161 | 1.87 | 6.20E-07 |
| | | | NM_147175 | | | |
| NAGA | 1555041_a_at | M29276 | NM_000262 | 4668 | 1.87 | 2.11E-04 |
| GCHFR | 204867_at | NM_005258 | NM_005258 | 2644 | 1.86 | 8.27E-03 |
| KIAA1596 | 234733_s_at | AK001672 | XM_048128 | 57697 | 1.86 | 9.32E-05 |
| RFC2 | 203696_s_at | NM_002914 | NM_002914 | 5982 | 1.86 | 8.69E-06 |
| | | | NM_181471 | | | |
| HUNK | 1555935_s_at | T58129 | NM_014586 | 30811 | 1.86 | 2.81E-04 |
| PEX13 | 1558164_s_at | BC040953 | NM_002618 | 5194 | 1.85 | 9.05E-05 |
| DTYMK | 1553984_s_at | AF258562 | NM_012145 | 1841 | 1.85 | 4.47E-04 |
| TCF3 | 209151_x_at | AA768906 | NM_003200 | 6929 | 1.85 | 2.01E-04 |
| LOC91614 | 228293_at | AJ245600 | NM_139160 | 91614 | 1.85 | 4.22E-03 |
| CDCA4 | 218399_s_at | NM_017955 | NM_017955 | 55038 | 1.84 | 3.08E-04 |
| | | | NM_145701 | | | |
| Cep152 | 215882_at | AK025247 | NM_014985 | 22995 | 1.84 | 1.38E-03 |
| TIMELESS | 203046_s_at | NM_003920 | NM_003920 | 8914 | 1.84 | 2.25E-04 |
| ZNF505 | 208119_s_at | NM_031218 | NM_00100412 6 | 81931 | 1.84 | 1.87E-04 |
| | | | NM_031218 | | | |
| SESN3 | 242899_at | N78412 | NM_144665 | 143686 | 1.84 | 2.44E-03 |
| FLJ14146 | 218546_at | NM_024709 | NM_024709 | 79762 | 1.83 | 4.55E-04 |
| PHF19 | 225533_at | AL117477 | NM_00100993 6 | 26147 | 1.83 | 6.38E-04 |
| | | | NM_015651 | | | |
| C19orf32 | 226597_at | AI348159 | NM_138393 | 92840 | 1.83 | 5.57E-05 |
| SATB2 | 213435_at | AB028957 | NM_015265 | 23314 | 1.83 | 9.07E-03 |
| PASK | 216945_x_at | U79240 | NM_015148 | 23178 | 1.83 | 1.22E-03 |
| MSH5 | 210410_s_at | AF034759 | NM_002441 | 4439 | 1.83 | 2.29E-04 |
| | | | NM_025259 | | | |
| | | | NM_172165 | | | |
| | | | NM_172166 | | | |
| CBLB | 227900_at | AV701750 | NM_170662 | 868 | 1.82 | 5.23E-03 |
| AI984541 | 235434_at | AI984541 | --- | --- | 1.82 | 1.15E-03 |
| NEDD1 | 1552417_a_at | NM_152905 | NM_152905 | 121441 | 1.82 | 2.63E-03 |
| KDELR3 | 204017_at | NM_006855 | NM_006855 | 11015 | 1.82 | 4.53E-03 |
| | | | NM_016657 | | | |
| TYMS | 228989_at | AW291159 | NM_001071 | 7298 | 1.82 | 3.40E-03 |
| PCNXL2 | 39650_s_at | AB007895 | NM_014801 | 80003 | 1.82 | 9.16E-05 |
| | | | NM_024938 | | | |
| HS6ST2 | 1552767_a_at | NM_147174 | NM_147174 | 90161 | 1.82 | 8.21E-07 |
| | | | NM_147175 | | | |
| KIF23 | 244427_at | AW192521 | NM_004856 | 9493 | 1.81 | 8.25E-03 |
| | | | NM_138555 | | | |
| EBF | 227646_at | BG435302 | NM_024007 | 1879 | 1.81 | 2.17E-05 |
| BE466145 | 227041_at | BE466145 | --- | --- | 1.81 | 5.47E-05 |
| KCNJ8 | 205303_at | BF514158 | NM_004982 | 3764 | 1.81 | 1.32E-06 |
| TRIP13 | 204033_at | NM_004237 | NM_004237 | 9319 | 1.80 | 1.65E-04 |
| SMAD3 | 218284_at | NM_015400 | NM_005902 | 4088 | 1.80 | 2.69E-03 |
| AI582818 | 229053_at | AI582818 | --- | --- | 1.80 | 1.03E-03 |
| TBC1D1 | 212350_at | AB029031 | NM_015173 | 23216 | 1.80 | 1.24E-04 |
| FEN1 | 204768_s_at | NM_004111 | NM_004111 | 2237 | 1.79 | 7.12E-05 |
| HELLS | 223556_at | AF155827 | NM_018063 | 3070 | 1.79 | 4.87E-04 |
| SFI1 | 36545_s_at | AB011114 | NM_00100746 7 | 9814 | 1.78 | 8.13E-03 |
| | | | NM_014775 | | | |
| WASPIP | 202664_at | AW058622 | NM_003387 | 7456 | 1.78 | 6.38E-03 |
| RASEF | 235144_at | AA826324 | NM_152573 | 158158 | 1.78 | 9.50E-04 |
| NOS1 | 239132_at | BE299830 | NM_000620 | 4842 | 1.78 | 9.36E-04 |
| DNMT3B | 220668_s_at | NM_006892 | NM_006892 | 1789 | 1.78 | 3.32E-05 |
| | | | NM_175848 | | | |
| | | | NM_175849 | | | |
| | | | NM_175850 | | | |
| H10545 | 242137_at | H10545 | --- | --- | 1.77 | 7.47E-04 |
| PKP4 | 214874_at | AL050364 | NM_00100547 6 | 8502 | 1.76 | 7.22E-04 |
| | | | NM_003628 | | | |
| T86830 | 227835_at | T86830 | XM_374317 | 389831 | 1.76 | 2.76E-03 |
| HSDL2 | 209512_at | BC004331 | NM_032303 | 84263 | 1.76 | 1.97E-03 |
| AI146450 | 228073_at | AI146450 | --- | --- | 1.76 | 4.60E-06 |
| BC041029 | 1569025_s_at | BC041029 | XM_371697 | 389211 | 1.76 | 4.47E-06 |
| AMOTL1 | 225459_at | AU157155 | NM_130847 | 154810 | 1.76 | 8.01E-05 |
| C2orf31 | 221245_s_at | NM_030804 | --- | 81561 | 1.75 | 5.43E-05 |
| STK3 | 204068_at | NM_006281 | NM_006281 | 6788 | 1.75 | 3.93E-05 |
| FAM19A4 | 242348_at | AA757457 | NM_00100552 7 | 151647 | 1.75 | 6.22E-07 |
| | | | NM_182522 | | | |
| CHTF18 | 226569_s_at | AK024476 | NM_022092 | 63922 | 1.75 | 5.44E-06 |
| SOX4 | 201417_at | AL136179 | NM_003107 | 6659 | 1.75 | 6.42E-05 |
| RRM1 | 201476_s_at | AI692974 | NM_001033 | 6240 | 1.74 | 5.08E-04 |
| AV717623 | 212738_at | AV717623 | --- | --- | 1.74 | 2.87E-04 |
| FGD4 | 227948_at | AI949549 | NM_139241 | 121512 | 1.74 | 3.49E-04 |
| NTRK2 | 221796_at | AA707199 | NM_00100709 7 | 4915 | 1.74 | 2.14E-04 |
| | | | NM_006180 | | | |
| SOX4 | 201416_at | BG528420 | NM_003107 | 6659 | 1.73 | 5.20E-04 |
| SESN3 | 225123_at | BE883841 | NM_144665 | 143686 | 1.73 | 4.70E-04 |
| C6orf139 | 219294_at | NM_018132 | NM_018132 | 55166 | 1.73 | 3.59E-05 |
| SRGAP1 | 233888_s_at | AK023899 | NM_020762 | 57522 | 1.73 | 7.93E-04 |
| CDK6 | 235287_at | AW192700 | NM_001259 | 1021 | 1.73 | 2.32E-03 |
| TNFAIP8 | 208296_x_at | NM_014350 | NM_014350 | 25816 | 1.73 | 9.57E-04 |
| TUBG1 | 201714_at | NM_001070 | NM_001070 | 7283 | 1.73 | 1.25E-04 |
| LOC221810 | 221910_at | BF131965 | --- | 221810 | 1.73 | 1.75E-04 |
| AI220427 | 230696_at | AI220427 | --- | --- | 1.73 | 4.25E-03 |
| AI685824 | 244565_at | AI685824 | XM_370580 | 387716 | 1.73 | 2.27E-04 |
| AL566034 | 224944_at | AL566034 | --- | --- | 1.72 | 1.01E-05 |
| WEE1 | 212533_at | X62048 | NM_003390 | 7465 | 1.72 | 1.55E-05 |
| RRM1 | 201477_s_at | NM_001033 | NM_001033 | 6240 | 1.72 | 1.46E-04 |
| EZH2 | 203358_s_at | NM_004456 | NM_004456 | 2146 | 1.72 | 4.30E-06 |
| | | | NM_152998 | | | |
| WHSC1 | 222777_s_at | AI770166 | NM_007331 | 7468 | 1.72 | 4.09E-04 |
| | | | NM_014919 | | | |
| | | | NM_133330 | | | |
| | | | NM_133331 | | | |
| | | | NM_133332 | | | |
| | | | NM_133333 | | | |
| | | | NM_133334 | | | |
| | | | NM_133335 | | | |
| | | | NM_133336 | | | |
| H2AFX | 205436_s_at | NM_002105 | NM_002105 | 3014 | 1.72 | 4.53E-05 |
| DKFZP434L011 | 227818_at | AL133609 | NM_022778 | 64793 | 1.72 | 2.15E-04 |
| C9orf100 | 230522_s_at | BG028209 | NM_032818 | 84904 | 1.72 | 3.63E-04 |
| ANKRD9 | 230972_at | AW194999 | NM_152326 | 122416 | 1.71 | 5.46E-06 |
| ACTA2 | 200974_at | NM_001613 | NM_001613 | 59 | 1.71 | 1.89E-03 |
| GALNT10 | 212256_at | BE906572 | NM_017540 | 55568 | 1.71 | 2.83E-03 |
| | | | NM_198321 | | | |
| AI826125 | 229866_at | AI826125 | --- | --- | 1.71 | 1.06E-03 |
| LRRC20 | 218550_s_at | NM_018205 | NM_018205 | 55222 | 1.70 | 6.18E-03 |
| | | | NM_018239 | | | |
| | | | NM_207119 | | | |
| C6orf213 | 1562443_at | AK095527 | NM_001010852 | 134829 | 1.70 | 2.22E-07 |
| EDG2 | 204037_at | BF055366 | NM_001401 | 1902 | 1.69 | 2.85E-03 |
| | | | NM_057159 | | | |
| DLL3 | 222898_s_at | BE350882 | NM_016941 | 10683 | 1.69 | 3.90E-05 |
| | | | NM_203486 | | | |
| TBPIP | 205956_x_at | NM_013290 | NM_013290 | 29893 | 1.69 | 8.09E-05 |
| | | | NM_016556 | | | |
| GFRA2 | 205721_at | U97145 | NM_001495 | 2675 | 1.69 | 3.03E-03 |
| AI140305 | 228849_at | AI140305 | --- | --- | 1.69 | 5.44E-03 |
| MBD2 | 214397_at | AI827820 | NM_003927 | 8932 | 1.68 | 7.38E-05 |
| | | | NM_015832 | | | |
| POLE | 216026_s_at | AL080203 | NM_006231 | 5426 | 1.68 | 1.73E-04 |
| TIMP3 | 201148_s_at | AW338933 | NM_000362 | 7078 | 1.68 | 3.70E-06 |
| RBMS3 | 206767_at | NM_014483 | NM_00100379 2 | 27303 | 1.68 | 5.38E-04 |
| | | | NM_00100379 3 | | | |
| | | | NM_014483 | | | |
| BE670307 | 227955_s_at | BE670307 | --- | --- | 1.68 | 3.67E-04 |
| LAMA4 | 202202_s_at | NM_002290 | NM_002290 | 3910 | 1.68 | 8.97E-03 |
| NUDT1 | 204766_s_at | NM_002452 | NM_002452 | 4521 | 1.68 | 4.44E-03 |
| | | | NM_198948 | | | |
| | | | NM_198949 | | | |
| | | | NM_198950 | | | |
| | | | NM_198952 | | | |
| | | | NM_198953 | | | |
| | | | NM_198954 | | | |
| LOC81691 | 208107_s_at | NM_030941 | NM_030941 | 81691 | 1.68 | 6.55E-05 |
| PKP4 | 201927_s_at | BG292559 | NM_00100547 6 | 8502 | 1.68 | 8.23E-03 |
| | | | NM_003628 | | | |
| AI919519 | 235092_at | AI919519 | --- | --- | 1.68 | 4.50E-04 |
| KPNA2 | 211762_s_at | BC005978 | NM_002266 | 3838 | 1.67 | 1.78E-06 |
| ZNF519 | 1568873_at | BC010705 | NM_145287 | 162655 | 1.67 | 8.56E-03 |
| PELO | 226731_at | AA156873 | NM_015946 | 53918 | 1.67 | 5.20E-03 |
| HIRIP3 | 204504_s_at | NM_003609 | NM_003609 | 8479 | 1.67 | 1.47E-05 |
| RAD54B | 235846_at | AI208616 | NM_012415 | 25788 | 1.67 | 2.55E-03 |
| | | | NM_134434 | | | |
| DKFZP761M151 | 225355_at | AK026748 | --- | 54492 | 1.67 | 5.04E-03 |
| SYN2 | 210247_at | AW139618 | NM_003178 | 6854 | 1.67 | 7.92E-04 |
| | | | NM_133625 | | | |
| SNAG1 | 226683_at | AU146771 | NM_052870 | 112574 | 1.67 | 1.69E-04 |
| CDC25A | 204695_at | AI343459 | NM_001789 | 993 | 1.66 | 4.73E-04 |
| | | | NM_201567 | | | |
| MEST | 202016_at | NM_002402 | NM_002402 | 4232 | 1.66 | 1.55E-04 |
| | | | NM_177524 | | | |
| | | | NM_177525 | | | |
| HPSE | 222881_at | AF155510 | NM_006665 | 10855 | 1.66 | 3.24E-04 |
| AI673553 | 203061_s_at | AI673553 | --- | --- | 1.66 | 1.97E-05 |
| SANG | 232881_at | AI500353 | --- | 149775 | 1.66 | 2.02E-04 |
| HGF | 210998_s_at | M77227 | NM_000601 | 3082 | 1.66 | 6.82E-03 |
| | | | NM_00101093 1 | | | |
| | | | NM_00101093 2 | | | |
| | | | NM_00101093 3 | | | |
| | | | NM_00101093 4 | | | |
| SCD4 | 224901_at | AL571375 | NM_024906 | 79966 | 1.66 | 1.68E-03 |
| RDH10 | 226021_at | AW150720 | NM_172037 | 157506 | 1.66 | 1.99E-03 |
| SYNPO2 | 232119_at | BF984227 | NM_133477 | 171024 | 1.66 | 5.45E-03 |
| LSM4 | 202737_s_at | NM_012321 | NM_012321 | 25804 | 1.65 | 5.27E-05 |
| BE857972 | 243781_at | BE857972 | --- | --- | 1.65 | 7.56E-04 |
| NET1 | 201829_at | AW263232 | NM_005863 | 10276 | 1.65 | 7.05E-05 |
| LYN | 210754_s_at | M79321 | NM_002350 | 4067 | 1.65 | 3.45E-05 |
| DACH1 | 228915_at | AI650353 | NM_004392 | 1602 | 1.65 | 6.68E-03 |
| | | | NM_080759 | | | |
| | | | NM_080760 | | | |
| LIG1 | 202726_at | NM_000234 | NM_000234 | 3978 | 1.65 | 3.00E-04 |
| LYN | 202626_s_at | NM_002350 | NM_002350 | 4067 | 1.64 | 1.87E-05 |
| ST8SIA2 | 221285_at | NM_006011 | NM_006011 | 8128 | 1.64 | 2.88E-03 |
| FLJ23322 | 233250_x_at | AL022313 | NM_024955 | 80020 | 1.64 | 1.54E-04 |
| GNG12 | 1555240_s_at | AF493879 | NM_018841 | 55970 | 1.64 | 9.06E-05 |
| BC025350 | 1569418_at | BC025350 | --- | --- | 1.63 | 1.39E-03 |
| EVE1 | 225162_at | BG285417 | NM_001009555 | 152503 | 1.63 | 1.62E-03 |
| SCML1 | 222747_s_at | BF001786 | NM_006746 | 6322 | 1.63 | 2.63E-04 |
| C20orf100 | 228737_at | AA211909 | NM_032883 | 84969 | 1.63 | 9.04E-04 |
| LMNB1 | 203276_at | NM_005573 | NM_005573 | 4001 | 1.63 | 1.56E-04 |
| TNFSF13B | 223501_at | AW151360 | NM_006573 | 10673 | 1.63 | 2.45E-03 |
| ZAK | 225662_at | H28667 | NM_016653 | 51776 | 1.63 | 2.26E-05 |
| | | | NM_133646 | | | |
| N36085 | 227221_at | N36085 | --- | --- | 1.63 | 2.85E-04 |
| SOX4 | 201418_s_at | NM_003107 | NM_003107 | 6659 | 1.62 | 4.87E-04 |
| LOC342892 | 244640_at | AW440392 | XM_292740 | 342892 | 1.62 | 9.38E-05 |
| MCM4 | 222037_at | AI859865 | NM_005914 | 4173 | 1.62 | 4.15E-03 |
| | | | NM_182746 | | | |
| RAD51C | 206066_s_at | NM_002876 | NM_002876 | 5889 | 1.62 | 2.63E-04 |
| | | | NM_058216 | | | |
| | | | NM_058217 | | | |
| SORT1 | 212807_s_at | BF447105 | NM_002959 | 6272 | 1.62 | 7.88E-04 |
| AV687517 | 228498_at | AV687517 | --- | --- | 1.62 | 4.00E-03 |
| FLJ44635 | 227618_at | AI250910 | NM_207422 | 392490 | 1.62 | 4.08E-03 |
| HMG2L1 | 212597_s_at | AL079310 | NM_00100368 1 | 10042 | 1.62 | 1.26E-04 |
| | | | NM_005487 | | | |
| | | | NM_014250 | | | |
| PLXNA2 | 213030_s_at | AI688418 | NM_025179 | 5362 | 1.62 | 1.87E-04 |
| ST8SIA2 | 239537_at | AW589904 | NM_006011 | 8128 | 1.61 | 3.33E-06 |
| SCML1 | 218793_s_at | NM_006746 | NM_006746 | 6322 | 1.61 | 6.46E-07 |
| KIAA1754L | 240037_at | R37132 | NM_00100894 9 | 150771 | 1.61 | 8.33E-03 |
| | | | NM_178495 | | | |
| COBLL1 | 203642_s_at | NM_014900 | NM_014900 | 22837 | 1.61 | 1.06E-03 |
| N26620 | 227503_at | N26620 | --- | --- | 1.61 | 1.67E-03 |
| RPA3 | 209507_at | BC005264 | NM_002947 | 6119 | 1.61 | 2.30E-04 |
| RODH | 205700_at | NM_003725 | NM_003725 | 8630 | 1.61 | 9.72E-03 |
| CARHSP1 | 224910_at | AL575747 | NM_014316 | 23589 | 1.61 | 7.34E-03 |
| AL049709 | 216548_x_at | AL049709 | --- | --- | 1.61 | 2.85E-04 |
| POLD3 | 212836_at | D26018 | NM_006591 | 10714 | 1.61 | 3.76E-04 |
| BZRP | 202096_s_at | NM_000714 | NM_000714 | 706 | 1.61 | 7.10E-03 |
| | | | NM_007311 | | | |
| LOC286044 | 222662_at | W60806 | --- | 286044 | 1.60 | 2.19E-04 |
| MICAL3 | 229474_at | BF055090 | XM_032996 | 57553 | 1.60 | 4.74E-03 |
| | | | XM_032997 | | | |
| C1QL1 | 205575_at | NM_006688 | NM_006688 | 10882 | 1.60 | 1.17E-03 |
| EGFR | 224999_at | BE878463 | NM_005228 | 1956 | 1.60 | 1.02E-02 |
| | | | NM_201282 | | | |
| | | | NM_201283 | | | |
| | | | NM_201284 | | | |
| AL040051 | 225481_at | AL040051 | --- | --- | 1.60 | 1.85E-03 |
| GLRX | 209276_s_at | AF162769 | NM_002064 | 2745 | 1.59 | 8.29E-03 |
| CDC25C | 217010_s_at | AF277724 | NM_001790 | 995 | 1.59 | 8.33E-03 |
| | | | NM_022809 | | | |
| DNM3 | 209839_at | AL136712 | NM_015569 | 26052 | 1.59 | 3.88E-04 |
| LOC81691 | 215215_s_at | AC004381 | NM_030941 | 81691 | 1.59 | 1.47E-05 |
| SCD4 | 224018_s_at | BC004936 | NM_024906 | 79966 | 1.59 | 5.27E-04 |
| PAG | 225626_at | AK000680 | NM_018440 | 55824 | 1.59 | 7.40E-03 |
| SLC8A1 | 235518_at | AI741439 | NM_021097 | 6546 | 1.58 | 5.49E-04 |
| W93046 | 230068_s_at | W93046 | --- | --- | 1.58 | 3.19E-03 |
| Cep70 | 224150_s_at | AF289495 | NM_024491 | 80321 | 1.58 | 1.32E-03 |
| DACH1 | 205471_s_at | AW772082 | NM_004392 | 1602 | 1.58 | 3.93E-03 |
| | | | NM_080759 | | | |
| | | | NM_080760 | | | |
| FLJ12484 | 219361_s_at | NM_022767 | NM_022767 | 64782 | 1.58 | 3.84E-04 |
| IMP-1 | 223689_at | AF198254 | NM_006546 | 10642 | 1.57 | 2.37E-03 |
| AI697657 | 229128_s_at | AI697657 | --- | --- | 1.57 | 1.62E-05 |
| LOC90139 | 227307_at | AL565381 | NM_130783 | 90139 | 1.57 | 2.80E-03 |
| SEZ6L | 216047_x_at | AL050253 | NM_021115 | 23544 | 1.57 | 1.21E-04 |
| CENPC1 | 204739_at | NM_001812 | NM_001812 | 1060 | 1.57 | 4.21E-04 |
| CKS2 | 204170_s_at | NM_001827 | NM_001827 | 1164 | 1.57 | 6.57E-04 |
| LOC92689 | 213455_at | W87466 | NM_138389 | 92689 | 1.57 | 6.69E-04 |
| ROR1 | 211057_at | BC006374 | NM_005012 | 4919 | 1.57 | 9.11E-04 |
| PHF19 | 227211_at | BE544837 | NM_00100993 6 | 26147 | 1.56 | 3.54E-04 |
| | | | NM_015651 | | | |
| PHF21B | 1562309_s_at | AK092243 | NM_138415 | 112885 | 1.56 | 5.12E-05 |
| USP48 | 229812_at | BE645018 | NM_032236 | 84196 | 1.56 | 4.62E-03 |
| LOC221362 | 213248_at | AL577024 | --- | 221362 | 1.56 | 9.52E-06 |
| CRYZ | 202950_at | NM_001889 | NM_001889 | 1429 | 1.56 | 3.29E-04 |
| GATM | 203178_at | NM_001482 | NM_001482 | 2628 | 1.56 | 1.90E-05 |
| NM_014641 | 203062_s_at | NM_014641 | --- | --- | 1.55 | 5.41E-05 |
| FLJ38964 | 235699_at | H19232 | NM_173527 | 161253 | 1.55 | 4.09E-03 |
| SPA17 | 205406_s_at | NM_017425 | NM_017425 | 53340 | 1.55 | 1.75E-03 |
| NRP1 | 212298_at | BE620457 | NM_003873 | 8829 | 1.55 | 7.51E-03 |
| NXN | 243503_at | AI632740 | NM_022463 | 64359 | 1.55 | 1.68E-04 |
| TCFL5 | 235694_at | N49233 | NM_006602 | 10732 | 1.55 | 7.24E-05 |
| UNG | 202330_s_at | NM_003362 | NM_003362 | 7374 | 1.54 | 5.21E-06 |
| | | | NM_080911 | | | |
| TREX2 | 213334_x_at | BE676218 | --- | 11219 | 1.54 | 6.50E-04 |
| WHSC1 | 209053_s_at | BE793789 | NM_007331 | 7468 | 1.54 | 1.93E-04 |
| | | | NM_014919 | | | |
| | | | NM_133330 | | | |
| | | | NM_133331 | | | |
| | | | NM_133332 | | | |
| | | | NM_133333 | | | |
| | | | NM_133334 | | | |
| | | | NM_133335 | | | |
| | | | NM_133336 | | | |
| ITGB3BP | 205176_s_at | NM_014288 | NM_014288 | 23421 | 1.54 | 1.06E-04 |
| LSM4 | 202736_s_at | AA112507 | NM_012321 | 25804 | 1.54 | 8.14E-05 |
| MPP5 | 226092_at | BF115203 | NM_022474 | 64398 | 1.54 | 7.77E-03 |
| MGC21654 | 214061_at | AI017564 | NM_145647 | 93594 | 1.54 | 4.22E-04 |
| C9orf76 | 218979_at | NM_024945 | NM_024945 | 80010 | 1.54 | 2.48E-04 |
| KLF7 | 238482_at | AL120562 | NM_003709 | 8609 | 1.54 | 5.35E-03 |
| LZTS1 | 47550_at | N21184 | NM_021020 | 11178 | 1.54 | 9.06E-03 |
| SLC8A1 | 238546_at | BF344961 | NM_021097 | 6546 | 1.54 | 1.55E-03 |
| FGD4 | 242445_at | AA296351 | NM_139241 | 121512 | 1.54 | 9.73E-03 |
| ZNF217 | 203739_at | NM_006526 | NM_006526 | 7764 | 1.54 | 4.11E-06 |
| TM4SF14 | 221002_s_at | NM_030927 | NM_030927 | 81619 | 1.54 | 1.44E-03 |
| NAGA | 202943_s_at | M38083 | NM_000262 | 4668 | 1.53 | 1.02E-03 |
| RAI14 | 202052_s_at | NM_015577 | NM_015577 | 26064 | 1.53 | 4.59E-04 |
| PARD6G | 227204_at | AI817448 | NM_032510 | 84552 | 1.53 | 7.67E-04 |
| MMD | 203414_at | NM_012329 | NM_012329 | 23531 | 1.53 | 7.58E-05 |
| C15orf23 | 225300_at | BF792864 | NM_033286 | 90417 | 1.53 | 6.24E-04 |
| ACO1 | 207071_s_at | NM_002197 | NM_002197 | 48 | 1.53 | 6.67E-03 |
| FLJ10514 | 218365_s_at | AI765051 | NM_018122 | 55157 | 1.53 | 2.72E-03 |
| RAB3B | 227123_at | AU156710 | NM_002867 | 5865 | 1.52 | 1.25E-03 |
| TCFL5 | 204849_at | NM_006602 | NM_006602 | 10732 | 1.52 | 8.79E-04 |
| GEMIN4 | 205527_s_at | NM_015487 | NM_015721 | 50628 | 1.52 | 2.20E-03 |
| CBLB | 209682_at | U26710 | NM_170662 | 868 | 1.52 | 9.18E-07 |
| HMG2L1 | 212596_s_at | AJ010070 | NM_00100368 1 | 10042 | 1.52 | 1.65E-04 |
| | | | NM_005487 | | | |
| | | | NM_014250 | | | |
| CARHSP1 | 218384_at | NM_014316 | NM_014316 | 23589 | 1.52 | 1.20E-03 |
| PTPRR | 210675_s_at | U77917 | NM_002849 | 5801 | 1.51 | 6.84E-03 |
| | | | NM_130846 | | | |
| ZNF85 | 1554445_at | BC008688 | NM_003429 | 7639 | 1.51 | 8.78E-03 |
| C20orf58 | 230668_at | AI758937 | NM_152864 | 128414 | 1.51 | 2.35E-04 |
| AK098258 | 1564175_at | AK098258 | --- | --- | 1.51 | 4.42E-03 |
| ADPN | 220675_s_at | NM_025225 | NM_025225 | 80339 | 1.51 | 6.52E-04 |
| RANBP1 | 202483_s_at | NM_002882 | NM_002882 | 5902 | 1.51 | 9.75E-06 |
| CDK6 | 224851_at | AW274756 | NM_001259 | 1021 | 1.51 | 3.51E-04 |
| GPR161 | 206972_s_at | NM_007369 | NM_007369 | 23432 | 1.51 | 3.61E-05 |
| | | | NM_153832 | | | |
| MCFP | 205716_at | NM_018843 | NM_018843 | 55972 | 1.51 | 5.29E-03 |
| RTN4IP1 | 224509_s_at | BC006399 | NM_032730 | 84816 | 1.51 | 1.29E-03 |
| RANGAP1 | 212125_at | NM_002883 | NM_002883 | 5905 | 1.50 | 4.37E-04 |
| BF057492 | 221871_s_at | BF057492 | --- | --- | 1.50 | 2.08E-03 |
| MMD | 244523_at | AW104453 | NM_012329 | 23531 | 1.50 | 4.38E-05 |
| PHF19 | 227212_s_at | BE544837 | NM_00100993 6 | 26147 | 1.50 | 4.91E-05 |
| | | | NM_015651 | | | |
| HMGB3 | 203744_at | NM_005342 | NM_005342 | 3149 | 1.50 | 1.38E-06 |
| RODH | 37512_at | U89281 | NM_003725 | 8630 | 1.50 | 6.29E-03 |
| AW665447 | 230113_at | AW665447 | --- | --- | 1.50 | 8.13E-03 |
| RANGAP1 | 1553535_a_at | NM_002883 | NM_002883 | 5905 | 1.50 | 6.68E-05 |
| PRIM1 | 205053_at | NM_000946 | NM_000946 | 5557 | 1.49 | 7.62E-05 |
| FEN1 | 204767_s_at | BC000323 | NM_004111 | 2237 | 1.49 | 2.41E-04 |
| MAN1A1 | 208116_s_at | NM_005907 | NM_005907 | 4121 | 1.49 | 2.54E-03 |
| SLC1A1 | 213664_at | AW235061 | NM_004170 | 6505 | 1.49 | 1.97E-04 |
| LOC283027 | 1562527_at | AF519622 | --- | 283027 | 1.49 | 6.56E-03 |
| BUB3 | 201457_x_at | AF081496 | NM_00100779 3 | 9184 | 1.49 | 1.77E-05 |
| | | | NM_004725 | | | |
| GPR161 | 230369_at | AI743151 | NM_007369 | 23432 | 1.49 | 2.32E-04 |
| | | | NM_153832 | | | |
| TRIM59 | 235476_at | AW182459 | NM_173084 | 286827 | 1.49 | 9.48E-03 |
| TLE4 | 204872_at | NM_007005 | NM_007005 | 7091 | 1.48 | 3.51E-05 |
| LOC401022 | 242042_s_at | AA406052 | --- | 401022 | 1.48 | 1.50E-03 |
| ZAK | 238613_at | AI475164 | NM_016653 | 51776 | 1.48 | 7.42E-03 |
| | | | NM_133646 | | | |
| DHRS10 | 228713_s_at | AI742586 | NM_016246 | 51171 | 1.48 | 3.31E-03 |
| FLJ10747 | 222673_x_at | AI582192 | NM_018202 | 159090 | 1.48 | 4.86E-05 |
| LOC159090 | | | NM_145284 | 55219 | | |
| MGC13186 | 223272_s_at | BC005102 | NM_032324 | 84284 | 1.47 | 5.80E-03 |
| FLJ11535 | 220798_x_at | NM_024888 | NM_024888 | 79948 | 1.47 | 8.54E-03 |
| C9orf81 | 228774_at | AW268594 | XM_095991 | 84131 | 1.47 | 2.00E-04 |
| DHX35 | 234728_s_at | AL023803 | NM_021931 | 60625 | 1.47 | 9.46E-04 |
| EPOR | 209962_at | M34986 | NM_000121 | 2057 | 1.47 | 4.25E-03 |
| DUT | 208955_at | AB049113 | NM_001948 | 1854 | 1.47 | 1.77E-03 |
| KHDRBS3 | 209781_s_at | AF069681 | NM_006558 | 10656 | 1.47 | 5.70E-06 |
| AA218973 | 237245_at | AA218973 | XM_372330 | 390009 | 1.47 | 2.67E-03 |
| PVT1 | 1558290_a_at | BG200951 | XM_372058 | 441378 | 1.47 | 4.77E-04 |
| LOC441378 | | | XM_499128 | 5820 | | |
| MCM7 | 210983_s_at | AF279900 | NM_005916 | 4176 | 1.47 | 5.48E-05 |
| | | | NM_182776 | | | |
| POLR3K | 218866_s_at | NM_016310 | NM_016310 | 51728 | 1.47 | 2.20E-03 |
| LOC159090 | 225361_x_at | AI348001 | NM_145284 | 159090 | 1.47 | 4.60E-05 |
| CNP | 208912_s_at | BC001362 | NM_033133 | 1267 | 1.47 | 7.89E-04 |
| LOC201725 | 235088_at | AI276663 | NM_001008393 | 201725 | 1.46 | 1.25E-03 |
| CHRNA3 | 211772_x_at | BC006114 | NM_000743 | 1136 | 1.46 | 8.95E-04 |
| LOC126295 | 1554628_at | BC028974 | NM_173480 | 126295 | 1.46 | 8.70E-03 |
| FARP1 | 201911_s_at | NM_005766 | NM_00100171 5 | 10160 | 1.46 | 7.82E-05 |
| | | | NM_005766 | | | |
| PAX5 | 221969_at | BF510692 | NM_016734 | 5079 | 1.46 | 8.27E-04 |
| HTR2B | 206638_at | NM_000867 | NM_000867 | 3357 | 1.46 | 5.49E-03 |
| SLC25A10 | 218275_at | NM_012140 | NM_012140 | 1468 | 1.46 | 6.48E-03 |
| HELLS | 242890_at | AI650364 | NM_018063 | 3070 | 1.46 | 2.08E-03 |
| CCBE1 | 243805_at | BG035826 | NM_133459 | 147372 | 1.46 | 3.38E-03 |
| BE877357 | 228314_at | BE877357 | --- | --- | 1.46 | 3.26E-03 |
| MAK3 | 222393_s_at | AU149868 | NM_025146 | 80218 | 1.46 | 4.59E-03 |
| BE222344 | 226034_at | BE222344 | --- | --- | 1.46 | 5.03E-04 |
| FLJ10375 | 243230_at | AA918375 | NM_018075 | 55129 | 1.46 | 6.10E-03 |
| BRI3BP | 231810_at | BG106919 | NM_080626 | 140707 | 1.46 | 5.53E-04 |
| LCRISP1 | 223475_at | AF142573 | NM_031461 | 83690 | 1.46 | 8.87E-04 |
| ARHGAP6 | 206167_s_at | NM_001174 | NM_001174 | 395 | 1.45 | 6.89E-03 |
| | | | NM_006125 | | | |
| | | | NM_013422 | | | |
| | | | NM_013423 | | | |
| | | | NM_013427 | | | |
| TLX3 | 208495_at | NM_021025 | NM_021025 | 30012 | 1.45 | 3.06E-04 |
| H2AFV | 202487_s_at | NM_012412 | NM_012412 | 94239 | 1.45 | 4.29E-03 |
| | | | NM_138635 | | | |
| | | | NM_201436 | | | |
| | | | NM_201516 | | | |
| | | | NM_201517 | | | |
| BIRC5 | 1555826_at | BQ021146 | NM_001168 | 332 | 1.45 | 3.48E-03 |
| AI357639 | 225716_at | AI357639 | --- | --- | 1.44 | 3.04E-04 |
| ARMC7 | 229906_at | BF194773 | NM_024585 | 79637 | 1.44 | 2.42E-03 |
| OSBPL10 | 219073_s_at | NM_017784 | NM_017784 | 114884 | 1.44 | 1.05E-03 |
| TUBB4 | 212664_at | AL567012 | NM_006087 | 10382 | 1.44 | 2.03E-03 |
| LRRIQ2 | 219886_at | NM_024548 | NM_024548 | 79598 | 1.44 | 8.76E-04 |
| CUGBP2 | 1557422_at | R49146 | NM_006561 | 10659 | 1.44 | 7.78E-03 |
| SRGAP2 | 213329_at | AA742261 | XM_059095 | 23380 | 1.44 | 1.39E-03 |
| NUP93 | 202188_at | NM_014669 | NM_014669 | 9688 | 1.44 | 2.68E-06 |
| LOC440288 | 238511_at | BF982733 | XM_496075 | 440288 | 1.44 | 3.83E-04 |
| AI346432 | 229132_at | AI346432 | --- | --- | 1.44 | 3.77E-04 |
| HSZFP36 | 229732_at | AI417785 | XM_375568 | 55552 | 1.43 | 1.60E-04 |
| DLG2 | 206253_at | NM_001364 | NM_001364 | 1740 | 1.43 | 7.70E-04 |
| PTX3 | 206157_at | NM_002852 | NM_002852 | 5806 | 1.43 | 3.96E-04 |
| FIGNL1 | 1552921_a_at | NM_022116 | NM_022116 | 63979 | 1.43 | 3.50E-04 |
| CHML | 206079_at | NM_001821 | NM_001821 | 1122 | 1.43 | 7.71E-03 |
| AI889959 | 227350_at | AI889959 | --- | --- | 1.43 | 3.94E-04 |
| TLE4 | 233575_s_at | AA705845 | NM_007005 | 7091 | 1.43 | 5.78E-04 |
| LOC388272 | 226608_at | AW157311 | NM_001001436 | 388272 | 1.43 | 2.91E-03 |
| MGC2603 | 220011_at | NM_024037 | NM_024037 | 79000 | 1.43 | 1.24E-04 |
| KIAA1853 | 230010_at | AA608629 | NM_194286 | 84530 | 1.43 | 8.35E-04 |
| LOC51760 | 205613_at | NM_016524 | NM_016524 | 51760 | 1.42 | 1.17E-03 |
| CSE1L | 210766_s_at | AF053640 | NM_001316 | 1434 | 1.42 | 1.06E-05 |
| | | | NM_177436 | | | |
| FAD158 | 223533_at | AL136919 | NM_032270 | 84230 | 1.42 | 2.21E-04 |
| FAM54A | 234944_s_at | AL138828 | NM_138419 | 113115 | 1.42 | 4.74E-03 |
| UNC84B | 212144_at | AL021707 | NM_015374 | 25777 | 1.42 | 1.88E-03 |
| BICD1 | 1554020_at | BC010091 | NM_00100339 8 | 636 | 1.42 | 9.68E-03 |
| | | | NM_001714 | | | |
| GNG2 | 1555766_a_at | AF493870 | NM_053064 | 54331 | 1.42 | 1.16E-06 |
| WDR51A | 234749_s_at | AL117629 | NM_015426 | 25886 | 1.42 | 5.03E-03 |
| EME1 | 234464_s_at | AK021607 | NM_152463 | 146956 | 1.42 | 1.54E-05 |
| TNFRSF19L | 227060_at | AW571669 | NM_032871 | 84957 | 1.42 | 3.79E-03 |
| | | | NM_152222 | | | |
| TSC | 218872_at | NM_017899 | NM_017899 | 54997 | 1.42 | 3.16E-03 |
| LOC201725 | 238015_at | BE620598 | NM_001008393 | 201725 | 1.42 | 7.24E-05 |
| FAM33A | 225686_at | BE048371 | NM_182620 | 348235 | 1.42 | 9.89E-05 |
| GTF2E1 | 205930_at | NM_005513 | NM_005513 | 2960 | 1.42 | 2.50E-04 |
| TYRO3 | 211432_s_at | U05682 | NM_006293 | 7301 | 1.42 | 1.33E-03 |
| GFPT2 | 205100_at | NM_005110 | NM_005110 | 9945 | 1.42 | 1.08E-03 |
| SNX10 | 218404_at | NM_013322 | NM_013322 | 29887 | 1.41 | 4.80E-04 |
| BU077901 | 1557214_at | BU077901 | --- | --- | 1.41 | 2.12E-04 |
| KPNA2 | 201088_at | NM_002266 | NM_002266 | 3838 | 1.41 | 1.16E-05 |
| LZTS1 | 219042_at | NM_021020 | NM_021020 | 11178 | 1.41 | 5.78E-03 |
| NOS1 | 207309_at | NM_000620 | NM_000620 | 4842 | 1.41 | 5.32E-04 |
| CGI-96 | 33307_at | AL022316 | NM_015703 | 27341 | 1.41 | 4.76E-04 |
| WDR34 | 224715_at | BG338983 | NM_052844 | 89891 | 1.40 | 1.09E-03 |
| RAB3B | 205924_at | BC005035 | NM_002867 | 5865 | 1.40 | 2.80E-03 |
| GCH1 | 204224_s_at | NM_000161 | NM_000161 | 2643 | 1.40 | 2.84E-03 |
| C14orf145 | 1557755_at | AW028337 | XM_496002 | 145508 | 1.40 | 1.22E-03 |
| PDLIM5 | 203242_s_at | BG054550 | NM_00101151 3 | 10611 | 1.40 | 6.09E-03 |
| | | | NM_00101151 4 | | | |
| | | | NM_00101151 5 | | | |
| | | | NM_00101151 6 | | | |
| | | | NM_006457 | | | |
| MPP5 | 219321_at | NM_022474 | NM_022474 | 64398 | 1.40 | 2.21E-05 |
| CDO1 | 204154_at | NM_001801 | NM_001801 | 1036 | 1.40 | 3.42E-05 |
| CHAF1B | 204775_at | NM_005441 | NM_005441 | 8208 | 1.40 | 9.66E-04 |
| RECQL | 210568_s_at | BC001052 | NM_002907 | 5965 | 1.40 | 3.54E-03 |
| | | | NM_032941 | | | |
| MAPK12 | 206106_at | AL022328 | NM_002969 | 6300 | 1.40 | 1.10E-06 |
| PTTG3 | 208511_at | NM_021000 | --- | 26255 | 1.39 | 1.10E-03 |
| HAT1 | 203138_at | NM_003642 | NM_003642 | 8520 | 1.39 | 2.54E-05 |
| MYD88 | 209124_at | U70451 | NM_002468 | 4615 | 1.39 | 2.89E-03 |
| NUDT15 | 219347_at | NM_018283 | NM_018283 | 55270 | 1.39 | 1.29E-04 |
| PCDH9 | 219737_s_at | AI524125 | NM_020403 NM_203487 | 5101 | 1.39 | 7.01E-03 |
| 182-FIP | 243119_at | AA810156 | NM_020772 | 57532 | 1.39 | 2.92E-04 |
| MCM4 | 212141_at | AA604621 | NM_005914 NM_182746 | 4173 | 1.39 | 3.82E-03 |
| RAB23 | 229504_at | AI810826 | NM_016277 NM_183227 | 51715 | 1.39 | 1.82E-05 |
| C9orf88 | 223019_at | BC001979 | NM_022833 | 64855 | 1.39 | 2.37E-03 |
| DLX6 | 242940_x_at | AA040332 | XM_376652 | 1750 | 1.39 | 2.34E-06 |
| | | | XM_379892 | | | |
| FLJ13909 | 219556_at | NM_025108 | NM_025108 | 80178 | 1.39 | 5.95E-04 |
| CDKN2C | 204159_at | NM_001262 | NM_001262 | 1031 | 1.39 | 5.88E-04 |
| | | | NM_078626 | | | |
| MEIS1 | 1559477_s_at | AL832770 | NM_002398 | 4211 | 1.39 | 3.54E-04 |
| STRA6 | 1569334_at | BC015881 | NM_022369 | 64220 | 1.39 | 6.72E-03 |
| AW069181 | 223519_at | AW069181 | --- | --- | 1.39 | 9.35E-04 |
| SEZ6L | 213609_s_at | AB023144 | NM_021115 | 23544 | 1.38 | 5.40E-04 |
| FKBP14 | 235311_at | BG110260 | NM_017946 | 55033 | 1.38 | 1.16E-04 |
| BG231758 | 227615_at | BG231758 | --- | --- | 1.38 | 1.85E-04 |
| GEMIN4 | 217099_s_at | AF258545 | NM_015721 | 50628 | 1.38 | 2.18E-03 |
| H2AFV | 227085_at | AI823792 | NM_012412 | 94239 | 1.38 | 1.65E-04 |
| | | | NM_138635 | | | |
| | | | NM_201436 | | | |
| | | | NM_201516 | | | |
| | | | NM_201517 | | | |
| KLF10 | 202393_s_at | NM_005655 | NM_005655 | 7071 | 1.38 | 4.37E-03 |
| MRE11A | 205395_s_at | NM_005590 | NM_005590 | 4361 | 1.38 | 9.90E-04 |
| | | | NM_005591 | | | |
| FDXR | 207813_s_at | NM_004110 | NM_004110 | 2232 | 1.38 | 6.17E-04 |
| | | | NM_024417 | | | |
| GNG4 | 205184_at | NM_004485 | NM_004485 | 2786 | 1.38 | 2.86E-05 |
| MYOHD1 | 219320_at | NM_025109 | NM_025109 | 80179 | 1.38 | 6.52E-04 |
| CTL2 | 224609_at | AI264216 | NM_020428 | 57153 | 1.37 | 2.31E-03 |
| FLJ13273 | 235387_at | AA740875 | NM_024751 | 79807 | 1.37 | 1.76E-05 |
| EDA2R | 221399_at | NM_021783 | NM_021783 | 60401 | 1.37 | 4.37E-03 |
| MGC10744 | 238590_x_at | BF981428 | NM_032354 | 84314 | 1.37 | 6.04E-03 |
| | | | NM_183065 | | | |
| ACYP1 | 205260_s_at | NM_001107 | NM_001107 | 97 | 1.37 | 7.99E-03 |
| | | | NM_203488 | | | |
| MGC12197 | 1555501_s_at | BC010357 | NM_016625 | 51319 | 1.37 | 1.22E-03 |
| MGC13186 | 226813_at | AA883980 | NM_032324 | 84284 | 1.37 | 9.86E-04 |
| MRE11A | 242456_at | AA931565 | NM_005590 | 4361 | 1.37 | 6.99E-03 |
| | | | NM_005591 | | | |
| BCR | 202315_s_at | NM_004327 | NM_004327 | 613 | 1.36 | 1.69E-04 |
| | | | NM_021574 | | | |
| CHRNA3 | 211587_x_at | M37981 | NM_000743 | 1136 | 1.36 | 8.91E-04 |
| GPR161 | 235961_at | BF248385 | NM_007369 | 23432 | 1.36 | 5.62E-05 |
| | | | NM_153832 | | | |
| DUSP4 | 204015_s_at | BC002671 | NM_001394 | 1846 | 1.36 | 6.68E-04 |
| | | | NM_057158 | | | |
| GNG4 | 1555765_a_at | AF493872 | NM_004485 | 2786 | 1.36 | 8.13E-05 |
| FLJ11712 | 219056_at | NM_024570 | NM_024570 | 79621 | 1:36 | 2.93E-03 |
| BUB3 | 209974_s_at | AF047473 | NM_00100779 3 | 9184 | 1.36 | 2.12E-06 |
| | | | NM_004725 | | | |
| LOC93081 | 213346_at | BE748563 | NM_138779 | 93081 | 1.36 | 2.01E-05 |
| UNQ470 | 228403_at | AL541276 | NM_198573 | 375704 | 1.36 | 9.85E-03 |
| FLJ20364 | 221685_s_at | AF269167 | NM_017785 | 54908 | 1.36 | 4.22E-05 |
| LOC283454 | 242715_at | AA331548 | --- | 283454 | 1.36 | 8.25E-04 |
| LOC388272 | 242555_at | AW578854 | NM_001001436 | 388272 | 1.35 | 7.50E-03 |
| SELM | 226051_at | BF973568 | NM_080430 | 140606 | 1.35 | 1.09E-04 |
| PAQR4 | 212858_at | AL520675 | NM_152341 | 124222 | 1.35 | 3.60E-05 |
| C20orf7 | 222894_x_at | AI640582 | NM_024120 | 79133 | 1.35 | 1.37E-04 |
| | | | NM_199052 | | | |
| PKP4 | 201929_s_at | NM_003628 | NM_00100547 6 | 8502 | 1.35 | 6.66E-05 |
| | | | NM_003628 | | | |
| CBX5 | 242069_at | BE568225 | NM_012117 | 23468 | 1.35 | 1.98E-03 |
| CPNE4 | 228796_at | BE645967 | NM_130808 | 131034 | 1.35 | 4.28E-03 |
| CSE1L | 201112_s_at | NM_001316 | NM_001316 | 1434 | 1.35 | 2.61E-06 |
| | | | NM_177436 | | | |
| TBC1D1 | 227945_at | BE882538 | NM_015173 | 23216 | 1.34 | 4.34E-03 |
| B4GALT1 | 201883_s_at | D29805 | NM_001497 | 2683 | 1.34 | 1.44E-03 |
| TAF5 | 1553528_a_at | NM_139052 | NM_006951 | 6877 | 1.34 | 9.10E-04 |
| CEP1 | 205642_at | NM_007018 | NM_007018 | 11064 | 1.34 | 3.35E-04 |
| STOM | 201061_s_at | M81635 | NM_004099 | 2040 | 1.34 | 1.41E-04 |
| | | | NM_198194 | | | |
| C6orf182 | 1554019_s_at | BC033448 | NM_173830 | 285753 | 1.34 | 1.74E-05 |
| LOC284465 | 229382_at | AW149270 | --- | 284465 | 1.34 | 3.05E-03 |
| C20orf108 | 224690_at | BG432350 | NM_080821 | 116151 | 1.34 | 1.10E-03 |
| TDRD7 | 213361_at | AW129593 | NM_014290 | 23424 | 1.34 | 5.01E-03 |
| CDK6 | 224847_at | AW051349 | NM_001259 | 1021 | 1.33 | 2.41E-04 |
| C20orf102 | 226973_at | AA206763 | NM_080607 | 128434 | 1.33 | 2.65E-03 |
| TMPO | 227578_at | H28597 | NM_003276 | 7112 | 1.33 | 1.51E-03 |
| TUBB6 | 209191_at | BC002654 | NM_032525 | 84617 | 1.33 | 1.57E-04 |
| ANP32E | 221505_at | AW612574 | NM_030920 | 81611 | 1.33 | 1.41E-05 |
| FLJ20516 | 219258_at | NM_017858 | NM_017858 | 54962 | 1.33 | 1.05E-03 |
| DC13 | 218447_at | NM_020188 | NM_020188 | 56942 | 1.33 | 3.60E-03 |
| CKLF | 219161_s_at | NM_016951 | NM_016326 | 51192 | 1.33 | 6.08E-05 |
| | | | NM_016951 | | | |
| | | | NM_181640 | | | |
| | | | NM_181641 | | | |
| DLL3 | 219537_x_at | NM_016941 | NM_016941 | 10683 | 1.33 | 1.14E-05 |
| | | | NM_203486 | | | |
| DOCK4 | 205003_at | NM_014705 | NM_014705 | 9732 | 1.33 | 1.67E-03 |
| UBAP2L | 201377_at | NM_014847 | NM_014847 | 9898 | 1.33 | 1.35E-05 |
| FLJ10036 | 218349_s_at | NM_017975 | NM_017975 | 55055 | 1.33 | 1.50E-03 |
| SERPING1 | 200986_at | NM_000062 | NM_000062 | 710 | 1.33 | 3.17E-03 |
| SCML2 | 206147_x_at | NM_006089 | NM_006089 | 10389 | 1.33 | 2.97E-05 |
| BE877775 | 1568955_at | BE877775 | --- | --- | 1.33 | 1.57E-05 |
| LOC440820 | 226602_s_at | T30183 | XM_496519 | 440820 | 1.33 | 3.16E-05 |
| FKBP5 | 224856_at | W86302 | NM_004117 | 2289 | 1.33 | 1.42E-03 |
| PPM1D | 230330_at | AI312083 | NM_003620 | 8493 | 1.33 | 4.50E-04 |
| DKFZp313A243: | 226278_at | AI150224 | --- | 258010 | 1.32 | 1.69E-04 |
| MAN1A1 | 221760_at | BG287153 | NM_005907 | 4121 | 1.32 | 1.75E-05 |
| RFWD3 | 218564_at | BC002574 | --- | 55159 | 1.32 | 4.99E-04 |
| C10orf78 | 238794_at | N23586 | NM_00100275 9 | 119392 | 1.32 | 9.64E-05 |
| | | | NM_145247 | | | |
| NEGR1 | 229461_x_at | AI123532 | NM_173808 | 257194 | 1.32 | 1.20E-03 |
| DCK | 203302_at | NM_000788 | NM_000788 | 1633 | 1.32 | 3.62E-05 |
| K-ALPHA-1 | 211072_x_at | BC006481 | NM_006082 | 10376 | 1.32 | 6.06E-06 |
| AI092265 | 228088_at | AI092265 | --- | --- | 1.32 | 9.95E-04 |
| UCHL5 | 220083_x_at | NM_016017 | NM_015984 | 51377 | 1.32 | 2.96E-04 |
| TRIM37 | 1554001_at | BC036012 | NM_00100520 7 | 4591 | 1.32 | 8.08E-04 |
| | | | NM_015294 | | | |
| MGC39900 | 214051_at | BF677486 | NM_194324 | 286527 | 1.32 | 7.28E-03 |
| NFYB | 218129_s_at | NM_006166 | NM_006166 | 4801 | 1.32 | 5.07E-03 |
| FAS | 204781_s_at | NM_000043 | NM_000043 | 355 | 1.32 | 1.70E-03 |
| | | | NM_152871 | | | |
| | | | NM_152872 | | | |
| | | | NM_152873 | | | |
| | | | NM_152874 | | | |
| | | | NM_152875 | | | |
| | | | NM_152876 | | | |
| | | | NM_152877 | | | |
| C2orf23 | 204364_s_at | BE535746 | NM_022912 | 65055 | 1.31 | 8.43E-05 |
| YPEL1 | 228296_at | AW450686 | NM_013313 | 29799 | 1.31 | 7.66E-03 |
| TRAF4 | 211899_s_at | AF082185 | NM_004295 | 9618 | 1.31 | 4.10E-03 |
| | | | NM_145751 | | | |
| GLS | 203158_s_at | AF097493 | NM_014905 | 2744 | 1.31 | 9.13E-04 |
| FARP1 | 201910_at | BF213279 | NM_00100171 5 | 10160 | 1.31 | 6.05E-04 |
| | | | NM_005766 | | | |
| C9orf40 | 222781_s_at | BF590861 | NM_017998 | 55071 | 1.31 | 1.00E-02 |
| ADCY3 | 235441_at | BF217471 | NM_004036 | 109 | 1.31 | 9.61E-03 |
| ZNF253 | 242919_at | AV683221 | NM_021047 | 114977 | 1.31 | 1.27E-03 |
| LOC114977 | | | | 56242 | | |
| NASP | 201970_s_at | NM_002482 | NM_002482 | 4678 | 1.31 | 8.90E-05 |
| | | | NM_152298 | | | |
| | | | NM_172164 | | | |
| ACTL6A | 202666_s_at | NM_004301 | NM_004301 | 86 | 1.30 | 1.03E-07 |
| | | | NM_177989 | | | |
| | | | NM_178042 | | | |
| HOXD3 | 206601_s_at | BC005124 | NM_006898 | 3232 | 1.30 | 3.02E-04 |
| SORD | 201563_at | L29008 | NM_003104 | 6652 | 1.30 | 6.16E-05 |
| COQ3 | 223515_s_at | AL136726 | NM_017421 | 51805 | 1.30 | 1.20E-05 |
| SKP2 | 203626_s_at | NM_005983 | NM_005983 | 6502 | 1.30 | 9.20E-03 |
| | | | NM_032637 | | | |
| CDK6 | 224848_at | AA922068 | NM_001259 | 1021 | 1.30 | 3.32E-03 |
| BOK | 223349_s_at | BE614255 | NM_032515 | 666 | 1.30 | 4.78E-06 |
| PCDH17 | 205656_at | NM_014459 | NM_014459 | 27253 | 1.30 | 1.41E-03 |
| BCAS4 | 220588_at | NM_017843 | NM_00101097 4 | 55653 | 1.30 | 3.33E-04 |
| | | | NM_017843 | | | |
| | | | NM_198799 | | | |
| TDP1 | 219715_s_at | NM_018319 | NM_00100874 4 | 55775 | 1.30 | 5.37E-05 |
| | | | NM_018319 | | | |
| LOC283683 | 217520_x_at | BG396614 | XM_290660 | 283683 | 1.30 | 9.14E-03 |
| LOC339003 | | | | 339003 | | |
| C6orf152 | 242006_at | AI379143 | NM_181714 | 167691 | 1.30 | 2.64E-03 |
| AA824298 | 222606_at | AA824298 | --- | --- | 1.30 | 2.38E-03 |
| PRPS1 | 208447_s_at | NM_002764 | NM_002764 | 5631 | 1.30 | 1.12E-04 |
| AA401256 | 230537_at | AA401256 | --- | --- | 1.30 | 2.49E-03 |
| SUV39H2 | 1554572_a_at | BC029360 | NM_024670 | 79723 | 1.29 | 2.41E-05 |
| LOC284244 | 214162_at | AF070541 | --- | 284244 | 1.29 | 9.39E-03 |
| WDR53 | 227814_at | AA789329 | NM_182627 | 348793 | 1.29 | 5.37E-06 |
| CPT1A | 210688_s_at | BC000185 | NM_001876 | 1374 | 1.29 | 5.31E-03 |
| RFC1 | 208021_s_at | NM_002913 | NM_002913 | 5981 | 1.29 | 1.61E-06 |
| MGC10812 | 223469_at | BC004942 | --- | 83542 | 1.29 | 3.11E-04 |
| CSE1L | 201111_at | AF053641 | NM_001316 | 1434 | 1.29 | 1.01E-03 |
| | | | NM_177436 | | | |
| PPFIBP2 | 212841_s_at | AI692180 | NM_003621 | 8495 | 1.29 | 4.46E-04 |
| CHRNA3 | 210221_at | BC000513 | NM_000743 | 1136 | 1.29 | 6.31E-03 |
| RBL1 | 1559307_s_at | BG387892 | NM_002895 | 5933 | 1.29 | 5.93E-05 |
| | | | NM_183404 | | | |
| ZNF43 | 206695_x_at | NM_003423 | NM_003423 | 7594 | 1.28 | 2.30E-04 |
| TM4SF7 | 209263_x_at | BC000389 | NM_003271 | 7106 | 1.28 | 2.01E-04 |
| PDLIM5 | 216804_s_at | AK027217 | NM_00101151 3 | 10611 | 1.28 | 1.45E-03 |
| | | | NM_00101151 4 | | | |
| | | | NM_00101151 5 | | | |
| | | | NM_00101151 6 | | | |
| | | | NM_006457 | | | |
| KCNJ8 | 205304_s_at | NM_004982 | NM_004982 | 3764 | 1.28 | 8.99E-05 |
| NLN | 225943_at | BF222737 | NM_020726 | 57486 | 1.28 | 6.53E-03 |
| ORF1-FL49 | 224707_at | AL522667 | NM_032412 | 84418 | 1.28 | 6.23E-05 |
| AL833487 | 1562244_at | AL833487 | --- | --- | 1.28 | 1.81E-04 |
| EB-1 | 227440_at | AW005572 | NM_020140 | 56899 | 1.28 | 2.28E-03 |
| | | | NM_152788 | | | |
| | | | NM_181670 | | | |
| MEIS1 | 204069_at | NM_002398 | NM_002398 | 4211 | 1.28 | 3.15E-04 |
| SESTD1 | 226763_at | AW409611 | NM_178123 | 91404 | 1.27 | 1.35E-03 |
| GNG2 | 224964_s_at | AK026424 | NM_053064 | 54331 | 1.27 | 1.89E-05 |
| DPF1 | 231599_x_at | AI939563 | NM_004647 | 8193 | 1.27 | 1.45E-03 |
| STMN1 | 200783_s_at | NM_005563 | NM_005563 | 3925 | 1.27 | 5.04E-04 |
| | | | NM_203399 | | | |
| | | | NM_203401 | | | |
| AF279774 | 216967_at | AF279774 | --- | --- | 1.27 | 8.55E-03 |
| DUSP4 | 204014_at | NM_001394 | NM_001394 | 1846 | 1.27 | 1.77E-04 |
| | | | NM_057158 | | | |
| MCM4 | 222036_s_at | AI859865 | NM_005914 | 4173 | 1.27 | 2.04E-03 |
| | | | NM_182746 | | | |
| C6orf151 | 1553274_a_at | NM_152551 | NM_152551 | 154007 | 1.27 | 8.31E-03 |
| RRM2B | 223342_at | AB036063 | NM_015713 | 50484 | 1.27 | 4.55E-04 |
| TDRKH | 223530_at | AF227192 | NM_006862 | 11022 | 1.27 | 1.02E-06 |
| PRIM2A | 215708_s_at | AL121975 | NM_000947 | 5558 | 1.26 | 1.78E-03 |
| GPR161 | 214104_at | AI703188 | NM_007369 | 23432 | 1.26 | 1.74E-05 |
| | | | NM_153832 | | | |
| NR3C1 | 211671_s_at | U01351 | NM_000176 | 2908 | 1.26 | 1.39E-04 |
| TCF7L2 | 212761_at | AI949687 | NM_030756 | 6934 | 1.26 | 8.24E-03 |
| HECA | 218603_at | NM_016217 | NM_016217 | 51696 | 1.26 | 8.85E-03 |
| KREMEN1 | 227250_at | BF221745 | NM_032045 | 83999 | 1.26 | 3.46E-04 |
| | | | NM_153379 | | | |
| RBMS3 | 241789_at | AW338699 | NM_00100379 2 | 27303 | 1.26 | 7.52E-03 |
| | | | NM_00100379 3 | | | |
| | | | NM_014483 | | | |
| PLAUR | 211924_s_at | AY029180 | NM_00100537 6 | 5329 | 1.26 | 2.35E-03 |
| | | | NM_00100537 7 | | | |
| | | | NM_002659 | | | |
| FLJ25059 | 229025_s_at | AW008627 | NM_144981 | 196294 | 1.26 | 2.34E-03 |
| FKBP5 | 204560_at | NM_004117 | NM_004117 | 2289 | 1.26 | 5.68E-03 |
| HOMER3 | 215489_x_at | AI871287 | NM_004838 | 9454 | 1.26 | 3.85E-03 |
| ZNF503 | 227195_at | AA603467 | NM_032772 | 84858 | 1.26 | 6.66E-04 |
| AL833634 | 1562699_at | AL833634 | --- | --- | 1.26 | 8.19E-05 |
| BAX | 211833_s_at | U19599 | NM_004324 | 581 | 1.25 | 6.21E-04 |
| | | | NM_138761 | | | |
| | | | NM_138762 | | | |
| | | | NM_138763 | | | |
| | | | NM_138764 | | | |
| | | | NM_138765 | | | |
| SUHW2 | 229360_at | N22886 | NM_080764 | 140883 | 1.25 | 5.46E-04 |
| CNTN4 | 229084_at | R42166 | NM_175607 | 152330 | 1.25 | 9.69E-04 |
| | | | NM_175612 | | | |
| | | | NM_175613 | | | |
| ZNF184 | 213452_at | AI811577 | NM_007149 | 7738 | 1.25 | 4.11E-05 |
| MPP1 | 202974_at | NM_002436 | NM_002436 | 4354 | 1.25 | 1.81E-03 |
| LOC93349 | 214791_at | AK023116 | NM_138402 | 93349 | 1.25 | 5.56E-06 |
| TOP3B | 215781_s_at | D87012 | NM_003935 | 8940 | 1.25 | 1.49E-03 |
| RTTN | 227072_at | BG167480 | NM_173630 | 25914 | 1.25 | 8.89E-06 |
| ASK | 204244_s_at | NM_006716 | NM_006716 | 10926 | 1.25 | 3.89E-05 |
| FANCG | 203564_at | NM_004629 | NM_004629 | 2189 | 1.25 | 1.29E-04 |
| WIG1 | 1555609_a_at | AF355465 | NM_022470 | 64393 | 1.25 | 2.45E-03 |
| | | | NM_152240 | | | |
| RFC1 | 209085_x_at | L14922 | NM_002913 | 5981 | 1.24 | 3.75E-07 |
| TRIM59 | 227801_at | N90779 | NM_173084 | 286827 | 1.24 | 3.52E-03 |
| KIAA1522 | 224746_at | AB040955 | XM_036299 | 57648 | 1.24 | 8.59E-03 |
| SLC17A6 | 220551_at | NM_020346 | NM_020346 | 57084 | 1.24 | 4.19E-04 |
| PFKFB3 | 202464_s_at | NM_004566 | NM_004566 | 5209 | 1.24 | 4.76E-04 |
| BF110268 | 238178_at | BF110268 | --- | --- | 1.24 | 1.57E-03 |
| TCF3 | 209152_s_at | AI655986 | NM_003200 | 6929 | 1.24 | 2.72E-03 |
| AMOTL1 | 225450_at | AI433831 | NM_130847 | 154810 | 1.24 | 5.65E-06 |
| TUBA2 | 210527_x_at | L11645 | NM_006001 | 7278 | 1.24 | 3.79E-05 |
| | | | NM_079836 | | | |
| AB033091 | 225295_at | AB033091 | --- | --- | 1.23 | 8.34E-06 |
| Cep70 | 219036_at | NM_024491 | NM_024491 | 80321 | 1.23 | 6.01E-03 |
| RTN4IP1 | 1555679_a_at | AF439711 | NM_032730 | 84816 | 1.23 | 3.15E-03 |
| HTATSF1 | 202601_s_at | AI373539 | NM_014500 | 27336 | 1.23 | 1.15E-03 |
| CA773938 | 1558517_s_at | CA773938 | --- | --- | 1.23 | 7.86E-04 |
| RAB27A | 210951_x_at | AF125393 | NM_004580 | 5873 | 1.23 | 4.76E-03 |
| | | | NM_183234 | | | |
| | | | NM_183235 | | | |
| | | | NM_183236 | | | |
| TLE3 | 228340_at | BE967118 | NM_005078 | 7090 | 1.23 | 4.90E-03 |
| AI452715 | 229817_at | AI452715 | --- | --- | 1.23 | 3.71E-03 |
| AK022598 | 232662_x_at | AK022598 | --- | --- | 1.22 | 2.67E-03 |
| RNF26 | 224947_at | AL559247 | NM_032015 | 79102 | 1.22 | 2.05E-04 |
| C2orf23 | 204365_s_at | NM_022912 | NM_022912 | 65055 | 1.22 | 9.80E-05 |
| KIAA0574 | 213997_at | AB011146 | --- | 23359 | 1.22 | 4.46E-03 |
| K-ALPHA-1 | 213646_x_at | BE300252 | NM_006082 | 10376 | 1.22 | 1.24E-05 |
| SKP2 | 203625_x_at | BG105365 | NM_005983 | 6502 | 1.22 | 4.38E-06 |
| | | | NM_032637 | | | |
| ZNF443 | 205928_at | NM_005815 | NM_005815 | 10224 | 1.22 | 2.72E-03 |
| MAPKAPK3 | 202788_at | NM_004635 | NM_004635 | 7867 | 1.22 | 1.73E-04 |
| RUSC1 | 206949_s_at | NM_014328 | NM_014328 | 23623 | 1.22 | 4.02E-04 |
| FIGNL1 | 222843_at | AK023411 | NM_022116 | 63979 | 1.22 | 1.26E-04 |
| SENP1 | 1552812_a_at | NM_014554 | NM_014554 | 29843 | 1.22 | 2.44E-03 |
| AW874669 | 239834_at | AW874669 | --- | --- | 1.22 | 1.90E-04 |
| USP1 | 202413_s_at | NM_003368 | NM_003368 | 7398 | 1.22 | 3.61E-04 |
| KIAA0318 | 238817_at | BF063412 | NM_015347 | 23504 | 1.22 | 1.61E-03 |
| MGC5576 | 201764_at | NM_024056 | NM_024056 | 79022 | 1.22 | 1.26E-03 |
| MGC34032 | 1552782_at | AK091400 | NM_152697 | 204962 | 1.21 | 2.92E-03 |
| septin 6 | 213666_at | AK026589 | NM_015129 | 23157 | 1.21 | 5.69E-03 |
| | | | | | | |
| | | | NM_145800 | | | |
| | | | NM_145802 | | | |
| AI143124 | 212832_s_at | AI143124 | --- | | 1.21 | 2.67E-04 |
| SLC2A4RG | 218494_s_at | NM_020062 | NM_020062 | | 1.21 | 6.93E-04 |
| ZNF423 | 214761_at | AW149417 | NM_015069 | | 1.21 | 8.23E-03 |
| CSRP2BP | 228543_at | AA780252 | NM_020536 | | 1.21 | 9.19E-06 |
| | | | NM_177926 | | | |
| MTUS1 | 212095_s_at | BE552421 | NM_00100192 4 | | 1.21 | 2.45E-04 |
| | | | NM_00100192 5 | | | |
| | | | NM_00100192 7 | | | |
| | | | NM_00100193 1 | | | |
| | | | NM_020749 | | | |
| BF056092 | 242629_at | BF056092 | --- | | 1.21 | 6.11E-03 |
| HPSE | 219403_s_at | NM_006665 | NM_006665 | | 1.21 | 1.02E-02 |
| BF445001 | 238212_at | BF445001 | --- | | 1.21 | 2.00E-03 |
| SRGAP2 | 1568957_x_at | BE877775 | XM_059095 | | 1.21 | 1.43E-04 |
| CDKN1A | 202284_s_at | NM_000389 | NM_000389 | 1026 | 1.21 | 1.36E-03 |
| | | | NM_078467 | | | |
| RAD51C | 209849_s_at | AF029669 | NM_002876 | 5889 | 1.21 | 2.70E-03 |
| | | | NM_058216 | | | |
| | | | NM_058217 | | | |
| GTPBP1 | 219357_at | NM_014027 | NM_004286 | 9567 | 1.20 | 7.29E-04 |
| SLC25A1 | 210010_s_at | U25147 | NM_005984 | 6576 | 1.20 | 5.15E-04 |
| KIAA1713 | 233536_at | AI202664 | XM_290811 | 80816 | 1.20 | 2.85E-03 |
| APAF1 | 204859_s_at | NM_013229 | NM_001160 | 317 | 1.20 | 5.73E-05 |
| | | | NM_013229 | | | |
| | | | NM_181861 | | | |
| | | | NM_181868 | | | |
| | | | NM_181869 | | | |
| WHSC1 | 222778_s_at | AW024870 | NM_007331 | 7468 | 1.20 | 4.19E-05 |
| | | | NM_014919 | | | |
| | | | NM_133330 | | | |
| | | | NM_133331 | | | |
| | | | NM_133332 | | | |
| | | | NM_133333 | | | |
| | | | NM_133334 | | | |
| | | | NM_133335 | | | |
| | | | NM_133336 | | | |
| T87730 | 242500_at | T87730 | --- | --- | 1.20 | 1.38E-03 |
| EHD4 | 209536_s_at | AF320070 | NM_139265 | 30844 | 1.20 | 5.84E-03 |
| WIG1 | 219628_at | NM_022470 | NM_022470 | 64393 | 1.20 | 8.50E-04 |
| | | | NM_152240 | | | |
| BF977829 | 229067_at | BF977829 | --- | --- | 1.20 | 3.26E-04 |
| NUP62 | 202153_s_at | NM_016553 | NM_012346 | 23636 | 1.20 | 3.85E-04 |
| | | | NM_016553 | | | |
| | | | NM_153718 | | | |
| | | | NM_153719 | | | |
| N50083 | 229110_at | N50083 | --- | --- | 1.20 | 1.89E-03 |
| ARL6 | 223735_at | AL136815 | NM_032146 | 84100 | 1.20 | 5.56E-03 |
| | | | NM_177976 | | | |
| MGC2574 | 203119_at | NM_024098 | NM_024098 | 79080 | 1.19 | 1.54E-04 |
| FLJ10407 | 218073_s_at | NM_018087 | NM_018087 | 55706 | 1.19 | 1.01E-02 |
| DKFZp313A243: | 230006_s_at | AI742358 | --- | 258010 | 1.19 | 1.25E-04 |
| C20orf7 | 227160_s_at | AW102828 | NM_024120 | 79133 | 1.19 | 8.46E-04 |
| | | | NM_199052 | | | |
| MVK | 215649_s_at | AF217536 | NM_000431 | 4598 | 1.19 | 4.73E-03 |
| PPP1R3D | 204554_at | AL109928 | NM_006242 | 5509 | 1.19 | 9.07E-04 |
| MGC14560 | 218461_at | NM_016301 | NM_016301 | 51184 | 1.19 | 2.49E-06 |
| AC007277 | 216508_x_at | AC007277 | --- | --- | 1.19 | 1.79E-05 |
| EPHA2 | 203499_at | NM_004431 | NM_004431 | 1969 | 1.19 | 7.06E-04 |
| UBE2L3 | 200684_s_at | AI819709 | NM_003347 | 7332 | 1.19 | 4.60E-07 |
| | | | NM_198157 | | | |
| NFS1 | 218455_at | NM_021100 | NM_021100 | 9054 | 1.19 | 2.24E-03 |
| | | | NM_181679 | | | |
| RELB | 205205_at | NM_006509 | NM_006509 | 5971 | 1.19 | 8.97E-03 |
| GAS2L1 | 31874_at | Y07846 | NM_006478 | 10634 | 1.19 | 1.72E-03 |
| | | | NM_152236 | | | |
| | | | NM_152237 | | | |
| FLJ33814 | 225644_at | BF060776 | NM_173510 | 150275 | 1.19 | 2.07E-05 |
| K-ALPHA-1 | 211058_x_at | BC006379 | NM_006082 | 10376 | 1.18 | 1.05E-05 |
| NUP62 | 207740_s_at | NM_012346 | NM_012346 | 23636 | 1.18 | 1.26E-04 |
| | | | NM_016553 | | | |
| | | | NM_153718 | | | |
| | | | NM_153719 | | | |
| K-ALPHA-1 | 201090_x_at | NM_006082 | NM_006082 | 10376 | 1.18 | 1.13E-05 |
| NLN | 225944_at | AW006938 | NM_020726 | 57486 | 1.18 | 5.24E-03 |
| ALS2CR4 | 1553956_at | BG194770 | NM_152388 | 65062 | 1.18 | 8.49E-04 |
| FZD2 | 210220_at | L37882 | NM_001466 | 2535 | 1.18 | 2.21E-05 |
| RET | 205879_x_at | BC004257 | NM_020630 | 5979 | 1.18 | 2.08E-04 |
| | | | NM_020975 | | | |
| FLJ23322 | 231846_at | AK026975 | NM_024955 | 80020 | 1.18 | 2.79E-04 |
| BE878277 | 212829_at | BE878277 | --- | --- | 1.18 | 6.73E-04 |
| ZNF286 | 239898_x_at | AI498484 | NM_020652 | 57335 | 1.18 | 2.96E-03 |
| HIVEP2 | 243254_at | T08739 | NM_006734 | 3097 | 1.18 | 6.77E-04 |
| SUCLG2 | 215772_x_at | AL050226 | NM_003848 | 8801 | 1.18 | 3.77E-04 |
| ARF4L | 203586_s_at | NM_001661 | NM_001661 | 379 | 1.18 | 3.10E-03 |
| DDX11 | 213378_s_at | AI983033 | NM_004399 | 1663 | 1.18 | 6.41E-03 |
| | | | NM_030653 | | | |
| | | | NM_030655 | | | |
| PLEKHF2 | 218640_s_at | NM_024613 | NM_024613 | 79666 | 1.18 | 2.90E-03 |
| MCM7 | 208795_s_at | D55716 | NM_005916 | 4176 | 1.18 | 2.26E-04 |
| | | | NM_182776 | | | |
| CDK6 | 207143_at | NM_001259 | NM_001259 | 1021 | 1.18 | 4.14E-04 |
| BAX | 208478_s_at | NM_004324 | NM_004324 | 581 | 1.17 | 5.30E-04 |
| | | | NM_138761 | | | |
| | | | NM_138762 | | | |
| | | | NM_138763 | | | |
| | | | NM_138764 | | | |
| | | | NM_138765 | | | |
| FLJ23322 | 220707_s_at | NM_024955 | NM_024955 | 80020 | 1.17 | 2.10E-03 |
| LOC139886 | 228654_at | AU145277 | XM_066946 | 139886 | 1.17 | 1.82E-06 |
| TANC | 225308_s_at | AB051515 | NM_033394 | 85461 | 1.17 | 1.16E-03 |
| FBXO22 | 225736_at | BE966247 | NM_012170 | 26263 | 1.17 | 3.53E-05 |
| | | | NM_147188 | | | |
| BE504215 | 228563_at | BE504215 | --- | --- | 1.17 | 2.17E-04 |
| FAM43A | 227410_at | AW264102 | NM_153690 | 131583 | 1.17 | 2.24E-04 |
| PXN | 201087_at | NM_002859 | NM_002859 | 5829 | 1.17 | 5.53E-04 |
| BCLP | 225536_at | AL545105 | NM_033504 | 113452 | 1.16 | 9.28E-03 |
| C1orf43 | 1555225_at | BC008306 | NM_015449 | 25912 | 1.16 | 4.74E-04 |
| | | | NM_138740 | | | |
| CABYR | 219928_s_at | NM_012189 | NM_012189 | 26256 | 1.16 | 4.76E-04 |
| | | | NM_138643 | | | |
| | | | NM_138644 | | | |
| | | | NM_153768 | | | |
| | | | NM_153769 | | | |
| | | | NM_153770 | | | |
| SEZ6L | 231650_s_at | BE672217 | NM_021115 | 23544 | 1.16 | 9.63E-04 |
| USP1 | 202412_s_at | AW499935 | NM_003368 | 7398 | 1.16 | 9.85E-04 |
| C21orf45 | 228597_at | AW151538 | NM_018944 | 54069 | 1.16 | 9.08E-04 |
| PDLIM5 | 203243_s_at | NM_006457 | NM_00101151 3 | 10611 | 1.16 | 3.70E-05 |
| | | | NM_00101151 4 | | | |
| | | | NM_00101151 5 | | | |
| | | | NM_00101151 6 | | | |
| | | | NM_006457 | | | |
| LGR4 | 218326_s_at | NM_018490 | NM_018490 | 55366 | 1.16 | 1.79E-03 |
| LOC284307 | 235773_at | AW029293 | NM_001010879 | 284307 | 1.16 | 2.08E-03 |
| ZCCHC4 | 220473_s_at | NM_014150 | XM_376310 | 29063 | 1.16 | 2.21E-04 |
| H10661 | 242317_at | H10661 | --- | --- | 1.16 | 4.34E-03 |
| MFGE8 | 210605_s_at | BC003610 | NM_005928 | 4240 | 1.16 | 5.08E-03 |
| CDC5L | 209055_s_at | AW268817 | NM_001253 | 988 | 1.16 | 2.21E-06 |
| ARL6IP | 211935_at | D31885 | NM_015161 | 23204 | 1.16 | 1.46E-04 |
| NAGA | 202944_at | NM_000262 | NM_000262 | 4668 | 1.16 | 4.02E-05 |
| RAB23 | 220955_x_at | NM_016277 | NM_016277 | 51715 | 1.16 | 5.82E-04 |
| | | | NM_183227 | | | |
| KCNN1 | 206231_at | NM_002248 | NM_002248 | 3780 | 1.16 | 4.89E-04 |
| ch-TOG | 1555278_a_at | BC035554 | NM_00100893 8 | 9793 | 1.16 | 1.57E-04 |
| | | | NM_014756 | | | |
| MPHOSPH9 | 206205_at | NM_022782 | NM_022782 | 10198 | 1.16 | 5.99E-04 |
| AI141426 | 229898_at | AI141426 | --- | --- | 1.16 | 4.31E-04 |
| POU2F1 | 206789_s_at | NM_002697 | NM_002697 | 5451 | 1.15 | 5.40E-03 |
| DKFZP434L187 | 230861_at | AA889613 | XM_496054 | 26082 | 1.15 | 5.85E-05 |
| C14orf160 | 224460_s_at | BC006117 | NM_024884 | 79944 | 1.15 | 3.17E-03 |
| PPM1D | 204566_at | NM_003620 | NM_003620 | 8493 | 1.15 | 1.21E-04 |
| KIAA1853 | 236151_at | BF315452 | NM_194286 | 84530 | 1.15 | 1.89E-03 |
| GLS | 203159_at | NM_014905 | NM_014905 | 2744 | 1.15 | 4.23E-03 |
| FLJ31842 | 234980_at | AI004375 | NM_152487 | 148534 | 1.15 | 5.24E-03 |
| FLJ20512 | 219219_at | NM_017854 | NM_017854 | 54958 | 1.15 | 2.66E-03 |
| C13orf7 | 219303_at | NM_024546 | NM_024546 | 79596 | 1.15 | 1.92E-07 |
| ADPN | 233030_at | AK025665 | NM_025225 | 80339 | 1.14 | 4.14E-03 |
| FANCL | 218397_at | NM_018062 | NM_018062 | 55120 | 1.14 | 1.80E-04 |
| MAK3 | 217745_s_at | NM_025146 | NM_025146 | 80218 | 1.14 | 3.70E-06 |
| H05254 | 244170_at | H05254 | --- | --- | 1.14 | 1.12E-03 |
| FAM14B | 230172_at | AL039706 | NM_145249 | 122509 | 1.14 | 5.19E-03 |
| | | | NM_206949 | | | |
| NR3C1 | 216321_s_at | X03348 | NM_000176 | 2908 | 1.14 | 4.77E-03 |
| FH | 203032_s_at | AI363836 | NM_000143 | 2271 | 1.14 | 1.19E-03 |
| SIVA | 203489_at | NM_006427 | NM_006427 | 10572 | 1.14 | 1.23E-04 |
| | | | NM_021709 | | | |
| C10orf58 | 224435_at | BC005871 | NM_032333 | 84293 | 1.14 | 2.67E-05 |
| MCAM | 211042_x_at | BC006329 | NM_006500 | 4162 | 1.14 | 2.97E-06 |
| LOC144997 | 227289_at | AU119437 | --- | 144997 | 1.14 | 8.41E-04 |
| SAFB2 | 32099_at | D50928 | NM_014649 | 9667 | 1.14 | 1.08E-03 |
| MTUS1 | 212093_s_at | AI695017 | NM_00100192 4 | 57509 | 1.14 | 1.51E-04 |
| | | | NM_00100192 5 | | | |
| | | | NM_00100192 7 | | | |
| | | | NM_00100193 1 | | | |
| | | | NM_020749 | | | |
| SORD | 201562_s_at | NM_003104 | NM_003104 | 6652 | 1.14 | 8.56E-04 |
| HMGB1 | 200679_x_at | BE311760 | NM_002128 | 3146 | 1.14 | 4.89E-05 |
| TUBB | 212320_at | BC001002 | NM_178014 | 203068 | 1.14 | 1.69E-04 |
| CDC42EP4 | 218062_x_at | NM_012121 | NM_012121 | 23580 | 1.13 | 4.75E-04 |
| PACSIN1 | 227053_at | N47315 | NM_020804 | 29993 | 1.13 | 8.60E-03 |
| KIAA1712 | 228334_x_at | AI633734 | NM_030633 | 80817 | 1.13 | 1.79E-03 |
| CDK2AP1 | 201938_at | NM_004642 | NM_004642 | 8099 | 1.13 | 4.73E-05 |
| AI650582 | 226475_at | AI650582 | --- | --- | 1.13 | 9.58E-03 |
| ZNF626 | 1552643_at | NM_145297 | NM_145297 | 199777 | 1.13 | 4.93E-04 |
| MAPKAPK3 | 202787_s_at | U43784 | NM_004635 | 7867 | 1.13 | 2.06E-03 |
| BTG3 | 205548_s_at | NM_006806 | NM_006806 | 10950 | 1.13 | 3.73E-03 |
| UBE2S | 202779_s_at | NM_014501 | NM_014501 | 27338 | 1.13 | 1.67E-04 |
| TP531NP1 | 225912_at | AW341649 | NM_033285 | 94241 | 1.13 | 6.83E-03 |
| Cep192 | 218827_s_at | NM_018069 | NM_018069 | 55125 | 1.13 | 3.17E-04 |
| | | | | | | |
| SUHW2 | 230789_at | AI015954 | NM_080764 | 140883 | 1.13 | 1.79E-05 |
| LOC128977 | 1553974_at | BC030758 | NM_173793 | 128977 | 1.12 | 4.40E-04 |
| CDH2 | 203441_s_at | NM_001792 | NM_001792 | 1000 | 1.12 | 5.79E-03 |
| ST8SIA1 | 210073_at | L32867 | NM_003034 | 6489 | 1.12 | 1.10E-03 |
| PTP4A1 | 200733_s_at | U48296 | NM_003463 | 7803 | 1.12 | 2.69E-03 |
| RNF34 | 219035_s_at | NM_025126 | NM_025126 | 80196 | 1.12 | 2.20E-04 |
| | | | NM_194271 | | | |
| DDX11 | 208159_x_at | NM_004399 | NM_004399 | 1663 | 1.12 | 2.66E-03 |
| | | | NM_030653 | | | |
| | | | NM_030655 | | | |
| PTGES2 | 218083_at | NM_025072 | NM_025072 | 80142 | 1.12 | 5.25E-05 |
| | | | NM_198938 | | | |
| | | | NM_198939 | | | |
| | | | NM_198940 | | | |
| PCCB | 212694_s_at | NM_000532 | NM_000532 | 5096 | 1.12 | 1.29E-03 |
| ETV4 | 211603_s_at | U35622 | NM_001986 | 2118 | 1.12 | 1.80E-03 |
| MGC7036 | 227983_at | AI810244 | NM_145058 | 196383 | 1.12 | 1.70E-05 |
| GAP43 | 204471_at | NM_002045 | NM_002045 | 2596 | 1.12 | 2.85E-05 |
| DATF1 | 222595_s_at | AL578222 | NM_022105 | 11083 | 1.12 | 9.82E-04 |
| | | | NM_080796 | | | |
| | | | NM_080797 | | | |
| C1orf41 | 215691_x_at | AV702994 | NM_016126 | 51668 | 1.11 | 6.15E-03 |
| CHES1 | 218031_s_at | NM_018589 | NM_005197 | 1112 | 1.11 | 3.51E-04 |
| LOC89944 | 213713_s_at | R48779 | NM_138342 | 89944 | 1.11 | 4.60E-03 |
| AI807356 | 227349_at | AI807356 | --- | --- | 1.11 | 7.01E-03 |
| RANGAP1 | 212127_at | BE379408 | NM_002883 | 5905 | 1.11 | 1.65E-03 |
| CHRNA5 | 206533_at | NM_000745 | NM_000745 | 1138 | 1.11 | 2.14E-03 |
| RNF138 | 239143_x_at | AW291944 | NM_016271 | 51444 | 1.11 | 1.74E-04 |
| | | | NM_198128 | | | |
| LOC440145 | 225578_at | AI885466 | XM_495961 | 440145 | 1.11 | 2.53E-04 |
| FGF1 | 1552721_a_at | NM_033136 | NM_000800 | 2246 | 1.11 | 1.39E-03 |
| | | | NM_033136 | | | |
| | | | NM_033137 | | | |
| AL533234 | 225725_at | AL533234 | --- | --- | 1.11 | 7.91E-04 |
| TM7SF3 | 222477_s_at | BC005176 | NM_016551 | 51768 | 1.11 | 5.89E-04 |
| FLJ90430 | 229533_x_at | AI963028 | NM_178558 | 340252 | 1.11 | 7.02E-03 |
| RPS27L | 218007_s_at | NM_015920 | NM_015920 | 51065 | 1.11 | 3.68E-03 |
| CRTAC1 | 221204_s_at | NM_018058 | NM_018058 | 55118 | 1.11 | 1.69E-03 |
| SKD3 | 221845_s_at | AI655698 | NM_030813 | 81570 | 1.11 | 1.33E-04 |
| ZNF85 | 206572_x_at | NM_003429 | NM_003429 | 7639 | 1.11 | 3.31E-03 |
| XRCC3 | 216299_s_at | AK022829 | NM_005432 | 7517 | 1.11 | 7.35E-05 |
| AKAP13 | 222024_s_at | AK022014 | NM_006738 | 11214 | 1.11 | 5.22E-03 |
| | | | NM_007200 | | | |
| | | | NM_144767 | | | |
| C9orf81 | 1557985_s_at | AA248753 | XM_095991 | 84131 | 1.11 | 6.85E-04 |
| PDCL | 204448_s_at | AF031463 | NM_005388 | 5082 | 1.10 | 4.06E-03 |
| RAPH1 | 243012_at | BF196252 | NM_025252 | 65059 | 1.10 | 2.75E-03 |
| | | | NM_203365 | | | |
| | | | NM_213589 | | | |
| DEK | 200934_at | NM_003472 | NM_003472 | 7913 | 1.10 | 5.01E-06 |
| PPIF | 201490_s_at | NM_005729 | NM_005729 | 10105 | 1.10 | 3.89E-06 |
| PTP4A1 | 200730_s_at | BF576710 | NM_003463 | 7803 | 1.10 | 4.25E-03 |
| AI888657 | 244189_at | AI888657 | --- | --- | 1.10 | 1.81E-05 |
| TCF7L2 | 216037_x_at | AA664011 | NM_030756 | 6934 | 1.10 | 1.01E-02 |
| SNX5 | 222417_s_at | AF121855 | NM_014426 | 27131 | 1.10 | 2.71E-03 |
| | | | NM_152227 | | | |
| SSH2 | 226080_at | BE676214 | NM_033389 | 85464 | 1.10 | 4.02E-04 |
| BE671123 | 235798_at | BE671123 | --- | --- | 1.10 | 2.84E-05 |
| MSH6 | 211450_s_at | D89646 | NM_000179 | 2956 | 1.10 | 4.87E-04 |
| PDZRN3 | 212915_at | AL569804 | NM_015009 | 23024 | 1.10 | 4.32E-03 |
| PPP3CB | 209817_at | M29550 | NM_021132 | 5532 | 1.10 | 4.16E-04 |
| FAM29A | 218602_s_at | NM_017645 | NM_017645 | 54801 | 1.09 | 1.73E-04 |
| MINA | 1554774_at | AB083191 | NM_032778 | 84864 | 1.09 | 7.48E-03 |
| | | | NM_153182 | | | |
| SRGAP1 | 1569269_s_at | BM912471 | NM_020762 | 57522 | 1.09 | 8.29E-03 |
| LOC152185 | 234995_at | AA668779 | NM_144718 | 152185 | 1.09 | 2.38E-04 |
| LOC63929 | 227910_at | AI635379 | NM_022098 | 63929 | 1.09 | 2.09E-03 |
| ITPR2 | 202662_s_at | NM_002223 | NM_002223 | 3709 | 1.09 | 4.74E-03 |
| PCDH17 | 228863_at | N69091 | NM_014459 | 27253 | 1.09 | 2.79E-04 |
| ELAVL1 | 201727_s_at | NM_001419 | NM_001419 | 1994 | 1.09 | 1.03E-02 |
| PKP4 | 201928_at | AA194254 | NM_00100547 6 | 8502 | 1.09 | 5.68E-05 |
| | | | NM_003628 | | | |
| MTUS1 | 212096_s_at | AL096842 | NM_00100192 4 | 57509 | 1.09 | 8.18E-06 |
| | | | NM_00100192 5 | | | |
| | | | NM_00100192 7 | | | |
| | | | NM_00100193 1 | | | |
| | | | NM_020749 | | | |
| SPOCK | 202363_at | AF231124 | NM_004598 | 6695 | 1.09 | 4.27E-04 |
| DIAPH1 | 209190_s_at | AF051782 | NM_005219 | 1729 | 1.09 | 3.00E-03 |
| CKLFSF7 | 226017_at | AI708432 | NM_138410 | 112616 | 1.09 | 7.48E-04 |
| | | | NM_181472 | | | |
| CORO6 | 1552301_a_at | NM_032854 | NM_032854 | 84940 | 1.09 | 3.51E-03 |
| C18orf9 | 219311_at | NM_024899 | NM_024899 | 79959 | 1.09 | 7.95E-04 |
| PDGFD | 219304_s_at | NM_025208 | NM_025208 | 80310 | 1.09 | 1.44E-03 |
| | | | NM_033135 | | | |
| UBE2L3 | 200676_s_at | NM_003347 | NM_003347 | 7332 | 1.09 | 3.98E-06 |
| | | | NM_198157 | | | |
| SLA/LP | 227982_at | AI806592 | NM_016955 | 51091 | 1.08 | 6.59E-03 |
| | | | NM_153825 | | | |
| HOMER3 | 204647_at | NM_004838 | NM_004838 | 9454 | 1.08 | 1.80E-04 |
| SNRPD3 | 202567_at | NM_004175 | NM_004175 | 6634 | 1.08 | 1.90E-05 |
| 3'HEXO | 226416_at | AL137679 | NM_153332 | 90459 | 1.08 | 3.50E-03 |
| LMNB2 | 216952_s_at | M94363 | NM_032737 | 84823 | 1.08 | 2.28E-04 |
| CBX5 | 209715_at | L07515 | NM_012117 | 23468 | 1.08 | 2.29E-03 |
| ASF1A | 203428_s_at | AB028628 | NM_014034 | 25842 | 1.08 | 4.83E-05 |
| UBE2L3 | 200682_s_at | BG531983 | NM_003347 | 7332 | 1.08 | 7.87E-06 |
| | | | NM_198157 | | | |
| PDLIM5 | 212412_at | AV715767 | NM_00101151 3 | 10611 | 1.08 | 2.29E-03 |
| | | | NM_00101151 4 | | | |
| | | | NM_00101151 5 | | | |
| | | | NM_00101151 6 | | | |
| | | | NM_006457 | | | |
| PLEKHG3 | 212823_s_at | AI738980 | NM_015549 | 26030 | 1.08 | 9.61E-03 |
| ASXL1 | 244519_at | AI829840 | NM_015338 | 171023 | 1.08 | 1.17E-03 |
| HIST1H4C | 205967_at | NM_003542 | NM_003542 | 8364 | 1.08 | 4.91E-03 |
| UBE2L3 | 200683_s_at | BE964689 | NM_003347 | 7332 | 1.07 | 1.25E-06 |
| | | | NM_198157 | | | |
| DPYSL5 | 222797_at | BF508726 | NM_020134 | 56896 | 1.07 | 7.59E-04 |
| ZFP36L2 | 201368_at | U07802 | NM_006887 | 678 | 1.07 | 1.86E-04 |
| PUSL1 | 228733_at | AU154793 | NM_153339 | 126789 | 1.07 | 3.30E-03 |
| AA973128 | 242294_at | AA973128 | --- | --- | 1.07 | 3.63E-03 |
| SAP30 | 204899_s_at | BF247098 | NM_003864 | 8819 | 1.07 | 7.88E-05 |
| ETV4 | 1554576_a_at | BC007242 | NM_001986 | 2118 | 1.07 | 2.79E-03 |
| RARA | 203749_s_at | AI806984 | NM_000964 | 5914 | 1.07 | 3.55E-03 |
| TUBB | 211714_x_at | BC005838 | NM_178014 | 203068 | 1.07 | 3.33E-04 |
| STARD4 | 226390_at | AA628398 | NM_139164 | 134429 | 1.07 | 1.71E-05 |
| TUBA6 | 211750_x_at | BC005946 | NM_032704 | 84790 | 1.07 | 1.66E-05 |
| CKLFSF8 | 235099_at | AW080832 | NM_178868 | 152189 | 1.07 | 5.68E-03 |
| THOC3 | 224623_at | BE614567 | NM_032361 | 84321 | 1.07 | 3.08E-05 |
| PRPS1 | 209440_at | BC001605 | NM_002764 | 5631 | 1.07 | 2.15E-05 |
| PRPS2 | 203401_at | NM_002765 | NM_002765 | 5634 | 1.07 | 2.40E-03 |
| CRY1 | 209674_at | D83702 | NM_004075 | 1407 | 1.06 | 3.45E-03 |
| DKFZP547N043 | 223511_at | AL512744 | NM_00101098 4 | 83932 | 1.06 | 1.17E-03 |
| | | | NM_032018 | | | |
| FSTL1 | 208782_at | BC000055 | NM_007085 | 11167 | 1.06 | 2.60E-03 |
| NDRG2 | 214279_s_at | W74452 | NM_016250 | 57447 | 1.06 | 7.80E-03 |
| | | | NM_201535 | | | |
| | | | NM_201536 | | | |
| | | | NM_201537 | | | |
| | | | NM_201538 | | | |
| | | | NM_201539 | | | |
| | | | NM_201540 | | | |
| | | | NM_201541 | | | |
| FLJ13273 | 1554518_at | BC032942 | NM_024751 | 79807 | 1.06 | 4.36E-05 |
| EPB41L1 | 212336_at | AA912711 | NM_012156 | 2036 | 1.06 | 6.75E-05 |
| | | | NM_177996 | | | |
| FAM55C | 243011_at | BF317081 | NM_145037 | 91775 | 1.06 | 9.28E-03 |
| TCF3 | 213730_x_at | BE962186 | NM_003200 | 6929 | 1.06 | 4.18E-04 |
| PLXNB2 | 208890_s_at | BC004542 | XM_371474 | 23654 | 1.06 | 2.15E-04 |
| BE218067 | 243662_at | BE218067 | --- | --- | 1.06 | 6.88E-03 |
| PDRG1 | 225075_at | AL031658 | NM_030815 | 81572 | 1.06 | 4.96E-03 |
| BTG3 | 213134_x_at | AI765445 | NM_006806 | 10950 | 1.06 | 2.21E-03 |
| CDC42EP4 | 214721_x_at | AL162074 | NM_012121 | 23580 | 1.06 | 6.10E-04 |
| LOC96610 | 1569013_s_at | BC033148 | NM_080926 | 96610 | 1.06 | 2.71E-03 |
| BF510533 | 228107_at | BF510533 | --- | --- | 1.06 | 2.14E-03 |
| TFDP1 | 204147_s_at | NM_007111 | NM_007111 | 7027 | 1.06 | 2.66E-03 |
| AI092770 | 230466_s_at | AI092770 | --- | --- | 1.06 | 1.88E-03 |
| SFRP1 | 202037_s_at | NM_003012 | NM_003012 | 6422 | 1.06 | 2.39E-04 |
| FBXO22 | 225734_at | AW294765 | NM_012170 | 26263 | 1.06 | 1.77E-05 |
| | | | NM_147188 | | | |
| TUBB | 209026_x_at | AF141349 | NM_178014 | 203068 | 1.06 | 6.65E-04 |
| ELK3 | 221773_at | AW575374 | NM_005230 | 2004 | 1.05 | 1.33E-05 |
| APG4C | 228190_at | BF197009 | NM_032852 | 84938 | 1.05 | 3.09E-03 |
| | | | NM_178221 | | | |
| BCL11A | 210347_s_at | AF080216 | NM_018014 | 53335 | 1.05 | 4.33E-03 |
| | | | NM_022893 | | | |
| | | | NM_138553 | | | |
| | | | NM_138559 | | | |
| MFAP2 | 203417_at | NM_017459 | NM_002403 | 4237 | 1.05 | 2.57E-04 |
| | | | NM_017459 | | | |
| C20orf108 | 224693_at | AI133137 | NM_080821 | 116151 | 1.05 | 1.58E-03 |
| CD24 | 209772_s_at | X69397 | NM_013230 | 934 | 1.05 | 1.98E-04 |
| FAS | 204780_s_at | AA164751 | NM_000043 | 355 | 1.05 | 3.67E-04 |
| | | | NM_152871 | | | |
| | | | NM_152872 | | | |
| | | | NM_152873 | | | |
| | | | NM_152874 | | | |
| | | | NM_152875 | | | |
| | | | NM_152876 | | | |
| | | | NM_152877 | | | |
| GGCX | 214005_at | BE326952 | NM_000821 | 2677 | 1.05 | 6.80E-03 |
| SESN3 | 235684_s_at | BF685808 | NM_144665 | 143686 | 1.05 | 8.56E-03 |
| ANAPC7 | 225554_s_at | AA131793 | NM_016238 | 51434 | 1.05 | 3.09E-06 |
| INA | 230859_at | BF111276 | NM_032727 | 9118 | 1.05 | 1.36E-03 |
| FKBP7 | 223667_at | AF092137 | NM_016105 | 51661 | 1.05 | 1.99E-03 |
| | | | NM_181342 | | | |
| ESRRG | 207981_s_at | NM_001438 | NM_001438 | 2104 | 1.05 | 6.81E-03 |
| | | | NM_206594 | | | |
| | | | NM_206595 | | | |
| RET | 215771_x_at | X15786 | NM_020630 | 5979 | 1.05 | 9.85E-04 |
| | | | NM_020975 | | | |
| CTNNAL1 | 202468_s_at | NM_003798 | NM_003798 | 8727 | 1.05 | 1.23E-03 |
| SMC1L1 | 201589_at | D80000 | NM_006306 | 8243 | 1.05 | 3.76E-05 |
| NFYB | 218128_at | AU151875 | NM_006166 | 4801 | 1.04 | 1.55E-04 |
| NFYB | 218127_at | AI804118 | NM_006166 | 4801 | 1.04 | 2.59E-03 |
| EPOR | 209963_s_at | M34986 | NM_000121 | 2057 | 1.04 | 9.69E-04 |
| MGC10854 | 223455_at | BG493862 | NM_032300 | 84260 | 1.04 | 2.09E-03 |
| TCF3 | 210776_x_at | M31222 | NM_003200 | 6929 | 1.04 | 6.32E-05 |
| ELAVL4 | 238073_at | R55745 | NM_021952 | 1996 | 1.04 | 6.02E-07 |
| N66584 | 230593_at | N66584 | --- | --- | 1.04 | 1.67E-03 |
| MGC13204 | 225836_s_at | AL528840 | NM_031465 | 83695 | 1.04 | 4.81E-04 |
| NUCKS | 222424_s_at | BC000805 | NM_022731 | 64710 | 1.04 | 1.65E-03 |
| FBXO22 | 225737_s_at | BE966247 | NM_012170 | 26263 | 1.04 | 2.33E-04 |
| | | | NM_147188 | | | |
| CXorf40 | 228809_at | AW006998 | NM_178124 | 91966 | 1.04 | 6.72E-03 |
| NEFL | 221805_at | AL537457 | NM_006158 | 4747 | 1.04 | 3.74E-03 |
| MTHFD1 | 202309_at | NM_005956 | NM_005956 | 4522 | 1.04 | 1.63E-04 |
| SRD5A1 | 210959_s_at | AF113128 | NM_001047 | 6715 | 1.04 | 3.17E-03 |
| NASP | 201969_at | AW003362 | NM_002482 | 4678 | 1.04 | 8.40E-03 |
| | | | NM_152298 | | | |
| | | | NM_172164 | | | |
| Septin 10 | 212698_s_at | BF966021 | NM_144710 | 151011 | 1.04 | 4.13E-03 |
| | | | NM_178584 | | | |
| PLCXD1 | 218951_s_at | NM_018390 | NM_018390 | 55344 | 1.04 | 2.13E-03 |
| CD24 | 208650_s_at | BG327863 | NM_013230 | 934 | 1.04 | 5.35E-03 |
| ZNF92 | 235170_at | T52999 | NM_007139 | 168374 | 1.04 | 1.71E-05 |
| | | | NM_152626 | | | |
| CENPF | 207331_at | NM_016343 | NM_016343 | 1063 | 1.03 | 3.14E-04 |
| AA766126 | 244385_at | AA766126 | --- | --- | 1.03 | 2.59E-04 |
| TENS1 | 217853_at | NM_022748 | NM_022748 | 64759 | 1.03 | 1.28E-03 |
| AL559474 | 235918_x_at | AL559474 | --- | --- | 1.03 | 7.29E-04 |
| FLJ10407 | 234672_s_at | AL354612 | NM_018087 | 55706 | 1.03 | 4.74E-05 |
| SET8 | 220200_s_at | NM_020382 | NM_020382 | 387893 | 1.03 | 5.39E-04 |
| FLJ21125 | 226204_at | AI832193 | NM_024627 | 79680 | 1.03 | 7.12E-03 |
| PANK2 | 218809_at | NM_024960 | NM_024960 | 80025 | 1.03 | 5.90E-06 |
| | | | NM_153637 | | | |
| | | | NM_153638 | | | |
| | | | NM_153639 | | | |
| | | | NM_153640 | | | |
| | | | NM_153641 | | | |
| TAF5 | 210053_at | AW138827 | NM_006951 | 6877 | 1.03 | 3.76E-04 |
| AI806853 | 225601_at | AI806853 | --- | --- | 1.03 | 2.08E-05 |
| MMP11 | 203878_s_at | NM_005940 | NM_005940 | 4320 | 1.03 | 2.69E-03 |
| TUBA6 | 209251_x_at | BC004949 | NM_032704 | 84790 | 1.03 | 2.38E-05 |
| BCL2L11 | 225606_at | AI949179 | NM_006538 | 10018 | 1.03 | 1.11E-04 |
| | | | NM_138621 | | | |
| | | | NM_138622 | | | |
| | | | NM_138623 | | | |
| | | | NM_138624 | | | |
| | | | NM_138625 | | | |
| | | | NM_138626 | | | |
| | | | NM_138627 | | | |
| | | | NM_207002 | | | |
| | | | NM_207003 | | | |
| ZGPAT | 221848_at | AL121845 | NM_032527 | 84619 | 1.03 | 4.13E-04 |
| | | | NM_181484 | | | |
| | | | NM_181485 | | | |
| C14orf106 | 241816_at | AW103300 | NM_018353 | 55320 | 1.03 | 2.05E-03 |
| BCL3 | 204908_s_at | NM_005178 | NM_005178 | 602 | 1.03 | 7.14E-04 |
| CBFA2T2 | 209145_s_at | AF068266 | NM_005093 | 9139 | 1.03 | 4.72E-03 |
| | | | NM_175864 | | | |
| MCFP | 227012_at | AI823986 | NM_018843 | 55972 | 1.03 | 1.94E-06 |
| THAP10 | 219596_at | NM_020147 | NM_020147 | 56906 | 1.03 | 7.28E-04 |
| AW511319 | 227167_s_at | AW511319 | --- | --- | 1.03 | 6.48E-04 |
| C2orf7 | 223312_at | BC005069 | NM_032319 | 84279 | 1.03 | 8.00E-05 |
| TTC9 | 213172_at | AW235608 | XM_027236 | 23508 | 1.03 | 5.24E-03 |
| DOT1L | 231297_at | AI479899 | NM_032482 | 84444 | 1.03 | 7.59E-03 |
| TM7SF3 | 226478_at | BF540749 | NM_016551 | 51768 | 1.02 | 1.16E-03 |
| NFIA | 224976_at | R37335 | NM_005595 | 4774 | 1.02 | 4.91E-03 |
| MGC72104 | 243689_s_at | AI681945 | NM_207350 | 284802 | 1.02 | 6.29E-03 |
| DGCR14 | 204383_at | NM_022719 | NM_022719 | 8220 | 1.02 | 2.13E-03 |
| TUBB2 | 208977_x_at | BC004188 | NM_006088 | 10383 | 1.02 | 1.96E-05 |
| ZNF505 | 215758_x_at | AC007204 | NM_00100412 6 | 81931 | 1.02 | 1.93E-03 |
| | | | NM_031218 | | | |
| C6orf115 | 223361_at | AF116682 | XM_371848 | 58527 | 1.02 | 3.11E-04 |
| RAB3C | 242328_at | BG055348 | NM_138453 | 115827 | 1.02 | 5.07E-03 |
| CECR5 | 218592_s_at | NM_017829 | NM_017829 | 27440 | 1.02 | 1.72E-05 |
| | | | NM_033070 | | | |
| PSMD5 | 203447_at | AU157008 | NM_005047 | 5711 | 1.02 | 3.28E-06 |
| PHF6 | 225501_at | AK027039 | NM_032335 | 84295 | 1.02 | 3.94E-05 |
| | | | NM_032458 | | | |
| CSRP2BP | 228544_s_at | AA780252 | NM_020536 | 57325 | 1.02 | 3.08E-03 |
| | | | NM_177926 | | | |
| M96 | 209704_at | AL523380 | NM_007358 | 22823 | 1.01 | 2.10E-04 |
| PELI2 | 219132_at | NM_021255 | NM_021255 | 57161 | 1.01 | 2.52E-05 |
| RPA1 | 201529_s_at | NM_002945 | NM_002945 | 6117 | 1.01 | 1.36E-04 |
| TCF3 | 213811_x_at | AW062341 | NM_003200 | 6929 | 1.01 | 2.33E-03 |
| C6orf151 | 226263_at | AA630674 | NM_152551 | 154007 | 1.01 | 1.66E-03 |
| C6orf4 | 215411_s_at | AL008730 | NM_147200 | 10758 | 1.01 | 1.04E-02 |
| | | | NM_147686 | | | |
| DUSP6 | 208893_s_at | BC005047 | NM_001946 | 1848 | 1.01 | 4.12E-03 |
| | | | NM_022652 | | | |
| PEG10 | 212094_at | AL582836 | XM_496907 | 23089 | 1.01 | 4.74E-03 |
| | | | XM_499343 | | | |
| CTNNBL1 | 221021_s_at | NM_030877 | NM_030877 | 56259 | 1.01 | 1.87E-05 |
| MPHOSPH9 | 237158_s_at | AW449069 | NM_022782 | 10198 | 1.01 | 8.39E-04 |
| SCLY | 221575_at | BC000586 | NM_016510 | 51540 | 1.01 | 5.24E-04 |
| NUP50 | 222583_s_at | AF116624 | NM_007172 | 10762 | 1.01 | 9.47E-04 |
| | | | NM_153645 | | | |
| | | | NM_153684 | | | |
| GSTO2 | 227163_at | AL162742 | NM_183239 | 119391 | 1.01 | 9.87E-03 |
| C20orf44 | 222470_s_at | AA033998 | NM_018244 | 55245 | 1.01 | 9.65E-05 |
| | | | NM_199487 | | | |
| | | | NM_199513 | | | |
| N56956 | 227254_at | N56956 | --- | --- | 1.01 | 6.12E-03 |
| BAGE4 | 1555408_at | AF218570 | NM_181704 | 85317 | 1.01 | 6.41E-03 |
| BAGE2 | | | NM_182482 | 85319 | | |
| ARL2L1 | 228201_at | BE858032 | NM_144996 | 200894 | 1.01 | 2.30E-03 |
| | | | NM_182896 | | | |
| TCF7L2 | 212762_s_at | AI375916 | NM_030756 | 6934 | 1.01 | 2.14E-03 |
| C21orf45 | 219004_s_at | NM_018944 | NM_018944 | 54069 | 1.01 | 1.32E-03 |
| C10orf58 | 228155_at | BF512388 | NM_032333 | 84293 | 1.00 | 1.86E-05 |
| LYK5 | 52169_at | AI302185 | NM_00100378 6 | 92335 | 1.00 | 6.85E-04 |
| | | | NM_00100378 7 | | | |
| | | | NM_00100378 8 | | | |
| | | | NM_153335 | | | |
| PGF | 209652_s_at | BC001422 | NM_002632 | 5228 | 1.00 | 5.14E-03 |
| FLJ14627 | 221908_at | AL120375 | NM_032814 | 84900 | 1.00 | 7.78E-04 |
| NUP37 | 218622_at | NM_024057 | NM_024057 | 79023 | 1.00 | 2.06E-03 |
| SYT3 | 223901_at | AL136594 | NM_032298 | 84258 | 1.00 | 6.98E-05 |
| NARG1 | 222837_s_at | AF327722 | NM_057175 | 80155 | 1.00 | 1.83E-03 |
| RNF144 | 204040_at | NM_014746 | NM_014746 | 9781 | 1.00 | 1.07E-06 |
| MRPL35 | 222775_s_at | AK026220 | NM_016622 | 51318 | 1.00 | 1.64E-03 |
| | | | NM_145644 | | | |
| ROR1 | 205805_s_at | NM_005012 | NM_005012 | 4919 | 1.00 | 3.27E-03 |
| POP1 | 213449_at | D31765 | NM_015029 | 10940 | 1.00 | 7.21E-03 |
| ABTB2 | 213497_at | AL050374 | NM_145804 | 25841 | 1.00 | 2.06E-03 |
| ASCC3 | 212815_at | AA156961 | NM_006828 | 10973 | 1.00 | 4.84E-03 |
| HDGF | 216484_x_at | L24521 | NM_004494 | 3068 | 1.00 | 1.89E-03 |
| DUSP6 | 208892_s_at | BC003143 | NM_001946 | 1848 | 1.00 | 2.56E-03 |
| | | | NM_022652 | | | |
| DCPS | 218774_at | NM_014026 | NM_014026 | 28960 | 1.00 | 6.63E-03 |
| AKIP | 228800_x_at | BE463815 | NM_017900 | 54998 | 1.00 | 1.79E-03 |
| C6orf60 | 220150_s_at | NM_024581 | NM_024581 | 79632 | 1.00 | 2.46E-03 |
| NXT2 | 209629_s_at | AF201942 | NM_018698 | 55916 | 1.00 | 6.17E-03 |
| SEC61A2 | 219499_at | NM_018144 | NM_018144 | 55176 | 1.00 | 1.35E-05 |
| AI095542 | 235759_at | AI095542 | --- | --- | 1.00 | 3.08E-04 |
| RPA1 | 201528_at | BG398414 | NM_002945 | 6117 | 1.00 | 7.67E-06 |
| FLJ14627 | 221909_at | AW299700 | NM_032814 | 84900 | 1.00 | 1.91E-03 |
| BE967311 | 226225_at | BE967311 | --- | --- | 0.99 | 6.96E-05 |
| KIAA0841 | 213054_at | AA845355 | XM_049237 | 23354 | 0.99 | 5.40E-05 |
| GNG2 | 224965_at | AU118419 | NM_053064 | 54331 | 0.99 | 5.01E-05 |
| ZNF238 | 207164_s_at | NM_006352 | NM_006352 | 10472 | 0.99 | 3.24E-03 |
| | | | NM_205768 | | | |
| PSMD9 | 209334_s_at | BC002383 | NM_002813 | 5715 | 0.99 | 2.73E-04 |
| SNX5 | 217792_at | NM_014426 | NM_014426 | 27131 | 0.99 | 1.19E-05 |
| | | | NM_152227 | | | |
| PHLDA3 | 218634_at | NM_012396 | NM_012396 | 23612 | 0.99 | 4.00E-04 |
| ITGA2B | 206494_s_at | NM_000419 | NM_000419 | 3674 | 0.99 | 8.82E-03 |
| SOX2 | 213722_at | AW007161 | NM_003106 | 6657 | 0.99 | 7.14E-03 |
| RALY | 201271_s_at | NM_016732 | NM_007367 | 22913 | 0.99 | 1.03E-05 |
| | | | NM_016732 | | | |
| PCNT1 | 218014_at | NM_024844 | NM_024844 | 79902 | 0.99 | 2.81E-03 |
| LOC400506 | 213237_at | AI652058 | XM_375305 | 400506 | 0.99 | 8.09E-04 |
| SLC6A15 | 240419_at | N25883 | NM_018057 | 55117 | 0.99 | 6.25E-03 |
| | | | NM_182767 | | | |
| CBFA2T2 | 209144_s_at | AI810484 | NM_005093 | 9139 | 0.99 | 5.43E-03 |
| | | | NM_175864 | | | |
| ASMTL | 36554_at | Y15521 | NM_004192 | 8623 | 0.99 | 2.79E-04 |
| NARG1 | 226998_at | AL556909 | NM_057175 | 80155 | 0.99 | 2.85E-05 |
| KLHL5 | 226001_at | AK002174 | NM_00100707 5 | 51088 | 0.99 | 5.50E-04 |
| | | | NM_199039 | | | |
| MGC59937 | 1569144_a_at | BC018787 | NM_199001 | 375791 | 0.98 | 9.69E-03 |
| NEDD4L | 212445_s_at | AI357376 | NM_015277 | 23327 | 0.98 | 3.46E-03 |
| COVA1 | 32042_at | S72904 | NM_006375 | 10495 | 0.98 | 1.78E-04 |
| | | | NM_182314 | | | |
| SWAP70 | 209306_s_at | AI139569 | NM_015055 | 23075 | 0.98 | 1.45E-03 |
| EPLIN | 217892_s_at | NM_016357 | NM_016357 | 51474 | 0.98 | 1.73E-03 |
| TEF | 225840_at | AA779795 | NM_003216 | 7008 | 0.98 | 3.69E-03 |
| KIAA0391 | 202713_s_at | AA129755 | XM_375074 | 9692 | 0.98 | 7.23E-05 |
| TXNRD1 | 201266_at | NM_003330 | NM_003330 | 7296 | 0.98 | 7.52E-04 |
| | | | NM_182729 | | | |
| | | | NM_182742 | | | |
| | | | NM_182743 | | | |
| AI220627 | 213608_s_at | AI220627 | XM_377713 | 402055 | 0.98 | 1.61E-04 |
| SLC25A17 | 211754_s_at | BC005957 | NM_006358 | 10478 | 0.98 | 1.22E-03 |
| DDX11 | 208149_x_at | NM_030653 | NM_004399 | 1663 | 0.98 | 2.49E-03 |
| | | | NM_030653 | | | |
| | | | NM_030655 | | | |
| CD24 | 266_s_at | L33930 | NM_013230 | 934 | 0.98 | 9.60E-04 |
| SNAP29 | 218327_s_at | NM_004782 | NM_004782 | 9342 | 0.98 | 4.38E-04 |
| DDEFL1 | 219103_at | NM_017707 | NM_017707 | 55616 | 0.98 | 8.35E-03 |
| MTAP | 211363_s_at | AF109294 | NM_002451 | 4507 | 0.98 | 8.59E-03 |
| H2AFJ | 225245_x_at | BG386566 | NM_018267 | 55766 | 0.98 | 9.29E-03 |
| | | | NM_177925 | | | |
| W91876 | 230240_at | W91876 | --- | --- | 0.98 | 6.38E-03 |
| FLJ38973 | 227973_at | BF130937 | NM_153689 | 205327 | 0.97 | 4.61E-03 |
| ARL6 | 235320_at | AL138043 | NM_032146 | 84100 | 0.97 | 9.35E-03 |
| | | | NM_177976 | | | |
| WWOX | 219077_s_at | NM_016373 | NM_016373 | 51741 | 0.97 | 2.03E-03 |
| | | | NM_018560 | | | |
| | | | NM_130788 | | | |
| | | | NM_130790 | | | |
| | | | NM_130791 | | | |
| | | | NM_130792 | | | |
| | | | NM_130844 | | | |
| XTP3TPA | 218069_at | NM_024096 | NM_024096 | 79077 | 0.97 | 1.83E-04 |
| NAPB | 225111_s_at | AK022817 | NM_022080 | 63908 | 0.97 | 1.32E-05 |
| MRVI1 | 226047_at | N66571 | NM_006069 | 10335 | 0.97 | 6.11E-03 |
| | | | NM_130385 | | | |
| H93038 | 241574_s_at | H93038 | --- | --- | 0.97 | 4.24E-03 |
| AI700633 | 212812_at | AI700633 | --- | --- | 0.97 | 4.71E-04 |
| DDAH2 | 215537_x_at | AJ012008 | NM_013974 | 23564 | 0.97 | 4.32E-03 |
| HSPC159 | 219998_at | NM_014181 | NM_014181 | 29094 | 0.97 | 7.46E-04 |
| GRK6 | 202849_x_at | NM_002082 | NM_00100410 5 | 2870 | 0.97 | 5.10E-03 |
| | | | NM_00100410 6 | | | |
| | | | NM_002082 | | | |
| ZNF219 | 219314_s_at | NM_016423 | NM_016423 | 51222 | 0.97 | 2.40E-03 |
| KLF3 | 219657_s_at | NM_016531 | NM_016531 | 51274 | 0.97 | 2.90E-03 |
| K03460 | 216323_x_at | K03460 | --- | --- | 0.96 | 1.11E-03 |
| IER5 | 218611_at | NM_016545 | NM_016545 | 51278 | 0.96 | 1.63E-03 |
| BC040884 | 1560156_at | BC040884 | --- | --- | 0.96 | 8.01E-03 |
| PROS1 | 207808_s_at | NM_000313 | NM_000313 | 5627 | 0.96 | 4.30E-03 |
| ALDH1B1 | 209645_s_at | NM_000692 | NM_000692 | 219 | 0.96 | 5.95E-03 |
| PEG10 | 212092_at | BE858180 | XM_496907 | 23089 | 0.96 | 1.83E-03 |
| | | | XM_499343 | | | |
| GRK6 | 211543_s_at | AF040752 | NM_00100410 5 | 2870 | 0.96 | 4.44E-04 |
| | | | NM_00100410 6 | | | |
| | | | NM_002082 | | | |
| RPP30 | 203436_at | NM_006413 | NM_006413 | 10556 | 0.96 | 4.98E-04 |
| AD-003 | 223369_at | BC001396 | NM_014064 | 28989 | 0.96 | 5.09E-03 |
| BG026236 | 230166_at | BG026236 | --- | --- | 0.96 | 6.20E-03 |
| KIAA2002 | 225913_at | AK025943 | XM_370878 | 79834 | 0.96 | 8.75E-03 |
| CD24 | 208651_x_at | M58664 | NM_013230 | 934 | 0.96 | 1.62E-03 |
| TCF3 | 209153_s_at | M31523 | NM_003200 | 6929 | 0.96 | 1.74E-04 |
| NMT1 | 201157 _s_at | AF020500 | NM_021079 | 4836 | 0.96 | 7.38E-03 |
| SURB7 | 209363_s_at | U46837 | NM_004264 | 9412 | 0.96 | 1.98E-04 |
| PHYHIPL | 226623_at | AI829726 | NM_032439 | 84457 | 0.96 | 4.52E-06 |
| HSPC117 | 200042_at | NM_014306 | NM_014306 | 51493 | 0.96 | 2.37E-05 |
| ALDH7A1 | 208950_s_at | BC002515 | NM_001182 | 501 | 0.96 | 1.54E-05 |
| TFDP2 | 226157_at | AI569747 | NM_006286 | 7029 | 0.95 | 1.73E-03 |
| SURB7 | 209362_at | AI688580 | NM_004264 | 9412 | 0.95 | 3.64E-04 |
| BC003636 | 222756_s_at | BC003636 | --- | --- | 0.95 | 1.07E-04 |
| SCLY | 59705_at | AA911739 | NM_016510 | 51540 | 0.95 | 8.43E-04 |
| MGC15634 | 242923_at | AW027457 | --- | 84841 | 0.95 | 9.36E-03 |
| KIBRA | 216074_x_at | AK001727 | NM_015238 | 23286 | 0.95 | 6.38E-03 |
| CASP8AP2 | 222201_s_at | AB037736 | NM_012115 | 9994 | 0.95 | 4.11E-04 |
| AARS | 201000_at | NM_001605 | NM_001605 | 16 | 0.95 | 1.89E-03 |
| LOC148203 | 229700_at | BE966267 | --- | 148203 | 0.95 | 3.12E-04 |
| CBLL1 | 220018_at | NM_024814 | NM_024814 | 79872 | 0.95 | 1.63E-03 |
| MRPL35 | 225255_at | AA810707 | NM_016622 | 51318 | 0.95 | 4.17E-03 |
| | | | NM_145644 | | | |
| FAM29A | 233655_s_at | AK022964 | NM_017645 | 54801 | 0.95 | 1.14E-05 |
| DKFZP566E144 | 223989_s_at | BC003502 | NM_015523 | 25996 | 0.95 | 1.35E-04 |
| PHF20 | 235389_at | BG168139 | NM_016436 | 51230 | 0.95 | 7.86E-06 |
| C20orf23 | 219570_at | NM_024704 | NM_024704 | 55614 | 0.95 | 7.76E-04 |
| MRS2L | 228542_at | AI631209 | NM_020662 | 57380 | 0.95 | 5.03E-04 |
| GPX3 | 201348_at | NM_002084 | NM_002084 | 2878 | 0.95 | 6.41E-03 |
| PRIM2A | 205628_at | NM_000947 | NM_000947 | 5558 | 0.95 | 1.53E-04 |
| PPAT | 209433_s_at | AI457120 | NM_002703 | 5471 | 0.95 | 1.28E-06 |
| SLC9A3R1 | 201349_at | NM_004252 | NM_004252 | 9368 | 0.95 | 3.16E-04 |
| SNX5 | 229980_s_at | AA131508 | NM_014426 | 27131 | 0.95 | 2.51E-04 |
| | | | NM_152227 | | | |
| C9orf122 | 1557014_a_at | BC041970 | XM_379636 | 158228 | 0.95 | 9.32E-03 |
| ETV6 | 235056_at | AV722693 | NM_001987 | 2120 | 0.95 | 1.50E-03 |
| ROD1 | 224617_at | AI735576 | NM_005156 | 9991 | 0.95 | 5.78E-04 |
| PPP2R5D | 202513_s_at | NM_006245 | NM_006245 | 5528 | 0.95 | 5.76E-03 |
| | | | NM_180976 | | | |
| | | | NM_180977 | | | |
| AI392908 | 230352_at | AI392908 | --- | --- | 0.95 | 5.22E-03 |
| PPAT | 209434_s_at | U00238 | NM_002703 | 5471 | 0.95 | 1.94E-06 |
| HSPC129 | 1555106_a_at | BC035744 | NM_016396 | 51496 | 0.94 | 1.87E-04 |
| KIAA1333 | 223256_at | AW007694 | NM_017769 | 55632 | 0.94 | 1.83E-05 |
| GAP43 | 216963_s_at | AF279774 | NM_002045 | 2596 | 0.94 | 8.23E-05 |
| C6orf155 | 226810_at | BE500942 | NM_024882 | 79940 | 0.94 | 3.25E-04 |
| FKBP7 | 224002_s_at | AF100751 | NM_016105 | 51661 | 0.94 | 7.01E-04 |
| | | | NM_181342 | | | |
| NIT2 | 218557_at | NM_020202 | NM_020202 | 56954 | 0.94 | 8.71E-04 |
| NTAN1 | 213061_s_at | AA643304 | NM_173474 | 123803 | 0.94 | 3.59E-03 |
| FBXL13 | 243057_at | AI923673 | NM_145032 | 222235 | 0.94 | 1.12E-03 |
| SURF5 | 206593_s_at | NM_006752 | NM_006752 | 6837 | 0.94 | 1.04E-03 |
| | | | NM_133640 | | | |
| | | | NM_181491 | | | |
| ASMTL | 209394_at | BC002508 | NM_004192 | 8623 | 0.94 | 7.15E-04 |
| PXMP4 | 219428_s_at | BF057649 | NM_007238 | 11264 | 0.94 | 1.03E-03 |
| | | | NM_183397 | | | |
| IGF1R | 203627_at | AI830698 | NM_000875 | 3480 | 0.94 | 5.54E-04 |
| HN1 | 217755_at | NM_016185 | NM_00100203 2 | 51155 | 0.94 | 1.06E-03 |
| | | | NM_00100203 3 | | | |
| | | | NM_016185 | | | |
| AL833487 | 1562245_a_at | AL833487 | --- | --- | 0.94 | 2.02E-03 |
| LIG3 | 204123_at | NM_013975 | NM_002311 | 3980 | 0.93 | 1.64E-05 |
| | | | NM_013975 | | | |
| CSTF1 | 202190_at | NM_001324 | NM_001324 | 1477 | 0.93 | 1.15E-03 |
| KIAA0406 | 212898_at | AB007866 | NM_014657 | 9675 | 0.93 | 4.37E-05 |
| LIG3 | 207348_s_at | NM_002311 | NM_002311 | 3980 | 0.93 | 5.83E-05 |
| | | | NM_013975 | | | |
| MAML3 | 242794_at | AI569476 | NM_018717 | 55534 | 0.93 | 5.09E-03 |
| SRD5A1 | 211056_s_at | BC006373 | NM_001047 | 6715 | 0.93 | 1.03E-02 |
| ELAVL4 | 206051_at | NM_021952 | NM_021952 | 1996 | 0.93 | 8.90E-05 |
| XRCC4 | 210813_s_at | BC005259 | NM_003401 | 7518 | 0.93 | 5.17E-03 |
| | | | NM_022406 | | | |
| | | | NM_022550 | | | |
| ALDH7A1 | 208951_at | BC002515 | NM_001182 | 501 | 0.93 | 4.00E-06 |
| SLCO3A1 | 227367_at | AW976431 | NM_013272 | 28232 | 0.93 | 4.38E-03 |
| OGFRL1 | 219582_at | NM_024576 | NM_024576 | 79627 | 0.93 | 1.65E-04 |
| CDH2 | 203440_at | M34064 | NM_001792 | 1000 | 0.93 | 9.39E-06 |
| FLJ31842 | 237515_at | AA054642 | NM_152487 | 148534 | 0.93 | 6.73E-04 |
| LOC283464 | 226868_at | BF977231 | XM_290597 | 283464 | 0.93 | 1.55E-04 |
| DNMT1 | 201697_s_at | NM_001379 | NM_001379 | 1786 | 0.93 | 1.35E-04 |
| H2AFZ | 200853_at | NM_002106 | NM_002106 | 3015 | 0.93 | 1.62E-06 |
| GAS2L1 | 209729_at | BC001782 | NM_006478 | 10634 | 0.93 | 6.61E-03 |
| | | | NM_152236 | | | |
| | | | NM_152237 | | | |
| IDH2 | 210046_s_at | U52144 | NM_002168 | 3418 | 0.93 | 2.00E-04 |
| LOC51337 | 218500_at | NM_016647 | NM_016647 | 51337 | 0.93 | 2.50E-03 |
| DKFZp313A243: | 230005_at | AI742358 | --- | 258010 | 0.93 | 1.41E-03 |
| CKLF | 223451_s_at | AF096895 | NM_016326 | 51192 | 0.93 | 7.64E-03 |
| | | | NM_016951 | | | |
| | | | NM_181640 | | | |
| | | | NM_181641 | | | |
| APEX2 | 204408_at | NM_014481 | NM_014481 | 27301 | 0.92 | 5.63E-04 |
| UBOX5 | 215544_s_at | AL121891 | NM_014948 | 22888 | 0.92 | 5.63E-03 |
| | | | NM_199415 | | | |
| TMEM38B | 222735_at | AW452608 | NM_018112 | 55151 | 0.92 | 1.79E-03 |
| GPX2 | 202831_at | NM_002083 | NM_002083 | 2877 | 0.92 | 9.43E-04 |
| MPHOSPH9 | 215731_s_at | X98258 | NM_022782 | 10198 | 0.92 | 1.91E-04 |
| HABP4 | 232341_x_at | AK025144 | NM_014282 | 22927 | 0.92 | 1.03E-02 |
| C20orf161 | 1553960_at | CA447177 | NM_033421 | 90203 | 0.92 | 8.23E-03 |
| | | | NM_152897 | | | |
| FKBP5 | 224840_at | AI753747 | NM_004117 | 2289 | 0.92 | 1.63E-03 |
| MGC3162 | 218860_at | NM_024078 | NM_024078 | 79050 | 0.92 | 6.27E-05 |
| DKFZp313A243: | 230285_at | BF447829 | --- | 258010 | 0.92 | 4.30E-03 |
| AA669799 | 36553_at | AA669799 | --- | --- | 0.92 | 2.04E-04 |
| N93774 | 229671_s_at | N93774 | --- | --- | 0.92 | 3.48E-03 |
| AW971254 | 222369_at | AW971254 | --- | --- | 0.92 | 3.06E-03 |
| BF516305 | 230728_at | BF516305 | --- | --- | 0.92 | 9.61E-04 |
| ALDH1A2 | 207016_s_at | AB015228 | NM_003888 | 8854 | 0.92 | 7.68E-03 |
| | | | NM_170696 | | | |
| | | | NM_170697 | | | |
| ZNF600 | 242463_x_at | AI620827 | NM_198457 | 162966 | 0.92 | 6.82E-03 |
| BUB3 | 201458_s_at | NM_004725 | NM_00100779 3 | 9184 | 0.92 | 3.29E-04 |
| | | | NM_004725 | | | |
| FZD8 | 227405_s_at | AW340311 | NM_031866 | 8325 | 0.92 | 7.53E-04 |
| RAD1 | 228535_at | AI796010 | NM_002853 | 5810 | 0.92 | 3.58E-04 |
| | | | NM_133282 | | | |
| | | | NM_133377 | | | |
| GCAT | 205164_at | NM_014291 | NM_014291 | 23464 | 0.92 | 4.73E-03 |
| DKFZp761A078 | 231895_at | AA501453 | NM_194292 | 163786 | 0.92 | 1.89E-04 |
| BE464337 | 229512_at | BE464337 | --- | --- | 0.92 | 2.81E-03 |
| AI670824 | 238887_at | AI670824 | --- | --- | 0.91 | 4.22E-03 |
| USP49 | 212872_s_at | AK023092 | NM_004275 | 9477 | 0.91 | 1.13E-04 |
| HTATSF1 | 202602_s_at | NM_014500 | NM_014500 | 27336 | 0.91 | 3.64E-05 |
| MAML3 | 228508_at | AI828006 | NM_018717 | 55534 | 0.91 | 2.27E-04 |
| UCHL5 | 219960_s_at | NM_015984 | NM_015984 | 51377 | 0.91 | 1.62E-03 |
| MKKS | 222530_s_at | AF275813 | NM_018848 | 8195 | 0.91 | 1.32E-04 |
| | | | NM_170784 | | | |
| C18orf9 | 52285_f_at | AW002970 | NM_024899 | 79959 | 0.91 | 2.78E-06 |
| ADRBK2 | 204183_s_at | AI478542 | NM_005160 | 157 | 0.91 | 5.18E-03 |
| LOC90313 | 225605_at | AL540867 | NM_138349 | 90313 | 0.91 | 5.20E-03 |
| FANCE | 220255_at | NM_021922 | NM_021922 | 2178 | 0.91 | 1.82E-03 |
| SH3BGRL2 | 225354_s_at | AL035700 | NM_031469 | 83699 | 0.91 | 3.32E-04 |
| YEATS4 | 218911_at | NM_006530 | NM_006530 | 8089 | 0.91 | 7.53E-05 |
| FKBP14 | 219390_at | NM_017946 | NM_017946 | 55033 | 0.91 | 3.85E-06 |
| KIAA1712 | 231850_x_at | AB051499 | NM_030633 | 80817 | 0.91 | 6.64E-03 |
| C10orf78 | 236027_at | N23587 | NM_00100275 9 | 119392 | 0.91 | 4.28E-03 |
| | | | NM_145247 | | | |
| BG261385 | 225906_at | BG261385 | XM_499448 | 442703 | 0.91 | 2.60E-03 |
| LOC339903 | 228721_at | AA225165 | XM_291063 | 339903 | 0.91 | 2.86E-03 |
| KIBRA | 213085_s_at | AB020676 | NM_015238 | 23286 | 0.91 | 5.30E-05 |
| KIAA1333 | 223255_at | AA642341 | NM_017769 | 55632 | 0.91 | 1.02E-03 |
| H2AFJ | 224301_x_at | BC003602 | NM_018267 | 55766 | 0.91 | 8.07E-03 |
| | | | NM_177925 | | | |
| HRASLS | 219984_s_at | NM_020386 | NM_020386 | 57110 | 0.91 | 1.08E-03 |
| TUBA3 | 212639_x_at | AL581768 | NM_006009 | 10376 | 0.91 | 2.50E-05 |
| K-ALPHA-1 | | | NM_006082 | 7846 | | |
| TOMM34 | 201870_at | NM_006809 | NM_006809 | 10953 | 0.91 | 6.55E-05 |
| RFX5 | 202964_s_at | NM_000449 | NM_000449 | 5993 | 0.91 | 7.31E-04 |
| AK1 | 202587_s_at | BC001116 | NM_000476 | 203 | 0.91 | 7.38E-03 |
| KATNA1 | 205526_s_at | NM_007044 | NM_007044 | 11104 | 0.91 | 2.54E-03 |
| PAICS | 201014_s_at | NM_006452 | NM_006452 | 10606 | 0.91 | 3.31E-03 |
| KIAA1576 | 226415_at | AA156723 | NM_020927 | 57687 | 0.90 | 4.18E-03 |
| CHML | 1565951_s_at | AJ293392 | NM_001821 | 1122 | 0.90 | 1.84E-04 |
| SLC2A8 | 218985_at | NM_014580 | NM_014580 | 29988 | 0.90 | 1.85E-04 |
| DKFZp779O175 | 238952_x_at | BF439163 | XM_375606 | 374899 | 0.90 | 1.98E-03 |
| FLJ43505 | 221477_s_at | BF575213 | NM_207468 | 400823 | 0.90 | 2.64E-05 |
| HTLF | 226711_at | BF590117 | NM_002158 | 3344 | 0.90 | 2.44E-05 |
| FLJ10385 | 44563_at | AI858000 | NM_018081 | 55135 | 0.90 | 1.42E-05 |
| FLJ16517 | 229349_at | AL039884 | NM_001004317 | 389421 | 0.90 | 7.06E-04 |
| SIX5 | 229009_at | AA527770 | NM_175875 | 147912 | 0.90 | 7.46E-03 |
| STMN3 | 222557_at | AL353715 | NM_015894 | 50861 | 0.90 | 3.11E-03 |
| PPP2R5D | 211159_s_at | AB000635 | NM_006245 | 5528 | 0.90 | 3.08E-03 |
| | | | NM_180976 | | | |
| | | | NM_180977 | | | |
| AI701857 | 243359_at | AI701857 | --- | --- | 0.90 | 7.55E-04 |
| PHYH | 203335_at | NM_006214 | NM_006214 | 5264 | 0.90 | 1.50E-03 |
| UFD1L | 209103_s_at | BC001049 | NM_005659 | 7353 | 0.90 | 7.15E-04 |
| RAD1 | 204460_s_at | AF074717 | NM_002853 | 5810 | 0.90 | 1.59E-03 |
| | | | NM_133282 | | | |
| | | | NM_133377 | | | |
| MPHOSPH9 | 221965_at | AI990326 | NM_022782 | 10198 | 0.90 | 5.47E-04 |
| MLF1 | 204783_at | AI911434 | NM_022443 | 4291 | 0.90 | 1.40E-03 |
| THOC4 | 226319_s_at | AF047002 | NM_005782 | 10189 | 0.90 | 3.55E-05 |
| TM4SF15 | 218693_at | NM_012339 | NM_012339 | 23555 | 0.90 | 1.06E-03 |
| MSH6 | 202911_at | NM_000179 | NM_000179 | 2956 | 0.90 | 8.89E-05 |
| ANXA6 | 200982_s_at | NM_001155 | NM_001155 | 309 | 0.90 | 2.34E-06 |
| | | | NM_004033 | | | |
| RAB23 | 223463_at | AF161486 | NM_016277 | 51715 | 0.90 | 1.40E-03 |
| | | | NM_183227 | | | |
| PCTK1 | 207239_s_at | NM_006201 | NM_006201 | 5127 | 0.90 | 2.68E-03 |
| | | | NM_033018 | | | |
| ZNF238 | 212774_at | AJ223321 | NM_006352 | 10472 | 0.90 | 9.30E-04 |
| | | | NM_205768 | | | |
| HIP1R | 209558_s_at | AB013384 | NM_003959 | 9026 | 0.90 | 2.26E-04 |
| UBAP2L | 201378_s_at | NM_014847 | NM_014847 | 9898 | 0.90 | 3.17E-04 |
| FLJ32745 | 235644_at | BF213953 | NM_144978 | 165055 | 0.90 | 2.95E-03 |
| GGCX | 214006_s_at | BE326952 | NM_000821 | 2677 | 0.89 | 1.19E-03 |
| HIP1 | 226364_at | AU145049 | NM_005338 | 3092 | 0.89 | 1.85E-04 |
| BG496998 | 225684_at | BG496998 | --- | --- | 0.89 | 7.38E-05 |
| PIGX | 1563111_a_at | AK000529 | NM_017861 | 54965 | 0.89 | 1.57E-03 |
| RAN | 200750_s_at | AF054183 | NM_006325 | 5901 | 0.89 | 7.04E-07 |
| LOC128977 | 227086_at | AA194312 | NM_173793 | 128977 | 0.89 | 7.56E-04 |
| PRIM2A | 1554885_a_at | BC017833 | NM_000947 | 5558 | 0.89 | 1.97E-03 |
| ACY1L2 | 225421_at | AI654133 | NM_001010853 | 135293 | 0.89 | 1.02E-04 |
| FLJ39582 | 239015_at | BE675073 | XM_498459 | 439931 | 0.89 | 4.23E-03 |
| | | | XM_499589 | | | |
| CDK5RAP2 | 233540_s_at | AK025867 | NM_00101164 9 | 55755 | 0.89 | 1.44E-03 |
| | | | NM_018249 | | | |
| HIP1 | 205425_at | NM_005338 | NM_005338 | 3092 | 0.89 | 3.21E-04 |
| RNF157 | 226433_at | BF056204 | NM_052916 | 114804 | 0.89 | 4.40E-04 |
| AL832468 | 1560116_a_at | AL832468 | --- | --- | 0.89 | 1.66E-04 |
| CDC5L | 209056_s_at | NM_001253 | NM_001253 | 988 | 0.89 | 2.06E-05 |
| C20orf17 | 235616_at | AI694059 | NM_173485 | 128553 | 0.89 | 7.79E-03 |
| STAU2 | 204226_at | NM_014393 | NM_014393 | 27067 | 0.89 | 4.24E-05 |
| TUBB2 | 213726_x_at | AA515698 | NM_006088 | 10383 | 0.89 | 1.59E-04 |
| TMEPAI | 222449_at | AL035541 | NM_020182 | 56937 | 0.89 | 8.38E-03 |
| | | | NM_199169 | | | |
| | | | NM_199170 | | | |
| | | | NM_199171 | | | |
| KIAA1958 | 239033_at | AI640482 | NM_133465 | 158405 | 0.89 | 2.35E-04 |
| TEAD3 | 209454_s_at | AF142482 | NM_003214 | 7005 | 0.89 | 3.41E-03 |
| KIAA1211 | 227231_at | AI991996 | XM_044178 | 57482 | 0.88 | 1.97E-03 |
| TMEFF1 | 205122_at | BF439316 | NM_003692 | 8577 | 0.88 | 6.32E-03 |
| SLC16A10 | 219915_s_at | NM_018593 | NM_018593 | 117247 | 0.88 | 7.05E-04 |
| ACPL2 | 226925_at | AW069729 | NM_152282 | 92370 | 0.88 | 5.44E-07 |
| C1orf41 | 203960_s_at | NM_016126 | NM_016126 | 51668 | 0.88 | 6.92E-03 |
| VPS29 | 223026_s_at | AF201946 | NM_016226 | 51699 | 0.88 | 3.36E-04 |
| | | | NM_057180 | | | |
| FRAT2 | 209864_at | AB045118 | NM_012083 | 23401 | 0.88 | 9.56E-03 |
| IGSF4 | 209031_at | AL519710 | NM_014333 | 23705 | 0.88 | 1.80E-03 |
| PARP2 | 215773_x_at | AJ236912 | NM_005484 | 10038 | 0.88 | 1.31E-03 |
| SLCO3A1 | 219229_at | NM_013272 | NM_013272 | 28232 | 0.88 | 8.63E-04 |
| RNPC1 | 212430_at | AL109955 | NM_017495 | 55544 | 0.88 | 4.48E-03 |
| | | | NM_183425 | | | |
| STAMBP | 202811_at | NM_006463 | NM_006463 | 10617 | 0.88 | 3.92E-04 |
| | | | NM_201647 | | | |
| | | | NM_213622 | | | |
| RPS6KA1 | 203379_at | NM_002953 | NM_00100666 5 | 6195 | 0.88 | 2.48E-03 |
| | | | NM_002953 | | | |
| BUB3 | 201456_s_at | AU160695 | NM_00100779 3 | 9184 | 0.88 | 1.21E-04 |
| | | | NM_004725 | | | |
| LOC375295 | 228564_at | AI569804 | XM_374020 | 375295 | 0.88 | 2.04E-03 |
| KCNQ2 | 210508_s_at | D82346 | NM_004518 | 3785 | 0.88 | 1.23E-04 |
| | | | NM_172106 | | | |
| | | | NM_172107 | | | |
| | | | NM_172108 | | | |
| | | | NM_172109 | | | |
| EIF4E | 201437_s_at | NM_001968 | NM_001968 | 1977 | 0.88 | 2.52E-03 |
| AGPS | 225113_at | BF688144 | NM_003659 | 8540 | 0.88 | 2.42E-03 |
| BRD4 | 202103_at | AI991631 | NM_014299 | 23476 | 0.88 | 2.06E-03 |
| | | | NM_058243 | | | |
| TOX | 204529_s_at | AI961231 | NM_014729 | 9760 | 0.88 | 4.56E-03 |
| Al032121 | 227422_at | AI032121 | --- | --- | 0.88 | 5.07E-04 |
| RAB3IP | 223471_at | BC002556 | NM_022456 | 117177 | 0.87 | 1.10E-03 |
| | | | NM_175623 | | | |
| | | | NM_175624 | | | |
| | | | NM_175625 | | | |
| | | | NM_175626 | | | |
| | | | NM_175627 | | | |
| DDT | 202929_s_at | NM_001355 | NM_001355 | 1652 | 0.87 | 4.60E-04 |
| C10orf77 | 219745_at | NM_024789 | NM_024789 | 79847 | 0.87 | 9.14E-03 |
| NUCKS | 224582_s_at | H09085 | NM_022731 | 64710 | 0.87 | 2.99E-05 |
| FBN1 | 202766_s_at | NM_000138 | NM_000138 | 2200 | 0.87 | 9.90E-03 |
| AA287457 | 226943_at | AA287457 | --- | --- | 0.87 | 2.30E-03 |
| C10orf86 | 219067_s_at | NM_017615 | NM_017615 | 54780 | 0.87 | 6.15E-05 |
| MAGE:3604678 | 1555875_at | BU733713 | --- | --- | 0.87 | 7.23E-03 |
| HSPA4L | 205543_at | NM_014278 | NM_014278 | 22824 | 0.87 | 7.62E-04 |
| PLS1 | 205190_at | NM_002670 | NM_002670 | 5357 | 0.87 | 7.93E-03 |
| AW299815 | 241815_at | AW299815 | --- | --- | 0.87 | 5.59E-03 |
| MRPL42 | 222466_s_at | AL136659 | NM_014050 | 28977 | 0.87 | 3.61E-06 |
| | | | NM_172177 | | | |
| | | | NM_172178 | | | |
| LYK5 | 221554_at | AF308302 | NM_00100378 6 | 92335 | 0.87 | 5.17E-03 |
| | | | NM_00100378 7 | | | |
| | | | NM_00100378 8 | | | |
| | | | NM_153335 | | | |
| C6orf61 | 219673_at | NM_017696 | --- | 54844 | 0.87 | 2.34E-04 |
| NR3C1 | 201865_x_at | AI432196 | NM_000176 | 2908 | 0.87 | 1.11E-03 |
| KIAA1280 | 219520_s_at | NM_018458 | NM_015691 | 55841 | 0.87 | 6.75E-05 |
| HMG20B | 209113_s_at | AF288679 | NM_006339 | 10362 | 0.87 | 8.93E-05 |
| KIAA1211 | 227230_s_at | BE855799 | XM_044178 | 57482 | 0.87 | 4.76E-04 |
| GAS6 | 202177_at | NM_000820 | NM_000820 | 2621 | 0.87 | 1.87E-03 |
| AKAP13 | 221718_s_at | M90360 | NM_006738 | 11214 | 0.87 | 4.55E-04 |
| | | | NM_007200 | | | |
| | | | NM_144767 | | | |
| H2AFZ | 213911_s_at | BF718636 | NM_002106 | 3015 | 0.87 | 6.81E-06 |
| NYREN18 | 1569030_s_at | BC034716 | NM_016118 | 51667 | 0.87 | 1.77E-03 |
| C20orf20 | 218586_at | NM_018270 | NM_018270 | 55257 | 0.86 | 1.58E-04 |
| PAICS | 201013_s_at | AA902652 | NM_006452 | 10606 | 0.86 | 5.07E-04 |
| HPS4 | 218402_s_at | NM_022081 | NM_022081 | 89781 | 0.86 | 9.72E-04 |
| | | | NM_152840 | | | |
| | | | NM_152841 | | | |
| | | | NM_152842 | | | |
| | | | NM_152843 | | | |
| GSS | 211630_s_at | L42531 | NM_000178 | 2937 | 0.86 | 3.22E-04 |
| AL136980 | 222603_at | AL136980 | --- | --- | 0.86 | 1.97E-03 |
| septin 11 | 201307_at | AL534972 | NM_018243 | 55752 | 0.86 | 6.58E-05 |
| PDLIM7 | 214266_s_at | AW206786 | NM_005451 | 9260 | 0.86 | 8.67E-04 |
| | | | NM_203352 | | | |
| | | | NM_203353 | | | |
| | | | NM_213636 | | | |
| HIP1 | 205426_s_at | U79734 | NM_005338 | 3092 | 0.86 | 1.59E-03 |
| BF447112 | 236192_at | BF447112 | --- | --- | 0.86 | 6.78E-04 |
| AW161909 | 232697_at | AW161909 | --- | --- | 0.86 | 1.71E-04 |
| SEC61A2 | 228747_at | AI694646 | NM_018144 | 55176 | 0.86 | 3.38E-04 |
| PXMP2 | 219076_s_at | NM_018663 | NM_018663 | 5827 | 0.86 | 2.72E-03 |
| AU145336 | 233446_at | AU145336 | --- | --- | 0.86 | 2.61E-03 |
| SIVA | 210792_x_at | AF033111 | NM_006427 | 10572 | 0.86 | 5.84E-04 |
| | | | NM_021709 | | | |
| NDUFA6 | 202000_at | BC002772 | NM_002490 | 4700 | 0.86 | 2.77E-03 |
| HMGB1 | 200680_x_at | NM_002128 | NM_002128 | 3146 | 0.85 | 7.80E-05 |
| septin 8 | 209000_s_at | BC001329 | XM_034872 | 23176 | 0.85 | 1.12E-03 |
| EIF2B3 | 218488_at | NM_020365 | NM_020365 | 8891 | 0.85 | 2.51E-06 |
| DCX | 204851_s_at | AF040254 | NM_000555 | 1641 | 0.85 | 1.15E-03 |
| | | | NM_178151 | | | |
| | | | NM_178152 | | | |
| | | | NM_178153 | | | |
| TBC1D16 | 1554701_a_at | BC028290 | NM_019020 | 125058 | 0.85 | 2.39E-03 |
| CARD10 | 210026_s_at | AY028896 | NM_014550 | 29775 | 0.85 | 2.51E-03 |
| VAV2 | 205536_at | NM_003371 | NM_003371 | 7410 | 0.85 | 2.59E-03 |
| NES | 218678_at | NM_024609 | NM_006617 | 10763 | 0.85 | 8.46E-03 |
| DRD2 | 216938_x_at | S69899 | NM_000795 | 1813 | 0.85 | 7.98E-03 |
| | | | NM_016574 | | | |
| PARP2 | 214086_s_at | AK001980 | NM_005484 | 10038 | 0.85 | 2.66E-03 |
| MGC13204 | 225837_at | AL577977 | NM_031465 | 83695 | 0.85 | 1.56E-03 |
| SLC29A1 | 201802_at | NM_004955 | NM_004955 | 2030 | 0.85 | 4.79E-03 |
| DONSON | 221677_s_at | AF232674 | NM_017613 | 29980 | 0.85 | 3.72E-03 |
| | | | NM_145794 | | | |
| | | | NM_145795 | | | |
| SLC37A4 | 217289_s_at | AF097831 | NM_001467 | 2542 | 0.85 | 2.49E-03 |
| HAND1 | 220138_at | NM_004821 | NM_004821 | 9421 | 0.85 | 7.06E-03 |
| ZGPAT | 57539_at | AA535065 | NM_032527 | 84619 | 0.85 | 3.82E-04 |
| | | | NM_181484 | | | |
| | | | NM_181485 | | | |
| CGI-96 | 202937_x_at | AL022316 | NM_015703 | 27341 | 0.85 | 4.33E-04 |
| NUP107 | 218768_at | NM_020401 | NM_020401 | 57122 | 0.84 | 7.62E-05 |
| AL133101 | 228007_at | AL133101 | --- | --- | 0.84 | 1.62E-04 |
| CALM3 | 200622_x_at | AV685208 | NM_005184 | 808 | 0.84 | 4.45E-03 |
| AW450360 | 222072_at | AW450360 | --- | --- | 0.84 | 4.39E-03 |
| BTBD3 | 202946_s_at | NM_014962 | NM_014962 NM_181443 | 22903 | 0.84 | 7.63E-04 |
| RBBP8 | 203344_s_at | NM_002894 | NM_002894 | 5932 | 0.84 | 2.62E-04 |
| | | | NM_203291 | | | |
| | | | NM_203292 | | | |
| FH | 203033_x_at | NM_000143 | NM_000143 | 2271 | 0.84 | 2.23E-06 |
| VAV2 | 226063_at | AA481141 | NM_003371 | 7410 | 0.84 | 1.78E-04 |
| MRPL49 | 201717_at | NM_004927 | NM_004927 | 740 | 0.84 | 3.26E-03 |
| TUBA3 | 209118_s_at | AF141347 | NM_006009 | 7846 | 0.84 | 7.92E-05 |
| CD24 | 216379_x_at | AK000168 | NM_013230 | 934 | 0.84 | 4.53E-05 |
| FLJ21415 | 218867_s_at | NM_024738 | NM_024738 | 79794 | 0.84 | 3.59E-03 |
| AA004210 | 229835_s_at | AA004210 | --- | --- | 0.84 | 1.26E-04 |
| PIGX | 1552291_at | NM_017861 | NM_017861 | 54965 | 0.84 | 2.94E-03 |
| L3MBTL2 | 1555815_a_at | AL136564 | NM_00100368 9 | 83746 | 0.84 | 6.19E-03 |
| | | | NM_031488 | | | |
| SEC23A | 212887_at | AI753659 | NM_006364 | 10484 | 0.84 | 2.12E-03 |
| AI589594 | 238694_at | AI589594 | --- | --- | 0.84 | 6.20E-04 |
| C20orf45 | 222441_x_at | BF032213 | NM_016045 | 51012 | 0.84 | 3.50E-03 |
| C8orf20 | 218777_at | NM_025232 | NM_025232 | 80346 | 0.84 | 8.62E-04 |
| PREX1 | 224925_at | AL445192 | NM_020820 | 57580 | 0.84 | 4.97E-03 |
| RPA2 | 201756_at | NM_002946 | NM_002946 | 6118 | 0.84 | 2.53E-03 |
| RECQL | 212918_at | AI962943 | NM_002907 | 5965 | 0.83 | 5.17E-03 |
| | | | NM_032941 | | | |
| HOXD4 | 205522_at | NM_014621 | NM_014621 | 3233 | 0.83 | 4.47E-03 |
| AU144918 | 233858_at | AU144918 | --- | --- | 0.83 | 9.47E-03 |
| DATF1 | 218325_s_at | NM_022105 | NM_022105 | 11083 | 0.83 | 3.04E-03 |
| | | | NM_080796 | | | |
| | | | NM_080797 | | | |
| EED | 209572_s_at | AF080227 | NM_003797 | 8726 | 0.83 | 6.60E-06 |
| | | | NM_152991 | | | |
| RIMS4 | 227644_at | AL049000 | NM_182970 | 140730 | 0.83 | 1.04E-03 |
| CHML | 226350_at | AU155565 | NM_001821 | 1122 | 0.83 | 1.58E-03 |
| RAD21 | 200607_s_at | BG289967 | NM_006265 | 5885 | 0.83 | 1.96E-03 |
| ZNF555 | 1556188_a_at | AF052118 | NM_152791 | 148254 | 0.83 | 3.68E-04 |
| FSCN1 | 210933_s_at | BC004908 | NM_003088 | 6624 | 0.83 | 1.24E-03 |
| KIAA1467 | 213234_at | AB040900 | XM_370682 | 57613 | 0.83 | 4.71E-03 |
| DDX54 | 224433_s_at | BC005848 | NM_024072 | 79039 | 0.83 | 4.90E-03 |
| TERT | 207199_at | NM_003219 | NM_003219 | 7015 | 0.83 | 3.88E-03 |
| | | | NM_198253 | | | |
| | | | NM_198254 | | | |
| | | | NM_198255 | | | |
| HRASLS | 219983_at | NM_020386 | NM_020386 | 57110 | 0.83 | 7.17E-03 |
| BACH | 215728_s_at | AL031848 | NM_007274 | 11332 | 0.83 | 3.84E-06 |
| | | | NM_181862 | | | |
| | | | NM_181863 | | | |
| | | | NM_181864 | | | |
| | | | NM_181865 | | | |
| | | | NM_181866 | | | |
| MSH5 | 221406_s_at | NM_025259 | NM_002441 | 4439 | 0.83 | 9.50E-03 |
| | | | NM_025259 | | | |
| | | | NM_172165 | | | |
| | | | NM_172166 | | | |
| FLJ22955 | 221224_s_at | NM_024819 | NM_024819 | 79877 | 0.83 | 2.19E-03 |
| ATF7IP | 218987_at | NM_018179 | NM_018179 | 55729 | 0.83 | 8.71E-04 |
| FLJ20508 | 218712_at | NM_017850 | NM_017850 | 54955 | 0.82 | 1.46E-03 |
| PHF5A | 225309_at | AL008582 | NM_032758 | 84844 | 0.82 | 1.72E-04 |
| C20orf27 | 50314_i_at | AI761506 | NM_017874 | 54976 | 0.82 | 7.41E-04 |
| SET8 | 225094_at | AL578116 | NM_020382 | 387893 | 0.82 | 8.35E-04 |
| KCNS3 | 205968_at | NM_002252 | NM_002252 | 3790 | 0.82 | 9.42E-03 |
| CHES1 | 222494_at | AW051527 | NM_005197 | 1112 | 0.82 | 1.04E-02 |
| RCBTB1 | 218352_at | NM_018191 | NM_018191 | 55213 | 0.82 | 8.95E-05 |
| NM_024601 | 220033_at | NM_024601 | --- | --- | 0.82 | 1.30E-03 |
| HCN3 | 228741_s_at | AA569959 | NM_020897 | 57657 | 0.82 | 2.94E-03 |
| ATP6V1A | 201971_s_at | NM_001690 | NM_001690 | 523 | 0.82 | 1.36E-03 |
| RNF26 | 224338_s_at | AB055622 | NM_032015 | 79102 | 0.82 | 2.45E-03 |
| BC042916 | 1559111_a_at | BC042916 | --- | --- | 0.82 | 8.94E-03 |
| PINX1 | 223907 _s_at | AF205718 | NM_017884 | 54984 | 0.82 | 1.62E-04 |
| AW771952 | 242434_at | AW771952 | --- | --- | 0.82 | 2.95E-03 |
| HNRPAB | 201277_s_at | NM_004499 | NM_004499 | 3182 | 0.82 | 1.19E-04 |
| | | | NM_031266 | | | |
| FH | 214170_x_at | AA669797 | NM_000143 | 2271 | 0.82 | 3.99E-05 |
| LOC283852 | 238554_at | BE501733 | --- | 283852 | 0.82 | 5.07E-03 |
| ZNF79 | 214138_at | AA284829 | NM_007135 | 7633 | 0.82 | 2.28E-04 |
| C22orf19 | 209418_s_at | BC003615 | NM_00100287 7 | 8563 | 0.82 | 2.69E-04 |
| | | | NM_00100287 8 | | | |
| | | | NM_00100287 9 | | | |
| | | | NM_003678 | | | |
| RAD21 | 200608_s_at | NM_006265 | NM_006265 | 5885 | 0.82 | 1.40E-03 |
| H1F0 | 208886_at | BC000145 | NM_005318 | 3005 | 0.82 | 3.44E-03 |
| MAPKAPK5 | 212871_at | NM_003668 | NM_003668 | 8550 | 0.82 | 5.71E-04 |
| | | | NM_139078 | | | |
| PPP1R13B | 216347_s_at | AK023188 | NM_015316 | 23368 | 0.82 | 4.23E-04 |
| ZNF278 | 211392_s_at | AF242522 | NM_014323 | 23598 | 0.81 | 1.59E-03 |
| | | | NM_032050 | | | |
| | | | NM_032051 | | | |
| | | | NM_032052 | | | |
| ENC2 | 210307_s_at | AL136796 | NM_022480 | 64410 | 0.81 | 6.42E-03 |
| DACH1 | 1562342_at | AL713717 | NM_004392 | 1602 | 0.81 | 4.53E-03 |
| | | | NM_080759 | | | |
| | | | NM_080760 | | | |
| ADRBK2 | 204184_s_at | NM_005160 | NM_005160 | 157 | 0.81 | 3.83E-04 |
| JUP | 201015_s_at | NM_021991 | NM_002230 | 3728 | 0.81 | 5.55E-03 |
| | | | NM_021991 | | | |
| KIAA1333 | 223258_s_at | BC000973 | NM_017769 | 55632 | 0.81 | 2.69E-03 |
| ETAA16 | 219216_at | NM_019002 | NM_019002 | 54465 | 0.81 | 1.33E-03 |
| LOC387882 | 225105_at | BF969397 | NM_207376 | 387882 | 0.81 | 9.88E-04 |
| KIAA1648 | 213058_at | AL033538 | --- | 284900 | 0.81 | 3.45E-03 |
| DYRK4 | 212954_at | AF263541 | NM_003845 | 8798 | 0.81 | 3.72E-03 |
| LOC200008 | 229605_at | AI150848 | NM_201546 | 200008 | 0.81 | 6.35E-06 |
| GPX3 | 214091_s_at | AW149846 | NM_002084 | 2878 | 0.81 | 1.38E-03 |
| LRP10 | 201412_at | NM_014045 | NM_014045 | 26020 | 0.81 | 4.50E-03 |
| LOC55954 | 218752_at | NM_019103 | NM_00100369 2 | 55954 | 0.81 | 9.27E-03 |
| | | | NM_019103 | | | |
| FLJ23441 | 219217_at | NM_024678 | NM_024678 | 79731 | 0.81 | 5.14E-06 |
| TH1L | 220607_x_at | NM_016397 | NM_016397 | 51497 | 0.81 | 1.63E-03 |
| | | | NM_198976 | | | |
| AD-003 | 223368_s_at | BC001396 | NM_014064 | 28989 | 0.81 | 2.11E-03 |
| HIP2 | 202347_s_at | AB022435 | NM_005339 | 3093 | 0.80 | 1.48E-04 |
| MGAT5B | 238445_x_at | AI609043 | NM_144677 | 146664 | 0.80 | 6.85E-04 |
| | | | NM_198955 | | | |
| EPOR | 37986_at | M60459 | NM_000121 | 2057 | 0.80 | 5.63E-03 |
| HIC2 | 212966_at | AL043112 | NM_015094 | 23119 | 0.80 | 3.15E-05 |
| GJA7 | 228776_at | AA430014 | NM_005497 | 10052 | 0.80 | 4.03E-05 |
| DNAJC9 | 213088_s_at | BE551340 | NM_015190 | 23234 | 0.80 | 2.60E-03 |
| HMGN2 | 208668_x_at | BC003689 | NM_005517 | 3151 | 0.80 | 1.11E-03 |
| MRPL42 | 217919_s_at | BE782148 | NM_014050 | 28977 | 0.80 | 2.11E-03 |
| | | | NM_172177 | | | |
| | | | NM_172178 | | | |
| HSPC129 | 223270_at | AF161543 | NM_016396 | 51496 | 0.80 | 1.61E-05 |
| HNRPA2B1 | 205292_s_at | NM_002137 | NM_002137 | 3181 | 0.80 | 3.05E-04 |
| | | | NM_031243 | | | |
| ANXA4 | 201301_s_at | BC000182 | NM_001153 | 307 | 0.80 | 8.27E-03 |
| SOX13 | 38918_at | AF083105 | NM_005686 | 9580 | 0.80 | 3.55E-03 |
| ACTR1A | 200721_s_at | NM_005736 | NM_005736 | 10121 | 0.80 | 2.85E-03 |
| MEIS2 | 207480_s_at | NM_020149 | NM_002399 | 4212 | 0.80 | 4.47E-04 |
| | | | NM_020149 | | | |
| | | | NM_170674 | | | |
| | | | NM_170675 | | | |
| | | | NM_170676 | | | |
| | | | NM_170677 | | | |
| | | | NM_172315 | | | |
| | | | NM_172316 | | | |
| APG3L | 221492_s_at | AF202092 | NM_022488 | 64422 | 0.80 | 1.43E-03 |
| CDYL | 203099_s_at | AF081258 | NM_004824 | 9425 | 0.80 | 3.28E-03 |
| | | | NM_170751 | | | |
| | | | NM_170752 | | | |
| ODZ3 | 219523_s_at | NM_018104 | XM_371717 | 55714 | 0.80 | 1.87E-03 |
| VAPB | 202550_s_at | NM_004738 | NM_004738 | 9217 | 0.80 | 8.33E-06 |
| SAP30 | 204900_x_at | NM_003864 | NM_003864 | 8819 | 0.80 | 1.56E-05 |
| HOXD1 | 205975_s_at | NM_024501 | NM_024501 | 3231 | 0.80 | 1.08E-03 |
| FLJ10385 | 220258_s_at | NM_018081 | NM_018081 | 55135 | 0.80 | 1.71E-03 |
| LRRC1 | 218816_at | NM_018214 | NM_018214 | 55227 | 0.80 | 5.23E-03 |
| | | | NM_025168 | | | |
| CD24 | 209771_x_at | AA761181 | NM_013230 | 934 | 0.80 | 5.76E-04 |
| SLC29A1 | 201801_s_at | AF079117 | NM_004955 | 2030 | 0.80 | 7.75E-04 |
| C10orf86 | 211376_s_at | BC005212 | NM_017615 | 54780 | 0.80 | 2.32E-05 |
| GNAZ | 204993_at | NM_002073 | NM_002073 | 2781 | 0.80 | 8.65E-05 |
| C15orf25 | 229208_at | AK022939 | NM_018097 | 55142 | 0.80 | 8.67E-03 |
| CPT1A | 203633_at | BF001714 | NM_001876 | 1374 | 0.80 | 1.57E-03 |
| LOC150383 | 227590_at | BE501980 | NM_00100891 7 | 150383 | 0.80 | 1.76E-05 |
| | | | NM_207327 | | | |
| MGC18216 | 225330_at | AL044092 | --- | 145815 | 0.80 | 7.91E-04 |
| 384D8-2 | 205086_s_at | NM_014551 | NM_014551 | 29781 | 0.80 | 8.25E-05 |
| | | | NM_152299 | | | |
| USP28 | 231837_at | AB040948 | NM_020886 | 57646 | 0.80 | 4.10E-04 |
| G22P1 | 200792_at | NM_001469 | NM_001469 | 2547 | 0.79 | 1.57E-06 |
| TIMM50 | 224913_s_at | AA877820 | NM_001001563 | 92609 | 0.79 | 1.54E-06 |
| OSRF | 219421_at | NM_012382 | NM_012382 | 23548 | 0.79 | 1.38E-03 |
| TIAF1 | 202039_at | NM_004740 | NM_004740 | 399687 | 0.79 | 7.17E-03 |
| MYO18A | | | NM_078471 | 9220 | | |
| | | | NM_203318 | | | |
| SNN | 218032_at | AF070673 | NM_003498 | 8303 | 0.79 | 3.27E-05 |
| HPS4 | 54037_at | AL041451 | NM_022081 | 89781 | 0.79 | 3.31E-05 |
| | | | NM_152840 | | | |
| | | | NM_152841 | | | |
| | | | NM_152842 | | | |
| | | | NM_152843 | | | |
| AI339606 | 222852_at | AI339606 | --- | --- | 0.79 | 3.52E-03 |
| CNN3 | 201445_at | NM_001839 | NM_001839 | 1266 | 0.79 | 2.38E-05 |
| C16orf34 | 212115_at | AK023154 | NM_144570 | 90861 | 0.79 | 1.93E-04 |
| OKFZP564K082 | 208091_s_at | NM_030796 | NM_030796 | 81552 | 0.79 | 4.71E-04 |
| LETM2 | 1557415_s_at | AI023774 | NM_144652 | 137994 | 0.79 | 1.29E-03 |
| AW665227 | 229713_at | AW665227 | --- | --- | 0.79 | 2.47E-05 |
| C14orf130 | 218108_at | NM_018108 | NM_018108 | 55148 | 0.79 | 4.99E-03 |
| | | | NM_175748 | | | |
| NEDD1 | 234984_at | AA236927 | NM_152905 | 121441 | 0.79 | 3.11E-04 |
| LOC57168 | 227015_at | Z99714 | NM_020437 | 57168 | 0.79 | 4.74E-06 |
| ZNF267 | 219540_at | AU150728 | NM_003414 | 10308 | 0.79 | 1.98E-03 |
| HIC2 | 212964_at | AI912206 | NM_015094 | 23119 | 0.79 | 6.64E-06 |
| BG150301 | 230271_at | BG150301 | --- | --- | 0.79 | 9.81E-03 |
| NM_014838 | 204799_at | NM_014838 | --- | --- | 0.79 | 1.18E-03 |
| FLJ14827 | 221777_at | BE966197 | NM_032848 | 84934 | 0.79 | 2.97E-05 |
| NP | 201695_s_at | NM_000270 | NM_000270 | 4860 | 0.78 | 8.55E-03 |
| BACH | 208002_s_at | NM_007274 | NM_007274 | 11332 | 0.78 | 1.62E-04 |
| | | | NM_181862 | | | |
| | | | NM_181863 | | | |
| | | | NM_181864 | | | |
| | | | NM_181865 | | | |
| | | | NM_181866 | | | |
| GPR155 | 244509_at | AW449728 | NM_152529 | 151556 | 0.78 | 8.05E-03 |
| EIF2S1 | 201144_s_at | NM_004094 | NM_004094 | 1965 | 0.78 | 4.48E-06 |
| CLEC11A | 205131_x_at | NM_002975 | NM_002975 | 6320 | 0.78 | 3.57E-03 |
| BCL11A | 219497_s_at | NM_022893 | NM_018014 | 53335 | 0.78 | 8.37E-05 |
| | | | NM_022893 | | | |
| | | | NM_138553 | | | |
| | | | NM_138559 | | | |
| MRPL2 | 229884_s_at | AA187325 | NM_015950 | 51069 | 0.78 | 6.69E-04 |
| PRKAB2 | 1558027_s_at | AL552001 | NM_005399 | 5565 | 0.78 | 1.67E-03 |
| DTX1 | 227336_at | AW576405 | NM_004416 | 1840 | 0.78 | 1.19E-03 |
| M96 | 203347_s_at | NM_007358 | NM_007358 | 22823 | 0.78 | 8.86E-03 |
| ACN9 | 218981_at | NM_020186 | NM_020186 | 57001 | 0.78 | 1.05E-03 |
| IMP-1 | 227377_at | AK022784 | NM_006546 | 10642 | 0.78 | 2.10E-04 |
| DVL2 | 57532_at | AW016304 | NM_004422 | 1856 | 0.78 | 1.49E-04 |
| DKFZp547A023 | 239525_at | AI733041 | NM_018704 | 55917 | 0.78 | 2.26E-04 |
| LOC91431 | 1565935_at | AF075091 | NM_138698 | 91431 | 0.78 | 1.72E-03 |
| H1FX | 204805_s_at | NM_006026 | NM_006026 | 8971 | 0.78 | 2.36E-03 |
| RNPEP | 208270_s_at | NM_020216 | NM_020216 | 6051 | 0.78 | 1.53E-03 |
| LOC400960 | 227969_at | AI627636 | XM_379102 | 400960 | 0.78 | 1.32E-04 |
| PXMP4 | 224210_s_at | BC001147 | NM_007238 | 11264 | 0.78 | 7.76E-03 |
| | | | NM_183397 | | | |
| MAP3K7IP1 | 203901_at | NM_006116 | NM_006116 | 10454 | 0.78 | 2.33E-04 |
| | | | NM_153497 | | | |
| LSM2 | 209449_at | AF196468 | NM_021177 | 57819 | 0.78 | 2.81E-03 |
| ODF2 | 225617_at | AL138382 | NM_002540 | 4957 | 0.78 | 1.50E-03 |
| | | | NM_153437 | | | |
| JARID2 | 203297_s_at | BG029530 | NM_004973 | 3720 | 0.77 | 5.30E-03 |
| C20orf27 | 218081_at | NM_017874 | NM_017874 | 54976 | 0.77 | 1.93E-04 |
| FLJ35779 | 227267_at | AI953478 | NM_152408 | 134359 | 0.77 | 5.87E-03 |
| FLJ38973 | 238974_at | N47077 | NM_153689 | 205327 | 0.77 | 3.60E-03 |
| LOC202181 | 232309_at | Y13871 | --- | 202181 | 0.77 | 9.94E-04 |
| DDX54 | 219111_s_at | NM_024072 | NM_024072 | 79039 | 0.77 | 5.24E-03 |
| NELL2 | 203413_at | NM_006159 | NM_006159 | 4753 | 0.77 | 1.82E-04 |
| AZI1 | 214742_at | AB029041 | NM_00100981 1 | 22994 | 0.77 | 4.54E-04 |
| | | | NM_014984 | | | |
| C13orf6 | 235348_at | AA404347 | NM_032859 | 84945 | 0.77 | 6.35E-07 |
| MKL1 | 212748_at | AB037859 | NM_020831 | 57591 | 0.77 | 1.75E-04 |
| MLF1 | 204784_s_at | NM_022443 | NM_022443 | 4291 | 0.77 | 4.84E-03 |
| RPP25 | 219143_s_at | NM_017793 | NM_017793 | 54913 | 0.77 | 2.33E-03 |
| MRPL39 | 218558_s_at | NM_017446 | NM_017446 | 54148 | 0.77 | 1.79E-03 |
| | | | NM_080794 | | | |
| MR-1 | 225298_at | AA074597 | NM_015488 | 25953 | 0.77 | 9.47E-03 |
| | | | NM_022572 | | | |
| SCOC | 223341_s_at | AF330205 | NM_032547 | 60592 | 0.77 | 5.34E-06 |
| GAMT | 1552474_a_at | NM_138924 | NM_000156 | 2593 | 0.77 | 8.68E-04 |
| | | | NM_138924 | | | |
| MGC61550 | 228791_at | BF434655 | NM_182616 | 348110 | 0.77 | 5.38E-04 |
| MGC4172 | 218756_s_at | NM_024308 | NM_024308 | 79154 | 0.76 | 8.18E-04 |
| IKIP | 227295_at | AW182575 | NM_153687 | 121457 | 0.76 | 1.09E-04 |
| | | | NM_201612 | | | |
| | | | NM_201613 | | | |
| | | | NM_201614 | | | |
| BCL11A | 219498_s_at | NM_018014 | NM_018014 | 53335 | 0.76 | 6.17E-05 |
| | | | NM_022893 | | | |
| | | | NM_138553 | | | |
| | | | NM_138559 | | | |
| LOC115098 | 225454_at | AW248770 | NM_138442 | 115098 | 0.76 | 2.43E-03 |
| NM_016534 | 219817_at | NM_016534 | --- | --- | 0.76 | 1.74E-03 |
| H15087 | 238186_at | H15087 | --- | --- | 0.76 | 4.91E-03 |
| D15Wsu75e | 212527_at | BF057059 | XM_039495 | 27351 | 0.76 | 5.47E-03 |
| TMEM19 | 229126_at | AI597651 | NM_018279 | 55266 | 0.76 | 9.91E-03 |
| TCF3 | 215260_s_at | X52078 | NM_003200 | 6929 | 0.76 | 3.41E-03 |
| PEX19 | 201706_s_at | BC000496 | NM_002857 | 5824 | 0.76 | 6.90E-07 |
| HSPC132 | 218403_at | NM_016399 | NM_016399 | 51499 | 0.76 | 1.21E-03 |
| TMEM33 | 222642_s_at | BC000948 | NM_018126 | 55161 | 0.76 | 1.16E-03 |
| LOC58489 | 225436_at | AI339710 | XM_051862 | 58489 | 0.76 | 6.79E-04 |
| RAB3D | 225001_at | AI744658 | NM_004283 | 9545 | 0.76 | 1.35E-04 |
| CBWD1 | 226193_x_at | AV709992 | NM_018491 | 150472 | 0.76 | 7.42E-06 |
| CBWD2 | | | NM_172003 | 220869 | | |
| LOC220869 | | | NM_201453 | 389760 | | |
| DC36 | | | | 55871 | | |
| C7orf31 | 229146_at | AA778688 | NM_138811 | 136895 | 0.76 | 4.75E-04 |
| ZNF569 | 1569366_a_at | BC038737 | NM_152484 | 148266 | 0.76 | 2.84E-03 |
| TOB2 | 222243_s_at | AB051450 | NM_016272 | 10766 | 0.76 | 1.66E-04 |
| BF978778 | 226832_at | BF978778 | XM_374070 | 389188 | 0.75 | 2.91E-03 |
| SULF2 | 233555_s_at | AL034418 | NM_018837 | 55959 | 0.75 | 2.13E-03 |
| | | | NM_198596 | | | |
| D50683 | 208944_at | D50683 | --- | --- | 0.75 | 8.75E-04 |
| TMEFF1 | 205123_s_at | NM_003692 | NM_003692 | 8577 | 0.75 | 5.76E-04 |
| CSNK2A1 | 206075_s_at | NM_001895 | NM_001895 | 1457 | 0.75 | 8.62E-03 |
| | | | NM_177559 | | | |
| | | | NM_177560 | | | |
| MYO1B | 212364_at | BF432550 | NM_012223 | 4430 | 0.75 | 1.60E-05 |
| TBL1XR1 | 222634_s_at | AF314544 | NM_024665 | 79718 | 0.75 | 8.23E-03 |
| C20orf6 | 222765_x_at | AL161659 | NM_016649 | 51575 | 0.75 | 2.08E-03 |
| TRA16 | 226839_at | N25631 | NM_176880 | 126382 | 0.75 | 4.39E-03 |
| TH1L | 225006_x_at | AJ238379 | NM_016397 | 51497 | 0.75 | 3.15E-03 |
| | | | NM_198976 | | | |
| LANCL1 | 202019_s_at | AI935255 | NM_006055 | 10314 | 0.75 | 3.29E-04 |
| ZFYVE21 | 219929_s_at | NM_024071 | NM_024071 | 79038 | 0.75 | 5.24E-04 |
| BF697865 | 235018_at | BF697865 | --- | --- | 0.75 | 3.31E-05 |
| RECQL | 212917_x_at | AI814728 | NM_002907 | 5965 | 0.75 | 1.03E-03 |
| | | | NM_032941 | | | |
| GPR158 | 232195_at | R41459 | NM_020752 | 57512 | 0.75 | 9.00E-03 |
| TH1L | 225865_x_at | AJ238374 | NM_016397 | 51497 | 0.75 | 5.42E-03 |
| | | | NM_198976 | | | |
| AW025579 | 213429_at | AW025579 | --- | --- | 0.75 | 2.61E-03 |
| NDUFA3 | 218563_at | NM_004542 | NM_004542 | 4696 | 0.75 | 1.05E-03 |
| AI857788 | 227126_at | AI857788 | --- | --- | 0.75 | 3.17E-04 |
| TRIM32 | 203846_at | BC003154 | NM_012210 | 22954 | 0.75 | 9.18E-04 |
| LZIC | 226087_at | BG180887 | NM_032368 | 84328 | 0.75 | 5.95E-03 |
| CBX1 | 201518_at | NM_006807 | NM_006807 | 10951 | 0.75 | 3.35E-03 |
| TUB | 228882_at | AL042088 | NM_003320 | 7275 | 0.75 | 1.66E-03 |
| | | | NM_177972 | | | |
| EPOR | 215054_at | H16758 | NM_000121 | 2057 | 0.74 | 2.51E-03 |
| PDXP | 223290_at | BC000320 | NM_020315 | 57026 | 0.74 | 3.77E-04 |
| GSS | 201415_at | NM_000178 | NM_000178 | 2937 | 0.74 | 8.16E-04 |
| SEC10L1 | 228418_at | BF509391 | NM_006544 | 10640 | 0.74 | 1.01E-02 |
| DNAJC9 | 213092_x_at | AW241779 | NM_015190 | 23234 | 0.74 | 4.38E-03 |
| SULF2 | 224724_at | AL133001 | NM_018837 | 55959 | 0.74 | 7.10E-04 |
| | | | NM_198596 | | | |
| KIAA0082 | 212380_at | D43949 | NM_015050 | 23070 | 0.74 | 9.43E-03 |
| NM_003349 | 201003_x_at | NM_003349 | --- | --- | 0.74 | 2.17E-03 |
| HH114 | 232506_s_at | AK026504 | NM_032499 | 84529 | 0.74 | 2.32E-03 |
| MAP3K13 | 211083_s_at | Z25428 | NM_004721 | 9175 | 0.74 | 1.51E-03 |
| SRR | 222844_s_at | AF169974 | NM_021947 | 63826 | 0.74 | 1.66E-03 |
| SIP1 | 205063_at | NM_003616 | NM_00100918 2 | 8487 | 0.74 | 5.42E-03 |
| | | | NM_00100918 3 | | | |
| | | | NM_003616 | | | |
| SMPD3 | 219695_at | NM_024703 | NM_018667 | 55512 | 0.74 | 8.85E-03 |
| ARL6IP6 | 225707_at | AL581082 | NM_152522 | 151188 | 0.74 | 3.27E-03 |
| LOC90525 | 228922_at | AI760446 | NM_138356 | 90525 | 0.74 | 8.81E-03 |
| PARP2 | 204752_x_at | NM_005484 | NM_005484 | 10038 | 0.74 | 8.00E-03 |
| EPB41L5 | 225855_at | AB046768 | NM_020909 | 57669 | 0.74 | 2.98E-04 |
| BG492376 | 1557961_s_at | BG492376 | --- | --- | 0.74 | 8.26E-03 |
| KLF3 | 225140_at | BF438116 | NM_016531 | 51274 | 0.74 | 1.89E-03 |
| R12027 | 241836_x_at | R12027 | --- | --- | 0.74 | 1.13E-03 |
| MCM8 | 233560_x_at | AA370141 | NM_032485 | 84515 | 0.74 | 7.31E-03 |
| | | | NM_182802 | | | |
| TFR2 | 210215_at | AF067864 | NM_003227 | 7036 | 0.74 | 3.11E-04 |
| EIF4E | 201435_s_at | AW268640 | NM_001968 | 1977 | 0.74 | 4.34E-05 |
| AK001164 | 232752_at | AK001164 | --- | --- | 0.74 | 3.62E-03 |
| FLJ21839 | 218480_at | NM_021831 | NM_021831 | 60509 | 0.73 | 2.20E-04 |
| DUT | 209932_s_at | U90223 | NM_001948 | 1854 | 0.73 | 1.26E-03 |
| RBBP7 | 201092_at | NM_002893 | NM_002893 | 5931 | 0.73 | 2.45E-03 |
| SCOC | 224786_at | AL133580 | NM_032547 | 60592 | 0.73 | 1.16E-06 |
| ELF2 | 210361_s_at | AF256223 | NM_006874 | 1998 | 0.73 | 1.61E-04 |
| | | | NM_201999 | | | |
| BF315093 | 227617_at | BF315093 | NM_001010866 | 199953 | 0.73 | 3.23E-03 |
| C6orf125 | 224448_s_at | BC006007 | NM_032340 | 84300 | 0.73 | 1.18E-03 |
| AK025512 | 225514_at | AK025512 | --- | --- | 0.73 | 2.88E-03 |
| TTLL4 | 203702_s_at | AL043927 | NM_014640 | 9654 | 0.73 | 1.86E-03 |
| PHOX2B | 207009_at | NM_003924 | NM_003924 | 8929 | 0.73 | 6.98E-04 |
| PAK4 | 33814_at | AF005046 | NM_005884 | 10298 | 0.73 | 6.87E-03 |
| TTC8 | 226120_at | AW293939 | NM_144596 | 123016 | 0.73 | 8.67E-04 |
| | | | NM_198309 | | | |
| | | | NM_198310 | | | |
| SSBP3 | 217991_x_at | NM_018070 | NM_00100995 5 | 23648 | 0.73 | 2.77E-03 |
| | | | NM_018070 | | | |
| | | | NM_145716 | | | |
| PPIF | 201489_at | BC005020 | NM_005729 | 10105 | 0.73 | 5.19E-05 |
| LOC155435 | 226732_at | AI823400 | NM_001008408 | 155435 | 0.73 | 8.92E-03 |
| RAD1 | 210216_x_at | AF084513 | NM_002853 | 5810 | 0.73 | 1.61E-04 |
| | | | NM_133282 | | | |
| | | | NM_133377 | | | |
| PDE7A | 223358_s_at | AW269834 | NM_002603 | 5150 | 0.73 | 1.04E-03 |
| | | | NM_002604 | | | |
| LOC284611 | 226568_at | AI478747 | NM_001010882 | 284611 | 0.73 | 4.22E-03 |
| MPHOSPH9 | 1558369_at | BC036600 | NM_022782 | 10198 | 0.73 | 9.01E-03 |
| AHCY | 200903_s_at | NM_000687 | NM_000687 | 191 | 0.73 | 6.38E-04 |
| KIAA1393 | 221952_x_at | AB037814 | NM_020810 | 57570 | 0.73 | 1.27E-03 |
| WDR50 | 203721_s_at | NM_016001 | NM_016001 | 51096 | 0.73 | 2.41E-06 |
| NRF1 | 204651_at | AW003022 | NM_005011 | 4899 | 0.72 | 2.06E-05 |
| ATP6V1A | 201972_at | AF113129 | NM_001690 | 523 | 0.72 | 6.11E-06 |
| ATP6V1C1 | 202872_at | AW024925 | NM_00100725 4 | 528 | 0.72 | 5.21E-03 |
| | | | NM_001695 | | | |
| AP3M1 | 222517_at | AU152391 | NM_012095 | 26985 | 0.72 | 8.61E-04 |
| | | | NM_207012 | | | |
| HIP1R | 38340_at | AB014555 | NM_003959 | 9026 | 0.72 | 9.01E-03 |
| CDKAL1 | 214877_at | BE794663 | NM_017774 | 54901 | 0.72 | 2.66E-07 |
| C10orf119 | 222464_s_at | BC004183 | NM_024834 | 79892 | 0.72 | 2.58E-04 |
| QRSL1 | 218949_s_at | NM_018292 | NM_018292 | 55278 | 0.72 | 1.91E-04 |
| HSPC129 | 223271_s_at | AF161543 | NM_016396 | 51496 | 0.72 | 1.33E-04 |
| MTAP | 204956_at | NM_002451 | NM_002451 | 4507 | 0.72 | 9.35E-03 |
| TNFRSF10B | 210405_x_at | AF153687 | NM_003842 | 8795 | 0.72 | 1.20E-05 |
| | | | NM_147187 | | | |
| NDUFS1 | 203039_s_at | NM_005006 | NM_005006 | 4719 | 0.72 | 3.56E-06 |
| SRPK1 | 202199_s_at | AW082913 | NM_003137 | 6732 | 0.71 | 2.30E-04 |
| B4GALT4 | 210540_s_at | BC004523 | NM_003778 | 8702 | 0.71 | 3.68E-03 |
| | | | NM_212543 | | | |
| RBM9 | 213901_x_at | AW149379 | NM_014309 | 23543 | 0.71 | 8.10E-03 |
| DENR | 234915_s_at | BC000925 | NM_003677 | 8562 | 0.71 | 1.01E-02 |
| CPOX | 204172_at | NM_000097 | NM_000097 | 1371 | 0.71 | 4.66E-04 |
| HSPC121 | 217777_s_at | NM_016395 | NM_016395 | 51495 | 0.71 | 2.24E-04 |
| RFX5 | 202963_at | AW027312 | NM_000449 | 5993 | 0.71 | 6.50E-03 |
| SHD1 | 205449_at | NM_013299 | NM_013299 | 29901 | 0.71 | 7.97E-03 |
| ARF3 | 200734_s_at | BG341906 | NM_001659 | 377 | 0.71 | 3.13E-05 |
| ING1 | 208415_x_at | NM_005537 | NM_005537 | 3621 | 0.71 | 1.86E-05 |
| | | | NM_198217 | | | |
| | | | NM_198218 | | | |
| | | | NM_198219 | | | |
| TH1L | 225261_x_at | AJ238376 | NM_016397 | 51497 | 0.71 | 2.65E-03 |
| | | | NM_198976 | | | |
| VCL | 200931_s_at | NM_014000 | NM_003373 | 7414 | 0.71 | 4.61E-03 |
| | | | NM_014000 | | | |
| ZNF545 | 235176_at | AA927918 | NM_133466 | 284406 | 0.71 | 8.64E-03 |
| UBOX5 | 204598_at | NM_014948 | NM_014948 | 22888 | 0.71 | 5.08E-03 |
| | | | NM_199415 | | | |
| ELAVL1 | 201726_at | BC003376 | NM_001419 | 1994 | 0.71 | 7.04E-03 |
| ITGB5 | 201125_s_at | NM_002213 | NM_002213 | 3693 | 0.71 | 2.15E-03 |
| NUP205 | 212247_at | AW008531 | NM_015135 | 23165 | 0.71 | 5.94E-04 |
| SLC17A5 | 221041_s_at | NM_012434 | NM_012434 | 26503 | 0.71 | 9.98E-03 |
| AI939588 | 241156_at | AI939588 | --- | --- | 0.71 | 3.21E-03 |
| SCRIB | 212556_at | AI469403 | NM_015356 | 23513 | 0.71 | 7.66E-03 |
| | | | NM_182706 | | | |
| SOCS4 | 226178_at | BF446961 | NM_080867 | 122809 | 0.71 | 1.73E-04 |
| | | | NM_199421 | | | |
| C6orf211 | 218195_at | NM_024573 | NM_024573 | 79624 | 0.71 | 6.55E-03 |
| PSMA7 | 216088_s_at | AL078633 | NM_002792 | 5688 | 0.71 | 2.39E-04 |
| | | | NM_152255 | | | |
| AKR1B1 | 201272_at | NM_001628 | NM_001628 | 231 | 0.71 | 4.69E-04 |
| HMG20B | 210719_s_at | BC002552 | NM_006339 | 10362 | 0.71 | 5.46E-03 |
| DZIP3 | 213186_at | BG502305 | NM_014648 | 9666 | 0.71 | 5.65E-03 |
| MFI2 | 223723_at | BC001875 | NM_005929 | 4241 | 0.71 | 2.36E-03 |
| | | | NM_033316 | | | |
| PPP1CC | 200726_at | NM_002710 | NM_002710 | 5501 | 0.71 | 1.35E-03 |
| AL521247 | 225046_at | AL521247 | XM_374317 | 389831 | 0.71 | 3.82E-03 |
| KIAA0841 | 213053_at | AW189966 | XM_049237 | 23354 | 0.71 | 9.24E-03 |
| PDE7A | 224046_s_at | U67932 | NM_002603 | 5150 | 0.71 | 5.94E-03 |
| | | | NM_002604 | | | |
| FBXO30 | 226541_at | AI808182 | NM_032145 | 84085 | 0.71 | 1.04E-02 |
| SRC | 213324_at | AK024281 | NM_005417 | 6714 | 0.70 | 5.83E-05 |
| | | | NM_198291 | | | |
| LOC57168 | 227014_at | BE550881 | NM_020437 | 57168 | 0.70 | 3.95E-04 |
| CIB2 | 205008_s_at | NM_006383 | NM_006383 | 10518 | 0.70 | 1.68E-03 |
| GPAM | 225420_at | AV699379 | NM_020918 | 57678 | 0.70 | 5.07E-03 |
| KLF7 | 1555420_a_at | BC012919 | NM_003709 | 8609 | 0.70 | 6.45E-03 |
| AA833830 | 230896_at | AA833830 | --- | --- | 0.70 | 7.62E-04 |
| BTBD14A | 243431_at | BF000597 | NM_ 144653 | 138151 | 0.70 | 2.99E-03 |
| MAPK11 | 211499_s_at | U92268 | NM_002751 | 5600 | 0.70 | 9.93E-03 |
| | | | NM_138993 | | | |
| FSCN1 | 201564_s_at | NM_003088 | NM_003088 | 6624 | 0.70 | 2.66E-03 |
| PLAGL2 | 202924_s_at | AL562280 | NM_002657 | 5326 | 0.70 | 3.13E-04 |
| USP39 | 217829_s_at | NM_006590 | NM_006590 | 10713 | 0.70 | 6.69E-04 |
| ABHD10 | 222697 _s_at | AI521709 | NM_018394 | 55347 | 0.70 | 3.65E-04 |
| C20orf142 | 233061_at | AL117382 | XM_371399 | 128486 | 0.70 | 8.68E-03 |
| GPR51 | 209990_s_at | AF056085 | NM_005458 | 9568 | 0.70 | 1.26E-03 |
| CELSR3 | 205165_at | NM_001407 | NM_001407 | 1951 | 0.70 | 1.21E-03 |
| SSBP2 | 210829_s_at | AF077048 | NM_012446 | 23635 | 0.70 | 7.97E-04 |
| POT1 | 204353_s_at | BC002923 | NM_015450 | 25913 | 0.70 | 1.78E-04 |
| B4GALT6 | 206233_at | AF097159 | NM_004775 | 9331 | 0.70 | 1.09E-03 |
| MARF (C3HC4) | 210075_at | AF151074 | NM_00100541 5 | 51257 | 0.70 | 7.91E-03 |
| | | | NM_00100541 6 | | | |
| | | | NM_016496 | | | |
| MGC4825 | 221620_s_at | AF061264 | NM_024122 | 79135 | 0.70 | 9.49E-03 |
| CBLL1 | 227187_at | AI824009 | NM_024814 | 79872 | 0.70 | 2.62E-03 |
| RAB11A | 200864_s_at | NM_004663 | NM_004663 | 8766 | 0.70 | 7.58E-03 |
| SLBP | 206052_s_at | NM_006527 | NM_006527 | 7884 | 0.70 | 1.28E-03 |
| TBX3 | 229576_s_at | N29712 | NM_005996 | 6926 | 0.70 | 1.56E-03 |
| | | | NM_016569 | | | |
| RNF10 | 207801_s_at | NM_014868 | NM_014868 | 9921 | 0.69 | 2.04E-03 |
| KIAA0153 | 1552257_a_at | NM_015140 | NM_015140 | 23170 | 0.69 | 9.62E-04 |
| THOC4 | 226320_at | AF047002 | NM_005782 | 10189 | 0.69 | 2.92E-03 |
| SDHA | 201093_x_at | NM_004168 | NM_004168 | 6389 | 0.69 | 1.14E-05 |
| FKBPL | 219187_at | NM_022110 | NM_022110 | 63943 | 0.69 | 5.41E-03 |
| EXOSC3 | 223489_x_at | AW015573 | NM_00100226 9 | 51010 | 0.69 | 2.58E-04 |
| | | | NM_016042 | | | |
| COCH | 1554242_a_at | BC007230 | NM_004086 | 1690 | 0.69 | 4.57E-03 |
| C16orf34 | 212109_at | AI590869 | NM_144570 | 90861 | 0.69 | 1.30E-04 |
| CITED1 | 207144_s_at | NM_004143 | NM_004143 | 4435 | 0.69 | 6.40E-03 |
| ZDHHC3 | 218077_s_at | BE542551 | NM_016598 | 51304 | 0.69 | 1.09E-03 |
| AW611729 | 228744_at | AW611729 | | --- | 0.69 | 1.38E-03 |
| MBD4 | 209580_s_at | AF114784 | NM_003925 | 8930 | 0.69 | 1.50E-04 |
| CKLF | 221058_s_at | NM_016326 | NM_016326 | 51192 | 0.69 | 6.66E-03 |
| | | | NM_016951 | | | |
| | | | NM_181640 | | | |
| | | | NM_181641 | | | |
| PPM1F | 37384_at | D86995 | NM_014634 | 9647 | 0.69 | 9.40E-05 |
| CSTF1 | 32723_at | L02547 | NM_001324 | 1477 | 0.69 | 7.49E-04 |
| KIAA1333 | 223257_at | AI823905 | NM_017769 | 55632 | 0.69 | 1.45E-04 |
| SHB | 1557458_s_at | BU685917 | NM_003028 | 6461 | 0.69 | 1.46E-03 |
| AA608749 | 228095_at | AA608749 | --- | --- | 0.69 | 8.96E-03 |
| MPST | 203524_s_at | NM_021126 | NM_021126 | 4357 | 0.69 | 4.69E-04 |
| METRN | 232269_x_at | BE965311 | NM_024042 | 79006 | 0.69 | 7.29E-03 |
| NUP43 | 219007_at | NM_024647 | NM_024647 | 348995 | 0.69 | 8.85E-05 |
| | | | NM_198887 | | | |
| TTYH2 | 223741_s_at | BC004233 | NM_032646 | 94015 | 0.69 | 9.46E-03 |
| | | | NM_052869 | | | |
| C20orf178 | 225119_at | AW299290 | NM_176812 | 128866 | 0.69 | 9.97E-06 |
| GTF3A | 215091_s_at | BE542815 | NM_002097 | 2971 | 0.69 | 1.55E-04 |
| NIPSNAP1 | 201708_s_at | AW083371 | NM_003634 | 8508 | 0.69 | 1.45E-03 |
| C20orf44 | 217935_s_at | NM_018244 | NM_018244 | 55245 | 0.68 | 2.08E-03 |
| | | | NM_199487 | | | |
| | | | NM_199513 | | | |
| PNRC2 | 222406_s_at | AV738970 | NM_017761 | 55629 | 0.68 | 5.58E-05 |
| IGFBP2 | 202718_at | NM_000597 | NM_000597 | 3485 | 0.68 | 8.62E-03 |
| PDE6D | 204091_at | NM_002601 | NM_002601 | 5147 | 0.68 | 3.97E-04 |
| KIAA0376 | 212480_at | AB002374 | NM_015330 | 23384 | 0.68 | 1.42E-03 |
| PLAGL2 | 202925_s_at | NM_002657 | NM_002657 | 5326 | 0.68 | 1.26E-03 |
| C6orf18 | 42361_g_at | AI588986 | NM_019052 | 54535 | 0.68 | 4.87E-04 |
| C16orf5 | 223960_s_at | AF195661 | NM_013399 | 29965 | 0.68 | 1.19E-03 |
| ZDHHC22 | 229875_at | AI363193 | NM_174976 | 283576 | 0.68 | 8.32E-03 |
| RetSat | 1566472_s_at | AK098125 | NM_017750 | 54884 | 0.68 | 8.62E-03 |
| CD47 | 213857_s_at | BG230614 | NM_001777 | 961 | 0.68 | 1.35E-04 |
| | | | NM_198793 | | | |
| ADRBK2 | 228771_at | AI651212 | NM_005160 | 157 | 0.68 | 4.73E-04 |
| KCTD9 | 218823_s_at | NM_017634 | NM_017634 | 54793 | 0.68 | 1.27E-03 |
| H49805 | 239911_at | H49805 | --- | --- | 0.68 | 6.32E-03 |
| ZA20D3 | 221613_s_at | AL136598 | NM_019006 | 54469 | 0.68 | 2.84E-04 |
| KELCHL | 221837_at | BG325646 | NM_032775 | 84861 | 0.68 | 4.31E-03 |
| HMOX2 | 218120_s_at | D21243 | NM_002134 | 3163 | 0.68 | 2.33E-04 |
| C22orf13 | 223039_at | BC004144 | NM_031444 | 83606 | 0.68 | 2.45E-03 |
| ZNF71 | 226514_at | W55975 | NM_021216 | 58491 | 0.68 | 1.83E-03 |
| MGC12466 | 223898_at | BC005360 | NM_033213 | 93474 | 0.68 | 6.20E-03 |
| HMGB1 | 224731_at | BF673940 | NM_002128 | 3146 | 0.68 | 1.63E-03 |
| TBC1D7 | 223461_at | AF151073 | NM_016495 | 51256 | 0.68 | 8.95E-04 |
| PDCL | 204449_at | NM_005388 | NM_005388 | 5082 | 0.68 | 1.36E-03 |
| RAD1 | 235253_at | AI742925 | NM_002853 | 5810 | 0.68 | 2.51E-03 |
| | | | NM_133282 | | | |
| | | | NM_133377 | | | |
| CKB | 200884_at | NM_001823 | NM_001823 | 1152 | 0.68 | 3.63E-05 |
| SRrp35 | 239295_at | AA889416 | NM_080743 | 135295 | 0.68 | 5.74E-03 |
| SARA2 | 218254_s_at | NM_016103 | NM_016103 | 51128 | 0.68 | 7.27E-06 |
| CBX2 | 226473_at | BE514414 | --- | 876 | 0.68 | 2.77E-04 |
| BLCAP | 201032_at | NM_006698 | NM_006698 | 10904 | 0.68 | 1.40E-04 |
| MGC5139 | 202365_at | BC004815 | --- | 84747 | 0.68 | 1.18E-03 |
| HIBADH | 224812_at | AL577446 | NM_152740 | 11112 | 0.68 | 4.29E-05 |
| MOBK1B | 201298_s_at | BC003398 | NM_018221 | 55233 | 0.68 | 1.42E-05 |
| NIPSNAP1 | 201709_s_at | NM_003634 | NM_003634 | 8508 | 0.67 | 3.88E-03 |
| CSNK2A1 | 212072_s_at | AL049761 | NM_001895 | 1457 | 0.67 | 4.08E-03 |
| | | | NM_177559 | | | |
| | | | NM_177560 | | | |
| DUT | 208956_x_at | U62891 | NM_001948 | 1854 | 0.67 | 2.02E-03 |
| HOOK3 | 224359_s_at | AF241830 | NM_032410 | 84376 | 0.67 | 9.68E-04 |
| CSTF3 | 229665_at | AI653037 | NM_001326 | 1479 | 0.67 | 9.91E-03 |
| RUVBL1 | 201614_s_at | NM_003707 | NM_003707 | 8607 | 0.67 | 3.37E-03 |
| PNRC2 | 217779_s_at | NM_017761 | NM_017761 | 55629 | 0.67 | 1.36E-03 |
| KIAA1893 | 227975_at | AW963386 | NM_052899 | 114787 | 0.67 | 8.58E-03 |
| KIAA1949 | 224927_at | BG251556 | --- | 170954 | 0.67 | 8.52E-03 |
| PSMB2 | 200039_s_at | NM_002794 | NM_002794 | 5690 | 0.67 | 2.36E-04 |
| RCOR2 | 236030_at | BF528119 | NM_173587 | 283248 | 0.67 | 3.41E-03 |
| AL157484 | 221861_at | AL157484 | --- | --- | 0.67 | 8.93E-03 |
| RBX1 | 218117_at | NM_014248 | NM_014248 | 9978 | 0.67 | 4.54E-03 |
| SIPA1 | 204164_at | NM_006747 | NM_006747 | 6494 | 0.67 | 5.05E-03 |
| | | | NM_153253 | | | |
| DHRS8 | 217989_at | NM_016245 | NM_016245 | 51170 | 0.67 | 3.77E-03 |
| FBXO45 | 225100_at | BF590021 | XM_117294 | 200933 | 0.67 | 5.60E-03 |
| C19orf25 | 226378_s_at | AI554705 | NM_152482 | 148223 | 0.67 | 2.15E-03 |
| E2F3 | 203693_s_at | NM_001949 | NM_001949 | 1871 | 0.67 | 9.86E-04 |
| SERAC1 | 232183_at | AA128978 | NM_032861 | 84947 | 0.67 | 2.83E-05 |
| LOC129138 | 231847_at | Z97630 | NM_138797 | 129138 | 0.67 | 4.79E-04 |
| AL524175 | 225479_at | AL524175 | --- | --- | 0.67 | 3.32E-04 |
| CCDC5 | 225297_at | AV715391 | NM_138443 | 115106 | 0.67 | 1.96E-04 |
| KIAA1166 | 225784_s_at | AU150745 | NM_018684 | 55906 | 0.67 | 4.05E-03 |
| SH3BP4 | 222258_s_at | AF015043 | NM_014521 | 23677 | 0.67 | 8.34E-05 |
| YTHDF1 | 221741_s_at | AL096828 | NM_017798 | 54915 | 0.66 | 8.07E-04 |
| DKFZP566E14 | 218194_at | NM_015523 | NM_015523 | 25996 | 0.66 | 9.77E-05 |
| HADHSC | 201035_s_at | BC000306 | NM_005327 | 3033 | 0.66 | 2.93E-03 |
| GALNT4 | 231832_at | AI890347 | NM_003774 | 8693 | 0.66 | 6.21E-03 |
| FLJ90709 | 243709_at | BG054799 | NM_173514 | 153129 | 0.66 | 1.04E-02 |
| SMARCB1 | 212167_s_at | AK021419 | NM_00100746 8 | 6598 | 0.66 | 3.27E-05 |
| | | | NM_003073 | | | |
| NDUFC1 | 203478_at | NM_002494 | NM_002494 | 4717 | 0.66 | 4.38E-03 |
| ATP1A1 | 220948_s_at | NM_000701 | NM_000701 | 476 | 0.66 | 9.29E-05 |
| | | | NM_00100158 6 | | | |
| KIAA0265 | 209256_s_at | AF277177 | NM_014997 | 23008 | 0.66 | 5.63E-03 |
| KIAA0153 | 216251_s_at | BF965437 | NM_015140 | 23170 | 0.66 | 1.12E-04 |
| CCNG1 | 208796_s_at | BC000196 | NM_004060 | 900 | 0.66 | 7.05E-03 |
| | | | NM_199246 | | | |
| DT1P1A10 | 213079_at | AA223871 | NM_058163 | 90121 | 0.66 | 1.80E-03 |
| FLJ32065 | 223443_s_at | BC003669 | NM_153032 | 201283 | 0.66 | 8.33E-04 |
| CORO1C | 222409_at | AL162070 | NM_014325 | 23603 | 0.66 | 6.46E-05 |
| GGA2 | 208914_at | BE646414 | NM_015044 | 23062 | 0.66 | 1.84E-03 |
| | | | NM_138640 | | | |
| SNRPB | 208821_at | J04564 | NM_003091 | 6628 | 0.66 | 8.27E-05 |
| | | | NM_198216 | | | |
| NUCKS | 224581_s_at | AI707721 | NM_022731 | 64710 | 0.66 | 5.13E-06 |
| FLJ40432 | 228810_at | AW135279 | NM_152523 | 151195 | 0.66 | 7.36E-05 |
| G6PC3 | 221759_at | AL583123 | NM_138387 | 92579 | 0.66 | 9.07E-04 |
| CAMTA1 | 225692_at | AV756867 | NM_015215 | 23261 | 0.66 | 1.84E-03 |
| CBX5 | 212126_at | BG391282 | NM_012117 | 23468 | 0.66 | 2.40E-03 |
| LIMK2 | 202193_at | NM_005569 | NM_005569 | 3985 | 0.66 | 8.69E-03 |
| | | | NM_016733 | | | |
| MRPL40 | 203152_at | NM_003776 | NM_003776 | 64976 | 0.66 | 7.24E-03 |
| CBR4 | 244052_at | AI469277 | NM_032783 | 84869 | 0.66 | 1.00E-03 |
| NKIRAS1 | 225930_at | AI970120 | NM_020345 | 28512 | 0.65 | 3.27E-04 |
| ASH2L | 209517 _s_at | AB020982 | NM_004674 | 9070 | 0.65 | 4.47E-04 |
| KRAS | 204009_s_at | W80678 | NM_004985 | 3845 | 0.65 | 2.50E-04 |
| | | | NM_033360 | | | |
| KELCHL | 221838_at | N38751 | NM_032775 | 84861 | 0.65 | 6.67E-04 |
| MRPL35 | 218890_x_at | NM_016622 | NM_016622 | 51318 | 0.65 | 6.17E-03 |
| | | | NM_145644 | | | |
| NUDT3 | 221579_s_at | AF062530 | NM_006703 | 11165 | 0.65 | 6.42E-03 |
| MAGEH1 | 218573_at | NM_014061 | NM_014061 | 28986 | 0.65 | 1.12E-04 |
| CCNK | 225824_at | AA528091 | NM_003858 | 317762 | 0.65 | 1.32E-04 |
| C14orf65 | | | | 8812 | | |
| CPE | 201117 _s_at | NM_001873 | NM_001873 | 1363 | 0.65 | 5.66E-03 |
| GAMT | 205354_at | NM_000156 | NM_000156 | 2593 | 0.65 | 1.31E-03 |
| | | | N M_138924 | | | |
| KIAA1423 | 225127_at | BF217531 | XM_376550 | 57583 | 0.65 | 2.39E-03 |
| EXOSC3 | 227916_x_at | AA747303 | NM_00100226 9 | 51010 | 0.65 | 9.83E-03 |
| | | | NM_016042 | | | |
| RNF166 | 227726_at | BF057084 | NM_178841 | 115992 | 0.65 | 8.38E-04 |
| SFI1 | 213431_x_at | AB011114 | NM_00100746 7 | 9814 | 0.65 | 6.82E-03 |
| | | | NM_014775 | | | |
| FLJ14721 | 226487_at | BE551088 | NM_032829 | 84915 | 0.65 | 9.93E-04 |
| FLJ21908 | 1557984_s_at | BI464019 | NM_024604 | 79657 | 0.65 | 4.41E-03 |
| TNFRSF10B | 209294_x_at | BC001281 | NM_003842 | 8795 | 0.65 | 9.89E-05 |
| | | | NM_147187 | | | |
| FLJ14827 | 44065_at | AI937468 | NM_032848 | 84934 | 0.65 | 1.53E-04 |
| TMEM33 | 218465_at | NM_018126 | NM_018126 | 55161 | 0.65 | 1.81E-03 |
| PDHA1 | 1555864_s_at | AW057819 | NM_000284 | 5160 | 0.65 | 2.47E-04 |
| SDCBP | 200958_s_at | NM_005625 | NM_00100706 7 | 6386 | 0.65 | 5.40E-03 |
| | | | NM_00100706 8 | | | |
| | | | NM_00100706 9 | | | |
| | | | NM_00100707 0 | | | |
| | | | NM_005625 | | | |
| TM4SF9 | 225387_at | AA059445 | NM_005723 | 10098 | 0.65 | 2.21E-04 |
| SARA2 | 1554482_a_at | BC002847 | NM_016103 | 51128 | 0.65 | 1.82E-04 |
| BE138575 | 214020_x_at | BE138575 | --- | --- | 0.65 | 1.59E-05 |
| LOC440885 | 239624_at | AA725362 | XM_498906 | 440885 | 0.65 | 3.36E-04 |
| MGC4308 | 224523_s_at | BC006475 | NM_032359 | 84319 | 0.65 | 5.54E-03 |
| SSBP2 | 203787_at | NM_012446 | NM_012446 | 23635 | 0.65 | 3.43E-03 |
| ELMOD2 | 1553928_at | NM_153702 | NM_153702 | 255520 | 0.65 | 3.72E-03 |
| KIAA1043 | 215146_s_at | AB028966 | --- | 23331 | 0.64 | 5.53E-04 |
| BC015774 | 1569973_at | BC015774 | XM_496867 | 441220 | 0.64 | 7.67E-03 |
| FLJ14166 | 219227_at | NM_024565 | NM_024565 | 79616 | 0.64 | 4.93E-03 |
| BRMS1L | 224484_s_at | BC006250 | NM_032352 | 84312 | 0.64 | 1.03E-02 |
| SIP1 | 211115_x_at | AB037703 | NM_00100918 2 | 8487 | 0.64 | 4.84E-05 |
| | | | NM_00100918 3 | | | |
| | | | NM_003616 | | | |
| RGS19 | 204336_s_at | NM_005873 | NM_005873 | 10287 | 0.64 | 2.62E-03 |
| HEMK1 | 52159_at | W93807 | NM_016173 | 51409 | 0.64 | 4.05E-03 |
| BCCIP | 218264_at | NM_016567 | NM_016567 | 56647 | 0.64 | 2.33E-04 |
| | | | NM_078468 | | | |
| | | | NM_078469 | | | |
| RPL5 | 213689_x_at | AL137958 | NM_000969 | 388650 | 0.64 | 1.28E-03 |
| LOC388650 | | | NM_00100660 5 | 6125 | | |
| CYCS | 208905_at | BC005299 | NM_018947 | 54205 | 0.64 | 6.99E-07 |
| ARF3 | 211622_s_at | M33384 | NM_001659 | 377 | 0.64 | 1.47E-04 |
| C19orf14 | 1553125_x_at | NM_173636 | NM_173636 | 284403 | 0.64 | 9.52E-03 |
| MGC10433 | 205740_s_at | NM_024321 | NM_024321 | 79171 | 0.64 | 3.28E-04 |
| PRKDC | 208694_at | U47077 | NM_006904 | 5591 | 0.64 | 8.30E-03 |
| SCDR10 | 1569905_at | BC018336 | NM_198533 | 374875 | 0.64 | 8.71E-03 |
| | | | NM_198704 | | | |
| | | | NM_198705 | | | |
| | | | NM_198706 | | | |
| | | | NM_198707 | | | |
| | | | NM_198708 | | | |
| TGIF2 | 216262_s_at | AL050318 | NM_021809 | 60436 | 0.64 | 1.37E-05 |
| LOC90268 | 229268_at | AA723152 | NM_138348 | 90268 | 0.64 | 3.05E-04 |
| PARP1 | 208644_at | M32721 | NM_001618 | 142 | 0.64 | 6.21E-05 |
| RGMB | 227339_at | BE206621 | NM_173670 | 285704 | 0.64 | 2.63E-04 |
| LOC285705 | | | | 285705 | | |
| ZNF250 | 213858_at | BE350026 | NM_021061 | 58500 | 0.64 | 6.68E-03 |
| ACTR1A | 200720_s_at | AL532341 | NM_005736 | 10121 | 0.64 | 3.97E-03 |
| FBXL10 | 229855_at | AA779208 | NM_00100536 6 | 84678 | 0.64 | 5.29E-03 |
| | | | NM_032590 | | | |
| PIPPIN | 209981_at | AL023553 | NM_014460 | 27254 | 0.64 | 8.32E-03 |
| C22orf16 | 224932_at | AI814909 | NM_213720 | 400916 | 0.64 | 5.31E-03 |
| DTNBP1 | 223445_at | AF061734 | NM_032122 | 84062 | 0.63 | 1.32E-03 |
| | | | NM_183040 | | | |
| | | | NM_183041 | | | |
| GRWD1 | 221549_at | AF337808 | NM_031485 | 83743 | 0.63 | 8.09E-03 |
| LARS2 | 34764_at | D21851 | NM_015340 | 23395 | 0.63 | 1.98E-03 |
| PPP5C | 201979_s_at | NM_006247 | NM_006247 | 5536 | 0.63 | 5.76E-05 |
| AW051899 | 242602_x_at | AW051899 | --- | --- | 0.63 | 2.62E-03 |
| FLJ21125 | 219560_at | NM_024627 | NM_024627 | 79680 | 0.63 | 9.02E-03 |
| MRPS28 | 219819_s_at | NM_014018 | NM_014018 | 28957 | 0.63 | 1.73E-03 |
| SENP1 | 226619_at | AW665440 | NM_014554 | 29843 | 0.63 | 3.06E-03 |
| CENTD2 | 34206_at | AB018325 | NM_015242 | 116985 | 0.63 | 2.69E-04 |
| | | | NM_139181 | | | |
| TPM1 | 206117_at | NM_000366 | NM_000366 | 7168 | 0.63 | 1.78E-03 |
| RAP1GDS1 | 209444_at | BC001851 | NM_021159 | 5910 | 0.63 | 2.24E-04 |
| CELSR3 | 40020_at | AB011536 | NM_001407 | 1951 | 0.63 | 6.88E-04 |
| PPM1F | 203063_at | NM_014634 | NM_014634 | 9647 | 0.63 | 3.60E-05 |
| HMGB1 | 214938_x_at | AF283771 | NM_002128 | 3146 | 0.63 | 4.46E-04 |
| AW294869 | 225227_at | AW294869 | --- | --- | 0.63 | 1.30E-03 |
| AI417537 | 227739_at | AI417537 | XM_372169 | 389822 | 0.63 | 9.49E-03 |
| GTF3A | 201338_x_at | NM_002097 | NM_002097 | 2971 | 0.63 | 4.05E-04 |
| EXOSC3 | 223490_s_at | AF281132 | NM_00100226 9 | 51010 | 0.63 | 4.03E-05 |
| | | | NM_016042 | | | |
| HSPA14 | 219212_at | NM_016299 | NM_016299 | 51182 | 0.63 | 2.58E-05 |
| DHX15 | 201386_s_at | AF279891 | NM_001358 | 1665 | 0.63 | 2.26E-05 |
| CDK5RAP2 | 220935_s_at | NM_018249 | NM_00101164 9 | 55755 | 0.62 | 1.12E-03 |
| | | | NM_018249 | | | |
| CBX5 | 231862_at | AK023520 | NM_012117 | 23468 | 0.62 | 8.64E-03 |
| AW024563 | 225712_at | AW024563 | --- | --- | 0.62 | 2.41E-03 |
| NME4 | 212739_s_at | AL523860 | NM_005009 | 4833 | 0.62 | 4.02E-04 |
| EEF2K | 225545_at | BF001312 | NM_013302 | 29904 | 0.62 | 1.58E-06 |
| ECHDC1 | 219974_x_at | NM_018479 | NM_018479 | 55862 | 0.62 | 1.24E-03 |
| SORT1 | 224818_at | BE622952 | NM_002959 | 6272 | 0.62 | 6.15E-03 |
| FKBP7 | 231130_at | AA683602 | NM_016105 | 51661 | 0.62 | 6.59E-03 |
| | | | NM_181342 | | | |
| PPP4R2 | 225519_at | AA206408 | NM_174907 | 151987 | 0.62 | 1.07E-03 |
| JAG1 | 216268_s_at | U77914 | NM_000214 | 182 | 0.62 | 3.91E-03 |
| OSBPL1A | 241693_at | BF246603 | NM_018030 | 114876 | 0.62 | 3.59E-03 |
| | | | NM_080597 | | | |
| | | | NM_133268 | | | |
| AP1B1 | 205423_at | NM_001127 | NM_001127 | 162 | 0.62 | 5.15E-06 |
| | | | NM_145730 | | | |
| SNRPB | 213175_s_at | AL049650 | NM_003091 | 6628 | 0.62 | 1.16E-05 |
| | | | NM_198216 | | | |
| MYH9 | 211926_s_at | AI827941 | NM_002473 | 4627 | 0.62 | 7.82E-06 |
| BICD2 | 213154_s_at | AI934125 | NM_00100380 0 | 23299 | 0.62 | 6.85E-03 |
| | | | NM_015250 | | | |
| SNX11 | 220140_s_at | NM_013323 | NM_013323 | 29916 | 0.62 | 6.32E-03 |
| | | | NM_152244 | | | |
| ZNF354A | 205427_at | NM_005649 | NM_005649 | 6940 | 0.62 | 9.78E-03 |
| ANK1 | 205390_s_at | NM_000037 | NM_000037 | 286 | 0.62 | 1.01E-02 |
| | | | NM_020475 | | | |
| | | | NM_020476 | | | |
| | | | NM_020477 | | | |
| | | | NM_020478 | | | |
| | | | NM_020479 | | | |
| | | | NM_020480 | | | |
| | | | NM_020481 | | | |
| SNRPG | 205644_s_at | NM_003096 | NM_003096 | 6637 | 0.62 | 1.58E-03 |
| 15E1.2 | 214711_at | BE568184 | NM_176818 | 283459 | 0.62 | 9.53E-03 |
| PPOX | 204788_s_at | NM_000309 | NM_000309 | 5498 | 0.62 | 5.07E-03 |
| GTF2F2 | 209595_at | BC001771 | NM_004128 | 2963 | 0.62 | 6.89E-04 |
| RAC3 | 206103_at | NM_005052 | NM_005052 | 5881 | 0.62 | 8.05E-04 |
| IRAK1BP1 | 213074_at | BG545769 | NM_001010844 | 134728 | 0.62 | 1.38E-03 |
| M96 | 203345_s_at | AI566096 | NM_007358 | 22823 | 0.62 | 1.24E-03 |
| CHPPR | 203208_s_at | NM_014637 | NM_014637 | 9650 | 0.62 | 5.77E-03 |
| CBFB | 206788_s_at | AF294326 | NM_001755 | 865 | 0.62 | 3.44E-03 |
| | | | NM_022845 | | | |
| HIVEP2 | 212641_at | AL023584 | NM_006734 | 3097 | 0.62 | 2.38E-03 |
| FDPS | 201275_at | NM_002004 | NM_002004 | 2224 | 0.62 | 1.13E-03 |
| SCC-112 | 213983_s_at | AW991219 | NM_015200 | 23244 | 0.62 | 4.84E-03 |
| NCOR2 | 207760_s_at | NM_006312 | NM_006312 | 9612 | 0.62 | 2.28E-03 |
| CIAPIN1 | 208424_s_at | NM_020313 | NM_020313 | 57019 | 0.62 | 2.19E-05 |
| STAT1 | JMISGF3A/M97 | HUMISGF3A/M97 | NM_007315 | 6772 | 0.62 | 9.80E-04 |
| | | | NM_139266 | | | |
| EIF5B | 201027_s_at | NM_015904 | NM_015904 | 9669 | 0.62 | 3.52E-04 |
| FBXO45 | 225099_at | AA126446 | XM_117294 | 200933 | 0.62 | 4.20E-03 |
| COPS6 | 213504_at | W63732 | NM_006833 | 10980 | 0.62 | 7.94E-03 |
| ACO2 | 200793_s_at | NM_001098 | NM_001098 | 50 | 0.61 | 1.19E-03 |
| HADHSC | 211569_s_at | AF001903 | NM_005327 | 3033 | 0.61 | 1.58E-03 |
| M6PRBP1 | 202122_s_at | NM_005817 | NM_005817 | 10226 | 0.61 | 5.73E-03 |
| GPR51 | 211679_x_at | AF095784 | NM_005458 | 9568 | 0.61 | 2.47E-03 |
| LOC63929 | 226501_at | BE966628 | NM_022098 | 63929 | 0.61 | 5.18E-04 |
| FLJ20331 | 218577_at | NM_017768 | NM_017768 | 55631 | 0.61 | 7.70E-03 |
| AI125670 | 231431_s_at | AI125670 | XM_373626 | 388114 | 0.61 | 5.65E-04 |
| RENT1 | 211168_s_at | D86988 | NM_002911 | 5976 | 0.61 | 5.90E-03 |
| COX7B | 202110_at | NM_001866 | NM_001866 | 1349 | 0.61 | 1.87E-03 |
| RBMS1 | 209868_s_at | D28482 | NM_002897 | 5937 | 0.61 | 1.58E-05 |
| | | | NM_016836 | | | |
| | | | NM_016839 | | | |
| NAP1L4 | 1560339_s_at | AK095320 | NM_005969 | 4676 | 0.61 | 7.56E-03 |
| DTNBP1 | 223446_s_at | AF061734 | NM_032122 | 84062 | 0.61 | 1.14E-03 |
| | | | NM_183040 | | | |
| | | | NM_183041 | | | |
| SUHW3 | 235520_at | AA805654 | NM_017666 | 55609 | 0.61 | 7.60E-03 |
| RAD1 | 204461_x_at | NM_002853 | NM_002853 | 5810 | 0.61 | 8.03E-03 |
| | | | NM_133282 | | | |
| | | | NM_133377 | | | |
| NAP1L4 | 201414_s_at | NM_005969 | NM_005969 | 4676 | 0.61 | 8.91E-03 |
| DBN1 | 202806_at | NM_004395 | NM_004395 | 1627 | 0.61 | 6.46E-03 |
| | | | NM_080881 | | | |
| LSM3 | 202209_at | NM_014463 | NM_014463 | 27258 | 0.61 | 3.78E-04 |
| ITGAV | 202351_at | AI093579 | NM_002210 | 3685 | 0.61 | 1.45E-03 |
| PDCD8 | 205512_s_at | NM_004208 | NM_004208 | 9131 | 0.61 | 3.22E-03 |
| | | | NM_145812 | | | |
| | | | NM_145813 | | | |
| GLT28D1 | 219015_s_at | NM_018466 | NM_018466 | 55849 | 0.61 | 1.02E-02 |
| DKFZp586M181 | 224776_at | BF513102 | NM_178819 | 137964 | 0.61 | 3.28E-04 |
| ZNF305 | 206507_at | NM_014724 | NM_014724 | 9753 | 0.61 | 6.84E-03 |
| MRPL37 | 222993_at | AF325707 | NM_016491 | 51253 | 0.61 | 3.36E-03 |
| MYO1B | 212365_at | BF215996 | NM_012223 | 4430 | 0.61 | 4.15E-06 |
| XRCC5 | 208642_s_at | AA205834 | NM_021141 | 7520 | 0.61 | 1.58E-05 |
| AK096932 | 1564220_a_at | AK096932 | --- | --- | 0.60 | 2.76E-03 |
| MGC50372 | 1558097_at | W21283 | NM_173566 | 253143 | 0.60 | 1.75E-03 |
| UBE2R2 | 226954_at | BE221883 | NM_017811 | 54926 | 0.60 | 1.57E-04 |
| LOC283484 | 1556940_at | AW665184 | --- | 283484 | 0.60 | 6.08E-03 |
| C20orf77 | 225024_at | AL117521 | NM_021215 | 58490 | 0.60 | 4.10E-05 |
| FLJ10350 | 217943_s_at | NM_018067 | NM_018067 | 55700 | 0.60 | 2.33E-03 |
| LOC84661 | 224129_s_at | AF226998 | NM_032574 | 84661 | 0.60 | 6.46E-03 |
| SMYD5 | 209516_at | U50383 | NM_006062 | 10322 | 0.60 | 2.51E-04 |
| H2AFJ | 220936_s_at | NM_018267 | NM_018267 | 55766 | 0.60 | 2.36E-03 |
| | | | NM_177925 | | | |
| XPNPEP1 | 208453_s_at | NM_006523 | NM_020383 | 7511 | 0.60 | 2.47E-03 |
| APG7L | 218673_s_at | NM_006395 | NM_006395 | 10533 | 0.60 | 7.62E-03 |
| RASGRP1 | 205590_at | NM_005739 | NM_005739 | 10125 | 0.60 | 8.56E-03 |
| KIAA1333 | 223254_s_at | AA887053 | NM_017769 | 55632 | 0.60 | 1.03E-02 |
| C6orf79 | 220094_s_at | NM_022102 | NM_022102 | 63933 | 0.60 | 1.45E-03 |
| PHACTR3 | 227949_at | AL357503 | NM_080672 | 116154 | 0.60 | 5.45E-04 |
| | | | NM_183244 | | | |
| | | | NM_183246 | | | |
| CSPG6 | 209259_s_at | AF020043 | NM_005445 | 9126 | 0.60 | 4.65E-03 |
| C6orf108 | 204238_s_at | NM_006443 | NM_006443 | 10591 | 0.60 | 6.02E-03 |
| | | | NM_199184 | | | |
| MGC15606 | 215968_at | AF055018 | NM_145037 | 91775 | 0.60 | 6.84E-04 |
| MGC13017 | 235006_at | AL571598 | NM_080656 | 91368 | 0.60 | 6.97E-03 |
| PA2G4 | 208676_s_at | U87954 | NM_006191 | 5036 | 0.60 | 2.40E-03 |
| MRPL2 | 218887_at | NM_015950 | NM_015950 | 51069 | 0.60 | 4.44E-04 |
| FUSIP1 | 206095_s_at | NM_006625 | NM_006625 | 10772 | 0.60 | 6.03E-03 |
| | | | NM_054016 | | | |
| NUP98 | 203194_s_at | AA527238 | NM_005387 | 4928 | 0.60 | 2.00E-03 |
| | | | NM_016320 | | | |
| | | | NM_139131 | | | |
| | | | NM_139132 | | | |
| G6PC3 | 44654_at | AI669655 | NM_138387 | 92579 | 0.60 | 2.87E-03 |
| BCR | 217223_s_at | U07000 | NM_004327 | 613 | 0.60 | 3.32E-03 |
| | | | NM_021574 | | | |
| AP3S2 | 202399_s_at | NM_005829 | NM_005829 | 10239 | 0.59 | 2.43E-03 |
| ZNF589 | 219968_at | NM_016089 | NM_016089 | 51385 | 0.59 | 2.39E-03 |
| AHCYL1 | 200850_s_at | NM_006621 | NM_006621 | 10768 | 0.59 | 5.75E-04 |
| SIN3A | 225135_at | AI433017 | NM_015477 | 25942 | 0.59 | 4.14E-03 |
| CHCHD3 | 217972_at | NM_017812 | NM_017812 | 54927 | 0.59 | 2.72E-03 |
| SSBP3 | 223635_s_at | BC003605 | NM_00100995 5 | 23648 | 0.59 | 1.16E-03 |
| | | | NM_018070 | | | |
| | | | NM_145716 | | | |
| AI127452 | 228443_s_at | AI127452 | --- | --- | 0.59 | 2.59E-03 |
| LOC113174 | 225614_at | AI815207 | NM_138421 | 113174 | 0.59 | 4.60E-04 |
| C20orf24 | 224376_s_at | AF274948 | NM_018840 | 55969 | 0.59 | 1.49E-04 |
| | | | NM_199483 | | | |
| | | | NM_199484 | | | |
| | | | NM_199485 | | | |
| PITPNB | 202522_at | AL031591 | NM_012399 | 23760 | 0.59 | 1.72E-04 |
| RPE | 216574_s_at | J04742 | NM_006916 | 6120 | 0.59 | 1.51E-03 |
| | | | NM_199229 | | | |
| AF061733 | 223477_s_at | AF061733 | --- | --- | 0.59 | 4.14E-03 |
| ECHDC1 | 223088_x_at | BC003549 | NM_018479 | 55862 | 0.59 | 2.67E-05 |
| MRPL30 | 223996_s_at | AF151083 | NM_016503 | 51263 | 0.59 | 5.32E-04 |
| | | | NM_145212 | | | |
| | | | NM_145213 | | | |
| CNOT4 | 203291_at | NM_013316 | NM_00100822 5 | 4850 | 0.59 | 1.86E-04 |
| | | | NM_013316 | | | |
| MGC32020 | 214816_x_at | BC003535 | NM_152266 | 91442 | 0.59 | 1.21E-03 |
| SLC4A7 | 209884_s_at | AF047033 | NM_003615 | 9497 | 0.59 | 8.63E-03 |
| ATXN10 | 208833_s_at | AF119662 | NM_013236 | 25814 | 0.59 | 1.71E-06 |
| POP7 | 209482_at | BC001430 | NM_005837 | 10248 | 0.59 | 4.07E-04 |
| LRRFIP1 | 223492_s_at | BC004958 | NM_004735 | 9208 | 0.59 | 2.17E-03 |
| HIG1 | 221896_s_at | BE739519 | NM_014056 | 25994 | 0.58 | 3.91E-03 |
| PBX3 | 204082_at | NM_006195 | NM_006195 | 5090 | 0.58 | 7.63E-03 |
| LZTR1 | 203412_at | NM_006767 | NM_006767 | 8216 | 0.58 | 7.27E-03 |
| SFI1 | 215699_x_at | AL096768 | NM_00100746 7 | 9814 | 0.58 | 6.00E-03 |
| | | | NM_014775 | | | |
| TOP3B | 213660_s_at | AA581879 | NM_003935 | 8940 | 0.58 | 3.68E-03 |
| PSMD12 | 202352_s_at | AI446530 | NM_002816 | 5718 | 0.58 | 1.19E-03 |
| | | | NM_174871 | | | |
| RAB7L1 | 218700_s_at | BC002585 | NM_003929 | 8934 | 0.58 | 7.31E-03 |
| NM_016402 | 217946_s_at | NM_016402 | --- | --- | 0.58 | 3.60E-04 |
| SIP1 | 211114_x_at | AB037702 | NM_00100918 2 | 8487 | 0.58 | 5.55E-03 |
| | | | NM_00100918 3 | | | |
| | | | NM_003616 | | | |
| COPS8 | 214260_at | AI079287 | NM_006710 | 10920 | 0.58 | 9.44E-03 |
| | | | NM_198189 | | | |
| ZNF141 | 206931_at | NM_003441 | NM_003441 | 7700 | 0.58 | 8.62E-05 |
| LANCL1 | 202020_s_at | NM_006055 | NM_006055 | 10314 | 0.58 | 6.88E-03 |
| PXK | 225796_at | AI684747 | NM_017771 | 54899 | 0.58 | 6.08E-04 |
| AMACR | 209424_s_at | AI796120 | NM_014324 | 23600 | 0.58 | 8.99E-03 |
| | | | NM_203382 | | | |
| RPE | 225040_s_at | AV699857 | NM_006916 | 6120 | 0.58 | 5.46E-04 |
| | | | NM_199229 | | | |
| GSPT1 | 215438_x_at | BE906054 | NM_002094 | 2935 | 0.58 | 1.02E-02 |
| ASXL1 | 212234_at | AL034550 | NM_015338 | 171023 | 0.58 | 1.46E-03 |
| EIF4ENIF1 | 218626_at | NM_019843 | NM_019843 | 56478 | 0.58 | 1.12E-04 |
| RIT1 | 239843_at | AI655057 | NM_006912 | 6016 | 0.58 | 2.20E-03 |
| TP53RK | 225402_at | BG339450 | NM_033550 | 112858 | 0.58 | 1.38E-04 |
| MAT2B | 217993_s_at | NM_013283 | NM_013283 | 27430 | 0.58 | 2.85E-03 |
| | | | NM_182796 | | | |
| TMCC3 | 226489_at | BG177562 | NM_020698 | 57458 | 0.58 | 3.41E-03 |
| POLRMT | 203782_s_at | NM_005035 | NM_005035 | 5442 | 0.58 | 2.34E-03 |
| BG109230 | 228567_at | BG109230 | --- | --- | 0.58 | 2.93E-04 |
| RBMS1 | 203748_x_at | NM_016839 | NM_002897 | 5937 | 0.58 | 1.17E-05 |
| | | | NM_016836 | | | |
| | | | NM_016839 | | | |
| LOC96610 | 226802_s_at | W87523 | NM_080926 | 96610 | 0.58 | 8.19E-03 |

**Table 8. Decreased Gene Expression in Cell Lines H1 and BB10 vs. Cell Line 2D6 in Differentiated State Using Microarray Analysis**

| **Gene Symbol** | **Probe Set ID** | **Representative Public ID** | **RefSeq Transcript ID** | **Entrez Gene** | **Log2 Ratio** | **pvalue** |
|---|---|---|---|---|---|---|
| CYTL1 | 219837 _s_at | NM_018659 | NM_018659 | 54360 | -6.87 | 8.62E-11 |
| IL17B | 220273_at | NM_014443 | NM_014443 | 27190 | -6.24 | 3.55E-08 |
| KIAA0125 | 206478_at | NM_014792 | NM_014792 | 9834 | -5.76 | 4.88E-04 |
| OLFML2A | 213075_at | AL050002 | NM_182487 | 169611 | -5.40 | 4.32E-12 |
| GNAS | 217057_s_at | AF107846 | NM_000516 | 2778 | -5.37 | 6.02E-03 |
| | | | NM_016592 | | | |
| | | | NM_080425 | | | |
| | | | NM_080426 | | | |
| THBS2 | 203083_at | NM_003247 | NM_003247 | 7058 | -5.31 | 9.79E-08 |
| CAP2 | 212554_at | N90755 | NM_006366 | 10486 | -5.29 | 4.85E-06 |
| CYGB | 226632_at | AL513673 | NM_134268 | 114757 | -5.15 | 2.38E-03 |
| MEOX2 | 206201_s_at | NM_005924 | NM_005924 | 4223 | -4.96 | 9.77E-08 |
| AI051127 | 231478_at | AI051127 | --- | --- | -4.84 | 1.89E-03 |
| CRH | 205630_at | NM_000756 | NM_000756 | 1392 | -4.78 | 2.26E-03 |
| NXF | 1554299_at | AB049469 | NM_178864 | 266743 | -4.75 | 3.31E-05 |
| SLC1A2 | 225491_at | AL157452 | NM_004171 | 6506 | -4.71 | 1.26E-09 |
| FGF13 | 205110_s_at | NM_004114 | NM_004114 | 2258 | -4.65 | 6.61E-06 |
| | | | NM_033642 | | | |
| MICAL2 | 212473_s_at | BE965029 | NM_014632 | 9645 | -4.59 | 1.59E-05 |
| CART | 206339_at | NM_004291 | NM_004291 | 9607 | -4.59 | 4.64E-03 |
| ADM | 202912_at | NM_001124 | NM_001124 | 133 | -4.47 | 5.16E-07 |
| OSCAR | 1554503_a_at | BC035023 | NM_130771 | 126014 | -4.38 | 3.92E-04 |
| | | | NM_133168 | | | |
| | | | NM_133169 | | | |
| | | | NM_206817 | | | |
| | | | NM_206818 | | | |
| HTR1E | 207404_s_at | NM_000865 | NM_000865 | 3354 | -4.24 | 8.64E-04 |
| BF681305 | 1561817_at | BF681305 | --- | --- | -4.22 | 5.84E-04 |
| EGFL7 | 218825_at | NM_016215 | NM_016215 | 51162 | -4.16 | 3.07E-05 |
| | | | NM_201446 | | | |
| SLC1A6 | 1554593_s_at | BC028721 | NM_005071 | 6511 | -4.16 | 8.15E-04 |
| GPR54 | 242517_at | AI819198 | NM_032551 | 84634 | -4.15 | 1.40E-04 |
| BC036254 | 1557443_s_at | BC036254 | --- | --- | -4.14 | 5.46E-06 |
| NDRG1 | 200632_s_at | NM_006096 | NM_006096 | 10397 | -4.10 | 8.60E-05 |
| LOC56901 | 218484_at | NM_020142 | NM_020142 | 56901 | -4.10 | 1.79E-03 |
| STX3A | 209238_at | BE966922 | NM_004177 | 6809 | -4.02 | 4.71E-04 |
| AQP1 | 209047_at | AL518391 | NM_000385 | 358 | -4.02 | 1.49E-03 |
| | | | NM_198098 | | | |
| AA530995 | 213832_at | AA530995 | --- | --- | -4.00 | 2.31E-03 |
| AL353759 | 215071_s_at | AL353759 | --- | --- | -3.90 | 1.96E-05 |
| ARHGAP9 | 224451_x_at | BC006107 | NM_032496 | 64333 | -3.89 | 8.61E-04 |
| CAP2 | 212551_at | NM_006366 | NM_006366 | 10486 | -3.86 | 4.69E-07 |
| LOC158572 | 243017_at | AA032156 | XM_379705 | 158572 | -3.85 | 1.09E-04 |
| PDE4C | 211818_s_at | U88712 | NM_000923 | 5143 | -3.78 | 3.71E-03 |
| RaLP | 230538_at | AI027957 | NM_203349 | 399694 | -3.67 | 1.39E-05 |
| PTGS1 | 215813_s_at | S36219 | NM_000962 | 5742 | -3.65 | 1.09E-08 |
| | | | NM_080591 | | | |
| CYGB | 1553572_a_at | NM_134268 | NM_134268 | 114757 | -3.65 | 2.42E-06 |
| ANXA2 | 201590_x_at | NM_004039 | NM_00100285 7 | 302 | -3.65 | 1.12E-05 |
| | | | NM_00100285 8 | | | |
| | | | NM_004039 | | | |
| NEBL | 203961_at | AL157398 | NM_006393 | 10529 | -3.63 | 1.91E-05 |
| | | | NM_213569 | | | |
| ANXA2 | 210427_x_at | BC001388 | NM_00100285 7 | 302 | -3.61 | 1.06E-05 |
| | | | NM_00100285 8 | | | |
| | | | NM_004039 | | | |
| BE219849 | 240180_at | BE219849 | --- | --- | -3.60 | 1.29E-05 |
| AU157716 | 227051_at | AU157716 | --- | --- | -3.56 | 1.17E-07 |
| RaLP | 235238_at | BF676462 | NM_203349 | 399694 | -3.55 | 6.19E-07 |
| RGS11 | 206107_at | NM_003834 | NM_003834 | 8786 | -3.53 | 1.56E-06 |
| | | | NM_183337 | | | |
| AI743780 | 229385_s_at | AI743780 | --- | --- | -3.53 | 5.61E-08 |
| CRH | 205629_s_at | BC002599 | NM_000756 | 1392 | -3.52 | 2.27E-03 |
| MGC4677 | 225799_at | BF209337 | NM_052871 | 112597 | -3.49 | 6.17E-05 |
| AW954199 | 239331_at | AW954199 | --- | --- | -3.49 | 1.64E-05 |
| PTGS1 | 205128_x_at | NM_000962 | NM_000962 | 5742 | -3.48 | 3.62E-08 |
| | | | NM_080591 | | | |
| BF449053 | 238018_at | BF449053 | --- | --- | -3.48 | 3.99E-07 |
| CDC42EP1 | 204693_at | NM_007061 | NM_007061 | 11135 | -3.46 | 1.15E-04 |
| | | | NM_152243 | | | |
| CDH10 | 220115_s_at | NM_006727 | NM_006727 | 1008 | -3.43 | 5.86E-06 |
| AI341823 | 239657_x_at | AI341823 | --- | --- | -3.42 | 9.66E-07 |
| HPCAL1 | 1560154_a_at | AK026500 | NM_002149 | 3241 | -3.40 | 3.33E-08 |
| | | | NM_134421 | | | |
| LOC283454 | 229552_at | AI698128 | --- | 283454 | -3.38 | 1.29E-03 |
| ABLIM3 | 205730_s_at | NM_014945 | NM_014945 | 22885 | -3.34 | 4.32E-05 |
| AI885066 | 239845_at | AI885066 | --- | --- | -3.32 | 1.56E-05 |
| AI810266 | 235456_at | AI810266 | --- | --- | -3.31 | 6.67E-04 |
| HIST1H3E | 236278_at | AV705309 | NM_003532 | 8353 | -3.26 | 1.95E-05 |
| SLC35F3 | 229065_at | BF968270 | NM_173508 | 148641 | -3.24 | 3.97E-06 |
| BHLHB2 | 201170_s_at | NM_003670 | NM_003670 | 8553 | -3.22 | 2.22E-03 |
| NEBL | 203962_s_at | NM_006393 | NM_006393 | 10529 | -3.21 | 3.48E-05 |
| | | | NM_213569 | | | |
| ANXA2 | 213503_x_at | BE908217 | NM_00100285 7 | 302 | -3.19 | 2.51E-07 |
| | | | NM_00100285 8 | | | |
| | | | NM_004039 | | | |
| PHKA2 | 1560358_at | BC038597 | NM_000292 | 5256 | -3.18 | 3.38E-03 |
| GRM5 | 214217_at | D60132 | NM_000842 | 2915 | -3.18 | 2.84E-04 |
| AL512727 | 215014_at | AL512727 | --- | --- | -3.18 | 8.51E-04 |
| STC1 | 204595_s_at | AI300520 | NM_003155 | 6781 | -3.16 | 9.00E-03 |
| BG325630 | 234973_at | BG325630 | --- | --- | -3.15 | 2.18E-03 |
| SYTL1 | 227134_at | AI341537 | NM_032872 | 84958 | -3.14 | 1.02E-05 |
| BC002465 | 216189_at | BC002465 | --- | --- | -3.11 | 3.46E-03 |
| C1orf24 | 217967_s_at | AF288391 | NM_022083 | 116496 | -3.11 | 5.38E-03 |
| | | | NM_052966 | | | |
| PDE4C | 206791_s_at | BF511742 | NM_000923 | 5143 | -3.08 | 2.33E-03 |
| HIST1H2BD | 209911_x_at | BC002842 | NM_021063 | 3017 | -3.08 | 9.52E-06 |
| | | | NM_138720 | | | |
| SYT5 | 206161_s_at | AI659957 | NM_003180 | 6861 | -3.06 | 3.57E-03 |
| LAMP3 | 205569_at | NM_014398 | NM_014398 | 27074 | -3.06 | 8.91E-03 |
| ADARB1 | 203865_s_at | NM_015833 | NM_001112 | 104 | -3.06 | 2.79E-04 |
| | | | NM_015833 | | | |
| | | | NM_015834 | | | |
| CYP2E1 | 1431_at | J02843 | NM_000773 | 1571 | -3.05 | 1.50E-03 |
| LOC150759 | 221973_at | AI983904 | XM_498456 | 150759 | -3.03 | 2.56E-03 |
| | | | XM_499585 | | | |
| SORCS1 | 1556891_at | BE328250 | NM_052918 | 114815 | -3.03 | 3.14E-05 |
| CREB5 | 232555_at | AI689210 | NM_004904 | 9586 | -3.03 | 1.60E-03 |
| | | | NM_182898 | | | |
| | | | NM_182899 | | | |
| ISG20 | 204698_at | NM_002201 | NM_002201 | 3669 | -3.00 | 1.76E-03 |
| AA417078 | 238619_at | AA417078 | --- | --- | -2.99 | 5.87E-05 |
| AW274468 | 242299_at | AW274468 | --- | --- | -2.98 | 2.44E-05 |
| PTPRH | 208300_at | NM_002842 | NM_002842 | 5794 | -2.97 | 3.46E-04 |
| BHLHB2 | 201169_s_at | BG326045 | NM_003670 | 8553 | -2.96 | 1.38E-03 |
| AW270105 | 229413_s_at | AW270105 | --- | --- | -2.92 | 9.07E-04 |
| LOC390916 | 235384_at | AW963217 | XM_372723 | 390916 | -2.90 | 9.52E-08 |
| SLCO1A2 | 211480_s_at | AF085224 | NM_005075 | 6579 | -2.88 | 2.29E-05 |
| | | | NM_021094 | | | |
| | | | NM_134431 | | | |
| HIST1H2BK | 209806_at | BC000893 | NM_080593 | 85236 | -2.88 | 3.64E-05 |
| SYT5 | 206162_x_at | NM_003180 | NM_003180 | 6861 | -2.86 | 6.38E-04 |
| DKFZp761O011 | 207797_s_at | NM_018409 | NM_018409 | 55805 | -2.86 | 2.54E-05 |
| CDKN1C | 219534_x_at | NM_000076 | NM_000076 | 1028 | -2.86 | 3.17E-04 |
| SCHIP1 | 1566772_at | AL832567 | NM_014575 | 29970 | -2.85 | 7.20E-03 |
| CDKN1C | 213182_x_at | R78668 | NM_000076 | 1028 | -2.85 | 3.87E-04 |
| FLJ13955 | 227001_at | AI096706 | NM_024759 | 79815 | -2.82 | 9.16E-04 |
| SYT13 | 226086_at | AB037848 | NM_020826 | 57586 | -2.80 | 7.99E-04 |
| M160 | 223655_at | AF264014 | NM_174941 | 283316 | -2.79 | 3.27E-04 |
| CDKN2A | 209644_x_at | U38945 | NM_000077 | 1029 | -2.78 | 1.33E-06 |
| | | | NM_058195 | | | |
| | | | NM_058197 | | | |
| AA954997 | 243436_at | AA954997 | --- | --- | -2.77 | 1.59E-05 |
| HIST2H2BE | 202708_s_at | NM_003528 | NM_003528 | 8349 | -2.75 | 8.53E-04 |
| GTF2IRD2 | 1557289_s_at | BU617476 | NM_173537 | 84163 | -2.75 | 3.68E-05 |
| AI079543 | 236949_at | AI079543 | XM_498613 | 440283 | -2.75 | 7.55E-03 |
| LONP | 221833_at | AI971258 | NM_031490 | 83752 | -2.75 | 1.32E-03 |
| AI681941 | 237663_at | AI681941 | --- | --- | -2.74 | 1.15E-04 |
| BE894882 | 235681_at | BE894882 | --- | --- | -2.73 | 3.37E-06 |
| GRIA2 | 205358_at | NM_000826 | NM_000826 | 2891 | -2.73 | 7.10E-03 |
| MYO15B | 219173_at | NM_024957 | XR_000222 | 80022 | -2.73 | 4.36E-05 |
| BC042953 | 1561242_at | BC042953 | --- | --- | -2.70 | 6.39E-03 |
| GRIA2 | 236538_at | BE219628 | NM_000826 | 2891 | -2.69 | 3.27E-03 |
| MYO15B | 59375_at | AI825877 | XR_000222 | 80022 | -2.68 | 1.90E-06 |
| AI927692 | 228528_at | AI927692 | --- | --- | -2.67 | 6.22E-06 |
| BC021677 | 1559631_at | BC021677 | --- | --- | -2.67 | 4.72E-04 |
| AA088430 | 242556_at | AA088430 | --- | --- | -2.65 | 5.20E-07 |
| AI769477 | 236875_at | AI769477 | --- | --- | -2.65 | 2.18E-08 |
| HSPA5 | 230031_at | AW052044 | NM_005347 | 3309 | -2.64 | 7.69E-03 |
| HRK | 206865_at | U76376 | NM_003806 | 8739 | -2.64 | 6.03E-03 |
| BE671060 | 235901_at | BE671060 | --- | --- | -2.63 | 9.58E-04 |
| PLA2G4C | 1554810_at | BC017956 | NM_003706 | 8605 | -2.63 | 4.54E-05 |
| CYP2E1 | 209976_s_at | AF182276 | NM_000773 | 1571 | -2.63 | 4.37E-03 |
| CDKN1C | 213348_at | N33167 | NM_000076 | 1028 | -2.62 | 4.09E-04 |
| ARHGAP9 | 232543_x_at | BE675685 | NM_032496 | 64333 | -2.62 | 7.93E-05 |
| AI798680 | 227943_at | AI798680 | --- | --- | -2.62 | 1.59E-05 |
| BG430958 | 1559455_at | BG430958 | --- | --- | -2.59 | 1.73E-06 |
| PPP2R2C | 1562290_at | AL157448 | NM_020416 | 5522 | -2.59 | 4.74E-04 |
| | | | NM_181876 | | | |
| ANK2 | 216195_at | AF131823 | NM_001148 | 287 | -2.59 | 2.14E-03 |
| | | | NM_020977 | | | |
| CYP2E1 | 209975_at | AF182276 | NM_000773 | 1571 | -2.58 | 3.47E-03 |
| BG231712 | 230408_at | BG231712 | --- | --- | -2.58 | 3.02E-03 |
| ACRBP | 223717_s_at | AB051833 | NM_032489 | 84519 | -2.58 | 1.02E-04 |
| AU145981 | 232439_at | AU145981 | --- | --- | -2.57 | 3.24E-05 |
| SIK2 | 233648_at | AK026914 | NM_015191 | 23235 | -2.55 | 1.93E-03 |
| ANK2 | 1561895_at | BC030740 | NM_001148 | 287 | -2.55 | 9.50E-04 |
| | | | NM_020977 | | | |
| RHOB | 1553962_s_at | BI668074 | NM_004040 | 388 | -2.54 | 1.06E-03 |
| BC040652 | 1559528_at | BC040652 | --- | --- | -2.53 | 1.20E-03 |
| CDKN1C | 216894_x_at | D64137 | NM_000076 | 1028 | -2.52 | 2.77E-04 |
| AQP1 | 207542_s_at | NM_000385 | NM_000385 | 358 | -2.52 | 6.49E-03 |
| | | | NM_198098 | | | |
| DKZP434N181 | 232257_s_at | AK022645 | NM_032137 | 84077 | -2.51 | 6.77E-03 |
| CTBP1 | 243180_at | AW452392 | NM_001328 | 1487 | -2.51 | 1.14E-05 |
| KIAA0500 | 213839_at | AW028110 | --- | 57237 | -2.51 | 5.06E-05 |
| AW606588 | 239008_at | AW606588 | --- | --- | -2.50 | 5.69E-04 |
| AA903473 | 235666_at | AA903473 | --- | --- | -2.50 | 1.43E-03 |
| DKFZp313N062 | 1555905_a_at | AI147556 | NM_173826 | 285343 | -2.49 | 1.95E-04 |
| VEGF | 211527_x_at | M27281 | NM_003376 | 7422 | -2.49 | 1.00E-03 |
| FAM46A | 224973_at | AL078599 | NM_017633 | 55603 | -2.48 | 2.76E-05 |
| ZNF395 | 223216_x_at | BC001237 | NM_018660 | 157574 | -2.48 | 7.42E-04 |
| FBXO16 | | | NM_172366 | 55893 | | |
| XPO1 | 230729_at | AI860764 | NM_003400 | 7514 | -2.48 | 1.68E-05 |
| C20orf119 | 231838_at | AK026760 | --- | 80336 | -2.47 | 1.43E-06 |
| ANK2 | 232606_at | AK021894 | NM_001148 | 287 | -2.46 | 1.49E-04 |
| | | | NM_020977 | | | |
| AV702101 | 238595_at | AV702101 | --- | --- | -2.46 | 3.03E-04 |
| AW364675 | 239066_at | AW364675 | --- | --- | -2.45 | 3.80E-06 |
| FAM46A | 221766_s_at | AW246673 | NM_017633 | 55603 | -2.45 | 2.44E-06 |
| AK096401 | 1556204_a_at | AK096401 | XM_375664 | 400721 | -2.44 | 7.27E-04 |
| ZNF395 | 221123_x_at | NM_018660 | NM_018660 | 55893 | -2.44 | 8.86E-04 |
| PTGER2 | 206631_at | NM_000956 | NM_000956 | 5732 | -2.43 | 2.99E-05 |
| FLJ31295 | 226509_at | AV700302 | NM_152320 | 121274 | -2.43 | 2.75E-05 |
| MEF2A | 239571_at | AI123399 | NM_005587 | 4205 | -2.42 | 5.57E-04 |
| AW298171 | 244346_at | AW298171 | --- | --- | -2.41 | 1.16E-05 |
| FLJ30594 | 235836_at | N51413 | --- | 150622 | -2.41 | 3.87E-03 |
| AW205919 | 229830_at | AW205919 | --- | --- | -2.40 | 1.33E-04 |
| BE671519 | 242236_at | BE671519 | --- | --- | -2.39 | 2.68E-03 |
| SIGIRR | 218921_at | NM_021805 | NM_021805 | 59307 | -2.39 | 3.99E-05 |
| AW665665 | 237187_at | AW665665 | --- | --- | -2.39 | 6.09E-04 |
| GPR92 | 230252_at | AW183080 | NM_020400 | 57121 | -2.39 | 1.54E-05 |
| MGC13040 | 224463_s_at | BC006128 | NM_032930 | 85016 | -2.39 | 2.97E-03 |
| ANKS1 | 212747_at | AI990523 | NM_015245 | 23294 | -2.38 | 1.37E-03 |
| ABCC8 | 210246_s_at | AF087138 | NM_000352 | 6833 | -2.38 | 2.60E-04 |
| RHOB | 212099_at | AI263909 | NM_004040 | 388 | -2.38 | 7.05E-04 |
| SMOC2 | 223235_s_at | AB014737 | NM_022138 | 64094 | -2.37 | 1.05E-03 |
| CENTG2 | 1559491_at | AL390180 | NM_014914 | 116987 | -2.36 | 1.41E-03 |
| ARTS-1 | 214012_at | BE551138 | NM_016442 | 51752 | -2.36 | 4.64E-05 |
| TM4SF8 | 232632_at | AU145719 | NM_005724 | 10099 | -2.36 | 2.15E-04 |
| | | | NM_198902 | | | |
| U66046 | 215057_at | U66046 | --- | --- | -2.36 | 2.03E-04 |
| KIAA0251 | 1558430_at | R08650 | NM_015027 | 23042 | -2.35 | 5.89E-03 |
| TIMP1 | 201666_at | NM_003254 | NM_003254 | 7076 | -2.35 | 1.13E-05 |
| AK024567 | 234597_at | AK024567 | --- | --- | -2.34 | 1.59E-04 |
| PPFIA4 | 214978_s_at | AK023365 | NM_015053 | 8497 | -2.34 | 1.46E-03 |
| BCL11B | 233302_at | AU146285 | NM_022898 | 64919 | -2.34 | 1.59E-04 |
| | | | NM_138576 | | | |
| C21orf4 | 228001_at | N51405 | NM_006134 | 757 | -2.34 | 7.50E-05 |
| ATF5 | 204999_s_at | BC005174 | NM_012068 | 22809 | -2.34 | 1.08E-03 |
| COL7A1 | 217312_s_at | L23982 | NM_000094 | 1294 | -2.33 | 2.40E-05 |
| AL049252 | 216527_at | AL049252 | --- | --- | -2.33 | 1.19E-03 |
| VGCNL1 | 228608_at | N49852 | NM_052867 | 259232 | -2.32 | 3.07E-06 |
| OLFM1 | 205591_at | NM_006334 | NM_006334 | 10439 | -2.32 | 9.85E-03 |
| | | | NM_014279 | | | |
| | | | NM_058199 | | | |
| ZNF395 | 232693_s_at | AK021850 | NM_018660 | 157574 | -2.32 | 2.21E-03 |
| FBXO16 | | | NM_172366 | 55893 | | |
| CNTN2 | 230045_at | BF740264 | NM_005076 | 6900 | -2.31 | 5.97E-04 |
| ZNF395 | 239619_at | BF431867 | NM_018660 | 55893 | -2.31 | 2.70E-03 |
| RASGEF1B | 230233_at | BF110534 | NM_152545 | 153020 | -2.31 | 2.05E-03 |
| AGPAT3 | 223182_s_at | AI337300 | NM_020132 | 56894 | -2.31 | 6.95E-03 |
| ZNF395 | 236474_at | AI797677 | NM_018660 | 55893 | -2.30 | 6.29E-03 |
| MPP5 | 242779_at | N36400 | NM_022474 | 64398 | -2.30 | 3.44E-08 |
| AL512728 | 216813_at | AL512728 | --- | --- | -2.30 | 1.89E-03 |
| PLEKHH1 | 225727_at | AB033026 | NM_020715 | 57475 | -2.29 | 1.26E-06 |
| PLEKHH1 | 225726_s_at | AB033026 | NM_020715 | 57475 | -2.29 | 3.43E-07 |
| AI123416 | 227591_at | AI123416 | --- | --- | -2.29 | 2.47E-04 |
| ANK2 | 202921_s_at | NM_001148 | NM_001148 | 287 | -2.29 | 7.41E-03 |
| | | | NM_020977 | | | |
| CKMT1 | 1561155_at | W76548 | NM_020990 | 1159 | -2.28 | 6.30E-03 |
| CFC1 | 223753_s_at | AF312769 | NM_032545 | 55997 | -2.27 | 1.79E-03 |
| BF111884 | 230491_at | BF111884 | --- | --- | -2.27 | 8.21E-06 |
| SEC31L1 | 1556416_s_at | AF086242 | NM_014933 | 22872 | -2.27 | 7.06E-05 |
| | | | NM_016211 | | | |
| SCN2A2 | 206381_at | NM_021007 | NM_021007 | 6326 | -2.26 | 6.33E-06 |
| PLEKHA5 | 237483_at | AI990790 | NM_019012 | 54477 | -2.25 | 8.37E-03 |
| EDG7 | 231192_at | AW274018 | NM_012152 | 23566 | -2.24 | 1.41E-03 |
| DTNA | 240125_at | AI697494 | NM_001390 | 1837 | -2.23 | 6.58E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| AF085937 | 1558695_at | AF085937 | --- | --- | -2.22 | 8.05E-03 |
| AI394334 | 232696_at | AI394334 | --- | --- | -2.22 | 2.96E-03 |
| UCN | 206072_at | NM_003353 | NM_003353 | 7349 | -2.22 | 5.09E-07 |
| DRD1IP | 219896_at | NM_015722 | NM_015722 | 50632 | -2.22 | 1.17E-04 |
| AA412686 | 244608_at | AA412686 | --- | --- | -2.21 | 3.65E-03 |
| TSAP6 | 218424_s_at | NM_018234 | NM_00100841 0 | 55240 | -2.21 | 8.31E-05 |
| | | | NM_018234 | | | |
| | | | NM_182915 | | | |
| LOC201158 | 1560776_at | AU121725 | NM_145301 | 201158 | -2.21 | 7.37E-04 |
| LAF4 | 244696_at | AI033582 | NM_002285 | 3899 | -2.21 | 6.82E-03 |
| S100A6 | 217728_at | NM_014624 | NM_014624 | 6277 | -2.20 | 8.83E-05 |
| BACH2 | 236307_at | AA085906 | NM_021813 | 60468 | -2.20 | 2.30E-03 |
| KIAA1102 | 241459_at | AI494113 | NM_014988 | 22998 | -2.20 | 4.06E-03 |
| COL5A1 | 212488_at | N30339 | NM_000093 | 1289 | -2.20 | 4.18E-06 |
| ENOSF1 | 213645_at | AF305057 | NM_017512 | 55556 | -2.19 | 2.12E-04 |
| LOC158863 | 232179_at | AL110203 | --- | 158863 | -2.19 | 1.08E-05 |
| ECEL1 | 219914_at | NM_004826 | NM_004826 | 9427 | -2.19 | 1.99E-03 |
| C21orf4 | 222907_x_at | BC000569 | NM_006134 | 757 | -2.18 | 3.97E-04 |
| SMYD2 | 233713_at | AK022181 | NM_020197 | 56950 | -2.18 | 6.04E-04 |
| PDZK6 | 228946_at | AW150229 | NM_015693 | 27152 | -2.18 | 3.60E-04 |
| C21orf4 | 225182_at | AL355685 | NM_006134 | 757 | -2.17 | 1.19E-03 |
| AA601031 | 228919_at | AA601031 | --- | --- | -2.17 | 3.77E-03 |
| N95363 | 213183_s_at | N95363 | --- | --- | -2.17 | 3.77E-04 |
| ARHGAP9 | 226906_s_at | AL548053 | NM_032496 | 64333 | -2.17 | 9.12E-04 |
| ZNF395 | 222536_s_at | N36098 | NM_018660 | 55893 | -2.16 | 4.88E-03 |
| FLJ12671 | 233728_at | AU148213 | NM_030980 | 81875 | -2.16 | 8.06E-04 |
| AU146864 | 232659_at | AU146864 | --- | --- | -2.16 | 2.93E-04 |
| SERPINE1 | 202628_s_at | NM_000602 | NM_000602 | 5054 | -2.14 | 2.61E-05 |
| TYRP1 | 242893_at | AW195492 | NM_000550 | 7306 | -2.14 | 1.35E-03 |
| SLC33A1 | 1559232_a_at | BC029450 | NM_004733 | 9197 | -2.14 | 4.34E-03 |
| AI695695 | 242051_at | AI695695 | --- | --- | -2.13 | 1.12E-03 |
| HRK | 206864_s_at | NM_003806 | NM_003806 | 8739 | -2.12 | 4.70E-03 |
| RLF | 241438_at | AI742686 | NM_012421 | 6018 | -2.12 | 4.81E-03 |
| CUL3 | 237376_at | BF115815 | NM_003590 | 8452 | -2.11 | 9.56E-06 |
| BG704082 | 1556216_s_at | BG704082 | --- | --- | -2.11 | 1.16E-03 |
| AI968130 | 240327_at | AI968130 | NM_015129 | 23157 | -2.11 | 3.06E-04 |
| | | | NM_145799 | | | |
| | | | NM_145800 | | | |
| | | | NM_145802 | | | |
| CNGA3 | 207261_at | NM_001298 | NM_001298 | 1261 | -2.11 | 6.85E-04 |
| AI803010 | 239503_at | AI803010 | --- | --- | -2.11 | 1.53E-03 |
| N73550 | 244112_x_at | N73550 | --- | --- | -2.10 | 2.68E-03 |
| BF110426 | 243237_at | BF110426 | XM_114222 | 200373 | -2.10 | 4.35E-04 |
| BM977131 | 1557478_at | BM977131 | --- | --- | -2.10 | 2.99E-03 |
| AV685992 | 241376_at | AV685992 | --- | --- | -2.09 | 4.64E-03 |
| PAN3 | 239393_at | AW510927 | NM_175854 | 255967 | -2.08 | 1.36E-04 |
| VEGF | 210513_s_at | AF091352 | NM_003376 | 7422 | -2.08 | 7.20E-03 |
| BDKRB2 | 205870_at | NM_000623 | NM_000623 | 624 | -2.08 | 2.97E-03 |
| ZNF395 | 218149_s_at | NM_017606 | NM_018660 | 55893 | -2.07 | 6.79E-04 |
| DEPDC6 | 218858_at | NM_022783 | NM_022783 | 64798 | -2.06 | 5.34E-04 |
| EVER1 | 214958_s_at | AK021738 | NM_007267 | 11322 | -2.06 | 2.91E-03 |
| LUC7L2 | 242024_at | T90999 | NM_016019 | 51631 | -2.06 | 2.82E-03 |
| KIAA0804 | 1559500_at | BC036005 | NM_00100992 1 | 23355 | -2.06 | 1.55E-05 |
| | | | NM_015303 | | | |
| FOXP4 | 229763_at | BE504097 | NM_032204 | 116113 | -2.05 | 6.69E-05 |
| ASCC2 | | | NM_138457 | 84164 | | |
| ATF5 | 204998_s_at | NM_012068 | NM_012068 | 22809 | -2.05 | 1.57E-03 |
| AA452540 | 232411_at | AA452540 | --- | --- | -2.04 | 3.30E-03 |
| FNBP1 | 230389_at | BE046511 | NM_015033 | 23048 | -2.04 | 1.05E-03 |
| AU144437 | 232887_at | AU144437 | --- | --- | -2.04 | 2.07E-03 |
| IDS | 1569315_s_at | BC032484 | NM_000202 | 3423 | -2.03 | 1.16E-03 |
| | | | NM_006123 | | | |
| CSTB | 236449_at | AI885390 | NM_000100 | 1476 | -2.03 | 8.87E-05 |
| IGF2 | 210881_s_at | M17863 | NM_000612 | 3481 | -2.03 | 1.78E-04 |
| LOC51315 | 222585_x_at | BE326678 | NM_016618 | 51315 | -2.03 | 7.77E-03 |
| TRIM66 | 213748_at | AW271713 | XM_084529 | 9866 | -2.02 | 8.91E-03 |
| AA705933 | 239866_at | AA705933 | --- | --- | -2.02 | 3.47E-04 |
| AI691075 | 229728_at | AI691075 | --- | --- | -2.02 | 7.84E-03 |
| HIST1H2AE | 214469_at | NM_021052 | NM_021052 | 3012 | -2.02 | 1.08E-06 |
| LOC402617 | 241353_s_at | AW471181 | XM_380151 | 402617 | -2.02 | 4.90E-04 |
| SERPINE1 | 202627_s_at | AL574210 | NM_000602 | 5054 | -2.01 | 9.89E-06 |
| SND1 | 228271_at | AW001186 | NM_014390 | 27044 | -2.01 | 5.63E-03 |
| MIG-6 | 224657_at | AL034417 | NM_018948 | 54206 | -2.01 | 2.57E-04 |
| NM_015583 | 208015_at | NM_015583 | --- | --- | -2.01 | 1.80E-04 |
| SMYD2 | 212921_at | AF070592 | NM_020197 | 56950 | -2.01 | 1.62E-06 |
| S100A4 | 203186_s_at | NM_002961 | NM_002961 | 6275 | -2.01 | 6.07E-04 |
| | | | NM_019554 | | | |
| HIST2H2AA | 214290_s_at | AI313324 | NM_003516 | 8337 | -2.01 | 7.64E-04 |
| LOC360030 | 236404_at | AW197320 | --- | 360030 | -2.01 | 7.91E-03 |
| AL119491 | 244292_at | AL119491 | --- | --- | -2.00 | 8.84E-03 |
| FNBP1 | 230086_at | AA937109 | NM_015033 | 23048 | -2.00 | 6.01E-03 |
| LGALS3 | 208949_s_at | BC001120 | NM_002306 | 3958 | -1.99 | 1.10E-03 |
| GALIG | | | NM_194327 | 81625 | | |
| CPEB4 | 242384_at | AI452469 | NM_030627 | 80315 | -1.99 | 8.34E-03 |
| AA278901 | 244055_at | AA278901 | --- | --- | -1.99 | 1.31E-04 |
| NSMAF | 232148_at | BF056507 | NM_003580 | 8439 | -1.98 | 6.51E-03 |
| HIST3H2A | 221582_at | BC001193 | NM_033445 | 92815 | -1.98 | 3.01E-04 |
| FLJ21657 | 1557828_a_at | BE675061 | NM_022483 | 64417 | -1.98 | 5.30E-03 |
| EVER1 | 230526_at | BF515959 | NM_007267 | 11322 | -1.98 | 5.20E-08 |
| SLITRK3 | 206732_at | NM_014926 | NM_014926 | 22865 | -1.97 | 7.66E-05 |
| AW973410 | 244310_at | AW973410 | XM_498613 | 440283 | -1.97 | 3.35E-03 |
| ZC3HDC1 | 218543_s_at | NM_022750 | NM_022750 | 64761 | -1.97 | 2.63E-03 |
| GPR18 | 244791_at | AA835936 | NM_005292 | 2841 | -1.97 | 1.52E-03 |
| LOC283666 | 226682_at | AW006185 | --- | 283666 | -1.96 | 1.61E-04 |
| DUSP5 | 209457_at | U16996 | NM_004419 | 1847 | -1.96 | 3.92E-06 |
| AL048861 | 1559148_at | AL048861 | --- | --- | -1.96 | 2.90E-03 |
| PKD2 | 240370_at | AI432451 | NM_000297 | 5311 | -1.96 | 3.38E-04 |
| AI963079 | 236411_at | AI963079 | --- | --- | -1.96 | 1.65E-03 |
| AI758408 | 227235_at | AI758408 | --- | --- | -1.96 | 1.28E-03 |
| R63578 | 242818_x_at | R63578 | --- | --- | -1.95 | 2.36E-03 |
| AW189467 | 230183_at | AW189467 | --- | --- | -1.95 | 3.15E-03 |
| PNMA3 | 223621_at | AL136878 | NM_013364 | 29944 | -1.95 | 4.80E-07 |
| LOC286411 | 1557133_at | AV753446 | --- | 286411 | -1.94 | 4.84E-04 |
| RHOQ | 1559582_at | BC033251 | NM_012249 | 23433 | -1.93 | 1.51E-03 |
| AI817960 | 239649_at | AI817960 | --- | --- | -1.93 | 8.93E-03 |
| FLJ10359 | 232414_at | AA827672 | XM_375853 | 55127 | -1.92 | 2.24E-04 |
| C16orf45 | 239971_at | AI298297 | NM_033201 | 89927 | -1.92 | 5.46E-03 |
| MGC29875 | 220251_at | NM_024998 | NM_014388 | 27042 | -1.92 | 3.38E-03 |
| FLJ10159 | 1563906_at | AK090879 | --- | 55084 | -1.92 | 4.66E-03 |
| FLJ10159 | 236602_at | AA833813 | --- | 55084 | -1.92 | 2.30E-04 |
| FAT4 | 219427_at | NM_024582 | NM_024582 | 79633 | -1.92 | 5.03E-03 |
| MGC45477 | 1554250_s_at | BC033812 | --- | 387277 | -1.92 | 2.59E-03 |
| ARMCX4 | 1552327_at | NM_152583 | NM_152583 | 158947 | -1.91 | 9.32E-07 |
| IGF2 | 202410 _x_at | NM_000612 | NM_000612 | 3481 | -1.91 | 1.38E-04 |
| PRR3 | 244204_at | W87300 | NM_025263 | 80742 | -1.91 | 2.49E-03 |
| FLJ20152 | 218510 _x_at | AI816291 | NM_019000 | 54463 | -1.90 | 5.85E-04 |
| PCBD2 | 240846_at | BF223271 | NM_032151 | 84105 | -1.90 | 3.18E-05 |
| C6orf198 | 227834_at | AL589605 | XM_371849 | 167838 | -1.90 | 5.03E-03 |
| MTX3 | 237681_at | AA699464 | NM_001010891 | 345778 | -1.89 | 3.91E-03 |
| BF514509 | 239049_at | BF514509 | --- | --- | -1.89 | 9.85E-04 |
| C6orf148 | 237347_at | AL039379 | NM_030568 | 80759 | -1.89 | 9.86E-05 |
| KIAA1904 | 1559072_a_at | BC032083 | NM_052906 | 114794 | -1.88 | 2.81E-04 |
| C6orf154 | 235935_at | AI539370 | XM_168060 | 221424 | -1.88 | 2.76E-04 |
| ATP2B3 | 242036_x_at | H09073 | NM_00100134 4 | 492 | -1.88 | 2.12E-05 |
| | | | NM_021949 | | | |
| BF433341 | 235977_at | BF433341 | --- | --- | -1.88 | 2.48E-05 |
| TMP21 | 239851_at | AW629289 | NM_006827 | 10972 | -1.88 | 5.43E-04 |
| GFM1 | 232295_at | AK000780 | NM_024996 | 85476 | -1.88 | 3.27E-03 |
| AAK1 | 214956_at | AF090101 | NM_014911 | 22848 | -1.87 | 3.23E-03 |
| LOC283439 | 238636_at | BG430061 | --- | 283439 | -1.87 | 2.24E-03 |
| FTHFSDC1 | 240552_at | AA811452 | NM_015440 | 25902 | -1.87 | 1.82E-03 |
| AW296421 | 230064_at | AW296421 | --- | --- | -1.87 | 6.51E-03 |
| PAPPA | 240295_at | AL045014 | NM_002581 | 5069 | -1.87 | 7.85E-05 |
| LOC55831 | 215250_at | AU147317 | NM_018447 | 55831 | -1.87 | 3.85E-03 |
| LOC253982 | 238142_at | AW029203 | NM_181718 | 253982 | -1.86 | 8.13E-03 |
| | | | NM_198907 | | | |
| NKTR | 243055_at | BF514072 | NM_005385 | 4820 | -1.86 | 2.53E-03 |
| AW294722 | 240246_at | AW294722 | --- | --- | -1.86 | 7.82E-03 |
| AK023394 | 232107_at | AK023394 | --- | --- | -1.86 | 4.70E-05 |
| VPS13C | 235023_at | AA828371 | NM_017684 | 54832 | -1.85 | 5.51E-03 |
| RHBDL1 | 207383_s_at | NM_003961 | NM_003961 | 9028 | -1.85 | 1.70E-04 |
| C21orf4 | 219600_s_at | NM_006134 | NM_006134 | 757 | -1.85 | 1.43E-03 |
| TPM3 | 238079_at | AV713323 | NM_152263 | 7170 | -1.85 | 1.29E-03 |
| | | | NM_153649 | | | |
| BACE2 | 217867_x_at | NM_012105 | NM_012105 | 25825 | -1.85 | 1.57E-03 |
| | | | NM_138991 | | | |
| | | | NM_138992 | | | |
| VEGF | 212171_x_at | H95344 | NM_003376 | 7422 | -1.84 | 1.68E-03 |
| ID2 | 213931_at | AI819238 | NM_002166 | 3398 | -1.84 | 3.39E-03 |
| LOC51315 | 218303_x_at | NM_016618 | NM_016618 | 51315 | -1.84 | 5.97E-03 |
| TMIE | 1553601_a_at | NM_147196 | NM_147196 | 259236 | -1.83 | 8.89E-04 |
| TM4SF2 | 234245_at | AL162055 | NM_004615 | 7102 | -1.83 | 7.61E-03 |
| FADS3 | 216080_s_at | AC004770 | NM_021727 | 3995 | -1.83 | 1.05E-04 |
| SNF1LK | 208078_s_at | NM_030751 | NM_173354 | 150094 | -1.83 | 2.37E-03 |
| FGD5 | 226985_at | AW269340 | NM_152536 | 152273 | -1.83 | 1.69E-03 |
| ATP2B3 | 207026_s_at | NM_021949 | NM_00100134 4 | 492 | -1.83 | 1.63E-04 |
| | | | NM_021949 | | | |
| LOC51315 | 233329_s_at | AK025986 | NM_016618 | 51315 | -1.83 | 5.27E-03 |
| FLJ00310 | 1569664_at | BC035915 | XM_496691 | 375010 | -1.82 | 5.02E-04 |
| FNBP1 | 244286_at | AI017983 | NM_015033 | 23048 | -1.82 | 1.10E-03 |
| ASAM | 228082_at | BF056275 | NM_024769 | 79827 | -1.82 | 4.85E-03 |
| VN1R1 | 221412_at | NM_020633 | NM_020633 | 57191 | -1.82 | 4.74E-05 |
| LOC124220 | 228058_at | AI559190 | NM_145252 | 124220 | -1.82 | 5.52E-05 |
| FLJ23754 | 1553248_at | NM_152675 | NM_152675 | 201252 | -1.81 | 6.36E-03 |
| HSD17B1 | 205829_at | NM_000413 | NM_000413 | 3292 | -1.81 | 5.39E-05 |
| ZNF407 | 233239_at | AK024882 | NM_017757 | 55628 | -1.81 | 4.86E-03 |
| ARTS-1 | 209788_s_at | AF183569 | NM_016442 | 51752 | -1.81 | 5.75E-03 |
| GBE1 | 203282_at | NM_000158 | NM_000158 | 2632 | -1.81 | 2.17E-03 |
| LONP | 221834_at | AV700132 | NM_031490 | 83752 | -1.81 | 7.26E-03 |
| SFXN3 | 217226_s_at | M95929 | NM_030971 | 81855 | -1.80 | 4.81E-04 |
| AK021973 | 234098_at | AK021973 | --- | --- | -1.80 | 1.25E-03 |
| AA682539 | 235575_at | AA682539 | --- | --- | -1.80 | 5.79E-03 |
| AI950023 | 244433_at | AI950023 | --- | --- | -1.80 | 3.52E-03 |
| C13orf22 | 1562238_at | AL832776 | NM_005800 | 10208 | -1.80 | 5.58E-04 |
| AI828075 | 230795_at | AI828075 | --- | --- | -1.79 | 7.39E-05 |
| AW172407 | 240908_at | AW172407 | XM_373053 | 391733 | -1.79 | 1.54E-03 |
| ARG2 | 203946_s_at | U75667 | NM_001172 | 384 | -1.79 | 8.38E-05 |
| A1582773 | 214373_at | AI582773 | XM_498956 | 440996 | -1.79 | 6.73E-03 |
| C1orf21 | 221272_s_at | NM_030806 | NM_030806 | 81563 | -1.79 | 1.58E-06 |
| BF431902 | 228469_at | BF431902 | --- | --- | -1.79 | 8.24E-03 |
| AI732874 | 241314_at | AI732874 | --- | --- | -1.79 | 6.26E-03 |
| AI521273 | 241702_at | AI521273 | --- | --- | -1.79 | 2.16E-03 |
| SFXN3 | 220974_x_at | NM_030971 | NM_030971 | 81855 | -1.78 | 5.28E-05 |
| AA394039 | 229720_at | AA394039 | --- | --- | -1.78 | 3.12E-04 |
| AI765607 | 244631_at | AI765607 | XM_374318 | 389834 | -1.78 | 7.00E-03 |
| PHACTR1 | 238297_at | AI884781 | NM_030948 | 221692 | -1.78 | 6.75E-03 |
| BF000047 | 235736_at | BF000047 | --- | --- | -1.78 | 2.66E-03 |
| PPP1R7 | 1559054_a_at | BC027905 | NM_002712 | 5510 | -1.78 | 8.88E-04 |
| SRP54 | 1557504_at | AI076351 | NM_003136 | 6729 | -1.77 | 3.30E-03 |
| MLLT2 | 237006_at | AA703523 | NM_005935 | 4299 | -1.77 | 1.07E-03 |
| KIAA1276 | 235288_at | AI627532 | XM_039169 | 27146 | -1.77 | 3.48E-04 |
| LOC360030 | 1565804_at | AK025161 | --- | 360030 | -1.77 | 7.53E-03 |
| PAQR6 | 219236_at | NM_024897 | NM_024897 | 79957 | -1.77 | 1.41E-03 |
| | | | NM_198406 | | | |
| KIAA2024 | 1566166_at | AL833423 | NM_172070 | 130507 | -1.77 | 1.09E-03 |
| FLJ20152 | 218532_s_at | NM_019000 | NM_019000 | 54463 | -1.77 | 2.47E-03 |
| ZAP128 | 202982_s_at | NM_006821 | NM_006821 | 10965 | -1.77 | 2.18E-03 |
| Al150690 | 228466_at | AI150690 | --- | --- | -1.76 | 1.91E-03 |
| AK098781 | 1560814_a_at | AK098781 | --- | --- | -1.76 | 9.35E-06 |
| FLJ11267 | 220216_at | NM_019607 | NM_019607 | 56260 | -1.76 | 2.56E-03 |
| CPEB1 | 219578_s_at | NM_030594 | NM_030594 | 64506 | -1.76 | 3.75E-03 |
| NIT1 | 202891_at | NM_005600 | NM_005600 | 4817 | -1.76 | 4.55E-04 |
| ZDHHC11 | 221646_s_at | AF267859 | NM_024786 | 79844 | -1.76 | 1.39E-04 |
| LASS5 | 1556783_a_at | AF088003 | NM_147190 | 91012 | -1.76 | 4.78E-03 |
| FLJ13231 | 1562387_at | BU189724 | NM_023073 | 65250 | -1.76 | 4.71E-03 |
| GNRHR | 1568661_at | BC039430 | NM_000406 | 2798 | -1.76 | 1.78E-03 |
| MDH1B | 1558077_s_at | BG202523 | NM_206892 | 130752 | -1.75 | 2.90E-03 |
| HIST1H3E | 214616_at | NM_003532 | NM_003532 | 8353 | -1.75 | 2.78E-05 |
| PHLDA2 | 209803_s_at | AF001294 | NM_003311 | 7262 | -1.75 | 1.03E-03 |
| GRM3 | 240004_at | BF436080 | NM_000840 | 2913 | -1.74 | 5.05E-03 |
| ABI1 | 238563_at | AV762916 | NM_005470 | 10006 | -1.74 | 2.03E-03 |
| RAB11FIP3 | 1563104_at | BC009036 | NM_014700 | 9727 | -1.74 | 2.52E-03 |
| C6orf52 | 236504_x_at | BE670432 | XM_371798 | 347744 | -1.74 | 5.49E-03 |
| AV658469 | 244699_at | AV658469 | --- | --- | -1.74 | 6.93E-07 |
| PLXNC1 | 206470_at | NM_005761 | NM_005761 | 10154 | -1.74 | 1.25E-03 |
| NTNG2 | 233072_at | AI348745 | NM_032536 | 84628 | -1.74 | 2.30E-03 |
| AMH | 206516_at | NM_000479 | NM_000479 | 268 | -1.73 | 4.35E-04 |
| AI814750 | 1565692_at | AI814750 | --- | --- | -1.73 | 7.35E-03 |
| NAG8 | 210109_at | AF191492 | NM_014411 | 27099 | -1.73 | 8.58E-04 |
| PLXNC1 | 206471_s_at | NM_005761 | NM_005761 | 10154 | -1.73 | 1.82E-03 |
| LOC283861 | 1554520_at | BC009880 | --- | 283861 | -1.73 | 1.36E-03 |
| HNRPM | 230392_at | AW298141 | NM_005968 | 4670 | -1.72 | 2.74E-04 |
| | | | NM_031203 | | | |
| AI802997 | 235211_at | AI802997 | --- | --- | -1.72 | 4.84E-03 |
| APOC1 | 204416_x_at | NM_001645 | NM_001645 | 341 | -1.72 | 7.65E-05 |
| ADCY1 | 213245_at | AL120173 | NM_021116 | 107 | -1.71 | 4.24E-03 |
| AF131805 | 233581_at | AF131805 | --- | --- | -1.71 | 2.73E-04 |
| B3GNT5 | 225612_s_at | BE672260 | NM_032047 | 84002 | -1.71 | 9.32E-03 |
| ANK2 | 202920_at | BF726212 | NM_001148 | 287 | -1.71 | 6.21E-03 |
| | | | NM_020977 | | | |
| TTC3 | 1569472_s_at | BC026260 | NM_00100189 4 | 7267 | -1.71 | 1.33E-03 |
| | | | NM_003316 | | | |
| HSPD1 | 241716_at | BF965447 | NM_002156 | 3329 | -1.71 | 1.25E-04 |
| | | | NM_199440 | | | |
| RHOU | 223168_at | AL096776 | NM_021205 | 58480 | -1.71 | 1.77E-05 |
| AI733281 | 239994_at | AI733281 | --- | --- | -1.71 | 1.76E-03 |
| BC015455 | 1569724_at | BC015455 | --- | --- | -1.71 | 1.78E-04 |
| RGS16 | 209324_s_at | BF304996 | NM_002928 | 6004 | -1.71 | 4.44E-03 |
| LOC285535 | 229130_at | AU145323 | --- | 285535 | -1.71 | 3.49E-03 |
| PDIP | 206691_s_at | NM_006849 | NM_006849 | 64714 | -1.71 | 2.28E-08 |
| BE221330 | 241624_at | BE221330 | XM_374318 | 389834 | -1.71 | 4.44E-03 |
| BF434212 | 237942_at | BF434212 | --- | --- | -1.71 | 2.50E-03 |
| FLJ14966 | 243611_at | BG231646 | NM_032867 | 84953 | -1.71 | 4.23E-03 |
| MGC10850 | 223707_at | BC004284 | --- | 84736 | -1.71 | 9.45E-04 |
| H15900 | 239833_at | H15900 | --- | --- | -1.71 | 4.92E-05 |
| SPPL3 | 1566897_at | AL834399 | NM_139015 | 121665 | -1.71 | 1.54E-04 |
| LOC256273 | 229466_at | AU144187 | --- | 256273 | -1.70 | 7.69E-03 |
| AI288679 | 242969_at | AI288679 | --- | --- | -1.70 | 1.70E-04 |
| LOC400988 | 230941_at | AI651340 | XM_379112 | 400988 | -1.70 | 2.26E-05 |
| MYO15B | 227743_at | AI671062 | XR_000222 | 80022 | -1.70 | 5.37E-03 |
| FLJ10159 | 229034_at | BF511724 | --- | 55084 | -1.70 | 1.89E-03 |
| ZNF42 | 40569_at | M58297 | NM_003422 | 7593 | -1.70 | 8.99E-05 |
| | | | NM_198055 | | | |
| TMP21 | 238886_at | BF056141 | NM_006827 | 10972 | -1.70 | 3.75E-03 |
| CAMK2B | 209956_s_at | U23460 | NM_001220 | 816 | -1.69 | 2.99E-03 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| SCFD1 | 233229_at | AU147042 | NM_016106 | 23256 | -1.69 | 1.27E-04 |
| | | | NM_182835 | | | |
| ERMAP | 219905_at | NM_018538 | NM_018538 | 114625 | -1.69 | 9.14E-06 |
| AI190292 | 240190_at | AI190292 | --- | --- | -1.69 | 6.34E-04 |
| VEGF | 210512_s_at | AF022375 | NM_003376 | 7422 | -1.69 | 7.38E-04 |
| DNAJB11 | 223054_at | BC001144 | NM_016306 | 51726 | -1.69 | 6.40E-03 |
| LOC401320 | 239313_at | AI803568 | XM_379482 XM_380098 | 401320 | -1.69 | 6.47E-03 |
| AL359574 | 234588_at | AL359574 | --- | --- | -1.68 | 5.27E-03 |
| GOPC | 236862_at | AA279958 | NM_020399 | 57120 | -1.68 | 4.91E-05 |
| MAP3K5 | 203836_s_at | D84476 | NM_005923 | 4217 | -1.68 | 3.70E-04 |
| AW118878 | 230736_at | AW118878 | XM_373451 | 387647 | -1.68 | 2.03E-04 |
| SLC7A1 | 215401_at | AU147698 | NM_003045 | 6541 | -1.68 | 3.38E-03 |
| PDE4DIP | 244511_at | AV700591 | NM_00100281 0 | 9659 | -1.68 | 1.87E-04 |
| | | | NM_00100281 1 | | | |
| | | | NM_00100281 2 | | | |
| | | | NM_014644 | | | |
| | | | NM_022359 | | | |
| BLCAP | 1557521_a_at | AA653638 | NM_006698 | 10904 | -1.68 | 7.83E-03 |
| PNCK | 229411_at | AI986390 | NM_198452 | 139728 | -1.68 | 1.49E-08 |
| TUSC3 | 232770_at | AK026149 | NM_006765 | 7991 | -1.67 | 3.31E-04 |
| | | | NM_178234 | | | |
| FBXW7 | 222729_at | BE551877 | NM_018315 | 55294 | -1.67 | 6.18E-06 |
| | | | NM_033632 | | | |
| DKFZP564O046 | 220843_s_at | NM_014156 | NM_015420 | 25879 | -1.67 | 4.38E-03 |
| FYN | 243006_at | BG222258 | NM_002037 | 2534 | -1.67 | 1.63E-03 |
| | | | NM_153047 | | | |
| | | | NM_153048 | | | |
| PDGFA | 205463_s_at | NM_002607 | --- | 5154 | -1.67 | 1.01E-03 |
| ST7L | 216123_x_at | AK024158 | NM_017744 | 54879 | -1.67 | 2.23E-03 |
| | | | NM_138727 | | | |
| | | | NM_138728 | | | |
| | | | NM_138729 | | | |
| | | | NM_198327 | | | |
| | | | NM_198328 | | | |
| ZNF42 | 204138_s_at | AI762174 | NM_003422 NM_198055 | 7593 | -1.67 | 1.08E-03 |
| FNBP1 | 239453_at | AA262084 | NM_015033 | 23048 | -1.67 | 5.15E-04 |
| GNAS | 214157_at | AA401492 | NM_000516 | 2778 | -1.67 | 1.44E-03 |
| | | | NM_016592 | | | |
| | | | NM_080425 | | | |
| | | | NM_080426 | | | |
| BRD4 | 232551_at | AA521443 | NM_014299 | 23476 | -1.67 | 8.62E-04 |
| | | | NM_058243 | | | |
| FLJ10287 | 232489_at | AK001149 | NM_019083 | 54482 | -1.67 | 4.17E-03 |
| TRIM2 | 214249_at | AK000179 | NM_015271 | 23321 | -1.66 | 3.49E-03 |
| AK021423 | 232890_at | AK021423 | --- | --- | -1.66 | 1.20E-03 |
| FARSLB | 223036_at | D84430 | NM_005687 | 10056 | -1.66 | 2.56E-03 |
| BE891071 | 1569147_at | BE891071 | XM_496749 | 441078 | -1.66 | 2.82E-03 |
| PRKACB | 235780_at | BE622723 | NM_002731 | 5567 | -1.66 | 3.63E-04 |
| | | | NM_182948 | | | |
| | | | NM_207578 | | | |
| SLC35D3 | 231341_at | BE670584 | NM_001008783 | 340146 | -1.66 | 8.93E-03 |
| N92599 | 241421_at | N92599 | --- | --- | -1.66 | 2.32E-03 |
| DR1 | 227074_at | AA524669 | NM_001938 | 1810 | -1.66 | 1.72E-03 |
| KIAA0657 | 227573_s_at | BF446688 | XM_051017 | 23363 | -1.66 | 3.36E-03 |
| SLCO1A2 | 207308_at | NM_021094 | NM_005075 | 6579 | -1.65 | 5.88E-03 |
| | | | NM_021094 | | | |
| | | | NM_134431 | | | |
| HNRPD | 236000_s_at | AA863112 | NM_00100381 0 | 3184 | -1.65 | 1.29E-03 |
| | | | NM_002138 | | | |
| | | | NM_031369 | | | |
| | | | NM_031370 | | | |
| LOC54103 | 222150_s_at | AK026747 | XM_379881 | 54103 | -1.65 | 2.42E-03 |
| | | | XM_499340 | | | |
| RORA | 1562529_s_at | BC040965 | NM_002943 | 6095 | -1.65 | 6.52E-04 |
| | | | NM_134260 | | | |
| | | | NM_134261 | | | |
| | | | NM_134262 | | | |
| HUMRGE/M100UMRGE/M1009-HUMRGE/M1009 | | | --- | --- | -1.65 | 3.90E-03 |
| FLJ44968 | 229961_x_at | AI871270 | NM_198537 | 374887 | -1.65 | 6.71E-04 |
| ARIH1 | 1558710_at | BI791845 | NM_005744 | 25820 | -1.64 | 1.02E-03 |
| GPX7 | 213170_at | AA406605 | NM_015696 | 2882 | -1.64 | 8.64E-04 |
| ERBB4 | 206794_at | NM_005235 | NM_005235 | 2066 | -1.64 | 1.93E-05 |
| LOC57228 | 209679_s_at | BC003379 | NM_020467 | 57228 | -1.64 | 4.85E-04 |
| UBE2D3 | 233303_at | AL110175 | NM_003340 | 7323 | -1.64 | 3.36E-03 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NMJ81893 | | | |
| AK000995 | 215349_at | AK000995 | --- | --- | -1.64 | 4.56E-03 |
| N70015 | 236510_at | N70015 | --- | --- | -1.64 | 1.10E-03 |
| BC031957 | 1565672_at | BC031957 | XM_498823 | 440703 | -1.63 | 8.75E-03 |
| FLJ36674 | 228482_at | AV702789 | NM_173622 | 284040 | -1.63 | 1.14E-03 |
| MGC43690 | 235576_at | AW474542 | NM_182552 | 253769 | -1.63 | 3.31E-12 |
| AU153281 | 231937_at | AU153281 | --- | --- | -1.63 | 1.84E-05 |
| AI807627 | 239734_at | AI807627 | --- | --- | -1.63 | 1.25E-03 |
| SLC4A3 | 205918_at | NM_005070 | NM_005070 | 6508 | -1.63 | 6.65E-04 |
| | | | NM_201574 | | | |
| NME5 | 206197_at | NM_003551 | NM_003551 | 8382 | -1.63 | 2.24E-03 |
| RGS16 | 209325_s_at | U94829 | NM_002928 | 6004 | -1.63 | 8.91E-03 |
| H46176 | 237083_at | H46176 | --- | --- | -1.62 | 2.84E-04 |
| CDKN2A | 207039_at | NM_000077 | NM_000077 | 1029 | -1.62 | 4.13E-03 |
| | | | NM_058195 | | | |
| | | | NM_058197 | | | |
| GLT25D2 | 209883_at | AF288389 | NM_015101 | 23127 | -1.62 | 6.05E-05 |
| BF002625 | 229530_at | BF002625 | --- | --- | -1.62 | 1.45E-03 |
| RIPK1 | 209941_at | U50062 | NM_003804 | 8737 | -1.62 | 6.97E-05 |
| FLJ45880 | 228538_at | BE671164 | NM_207404 | 389114 | -1.62 | 5.47E-06 |
| LOC286161 | 228615_at | AW291761 | --- | 286161 | -1.62 | 3.70E-03 |
| FLJ32096 | 1555870_at | AK056658 | NM_173795 | 148646 | -1.62 | 9.30E-03 |
| NUP54 | 215213_at | AU146949 | NM_017426 | 53371 | -1.61 | 3.52E-07 |
| DKFZp313N062 | 1555906_s_at | AI147556 | NM_173826 | 285343 | -1.61 | 1.21E-04 |
| GOLGIN-67 | 213650_at | AW006438 | NM_015003 | 23015 | -1.61 | 4.39E-03 |
| | | | NM_181076 | | | |
| | | | NM_181077 | | | |
| AHI1 | 221569_at | AL136797 | NM_017651 | 54806 | -1.61 | 1.18E-03 |
| PLXNC1 | 213241_at | AF035307 | NM_005761 | 10154 | -1.61 | 1.31E-03 |
| AMY2A | 208498_s_at | NM_004038 | NM_000699 | 279 | -1.61 | 1.12E-05 |
| C9orf41 | 1564690_at | BC022435 | NM_152420 | 138199 | -1.60 | 3.52E-05 |
| MGC29898 | 235259_at | AA398590 | NM_145048 | 133015 | -1.60 | 1.34E-04 |
| AV700050 | 228835_at | AV700050 | --- | --- | -1.60 | 3.88E-05 |
| LGALS3BP | 200923_at | NM_005567 | NM_005567 | 3959 | -1.60 | 1.65E-06 |
| CRYZL1 | 243091_at | BF435599 | NM_005111 | 9946 | -1.60 | 1.91E-03 |
| | | | NM_145311 | | | |
| | | | NM_145858 | | | |
| ZDHHC11 | 1552283_s_at | NM_024786 | NM_024786 | 79844 | -1.60 | 2.31E-05 |
| CENTG2 | 233225_at | AK000798 | NM_014914 | 116987 | -1.59 | 6.55E-03 |
| CLCN3 | 240237_at | H23230 | NM_001829 | 1182 | -1.59 | 6.03E-03 |
| | | | NM_173872 | | | |
| WASL | 216094_at | AK025323 | NM_003941 | 8976 | -1.59 | 7.27E-03 |
| CDK3 | 229468_at | AI885421 | NM_001258 | 1018 | -1.59 | 7.50E-06 |
| PRKWNK1 | 216022_at | AL049278 | NM_018979 | 65125 | -1.59 | 2.19E-03 |
| CALR | 214315_x_at | AI348935 | NM_004343 | 811 | -1.59 | 3.10E-03 |
| NM_018289 | 219794_at | NM_018289 | --- | --- | -1.59 | 2.26E-07 |
| LOC392790 | 243171_at | AA570178 | XM_374751 | 392790 | -1.58 | 5.98E-03 |
| AA166617 | 230198_at | AA166617 | --- | --- | -1.58 | 3.03E-03 |
| TP53BP1 | 1569098_s_at | BU508386 | NM_005657 | 7158 | -1.58 | 5.76E-04 |
| C21orf66 | 240105_at | A1021902 | NM_013329 | 94104 | -1.58 | 4.09E-03 |
| | | | NM_016631 | | | |
| | | | NM_058191 | | | |
| | | | NM_145328 | | | |
| BC014200 | 1570061_at | BC014200 | --- | --- | -1.58 | 7.06E-05 |
| KIAA1045 | 213636_at | AB028968 | XM_048592 | 23349 | -1.57 | 2.94E-03 |
| AK024175 | 222152_at | AK024175 | XM_373053 | 391733 | -1.57 | 8.76E-03 |
| DDHD1 | 231165_at | BE857355 | NM_030637 | 80821 | -1.57 | 1.47E-03 |
| DNAH5 | 243938_x_at | AI872645 | NM_001369 | 1767 | -1.57 | 4.06E-03 |
| LSS | 211018_at | D63807 | NM_00100143 8 | 4047 | -1.57 | 2.49E-03 |
| | | | NM_002340 | | | |
| BC026294 | 1569510_at | BC026294 | --- | --- | -1.57 | 3.20E-06 |
| FLJ32954 | 235349_at | AI261321 | NM_144713 | 151393 | -1.57 | 7.97E-05 |
| ISG20 | 33304_at | U88964 | NM_002201 | 3669 | -1.56 | 1.82E-03 |
| GPR89 | 1562412_at | BC020562 | NM_016334 | 51463 | -1.56 | 5.13E-05 |
| AL122039 | 234260_at | AL122039 | --- | --- | -1.56 | 3.46E-03 |
| ARSD | 225280_x_at | N51673 | NM_001669 | 414 | -1.56 | 3.54E-03 |
| | | | NM_009589 | | | |
| MKLN1 | 233219_at | AK023427 | NM_013255 | 4289 | -1.56 | 7.04E-03 |
| LOC91526 | 228471_at | AA744636 | NM_153697 | 91526 | -1.56 | 1.49E-03 |
| PKD1 | 216949_s_at | L39891 | NM_000296 | 5310 | -1.56 | 4.32E-05 |
| | | | NM_00100994 4 | | | |
| FLJ12666 | 215029_at | AL117451 | NM_024595 | 79647 | -1.56 | 2.57E-03 |
| BF512871 | 240174_at | BF512871 | XM_373962 | 388903 | -1.55 | 2.49E-04 |
| MGC13098 | 222208_s_at | W07700 | XM_379817 | 84820 | -1.55 | 3.85E-03 |
| | | | XM_499280 | | | |
| AI271434 | 243668_at | AI271434 | --- | --- | -1.55 | 7.05E-03 |
| CCPG1 | 221511_x_at | AB033080 | NM_004748 | 9236 | -1.55 | 8.33E-03 |
| | | | NM_020739 | | | |
| ENOSF1 | 204143_s_at | NM_017512 | NM_017512 | 55556 | -1.55 | 5.87E-03 |
| ARG2 | 203945_at | NM_001172 | NM_001172 | 384 | -1.55 | 3.00E-03 |
| AA583038 | 238498_at | AA583038 | --- | --- | -1.55 | 5.09E-04 |
| BG548427 | 235547_at | BG548427 | --- | --- | -1.55 | 1.16E-03 |
| LOC338620 | 230930_at | AI435839 | --- | 338620 | -1.55 | 3.00E-03 |
| LOC286208 | 1560089_at | AL833509 | XM_379668 | 286208 | -1.54 | 2.16E-03 |
| AA504346 | 242170_at | AA504346 | --- | --- | -1.54 | 7.12E-03 |
| T77543 | 237530_at | T77543 | --- | --- | -1.54 | 9.66E-04 |
| MLSTD1 | 1557224_at | BM682057 | NM_018099 | 55711 | -1.54 | 4.35E-03 |
| SIGIRR | 52940_at | AA085764 | NM_021805 | 59307 | -1.54 | 4.74E-03 |
| T91323 | 241093_at | T91323 | NM_015129 | 23157 | -1.54 | 8.22E-03 |
| | | | NM_145799 | | | |
| | | | NM_145800 | | | |
| | | | NM_145802 | | | |
| GRM8 | 1556800_a_at | BC041397 | NM_000845 | 2918 | -1.54 | 5.54E-05 |
| ILK | 235906_at | BE962235 | NM_004517 | 3611 | -1.54 | 5.88E-04 |
| LOC284408 | 1558809_s_at | AK094324 | --- | 284408 | -1.53 | 2.05E-03 |
| PTPN23 | 1569104_a_at | BE646227 | NM_015466 | 25930 | -1.53 | 9.23E-04 |
| N36832 | 226396_at | N36832 | --- | --- | -1.53 | 1.87E-03 |
| CALR | 212953_x_at | BE251303 | NM_004343 | 811 | -1.53 | 1.01E-03 |
| HIST1H2BN | 239041_at | AV747226 | NM_003520 | 8341 | -1.53 | 9.40E-03 |
| KLF6 | 211610_at | U51869 | NM_00100849 0 | 1316 | -1.53 | 7.45E-04 |
| | | | NM_001300 | | | |
| TTC17 | 232323_s_at | AK026217 | NM_018259 | 55761 | -1.53 | 2.27E-05 |
| HBS1L | 209315_at | AW297143 | NM_006620 | 10767 | -1.53 | 3.94E-03 |
| AK024879 | 216751_at | AK024879 | --- | --- | -1.53 | 7.76E-03 |
| WDFY3 | 238660_at | AI732512 | NM_014991 | 23001 | -1.53 | 4.22E-05 |
| | | | NM_178583 | | | |
| | | | NM_178585 | | | |
| FLJ20719 | 230712_at | AI634549 | XM_373827 | 55672 | -1.52 | 3.20E-03 |
| FLJ43663 | 243716_at | AI140617 | XM_379520 | 378805 | -1.52 | 8.90E-03 |
| | | | XM_380146 | | | |
| AKAP9 | 215483_at | AK000270 | NM_005751 | 10142 | -1.52 | 1.39E-03 |
| | | | NM_147166 | | | |
| | | | NM_147171 | | | |
| | | | NM_147185 | | | |
| AL572553 | 241395_at | AL572553 | --- | --- | -1.52 | 4.62E-03 |
| ZNF24 | 242210_at | AA749167 | NM_006965 | 7572 | -1.52 | 5.33E-03 |
| BE503598 | 1557658_at | BE503598 | --- | --- | -1.52 | 5.96E-04 |
| LOC440078 | 1570397_x_at | BC034777 | XM_498534 | 440078 | -1.52 | 1.46E-03 |
| BLZF1 | 1558560_s_at | AI002966 | NM_003666 | 8548 | -1.51 | 3.05E-08 |
| KCNK3 | 235108_at | BG105700 | NM_002246 | 3777 | -1.51 | 1.92E-03 |
| EVER1 | 204328_at | NM_007267 | NM_007267 | 11322 | -1.51 | 7.60E-03 |
| LOC285989 | 235231_at | AI355709 | NM_213603 | 285989 | -1.51 | 9.23E-03 |
| AW665096 | 214188_at | AW665096 | --- | --- | -1.51 | 1.36E-03 |
| LTBP3 | 219922_s_at | NM_021070 | NM_021070 | 4054 | -1.51 | 8.21E-06 |
| H2BFS | 208579_x_at | NM_017445 | NM_017445 | 54145 | -1.51 | 3.28E-04 |
| PGAP1 | 220576_at | NM_024989 | NM_024989 | 80055 | -1.51 | 4.78E-04 |
| ACTN4 | 232058_at | AU158358 | NM_004924 | 81 | -1.51 | 6.69E-03 |
| FGD5 | 226984_at | H09596 | NM_152536 | 152273 | -1.50 | 8.21E-03 |
| HPCAL4 | 219671_at | AL136591 | NM_016257 | 51440 | -1.50 | 7.58E-05 |
| ZNF395 | 232694_at | AK021850 | NM_018660 | 55893 | -1.50 | 1.30E-05 |
| RBM25 | 1557081_at | AA580691 | NM_021239 | 58517 | -1.50 | 1.01E-03 |
| HPCAL1 | 205462_s_at | NM_002149 | NM_002149 | 3241 | -1.50 | 9.84E-04 |
| | | | NM_134421 | | | |
| KIAA0657 | 227574_at | BF446688 | XM_051017 | 23363 | -1.50 | 3.00E-03 |
| LOC441168 | 229390_at | AV734646 | XM_496823 | 441168 | -1.49 | 9.36E-03 |
| AW241864 | 214353_at | AW241864 | --- | --- | -1.49 | 4.23E-03 |
| PTPRN | 204945_at | NM_002846 | NM_002846 | 5798 | -1.49 | 4.07E-04 |
| N75937 | 240072_at | N75937 | --- | --- | -1.49 | 2.94E-03 |
| FLJ10404 | 243047_at | AV698751 | NM_019057 | 54540 | -1.48 | 2.74E-03 |
| LOC400729 | 243021_at | AI344101 | XM_378824 | 400729 | -1.48 | 9.58E-03 |
| LOC440745 | | | XM_498840 | 440745 | | |
| AV709727 | 225996_at | AV709727 | --- | --- | -1.48 | 1.09E-03 |
| AU159412 | 216029_at | AU159412 | --- | --- | -1.48 | 8.93E-03 |
| AU146999 | 215986_at | AU146999 | --- | --- | -1.48 | 7.62E-05 |
| MAP3K5 | 203837_at | NM_005923 | NM_005923 | 4217 | -1.48 | 5.81E-04 |
| AW300500 | 228907 _at | AW300500 | --- | --- | -1.48 | 3.51E-04 |
| SORCS1 | 228194_s_at | AI675836 | NM_052918 | 114815 | -1.48 | 9.87E-06 |
| UBE2D3 | 242403_at | AI459177 | NM_003340 | 7323 | -1.48 | 3.32E-04 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| CDK3 | 207188_at | NM_001258 | NM_001258 | 1018 | -1.48 | 1.93E-05 |
| UBE2D3 | 240383_at | AI239832 | NM_003340 | 7323 | -1.48 | 1.55E-03 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| PRKACB | 202741_at | AA130247 | NM_002731 | 5567 | -1.47 | 3.58E-03 |
| | | | NM_182948 | | | |
| | | | NM_207578 | | | |
| AA868193 | 244031_at | AA868193 | --- | --- | -1.47 | 1.71E-03 |
| RCN1 | 201063_at | NM_002901 | NM_002901 | 5954 | -1.47 | 7.39E-03 |
| FLJ10378 | 242153_at | AW264125 | NM_018078 | 55132 | -1.47 | 7.26E-03 |
| | | | NM_032239 | | | |
| | | | NM_178043 | | | |
| THRAP2 | 216109_at | AK025348 | NM_015335 | 23389 | -1.47 | 2.04E-04 |
| HIAT1 | 242374_at | AA747563 | NM_033055 | 64645 | -1.47 | 2.87E-03 |
| BE552011 | 230208_at | BE552011 | --- | --- | -1.47 | 1.34E-04 |
| BG396868 | 1559957 _a_at | BG396868 | --- | --- | -1.47 | 8.59E-04 |
| SMURF2 | 241900_at | AW195928 | NM_022739 | 64750 | -1.47 | 3.53E-05 |
| SERP1 | 235475_at | AI580135 | NM_014445 | 27230 | -1.47 | 1.72E-03 |
| BF347758 | 240222_at | BF347758 | --- | --- | -1.47 | 2.67E-04 |
| CAMK2B | 210404_x_at | AF078803 | NM_001220 | 816 | -1.47 | 1.61E-03 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| MYO9A | 242512_at | AI382029 | NM_006901 | 4649 | -1.47 | 5.15E-03 |
| AKNA | 225701_at | AK024431 | NM_030767 | 80709 | -1.47 | 5.81E-03 |
| GPM6B | 240286_at | AL041745 | NM_00100199 4 | 2824 | -1.46 | 1.79E-03 |
| | | | NM_00100199 5 | | | |
| | | | NM_00100199 6 | | | |
| | | | NM_005278 | | | |
| STK4 | 223746_at | BC005231 | NM_006282 | 6789 | -1.46 | 1.31E-04 |
| BG178274 | 214757_at | BG178274 | --- | --- | -1.46 | 2.61E-04 |
| AW015870 | 240352_at | AW015870 | --- | --- | -1.46 | 3.79E-05 |
| C21orf66 | 239407_at | AI793248 | NM_013329 | 94104 | -1.46 | 4.76E-03 |
| | | | NM_016631 | | | |
| | | | NM_058191 | | | |
| | | | NM_145328 | | | |
| KIN | 236887_at | AA768850 | NM_012311 | 22944 | -1.46 | 5.23E-03 |
| PCNXL2 | 229202_at | AI768826 | NM_014801 | 80003 | -1.46 | 6.03E-04 |
| | | | NM_024938 | | | |
| KCNQ2 | 205737_at | NM_004518 | NM_004518 | 3785 | -1.46 | 2.13E-04 |
| | | | NM_172106 | | | |
| | | | NM_172107 | | | |
| | | | NM_172108 | | | |
| | | | NM_172109 | | | |
| C2orf17 | 222129_at | AK026155 | NM_024293 | 79137 | -1.46 | 1.21E-03 |
| FBXW7 | 218751_s_at | NM_018315 | NM_018315 | 55294 | -1.46 | 1.23E-07 |
| | | | NM_033632 | | | |
| U20648 | 216677_at | U20648 | --- | --- | -1.46 | 7.32E-05 |
| AGPAT3 | 223184_s_at | BC004219 | NM_020132 | 56894 | -1.46 | 2.96E-03 |
| ACYP2 | 206833_s_at | NM_001108 | NM_138448 | 98 | -1.46 | 1.16E-04 |
| TTC17 | 224852_at | BE964325 | NM_018259 | 55761 | -1.46 | 6.04E-05 |
| FLJ10159 | 218974_at | NM_018013 | --- | 55084 | -1.46 | 5.54E-04 |
| ZNF160 | 239954_at | AA701249 | NM_033288 | 90338 | -1.46 | 7.17E-03 |
| | | | NM_198893 | | | |
| SRP54 | 1557505_a_at | AI076351 | NM_003136 | 6729 | -1.45 | 1.01E-03 |
| AK023798 | 216838_at | AK023798 | --- | --- | -1.45 | 5.04E-04 |
| HMGB2 | 243368_at | AI572938 | NM_002129 | 3148 | -1.45 | 8.71E-03 |
| UBE2J1 | 236528_at | N64079 | NM_016021 | 51465 | -1.45 | 2.73E-03 |
| DKFZp313N062 | 226688_at | AW003508 | NM_173826 | 285343 | -1.45 | 6.50E-03 |
| GUCY1A3 | 221942_s_at | AI719730 | NM_000856 | 2982 | -1.45 | 5.82E-05 |
| LISCH7 | 208190_s_at | NM_015925 | NM_015925 | 51599 | -1.45 | 2.12E-03 |
| | | | NM_205834 | | | |
| | | | NM_205835 | | | |
| AI140531 | 1557812_a_at | AI140531 | --- | --- | -1.45 | 6.90E-03 |
| KIAA1276 | 227505_at | N64630 | XM_039169 | 27146 | -1.45 | 1.75E-03 |
| BC040287 | 1558605_at | BC040287 | --- | --- | -1.44 | 4.22E-03 |
| AI800713 | 235122_at | AI800713 | --- | --- | -1.44 | 3.58E-04 |
| BC033530 | 1560209_at | BC033530 | --- | --- | -1.44 | 1.67E-03 |
| HLCS | 207833_s_at | NM_000411 | NM_000411 | 3141 | -1.44 | 7.21E-03 |
| MCF2L | 239636_at | W74618 | NM_024979 | 23263 | -1.44 | 1.41E-03 |
| KIAA1276 | 233823_at | AB033102 | XM_039169 | 27146 | -1.44 | 3.24E-04 |
| OR7D2 | 1562337_at | AK095468 | NM_175883 | 162998 | -1.44 | 7.48E-04 |
| JAK2 | 1562031_at | BC043187 | NM_004972 | 3717 | -1.44 | 4.16E-04 |
| PSMB7 | 244801_at | AI248671 | NM_002799 | 5695 | -1.44 | 4.98E-03 |
| KIAA1411 | 233730_at | AL078591 | NM_020819 | 57579 | -1.43 | 3.20E-05 |
| AL530703 | 231882_at | AL530703 | XM_498570 | 440156 | -1.43 | 5.81E-04 |
| FLJ32731 | 227564_at | BF433005 | XM_372038 | 138050 | -1.43 | 7.92E-05 |
| DUSP16 | 1563505_at | AL833073 | NM_030640 | 80824 | -1.43 | 4.10E-05 |
| MGC4171 | 219722_s_at | NM_024307 | NM_024307 | 79153 | -1.43 | 5.72E-03 |
| RGAG4 | 227823_at | BE348679 | XM_291322 | 340526 | -1.43 | 3.08E-05 |
| MGC15875 | 226783_at | AI762154 | NM_032921 | 85007 | -1.43 | 1.81E-03 |
| | | | NM_153373 | | | |
| FIP1L1 | 233405_at | AU155384 | NM_030917 | 81608 | -1.43 | 3.48E-03 |
| PKD1 | 202328_s_at | NM_000296 | NM_000296 | 5310 | -1.43 | 1.82E-04 |
| | | | NM_00100994 4 | | | |
| CRYGS | 223643_at | AI762135 | NM_017541 | 1427 | -1.43 | 5.80E-03 |
| MGC42174 | 244304_at | AW129650 | NM_152383 | 129563 | -1.43 | 4.62E-03 |
| OFD1 | 241751_at | AW292752 | NM_003611 | 8481 | -1.42 | 4.86E-03 |
| PHC3 | 233431_x_at | AU148142 | NM_024947 | 80012 | -1.42 | 1.22E-03 |
| AK022100 | 233406_at | AK022100 | --- | --- | -1.42 | 1.03E-03 |
| PPARG | 208510_s_at | NM_015869 | NM_005037 | 5468 | -1.42 | 1.45E-03 |
| | | | NM_015869 | | | |
| | | | NM_138711 | | | |
| | | | NM_138712 | | | |
| CLCN3 | 201733_at | AA902971 | NM_001829 | 1182 | -1.42 | 1.12E-03 |
| | | | NM_173872 | | | |
| AW043836 | 239423_at | AW043836 | --- | --- | -1.42 | 2.43E-03 |
| AF086443 | 1560982_at | AF086443 | --- | --- | -1.42 | 2.70E-04 |
| R05895 | 239512_at | R05895 | --- | --- | -1.42 | 2.11E-03 |
| WDR45 | 209217_s_at | BC000464 | NM_007075 | 11152 | -1.42 | 3.54E-03 |
| BF512190 | 235406_x_at | BF512190 | --- | --- | -1.41 | 1.37E-03 |
| BE738279 | 242546_at | BE738279 | XM_498570 | 440156 | -1.41 | 1.24E-03 |
| AI929077 | 244818_at | AI929077 | --- | --- | -1.41 | 2.46E-03 |
| T96555 | 243818_at | T96555 | --- | --- | -1.41 | 1.37E-03 |
| DUSP16 | 1558739_at | R30807 | NM_030640 | 80824 | -1.41 | 3.84E-04 |
| LOC389087 | 243647_at | AI808306 | XM_374028 | 389087 | -1.41 | 9.78E-03 |
| HIST1H2BE | 208527_x_at | NM_003523 | NM_003523 | 8344 | -1.41 | 3.37E-05 |
| AU 155234 | 233289_at | AU 155234 | --- | --- | -1.41 | 2.99E-03 |
| AI698684 | 1558626_at | AI698684 | --- | --- | -1.40 | 3.33E-04 |
| KIAA1102 | 1569208_a_at | BC020895 | NM_014988 | 22998 | -1.40 | 4.93E-03 |
| AW150212 | 1556038_at | AW150212 | --- | --- | -1.40 | 1.33E-03 |
| ABCC5 | 226363_at | BE550362 | NM_005688 | 10057 | -1.40 | 4.83E-05 |
| FLJ13105 | 233083_at | AV706915 | XM_496701 | 80068 | -1.40 | 1.72E-03 |
| AI963605 | 230269_at | AI963605 | --- | --- | -1.40 | 1.31E-03 |
| ACHE | 205377_s_at | AI190022 | NM_000665 | 43 | -1.40 | 2.24E-04 |
| | | | NM_015831 | | | |
| WDR45 | 209216_at | BC000464 | NM_007075 | 11152 | -1.39 | 3.52E-03 |
| KIAA0804 | 239917_at | R07848 | NM_00100992 1 | 23355 | -1.39 | 2.63E-03 |
| | | | NM_015303 | | | |
| SIAH1 | 232365_at | BE676461 | NM_00100661 0 | 6477 | -1.39 | 1.23E-03 |
| | | | NM_00100661 1 | | | |
| | | | NM_003031 | | | |
| MUC20 | 226622_at | AW084511 | NM_152673 | 200958 | -1.39 | 3.06E-05 |
| TGFB114 | 237673_at | BE465103 | NM_006022 | 8848 | -1.39 | 2.34E-03 |
| | | | NM_183422 | | | |
| ZNF596 | 240324_at | H50654 | NM_173539 | 169270 | -1.39 | 3.41E-04 |
| NOL3 | 1558738_at | BU785956 | NM_003946 | 8996 | -1.39 | 1.03E-04 |
| CRYGS | 223644_s_at | AF161703 | NM_017541 | 1427 | -1.38 | 8.43E-03 |
| TSGA10 | 1555932_at | AK094208 | NM_025244 | 80705 | -1.38 | 2.99E-03 |
| | | | NM_182911 | | | |
| MST1 | 216320_x_at | U37055 | NM_020998 | 4485 | -1.38 | 1.28E-03 |
| LOC440722 | 238503_at | AI142850 | XM_498831 | 440722 | -1.38 | 8.92E-04 |
| FLJ10707 | 239957_at | AW510793 | XM_371614 | 55209 | -1.38 | 8.78E-03 |
| SLC37A1 | 218928_s_at | NM_018964 | NM_018964 | 54020 | -1.38 | 1.97E-03 |
| SMG1 | 244766_at | BG180003 | NM_014006 | 23049 | -1.38 | 8.25E-03 |
| KIAA0220 | | | NM_015092 | 283846 | | |
| LOC440345 | | | XM_496125 | 440345 | | |
| CENTG2 | 240758_at | AA706753 | NM_014914 | 116987 | -1.38 | 8.21E-03 |
| VPS13C | 232386_at | AV703288 | NM_017684 | 54832 | -1.38 | 1.41E-04 |
| ZNF42 | 210336_x_at | AF055078 | NM_003422 | 7593 | -1.38 | 7.52E-05 |
| | | | NM_198055 | | | |
| MCCC2 | 1560033_at | AK090834 | NM_022132 | 64087 | -1.37 | 6.91E-05 |
| USP40 | 225089_at | AA127740 | NM_018218 | 55230 | -1.37 | 9.95E-03 |
| ZNF589 | 1569108_a_at | BC028160 | NM_016089 | 51385 | -1.37 | 3.43E-03 |
| CITED2 | 207980_s_at | NM_006079 | NM_006079 | 10370 | -1.37 | 6.11E-03 |
| GAL | 214240_at | AL556409 | NM_015973 | 51083 | -1.37 | 2.81E-03 |
| RAGE | 205130_at | NM_014226 | NM_014226 | 5891 | -1.37 | 8.58E-03 |
| AA516469 | 236375_at | AA516469 | --- | --- | -1.37 | 2.01E-03 |
| AI915861 | 1557348_at | AI915861 | --- | --- | -1.37 | 7.72E-04 |
| AI861893 | 242888_at | AI861893 | --- | --- | -1.37 | 5.02E-03 |
| EDNRA | 243555_at | AA573452 | NM_001957 | 1909 | -1.37 | 2.53E-03 |
| C14orf92 | 232097_at | AA657818 | NM_014828 | 9878 | -1.37 | 1.70E-03 |
| LOC92482 | 232885_at | AK021501 | XM_378250 | 92482 | -1.37 | 7.03E-04 |
| KIAA0999 | 242920_at | AW590838 | NM_025164 | 23387 | -1.37 | 7.90E-03 |
| KCNK12 | 220448_at | NM_022055 | NM_022055 | 56660 | -1.37 | 2.31E-05 |
| TGFB114 | 237745_at | BE670165 | NM_006022 | 8848 | -1.37 | 1.15E-03 |
| | | | NM_183422 | | | |
| CDC2L5 | 242693_at | AW664859 | NM_003718 | 8621 | -1.37 | 7.67E-03 |
| | | | NM_031267 | | | |
| ZNF42 | 204139_x_at | NM_003422 | NM_003422 NM_198055 | 7593 | -1.36 | 1.36E-04 |
| BRUNOL5 | 232416_at | AL390161 | NM_021938 | 60680 | -1.36 | 9.03E-03 |
| AKAP12 | 210517_s_at | AB003476 | NM_005100 NM_144497 | 9590 | -1.36 | 3.29E-03 |
| RGS12 | 1555022_at | AF464736 | NM_002926 | 6002 | -1.36 | 1.77E-05 |
| | | | NM_198227 | | | |
| | | | NM_198229 | | | |
| | | | NM_198230 | | | |
| | | | NM_198430 | | | |
| | | | NM_198432 | | | |
| | | | NM_198587 | | | |
| LOC401320 | 238540_at | AA933883 | XM_379482 | 401320 | -1.36 | 1.06E-04 |
| | | | XM_380098 | | | |
| CUL4A | 232466_at | AU155661 | NM_00100889 5 | 8451 | -1.36 | 5.42E-03 |
| | | | NM_003589 | | | |
| SENP6 | 240016_at | AW273732 | NM_015571 | 26054 | -1.36 | 2.45E-03 |
| NBR2 | 1553992_s_at | BC034248 | NM_005821 | 10230 | -1.36 | 5.37E-03 |
| TBC1D8 | 221592_at | BC001663 | NM_007063 | 11138 | -1.36 | 4.54E-03 |
| MBNL1 | 1556658_a_at | AA744622 | NM_021038 | 4154 | -1.35 | 6.66E-03 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| HIST2H2AA | 218280_x_at | NM_003516 | NM_003516 | 8337 | -1.35 | 5.61E-04 |
| HECTD1 | 242349_at | AW275658 | NM_015382 | 25831 | -1.35 | 3.14E-05 |
| CSGIcA-T | 221799_at | AB037823 | NM_019015 | 54480 | -1.35 | 9.13E-03 |
| TD-60 | 243297_at | AI498610 | NM_018715 | 55920 | -1.35 | 3.64E-05 |
| AW117322 | 239415_at | AW117322 | XM_374094 | 389237 | -1.35 | 5.46E-05 |
| ERCC3 | 215545_at | AK024185 | NM_000122 | 2071 | -1.35 | 7.55E-03 |
| AW148862 | 235217_at | AW148862 | XM_380136 | 402582 | -1.35 | 8.44E-03 |
| NAP1L3 | 204749_at | NM_004538 | NM_004538 | 4675 | -1.35 | 1.79E-04 |
| HIST1H2BD | 222067_x_at | AL353759 | NM_021063 | 3017 | -1.34 | 1.71E-05 |
| | | | NM_138720 | | | |
| CITED2 | 209357_at | AF109161 | NM_006079 | 10370 | -1.34 | 3.87E-03 |
| NM_024914 | 220450_at | NM_024914 | --- | --- | -1.34 | 5.65E-04 |
| USP36 | 220370_s_at | NM_025090 | NM_025090 | 57602 | -1.34 | 2.56E-03 |
| ARMCX4 | 1564399_a_at | AK096955 | NM_152583 | 158947 | -1.34 | 8.99E-04 |
| GOLGA4 | 215203_at | AW438464 | NM_002078 | 2803 | -1.34 | 1.95E-03 |
| R92984 | 231088_at | R92984 | --- | --- | -1.34 | 4.79E-04 |
| LOC200030 | 242191_at | AI701905 | NM_183372 | 200030 | -1.34 | 1.70E-03 |
| LOC400781 | | | XM_371384 | 400781 | | |
| AG1 | | | XM_378897 | 440673 | | |
| | | | XM_496394 | | | |
| USP40 | 225085_at | AA522888 | NM_018218 | 55230 | -1.34 | 5.51E-03 |
| AK026429 | 233038_at | AK026429 | --- | --- | -1.34 | 8.07E-03 |
| AW977516 | 235172_at | AW977516 | --- | --- | -1.33 | 1.91E-03 |
| AK023334 | 232975_at | AK023334 | --- | --- | -1.33 | 3.06E-03 |
| CPSF6 | 238651_at | BF512491 | NM_007007 | 11052 | -1.33 | 2.21E-03 |
| LOC158301 | 239193_at | BF060981 | --- | 158301 | -1.33 | 9.50E-03 |
| TJP2 | 232017_at | AK025185 | NM_004817 | 9414 | -1.33 | 4.30E-04 |
| | | | NM_201629 | | | |
| AW591282 | 231374_at | AW591282 | --- | --- | -1.33 | 1.30E-04 |
| C21orf2 | 203995_at | NM_004928 | NM_004928 | 755 | -1.33 | 2.28E-03 |
| BF063821 | 230598_at | BF063821 | --- | --- | -1.33 | 2.29E-03 |
| AI743452 | 237031_at | AI743452 | --- | --- | -1.33 | 6.25E-04 |
| AI825068 | 239798_at | AI825068 | --- | --- | -1.33 | 7.63E-03 |
| SYVN1 | 225090_at | AA844682 | NM_032431 | 84447 | -1.32 | 5.82E-03 |
| | | | NM_172230 | | | |
| FLJ32731 | 1557064_s_at | AW328331 | XM_372038 | 138050 | -1.32 | 3.89E-06 |
| SLC12A7 | 218066_at | NM_006598 | NM_006598 | 10723 | -1.32 | 7.75E-04 |
| UBE2Q | 231816_s_at | AI986085 | NM_017582 | 55585 | -1.32 | 6.59E-03 |
| GOLGIN-67 | 208797_s_at | AI829170 | NM_015003 | 23015 | -1.32 | 1.73E-04 |
| | | | NM_181076 | | | |
| | | | NM_181077 | | | |
| THRAP2 | 239561_at | AA780679 | NM_015335 | 23389 | -1.32 | 1.91E-03 |
| PCDHB16 | 232099_at | AB046841 | NM_020957 | 57717 | -1.32 | 2.25E-03 |
| GPR123 | 239221_at | AI884909 | NM_032422 | 84435 | -1.31 | 2.84E-03 |
| C1orf21 | 223127_s_at | AW003850 | NM_030806 | 81563 | -1.31 | 8.71E-04 |
| LOC54103 | 213142_x_at | AV700415 | XM_379881 | 54103 | -1.31 | 2.15E-03 |
| | | | XM_499340 | | | |
| LOC90408 | 1560081_at | AL702091 | --- | 90408 | -1.31 | 3.47E-04 |
| HEMK1 | 1556092_s_at | BQ025558 | NM_016173 | 51409 | -1.31 | 2.82E-03 |
| AW294192 | 228040_at | AW294192 | --- | --- | -1.31 | 1.56E-03 |
| ZNF207 | 1556035_s_at | AI201248 | NM_003457 | 7756 | -1.31 | 6.92E-03 |
| BU680365 | 1562497_at | BU680365 | --- | --- | -1.31 | 9.25E-03 |
| MGC24132 | 1557991_at | AK057791 | NM_152396 | 131965 | -1.31 | 9.13E-04 |
| CLCN3 | 201734_at | AI760629 | NM_001829 | 1182 | -1.31 | 2.96E-03 |
| | | | NM_173872 | | | |
| PPIB | 200968_s_at | NM_000942 | NM_000942 | 5479 | -1.31 | 5.05E-03 |
| MYNN | 1566108_at | AK056483 | NM_018657 | 55892 | -1.31 | 3.79E-03 |
| MFN1 | 236417_at | BE644770 | NM_017927 | 55669 | -1.31 | 5.74E-03 |
| | | | NM_033540 | | | |
| TTC17 | 224849_at | AK023161 | NM_018259 | 55761 | -1.31 | 2.03E-04 |
| HNRPD | 229434_at | AA865357 | NM_00100381 0 | 3184 | -1.30 | 4.38E-03 |
| | | | NM_002138 | | | |
| | | | NM_031369 | | | |
| | | | NM_031370 | | | |
| BE217923 | 228686_at | BE217923 | --- | --- | -1.30 | 1.31E-04 |
| WDR11 | 229694_at | BF062828 | NM_018117 | 55717 | -1.30 | 5.51E-03 |
| AA703326 | 241863_x_at | AA703326 | --- | --- | -1.30 | 3.55E-03 |
| ZNF117 | 235408_x_at | AW058673 | NM_024498 | 7670 | -1.30 | 8.70E-03 |
| COL5A1 | 212489_at | AI983428 | NM_000093 | 1289 | -1.30 | 8.41E-03 |
| AKAP12 | 241679_at | AI672553 | NM_005100 | 9590 | -1.30 | 1.14E-03 |
| | | | NM_144497 | | | |
| DKFZp762K222 | 226822_at | AL512713 | NM_020225 | 56977 | -1.30 | 1.52E-04 |
| PTBP2 | 236962_at | AA521018 | NM_021190 | 58155 | -1.30 | 3.82E-03 |
| C7orf28B | 215024_at | AK000993 | NM_198097 | 221960 | -1.29 | 6.75E-04 |
| EFHC1 | 225656_at | AI564473 | NM_018100 | 114327 | -1.29 | 8.52E-03 |
| KIAA1970 | 1568858_at | BC013247 | NM_133451 | 124454 | -1.29 | 1.47E-05 |
| CAMK2B | 211483_x_at | AF081924 | NM_001220 | 816 | -1.29 | 6.21E-03 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| LOC142678 | 226644_at | BE222279 | NM_080875 | 142678 | -1.29 | 4.59E-04 |
| IGBP1 | 242337_at | AI347128 | NM_001551 | 3476 | -1.29 | 4.53E-03 |
| MAP7 | 202890_at | AW242297 | NM_003980 | 9053 | -1.29 | 9.83E-04 |
| AK3 | 230630_at | AI566130 | NM_00100535 3 | 205 | -1.29 | 3.77E-03 |
| | | | NM_013410 | | | |
| | | | NM_203464 | | | |
| AI434790 | 239070_at | AI434790 | --- | --- | -1.29 | 2.24E-04 |
| CD302 | 203799_at | NM_014880 | NM_014880 | 9936 | -1.29 | 1.51E-04 |
| VPS13C | 218396_at | NM_017684 | NM_017684 | 54832 | -1.29 | 1.69E-04 |
| CUL3 | 242362_at | AI797788 | NM_003590 | 8452 | -1.29 | 3.18E-03 |
| AW971123 | 236446_at | AW971123 | --- | --- | -1.28 | 3.31E-03 |
| FLJ13150 | 219504_s_at | NM_024813 | NM_024813 | 79871 | -1.28 | 6.46E-06 |
| EPAS1 | 230711_at | AA777349 | NM_001430 | 2034 | -1.28 | 7.58E-03 |
| LOC91526 | 226641_at | AU157224 | NM_153697 | 91526 | -1.28 | 3.21E-03 |
| AW467480 | 236202_at | AW467480 | --- | --- | -1.28 | 1.77E-03 |
| MTHFR | 226929_at | AA524272 | NM_005957 | 4524 | -1.28 | 1.48E-04 |
| MKLN1 | 1560145_at | BQ942131 | NM_013255 | 4289 | -1.28 | 4.90E-03 |
| ZNF207 | 231848_x_at | AW192569 | NM_003457 | 7756 | -1.28 | 7.40E-03 |
| AI796536 | 229810_at | AI796536 | --- | --- | -1.28 | 5.96E-04 |
| BF724558 | 241722_x_at | BF724558 | --- | --- | -1.28 | 4.48E-03 |
| AI825987 | 235889_at | AI825987 | --- | --- | -1.27 | 2.27E-04 |
| TXNDC4 | 208958_at | AI827677 | NM_015051 | 23071 | -1.27 | 7.53E-04 |
| ADORA2B | 205891_at | NM_000676 | NM_000676 | 136 | -1.27 | 7.80E-03 |
| FLJ21159 | 220145_at | NM_024826 | NM_024826 | 79884 | -1.27 | 1.45E-05 |
| WSB1 | 201294_s_at | N24643 | NM_015626 | 26118 | -1.27 | 4.68E-03 |
| | | | NM_134264 | | | |
| | | | NM_134265 | | | |
| AK3 | 225342_at | AK026966 | NM_00100535 3 | 205 | -1.27 | 9.13E-03 |
| | | | NM_013410 | | | |
| | | | NM_203464 | | | |
| PLCB4 | 203896_s_at | NM_000933 | NM_000933 | 5332 | -1.27 | 1.40E-04 |
| | | | NM_182797 | | | |
| RAPGEF2 | 215992_s_at | AL117397 | XM_376350 | 9693 | -1.27 | 1.92E-05 |
| PDXK | 218019_s_at | NM_021941 | NM_003681 | 8566 | -1.27 | 9.30E-03 |
| DUSP16 | 224336_s_at | AB052156 | NM_030640 | 80824 | -1.27 | 3.94E-03 |
| BACE2 | 222446_s_at | AF178532 | NM_012105 | 25825 | -1.27 | 4.30E-04 |
| | | | NM_138991 | | | |
| | | | NM_138992 | | | |
| PUM2 | 216121_at | AL080106 | NM_015317 | 23369 | -1.27 | 1.02E-05 |
| AVEN | 219366_at | NM_020371 | NM_020371 | 57099 | -1.27 | 5.67E-03 |
| NMB | 205204_at | NM_021077 | NM_021077 | 4828 | -1.26 | 3.26E-05 |
| | | | NM_205858 | | | |
| EPOR | 396_f_at | X97671 | NM_000121 | 2057 | -1.26 | 8.71E-04 |
| CSRP1 | 200621_at | NM_004078 | NM_004078 | 1465 | -1.26 | 9.64E-03 |
| MYT1L | 1554633_a_at | AF036943 | NM_015025 | 23040 | -1.26 | 4.55E-03 |
| TAPBP | 208829_at | AF029750 | NM_003190 | 6892 | -1.26 | 8.92E-06 |
| | | | NM_172208 | | | |
| | | | NM_172209 | | | |
| AI741597 | 230302_at | AI741597 | --- | --- | -1.26 | 3.47E-03 |
| C10orf10 | 209183_s_at | AL136653 | NM_007021 | 11067 | -1.26 | 1.25E-03 |
| FLJ31951 | 236322_at | AA830854 | NM_144726 | 153830 | -1.26 | 1.23E-03 |
| HERC4 | 225989_at | AB046813 | NM_015601 | 26091 | -1.26 | 1.35E-04 |
| PTGS1 | 238669_at | BE613133 | NM_000962 | 5742 | -1.26 | 7.68E-05 |
| | | | NM_080591 | | | |
| GTPBP2 | 221050_s_at | NM_019096 | NM_019096 | 54676 | -1.26 | 5.82E-03 |
| CNOT4 | 1559347_at | BI599409 | NM_00100822 5 | 4850 | -1.26 | 4.86E-03 |
| | | | NM_013316 | | | |
| BM914560 | 1558220_at | BM914560 | XM_496669 | 440993 | -1.26 | 9.16E-03 |
| C6orf111 | 230375_at | AI936531 | NM_032870 | 25957 | -1.26 | 1.30E-03 |
| WBSCR19 | 232964_at | AL137266 | NM_175064 | 285955 | -1.26 | 4.75E-04 |
| AW104619 | 229754_at | AW104619 | --- | --- | -1.26 | 3.59E-03 |
| DISC1 | 244642_at | AI761381 | NM_018662 | 27185 | -1.26 | 7.13E-03 |
| FLJ32130 | 1554770_x_at | BC040642 | NM_152458 | 146540 | -1.26 | 1.11E-03 |
| AW192718 | 1559437_at | AW192718 | XM_373180 | 392084 | -1.25 | 5.77E-03 |
| BC014318 | 1558236_at | BC014318 | --- | --- | -1.25 | 1.70E-04 |
| EVC | 219432_at | NM_014556 | NM_014556 | 2121 | -1.25 | 3.41E-03 |
| | | | NM_153717 | | | |
| PPAPDC1 | 236044_at | BF130943 | XM_113641 | 196051 | -1.25 | 1.93E-04 |
| AI732885 | 214388_at | AI732885 | --- | --- | -1.25 | 1.01E-03 |
| AI588962 | 230477_at | AI588962 | --- | --- | -1.25 | 1.64E-04 |
| HIST1H2BI | 208523_x_at | NM_003525 | NM_003525 | 8346 | -1.25 | 1.67E-03 |
| HRMT1L1 | 216069_at | AL050065 | NM_001535 | 3275 | -1.25 | 6.45E-05 |
| | | | NM_206962 | | | |
| DKFZP434I0714 | 231954_at | AL137273 | --- | 54553 | -1.25 | 1.77E-03 |
| ZNF654 | 219239_s_at | NM_018293 | NM_018293 | 55279 | -1.25 | 8.76E-04 |
| BC035105 | 1569482_at | BC035105 | --- | --- | -1.25 | 1.09E-03 |
| ZFYVE16 | 240859_at | N20928 | NM_014733 | 9765 | -1.25 | 8.54E-03 |
| AW303371 | 242096_at | AW303371 | --- | --- | -1.25 | 8.48E-03 |
| AK025360 | 216173_at | AK025360 | --- | --- | -1.25 | 7.46E-03 |
| LOC440157 | 1558139_at | BE730158 | XM_495969 | 440157 | -1.25 | 8.53E-03 |
| MGC15407 | 224968_at | AL518311 | NM_080667 | 112942 | -1.25 | 1.67E-04 |
| W18186 | 239842_x_at | W18186 | --- | --- | -1.25 | 3.42E-04 |
| FLJ32130 | 1554769_at | BC040642 | NM_152458 | 146540 | -1.25 | 1.81E-04 |
| PER2 | 205251_at | NM_022817 | NM_003894 | 8864 | -1.25 | 4.90E-03 |
| | | | NM_022817 | | | |
| NOL3 | 221567_at | AF064599 | NM_003946 | 8996 | -1.25 | 1.02E-04 |
| ORMDL1 | 228801_at | AI809749 | NM_016467 | 94101 | -1.25 | 1.54E-07 |
| FLJ23825 | 1555866_a_at | BM980844 | NM_173620 | 284004 | -1.25 | 2.44E-03 |
| ACRV1 | 207969_x_at | NM_020109 | NM_001612 | 56 | -1.24 | 3.48E-04 |
| | | | NM_020069 | | | |
| | | | NM_020107 | | | |
| | | | NM_020108 | | | |
| | | | NM_020109 | | | |
| | | | NM_020110 | | | |
| | | | NM_020111 | | | |
| | | | NM_020113 | | | |
| | | | NM_020114 | | | |
| | | | NM_020115 | | | |
| CHD5 | 213965_s_at | AL035406 | NM_015557 | 26038 | -1.24 | 1.51E-04 |
| C17orf27 | 1569078_at | AF318359 | NM_020914 | 57674 | -1.24 | 4.95E-03 |
| RAPGEF2 | 238176_at | T86196 | XM_376350 | 9693 | -1.24 | 9.33E-03 |
| JMJD2B | 1558448_a_at | BC032415 | NM_015015 | 23030 | -1.24 | 7.07E-03 |
| SIPA1L2 | 1559469_s_at | BC006013 | NM_020808 | 57568 | -1.24 | 5.27E-03 |
| AI018235 | 240314_at | AI018235 | --- | --- | -1.24 | 4.83E-03 |
| BC007626 | 1562946_at | BC007626 | --- | --- | -1.24 | 5.88E-05 |
| TIP120A | 239771_at | BG399629 | NM_018448 | 55832 | -1.24 | 1.29E-03 |
| BF507816 | 240517_at | BF507816 | --- | --- | -1.24 | 6.12E-03 |
| T79942 | 228465_at | T79942 | --- | --- | -1.23 | 1.38E-03 |
| RPIP8 | 206196_s_at | NM_006695 | NM_006695 | 10900 | -1.23 | 2.25E-03 |
| SFRS12 | 243361_at | N51597 | NM_139168 | 140890 | -1.23 | 4.89E-03 |
| FBXW7 | 229419_at | BF222826 | NM_018315 | 55294 | -1.23 | 3.99E-04 |
| | | | NM_033632 | | | |
| PRKCA | 213093_at | AI471375 | NM_002737 | 5578 | -1.23 | 4.72E-04 |
| AI042344 | 240121_x_at | AI042344 | --- | --- | -1.23 | 3.31E-05 |
| ABCA2 | 210100_s_at | AF327657 | NM_001606 | 20 | -1.23 | 2.95E-04 |
| | | | NM_212533 | | | |
| CHST1 | 205567_at | NM_003654 | NM_003654 | 8534 | -1.23 | 4.23E-03 |
| GRIK2 | 1563754_at | AJ252246 | NM_021956 | 2898 | -1.23 | 1.60E-03 |
| | | | NM_175768 | | | |
| AI887898 | 228423_at | AI887898 | XM_374094 | 389237 | -1.23 | 7.61E-04 |
| FADS3 | 204257_at | NM_021727 | NM_021727 | 3995 | -1.23 | 4.30E-04 |
| BE551142 | 241091_at | BE551142 | XM_497668 | 388620 | -1.23 | 4.14E-03 |
| BC014318 | 1558237_x_at | BC014318 | --- | --- | -1.23 | 7.31E-04 |
| TCEA1 | 1566207_at | BQ286789 | NM_006756 | 6917 | -1.23 | 9.86E-06 |
| | | | NM_201437 | | | |
| PLCE1 | 205111_s_at | NM_016341 | NM_016341 | 51196 | -1.23 | 4.76E-07 |
| KIAA0125 | 220377_at | NM_014151 | NM_014792 | 9834 | -1.23 | 3.84E-04 |
| PIK3C2A | 241905_at | AA579047 | NM_002645 | 5286 | -1.22 | 8.81E-03 |
| USP15 | 233473_x_at | AU156202 | NM_006313 | 9958 | -1.22 | 5.44E-03 |
| DHX36 | 1559039_at | AK096808 | NM_020865 | 170506 | -1.22 | 5.13E-03 |
| KIAA0368 | 236368_at | BF059292 | XM_036708 | 23392 | -1.22 | 1.71E-04 |
| AI971535 | 229626_at | AI971535 | XM_370691 | 387856 | -1.22 | 3.12E-04 |
| NOL3 | 221566_s_at | AF043244 | NM_003946 | 8996 | -1.22 | 9.37E-04 |
| ADAM8 | 205180_s_at | NM_001109 | NM_001109 | 101 | -1.22 | 7.63E-03 |
| FGF7 | 230918_at | BE856598 | NM_002009 | 2252 | -1.22 | 1.18E-03 |
| C21orf25 | 212875_s_at | AP001745 | NM_199050 | 25966 | -1.22 | 1.75E-03 |
| C21orf57 | 227421_at | BF026326 | NM_00100611 4 | 54059 | -1.22 | 4.19E-04 |
| | | | NM_058181 | | | |
| BF061976 | 243499_at | BF061976 | --- | --- | -1.22 | 3.63E-03 |
| RLF | 204243_at | NM_012421 | NM_012421 | 6018 | -1.21 | 3.24E-05 |
| MLLT2 | 201924_at | NM_005935 | NM_005935 | 4299 | -1.21 | 1.72E-03 |
| BE467577 | 228251_at | BE467577 | --- | --- | -1.21 | 2.37E-03 |
| WSB1 | 201295_s_at | BF111821 | NM_015626 | 26118 | -1.21 | 2.55E-03 |
| | | | NM_134264 | | | |
| | | | NM_134265 | | | |
| PR47 | 1560526_at | BQ880034 | --- | 266671 | -1.21 | 4.02E-03 |
| PRPH | 213847_at | NM_006262 | NM_006262 | 5630 | -1.21 | 3.82E-03 |
| H75391 | 1559515_at | H75391 | --- | --- | -1.21 | 3.64E-04 |
| AI740589 | 243515_at | AI740589 | --- | --- | -1.21 | 7.40E-04 |
| OLFM3 | 1554526_at | BC022531 | NM_058170 | 118427 | -1.21 | 1.84E-04 |
| C14orf135 | 1563259_at | AK024838 | NM_022495 | 64430 | -1.21 | 9.40E-04 |
| U00954 | 232180_at | U00954 | --- | --- | -1.21 | 4.24E-05 |
| AI631888 | 235532_at | AI631888 | --- | --- | -1.21 | 1.03E-03 |
| EXOC7 | 214802_at | AK022397 | NM_015219 | 23265 | -1.21 | 8.35E-03 |
| SIPA1L2 | 225056_at | AB037810 | NM_020808 | 57568 | -1.20 | 7.73E-03 |
| HSPC065 | 222890_at | BG054922 | NM_014157 | 29070 | -1.20 | 4.19E-03 |
| IER3 | 201631_s_at | NM_003897 | NM_003897 | 8870 | -1.20 | 3.48E-04 |
| | | | NM_052815 | | | |
| BF059537 | 240020_at | BF059537 | --- | --- | -1.20 | 2.65E-04 |
| BE503598 | 1557659_a_at | BE503598 | --- | --- | -1.20 | 1.58E-03 |
| FLJ13710 | 222835_at | BG163478 | NM_024817 | 79875 | -1.20 | 8.19E-03 |
| C14orf120 | 216263_s_at | AK022215 | XM_033371 | 25983 | -1.20 | 5.96E-07 |
| FNBP1 | 212288_at | AB011126 | NM_015033 | 23048 | -1.20 | 9.04E-06 |
| TTC11 | 244082_at | BF507959 | NM_016068 | 51024 | -1.20 | 7.10E-03 |
| ABR | 214671_s_at | L19704 | NM_001092 | 29 | -1.20 | 5.25E-03 |
| | | | NM_021962 | | | |
| ABTB1 | 229164_s_at | AI337369 | NM_032548 | 80325 | -1.20 | 3.15E-03 |
| | | | NM_172027 | | | |
| | | | NM_172028 | | | |
| TRIM50C | 1554182_at | BC033871 | NM_198853 | 378108 | -1.19 | 6.94E-05 |
| ADAMTS1 | 222162_s_at | AK023795 | NM_006988 | 9510 | -1.19 | 1.75E-03 |
| TIGD4 | 232998_at | AK024235 | NM_145720 | 201798 | -1.19 | 1.23E-04 |
| KIAA0220 | 231989_s_at | AC003007 | --- | 283846 | -1.19 | 6.25E-03 |
| AI435590 | 237192_at | AI435590 | --- | --- | -1.19 | 7.36E-04 |
| NOL3 | 59625_at | AI912351 | NM_003946 | 8996 | -1.19 | 4.25E-05 |
| FOXR2 | 1569669_at | BM550294 | NM_198451 | 139628 | -1.19 | 6.60E-03 |
| AW628665 | 235555_at | AW628665 | --- | --- | -1.19 | 7.01E-03 |
| LOC285831 | 228857_at | AA775731 | --- | 285831 | -1.19 | 2.24E-04 |
| C15orf28 | 220710_at | NM_024970 | --- | 80035 | -1.19 | 7.83E-03 |
| KIAA1967 | 240721_at | BE672858 | NM_021174 | 57805 | -1.19 | 4.55E-04 |
| | | | NM_199205 | | | |
| BRPF3 | 225217_s_at | AB033112 | NM_015695 | 27154 | -1.19 | 3.39E-04 |
| PPP3CA | 236545_at | AA532718 | NM_000944 | 5530 | -1.18 | 1.89E-03 |
| BF514509 | 239050_s_at | BF514509 | --- | --- | -1.18 | 8.62E-03 |
| AA639707 | 243837_x_at | AA639707 | --- | --- | -1.18 | 1.88E-04 |
| WSB1 | 210561_s_at | AL110243 | NM_015626 | 26118 | -1.18 | 7.42E-04 |
| | | | NM_134264 | | | |
| | | | NM_134265 | | | |
| AA888777 | 242740_at | AA888777 | --- | --- | -1.18 | 1.23E-03 |
| BMPR2 | 238393_at | AL047534 | NM_001204 | 659 | -1.18 | 5.95E-03 |
| | | | NM_033346 | | | |
| BACH2 | 1556451_at | AL833645 | NM_021813 | 60468 | -1.18 | 2.17E-04 |
| CRELD1 | 203368_at | NM_015513 | NM_015513 | 78987 | -1.18 | 3.92E-03 |
| RPL37 | 224766_at | AW008221 | NM_000997 | 6167 | -1.18 | 6.73E-06 |
| CSTB | 201201_at | NM_000100 | NM_000100 | 1476 | -1.18 | 2.39E-06 |
| MGC12458 | 211200_s_at | BC002836 | NM_032328 | 84288 | -1.18 | 4.74E-03 |
| C9orf111 | 228383_at | AK024443 | NM_152286 | 375775 | -1.18 | 4.27E-03 |
| PLRG1 | 227246_at | BF438014 | NM_002669 | 5356 | -1.18 | 8.04E-04 |
| ZFYVE9 | 208446_s_at | NM_007323 | NM_004799 | 9372 | -1.18 | 3.59E-03 |
| | | | NM_007323 | | | |
| | | | NM_007324 | | | |
| ZNF226 | 224004_at | AF246126 | NM_015919 | 7769 | -1.17 | 2.52E-04 |
| | | | NM_016444 | | | |
| NM_015437 | 207799_x_at | NM_015437 | --- | --- | -1.17 | 7.74E-04 |
| AFTIPHILIN | 244312_at | AW195572 | NM_00100224 3 | 54812 | -1.17 | 6.11E-03 |
| | | | NM_017657 | | | |
| | | | NM_203437 | | | |
| AL137378 | 216144_at | AL137378 | --- | --- | -1.17 | 1.87E-03 |
| NUP153 | 239948_at | AA004800 | NM_005124 | 9972 | -1.17 | 4.63E-03 |
| OLFM3 | 1554524_a_at | AF397394 | NM_058170 | 118427 | -1.17 | 4.35E-05 |
| N30152 | 229850_at | N30152 | --- | --- | -1.17 | 1.02E-03 |
| AI934828 | 227116_at | AI934828 | XM_375373 | 400547 | -1.17 | 4.08E-03 |
| LRRFIP2 | 230082_at | AW137053 | NM_006309 | 9209 | -1.17 | 1.78E-03 |
| | | | NM_017724 | | | |
| HPCAL1 | 212552_at | BE617588 | NM_002149 | 3241 | -1.17 | 1.73E-04 |
| | | | NM_134421 | | | |
| DRPLA | 236961_at | AI539426 | NM_00100702 6 | 1822 | -1.17 | 2.23E-03 |
| | | | NM_001940 | | | |
| CLCN3 | 201732_s_at | AF029346 | NM_001829 | 1182 | -1.17 | 2.63E-03 |
| | | | NM_173872 | | | |
| RFX2 | 226872_at | AK024288 | NM_000635 | 5990 | -1.17 | 2.24E-03 |
| | | | NM_134433 | | | |
| HNRPD | 235999_at | AA863112 | NM_00100381 0 | 3184 | -1.17 | 1.28E-04 |
| | | | NM_002138 | | | |
| | | | NM_031369 | | | |
| | | | NM_031370 | | | |
| WNK1 | 211993_at | AI768512 | NM_018979 | 65125 | -1.17 | 1.66E-03 |
| LOC401320 | 1558459_s_at | BC041636 | XM_379482 | 401320 | -1.17 | 2.08E-03 |
| | | | XM_380098 | | | |
| NM_025033 | 208301_at | NM_025033 | --- | --- | -1.17 | 9.10E-05 |
| KIAA1040 | 242225_at | AI569482 | NM_015026 | 23041 | -1.17 | 5.75E-03 |
| C9orf68 | 220218_at | NM_017985 | NM_017985 | 55064 | -1.16 | 4.90E-03 |
| C21orf66 | 1555125_at | BC030539 | NM_013329 | 94104 | -1.16 | 3.17E-05 |
| | | | NM_016631 | | | |
| | | | NM_058191 | | | |
| | | | NM_145328 | | | |
| GAD2 | 243265_at | AW444497 | NM_000818 | 2572 | -1.16 | 1.34E-04 |
| ABTB1 | 226442_at | H06267 | NM_032548 | 80325 | -1.16 | 2.47E-05 |
| | | | NM_172027 | | | |
| | | | NM_172028 | | | |
| ZNF644 | 1557751_at | AI537913 | NM_016620 | 84146 | -1.16 | 6.37E-03 |
| | | | NM_032186 | | | |
| | | | NM_201269 | | | |
| CENTB2 | 240759_at | AW593931 | NM_012287 | 23527 | -1.16 | 5.67E-03 |
| CAPN2 | 208683_at | M23254 | NM_001748 | 824 | -1.16 | 3.02E-04 |
| TMEM5 | 204808_s_at | NM_014254 | NM_014254 | 10329 | -1.16 | 1.28E-04 |
| HIST1H2BF | 208490_x_at | NM_003522 | NM_003522 | 8343 | -1.16 | 7.03E-04 |
| BCAP29 | 230150_at | N57499 | NM_00100840 5 | 55973 | -1.16 | 2.32E-03 |
| | | | NM_00100840 6 | | | |
| | | | NM_00100840 7 | | | |
| | | | NM_018844 | | | |
| PERLD1 | 55616_at | AI703342 | NM_033419 | 93210 | -1.16 | 4.41E-03 |
| DTNA | 227084_at | AW339310 | NM_001390 | 1837 | -1.16 | 3.40E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| CHD9 | 220586_at | NM_025134 | NM_025134 | 80205 | -1.16 | 3.15E-03 |
| BF478226 | 241684_at | BF478226 | --- | --- | -1.16 | 3.08E-04 |
| SLC27A3 | 222217_s_at | BC003654 | NM_024330 | 11000 | -1.16 | 7.01E-04 |
| PLOD1 | 200827_at | NM_000302 | NM_000302 | 5351 | -1.16 | 1.10E-03 |
| AI216567 | 236754_at | AI216567 | --- | --- | -1.15 | 1.78E-03 |
| AI693178 | 228101_at | AI693178 | --- | --- | -1.15 | 1.78E-06 |
| ZNF587 | 242564_at | AI703142 | NM_032828 | 84914 | -1.15 | 8.15E-04 |
| ZDHHC11 | 232417_x_at | AU150300 | NM_024786 | 79844 | -1.15 | 1.16E-03 |
| AK3 | 204348_s_at | NM_013410 | NM_00100535 3 | 205 | -1.15 | 4.21E-03 |
| | | | NM_013410 | | | |
| | | | NM_203464 | | | |
| EFNB3 | 205031_at | NM_001406 | NM_001406 | 1949 | -1.15 | 1.14E-04 |
| NYREN18 | 1559732_at | AK056624 | NM_016118 | 51667 | -1.15 | 9.75E-05 |
| SIPA1L2 | 233587_s_at | AK022852 | NM_020808 | 57568 | -1.15 | 1.97E-03 |
| AI287817 | 240095_at | AI287817 | --- | --- | -1.15 | 5.78E-04 |
| IARS | 239794_at | AI356405 | NM_002161 | 3376 | -1.15 | 5.87E-03 |
| | | | NM_013417 | | | |
| WSB1 | 201296_s_at | NM_015626 | NM_015626 | 26118 | -1.15 | 2.42E-03 |
| | | | NM_134264 | | | |
| | | | NM_134265 | | | |
| AB037813 | 231969_at | AB037813 | --- | --- | -1.15 | 9.45E-05 |
| AI022648 | 242972_at | AI022648 | --- | --- | -1.15 | 6.12E-03 |
| AA651954 | 236626_at | AA651954 | --- | --- | -1.14 | 1.11E-04 |
| TAGLN2 | 200916_at | NM_003564 | NM_003564 | 8407 | -1.14 | 7.20E-03 |
| AI263044 | 214376_at | AI263044 | --- | --- | -1.14 | 4.04E-03 |
| ZNF333 | 1552375_at | NM_032433 | NM_032433 | 84449 | -1.14 | 4.08E-03 |
| HSPB1 | 201841_s_at | NM_001540 | NM_001540 | 3315 | -1.14 | 8.01E-03 |
| FLJ25778 | 238350_at | AW967956 | XM_376679 | 254048 | -1.14 | 8.71E-03 |
| | | | XM_379933 | | | |
| KIAA1731 | 1565830_at | AL833615 | XM_374922 | 85459 | -1.14 | 4.73E-05 |
| FLJ32130 | 242272_at | AI375066 | NM_152458 | 146540 | -1.14 | 3.52E-04 |
| FLJ39485 | 236728_at | AW070437 | NM_175920 | 285603 | -1.14 | 6.01E-03 |
| AKAP14 | 228493_at | T87628 | NM_00100853 4 | 158798 | -1.14 | 3.57E-03 |
| | | | NM_00100853 5 | | | |
| | | | NM_178813 | | | |
| NEDD5 | 232637_at | AK025163 | NM_00100849 1 | 4735 | -1.14 | 1.03E-04 |
| | | | NM_00100849 2 | | | |
| | | | NM_004404 | | | |
| | | | NM_006155 | | | |
| COH1 | 236254_at | BE048857 | NM_015243 | 157680 | -1.14 | 9.97E-04 |
| | | | NM_017890 | | | |
| | | | NM_152564 | | | |
| | | | NM_181661 | | | |
| | | | NM_184042 | | | |
| COLEC11 | 219873_at | NM_024027 | NM_024027 | 78989 | -1.14 | 2.80E-04 |
| | | | NM_199235 | | | |
| TMPIT | 234084_x_at | AU147104 | NM_031925 | 83862 | -1.14 | 4.48E-03 |
| P4HA2 | 1568611_at | CA418310 | NM_004199 | 8974 | -1.14 | 5.99E-03 |
| APOC1 | 213553_x_at | W79394 | NM_001645 | 341 | -1.14 | 2.08E-03 |
| BI868311 | 1565651_at | BI868311 | --- | --- | -1.14 | 1.59E-03 |
| NRD1 | 242235_x_at | AW978721 | NM_002525 | 4898 | -1.13 | 6.03E-03 |
| ABCA2 | 212772_s_at | AL162060 | NM_001606 | 20 | -1.13 | 2.55E-04 |
| | | | NM_212533 | | | |
| EGLN1 | 1561893_at | BC024172 | NM_022051 | 54583 | -1.13 | 2.07E-06 |
| ATXN7L1 | 227732_at | AB033044 | --- | 57485 | -1.13 | 9.68E-04 |
| SSR1 | 226712_at | BF206389 | NM_003144 | 6745 | -1.13 | 4.37E-03 |
| KIAA1045 | 37566_at | AB028968 | XM_048592 | 23349 | -1.13 | 1.00E-03 |
| COPA | 1559862_at | BC037941 | NM_004371 | 1314 | -1.13 | 3.33E-04 |
| ARGBP2 | 204288_s_at | NM_021069 | NM_003603 | 8470 | -1.13 | 5.18E-04 |
| | | | NM_021069 | | | |
| PLCE1 | 205112_at | NM_016341 | NM_016341 | 51196 | -1.13 | 2.45E-03 |
| PDGFRB | 202273_at | NM_002609 | NM_002609 | 5159 | -1.13 | 4.37E-03 |
| METAP2 | 240769_at | AI733395 | NM_006838 | 10988 | -1.13 | 6.92E-06 |
| GNPTG | 224887_at | AF302786 | NM_032520 | 84572 | -1.13 | 7.70E-05 |
| TAGLN2 | 210978_s_at | BC002616 | NM_003564 | 8407 | -1.13 | 6.34E-03 |
| BDP1 | 231939_s_at | AJ238520 | NM_018429 | 55814 | -1.13 | 5.92E-04 |
| AA524740 | 225249_at | AA524740 | --- | --- | -1.13 | 5.63E-03 |
| MCM3AP | 212269_s_at | AJ010089 | NM_003906 | 8888 | -1.13 | 7.20E-04 |
| HEL308 | 228736_at | AW084661 | NM_133636 | 113510 | -1.12 | 4.79E-04 |
| BF540795 | 243043_at | BF540795 | --- | --- | -1.12 | 1.49E-03 |
| BF339133 | 213296_at | BF339133 | --- | --- | -1.12 | 2.35E-03 |
| BF940997 | 239857_at | BF940997 | --- | --- | -1.12 | 2.17E-03 |
| SFRS11 | 237746_at | Al168187 | NM_004768 | 9295 | -1.12 | 3.42E-03 |
| ADAMTS1 | 222486_s_at | AF060152 | NM_006988 | 9510 | -1.12 | 1.60E-03 |
| AI654130 | 241936_x_at | AI654130 | --- | --- | -1.12 | 3.27E-03 |
| NSUN4 | 1559848_at | BC016907 | NM_199044 | 387338 | -1.12 | 1.76E-03 |
| AA830545 | 235730_at | AA830545 | --- | --- | -1.12 | 9.65E-03 |
| ABR | 212895_s_at | AL527773 | NM_001092 | 29 | -1.12 | 2.02E-04 |
| | | | NM_021962 | | | |
| AW150212 | 1556039_s_at | AW150212 | --- | --- | -1.12 | 2.68E-03 |
| ADAMTS20 | 220717_at | NM_025003 | NM_025003 | 80070 | -1.12 | 3.36E-03 |
| | | | NM_175851 | | | |
| WDFY3 | 212602_at | AI806395 | NM_014991 | 23001 | -1.12 | 1.05E-05 |
| | | | NM_178583 | | | |
| | | | NM_178585 | | | |
| C1orf8 | 241018_at | BF446758 | NM_004872 | 9528 | -1.12 | 3.93E-03 |
| CLTC | 220855_at | NM_014127 | NM_004859 | 1213 | -1.11 | 8.87E-04 |
| BC035773 | 1569665_at | BC035773 | --- | --- | -1.11 | 8.41E-03 |
| KDELR2 | 242725_at | BG026159 | NM_006854 | 11014 | -1.11 | 8.62E-03 |
| SMAD4 | 1565703_at | AL832789 | NM_005359 | 4089 | -1.11 | 6.90E-03 |
| SIRT5 | 221010_s_at | NM_031244 | NM_012241 | 23408 | -1.11 | 4.63E-03 |
| | | | NM_031244 | | | |
| MAD | 226275_at | AI188653 | NM_002357 | 4084 | -1.11 | 1.65E-03 |
| ARSD | 223695_s_at | BC003660 | NM_001669 | 414 | -1.11 | 8.01E-03 |
| | | | NM_009589 | | | |
| LOC284361 | 224727_at | AL045545 | NM_175063 | 284361 | -1.11 | 1.48E-03 |
| | | | NM_206538 | | | |
| DMPK | 37996_s_at | L08835 | NM_004409 | 1760 | -1.11 | 9.75E-05 |
| HCG8 | 215985_at | X92110 | --- | 80869 | -1.11 | 6.05E-03 |
| MGC15875 | 232488_at | AK023470 | NM_032921 | 85007 | -1.11 | 4.98E-04 |
| | | | NM_153373 | | | |
| PLD3 | 201050_at | NM_012268 | NM_012268 | 23646 | -1.11 | 9.93E-04 |
| C20orf35 | 238470_at | BF129779 | NM_018478 | 55861 | -1.11 | 8.61E-04 |
| | | | NM_033542 | | | |
| AA573901 | 227783_at | AA573901 | --- | --- | -1.11 | 5.51E-04 |
| PROX1 | 242119_at | AW451938 | NM_002763 | 5629 | -1.11 | 2.23E-03 |
| AI862542 | 235696_at | AI862542 | --- | --- | -1.11 | 2.47E-03 |
| ZNF596 | 232641_at | AC004908 | NM_173539 | 169270 | -1.11 | 3.61E-03 |
| GOLGIN-67 | 210424_s_at | AF163441 | NM_015003 | 23015 | -1.10 | 3.73E-04 |
| | | | NM_181076 | | | |
| | | | NM_181077 | | | |
| JMY | 226352_at | BF447037 | NM_152405 | 133746 | -1.10 | 1.15E-05 |
| APXL2 | 239435_x_at | AA588854 | NM_133456 | 134549 | -1.10 | 2.97E-03 |
| BC004287 | 223366_at | BC004287 | --- | --- | -1.10 | 3.09E-03 |
| NM_025120 | 220728_at | NM_025120 | --- | --- | -1.10 | 4.73E-03 |
| BF510762 | 230262_at | BF510762 | --- | --- | -1.10 | 2.62E-04 |
| AI741900 | 239140_at | AI741900 | --- | --- | -1.10 | 7.72E-05 |
| INADL | 223681_s_at | AB044807 | NM_005799 | 10207 | -1.10 | 3.79E-04 |
| | | | NM_170605 | | | |
| | | | NM_176877 | | | |
| | | | NM_176878 | | | |
| PCDHB14 | 231726_at | NM_018934 | NM_018934 | 56122 | -1.10 | 7.91E-03 |
| AI435590 | 237193_s_at | AI435590 | --- | --- | -1.10 | 9.64E-03 |
| NPIP | 204538_x_at | NM_006985 | NM_006985 | 9284 | -1.10 | 2.65E-04 |
| SR140 | 236696_at | BE464843 | XM_031553 | 23350 | -1.09 | 5.65E-03 |
| CCNL2 | 221427_s_at | NM_030937 | NM_030937 | 81669 | -1.09 | 1.71E-03 |
| GFRA3 | 214479_at | NM_001496 | NM_001496 | 2676 | -1.09 | 9.83E-03 |
| AI161338 | 65472_at | AI161338 | XM_371506 | 388969 | -1.09 | 3.32E-03 |
| AF164622 | 210425_x_at | AF164622 | --- | --- | -1.09 | 5.81E-04 |
| SLC36A1 | 213119_at | AW058600 | NM_078483 | 206358 | -1.09 | 1.68E-03 |
| NOR1 | 227359_at | AI911248 | NM_145047 | 127700 | -1.09 | 2.57E-03 |
| | | | NM_206837 | | | |
| UBXD1 | 220757_s_at | NM_025241 | NM_025241 | 80700 | -1.09 | 1.00E-03 |
| C20orf103 | 219463_at | NM_012261 | NM_012261 | 24141 | -1.09 | 5.61E-03 |
| HERC1 | 240703_s_at | AW591969 | NM_003922 | 8925 | -1.09 | 1.25E-03 |
| FLJ25952 | 243786_at | AA454190 | NM_153251 | 253832 | -1.09 | 1.88E-03 |
| AK023264 | 233068_at | AK023264 | --- | --- | -1.08 | 5.48E-03 |
| UBE2D3 | 230171_at | AW662789 | NM_003340 | 7323 | -1.08 | 3.01E-03 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| PRKCSH | 200707_at | NM_002743 | NM_00100132 9 | 5589 | -1.08 | 1.56E-03 |
| | | | NM_002743 | | | |
| BRUNOL5 | 230497_at | BE503640 | NM_021938 | 60680 | -1.08 | 1.26E-03 |
| FLJ35390 | 1558123_at | AK092709 | --- | 255031 | -1.08 | 6.65E-03 |
| IPO9 | 225728_at | AI659533 | NM_018085 | 55705 | -1.08 | 2.88E-03 |
| HNRPC | 1568941_a_at | BC022884 | NM_004500 | 3183 | -1.08 | 3.18E-03 |
| | | | NM_031314 | | | |
| MCM3AP | 215581_s_at | AK022303 | NM_003906 | 8888 | -1.08 | 1.75E-03 |
| LOC399491 | 215921_at | AC002544 | --- | 399491 | -1.08 | 2.25E-04 |
| SMU1 | 228180_at | AA805653 | NM_018225 | 55234 | -1.08 | 3.97E-03 |
| AKT2 | 236664_at | AA448167 | NM_001626 | 208 | -1.08 | 1.96E-03 |
| JAK1 | 234193_at | AK025218 | NM_002227 | 3716 | -1.08 | 2.70E-03 |
| INADL | 214705_at | AJ001306 | NM_005799 | 10207 | -1.08 | 9.90E-04 |
| | | | NM_170605 | | | |
| | | | NM_176877 | | | |
| | | | NM_176878 | | | |
| C9orf10OS | 1558761_a_at | AK093641 | NM_198841 | 158293 | -1.08 | 2.09E-05 |
| H2AFY | 226840_at | AW291297 | NM_004893 | 9555 | -1.08 | 1.19E-03 |
| | | | NM_138609 | | | |
| | | | NM_138610 | | | |
| DUSP16 | 224832_at | AB051487 | NM_030640 | 80824 | -1.08 | 1.39E-04 |
| AL390216 | 234317_s_at | AL390216 | --- | --- | -1.07 | 4.32E-05 |
| PLA2G4B | 219095_at | NM_005090 | NM_005090 | 8681 | -1.07 | 7.70E-05 |
| AF131844 | 222184_at | AF131844 | --- | --- | -1.07 | 1.01E-03 |
| AI939452 | 240892_at | AI939452 | --- | --- | -1.07 | 2.32E-03 |
| ANKHD1 | 229457_at | AU148042 | NM_017747 | 54882 | -1.07 | 1.15E-03 |
| | | | NM_017978 | | | |
| | | | NM_024668 | | | |
| AI916303 | 228207_at | AI916303 | --- | --- | -1.07 | 4.41E-04 |
| AA033699 | 229111_at | AA033699 | --- | --- | -1.07 | 9.78E-03 |
| GOLGIN-67 | 208798_x_at | AF204231 | NM_015003 | 23015 | -1.07 | 8.11E-04 |
| | | | NM_181076 | | | |
| | | | NM_181077 | | | |
| SYNE1 | 209447_at | AF043290 | NM_015293 | 23345 | -1.07 | 2.21E-03 |
| | | | NM_033071 | | | |
| | | | NM_133650 | | | |
| | | | NM_182961 | | | |
| AL080112 | 216858_x_at | AL080112 | --- | --- | -1.07 | 8.82E-03 |
| MAP7 | 215471_s_at | AJ242502 | NM_003980 | 9053 | -1.07 | 7.77E-04 |
| BC025319 | 1568974_at | BC025319 | --- | --- | -1.06 | 9.12E-03 |
| DJ328E19.C1. 1 | 214693_x_at | BE732345 | NM_015383 | 200030 | -1.06 | 3.98E-04 |
| | | | NM_173638 | 25832 | | |
| LOC200030 | | | NM_183372 | 284565 | | |
| MGC8902 | | | XM_371384 | 401967 | | |
| LOC401967 | | | XM_496394 | 440673 | | |
| AG1 | | | XM_496406 | | | |
| PIGF | 239258_at | BE551407 | NM_002643 | 5281 | -1.06 | 3.15E-04 |
| | | | NM_173074 | | | |
| NPIP | 214870_x_at | AC002045 | NM_006985 | 339047 | -1.06 | 2.19E-04 |
| LOC339047 | | | XM_290671 | 440341 | | |
| LOC440341 | | | XM_352159 | 9284 | | |
| KIF5C | 203129_s_at | BF059313 | XM_377774 | 3800 | -1.06 | 2.07E-03 |
| KCTD13 | 45653_at | AW026481 | NM_178863 | 253980 | -1.06 | 5.88E-06 |
| FLJ10287 | 219130_at | NM_019083 | NM_019083 | 54482 | -1.06 | 3.45E-03 |
| PSMD7 | 244515_at | AI640348 | NM_002811 | 5713 | -1.06 | 5.80E-03 |
| AK3 | 204347_at | AI653169 | NM_00100292 1 | 205 | -1.06 | 8.16E-03 |
| | | | | 387851 | | |
| | | | NM_00100535 3 | | | |
| | | | NM_013410 | | | |
| | | | NM_203464 | | | |
| BACE1 | 217904_s_at | NM_012104 | NM_012104 | 23621 | -1.06 | 1.51E-04 |
| | | | NM_138971 | | | |
| | | | NM_138972 | | | |
| | | | NM_138973 | | | |
| WDFY3 | 212598_at | BE348236 | NM_014991 | 23001 | -1.06 | 4.22E-03 |
| | | | NM_178583 | | | |
| | | | NM_178585 | | | |
| CLCN3 | 201735_s_at | NM_001829 | NM_001829 | 1182 | -1.06 | 2.95E-03 |
| | | | NM_173872 | | | |
| PHACTR1 | 213638_at | AW054711 | NM_030948 | 221692 | -1.06 | 8.09E-03 |
| PPIE | 229375_at | AL526713 | NM_006112 | 10450 | -1.06 | 2.02E-06 |
| | | | NM_203456 | | | |
| | | | NM_203457 | | | |
| C10orf57 | 218174_s_at | NM_025125 | NM_025125 | 80195 | -1.06 | 8.98E-04 |
| PEX6 | 238672_at | AW953952 | NM_000287 | 5190 | -1.06 | 6.15E-04 |
| BDH | 211715_s_at | BC005844 | NM_004051 | 622 | -1.06 | 4.14E-04 |
| | | | NM_203314 | | | |
| | | | NM_203315 | | | |
| BF110411 | 221878_at | BF110411 | XM_371506 | 388969 | -1.05 | 8.17E-03 |
| R93413 | 236590_at | R93413 | | --- | -1.05 | 6.60E-04 |
| FLJ36674 | 244698_at | AW297656 | NM_173622 | 284040 | -1.05 | 4.58E-03 |
| AA729232 | 241359_at | AA729232 | | --- | -1.05 | 9.24E-03 |
| FLJ21159 | 235550_at | AV751613 | NM_024826 | 79884 | -1.05 | 4.53E-03 |
| SMPD1 | 209420_s_at | M59916 | NM_000543 | 6609 | -1.05 | 8.56E-04 |
| | | | NM_00100759 3 | | | |
| AA281853 | 239505_at | AA281853 | | --- | -1.05 | 8.55E-04 |
| KCNMA1 | 221584_s_at | U11058 | NM_002247 | 3778 | -1.05 | 7.34E-05 |
| LOC439928 | 1569519_at | BC039454 | XM_498427 | 439928 | -1.05 | 2.09E-03 |
| PAM | 202336_s_at | NM_000919 | NM_000919 | 5066 | -1.05 | 1.27E-03 |
| | | | NM_138766 | | | |
| | | | NM_138821 | | | |
| | | | NM_138822 | | | |
| DCTD | 201571_s_at | AI656493 | NM_001921 | 1635 | -1.05 | 9.35E-05 |
| ZNF573 | 217627_at | BE515346 | NM_152360 | 126231 | -1.05 | 5.34E-04 |
| WDR19 | 232163_at | AI982884 | NM_025132 | 57728 | -1.05 | 4.32E-04 |
| ETFDH | 33494_at | S69232 | NM_004453 | 2110 | -1.05 | 1.51E-03 |
| RRAGC | 222514_at | AK023373 | NM_022157 | 64121 | -1.05 | 7.18E-03 |
| C14orf139 | 219563_at | NM_024633 | NM_024633 | 79686 | -1.05 | 7.66E-03 |
| DNASE1 | 1560536_at | BC035759 | NM_005223 | 1773 | -1.05 | 1.80E-03 |
| D17262 | 1567997_x_at | D17262 | | --- | -1.04 | 5.43E-03 |
| BTG3 | 215425_at | AL049332 | NM_006806 | 10950 | -1.04 | 5.78E-04 |
| ATE1 | 238252_at | AI040743 | NM_00100197 6 | 11101 | -1.04 | 5.92E-04 |
| | | | NM_007041 | | | |
| KCTD13 | 221889_at | AW026481 | NM_178863 | 253980 | -1.04 | 1.16E-05 |
| RRAGC | 218088_s_at | NM_022157 | NM_022157 | 64121 | -1.04 | 8.27E-04 |
| ZZEF1 | 212601_at | AB007859 | NM_015113 | 23140 | -1.04 | 4.08E-03 |
| BCL11B | 222895_s_at | AA918317 | NM_022898 | 64919 | -1.04 | 6.07E-04 |
| | | | NM_138576 | | | |
| KIAA0999 | 1563455_at | AL832068 | NM_025164 | 23387 | -1.04 | 9.56E-03 |
| ZFYVE26 | 37943_at | AB002319 | NM_015346 | 23503 | -1.04 | 9.36E-03 |
| NSUN6 | 242239_at | AW970888 | NM_182543 | 221078 | -1.04 | 8.03E-03 |
| BE674309 | 230913_at | BE674309 | --- | --- | -1.04 | 1.59E-04 |
| AA910371 | 212952_at | AA910371 | --- | --- | -1.04 | 3.15E-03 |
| C21orf51 | 228239_at | AA148789 | NM_058182 | 54065 | -1.04 | 6.43E-06 |
| PPT1 | 200975_at | NM_000310 | NM_000310 | 5538 | -1.04 | 2.75E-05 |
| TTC3 | 208663_s_at | AI652848 | NM_00100189 4 | 7267 | -1.04 | 9.53E-06 |
| | | | NM_003316 | | | |
| ZCSL3 | 1570048_at | BC036571 | NM_181706 | 120526 | -1.04 | 6.02E-03 |
| ZNF3 | 232497_at | AF217988 | NM_017715 | 7551 | -1.04 | 7.84E-04 |
| | | | NM_032924 | | | |
| RRN3 | 230328_at | BF057656 | NM_018427 | 54700 | -1.04 | 1.78E-03 |
| FTH1 | 214211_at | AA083483 | NM_002032 | 2495 | -1.03 | 2.78E-03 |
| SERF2 | 226692_at | AI092931 | NM_005770 | 10169 | -1.03 | 3.64E-03 |
| MTCP1 | 239236_at | BE465277 | NM_014221 | 4515 | -1.03 | 2.24E-03 |
| PDCD2 | 228420_at | AW590850 | NM_002598 | 5134 | -1.03 | 8.82E-03 |
| | | | NM_144781 | | | |
| SPOCK2 | 202524_s_at | NM_014767 | NM_014767 | 9806 | -1.03 | 2.19E-06 |
| CYLD | 221905_at | BF516433 | NM_015247 | 1540 | -1.03 | 1.43E-04 |
| IREB2 | 1563130_a_at | AL109710 | NM_004136 | 3658 | -1.03 | 3.66E-03 |
| HRMT1L1 | 221564_at | AL570294 | NM_001535 | 3275 | -1.03 | 1.20E-06 |
| | | | NM_206962 | | | |
| DDOST | 208674_x_at | BC002594 | NM_005216 | 1650 | -1.03 | 1.01E-03 |
| LOC339047 | 214682_at | AK023376 | XM_290671 | 339047 | -1.02 | 4.06E-03 |
| MGC15875 | 226519_s_at | AL561859 | NM_032921 | 85007 | -1.02 | 1.51E-03 |
| | | | NM_153373 | | | |
| HNRPM | 233605_x_at | AK022050 | NM_005968 | 4670 | -1.02 | 3.07E-03 |
| | | | NM_031203 | | | |
| LOC144363 | 225469_at | AA015609 | NM_00100166C | 144363 | -1.02 | 6.55E-04 |
| ZNF346 | 232753_at | AU147613 | NM_012279 | 23567 | -1.02 | 9.40E-04 |
| C3orf6 | 228693_at | AI681307 | NM_174908 | 152137 | -1.02 | 2.49E-03 |
| | | | NM_178335 | | | |
| AK024681 | 231972_at | AK024681 | --- | --- | -1.02 | 1.17E-03 |
| UBXD1 | 223012_at | AF272894 | NM_025241 | 80700 | -1.02 | 3.65E-04 |
| HERC4 | 225988_at | AI819938 | NM_015601 | 26091 | -1.02 | 2.15E-03 |
| MYO5A | 227761_at | AW235548 | NM_000259 | 4644 | -1.02 | 8.53E-03 |
| LTBP3 | 227308_x_at | AW515704 | NM_021070 | 4054 | -1.02 | 6.79E-07 |
| C14orf118 | 229520_s_at | BF060678 | NM_017926 | 55668 | -1.02 | 4.18E-03 |
| | | | NM_017972 | | | |
| RHOU | 223169_s_at | AB051826 | NM_021205 | 58480 | -1.02 | 9.28E-03 |
| SH3BGR | 204979_s_at | NM_007341 | NM_00100171 3 | 6450 | -1.02 | 1.78E-03 |
| | | | NM_007341 | | | |
| BC014481 | 1569368_at | BC014481 | --- | --- | -1.02 | 3.54E-03 |
| LOC158230 | 243595_at | AI589280 | --- | 158230 | -1.02 | 4.74E-04 |
| BC031322 | 1555900_at | BC031322 | --- | --- | -1.02 | 1.29E-03 |
| SPG3A | 223340_at | AF131801 | NM_015915 | 51062 | -1.02 | 7.49E-03 |
| | | | NM_181598 | | | |
| AA448858 | 238948_at | AA448858 | --- | --- | -1.02 | 3.09E-03 |
| MGC33486 | 235614_at | AI215667 | NM_153266 | 256472 | -1.01 | 2.05E-05 |
| M78162 | 217536_x_at | M78162 | --- | --- | -1.01 | 4.51E-03 |
| PHKA1 | 229876_at | BE503584 | NM_002637 | 5255 | -1.01 | 4.38E-04 |
| STX6 | 244041_at | AI884934 | NM_005819 | 10228 | -1.01 | 8.65E-03 |
| FLJ23518 | 233400_at | AU158247 | NM_024725 | 79780 | -1.01 | 3.50E-03 |
| PPIE | 210502_s_at | AF042386 | NM_006112 | 10450 | -1.01 | 4.24E-03 |
| | | | NM_203456 | | | |
| | | | NM_203457 | | | |
| DDOST | 208675_s_at | D29643 | NM_005216 | 1650 | -1.01 | 2.59E-03 |
| OPTN | 202073_at | AV757675 | NM_00100821 1 | 10133 | -1.01 | 2.57E-05 |
| | | | NM_00100821 2 | | | |
| | | | NM_00100821 3 | | | |
| | | | NM_021980 | | | |
| Cep164 | 1558953_s_at | BC000602 | NM_014956 | 22897 | -1.01 | 1.37E-03 |
| SLC19A2 | 1556602_at | AW614839 | NM_006996 | 10560 | -1.00 | 2.90E-03 |
| DMPK | 217066_s_at | M87313 | NM_004409 | 1760 | -1.00 | 8.62E-03 |
| SFRS15 | 222311_s_at | AA648521 | NM_020706 | 57466 | -1.00 | 2.57E-03 |
| LYPLAL1 | 230174_at | AI953360 | NM_138794 | 127018 | -1.00 | 3.17E-03 |
| AK000970 | 215512_at | AK000970 | NM_005885 | 10299 | -1.00 | 2.13E-03 |
| LOC492304 | 202409_at | X07868 | NM_001007139 | 492304 | -1.00 | 1.87E-03 |
| PIGH | 209625_at | BC004100 | NM_004569 | 5283 | -1.00 | 6.00E-07 |
| MEIS3 | 1569402_at | BC014430 | NM_00100981 3 | 56917 | -1.00 | 8.61E-03 |
| | | | NM_020160 | | | |
| DDX52 | 1570415_at | BC012557 | NM_007010 | 11056 | -1.00 | 1.89E-03 |
| | | | NM_152300 | | | |
| DTNA | 211493_x_at | U46744 | NM_001390 | 1837 | -1.00 | 7.35E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| EIF5 | 208707_at | BE552334 | NM_001969 | 1983 | -1.00 | 8.81E-03 |
| | | | NM_183004 | | | |
| ADRBK2 | FFX-hum_alu_i | AFFX-hum_alu | NM_000021 | 10001 | -1.00 | 5.84E-04 |
| ALDH3B1 | | | NM_000051 | 10020 | | |
| ALDH3A2 | | | NM_000136 | 10045 | | |
| ANXA2 | | | NM_000141 | 10160 | | |
| BIRC4 | | | NM_000144 | 10197 | | |
| APP | | | NM_000202 | 10198 | | |
| ATM | | | NM_000227 | 10206 | | |
| ATOX1 | | | NM_000230 | 10207 | | |
| ATP1B3 | | | NM_000294 | 1021 | | |
| ATP5D | | | NM_000297 | 10225 | | |
| BAD | | | NM_000314 | 10277 | | |
| BCAT2 | | | NM_000356 | 10402 | | |
| BDNF | | | NM_000364 | 10457 | | |
| BMPR2 | | | NM_000382 | 10513 | | |
| BRCA1 | | | NM_000441 | 10524 | | |
| C11orf3 | | | NM_000484 | 10606 | | |
| C21orf4 | | | NM_000527 | 10609 | | |
| CACNB2 | | | NM_000554 | 10611 | | |
| CCM1 | | | | 10712 | | |
| CD47 | | | | 10730 | | |
| CDK6 | | | | 10801 | | |
| CHKA | | | | 10806 | | |
| CHRNB1 | | | | 10848 | | |
| AP3S1 | | | | 10921 | | |
| SLC31A1 | | | | 11011 | | |
| CP | | | | 11019 | | |
| DJ462O23.2 | 225876_at | T84558 | NM_020448 | 57185 | -1.00 | 6.61E-04 |
| BE467087 | 231075_x_at | BE467087 | --- | --- | -1.00 | 8.51E-03 |
| ITSN1 | 216472_at | AF003737 | NM_00100113 2 | 6453 | -1.00 | 2.41E-03 |
| | | | NM_003024 | | | |
| DMWD | 213231_at | L19267 | XM_496318 | 1762 | -1.00 | 1.12E-03 |
| RPL37 | 224767_at | AL044126 | NM_000997 | 6167 | -1.00 | 1.07E-03 |
| LMO4 | 229537_at | AI694521 | NM_006769 | 8543 | -1.00 | 2.20E-03 |
| ACE | 227463_at | AW057540 | NM_000789 | 1636 | -1.00 | 1.34E-03 |
| | | | NM_152830 | | | |
| | | | NM_152831 | | | |
| KIAA1345 | 234936_s_at | AK023876 | XM_106386 | 57545 | -0.99 | 1.60E-03 |
| AG1 | 215434_x_at | AV684285 | XM_371384 | 440673 | -0.99 | 1.70E-03 |
| | | | XM_496394 | | | |
| BE645588 | 243429_at | BE645588 | --- | --- | -0.99 | 7.12E-03 |
| PAM | 214620_x_at | BF038548 | NM_000919 | 5066 | -0.99 | 4.07E-03 |
| | | | NM_138766 | | | |
| | | | NM_138821 | | | |
| | | | NM_138822 | | | |
| LRRFIP1 | 201861_s_at | BF965566 | NM_004735 | 9208 | -0.99 | 7.92E-06 |
| RAD50 | 238656_at | AA877043 | NM_005732 | 10111 | -0.99 | 8.65E-03 |
| | | | NM_133482 | | | |
| LOC338758 | 238893_at | AI377324 | --- | 338758 | -0.99 | 2.11E-03 |
| BE856007 | 238676_at | BE856007 | --- | --- | -0.99 | 5.32E-06 |
| Lrp2bp | 227337_at | AA886870 | NM_181726 | 353322 | -0.99 | 6.37E-04 |
| SMURF2 | 227489_at | BE962027 | NM_022739 | 64750 | -0.99 | 4.11E-04 |
| PGRMC2 | 213227_at | BE879873 | NM_006320 | 10424 | -0.99 | 1.98E-03 |
| TMEM41B | 212623_at | AU153138 | --- | 23027 | -0.99 | 8.63E-03 |
| BE670056 | 226663_at | BE670056 | --- | --- | -0.99 | 6.70E-03 |
| MYO9B | 242742_at | AI863135 | NM_004145 | 4650 | -0.99 | 3.16E-03 |
| DKFZp313N062 | 241666_at | BG493222 | NM_173826 | 285343 | -0.99 | 1.70E-03 |
| HK2 | 202934_at | AI761561 | NM_000189 | 3099 | -0.99 | 4.09E-03 |
| DKFZp547I014 | 207283_at | NM_020217 | NM_020217 | 56969 | -0.99 | 6.98E-04 |
| MAP1LC3B | 208786_s_at | AF183417 | NM_022818 | 81631 | -0.99 | 3.39E-03 |
| AKAP12 | 227530_at | BF511276 | NM_005100 | 9590 | -0.99 | 7.30E-03 |
| | | | NM_144497 | | | |
| AB014532 | 222796_at | AB014532 | --- | --- | -0.98 | 6.13E-04 |
| C1orf50 | 219406_at | NM_024097 | NM_024097 | 79078 | -0.98 | 2.14E-04 |
| RPL13 | 1565759_at | BG396520 | NM_000977 | 6137 | -0.98 | 2.65E-04 |
| | | | NM_033251 | | | |
| MGC43690 | 235298_at | AW194823 | NM_182552 | 253769 | -0.98 | 3.94E-04 |
| AF086554 | 1558504_at | AF086554 | XM_373889 | 440721 | -0.98 | 2.45E-03 |
| FLJ11196 | 218651_s_at | NM_018357 | NM_018357 | 55323 | -0.98 | 1.86E-03 |
| | | | NM_197958 | | | |
| C17orf28 | 225981_at | AW139549 | NM_030630 | 283987 | -0.98 | 3.41E-04 |
| SFXN3 | 1559993_at | AK091504 | NM_030971 | 81855 | -0.98 | 4.54E-03 |
| C6orf111 | 226412_at | N66397 | NM_032870 | 25957 | -0.98 | 1.98E-03 |
| RPL13 | 229590_at | AI369389 | NM_000977 | 6137 | -0.98 | 1.00E-03 |
| | | | NM_033251 | | | |
| LOC133308 | 1564746_at | BC009732 | NM_178833 | 133308 | -0.98 | 1.21E-04 |
| AK027185 | 232102_at | AK027185 | --- | --- | -0.98 | 1.28E-04 |
| AW290940 | 226773_at | AW290940 | --- | --- | -0.98 | 1.34E-04 |
| AB040946 | 222564_at | AB040946 | --- | --- | -0.98 | 1.34E-03 |
| ILVBL | 202993_at | NM_006844 | NM_006844 | 10994 | -0.97 | 1.11E-03 |
| | | | NM_176826 | | | |
| NPR2 | 204310_s_at | NM_003995 | NM_000907 | 4882 | -0.97 | 1.05E-03 |
| | | | NM_003995 | | | |
| PAM | 212958_x_at | AI022882 | NM_000919 | 5066 | -0.97 | 2.87E-03 |
| | | | NM_138766 | | | |
| | | | NM_138821 | | | |
| | | | NM_138822 | | | |
| F11066 | 242973_at | F11066 | --- | --- | -0.97 | 5.82E-03 |
| SPIN3 | 1554099_a_at | BC032490 | NM_001010862 | 169981 | -0.97 | 4.95E-05 |
| AA167167 | 243684_at | AA167167 | --- | --- | -0.97 | 1.78E-03 |
| LANCL2 | 218219_s_at | NM_018697 | NM_018697 | 55915 | -0.97 | 9.88E-03 |
| AFFX-BioB-3 | AFFX-BioB-3_a | AFFX-BioB-3 | --- | --- | -0.97 | 1.52E-04 |
| AHI1 | 220841_s_at | NM_017651 | NM_017651 | 54806 | -0.97 | 4.15E-04 |
| AI675308 | 1569154_a_at | AI675308 | --- | --- | -0.97 | 2.61E-03 |
| AI339837 | 241741_at | AI339837 | --- | --- | -0.97 | 9.92E-03 |
| HNLF | 238525_at | BF987157 | NM_182547 | 222068 | -0.97 | 3.73E-05 |
| NM_024658 | 218305_at | NM_024658 | --- | --- | -0.97 | 8.21E-03 |
| FLJ30294 | 238867_at | AI298041 | NM_144632 | 130827 | -0.97 | 9.74E-03 |
| FBXO9 | 1559096_x_at | AK095307 | NM_012347 | 26268 | -0.97 | 6.38E-05 |
| | | | NM_033480 | | | |
| | | | NM_033481 | | | |
| PLP2 | 201136_at | NM_002668 | NM_002668 | 5355 | -0.97 | 1.29E-04 |
| ZNF542 | 229187_at | AI026708 | NM_194319 | 147947 | -0.97 | 7.05E-03 |
| TTLL3 | 210129_s_at | AF078842 | NM_015644 | 26140 | -0.97 | 6.31E-03 |
| D4S234E | 213533_at | M98528 | NM_014392 | 27065 | -0.97 | 1.57E-04 |
| SHC2 | 213464_at | AV705938 | XM_375550 | 25759 | -0.97 | 4.07E-06 |
| GCC1 | 243306_s_at | BE276093 | NM_024523 | 79571 | -0.97 | 2.70E-07 |
| TSGA2 | 230093_at | AI683428 | NM_080860 | 89765 | -0.97 | 4.32E-03 |
| HDLBP | 222916_s_at | AF116718 | NM_005336 | 3069 | -0.97 | 5.58E-03 |
| | | | NM_203346 | | | |
| C9orf91 | 221865_at | BF969986 | NM_153045 | 203197 | -0.96 | 6.15E-03 |
| CYorf15B | 223646_s_at | AF332225 | NM_032576 | 84663 | -0.96 | 4.37E-03 |
| LTB4R | 236172_at | AW206817 | NM_181657 | 1241 | -0.96 | 5.00E-03 |
| MGC11332 | 213403_at | BF223370 | NM_032718 | 84804 | -0.96 | 5.81E-03 |
| SPIRE2 | 227706_at | AI215798 | NM_032451 | 84501 | -0.96 | 7.98E-05 |
| AU146901 | 233264_at | AU146901 | --- | --- | -0.96 | 1.84E-03 |
| BG000693 | 1559932_at | BG000693 | --- | --- | -0.96 | 1.63E-04 |
| FLJ20758 | 228512_at | AW138833 | NM_017952 | 55037 | -0.96 | 1.99E-03 |
| UNC5D | 231325_at | AI802048 | NM_080872 | 137970 | -0.96 | 4.13E-05 |
| THRAP2 | 242911_at | AW999644 | NM_015335 | 23389 | -0.96 | 5.60E-03 |
| CLDN15 | 219640_at | NM_014343 | NM_014343 | 24146 | -0.96 | 1.04E-03 |
| | | | NM_138429 | | | |
| DUSP16 | 1558740_s_at | R30807 | NM_030640 | 80824 | -0.96 | 9.85E-03 |
| TMEM1 | 209412_at | U61500 | NM_00100172 3 | 7109 | -0.96 | 5.34E-03 |
| | | | NM_003274 | | | |
| DDX26 | 222239_s_at | AL117626 | NM_012141 | 26512 | -0.95 | 1.60E-03 |
| LOC388526 | 235322_at | BF038869 | XM_371157 | 388526 | -0.95 | 1.09E-04 |
| OS-9 | 215399_s_at | AI683900 | NM_006812 | 10956 | -0.95 | 7.81E-03 |
| PRKCSH | 214080_x_at | AI815793 | NM_00100132 9 | 5589 | -0.95 | 2.14E-04 |
| | | | NM_002743 | | | |
| BF976290 | 238768_at | BF976290 | XM_371506 | 388969 | -0.95 | 9.91E-04 |
| FLJ14107 | 207287_at | NM_025026 | NM_025026 | 80094 | -0.95 | 1.43E-05 |
| ARHGAP23 | 226638_at | AV701292 | XM_290799 | 57636 | -0.95 | 3.20E-04 |
| AI252004 | 242696_at | AI252004 | --- | --- | -0.95 | 2.67E-03 |
| TGFB1I1 | 209651_at | BC001830 | NM_015927 | 7041 | -0.95 | 7.45E-03 |
| AI598222 | 235679_at | AI598222 | --- | --- | -0.95 | 1.26E-03 |
| AA131302 | 236032_at | AA131302 | --- | --- | -0.95 | 5.35E-04 |
| AI929792 | 236081_at | AI929792 | --- | --- | -0.95 | 6.74E-05 |
| N453I2 | 228159_at | N45312 | --- | --- | -0.95 | 5.76E-04 |
| MONDOA | 202519_at | NM_014938 | NM_014938 | 22877 | -0.95 | 6.48E-04 |
| PLCB4 | 203895_at | AL535113 | NM_000933 | 5332 | -0.95 | 8.09E-05 |
| | | | NM_182797 | | | |
| RNF149 | 225414_at | AL558987 | NM_173647 | 284996 | -0.95 | 9.45E-03 |
| DNASE1 | 1557189_at | AW468509 | NM_005223 | 1773 | -0.95 | 4.38E-04 |
| OS-9 | 200714_x_at | NM_006812 | NM_006812 | 10956 | -0.95 | 4.30E-03 |
| CCNL2 | 222999_s_at | AF251294 | NM_030937 | 81669 | -0.95 | 5.76E-03 |
| NPD014 | 209006_s_at | AF247168 | NM_020317 | 57035 | -0.95 | 1.08E-03 |
| | | | NM_207035 | | | |
| LOC154822 | 243261_at | BF530486 | --- | 154822 | -0.94 | 6.74E-03 |
| TARSL2 | 227611_at | AA442856 | NM_152334 | 123283 | -0.94 | 4.59E-03 |
| AA827805 | 241364_at | AA827805 | --- | --- | -0.94 | 9.38E-03 |
| KRT18 | 201596_x_at | NM_000224 | NM_000224 | 3875 | -0.94 | 2.40E-03 |
| | | | NM_199187 | | | |
| CYLD | 213295_at | AA555096 | NM_015247 | 1540 | -0.94 | 2.37E-04 |
| BC011119 | 1569723_a_at | BC011119 | --- | --- | -0.94 | 7.41E-03 |
| PHF17 | 225820_at | AV646599 | NM_024900 | 79960 | -0.94 | 3.79E-03 |
| | | | NM_199320 | | | |
| C10orf104 | 229145_at | AA541762 | NM_173473 | 119504 | -0.94 | 1.46E-03 |
| BF062399 | 240141_at | BF062399 | --- | --- | -0.94 | 1.03E-03 |
| LOC286437 | 239762_at | AI080505 | --- | 286437 | -0.94 | 1.26E-04 |
| AA714835 | 1556646_at | AA714835 | --- | --- | -0.94 | 6.42E-04 |
| MGC3222 | 233480_at | AK026869 | NM_024334 | 79188 | -0.94 | 1.32E-03 |
| NEDD4 | 213012_at | D42055 | NM_006154 | 4734 | -0.94 | 3.31E-03 |
| | | | NM_198400 | | | |
| MGC26979 | 232023_at | AL575584 | NM_153704 | 91147 | -0.94 | 2.50E-03 |
| BE669703 | 229541_at | BE669703 | --- | --- | -0.94 | 5.09E-04 |
| AW665287 | 243725_at | AW665287 | --- | --- | -0.94 | 8.33E-04 |
| SPG7 | 202104_s_at | NM_003119 | NM_003119 | 6687 | -0.94 | 4.93E-03 |
| | | | NM_199367 | | | |
| FFX-r2-Ec-bioB=X-r2-Ec-bioB-5AFFX-r2-Ec-bioB-5 | | | - | - | -0.94 | 1.31E-04 |
| AG1 | 212854_x_at | AB051480 | XM_371384 | 440673 | -0.94 | 2.84E-03 |
| | | | XM_496394 | | | |
| BF478154 | 242620_at | BF478154 | --- | --- | -0.94 | 2.31E-03 |
| PPM1K | 235061_at | AV706522 | NM_152542 | 152926 | -0.94 | 2.87E-04 |
| MDS010 | 218587_s_at | NM_020231 | NM_020231 | 56983 | -0.94 | 7.79E-03 |
| | | | NM_152305 | | | |
| RNF13 | 201779_s_at | AF070558 | NM_007282 | 11342 | -0.93 | 1.21E-06 |
| | | | NM_183381 | | | |
| | | | NM_183382 | | | |
| | | | NM_183383 | | | |
| | | | NM_183384 | | | |
| ATP8B2 | 226771_at | AB032963 | NM_00100585 5 | 57198 | -0.93 | 1.31E-06 |
| | | | NM_020452 | | | |
| DCTD | 210137_s_at | BC001286 | NM_001921 | 1635 | -0.93 | 4.45E-04 |
| LOC286260 | 232420_x_at | AA706480 | --- | 286260 | -0.93 | 3.00E-04 |
| IDS | 217432_s_at | AF179281 | NM_000202 | 3423 | -0.93 | 1.24E-03 |
| | | | NM_006123 | | | |
| ANKRD10 | 223251_s_at | BC001727 | NM_017664 | 55608 | -0.93 | 6.05E-05 |
| AK098354 | 1555824_a_at | AK098354 | --- | --- | -0.93 | 2.66E-04 |
| DOCK9 | 212538_at | AL576253 | NM_015296 | 23348 | -0.93 | 3.70E-03 |
| DRE1 | 206551_x_at | NM_017644 | NM_017644 | 54800 | -0.93 | 7.84E-04 |
| SNF1LK2 | 213221_s_at | AB018324 | NM_015191 | 23235 | -0.93 | 7.54E-03 |
| KIAA1546 | 227624_at | AB046766 | XM_042301 | 57667 | -0.93 | 7.15E-03 |
| AI417992 | 240513_at | AI417992 | --- | --- | -0.93 | 7.71E-03 |
| U2AF1 | 231904_at | AU122448 | NM_006758 | 7307 | -0.92 | 7.45E-04 |
| KIAA0492 | 215109_at | R02172 | XM_378914 | 57238 | -0.92 | 3.40E-03 |
| LOC158563 | 226273_at | AA218974 | --- | 158563 | -0.92 | 1.79E-04 |
| AF130054 | 211452_x_at | AF130054 | --- | --- | -0.92 | 4.58E-03 |
| NCOA7 | 225344_at | AL035689 | NM_181782 | 135112 | -0.92 | 1.11E-03 |
| LOC284356 | 1555842_at | BM978026 | --- | 284356 | -0.92 | 8.07E-03 |
| EXOSC3 | 242590_at | BE464643 | NM_00100226 9 | 51010 | -0.92 | 4.59E-03 |
| | | | NM_016042 | | | |
| MALAT1 | 224558_s_at | AI446756 | --- | 378938 | -0.92 | 8.89E-03 |
| C20orf44 | 229672_at | AA707125 | NM_018244 | 55245 | -0.92 | 1.02E-03 |
| | | | NM_199487 | | | |
| | | | NM_199513 | | | |
| N64760 | 212622_at | N64760 | --- | --- | -0.92 | 3.14E-04 |
| HRMT1L1 | 202098_s_at | NM_001535 | NM_001535 | 3275 | -0.92 | 5.36E-05 |
| | | | NM_206962 | | | |
| NEU1 | 208926_at | U84246 | NM_000434 | 4758 | -0.92 | 6.42E-03 |
| AF131777 | 215504_x_at | AF131777 | --- | --- | -0.92 | 7.86E-03 |
| PS1TP4 | 226381_at | AW450329 | --- | 414327 | -0.92 | 9.25E-03 |
| C3orf6 | 235051_at | BF696931 | NM_174908 | 152137 | -0.92 | 9.12E-03 |
| | | | NM_178335 | | | |
| MAP1LC3B | 208785_s_at | BE893893 | NM_022818 | 81631 | -0.91 | 1.87E-03 |
| NM_024900 | 218517_at | NM_024900 | --- | --- | -0.91 | 8.37E-03 |
| P4HA2 | 202733_at | NM_004199 | NM_004199 | 8974 | -0.91 | 4.34E-03 |
| MBTD1 | 207115_x_at | NM_017643 | NM_017643 | 54799 | -0.91 | 1.43E-03 |
| BF476403 | 230218_at | BF476403 | --- | --- | -0.91 | 2.19E-03 |
| AL080160 | 217446_x_at | AL080160 | --- | --- | -0.91 | 1.65E-03 |
| C19orf4 | 219005_at | NM_012109 | NM_012109 | 25789 | -0.91 | 5.96E-04 |
| LOC399491 | 214035_x_at | AA308853 | --- | 399491 | -0.91 | 6.27E-04 |
| CHPT1 | 1559739_at | AK025141 | NM_020244 | 56994 | -0.91 | 6.26E-03 |
| U2AF1L1 | 206512_at | NM_005083 | --- | 7310 | -0.91 | 8.01E-03 |
| C20orf44 | 244795_at | AV693986 | NM_018244 | 55245 | -0.91 | 4.26E-04 |
| | | | NM_199487 | | | |
| | | | NM_199513 | | | |
| LOC339047 | 221501_x_at | AF229069 | XM_290671 | 339047 | -0.91 | 2.33E-04 |
| FLJ35093 | 235125_x_at | AI078279 | NM_198549 | 374986 | -0.91 | 2.80E-03 |
| SLC22A17 | 218675_at | NM_020372 | NM_016609 | 51310 | -0.91 | 2.67E-06 |
| | | | NM_020372 | | | |
| EIF4A1 | 214805_at | U79273 | NM_001416 | 1973 | -0.91 | 7.74E-03 |
| Cep164 | 204251_s_at | NM_014956 | NM_014956 | 22897 | -0.91 | 4.21E-03 |
| AK2 | 212172_at | AW277253 | NM_001625 | 204 | -0.91 | 1.68E-04 |
| | | | NM_013411 | | | |
| | | | NM_172199 | | | |
| NFKB1 | 209239_at | M55643 | NM_003998 | 4790 | -0.91 | 2.66E-03 |
| AL833666 | 1566854_at | AL833666 | --- | --- | -0.91 | 3.74E-03 |
| BE857570 | 235677_at | BE857570 | --- | --- | -0.91 | 6.67E-04 |
| KIAA1276 | 227504_s_at | N64630 | XM_039169 | 27146 | -0.91 | 9.00E-05 |
| KIAA0590 | 204792_s_at | NM_014714 | NM_014714 | 9742 | -0.91 | 2.73E-03 |
| AV733292 | 225444_at | AV733292 | --- | --- | -0.91 | 9.09E-04 |
| BF057855 | 236829_at | BF057855 | --- | --- | -0.91 | 4.03E-03 |
| BC020820 | 1562962_at | BC020820 | --- | --- | -0.91 | 1.89E-04 |
| FFX-r2-Ec-bioB=X-r2-Ec-bioB-3AFFX-r2-Ec-bioB-3 | | | --- | --- | -0.91 | 4.89E-04 |
| AF086478 | 1558948_a_at | AF086478 | --- | --- | -0.91 | 5.21E-03 |
| PHF20L1 | 222133_s_at | AK022280 | NM_016018 | 51105 | -0.90 | 9.98E-03 |
| | | | NM_024878 | | | |
| | | | NM_032205 | | | |
| | | | NM_198513 | | | |
| HERC4 | 208055_s_at | NM_015601 | NM_015601 | 26091 | -0.90 | 1.92E-03 |
| R42552 | 239718_at | R42552 | --- | --- | -0.90 | 1.67E-03 |
| FBXO9 | 1559094_at | AK095307 | NM_012347 | 26268 | -0.90 | 1.55E-04 |
| | | | NM_033480 | | | |
| | | | NM_033481 | | | |
| OSBP | 1563051_at | BC017975 | NM_002556 | 5007 | -0.90 | 8.83E-03 |
| GPR27 | 227769_at | AI703476 | NM_018971 | 2850 | -0.90 | 3.33E-03 |
| C21orf33 | 202217_at | NM_004649 | NM_004649 | 8209 | -0.90 | 2.00E-03 |
| | | | NM_198155 | | | |
| RGS8 | 234297_at | AL359941 | NM_033345 | 85397 | -0.90 | 4.04E-03 |
| AFFX-BioB-M AFFX-BioB-M_a AFFX-BioB-M | | | --- | --- | -0.90 | 9.42E-05 |
| POLR3E | 233458_at | AB040885 | NM_018119 | 55718 | -0.90 | 1.75E-03 |
| DKFZp761B107 | 226139_at | BE670598 | NM_173463 | 91050 | -0.90 | 4.14E-03 |
| AA923289 | 236666_s_at | AA923289 | XM_372416 | 390205 | -0.90 | 1.19E-03 |
| BE247450 | 244443_at | BE247450 | XM_498623 | 440309 | -0.90 | 3.66E-03 |
| SEMA4B | 234725_s_at | AK026133 | NM_020210 | 10509 | -0.90 | 1.81E-03 |
| | | | NM_198925 | | | |
| WDFY3 | 212606_at | AL536319 | NM_014991 | 23001 | -0.90 | 3.35E-04 |
| | | | NM_178583 | | | |
| | | | NM_178585 | | | |
| NM_018336 | 205955_at | NM_018336 | --- | --- | -0.90 | 5.74E-04 |
| SELS | 223209_s_at | AF328864 | NM_018445 | 55829 | -0.90 | 2.33E-03 |
| | | | NM_203472 | | | |
| CDH20 | 243657_at | AW593060 | NM_031891 | 28316 | -0.90 | 7.78E-03 |
| KIAA1223 | 225735_at | AL110131 | XM_048747 | 57182 | -0.90 | 7.05E-04 |
| BE502826 | 231550_at | BE502826 | --- | --- | -0.90 | 5.25E-03 |
| DDX26 | 218819_at | NM_012141 | NM_012141 | 26512 | -0.90 | 1.74E-03 |
| FLJ32731 | 218017_s_at | NM_025070 | XM_372038 | 138050 | -0.90 | 3.84E-07 |
| FLJ32499 | 1552711_a_at | NM_144607 | NM_144607 | 124637 | -0.90 | 6.17E-05 |
| AFFX-BioB-5 | AFFX-BioB-5_a | AFFX-BioB-5 | --- | --- | -0.89 | 2.50E-05 |
| TMEM17 | 1557137_at | AA084725 | NM_198276 | 200728 | -0.89 | 9.22E-04 |
| R26456 | 243135_x_at | R26456 | --- | --- | -0.89 | 2.43E-03 |
| FFX-r2-Ec-bioC=X-r2-Ec-bioC-3AFFX-r2-Ec-bioC-3 | | | --- | --- | -0.89 | 1.35E-04 |
| RAB5A | 215956_at | AK022065 | NM_ 004162 | 5868 | -0.89 | 3.52E-03 |
| FFX-r2-Ec-bioB·=X-r2-Ec-bioB-NAFFX-r2-Ec-bioB-N | | | --- | --- | -0.89 | 3.74E-05 |
| NXPH4 | 221967_at | AI933199 | NM_007224 | 11247 | -0.89 | 1.39E-05 |
| PERLD1 | 221811_at | BF033007 | NM_033419 | 93210 | -0.89 | 3.44E-04 |
| WDR21 | 214758_at | AL080157 | NM_015604 | 26094 | -0.89 | 2.29E-04 |
| | | | NM_181340 | | | |
| | | | NM_181341 | | | |
| MDS009 | 236649_at | AA907927 | NM_020234 | 56986 | -0.89 | 1.30E-03 |
| DISP1 | 228184_at | AK023679 | NM_032890 | 84976 | -0.89 | 6.47E-05 |
| HIST1H2BH | 208546_x_at | NM_003524 | NM_003524 | 8345 | -0.89 | 5.00E-03 |
| MLR1 | 240592_at | AI939336 | NM_153686 | 254251 | -0.89 | 2.34E-03 |
| AF088033 | 1556228_a_at | AF088033 | --- | --- | -0.89 | 7.95E-05 |
| FLJ10815 | 228951_at | AI631572 | NM_018231 | 55238 | -0.89 | 1.85E-03 |
| UTP14A | 221514_at | BC001149 | NM_006649 | 10813 | -0.89 | 9.63E-03 |
| BBP | 213882_at | AA012917 | NM_032027 | 83941 | -0.89 | 3.54E-05 |
| NCSTN | 208759_at | AF240468 | NM_015331 | 23385 | -0.89 | 6.91E-06 |
| LOC283922 | 228688_at | AA843726 | XM_208908 | 283922 | -0.88 | 3.60E-03 |
| Cep164 | 204250_s_at | AI655714 | NM_014956 | 22897 | -0.88 | 2.74E-03 |
| ZFYVE26 | 213073_at | AB002319 | NM_015346 | 23503 | -0.88 | 1.02E-03 |
| AI525856 | 239016_at | AI525856 | --- | --- | -0.88 | 4.67E-05 |
| IREB2 | 1555476_at | BC017880 | NM_004136 | 3658 | -0.88 | 1.40E-03 |
| W80462 | 242194_at | W80462 | --- | --- | -0.88 | 2.61E-03 |
| C13orf21 | 228044_at | AI420319 | NM_001010897 | 387923 | -0.88 | 1.82E-03 |
| WTAP | 1560274_at | BF679507 | NM_004906 | 9589 | -0.88 | 2.07E-04 |
| | | | NM_152857 | | | |
| | | | NM_152858 | | | |
| MGC4278 | 220352_x_at | NM_024305 | --- | 79151 | -0.88 | 6.74E-03 |
| PHF17 | 225816_at | AW138134 | NM_024900 | 79960 | -0.88 | 3.82E-03 |
| | | | NM_199320 | | | |
| LOC401551 | 243900_at | AI190548 | XM_376909 | 401551 | -0.88 | 6.97E-03 |
| FLJ35630 | 229603_at | AA971753 | NM_152618 | 166379 | -0.88 | 2.21E-03 |
| KIAA1008 | 218362_s_at | NM_014953 | NM_014953 | 22894 | -0.88 | 7.57E-04 |
| SON | 201085_s_at | AA664291 | NM_003103 | 6651 | -0.88 | 5.49E-03 |
| | | | NM_032195 | | | |
| | | | NM_058183 | | | |
| | | | NM_138925 | | | |
| | | | NM_138926 | | | |
| | | | NM_138927 | | | |
| AK021474 | 215892_at | AK021474 | --- | --- | -0.88 | 5.75E-03 |
| DCTD | 201572_x_at | NM_001921 | NM_001921 | 1635 | -0.88 | 1.87E-04 |
| H73101 | 1556984_at | H73101 | --- | --- | -0.87 | 3.73E-03 |
| USP34 | 230148_at | AI831431 | NM_014709 | 9736 | -0.87 | 8.65E-03 |
| Ufc1 | 217797_at | NM_016406 | NM_016406 | 51506 | -0.87 | 7.99E-05 |
| BE044193 | 229384_at | BE044193 | --- | --- | -0.87 | 8.85E-03 |
| VMP1 | 224917_at | BF674052 | NM_030938 | 81671 | -0.87 | 8.73E-03 |
| KIAA0711 | 204301_at | NM_014867 | NM_014867 | 9920 | -0.87 | 4.26E-05 |
| C21orf66 | 218515_at | NM_016631 | NM_013329 | 94104 | -0.87 | 2.26E-04 |
| | | | NM_016631 | | | |
| | | | NM_058191 | | | |
| | | | NM_145328 | | | |
| D4S234E | 209569_x_at | NM_014392 | NM_014392 | 27065 | -0.87 | 6.40E-03 |
| TJP2 | 202085_at | NM_004817 | NM_004817 | 9414 | -0.87 | 5.83E-04 |
| | | | NM_201629 | | | |
| MAN1A2 | 217282_at | AK001970 | NM_006699 | 10905 | -0.87 | 5.09E-03 |
| C15orf17 | 224798_s_at | AI079857 | NM_020447 | 57184 | -0.87 | 5.80E-03 |
| SLC20A2 | 231818_x_at | AK023965 | NM_006749 | 6575 | -0.87 | 8.22E-03 |
| C14orf103 | 222840_at | H96715 | NM_018036 | 55102 | -0.87 | 2.43E-03 |
| AFFX-BioC-3 | AFFX-BioC-3_a | AFFX-BioC-3 | --- | --- | -0.87 | 3.17E-05 |
| MGLL | 211026_s_at | BC006230 | NM_00100379 4 | 11343 | -0.87 | 3.12E-03 |
| | | | NM_007283 | | | |
| FZD6 | 203987_at | NM_003506 | NM_003506 | 8323 | -0.87 | 9.59E-03 |
| AFFX-BioC-5 | AFFX-BioC-5_a | AFFX-BioC-5 | --- | --- | -0.87 | 1.11E-04 |
| ENPP2 | 210839_s_at | D45421 | NM_006209 | 5168 | -0.87 | 1.49E-04 |
| DKFZP564D166 | 231799_at | AK021886 | XM_371074 | 26115 | -0.87 | 1.88E-04 |
| ANKRD20A | 1569608_x_at | BC016022 | NM_032250 | 440841 | -0.87 | 2.10E-03 |
| LOC440841 | | | XM_496541 | 84210 | | |
| AW341304 | 237332_at | AW341304 | --- | --- | -0.87 | 1.73E-03 |
| USP36 | 224979_s_at | AU154368 | NM_025090 | 57602 | -0.87 | 8.89E-04 |
| FLJ10980 | 225327_at | AB037791 | NM_019600 | 56204 | -0.87 | 3.59E-05 |
| AI768374 | 228661_s_at | AI768374 | --- | --- | -0.87 | 3.95E-04 |
| CANX | 200068_s_at | M94859 | NM_001746 | 821 | -0.87 | 7.71E-03 |
| MED6 | 207078_at | NM_005466 | NM_005466 | 10001 | -0.87 | 9.36E-03 |
| C3orf6 | 226713_at | AI247881 | NM_174908 | 152137 | -0.87 | 4.87E-03 |
| | | | NM_178335 | | | |
| DJ328E19.C1. 1 | 201104_x_at | NM_015383 | NM_015383 | 200030 | -0.87 | 9.45E-04 |
| | | | NM_173638 | 25832 | | |
| LOC200030 | | | NM_183372 | 284565 | | |
| MGC8902 | | | XM_371384 | 440673 | | |
| AG1 | | | XM_496394 | | | |
| CARD14 | 240392_at | BF752351 | NM_024110 | 79092 | -0.87 | 6.29E-03 |
| | | | NM_052819 | | | |
| FKBP2 | 203391_at | NM_004470 | NM_004470 | 2286 | -0.86 | 6.91E-03 |
| | | | NM_057092 | | | |
| CBS | 212816_s_at | BE613178 | NM_000071 | 875 | -0.86 | 6.44E-03 |
| AI695743 | 244534_at | AI695743 | --- | --- | -0.86 | 1.23E-04 |
| NGLY1 | 220742_s_at | NM_018297 | NM_018297 | 55768 | -0.86 | 4.72E-04 |
| H19 | 224997_x_at | AL575306 | --- | 283120 | -0.86 | 2.98E-03 |
| DYRK1B | 204954_s_at | NM_004714 | NM_004714 | 9149 | -0.86 | 6.27E-03 |
| | | | NM_006483 | | | |
| | | | NM_006484 | | | |
| IDS | 202439_s_at | NM_000202 | NM_000202 | 3423 | -0.86 | 1.14E-03 |
| | | | NM_006123 | | | |
| LOC284267 | 228453_at | AW004076 | --- | 284267 | -0.86 | 6.01E-03 |
| JUN | 201466_s_at | NM_002228 | NM_002228 | 3725 | -0.86 | 6.35E-03 |
| TRAP1 | 228929_at | AI341246 | NM_016292 | 10131 | -0.86 | 7.28E-03 |
| TSC1 | 209390_at | AF013168 | NM_000368 | 7248 | -0.86 | 1.82E-03 |
| | | | NM_00100856 7 | | | |
| MLR1 | 235970_at | AI807408 | NM_153686 | 254251 | -0.86 | 7.89E-04 |
| AI086530 | 217604_at | AI086530 | --- | --- | -0.86 | 1.82E-03 |
| DVL3 | 201908_at | NM_004423 | NM_004423 | 1857 | -0.86 | 2.67E-04 |
| HIST1H3D | 214522_x_at | NM_021065 | NM_003530 | 8351 | -0.86 | 4.36E-05 |
| -HUMRGE/M10IUMRGE/M100K-HUMRGE/M100 | | | --- | --- | -0.86 | 2.30E-03 |
| TPP1 | 200742_s_at | BG231932 | NM_000391 | 1200 | -0.86 | 7.91E-03 |
| TTC3 | 208664_s_at | AU131711 | NM_00100189 4 | 7267 | -0.86 | 7.07E-03 |
| | | | NM_003316 | | | |
| RFPL2 | 207227_x_at | NM_006605 | NM_006605 | 10739 | -0.86 | 3.03E-03 |
| D4S234E | 209570_s_at | BC001745 | NM_014392 | 27065 | -0.86 | 1.12E-04 |
| LOC146174 | 230721_at | BF436957 | NM_173501 | 146174 | -0.86 | 5.19E-04 |
| C9orf68 | 1554708_s_at | BC034293 | NM_017985 | 55064 | -0.86 | 1.35E-03 |
| R3HDM | 240503_at | AW274946 | NM_015361 | 23518 | -0.86 | 6.04E-03 |
| SNCA | 204467_s_at | NM_000345 | NM_000345 | 6622 | -0.85 | 9.17E-05 |
| | | | NM_007308 | | | |
| AK094546 | 1556205_at | AK094546 | --- | --- | -0.85 | 6.76E-04 |
| KIAA1450 | 225924_at | AI478634 | --- | 57600 | -0.85 | 6.97E-03 |
| SKP1A | 200719_at | BE964043 | NM_006930 | 6500 | -0.85 | 3.06E-03 |
| | | | NM_170679 | | | |
| PGK1 | 228483_s_at | BE856250 | NM_000291 | 5230 | -0.85 | 2.17E-03 |
| LOC200030 | 229447_x_at | N32025 | NM_183372 | 200030 | -0.85 | 8.99E-04 |
| PRKCA | 1560074_at | AL119889 | NM_002737 | 5578 | -0.85 | 1.54E-03 |
| AI332346 | 225445_at | AI332346 | --- | --- | -0.85 | 1.78E-03 |
| COG1 | 231813_s_at | AV746580 | NM_018714 | 9382 | -0.85 | 1.02E-03 |
| FLJ20203 | 219846_at | NM_025174 | NM_032292 | 54856 | -0.85 | 1.98E-03 |
| GALNS | 206335_at | NM_000512 | NM_000512 | 2588 | -0.85 | 9.33E-03 |
| CASP9 | 203984_s_at | U60521 | NM_001229 | 842 | -0.85 | 1.05E-03 |
| | | | NM_032996 | | | |
| AU147777 | 232145_at | AU147777 | XM_371506 | 388969 | -0.85 | 7.96E-03 |
| MGC52110 | 226039_at | AW006441 | NM_001008215 | 493753 | -0.85 | 9.51E-06 |
| MACF1 | 208634_s_at | AB029290 | NM_012090 | 23499 | -0.85 | 2.09E-03 |
| | | | NM_033044 | | | |
| FLJ41603 | 227717_at | BF939317 | NM_001001669 | 389337 | -0.84 | 5.48E-03 |
| FLJ12716 | 233558_s_at | AK023390 | NM_021942 | 60684 | -0.84 | 1.27E-03 |
| | | | NM_199053 | | | |
| BE676062 | 231249_at | BE676062 | --- | --- | -0.84 | 2.18E-03 |
| KIAA0415 | 209913_x_at | AB007875 | XM_166527 | 9907 | -0.84 | 3.69E-03 |
| | | | XM_379771 | | | |
| | | | XM_499251 | | | |
| | | | XM_499396 | | | |
| PCAF | 203845_at | AV727449 | NM_003884 | 8850 | -0.84 | 4.85E-03 |
| MONDOA | 211789_s_at | AF312918 | NM_014938 | 22877 | -0.84 | 4.65E-03 |
| C6orf70 | 227656_at | AW968493 | XM_376556 | 55780 | -0.84 | 1.48E-03 |
| PTPN21 | 226380_at | N21442 | NM_007039 | 11099 | -0.84 | 1.06E-03 |
| PNMT | 206793_at | NM_002686 | NM_002686 | 5409 | -0.84 | 2.71E-03 |
| ALG5 | 218203_at | NM_013338 | NM_013338 | 29880 | -0.84 | 8.89E-03 |
| LOC90110 | 226842_at | AV694039 | --- | 90110 | -0.84 | 5.28E-03 |
| CYLD | 39582_at | AL050166 | NM_015247 | 1540 | -0.84 | 4.04E-03 |
| LOC150271 | 228658_at | R54042 | --- | 150271 | -0.84 | 6.95E-03 |
| FER1L4 | 222245_s_at | AF218012 | --- | 80307 | -0.84 | 5.44E-05 |
| MECT1 | 213091_at | AB014516 | NM_015321 | 23373 | -0.84 | 2.37E-04 |
| | | | NM_025021 | | | |
| AL359605 | 227318_at | AL359605 | --- | --- | -0.84 | 4.99E-06 |
| SH3GL2 | 205751_at | NM_003026 | NM_003026 | 6456 | -0.84 | 3.31E-04 |
| MUC1 | 213693_s_at | AI610869 | NM_002456 | 4582 | -0.84 | 6.09E-04 |
| | | | NM_182741 | | | |
| RSHL2 | 223713_at | AF353618 | NM_031924 | 83861 | -0.84 | 3.80E-03 |
| MRPS33 | 218654_s_at | NM_016071 | NM_016071 | 51650 | -0.83 | 2.36E-03 |
| | | | NM_053035 | | | |
| FBLP-1 | 1554795_a_at | BC019895 | NM_017556 | 54751 | -0.83 | 5.75E-03 |
| XPOT | 227117_at | AW242820 | NM_007235 | 11260 | -0.83 | 5.63E-03 |
| ACRV1 | 206776_x_at | NM_001612 | NM_001612 | 56 | -0.83 | 7.51E-03 |
| | | | NM_020069 | | | |
| | | | NM_020107 | | | |
| | | | NM_020108 | | | |
| | | | NM_020109 | | | |
| | | | NM_020110 | | | |
| | | | NM_020111 | | | |
| | | | NM_020113 | | | |
| | | | NM_020114 | | | |
| | | | NM_020115 | | | |
| AI334297 | 225961_at | AI334297 | --- | --- | -0.83 | 5.29E-04 |
| TMEM34 | 219074_at | NM_018241 | NM_018241 | 55751 | -0.83 | 1.81E-03 |
| AI494500 | 241448_at | AI494500 | --- | --- | -0.83 | 9.31E-04 |
| LOC440160 | 239010_at | AI744280 | XM_498571 | 440160 | -0.83 | 6.65E-03 |
| PPID | 204186_s_at | AI014573 | NM_005038 | 5481 | -0.83 | 2.63E-04 |
| PAK3 | 236277_at | H23551 | NM_002578 | 5063 | -0.83 | 1.34E-04 |
| NPM1 | 221923_s_at | AA191576 | NM_002520 | 4869 | -0.83 | 5.74E-03 |
| | | | NM_199185 | | | |
| ProSAPiP1 | 204447_at | NM_014731 | NM_014731 | 9762 | -0.83 | 4.47E-04 |
| NSMAF | 232149_s_at | BF056507 | NM_003580 | 8439 | -0.83 | 6.67E-03 |
| MGC21881 | 1555874_x_at | BC019880 | NM_203448 | 389741 | -0.83 | 1.97E-03 |
| FLJ37953 | 241825_at | AI265967 | NM_152382 | 129450 | -0.83 | 2.38E-03 |
| INPP5A | 203006_at | NM_005539 | NM_005539 | 3632 | -0.83 | 5.00E-04 |
| SLC25A16 | 210686_x_at | BC001407 | NM_152707 | 8034 | -0.83 | 2.87E-03 |
| CBS | 1553972_a_at | BC007257 | NM_000071 | 875 | -0.83 | 5.27E-03 |
| KIAA0804 | 209553_at | BC001001 | NM_00100992 1 | 23355 | -0.83 | 9.90E-04 |
| | | | NM_015303 | | | |
| AW945538 | 239208_s_at | AW945538 | --- | --- | -0.83 | 3.44E-04 |
| AK021583 | 232931_at | AK021583 | --- | --- | -0.82 | 4.13E-03 |
| BDP1 | 224227_s_at | AF298152 | NM_018429 | 55814 | -0.82 | 4.88E-03 |
| SPG7 | 214494_s_at | NM_005200 | NM_003119 | 6687 | -0.82 | 9.51E-03 |
| | | | NM_199367 | | | |
| ZNF599 | 1558390_at | BC033354 | NM_00100724 7 | 148103 | -0.82 | 1.18E-03 |
| | | | NM_00100724 8 | | | |
| FFX-r2-Ec-bioCFX-r2-Ec-bioC-5AFFX-r2-Ec-bioC-5 | | | --- | --- | -0.82 | 2.81E-04 |
| KIAA0690 | 1559127_x_at | BC042908 | NM_015179 | 23223 | -0.82 | 1.09E-04 |
| KIAA1546 | 1569385_s_at | BC019007 | XM_042301 | 57667 | -0.82 | 3.51E-04 |
| SSR4 | 201004_at | NM_006280 | NM_006280 | 6748 | -0.82 | 1.83E-03 |
| TRIT1 | 218617_at | NM_017646 | NM_017646 | 54802 | -0.82 | 5.10E-05 |
| MAPK10 | 204813_at | NM_002753 | NM_002753 | 5602 | -0.82 | 2.98E-03 |
| | | | NM_138980 | | | |
| | | | NM_138981 | | | |
| | | | NM_138982 | | | |
| CGI-119 | 219206_x_at | NM_016056 | NM_016056 | 51643 | -0.82 | 3.02E-03 |
| GPRASP1 | 204793_at | NM_014710 | NM_014710 | 9737 | -0.82 | 2.03E-04 |
| ARHGAP1 | 241419_at | AA709154 | NM_004308 | 392 | -0.82 | 2.43E-03 |
| KIAA0934 | 212504_at | N31807 | NM_014974 | 22982 | -0.82 | 2.71E-04 |
| LOC51619 | 240014_at | AI821720 | NM_015983 | 51619 | -0.82 | 7.64E-03 |
| MEF2A | 212535_at | AA142929 | NM_005587 | 4205 | -0.81 | 8.41E-04 |
| AI859463 | 242111_at | AI859463 | --- | --- | -081 | 3.79E-03 |
| KLF15 | 231015_at | AW014734 | NM_014079 | 28999 | -0.81 | 3.05E-03 |
| MGC8902 | 213612_x_at | AI800419 | NM_173638 | 284565 | -0.81 | 1.93E-03 |
| AG1 | | | XM_371384 | 440673 | | |
| | | | XM_496394 | | | |
| FLJ10038 | 205511_at | NM_017976 | --- | 55056 | -0.81 | 1.24E-03 |
| PCDHA2 | 210572_at | BC003126 | NM_018905 | 56146 | -0.81 | 3.16E-04 |
| | | | NM_031495 | | | |
| | | | NM_031496 | | | |
| EYA1 | 214608_s_at | AJ000098 | NM_000503 | 2138 | -0.81 | 3.74E-03 |
| | | | NM_172058 | | | |
| | | | NM_172059 | | | |
| | | | NM_172060 | | | |
| CENTG1 | 229917_at | BE551002 | NM_014770 | 116986 | -0.81 | 1.70E-03 |
| PLRG1 | 225194_at | NM_002669 | NM_002669 | 5356 | -0.81 | 2.39E-03 |
| DUSP16 | 236511_at | AI798976 | NM_030640 | 80824 | -0.81 | 8.12E-03 |
| LRRFIP1 | 201862_s_at | NM_004735 | NM_004735 | 9208 | -0.81 | 2.88E-04 |
| AI393706 | 238944_at | AI393706 | --- | --- | -0.81 | 3.70E-03 |
| TNIP1 | 207196_s_at | NM_006058 | NM_006058 | 10318 | -0.81 | 3.18E-03 |
| SYNCRIP | 236146_at | BF593158 | NM_006372 | 10492 | -0.81 | 3.42E-03 |
| TAS2R5 | 231757_at | NM_018980 | NM_018980 | 54429 | -0.81 | 1.10E-03 |
| PABPC4 | 201064_s_at | NM_003819 | NM_003819 | 8761 | -0.81 | 1.63E-03 |
| AW367380 | 236408_at | AW367380 | --- | --- | -0.81 | 6.92E-03 |
| C21orf127 | 234676_s_at | AK021678 | NM_013240 | 29104 | -0.81 | 6.83E-03 |
| | | | NM_182749 | | | |
| NM_018678 | 220494_s_at | NM_018678 | --- | --- | -0.81 | 9.98E-03 |
| AF5Q31 | 232864_s_at | N59653 | NM_014423 | 27125 | -0.81 | 5.40E-03 |
| LTB4R | 216388_s_at | U33448 | NM_181657 | 1241 | -0.80 | 3.43E-03 |
| ENPP2 | 209392_at | L35594 | NM_006209 | 5168 | -0.80 | 1.95E-05 |
| SMURF2 | 205596_s_at | AY014180 | NM_022739 | 64750 | -0.80 | 2.21E-04 |
| STS | 243858_at | AA699970 | NM_000351 | 412 | -0.80 | 1.20E-03 |
| KLHDC1 | 1552733_at | NM_172193 | NM_172193 | 122773 | -0.80 | 1.05E-03 |
| ASTN2 | 215407_s_at | AK024064 | NM_014010 | 23245 | -0.80 | 2.08E-03 |
| | | | NM_198186 | | | |
| | | | NM_198187 | | | |
| | | | NM_198188 | | | |
| BQ008117 | 1556659_at | BQ008117 | --- | --- | -0.80 | 5.03E-03 |
| FLJ10534 | 239042_at | AI282511 | NM_018128 | 55720 | -0.80 | 2.54E-03 |
| RABL5 | 218785_s_at | NM_022777 | NM_022777 | 64792 | -0.80 | 2.32E-03 |
| RNASET2 | 217983_s_at | NM_003730 | NM_003730 | 8635 | -0.80 | 7.39E-04 |
| BTBD14B | 215587_x_at | AK023891 | NM_052876 | 112939 | -0.80 | 3.30E-03 |
| FALZ | 232909_s_at | AU146870 | NM_004459 | 2186 | -0.80 | 7.77E-03 |
| | | | NM_182641 | | | |
| NEUGRIN | 228541_x_at | AI049608 | NM_016645 | 51335 | -0.80 | 2.24E-03 |
| UNC5B | 213100_at | AA127885 | NM_170744 | 219699 | -0.80 | 6.29E-03 |
| LOC439987 | 229609_at | BF224281 | XM_495841 | 439987 | -0.80 | 7.79E-04 |
| LOC439993 | | | XM_498488 | 439993 | | |
| RPIP8 | 213439_x_at | AI197870 | NM_006695 | 10900 | -0.80 | 3.47E-05 |
| LOC285949 | 1563781_at | AK096687 | --- | 285949 | -0.80 | 3.61E-03 |
| FLJ22313 | 236170_x_at | AI377423 | NM_022373 | 64224 | -0.80 | 5.62E-04 |
| AQP3 | 39248_at | N74607 | NM_004925 | 360 | -0.80 | 4.75E-03 |
| FALZ | 209271_at | AB032251 | NM_004459 | 2186 | -0.80 | 9.58E-03 |
| | | | NM_182641 | | | |
| LOC440118 FX-M27830_M_AFFX-M27830_M | | | XM_498554 | 440118 | -0.80 | 9.04E-04 |
| ARF1 | 232175_at | AI972094 | NM_001658 | 375 | -0.80 | 1.30E-04 |
| ERBB2 | 216836_s_at | X03363 | NM_00100586 2 | 2064 | -0.80 | 1.70E-04 |
| | | | NM_004448 | | | |
| LOC440823 | 227168_at | BF475488 | XM_498878 | 440823 | -0.80 | 8.41E-03 |
| DUSP8 | 206374_at | NM_004420 | NM_004420 | 1850 | -0.80 | 9.45E-04 |
| ARHGAP1 | 202117_at | BG468434 | NM_004308 | 392 | -0.79 | 5.77E-04 |
| CYLD | 221903_s_at | BE046443 | NM_015247 | 1540 | -0.79 | 4.50E-05 |
| AFFX-BioDn-5 | AFFX-BioDn-5_a | AFFX-BioDn-5 | --- | --- | -0.79 | 2.95E-05 |
| CARS | 202402_s_at | NM_001751 | NM_001751 | 833 | -0.79 | 7.28E-03 |
| | | | NM_139273 | | | |
| ASTN2 | 209693_at | AF116574 | NM_014010 | 23245 | -0.79 | 9.79E-04 |
| | | | NM_198186 | | | |
| | | | NM_198187 | | | |
| | | | NM_198188 | | | |
| AK025546 | 232161_x_at | AK025546 | --- | --- | -0.79 | 4.20E-03 |
| PTPRD | 214043_at | BF062299 | NM_002839 | 5789 | -0.79 | 8.45E-03 |
| | | | NM_130391 | | | |
| | | | NM_130392 | | | |
| | | | NM_130393 | | | |
| ATP6V0A1 | 212383_at | AL096733 | NM_005177 | 535 | -0.79 | 9.12E-04 |
| AIG1 | 223136_at | AF151861 | NM_016108 | 51390 | -0.79 | 8.00E-03 |
| ANXA7 | 242027_at | AI732079 | NM_001156 | 310 | -0.79 | 8.52E-04 |
| | | | NM_004034 | | | |
| HDLBP | 225012_at | BE378479 | NM_005336 | 3069 | -0.79 | 4.04E-04 |
| | | | NM_203346 | | | |
| C10orf51 | 231196_x_at | AI333226 | --- | 387644 | -0.79 | 1.33E-05 |
| WASL | 227010_at | BF438330 | NM_003941 | 8976 | -0.79 | 1.01E-03 |
| RSNL2 | 226425_at | AU144247 | NM_024692 | 79745 | -0.79 | 4.96E-05 |
| ATP9A | 216129_at | AL117659 | XM_030577 | 10079 | -0.79 | 2.42E-04 |
| AA888098 | 244426_at | AA888098 | --- | --- | -0.79 | 7.13E-04 |
| DYM | 220774_at | NM_017653 | NM_017653 | 54808 | -0.79 | 1.83E-03 |
| C2orf17 | 221983_at | AL040896 | NM_024293 | 79137 | -0.79 | 6.51E-03 |
| MGC4618 | 223462_at | BC005158 | NM_032326 | 84286 | -0.78 | 2.33E-03 |
| RSL1D1 | 212019_at | AK025446 | NM_015659 | 26156 | -0.78 | 3.44E-03 |
| LOC200030 | 201103_x_at | BE299495 | NM_173638 | 200030 | -0.78 | 1.09E-03 |
| MGC8902 | | | NM_183372 | 284565 | | |
| AG1 | | | XM_371384 | 440673 | | |
| | | | XM_496394 | | | |
| WDR13 | 222138_s_at | AF158978 | NM_017883 | 64743 | -0.78 | 1.22E-03 |
| SERP1 | 200971_s_at | NM_014445 | NM_014445 | 27230 | -0.78 | 1.90E-03 |
| KIAA1049 | 213311_s_at | BF000251 | NM_014972 | 22980 | -0.78 | 2.49E-03 |
| BG502771 | 226397_s_at | BG502771 | --- | --- | -0.78 | 2.83E-03 |
| AI652868 | 225811_at | AI652868 | --- | --- | -0.78 | 5.96E-03 |
| AL559202 | 226458_at | AL559202 | --- | --- | -0.78 | 8.12E-03 |
| R15431 | 236440_at | R15431 | --- | --- | -0.78 | 1.66E-05 |
| SLC43A1 | 204394_at | NM_003627 | NM_003627 | 8501 | -0.78 | 3.44E-03 |
| C18orf17 | 238480_at | AI871745 | NM_153211 | 125488 | -0.78 | 7.85E-04 |
| C11orf31 | 228331_at | AA526939 | NM_170746 | 280636 | -0.78 | 1.80E-03 |
| SLC9A3 | 226125_at | BF346665 | NM_004174 | 6550 | -0.78 | 1.02E-03 |
| EPC2 | 225838_at | AK002010 | NM_015630 | 26122 | -0.78 | 1.65E-04 |
| KIAA0934 | 212503_s_at | N22859 | NM_014974 | 22982 | -0.78 | 2.51E-04 |
| EFHC1 | 231026_at | AW451135 | NM_018100 | 114327 | -0.78 | 9.28E-03 |
| AA883831 | 244042_x_at | AA883831 | --- | --- | -0.78 | 9.44E-03 |
| NIN | 225921_at | AL359571 | NM_016350 | 51199 | -0.78 | 1.33E-04 |
| | | | NM_020921 | | | |
| | | | NM_182944 | | | |
| | | | NM_182945 | | | |
| | | | NM_182946 | | | |
| AW502656 | 240800_x_at | AW502656 | --- | --- | -0.77 | 2.82E-05 |
| SMYD2 | 212922_s_at | AI809870 | NM_020197 | 56950 | -0.77 | 4.17E-03 |
| NDUFV3 | 226616_s_at | NM_021075 | NM_00100150 3 | 4731 | -0.77 | 3.48E-03 |
| | | | NM_021075 | | | |
| LSS | 202245_at | AW084510 | NM_00100143 8 | 4047 | -0.77 | 1.95E-04 |
| | | | NM_002340 | | | |
| TTC3 | 208662_s_at | AI885338 | NM_00100189 4 | 7267 | -0.77 | 1.75E-04 |
| | | | NM_003316 | | | |
| IL10RB | 209575_at | BC001903 | NM_000628 | 3588 | -0.77 | 1.78E-05 |
| PLTP | 202075_s_at | NM_006227 | NM_006227 | 5360 | -0.77 | 6.25E-03 |
| | | | NM_182676 | | | |
| ANKRD17 | 225852_at | BE463523 | NM_032217 | 26057 | -0.77 | 1.79E-05 |
| | | | NM_198889 | | | |
| LOC283981 | 232463_at | T77995 | --- | 283981 | -0.77 | 9.63E-03 |
| AI146751 | 242565_x_at | AI146751 | --- | --- | -0.77 | 1.83E-03 |
| GDF1 | 206397_x_at | NM_001492 | NM_001492 | 10715 | -0.77 | 1.04E-04 |
| LASS1 | | | NM_021267 | 2657 | | |
| | | | NM_198207 | | | |
| FLJ10276 | 218004_at | NM_018045 | NM_018045 | 55108 | -0.77 | 1.60E-03 |
| BF223556 | 229376_at | BF223556 | --- | --- | -0.77 | 3.00E-03 |
| KIAA1109 | 212779_at | AB029032 | XM_371706 | 84162 | -0.77 | 8.29E-04 |
| AI983303 | 237877_at | AI983303 | --- | --- | -0.77 | 2.54E-05 |
| JMJD2A | 203204_s_at | BC002558 | NM_014663 | 9682 | -0.77 | 5.29E-03 |
| RBM8A | 217856_at | AF182415 | NM_005105 | 9939 | -0.77 | 1.77E-03 |
| FLJ12716 | 218179_s_at | NM_021942 | NM_021942 | 60684 | -0.77 | 4.05E-04 |
| | | | NM_199053 | | | |
| CGI-119 | 222845_x_at | AF161526 | NM_016056 | 51643 | -0.77 | 4.64E-05 |
| BI520422 | 1559467_at | BI520422 | --- | --- | -0.77 | 6.79E-03 |
| AA134418 | 232150_at | AA134418 | --- | --- | -0.77 | 3.88E-03 |
| ZNF323 | 222016_s_at | AW086021 | NM_030899 | 64288 | -0.77 | 3.58E-03 |
| | | | NM_145909 | | | |
| GLG1 | 214730_s_at | AK025457 | NM_012201 | 2734 | -0.77 | 8.94E-05 |
| AK024602 | 222207_x_at | AK024602 | --- | --- | -0.76 | 4.03E-03 |
| BE964222 | 226589_at | BE964222 | --- | --- | -0.76 | 2.53E-04 |
| GNAS | 228173_at | AA810695 | NM_000516 | 2778 | -0.76 | 5.49E-03 |
| | | | NM_016592 | | | |
| | | | NM_080425 | | | |
| | | | NM_080426 | | | |
| MONDOA | 225157_at | AW245631 | NM_014938 | 22877 | -0.76 | 3.06E-04 |
| KIAA2010 | 215607_x_at | AU144530 | NM_017936 | 55671 | -0.76 | 3.17E-03 |
| | | | NM_032560 | | | |
| DJ328E19.C1. | 226740_x_at | BF740216 | NM_015383 | 200030 | -0.76 | 1.09E-03 |
| 1 | | | NM_173638 | 25832 | | |
| LOC200030 | | | NM_183372 | 284565 | | |
| MGC8902 | | | XM_371384 | 439928 | | |
| LOC439928 | | | XM_496394 | 440673 | | |
| AG1 | | | XM_498427 | | | |
| LEAP-2 | 1552362_a_at | NM_052971 | NM_052971 | 116842 | -0.76 | 5.61E-03 |
| BRUNOL4 | 238966_at | AA678985 | NM_020180 | 56853 | -0.76 | 8.60E-03 |
| AA827878 | 223577_x_at | AA827878 | --- | --- | -0.76 | 5.84E-03 |
| CDKL1 | 244670_at | AI767697 | NM_004196 | 8814 | -0.76 | 4.97E-03 |
| NALP1 | 218380_at | NM_021730 | NM_014922 | 22861 | -0.76 | 2.51E-03 |
| | | | NM_033004 | | | |
| | | | NM_033006 | | | |
| | | | NM_033007 | | | |
| MAN1B1 | 65884_at | AA631254 | NM_016219 | 11253 | -0.76 | 3.92E-04 |
| GOSR2 | 235041_at | AW005457 | NM_004287 | 9570 | -0.76 | 9.63E-04 |
| | | | NM_054022 | | | |
| BI496583 | 1557675_at | BI496583 | --- | --- | -0.76 | 7.35E-03 |
| AA194266 | 238728_at | AA194266 | --- | --- | -0.76 | 2.48E-03 |
| EFEMP2 | 209356_x_at | AB030655 | NM_016938 | 30008 | -0.76 | 5.52E-03 |
| NM_018705 | 220572_at | NM_018705 | --- | --- | -0.76 | 3.25E-03 |
| MGC2747 | 224712_x_at | AI656658 | NM_024104 | 79086 | -0.76 | 2.38E-04 |
| PIK3R1 | 212239_at | AI680192 | NM_181504 | 5295 | -0.76 | 7.57E-03 |
| | | | NM_181523 | | | |
| | | | NM_181524 | | | |
| RAB11B | 34478_at | X79780 | NM_004218 | 9230 | -0.76 | 5.20E-04 |
| FLJ23867 | 226706_at | BE301252 | --- | 200058 | -0.76 | 2.32E-03 |
| NELF | 221214_s_at | NM_015537 | NM_015537 | 26012 | -0.76 | 9.78E-05 |
| MAP2K7 | 216206_x_at | BC005365 | NM_145185 | 5609 | -0.75 | 1.08E-03 |
| FLJ43806 | 213752_at | BF111846 | NM_201628 | 399563 | -0.75 | 3.92E-03 |
| CHMP1.5 | 218177_at | AA293502 | NM_020412 | 57132 | -0.75 | 3.04E-04 |
| KNDC1 | 230359_at | N45097 | NM_033404 | 85442 | -0.75 | 6.69E-04 |
| | | | NM_152643 | | | |
| HNRPU | 236244_at | AI458297 | NM_004501 | 3192 | -0.75 | 7.10E-03 |
| | | | NM_031844 | | | |
| C1orf50 | 62212_at | W37846 | NM_024097 | 79078 | -0.75 | 7.56E-04 |
| KIAA0913 | 212359_s_at | W89120 | NM_015037 | 23053 | -0.75 | 2.87E-05 |
| PPP2CA | 215628_x_at | AL049285 | NM_002715 | 5515 | -0.75 | 2.81E-04 |
| C6orf160 | 225155_at | BG339050 | XM_499041 | 441164 | -0.75 | 3.83E-03 |
| CD151 | 204306_s_at | NM_004357 | NM_004357 | 977 | -0.75 | 1.69E-06 |
| | | | NM_139030 | | | |
| HLCS | 209399_at | D87328 | NM_000411 | 3141 | -0.75 | 2.42E-04 |
| AA017536 | 242270_at | AA017536 | --- | --- | -0.75 | 2.11E-05 |
| FLJ43663 | 215330_at | AL049991 | XM_379520 | 378805 | -0.75 | 1.78E-05 |
| | | | XM_380146 | | | |
| MRRF | 225126_at | BF438352 | NM_138777 | 92399 | -0.75 | 5.23E-03 |
| | | | NM_199176 | | | |
| | | | NM_199177 | | | |
| WNK1 | 211992_at | AI445745 | NM_018979 | 65125 | -0.75 | 3.85E-03 |
| AI870959 | 244877_at | AI870959 | --- | --- | -0.75 | 1.78E-03 |
| LOC94431 | 216908_x_at | AF001549 | NM_145237 | 94431 | -0.75 | 2.16E-03 |
| BG527339 | 226457_at | BG527339 | --- | --- | -0.75 | 3.79E-03 |
| AA928506 | 213750_at | AA928506 | --- | --- | -0.74 | 4.38E-04 |
| GP1BB | 206655_s_at | NM_000407 | NM_000407 | 2812 | -0.74 | 4.60E-04 |
| AA629075 | 236653_at | AA629075 | --- | --- | -0.74 | 1.35E-04 |
| TTC3 | 208661_s_at | AW510696 | NM_00100189 4 | 7267 | -0.74 | 1.79E-05 |
| | | | NM_003316 | | | |
| TNRC6C | 227149_at | AB046802 | NM_018996 | 57690 | -0.74 | 5.66E-03 |
| POFUT2 | 209578_s_at | BC000626 | NM_015227 | 23275 | -0.74 | 1.92E-03 |
| | | | NM_133634 | | | |
| | | | NM_133635 | | | |
| CLDN12 | 223249_at | AL136770 | NM_012129 | 9069 | -0.74 | 2.31E-03 |
| PICALM | 239102_s_at | AW293296 | NM_00100866 0 | 8301 | -0.74 | 3.04E-03 |
| | | | NM_007166 | | | |
| SKIV2L | 203727_at | NM_006929 | NM_006929 | 6499 | -0.74 | 4.08E-04 |
| LOC125150 | 228719_at | BE645222 | --- | 125150 | -0.74 | 7.64E-03 |
| AA026666 | 235611_at | AA026666 | --- | --- | -0.74 | 3.78E-03 |
| KIAA0323 | 212356_at | AB002321 | NM_015299 | 23351 | -0.74 | 1.24E-03 |
| BC031035 | 1569503_at | BC031035 | --- | --- | -0.74 | 1.39E-05 |
| UNC5B | 226899_at | AK022859 | NM_170744 | 219699 | -0.74 | 6.84E-03 |
| KCNRG | 239098_at | W68737 | NM_173605 | 283518 | -0.74 | 5.28E-03 |
| | | | NM_199464 | | | |
| DPCD | 226009_at | AI129328 | NM_015448 | 25911 | -0.74 | 8.18E-03 |
| LEPREL2 | 204854_at | NM_014262 | NM_014262 | 10536 | -0.74 | 3.69E-03 |
| AW341225 | 1556690_s_at | AW341225 | --- | --- | -0.74 | 1.25E-06 |
| DPP7 | 224814_at | NM_013379 | NM_013379 | 29952 | -0.74 | 2.41E-04 |
| GAS5 | 224841_x_at | BF316352 | --- | 60674 | -0.74 | 1.39E-03 |
| TRIM8 | 223132_s_at | AF220034 | NM_030912 | 81603 | -0.73 | 1.24E-05 |
| BF056548 | 232140_at | BF056548 | XM_499148 | 441443 | -0.73 | 8.09E-03 |
| PPT2 | 208469_s_at | NM_030652 | NM_005155 | 80864 | -0.73 | 3.43E-03 |
| EGFL8 | | | NM_030652 | 9374 | | |
| | | | NM_138717 | | | |
| | | | NM_138934 | | | |
| SH3YL1 | 204019_s_at | NM_015677 | NM_015677 | 26751 | -0.73 | 4.63E-04 |
| EXOSC6 | 227696_at | AI701408 | NM_058219 | 118460 | -0.73 | 1.43E-03 |
| SNIP | 232547_at | BF062187 | NM_025248 | 80725 | -0.73 | 8.20E-06 |
| AL833564 | 1563638_at | AL833564 | --- | --- | -0.73 | 4.87E-05 |
| MACF1 | 208633_s_at | W61052 | NM_012090 | 23499 | -0.73 | 9.23E-04 |
| | | | NM_033044 | | | |
| CIB1 | 201953_at | NM_006384 | NM_006384 | 10519 | -0.73 | 5.74E-04 |
| RAMP1 | 204916_at | NM_005855 | NM_005855 | 10267 | -0.73 | 1.63E-03 |
| AA694209 | 229066_at | AA694209 | --- | --- | -0.73 | 9.44E-03 |
| NOTCH4 | 205247_at | AI743713 | NM_004557 | 4855 | -0.73 | 5.43E-03 |
| BF000009 | 243952_at | BF000009 | --- | --- | -0.73 | 9.42E-03 |
| NR2F1 | 209506_s_at | BC004154 | NM_005654 | 7025 | -0.73 | 4.54E-03 |
| LOC92154 | 1555894_s_at | AA829283 | NM_138383 | 92154 | -0.73 | 9.70E-03 |
| C9orf94 | 229585_at | AI803088 | NM_152702 | 206938 | -0.73 | 2.44E-03 |
| MAN1A2 | 217922_at | AL157902 | NM_006699 | 10905 | -0.73 | 1.21E-03 |
| CGI-119 | 223892_s_at | AF182414 | NM_016056 | 51643 | -0.73 | 1.67E-03 |
| STAF65(gamma | 201836_s_at | AU154740 | NM_014860 | 9913 | -0.73 | 6.03E-03 |
| PLXNA3 | 203623_at | AI675453 | NM_017514 | 55558 | -0.73 | 4.76E-04 |
| SPATA11 | 223318_s_at | BC004393 | NM_032306 | 84266 | -0.73 | 9.26E-03 |
| BE674055 | 236109_at | BE674055 | --- | --- | -0.73 | 8.42E-03 |
| KIAA2026 | 228446_at | BF062203 | XM_088551 | 158358 | -0.73 | 2.23E-03 |
| CLN3 | 210859_x_at | AF077973 | NM_000086 | 1201 | -0.72 | 4.13E-03 |
| AW129145 | 238992_at | AW129145 | --- | --- | -0.72 | 3.88E-03 |
| LOC201158 | 1552377_s_at | NM_145301 | NM_145301 | 201158 | -0.72 | 2.76E-03 |
| FLJ31265 | 228341_at | AI809108 | NM_152395 | 131870 | -0.72 | 1.87E-03 |
| BF111169 | 230820_at | BF111169 | --- | --- | -0.72 | 1.14E-04 |
| D8S2298E | 215983_s_at | D83768 | NM_005671 | 7993 | -0.72 | 6.50E-03 |
| AI424847 | 243218_at | AI424847 | --- | --- | -0.72 | 4.14E-03 |
| PCDHA9 | 224212_s_at | AF169689 | NM_014005 | 56134 | -0.72 | 5.55E-04 |
| PCDHAC2 | | | NM_018898 | 56135 | | |
| PCDHAC1 | | | NM_018899 | 56136 | | |
| PCDHA13 | | | NM_018900 | 56137 | | |
| PCDHA12 | | | NM_018901 | 56138 | | |
| PCDHA11 | | | NM_018902 | 56139 | | |
| PCDHA10 | | | NM_018903 | 56140 | | |
| PCDHA8 | | | NM_018904 | 56141 | | |
| PCDHA7 | | | NM_018905 | 56142 | | |
| PCDHA6 | | | NM_018906 | 56143 | | |
| PCDHA5 | | | NM_018907 | 56144 | | |
| PCDHA4 | | | NM_018908 | 56145 | | |
| PCDHA3 | | | NM_018909 | 56146 | | |
| PCDHA2 | | | NM_018910 | 56147 | | |
| PCDHA1 | | | NM_018911 | 9752 | | |
| | | | NM_031410 | | | |
| | | | NM_031411 | | | |
| | | | NM_031495 | | | |
| RBM5 | 201394_s_at | U23946 | NM_005778 | 10181 | -0.72 | 2.11E-03 |
| 4FFX-BioDn-3 | AFFX-BioDn-3_a | AFFX-BioDn-3 | --- | --- | -0.72 | 1.31E-04 |
| WBSCR16 | 1554409_at | BC040695 | NM_030798 | 81554 | -0.72 | 5.09E-04 |
| | | | NM_148842 | | | |
| SYNJ1 | 212990_at | AB020717 | NM_003895 | 8867 | -0.72 | 6.07E-05 |
| | | | NM_203446 | | | |
| C19orf2 | 222266_at | BF796940 | NM_003796 | 8725 | -0.72 | 1.36E-03 |
| | | | NM_134447 | | | |
| RAPGEF2 | 203097_s_at | NM_014247 | XM_376350 | 9693 | -0.72 | 7.01E-03 |
| PQBP1 | 207769_s_at | NM_005710 | NM_005710 | 10084 | -0.72 | 2.58E-03 |
| | | | NM_144494 | | | |
| | | | NM_144495 | | | |
| LOC162967 | 1555790_a_at | BC036301 | NM_152681 | 162967 | -0.72 | 3.31E-03 |
| FLJ38482 | | | | 201931 | | |
| EGFL4 | 214778_at | AB011541 | NM_001410 | 1954 | -0.72 | 3.38E-05 |
| FLJ21148 | 219751_at | NM_024860 | NM_024860 | 79918 | -0.72 | 3.93E-04 |
| KIAA0773 | 205368_at | NM_014690 | NM_014690 | 9715 | -0.72 | 3.08E-03 |
| FBLP-1 | 1555480_a_at | AF459643 | NM_017556 | 54751 | -0.71 | 6.08E-03 |
| PCDHA3 | 211870_s_at | AF152481 | NM_018906 | 56145 | -0.71 | 8.36E-04 |
| | | | NM_031497 | | | |
| ULK3 | 225067_at | AL117482 | NM_015518 | 25989 | -0.71 | 5.76E-04 |
| ALS4 | 201965_s_at | NM_015046 | NM_015046 | 23064 | -0.71 | 1.86E-03 |
| RASEF | 243862_at | AW205358 | NM_152573 | 158158 | -0.71 | 2.57E-03 |
| C16orf45 | 212736_at | BE299456 | NM_033201 | 89927 | -0.71 | 9.07E-06 |
| PAK7 | 213990_s_at | BF056517 | NM_020341 | 57144 | -0.71 | 5.12E-03 |
| | | | NM_177990 | | | |
| SF3B3 | 200688_at | D13642 | NM_012426 | 23450 | -0.71 | 6.09E-04 |
| BRSK1 | 1552504_a_at | NM_032430 | NM_032430 | 84446 | -0.71 | 8.13E-04 |
| C14orf103 | 226684_at | AI217906 | NM_018036 | 55102 | -0.71 | 3.88E-03 |
| SLC35E1 | 229410_at | AI659219 | NM_024881 | 79939 | -0.71 | 9.27E-04 |
| NFYC | 202215_s_at | NM_014223 | NM_014223 | 4802 | -0.71 | 1.98E-03 |
| MRPL53 | 225523_at | AV726817 | NM_053050 | 116540 | -0.71 | 8.44E-04 |
| C5orf14 | 220495_s_at | NM_024715 | NM_024715 | 79770 | -0.71 | 8.19E-03 |
| RUFY1 | 218243_at | NM_025158 | NM_025158 | 80230 | -0.71 | 4.37E-04 |
| C11orf11 | 214128_at | AB014559 | NM_006133 | 747 | -0.71 | 6.28E-03 |
| ZNF404 | 239043_at | AA084273 | XM_292765 | 342908 | -0.71 | 6.01E-03 |
| TTC3 | 208073_x_at | NM_003316 | NM_001001894 | 7267 | -0.71 | 3.39E-04 |
| | | | NM_003316 | | | |
| RNASET2 | 217984_at | NM_003730 | NM_003730 | 8635 | -0.71 | 8.81E-04 |
| AI200555 | 228275_at | AI200555 | --- | --- | -0.71 | 1.65E-03 |
| NM_021031 | 220452_x_at | NM_021031 | --- | --- | -0.71 | 1.15E-03 |
| MGC16169 | 240269_at | BF590274 | NM_033115 | 93627 | -0.71 | 5.80E-03 |
| BF590958 | 226243_at | BF590958 | XM_372919 | 391356 | -0.71 | 5.37E-05 |
| RNF139 | 209510_at | AF064801 | NM_007218 | 11236 | -0.71 | 5.26E-04 |
| CNOT4 | 210203_at | R64001 | NM_001008225 | 4850 | -0.71 | 8.35E-05 |
| | | | NM_013316 | | | |
| MCM3APAS | 220459_at | NM_018118 | --- | 114044 | -0.70 | 3.02E-03 |
| MGC42090 | 236705_at | AA017245 | NM_152774 | 256130 | -0.70 | 8.57E-03 |
| KIAA2026 | 238490_at | BG109896 | XM_088551 | 158358 | -0.70 | 5.75E-03 |
| SLC35E1 | 235035_at | BF342223 | NM_024881 | 79939 | -0.70 | 2.86E-03 |
| KIAA0690 | 1559126_at | BC042908 | NM_015179 | 23223 | -0.70 | 2.78E-04 |
| TIM14 | 225359_at | BF666961 | NM_145261 | 131118 | -0.70 | 5.32E-04 |
| | | | NM_201259 | | | |
| | | | NM_201260 | | | |
| | | | NM_201261 | | | |
| PHKA1 | 205450_at | NM_002637 | NM_002637 | 5255 | -0.70 | 2.40E-03 |
| RNF13 | 201780_s_at | NM_007282 | NM_007282 | 11342 | -0.70 | 6.65E-03 |
| | | | NM_183381 | | | |
| | | | NM_183382 | | | |
| | | | NM_183383 | | | |
| | | | NM_183384 | | | |
| CLASP2 | 212309_at | AV725315 | NM_015097 | 23122 | -0.70 | 9.41E-03 |
| VPS11 | 203292_s_at | NM_021729 | NM_021729 | 55823 | -0.70 | 2.21E-03 |
| AI791187 | 241724_x_at | AI791187 | --- | --- | -0.70 | 4.52E-03 |
| DCTN4 | 233490_at | AI985890 | NM_016221 | 51164 | -0.70 | 4.12E-03 |
| LASS5 | 224951_at | BE348305 | NM_147190 | 91012 | -0.70 | 5.49E-04 |
| SLC35E1 | 227518_at | AW051365 | NM_024881 | 79939 | -0.70 | 4.70E-03 |
| SPPL3 | 224638_at | AI338356 | NM_139015 | 121665 | -0.70 | 2.98E-03 |
| C11orf1 | 231530_s_at | BG150085 | NM_022761 | 64776 | -0.70 | 2.09E-04 |
| RNF103 | 202636_at | NM_005667 | NM_005667 | 7844 | -0.69 | 5.99E-04 |
| LOC134218 | 230893_at | AI223870 | NM_194283 | 134218 | -0.69 | 5.69E-03 |
| C21orf59 | 218123_at | NM_017835 | NM_017835 | 56683 | -0.69 | 2.83E-04 |
| | | | NM_021254 | | | |
| C19orf12 | 227704_at | N21279 | NM_031448 | 83636 | -0.69 | 4.72E-03 |
| ZF | 213584_s_at | AI432137 | NM_021212 | 58487 | -0.69 | 2.28E-04 |
| MGC4796 | 223852_s_at | BC005169 | NM_032017 | 83931 | -0.69 | 5.00E-03 |
| C9orf112 | 225586_at | BF222775 | NM_138778 | 92715 | -0.69 | 2.74E-06 |
| AK023354 | 222252_x_at | AK023354 | --- | --- | -0.69 | 9.83E-04 |
| AW873330 | 225674_at | AW873330 | --- | --- | -0.69 | 9.10E-04 |
| INSR | 213792_s_at | AA485908 | NM_000208 | 3643 | -0.69 | 6.19E-03 |
| C6orf111 | 212179_at | AW157501 | NM_032870 | 25957 | -0.69 | 2.41E-03 |
| AY094612 | 1566887_x_at | AY094612 | XM_499198 | 441699 | -0.69 | 9.34E-03 |
| ZNF275 | 225383_at | BF793625 | XM_372267 | 10838 | -0.68 | 4.76E-03 |
| AA811371 | 238098_at | AA811371 | --- | --- | -0.68 | 6.60E-03 |
| RABGAP1L | 203020_at | NM_014857 | NM_014857 | 9910 | -0.68 | 1.28E-04 |
| ARHGEF2 | 235595_at | AW299534 | NM_004723 | 9181 | -0.68 | 2.77E-03 |
| C6orf52 | 239417_x_at | AW103116 | XM_371798 | 347744 | -0.68 | 7.21E-05 |
| DCL-1 | 232371_at | AU155401 | NM_014880 | 9936 | -0.68 | 4.13E-03 |
| PPID | 204185_x_at | NM_005038 | NM_005038 | 5481 | -0.68 | 1.13E-03 |
| TP53 | 201746_at | NM_000546 | NM_000546 | 7157 | -0.68 | 6.97E-03 |
| DHRSX | 225503_at | AL547782 | NM_145177 | 207063 | -0.68 | 8.56E-05 |
| ATF6 | 226941_at | BF439325 | NM_007348 | 22926 | -0.68 | 1.77E-03 |
| PIK3R1 | 212240_s_at | AI679268 | NM_181504 | 5295 | -0.68 | 2.27E-03 |
| | | | NM_181523 | | | |
| | | | NM_181524 | | | |
| FFX-r2-Ec-bioDFX-r2-Ec-bioD-5AFFX-r2-Ec-bioD-5 | | | --- | --- | -0.68 | 7.51E-04 |
| CCNL1 | 220046_s_at | NM_020307 | NM_020307 | 57018 | -0.68 | 5.67E-04 |
| BC017431 | 1570087_at | BC017431 | --- | --- | -0.68 | 5.92E-03 |
| P29 | 202553_s_at | NM_015484 | NM_015484 | 25949 | -0.68 | 7.53E-03 |
| | | | NM_207170 | | | |
| KIAA0256 | 212451_at | N52532 | NM_014701 | 9728 | -0.68 | 2.25E-03 |
| PPP3CA | 202425_x_at | NM_000944 | NM_000944 | 5530 | -0.68 | 4.15E-03 |
| QTRT1 | 221270_s_at | NM_031209 | NM_031209 | 81890 | -0.68 | 7.11E-04 |
| C21orf2 | 203994_s_at | U84569 | NM_004928 | 755 | -0.67 | 4.72E-06 |
| KCNAB1 | 210471_s_at | U33428 | NM_003471 | 7881 | -0.67 | 3.93E-03 |
| | | | NM_172159 | | | |
| | | | NM_172160 | | | |
| ASB6 | 222524_s_at | AF151697 | NM_017873 | 140459 | -0.67 | 1.52E-04 |
| | | | NM_177999 | | | |
| BF438106 | 228045_at | BF438106 | --- | --- | -0.67 | 9.75E-03 |
| NFYC | 211797_s_at | U62296 | NM_014223 | 4802 | -0.67 | 5.31E-04 |
| AI344253 | 238412_at | AI344253 | --- | --- | -0.67 | 1.27E-03 |
| ZNF551 | 211721_s_at | BC005868 | NM_138347 | 90233 | -0.67 | 3.58E-03 |
| TETRAN | 209215_at | L11669 | NM_001120 | 10227 | -0.67 | 2.16E-05 |
| C9orf60 | 202452_at | AI991574 | NM_006336 | 10444 | -0.67 | 7.13E-04 |
| IGF2AS | 220561_at | NM_016412 | NM_016412 | 51214 | -0.67 | 8.13E-03 |
| ERO1L | 225750_at | BE966748 | NM_014584 | 30001 | -0.67 | 2.29E-06 |
| MGC16028 | 226196_s_at | AW827281 | NM_052873 | 112752 | -0.67 | 6.84E-03 |
| KIF1A | 225482_at | AL533416 | NM_004321 | 547 | -0.67 | 2.15E-03 |
| CAMTA1 | 213268_at | Z98884 | NM_015215 | 23261 | -0.67 | 4.84E-03 |
| MAPT | 203930_s_at | NM_016835 | NM_005910 | 4137 | -0.67 | 2.43E-03 |
| | | | NM_016834 | | | |
| | | | NM_016835 | | | |
| | | | NM_016841 | | | |
| MGC8721 | 200847_s_at | NM_016127 | NM_016127 | 51669 | -0.67 | 2.91E-03 |
| LOC158563 | 226274_at | AK025562 | --- | 158563 | -0.67 | 3.76E-04 |
| MAN1B1 | 218636_s_at | NM_016219 | NM_016219 | 11253 | -0.67 | 1.77E-05 |
| SFRS7 | 213649_at | AA524053 | NM_006276 | 6432 | -0.67 | 7.31E-05 |
| AK024117 | 234969_s_at | AK024117 | --- | --- | -0.67 | 8.03E-04 |
| AF336878 | 211454_x_at | AF336878 | --- | --- | -0.67 | 4.04E-03 |
| SPTBN1 | 212071_s_at | BE968833 | NM_003128 | 6711 | -0.67 | 1.01E-04 |
| | | | NM_178313 | | | |
| PCBD1 | 203557_s_at | NM_000281 | NM_000281 | 5092 | -0.67 | 1.83E-04 |
| | | | NM_00100193 9 | | | |
| CNOT6L | 227119_at | BF103856 | NM_144571 | 246175 | -0.67 | 7.28E-04 |
| SLC19A1 | 229489_at | AI681043 | NM_003056 | 6573 | -0.67 | 1.83E-03 |
| | | | NM_194255 | | | |
| C11orf1 | 222785_x_at | AJ250229 | NM_022761 | 64776 | -0.67 | 1.97E-04 |
| ACOX1 | 227962_at | BF435852 | NM_004035 | 51 | -0.67 | 9.83E-03 |
| | | | NM_007292 | | | |
| VGLL4 | 214004_s_at | AI806207 | NM_014667 | 9686 | -0.67 | 1.59E-04 |
| FBXL6 | 219189_at | NM_024555 | NM_012162 | 26233 | -0.67 | 5.47E-04 |
| | | | NM_024555 | | | |
| EIF3S9 | 242550_at | AA628539 | NM_003751 | 8662 | -0.67 | 2.25E-03 |
| | | | NM_182712 | | | |
| CYHR1 | 223421_at | BC005073 | NM_032687 | 50626 | -0.67 | 2.86E-03 |
| PQBP1 | 214527_s_at | AB041836 | NM_005710 | 10084 | -0.67 | 7.86E-03 |
| | | | NM_144494 | | | |
| | | | NM_144495 | | | |
| AA813998 | 235837_at | AA813998 | --- | --- | -0.67 | 9.41E-03 |
| MYST4 | 211874_s_at | AF119230 | NM_012330 | 23522 | -0.66 | 1.12E-03 |
| CXXC5 | 233955_x_at | AK001782 | NM_016463 | 51523 | -0.66 | 8.92E-03 |
| LOC54499 | 208715_at | BF002031 | NM_019026 | 54499 | -0.66 | 3.49E-04 |
| LOC286437 | 229531_at | AW182938 | --- | 286437 | -0.66 | 9.60E-03 |
| LOC283970 | 221992_at | AI925734 | XM_498469 | 283970 | -0.66 | 7.97E-05 |
| PPT2 | 209826_at | AF020544 | NM_005155 | 9374 | -0.66 | 6.89E-03 |
| | | | NM_138717 | | | |
| | | | NM_138934 | | | |
| GPR85 | 234303_s_at | AL161959 | NM_018970 | 54329 | -0.66 | 2.74E-03 |
| KIAA0350 | 242728_at | AA705118 | NM_015226 | 23274 | -0.66 | 8.55E-03 |
| C18orf23 | 230143_at | BF433220 | NM_152470 | 147341 | -0.66 | 6.22E-03 |
| LOC493856 | 226689_at | AI749451 | NM_001008388 | 493856 | -0.66 | 3.86E-04 |
| L1CAM | 204584_at | AI653981 | NM_000425 | 3897 | -0.66 | 3.46E-03 |
| | | | NM_024003 | | | |
| FLNA | 214752_x_at | AI625550 | NM_001456 | 2316 | -0.66 | 5.06E-03 |
| CSNK1G1 | 220640_at | NM_022048 | NM_001011664 | 53944 | -0.66 | 7.24E-03 |
| | | | NM_022048 | | | |
| C19orf24 | 221587_s_at | BC000890 | NM_017914 | 55009 | -0.66 | 1.58E-03 |
| KIF5C | 203130_s_at | NM_004522 | XM_377774 | 3800 | -0.66 | 3.64E-03 |
| CNOT4 | 210867_at | AF180475 | NM_001008225 | 4850 | -0.66 | 2.10E-03 |
| | | | NM_013316 | | | |
| ZNF502 | 229532_at | AI056364 | NM_033210 | 91392 | -0.66 | 4.74E-03 |
| PCDHA9 | 210674_s_at | AF152308 | NM_014005 | 56134 | -0.66 | 1.36E-03 |
| PCDHAC2 | | | NM_018898 | 56135 | | |
| PCDHAC1 | | | NM_018899 | 56136 | | |
| PCDHA13 | | | NM_018900 | 56137 | | |
| PCDHA12 | | | NM_018901 | 56138 | | |
| PCDHA11 | | | NM_018902 | 56139 | | |
| PCDHA10 | | | NM_018903 | 56140 | | |
| PCDHA8 | | | NM_018904 | 56141 | | |
| PCDHA7 | | | NM_018905 | 56142 | | |
| PCDHA6 | | | NM_018906 | 56143 | | |
| PCDHA5 | | | NM_018907 | 56144 | | |
| PCDHA4 | | | NM_018908 | 56145 | | |
| PCDHA3 | | | NM_018909 | 56146 | | |
| PCDHA2 | | | NM_018910 | 56147 | | |
| PCDHA1 | | | NM_018911 | 9752 | | |
| | | | NM_031410 | | | |
| | | | NM_031411 | | | |
| | | | NM_031495 | | | |
| INPP5B | 213804_at | AI039084 | NM_005540 | 3633 | -0.66 | 6.73E-03 |
| RNF41 | 201962_s_at | NM_005785 | NM_005785 | 10193 | -0.66 | 3.92E-04 |
| | | | NM_194358 | | | |
| | | | NM_194359 | | | |
| MLL2 | 227528_s_at | AI394529 | NM_003482 | 8085 | -0.66 | 1.08E-03 |
| EPC1 | 223875_s_at | AF277374 | NM_025209 | 80314 | -0.66 | 6.07E-03 |
| CLCN5 | 232127_at | AK021494 | NM_000084 | 1184 | -0.66 | 4.77E-03 |
| BE549909 | 228172_at | BE549909 | --- | --- | -0.66 | 4.49E-03 |
| DTX3 | 49049_at | N92708 | NM_178502 | 196403 | -0.66 | 9.76E-03 |
| AA491228 | 242176_at | AA491228 | --- | --- | -0.66 | 3.24E-03 |
| USP16 | 222616_s_at | AI806796 | NM_00100199 2 | 10600 | -0.66 | 3.36E-03 |
| | | | NM_006447 | | | |
| FLJ10824 | 212370_x_at | AL080183 | NM_001005751 | 387680 | -0.66 | 2.44E-04 |
| LOC387680 | | | | 55747 | | |
| | | | XM_290482 | | | |
| NFX1 | 1553103_at | NM_147134 | NM_002504 | 4799 | -0.66 | 5.66E-04 |
| | | | NM_147133 | | | |
| | | | NM_147134 | | | |
| MAPRE3 | 203841_x_at | BG222594 | NM_012326 | 22924 | -0.66 | 1.69E-04 |
| AW025023 | 228503_at | AW025023 | --- | --- | -0.66 | 6.46E-03 |
| EIF4G3 | 201935_s_at | AI768122 | NM_003760 | 8672 | -0.65 | 2.11E-03 |
| KIAA1223 | 225731_at | BF196876 | XM_048747 | 57182 | -0.65 | 2.55E-03 |
| RUFY1 | 233380_s_at | AA429145 | NM_025158 | 80230 | -0.65 | 2.87E-03 |
| TMEM43 | 222418_s_at | AA115485 | NM_024334 | 79188 | -0.65 | 6.70E-03 |
| SCMH1 | 221216_s_at | NM_012236 | NM_012236 | 22955 | -0.65 | 1.81E-06 |
| NSMAF | 203269_at | NM_003580 | NM_003580 | 8439 | -0.65 | 4.56E-03 |
| TPP1 | 200743_s_at | NM_000391 | NM_000391 | 1200 | -0.65 | 7.89E-04 |
| BSCL2 | 208906_at | BC004911 | NM_032667 | 26580 | -0.65 | 1.19E-03 |
| SNCA | 204466_s_at | BG260394 | NM_000345 | 6622 | -0.65 | 3.00E-06 |
| | | | NM_007308 | | | |
| AW452971 | 231152_at | AW452971 | --- | --- | -0.65 | 2.61E-03 |
| YWHAZ | 214848_at | U79277 | NM_003406 | 7534 | -0.65 | 2.64E-04 |
| | | | NM_145690 | | | |
| MANSC1 | 220945_x_at | NM_018050 | NM_018050 | 54682 | -0.65 | 8.05E-04 |
| SELT | 225561_at | BF692332 | NM_016275 | 51714 | -0.65 | 1.05E-03 |
| CHD2 | 228999_at | AW514564 | NM_001271 | 1106 | -0.65 | 1.05E-04 |
| AI458020 | 238026_at | AI458020 | --- | --- | -0.65 | 6.28E-04 |
| MGC35048 | 216135_at | AK000122 | NM_153208 | 124152 | -0.65 | 3.59E-03 |
| AI937687 | 232218_at | AI937687 | --- | --- | -0.65 | 1.85E-03 |
| PSENEN | 218302_at | NM_018468 | NM_172341 | 55851 | -0.65 | 3.74E-03 |
| LEMD3 | 218604_at | NM_014319 | NM_014319 | 23592 | -0.65 | 8.61E-05 |
| KIAA0368 | 212427_at | AB002366 | XM_036708 | 23392 | -0.65 | 3.72E-04 |
| CTSB | 213275_x_at | W47179 | NM_001908 | 1508 | -0.65 | 8.91E-03 |
| | | | NM_147780 | | | |
| | | | NM_147781 | | | |
| | | | NM_147782 | | | |
| | | | NM_147783 | | | |
| USP32 | 244871_s_at | AW268357 | NM_032582 | 84669 | -0.65 | 2.06E-03 |
| GAS5 | 224741_x_at | BG329175 | --- | 60674 | -0.65 | 2.74E-03 |
| ATP13A1 | 218052_s_at | NM_020410 | NM_020410 | 57130 | -0.65 | 1.62E-03 |
| AA973100 | 230725_at | AA973100 | --- | --- | -0.65 | 1.61E-03 |
| MBD3 | 222183_x_at | AL390153 | NM_003926 | 283579 | -0.64 | 1.72E-03 |
| FLJ25976 | | | NM_174943 | 53615 | | |
| CD248 | 219025_at | NM_020404 | NM_020404 | 57124 | -0.64 | 8.85E-04 |
| PERQ1 | 228755_at | NM_022574 | NM_022574 | 64599 | -0.64 | 4.85E-03 |
| FLJ10618 | 237741_at | AW514168 | NM_018155 | 55186 | -0.64 | 7.33E-03 |
| TGFBRAP1 | 205210_at | NM_004257 | NM_004257 | 9392 | -0.64 | 3.31E-03 |
| SNAP91 | 204953_at | NM_014841 | NM_014841 | 9892 | -0.64 | 7.24E-03 |
| AI631833 | 227396_at | AI631833 | --- | --- | -0.64 | 8.39E-03 |
| MICAL2 | 212472_at | BE965029 | NM_014632 | 9645 | -0.64 | 2.60E-04 |
| KIAA0592 | 211068_x_at | BC006456 | NM_015262 | 253725 | -0.64 | 1.32E-03 |
| RBM8A | 213852_at | BG289199 | NM_005105 | 9939 | -0.64 | 9.90E-03 |
| TMAP1 | 227326_at | BE966768 | NM_00100852 8 | 439921 | -0.64 | 8.06E-03 |
| | | | NM_00100852 9 | | | |
| | | | NM_198530 | | | |
| MRPL1 | 223154_at | AF212225 | NM_020236 | 65008 | -0.64 | 1.18E-03 |
| IDS | 1559136_s_at | AU140213 | NM_000202 | 3423 | -0.64 | 1.45E-03 |
| | | | NM_006123 | | | |
| TRIM8 | 221012_s_at | NM_030912 | NM_030912 | 81603 | -0.64 | 3.17E-04 |
| C1orf27 | 222720_x_at | W72877 | NM_017847 | 54953 | -0.64 | 2.02E-04 |
| TPP1 | 214196_s_at | AA602532 | NM_000391 | 1200 | -0.64 | 2.31E-03 |
| PMS2L2 | 215412_x_at | AB017007 | NM_002679 | 5380 | -0.64 | 9.44E-03 |
| PTEN | 225363_at | AK024986 | NM_000314 | 5728 | -0.64 | 4.41E-03 |
| C15orf24 | 217898_at | NM_020154 | NM_020154 | 56851 | -0.64 | 1.32E-03 |
| SELT | 217811_at | NM_016275 | NM_016275 | 51714 | -0.64 | 1.82E-04 |
| URB | 225242_s_at | AW303375 | NM_199511 | 151887 | -0.64 | 6.81E-03 |
| | | | NM_199512 | | | |
| EFHC1 | 219833_s_at | NM_018100 | NM_018100 | 114327 | -0.64 | 1.22E-03 |
| AW970002 | 235660_at | AW970002 | --- | --- | -0.64 | 4.41E-03 |
| C14orf43 | 225980_at | AV740879 | NM_194278 | 91748 | -0.64 | 1.54E-03 |
| DKFZP564J102 | 214890_s_at | AL080065 | NM_00100665 5 | 25854 | -0.63 | 7.10E-03 |
| | | | NM_015398 | | | |
| STARD7 | 236484_at | AW850611 | NM_020151 | 56910 | -0.63 | 1.49E-04 |
| | | | NM_139267 | | | |
| AW519141 | 227444_at | AW519141 | --- | --- | -0.63 | 1.87E-03 |
| TTC3 | 210645_s_at | D83077 | NM_00100189 4 | 7267 | -0.63 | 2.39E-03 |
| | | | NM_003316 | | | |
| SCN3B | 204722_at | AW007335 | NM_018400 | 55800 | -0.63 | 5.01E-05 |
| ATF6 | 217550_at | AA576497 | NM_007348 | 22926 | -0.63 | 5.13E-03 |
| OFD1 | 203569_s_at | NM_003611 | NM_003611 | 8481 | -0.63 | 4.46E-04 |
| ITPK1 | 210740_s_at | AF279372 | NM_014216 | 3705 | -0.63 | 7.31E-04 |
| CCNDBP1 | 223084_s_at | AF246144 | NM_012142 | 23582 | -0.63 | 1.14E-04 |
| | | | NM_037370 | | | |
| BEX2 | 224367_at | AF251053 | NM_032621 | 84707 | -0.63 | 5.01E-03 |
| KIAA1387 | 226230_at | AA541716 | NM_020463 | 57223 | -0.63 | 8.30E-03 |
| DND1 | 222070_at | AW090043 | NM_194249 | 373863 | -0.63 | 9.36E-03 |
| MLL2 | 227527_at | AI394529 | NM_003482 | 8085 | -0.63 | 6.88E-03 |
| C5orf14 | 227873_at | BE547587 | NM_024715 | 79770 | -0.63 | 3.10E-03 |
| TMED4 | 224680_at | AL539253 | NM_182547 | 222068 | -0.63 | 6.32E-04 |
| MTMR1 | 216095_x_at | AF057354 | NM_003828 | 8776 | -0.63 | 7.62E-03 |
| | | | NM_176789 | | | |
| MAP3K12 | 205448_s_at | NM_006301 | NM_006301 | 7786 | -0.63 | 1.84E-03 |
| KIAA0276 | 243691_at | AA150455 | NM_015115 | 23142 | -0.63 | 1.60E-03 |
| AV736334 | 237561_x_at | AV736334 | --- | --- | -0.63 | 5.35E-03 |
| LOC148189 | 235191_at | BE780373 | --- | 148189 | -0.63 | 7.40E-03 |
| FBXL2 | 214436_at | AF176518 | NM_012157 | 25827 | -0.63 | 6.41E-03 |
| HT036 | 223769_x_at | AF284750 | NM_031207 | 81888 | -0.63 | 1.42E-04 |
| MLX | 217910_x_at | NM_013383 | NM_170607 | 6945 | -0.62 | 8.02E-03 |
| | | | NM_198204 | | | |
| | | | NM_198205 | | | |
| C5orf5 | 218518_at | NM_016603 | NM_016603 | 51306 | -0.62 | 3.54E-03 |
| AI652919 | 230581_at | AI652919 | --- | --- | -0.62 | 3.52E-03 |
| BF196255 | 229921_at | BF196255 | --- | --- | -0.62 | 4.55E-03 |
| KCNF1 | 210263_at | AF029780 | NM_002236 | 3754 | -0.62 | 7.86E-03 |
| LKAP | 202386_s_at | NM_019081 | NM_014647 | 9665 | -0.62 | 2.93E-05 |
| | | | NM_019081 | | | |
| AU151222 | 227526_at | AU151222 | --- | --- | -0.62 | 1.49E-04 |
| ZNF266 | 214686_at | AA868898 | NM_006631 | 10781 | -0.62 | 3.90E-03 |
| | | | NM_198058 | | | |
| MAGI-3 | 226770_at | AI692181 | NM_020965 | 260425 | -0.62 | 8.14E-04 |
| | | | NM_152900 | | | |
| HNRPR | 208765_s_at | NM_005826 | NM_005826 | 10236 | -0.62 | 1.26E-03 |
| TSTA3 | 36936_at | U58766 | NM_003313 | 7264 | -0.62 | 6.42E-03 |
| AK001513 | 233642_s_at | AK001513 | --- | --- | -0.62 | 2.84E-04 |
| OGT | 212307_s_at | BF001665 | NM_003605 | 8473 | -0.62 | 2.58E-03 |
| | | | NM_181672 | | | |
| | | | NM_181673 | | | |
| POMT2 | 225560_at | AK023804 | NM_013382 | 29954 | -0.62 | 4.78E-03 |
| KIAA1340 | 225732_at | AU146850 | NM_020782 | 57542 | -0.62 | 5.78E-03 |
| FLJ21148 | 1554555_a_at | BC022451 | NM_024860 | 79918 | -0.61 | 3.94E-04 |
| ZFX | 229022_at | AI745209 | NM_003410 | 7543 | -0.61 | 5.57E-03 |
| GPR56 | 212070_at | AL554008 | NM_005682 | 9289 | -0.61 | 6.44E-03 |
| | | | NM_201524 | | | |
| | | | NM_201525 | | | |
| BBS7 | 235007_at | AI683802 | NM_018190 | 55212 | -0.61 | 9.30E-03 |
| | | | NM_176824 | | | |
| EIF2C4 | 227930_at | AI669957 | NM_017629 | 192670 | -0.61 | 7.32E-03 |
| FLJ13868 | 222190_s_at | BC000822 | NM_022744 | 64755 | -0.61 | 6.09E-03 |
| AI524240 | 238863_x_at | AI524240 | --- | --- | -0.61 | 5.90E-03 |
| AI140364 | 221740_x_at | AI140364 | XM_373737 | 388397 | -0.61 | 5.62E-03 |
| PR01853 | 220943_s_at | NM_018607 | NM_018607 | 55471 | -0.61 | 4.64E-04 |
| | | | NM_144736 | | | |
| FLJ20850 | 51200_at | AI744084 | NM_017967 | 55049 | -0.61 | 7.02E-05 |
| TGDS | 237346_at | AA976208 | NM_014305 | 23483 | -0.61 | 7.82E-03 |
| COMMD10 | 240595_at | AA928078 | NM_016144 | 51397 | -0.61 | 3.63E-04 |
| FLJ31340 | 235301_at | AI797353 | NM_152748 | 222223 | -0.61 | 4.73E-04 |
| C2orf17 | 221984_s_at | AL040896 | NM_024293 | 79137 | -0.61 | 7.61E-03 |
| VPS54 | 222627_at | AK002205 | NM_00100573 9 | 51542 | -0.61 | 8.85E-03 |
| | | | NM_016516 | | | |
| WDR9 | 225446_at | AI638279 | NM_00100724 6 | 54014 | -0.61 | 6.06E-03 |
| | | | NM_018963 | | | |
| | | | NM_033656 | | | |
| LOC157697 | 227016_at | AA767385 | NM_207332 | 157697 | -0.61 | 8.34E-03 |
| SLC35E1 | 79005_at | AA504646 | NM_024881 | 79939 | -0.61 | 2.09E-03 |
| RFX1 | 226786_at | BF507952 | NM_002918 | 5989 | -0.61 | 1.11E-03 |
| MDS028 | 220589_s_at | NM_018463 | NM_018463 | 55846 | -0.61 | 4.18E-04 |
| RNF111 | 241413_at | W80457 | NM_017610 | 54778 | -0.61 | 4.93E-03 |
| RPL10 | 221989_at | AW057781 | NM_006013 | 6134 | -0.61 | 8.66E-03 |
| NR1H2 | 218215_s_at | NM_007121 | NM_007121 | 7376 | -0.61 | 3.22E-05 |
| TSPYL2 | 218012_at | NM_022117 | NM_022117 | 64061 | -0.61 | 4.00E-03 |
| COG7 | 213190_at | R61519 | NM_153603 | 91949 | -0.60 | 1.96E-03 |
| NM_018507 | 219599_at | NM_018507 | --- | --- | -0.60 | 7.03E-03 |
| ZNF638 | 213775_x_at | AI357871 | NM_014497 | 27332 | -0.60 | 9.27E-03 |
| ABCD4 | 203981_s_at | AL574660 | NM_005050 | 5826 | -0.60 | 1.48E-04 |
| | | | NM_020323 | | | |
| | | | NM_020324 | | | |
| | | | NM_020325 | | | |
| | | | NM_020326 | | | |
| MKLN1 | 243959_at | N35099 | NM_013255 | 4289 | -0.60 | 8.87E-04 |
| FFX-r2-Ec-bioDFX-r2-Ec-bioD-3AFFX-r2-Ec-bioD-5 | | | --- | - | -0.60 | 1.47E-03 |
| KIAA1277 | 53991_at | AA127623 | NM_015689 | 27147 | -0.60 | 1.38E-03 |
| RAB11B | 217793_at | AL575337 | NM_004218 | 9230 | -0.60 | 8.56E-04 |
| KRTCAP3 | 235148_at | BF680458 | NM_173853 | 200634 | -0.60 | 6.87E-03 |
| ILVBL | 210624_s_at | BC000109 | NM_006844 | 10994 | -0.60 | 9.87E-03 |
| | | | NM_176826 | | | |
| CACNB1 | 210185_at | AB054985 | NM_000723 | 782 | -0.60 | 3.90E-04 |
| | | | NM_199247 | | | |
| | | | NM_199248 | | | |
| ZFYVE28 | 1569369_at | BC035793 | NM_020972 | 57732 | -0.60 | 2.58E-05 |
| C21orf127 | 220311_at | NM_013240 | NM_013240 | 29104 | -0.60 | 3.00E-03 |
| | | | NM_182749 | | | |
| SNAPC3 | 229712_at | AI066599 | NM_003084 | 6619 | -0.60 | 8.08E-04 |
| JAM3 | 212813_at | AA149644 | NM_032801 | 83700 | -0.60 | 2.27E-05 |
| KPNA1 | 202057_at | BC002374 | NM_002264 | 3836 | -0.60 | 8.33E-03 |
| HEL308 | 1554342_s_at | BC011863 | NM_133636 | 113510 | -0.60 | 9.43E-03 |
| ET | 221192_x_at | NM_024311 | NM_024311 | 79157 | -0.60 | 2.02E-03 |
| SNRPB2 | 1561965_at | BE466925 | NM_003092 | 6629 | -0.60 | 2.74E-03 |
| | | | NM_198220 | | | |
| MLL | 212080_at | AV714029 | NM_005933 | 4297 | -0.60 | 4.11E-03 |
| FAIM2 | 203618_at | AB023167 | NM_012306 | 23017 | -0.60 | 3.14E-03 |
| RASA2 | 230669_at | W38444 | NM_006506 | 5922 | -0.59 | 4.05E-03 |
| GRIK5 | 214966_at | S40369 | NM_002088 | 2901 | -0.59 | 4.00E-03 |
| PARP6 | 234710_s_at | AK021790 | NM_020213 | 56965 | -0.59 | 2.29E-04 |
| | | | NM_020214 | | | |
| DXYS155E | 203624_at | NM_005088 | NM_005088 | 8227 | -0.59 | 9.05E-03 |
| DUSP3 | 201536_at | AL048503 | NM_004090 | 1845 | -0.59 | 5.91E-03 |
| FLJ10324 | 223693_s_at | AL136731 | NM_018059 | 55698 | -0.59 | 4.22E-04 |
| KATNB1 | 203163_at | NM_005886 | NM_005886 | 10300 | -0.59 | 1.72E-03 |
| C12orf10 | 218220_at | NM_021640 | NM_021640 | 60314 | -0.59 | 4.83E-03 |
| SCN3B | 204723_at | AB032984 | NM_018400 | 55800 | -0.59 | 5.81E-04 |
| IL17D | 227401_at | BE856748 | NM_138284 | 53342 | -0.59 | 5.23E-04 |
| TUSC3 | 209228_x_at | U42349 | NM_006765 | 7991 | -0.59 | 8.59E-05 |
| | | | NM_178234 | | | |
| ATP6AP1 | 207809_s_at | NM_001183 | NM_001183 | 537 | -0.59 | 3.60E-03 |
| DKFZP564D166 | 224952_at | BF115054 | XM_371074 | 26115 | -0.59 | 7.36E-06 |
| SFRS5 | 203380_x_at | NM_006925 | NM_006925 | 6430 | -0.59 | 3.73E-03 |
| MRX85 | 225867 _at | BE741869 | NM_138440 | 114990 | -0.59 | 2.02E-03 |
| SBF2 | 226169_at | AW276572 | NM_030962 | 81846 | -0.59 | 9.95E-04 |
| CDC16 | 209658_at | AF164598 | NM_003903 | 8881 | -0.59 | 7.37E-03 |
| LOC493856 | 226686_at | AI188518 | NM_001008388 | 493856 | -0.59 | 3.94E-03 |
| LAMB2 | 216264_s_at | X79683 | NM_002292 | 3913 | -0.59 | 5.67E-03 |
| FLJ10826 | 225110_at | AK024314 | NM_018233 | 55239 | -0.59 | 4.75E-03 |
| TLOC1 | 208942_s_at | BE866511 | NM_003262 | 7095 | -0.59 | 2.74E-03 |
| VPS24 | 222436_s_at | AF219226 | NM_00100575 3 | 51652 | -0.59 | 9.34E-04 |
| | | | NM_016079 | | | |
| ECE2 | 230706_s_at | AI084921 | NM_014693 | 9718 | -0.59 | 5.56E-04 |
| ZNF292 | 212368_at | AA972711 | XM_048070 | 23036 | -0.59 | 8.05E-04 |
| AK022413 | 233658_at | AK022413 | --- | --- | -0.59 | 7.17E-03 |
| FLNA | 200859_x_at | NM_001456 | NM_001456 | 2316 | -0.59 | 6.02E-03 |
| TIGD1 | 1553099_at | NM_145702 | NM_145702 | 200765 | -0.59 | 5.58E-03 |
| HUMPPA | 213230_at | AI422335 | NM_014603 | 30850 | -0.59 | 2.36E-04 |
| NUDT13 | 214136_at | W80642 | NM_015901 | 25961 | -0.58 | 8.37E-03 |
| NMT2 | 215069_at | AK025065 | NM_004808 | 9397 | -0.58 | 5.28E-03 |
| LOC51255 | 223064_at | AF151072 | NM_016494 | 51255 | -0.58 | 8.47E-04 |
| NPD014 | 209007_s_at | AF267856 | NM_020317 | 57035 | -0.58 | 6.29E-03 |
| | | | NM_207035 | | | |
| DGKD | 208072_s_at | NM_003648 | NM_003648 | 8527 | -0.58 | 5.37E-04 |
| | | | NM_152879 | | | |
| FLJ10824 | 212929_s_at | W68158 | NM_00100575 1 | 387680 | -0.58 | 9.32E-03 |
| LOC387680 | | | | 55747 | | |
| | | | XM_290482 | | | |
| AL049985 | 215507_x_at | AL049985 | --- | --- | -0.58 | 4.06E-03 |
| PCNXL2 | 1554256_a_at | BC008300 | NM_014801 | 80003 | -0.58 | 9.20E-03 |
| | | | NM_024938 | | | |
| MGC54289 | 225228_at | AI474054 | NM_178454 | 128338 | -0.58 | 2.51E-04 |
| FBXL14 | 213145_at | BF001666 | NM_152441 | 144699 | -0.58 | 2.17E-03 |
| TFG | 239385_at | AI150613 | NM_00100756 5 | 10342 | -0.58 | 1.31E-03 |
| | | | NM_006070 | | | |
| MACF1 | 215222_x_at | AK023406 | NM_012090 | 23499 | -0.58 | 6.16E-03 |
| | | | NM_033044 | | | |
| AW268719 | 236798_at | AW268719 | --- | --- | -0.58 | 1.80E-04 |
| BRUNOL6 | 227775_at | BE467313 | NM_052840 | 60677 | -0.58 | 7.34E-03 |
| CLN3 | 209275_s_at | AF015593 | NM_000086 | 1201 | -0.58 | 5.20E-04 |
| CKLFSF3 | 224733_at | AL574900 | NM_144601 | 123920 | -0.58 | 6.40E-04 |
| | | | NM_181553 | | | |
| | | | NM_181554 | | | |
| | | | NM_181555 | | | |
| DSCR1 | 208370_s_at | NM_004414 | NM_004414 | 1827 | -0.58 | 2.94E-03 |
| | | | NM_203417 | | | |
| | | | NM_203418 | | | |
| PFDN5 | 210908_s_at | AB055804 | NM_002624 | 5204 | -0.58 | 9.12E-04 |
| | | | NM_145896 | | | |
| | | | NM_145897 | | | |
| AA206016 | 228191_at | AA206016 | --- | --- | -0.58 | 2.94E-03 |
| IDS | 212221_x_at | AV703259 | NM_000202 | 3423 | -0.58 | 7.55E-06 |
| | | | NM_006123 | | | |
| ZNF295 | 225539_at | AP001745 | NM_020727 | 49854 | -0.58 | 4.67E-03 |
| AW292996 | 236330_at | AW292996 | --- | --- | -0.58 | 3.90E-03 |
| LOC253982 | 214993_at | AF070642 | NM_181718 | 253982 | -0.58 | 6.71E-04 |
| | | | NM_198907 | | | |
| USP24 | 212388_at | AB028980 | XM_371254 | 23358 | -0.58 | 1.07E-03 |
| AW237752 | 229699_at | AW237752 | --- | --- | -0.58 | 7.54E-03 |
| UNQ1912 | 226752_at | AI816071 | NM_198507 | 345757 | -0.58 | 1.56E-03 |
| PHC1 | 225958_at | AI554106 | NM_004426 | 1911 | -0.58 | 2.64E-03 |
| PAN3 | 225563_at | AI970788 | NM_175854 | 255967 | -0.58 | 1.42E-04 |

**Table 9. Increased Gene Expression in Cell Lines H1 and BB10 vs. SiMa Cell Line in Undifferentiated State Using Microarray Analysis**

| **Gene Symbol** | **Probe Set ID** | **Representative Public ID** | **RefSeq Transcript ID** | **Entrez Gene ID** | **Log2 Ratio** | **pvalue** |
|---|---|---|---|---|---|---|
| DAZ1 | 208282_x_at | NM_020363 | NM_00100537 5 | 1617 | 6.15 | 6.22E-05 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| BF476109 | 1556300_s_at | BF476109 | --- | --- | 6.07 | 4.58E-08 |
| DAZ1 | 207909_x_at | U21663 | NM_00100537 5 | 1617 | 6.03 | 3.64E-05 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| TIMP3 | 201147_s_at | BF347089 | NM_000362 | 7078 | 5.78 | 9.00E-07 |
| LUM | 201744_s_at | NM_002345 | NM_002345 | 4060 | 5.28 | 9.11E-07 |
| SIM1 | 206876_at | AL121948 | NM_005068 | 6492 | 5.13 | 3.40E-06 |
| TIMP3 | 201149_s_at | U67195 | NM_000362 | 7078 | 5.09 | 4.07E-05 |
| CXCR4 | 209201_x_at | L01639 | NM_00100854 0 | 7852 | 4.91 | 1.18E-03 |
| | | | NM_003467 | | | |
| GRP | 206326_at | NM_002091 | NM_002091 | 2922 | 4.77 | 1.84E-06 |
| MGC24103 | 232568_at | AU145658 | --- | 158295 | 4.71 | 1.34E-05 |
| CXCR4 | 211919_s_at | AF348491 | NM_00100854 0 | 7852 | 4.65 | 1.78E-03 |
| | | | NM_003467 | | | |
| KCTD12 | 212192_at | AI718937 | NM_138444 | 115207 | 4.61 | 1.59E-05 |
| DAZ1 | 207912_s_at | NM_004081 | NM_00100537 5 | 1617 | 4.39 | 5.11E-04 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| DAZ1 | 216922_x_at | AF271088 | NM_00100537 5 | 1617 | | 4.72E-04 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| DAZ1 | 208281_x_at | NM_020364 | | 1617 | 4.30 | 3.22E-04 |
| DAZ3 | | | NM_00100537 5 | 57054 | | |
| DAZ2 | | | | 57055 | | |
| DAZ4 | | | NM_00100578 5 | 57135 | | |
| | | | NM_00100578 6 | | | |
| | | | NM_004081 | | | |
| | | | NM_020363 | | | |
| | | | NM_020364 | | | |
| | | | NM_020420 | | | |
| PTPRE | 221840_at | AA775177 | NM_006504 | 5791 | 4.28 | 4.00E-07 |
| | | | NM_130435 | | | |
| AF086027 | 1559697_a_at | AF086027 | --- | --- | 4.28 | 1.11E-04 |
| BNC2 | 233036_at | AU146418 | NM_017637 | 54796 | 4.20 | 2.05E-04 |
| H99792 | 225710_at | H99792 | --- | --- | 4.20 | 1.70E-06 |
| DKFZp667G211 | 214030_at | BE501352 | XM_376254 | 131544 | 4.19 | 1.81E-09 |
| AL049260 | 216518_at | AL049260 | --- | --- | 4.16 | 8.81E-04 |
| AU144918 | 233858_at | AU144918 | --- | --- | 4.15 | 1.87E-05 |
| GPC6 | 233090_at | AU144140 | NM_005708 | 10082 | 4.08 | 1.76E-03 |
| TIMP3 | 201150_s_at | NM_000362 | NM_000362 | 7078 | 4.04 | 7.75E-05 |
| ALCAM | 240655_at | BE502785 | NM_001627 | 214 | 3.98 | 1.71E-04 |
| SSBP2 | 1557813_at | BF724621 | NM_012446 | 23635 | 3.98 | 1.18E-03 |
| C1orf1 | 233306_at | AK021509 | --- | 711 | 3.97 | 8.32E-06 |
| ARHGAP6 | 206167_s_at | NM_001174 | NM_001174 | 395 | 3.91 | 9.85E-05 |
| | | | NM_006125 | | | |
| | | | NM_013422 | | | |
| | | | NM_013423 | | | |
| | | | NM_013427 | | | |
| TFPI2 | 209278_s_at | L27624 | NM_006528 | 7980 | 3.87 | 1.53E-06 |
| AL049337 | 215768_at | AL049337 | --- | --- | 3.87 | 3.23E-03 |
| FLJ39639 | 243547_at | BE176531 | XM_370932 | 283876 | 3.83 | 3.19E-04 |
| KIAA1944 | 236377_at | AW166133 | NM_133448 | 121256 | 3.80 | 2.79E-03 |
| AF086027 | 1559696_at | AF086027 | --- | --- | 3.77 | 3.26E-05 |
| VIP | 206577_at | NM_003381 | NM_003381 | 7432 | 3.72 | 1.05E-03 |
| | | | NM_194435 | | | |
| CCDC3 | 223316_at | AL136562 | NM_031455 | 83643 | 3.72 | 8.39E-06 |
| POLQ | 207746_at | NM_014125 | NM_006596 | 10721 | 3.54 | 1.20E-04 |
| | | | NM_199420 | | | |
| SLAC2-B | 214734_at | AB014524 | NM_015065 | 23086 | 3.53 | 2.59E-03 |
| GPC6 | 232453_at | AF339813 | NM_005708 | 10082 | 3.52 | 8.95E-04 |
| MBNL1 | 241681_at | AW296451 | NM_021038 | 4154 | 3.50 | 2.40E-03 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| EML4 | 233940_at | AK022801 | NM_019063 | 27436 | 3.49 | 4.67E-03 |
| AW974642 | 1558410_s_at | AW974642 | XM_499534 | 442612 | 3.49 | 1.47E-03 |
| RGS5 | 1555725_a_at | AF493929 | NM_003617 | 8490 | 3.47 | 3.83E-03 |
| TIMP3 | 201148_s_at | AW338933 | NM_000362 | 7078 | 3.46 | 9.21E-03 |
| AI939547 | 243004_at | AI939547 | --- | --- | 3.45 | 6.37E-03 |
| SLITRK5 | 214930_at | AW449813 | NM_015567 | 26050 | 3.42 | 1.80E-05 |
| GFRA2 | 205722_s_at | NM_001495 | NM_001495 | 2675 | 3.38 | 1.70E-03 |
| RGS5 | 209070_s_at | AI183997 | NM_003617 | 8490 | 3.32 | 3.17E-03 |
| EML1 | 204797_s_at | NM_004434 | NM_00100870 7 | 2009 | 3.29 | 2.09E-04 |
| | | | NM_004434 | | | |
| FBXL7 | 241457_at | AI821935 | NM_012304 | 23194 | 3.27 | 1.14E-03 |
| PLK2 | 201939_at | NM_006622 | NM_006622 | 10769 | 3.27 | 1.65E-06 |
| NEGR1 | 243357_at | AA115106 | NM_173808 | 257194 | 3.26 | 2.19E-06 |
| ELOVL7 | 227180_at | AW138767 | XM_371740 | 79993 | 3.26 | 3.23E-03 |
| SPAG6 | 210033_s_at | AF079363 | NM_012443 | 9576 | 3.23 | 3.60E-05 |
| | | | NM_172242 | | | |
| FLJ39660 | 1553459_at | NM_173646 | NM_173646 | 284992 | 3.22 | 5.48E-03 |
| NEDD9 | 202150_s_at | U64317 | NM_006403 | 4739 | 3.22 | 1.70E-04 |
| | | | NM_182966 | | | |
| PDZRN3 | 233442_at | AU147500 | NM_015009 | 23024 | 3.21 | 2.82E-05 |
| TRIM29 | 211002_s_at | AF230389 | NM_012101 | 23650 | 3.18 | 3.79E-05 |
| | | | NM_058193 | | | |
| PCDH9 | 219737_s_at | AI524125 | NM_020403 | 5101 | 3.13 | 7.67E-06 |
| | | | NM_203487 | | | |
| FAT | 201579_at | NM_005245 | NM_005245 | 2195 | 3.11 | 4.81E-03 |
| NEDD9 | 202149_at | AL136139 | NM_006403 | 4739 | 3.08 | 2.64E-04 |
| | | | NM_182966 | | | |
| ITGA6 | 201656_at | NM_000210 | NM_000210 | 3655 | 3.07 | 8.91E-05 |
| DAZ1 | 216351_x_at | AF248483 | NM_00100537 5 | 1617 | 3.06 | 3.84E-04 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| FLJ37266 | 1566948_at | AL831894 | NM_175892 | 283225 | 3.06 | 3.09E-03 |
| EFNA5 | 1559360_at | AL833045 | NM_001962 | 1946 | 3.03 | 1.65E-05 |
| RPL10A | 242188_at | AI743332 | NM_007104 | 4736 | 3.03 | 2.49E-03 |
| BE466926 | 217671_at | BE466926 | --- | --- | 3.02 | 9.00E-03 |
| PTPRG | 1569323_at | BU853579 | NM_002841 | 5793 | 3.01 | 1.51E-05 |
| CUGBP2 | 202158_s_at | NM_006561 | NM_006561 | 10659 | 3.01 | 8.47E-05 |
| TULP4 | 232372_at | AL157491 | NM_00100746 6 | 56995 | 3.01 | 6.44E-03 |
| | | | NM_020245 | | | |
| BC043430 | 1558714_at | BC043430 | --- | --- | 2.99 | 6.39E-03 |
| LOC340109 | 1557765_at | BC039509 | XM_379322 | 340109 | 2.99 | 6.58E-05 |
| DKFZp7610201 | 232313_at | AL122107 | XM_044062 | 92293 | 2.98 | 3.82E-05 |
| RGS5 | 218353_at | NM_025226 | NM_003617 | 8490 | 2.96 | 3.43E-03 |
| CHRNA7 | 210123_s_at | U62436 | NM_000746 | 1139 | 2.96 | 5.22E-06 |
| CHRFAM7A | | | NM_139320 | 89832 | | |
| | | | NM_148911 | | | |
| LOC440265 | 1569476_at | BC033224 | XM_378506 | 440265 | 2.90 | 2.51E-03 |
| ARHGEF6 | 209539_at | D25304 | NM_004840 | 9459 | 2.90 | 7.95E-05 |
| AL049277 | 234839_at | AL049277 | --- | --- | 2.89 | 4.82E-03 |
| KIAA0265 | 238000_at | BF195340 | NM_014997 | 23008 | 2.85 | 2.90E-04 |
| DACT1 | 219179_at | NM_016651 | NM_016651 | 51339 | 2.85 | 4.31E-04 |
| KCTD12 | 212188_at | AA551075 | NM_138444 | 115207 | 2.84 | 1.79E-04 |
| RGS5 | 209071_s_at | AF159570 | NM_003617 | 8490 | 2.84 | 3.29E-03 |
| AL832561 | 1561689_at | AL832561 | --- | --- | 2.84 | 3.60E-05 |
| AA872187 | 230332_at | AA872187 | --- | --- | 2.82 | 1.50E-03 |
| BG542611 | 1556277_a_at | BG542611 | --- | --- | 2.81 | 2.09E-03 |
| PIAS2 | 244633_at | AA404996 | NM_004671 | 9063 | 2.80 | 3.93E-05 |
| | | | NM_173206 | | | |
| AI630830 | 243150_at | AI630830 | --- | --- | 2.79 | 2.12E-03 |
| UCHL1 | 1555834_at | AW974143 | NM_004181 | 7345 | 2.77 | 3.11E-03 |
| TRIO | 235705_at | BF676361 | NM_007118 | 7204 | 2.76 | 4.51E-04 |
| AK022040 | 233445_at | AK022040 | --- | --- | 2.76 | 7.74E-04 |
| CBLN2 | 242301_at | R60224 | NM_182511 | 147381 | 2.76 | 3.83E-03 |
| MID1 | 242671_at | BF055144 | NM_000381 | 4281 | 2.74 | 4.62E-04 |
| | | | NM_033290 | | | |
| | | | NM_033291 | | | |
| BC041991 | 1561690_at | BC041991 | --- | --- | 2.73 | 2.25E-03 |
| EML4 | 232587_at | AI208611 | NM_019063 | 27436 | 2.72 | 8.16E-03 |
| TRIM29 | 202504_at | NM_012101 | NM_012101 | 23650 | 2.70 | 1.08E-03 |
| | | | NM_058193 | | | |
| AW139261 | 243934_at | AW139261 | --- | --- | 2.69 | 5.92E-03 |
| ROBO2 | 226709_at | BE858548 | XM_031246 | 6092 | 2.69 | 1.07E-04 |
| MEF2A | 239571_at | AI123399 | NM_005587 | 4205 | 2.68 | 1.53E-04 |
| KIAA1128 | 240499_at | AA482221 | NM_018999 | 54462 | 2.68 | 8.42E-03 |
| KLF7 | 239809_at | BF197708 | NM_003709 | 8609 | 2.65 | 5.01E-03 |
| CALCA | 217561_at | BF447272 | NM_001741 | 796 | 2.62 | 2.08E-03 |
| LOC51760 | 205613_at | NM_016524 | NM_016524 | 51760 | 2.61 | 5.89E-03 |
| EDD | 232264_at | AK022204 | NM_015902 | 51366 | 2.61 | 7.50E-04 |
| LOC440161 | 243241_at | AW341473 | XM_498572 | 440161 | 2.61 | 2.72E-04 |
| SCHIP1 | 1566772_at | AL832567 | NM_014575 | 29970 | 2.61 | 1.17E-03 |
| TFPI2 | 209277_at | AL574096 | NM_006528 | 7980 | 2.61 | 6.75E-04 |
| EML4 | 1556732_at | BC039354 | NM_019063 | 27436 | 2.59 | 2.34E-03 |
| AA808231 | 242025_at | AA808231 | --- | --- | 2.59 | 1.52E-04 |
| CUGBP2 | 202157_s_at | U69546 | NM_006561 | 10659 | 2.59 | 6.58E-06 |
| PAPD4 | 222282_at | AV761453 | NM_173797 | 167153 | 2.57 | 4.37E-03 |
| MGC14161 | 240502_at | AW015920 | NM_032892 | 84978 | 2.55 | 2.58E-03 |
| UBE3A | 1560741_at | AL832250 | | 7337 | 2.54 | 5.57E-03 |
| | | | NM_000462 | | | |
| | | | NM_130838 | | | |
| | | | NM_130839 | | | |
| LRRC5 | 234148_at | AK025238 | NM_018103 | 55144 | 2.54 | 8.12E-04 |
| PEX5L | 1562473_at | BC037943 | NM_016559 | 51555 | 2.54 | 7.14E-04 |
| IMP-2 | 218847_at | NM_006548 | NM_00100722 5 | 10644 | 2.54 | 9.50E-05 |
| | | | NM_006548 | | | |
| C2orf31 | 221245_s_at | NM_030804 | --- | 81561 | 2.51 | 8.63E-05 |
| AK024882 | 233228_at | AK024882 | --- | --- | 2.50 | 4.73E-04 |
| TAS2R45 | 234042_at | AF264628 | NM_176886 | 259291 | 2.50 | 4.78E-04 |
| KIAA1102 | 241459_at | AI494113 | NM_014988 | 22998 | 2.48 | 4.62E-05 |
| BC041965 | 1560411_at | BC041965 | --- | --- | 2.47 | 7.60E-03 |
| USP31 | 239348_at | AI285970 | NM_020718 | 57478 | 2.47 | 5.27E-03 |
| AU144294 | 215574_at | AU144294 | --- | --- | 2.47 | 3.25E-03 |
| RNF152 | 233180_at | AU147152 | NM_173557 | 220441 | 2.46 | 7.81E-03 |
| LOC440738 | 221697 _at | AF276659 | NM_001004343 | 440738 | 2.45 | 2.37E-03 |
| KATNAL1 | 243828_at | AA758906 | NM_032116 | 84056 | 2.44 | 1.32E-03 |
| MGC39558 | 1562391_at | AK091497 | NM_152490 | 148789 | 2.44 | 7.21E-03 |
| GNG11 | 204115_at | NM_004126 | NM_004126 | 2791 | 2.43 | 1.79E-04 |
| AI076172 | 236355_s_at | AI076172 | XM_495841 | 439993 | 2.42 | 3.12E-03 |
| ITPR1 | 240052_at | AA648993 | NM_002222 | 3708 | 2.42 | 1.52E-04 |
| IARS | 239794_at | AI356405 | NM_002161 | 3376 | 2.42 | 2.89E-04 |
| | | | NM_013417 | | | |
| PLXNA4 | 232317_at | AB046770 | XM_039393 | 57671 | 2.41 | 8.26E-05 |
| | | | XM_379927 | | | |
| AURKB | 239219_at | N55457 | NM_004217 | 9212 | 2.41 | 5.13E-04 |
| FBXL7 | 233276_at | AU146390 | NM_012304 | 23194 | 2.41 | 3.40E-04 |
| CDH2 | 237305_at | AW450381 | NM_001792 | 1000 | 2.40 | 2.95E-03 |
| SPRY4 | 1566968_at | AK024556 | NM_030964 | 81848 | 2.40 | 1.67E-05 |
| SYT4 | 223529_at | AB037763 | NM_020783 | 6860 | 2.40 | 4.26E-04 |
| PPP4R2 | 1560926_at | AF085924 | NM_174907 | 151987 | 2.38 | 6.45E-03 |
| BE674460 | 237849_at | BE674460 | --- | --- | 2.38 | 7.15E-04 |
| AK024956 | 232726_at | AK024956 | --- | --- | 2.37 | 2.24E-03 |
| PITPNB | 242074_at | AA833902 | NM_012399 | 23760 | 2.37 | 2.45E-04 |
| KIAA0485 | 214295_at | AW129056 | --- | 57235 | 2.36 | 6.51E-04 |
| BF000203 | 236385_at | BF000203 | --- | --- | 2.33 | 1.55E-03 |
| BNC2 | 220272_at | NM_017637 | NM_017637 | 54796 | 2.33 | 3.96E-04 |
| SDFR1 | 242865_at | AI332638 | NM_012428 | 27020 | 2.31 | 1.45E-04 |
| | | | NM_017455 | | | |
| DTNA | 1557803_at | AF086074 | NM_001390 | 1837 | 2.31 | 3.61E-04 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| NRCAM | 233401_at | BF723605 | NM_005010 | 4897 | 2.31 | 1.07E-03 |
| FOXO3A | 1569477_at | BC025999 | NM_001455 | 2309 | 2.31 | 5.92E-03 |
| | | | NM_201559 | | | |
| MSN | 200600_at | NM_002444 | NM_002444 | 4478 | 2.30 | 1.14E-05 |
| AL832142 | 1561195_at | AL832142 | --- | --- | 2.30 | 2.10E-03 |
| EDG3 | 228176_at | AA534817 | NM_005226 | 1903 | 2.29 | 2.67E-03 |
| ASCL1 | 209985_s_at | BE797438 | NM_004316 | 429 | 2.28 | 9.42E-05 |
| LOC146174 | 233832_at | AK021824 | NM_173501 | 146174 | 2.27 | 3.37E-04 |
| PLXNA4 | 228104_at | AL117427 | XM_039393 | 57671 | 2.26 | 7.59E-04 |
| | | | XM_379927 | | | |
| KIDINS220 | 1557246_at | AA992480 | NM_020738 | 57498 | 2.26 | 5.87E-03 |
| GFRA2 | 205721_at | U97145 | NM_001495 | 2675 | 2.25 | 3.08E-03 |
| LOC440092 | 1570571_at | BC035168 | XM_498540 | 440092 | 2.25 | 3.18E-05 |
| ROBO2 | 226766_at | AB046788 | XM_031246 | 6092 | 2.25 | 3.58E-05 |
| GAB2 | 1566959_at | AL049273 | NM_012296 | 9846 | 2.24 | 9.27E-03 |
| | | | NM_080491 | | | |
| ARHGAP6 | 1563386_at | AL079282 | NM_001174 | 395 | 2.24 | 2.24E-03 |
| | | | NM_006125 | | | |
| | | | NM_013422 | | | |
| | | | NM_013423 | | | |
| | | | NM_013427 | | | |
| PCNX | 215175_at | AB023212 | NM_014982 | 22990 | 2.24 | 3.95E-03 |
| C10orf18 | 216211_at | AL049233 | XM_374765 | 54906 | 2.23 | 1.64E-03 |
| AI678013 | 240165_at | AI678013 | --- | --- | 2.22 | 1.17E-03 |
| AI653240 | 240247_at | AI653240 | XM_496059 | 440261 | 2.21 | 1.90E-04 |
| GFM1 | 232295_at | AK000780 | NM_024996 | 85476 | 2.20 | 4.88E-03 |
| AF116659 | 210717_at | AF116659 | --- | --- | 2.20 | 2.72E-03 |
| NM_018611 | 220666_at | NM_018611 | --- | --- | 2.20 | 3.83E-03 |
| PFKFB3 | 202464_s_at | NM_004566 | NM_004566 | 5209 | 2.20 | 1.42E-03 |
| AI671885 | 230494_at | AI671885 | XM_379123 | 400999 | 2.20 | 4.83E-04 |
| TPBG | 203476_at | NM_006670 | NM_006670 | 7162 | 2.20 | 1.21E-07 |
| ITGA6 | 215177_s_at | AV733308 | NM_000210 | 3655 | 2.19 | 2.48E-03 |
| AW008791 | 240315_at | AW008791 | XM_294019 | 345930 | 2.19 | 7.20E-03 |
| C10orf18 | 244165_at | AI809511 | XM_374765 | 54906 | 2.18 | 1.55E-03 |
| FLJ21148 | 231005_at | T91195 | NM_024860 | 79918 | 2.18 | 3.18E-04 |
| DEFB32 | 243311_at | BE044548 | NM_207469 | 400830 | 2.18 | 1.71E-03 |
| THRAP2 | 232307_at | AK021554 | NM_015335 | 23389 | 2.18 | 7.79E-04 |
| BF057073 | 231310_at | BF057073 | --- | --- | 2.16 | 3.16E-05 |
| CFDP1 | 210701_at | D85939 | NM_006324 | 10428 | 2.16 | 1.15E-03 |
| SYNE2 | 242774_at | AI684761 | NM_015180 | 23224 | 2.16 | 3.57E-03 |
| | | | NM_182910 | | | |
| | | | NM_182912 | | | |
| | | | NM_182913 | | | |
| | | | NM_182914 | | | |
| RNF182 | 230720_at | AI884906 | NM_152737 | 221687 | 2.15 | 1.49E-04 |
| PAPD4 | 242144_at | AW104325 | NM_173797 | 167153 | 2.15 | 8.28E-03 |
| DKFZp761D221 | 241872_at | AI149963 | NM_032291 | 84251 | 2.15 | 7.56E-03 |
| NFIB | 230791_at | AU146924 | NM_005596 | 4781 | 2.14 | 1.39E-03 |
| PTGFRN | 224937_at | BF311866 | NM_020440 | 5738 | 2.14 | 1.95E-03 |
| FOXO3A | 242320_at | AI435586 | NM_001455 | 2309 | 2.13 | 1.69E-03 |
| | | | NM_201559 | | | |
| SSBP2 | 1557814_a_at | BF724621 | NM_012446 | 23635 | 2.13 | 6.51E-03 |
| KLF12 | 243089_at | AA551114 | NM_007249 | 11278 | 2.13 | 1.78E-03 |
| | | | NM_016285 | | | |
| EXT1 | 232174_at | AA480392 | NM_000127 | 2131 | 2.13 | 2.01E-04 |
| MGC4504 | 219270_at | NM_024111 | NM_024111 | 79094 | 2.13 | 2.30E-03 |
| TTL | 244078_at | AI438957 | NM_153712 | 150465 | 2.12 | 1.97E-04 |
| ODZ3 | 233291_at | AU145618 | XM_371717 | 55714 | 2.11 | 2.81E-04 |
| MSI2 | 239232_at | AA521410 | NM_138962 | 124540 | 2.11 | 7.13E-04 |
| | | | NM_170721 | | | |
| ZNF403 | 233937_at | AK024883 | NM_024835 | 79893 | 2.11 | 2.80E-04 |
| FRZB | 203698_s_at | NM_001463 | NM_001463 | 2487 | 2.10 | 6.12E-03 |
| ATP1B4 | 243737_at | AI082610 | NM_012069 | 23439 | 2.10 | 3.38E-03 |
| AI077566 | 243994_at | AI077566 | --- | --- | 2.09 | 3.50E-03 |
| GATA4 | 243692_at | AW181962 | NM_002052 | 2626 | 2.09 | 2.75E-03 |
| ID2 | 213931_at | AI819238 | NM_002166 | 3398 | 2.09 | 3.49E-04 |
| N21631 | 231329_at | N21631 | --- | --- | 2.08 | 3.60E-03 |
| GNAI1 | 209576_at | AL049933 | NM_002069 | 2770 | 2.08 | 4.56E-05 |
| ZNF292 | 236435_at | BE219036 | XM_048070 | 23036 | 2.08 | 7.00E-03 |
| NEDD9 | 233223_at | AK000850 | NM_006403 | 4739 | 2.07 | 4.93E-04 |
| | | | NM_182966 | | | |
| UBE2E3 | 232528_at | AI338705 | NM_006357 | 10477 | 2.06 | 7.31E-03 |
| | | | NM_182678 | | | |
| PAN3 | 239393_at | AW510927 | NM_175854 | 255967 | 2.05 | 4.66E-03 |
| SMARCC1 | 239238_at | AI208857 | NM_003074 | 6599 | 2.04 | 7.80E-04 |
| MTMR2 | 231057_at | AU144266 | NM_016156 | 8898 | 2.04 | 8.90E-05 |
| | | | NM_201278 | | | |
| | | | NM_201281 | | | |
| MGC33926 | 229302_at | AA058832 | NM_152390 | 130733 | 2.04 | 4.33E-05 |
| DREV1 | 230395_at | AI536637 | NM_016025 | 51108 | 2.04 | 3.23E-03 |
| AF238870 | 215811_at | AF238870 | --- | --- | 2.04 | 1.97E-03 |
| DMXL2 | 215761_at | AK000156 | NM_015263 | 23312 | 2.04 | 2.78E-04 |
| MAOA | 204388_s_at | NM_000240 | NM_000240 | 4128 | 2.04 | 7.48E-04 |
| OSBPL3 | 209626_s_at | AI202969 | NM_015550 | 26031 | 2.04 | 1.76E-03 |
| | | | NM_145320 | | | |
| | | | NM_145321 | | | |
| | | | NM_145322 | | | |
| | | | NM_145323 | | | |
| | | | NM_145324 | | | |
| AL161982 | 1563494_at | AL161982 | --- | --- | 2.03 | 1.29E-03 |
| SALL4 | 229661_at | NM_020436 | NM_020436 | 57167 | 2.03 | 2.39E-03 |
| DJ971N18.2 | 240094_at | AL042660 | NM_021156 | 56255 | 2.03 | 1.10E-03 |
| GOLPH4 | 233877_at | AK000777 | NM_014498 | 27333 | 2.02 | 1.37E-03 |
| MSX2 | 205555_s_at | D31771 | NM_002449 | 4488 | 2.02 | 2.73E-03 |
| SR140 | 236696_at | BE464843 | XM_031553 | 23350 | 2.02 | 1.40E-03 |
| PSCD3 | 243752_s_at | AI870144 | NM_004227 | 9265 | 2.02 | 5.25E-05 |
| JMJD2C | 239285_at | AI341227 | NM_015061 | 23081 | 2.02 | 1.02E-03 |
| THRAP2 | 238883_at | AW975051 | NM_015335 | 23389 | 2.01 | 3.31E-03 |
| BF508839 | 239907_at | BF508839 | --- | --- | 2.01 | 1.50E-03 |
| VRK1 | 241737_x_at | T89693 | NM_003384 | 7443 | 2.01 | 4.67E-05 |
| AA417878 | 243463_s_at | AA417878 | --- | --- | 2.01 | 4.39E-03 |
| RAP1GDS1 | 237856_at | AI052055 | NM_021159 | 5910 | 1.99 | 6.70E-03 |
| BF724206 | 244457_at | BF724206 | --- | --- | 1.99 | 5.28E-03 |
| MAOA | 212741_at | AA923354 | NM_000240 | 4128 | 1.98 | 7.12E-04 |
| TPTE | 233879_at | AL137389 | NM_013315 | 7179 | 1.97 | 6.72E-03 |
| | | | NM_199259 | | | |
| | | | NM_199260 | | | |
| | | | NM_199261 | | | |
| SPAG6 | 210032_s_at | AI651156 | NM_012443 | 9576 | 1.97 | 2.71E-04 |
| | | | NM_172242 | | | |
| VDP | 222316_at | AW973253 | NM_003715 | 8615 | 1.97 | 8.98E-03 |
| FLJ35740 | 244297_at | AI806762 | NM_147195 | 253650 | 1.97 | 7.86E-03 |
| SPPL3 | 1554565_x_at | BC011943 | NM_139015 | 121665 | 1.96 | 5.95E-04 |
| BC028185 | 1569609_at | BC028185 | XM_496328 | 440546 | 1.96 | 4.09E-05 |
| MYH10 | 237491_at | AA700633 | NM_005964 | 4628 | 1.96 | 3.02E-03 |
| TMP21 | 238886_at | BF056141 | NM_006827 | 10972 | 1.96 | 1.48E-03 |
| TFCP2 | 242859_at | BE156563 | NM_005653 | 7024 | 1.95 | 8.85E-03 |
| BE856929 | 239349_at | BE856929 | --- | --- | 1.95 | 4.12E-03 |
| LOC151878 | 222372_at | AW971248 | --- | 151878 | 1.95 | 1.02E-03 |
| SEC15L2 | 1569202_x_at | BF847120 | XM_039570 | 23233 | 1.94 | 2.05E-05 |
| SCFD1 | 243525_at | AA808051 | NM_016106 | 23256 | 1.94 | 1.42E-03 |
| | | | NM_182835 | | | |
| RTN4 | 243031_at | N90377 | NM_007008 | 57142 | 1.94 | 3.06E-03 |
| | | | NM_020532 | | | |
| | | | NM_153828 | | | |
| | | | NM_207520 | | | |
| | | | NM_207521 | | | |
| TRA1 | 239451_at | AI684643 | NM_003299 | 7184 | 1.93 | 2.01E-03 |
| CSE1L | 210765_at | AF053640 | NM_001316 | 1434 | 1.93 | 2.75E-04 |
| | | | NM_177436 | | | |
| ATXN2 | 238589_s_at | AW601184 | NM_002973 | 6311 | 1.93 | 1.04E-03 |
| GNAI1 | 227692_at | AU153866 | NM_002069 | 2770 | 1.93 | 7.06E-05 |
| TCF12 | 235925_at | AW057520 | NM_003205 | 6938 | 1.93 | 2.43E-03 |
| | | | NM_207036 | | | |
| | | | NM_207037 | | | |
| | | | NM_207038 | | | |
| | | | NM_207040 | | | |
| LASS5 | 238299_at | AW005866 | NM_147190 | 91012 | 1.93 | 1.53E-04 |
| AA766886 | 239171_at | AA766886 | --- | --- | 1.92 | 7.67E-03 |
| KIAA1102 | 1569208_a_at | BC020895 | NM_014988 | 22998 | 1.92 | 1.80E-03 |
| SARS | 237689_at | BF111108 | NM_006513 | 6301 | 1.92 | 5.62E-03 |
| VTI1A | 233323_at | AK024973 | NM_145206 | 143187 | 1.91 | 6.85E-04 |
| B4GALT5 | 1559391_s_at | AI084451 | NM_004776 | 9334 | 1.91 | 9.95E-03 |
| AA804267 | 243185_at | AA804267 | --- | --- | 1.91 | 1.67E-03 |
| MGC34032 | 1569112_at | AW020413 | NM_152697 | 204962 | 1.90 | 1.85E-03 |
| AL050204 | 213929_at | AL050204 | --- | --- | 1.90 | 5.32E-03 |
| FLJ31951 | 236322_at | AA830854 | NM_144726 | 153830 | 1.89 | 9.55E-03 |
| ASCL1 | 209987_s_at | BC002341 | NM_004316 | 429 | 1.89 | 1.70E-06 |
| LOC441157 | 1558795_at | AL833240 | XM_499037 | 441157 | 1.89 | 7.22E-05 |
| NOL7 | 242143_at | BE674964 | NM_016167 | 51406 | 1.89 | 1.62E-03 |
| XPO1 | 235927_at | BE350122 | NM_003400 | 7514 | 1.88 | 1.14E-04 |
| FOXP1 | 240666_at | AI732568 | NM_032682 | 27086 | 1.88 | 6.71E-03 |
| CD9 | 201005_at | NM_001769 | NM_001769 | 928 | 1.87 | 3.75E-03 |
| TNKS | 238108_at | AI401627 | NM_003747 | 8658 | 1.87 | 8.80E-03 |
| NID | 202007_at | BF940043 | NM_002508 | 4811 | 1.87 | 3.81E-03 |
| MARS | 213672_at | AA621558 | NM_004990 | 4141 | 1.86 | 2.54E-04 |
| C20orf23 | 232083_at | AB046810 | NM_024704 | 55614 | 1.86 | 1.17E-04 |
| KIAA1713 | 233536_at | AI202664 | XM_290811 | 80816 | 1.86 | 3.15E-05 |
| U80755 | 243820_at | U80755 | --- | --- | 1.85 | 8.97E-05 |
| AA700817 | 241997_at | AA700817 | --- | --- | 1.85 | 4.70E-03 |
| UBE2E1 | 215577_at | AU146791 | NM_003341 | 7324 | 1.85 | 2.95E-03 |
| | | | NM_182666 | | | |
| AP2B1 | 233800_at | AA805082 | NM_001282 | 163 | 1.84 | 4.55E-05 |
| AGTPBP1 | 242008_at | BE350312 | NM_015239 | 23287 | 1.83 | 9.60E-03 |
| RPL4 | 1562440_at | BC026249 | NM_000968 | 6124 | 1.83 | 1.24E-03 |
| LOC441157 | 1558796_a_at | AL833240 | XM_499037 | 441157 | 1.83 | 3.63E-06 |
| EIF3S8 | 236700_at | AI377875 | NM_003752 | 8663 | 1.82 | 1.24E-07 |
| ZDHHC2 | 243528_at | AW014108 | NM_016353 | 51201 | 1.82 | 8.62E-05 |
| WSB1 | 227501_at | AI377135 | NM_015626 | 26118 | 1.82 | 2.98E-03 |
| | | | NM_134264 | | | |
| | | | NM_134265 | | | |
| PTPN12 | 244356_at | AL079909 | NM_002835 | 5782 | 1.81 | 5.18E-03 |
| RB1CC1 | 237626_at | AI801666 | NM_014781 | 9821 | 1.81 | 2.19E-03 |
| PCTK2 | 243993_at | AA436887 | NM_002595 | 5128 | 1.80 | 9.68E-03 |
| PIP5K1A | 235646_at | BF515595 | NM_003557 | 8394 | 1.80 | 5.13E-03 |
| AU146318 | 232355_at | AU146318 | --- | --- | 1.80 | 7.55E-03 |
| N59650 | 244265_at | N59650 | --- | --- | 1.80 | 8.30E-03 |
| YARS | 238760_at | AW452122 | NM_003680 | 8565 | 1.80 | 4.51E-03 |
| RNF159 | 241956_at | AI521883 | NM_032373 | 84333 | 1.80 | 5.20E-05 |
| AI820961 | 240331_at | AI820961 | --- | --- | 1.79 | 7.22E-03 |
| FLJ30058 | 238047_at | AA405456 | NM_144967 | 158763 | 1.79 | 5.67E-03 |
| GULP1 | 223837_at | BC001103 | NM_016315 | 51454 | 1.79 | 6.85E-04 |
| CGI-26 | 230892_at | AI912194 | NM_015954 | 51071 | 1.79 | 1.73E-03 |
| LRCH3 | 1559490_at | AL832278 | NM_032773 | 84859 | 1.79 | 6.87E-03 |
| HIRA | 240451_at | AA004844 | NM_003325 | 7290 | 1.79 | 2.21E-05 |
| SRPK2 | 239811_at | BF954306 | NM_182691 | 6733 | 1.78 | 5.28E-03 |
| | | | NM_182692 | | | |
| C9orf33 | 217130_at | AF072164 | --- | 90477 | 1.78 | 1.23E-04 |
| AI916641 | 242471_at | AI916641 | --- | --- | 1.78 | 4.35E-04 |
| SCC-112 | 241798_at | AI339930 | NM_015200 | 23244 | 1.78 | 1.62E-03 |
| SLC33A1 | 1559232_a_at | BC029450 | NM_004733 | 9197 | 1.78 | 1.37E-04 |
| ATRX | 236778_at | AA826176 | NM_000489 | 546 | 1.78 | 2.50E-03 |
| | | | NM_138270 | | | |
| | | | NM_138271 | | | |
| ZFYVE16 | 1554638_at | BC032227 | NM_014733 | 9765 | 1.78 | 1.30E-03 |
| FLJ13089 | 236816_at | BF110370 | NM_024953 | 80018 | 1.78 | 9.12E-03 |
| SLC7A2 | 225516_at | AA876372 | NM_001008539 | 6542 | 1.78 | 2.48E-06 |
| | | | NM_003046 | | | |
| DBH | 206450_at | NM_000787 | NM_000787 | 1621 | 1.77 | 5.25E-04 |
| H15073 | 236685_at | H15073 | --- | --- | 1.77 | 1.63E-04 |
| R45137 | 238304_at | R45137 | --- | --- | 1.77 | 4.97E-03 |
| FBXW8 | 237317_at | AW136338 | NM_012174 | 26259 | 1.76 | 4.78E-03 |
| | | | NM_153348 | | | |
| MLL3 | 244010_at | AI057455 | NM_021230 | 58508 | 1.76 | 5.04E-05 |
| | | | NM_170606 | | | |
| SLC37A3 | 1569666_s_at | BC035773 | NM_032295 | 84255 | 1.76 | 1.21E-05 |
| | | | NM_207113 | | | |
| CNOT2 | 222313_at | AW972359 | NM_014515 | 4848 | 1.76 | 1.85E-04 |
| NS5ATP13TP2 | 1558613_at | BC040647 | NM_178507 | 220323 | 1.75 | 7.91E-04 |
| MBNL1 | 1556657_at | AA744622 | NM_021038 | 4154 | 1.74 | 1.02E-04 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| AU152272 | 1556650_at | AU152272 | --- | --- | 1.74 | 2.69E-03 |
| AI372879 | 237134_at | AI372879 | --- | --- | 1.73 | 1.97E-03 |
| N23846 | 1557804_at | N23846 | --- | --- | 1.72 | 6.11E-03 |
| C10orf47 | 229801_at | AI640157 | NM_153256 | 254427 | 1.72 | 6.95E-03 |
| W72466 | 231467_at | W72466 | --- | --- | 1.72 | 2.85E-05 |
| MAPRE2 | 1564733_at | BC013931 | NM_014268 | 10982 | 1.72 | 1.15E-03 |
| R38403 | 240425_x_at | R38403 | --- | --- | 1.72 | 2.69E-03 |
| AF090944 | 233995_at | AF090944 | --- | --- | 1.72 | 3.50E-04 |
| RANBP17 | 234137_s_at | AK022022 | NM_022897 | 64901 | 1.71 | 3.68E-03 |
| OAZIN | 240231_at | AI742383 | NM_015878 | 51582 | 1.71 | 1.34E-07 |
| | | | NM_148174 | | | |
| L3MBTL | 242637_at | C14069 | NM_015478 | 26013 | 1.71 | 6.68E-03 |
| | | | NM_032107 | | | |
| AB062477 | 1555303_at | AB062477 | --- | --- | 1.71 | 6.75E-03 |
| AA682539 | 235575_at | AA682539 | --- | --- | 1.70 | 8.28E-03 |
| AL353942 | 216147_at | AL353942 | NM_018243 | 55752 | 1.70 | 3.06E-04 |
| VPS26 | 243316_x_at | AA004710 | NM_004896 | 9559 | 1.70 | 1.14E-04 |
| LAF4 | 243967_at | AI424899 | NM_002285 | 3899 | 1.70 | 8.24E-03 |
| SEZ6L | 240709_at | AW204757 | NM_021115 | 23544 | 1.70 | 9.27E-04 |
| AVO3 | 237330_at | AA603494 | XM_380173 | 253260 | 1.70 | 6.44E-04 |
| TPARL | 1560622_at | AK000203 | NM_018475 | 55858 | 1.69 | 1.26E-03 |
| PDLIM5 | 213684_s_at | BF671400 | NM_00101151 3 | 10611 | 1.69 | 6.08E-03 |
| | | | NM_00101151 4 | | | |
| | | | NM_00101151 5 | | | |
| | | | NM_00101151 6 | | | |
| | | | NM_006457 | | | |
| SOS2 | 233369_at | AU146027 | NM_006939 | 6655 | 1.69 | 9.57E-03 |
| IREB2 | 1563130_a_at | AL109710 | NM_004136 | 3658 | 1.68 | 7.14E-06 |
| MPZL1 | 210210_at | AF181660 | NM_003953 | 9019 | 1.68 | 5.09E-05 |
| | | | NM_024569 | | | |
| AW206536 | 243470_at | AW206536 | --- | --- | 1.68 | 4.56E-03 |
| TOP1 | 239404_at | BF840360 | NM_003286 | 7150 | 1.67 | 5.17E-04 |
| AA936428 | 244674_at | AA936428 | --- | --- | 1.67 | 1.91E-03 |
| PUM2 | 235138_at | AA565051 | NM_015317 | 23369 | 1.67 | 8.24E-04 |
| MINA | 213189_at | BE966695 | NM_032778 | 84864 | 1.67 | 9.49E-05 |
| | | | NM_153182 | | | |
| LOC152185 | 239091_at | AA497043 | NM_144718 | 152185 | 1.67 | 4.23E-04 |
| SEC31L1 | 1556416_s_at | AF086242 | NM_014933 | 22872 | 1.67 | 6.36E-04 |
| | | | NM_016211 | | | |
| MAOA | 204389_at | NM_000240 | NM_000240 | 4128 | 1.66 | 2.78E-04 |
| ALCAM | 1569362_at | BC041127 | NM_001627 | 214 | 1.66 | 8.23E-03 |
| KRT18 | 201596_x_at | NM_000224 | NM_000224 | 3875 | 1.66 | 1.32E-03 |
| | | | NM_199187 | | | |
| N73550 | 244112_x_at | N73550 | --- | --- | 1.66 | 5.23E-03 |
| MKLN1 | 1560145_at | BQ942131 | NM_013255 | 4289 | 1.65 | 4.78E-03 |
| ARHGAP5 | 242110_at | AV735587 | NM_001173 | 394 | 1.65 | 8.85E-03 |
| GPI | 232002_at | AW843302 | NM_000175 | 2821 | 1.65 | 6.38E-04 |
| STAU | 1569527_at | BC017275 | NM_004602 | 6780 | 1.65 | 2.32E-04 |
| | | | NM_017452 | | | |
| | | | NM_017453 | | | |
| | | | NM_017454 | | | |
| BF512254 | 242695_at | BF512254 | --- | --- | 1.65 | 2.52E-03 |
| EIF4ENIF1 | 242983_at | AI806626 | NM_019843 | 56478 | 1.65 | 4.13E-03 |
| AU144781 | 233300_at | AU144781 | --- | --- | 1.65 | 2.25E-03 |
| DKFZp547A023 | 214731_at | AB037854 | NM_018704 | 55917 | 1.65 | 8.41E-03 |
| LBH | 221011_s_at | NM_030915 | NM_030915 | 81606 | 1.64 | 3.86E-05 |
| KIAA0217 | 241965_at | BF589232 | NM_015155 | 23185 | 1.64 | 3.53E-04 |
| MED31 | 243319_at | AI274981 | NM_016060 | 51003 | 1.64 | 2.26E-04 |
| DLEU2 | 1556821_x_at | H48516 | NM_006021 | 8847 | 1.64 | 8.43E-03 |
| AA195014 | 243769_at | AA195014 | --- | --- | 1.63 | 9.11E-03 |
| N50083 | 229110_at | N50083 | --- | --- | 1.63 | 1.73E-03 |
| N46436 | 1556568_a_at | N46436 | --- | --- | 1.63 | 4.33E-04 |
| RAPGEF5 | 204680_s_at | AI263837 | XM_499059 | 9771 | 1.62 | 1.84E-03 |
| AW003140 | 227576_at | AW003140 | --- | --- | 1.62 | 5.17E-03 |
| AL119001 | 233440_at | AL119001 | --- | --- | 1.62 | 5.61E-03 |
| CPSF6 | 238651_at | BF512491 | NM_007007 | 11052 | 1.62 | 4.97E-03 |
| RBM5 | 230742_at | AA742596 | NM_005778 | 10181 | 1.62 | 6.51E-03 |
| CADPS | 233950_at | AK000873 | NM_003716 | 8618 | 1.62 | 3.44E-04 |
| | | | NM_183393 | | | |
| | | | NM_183394 | | | |
| SPON1 | 209437_s_at | AB051390 | NM_006108 | 10418 | 1.62 | 1.60E-03 |
| SCFD1 | 241775_at | AW298119 | NM_016106 | 23256 | 1.61 | 2.89E-03 |
| | | | NM_182835 | | | |
| CDC14A | 210742_at | AF064103 | NM_003672 | 8556 | 1.61 | 4.92E-03 |
| | | | NM_033312 | | | |
| | | | NM_033313 | | | |
| FZD5 | 206136_at | NM_003468 | NM_003468 | 7855 | 1.61 | 1.56E-03 |
| ZNF451 | 215012_at | AU144775 | NM_015555 | 26036 | 1.61 | 9.28E-03 |
| PCGF5 | 226326_at | AI798098 | NM_032373 | 84333 | 1.61 | 2.79E-05 |
| PTGFRN | 224950_at | BF476250 | NM_020440 | 5738 | 1.61 | 8.83E-03 |
| TSC | 218872_at | NM_017899 | NM_017899 | 54997 | 1.61 | 5.32E-04 |
| GRM8 | 216255_s_at | AC000099 | NM_000845 | 2918 | 1.60 | 3.03E-05 |
| MGC47869 | 243056_at | AI301111 | NM_175874 | 144608 | 1.60 | 1.26E-03 |
| DNAPTP6 | 244597_at | AA701247 | NM_015535 | 26010 | 1.60 | 2.38E-03 |
| BC028606 | 1555485_s_at | BC028606 | --- | --- | 1.60 | 5.40E-03 |
| ARID1B | 1558822_at | AF147412 | NM_017519 | 57492 | 1.60 | 2.93E-03 |
| | | | NM_020732 | | | |
| | | | NM_175863 | | | |
| BF841212 | 232096_x_at | BF841212 | --- | --- | 1.60 | 1.12E-03 |
| AU144005 | 232797 _at | AU144005 | --- | --- | 1.60 | 2.27E-03 |
| LOC158160 | 244075_at | BF224218 | NM_182829 | 158160 | 1.60 | 3.37E-03 |
| MYO6 | 210480_s_at | U90236 | NM_004999 | 4646 | 1.59 | 6.83E-04 |
| MBNL2 | 236699_at | AL566294 | NM_144778 | 10150 | 1.59 | 3.46E-03 |
| | | | NM_207304 | | | |
| BI868311 | 1565651_at | BI868311 | --- | --- | 1.59 | 4.00E-03 |
| LETMD1 | 242424_at | AA345855 | NM_015416 | 25875 | 1.59 | 5.03E-04 |
| N75937 | 240072_at | N75937 | --- | --- | 1.59 | 6.26E-03 |
| H04482 | 243287_s_at | H04482 | --- | --- | 1.59 | 3.04E-03 |
| NFIB | 233304_at | AU145782 | NM_005596 | 4781 | 1.59 | 6.80E-03 |
| PPP3R1 | 1565811_at | BC033945 | NM_000945 | 5534 | 1.59 | 6.08E-05 |
| DLEU2 | 216870_x_at | AF264787 | NM_006021 | 8847 | 1.59 | 2.46E-04 |
| COBRA1 | 1556434_at | BQ777552 | NM_015456 | 25920 | 1.59 | 2.75E-04 |
| C6orf60 | 1558523_at | AJ420563 | NM_024581 | 79632 | 1.58 | 6.89E-03 |
| MAK3 | 231199_at | AA701676 | NM_025146 | 80218 | 1.58 | 3.34E-03 |
| KIAA0485 | 242824_at | AW191647 | --- | 57235 | 1.57 | 3.46E-05 |
| CTBP2 | 233315_at | AK024947 | NM_001329 | 1488 | 1.57 | 2.17E-05 |
| | | | NM_022802 | | | |
| AA935659 | 240321_at | AA935659 | --- | --- | 1.57 | 9.78E-03 |
| TAF15 | 227884_at | AW296067 | NM_003487 | 8148 | 1.56 | 6.85E-03 |
| | | | NM_139215 | | | |
| KIF5C | 1557089_at | CA449408 | XM_377774 | 3800 | 1.56 | 2.83E-04 |
| KIAA0143 | 232356_at | AW003230 | NM_015137 | 23167 | 1.56 | 7.00E-03 |
| CPVL | 208146_s_at | NM_031311 | NM_019029 | 54504 | 1.56 | 4.48E-03 |
| | | | NM_031311 | | | |
| DMXL1 | 241472_at | N74444 | NM_005509 | 1657 | 1.55 | 8.84E-04 |
| SNX5 | 229981_at | AA131508 | NM_014426 | 27131 | 1.55 | 1.33E-05 |
| | | | NM_152227 | | | |
| ALCAM | 201951_at | BF242905 | NM_001627 | 214 | 1.55 | 2.09E-06 |
| ANKRD11 | 228866_at | BF514864 | NM_013275 | 29123 | 1.55 | 2.95E-04 |
| ID2 | 201565_s_at | NM_002166 | NM_002166 | 3398 | 1.55 | 9.49E-04 |
| PFAAP5 | 214753_at | AW084068 | NM_014887 | 10443 | 1.55 | 8.30E-04 |
| KIAA0318 | 238817_at | BF063412 | NM_015347 | 23504 | 1.55 | 8.89E-05 |
| SR140 | 236431_at | AI674977 | XM_031553 | 23350 | 1.54 | 8.08E-03 |
| API5 | 229692_at | AW135003 | NM_006595 | 8539 | 1.54 | 1.07E-03 |
| AA516469 | 236375_at | AA516469 | --- | --- | 1.54 | 6.79E-03 |
| NUPL1 | 241425_at | AA769986 | NM_00100856 4 | 9818 | 1.54 | 4.72E-05 |
| | | | NM_00100856 5 | | | |
| | | | NM_014089 | | | |
| AI791138 | 242968_at | AI791138 | --- | --- | 1.54 | 8.93E-03 |
| JAK1 | 234193_at | AK025218 | NM_002227 | 3716 | 1.54 | 3.33E-04 |
| AW052186 | 239942_at | AW052186 | --- | --- | 1.53 | 6.32E-03 |
| KIF23 | 244427_at | AW192521 | NM_004856 | 9493 | 1.53 | 7.98E-03 |
| | | | NM_138555 | | | |
| AI800470 | 242836_at | AI800470 | --- | --- | 1.53 | 5.90E-04 |
| BF509125 | 232198_at | BF509125 | --- | --- | 1.52 | 1.16E-04 |
| SPON1 | 209436_at | AB018305 | NM_006108 | 10418 | 1.52 | 2.06E-04 |
| DLEU2 | 1556820_a_at | H48516 | NM_006021 | 8847 | 1.52 | 1.57E-03 |
| AK025105 | 232476_at | AK025105 | --- | --- | 1.52 | 2.32E-03 |
| AA228366 | 236251_at | AA228366 | --- | --- | 1.52 | 4.86E-03 |
| H37807 | 241060_x_at | H37807 | --- | --- | 1.52 | 4.23E-03 |
| BNC2 | 238478_at | H97386 | NM_017637 | 54796 | 1.52 | 2.14E-05 |
| CADPS | 1568604_a_at | AI912173 | NM_003716 | 8618 | 1.51 | 7.84E-07 |
| | | | NM_183393 | | | |
| | | | NM_183394 | | | |
| SCARB2 | 215754_at | AU148040 | NM_005506 | 950 | 1.51 | 1.21E-03 |
| MBNL1 | 238558_at | AI445833 | NM_021038 | 4154 | 1.51 | 1.09E-03 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| BC003528 | 210920_x_at | BC003528 | --- | --- | 1.51 | 2.78E-03 |
| GPR18 | 233554_at | AF339764 | NM_005292 | 2841 | 1.51 | 8.61E-03 |
| UBE3B | 213822_s_at | BE856776 | NM_130466 | 89910 | 1.51 | 8.72E-03 |
| | | | NM_183414 | | | |
| | | | NM_183415 | | | |
| WWOX | 1556595_at | BQ025203 | NM_016373 | 51741 | 1.51 | 1.78E-03 |
| | | | NM_018560 | | | |
| | | | NM_130788 | | | |
| | | | NM_130790 | | | |
| | | | NM_130791 | | | |
| | | | NM_130792 | | | |
| | | | NM_130844 | | | |
| AI740589 | 243515_at | AI740589 | --- | --- | 1.51 | 4.92E-04 |
| N26620 | 227503_at | N26620 | --- | --- | 1.51 | 2.93E-03 |
| C18965 | 242853_at | C18965 | XM_379243 | 401106 | 1.51 | 5.39E-03 |
| CPNE4 | 239990_at | AI821426 | NM_130808 | 131034 | 1.50 | 1.95E-03 |
| FBXL11 | 215191_at | AW836210 | NM_012308 | 22992 | 1.50 | 3.06E-03 |
| CHD1L | 238070_at | AA573217 | NM_004284 | 9557 | 1.50 | 8.83E-03 |
| JARID2 | 232835_at | AA533080 | NM_004973 | 3720 | 1.50 | 3.50E-04 |
| MIB1 | 240446_at | AI798164 | NM_020774 | 57534 | 1.50 | 4.01E-04 |
| Z39232 | 1560924_at | Z39232 | --- | --- | 1.49 | 6.88E-04 |
| PLEKHE1 | 212719_at | AB011178 | NM_194449 | 23239 | 1.49 | 2.28E-04 |
| UBE1C | 1556339_a_at | BM353142 | NM_003968 | 9039 | 1.49 | 7.82E-04 |
| | | | NM_198195 | | | |
| | | | NM_198197 | | | |
| NM_030892 | 220969_s_at | NM_030892 | --- | --- | 1.49 | 4.83E-04 |
| TBX3 | 219682_s_at | NM_016569 | NM_005996 | 6926 | 1.48 | 2.65E-03 |
| | | | NM_016569 | | | |
| PHGDHL1 | 241932_at | AI073803 | NM_177967 | 337867 | 1.48 | 5.92E-03 |
| LOC158301 | 239193_at | BF060981 | --- | 158301 | 1.48 | 1.36E-03 |
| FLJ10579 | 230843_at | AA482037 | NM_018145 | 55177 | 1.48 | 6.04E-04 |
| RPL10A | 234224_at | AL137541 | NM_007104 | 4736 | 1.48 | 5.43E-03 |
| C10orf119 | 242167_at | AA703310 | NM_024834 | 79892 | 1.48 | 9.08E-06 |
| CORO6 | 1552301_a_at | NM_032854 | NM_032854 | 84940 | 1.48 | 5.48E-03 |
| MGC15912 | 239635_at | BF510708 | NM_032886 | 84972 | 1.47 | 4.46E-03 |
| DTNB | 233727_at | AL157472 | NM_021907 | 1838 | 1.47 | 4.45E-03 |
| | | | NM_033147 | | | |
| | | | NM_033148 | | | |
| | | | NM_183360 | | | |
| | | | NM_183361 | | | |
| FLJ22955 | 232447_at | AU147405 | NM_024819 | 79877 | 1.46 | 3.56E-03 |
| LOC151194 | 236607_at | AI921882 | NM_145280 | 151194 | 1.46 | 8.35E-04 |
| BC035170 | 1561079_at | BC035170 | --- | --- | 1.46 | 5.42E-03 |
| XPO7 | 240168_at | AA679589 | NM_015024 | 23039 | 1.46 | 3.12E-03 |
| EZH2 | 215006_at | AK023816 | NM_004456 | 2146 | 1.46 | 2.64E-03 |
| | | | NM_152998 | | | |
| LTB4DH | 228825_at | BE566894 | NM_012212 | 22949 | 1.46 | 1.02E-03 |
| BBX | 1557240_a_at | BU689085 | NM_020235 | 56987 | 1.46 | 5.33E-03 |
| ARIH2 | 1559121_s_at | AI767566 | NM_006321 | 10425 | 1.46 | 8.11E-04 |
| ANKRD28 | 229307_at | N32051 | NM_015199 | 23243 | 1.45 | 2.51E-03 |
| LRRFIP2 | 232704_s_at | AK025207 | NM_006309 | 9209 | 1.45 | 2.05E-03 |
| | | | NM_017724 | | | |
| PHLDA1 | 218000_s_at | NM_007350 | NM_007350 | 22822 | 1.45 | 5.61E-03 |
| AF070602 | 216214_at | AF070602 | --- | --- | 1.44 | 3.75E-03 |
| C6orf133 | 215558_at | AK001118 | NM_015255 | 23304 | 1.44 | 1.76E-04 |
| AW083060 | 243274_x_at | AW083060 | --- | --- | 1.44 | 6.61E-03 |
| ATOH8 | 228890_at | BF434995 | NM_032827 | 84913 | 1.43 | 9.99E-03 |
| CHC1L | 230292_at | AA868809 | NM_001268 | 1102 | 1.43 | 2.15E-03 |
| SMG1 | 244766_at | BG180003 | NM_014006 | 23049 | 1.43 | 1.93E-03 |
| KIAA0220 | | | NM_015092 | 283846 | | |
| LOC440345 | | | XM_496125 | 440345 | | |
| T98846 | 239654_at | T98846 | --- | --- | 1.43 | 7.51E-05 |
| ZA20D3 | 222186_at | AL109684 | NM_019006 | 54469 | 1.43 | 2.35E-03 |
| SMC5L1 | 1562948_at | BC035661 | NM_015110 | 23137 | 1.43 | 7.05E-03 |
| SMC4L1 | 237246_at | AI797163 | NM_00100279 9 | 10051 | 1.43 | 7.94E-03 |
| | | | NM_00100280 0 | | | |
| | | | NM_005496 | | | |
| SLD5 | 1554356_at | BC027454 | NM_032336 | 84296 | 1.43 | 1.24E-03 |
| AW451426 | 244480_at | AW451426 | --- | --- | 1.42 | 1.53E-04 |
| ZSWIM6 | 1560199_x_at | AU120130 | XM_035299 | 57688 | 1.42 | 2.96E-04 |
| H06497 | 243211_at | H06497 | --- | --- | 1.42 | 1.88E-03 |
| ENC1 | 201340_s_at | AF010314 | NM_003633 | 8507 | 1.42 | 8.83E-03 |
| AW963544 | 238580_at | AW963544 | --- | --- | 1.42 | 1.14E-04 |
| IGSF4 | 232793_at | AL359567 | NM_014333 | 23705 | 1.42 | 6.90E-04 |
| AK022100 | 233406_at | AK022100 | --- | --- | 1.41 | 8.65E-03 |
| CENTB2 | 240759_at | AW593931 | NM_012287 | 23527 | 1.41 | 8.75E-04 |
| PTPRK | 203038_at | NM_002844 | NM_002844 | 5796 | 1.41 | 2.80E-04 |
| LOC286109 | 243304_at | AI733824 | --- | 286109 | 1.41 | 8.07E-03 |
| ASCL1 | 213768_s_at | AW950513 | NM_004316 | 429 | 1.40 | 2.65E-05 |
| AW293174 | 244290_at | AW293174 | XM_499534 | 442612 | 1.40 | 2.89E-05 |
| MGC15937 | 227794_at | BF432254 | NM_080661 | 92292 | 1.40 | 2.78E-03 |
| PTK2 | 1559529_at | BC043202 | NM_005607 | 5747 | 1.40 | 3.63E-03 |
| | | | NM_153831 | | | |
| MAN2A1 | 205105_at | NM_002372 | NM_002372 | 4124 | 1.39 | 3.32E-04 |
| CBLB | 233614_at | AU145361 | NM_170662 | 868 | 1.39 | 9.21E-04 |
| KIAA0998 | 215898_at | AK021879 | NM_015072 | 23093 | 1.39 | 5.01E-03 |
| PMAIP1 | 204285_s_at | AI857639 | NM_021127 | 5366 | 1.39 | 3.88E-06 |
| FNBP1L | 242310_at | AA665058 | NM_017737 | 54874 | 1.38 | 4.27E-03 |
| AI755057 | 233055_at | AI755057 | --- | --- | 1.38 | 1.05E-03 |
| RAB3-GAP150 | 240234_at | AI216539 | NM_012414 | 25782 | 1.38 | 7.73E-03 |
| ACSL4 | 1557418_at | W95007 | NM_004458 | 2182 | 1.38 | 5.61E-04 |
| | | | NM_022977 | | | |
| FAM13A1 | 232628_at | AI740629 | NM_014883 | 10144 | 1.37 | 4.61E-03 |
| FLJ23451 | 1569601_at | BF106979 | NM_024766 | 79823 | 1.37 | 4.24E-05 |
| ZNF217 | 203739_at | NM_006526 | NM_006526 | 7764 | 1.37 | 5.73E-03 |
| BF197705 | 230401_at | BF197705 | --- | --- | 1.37 | 9.92E-04 |
| HEG | 212822_at | AA121502 | XM_087386 | 57493 | 1.37 | 2.43E-04 |
| MGC15523 | 1563226_at | BC020925 | NM_138570 | 124565 | 1.37 | 7.40E-03 |
| AA156721 | 201952_at | AA156721 | --- | --- | 1.37 | 1.55E-04 |
| AL110237 | 232959_at | AL110237 | --- | --- | 1.37 | 7.09E-03 |
| PSME4 | 237180_at | T97717 | NM_014614 | 23198 | 1.37 | 1.78E-03 |
| IGSF4 | 244345_at | AI627453 | NM_014333 | 23705 | 1.36 | 7.17E-03 |
| MRPS25 | 244019_at | T89845 | NM_022497 | 64432 | 1.36 | 2.01E-03 |
| BF801735 | 238712_at | BF801735 | --- | --- | 1.36 | 2.63E-03 |
| SPRY4 | 221489_s_at | W48843 | NM_030964 | 81848 | 1.35 | 7.35E-04 |
| SCN7A | 239984_at | AI333640 | NM_002976 | 6332 | 1.35 | 8.33E-04 |
| HIVEP2 | 243254_at | T08739 | NM_006734 | 3097 | 1.35 | 5.09E-03 |
| PTPN11 | 241930_x_at | AA223204 | NM_002834 | 5781 | 1.35 | 9.28E-04 |
| AW235215 | 238913_at | AW235215 | --- | --- | 1.35 | 9.92E-03 |
| C10orf6 | 1553909_x_at | NM_144592 | NM_018121 | 55719 | 1.35 | 2.80E-04 |
| ZFHX4 | 219779_at | NM_024721 | NM_024721 | 79776 | 1.35 | 1.82E-05 |
| MEST | 202016_at | NM_002402 | NM_002402 | 4232 | 1.34 | 2.65E-04 |
| | | | NM_177524 | | | |
| | | | NM_177525 | | | |
| TRIO | 231403_at | N21108 | NM_007118 | 7204 | 1.34 | 9.69E-03 |
| UBE3C | 242673_at | AA931284 | NM_014671 | 9690 | 1.34 | 3.34E-04 |
| PPP2R2A | 236492_at | AI934447 | NM_002717 | 5520 | 1.34 | 4.98E-03 |
| HIAT1 | 230868_at | BF433103 | NM_033055 | 64645 | 1.34 | 7.24E-04 |
| LOC440971 | 1556744_a_at | AI732587 | XM_496651 | 440971 | 1.33 | 3.92E-03 |
| CUGBP2 | 202156_s_at | N36839 | NM_006561 | 10659 | 1.33 | 2.42E-03 |
| CNTN4 | 229084_at | R42166 | NM_175607 | 152330 | 1.33 | 8.25E-03 |
| | | | NM-175612 | | | |
| | | | NM_175613 | | | |
| FLJ11259 | 218627_at | NM_018370 | NM_018370 | 55332 | 1.32 | 4.63E-03 |
| CEPT1 | 1561884_at | AL833102 | NM_00100779 4 | 10390 | 1.32 | 1.40E-04 |
| | | | NM_006090 | | | |
| AL049988 | 216614_at | AL049988 | --- | --- | 1.31 | 9.72E-03 |
| CSPG6 | 1556925_at | AL360194 | NM_005445 | 9126 | 1.31 | 8.99E-03 |
| ASXL1 | 242439_s_at | AI819150 | NM_015338 | 171023 | 1.31 | 1.22E-05 |
| PVT1 | 1558290_a_at | BG200951 | XM_372058 | 441378 | 1.31 | 1.11E-04 |
| LOC441378 | | | XM_499128 | 5820 | | |
| TUBB6 | 209191_at | BC002654 | NM_032525 | 84617 | 1.31 | 5.17E-04 |
| IRAK1 BP1 | 233290_at | AU145280 | NM_001010844 | 134728 | 1.31 | 2.71E-03 |
| AL137730 | 226999_at | AL137730 | --- | --- | 1.31 | 6.07E-03 |
| PTEN | 233314_at | AK021487 | NM_000314 | 5728 | 1.31 | 7.60E-03 |
| ZNF215 | 220214_at | NM_013250 | NM_013250 | 7762 | 1.31 | 5.81E-03 |
| LRPPRC | 1557360_at | CA430402 | NM_133259 | 10128 | 1.30 | 1.55E-03 |
| DKFZP434C212 | 231552_at | AW451785 | NM_015635 | 26130 | 1.30 | 5.13E-04 |
| AI651786 | 237945_at | AI651786 | --- | --- | 1.30 | 4.28E-03 |
| AU155234 | 233289_at | AU155234 | --- | --- | 1.30 | 9.43E-03 |
| AA648983 | 240478_at | AA648983 | --- | --- | 1.30 | 9.77E-05 |
| AA412686 | 244608_at | AA412686 | --- | --- | 1.30 | 4.76E-03 |
| RBMS3 | 240245_at | AW237264 | NM_00100379 2 | 27303 | 1.30 | 6.53E-03 |
| | | | NM_00100379 3 | | | |
| | | | NM_014483 | | | |
| AK021457 | 216682_s_at | AK021457 | --- | --- | 1.30 | 2.30E-04 |
| SHPRH | 243964_at | AI631201 | NM_173082 | 257218 | 1.29 | 9.11E-03 |
| METAP2 | 244185_at | AA921841 | NM_006838 | 10988 | 1.29 | 5.27E-05 |
| MAB21L2 | 210302_s_at | AF262032 | NM_006439 | 10586 | 1.29 | 1.50E-04 |
| AA543084 | 236480_at | AA543084 | --- | --- | 1.29 | 3.14E-04 |
| AW271626 | 237441_at | AW271626 | --- | --- | 1.29 | 2.95E-05 |
| SF3B14 | 1561923_a_at | AF147425 | NM_016047 | 51639 | 1.29 | 7.66E-03 |
| PRDX6 | 242751_at | N55072 | NM_004905 | 9588 | 1.28 | 1.80E-03 |
| SMU1 | 228180_at | AA805653 | NM_018225 | 55234 | 1.28 | 6.63E-04 |
| BF001773 | 241906_at | BF001773 | --- | --- | 1.28 | 9.10E-03 |
| PFAAP5 | 221899_at | AI809961 | NM_014887 | 10443 | 1.28 | 8.55E-03 |
| FLJ13273 | 220063_at | NM_024751 | NM_024751 | 79807 | 1.28 | 4.70E-04 |
| HM13 | 1560297_at | BG928538 | NM_030789 | 81502 | 1.28 | 3.78E-03 |
| | | | NM_178580 | | | |
| | | | NM_178581 | | | |
| | | | NM_178582 | | | |
| DRF1 | 206661_at | NM_025104 | NM_025104 | 80174 | 1.28 | 5.80E-04 |
| | | | NM_145663 | | | |
| LOC284219 | 1556425_a_at | BF509747 | --- | 284219 | 1.27 | 2.33E-03 |
| ANXA2 | 213503_x_at | BE908217 | NM_00100285 7 | 302 | 1.27 | 2.58E-03 |
| | | | NM_00100285 8 | | | |
| | | | NM_004039 | | | |
| SPON1 | 213994_s_at | AI885290 | NM_006108 | 10418 | 1.27 | 2.99E-03 |
| M11S1 | 244778_x_at | N63691 | NM_005898 | 4076 | 1.27 | 1.16E-03 |
| | | | NM_203364 | | | |
| LOC253981 | 226430_at | AI394438 | --- | 253981 | 1.26 | 6.58E-04 |
| FUBP1 | 240307_at | N54783 | NM_003902 | 8880 | 1.26 | 5.57E-04 |
| NKTR | 231235_at | AA732581 | NM_005385 | 4820 | 1.26 | 6.07E-03 |
| ARHGAP24 | 223422_s_at | AI743534 | NM_031305 | 83478 | 1.26 | 2.57E-03 |
| KIAA1838 | 241152_at | BE466903 | NM_032448 | 84498 | 1.26 | 4.94E-03 |
| USP34 | 207365_x_at | NM_014709 | NM_014709 | 9736 | 1.25 | 1.35E-04 |
| DHX57 | 239311_at | AI367034 | NM_144995 | 90957 | 1.25 | 7.92E-03 |
| | | | NM_145646 | | | |
| | | | NM_198963 | | | |
| UBE2H | 239721_at | AI280328 | NM_003344 | 7328 | 1.25 | 7.22E-03 |
| | | | NM_182697 | | | |
| ID1 | 208937_s_at | D13889 | NM_002165 NM_181353 | 3397 | 1.25 | 1.45E-03 |
| MARK3 | 239166_at | R98192 | NM_002376 | 4140 | 1.24 | 8.08E-04 |
| APBB2 | 213419_at | U62325 | NM_173075 | 323 | 1.24 | 5.44E-04 |
| SVH | 1558748_at | AL832759 | NM_031905 | 83787 | 1.24 | 2.00E-03 |
| ASXL1 | 242438_at | AI819150 | NM_015338 | 171023 | 1.24 | 6.76E-05 |
| P15RS | 228566_at | AA778694 | NM_018170 | 55197 | 1.24 | 2.39E-04 |
| LOC115648 | 1558486_at | BC022394 | NM_145326 | 115648 | 1.24 | 3.07E-04 |
| CADPS | 1568603_at | AI912173 | NM_003716 | 8618 | 1.24 | 3.75E-03 |
| | | | NM_183393 | | | |
| | | | NM_183394 | | | |
| AI291804 | 242856_at | AI291804 | --- | --- | 1.24 | 8.05E-05 |
| TCTEL1 | 242109_at | A1038577 | NM_006519 | 6993 | 1.23 | 3.41E-03 |
| QKI | 241938_at | AA935633 | NM_006775 | 9444 | 1.23 | 1.41E-04 |
| | | | NM_206853 | | | |
| | | | NM_206854 | | | |
| | | | NM_206855 | | | |
| SCARA3 | 223842_s_at | AB007830 | NM_016240 | 51435 | 1.22 | 3.50E-04 |
| | | | NM_182826 | | | |
| MRPS5 | 236779_at | BG539414 | NM_031902 | 64969 | 1.22 | 1.33E-03 |
| BE670307 | 227955_s_at | BE670307 | --- | --- | 1.22 | 9.05E-06 |
| DNAPTP6 | 241812_at | AV648669 | NM_015535 | 26010 | 1.22 | 8.50E-03 |
| PCGF5 | 229194_at | AL045882 | NM_032373 | 84333 | 1.22 | 2.28E-04 |
| AU145679 | 233607_at | AU145679 | --- | --- | 1.21 | 4.54E-03 |
| BE464799 | 214036_at | BE464799 | --- | --- | 1.21 | 5.03E-05 |
| SLCO3A1 | 219229_at | NM_013272 | NM_013272 | 28232 | 1.21 | 2.66E-03 |
| BF433200 | 242467_at | BF433200 | --- | --- | 1.21 | 7.14E-03 |
| PPARG | 232331_at | AK027107 | NM_005037 | 5468 | 1.21 | 6.85E-03 |
| | | | NM_015869 | | | |
| | | | NM-138711 | | | |
| | | | NM_138712 | | | |
| FNTA | 240527 _at | AI953011 | NM_002027 | 2339 | 1.21 | 1.22E-03 |
| TCF7L2 | 216035_x_at | AV721430 | NM_030756 | 6934 | 1.21 | 2.15E-05 |
| POLE | 216026_s_at | AL080203 | NM_006231 | 5426 | 1.21 | 2.59E-03 |
| ZA20D3 | 238812_at | AA741296 | NM_019006 | 54469 | 1.21 | 9.92E-03 |
| SFRS2IP | 232597_x_at | AK025132 | NM_004719 | 9169 | 1.21 | 1.99E-03 |
| TH | 208291_s_at | NM_000360 | NM_000360 | 7054 | 1.21 | 8.60E-04 |
| | | | NM_199292 | | | |
| | | | NM_199293 | | | |
| NKTR | 202380_s_at | NM_005385 | NM_005385 | 4820 | 1.21 | 1.31E-03 |
| PTPN13 | 243792_x_at | AI281371 | NM_006264 | 5783 | 1.20 | 9.38E-03 |
| | | | NM_080683 | | | |
| | | | NM_080684 | | | |
| | | | NM_080685 | | | |
| UBE3B | 232301_at | AK022019 | NM_130466 | 89910 | 1.20 | 1.77E-04 |
| | | | NM_183414 | | | |
| | | | NM_183415 | | | |
| RAB12 | 238714_at | AA504249 | XM_113967 | 201475 | 1.20 | 7.92E-04 |
| AW299775 | 235008_at | AW299775 | --- | --- | 1.20 | 1.71E-03 |
| HSD17B12 | 242616_at | W80359 | NM_016142 | 51144 | 1.19 | 8.50E-03 |
| R16900 | 242514_at | R16900 | --- | --- | 1.19 | 2.39E-03 |
| DST | 232098_at | AK025142 | NM_001723 | 667 | 1.19 | 4.65E-03 |
| | | | NM_015548 | | | |
| | | | NM_020388 | | | |
| | | | NM_183380 | | | |
| DKFZP564C186 | 1559139_at | BC009786 | NM_015658 | 26155 | 1.19 | 3.03E-03 |
| C9orf3 | 233599_at | AK025151 | NM_032823 | 84909 | 1.19 | 1.52E-03 |
| AI859990 | 244197_x_at | AI859990 | --- | --- | 1.18 | 1.42E-03 |
| SPON1 | 213993_at | AI885290 | NM_006108 | 10418 | 1.18 | 8.98E-03 |
| CENPF | 207331_at | NM_016343 | NM_016343 | 1063 | 1.18 | 6.92E-03 |
| CDC2L5 | 232266_x_at | AK024379 | NM_003718 | 8621 | 1.18 | 2.93E-03 |
| | | | NM_031267 | | | |
| EXT1 | 237310_at | AI743607 | NM_000127 | 2131 | 1.18 | 4.88E-03 |
| C1QDC1 | 218456_at | NM_023925 | NM_00100225 9 | 65981 | 1.18 | 2.52E-06 |
| | | | NM_023925 | | | |
| | | | NM_032156 | | | |
| FLJ45645 | 243038_at | AW292769 | NM_198557 | 375287 | 1.18 | 6.22E-03 |
| H43040 | 231108_at | H43040 | --- | --- | 1.18 | 2.07E-04 |
| SEC63 | 242749_at | AI022173 | NM_007214 | 11231 | 1.18 | 3.85E-03 |
| KLF7 | 238482_at | AL120562 | NM_003709 | 8609 | 1.17 | 4.23E-04 |
| API5 | 214960_at | AF229253 | NM_006595 | 8539 | 1.17 | 1.28E-04 |
| ENC1 | 201341_at | NM_003633 | NM_003633 | 8507 | 1.17 | 9.25E-04 |
| SLC16A4 | 228455_at | AI092824 | NM_004696 | 9122 | 1.17 | 3.54E-03 |
| ANP32A | 235954_at | AA972597 | NM_006305 | 8125 | 1.17 | 4.73E-03 |
| UBE2E1 | 236062_at | AI742722 | NM_003341 | 7324 | 1.17 | 2.49E-03 |
| | | | NM_182666 | | | |
| EVL | 227232_at | T58044 | NM_016337 | 51466 | 1.17 | 1.12E-03 |
| SLC6A2 | 210353_s_at | M65105 | NM_001043 | 6530 | 1.17 | 3.19E-03 |
| Cep63 | 238170_at | BE327727 | NM_025180 | 80254 | 1.17 | 6.06E-05 |
| AU147479 | 233359_at | AU147479 | --- | --- | 1.16 | 1.44E-03 |
| AF010144 | 207953_at | AF010144 | --- | --- | 1.16 | 6.11E-05 |
| TLE3 | 228340_at | BE967118 | NM_005078 | 7090 | 1.16 | 2.16E-03 |
| LOC338692 | 238642_at | AW367571 | NM_207354 | 338692 | 1.16 | 6.72E-03 |
| BF981643 | 214720_x_at | BF981643 | NM_144710 | 151011 | 1.16 | 3.74E-03 |
| | | | NM_178584 | | | |
| AU152763 | 232939_at | AU152763 | --- | --- | 1.16 | 1.12E-03 |
| COMMD10 | 1560349_at | BC036897 | NM_016144 | 51397 | 1.16 | 7.89E-03 |
| MDN1 | 1569484_s_at | AL603301 | NM_014611 | 23195 | 1.16 | 1.37E-03 |
| FLJ10213 | 219906_at | NM_018029 | NM_018029 | 55096 | 1.16 | 2.68E-04 |
| AF5Q31 | 1555436_a_at | BC025700 | NM_014423 | 27125 | 1.16 | 4.10E-04 |
| BE467787 | 243835_at | BE467787 | --- | --- | 1.16 | 3.16E-03 |
| NOSTRIN | 235506_at | AI076788 | NM_052946 | 115677 | 1.16 | 5.52E-03 |
| WWOX | 242099_at | T83938 | NM_016373 | 51741 | 1.16 | 1.72E-03 |
| | | | NM_018560 | | | |
| | | | NM_130788 | | | |
| | | | NM_130790 | | | |
| | | | NM_130791 | | | |
| | | | NM_130792 | | | |
| | | | NM_130844 | | | |
| NKTR | 202379_s_at | AI361805 | NM_005385 | 4820 | 1.16 | 2.17E-03 |
| TXNRD1 | 201266_at | NM_003330 | NM_003330 | 7296 | 1.15 | 1.40E-04 |
| | | | NM_182729 | | | |
| | | | NM_182742 | | | |
| | | | NM_182743 | | | |
| STRA6 | 1569334_at | BC015881 | NM_022369 | 64220 | 1.15 | 2.00E-03 |
| DLEU2 | 215629_s_at | AA905286 | NM_006021 | 8847 | 1.15 | 8.95E-03 |
| MSH3 | 233263_at | AU147635 | NM_002439 | 4437 | 1.15 | 3.30E-04 |
| YAP | 244803_at | AI335191 | NM_018253 | 55249 | 1.14 | 3.17E-03 |
| | | | NM_139118 | | | |
| | | | NM_139119 | | | |
| | | | NM_139120 | | | |
| | | | NM_139121 | | | |
| ANAPC5 | 235926_at | AI312527 | NM_016237 | 51433 | 1.14 | 3.93E-03 |
| LOC152573 | 229485_x_at | AI735586 | XM_496688 | 152573 | 1.14 | 9.91E-03 |
| VPS16 | 233313_at | AU158316 | NM_022575 | 64601 | 1.14 | 1.33E-03 |
| | | | NM_080413 | | | |
| | | | NM_080414 | | | |
| TNRC6 | 233836_at | AK025277 | NM_014494 | 27327 | 1.14 | 4.69E-03 |
| | | | NM_020847 | | | |
| PCGF5 | 227935_s_at | AA522681 | NM_032373 | 84333 | 1.13 | 1.23E-03 |
| LOC255326 | 233271_at | AU145563 | XM_497224 | 255326 | 1.13 | 9.42E-03 |
| KIAA1881 | 207730_x_at | NM_017932 | XM_375558 | 114782 | 1.13 | 8.71E-04 |
| NAPE-PLD | 233539_at | AK000801 | NM_198990 | 222236 | 1.13 | 6.74E-03 |
| PGAP1 | 220576_at | NM_024989 | NM_024989 | 80055 | 1.13 | 4.35E-04 |
| FLJ10707 | 239957 _at | AW510793 | XM_371614 | 55209 | 1.13 | 3.10E-03 |
| SIVA | 222030_at | AW024335 | NM_006427 | 10572 | 1.13 | 8.59E-03 |
| | | | NM_021709 | | | |
| MGC34032 | 1552782_at | AK091400 | NM_152697 | 204962 | 1.13 | 6.36E-03 |
| TP53I3 | 210609_s_at | BC000474 | NM_004881 | 9540 | 1.13 | 6.61E-03 |
| | | | NM_147184 | | | |
| SYT11 | 232677_at | AU146128 | NM_152280 | 23208 | 1.13 | 3.78E-05 |
| ZAK | 1555259_at | AF465843 | NM_016653 | 51776 | 1.13 | 4.30E-04 |
| | | | NM_133646 | | | |
| MTBP | 233436_at | AK022122 | NM_022045 | 27085 | 1.12 | 2.65E-03 |
| SDHA | 230077_at | W90764 | NM_004168 | 255812 | 1.12 | 7.47E-04 |
| SDHAL2 | | | XM_171032 | 6389 | | |
| EIF4A1 | 214805_at | U79273 | NM_001416 | 1973 | 1.12 | 7.09E-03 |
| ZNF131 | 1557384_at | AL832081 | --- | 7690 | 1.12 | 3.31E-03 |
| TJP1 | 244476_at | R39769 | NM_003257 | 7082 | 1.12 | 1.55E-03 |
| | | | NM_175610 | | | |
| NM_000575 | 208200_at | NM_000575 | --- | --- | 1.12 | 9.29E-03 |
| SFI1 | 36545_s_at | AB011114 | NM_00100746 7 | 9814 | 1.12 | 1.54E-03 |
| | | | NM_014775 | | | |
| GALNT7 | 222587_s_at | BF699855 | NM_017423 | 51809 | 1.12 | 1.21E-03 |
| AA176313 | 1566113_at | AA176313 | --- | --- | 1.12 | 7.32E-03 |
| RABGAP1L | 1565762_at | AK074233 | NM_014857 | 9910 | 1.12 | 4.63E-03 |
| MED6 | 207078_at | NM_005466 | NM_005466 | 10001 | 1.12 | 8.69E-03 |
| NDUFS1 | 1559691_at | BC032767 | NM_005006 | 4719 | 1.11 | 7.49E-03 |
| SYNCRIP | 1555427_s_at | AY034482 | NM_006372 | 10492 | 1.11 | 3.11E-03 |
| TH1L | 220607_x_at | NM_016397 | NM_016397 | 51497 | 1.11 | 1.11E-03 |
| | | | NM_198976 | | | |
| SMURF2 | 230099_at | AI139993 | NM_022739 | 64750 | 1.11 | 9.53E-03 |
| KHDRBS3 | 209781_s_at | AF069681 | NM_006558 | 10656 | 1.11 | 6.19E-04 |
| LOC90799 | 223815_at | AF130069 | NM_138363 | 90799 | 1.10 | 9.64E-03 |
| ASCL1 | 209988_s_at | BC001638 | NM_004316 | 429 | 1.10 | 6.04E-04 |
| AA192765 | 238172_at | AA192765 | --- | --- | 1.10 | 7.54E-04 |
| C7orf28B | 215024_at | AK000993 | NM_198097 | 221960 | 1.10 | 8.32E-03 |
| SURF5 | 206593_s_at | NM_006752 | NM_006752 | 6837 | 1.10 | 8.66E-03 |
| | | | NM_13364 | | | |
| | | | NM_181491 | | | |
| SNTB2 | 232784_at | R42604 | NM_006750 | 6645 | 1.10 | 1.54E-05 |
| | | | NM_130845 | | | |
| TD-60 | 243297_at | AI498610 | NM_018715 | 55920 | 1.10 | 6.95E-03 |
| HES1 | 203394_s_at | BE973687 | NM_005524 | 3280 | 1.10 | 9.57E-05 |
| CAS1 | 239545_at | AI335004 | NM_022900 | 64921 | 1.10 | 4.20E-03 |
| PSMB7 | 244801_at | AI248671 | NM_002799 | 5695 | 1.10 | 1.35E-04 |
| MBNL2 | 232138_at | AW276914 | NM_144778 | 10150 | 1.10 | 6.31E-04 |
| | | | NM_207304 | | | |
| hSyn | 231087_at | AL046412 | NM_018157 | 55188 | 1.09 | 8.23E-03 |
| LRRFIP1 | 238840_at | AW082668 | NM_004735 | 9208 | 1.09 | 3.75E-03 |
| SLC7A5 | 201195_s_at | AB018009 | NM_003486 | 8140 | 1.09 | 1.06E-04 |
| RDH11 | 217042_at | AL096716 | NM_016026 | 51109 | 1.09 | 1.95E-03 |
| LUC7L2 | 243852_at | AI963460 | NM_016019 | 51631 | 1.09 | 4.04E-03 |
| SEC8L1 | 216538_at | AL049351 | NM_021807 | 60412 | 1.09 | 2.92E-04 |
| NM_006896 | 206848_at | NM_006896 | --- | --- | 1.09 | 2.52E-04 |
| PSMD7 | 244515_at | AI640348 | NM_002811 | 5713 | 1.08 | 1.31E-05 |
| SYMPK | 1554595_at | BC030214 | NM_004819 | 8189 | 1.08 | 6.40E-03 |
| ELOVL5 | 1566079_at | AL833001 | NM_021814 | 60481 | 1.08 | 7.71E-03 |
| FLJ10357 | 241627_x_at | AI640434 | NM_018071 | 55701 | 1.08 | 1.28E-03 |
| PASK | 213534_s_at | D50925 | NM_015148 | 23178 | 1.08 | 6.82E-03 |
| CALM2 | 235190_at | BF591288 | NM_001743 | 805 | 1.08 | 1.73E-04 |
| SEMA5A | 205405_at | NM_003966 | NM_003966 | 9037 | 1.08 | 7.59E-05 |
| ITSN1 | 215791_at | AF003738 | NM_00100113 2 | 6453 | 1.08 | 1.59E-03 |
| | | | NM_003024 | | | |
| PBX4 | 230536_at | AJ300182 | NM_025245 | 80714 | 1.07 | 9.77E-04 |
| MAN2A1 | 226538_at | AV700323 | NM_002372 | 4124 | 1.07 | 2.26E-04 |
| MGC2803 | 230213_at | BE220399 | NM_024038 | 79002 | 1.07 | 9.35E-05 |
| ABCD1 | 205142_x_at | NM_000033 | NM_000033 | 215 | 1.07 | 8.99E-03 |
| ZNF537 | 223392_s_at | BF510588 | NM_020856 | 57616 | 1.07 | 1.32E-04 |
| AI733583 | 243235_at | AI733583 | --- | --- | 1.07 | 3.45E-03 |
| CROP | 242389_at | BE887449 | NM_006107 | 51747 | 1.07 | 1.26E-03 |
| | | | NM_016424 | | | |
| SSH2 | 230590_at | BE675486 | NM_033389 | 85464 | 1.07 | 5.70E-04 |
| NDUFS8 | 232169_x_at | AK002110 | NM_002496 | 4728 | 1.06 | 6.64E-04 |
| VAPA | 242780_at | AI332536 | NM_003574 | 9218 | 1.06 | 7.88E-03 |
| | | | NM_194434 | | | |
| FDFT1 | 238666_at | BF438300 | NM_004462 | 2222 | 1.06 | 1.67E-03 |
| NQO1 | 210519_s_at | BC000906 | NM_000903 | 1728 | 1.06 | 4.23E-03 |
| KLHL5 | 233866_at | AK001836 | NM_00100707 5 | 51088 | 1.06 | 5.76E-03 |
| | | | NM_199039 | | | |
| SLC8A3 | 152403_a_at | AK096553 | NM_033262 | 6547 | 1.06 | 2.76E-05 |
| | | | NM_058240 | | | |
| | | | NM_182932 | | | |
| | | | NM_182933 | | | |
| | | | NM_182936 | | | |
| | | | NM_183002 | | | |
| DPF1 | 206531_at | NM_004647 | NM_004647 | 8193 | 1.06 | 4.01E-03 |
| MCCC2 | 1556345_s_at | AF086534 | NM_022132 | 64087 | 1.06 | 2.60E-03 |
| NOS1 | 239132_at | BE299830 | NM_000620 | 4842 | 1.05 | 1.68E-03 |
| DOK4 | 209691_s_at | BC003541 | NM_018110 | 55715 | 1.05 | 9.53E-03 |
| SESN2 | 223195_s_at | BF131886 | NM_031459 | 83667 | 1.05 | 7.34E-03 |
| AK025909 | 233814_at | AK025909 | --- | --- | 1.05 | 2.17E-06 |
| FBN1 | 202766_s_at | NM_000138 | NM_000138 | 2200 | 1.05 | 4.10E-03 |
| UCHL1 | 201387_s_at | NM_004181 | NM_004181 | 7345 | 1.05 | 2.84E-03 |
| DIAPH1 | 209190_s_at | AF051782 | NM_005219 | 1729 | 1.05 | 3.26E-04 |
| KIAA0152 | 200616_s_at | BC000371 | NM_014730 | 9761 | 1.04 | 2.72E-06 |
| GAP43 | 204471_at | NM_002045 | NM_002045 | 2596 | 1.04 | 4.27E-04 |
| KIAA0528 | 240205_x_at | AW188170 | NM_014802 | 9847 | 1.04 | 5.35E-03 |
| AK025343 | 216176_at | AK025343 | --- | --- | 1.04 | 4.13E-04 |
| WDR45 | 215553_x_at | AK024315 | NM_007075 | 11152 | 1.04 | 3.37E-03 |
| NLN | 234762_x_at | AK026655 | NM_020726 | 57486 | 1.03 | 6.21E-03 |
| AI589594 | 238694_at | AI589594 | --- | --- | 1.03 | 1.36E-04 |
| CTSL2 | 210074_at | AF070448 | NM_001333 | 1515 | 1.03 | 7.07E-03 |
| COBLL1 | 203642_s_at | NM_014900 | NM_014900 | 22837 | 1.03 | 7.47E-03 |
| MGC15407 | 235662_at | AI125867 | NM_080667 | 112942 | 1.03 | 8.10E-03 |
| TRNT1 | 223814_at | BC005184 | NM_016000 | 51095 | 1.03 | 8.10E-04 |
| | | | NM_182916 | | | |
| AI377043 | 230722_at | AI377043 | --- | --- | 1.03 | 2.26E-03 |
| MGC45866 | 232475_at | BC002881 | NM_152259 | 90381 | 1.03 | 4.71E-03 |
| KIAA2024 | 1566166_at | AL833423 | NM_172070 | 130507 | 1.03 | 3.17E-04 |
| SRGAP2 | 1556203_a_at | AI263819 | XM_059095 | 23380 | 1.03 | 1.13E-03 |
| CHTF18 | 226569_s_at | AK024476 | NM_022092 | 63922 | 1.02 | 1.51E-03 |
| TCF7L2 | 216511_s_at | AJ270770 | NM_030756 | 6934 | 1.02 | 1.84E-07 |
| SDFR1 | 228723_at | AL360198 | NM_012428 | 27020 | 1.02 | 2.35E-04 |
| | | | NM_017455 | | | |
| CDCA7 | 230060_at | AI277642 | NM_031942 | 83879 | 1.02 | 6.93E-03 |
| | | | NM_145810 | | | |
| CIAPIN1 | 230838_s_at | AW771492 | NM_020313 | 57019 | 1.02 | 6.62E-03 |
| SDC4 | 202071_at | NM_002999 | NM_002999 | 6385 | 1.02 | 4.53E-03 |
| STK36 | 234005_x_at | AK022692 | NM_015690 | 27148 | 1.02 | 9.55E-05 |
| DLGAP4 | 233056_x_at | AK024674 | NM_014902 | 22839 | 1.02 | 5.12E-03 |
| | | | NM_183006 | | | |
| CNOT7 | 233019_at | AU145061 | NM_013354 | 29883 | 1.02 | 3.38E-03 |
| | | | NM_054026 | | | |
| SKIL | 217591_at | BF725121 | NM_005414 | 6498 | 1.01 | 8.82E-06 |
| GARNL1 | 215162_at | AB020691 | NM_014990 | 253959 | 1.01 | 3.09E-04 |
| | | | NM_194301 | | | |
| RFC5 | 203210_s_at | NM_007370 | NM_007370 | 5985 | 1.01 | 1.06E-03 |
| | | | NM_181578 | | | |
| AK024136 | 217152_at | AK024136 | --- | --- | 1.01 | 3.14E-03 |
| ATP8B1 | 214594_x_at | BG252666 | NM_005603 | 5205 | 1.01 | 6.15E-03 |
| AL050032 | 216621_at | AL050032 | --- | --- | 1.01 | 1.08E-05 |
| CKLFSF7 | 1560754_at | AL832450 | NM_138410 | 112616 | 1.01 | 1.86E-03 |
| | | | NM_181472 | | | |
| KIAA1212 | 1562648_at | BC035848 | NM_018084 | 55704 | 1.01 | 4.98E-03 |
| CAPN1 | 232012_at | AV691296 | NM_005186 | 823 | 1.01 | 1.96E-03 |
| ZNF207 | 231848_x_at | AW192569 | NM_003457 | 7756 | 1.01 | 7.04E-03 |
| TRA2A | 229574_at | AI268231 | NM_013293 | 29896 | 1.01 | 7.43E-03 |
| AU146021 | 232478_at | AU146021 | --- | --- | 1.01 | 7.34E-03 |
| KNTC1 | 206316_s_at | NM_014708 | NM_014708 | 9735 | 1.00 | 8.69E-06 |
| DENR | 238982_at | AW665791 | NM_003677 | 8562 | 1.00 | 3.31E-03 |
| DNCH2 | 1565149_at | AF288405 | XM_370652 | 79659 | 1.00 | 2.59E-06 |
| PRDX2 | 215067_x_at | AU147942 | NM_005809 | 7001 | 1.00 | 5.62E-03 |
| | | | NM_181737 | | | |
| | | | NM_181738 | | | |
| PRDX6 | 238951_at | BF843343 | NM_004905 | 9588 | 1.00 | 2.00E-04 |
| RIOK3 | 215588_x_at | AK024958 | NM_003831 | 8780 | 1.00 | 5.41E-04 |
| | | | NM_145906 | | | |
| MID1 | 203636_at | BE967532 | NM_000381 | 4281 | 1.00 | 2.42E-03 |
| | | | NM_033290 | | | |
| | | | NM_033291 | | | |
| E2-230K | 1554814_at | BC007822 | NM_022066 | 63893 | 1.00 | 2.86E-03 |
| ZFOC1 | 1558816_at | BC037793 | NM_152437 | 144348 | 1.00 | 1.23E-04 |
| EFHC1 | 225656_at | AI564473 | NM_018100 | 114327 | 1.00 | 7.26E-03 |
| KIAA0999 | 242920_at | AW590838 | NM_025164 | 23387 | 1.00 | 6.83E-03 |
| GRK6 | 202848_s_at | BG423052 | NM_00100410 5 | 2870 | 0.99 | 2.95E-03 |
| | | | NM_00100410 6 | | | |
| | | | NM_002082 | | | |
| STS | 243858_at | AA699970 | NM_000351 | 412 | 0.99 | 1.31E-03 |
| AZI2 | 223846_at | BC001139 | NM_022461 | 64343 | 0.99 | 9.97E-05 |
| | | | NM_203326 | | | |
| AW069499 | 230886_at | AW069499 | --- | --- | 0.99 | 5.25E-04 |
| NKTR | 215338_s_at | AI688640 | NM_005385 | 4820 | 0.99 | 6.23E-03 |
| FLJ10408 | 220535_at | NM_018088 | NM_018088 | 55138 | 0.99 | 2.76E-03 |
| PDZRN3 | 232776_at | AU145289 | NM_015009 | 23024 | 0.99 | 5.32E-03 |
| ACVR2 | 244332_at | AW974077 | NM_001616 | 92 | 0.99 | 5.39E-06 |
| GLS | 223080_at | AK025021 | NM_014905 | 2744 | 0.99 | 2.09E-03 |
| RAB35 | 205461_at | NM_006861 | NM_006861 | 11021 | 0.99 | 8.15E-03 |
| FLJ21415 | 218867_s_at | NM_024738 | NM_024738 | 79794 | 0.99 | 4.67E-03 |
| MTMR9 | 233101_at | AK022071 | NM_015458 | 66036 | 0.98 | 3.57E-04 |
| TDG | 203742_s_at | BF674842 | NM_00100841 1 | 6996 | 0.98 | 2.79E-04 |
| | | | NM_003211 | | | |
| DKFZP434K046 | 1570227_at | BC016065 | NM_020312 | 57017 | 0.98 | 7.84E-03 |
| RBL1 | 1555004_a_at | BC032247 | NM_002895 | 5933 | 0.98 | 3.12E-03 |
| | | | NM_183404 | | | |
| MYST3 | 242480_at | AA868356 | NM_006766 | 7994 | 0.98 | 3.48E-03 |
| TXNDC5 | 243405_at | AI097337 | NM_022085 | 81567 | 0.98 | 1.17E-03 |
| | | | NM_030810 | | | |
| C14orf153 | 232814_x_at | AK024310 | NM_032374 | 84334 | 0.98 | 2.05E-03 |
| FLJ10569 | 237504_at | AW002398 | NM_018142 | 55174 | 0.98 | 5.78E-05 |
| AW451634 | 229470_at | AW451634 | --- | --- | 0.98 | 2.25E-03 |
| ATP2B1 | 209281_s_at | M95541 | NM_00100132 3 | 490 | 0.98 | 7.26E-05 |
| | | | NM_001682 | | | |
| CHPT1 | 1559739_at | AK025141 | NM_020244 | 56994 | 0.98 | 1.14E-03 |
| PFKFB4 | 228499_at | AL038787 | NM_004567 | 5210 | 0.98 | 1.03E-04 |
| AI435248 | 242837_at | AI435248 | --- | --- | 0.98 | 3.31E-03 |
| AL567118 | 241388_at | AL567118 | --- | --- | 0.97 | 9.39E-03 |
| ZNF274 | 232436_at | AI057616 | NM_016324 | 10782 | 0.97 | 8.11E-03 |
| | | | NM_016325 | | | |
| | | | NM_133502 | | | |
| SLCO3A1 | 229776_at | AW138118 | NM_013272 | 28232 | 0.97 | 3.95E-03 |
| DRIM | 209725_at | AF072718 | NM_014503 | 27340 | 0.97 | 6.32E-03 |
| AARS | 201000_at | NM_001605 | NM_001605 | 16 | 0.97 | 3.59E-03 |
| BE857972 | 243781_at | BE857972 | --- | --- | 0.97 | 2.71E-04 |
| AU146017 | 234082_at | AU146017 | --- | --- | 0.97 | 1.73E-03 |
| MGC20481 | 243513_at | AA971848 | NM_052849 | 90416 | 0.96 | 3.69E-03 |
| CPEB4 | 242384_at | AI452469 | NM_030627 | 80315 | 0.96 | 8.12E-03 |
| KDELC2 | 225128_at | AL548941 | NM_153705 | 143888 | 0.96 | 3.31E-03 |
| R84398 | 241993_x_at | R84398 | --- | --- | 0.96 | 3.54E-03 |
| PR47 | 1560526_at | BQ880034 | --- | 266671 | 0.96 | 4.91E-03 |
| GTSE1 | 204315_s_at | AI340239 | NM_016426 | 51512 | 0.96 | 1.04E-03 |
| WDR11 | 229694_at | BF062828 | NM_018117 | 55717 | 0.96 | 6.59E-03 |
| CTNNB1 | 242558_at | AW362945 | NM_001904 | 1499 | 0.96 | 4.14E-03 |
| AI760298 | 244054_at | AI760298 | --- | --- | 0.96 | 2.18E-05 |
| IQWD1 | 235613_at | BF476152 | NM_018442 | 55827 | 0.96 | 6.64E-03 |
| AW024890 | 229942_at | AW024890 | --- | --- | 0.96 | 6.64E-03 |
| DDHD1 | 231165_at | BE857355 | NM_030637 | 80821 | 0.96 | 8.45E-03 |
| CDYL | 240594_at | W86659 | NM_004824 | 9425 | 0.96 | 4.83E-03 |
| | | | NM_170751 | | | |
| | | | NM_170752 | | | |
| U2AF1 | 232141_at | AU144161 | NM_006758 | 7307 | 0.96 | 2.17E-05 |
| RAPGEF2 | 238176_at | T86196 | XM_376350 | 9693 | 0.96 | 9.65E-03 |
| C14orf135 | 1563259_at | AK024838 | NM_022495 | 64430 | 0.95 | 1.91E-04 |
| GPX3 | 214091_s_at | AW149846 | NM_002084 | 2878 | 0.95 | 6.75E-03 |
| DKFZp434P055 | 232398_at | AK001064 | NM_173466 | 91531 | 0.95 | 1.46E-03 |
| MATR3 | 242260_at | BG283790 | NM_018834 | 9782 | 0.95 | 4.52E-04 |
| STK36 | 231806_s_at | AL133630 | NM_015690 | 27148 | 0.95 | 3.18E-03 |
| DTX1 | 1559618_at | BQ188678 | NM_004416 | 1840 | 0.95 | 2.32E-03 |
| AL832061 | 1559436_x_at | AL832061 | --- | --- | 0.95 | 1.65E-03 |
| VPS29 | 223026_s_at | AF201946 | NM_016226 | 51699 | 0.95 | 6.16E-04 |
| | | | NM_057180 | | | |
| SEC61A2 | 228747_at | AI694646 | NM_018144 | 55176 | 0.95 | 9.12E-03 |
| USP7 | 230761_at | AI972599 | NM_003470 | 7874 | 0.95 | 4.14E-06 |
| STK32B | 219686_at | NM_018401 | NM_018401 | 55351 | 0.95 | 7.68E-03 |
| SMO | 218629_at | NM_005631 | NM_005631 | 6608 | 0.95 | 6.28E-03 |
| TCF7L2 | 212761_at | AI949687 | NM_030756 | 6934 | 0.95 | 3.71E-03 |
| PDK1 | 226452_at | AU146532 | NM_002610 | 5163 | 0.94 | 7.99E-04 |
| MAP3K1 | 225927_at | AA541479 | XM_042066 | 4214 | 0.94 | 1.85E-03 |
| PMAIP1 | 204286_s_at | NM_021127 | NM_021127 | 5366 | 0.94 | 9.09E-04 |
| AL049260 | 216524_x_at | AL049260 | --- | --- | 0.94 | 1.42E-03 |
| GTF2H3 | 222104_x_at | AI569458 | NM_001516 | 2967 | 0.94 | 2.18E-03 |
| MGC13098 | 214740_at | BE676209 | NM_032958 | 246721 | 0.94 | 6.48E-03 |
| POLR2J2 | | | NM_032959 | 84820 | | |
| | | | NM_145325 | | | |
| | | | XM_379817 | | | |
| | | | XM_499280 | | | |
| FLJ31795 | 1566480_x_at | AK096357 | NM_144609 | 124808 | 0.94 | 1.19E-03 |
| MID1 | 203637_s_at | NM_000381 | NM_000381 | 4281 | 0.94 | 8.91E-03 |
| | | | NM_033290 | | | |
| | | | NM_033291 | | | |
| AI692624 | 1556352_at | AI692624 | --- | --- | 0.94 | 3.86E-03 |
| AL035461 | 231827_at | AL035461 | --- | --- | 0.94 | 8.02E-03 |
| NFKBIZ | 223218_s_at | AB037925 | NM_00100547 4 | 64332 | 0.93 | 9.02E-03 |
| | | | NM_031419 | | | |
| R78413 | 240538_at | R78413 | --- | -- | 0.93 | 5.24E-03 |
| UBXD4 | 235327_x_at | BG111015 | NM_181713 | 165324 | 0.93 | 2.26E-06 |
| PABPC1 | 236907_at | AI760366 | NM_002568 | 26986 | 0.93 | 9.37E-03 |
| RNF34 | 219035_s_at | NM_025126 | NM_025126 | 80196 | 0.93 | 1.26E-05 |
| | | | NM_194271 | | | |
| FLJ11539 | 219791_s_at | NM_024748 | XM_496724 | 79804 | 0.93 | 1.41E-03 |
| FLJ10156 | 221591_s_at | BC005004 | NM_019013 | 54478 | 0.93 | 4.68E-04 |
| CREBL1 | 1554487_a_at | BC008394 | | 1388 | 0.93 | 4.64E-05 |
| | | | NM_004381 | | | |
| RBBP4 | 239071_at | AI972451 | NM_005610 | 5928 | 0.93 | 3.91E-03 |
| SNRPA1 | 242146_at | AA872471 | NM_003090 | 6627 | 0.93 | 4.37E-03 |
| DERL3 | 231324_at | AW452134 | NM_00100286 2 | 91319 | 0.93 | 9.94E-05 |
| | | | NM_198440 | | | |
| DDX50 | 1568815_a_at | AA903184 | NM_024045 | 79009 | 0.93 | 3.15E-03 |
| KCTD3 | 241903_at | AI688631 | NM_016121 | 51133 | 0.93 | 1.35E-03 |
| TBX3 | 225544_at | AI806338 | NM_005996 | 6926 | 0.93 | 2.95E-03 |
| | | | NM_016569 | | | |
| EXOC7 | 214802_at | AK022397 | NM_015219 | 23265 | 0.93 | 6.60E-03 |
| ZDHHC13 | 243250_at | AI922173 | NM_00100148 3 | 54503 | 0.93 | 7.75E-03 |
| | | | NM_019028 | | | |
| NSF | 1566785_x_at | AK025172 | NM_006178 | 4905 | 0.92 | 1.01E-03 |
| C9orf3 | 237716_at | AA699673 | NM_032823 | 84909 | 0.92 | 2.56E-03 |
| GPR125 | 210473_s_at | M37712 | NM_145290 | 166647 | 0.92 | 5.29E-07 |
| ZNF160 | 214715_x_at | AK024789 | NM_033288 | 90338 | 0.92 | 1.07E-03 |
| | | | NM_198893 | | | |
| CALU | 238908_at | BG392414 | NM_001219 | 813 | 0.92 | 2.85E-05 |
| TCF7L2 | 216037_x_at | AA664011 | NM_030756 | 6934 | 0.92 | 1.59E-04 |
| MBNL2 | 232369_at | AF339768 | NM_144778 | 10150 | 0.92 | 6.38E-03 |
| | | | NM_207304 | | | |
| DHX36 | 1559039_at | AK096808 | NM_020865 | 170506 | 0.92 | 4.13E-03 |
| BE563152 | 224806_at | BE563152 | XM_498674 | 440448 | 0.92 | 7.47E-03 |
| TM4SF8 | 232632_at | AU145719 | NM_005724 | 10099 | 0.92 | 1.10E-05 |
| | | | NM_198902 | | | |
| BTBD14B | 215587_x_at | AK023891 | NM_052876 | 112939 | 0.92 | 2.11E-03 |
| AI821721 | 241223_x_at | AI821721 | --- | --- | 0.91 | 3.84E-03 |
| SREBF1 | 235862_at | AA954908 | NM_00100529 1 | 6720 | 0.91 | 1.46E-03 |
| | | | NM_004176 | | | |
| AI570571 | 236092_at | AI570571 | --- | --- | 0.91 | 2.41E-03 |
| FLJ13491 | 219245_s_at | AI309636 | NM_024623 | 79676 | 0.91 | 9.68E-06 |
| MDH1 | 235374_at | AW952547 | NM_005917 | 4190 | 0.91 | 1.14E-03 |
| PASK | 216945_x_at | U79240 | NM_015148 | 23178 | 0.91 | 3.14E-03 |
| DTYMK | 1553983_at | AF258562 | NM_012145 | 1841 | 0.90 | 9.36E-03 |
| CLU | 208792_s_at | M25915 | NM_001831 | 1191 | 0.90 | 3.22E-05 |
| | | | NM_203339 | | | |
| C20orf172 | 219512_at | NM_024918 | NM_024918 | 79980 | 0.90 | 1.35E-03 |
| PTPN1 | 202716_at | NM_002827 | NM_002827 | 5770 | 0.90 | 4.52E-04 |
| ENTPD6 | 201704_at | NM_001247 | NM_001247 | 955 | 0.90 | 9.65E-03 |
| DKFZP564C196 | 236367 _ at | N33174 | XM_498825 | 284649 | 0.90 | 3.20E-03 |
| GPT2 | 224839_s_at | BG328998 | NM_133443 | 84706 | 0.89 | 2.50E-03 |
| TH1L | 225006_x_at | AJ238379 | NM_016397 | 51497 | 0.89 | 1.75E-05 |
| | | | NM_198976 | | | |
| AW975183 | 244340_x_at | AW975183 | --- | --- | 0.89 | 1.08E-03 |
| AW131450 | 235513_at | AW131450 | --- | --- | 0.89 | 1.09E-05 |
| AK023783 | 215604_x_at | AK023783 | --- | --- | 0.89 | 6.48E-03 |
| KIAA0582 | 239442_at | BF589179 | NM_015147 | 23177 | 0.89 | 1.66E-03 |
| BTBD7 | 217141_at | AL049394 | NM_00100286 0 | 55727 | 0.89 | 2.17E-03 |
| | | | NM_018167 | | | |
| DNAPTP6 | 222154_s_at | AK002064 | NM_015535 | 26010 | 0.89 | 1.33E-03 |
| RNPEP | 208270_s_at | NM_020216 | NM_020216 | 6051 | 0.89 | 9.93E-04 |
| KIAA0232 | 232595_at | AK023294 | NM_014743 | 9778 | 0.89 | 2.09E-03 |
| ZNF503 | 227195_at | AA603467 | NM_032772 | 84858 | 0.88 | 1.25E-04 |
| RABL4 | 213784_at | AL037167 | NM_006860 | 11020 | 0.88 | 4.07E-03 |
| KLF7 | 204334_at | AA488672 | NM_003709 | 8609 | 0.88 | 5.59E-04 |
| FLJ13611 | 234788_x_at | AK024819 | NM_024941 | 80006 | 0.88 | 4.20E-03 |
| TFF3 | 204623_at | NM_003226 | NM_003226 | 7033 | 0.88 | 1.84E-03 |
| C7orf24 | 239841_at | AA137038 | NM_024051 | 79017 | 0.88 | 5.49E-05 |
| FLJ14346 | 220720_x_at | NM_025029 | NM_025029 | 80097 | 0.88 | 2.42E-03 |
| PGF | 215179_x_at | AK023843 | NM_002632 | 5228 | 0.88 | 6.50E-04 |
| BTBD9 | 233041_x_at | AK025009 | NM_152733 | 114781 | 0.87 | 5.26E-03 |
| ZFP64 | 242759_at | AI821726 | NM_018197 | 55734 | 0.87 | 1.39E-03 |
| | | | NM_022088 | | | |
| | | | NM_199426 | | | |
| | | | NM_199427 | | | |
| AW139789 | 232134_at | AW139789 | --- | --- | 0.87 | 2.61E-05 |
| KIAA1706 | 1563621_at | AL713724 | NM_030636 | 80820 | 0.87 | 9.21E-03 |
| GNAS | 228173_at | AA810695 | NM_000516 | 2778 | 0.87 | 1.14E-03 |
| | | | NM_016592 | | | |
| | | | NM_080425 | | | |
| | | | NM_080426 | | | |
| ESPL1 | 204817_at | NM_012291 | NM_012291 | 9700 | 0.87 | 4.80E-03 |
| AI791828 | 237868_x_at | AI791828 | --- | --- | 0.87 | 3.13E-05 |
| RBM6 | 1556672_a_at | AI190489 | NM_005777 | 10180 | 0.87 | 7.88E-04 |
| SBNO1 | 216161_at | AK024128 | NM_018183 | 55206 | 0.87 | 2.11E-03 |
| BF056892 | 229024_at | BF056892 | --- | --- | 0.87 | 7.14E-06 |
| CDC42BPA | 215296_at | AK027000 | NM_003607 | 8476 | 0.87 | 5.58E-03 |
| | | | NM_014826 | | | |
| BE727883 | 226495_at | BE727883 | XM_498844 | 440752 | 0.87 | 7.67E-03 |
| ANK2 | 216195_at | AF131823 | NM_001148 | 287 | 0.86 | 2.02E-04 |
| | | | NM_020977 | | | |
| SFI1 | 215699_x_at | AL096768 | NM_00100746 7 | 9814 | 0.86 | 4.61E-04 |
| | | | NM_014775 | | | |
| TH1L | 225261_x_at | AJ238376 | NM_016397 | 51497 | 0.86 | 4.08E-05 |
| | | | NM_198976 | | | |
| SEMA6A | 215028_at | AB002438 | NM_020796 | 57556 | 0.86 | 1.40E-04 |
| LOC91431 | 228859_at | BF056790 | NM_138698 | 91431 | 0.86 | 3.09E-03 |
| FTHFSDC1 | 240552_at | AA811452 | NM_015440 | 25902 | 0.86 | 8.65E-04 |
| TRIM36 | 219736_at | NM_018700 | NM_018700 | 55521 | 0.86 | 8.76E-03 |
| DDX11 | 208159_x_at | NM_004399 | NM_004399 | 1663 | 0.86 | 1.06E-03 |
| | | | NM_030653 | | | |
| | | | NM_030655 | | | |
| NFATC2IP | 212809_at | AA152202 | NM_032815 | 84901 | 0.86 | 5.06E-04 |
| M-RIP | 231255_at | AI970090 | NM_015134 | 23164 | 0.86 | 6.68E-04 |
| | | | NM_201274 | | | |
| PCNT1 | 218014_at | NM_024844 | NM_024844 | 79902 | 0.86 | 7.48E-05 |
| ADAM33 | 233868_x_at | AL117415 | NM_025220 | 80332 | 0.85 | 1.87E-04 |
| | | | NM_153202 | | | |
| FLJ10569 | 229633_at | AA115512 | NM_018142 | 55174 | 0.85 | 3.01E-04 |
| AI734111 | 222284_at | AI734111 | --- | --- | 0.85 | 2.27E-03 |
| RPL35A | 215208_x_at | AK021571 | NM_000996 | 6165 | 0.85 | 1.22E-03 |
| SNCAIP | 219511_s_at | NM_005460 | NM_005460 | 9627 | 0.85 | 1.37E-03 |
| GRIK2 | 1560265_at | BQ434382 | NM_021956 | 2898 | 0.85 | 8.83E-04 |
| | | | NM_175768 | | | |
| CCND1 | 208711_s_at | BC000076 | NM_053056 | 595 | 0.85 | 5.90E-05 |
| NM_003349 | 201003_x_at | NM_003349 | --- | --- | 0.85 | 1.49E-04 |
| FLJ13089 | 225888_at | N62802 | NM_024953 | 80018 | 0.84 | 3.59E-04 |
| SLB | 226324_s_at | AB033005 | NM_015662 | 26160 | 0.84 | 6.60E-03 |
| SFRS15 | 222310_at | AA648521 | NM_020706 | 57466 | 0.84 | 2.91E-03 |
| AP3M2 | 1569053_at | BG110196 | NM_006803 | 10947 | 0.84 | 9.81E-04 |
| STRA6 | 1569335_a_at | BC015881 | NM_022369 | 64220 | 0.83 | 1.47E-03 |
| TH1L | 225865_x_at | AJ238374 | NM_016397 | 51497 | 0.83 | 3.21E-04 |
| | | | NM_198976 | | | |
| SLC2A4RG | 218494_s_at | NM_020062 | NM_020062 | 56731 | 0.83 | 8.97E-05 |
| NCOA5 | 239815_at | R16784 | NM_020967 | 57727 | 0.83 | 7.12E-03 |
| AARSL | 231845_at | AI721172 | NM_020745 | 57505 | 0.83 | 4.13E-04 |
| AA767373 | 236327_at | AA767373 | --- | --- | 0.83 | 1.12E-03 |
| KIAA1434 | 230492_s_at | BE328402 | NM_019593 | 56261 | 0.83 | 8.78E-04 |
| RGMA | 223468_s_at | AL136826 | NM_020211 | 56963 | 0.83 | 2.26E-03 |
| T86613 | 235804_at | T86613 | --- | --- | 0.83 | 9.52E-05 |
| LMO4 | 209205_s_at | BC003600 | NM_006769 | 8543 | 0.83 | 5.83E-04 |
| AL044078 | 221860_at | AL044078 | --- | --- | 0.83 | 2.22E-04 |
| AA824321 | 227547_at | AA824321 | --- | --- | 0.82 | 1.63E-03 |
| C9orf64 | 223697_x_at | BC004407 | NM_032307 | 84267 | 0.82 | 8.80E-05 |
| EIF3S9 | 242550_at | AA628539 | NM_003751 | 8662 | 0.82 | 2.91E-04 |
| | | | NM_182712 | | | |
| LOC144766 | 243986_at | BE409452 | XM_378419 | 144766 | 0.82 | 2.37E-03 |
| C14orf145 | 1557755_at | AW028337 | XM_496002 | 145508 | 0.82 | 3.02E-03 |
| FAM19A5 | 237094_at | AI953086 | NM_015381 | 25817 | 0.82 | 1.93E-05 |
| CDKN2C | 211792_s_at | U17074 | NM_001262 | 1031 | 0.82 | 7.21E-03 |
| | | | NM_078626 | | | |
| STMN3 | 222557_at | AL353715 | NM_015894 | 50861 | 0.82 | 3.09E-03 |
| C6orf111 | 230375_at | AI936531 | NM_032870 | 25957 | 0.82 | 6.14E-04 |
| CBWD1 | 226193_x_at | AV709992 | NM_018491 | 150472 | 0.82 | 7.60E-03 |
| CBWD2 | | | NM_172003 | 220869 | | |
| LOC220869 | | | NM_201453 | 389760 | | |
| DC36 | | | | 55871 | | |
| ATF3 | 202672_s_at | NM_001674 | NM_001674 | 467 | 0.82 | 4.70E-03 |
| | | | NM_004024 | | | |
| AW629478 | 244625_at | AW629478 | --- | --- | 0.82 | 1.02E-03 |
| RAD51C | 206066_s_at | NM_002876 | NM_002876 | 5889 | 0.82 | 1.65E-03 |
| | | | NM_058216 | | | |
| | | | NM_058217 | | | |
| C15orf25 | 220071_x_at | NM_018097 | NM_018097 | 55142 | 0.82 | 5.19E-04 |
| C22orf18 | 218741_at | NM_024053 | NM_00100287 6 | 79019 | 0.81 | 7.04E-03 |
| | | | NM_024053 | | | |
| GSS | 211630_s_at | L42531 | NM_000178 | 2937 | 0.81 | 4.31E-03 |
| IREB2 | 1563129_at | AL109710 | NM_004136 | 3658 | 0.81 | 5.38E-03 |
| EPIM | 213434_at | H95263 | NM_001980 | 2054 | 0.81 | 1.48E-05 |
| | | | NM_194356 | | | |
| DZIP1L | 239785_at | BF340245 | NM_173543 | 199221 | 0.81 | 7.64E-03 |
| DHX37 | 223364_s_at | BC004463 | NM_032656 | 57647 | 0.81 | 4.75E-03 |
| BC041029 | 1569025_s_at | BC041029 | XM_371697 | 389211 | 0.81 | 2.66E-03 |
| NY-REN-7 | 214945_at | AW514267 | NM_173663 | 285596 | 0.81 | 9.82E-03 |
| LOC389347 | | | XM_371783 | 389347 | | |
| AA778747 | 241941_at | AA778747 | --- | --- | 0.81 | 3.29E-04 |
| $PDE4C | 206792_x_at | NM_000923 | NM_000923 | 5143 | 0.81 | 3.77E-04 |
| SYNCRIP | 209025_s_at | AF037448 | NM_006372 | 10492 | 0.81 | 1.94E-03 |
| DRPLA | 236961_at | AI539426 | NM_00100702 6 | 1822 | 0.80 | 7.80E-03 |
| | | | NM_001940 | | | |
| ZNF611 | 208137_x_at | NM_030972 | NM_030972 | 81856 | 0.80 | 1.06E-03 |
| BACH2 | 215907_at | AK027193 | NM_021813 | 60468 | 0.80 | 1.49E-03 |
| AL038191 | 240271_at | AL038191 | --- | --- | 0.80 | 4.41E-04 |
| FANCC | 205189_s_at | NM_000136 | NM_000136 | 2176 | 0.80 | 6.32E-04 |
| RPL10A | 232592_at | AU146731 | NM_007104 | 4736 | 0.80 | 4.23E-03 |
| GALNT7 | 218313_s_at | NM_017423 | NM_017423 | 51809 | 0.80 | 5.54E-03 |
| SCML1 | 222747_s_at | BF001786 | NM_006746 | 6322 | 0.79 | 2.71E-03 |
| DERA | 218102_at | NM_015954 | NM_015954 | 51071 | 0.79 | 1.87E-03 |
| AF131796 | 231916_at | AF131796 | --- | --- | 0.79 | 4.14E-03 |
| CCNE1 | 213523_at | AI671049 | NM_001238 | 898 | 0.79 | 8.53E-03 |
| | | | NM_057182 | | | |
| WWOX | 233161_at | AK022464 | NM_016373 | 51741 | 0.79 | 2.29E-03 |
| | | | NM_018560 | | | |
| | | | NM_130788 | | | |
| | | | NM_130790 | | | |
| | | | NM_130791 | | | |
| | | | NM_130792 | | | |
| | | | NM_130844 | | | |
| SRP54 | 1557504_at | AI076351 | NM_003136 | 6729 | 0.79 | 7.25E-03 |
| BG149337 | 229814_at | BG149337 | --- | --- | 0.79 | 7.38E-03 |
| BE676171 | 237156_at | BE676171 | --- | --- | 0.79 | 4.22E-03 |
| NR2C1 | 210530_s_at | M21985 | NM_003297 | 7181 | 0.79 | 4.35E-03 |
| NKTR | 243055_at | BF514072 | NM_005385 | 4820 | 0.78 | 9.55E-03 |
| KIAA0152 | 200617_at | NM_014730 | NM_014730 | 9761 | 0.78 | 6.41E-03 |
| EMILIN3 | 228307_at | AL137580 | NM_052846 | 90187 | 0.78 | 3.72E-03 |
| TM4SF14 | 221002_s_at | NM_030927 | NM_030927 | 81619 | 0.78 | 3.35E-03 |
| AI151104 | 237475_x_at | AI151104 | --- | --- | 0.78 | 1.69E-03 |
| NUP153 | 239948_at | AA004800 | NM_005124 | 9972 | 0.78 | 1.85E-03 |
| PELI2 | 219132_at | NM_021255 | NM_021255 | 57161 | 0.78 | 9.97E-04 |
| THOC4 | 226319_s_at | AF047002 | NM_005782 | 10189 | 0.78 | 3.97E-03 |
| HOXD4 | 1552337_s_at | NM_014621 | NM_014621 | 3233 | 0.78 | 4.73E-03 |
| PPM1B | 232580_x_at | AL117553 | NM_002706 | 5495 | 0.78 | 2.42E-03 |
| | | | NM_177968 | | | |
| | | | NM_177969 | | | |
| AK000864 | 216290_x_at | AK000864 | --- | --- | 0.78 | 9.80E-03 |
| KIFC1 | 209680_s_at | BC000712 | XM_371813 | 3833 | 0.78 | 2.91E-03 |
| GSS | 201415_at | NM_000178 | NM_000178 | 2937 | 0.78 | 7.64E-03 |
| ACPL2 | 236908_at | BE550429 | NM_152282 | 92370 | 0.78 | 2.99E-03 |
| AA348683 | 240198_at | AA348683 | --- | --- | 0.77 | 1.02E-05 |
| ORF1-FL49 | 224707_at | AL522667 | NM_032412 | 84418 | 0.77 | 3.79E-04 |
| LOC340085 | 232455_x_at | AU145408 | --- | 340085 | 0.77 | 4.32E-04 |
| CNP | 1556053_at | AL556987 | NM_033133 | 1267 | 0.77 | 2.24E-04 |
| AK098337 | 1566144_at | AK098337 | XM_496690 | 401131 | 0.77 | 1.47E-03 |
| HEG | 213069_at | AI148659 | XM_087386 | 57493 | 0.77 | 1.93E-03 |
| AI833186 | 242593_at | AI833186 | --- | --- | 0.77 | 9.83E-04 |
| GPR51 | 217077_s_at | AF095723 | NM_005458 | 9568 | 0.77 | 7.45E-03 |
| KIAA1434 | 224826_at | AK001947 | NM_019593 | 56261 | 0.77 | 3.37E-04 |
| TYRO3 | 211432_s_at | U05682 | NM_006293 | 7301 | 0.77 | 1.71E-03 |
| GNB4 | 223488_s_at | BC000873 | NM_021629 | 59345 | 0.77 | 9.63E-03 |
| MPHOSPH9 | 237158_s_at | AW449069 | NM_022782 | 10198 | 0.77 | 6.87E-03 |
| FBXW12 | 215600_x_at | AK022174 | NM_207102 | 285231 | 0.77 | 4.32E-03 |
| PTK7 | 1555324_at | BC046109 | NM_002821 | 5754 | 0.77 | 9.93E-03 |
| | | | NM_152880 | | | |
| | | | NM_152881 | | | |
| | | | NM_152882 | | | |
| | | | NM_152883 | | | |
| KLHL5 | 226001_at | AK002174 | NM_00100707 5 | 51088 | 0.77 | 7.30E-03 |
| | | | NM_199039 | | | |
| CRTAC1 | 221204_s_at | NM_018058 | NM_018058 | 55118 | 0.76 | 5.35E-03 |
| AK021514 | 215978_x_at | AK021514 | --- | --- | 0.76 | 7.15E-03 |
| N53564 | 242280_x_at | N53564 | --- | --- | 0.76 | 1.02E-04 |
| OLFML3 | 218162_at | NM_020190 | NM_020190 | 56944 | 0.76 | 3.61E-04 |
| PDCD2 | 243773_at | H65902 | NM_002598 | 5134 | 0.76 | 9.35E-03 |
| | | | NM_144781 | | | |
| H09533 | 239748_x_at | H09533 | --- | --- | 0.76 | 6.06E-04 |
| SH3MD1 | 224817_at | W93554 | NM_014631 | 9644 | 0.76 | 1.12E-05 |
| C9orf86 | 219828_at | NM_024718 | NM_024718 | 55684 | 0.76 | 2.56E-03 |
| TBX3 | 243234_at | BG055137 | NM_005996 | 6926 | 0.76 | 4.38E-03 |
| | | | NM_016569 | | | |
| PHF20L1 | 1554472_a_at | BC015211 | NM_016018 | 51105 | 0.76 | 3.06E-03 |
| | | | NM_024878 | | | |
| | | | NM_032205 | | | |
| | | | NM_198513 | | | |
| FLJ10404 | 243047_at | AV698751 | NM_019057 | 54540 | 0.76 | 6.86E-03 |
| SIRT2 | 1558331_at | BG722779 | NM_012237 | 22933 | 0.76 | 6.78E-03 |
| | | | NM_030593 | | | |
| BRI3BP | 231810_at | BG106919 | NM_080626 | 140707 | 0.76 | 1.27E-03 |
| C19orf14 | 215218_s_at | AC004144 | NM_173636 | 284403 | 0.76 | 9.95E-03 |
| AFG3L1 | 1552287_s_at | NM_001132 | NM_001132 | 172 | 0.76 | 4.38E-03 |
| MUM1 | 229033_s_at | AA143060 | NM_032853 | 84939 | 0.76 | 1.74E-04 |
| DHX35 | 218579_s_at | NM_021931 | NM_021931 | 60625 | 0.76 | 1.36E-03 |
| ESPL1 | 38158_at | D79987 | NM_012291 | 9700 | 0.75 | 5.71E-03 |
| WBSCR20C | 213460_x_at | N29665 | NM_032158 | 260294 | 0.75 | 3.30E-04 |
| | | | NM_148936 | | | |
| | | | NM_148980 | | | |
| | | | NM_149379 | | | |
| SFI1 | 213431_x_at | AB011114 | NM_00100746 7 | 9814 | 0.75 | 3.65E-05 |
| | | | NM_014775 | | | |
| AI252004 | 242696_at | AI252004 | --- | --- | 0.75 | 1.12E-03 |
| SLC15A4 | 225043_at | AW304786 | NM_145648 | 121260 | 0.75 | 9.55E-03 |
| PME-1 | 243443_at | AI076511 | NM_016147 | 51400 | 0.75 | 4.15E-03 |
| TUBGCP2 | 244842_x_at | AA235663 | NM_006659 | 10844 | 0.75 | 5.81E-03 |
| AI041522 | 228030_at | AI041522 | --- | --- | 0.75 | 8.68E-03 |
| KIAA0992 | 234135_x_at | AK021652 | NM_016081 | 23022 | 0.75 | 3.75E-04 |
| NQO1 | 201467_s_at | AI039874 | NM_000903 | 1728 | 0.75 | 9.53E-03 |
| TJP2 | 243148_at | AA007423 | NM_004817 | 9414 | 0.75 | 2.81E-03 |
| | | | NM_201629 | | | |
| IQGAP1 | 213446_s_at | AI679073 | NM_003870 | 8826 | 0.75 | 1.44E-03 |
| SEC11L3 | 223299_at | AF212233 | NM_033280 | 90701 | 0.75 | 7.34E-04 |
| LOC283871 | 222622_at | BG284709 | XM_208887 | 283871 | 0.75 | 4.92E-03 |
| T96555 | 243818_at | T96555 | --- | --- | 0.75 | 3.01E-03 |
| TCEA1 | 1566207_at | BQ286789 | NM_006756 | 6917 | 0.74 | 1.85E-03 |
| | | | NM_201437 | | | |
| ZNF44 | 233775_x_at | AK023304 | NM_016264 | 51710 | 0.74 | 1.98E-04 |
| AK024599 | 233702_x_at | AK024599 | --- | --- | 0.74 | 2.99E-03 |
| C20orf44 | 244795_at | AV693986 | NM_018244 | 55245 | 0.74 | 9.01E-03 |
| | | | NM_199487 | | | |
| | | | NM_199513 | | | |
| N4BP2 | 231996_at | AB037834 | NM_018177 | 55728 | 0.74 | 6.20E-06 |
| CLIC4 | 201559_s_at | AF109196 | NM_013943 | 25932 | 0.74 | 9.19E-03 |
| SEC15L1 | 232599_at | AF220217 | NM_019053 | 54536 | 0.74 | 7.90E-03 |
| AU145662 | 233399_x_at | AU145662 | XM_499131 | 441383 | 0.74 | 1.87E-03 |
| RAD54L | 204558_at | NM_003579 | NM_003579 | 8438 | 0.74 | 1.24E-03 |
| SLC4A8 | 207056_s_at | NM_004858 | NM_004858 | 9498 | 0.74 | 5.18E-03 |
| PLS3 | 201215_at | NM_005032 | NM_005032 | 5358 | 0.74 | 9.88E-03 |
| C10orf6 | 203482_at | AL133215 | NM_018121 | 55719 | 0.74 | 9.76E-03 |
| PDLIM7 | 1569150_x_at | BC023629 | NM_005451 | 9260 | 0.73 | 1.56E-03 |
| | | | NM_203352 | | | |
| | | | NM_203353 | | | |
| | | | NM_213636 | | | |
| AI939447 | 241268_x_at | AI939447 | --- | --- | 0.73 | 2.79E-04 |
| FLJ12649 | 219626_at | NM_024597 | XM_291344 | 79649 | 0.73 | 8.01E-04 |
| PPHLN1 | 234459_at | AK000186 | NM_016488 | 51535 | 0.73 | 6.98E-03 |
| | | | NM_201438 | | | |
| | | | NM_201439 | | | |
| | | | NM_201440 | | | |
| | | | NM_201515 | | | |
| DBT | 205370_x_at | NM_001918 | NM_001918 | 1629 | 0.73 | 4.67E-03 |
| CBX3 | 230998_at | AV702506 | NM_007276 | 11335 | 0.73 | 8.36E-03 |
| | | | NM_016587 | | | |
| ASAHL | 214765_s_at | AK024677 | NM_014435 | 27163 | 0.73 | 1.72E-05 |
| AF130054 | 211452_x_at | AF130054 | --- | --- | 0.73 | 5.36E-04 |
| CHKB | 204193_at | NM_005198 | NM_005198 | 1120 | 0.72 | 3.15E-04 |
| | | | NM_152253 | | | |
| IQGAP1 | 210840_s_at | D29640 | NM_003870 | 8826 | 0.72 | 7.05E-05 |
| ASAHL | 232072_at | AK025371 | NM_014435 | 27163 | 0.72 | 1.08E-03 |
| MDS028 | 220590_at | NM_018463 | NM_018463 | 55846 | 0.72 | 2.33E-04 |
| SPCS3 | 222753_s_at | AL136660 | NM_021928 | 60559 | 0.72 | 6.00E-03 |
| TERT | 207199_at | NM_003219 | NM_003219 | 7015 | 0.72 | 3.23E-05 |
| | | | NM_198253 | | | |
| | | | NM_198254 | | | |
| | | | NM_198255 | | | |
| BI496583 | 1557675_at | BI496583 | --- | --- | 0.72 | 4.63E-03 |
| KIAA1731 | 1565829_at | BU619319 | XM_374922 | 85459 | 0.72 | 6.04E-03 |
| UPF3A | 206958_s_at | AF318575 | NM_023011 | 65110 | 0.72 | 6.99E-04 |
| | | | NM_080687 | | | |
| GPR125 | 1555122_at | BC026009 | NM_145290 | 166647 | 0.72 | 4.13E-03 |
| FMNL2 | 1556656_at | BF477401 | NM_00100441 7 | 114793 | 0.72 | 1.56E-03 |
| | | | NM_00100442 1 | | | |
| | | | NM_00100442 2 | | | |
| | | | NM_052905 | | | |
| UBE2J1 | 236528_at | N64079 | NM_016021 | 51465 | 0.72 | 2.03E-03 |
| AF15Q14 | 1552680_a_at | NM_020380 | NM_020380 | 57082 | 0.71 | 8.46E-03 |
| | | | NM_144508 | | | |
| | | | NM_170589 | | | |
| RAB11FIP3 | 228613_at | BF183535 | NM_014700 | 9727 | 0.71 | 5.28E-03 |
| GARNL1 | 234923_at | AK022988 | NM_014990 | 253959 | 0.71 | 7.34E-03 |
| | | | NM_194301 | | | |
| TK1 | 1554408_a_at | BC007986 | NM_003258 | 7083 | 0.71 | 4.05E-03 |
| BE503981 | 229593_at | BE503981 | --- | --- | 0.71 | 2.45E-05 |
| MARS | 201475_x_at | NM_004990 | NM_004990 | 4141 | 0.71 | 6.05E-03 |
| IDH3B | 210014_x_at | AF023266 | NM_006899 | 3420 | 0.71 | 6.81E-03 |
| | | | NM_174855 | | | |
| | | | NM_174856 | | | |
| ETV1 | 217061_s_at | AC004857 | NM_004956 | 2115 | 0.71 | 4.82E-03 |
| AI829569 | 236671_at | AI829569 | --- | --- | 0.71 | 5.08E-04 |
| MCM4 | 212141_at | AA604621 | NM_005914 | 4173 | 0.71 | 2.19E-03 |
| | | | NM_182746 | | | |
| HRPT2 | 215398_at | AF038194 | NM_024529 | 79577 | 0.70 | 2.72E-03 |
| SFRS14 | 212001_at | AV738039 | NM_014884 | 10147 | 0.70 | 1.89E-04 |
| GEMIN4 | 217099_s_at | AF258545 | NM_015721 | 50628 | 0.70 | 2.89E-03 |
| RRAGD | 221524_s_at | AF272036 | NM_021244 | 58528 | 0.70 | 4.52E-03 |
| PSAT1 | 220892_s_at | NM_021154 | NM_021154 | 29968 | 0.70 | 4.14E-03 |
| | | | NM_058179 | | | |
| NSUN5 | 213670_x_at | AI768378 | NM_018044 | 155400 | 0.70 | 3.58E-05 |
| WBSCR20B | | | NM_032158 | 260294 | | |
| WBSCR20C | | | NM_145645 | 55695 | | |
| | | | NM_148936 | | | |
| | | | NM_148956 | | | |
| | | | NM_148980 | | | |
| | | | NM_149379 | | | |
| KIAA0286 | 212621_at | AB006624 | NM_015257 | 23306 | 0.70 | 5.42E-03 |
| AFG3L1 | 236250_at | AI859065 | NM_001132 | 172 | 0.70 | 4.29E-03 |
| MAD2L2 | 223234_at | AF080398 | NM_006341 | 10459 | 0.70 | 6.48E-03 |
| BF062155 | 238279_x_at | BF062155 | --- | --- | 0.70 | 1.33E-03 |
| PSMD7 | 238738_at | AW361702 | NM_002811 | 5713 | 0.70 | 1.94E-03 |
| TUBG1 | 201714_at | NM_001070 | NM_001070 | 7283 | 0.69 | 9.09E-03 |
| CSTF1 | 202190_at | NM_001324 | NM_001324 | 1477 | 0.69 | 3.88E-03 |
| T71269 | 234033_at | T71269 | --- | --- | 0.69 | 3.53E-03 |
| CROP | 241792_x_at | N36160 | NM_006107 | 51747 | 0.69 | 2.36E-03 |
| | | | NM_016424 | | | |
| SLIT3 | 203813_s_at | NM_003062 | NM_003062 | 6586 | 0.69 | 8.85E-03 |
| AI857788 | 227126_at | AI857788 | --- | --- | 0.69 | 3.64E-04 |
| FLJ12700 | 219627_at | NM_024910 | NM_024910 | 79970 | 0.69 | 5.30E-04 |
| FLJ40432 | 228810_at | AW135279 | NM_152523 | 151195 | 0.69 | 9.81E-04 |
| DGCR8 | 219811_at | NM_022720 | NM_022720 | 54487 | 0.69 | 3.02E-03 |
| SHMT1 | 209980_s_at | L23928 | NM_004169 | 6470 | 0.68 | 2.17E-03 |
| | | | NM_148918 | | | |
| UPF3A | 217596_at | AA649851 | NM_023011 | 65110 | 0.68 | 3.51E-03 |
| | | | NM_080687 | | | |
| PPT2 | 209826_at | AF020544 | NM_005155 | 9374 | 0.68 | 3.49E-04 |
| | | | NM_138717 | | | |
| | | | NM_138934 | | | |
| THOC4 | 226320_at | AF047002 | NM_005782 | 10189 | 0.68 | 4.54E-03 |
| NDUFA5 | 215850_s_at | AK022209 | NM_005000 | 4698 | 0.68 | 8.18E-03 |
| DTYMK | 203270_at | NM_012145 | NM_012145 | 1841 | 0.68 | 7.28E-03 |
| FLJ10199 | 218815_s_at | NM_018022 | NM_018022 | 55092 | 0.68 | 3.91E-03 |
| CRSP6 | 232483_at | AK022156 | NM_004268 | 9440 | 0.68 | 5.79E-04 |
| PTPRF | 215066_at | AU158443 | NM_002840 | 5792 | 0.68 | 3.31E-03 |
| | | | NM_130440 | | | |
| ADD1 | 214736_s_at | BE898639 | NM_001119 | 118 | 0.68 | 1.78E-03 |
| | | | NM_014189 | | | |
| | | | NM_014190 | | | |
| | | | NM_176801 | | | |
| C9orf88 | 223019_at | BC001979 | NM_022833 | 64855 | 0.68 | 9.03E-04 |
| FLJ34443 | 241408_at | AA531337 | NM_175918 | 285464 | 0.68 | 9.11E-03 |
| TK2 | 208246_x_at | NM_017618 | NM_004614 | 7084 | 0.67 | 5.00E-03 |
| C10orf79 | 243896_at | AI809779 | NM_025145 | 80217 | 0.67 | 4.78E-03 |
| SRGAP2 | 1556202_at | AI263819 | XM_059095 | 23380 | 0.67 | 9.56E-03 |
| PANK2 | 218809_at | NM_024960 | NM_024960 | 80025 | 0.67 | 2.17E-06 |
| | | | NM_153637 | | | |
| | | | NM_153638 | | | |
| | | | NM_153639 | | | |
| | | | NM_153640 | | | |
| | | | NM_153641 | | | |
| HOXD4 | 205522_at | NM_014621 | NM_014621 | 3233 | 0.67 | 5.20E-03 |
| NES | 218678_at | NM_024609 | NM_006617 | 10763 | 0.67 | 1.78E-03 |
| MICAL3 | 229474_at | BF055090 | XM_032996 | 57553 | 0.67 | 3.47E-03 |
| | | | XM_032997 | | | |
| TCF7L2 | 212762_s_at | AI375916 | NM_030756 | 6934 | 0.67 | 3.83E-03 |
| TPD52L2 | 201379_s_at | NM_003288 | NM_003288 | 7165 | 0.66 | 4.00E-03 |
| | | | NM_199359 | | | |
| | | | NM_199360 | | | |
| | | | NM_199361 | | | |
| | | | NM_199362 | | | |
| | | | NM_199363 | | | |
| PVRL3 | 213325_at | AA129716 | NM_015480 | 25945 | 0.66 | 2.59E-04 |
| ASAHL | 227135_at | AI436803 | NM_014435 | 27163 | 0.66 | 2.36E-03 |
| AI197932 | 229498_at | AI197932 | --- | --- | 0.66 | 6.38E-03 |
| KIAA0931 | 213407_at | AB023148 | NM_015020 | 23035 | 0.66 | 4.47E-03 |
| AK057923 | 1556364_at | AK057923 | XM_498935 | 440960 | 0.66 | 9.42E-03 |
| ADRBK2 | 204183_s_at | AI478542 | NM_005160 | 157 | 0.66 | 2.10E-04 |
| SNX5 | 223666_at | BC002724 | NM_014426 | 27131 | 0.66 | 5.32E-03 |
| | | | NM_152227 | | | |
| WBSCR23 | 221176_x_at | NM_025042 | NM_025042 | 80112 | 0.66 | 5.53E-06 |
| AK024901 | 234326_at | AK024901 | --- | --- | 0.65 | 1.50E-04 |
| ZNF500 | 41113_at | AI871396 | NM_021646 | 26048 | 0.65 | 1.77E-03 |
| DMN | 212730_at | AK026420 | NM_015286 | 23336 | 0.65 | 3.64E-03 |
| | | | NM_145728 | | | |
| ANKH | 239449_at | AV693734 | NM_054027 | 56172 | 0.65 | 2.03E-03 |
| C14orf120 | 216263_s_at | AK022215 | XM_033371 | 25983 | 0.65 | 1.76E-03 |
| ZNF291 | 215848_at | AW139369 | NM_020843 | 49855 | 0.65 | 3.43E-03 |
| RFC1 | 209085_x_at | L14922 | NM_002913 | 5981 | 0.65 | 2.64E-04 |
| SLIT2 | 209897_s_at | AF055585 | NM_004787 | 9353 | 0.65 | 2.23E-03 |
| LOC134147 | 234981_x_at | BE537881 | NM_138809 | 134147 | 0.65 | 2.57E-03 |
| GPR51 | 209991_x_at | AF069755 | NM_005458 | 9568 | 0.65 | 6.51E-04 |
| AL037473 | 1557780_at | AL037473 | --- | --- | 0.64 | 9.19E-03 |
| C14orf111 | 215567_at | AU144919 | NM_015962 | 51077 | 0.64 | 2.31E-03 |
| CBX5 | 241875_at | AA204654 | NM_012117 | 23468 | 0.64 | 8.07E-04 |
| UCKL1 | 218533_s_at | NM_017859 | NM_017859 | 54963 | 0.64 | 7.28E-03 |
| BG231554 | 243179_at | BG231554 | --- | --- | 0.64 | 5.62E-04 |
| PLK4 | 204887_s_at | NM_014264 | NM_014264 | 10733 | 0.64 | 7.56E-04 |
| OGG1 | 205760_s_at | NM_016821 | NM_002542 | 4968 | 0.64 | 4.62E-04 |
| | | | NM_016819 | | | |
| | | | NM_016820 | | | |
| | | | NM_016821 | | | |
| | | | NM_016826 | | | |
| | | | NM_016827 | | | |
| | | | NM_016828 | | | |
| | | | NM_016829 | | | |
| PAICS | 201014_s_at | NM_006452 | NM_006452 | 10606 | 0.64 | 8.63E-05 |
| RNF150 | 227657_at | AA722069 | NM_020724 | 57484 | 0.64 | 2.38E-03 |
| ETV1 | 217053_x_at | X87175 | NM_004956 | 2115 | 0.64 | 6.17E-03 |
| HRB2 | 232441_at | AU147079 | NM_007043 | 11103 | 0.64 | 8.66E-03 |
| CTNNBL1 | 221021_s_at | NM_030877 | NM_030877 | 56259 | 0.64 | 2.56E-04 |
| FLJ33915 | 229892_at | AW628843 | NM_182613 | 347918 | 0.64 | 7.04E-03 |
| TORC3 | 232879_at | AK024981 | NM_022769 | 64784 | 0.64 | 5.57E-03 |
| IPW | 213447_at | AI672541 | --- | 3653 | 0.64 | 3.46E-03 |
| ZNF337 | 214760_at | AL049942 | NM_015655 | 26152 | 0.64 | 6.30E-03 |
| SNTA1 | 203516_at | NM_003098 | NM_003098 | 6640 | 0.64 | 5.62E-03 |
| STX1A | 204729_s_at | NM_004603 | NM_004603 | 6804 | 0.64 | 4.16E-04 |
| RAN | 200750_s_at | AF054183 | NM_006325 | 5901 | 0.64 | 4.86E-04 |
| FLJ11029 | 232215_x_at | AK000296 | NM_018304 | 55771 | 0.63 | 8.93E-04 |
| H2AFX | 205436_s_at | NM_002105 | NM_002105 | 3014 | 0.63 | 1.85E-03 |
| LOC338799 | 1556042_s_at | BI912454 | --- | 338799 | 0.63 | 2.22E-03 |
| BC022346 | 1570210_x_at | BC022346 | --- | --- | 0.63 | 5.53E-03 |
| C10orf58 | 224435_at | BC005871 | NM_032333 | 84293 | 0.63 | 3.19E-06 |
| LOC338799 | 226369_at | AW138760 | --- | 338799 | 0.62 | 4.89E-03 |
| C9orf81 | 1557985_s_at | AA248753 | XM_095991 | 84131 | 0.62 | 4.31E-03 |
| AK023870 | 233427_x_at | AK023870 | --- | --- | 0.62 | 7.36E-04 |
| FLJ10504 | 218296_x_at | NM_018116 | NM_018116 | 55154 | 0.62 | 1.11E-03 |
| LOC155435 | 225839_at | AW290882 | NM_001008408 | 155435 | 0.62 | 7.67E-03 |
| GNG12 | 212294_at | BG111761 | NM_018841 | 55970 | 0.62 | 4.81E-04 |
| FLJ23342 | 236059_at | AW168168 | NM_024631 | 79684 | 0.62 | 8.20E-03 |
| LM07 | 233646_at | AK027238 | NM_005358 | 4008 | 0.62 | 7.32E-03 |
| PHF20L1 | 222133_s_at | AK022280 | NM_016018 | 51105 | 0.62 | 1.32E-03 |
| | | | NM_024878 | | | |
| | | | NM_032205 | | | |
| | | | NM_198513 | | | |
| C14orf143 | 214264_s_at | AI656610 | NM_145231 | 90141 | 0.62 | 5.71E-03 |
| LOC157627 | 1556046_a_at | AL832535 | --- | 157627 | 0.62 | 4.24E-03 |
| MAP3K13 | 206249_at | NM_004721 | NM_004721 | 9175 | 0.62 | 4.38E-03 |
| VGF | 205586_x_at | NM_003378 | NM_003378 | 7425 | 0.62 | 1.70E-03 |
| BLCAP | 201032_at | NM_006698 | NM_006698 | 10904 | 0.62 | 1.91E-04 |
| POLE2 | 205909_at | NM_002692 | NM_002692 | 5427 | 0.62 | 9.76E-03 |
| ZGPAT | 221848_at | AL121845 | NM_032527 | 84619 | 0.61 | 1.83E-03 |
| | | | NM_181484 | | | |
| | | | NM_181485 | | | |
| STN2 | 227461_at | AA632295 | NM_033104 | 85439 | 0.61 | 5.56E-03 |
| SFRS11 | 213742_at | AW241752 | NM_004768 | 9295 | 0.61 | 8.24E-03 |
| GPR51 | 209990_s_at | AF056085 | NM_005458 | 9568 | 0.61 | 8.69E-04 |
| TGDS | 237346_at | AA976208 | NM_014305 | 23483 | 0.61 | 2.60E-03 |
| PANK2 | 235130_at | AV703394 | NM_024960 | 80025 | 0.61 | 2.05E-03 |
| | | | NM_153637 | | | |
| | | | NM_153638 | | | |
| | | | NM_153639 | | | |
| | | | NM_153640 | | | |
| | | | NM_153641 | | | |
| BAX | 208478_s_at | NM_004324 | NM_004324 | 581 | 0.61 | 2.28E-03 |
| | | | NM_138761 | | | |
| | | | NM_138762 | | | |
| | | | NM_138763 | | | |
| | | | NM_138764 | | | |
| | | | NM_138765 | | | |
| RNF10 | 207801_s_at | NM_014868 | NM_014868 | 9921 | 0.61 | 9.50E-03 |
| SPAG5 | 203145_at | NM_006461 | NM_006461 | 10615 | 0.61 | 3.43E-03 |
| TOP3A | 204946_s_at | NM_004618 | NM_004618 | 7156 | 0.61 | 4.78E-04 |
| REC14 | 215156_at | AL109709 | NM_025234 | 80349 | 0.61 | 9.61E-04 |
| MGC4308 | 220612_at | NM_014135 | NM_032359 | 84319 | 0.61 | 7.73E-03 |
| AI144156 | 238670_at | AI144156 | --- | --- | 0.61 | 2.33E-03 |
| TBPIP | 205956_x_at | NM_013290 | NM_013290 | 29893 | 0.61 | 9.34E-05 |
| | | | NM_016556 | | | |
| AW517479 | 242608_x_at | AW517479 | --- | --- | 0.60 | 4.70E-03 |
| DXS9879E | 219061_s_at | NM_006014 | NM_00601 | 8270 | 0.60 | 7.83E-03 |
| 384D8-2 | 205086_s_at | NM_014551 | NM_014551 | 29781 | 0.60 | 3.91E-03 |
| | | | NM_152299 | | | |
| DKFZP564K196 | 223170_at | AF132000 | NM_015544 | 26022 | 0.60 | 2.90E-03 |
| SOX2 | 213722_at | AW007161 | NM_003106 | 6657 | 0.60 | 2.03E-03 |
| CGI-96 | 33307_at | AL022316 | NM_015703 | 27341 | 0.60 | 7.39E-03 |
| BE464132 | 236841_at | BE464132 | XM_496585 | 440897 | 0.60 | 1.92E-03 |
| AK025048 | 1565705_x_at | AK025048 | --- | --- | 0.60 | 7.42E-03 |
| HEXA | 215155_at | J04178 | NM_000520 | 3073 | 0.60 | 8.59E-03 |
| NY-REN-41 | 226287_at | AI458313 | NM_080654 | 91057 | 0.60 | 6.85E-03 |
| AK023354 | 222252_x_at | AK023354 | --- | --- | 0.60 | 7.20E-03 |
| KIAA0582 | 1559159_at | AK094069 | NM_015147 | 23177 | 0.59 | 2.73E-03 |
| PDE8A | 212521_s_at | BE568219 | NM_002605 | 5151 | 0.59 | 7.81E-03 |
| | | | NM_173454 | | | |
| | | | NM_173455 | | | |
| | | | NM_173456 | | | |
| | | | NM_173457 | | | |
| BE671060 | 235901_at | BE671060 | --- | --- | 0.59 | 7.80E-03 |
| KIAA1423 | 225127_at | BF217531 | XM_376550 | 57583 | 0.59 | 2.86E-03 |
| DDX54 | 219111_s_at | NM_024072 | NM_024072 | 79039 | 0.59 | 5.24E-03 |
| ADCYAP1 | 230237_at | BE220888 | NM_001117 | 116 | 0.59 | 4.24E-03 |
| CDKN1A | 202284_s_at | NM_000389 | NM_000389 | 1026 | 0.59 | 1.54E-03 |
| | | | NM_078467 | | | |
| PWP1 | 201606_s_at | BE796924 | NM_007062 | 11137 | 0.59 | 3.48E-04 |
| ZNF505 | 215758_x_at | AC007204 | NM_00100412 6 | 81931 | 0.59 | 1.44E-03 |
| | | | NM_031218 | | | |
| FLJ00312 | 221971_x_at | BE672818 | XM_374801 | 399761 | 0.59 | 7.23E-03 |
| IGBP1 | 242337_at | AI347128 | NM_001551 | 3476 | 0.59 | 9.36E-03 |
| ANKRD11 | 219437_s_at | NM_013275 | NM_013275 | 29123 | 0.59 | 5.45E-03 |
| TRAF4 | 202871_at | NM_004295 | NM_004295 | 9618 | 0.59 | 3.31E-03 |
| | | | NM_145751 | | | |
| FLJ10404 | 218920_at | NM_019057 | NM_019057 | 54540 | 0.59 | 1.51E-03 |
| SS18L1 | 213140_s_at | AB014593 | NM_015558 | 26039 | 0.59 | 7.09E-05 |
| | | | NM_198935 | | | |
| C20orf161 | 236514_at | AI885067 | NM_033421 | 90203 | 0.58 | 6.89E-03 |
| | | | NM_152897 | | | |
| CTNNB1 | 223679_at | AF130085 | NM_001904 | 1499 | 0.58 | 7.83E-04 |
| NSFL1C | 220248_x_at | NM_018839 | NM_016143 | 55968 | 0.58 | 4.26E-03 |
| | | | NM_018839 | | | |
| | | | NM_182483 | | | |
| MGC15606 | 215968_at | AF055018 | NM_145037 | 91775 | 0.58 | 2.12E-03 |
| VTN | 204534_at | NM_000638 | NM_000638 | 7448 | 0.58 | 1.72E-03 |
| SNX5 | 217792_at | NM_014426 | NM_014426 | 27131 | 0.58 | 4.70E-03 |
| | | | NM_152227 | | | |
| LOC157627 | 214839_at | AF052108 | --- | 157627 | 0.58 | 4.90E-03 |
| NSFL1C | 232520_s_at | AK023585 | NM_016143 | 55968 | 0.58 | 7.68E-03 |
| | | | NM_018839 | | | |
| | | | NM_182483 | | | |
| E2IGS | 223193_x_at | AF201944 | NM_014367 | 26355 | 0.58 | 3.89E-04 |
| ANKFY1 | 224900_at | AK025960 | NM_016376 | 51479 | 0.58 | 6.56E-03 |
| | | | NM_020740 | | | |
| HNRPA3 | 211931_s_at | BG505670 | NM_194247 | 220988 | 0.58 | 1.97E-03 |
| COH1 | 1553852_at | NM_152564 | NM_015243 | 157680 | 0.58 | 8.57E-03 |
| | | | NM_017890 | | | |
| | | | NM_152564 | | | |
| | | | NM_181661 | | | |
| | | | NM_184042 | | | |
| ZNF305 | 240181_at | AI939511 | NM_014724 | 9753 | 0.58 | 6.71E-03 |
| RFC5 | 203209_at | BC001866 | NM_007370 | 5985 | 0.58 | 7.00E-03 |
| | | | NM_181578 | | | |

**Table 10. Decreased Gene Expression in Cell Lines H1 and BB10 vs. SiMa Cell Line in Undifferentiated State Using Microarray Analysis**

| **Gene Symbol** | **Probe Set ID** | **Representative Public ID** | **RefSeq Transcript ID** | **Entrez Gene ID** | **Log2 Ratio** | **pvalue** |
|---|---|---|---|---|---|---|
| HSPA1A | 200799_at | NM_005345 | NM_005345 | 3303 | -6.50 | 8.44E-07 |
| CYTL1 | 219837_s_at | NM_018659 | NM_018659 | 54360 | -5.69 | 4.29E-10 |
| CYGB | 226632_at | AL513673 | NM_134268 | 114757 | -5.62 | 1.77E-03 |
| SYT13 | 226086_at | AB037848 | NM_020826 | 57586 | -5.23 | 2.54E-09 |
| H39185 | 240433_x_at | H39185 | --- | --- | -5.19 | 1.50E-07 |
| AV724769 | 235118_at | AV724769 | --- | --- | -5.15 | 5.31E-08 |
| AW188087 | 226809_at | AW188087 | --- | --- | -5.07 | 1.50E-07 |
| IGFBP7 | 201162_at | NM_001553 | NM_001553 | 3490 | -4.97 | 1.64E-07 |
| MEOX2 | 206201_s_at | NM_005924 | NM_005924 | 4223 | -4.95 | 5.73E-08 |
| NNAT | 204239_s_at | NM_005386 | NM_005386 | 4826 | -4.84 | 3.41E-07 |
| | | | NM_181689 | | | |
| CYGB | 1553572_a_at | NM_134268 | NM_134268 | 114757 | -4.74 | 1.06E-03 |
| IGSF4D | 1552754_a_at | AA640422 | NM_153184 | 253559 | -4.44 | 9.24E-10 |
| IGFBP7 | 201163_s_at | NM_001553 | NM_001553 | 3490 | -4.43 | 2.10E-04 |
| CAP2 | 212554_at | N90755 | NM_006366 | 10486 | -4.40 | 7.38E-04 |
| AF150317 | 243111_at | AF150317 | --- | --- | -4.32 | 6.95E-07 |
| FGF1 | 1552721_a_at | NM_033136 | NM_000800 | 2246 | -4.30 | 3.01E-04 |
| | | | NM_033136 | | | |
| | | | NM_033137 | | | |
| LMCD1 | 218574_s_at | NM_014583 | NM_014583 | 29995 | -4.23 | 1.05E-04 |
| TNFRSF10D | 227345_at | AI738556 | NM_003840 | 8793 | -4.09 | 1.08E-04 |
| IGFBP5 | 211959_at | AW007532 | NM_000599 | 3488 | -4.02 | 3.56E-04 |
| CART | 206339_at | NM_004291 | NM_004291 | 9607 | -4.01 | 4.51E-06 |
| ASAM | 226834_at | BG112263 | NM_024769 | 79827 | -3.99 | 9.02E-04 |
| AI093492 | 237435_at | AI093492 | --- | --- | -3.95 | 2.11E-04 |
| FUCA2 | 223120_at | BC003060 | NM_032020 | 2519 | -3.94 | 2.55E-03 |
| PAPPA | 224942_at | BG434272 | NM_002581 | 5069 | -3.81 | 5.43E-06 |
| PRAME | 204086_at | NM_006115 | NM_006115 | 23532 | -3.67 | 4.15E-07 |
| | | | NM_206953 | | | |
| | | | NM_206954 | | | |
| | | | NM_206955 | | | |
| | | | NM_206956 | | | |
| DHRS3 | 202481_at | NM_004753 | NM_004753 | 9249 | -3.66 | 8.82E-07 |
| TRHDE | 219937_at | NM_013381 | NM_013381 | 29953 | -3.59 | 4.97E-05 |
| C9orf94 | 229585_at | AI803088 | NM_152702 | 206938 | -3.59 | 2.72E-04 |
| DSCAM | 237268_at | BE503065 | NM_001389 | 1826 | -3.57 | 1.08E-07 |
| | | | NM_206887 | | | |
| FGF13 | 205110_s_at | NM_004114 | NM_004114 | 2258 | -3.51 | 5.78E-05 |
| | | | NM_033642 | | | |
| FGF1 | 205117_at | X59065 | NM_000800 | 2246 | -3.50 | 1.90E-03 |
| | | | NM_033136 | | | |
| | | | NM_033137 | | | |
| PTN | 211737_x_at | BC005916 | NM_002825 | 5764 | -3.44 | 5.00E-04 |
| GNAS | 217057_s_at | AF107846 | NM_000516 | 2778 | -3.42 | 1.41E-03 |
| | | | NM_016592 | | | |
| | | | NM_080425 | | | |
| | | | NM_080426 | | | |
| FLJ45880 | 228538_at | BE671164 | NM_207404 | 389114 | -3.42 | 1.47E-04 |
| ADAMTS3 | 214913_at | AB002364 | NM_014243 | 9508 | -3.41 | 2.23E-04 |
| CYP2E1 | 1431_at | J02843 | NM_000773 | 1571 | -3.38 | 7.97E-04 |
| AL353759 | 215071_s_at | AL353759 | --- | --- | -3.37 | 9.25E-05 |
| PAPPA | 224940_s_at | BF107618 | NM_002581 | 5069 | -3.36 | 7.36E-06 |
| BF941609 | 240218_at | BF941609 | --- | --- | -3.35 | 3.21E-05 |
| FGF1 | 208240_s_at | NM_013394 | NM_000800 | 2246 | -3.35 | 4.91E-04 |
| | | | | | | |
| | | | NM_033137 | | | |
| TCEAL2 | 211276_at | AF063606 | NM_080390 | 140597 | -3.35 | 4.16E-05 |
| TncRNA | 224565_at | BE675516 | --- | 283131 | -3.34 | 1.99E-04 |
| IGSF4D | 1555544_a_at | AF538973 | NM_153184 | 253559 | -3.30 | 7.37E-07 |
| MGC45474 | 228221_at | AA809640 | NM_152369 | 126969 | -3.27 | 7.37E-04 |
| KIAA1598 | 221802_s_at | AU157109 | NM_018330 | 57698 | -3.25 | 5.86E-06 |
| FGL1 | 205305_at | NM_004467 | NM_004467 | 2267 | -3.25 | 2.90E-06 |
| | | | NM_147203 | | | |
| | | | NM_201552 | | | |
| | | | NM_201553 | | | |
| AU157716 | 227051_at | AU157716 | --- | --- | -3.23 | 7.84E-06 |
| TMIE | 1553601_a_at | NM_147196 | NM_147196 | 259236 | -3.22 | 3.71E-05 |
| IGSF4D | 1552752_a_at | NM_153184 | NM_153184 | 253559 | -3.21 | 2.78E-04 |
| PAPPA | 224941_at | BF107618 | NM_002581 | 5069 | -3.15 | 3.66E-05 |
| CFC1 | 223753_s_at | AF312769 | NM_032545 | 55997 | -3.15 | 1.39E-05 |
| PAPPA | 228128_x_at | AI110886 | NM_002581 | 5069 | -3.07 | 9.98E-07 |
| AA452540 | 232411_at | AA452540 | --- | --- | -3.05 | 1.28E-04 |
| AW003173 | 230746_s_at | AW003173 | --- | --- | -3.03 | 3.62E-03 |
| PAPPA | 201981_at | AA148534 | NM_002581 | 5069 | -3.03 | 2.64E-07 |
| BF432956 | 229057_at | BF432956 | --- | --- | -3.01 | 5.34E-06 |
| IL7 | 206693_at | NM_000880 | NM_000880 | 3574 | -3.01 | 6.79E-05 |
| N92599 | 241421_at | N92599 | --- | --- | -3.01 | 2.03E-05 |
| SAMD4 | 212845_at | AB028976 | NM_015589 | 23034 | -2.94 | 1.71E-04 |
| N95363 | 213183_s_at | N95363 | --- | --- | -2.94 | 7.60E-03 |
| PPAPDC1 | 236044_at | BF130943 | XM_113641 | 196051 | -2.94 | 8.60E-08 |
| DKFZP564I0422 | 223588_at | AL136607 | NM_031435 | 83591 | -2.93 | 1.23E-03 |
| KCNMA1 | 221584_s_at | U11058 | NM_002247 | 3778 | -2.92 | 4.15E-06 |
| AI685824 | 244565_at | AI685824 | XM_370580 | 387716 | -2.91 | 4.49E-04 |
| LOC56901 | 218484_at | NM_020142 | NM_020142 | 56901 | -2.88 | 8.77E-04 |
| BACE2 | 217867_x_at | NM_012105 | NM_012105 | 25825 | -2.87 | 7.40E-04 |
| | | | | | | |
| | | | NM_138992 | | | |
| BF110050 | 241100_at | BF110050 | --- | --- | -2.81 | 1.52E-03 |
| AU144437 | 232887_at | AU144437 | --- | --- | -2.81 | 4.16E-03 |
| GABRA5 | 206456_at | NM_000810 | NM_000810 | 2558 | -2.79 | 1.41E-03 |
| SYNPR | 230303_at | H11380 | NM_144642 | 132204 | -2.77 | 1.02E-03 |
| CYP2E1 | 209975_at | AF182276 | NM_000773 | 1571 | -2.76 | 3.21E-03 |
| GAL | 214240_at | AL556409 | NM_015973 | 51083 | -2.75 | 1.20E-03 |
| LOC51315 | 222585_x_at | BE326678 | NM_016618 | 51315 | -2.72 | 1.70E-05 |
| CDH12 | 207149_at | L33477 | NM_004061 | 1010 | -2.72 | 1.51E-05 |
| N39597 | 1555993_at | N39597 | --- | --- | -2.70 | 1.39E-03 |
| SYNPO2 | 227662_at | AA541622 | NM_133477 | 171024 | -2.69 | 4.62E-05 |
| NPY | 206001_at | NM_000905 | NM_000905 | 4852 | -2.68 | 9.89E-05 |
| FGF19 | 223761_at | AF110400 | NM_005117 | 9965 | -2.65 | 8.85E-06 |
| SLC35F3 | 229065_at | BF968270 | NM_173508 | 148641 | -2.64 | 8.85E-06 |
| SSPN | 204963_at | AL_136756 | NM_005086 | 8082 | -2.63 | 1.78E-03 |
| CYP2E1 | 209976_s_at | AF182276 | NM_000773 | 1571 | -2.63 | 7.81E-04 |
| MGC13040 | 224463_s_at | BC006128 | NM_032930 | 85016 | -2.62 | 4.90E-03 |
| ADCY1 | 213245_at | AL120173 | NM_021116 | 107 | -2.61 | 5.07E-04 |
| PAPPA | 201982_s_at | NM_002581 | NM_002581 | 5069 | -2.60 | 4.39E-03 |
| ERBB4 | 206794_at | NM_005235 | NM_005235 | 2066 | -2.60 | 3.50E-03 |
| AI826125 | 229866_at | AI826125 | --- | --- | -2.60 | 1.64E-04 |
| DKK1 | 204602_at | NM_012242 | NM_012242 | 22943 | -2.56 | 1.78E-04 |
| BC020585 | 1568817_at | BC020585 | --- | --- | -2.56 | 2.42E-05 |
| COL5A1 | 212488_at | N30339 | NM_000093 | 1289 | -2.55 | 6.89E-04 |
| PTPRR | 206084_at | NM_002849 | NM_002849 | 5801 | -2.54 | 2.83E-04 |
| | | | NM_130846 | | | |
| BACE2 | 222446_s_at | AF178532 | NM_012105 | 25825 | -2.54 | 8.21E-05 |
| | | | NM_138991 | | | |
| | | | NM_138992 | | | |
| P2RX5 | 210448_s_at | U49396 | NM_002561 | 5026 | -2.50 | 4.92E-03 |
| | | | NM_175080 | | | |
| | | | NM_175081 | | | |
| MBD5 | 227839_at | AI911379 | NM_018328 | 55777 | -2.49 | 1.78E-03 |
| AA243659 | 209683_at | AA243659 | --- | --- | -2.44 | 6.66E-07 |
| LOC51315 | 233329_s_at | AK025986 | NM_016618 | 51315 | -2.44 | 4.58E-05 |
| KIAA0774 | 214961_at | AI818409 | XM_166270 | 23281 | -2.43 | 3.12E-05 |
| DDAH1 | 209094_at | AL078459 | NM_012137 | 23576 | -2.43 | 1.54E-04 |
| FLJ20701 | 219093_at | NM_017933 | NM_017933 | 55022 | -2.43 | 3.02E-03 |
| ICAM2 | 204683_at | NM_000873 | NM_000873 | 3384 | -2.41 | 3.71E-04 |
| A1051127 | 231478_at | A1051127 | --- | --- | -2.41 | 3.13E-03 |
| LOC134466 | 244289_at | AI242408 | XM_376436 | 134466 | -2.40 | 8.79E-03 |
| AI767727 | 229815_at | AI767727 | --- | --- | -2.40 | 5.54E-04 |
| BF591692 | 231532_at | BF591692 | --- | --- | -2.39 | 6.45E-04 |
| GRM5 | 214217_at | D60132 | NM_000842 | 2915 | -2.39 | 1.63E-06 |
| ERBB4 | 214053_at | AW772192 | NM_005235 | 2066 | -2.39 | 1.44E-04 |
| AA854843 | 236777_at | AA854843 | --- | --- | -2.38 | 3.59E-03 |
| DKFZp313N062 | 1555905_a_at | AI147556 | NM_173826 | 285343 | -2.38 | 1.45E-03 |
| LOC128153 | 230763_at | AA905508 | NM_138796 | 128153 | -2.38 | 2.95E-03 |
| ANK3 | 206385_s_at | NM_020987 | NM_001149 | 288 | -2.37 | 4.86E-04 |
| | | | NM_020987 | | | |
| AI810266 | 235456_at | AI810266 | --- | --- | -2.36 | 7.31E-07 |
| LOC390916 | 235384_at | AW963217 | XM_372723 | 390916 | -2.36 | 8.86E-07 |
| AV719355 | 217554_at | AV719355 | --- | --- | -2.36 | 2.61E-03 |
| KIAA0774 | 1554755_a_at | BC032481 | XM_166270 | 23281 | -2.36 | 1.10E-03 |
| HHLA3 | 220387_s_at | NM_007071 | NM_007071 | 11147 | -2.36 | 2.06E-03 |
| SMARCA2 | 206542_s_at | AV725365 | NM_003070 | 6595 | -2.35 | 7.68E-04 |
| | | | NM_139045 | | | |
| CNGA3 | 207261_at | NM_001298 | NM_001298 | 1261 | -2.32 | 3.40E-05 |
| FAM49A | 230276_at | AI934342 | NM_030797 | 81553 | -2.30 | 9.19E-08 |
| LOC339290 | 217506_at | H49382 | XM_375527 | 339290 | -2.30 | 8.85E-05 |
| HTATIP2 | 209448_at | BC002439 | NM_006410 | 10553 | -2.30 | 6.50E-03 |
| FLJ31659 | 1553710_at | NM_153027 | NM_153027 | 152756 | -2.30 | 6.35E-06 |
| LOC51315 | 218303_x_at | NM_016618 | NM_016618 | 51315 | -2.30 | 8.49E-07 |
| ATRNL1 | 213744_at | AI817331 | NM_207303 | 26033 | -2.29 | 8.46E-08 |
| SMARCA2 | 206544_x_at | NM_003070 | NM_003070 | 6595 | -2.27 | 4.88E-04 |
| | | | NM_139045 | | | |
| GAL | 207466_at | NM_015973 | NM_015973 | 51083 | -2.25 | 6.61E-13 |
| PTGER2 | 206631_at | NM_000956 | NM_000956 | 5732 | -2.25 | 2.22E-05 |
| PTPRR | 210675_s_at | U77917 | NM_002849 | 5801 | -2.23 | 8.91E-04 |
| | | | NM_130846 | | | |
| BF000047 | 235736_at | BF000047 | --- | --- | -2.22 | 1.49E-04 |
| TICAM2 | 228234_at | AI423165 | NM_021649 | 353376 | -2.22 | 4.67E-04 |
| NCAM2 | 205669_at | NM_004540 | NM_004540 | 4685 | -2.22 | 2.95E-06 |
| IRS1 | 204686_at | NM_005544 | NM_005544 | 3667 | -2.22 | 2.74E-03 |
| TRIM61 | 238990_x_at | AI763262 | XM_373038 | 391712 | -2.21 | 1.88E-04 |
| AF085995 | 1561759_at | AF085995 | --- | --- | -2.21 | 9.13E-03 |
| AI732969 | 240173_at | AI732969 | --- | --- | -2.20 | 1.87E-05 |
| AI819043 | 229228_at | AI819043 | --- | --- | -2.20 | 4.58E-04 |
| BF432875 | 235782_at | BF432875 | XM_497668 | 388620 | -2.20 | 1.92E-05 |
| GPX7 | 213170_at | AA406605 | NM_015696 | 2882 | -2.19 | 2.99E-04 |
| NLF1 | 241031_at | BE218239 | NM_207322 | 145741 | -2.18 | 8.81E-05 |
| BE674309 | 230913_at | BE674309 | --- | --- | -2.18 | 1.83E-04 |
| CAP2 | 212551_at | NM_006366 | NM_006366 | 10486 | -2.17 | 5.50E-04 |
| PRO1580 | 213550_s_at | AA993683 | NM_018502 | 55374 | -2.17 | 4.95E-03 |
| CYB5R2 | 220230_s_at | NM_016229 | NM_00100133 6 | 51700 | -2.15 | 7.15E-04 |
| | | | NM_016229 | | | |
| ZAP128 | 202982_s_at | NM_006821 | NM_006821 | 10965 | -2.14 | 1.68E-03 |
| RAC2 | 207419_s_at | NM_002872 | NM_002872 | 5880 | -2.14 | 2.67E-03 |
| LOC153682 | 232794_at | AL137383 | --- | 153682 | -2.13 | 5.45E-03 |
| GRIA2 | 236538_at | BE219628 | NM_000826 | 2891 | -2.13 | 1.05E-03 |
| IL20RA | 219115_s_at | NM_014432 | NM_014432 | 53832 | -2.12 | 6.78E-03 |
| SCN5A | 207413_s_at | NM_000335 | NM_000335 | 6331 | -2.12 | 2.22E-04 |
| | | | NM_198056 | | | |
| TSAP6 | 218424_s_at | NM_018234 | NM_00100841 0 | 55240 | -2.12 | 1.68E-04 |
| | | | NM_018234 | | | |
| | | | NM_182915 | | | |
| FAM49A | 208092_s_at | NM_030797 | NM_030797 | 81553 | -2.11 | 4.87E-05 |
| HIST2H2BE | 202708_s_at | NM_003528 | NM_003528 | 8349 | -2.10 | 5.97E-05 |
| GLI2 | 207034_s_at | NM_030379 | NM_005270 | 2736 | -2.09 | 5.75E-03 |
| | | | NM_030379 | | | |
| | | | NM_030380 | | | |
| | | | NM_030381 | | | |
| LOC441422 | 236453_at | AW243154 | XR_000264 | 441422 | -2.09 | 2.02E-04 |
| SYNPO2 | 225720_at | AW009747 | NM_133477 | 171024 | -2.09 | 6.04E-03 |
| LEPR | 209894_at | U50748 | NM_00100367 9 | 3953 | -2.09 | 3.79E-04 |
| | | | NM_00100368 0 | | | |
| | | | NM_002303 | | | |
| HNRPR | 232004_at | AK001846 | NM_005826 | 10236 | -2.09 | 1.55E-05 |
| AI222435 | 230319_at | AI222435 | --- | --- | -2.09 | 7.23E-05 |
| MGC15668 | 229332_at | AI653050 | NM_032756 | 84842 | -2.08 | 1.02E-03 |
| SCAP1 | 1560861_at | BG210619 | NM_003726 | 8631 | -2.08 | 5.32E-04 |
| LRIG1 | 211596_s_at | AB050468 | NM_015541 | 26018 | -2.08 | 7.64E-03 |
| SCN3A | 210432_s_at | AF225986 | NM_006922 | 6328 | -2.07 | 3.67E-03 |
| ADARB1 | 203865_s_at | NM_015833 | NM_001112 | 104 | -2.07 | 4.67E-06 |
| | | | NM_015833 | | | |
| | | | NM_015834 | | | |
| AB051467 | 232738_at | AB051467 | --- | --- | -2.06 | 1.71E-05 |
| CREB5 | 205931_s_at | NM_004904 | NM_004904 | 9586 | -2.06 | 8.68E-04 |
| | | | NM_182898 | | | |
| | | | NM_182899 | | | |
| ECHDC3 | 219298_at | NM_024693 | NM_024693 | 79746 | -2.05 | 6.42E-03 |
| CRYGD | 207532_at | NM_006891 | NM_006891 | 1421 | -2.05 | 2.10E-03 |
| ICAM2 | 213620_s_at | AA126728 | NM_000873 | 3384 | -2.05 | 3.74E-04 |
| CIP29 | 217498_at | BG389073 | NM_033082 | 84324 | -2.04 | 3.37E-04 |
| AA700440 | 228329_at | AA700440 | --- | --- | -2.04 | 4.62E-07 |
| ATRNL1 | 213745_at | AW151108 | NM_207303 | 26033 | -2.04 | 9.28E-06 |
| SLC6A15 | 239352_at | AI368909 | NM_018057 | 55117 | -2.03 | 1.76E-04 |
| | | | NM_182767 | | | |
| RGAG4 | 227823_at | BE348679 | XM_291322 | 340526 | -2.03 | 2.13E-04 |
| AW136654 | 240802_at | AW136654 | --- | --- | -2.02 | 2.07E-03 |
| MTL5 | 219786_at | NM_004923 | NM_004923 | 9633 | -2.02 | 3.30E-04 |
| FBI4 | 1562953_s_at | BC019264 | NM_205857 | 404201 | -2.02 | 4.82E-03 |
| ZNF91 | 238466_at | R43486 | NM_003430 | 7644 | -2.01 | 8.53E-04 |
| LOC441168 | 229390_at | AV734646 | XM_496823 | 441168 | -2.01 | 1.83E-04 |
| BF343672 | 227925_at | BF343672 | XM_378321 | 399972 | -2.01 | 1.32E-04 |
| BC014056 | 1561906_at | BC014056 | --- | --- | -2.01 | 8.02E-04 |
| FGF18 | 206987_x_at | NM_003862 | NM_003862 | 8817 | -2.01 | 6.16E-03 |
| | | | NM_033649 | | | |
| CHST7 | 206756_at | NM_019886 | NM_019886 | 56548 | -2.00 | 1.29E-04 |
| RAC2 | 213603_s_at | BE138888 | NM_002872 | 5880 | -2.00 | 2.89E-05 |
| MCTP1 | 235740_at | BG250585 | NM_00100279 6 | 79772 | -2.00 | 2.38E-08 |
| | | | NM_024717 | | | |
| C6orf198 | 227834_at | AL589605 | XM_371849 | 167838 | -2.00 | 1.53E-03 |
| SYNPO2 | 225894_at | AL589603 | NM_133477 | 171024 | -1.99 | 3.38E-04 |
| FBI4 | 238081_at | AI694300 | NM_205857 | 404201 | -1.99 | 1.05E-03 |
| GABRA5 | 215531_s_at | BF966183 | NM_000810 | 2558 | -1.98 | 6.28E-06 |
| SORCS2 | 228720_at | AB037750 | NM_020777 | 57537 | -1.98 | 7.96E-05 |
| MRAS | 225185_at | BF343625 | NM_012219 | 22808 | -1.97 | 6.31E-04 |
| BE962770 | 229032_at | BE962770 | --- | --- | -1.97 | 6.94E-03 |
| ATP2B3 | 242036_x_at | H09073 | NM_00100134 4 | 492 | -1.96 | 1.23E-03 |
| | | | NM_021949 | | | |
| SSPN | 226932_at | AW467136 | NM_005086 | 8082 | -1.95 | 4.20E-05 |
| AA976778 | 230679_at | AA976778 | --- | --- | -1.95 | 3.35E-04 |
| LOC285878 | 1560692_at | BC037834 | --- | 285878 | -1.94 | 3.90E-04 |
| BF114967 | 231067_s_at | BF114967 | --- | --- | -1.93 | 1.31E-04 |
| C10orf59 | 223824_at | BC005364 | NM_018363 | 55328 | -1.93 | 8.06E-03 |
| ASAM | 228082_at | BF056275 | NM_024769 | 79827 | -1.93 | 2.50E-04 |
| OXTR | 206825_at | NM_000916 | NM_000916 | 5021 | -1.93 | 2.27E-04 |
| CNNM1 | 220166_at | NM_020348 | NM_020348 | 26507 | -1.92 | 1.23E-04 |
| PLCD4 | 224505_s_at | BC006355 | NM_032726 | 84812 | -1.92 | 2.15E-05 |
| NEFH | 33767_at | X15306 | NM_021076 | 4744 | -1.92 | 2.20E-03 |
| LOC339290 | 228160_at | AI433706 | XM_375527 | 339290 | -1.92 | 5.10E-03 |
| EDG7 | 231192_at | AW274018 | NM_012152 | 23566 | -1.91 | 7.48E-04 |
| BF002625 | 229530_at | BF002625 | --- | --- | -1.90 | 4.09E-04 |
| COL13A1 | 211343_s_at | M33653 | NM_005203 | 1305 | -1.90 | 5.01E-06 |
| | | | NM_080798 | | | |
| | | | NM_080799 | | | |
| | | | NM_080800 | | | |
| | | | NM_080801 | | | |
| | | | NM_080802 | | | |
| | | | NM_080803 | | | |
| | | | NM_080804 | | | |
| | | | NM_080805 | | | |
| | | | NM_080806 | | | |
| | | | NM_080807 | | | |
| | | | NM_080808 | | | |
| | | | NM_080809 | | | |
| | | | NM_080810 | | | |
| | | | NM_080811 | | | |
| | | | NM_080812 | | | |
| | | | NM_080813 | | | |
| | | | NM_080814 | | | |
| | | | NM_080815 | | | |
| A1015847 | 1568838_at | AI015847 | --- | --- | -1.90 | 6.44E-04 |
| AA705933 | 239866_at | AA705933 | --- | --- | -1.89 | 7.57E-04 |
| NDFIP2 | 1568807_a_at | AI301081 | NM_019080 | 54602 | -1.89 | 6.50E-05 |
| PRKACB | 202742_s_at | NM_002731 | NM_002731 | 5567 | -1.88 | 2.83E-04 |
| | | | NM_182948 | | | |
| | | | NM_207578 | | | |
| FLJ20449 | 1570153_at | BC013944 | NM_017826 | 54937 | -1.88 | 1.81E-03 |
| FSTL1 | 208782_at | BC000055 | NM_007085 | 11167 | -1.88 | 4.35E-03 |
| PRSS11 | 201185_at | NM_002775 | NM_002775 | 5654 | -1.87 | 2.45E-04 |
| EBF | 229487_ at | W73890 | NM_024007 | 1879 | -1.86 | 2.25E-05 |
| SLC16A14 | 238029_s_at | R15072 | NM_152527 | 151473 | -1.86 | 2.63E-05 |
| LEPR | 211356_x_at | U66495 | NM_00100367 9 | 3953 | -1.86 | 9.14E-08 |
| | | | NM_00100368 0 | | | |
| | | | NM_002303 | | | |
| LOC339290 | 215283_at | U79248 | XM_375527 | 339290 | -1.85 | 3.14E-04 |
| BE672499 | 230962_at | BE672499 | --- | --- | -1.85 | 3.97E-04 |
| AI435590 | 237193_s_at | AI435590 | --- | --- | -1.84 | 4.11E-05 |
| C10orf10 | 209183_s_at | AL136653 | NM_007021 | 11067 | -1.84 | 9.62E-05 |
| ARTS-1 | 214012_at | BE551138 | NM_016442 | 51752 | -1.84 | 7.70E-04 |
| M160 | 223655_at | AF264014 | NM_174941 | 283316 | -1.83 | 4.27E-03 |
| AI803010 | 239503_at | AI803010 | --- | --- | -1.83 | 6.21E-03 |
| GLI2 | 228537_at | AI797248 | NM_005270 | 2736 | -1.83 | 6.30E-04 |
| | | | NM_030379 | | | |
| | | | NM_030380 | | | |
| | | | NM_030381 | | | |
| AI935541 | 236220_at | AI935541 | --- | --- | -1.83 | 8.96E-04 |
| HEBP2 | 203430_at | NM_014320 | NM_014320 | 23593 | -1.82 | 6.64E-03 |
| DKFZp313N062 | 226688_at | AW003508 | NM_173826 | 285343 | -1.82 | 8.96E-06 |
| EBF | 232204_at | AF208502 | NM_024007 | 1879 | -1.81 | 6.25E-06 |
| NT5E | 203939_at | NM_002526 | NM_002526 | 4907 | -1.81 | 2.34E-05 |
| D4S234E | 209570_s_at | BC001745 | NM_014392 | 27065 | -1.80 | 1.14E-04 |
| LOC133308 | 229491_at | BF433180 | NM_178833 | 133308 | -1.79 | 1.59E-03 |
| BHLHB2 | 201170_s_at | NM_003670 | NM_003670 | 8553 | -1.79 | 7.17E-03 |
| AI768374 | 228661_s_at | AI768374 | --- | --- | -1.79 | 9.50E-04 |
| AL355688 | 232081_at | AL355688 | --- | --- | -1.79 | 5.36E-03 |
| RPL39L | 210115_at | L05096 | NM_052969 | 116832 | -1.79 | 6.47E-03 |
| BCL2 | 203685_at | NM_000633 | NM_000633 | 596 | -1.78 | 4.07E-05 |
| | | | NM_000657 | | | |
| LRBA | 214109_at | AI659561 | NM_006726 | 987 | -1.78 | 1.89E-03 |
| C9orf150 | 227443_at | AI972386 | NM_203403 | 286343 | -1.78 | 2.50E-04 |
| KCNAB1 | 210078_s_at | L39833 | NM_003471 | 7881 | -1.78 | 2.11E-03 |
| | | | NM_172159 | | | |
| | | | NM_172160 | | | |
| PCDHB16 | 232099_at | AB046841 | | 57717 | -1.77 | 1.97E-03 |
| | | | NM_020957 | | | |
| SYNPO2 | 225895_at | AI634580 | NM_133477 | 171024 | -1.77 | 6.20E-04 |
| AK000776 | 232060_at | AK000776 | --- | --- | -1.75 | 1.44E-04 |
| COL6A1 | 213428_s_at | AA292373 | NM_001848 | 1291 | -1.75 | 5.72E-04 |
| LEPR | 211355_x_at | U52914 | NM_00100367 9 | 3953 | -1.74 | 2.06E-06 |
| | | | NM_00100368 0 | | | |
| | | | NM_002303 | | | |
| PABPC5 | 233136_at | AL122118 | NM_080832 | 140886 | -1.74 | 7.51E-04 |
| PROM1 | 204304_s_at | NM_006017 | NM_006017 | 8842 | -1.74 | 6.03E-04 |
| AGPAT3 | 223182_s_at | AI337300 | NM_020132 | 56894 | -1.73 | 3.62E-04 |
| ANK3 | 209442_x_at | AL136710 | NM_001149 | 288 | -1.73 | 1.10E-03 |
| | | | NM_020987 | | | |
| CACNA1D | 210108_at | BE550599 | NM_000720 | 776 | -1.72 | 3.20E-04 |
| H2AFJ | 220936_s_at | NM_018267 | NM_018267 | 55766 | -1.71 | 9.12E-04 |
| | | | NM_177925 | | | |
| LOC285878 | 230117_at | AI420977 | --- | 285878 | -1.71 | 1.65E-04 |
| MGC70870 | 244125_at | AA837131 | NM_203481 | 403340 | -1.70 | 1.71E-03 |
| LDB2 | 206481_s_at | NM_001290 | NM_001290 | 9079 | -1.70 | 9.86E-03 |
| AI004009 | 222288_at | AI004009 | XM_371647 | 389129 | -1.70 | 6.64E-04 |
| OPRM1 | 207989_at | NM_000914 | NM_000914 | 4988 | -1.69 | 1.26E-05 |
| | | | NM_00100850 3 | | | |
| | | | NM_00100850 4 | | | |
| | | | NM_00100850 5 | | | |
| OPN3 | 224392_s_at | AF303588 | NM_014322 | 23596 | -1.69 | 6.03E-04 |
| FGD5 | 226985_at | AW269340 | NM_152536 | 152273 | -1.68 | 4.08E-06 |
| EBF | 227646_at | BG435302 | NM_024007 | 1879 | -1.68 | 1.59E-04 |
| KIAA0500 | 213839_at | AW028110 | --- | 57237 | -1.68 | 4.44E-03 |
| BF342356 | 238934_at | BF342356 | --- | --- | -1.68 | 6.92E-03 |
| PRKCH | 218764_at | NM_024064 | NM_006255 | 5583 | -1.67 | 7.79E-05 |
| ARSD | 223696_at | BC003660 | NM_001669 | 414 | -1.66 | 9.99E-03 |
| | | | NM_009589 | | | |
| C9orf94 | 239909_at | AI937348 | NM_152702 | 206938 | -1.66 | 7.20E-03 |
| C6orf155 | 226810_at | BE500942 | NM_024882 | 79940 | -1.65 | 2.05E-03 |
| LOC151194 | 1553743_at | NM_145280 | NM_145280 | 151194 | -1.65 | 6.80E-03 |
| SYNPO2 | 225721_at | AI658662 | NM_133477 | 171024 | -1.64 | 9.76E-03 |
| AL037414 | 230055_at | AL037414 | --- | --- | -1.64 | 3.29E-04 |
| AW572379 | 229569_at | AW572379 | --- | --- | -1.63 | 2.80E-04 |
| FRMD4B | 213056_at | AU145019 | XM_114303 | 23150 | -1.63 | 2.55E-03 |
| D4S234E | 213533_at | M98528 | NM_014392 | 27065 | -1.63 | 1.82E-06 |
| PTP4A3 | 209695_at | BC003105 | NM_007079 | 11156 | -1.62 | 4.14E-06 |
| | | | NM_032611 | | | |
| ACYP2 | 206833_s_at | NM_001108 | NM_138448 | 98 | -1.62 | 7.71E-04 |
| MCTP1 | 220122_at | NM_024717 | NM_00100279 6 | 79772 | -1.62 | 6.95E-05 |
| | | | NM_024717 | | | |
| ALDH1A2 | 207016_s_at | AB015228 | NM_003888 | 8854 | -1.61 | 4.22E-04 |
| | | | NM_170696 | | | |
| | | | NM_170697 | | | |
| CD302 | 203799_at | NM_014880 | NM_014880 | 9936 | -1.61 | 1.17E-05 |
| F10269 | 1557746_at | F10269 | --- | --- | -1.61 | 1.03E-03 |
| FLJ20152 | 218532_s_at | NM_019000 | NM_019000 | 54463 | -1.61 | 2.67E-03 |
| FBI4 | 241401_at | BG496631 | NM_205857 | 404201 | -1.60 | 1.55E-03 |
| BC031271 | 1569785_at | BC031271 | --- | --- | -1.60 | 4.07E-04 |
| BE046461 | 215303_at | BE046461 | --- | --- | -1.60 | 5.17E-04 |
| FLJ10970 | 219230_at | NM_018286 | NM_018286 | 55273 | -1.59 | 1.10E-04 |
| CA336272 | 1558512_at | CA336272 | --- | --- | -1.59 | 1.57E-04 |
| PRKACB | 202741_at | AA130247 | NM_002731 | 5567 | -1.59 | 1.12E-03 |
| | | | NM_182948 | | | |
| | | | NM_207578 | | | |
| PDE4DIP | 213388_at | H15535 | NM_00100281 0 | 9659 | -1.59 | 6.05E-04 |
| | | | NM_00100281 1 | | | |
| | | | NM_00100281 2 | | | |
| | | | NM_014644 | | | |
| | | | NM_022359 | | | |
| DKFZp762H185 | 226657_at | H27948 | --- | 256306 | -1.59 | 9.18E-03 |
| ST18 | 206135_at | NM_014682 | NM_014682 | 9705 | -1.58 | 4.56E-07 |
| DBT | 231919_at | AK024946 | NM_001918 | 1629 | -1.58 | 1.11E-03 |
| STMN2 | 203001_s_at | NM_007029 | NM_007029 | 11075 | -1.58 | 5.47E-03 |
| SIX2 | 206510_at | AF332197 | NM_016932 | 10736 | -1.56 | 2.79E-05 |
| NEBL | 203961_at | AL157398 | NM_006393 | 10529 | -1.56 | 7.89E-03 |
| | | | NM_213569 | | | |
| PCTK3 | 214797_s_at | BC000281 | NM_002596 | 5129 | -1.56 | 8.81E-03 |
| | | | NM_212502 | | | |
| | | | NM_212503 | | | |
| DOC1 | 1554966_a_at | AF329092 | NM_014890 | 11259 | -1.56 | 5.32E-04 |
| | | | NM_182909 | | | |
| AI800713 | 235122_at | AI800713 | --- | --- | -1.56 | 7.94E-04 |
| NEFH | 204412_s_at | NM_021076 | NM_021076 | 4744 | -1.55 | 3.87E-03 |
| MEGF11 | 1552439_s_at | NM_032445 | NM_032445 | 84465 | -1.55 | 7.12E-04 |
| AI796536 | 229810_at | AI796536 | --- | --- | -1.55 | 6.60E-04 |
| MGC11332 | 213393_at | AI767210 | NM_032718 | 84804 | -1.54 | 1.06E-04 |
| RBMS3 | 237860_at | AI821998 | NM_00100379 2 | 27303 | -1.54 | 9.15E-03 |
| | | | NM_00100379 3 | | | |
| | | | NM_014483 | | | |
| LOC285878 | 236308_at | D60436 | --- | 285878 | -1.53 | 2.60E-04 |
| LRRN6A | 227933_at | AI193252 | NM_032808 | 84894 | -1.53 | 5.09E-06 |
| NHS | 228933_at | BF111819 | NM_198270 | 4810 | -1.53 | 3.83E-03 |
| EYA1 | 214608_s_at | AJ000098 | NM_000503 | 2138 | -1.53 | 5.85E-05 |
| | | | NM_172058 | | | |
| | | | NM_172059 | | | |
| | | | NM_172060 | | | |
| AI263044 | 214376_at | AI263044 | --- | --- | -1.53 | 1.69E-04 |
| ANKH | 220076_at | NM_019847 | NM_054027 | 56172 | -1.52 | 2.18E-03 |
| FAM38B | 219602_s_at | NM_022068 | NM_022068 | 63895 | -1.52 | 3.24E-04 |
| LOC133308 | 1564746_at | BC009732 | NM_178833 | 133308 | -1.52 | 3.24E-03 |
| AA747287 | 242273_at | AA747287 | --- | --- | -1.52 | 3.10E-04 |
| IL10RB | 209575_at | BC001903 | NM_000628 | 3588 | -1.52 | 4.38E-05 |
| BACE1 | 217904_s_at | NM_012104 | NM_012104 | 23621 | -1.51 | 1.09E-04 |
| | | | NM_138971 | | | |
| | | | NM_138972 | | | |
| | | | NM_138973 | | | |
| DISP1 | 228184_at | AK023679 | NM_032890 | 84976 | -1.51 | 7.96E-03 |
| KIAA1277 | 221886_at | AL037701 | NM_015689 | 27147 | -1.51 | 1.64E-03 |
| AI276956 | 230766_at | AI276956 | --- | --- | -1.51 | 4.00E-04 |
| FAM13C1 | 1554547_at | BC036453 | NM_00100197 1 | 220965 | -1.51 | 4.02E-03 |
| | | | NM_198215 | | | |
| AKAP12 | 227529_s_at | BF511276 | NM_005100 | 9590 | -1.50 | 2.52E-04 |
| | | | NM_144497 | | | |
| POSTN | 1555778_a_at | AY140646 | NM_006475 | 10631 | -1.50 | 1.94E-04 |
| BF114745 | 235459_at | BF114745 | --- | --- | -1.50 | 2.88E-03 |
| TNFSF4 | 207426_s_at | NM_003326 | NM_003326 | 7292 | -1.49 | 1.01E-03 |
| STMN2 | 203000_at | BF967657 | NM_007029 | 11075 | -1.49 | 4.14E-03 |
| NR2F1 | 209505_at | AI951185 | NM_005654 | 7025 | -1.49 | 3.03E-04 |
| PTN | 209466_x_at | M57399 | NM_002825 | 5764 | -1.49 | 6.92E-06 |
| AI821995 | 240201_at | AI821995 | --- | --- | -1.49 | 2.74E-03 |
| MGC11332 | 213403_at | BF223370 | NM_032718 | 84804 | -1.49 | 1.36E-03 |
| KCNJ6 | 210454_s_at | U24660 | NM_002240 | 3763 | -1.49 | 5.76E-03 |
| MGC15730 | 227154_at | AL566367 | NM_032880 | 84966 | -1.48 | 5.19E-03 |
| T87730 | 242500_at | T87730 | --- | --- | -1.48 | 1.37E-05 |
| LAF4 | 227198_at | AW085505 | NM_002285 | 3899 | -1.48 | 1.11E-04 |
| AL137538 | 232679_at | AL137538 | --- | --- | -1.48 | 6.18E-03 |
| TOX | 204530_s_at | NM_014729 | NM_014729 | 9760 | -1.48 | 2.48E-03 |
| SB145 | 232283_at | AL122088 | NM_212551 | 388695 | -1.48 | 8.90E-03 |
| PPP1R14A | 227006_at | AA156998 | NM_033256 | 94274 | -1.47 | 1.25E-04 |
| CRABP2 | 202575_at | NM_001878 | NM_001878 | 1382 | -1.47 | 1.13E-03 |
| AV723260 | 1560425_s_at | AV723260 | --- | --- | -1.47 | 8.62E-03 |
| D4S234E | 209569_x_at | NM_014392 | NM_014392 | 27065 | -1.47 | 3.74E-05 |
| CDKN1C | 213348_at | N33167 | NM_000076 | 1028 | -1.47 | 5.81E-05 |
| LOC148418 | 229402_at | AI631824 | NM_001010971 | 148418 | -1.46 | 5.19E-04 |
| DKFZP434H132 | 204494_s_at | AW516789 | NM_015492 | 56905 | -1.46 | 5.88E-04 |
| ZNF528 | 215019_x_at | AW474158 | NM_032423 | 84436 | -1.46 | 4.79E-07 |
| GCH1 | 204224_s_at | NM_000161 | NM_000161 | 2643 | -1.46 | 8.78E-04 |
| PCSK1 | 205825_at | NM_000439 | NM_000439 | 5122 | -1.46 | 1.15E-03 |
| WDR9 | 225446_at | AI638279 | NM_00100724 6 | 54014 | -1.44 | 6.32E-05 |
| | | | NM_018963 | | | |
| | | | NM_033656 | | | |
| DOC1 | 204135_at | NM_014890 | NM_014890 | 11259 | -1.44 | 6.57E-03 |
| | | | NM_182909 | | | |
| NDRG1 | 200632_s_at | NM_006096 | NM_006096 | 10397 | -1.44 | 6.23E-06 |
| AI028528 | 241804_at | AI028528 | --- | --- | -1.43 | 8.22E-04 |
| LOC253012 | 242601_at | AA600175 | NM_198151 | 253012 | -1.43 | 1.56E-03 |
| DMD | 203881_s_at | NM_004010 | NM_000109 | 1756 | -1.43 | 1.85E-03 |
| | | | NM_004006 | | | |
| | | | NM_004007 | | | |
| | | | NM_004009 | | | |
| | | | NM_004010 | | | |
| | | | NM_004011 | | | |
| | | | NM_004012 | | | |
| | | | NM_004013 | | | |
| | | | NM_004014 | | | |
| | | | NM_004015 | | | |
| | | | NM_004016 | | | |
| | | | NM_004017 | | | |
| | | | NM_004018 | | | |
| | | | NM_004019 | | | |
| | | | NM_004020 | | | |
| | | | NM_004021 | | | |
| | | | NM_004022 | | | |
| | | | NM_004023 | | | |
| ZFHX1B | 203603_s_at | NM_014795 | NM_014795 | 9839 | -1.42 | 9.52E-03 |
| C21orf86 | 228908_s_at | AW131553 | NM_153454 | 257103 | -1.42 | 1.26E-03 |
| MYT1L | 210016_at | BF223003 | NM_015025 | 23040 | -1.42 | 2.69E-04 |
| C1GALT1C1 | 238989_at | BF749723 | NM_00101155 1 | 29071 | -1.42 | 2.08E-03 |
| | | | NM_152692 | | | |
| LOC402524 | 242206_at | AW590588 | XM_379850 | 402524 | -1.42 | 6.10E-04 |
| CAMK2B | 209956_s_at | U23460 | NM_001220 | 816 | -1.42 | 1.44E-03 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| ST6GALNAC5 | 220979_s_at | NM_030965 | NM_030965 | 81849 | -1.41 | 5.22E-04 |
| AI341823 | 239657_x_at | AI341823 | --- | --- | -1.41 | 2.83E-03 |
| AI733281 | 239994_at | AI733281 | --- | --- | -1.41 | 8.41E-03 |
| SH3GL2 | 205751_at | NM_003026 | NM_003026 | 6456 | -1.41 | 6.60E-04 |
| AW450420 | 236373_at | AW450420 | --- | --- | -1.41 | 5.40E-03 |
| PH-4 | 222125_s_at | BC000580 | NM_017732 | 54681 | -1.41 | 2.61E-03 |
| | | | NM_177938 | | | |
| | | | NM_177939 | | | |
| CDKN2A | 209644_x_at | U38945 | NM_000077 | 1029 | -1.40 | 1.98E-05 |
| | | | NM_058195 | | | |
| | | | NM_058197 | | | |
| AI912571 | 228333_at | AI912571 | --- | --- | -1.40 | 5.33E-03 |
| BF059209 | 240214_at | BF059209 | --- | --- | -1.40 | 2.18E-03 |
| ARSD | 232423_at | AU144083 | NM_001669 | 414 | -1.39 | 1.43E-03 |
| | | | NM_009589 | | | |
| NEDL1 | 243743_at | AI373512 | NM_015052 | 23072 | -1.39 | 2.52E-03 |
| FRAS1 | 226145_s_at | AL157471 | NM_020875 | 80144 | -1.39 | 8.77E-06 |
| | | | NM_025074 | | | |
| | | | NM_032863 | | | |
| | | | NM_206841 | | | |
| UNC5A | 236448_at | R37358 | XM_030300 | 90249 | -1.38 | 1.31E-05 |
| FLJ35808 | 230924_at | AI698574 | NM_173623 | 284076 | -1.38 | 5.69E-04 |
| LOC222070 | 51774_s_at | AW014299 | --- | 222070 | -1.38 | 4.64E-03 |
| C6orf199 | 1552299_at | NM_145025 | NM_145025 | 221264 | -1.38 | 8.50E-03 |
| CAPN2 | 208683_at | M23254 | NM_001748 | 824 | -1.38 | 1.58E-03 |
| BE503598 | 1557658_at | BE503598 | --- | --- | -1.38 | 5.77E-03 |
| HOXD10 | 229400_at | AW299531 | NM_002148 | 3236 | -1.37 | 2.17E-03 |
| GLIS3 | 229435_at | AW025602 | NM_152629 | 169792 | -1.37 | 1.72E-05 |
| NAP1L3 | 204749_at | NM_004538 | NM_004538 | 4675 | -1.37 | 5.99E-03 |
| FLJ45244 | 229227_at | BF511219 | NM_207443 | 400242 | -1.37 | 6.16E-03 |
| D59759 | 231560_at | D59759 | --- | --- | -1.37 | 1.31E-06 |
| ANK2 | 202920_at | BF726212 | NM_001148 | 287 | -1.35 | 3.09E-05 |
| | | | NM_020977 | | | |
| FLJ30655 | 236487_at | AW513286 | NM_144643 | 132320 | -1.35 | 3.30E-03 |
| GLIS3 | 230258_at | AI277316 | NM_152629 | 169792 | -1.35 | 1.33E-03 |
| SH3BGR | 204979_s_at | NM_007341 | NM_00100171 3 | 6450 | -1.34 | 1.12E-04 |
| | | | NM_007341 | | | |
| RAI2 | 219440_at | NM_021785 | NM_021785 | 10742 | -1.34 | 2.50E-04 |
| ATXN1 | 203232_s_at | NM_000332 | NM_000332 | 6310 | -1.34 | 8.99E-05 |
| CRSP2 | 215167_at | BE567032 | NM_004229 | 9282 | -1.34 | 6.26E-03 |
| AI740460 | 236856_x_at | AI740460 | --- | --- | -1.34 | 3.19E-03 |
| MGC42090 | 236705_at | AA017245 | NM_152774 | 256130 | -1.34 | 3.46E-04 |
| SNF1LK | 208078_s_at | NM_030751 | NM_173354 | 150094 | -1.33 | 4.49E-03 |
| N4BP3 | 214775_at | AW139448 | XM_038920 | 23138 | -1.33 | 8.59E-05 |
| AL117598 | 231964_at | AL117598 | --- | --- | -1.33 | 5.64E-06 |
| NT5E | 227486_at | AI086864 | NM_002526 | 4907 | -1.33 | 9.12E-04 |
| AL390170 | 213904_at | AL390170 | --- | --- | -1.33 | 7.47E-04 |
| N50117 | 240236_at | N50117 | --- | --- | -1.33 | 1.78E-03 |
| AK095719 | 1556696_s_at | AK095719 | XM_499008 | 441094 | -1.32 | 6.81E-03 |
| W80446 | 231013_at | W80446 | --- | --- | -1.32 | 9.26E-04 |
| C21orf66 | 239407_at | AI793248 | NM_013329 | 94104 | -1.32 | 1.06E-03 |
| | | | NM_016631 | | | |
| | | | NM_058191 | | | |
| | | | NM_145328 | | | |
| RASGEF1B | 230233_at | BF110534 | NM_152545 | 153020 | -1.32 | 5.98E-03 |
| DCAMKL1 | 205399_at | NM_004734 | NM_004734 | 9201 | -1.32 | 8.09E-04 |
| SCOTIN | 222986_s_at | BC001463 | NM_016479 | 51246 | -1.32 | 7.27E-03 |
| C21orf5 | 205248_at | NM_005128 | NM_005128 | 9980 | -1.32 | 2.95E-04 |
| HEL308 | 228736_at | AW084661 | NM_133636 | 113510 | -1.32 | 6.40E-04 |
| HDAC4 | 228813_at | AW206037 | NM_006037 | 9759 | -1.31 | 4.97E-04 |
| ST7L | 1552739_s_at | NM_138729 | NM_017744 | 54879 | -1.31 | 2.31E-03 |
| | | | NM_138727 | | | |
| | | | NM_138728 | | | |
| | | | NM_138729 | | | |
| | | | NM_198327 | | | |
| | | | NM_198328 | | | |
| DACH2 | 239738_at | AW780006 | NM_053281 | 117154 | -1.30 | 1.49E-03 |
| RAB3-GAP150 | 243851_at | AI860647 | NM_012414 | 25782 | -1.29 | 9.51E-03 |
| C9orf40 | 218904_s_at | NM_017998 | NM_017998 | 55071 | -1.29 | 9.53E-03 |
| AK027226 | 232538_at | AK027226 | --- | --- | -1.29 | 5.59E-03 |
| ZSWIM5 | 232336_at | AI479419 | XM_046581 | 57643 | -1.28 | 1.68E-03 |
| CEBPD | 203973_s_at | NM_005195 | NM_005195 | 1052 | -1.28 | 2.94E-03 |
| AA687479 | 242826_at | AA687479 | --- | --- | -1.28 | 1.21E-04 |
| SIAT7E | 230482_at | AF131837 | NM_030965 | 81849 | -1.28 | 1.28E-03 |
| AI694767 | 229768_at | AI694767 | --- | --- | -1.28 | 5.06E-03 |
| FLJ20753 | 229918_at | AB046860 | XM_371082 | 55036 | -1.28 | 9.34E-04 |
| GUCY1A3 | 221942_s_at | AI719730 | NM_000856 | 2982 | -1.28 | 2.15E-03 |
| PTP4A3 | 206574_s_at | NM_007079 | NM_007079 | 11156 | -1.27 | 9.96E-03 |
| | | | NM_032611 | | | |
| AI354636 | 235171_at | AI354636 | --- | --- | -1.27 | 2.80E-03 |
| STARD13 | 213103_at | AA128023 | NM_052851 | 90627 | -1.27 | 1.35E-05 |
| | | | NM_178006 | | | |
| | | | NM_178007 | | | |
| | | | NM_178008 | | | |
| AKAP12 | 227530_at | BF511276 | NM_005100 | 9590 | -1.27 | 1.01E-04 |
| | | | NM_144497 | | | |
| AI825987 | 235889_at | AI825987 | --- | --- | -1.27 | 9.63E-04 |
| KLF9 | 203543_s_at | NM_001206 | NM_001206 | 687 | -1.27 | 1.17E-03 |
| RAB6B | 221792_at | AW118072 | NM_016577 | 51560 | -1.27 | 2.69E-03 |
| HR | 241355_at | BF528433 | NM_005144 | 55806 | -1.27 | 2.35E-03 |
| | | | NM_018411 | | | |
| BBS7 | 219688_at | NM_018190 | NM_018190 | 55212 | -1.27 | 4.54E-05 |
| | | | NM_176824 | | | |
| BE467383 | 228932_at | BE467383 | XM_497668 | 388620 | -1.27 | 3.12E-03 |
| HPCA | 205454_at | BC001777 | NM_002143 | 3208 | -1.26 | 1.22E-03 |
| KIAA1223 | 225735_at | AL110131 | XM_048747 | 57182 | -1.26 | 2.58E-04 |
| AK021551 | 232589_at | AK021551 | --- | --- | -1.26 | 7.56E-05 |
| BE894882 | 235681_at | BE894882 | --- | --- | -1.26 | 2.96E-05 |
| TOX | 204529_s_at | AI961231 | NM_014729 | 9760 | -1.25 | 8.61E-05 |
| STAR | 204548_at | NM_000349 | NM_000349 | 6770 | -1.25 | 2.94E-05 |
| | | | NM_00100724 3 | | | |
| AL833383 | 1563776_at | AL833383 | --- | --- | -1.25 | 1.41E-03 |
| PPP3CA | 202429_s_at | AL353950 | NM_000944 | 5530 | -1.25 | 1.86E-05 |
| SUCLG2 | 215772_x_at | AL050226 | NM_003848 | 8801 | -1.25 | 6.75E-04 |
| RAB26 | 219562_at | NM_014353 | NM_014353 | 25837 | -1.25 | 6.02E-04 |
| GRIP2 | 216481_at | AF052177 | XM_042936 | 80852 | -1.25 | 7.55E-03 |
| AA135722 | 237419_at | AA135722 | --- | --- | -1.25 | 3.44E-04 |
| PTPRD | 214043_at | BF062299 | NM_002839 | 5789 | -1.25 | 5.77E-06 |
| | | | NM_130391 | | | |
| | | | NM_130392 | | | |
| | | | NM_130393 | | | |
| CDS1 | 226185_at | AK026697 | NM_001263 | 1040 | -1.24 | 5.63E-04 |
| CXorf32 | 229234_at | AW007160 | NM_001010888 | 340554 | -1.24 | 3.78E-03 |
| ACAD8 | 238175_at | AI806909 | NM_014384 | 27034 | -1.24 | 8.68E-03 |
| R71596 | 229580_at | R71596 | --- | --- | -1.24 | 6.01E-03 |
| H46176 | 237083_at | H46176 | --- | --- | -1.24 | 1.64E-03 |
| HPS3 | 227139_s_at | AA219354 | NM_032383 | 84343 | -1.24 | 1.38E-04 |
| PTPRD | 213362_at | N73931 | NM_002839 | 5789 | -1.23 | 1.49E-04 |
| | | | NM_130391 | | | |
| | | | NM_130392 | | | |
| | | | NM_130393 | | | |
| FLJ23861 | 230561_s_at | AA252512 | NM_152519 | 151050 | -1.23 | 2.64E-04 |
| CD200 | 209582_s_at | H23979 | NM_00100419 6 | 4345 | -1.23 | 2.09E-03 |
| | | | NM_00100419 7 | | | |
| | | | NM_005944 | | | |
| SDK2 | 242064_at | N23651 | NM_019064 | 54549 | -1.23 | 5.82E-04 |
| AI051572 | 243310_at | AI051572 | --- | --- | -1.23 | 7.64E-03 |
| GFRA3 | 229936_at | AA694259 | NM_001496 | 2676 | -1.22 | 3.88E-03 |
| SUCLG2 | 212459_x_at | BF593940 | NM_003848 | 8801 | -1.22 | 2.39E-04 |
| POU6F1 | 205878_at | NM_002702 | NM_002702 | 5463 | -1.22 | 2.77E-03 |
| NRP2 | 229225_at | N90777 | NM_003872 | 8828 | -1.22 | 2.35E-03 |
| | | | NM_018534 | | | |
| | | | NM_201264 | | | |
| | | | NM_201266 | | | |
| | | | NM_201267 | | | |
| | | | NM_201279 | | | |
| H2AFJ | 228213_at | AW241813 | NM_018267 | 55766 | -1.22 | 1.63E-03 |
| | | | NM_177925 | | | |
| BE220828 | 237052_x_at | BE220828 | --- | --- | -1.21 | 3.13E-05 |
| SLC6A15 | 206376_at | NM_018057 | NM_018057 | 55117 | -1.21 | 3.32E-03 |
| | | | NM_182767 | | | |
| LGALS3BP | 200923_at | NM_005567 | NM_005567 | 3959 | -1.21 | 2.42E-05 |
| AI949265 | 229156_s_at | AI949265 | XM_499089 | 441301 | -1.21 | 9.17E-05 |
| ANXA4 | 201302_at | NM_001153 | NM_001153 | 307 | -1.21 | 7.33E-03 |
| HIST1H2BD | 209911_x_at | BC002842 | | 3017 | -1.21 | 8.46E-04 |
| | | | NM_021063 | | | |
| | | | NM_138720 | | | |
| PTPRN2 | 203029_s_at | NM_002847 | NM_002847 | 5799 | -1.20 | 5.18E-04 |
| | | | NM_130842 | | | |
| | | | NM_130843 | | | |
| RFPL1S | 214120_at | AW157535 | --- | 10740 | -1.20 | 2.59E-05 |
| IFNAR1 | 225661_at | AA811138 | NM_000629 | 3454 | -1.20 | 4.10E-04 |
| GLIS1 | 244128_x_at | AA872588 | NM_147193 | 148979 | -1.20 | 6.88E-03 |
| AGPAT3 | 223183_at | AI928403 | NM_020132 | 56894 | -1.20 | 5.35E-03 |
| PPEF1 | 206547_s_at | NM_006240 | NM_006240 | 5475 | -1.20 | 2.16E-03 |
| | | | NM_152223 | | | |
| | | | NM_152224 | | | |
| | | | NM_152225 | | | |
| | | | NM_152226 | | | |
| BCL6B | 243825_at | T79768 | NM_181844 | 255877 | -1.20 | 2.18E-03 |
| ABCG1 | 204567_s_at | NM_004915 | NM_004915 | 9619 | -1.19 | 4.03E-05 |
| | | | NM_016818 | | | |
| | | | NM_207174 | | | |
| | | | NM_207627 | | | |
| | | | NM_207628 | | | |
| | | | NM_207629 | | | |
| | | | NM_207630 | | | |
| DJ462O23.2 | 225876_at | T84558 | NM_020448 | 57185 | -1.19 | 3.26E-03 |
| CD200 | 209583_s_at | AF063591 | NM_00100419 6 | 4345 | -1.19 | 1.77E-04 |
| | | | NM_00100419 7 | | | |
| | | | NM_005944 | | | |
| C10orf89 | 230129_at | BF589448 | NM_153336 | 118672 | -1.19 | 2.60E-03 |
| MCPH1 | 219592_at | NM_024596 | NM_024596 | 79648 | -1.19 | 2.64E-03 |
| LOC285705 | 226989_at | BE855765 | --- | 285705 | -1.19 | 2.15E-04 |
| MANSC1 | 220945_x_at | NM_018050 | NM_018050 | 54682 | -1.19 | 1.43E-03 |
| BF057799 | 236325_at | BF057799 | --- | --- | -1.19 | 5.51E-03 |
| OIP106 | 226013_at | AW451452 | NM_014965 | 22906 | -1.18 | 4.67E-03 |
| RPL37 | 224763_at | BF724210 | NM_000997 | 6167 | -1.18 | 3.75E-03 |
| N30152 | 229850_at | N30152 | --- | --- | -1.18 | 6.56E-03 |
| DTX3L | 225415_at | AA577672 | NM_138287 | 151636 | -1.18 | 8.51E-04 |
| RAB6B | 225259_at | AI805050 | NM_016577 | 51560 | -1.18 | 4.83E-03 |
| ROR1 | 205805_s_at | NM_005012 | NM_005012 | 4919 | -1.18 | 2.35E-05 |
| DKFZp762K222 | 226822_at | AL512713 | NM_020225 | 56977 | -1.18 | 8.30E-04 |
| AI631888 | 235532_at | AI631888 | --- | --- | -1.17 | 3.83E-08 |
| PDGFRB | 202273_at | NM_002609 | NM_002609 | 5159 | -1.17 | 6.86E-03 |
| TLE1 | 228284_at | BE302305 | NM_005077 | 7088 | -1.17 | 1.65E-03 |
| APG5L | 202512_s_at | NM_004849 | NM_004849 | 9474 | -1.17 | 3.29E-03 |
| FLJ12572 | 219838_at | NM_022905 | NM_022905 | 64927 | -1.17 | 4.22E-04 |
| BACH2 | 221234_s_at | NM_021813 | NM_021813 | 60468 | -1.17 | 1.76E-04 |
| FLJ38964 | 235699_at | H19232 | NM_173527 | 161253 | -1.17 | 4.21E-03 |
| BE670797 | 230958_s_at | BE670797 | --- | --- | -1.17 | 5.70E-04 |
| AW665538 | 244532_x_at | AW665538 | XM_496692 | 441014 | -1.16 | 3.31E-03 |
| AI828075 | 230795_at | AI828075 | --- | --- | -1.16 | 1.67E-03 |
| NFATC4 | 236270_at | AI806528 | NM_004554 | 4776 | -1.16 | 6.47E-05 |
| SERTAD4 | 230660_at | AU146709 | NM_019605 | 56256 | -1.16 | 3.68E-03 |
| AL833609 | 1561660_at | AL833609 | XM_377034 | 401612 | -1.16 | 6.14E-03 |
| AI097640 | 213484_at | AI097640 | --- | --- | -1.16 | 6.41E-03 |
| CDKN1C | 213182_x_at | R78668 | NM_000076 | 1028 | -1.16 | 2.89E-03 |
| BC040901 | 1559534_at | BC040901 | --- | --- | -1.15 | 6.40E-03 |
| SLC25A12 | 203339_at | AI887457 | NM_003705 | 8604 | -1.15 | 3.28E-05 |
| TUBA1 | 212242_at | AL565074 | NM_006000 | 7277 | -1.15 | 4.37E-03 |
| RUNX1 | 209360_s_at | D43968 | NM_00100189 0 | 861 | -1.15 | 9.21E-03 |
| | | | NM_001754 | | | |
| FLJ35093 | 243042_at | BE645144 | NM_198549 | 374986 | -1.15 | 8.16E-03 |
| BE552011 | 230208_at | BE552011 | --- | --- | -1.15 | 8.27E-03 |
| MGC9850 | 224874_at | N32181 | NM_152705 | 219404 | -1.14 | 5.37E-04 |
| DKFZP43410714 | 231954_at | AL137273 | --- | 54553 | -1.14 | 3.90E-03 |
| AF130089 | 221590_s_at | AF130089 | --- | --- | -1.14 | 3.07E-03 |
| OMA1 | 226019_at | AI927931 | NM_145243 | 115209 | -1.14 | 7.48E-03 |
| SH3YL1 | 204019_s_at | NM_015677 | NM_015677 | 26751 | -1.14 | 4.21E-03 |
| PBX1 | 212148_at | AL049381 | NM_002585 | 5087 | -1.14 | 4.16E-04 |
| BID | 229321_s_at | AK026026 | NM_001196 | 637 | -1.14 | 6.39E-03 |
| | | | NM_197966 | | | |
| | | | NM_197967 | | | |
| MLSTD1 | 239108_at | H16791 | NM_018099 | 55711 | -1.14 | 8.13E-03 |
| RIS1 | 213338_at | BF062629 | NM_015444 | 25907 | -1.14 | 7.59E-03 |
| CDKN1C | 216894_x_at | D64137 | NM_000076 | 1028 | -1.13 | 1.04E-03 |
| LOC283824 | 213725_x_at | AI693140 | --- | 283824 | -1.13 | 9.96E-05 |
| BBP | 213882_at | AA012917 | NM_032027 | 83941 | -1.13 | 2.41E-03 |
| MLSTD1 | 220615_s_at | NM_018099 | NM_018099 | 55711 | -1.13 | 4.02E-03 |
| AA045174 | 213158_at | AA045174 | --- | --- | -1.13 | 1.45E-03 |
| AKAP12 | 210517_s_at | AB003476 | NM_005100 | 9590 | -1.13 | 3.33E-04 |
| | | | NM_144497 | | | |
| ZNF585A | 229794_at | BE550717 | NM_152655 | 199704 | -1.13 | 3.01E-03 |
| | | | NM_199126 | | | |
| NUDT6 | 230329_s_at | AI580268 | NM_007083 | 11162 | -1.13 | 7.27E-05 |
| | | | NM_198041 | | | |
| AU157441 | 226511_at | AU157441 | --- | --- | -1.13 | 2.71E-03 |
| AI056683 | 228171_s_at | AI056683 | --- | --- | -1.12 | 4.23E-03 |
| OLFM3 | 1554526_at | BC022531 | NM_058170 | 118427 | -1.12 | 3.96E-04 |
| MARCH-I | 229383_at | AI707896 | NM_017923 | 55016 | -1.12 | 2.06E-03 |
| KCNQ2 | 205737_at | NM_004518 | NM_004518 | 3785 | -1.12 | 1.40E-04 |
| | | | NM_172106 | | | |
| | | | NM_172107 | | | |
| | | | NM_172108 | | | |
| | | | NM_172109 | | | |
| SLC4A3 | 205918_at | NM_005070 | NM_005070 | 6508 | -1.12 | 4.66E-03 |
| | | | NM_201574 | | | |
| ARC | 210090_at | AF193421 | NM_015193 | 23237 | -1.12 | 8.17E-03 |
| TRAM1L1 | 244334_at | AA169554 | NM_152402 | 133022 | -1.12 | 5.89E-03 |
| EDG4 | 206723_s_at | AF011466 | NM_004720 | 9170 | -1.12 | 2.48E-05 |
| BE552208 | 229359_at | BE552208 | --- | --- | -1.12 | 7.11E-03 |
| PLXNC1 | 206471_s_at | NM_005761 | NM_005761 | 10154 | -1.12 | 3.43E-05 |
| SUHW4 | 221213_s_at | NM_017661 | NM_00100284 3 | 54816 | -1.12 | 2.43E-03 |
| | | | NM_00100284 4 | | | |
| | | | NM_017661 | | | |
| PALM2-AKAP2 | 226694_at | BG540494 | NM_007203 | 445815 | -1.12 | 1.75E-04 |
| | | | NM_147150 | | | |
| BF058422 | 226311_at | BF058422 | --- | --- | -1.12 | 6.35E-03 |
| CDKN1C | 219534_x_at | NM_000076 | NM_000076 | 1028 | -1.11 | 7.81E-05 |
| MGC9850 | 224857_s_at | BE378670 | NM_152705 | 219404 | -1.11 | 3.87E-03 |
| LOC157378 | 238045_at | BG167675 | NM_194291 | 157378 | -1.11 | 2.92E-03 |
| FBXO3 | 238686_at | AA130258 | NM_012175 | 26273 | -1.11 | 1.55E-03 |
| | | | NM_033406 | | | |
| EFEMP2 | 209356_x_at | AB030655 | NM_016938 | 30008 | -1.11 | 2.18E-03 |
| DKFZp434O052 | 1557879_at | BF110506 | NM_194302 | 255101 | -1.10 | 3.12E-03 |
| AK097571 | 1556261_a_at | AK097571 | --- | --- | -1.10 | 2.59E-03 |
| C6orf189 | 228875_at | AI540210 | --- | 221303 | -1.10 | 1.14E-04 |
| SLC1A2 | 225491_at | AL157452 | NM_004171 | 6506 | -1.10 | 9.00E-03 |
| NFIB | 243933_at | AI096634 | NM_005596 | 4781 | -1.10 | 5.38E-03 |
| FLJ32954 | 235349_at | AI261321 | NM_144713 | 151393 | -1.10 | 6.76E-03 |
| KCNK10 | 220727_at | NM_021161 | NM_021161 | 54207 | -1.10 | 5.91E-04 |
| | | | NM_138317 | | | |
| | | | NM_138318 | | | |
| SORL1 | 212560_at | AV728268 | NM_003105 | 6653 | -1.10 | 4.61E-03 |
| AIP1 | 209737_at | AB014605 | NM_012301 | 9863 | -1.10 | 3.84E-03 |
| AI827550 | 229606_at | AI827550 | --- | --- | -1.10 | 6.71E-07 |
| SH3KBP1 | 1554168_a_at | AF542051 | NM_031892 | 30011 | -1.10 | 5.41E-04 |
| OPTN | 202073_at | AV757675 | NM_00100821 1 | 10133 | -1.09 | 5.67E-03 |
| | | | NM_00100821 2 | | | |
| | | | NM_00100821 3 | | | |
| | | | NM_021980 | | | |
| ATF5 | 204998_s_at | NM_012068 | NM_012068 | 22809 | -1.09 | 5.15E-03 |
| LOC401264 | 227298_at | AI806330 | XM_379417 | 401264 | -1.09 | 6.06E-03 |
| NSE1 | 229546_at | AI378035 | NM_145175 | 151354 | -1.09 | 5.14E-03 |
| LOC91526 | 228471_at | AA744636 | NM_153697 | 91526 | -1.09 | 4.63E-03 |
| IL17D | 228977_at | AI669535 | NM_138284 | 53342 | -1.09 | 2.53E-03 |
| RG9MTD2 | 230243_at | BE671949 | NM_152292 | 93587 | -1.09 | 7.55E-03 |
| PLXNC1 | 213241_at | AF035307 | NM_005761 | 10154 | -1.09 | 3.36E-03 |
| PCDHB10 | 223854_at | AF131761 | | 56126 | -1.09 | 4.88E-04 |
| | | | NM_018930 | | | |
| PITPNC1 | 229414_at | AI676095 | NM_012417 | 26207 | -1.08 | 3.16E-04 |
| | | | NM_181671 | | | |
| DKFZp761D221 | 223672_at | AL136561 | NM_032291 | 84251 | -1.08 | 6.25E-03 |
| FKBP7 | 231130_at | AA683602 | NM_016105 | 51661 | -1.08 | 3.66E-03 |
| | | | NM_181342 | | | |
| CSPG3 | 205143_at | NM_004386 | NM_004386 | 1463 | -1.08 | 3.42E-03 |
| INSL3 | 214572_s_at | NM_005543 | NM_005543 | 3640 | -1.08 | 9.70E-03 |
| SLC12A7 | 218066_at | NM_006598 | NM_006598 | 10723 | -1.08 | 2.00E-03 |
| AI695743 | 244534_at | AI695743 | --- | --- | -1.08 | 4.40E-04 |
| DNAJC12 | 218976_at | NM_021800 | NM_021800 | 56521 | -1.08 | 9.46E-05 |
| | | | NM_201262 | | | |
| LOC285550 | 236321_at | AW439843 | XM_209656 | 285550 | -1.08 | 4.80E-03 |
| BF476403 | 230218_at | BF476403 | --- | --- | -1.08 | 8.15E-04 |
| AI798680 | 227943_at | AI798680 | --- | --- | -1.08 | 2.18E-03 |
| LOC196394 | 226258_at | BG031897 | NM_207337 | 196394 | -1.08 | 7.97E-03 |
| AB037765 | 226747_at | AB037765 | --- | --- | -1.08 | 2.32E-03 |
| SIX3 | 242054_s_at | AW473656 | NM_005413 | 6496 | -1.07 | 3.51E-04 |
| AI741900 | 239140_at | AI741900 | --- | --- | -1.07 | 1.53E-03 |
| FLJ33996 | 235969_at | R40373 | --- | 283401 | -1.07 | 5.06E-03 |
| SLC24A1 | 206081_at | NM_004727 | NM_004727 | 9187 | -1.07 | 7.02E-03 |
| BID | 227143_s_at | AA706658 | NM_001196 | 637 | -1.07 | 1.25E-03 |
| | | | NM_197966 | | | |
| | | | NM_197967 | | | |
| LRRC14 | 32062_at | D25216 | NM_014665 | 9684 | -1.07 | 1.47E-03 |
| LOC442647 | 229157_at | AI949265 | XM_499544 | 442647 | -1.07 | 1.43E-03 |
| FBXW7 | 229419_at | BF222826 | NM_018315 | 55294 | -1.07 | 8.90E-07 |
| | | | NM_033632 | | | |
| AI003508 | 244114_x_at | AI003508 | XM_496690 | 401131 | -1.07 | 2.92E-03 |
| ZNF651 | 226484_at | AA532640 | NM_145166 | 92999 | -1.07 | 1.09E-03 |
| MMS19L | 229226_at | BE465026 | NM_022362 | 64210 | -1.07 | 9.65E-05 |
| MAP6 | 228943_at | AW003666 | NM_033063 | 4135 | -1.07 | 6.70E-03 |
| | | | NM_207577 | | | |
| AA928506 | 213750_at | AA928506 | --- | --- | -1.06 | 2.13E-03 |
| H16409 | 227623_at | H16409 | --- | --- | -1.06 | 9.62E-05 |
| H28915 | 244359_s_at | H28915 | XM_496692 | 441014 | -1.06 | 5.58E-03 |
| USP53 | 230083_at | AW188464 | NM_019050 | 54532 | -1.06 | 3.73E-03 |
| LOC90693 | 228087 _ at | AK026684 | NM_138771 | 90693 | -1.06 | 8.05E-03 |
| AA194266 | 238728_at | AA194266 | --- | --- | -1.06 | 5.18E-03 |
| KIAA1324 | 221874_at | AB037745 | NM_020775 | 57535 | -1.06 | 6.36E-04 |
| HNRPR | 208766_s_at | BC001449 | NM_005826 | 10236 | -1.06 | 6.35E-05 |
| EGFR | 224999_at | BE878463 | NM_005228 | 1956 | -1.06 | 8.72E-03 |
| | | | NM_201282 | | | |
| | | | NM_201283 | | | |
| | | | NM_201284 | | | |
| HSPC065 | 222890_at | BG054922 | NM_014157 | 29070 | -1.06 | 6.01E-03 |
| FLJ30655 | 1569495_at | BC040258 | NM_144643 | 132320 | -1.06 | 6.57E-04 |
| PALM2-AKAP2 | 202760_s_at | NM_007203 | NM_007203 | 445815 | -1.05 | 1.16E-05 |
| | | | NM_147150 | | | |
| MCF2L | 35147_at | AB002360 | NM_024979 | 23263 | -1.05 | 1.55E-04 |
| DACH1 | 228915_at | AI650353 | NM_004392 | 1602 | -1.05 | 1.79E-03 |
| | | | NM_080759 | | | |
| | | | NM_080760 | | | |
| OLFM3 | 1554524_a_at | AF397394 | NM_058170 | 118427 | -1.05 | 2.90E-05 |
| LTBP3 | 219922_s_at | NM_021070 | NM_021070 | 4054 | -1.05 | 5.30E-03 |
| RPH3AL | 221614_s_at | BC005153 | NM_006987 | 9501 | -1.05 | 1.97E-03 |
| FLJ20160 | 225325_at | AA133311 | NM_017694 | 54842 | -1.05 | 8.44E-05 |
| PITPNC1 | 238649_at | AA815089 | NM_012417 | 26207 | -1.05 | 5.05E-03 |
| | | | NM_181671 | | | |
| AW959771 | 236350_at | AW959771 | --- | --- | -1.05 | 4.54E-03 |
| BU689502 | 1557302_at | BU689502 | --- | --- | -1.05 | 1.32E-04 |
| TXNDC4 | 208958_at | AI827677 | NM_015051 | 23071 | -1.04 | 4.97E-03 |
| SVH | 1554480_a_at | AY150851 | NM_031905 | 83787 | -1.04 | 4.17E-03 |
| KIAA1731 | 1565830_at | AL833615 | XM_374922 | 85459 | -1.04 | 8.88E-03 |
| AGPAT3 | 223184_s_at | BC004219 | NM_020132 | 56894 | -1.04 | 1.18E-03 |
| R53065 | 240430_at | R53065 | --- | --- | -1.04 | 1.68E-04 |
| LOC339290 | 226235_at | A1051299 | XM_375527 | 339290 | -1.04 | 3.78E-03 |
| GAB1 | 229114_at | AW237741 | NM_002039 | 2549 | -1.04 | 3.32E-04 |
| | | | NM_207123 | | | |
| LOC286052 | 241370_at | AA278233 | --- | 286052 | -1.04 | 1.43E-03 |
| LRP16 | 219188_s_at | NM_014067 | NM_014067 | 28992 | -1.04 | 8.11E-03 |
| ZNF505 | 1568720_at | BC018100 | NM_00100412 6 | 81931 | -1.04 | 3.98E-03 |
| | | | NM_031218 | | | |
| WBP11 | 244647_at | AA233885 | NM_016312 | 51729 | -1.04 | 6.62E-03 |
| AW590679 | 229300_at | AW590679 | --- | --- | -1.04 | 6.25E-06 |
| BBS7 | 235007_at | AI683802 | NM_018190 | 55212 | -1.03 | 2.92E-03 |
| | | | NM_176824 | | | |
| FMNL3 | 238823_at | AA481044 | NM_175736 | 91010 | -1.03 | 1.64E-04 |
| | | | NM_198900 | | | |
| PAK1 | 230100_x_at | AU147145 | NM_002576 | 5058 | -1.03 | 4.89E-03 |
| HPCAL1 | 212552_at | BE617588 | NM_002149 | 3241 | -1.03 | 6.95E-10 |
| | | | NM_134421 | | | |
| PHKB | 238601_at | AI798207 | NM_000293 | 5257 | -1.03 | 3.11E-03 |
| NF2 | 211017_s_at | AF123570 | NM_000268 | 4771 | -1.02 | 2.45E-03 |
| | | | NM_016418 | | | |
| | | | NM_181825 | | | |
| | | | NM_181826 | | | |
| | | | NM_181827 | | | |
| | | | NM_181828 | | | |
| | | | NM_181829 | | | |
| | | | NM_181830 | | | |
| | | | NM_181831 | | | |
| | | | NM_181832 | | | |
| | | | NM_181833 | | | |
| | | | NM_181834 | | | |
| | | | NM_181835 | | | |
| NFASC | 213438_at | AA995925 | NM_00100538 7 | 23114 | -1.02 | 2.89E-05 |
| | | | NM_00100538 8 | | | |
| | | | NM_00100538 9 | | | |
| | | | NM_015090 | | | |
| RBBP6 | 1552329_at | BC029352 | NM_006910 | 5930 | -1.02 | 4.18E-03 |
| | | | NM_018703 | | | |
| | | | NM_032626 | | | |
| BE669703 | 229541_at | BE669703 | --- | --- | -1.02 | 1.41E-04 |
| CTSC | 225646_at | AI246687 | NM_001814 | 1075 | -1.02 | 7.92E-03 |
| | | | NM_148170 | | | |
| TBC1D12 | 221858_at | N34407 | XM_051081 | 23232 | -1.02 | 1.15E-04 |
| C21orf86 | 228909_at | AW131553 | NM_153454 | 257103 | -1.02 | 1.26E-03 |
| AI991451 | 228412_at | AI991451 | --- | --- | -1.01 | 8.75E-03 |
| FLJ25168 | 1553400_a_at | NM_152466 | NM_152466 | 147081 | -1.01 | 1.66E-03 |
| LOC157378 | 241342_at | BG288115 | NM_194291 | 157378 | -1.01 | 3.97E-06 |
| BG150485 | 228754_at | BG150485 | --- | --- | -1.01 | 3.89E-04 |
| W56760 | 230683_at | W56760 | --- | --- | -1.01 | 1.42E-03 |
| BF512183 | 229885_at | BF512183 | --- | --- | -1.01 | 4.33E-04 |
| KIAA1573 | 225627_s_at | AK024256 | NM_020925 | 57685 | -1.01 | 1.13E-03 |
| GPR123 | 239221_at | AI884909 | NM_032422 | 84435 | -1.01 | 2.88E-03 |
| IPP | 241393_at | R78604 | NM_005897 | 3652 | -1.01 | 4.60E-03 |
| ZNF626 | 1552643_at | NM_145297 | NM_145297 | 199777 | -1.01 | 2.86E-03 |
| AV723984 | 230403_at | AV723984 | --- | --- | -1.01 | 9.06E-04 |
| AI672159 | 226550_at | AI672159 | --- | --- | -1.00 | 3.25E-03 |
| TMEM28 | 206299_at | NM_015686 | NM_015686 | 27112 | -1.00 | 3.66E-04 |
| AI627858 | 236105_at | AI627858 | --- | --- | -1.00 | 9.19E-03 |
| ZNF452 | 244758_at | AA480069 | NM_052923 | 114821 | -1.00 | 6.80E-05 |
| NME7 | 227556_at | A1094580 | NM_013330 NM_197972 | 29922 | -1.00 | 7.25E-03 |
| PLRG1 | 227246_at | BF438014 | NM_002669 | 5356 | -1.00 | 1.86E-03 |
| SLIT1 | 213601_at | AB011537 | NM_003061 | 6585 | -1.00 | 3.09E-04 |
| CTMP | 229253_at | AI184512 | NM_053055 | 117145 | -1.00 | 9.64E-03 |
| | | | NM_176853 | | | |
| AK056973 | 1557135_at | AK056973 | --- | --- | -1.00 | 3.03E-04 |
| LOC169834 | 230876_at | AI827906 | XM_095965 | 169834 | -1.00 | 1.27E-03 |
| FLJ22624 | 236560_at | BE218020 | NM_024808 | 79866 | -1.00 | 4.76E-03 |
| VPS41 | 235625_at | AW963328 | NM_014396 | 27072 | -1.00 | 3.16E-03 |
| | | | NM_080631 | | | |
| CDC42EP3 | 225685_at | A1801777 | NM_006449 | 10602 | -1.00 | 1.49E-04 |
| LRRTM2 | 206408_at | NM_015564 | NM_015564 | 26045 | -1.00 | 5.75E-03 |
| PHF10 | 221787_at | BF431618 | NM_018288 NM_133325 | 55274 | -1.00 | 3.14E-06 |
| PHKA2 | 1560358_at | BC038597 | NM_000292 | 5256 | -0.99 | 4.51E-03 |
| NM_015492 | 204495_s_at | NM_015492 | --- | --- | -0.99 | 2.51E-03 |
| AU151107 | 230650_at | AU151107 | --- | --- | -0.99 | 3.88E-03 |
| SLICK | 244455_at | AI732637 | NM_198503 | 343450 | -0.99 | 1.51E-03 |
| FLJ30294 | 238867_at | AI298041 | NM_144632 | 130827 | -0.99 | 9.15E-04 |
| LOC340281 | 240194_at | AA406397 | --- | 340281 | -0.99 | 3.47E-03 |
| LIPT1 | 205571_at | NM_015929 | NM_015929 | 51601 | -0.99 | 6.75E-03 |
| | | | NM_145196 | | | |
| | | | NM_145197 | | | |
| | | | NM_145198 | | | |
| | | | NM_145199 | | | |
| DKFZP434H132 | 215087_at | AL109730 | NM_015492 | 56905 | -0.99 | 7.16E-06 |
| RAPGEF3 | 210051_at | U78168 | NM_006105 | 10411 | -0.98 | 7.80E-03 |
| BTD | 214116_at | AI767414 | NM_000060 | 686 | -0.98 | 4.23E-03 |
| ZCCHC7 | 226496_at | BG291039 | NM_032226 | 84186 | -0.98 | 5.34E-03 |
| C10orf104 | 229145_at | AA541762 | NM_173473 | 119504 | -0.98 | 9.01E-04 |
| LOC222070 | 58900_at | AW025284 | --- | 222070 | -0.98 | 4.22E-04 |
| ESRRG | 207981_s_at | NM_001438 | NM_001438 | 2104 | -0.98 | 6.26E-03 |
| | | | NM_206594 | | | |
| | | | NM_206595 | | | |
| PRPS2 | 203401_at | NM_002765 | NM_002765 | 5634 | -0.98 | 9.17E-04 |
| TM4SF2 | 202242_at | NM_004615 | NM_004615 | 7102 | -0.98 | 4.04E-05 |
| PCAF | 203845_at | AV727449 | NM_003884 | 8850 | -0.98 | 3.42E-06 |
| LOC284669 | 1559483_at | AK092251 | --- | 284669 | -0.98 | 1.08E-03 |
| ARMCX5 | 1555902_at | BC022066 | NM_022838 | 64860 | -0.98 | 6.50E-03 |
| AI862542 | 235696_at | AI862542 | --- | --- | -0.98 | 7.90E-04 |
| PLEKHA2 | 225136_at | BF968578 | XM_496973 | 59339 | -0.98 | 1.31E-03 |
| ACSL4 | 202422_s_at | NM_022977 | NM_004458 | 2182 | -0.98 | 1.83E-03 |
| | | | NM_022977 | | | |
| PIN4 | 243226_at | N53548 | NM_006223 | 5303 | -0.97 | 9.48E-04 |
| BF510890 | 243406_at | BF510890 | --- | --- | -0.97 | 2.52E-03 |
| FLJ11000 | 235159_at | AW296028 | NM_018295 | 55281 | -0.97 | 8.58E-03 |
| TPMT | 203672_x_at | U12387 | NM_000367 | 7172 | -0.97 | 3.95E-03 |
| NCOA7 | 225344_at | AL035689 | NM_181782 | 135112 | -0.97 | 1.02E-03 |
| NUDT6 | 220183_s_at | NM_007083 | NM_007083 | 11162 | -0.97 | 1.63E-05 |
| | | | NM_198041 | | | |
| CACNA1B | 235781_at | AA448208 | NM_000718 | 774 | -0.97 | 2.17E-03 |
| LOC253982 | 214993_at | AF070642 | NM_181718 | 253982 | -0.97 | 5.64E-03 |
| | | | NM_198907 | | | |
| NOTCH4 | 205247_at | AI743713 | NM_004557 | 4855 | -0.96 | 5.91E-03 |
| DACH1 | 205472_s_at | NM_004392 | NM_004392 | 1602 | -0.96 | 6.40E-04 |
| | | | NM_080759 | | | |
| | | | NM_080760 | | | |
| C21orf25 | 212875_s_at | AP001745 | NM_199050 | 25966 | -0.96 | 1.81E-04 |
| KIAA1377 | 235956_at | AI797063 | NM_020802 | 57562 | -0.96 | 2.43E-03 |
| KIAA1729 | 230715_at | AI138969 | NM_053042 | 85460 | -0.96 | 2.87E-03 |
| TMEM18 | 225487_at | AI074464 | NM_152834 | 129787 | -0.96 | 3.04E-04 |
| CITED2 | 209357_at | AF109161 | NM_006079 | 10370 | -0.96 | 4.57E-03 |
| AI768895 | 231069_at | AI768895 | --- | --- | -0.96 | 4.30E-03 |
| DKFZp451M211 | 239240_at | AI814116 | NM_182585 | 285023 | -0.96 | 8.59E-03 |
| N50119 | 235635_at | N50119 | --- | --- | -0.96 | 3.56E-03 |
| AI802997 | 235211_at | AI802997 | --- | --- | -0.96 | 4.66E-05 |
| PRPH | 213847_at | NM_006262 | NM_006262 | 5630 | -0.95 | 1.14E-03 |
| C14orf167 | 227446_s_at | BF445127 | --- | 55449 | -0.95 | 4.86E-03 |
| NEIL2 | 226585_at | BE466620 | NM_145043 | 252969 | -0.95 | 3.33E-04 |
| LOC401020 | 1560475_at | AK092134 | XM_379145 | 401020 | -0.95 | 4.55E-03 |
| CXorf45 | 205583_s_at | NM_024810 | NM_024810 | 79868 | -0.95 | 2.74E-03 |
| MLLT4 | 238871_at | AI086124 | NM_005936 | 4301 | -0.95 | 6.94E-03 |
| PPP3CA | 202457_s_at | AA911231 | NM_000944 | 5530 | -0.95 | 7.65E-04 |
| MTUS1 | 212095_s_at | BE552421 | NM_00100192 4 | 57509 | -0.95 | 1.54E-03 |
| | | | NM_00100192 5 | | | |
| | | | NM_00100192 7 | | | |
| | | | NM_00100193 1 | | | |
| | | | NM_020749 | | | |
| CARD8 | 1554479_a_at | AF511652 | NM_014959 | 22900 | -0.95 | 1.49E-03 |
| HS3ST5 | 240479_at | AW449310 | NM_153612 | 222537 | -0.95 | 4.10E-03 |
| CDC42EP3 | 209286_at | AI754416 | NM_006449 | 10602 | -0.94 | 2.27E-05 |
| CLDN12 | 223249_at | AL136770 | NM_012129 | 9069 | -0.94 | 7.31E-04 |
| MGC70870 | 242136_x_at | T66145 | NM_203481 | 403340 | -0.94 | 4.23E-04 |
| LIFR | 225575_at | AI680541 | NM_002310 | 3977 | -0.94 | 2.42E-03 |
| AA002140 | 241365_at | AA002140 | --- | --- | -0.94 | 3.00E-03 |
| LOC153364 | 230298_at | AI692906 | NM_203406 | 153364 | -0.94 | 5.23E-03 |
| BRUNOL4 | 238966_at | AA678985 | NM_020180 | 56853 | -0.94 | 6.55E-04 |
| AV700050 | 228835_at | AV700050 | --- | --- | -0.94 | 3.53E-03 |
| TRIM4 | 223384_s_at | BE501464 | | 89122 | -0.94 | 6.97E-03 |
| | | | NM_033017 | | | |
| | | | NM_033091 | | | |
| FLJ23560 | 219487_at | NM_024685 | NM_024685 | 79738 | -0.94 | 1.67E-03 |
| PDGFRL | 205226_at | NM_006207 | NM_006207 | 5157 | -0.94 | 3.47E-03 |
| BG502771 | 226397_s_at | BG502771 | --- | --- | -0.94 | 6.49E-05 |
| AI392908 | 230352_at | AI392908 | --- | --- | -0.94 | 3.46E-03 |
| LOC93622 | 225391_at | AL562398 | NM_138699 | 93622 | -0.94 | 5.23E-05 |
| IMMP2L | 227153_at | AI784580 | NM_032549 | 83943 | -0.94 | 1.01E-03 |
| DACH1 | 205471_s_at | AW772082 | NM_004392 | 1602 | -0.94 | 4.97E-05 |
| | | | NM_080759 | | | |
| | | | NM_080760 | | | |
| AI963605 | 230269_at | AI963605 | --- | --- | -0.94 | 1.40E-03 |
| R45950 | 229994_at | R45950 | --- | --- | -0.93 | 1.97E-03 |
| RGMB | 227339_at | BE206621 | NM_173670 | 285704 | -0.93 | 3.25E-06 |
| LOC285705 | | | | 285705 | | |
| NIN | 223981_at | AF223937 | NM_016350 | 51199 | -0.93 | 8.04E-03 |
| | | | NM_020921 | | | |
| | | | NM_182944 | | | |
| | | | NM_182945 | | | |
| | | | NM_182946 | | | |
| DKFZp313N062 | 241666_at | BG493222 | NM_173826 | 285343 | -0.93 | 6.42E-03 |
| DKFZP564D172 | 212936_at | AI927701 | NM_032042 | 83989 | -0.93 | 2.49E-05 |
| CaMKIINalpha | 218309_at | NM_018584 | NM_018584 | 55450 | -0.93 | 3.41E-05 |
| MAPK10 | 237413_at | AI568447 | NM_002753 | 5602 | -0.93 | 2.01E-04 |
| | | | NM_138980 | | | |
| | | | NM_138981 | | | |
| | | | NM_138982 | | | |
| CXXC4 | 220277_at | NM_025212 | NM_025212 | 80319 | -0.93 | 1.90E-04 |
| HEL308 | 1554341_a_at | BC011863 | NM_133636 | 113510 | -0.93 | 1.26E-04 |
| SIGIRR | 218921_at | NM_021805 | NM_021805 | 59307 | -0.92 | 3.82E-03 |
| FBXL17 | 227203_at | AA115629 | NM_022824 | 64839 | -0.92 | 1.44E-03 |
| ATXN1 | 203231_s_at | AW235612 | NM_000332 | 6310 | -0.92 | 5.79E-05 |
| USP46 | 203869_at | AK024318 | NM_022832 | 64854 | -0.92 | 3.21E-04 |
| CSE-C | 224391_s_at | AF303378 | NM_170601 | 54414 | -0.92 | 2.82E-03 |
| GGPS1 | 202321_at | AW299507 | NM_004837 | 9453 | -0.92 | 2.16E-03 |
| MUTED | 226543_at | AI768114 | NM_201280 | 63915 | -0.92 | 3.41E-03 |
| KIAA0564 | 212946_at | AK025432 | NM_00100981 4 | 23078 | -0.92 | 4.42E-03 |
| | | | NM_015058 | | | |
| JMY | 226352_at | BF447037 | NM_152405 | 133746 | -0.92 | 2.68E-04 |
| PALM2-AKAP2 | 202759_s_at | BE879367 | NM_007203 | 445815 | -0.92 | 1.79E-04 |
| | | | NM_147150 | | | |
| AI631072 | 228638_at | AI631072 | --- | --- | -0.92 | 9.69E-03 |
| MCAM | 209087_x_at | AF089868 | NM_006500 | 4162 | -0.92 | 2.51E-03 |
| PSD3 | 203355_s_at | NM_015310 | NM_015310 | 23362 | -0.92 | 4.84E-03 |
| | | | NM_206909 | | | |
| PTEN | 228006_at | BG403361 | NM_000314 | 5728 | -0.92 | 3.02E-03 |
| ABAT | 209459_s_at | AF237813 | NM_000663 | 18 | -0.92 | 5.61E-03 |
| | | | NM_020686 | | | |
| AI887898 | 228423_at | AI887898 | XM_374094 | 389237 | -0.92 | 5.43E-04 |
| MGC14595 | 238562_at | BE542779 | NM_032334 | 84294 | -0.92 | 1.59E-05 |
| AA757630 | 236724_at | AA757630 | --- | --- | -0.91 | 2.99E-04 |
| BF510563 | 228561_at | BF510563 | --- | --- | -0.91 | 1.65E-05 |
| ETS2 | 201328_at | AL575509 | NM_005239 | 2114 | -0.91 | 6.50E-05 |
| TRIM32 | 236233_at | AI768810 | NM_012210 | 22954 | -0.91 | 7.51E-03 |
| CDC42EP3 | 209288_s_at | AL136842 | NM_006449 | 10602 | -0.91 | 1.33E-04 |
| CNOT6L | 227119_at | BF103856 | NM_144571 | 246175 | -0.91 | 2.60E-06 |
| AI754871 | 229075_at | AI754871 | --- | --- | -0.91 | 2.23E-03 |
| PSD3 | 218613_at | NM_018422 | NM_015310 | 23362 | -0.91 | 2.81E-05 |
| | | | NM_206909 | | | |
| SLC20A2 | 229371_at | BF940010 | NM_006749 | 6575 | -0.91 | 2.66E-04 |
| SNX10 | 218404_at | NM_013322 | NM_013322 | 29887 | -0.90 | 3.48E-03 |
| KCTD18 | 226493_at | AI627249 | NM_152387 | 130535 | -0.90 | 7.20E-03 |
| MGC31963 | 1558692_at | AW090182 | NM_144580 | 112770 | -0.90 | 9.19E-03 |
| C21orf51 | 228239_at | AA148789 | NM_058182 | 54065 | -0.90 | 3.56E-03 |
| T79639 | 243466_at | T79639 | --- | --- | -0.90 | 3.38E-03 |
| BE671727 | 244177_at | BE671727 | --- | --- | -0.90 | 7.50E-03 |
| WDR32 | 219001_s_at | NM_024345 | NM_024345 | 79269 | -0.90 | 1.46E-03 |
| AW770320 | 242523_at | AW770320 | --- | --- | -0.90 | 5.17E-03 |
| FLJ31265 | 228341_at | AI809108 | NM_152395 | 131870 | -0.90 | 9.74E-03 |
| TENS1 | 217853_at | NM_022748 | NM_022748 | 64759 | -0.90 | 3.93E-03 |
| AFFX-BioB-3 | AFFX-BioB-3_a | AFFX-BioB-3 | --- | --- | -0.90 | 4.10E-03 |
| AL359605 | 227318_at | AL359605 | --- | --- | -0.90 | 4.75E-04 |
| FIP1L1 | 1554424_at | BC017724 | NM_030917 | 81608 | -0.90 | 2.24E-05 |
| MGC22773 | 235498_at | AI269596 | NM_145258 | 127255 | -0.89 | 3.93E-07 |
| A1765014 | 224649_x_at | A1765014 | --- | --- | -0.89 | 3.50E-03 |
| ACSL6 | 229725_at | AV705292 | NM_00100918 5 | 23305 | -0.89 | 6.40E-03 |
| | | | NM_015256 | | | |
| HNRPR | 208765_s_at | NM_005826 | NM_005826 | 10236 | -0.89 | 9.23E-05 |
| GATM | 216733_s_at | X86401 | NM_001482 | 2628 | -0.89 | 6.06E-03 |
| SAT | 213988_s_at | BE971383 | NM_002970 | 6303 | -0.89 | 6.15E-03 |
| MGC16275 | 1558167_a_at | BM824870 | --- | 85001 | -0.89 | 3.24E-03 |
| AI743452 | 237031_at | A1743452 | --- | --- | -0.89 | 2.69E-03 |
| FLJ11011 | 222656_at | AI625741 | NM_00100148 1 | 55284 | -0.89 | 2.46E-03 |
| | | | NM_00100148 2 | | | |
| | | | NM_018299 | | | |
| BF510762 | 230262_at | BF510762 | --- | --- | -0.89 | 7.78E-05 |
| SOCS5 | 208127_s_at | NM_014011 | NM_014011 | 9655 | -0.89 | 2.64E-03 |
| | | | NM_144949 | | | |
| LTBP3 | 227308_x_at | AW515704 | NM_021070 | 4054 | -0.89 | 1.44E-04 |
| AI826437 | 229975_at | AI826437 | --- | --- | -0.89 | 3.65E-04 |
| BRAF | 236402_at | AW184034 | NM_004333 | 673 | -0.89 | 8.26E-03 |
| LNX | 227293_at | AI264003 | NM_032622 | 84708 | -0.88 | 1.56E-03 |
| F3 | 204363_at | NM_001993 | NM_001993 | 2152 | -0.88 | 2.35E-03 |
| COVA1 | 204643_s_at | NM_006375 | NM_006375 | 10495 | -0.88 | 9.34E-03 |
| | | | NM_182314 | | | |
| TPD52 | 201691_s_at | NM_005079 | NM_005079 | 7163 | -0.88 | 6.44E-04 |
| GRIK2 | 1560142_at | AJ301610 | NM_021956 | 2898 | -0.88 | 5.49E-03 |
| | | | NM_175768 | | | |
| A1025829 | 235227_at | A1025829 | --- | --- | -0.88 | 2.45E-03 |
| AI761584 | 229654_at | A1761584 | --- | --- | -0.88 | 9.83E-03 |
| MAP3K5 | 203836_s_at | D84476 | NM_005923 | 4217 | -0.88 | 7.19E-03 |
| NOX4 | 219773_at | NM_016931 | NM_016931 | 50507 | -0.88 | 6.53E-03 |
| FBXL17 | 238174_at | AI694129 | NM_022824 | 64839 | -0.88 | 4.28E-04 |
| ERCC6 | 207347_at | NM_000124 | NM_000124 | 2074 | -0.88 | 1.34E-03 |
| TMEM34 | 219074_at | NM_018241 | NM_018241 | 55751 | -0.88 | 2.71E-03 |
| MGC52110 | 226039_at | AW006441 | NM_001008215 | 493753 | -0.87 | 3.18E-05 |
| SPHAR | 206272_at | NM_006542 | NM_006542 | 10638 | -0.87 | 3.60E-03 |
| FCMD | 205283_at | NM_006731 | NM_006731 | 2218 | -0.87 | 3.96E-03 |
| BAI3 | 205638_at | NM_001704 | NM_001704 | 577 | -0.87 | 8.83E-03 |
| N29877 | 231169_at | N29877 | --- | --- | -0.87 | 2.33E-03 |
| MBNL1 | 201153_s_at | NM_021038 | NM_021038 | 4154 | -0.87 | 2.24E-05 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| CLCN3 | 201734_at | AI760629 | NM_001829 | 1182 | -0.87 | 7.31E-03 |
| | | | NM_173872 | | | |
| MGC13024 | 221864_at | AW517464 | NM_152288 | 93129 | -0.87 | 9.69E-03 |
| XPR1 | 226615_at | BE439489 | NM_004736 | 9213 | -0.87 | 2.22E-04 |
| CAMK2B | 210404_x_at | AF078803 | NM_001220 | 816 | -0.87 | 1.66E-03 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| CHD2 | 228999_at | AW514564 | NM_001271 | 1106 | -0.86 | 2.91E-03 |
| TMEM1 | 209412_at | U61500 | NM_00100172 3 | 7109 | -0.86 | 2.68E-03 |
| | | | NM_003274 | | | |
| FLJ32130 | 1554769_at | BC040642 | NM_152458 | 146540 | -0.86 | 3.30E-03 |
| MCF2L | 212935_at | AB002360 | NM_024979 | 23263 | -0.86 | 3.60E-04 |
| PGRMC2 | 213227_at | BE879873 | NM_006320 | 10424 | -0.86 | 1.58E-03 |
| BF434383 | 235473_at | BF434383 | --- | --- | -0.86 | 5.25E-03 |
| WARS2 | 222734_at | BF515963 | NM_015836 | 10352 | -0.86 | 9.52E-03 |
| | | | NM_201263 | | | |
| ASB13 | 218862_at | NM_024701 | NM_024701 | 79754 | -0.86 | 5.98E-03 |
| ARSD | 225280_x_at | N51673 | NM_001669 | 414 | -0.86 | 4.80E-03 |
| | | | NM_009589 | | | |
| HEL308 | 1554342_s_at | BC011863 | NM_133636 | 113510 | -0.86 | 8.97E-03 |
| GLCE | 213552_at | W87398 | NM_015554 | 26035 | -0.86 | 3.64E-03 |
| ZNF25 | 235164_at | BG433539 | NM_145011 | 219749 | -0.86 | 8.82E-03 |
| N56912 | 221944_at | N56912 | XM_496112 | 440332 | -0.86 | 2.61E-03 |
| BMPR2 | 225144_at | AI457436 | NM_001204 | 659 | -0.86 | 9.23E-05 |
| | | | NM_033346 | | | |
| A1796858 | 236164_at | AI796858 | --- | --- | -0.86 | 6.19E-05 |
| HPS1 | 210112_at | U96721 | NM_000195 | 3257 | -0.86 | 9.98E-03 |
| | | | NM_182637 | | | |
| | | | NM_182638 | | | |
| | | | NM_182639 | | | |
| SCRN3 | 219234_x_at | NM_024583 | NM_024583 | 79634 | -0.86 | 1.04E-03 |
| EFNB3 | 205031_at | NM_001406 | NM_001406 | 1949 | -0.86 | 4.69E-03 |
| ZNF25 | 228185_at | N32599 | NM_145011 | 219749 | -0.85 | 9.68E-03 |
| AW274846 | 228108_at | AW274846 | --- | --- | -0.85 | 3.72E-03 |
| STC1 | 204595_s_at | AI300520 | NM_003155 | 6781 | -0.85 | 1.72E-03 |
| AW044658 | 229201_at | AW044658 | --- | --- | -0.85 | 7.58E-05 |
| CA442342 | 1556097_at | CA442342 | --- | --- | -0.85 | 6.36E-03 |
| FLJ21159 | 235550_at | AV751613 | NM_024826 | 79884 | -0.85 | 6.98E-04 |
| PKNOX1 | 54051_at | H59033 | NM_004571 | 5316 | -0.85 | 1.84E-03 |
| | | | NM_197976 | | | |
| PDE2A | 204134_at | NM_002599 | NM_002599 | 5138 | -0.85 | 1.09E-04 |
| DHRSX | 225503_at | AL547782 | NM_145177 | 207063 | -0.85 | 2.79E-03 |
| SLC19A1 | 229489_at | AI681043 | NM_003056 | 6573 | -0.85 | 2.68E-03 |
| | | | NM_194255 | | | |
| C13orf24 | 213239_at | NM_006346 | NM_006346 | 10464 | -0.85 | 2.61E-04 |
| SRGAP3 | 209794_at | AB007871 | NM_014850 | 9901 | -0.85 | 7.85E-03 |
| C21orf127 | 234676_s_at | AK021678 | NM_013240 | 29104 | -0.85 | 8.73E-03 |
| | | | NM_182749 | | | |
| PARP11 | 229138_at | AV747166 | NM_020367 | 57097 | -0.84 | 1.23E-03 |
| ELAC1 | 219325_s_at | NM_018696 | NM_018696 | 55520 | -0.84 | 9.17E-03 |
| AV702357 | 229823_at | AV702357 | --- | --- | -0.84 | 2.59E-04 |
| FLRT1 | 210414_at | AF169675 | NM_013280 | 23769 | -0.84 | 8.98E-03 |
| C2orf17 | 222129_at | AK026155 | NM_024293 | 79137 | -0.84 | 2.37E-03 |
| PF6 | 1557593_at | AA250798 | NM_206996 | 200162 | -0.84 | 1.52E-03 |
| BC040191 | 1568623_a_at | BC040191 | --- | --- | -0.84 | 5.28E-03 |
| H99038 | 227338_at | H99038 | XM_498946 | 440983 | -0.84 | 9.90E-03 |
| MANBA | 203778_at | NM_005908 | NM_005908 | 4126 | -0.84 | 4.77E-03 |
| DELGEF | 232983_s_at | AJ243951 | NM_012139 | 26297 | -0.84 | 7.32E-03 |
| ATP2C1 | 209934_s_at | AF225981 | NM_00100148 5 | 27032 | -0.84 | 5.96E-03 |
| | | | NM_00100148 6 | | | |
| | | | NM_00100148 7 | | | |
| | | | NM_014382 | | | |
| LOC92249 | 212957_s_at | AU154785 | XM_499179 | 92249 | -0.83 | 7.94E-04 |
| SPATA7 | 234929_s_at | AF144488 | NM_018418 | 55812 | -0.83 | 9.12E-03 |
| AW269338 | 235691_at | AW269338 | XM_378880 | 400764 | -0.83 | 1.62E-03 |
| ZNF295 | 225539_at | AP001745 | NM_020727 | 49854 | -0.83 | 1.11E-03 |
| KIAA1223 | 225731_at | BF196876 | XM_048747 | 57182 | -0.83 | 1.79E-06 |
| BF207861 | 239768_x_at | BF207861 | --- | --- | -0.83 | 3.46E-03 |
| BG236351 | 217598_at | BG236351 | --- | --- | -0.83 | 1.23E-03 |
| B3GTL | 227083_at | N51325 | NM_194318 | 145173 | -0.83 | 2.95E-05 |
| QPCT | 205174_s_at | NM_012413 | NM_012413 | 25797 | -0.83 | 8.28E-03 |
| AL045516 | 232166_at | AL045516 | --- | --- | -0.83 | 2.57E-03 |
| ZNF148 | 230821_at | AW594167 | NM_021964 | 7707 | -0.83 | 3.02E-03 |
| GNG2 | 224964_s_at | AK026424 | NM_053064 | 54331 | -0.83 | 6.30E-03 |
| CTPS2 | 219080_s_at | NM_019857 | NM_019857 | 56474 | -0.83 | 3.00E-04 |
| | | | NM_175859 | | | |
| CAMK2D | 225019_at | BF797381 | NM_001221 | 817 | -0.83 | 8.53E-03 |
| | | | NM_172115 | | | |
| | | | NM_172127 | | | |
| | | | NM_172128 | | | |
| CYLD | 213295_at | AA555096 | NM_015247 | 1540 | -0.83 | 7.35E-04 |
| TMEM5 | 204808_s_at | NM_014254 | NM_014254 | 10329 | -0.83 | 2.63E-03 |
| FLJ35036 | 225512_at | A1005245 | XM_172341 | 253461 | -0.83 | 6.24E-04 |
| LOC51334 | 220014_at | NM_016644 | NM_016644 | 51334 | -0.82 | 521E-04 |
| MGC34646 | 224996_at | N30209 | NM_173519 | 157807 | -0.82 | 6.97E-05 |
| NDP52 | 235076_at | AA149539 | NM_005831 | 10241 | -0.82 | 2.69E-03 |
| FLJ38991 | 227442_at | BG283902 | NM_173827 | 285521 | -0.82 | 2.38E-03 |
| LOC144363 | 225469_at | AA015609 | NM_00100166C | 144363 | -0.82 | 6.00E-03 |
| AI627803 | 236194_at | AI627803 | --- | --- | -0.82 | 8.90E-03 |
| EIF4E3 | 238461_at | AA228031 | --- | 317649 | -0.82 | 8.67E-03 |
| LOC284262 | 227478_at | BF739885 | --- | 284262 | -0.82 | 1.50E-05 |
| LRCH2 | 227688_at | AK022128 | NM_020871 | 57631 | -0.82 | 4.46E-04 |
| AI635131 | 1557192_at | AI635131 | XM_375410 | 400574 | -0.82 | 2.14E-03 |
| AA935515 | 242790_at | AA935515 | --- | --- | -0.82 | 4.73E-04 |
| ZNF423 | 214761_at | AW149417 | NM_015069 | 23090 | -0.82 | 6.45E-03 |
| FBXO4 | 223493_at | AF129534 | NM_012176 | 26272 | -0.82 | 2.52E-03 |
| | | | NM_033484 | | | |
| AI417268 | 225028_at | AI417268 | --- | --- | -0.82 | 8.20E-03 |
| EIF2C1 | 228120_at | AW136032 | NM_012199 | 26523 | -0.81 | 1.09E-05 |
| FLJ22313 | 236170_x_at | AI377423 | NM_022373 | 64224 | -0.81 | 4.47E-03 |
| LOC401500 | 233946_at | AL512690 | XM_379629 | 401500 | -0.81 | 2.52E-03 |
| AFFX-BioB-M | AFFX-BioB-M_a | AFFX-BioB-M | --- | --- | -0.81 | 3.21E-03 |
| AI149508 | 228416_at | AI149508 | --- | --- | -0.81 | 1.26E-04 |
| ZNF287 | 220055_at | NM_020653 | NM_020653 | 57336 | -0.81 | 1.85E-04 |
| FLJ30851 | 230435_at | BF108666 | NM_198553 | 375190 | -0.81 | 5.13E-03 |
| BC042959 | 1556194_a_at | BC042959 | --- | --- | -0.81 | 2.89E-03 |
| CA5BL | 238435_at | AA521288 | XM_291346 | 340591 | -0.81 | 4.85E-03 |
| WIZ | 221784_at | AI089655 | XM_372716 | 58525 | -0.81 | 1.35E-03 |
| AW086077 | 232800_at | AW086077 | --- | --- | -0.81 | 3.68E-03 |
| FLJ22795 | 223327_x_at | AF316855 | NM_025084 | 80154 | -0.81 | 4.57E-03 |
| AI075662 | 238038_at | AI075662 | --- | --- | -0.81 | 4.28E-03 |
| PCDHB14 | 231726_at | NM_018934 | | 56122 | -0.81 | 5.56E-03 |
| | | | NM_018934 | | | |
| LOC130355 | 227840_at | AA738440 | XM_496588 | 130355 | -0.81 | 2.33E-05 |
| FLJ10378 | 231824_at | AK027164 | NM_018078 | 55132 | -0.81 | 7.52E-03 |
| | | | NM_032239 | | | |
| | | | NM_178043 | | | |
| CAMK2B | 211483_x_at | AF081924 | NM_001220 | 816 | -0.81 | 2.75E-04 |
| | | | NM_172078 | | | |
| | | | NM_172079 | | | |
| | | | NM_172080 | | | |
| | | | NM_172081 | | | |
| | | | NM_172082 | | | |
| | | | NM_172083 | | | |
| | | | NM_172084 | | | |
| RPL4 | 226556_at | BF431260 | NM_000968 | 6124 | -0.81 | 2.30E-07 |
| C21orf108 | 212996_s_at | AI803485 | --- | 9875 | -0.81 | 4.93E-03 |
| CYLD | 39582_at | AL050166 | NM_015247 | 1540 | -0.81 | 8.02E-04 |
| MBNL1 | 201151_s_at | BF512200 | NM_021038 | 4154 | -0.80 | 1.13E-03 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| HERC4 | 208055_s_at | NM_015601 | NM_015601 | 26091 | -0.80 | 1.46E-03 |
| AI929792 | 236081_at | AI929792 | --- | --- | -0.80 | 2.95E-03 |
| SYNJ1 | 212990_at | AB020717 | NM_003895 NM_203446 | 8867 | -0.80 | 1.01E-03 |
| C9orf123 | 224860_at | AL137489 | XM_372075 | 90871 | -0.80 | 8.84E-04 |
| C21orf66 | 1556789_a_at | AK056384 | NM_013329 | 94104 | -0.80 | 4.85E-03 |
| | | | NM_016631 | | | |
| | | | NM_058191 | | | |
| | | | NM_145328 | | | |
| BMPR2 | 231873_at | AL046696 | NM_001204 | 659 | -0.80 | 1.31E-04 |
| | | | NM_033346 | | | |
| PCBP3 | 230486_at | BG025371 | NM_020528 | 54039 | -0.80 | 5.61E-04 |
| JAM2 | 229127_at | BF195118 | NM_021219 | 58494 | -0.80 | 4.44E-03 |
| COG6 | 225769_at | AF116827 | NM_020751 | 57511 | -0.80 | 4.32E-03 |
| FLJ21159 | 220145_at | NM_024826 | NM_024826 | 79884 | -0.80 | 5.05E-03 |
| HSD17B12 | 217869_at | NM_016142 | NM_016142 | 51144 | -0.80 | 5.67E-03 |
| SMAP-5 | 224953_at | AW001618 | NM_030799 | 81555 | -0.80 | 5.29E-03 |
| SAV1 | 218276_s_at | NM_021818 | NM_021818 | 60485 | -0.80 | 1.52E-03 |
| PPID | 204186_s_at | A1014573 | NM_005038 | 5481 | -0.80 | 5.72E-04 |
| AZI2 | 227905_s_at | BF000175 | NM_022461 | 64343 | -0.79 | 9.88E-05 |
| | | | NM_203326 | | | |
| BE219874 | 236111_at | BE219874 | --- | --- | -0.79 | 6.26E-03 |
| KPNA1 | 202057_at | BC002374 | NM_002264 | 3836 | -0.79 | 5.21E-03 |
| LOC339803 | 227940_at | N90774 | --- | 339803 | -0.79 | 1.13E-04 |
| ALKBH | 236721_at | AI922200 | NM_006020 | 8846 | -0.79 | 2.25E-03 |
| AW237752 | 229699_at | AW237752 | --- | --- | -0.79 | 8.75E-03 |
| SLC22A17 | 218675_at | NM_020372 | NM_016609 | 51310 | -0.79 | 1.71E-04 |
| | | | NM_020372 | | | |
| TOB1 | 228834_at | BF240286 | NM_005749 | 10140 | -0.79 | 7.87E-04 |
| MED28 | 224416_s_at | AF358829 | NM_025205 | 80306 | -0.79 | 1.39E-04 |
| ZNF277 | 1555193_a_at | BC020626 | NM_021994 | 11179 | -0.79 | 9.56E-03 |
| WDFY3 | 212598_at | BE348236 | NM_014991 | 23001 | -0.79 | 6.62E-03 |
| | | | NM_178583 | | | |
| | | | NM_178585 | | | |
| SGNE1 | 203889_at | NM_003020 | NM_003020 | 6447 | -0.78 | 3.31E-04 |
| ANKRD32 | 223542_at | AL136560 | NM_032290 | 84250 | -0.78 | 2.09E-04 |
| CLCN3 | 201733_at | AA902971 | NM_001829 | 1182 | -0.78 | 1.25E-06 |
| | | | NM_173872 | | | |
| EIF4E3 | 225939_at | AL161983 | --- | 317649 | -0.78 | 2.79E-03 |
| CARF | 218929_at | NM_017632 | NM_017632 | 55602 | -0.78 | 2.99E-03 |
| DCX | 204851_s_at | AF040254 | NM_000555 | 1641 | -0.78 | 7.79E-03 |
| | | | NM_178151 | | | |
| | | | NM_178152 | | | |
| | | | NM_178153 | | | |
| C1orf25 | 220992_s_at | NM_030934 | NM_030934 | 81627 | -0.78 | 2.30E-03 |
| TNFRSF25 | 219423_x_at | NM_003790 | NM_003790 | 8718 | -0.78 | 8.35E-03 |
| | | | NM_14896S | | | |
| | | | NM_148966 | | | |
| | | | NM_148967 | | | |
| | | | NM_148968 | | | |
| | | | NM_148969 | | | |
| | | | NM_148970 | | | |
| | | | NM_148971 | | | |
| | | | NM_148972 | | | |
| | | | NM_148973 | | | |
| | | | NM_148974 | | | |
| AOF2 | 212348_s_at | AB011173 | NM_00100999 9 | 23028 | -0.78 | 3.47E-03 |
| | | | NM_015013 | | | |
| AW025980 | 241864_x_at | AW025980 | XM_371647 | 389129 | -0.78 | 5.72E-04 |
| ACBD5 | 225663_at | AL047650 | NM_145698 | 91452 | -0.78 | 1.80E-03 |
| FRS2 | 226045_at | AW264515 | NM_006654 | 10818 | -0.78 | 5.02E-03 |
| MYOZ3 | 213955_at | BE673151 | NM_133371 | 91977 | -0.78 | 7.08E-03 |
| DBT | 205369_x_at | J03208 | NM_001918 | 1629 | -0.78 | 5.34E-03 |
| AI061377 | 235233_s_at | A1061377 | --- | --- | -0.78 | 2.35E-03 |
| ENOSF1 | 204142_at | NM_017512 | NM_017512 | 55556 | -0.78 | 2.12E-03 |
| CA5BL | 239106_at | AI801563 | XM_291346 | 340591 | -0.77 | 3.87E-03 |
| GTDC1 | 219770_at | NM_024659 | NM_00100663 6 | 79712 | -0.77 | 5.30E-04 |
| | | | NM_024659 | | | |
| RAB18 | 243496_at | AW367507 | NM_021252 | 22931 | -0.77 | 5.05E-03 |
| AK025253 | 227009_at | AK025253 | --- | --- | -0.77 | 2.24E-03 |
| MRPS6 | 212944_at | AK024896 | NM_032476 | 64968 | -0.77 | 9.32E-03 |
| AFAP | 203563_at | NM_021638 | NM_021638 | 60312 | -0.77 | 4.32E-04 |
| | | | NM_198595 | | | |
| RGS7 | 1554500_a_at | AF493931 | NM_002924 | 6000 | -0.77 | 5.33E-04 |
| QSCN6 | 201482_at | NM_002826 | NM_00100412 8 | 5768 | -0.77 | 5.46E-04 |
| | | | NM_002826 | | | |
| C21orf57 | 227421_at | BF026326 | NM_00100611 4 | 54059 | -0.77 | 2.03E-03 |
| | | | NM_058181 | | | |
| PLEKHA3 | 227658_s_at | BE550332 | NM_019091 | 65977 | -0.77 | 2.93E-04 |
| FLJ10980 | 225327_at | AB037791 | NM_019600 | 56204 | -0.77 | 3.59E-03 |
| PURA | 229167_at | AI129941 | NM_005859 | 5813 | -0.77 | 7.80E-03 |
| FFX-r2-Ec-bioC | FX-r2-Ec-bioC-5 | AFFX-r2-Ec-bioC-5AF | --- | --- | -0.77 | 1.36E-03 |
| HSPA12A | 214434_at | AB007877 | XM_048898 | 259217 | -0.77 | 5.03E-03 |
| ZNF250 | 213858_at | BE350026 | NM_021061 | 58500 | -0.77 | 2.48E-03 |
| FFX-r2-Ec-bioB | FX-r2-Ec-bioB-5 | AFFX-r2-Ec-bioB-5AF | --- | --- | -0.77 | 4.87E-03 |
| PDXK | 222492_at | AW262867 | NM_003681 | 8566 | -0.77 | 7.16E-03 |
| H09780 | 230869_at | H09780 | --- | --- | -0.76 | 5.31E-03 |
| LOC283481 | 230351_at | AW070248 | --- | 283481 | -0.76 | 1.35E-04 |
| LOC128153 | 1552269_at | NM_138796 | NM_138796 | 128153 | -0.76 | 8.35E-03 |
| PTP4A2 | 208617_s_at | AF208850 | NM_003479 | 8073 | -0.76 | 8.88E-05 |
| | | | NM_080391 | | | |
| | | | NM_080392 | | | |
| AB029551 | 201845_s_at | AB029551 | --- | --- | -0.76 | 1.96E-04 |
| KIAA1387 | 226230_at | AA541716 | NM_020463 | 57223 | -0.76 | 1.87E-03 |
| C21orf86 | 226995_at | AV705934 | NM_153454 | 257103 | -0.76 | 5.79E-05 |
| ISYNA1 | 222240_s_at | AL137749 | NM_016368 | 51477 | -0.76 | 3.08E-03 |
| RNF13 | 201780_s_at | NM_007282 | NM_007282 | 11342 | -0.76 | 6.77E-04 |
| | | | NM_183381 | | | |
| | | | NM_183382 | | | |
| | | | NM_183383 | | | |
| | | | NM_183384 | | | |
| JMJD2C | 209984_at | AB037901 | NM_015061 | 23081 | -0.76 | 3.62E-03 |
| GTDC1 | 238585_at | AW407668 | NM_00100663 6 | 79712 | -0.76 | 1.71E-03 |
| | | | NM_024659 | | | |
| AI955713 | 227193_at | AI955713 | --- | --- | -0.76 | 2.70E-03 |
| FKBP1A | 210187_at | BC005147 | NM_000801 | 2280 | -0.76 | 7.11E-03 |
| | | | NM_054014 | | | |
| AW117322 | 239415_at | AW117322 | XM_374094 | 389237 | -0.76 | 5.02E-03 |
| BBP | 213883_s_at | AA012917 | NM_032027 | 83941 | -0.76 | 5.76E-03 |
| LOC284804 | 1559161_at | AI871356 | --- | 284804 | -0.75 | 6.44E-03 |
| DJ159A19.3 | 205002_at | NM_015699 | XM_372774 | 27245 | -0.75 | 8.45E-04 |
| AFFX-BioDn-3A | FFX-BioDn-3_a | AFFX-BioDn-3 | --- | --- | -0.75 | 2.83E-03 |
| LAMB1 | 201505_at | NM_002291 | NM_002291 | 3912 | -0.75 | 9.18E-03 |
| POLR3A | 227872_at | AA527570 | NM_007055 | 11128 | -0.75 | 9.27E-04 |
| CDC2L6 | 212899_at | AB028951 | NM_015076 | 23097 | -0.75 | 2.97E-05 |
| GRK4 | 241989_at | AW269179 | NM_00100405 6 | 2868 | -0.75 | 9.15E-03 |
| | | | NM_00100405 7 | | | |
| | | | NM_005307 | | | |
| | | | NM_182982 | | | |
| FEV | 207260_at | NM_017521 | NM_017521 | 54738 | -0.75 | 5.52E-04 |
| MGC35274 | 226748_at | AI674731 | NM_153374 | 256586 | -0.75 | 6.51E-03 |
| PRKCB1 | 209685_s_at | M13975 | NM_002738 | 5579 | -0.75 | 4.16E-03 |
| | | | NM_212535 | | | |
| OXR1 | 222553_x_at | AL541048 | NM_181354 | 55074 | -0.75 | 3.29E-03 |
| ARP3BETA | 218868_at | NM_020445 | NM_020445 | 57180 | -0.75 | 9.91E-03 |
| PFN2 | 237116_at | AI262277 | NM_002628 | 5217 | -0.74 | 8.35E-03 |
| | | | NM_053024 | | | |
| LZTFL1 | 218437_s_at | NM_020347 | NM_020347 | 54585 | -0.74 | 3.96E-03 |
| C6orf96 | 220329_s_at | NM_017909 | NM_017909 | 55005 | -0.74 | 1.80E-03 |
| BE890365 | 222738_at | BE890365 | --- | --- | -0.74 | 9.64E-03 |
| BG112359 | 225785_at | BG112359 | --- | --- | -0.74 | 2.08E-03 |
| ICA1 | 204002_s_at | NM_022307 | NM_004968 | 3382 | -0.74 | 2.94E-03 |
| | | | NM_022307 | | | |
| | | | NM_022308 | | | |
| ENOSF1 | 213645_at | AF305057 | NM_017512 | 55556 | -0.74 | 4.62E-03 |
| CAMK2D | 228555_at | AA029441 | NM_001221 | 817 | -0.74 | 1.79E-03 |
| | | | NM_172115 | | | |
| | | | NM_172127 | | | |
| | | | NM_172128 | | | |
| AI889373 | 227414_at | AI889373 | --- | --- | -0.74 | 1.82E-03 |
| GPR27 | 227769_at | AI703476 | NM_018971 | 2850 | -0.74 | 8.05E-03 |
| AK055769 | 1558801_at | AK055769 | --- | --- | -0.74 | 7.07E-03 |
| AFFX-BioB-5 | AFFX-BioB-5_a | AFFX-BioB-5 | --- | --- | -0.73 | 3.91E-03 |
| AA928542 | 226831_at | AA928542 | --- | --- | -0.73 | 8.48E-04 |
| PAM | 202336_s_at | NM_000919 | NM_000919 | 5066 | -0.73 | 2.84E-03 |
| | | | NM_13766 | | | |
| | | | NM_138821 | | | |
| | | | NM_138822 | | | |
| THNSL1 | 222931_s_at | AI809864 | NM_024838 | 79896 | -0.73 | 3.88E-03 |
| MGC14595 | 227836_at | AI859767 | NM_032334 | 84294 | -0.73 | 6.40E-03 |
| PRKCE | 226101_at | AI093546 | NM_005400 | 5581 | -0.73 | 3.23E-03 |
| LOC92482 | 213224_s_at | AK025724 | XM_378250 | 92482 | -0.73 | 5.89E-03 |
| AKAP14 | 228493_at | T87628 | NM_00100853 4 | 158798 | -0.73 | 8.43E-03 |
| | | | NM_00100853 5 | | | |
| | | | NM_178813 | | | |
| DUSP16 | 224336_s_at | AB052156 | NM_030640 | 80824 | -0.73 | 4.69E-04 |
| IRF2BP2 | 224571_at | BF968057 | NM_182972 | 359948 | -0.73 | 6.05E-03 |
| C6orf117 | 243177_at | AI912225 | NM_138409 | 112609 | -0.73 | 7.88E-03 |
| ADD2 | 205268_s_at | NM_017488 | NM_001617 | 119 | -0.73 | 4.20E-07 |
| | | | NM_017482 | | | |
| | | | NM_017483 | | | |
| | | | NM_017484 | | | |
| | | | NM_017485 | | | |
| | | | NM_017486 | | | |
| | | | NM_017487 | | | |
| | | | NM_017488 | | | |
| PCSK6 | 207414_s_at | NM_002570 | NM_002570 | 5046 | -0.73 | 3.49E-03 |
| | | | NM_138319 | | | |
| | | | NM_138320 | | | |
| | | | NM_138321 | | | |
| | | | NM_138322 | | | |
| | | | NM_138323 | | | |
| | | | NM_138324 | | | |
| | | | NM_138325 | | | |
| H62221 | 243414_at | H62221 | --- | --- | -0.73 | 2.65E-03 |
| AFFX-BioC-5 | AFFX-BioC-5_a | AFFX-BioC-5 | --- | --- | -0.73 | 6.02E-04 |
| BF342524 | 235074_at | BF342524 | --- | --- | -0.73 | 2.25E-03 |
| LOC96610 | 226802_s_at | W87523 | | 96610 | -0.73 | 9.29E-03 |
| | | | NM_080926 | | | |
| BE645776 | 227288_at | BE645776 | XM_068632 | 133993 | -0.73 | 7.66E-04 |
| KIAA0423 | 213304_at | AI823592 | NM_015091 | 23116 | -0.73 | 2.19E-03 |
| ENOSF1 | 204143_s_at | NM_017512 | NM_017512 | 55556 | -0.73 | 2.84E-05 |
| DSCR5 | 221689_s_at | AB035745 | NM_016430 | 51227 | -0.73 | 4.01E-05 |
| | | | NM_153681 | | | |
| | | | NM_153682 | | | |
| MGC54289 | 225228_at | AI474054 | NM_178454 | 128338 | -0.72 | 2.77E-05 |
| AK026379 | 232687_at | AK026379 | --- | --- | -0.72 | 1.93E-04 |
| MGC72075 | 228600_x_at | BE220330 | NM_199136 | 340277 | -0.72 | 5.28E-04 |
| ASH1L | 226447_at | BG290742 | NM_018489 | 55870 | -0.72 | 2.78E-03 |
| KIAA0974 | 213463_s_at | AW300504 | --- | 317662 | -0.72 | 4.74E-04 |
| C6orf157 | 227790_at | AV692609 | NM_198920 | 90025 | -0.72 | 3.66E-03 |
| CLCN4 | 214769_at | AF052117 | NM_001830 | 1183 | -0.72 | 3.22E-04 |
| LOC440360 | 236838_at | H11609 | XM_498641 | 440360 | -0.72 | 2.56E-03 |
| SYNCRIP | 217833_at | AL520908 | NM_006372 | 10492 | -0.72 | 4.62E-04 |
| FFX-r2-Ec-bioB | FX-r2-Ec-bioB-N | AFFX-r2-Ec-bioB-M | --- | --- | -0.72 | 7.96E-03 |
| AKT3 | 212609_s_at | U79271 | NM_005465 | 10000 | -0.72 | 5.74E-03 |
| | | | NM_181690 | | | |
| PP1057 | 220968_s_at | NM_031285 | --- | 83441 | -0.72 | 6.75E-03 |
| GM2A | 1559776_at | H41167 | NM_000405 | 2760 | -0.72 | 1.46E-03 |
| FLJ37228 | 1556641_at | AK094547 | --- | 285264 | -0.72 | 6.35E-03 |
| NAV1 | 227584_at | BF339566 | NM_020443 | 89796 | -0.71 | 9.22E-05 |
| MGC10198 | 225574_at | BE613081 | NM_152682 | 201965 | -0.71 | 1.36E-03 |
| PPP2R5C | 201877_s_at | NM_002719 | NM_002719 | 5527 | -0.71 | 1.25E-03 |
| | | | NM_178586 | | | |
| | | | NM_178587 | | | |
| | | | NM_178588 | | | |
| RAFTLIN | 212646_at | D42043 | NM_015150 | 23180 | -0.71 | 7.61E-06 |
| DKFZP564O243 | 210006_at | BC002571 | NM_015407 | 25864 | -0.71 | 5.84E-03 |
| SLC35A5 | 218519_at | NM_017945 | NM_017945 | 55032 | -0.71 | 5.19E-03 |
| BF526978 | 227125_at | BF526978 | --- | --- | -0.71 | 2.88E-03 |
| RBM16 | 227866_at | AI743557 | NM_014892 | 22828 | -0.71 | 4.70E-04 |
| gm117 | 239038_at | AW015063 | NM_198077 | 148423 | -0.71 | 2.85E-05 |
| AB058893 | 223973_at | AB058893 | --- | --- | -0.71 | 3.00E-03 |
| DUSP16 | 224832_at | AB051487 | NM_030640 | 80824 | -0.71 | 8.84E-04 |
| FFX-r2-Ec-bioC | X-r2-Ec-bioC-3 | AFFX-r2-Ec-bioC-3 | --- | --- | -0.71 | 2.47E-03 |
| AL355532 | 213845_at | AL355532 | --- | --- | -0.71 | 1.72E-03 |
| LOC284409 | 242016_at | BE857296 | XM_211447 | 284409 | -0.70 | 7.46E-03 |
| PAK1 | 226507_at | AU154408 | NM_002576 | 5058 | -0.70 | 1.89E-03 |
| BC015852 | 1560570_a_at | BC015852 | --- | --- | -0.70 | 5.93E-04 |
| EPM2AIP1 | 227847_at | BF432224 | NM_014805 | 9852 | -0.70 | 6.03E-03 |
| 13CDNA73 | 204072_s_at | NM_023037 | NM_023037 | 10129 | -0.70 | 1.50E-04 |
| DECR1 | 202447_at | NM_001359 | NM_001359 | 1666 | -0.70 | 7.20E-03 |
| USP38 | 223288_at | AW977401 | NM_032557 | 84640 | -0.70 | 6.63E-03 |
| RAB4A | 203581_at | BC002438 | NM_004578 | 5867 | -0.70 | 1.97E-03 |
| PLEKHA3 | 223370_at | AF286162 | NM_019091 | 65977 | -0.70 | 4.19E-03 |
| AFFX-BioC-3 | AFFFX-BioC-3_a | AFFX-BioC-3 | --- | --- | -0.70 | 1.62E-03 |
| PPID | 204185_x_at | NM_005038 | NM_005038 | 5481 | -0.70 | 5.63E-04 |
| PAM | 214620_x_at | BF038548 | NM_000919 | 5066 | -0.70 | 2.61E-04 |
| | | | NM_138766 | | | |
| | | | NM_138821 | | | |
| | | | NM_138822 | | | |
| ARL7 | 202207_at | BG435404 | NM_005737 | 10123 | -0.70 | 1.62E-03 |
| DNAJC6 | 204720_s_at | AV729634 | NM_014787 | 9829 | -0.70 | 2.00E-04 |
| SYNCOILIN | 225396_at | AI928212 | NM_030786 | 81493 | -0.70 | 5.70E-03 |
| ANXA5 | 200782_at | NM_001154 | NM_001154 | 308 | -0.70 | 5.88E-03 |
| MGC14595 | 238561_s_at | BE542779 | NM_032334 | 84294 | -0.70 | 6.39E-05 |
| SELM | 226051_at | BF973568 | NM_080430 | 140606 | -0.70 | 4.02E-03 |
| AA489100 | 228390_at | AA489100 | --- | --- | -0.70 | 5.43E-03 |
| AA740675 | 244032_at | AA740675 | --- | --- | -0.70 | 9.18E-04 |
| ALDH5A1 | 203608_at | AL031230 | NM_001080 | 7915 | -0.69 | 3.13E-03 |
| | | | NM_170740 | | | |
| BE646451 | 235893_at | BE646451 | --- | --- | -0.69 | 9.91E-03 |
| DUSP7 | 213848_at | AI655015 | NM_001947 | 1849 | -0.69 | 9.16E-03 |
| MGC42174 | 242539_at | AW665509 | NM_152383 | 129563 | -0.69 | 2.67E-03 |
| FBXO25 | 225591_at | AA749085 | NM_012173 | 26260 | -0.69 | 4.48E-03 |
| | | | NM_183420 | | | |
| | | | NM_183421 | | | |
| CNOT6L | 226153_s_at | AW514857 | NM_144571 | 246175 | -0.69 | 4.37E-04 |
| NPR2 | 204310_s_at | NM_003995 | NM_000907 | 4882 | -0.69 | 5.97E-03 |
| | | | NM_003995 | | | |
| DEPDC6 | 218858_at | NM_022783 | NM_022783 | 64798 | -0.69 | 2.62E-03 |
| GARNL4 | 213280_at | AK000478 | NM_015085 | 23108 | -0.69 | 5.49E-03 |
| ANKH | 223093_at | T99215 | NM_054027 | 56172 | -0.69 | 3.95E-03 |
| NUDT11 | 219855_at | NM_018159 | NM_018159 | 55190 | -0.69 | 1.75E-03 |
| IFNAR2 | 204785_x_at | NM_000874 | NM_000874 | 3455 | -0.69 | 7.44E-05 |
| | | | NM_207584 | | | |
| | | | NM_207585 | | | |
| ZNF148 | 235166_at | T08836 | NM_021964 | 7707 | -0.68 | 9.51E-03 |
| LOC377064 | 229986_at | AW205616 | XM_496648 | 377064 | -0.68 | 3.34E-03 |
| RSL1D1 | 212019_at | AK025446 | NM_015659 | 26156 | -0.68 | 2.80E-03 |
| C10orf22 | 212500_at | AL049319 | NM_032804 | 84890 | -0.68 | 8.22E-03 |
| DAZAP2 | 214334_x_at | N34846 | NM_014764 | 9802 | -0.68 | 2.12E-03 |
| WDFY3 | 212606_at | AL536319 | NM_014991 | 23001 | -0.68 | 8.12E-05 |
| | | | NM_178583 | | | |
| | | | NM_178585 | | | |
| PTPN23 | 223150_s_at | AF290614 | NM_015466 | 25930 | -0.68 | 2.67E-03 |
| SNAP91 | 204953_at | NM_014841 | NM_014841 | 9892 | -0.68 | 1.47E-04 |
| DSU | 232682_at | BG167210 | NM_018000 | 55686 | -0.68 | 2.28E-04 |
| ZNF490 | 231988_x_at | AB033024 | NM_020714 | 57474 | -0.68 | 8.43E-04 |
| FFX-r2-Ec-bioD | FX-r2-Ec-bioD-3 | AFFX-r2-Ec-bioD-3 | --- | --- | -0.68 | 8.66E-03 |
| ZBTB1 | 242258_at | AW168154 | NM_014950 | 22890 | -0.68 | 7.93E-03 |
| FFX-r2-Ec-bioB | FX-r2-Ec-bioB-3 | AFFX-r2-Ec-bioB-3 | --- | --- | -0.68 | 4.88E-03 |
| DLG3 | 212727_at | AB033058 | NM_021120 | 1741 | -0.68 | 2.75E-05 |
| P15RS | 225953_at | AI684281 | NM_018170 | 55197 | -0.68 | 4.56E-03 |
| LRBA | 212692_s_at | W60686 | NM_006726 | 987 | -0.68 | 1.07E-06 |
| ZBED3 | 228402_at | AI679968 | NM_032367 | 84327 | -0.68 | 2.51E-03 |
| DNASE1 L1 | 203912_s_at | NM_006730 | NM_00100993 2 | 1774 | -0.68 | 4.97E-03 |
| | | | NM_00100993 3 | | | |
| | | | NM_00100993 4 | | | |
| | | | NM_006730 | | | |
| ABR | 212895_s_at | AL527773 | NM_001092 | 29 | -0.68 | 9.54E-04 |
| | | | NM_021962 | | | |
| AF147335 | 1557518_a_at | AF147335 | --- | --- | -0.68 | 3.85E-03 |
| HNRPD | 229129_at | AI948456 | NM_00100381 0 | 3184 | -0.68 | 9.99E-03 |
| | | | NM_002138 | | | |
| | | | NM_031369 | | | |
| | | | NM_031370 | | | |
| LOC387895 | 243905_at | AI652676 | XM_373553 | 387895 | -0.68 | 4.51E-03 |
| PLCE1 | 205111_s_at | NM_016341 | NM_016341 | 51196 | -0.68 | 3.83E-04 |
| ANTXR2 | 228573_at | BE673665 | NM_058172 | 118429 | -0.68 | 3.75E-03 |
| MGC48625 | 1569039_s_at | BC029855 | NM_182609 | 342926 | -0.68 | 2.30E-04 |
| MGC47869 | 229888_at | AA861435 | NM_175874 | 144608 | -0.68 | 3.57E-03 |
| ZNF197 | 233070_at | AL117657 | NM_006991 | 10168 | -0.68 | 1.50E-04 |
| DYRK2 | 202969_at | AI216690 | NM_003583 | 8445 | -0.68 | 3.24E-03 |
| | | | NM_006482 | | | |
| LOC285513 | 1556697_at | AI819722 | NM_198281 | 285513 | -0.67 | 1.21E-05 |
| CRYZL1 | 219767_s_at | NM_005111 | NM_005111 | 9946 | -0.67 | 6.15E-03 |
| | | | NM_145311 | | | |
| | | | NM_145858 | | | |
| AA935461 | 242579_at | AA935461 | --- | --- | -0.67 | 3.50E-04 |
| FLJ30990 | 1554588_a_at | BC033795 | NM_152517 | 150737 | -0.67 | 7.72E-03 |
| RIPK5 | 229017_s_at | N31717 | NM_015375 | 25778 | -0.67 | 5.71E-03 |
| | | | NM_199462 | | | |
| N63920 | 226179_at | N63920 | --- | --- | -0.67 | 9.08E-03 |
| NM_022335 | 206936_x_at | NM_022335 | --- | --- | -0.67 | 1.76E-04 |
| SLC25A12 | 203340_s_at | NM_003705 | NM_003705 | 8604 | -0.67 | 6.85E-03 |
| MED28 | 218438_s_at | NM_025205 | NM_025205 | 80306 | -0.67 | 1.38E-03 |
| RAD52 | 236042_at | AI700506 | NM_002879 | 5893 | -0.67 | 1.28E-04 |
| | | | NM_134422 | | | |
| | | | NM_134423 | | | |
| | | | NM_134424 | | | |
| EPHA8 | 231796_at | AL035703 | NM_00100694 3 | 2046 | -0.67 | 5.79E-03 |
| | | | NM_020526 | | | |
| DSU | 219648_at | NM_018000 | NM_018000 | 55686 | -0.67 | 3.20E-04 |
| AA843541 | 229710_at | AA843541 | --- | --- | -0.67 | 9.95E-04 |
| AA461190 | 244294_at | AA461190 | --- | --- | -0.67 | 9.45E-03 |
| JAM2 | 219213_at | NM_021219 | NM_021219 | 58494 | -0.66 | 1.52E-03 |
| CDK6 | 224847_at | AW051349 | NM_001259 | 1021 | -0.66 | 3.40E-03 |
| MCM3APAS | 1560542_at | BC016306 | --- | 114044 | -0.66 | 9.71E-03 |
| LOC157657 | 244569_at | N92755 | NM_177965 | 157657 | -0.66 | 1.12E-04 |
| LOC286334 | 226590_at | AA910497 | --- | 286334 | -0.66 | 5.51E-03 |
| C3orf6 | 225331_at | BF941088 | NM_174908 | 152137 | -0.66 | 4.75E-03 |
| | | | NM_178335 | | | |
| KIAA1579 | 231851_at | AL359613 | NM_018211 | 55225 | -0.66 | 4.44E-03 |
| KIAA0241 | 212474_at | D87682 | NM_015060 | 23080 | -0.66 | 2.52E-03 |
| TFAP2B | 214451_at | NM_003221 | NM_003221 | 7021 | -0.66 | 6.69E-04 |
| AI218542 | 236089_at | AI218542 | --- | --- | -0.66 | 8.36E-03 |
| EEF2K | 225546_at | W68180 | NM_013302 | 29904 | -0.66 | 1.99E-03 |
| KIAA0999 | 204157_s_at | NM_025164 | NM_025164 | 23387 | -0.65 | 3.68E-05 |
| FXR1 | 201637_s_at | NM_005087 | NM_005087 | 8087 | -0.65 | 8.08E-03 |
| AA406435 | 230747_s_at | AA406435 | --- | --- | -0.65 | 6.70E-03 |
| ZNF626 | 235687_at | AA908777 | NM_145297 | 199777 | -0.65 | 5.29E-03 |
| KIAA0582 | 212675_s_at | AB011154 | NM_015147 | 23177 | -0.65 | 7.85E-03 |
| DTNA | 227084_at | AW339310 | NM_001390 | 1837 | -0.65 | 3.98E-03 |
| | | | NM_001391 | | | |
| | | | NM_001392 | | | |
| | | | NM_032975 | | | |
| | | | NM_032978 | | | |
| | | | NM_032979 | | | |
| | | | NM_032980 | | | |
| | | | NM_032981 | | | |
| C9orf109 | 229059_at | AI765785 | XM_379665 | 286333 | -0.65 | 1.91E-03 |
| TBC1D12 | 1557609_s_at | AW188458 | XM_051081 | 23232 | -0.65 | 4.03E-03 |
| LIN7C | 223350_x_at | N63709 | NM_018362 | 55327 | -0.65 | 3.22E-03 |
| PIK3R2 | 1568629_s_at | BC033311 | NM_005027 | 5296 | -0.65 | 7.91E-05 |
| AU151635 | 232111_at | AU151635 | --- | --- | -0.65 | 6.09E-04 |
| NM_018678 | 220494_s_at | NM_018678 | --- | --- | -0.65 | 4.32E-03 |
| FAM8A1 | 203420_at | NM_016255 | NM_016255 | 51439 | -0.65 | 8.87E-03 |
| AL136588 | 223614_at | AL136588 | --- | --- | -0.65 | 3.95E-04 |
| KIAA0179 | 212846_at | AA811192 | NM_015056 | 23076 | -0.65 | 7.52E-03 |
| BF509796 | 229318_at | BF509796 | --- | --- | -0.65 | 2.51E-03 |
| SEC31L1 | 200945_s_at | NM_014933 | NM_014933 | 22872 | -0.65 | 8.63E-04 |
| | | | NM_016211 | | | |
| KIAA0711 | 204301_at | NM_014867 | NM_014867 | 9920 | -0.65 | 1.44E-03 |
| AW025141 | 228400_at | AW025141 | --- | --- | -0.64 | 6.23E-03 |
| SERP1 | 235475_at | AI580135 | NM_014445 | 27230 | -0.64 | 8.89E-03 |
| C2orf32 | 226751_at | AW193693 | NM_015463 | 25927 | -0.64 | 6.94E-04 |
| KIAA1935 | 228336_at | AW511595 | XM_087672 | 114825 | -0.64 | 2.59E-03 |
| PDE4D | 228962_at | BF507941 | NM_006203 | 5144 | -0.64 | 2.48E-03 |
| CDC14B | 221556_at | BF792631 | NM_003671 | 8555 | -0.64 | 2.02E-03 |
| | | | NM_033331 | | | |
| | | | NM_033332 | | | |
| FANCF | 218689_at | NM_022725 | NM_022725 | 2188 | -0.64 | 5.25E-03 |
| BCAR3 | 204032_at | NM_003567 | NM_003567 | 8412 | -0.64 | 3.94E-03 |
| UBE2D3 | 200667_at | BF448062 | NM_003340 | 7323 | -0.64 | 2.05E-03 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| MGC29463 | 238115_at | T03492 | NM_152686 | 202052 | -0.64 | 4.51E-03 |
| AFFX-BioDn-5A | FFX-BioDn-5_a | AFFX-BioDn-5 | --- | --- | -0.64 | 1.45E-03 |
| TPD52 | 201690_s_at | AA524023 | NM_005079 | 7163 | -0.64 | 8.25E-04 |
| HPSE | 222881_at | AF155510 | NM_006665 | 10855 | -0.64 | 2.12E-03 |
| MAN1A2 | 217920_at | H97940 | NM_006699 | 10905 | -0.64 | 2.61E-03 |
| HRMT1L1 | 221564_at | AL570294 | NM_001535 | 3275 | -0.64 | 5.66E-03 |
| | | | NM_206962 | | | |
| C22orf9 | 227144_at | AA476916 | NM_00100988 0 | 23313 | -0.63 | 8.11E-03 |
| | | | NM_015264 | | | |
| RGS12 | 1555022_at | AF464736 | NM_002926 | 6002 | -0.63 | 3.89E-05 |
| | | | NM_198227 | | | |
| | | | NM_198229 | | | |
| | | | NM_198230 | | | |
| | | | NM_198430 | | | |
| | | | NM_198432 | | | |
| | | | NM_198587 | | | |
| FFX-r2-Ec-bioD | FX-r2-Ec-bioD-5 | AFFX-r2-Ec-bioD-5 | --- | --- | -0.63 | 3.52E-03 |
| AI766065 | 224647_at | AI766065 | --- | --- | -0.63 | 4.41E-04 |
| TDRD3 | 1569069_s_at | BC020604 | NM_030794 | 81550 | -0.63 | 4.16E-03 |
| SOS1 | 227426_at | AV702692 | NM_005633 | 6654 | -0.63 | 3.92E-03 |
| SMAD2 | 203077_s_at | NM_005901 | NM_00100365 2 | 4087 | -0.63 | 7.24E-03 |
| | | | NM_005901 | | | |
| MED28 | 228992_at | BF064162 | NM_025205 | 80306 | -0.63 | 2.06E-05 |
| BICD1 | 1556051_a_at | CA777994 | NM_00100339 8 | 636 | -0.63 | 6.24E-04 |
| | | | NM_001714 | | | |
| GOLPH3L | 218361_at | NM_018178 | NM_018178 | 55204 | -0.63 | 9.58E-04 |
| MBNL1 | 201152_s_at | N31913 | NM_021038 | 4154 | -0.63 | 4.11E-05 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| LZTFL1 | 222632_s_at | AA843132 | NM_020347 | 54585 | -0.63 | 4.52E-04 |
| RUFY1 | 233380_s_at | AA429145 | NM_025158 | 80230 | -0.63 | 8.27E-04 |
| RBM18 | 238963_at | BG106093 | NM_033117 | 92400 | -0.63 | 1.19E-03 |
| UBXD1 | 220757_s_at | NM_025241 | NM_025241 | 80700 | -0.63 | 9.92E-04 |
| NCAM1 | 227394_at | W94001 | NM_000615 | 4684 | -0.63 | 2.48E-03 |
| | | | NM_181351 | | | |
| C14orf4 | 223474_at | AI932310 | NM_024496 | 64207 | -0.63 | 3.20E-04 |
| PEX11A | 205161_s_at | NM_003847 | NM_003847 | 8800 | -0.63 | 1.24E-04 |
| BF692729 | 1558044_s_at | BF692729 | --- | --- | -0.62 | 1.31E-03 |
| AW290940 | 226773_at | AW290940 | --- | --- | -0.62 | 4.57E-04 |
| DDHD1 | 225970_at | AA029818 | NM_030637 | 80821 | -0.62 | 2.27E-03 |
| C9orf10OS | 239391_at | AA195528 | NM_198841 | 158293 | -0.62 | 7.21E-03 |
| AI694544 | 235215_at | AI694544 | --- | --- | -0.62 | 3.54E-03 |
| TMEM45A | 219410_at | NM_018004 | NM_018004 | 55076 | -0.62 | 1.39E-03 |
| CXorf38 | 228700_at | AA063608 | NM_144970 | 159013 | -0.62 | 5.45E-03 |
| LOC57149 | 203897_at | BE963444 | NM_020424 | 57149 | -0.62 | 2.10E-03 |
| LOC54499 | 208715_at | BF002031 | NM_019026 | 54499 | -0.62 | 1.19E-03 |
| SCAMP1 | 212425_at | AL049223 | NM_004866 | 9522 | -0.62 | 8.35E-03 |
| | | | NM_052822 | | | |
| IMPACT | 222698_s_at | AF208694 | NM_018439 | 55364 | -0.62 | 7.05E-03 |
| TRPM1 | 243741_at | AW665484 | NM_002420 | 4308 | -0.62 | 7.71E-03 |
| TMAP1 | 227326_at | BE966768 | NM_00100852 8 | 439921 | -0.62 | 4.82E-03 |
| | | | NM_00100852 9 | | | |
| | | | NM_198530 | | | |
| NLK | 222589_at | AI967933 | NM_016231 | 51701 | -0.62 | 9.48E-03 |
| KIAA0280 | 36612_at | D87470 | XM_370635 | 23201 | -0.62 | 7.88E-03 |
| SYNCRIP | 236146_at | BF593158 | NM_006372 | 10492 | -0.62 | 2.46E-04 |
| NAV1 | 224772_at | AB032977 | NM_020443 | 89796 | -0.61 | 1.83E-03 |
| AI216347 | 242087_x_at | AI216347 | --- | --- | -0.61 | 7.98E-03 |
| RTN4R | 228550_at | NM_023004 | NM_023004 | 65078 | -0.61 | 2.34E-03 |
| TLOC1 | 225352_at | AI763287 | NM_003262 | 7095 | -0.61 | 1.21E-03 |
| AL137344 | 232390_at | AL137344 | --- | --- | -0.61 | 8.75E-06 |
| COMMD2 | 226910_at | AW008502 | NM_016094 | 51122 | -0.61 | 9.68E-03 |
| LOC57228 | 209679_s_at | BC003379 | NM_020467 | 57228 | -0.61 | 9.52E-03 |
| AW873330 | 225674_at | AW873330 | --- | --- | -0.61 | 7.46E-03 |
| RAD50 | 208393_s_at | NM_005732 | NM_005732 | 10111 | -0.61 | 2.10E-06 |
| | | | NM_133482 | | | |
| ZFY | 230760_at | BF592062 | NM_003411 | 7544 | -0.61 | 8.36E-05 |
| C21orf33 | 202217_at | NM_004649 | NM_004649 | 8209 | -0.61 | 9.07E-03 |
| | | | NM_198155 | | | |
| PAM | 212958_x_at | AI022882 | NM_000919 | 5066 | -0.61 | 1.54E-03 |
| | | | NM_138766 | | | |
| | | | NM_138821 | | | |
| | | | NM_138822 | | | |
| PDCD2 | 204025_s_at | NM_002598 | NM_002598 | 5134 | -0.61 | 4.97E-03 |
| | | | NM_144781 | | | |
| AHI1 | 221569_at | AL136797 | NM_017651 | 54806 | -0.61 | 2.59E-03 |
| TPD52 | 201689_s_at | BE974098 | NM_005079 | 7163 | -0.61 | 5.72E-03 |
| AV721564 | 227620_at | AV721564 | --- | --- | -0.61 | 9.48E-03 |
| ZNF540 | 229328_at | T90358 | NM_152606 | 163255 | -0.61 | 8.93E-03 |
| DKFZp761D19 | | | XM_375608 | 400692 | | |
| GAB1 | 226002_at | AK022142 | NM_002039 | 2549 | -0.60 | 4.32E-04 |
| | | | NM_207123 | | | |
| R02709 | 242149_at | R02709 | --- | --- | -0.60 | 8.54E-03 |
| DCX | 204850_s_at | NM_000555 | NM_000555 | 1641 | -0.60 | 4.23E-03 |
| | | | NM_178151 | | | |
| | | | NM_178152 | | | |
| | | | NM_178153 | | | |
| PPARG | 208510_s_at | NM_015869 | NM_005037 | 5468 | -0.60 | 1.82E-03 |
| | | | NM_01S869 | | | |
| | | | NM_138711 | | | |
| | | | NM_138712 | | | |
| PCYOX1 | 225274_at | BF247054 | NM_016297 | 51449 | -0.60 | 6.39E-03 |
| M17S2 | 201384_s_at | NM_005899 | NM_005899 | 4077 | -0.60 | 9.84E-04 |
| | | | NM_031858 | | | |
| | | | NM_031862 | | | |
| KBTBD7 | 223412_at | AL136782 | NM_032138 | 84078 | -0.60 | 3.40E-03 |
| AK094546 | 1556205_at | AK094546 | --- | --- | -0.60 | 8.73E-03 |
| GAJ | 223700_at | AY028916 | NM_032117 | 84057 | -0.60 | 2.55E-03 |
| BACE1 | 222462_s_at | AI653425 | NM_012104 | 23621 | -0.60 | 5.62E-04 |
| | | | NM_138971 | | | |
| | | | NM_138972 | | | |
| | | | NM_138973 | | | |
| RAB26 | 50965_at | AI690165 | NM_014353 | 25837 | -0.60 | 2.77E-03 |
| AGPAT4 | 228667_at | AI733330 | NM_020133 | 56895 | -0.60 | 7.61E-03 |
| LOC134147 | 227522_at | AA209487 | NM_138809 | 134147 | -0.60 | 9.04E-03 |
| MRPS30 | 222275_at | AI039469 | NM_016640 | 10884 | -0.60 | 1.13E-03 |
| AV699883 | 230782_at | AV699883 | --- | --- | -0.60 | 1.72E-03 |
| DJ12208.2 | 227797_x_at | AI652464 | NM_020466 | 57226 | -0.60 | 5.64E-03 |
| ULK2 | 204062_s_at | BG526973 | NM_014683 | 9706 | -0.60 | 2.35E-03 |
| AL552727 | 244080_at | AL552727 | --- | --- | -0.60 | 4.85E-03 |
| DDEF1 | 224791_at | AW513835 | NM_018482 | 50807 | -0.60 | 1.40E-04 |
| TIAM1 | 213135_at | U90902 | NM_003253 | 7074 | -0.60 | 7.17E-03 |
| HOXC6 | 206858_s_at | NM_004503 | NM_004503 | 3223 | -0.60 | 3.44E-03 |
| | | | NM_153693 | | | |
| POFUT2 | 209578_s_at | BC000626 | NM_015227 | 23275 | -0.60 | 1.08E-03 |
| | | | NM_133634 | | | |
| | | | NM_133635 | | | |
| ZFP36L2 | 201368_at | U07802 | NM_006887 | 678 | -0.60 | 1.13E-03 |
| BC016176 | 1561529_at | BC016176 | --- | --- | -0.60 | 5.78E-03 |
| CNOT8 | 202163_s_at | NM_004779 | NM_004779 | 9337 | -0.60 | 7.52E-03 |
| LOC124220 | 228058_at | AI559190 | NM_145252 | 124220 | -0.59 | 4.64E-03 |
| ZC3HDC1 | 218543_s_at | NM_022750 | NM_022750 | 64761 | -0.59 | 7.38E-03 |
| KIAA1126 | 225598_at | AB032952 | XM_050325 | 57210 | -0.59 | 1.48E-03 |
| W26593 | 213048_s_at | W26593 | --- | --- | -0.59 | 5.40E-03 |
| FLJ20534 | 218646_at | NM_017867 | NM_017867 | 54969 | -0.59 | 1.34E-04 |
| LOC126167 | 1560779_a_at | AK092139 | XM_058997 | 126167 | -0.59 | 2.41E-03 |
| AA449838 | 241411_at | AA449838 | --- | --- | -0.59 | 3.04E-03 |
| FLJ38451 | 235052_at | AV758821 | NM_175872 | 126375 | -0.59 | 3.59E-03 |
| MAP2 | 225540_at | BF342661 | NM_002374 | 4133 | -0.59 | 2.90E-03 |
| | | | NM_031845 | | | |
| | | | NM_031846 | | | |
| | | | NM_031847 | | | |
| CPD | 201941_at | BE349147 | NM_001304 | 1362 | -0.59 | 3.19E-04 |
| RP42 | 218583_s_at | NM_020640 | NM_020640 | 54165 | -0.59 | 3.27E-06 |
| RBMX | 213762_x_at | AI452524 | NM_002139 | 27316 | -0.59 | 2.27E-04 |
| LRIG2 | 238653_at | BF967997 | NM_014813 | 9860 | -0.59 | 4.27E-03 |
| BF431965 | 213567_at | BF431965 | --- | --- | -0.59 | 7.58E-03 |
| TRIM23 | 204732_s_at | AI021991 | NM_001656 | 373 | -0.59 | 9.14E-03 |
| | | | NM_033227 | | | |
| | | | NM_033228 | | | |
| C9orf67 | 224506_s_at | BC006362 | NM_032728 | 84814 | -0.58 | 1.45E-03 |
| AB002295 | 204307_at | AB002295 | --- | --- | -0.58 | 2.09E-03 |
| KIAA1546 | 227624_at | AB046766 | XM_042301 | 57667 | -0.58 | 4.89E-07 |
| PPM1A | 227728_at | AA886888 | NM_021003 | 5494 | -0.58 | 7.82E-03 |
| | | | NM_177951 | | | |
| | | | NM_177952 | | | |
| ZNF148 | 239024_at | AI478771 | NM_021964 | 7707 | -0.58 | 2.84E-04 |
| MARCH-I | 235385_at | AI935334 | NM_017923 | 55016 | -0.58 | 6.65E-03 |
| AB033091 | 225295_at | AB033091 | --- | --- | -0.58 | 3.88E-03 |
| IRF2BP2 | 224570_s_at | AA166696 | NM_182972 | 359948 | -0.58 | 4.89E-04 |
| D31763 | 231864_at | D31763 | --- | --- | -0.58 | 1.86E-03 |
| LOC151146 | 227604_at | BG292251 | --- | 151146 | -0.58 | 8.00E-03 |
| UBE2D3 | 229355_at | AU150386 | NM_003340 | 7323 | -0.58 | 6.55E-06 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| CUTL1 | 214743_at | BE046521 | NM_001913 | 1523 | -0.58 | 3.01E-04 |
| | | | NM_181500 | | | |
| | | | NM_181552 | | | |
| LOC152485 | 228046_at | AA741243 | NM_178835 | 152485 | -0.58 | 8.28E-03 |
| CLPX | 223507_at | AL136922 | NM_006660 | 10845 | -0.58 | 6.77E-03 |
| MAPKAP1 | 222426_at | BG499947 | NM_00100661 7 | 79109 | -0.58 | 3.51E-03 |
| | | | NM_00100661 8 | | | |
| | | | NM_00100661 9 | | | |
| | | | NM_00100662 0 | | | |
| | | | NM_00100662 1 | | | |
| | | | NM_024117 | | | |
| BRUNOL4 | 223654_s_at | BC004167 | NM_020180 | 56853 | -0.58 | 2.18E-03 |
| ARFIP1 | 218230_at | AL044651 | NM_014447 | 27236 | -0.58 | 3.04E-03 |
| SEC5L1 | 226270_at | AL562686 | NM_018303 | 55770 | -0.58 | 8.15E-06 |

**Table 11. Increased Gene Expression in Cell Lines H1 and BB10 vs. SiMa Cell Line in Differentiated State Using Microarray Analysis**

| **Gene Symbol** | **Probe Set ID** | **Representative Public ID** | **RefSeq Transcript ID** | **Entrez Gene** | **Log2 Ratio** | **pvalue** |
|---|---|---|---|---|---|---|
| OKFZp667G211 | 214030_at | BE501352 | XM_376254 | 131544 | 5.13 | 6.09E-05 |
| BF476109 | 1556300_s_at | BF476109 | --- | --- | 5.09 | 2.02E-05 |
| H99792 | 225710_at | H99792 | --- | --- | 4.92 | 2.07E-08 |
| SLITRK5 | 214930_at | AW449813 | NM_015567 | 26050 | 4.63 | 3.87E-06 |
| DAZ1 | 207909_x_at | U21663 | NM_00100537 5 | 1617 | 4.60 | 2.31E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| RGS5 | 218353_at | NM_025226 | NM_003617 | 8490 | 4.54 | 1.63E-03 |
| SIM1 | 206876_at | AL121948 | NM_005068 | 6492 | 4.53 | 2.11E-04 |
| KCTD12 | 212192_at | AI718937 | NM_138444 | 115207 | 4.51 | 4.17E-05 |
| RGS5 | 1555725_a_at | AF493929 | NM_003617 | 8490 | 4.49 | 3.25E-05 |
| FLJ39639 | 243547_at | BE176531 | XM_370932 | 283876 | 4.37 | 2.87E-03 |
| DAZ1 | 208282_x_at | NM_020363 | NM_00100537 5 | 1617 | 4.32 | 3.54E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| PCDH9 | 219737_s_at | AI524125 | NM_020403 | 5101 | 4.30 | 1.19E-03 |
| | | | NM_203487 | | | |
| RGS5 | 209071_s_at | AF159570 | NM_003617 | 8490 | 4.10 | 1.32E-04 |
| RGS5 | 209070_s_at | AI183997 | NM_003617 | 8490 | 4.07 | 1.28E-04 |
| DKFZp761O201 | 232313_at | AL122107 | XM_044062 | 92293 | 4.01 | 2.30E-06 |
| MAB21L2 | 210302_s_at | AF262032 | NM_006439 | 10586 | 3.99 | 2.81E-04 |
| ALCAM | 240655_at | BE502785 | NM_001627 | 214 | 3.98 | 3.07E-05 |
| DAZ1 | 207912_s_at | NM_004081 | NM_00100537 5 | 1617 | 3.96 | 1.79E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| GRP | 206326_at | NM_002091 | NM_002091 | 2922 | 3.94 | 1.52E-05 |
| PDZRN3 | 233442_at | AU147500 | NM_015009 | 23024 | 3.92 | 2.66E-03 |
| MAB21L1 | 206163_at | NM_005584 | NM_005584 | 4081 | 3.91 | 6.73E-06 |
| TIMP3 | 201149_s_at | U67195 | NM_000362 | 7078 | 3.84 | 5.07E-03 |
| TIMP3 | 201147_s_at | BF347089 | NM_000362 | 7078 | 3.61 | 7.18E-03 |
| PDZRN3 | 212915_at | AL569804 | NM_015009 | 23024 | 3.58 | 7.05E-03 |
| LOC440738 | 221697_at | AF276659 | NM_001004343 | 440738 | 3.54 | 3.13E-03 |
| GNG11 | 204115_at | NM_004126 | NM_004126 | 2791 | 3.52 | 6.00E-04 |
| PLK2 | 201939_at | NM_006622 | NM_006622 | 10769 | 3.49 | 8.48E-05 |
| CXCR4 | 217028_at | AJ224869 | NM_00100854 0 | 7852 | 3.49 | 2.53E-05 |
| | | | NM_003467 | | | |
| DLL1 | 224215_s_at | AF196571 | NM_005618 | 28514 | 3.40 | 1.98E-05 |
| LOC340109 | 1557765_at | BC039509 | XM_379322 | 340109 | 3.33 | 4.27E-04 |
| CCDC3 | 223316_at | AL136562 | NM_031455 | 83643 | 3.32 | 3.45E-05 |
| P2RY5 | 218589_at | NM_005767 | NM_005767 | 10161 | 3.27 | 1.73E-03 |
| QKI | 212636_at | AL031781 | NM_006775 | 9444 | 3.23 | 3.28E-03 |
| | | | NM_206853 | | | |
| | | | NM_206854 | | | |
| | | | NM_206855 | | | |
| DAZ1 | 208281_x_at | NM_020364 | NM_00100537 5 | 1617 | 3.19 | 3.17E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| PCDH9 | 238919_at | R49295 | NM_020403 | 5101 | 3.15 | 4.07E-03 |
| | | | NM_203487 | | | |
| AI242023 | 240458_at | AI242023 | --- | --- | 3.11 | 1.45E-04 |
| SLC4A11 | 223748_at | AF336127 | NM_032034 | 83959 | 3.10 | 1.45E-03 |
| RPL10A | 242188_at | AI743332 | NM_007104 | 4736 | 3.09 | 2.35E-03 |
| POLQ | 207746_at | NM_014125 | NM_006596 | 10721 | 3.07 | 7.89E-03 |
| | | | | | | |
| MGC24103 | 232568_at | AU145658 | --- | 158295 | 3.07 | 8.38E-03 |
| SPAG6 | 210033_s_at | AF079363 | NM_012443 | 9576 | 3.06 | 1.67E-04 |
| | | | NM_172242 | | | |
| RNF182 | 230720_at | AI884906 | NM_152737 | 221687 | 3.05 | 5.00E-05 |
| LOC440928 | 235147_at | R56118 | XM_498917 | 440928 | 3.04 | 2.36E-03 |
| AL049260 | 216518_at | AL049260 | --- | --- | 3.01 | 8.57E-03 |
| ITGA6 | 201656_at | NM_000210 | NM_000210 | 3655 | 3.00 | 2.58E-04 |
| KCTD12 | 212188_at | AA551075 | NM_138444 | 115207 | 2.98 | 5.67E-04 |
| CXCR4 | 211919_s_at | AF348491 | NM_00100854 0 | 7852 | 2.98 | 7.80E-03 |
| | | | NM_003467 | | | |
| NEGR1 | 243357_at | AA115106 | NM_173808 | 257194 | 2.98 | 6.99E-05 |
| MGC34032 | 1569112_at | AW020413 | NM_152697 | 204962 | 2.94 | 4.27E-04 |
| PTPRG | 1569323_at | BU853579 | NM_002841 | 5793 | 2.92 | 3.90E-03 |
| CXCR4 | 209201_x_at | L01639 | NM_00100854 0 | 7852 | 2.90 | 6.72E-03 |
| | | | NM_003467 | | | |
| CBFA2T2 | 233243_at | AU144676 | NM_005093 | 9139 | 2.88 | 2.26E-03 |
| | | | NM_175864 | | | |
| DAZ1 | 216922_x_at | AF271088 | NM_00100537 5 | 1617 | 2.88 | 7.32E-03 |
| DAZ3 | | | | 57054 | | |
| DAZ2 | | | NM_00100578 5 | 57055 | | |
| DAZ4 | | | | 57135 | | |
| | | | NM_00100578 6 | | | |
| | | | NM_004081 | | | |
| | | | NM_020363 | | | |
| | | | NM_020364 | | | |
| | | | NM_020420 | | | |
| CGNL1 | 225817_at | AB051536 | NM_032866 | 84952 | 2.86 | 3.83E-03 |
| FBXL7 | 241457_at | AI821935 | NM_012304 | 23194 | 2.84 | 1.42E-03 |
| TIMP3 | 201148_s_at | AW338933 | NM_000362 | 7078 | 2.79 | 6.12E-03 |
| BNC2 | 233036_at | AU146418 | NM_017637 | 54796 | 2.78 | 9.59E-03 |
| EDG2 | 204036_at | AW269335 | NM_001401 | 1902 | 2.73 | 5.34E-03 |
| | | | NM_057159 | | | |
| BE674460 | 237849_at | BE674460 | --- | --- | 2.73 | 7.55E-03 |
| MAB21L2 | 210303_at | AF262032 | NM_006439 | 10586 | 2.69 | 1.03E-03 |
| PDE5A | 227088_at | BF221547 | NM_001083 | 8654 | 2.65 | 4.68E-03 |
| | | | NM_033430 | | | |
| | | | NM_033431 | | | |
| | | | NM_033437 | | | |
| BC043430 | 1558714_at | BC043430 | --- | --- | 2.65 | 4.03E-03 |
| AA699443 | 241838_at | AA699443 | --- | --- | 2.64 | 9.15E-04 |
| AL049337 | 215768_at | AL049337 | --- | --- | 2.62 | 9.86E-03 |
| RASEF | 1553986_at | BC023566 | NM_152573 | 158158 | 2.61 | 4.66E-04 |
| PTGFRN | 224937_at | BF311866 | NM_020440 | 5738 | 2.61 | 1.34E-06 |
| AK025909 | 233814_at | AK025909 | --- | --- | 2.60 | 1.65E-03 |
| CPVL | 208146_s_at | NM_031311 | NM_019029 | 54504 | 2.59 | 5.34E-04 |
| | | | NM_031311 | | | |
| TRIM36 | 219736_at | NM_018700 | NM_018700 | 55521 | 2.59 | 6.52E-04 |
| AI377043 | 230722_at | AI377043 | --- | --- | 2.58 | 7.06E-03 |
| SLAC2-B | 214734_at | AB014524 | NM_015065 | 23086 | 2.58 | 1.44E-04 |
| AI077566 | 243994_at | AI077566 | --- | --- | 2.57 | 2.62E-03 |
| EFNA5 | 1559360_at | AL833045 | NM_001962 | 1946 | 2.53 | 7.18E-04 |
| BE464799 | 214036_at | BE464799 | --- | --- | 2.53 | 1.16E-03 |
| MSN | 200600_at | NM_002444 | NM_002444 | 4478 | 2.52 | 1.01E-06 |
| RAD23B | 214422_at | T93562 | NM_002874 | 5887 | 2.52 | 2.47E-03 |
| TULP4 | 232372_at | AL157491 | NM_00100746 6 | 56995 | 2.51 | 4.65E-03 |
| | | | NM_020245 | | | |
| SCN7A | 239984_at | AI333640 | NM_002976 | 6332 | 2.51 | 8.48E-03 |
| ITGA6 | 215177_s_at | AV733308 | NM_000210 | 3655 | 2.50 | 6.62E-04 |
| NFIB | 211466_at | U70862 | NM_005596 | 4781 | 2.50 | 1.09E-05 |
| EML1 | 204797_s_at | NM_004434 | NM_00100870 7 | 2009 | 2.50 | 1.46E-03 |
| | | | NM_004434 | | | |
| BNC2 | 220272_at | NM_017637 | NM_017637 | 54796 | 2.49 | 3.27E-04 |
| BE348318 | 236167_at | BE348318 | --- | --- | 2.49 | 5.60E-03 |
| AW963185 | 241700_at | AW963185 | --- | --- | 2.47 | 1.92E-04 |
| ENC1 | 201340_s_at | AF010314 | NM_003633 | 8507 | 2.47 | 8.66E-03 |
| RBMS3 | 233608_at | AU146417 | NM_00100379 2 | 27303 | 2.44 | 5.92E-03 |
| | | | NM_00100379 3 | | | |
| | | | NM_014483 | | | |
| DACH1 | 1567101_at | AF147347 | NM_004392 | 1602 | 2.42 | 9.28E-03 |
| | | | NM_080759 | | | |
| | | | NM_080760 | | | |
| AA654772 | 241391_at | AA654772 | --- | --- | 2.41 | 7.59E-03 |
| BG400570 | 1565689_at | BG400570 | --- | --- | 2.41 | 6.41E-03 |
| PIAS1 | 222371_at | AI732802 | NM_016166 | 8554 | 2.40 | 3.91E-03 |
| EDD | 232264_at | AK022204 | NM_015902 | 51366 | 2.40 | 1.41E-03 |
| NOL7 | 242143_at | BE674964 | NM_016167 | 51406 | 2.39 | 4.55E-03 |
| U80755 | 243820_at | U80755 | --- | --- | 2.39 | 2.59E-03 |
| ASCL1 | 209985_s_at | BE797438 | NM_004316 | 429 | 2.37 | 8.08E-03 |
| AW052186 | 239942_at | AW052186 | --- | --- | 2.37 | 9.33E-03 |
| THRAP2 | 238883_at | AW975051 | NM_015335 | 23389 | 2.37 | 9.49E-03 |
| ROBO2 | 226766_at | AB046788 | XM_031246 | 6092 | 2.36 | 7.82E-03 |
| INA | 230859_at | BF111276 | NM_032727 | 9118 | 2.36 | 2.50E-05 |
| CHC1L | 230292_at | AA868809 | NM_001268 | 1102 | 2.36 | 6.84E-03 |
| CHRNA7 | 210123_s_at | U62436 | NM_000746 | 1139 | 2.33 | 2.81E-03 |
| CHRFAM7A | | | NM_139320 | 89832 | | |
| | | | NM_148911 | | | |
| ARID1B | 240008_at | AI955765 | NM_017519 | 57492 | 2.32 | 7.87E-03 |
| | | | NM_020732 | | | |
| | | | NM_175863 | | | |
| SSH2 | 230970_at | AA975530 | NM_033389 | 85464 | 2.29 | 6.93E-03 |
| BE670307 | 227955_s_at | BE670307 | --- | --- | 2.28 | 5.03E-04 |
| EB-1 | 227441_s_at | AW005572 | NM_020140 | 56899 | 2.28 | 6.31E-03 |
| | | | NM_152788 | | | |
| | | | NM_181670 | | | |
| GNAI1 | 209576_at | AL049933 | NM_002069 | 2770 | 2.27 | 8.26E-06 |
| PIAS1 | 1559156_at | BC036508 | NM_016166 | 8554 | 2.27 | 5.54E-03 |
| CUGBP2 | 202158_s_at | NM_006561 | NM_006561 | 10659 | 2.26 | 8.77E-03 |
| LOC441157 | 1558795_at | AL833240 | XM_499037 | 441157 | 2.25 | 6.17E-05 |
| KIAA0265 | 238000_at | BF195340 | NM_014997 | 23008 | 2.23 | 9.27E-03 |
| SOX2 | 213721_at | L07335 | NM_003106 | 6657 | 2.23 | 1.49E-03 |
| ENC1 | 201341_at | NM_003633 | NM_003633 | 8507 | 2.23 | 8.95E-03 |
| PLXNA2 | 222159_at | AK023058 | NM_025179 | 5362 | 2.21 | 5.06E-04 |
| GNAI1 | 227692_at | AU153866 | NM_002069 | 2770 | 2.21 | 2.84E-06 |
| NRXN1 | 209915_s_at | AB035356 | NM_004801 | 9378 | 2.21 | 3.08E-03 |
| | | | NM_138735 | | | |
| OLFML3 | 218162_at | NM_020190 | NM_020190 | 56944 | 2.19 | 8.33E-04 |
| CBLB | 233614_at | AU145361 | NM_170662 | 868 | 2.19 | 8.28E-03 |
| CG012 | 215318_at | AL049782 | --- | 116829 | 2.19 | 4.30E-03 |
| SESN3 | 235150_at | AW376955 | NM_144665 | 143686 | 2.18 | 1.58E-03 |
| SOX2 | 228038_at | AI669815 | NM_003106 | 6657 | 2.18 | 1.19E-03 |
| C1orf43 | 1555225_at | BC008306 | NM_015449 | 25912 | 2.17 | 1.21E-05 |
| | | | NM_138740 | | | |
| SPAG6 | 210032_s_at | AI651156 | NM_012443 | 9576 | 2.16 | 1.98E-03 |
| | | | NM_172242 | | | |
| ASCL1 | 209987_s_at | BC002341 | NM_004316 | 429 | 2.15 | 4.18E-03 |
| RASEF | 235144_at | AA826324 | NM_152573 | 158158 | 2.14 | 2.84E-04 |
| OSBPL3 | 209626_s_at | AI202969 | NM_015550 | 26031 | 2.14 | 4.14E-04 |
| | | | NM_145320 | | | |
| | | | NM_145321 | | | |
| | | | NM_145322 | | | |
| | | | NM_145323 | | | |
| | | | NM_145324 | | | |
| HGF | 210997_at | M77227 | NM_000601 | 3082 | 2.13 | 3.66E-03 |
| | | | NM_00101093 1 | | | |
| | | | NM_00101093 2 | | | |
| | | | NM_00101093 3 | | | |
| | | | NM_00101093 4 | | | |
| SLC7A2 | 225516_at | AA876372 | NM_00100853 9 | 6542 | 2.13 | 9.92E-04 |
| | | | NM_003046 | | | |
| EDG3 | 228176_at | AA534817 | NM_005226 | 1903 | 2.13 | 4.95E-04 |
| EFNB2 | 202668_at | BF001670 | NM_004093 | 1948 | 2.13 | 5.45E-03 |
| BF513404 | 239469_at | BF513404 | --- | --- | 2.12 | 1.51E-03 |
| LRRC16 | 219573_at | NM_017640 | NM_017640 | 55604 | 2.11 | 8.45E-04 |
| ARHGAP20 | 228368_at | AI936560 | NM_020809 | 57569 | 2.11 | 3.27E-04 |
| ZFHX4 | 219779_at | NM_024721 | NM_024721 | 79776 | 2.10 | 3.71E-04 |
| AW953794 | 230968_at | AW953794 | --- | --- | 2.09 | 6.43E-03 |
| LOC23117 | 238341_at | BF677084 | XM_290670 | 23117 | 2.09 | 3.42E-03 |
| AK022040 | 233445_at | AK022040 | --- | --- | 2.08 | 3.85E-03 |
| TOP1 | 238988_at | AI863675 | NM_003286 | 7150 | 2.08 | 5.99E-03 |
| ALCAM | 1569362_at | BC041127 | NM_001627 | 214 | 2.07 | 5.59E-04 |
| XPO1 | 1560386_at | BC041658 | NM_003400 | 7514 | 2.07 | 4.79E-04 |
| SPAG9 | 1554543_at | BC007524 | NM_003971 | 9043 | 2.07 | 1.57E-04 |
| | | | NM_172345 | | | |
| RAF1 | 244373_at | AI740571 | NM_002880 | 5894 | 2.07 | 9.16E-04 |
| GFRA2 | 205721_at | U97145 | NM_001495 | 2675 | 2.06 | 4.42E-03 |
| CXCL12 | 209687_at | U19495 | NM_000609 | 6387 | 2.04 | 2.46E-04 |
| | | | NM_199168 | | | |
| MGC34032 | 235763_at | AA001450 | NM_152697 | 204962 | 2.04 | 3.86E-03 |
| AI671885 | 230494_at | AI671885 | XM_379123 | 400999 | 2.01 | 7.81E-03 |
| GPR125 | 1569542_at | BC020926 | NM_145290 | 166647 | 1.99 | 8.83E-03 |
| BF057073 | 231310_at | BF057073 | --- | --- | 1.97 | 9.44E-04 |
| PGAP1 | 233479_at | AU148008 | NM_024989 | 80055 | 1.95 | 7.98E-03 |
| LOC440265 | 1569476_at | BC033224 | XM_378506 | 440265 | 1.95 | 1.83E-03 |
| TFPI2 | 209278_s_at | L27624 | NM_006528 | 7980 | 1.95 | 3.05E-03 |
| KIAA1713 | 233536_at | AI202664 | XM_290811 | 80816 | 1.94 | 5.70E-05 |
| H37807 | 241060_x_at | H37807 | --- | --- | 1.94 | 5.73E-03 |
| AA417878 | 243463_s_at | AA417878 | --- | --- | 1.93 | 4.35E-04 |
| ZNF537 | 223392_s_at | BF510588 | NM_020856 | 57616 | 1.93 | 5.36E-04 |
| PTPRG | 204944_at | NM_002841 | NM_002841 | 5793 | 1.93 | 4.06E-07 |
| MAOA | 204388_s_at | NM_000240 | NM_000240 | 4128 | 1.92 | 2.34E-03 |
| TFPI2 | 209277_at | AL574096 | NM_006528 | 7980 | 1.91 | 9.47E-03 |
| ASCL1 | 213768_s_at | AW950513 | NM_004316 | 429 | 1.91 | 9.81E-03 |
| GFM1 | 232295_at | AK000780 | NM_024996 | 85476 | 1.90 | 3.91E-03 |
| AF131796 | 231916_at | AF131796 | --- | --- | 1.90 | 9.65E-06 |
| SLCO3A1 | 219229_at | NM_013272 | NM_013272 | 28232 | 1.89 | 6.98E-07 |
| FLJ34154 | 1561130_at | AF086006 | NM_173813 | 283450 | 1.89 | 4.96E-03 |
| MSI2 | 239232_at | AA521410 | NM_138962 | 124540 | 1.87 | 7.62E-03 |
| | | | NM_170721 | | | |
| MAOA | 212741_at | AA923354 | NM_000240 | 4128 | 1.87 | 3.66E-04 |
| PRKCBP1 | 207130_at | NM_018634 | NM_012408 | 23613 | 1.87 | 5.52E-03 |
| | | | NM_183047 | | | |
| | | | NM_183048 | | | |
| PPP3R1 | 1565811_at | BC033945 | NM_000945 | 5534 | 1.86 | 2.12E-03 |
| CUGBP2 | 202157_s_at | U69546 | NM_006561 | 10659 | 1.86 | 4.76E-03 |
| AW024890 | 229942_at | AW024890 | --- | --- | 1.86 | 2.32E-04 |
| NOS1 | 239132_at | BE299830 | NM_000620 | 4842 | 1.85 | 4.05E-05 |
| AI733341 | 240911_at | AI733341 | --- | --- | 1.83 | 2.70E-06 |
| MAOA | 204389_at | NM_000240 | NM_000240 | 4128 | 1.83 | 5.67E-03 |
| A1919519 | 235092_at | AI919519 | --- | --- | 1.83 | 1.25E-03 |
| HGF | 210755_at | U46010 | NM_000601 | 3082 | 1.83 | 4.16E-03 |
| | | | NM_00101093 1 | | | |
| | | | NM_00101093 2 | | | |
| | | | NM_00101093 3 | | | |
| | | | NM_00101093 4 | | | |
| EB-1 | 227439_at | AI806510 | NM_020140 | 56899 | 1.83 | 4.67E-03 |
| | | | NM_152788 | | | |
| | | | NM_181670 | | | |
| NRG3 | 229233_at | H05240 | NM_001010848 | 10718 | 1.81 | 5.10E-03 |
| IREB2 | 1563130_a_at | AL109710 | NM_004136 | 3658 | 1.81 | 4.45E-05 |
| ASCL1 | 209988_s_at | BC001638 | NM_004316 | 429 | 1.81 | 9.72E-03 |
| BF509781 | 238970_at | BF509781 | --- | --- | 1.81 | 5.78E-03 |
| AI651786 | 237945_at | AI651786 | --- | --- | 1.80 | 9.11E-04 |
| AW206536 | 243470_at | AW206536 | --- | --- | 1.79 | 9.87E-03 |
| CAS1 | 239545_at | AI335004 | NM_022900 | 64921 | 1.78 | 2.35E-04 |
| FLJ11259 | 218627_at | NM_018370 | NM_018370 | 55332 | 1.78 | 3.98E-03 |
| TPBG | 203476_at | NM_006670 | NM_006670 | 7162 | 1.78 | 3.60E-03 |
| IGSF4 | 244345_at | AI627453 | NM_014333 | 23705 | 1.78 | 1.19E-03 |
| LOC441157 | 1558796_a_at | AL833240 | XM_499037 | 441157 | 1.78 | 6.11E-04 |
| C10orf18 | 216211_at | AL049233 | XM_374765 | 54906 | 1.78 | 5.69E-03 |
| TRIM68 | 219405_at | NM_018073 | NM_018073 | 55128 | 1.77 | 9.35E-03 |
| NM_018616 | 220702_at | NM_018616 | --- | --- | 1.76 | 8.03E-03 |
| SF1 | 210172_at | D26121 | NM_004630 | 7536 | 1.76 | 6.92E-03 |
| | | | NM_201995 | | | |
| | | | NM_201997 | | | |
| | | | NM_201998 | | | |
| AI140305 | 228849_at | AI140305 | --- | --- | 1.73 | 4.71E-03 |
| BE856929 | 239349_at | BE856929 | --- | --- | 1.73 | 8.07E-03 |
| CDC23 | 223651_x_at | BC005258 | NM_004661 | 8697 | 1.73 | 3.36E-03 |
| BG542611 | 1556277_a_at | BG542611 | --- | --- | 1.73 | 3.65E-03 |
| LOC253981 | 226430_at | AI394438 | --- | 253981 | 1.72 | 2.43E-04 |
| FNDC5 | 226096_at | AI760132 | NM_153756 | 252995 | 1.72 | 6.85E-03 |
| VRK1 | 241737_x_at | T89693 | NM_003384 | 7443 | 1.72 | 8.45E-04 |
| PDE8B | 243590_at | AA860184 | NM_003719 | 8622 | 1.71 | 3.11E-03 |
| SESN3 | 225123_at | BE883841 | NM_144665 | 143686 | 1.71 | 4.09E-04 |
| MAK3 | 244341_at | AA827728 | NM_025146 | 80218 | 1.71 | 1.72E-03 |
| FLJ35740 | 244297_at | AI806762 | NM_147195 | 253650 | 1.70 | 9.74E-03 |
| THOC3 | 236149_at | AA502999 | NM_032361 | 84321 | 1.69 | 6.40E-03 |
| INSM1 | 206502_s_at | NM_002196 | NM_002196 | 3642 | 1.69 | 4.60E-03 |
| SESN3 | 242899_at | N78412 | NM_144665 | 143686 | 1.69 | 1.78E-03 |
| PTGFRN | 224950_at | BF476250 | NM_020440 | 5738 | 1.69 | 3.70E-03 |
| HIC2 | 1559600_at | AL831831 | NM_015094 | 23119 | 1.69 | 1.03E-03 |
| AI023295 | 237600_at | AI023295 | --- | --- | 1.67 | 3.22E-03 |
| GLMN | 236924_at | AA814383 | NM_007070 | 11146 | 1.67 | 2.06E-03 |
| | | | NM_053274 | | | |
| N50083 | 229110_at | N50083 | --- | --- | 1.67 | 1.06E-03 |
| BG542645 | 242793_at | BG542645 | --- | --- | 1.66 | 9.11E-03 |
| FLJ37266 | 228973_at | AA057445 | NM_175892 | 283225 | 1.65 | 3.82E-03 |
| AK022258 | 233286_at | AK022258 | --- | --- | 1.64 | 4.08E-03 |
| FNDC5 | 226097_at | AI686488 | NM_153756 | 252995 | 1.64 | 5.89E-03 |
| JJAZ1 | 1566191_at | AI907884 | NM_015355 | 23512 | 1.63 | 6.56E-03 |
| CNTN1 | 227202_at | AW072790 | NM_001843 | 1272 | 1.63 | 3.78E-03 |
| | | | NM_175038 | | | |
| MYLIP | 228098_s_at | AW292746 | NM_013262 | 29116 | 1.63 | 2.87E-04 |
| NM_024972 | 220898_at | NM_024972 | --- | --- | 1.63 | 5.35E-03 |
| AL036662 | 235984_at | AL036662 | --- | --- | 1.62 | 1.89E-04 |
| TIA1 | 1558922_at | CA776505 | NM_022037 | 7072 | 1.62 | 3.98E-03 |
| | | | NM_022173 | | | |
| MYO6 | 203216_s_at | NM_004999 | NM_004999 | 4646 | 1.62 | 5.80E-04 |
| ZNF537 | 223393_s_at | AL136805 | NM_020856 | 57616 | 162 | 7.69E-04 |
| DNAPTP6 | 244597_at | AA701247 | NM_015535 | 26010 | 1.61 | 2.65E-03 |
| PIP5K1A | 235646_at | BF515595 | NM_003557 | 8394 | 1.61 | 1.66E-03 |
| ANAPC7 | 225521_at | AL137586 | NM_016238 | 51434 | 1.61 | 5.35E-03 |
| AF5Q31 | 232865_at | N59653 | NM_014423 | 27125 | 1.60 | 2.42E-04 |
| C2orf31 | 221245_s_at | NM_030804 | --- | 81561 | 1.60 | 3.45E-03 |
| FAM55C | 243011_at | BF317081 | NM_145037 | 91775 | 1.59 | 1.18E-03 |
| LHFPL2 | 212658_at | N66633 | NM_005779 | 10184 | 1.59 | 1.64E-03 |
| PSCD3 | 243752_s_at | AI870144 | NM_004227 | 9265 | 1.59 | 2.84E-03 |
| LOC90701 | 235676_at | BF055352 | NM_033280 | 90701 | 1.59 | 6.05E-03 |
| SMAD4 | 1565703_at | AL832789 | NM_005359 | 4089 | 1.59 | 3.91E-03 |
| PITPNB | 242074_at | AA833902 | NM_012399 | 23760 | 1.58 | 1.84E-03 |
| DKFZp761D221 | 1569603_at | BC020900 | NM_032291 | 84251 | 1.57 | 3.47E-03 |
| MINA | 213189_at | BE966695 | NM_032778 | 84864 | 1.57 | 9.86E-05 |
| | | | NM_153182 | | | |
| YEATS2 | 236462_at | AA742310 | NM_018023 | 55689 | 1.57 | 6.12E-04 |
| ARIH1 | 242431_at | AI656728 | NM_005744 | 25820 | 1.56 | 7.68E-03 |
| XPO1 | 235927 _ at | BE350122 | NM_003400 | 7514 | 1.56 | 1.83E-05 |
| SPON1 | 213994_s_at | AI885290 | NM_006108 | 10418 | 1.55 | 3.33E-03 |
| SIMP | 238303_at | AW070371 | NM_178862 | 201595 | 1.55 | 2.24E-03 |
| KLF12 | 243089_at | AA551114 | NM_007249 | 11278 | 1.55 | 7.90E-03 |
| | | | NM_016285 | | | |
| MAK3 | 231199_at | AA701676 | NM_025146 | 80218 | 1.55 | 2.30E-03 |
| JARID2 | 232835_at | AA533080 | NM_004973 | 3720 | 1.55 | 2.53E-03 |
| H93038 | 241574_s_at | H93038 | --- | --- | 1.54 | 2.24E-03 |
| TGFBR1 | 236561_at | AV700621 | NM_004612 | 7046 | 1.54 | 4.78E-03 |
| PRSS12 | 213802_at | AI810767 | NM_003619 | 8492 | 1.53 | 6.28E-03 |
| PLCL2 | 213309_at | AL117515 | NM_015184 | 23228 | 1.52 | 6.87E-03 |
| PIAS1 | 1558418_at | BC017507 | NM_016166 | 8554 | 1.52 | 2.09E-03 |
| USP48 | 229812_at | BE645018 | NM_032236 | 84196 | 1.51 | 3.09E-05 |
| SPOCK | 202363_at | AF231124 | NM_004598 | 6695 | 1.51 | 5.55E-05 |
| MLL3 | 244010_at | AI057455 | NM_021230 | 58508 | 1.50 | 4.50E-03 |
| | | | NM_170606 | | | |
| STRA6 | 1569335_a_at | BC015881 | NM_022369 | 64220 | 1.50 | 2.05E-03 |
| UCHL1 | 1555834_at | AW974143 | NM_004181 | 7345 | 1.50 | 5.86E-03 |
| AK025064 | 215221_at | AK025064 | --- | --- | 1.49 | 5.96E-03 |
| MINA | 213188_s_at | AI823896 | NM_032778 | 84864 | 1.48 | 4.75E-05 |
| | | | NM_153182 | | | |
| PSME4 | 237180_at | T97717 | NM_014614 | 23198 | 1.48 | 3.52E-03 |
| SLC4A11 | 233550_s_at | AL109976 | NM_032034 | 83959 | 1.48 | 5.66E-03 |
| H04482 | 243287_s_at | H04482 | --- | --- | 1.48 | 5.28E-03 |
| REPS1 | 215201_at | AW166925 | NM_031922 | 85021 | 1.48 | 5.00E-03 |
| OAZIN | 240231_at | AI742383 | NM_015878 | 51582 | 1.47 | 6.87E-03 |
| | | | NM_148174 | | | |
| CBLB | 227900_at | AV701750 | NM_170662 | 868 | 1.47 | 7.93E-03 |
| SLC17A6 | 220551_at | NM_020346 | NM_020346 | 57084 | 1.47 | 4.11E-04 |
| CUGBP2 | 202156_s_at | N36839 | NM_006561 | 10659 | 1.47 | 2.41E-03 |
| R76828 | 1558877_at | R76828 | --- | --- | 1.46 | 4.33E-04 |
| C9orf102 | 1557164_a_at | BM683657 | NM_020207 | 56959 | 1.46 | 2.47E-03 |
| CLSTN2 | 219414_at | NM_022131 | NM_022131 | 64084 | 1.46 | 5.74E-03 |
| FAM19A4 | 242348_at | AA757457 | NM_00100552 7 | 151647 | 1.45 | 2.73E-05 |
| | | | NM_182522 | | | |
| HTR2B | 206638_at | NM_000867 | NM_000867 | 3357 | 1.44 | 5.90E-03 |
| AW197616 | 230787_at | AW197616 | --- | --- | 1.44 | 1.22E-03 |
| BC035170 | 1561079_at | BC035170 | --- | --- | 1.44 | 4.66E-03 |
| CUGBP2 | 1557422_at | R49146 | NM_006S61 | 10659 | 1.44 | 7.71E-03 |
| LCRISP1 | 223475_at | AF142573 | NM_031461 | 83690 | 1.44 | 3.45E-03 |
| SNCAIP | 219511_s_at | NM_005460 | NM_005460 | 9627 | 1.44 | 9.60E-03 |
| RNF34 | 236288_at | AW572402 | NM_025126 | 80196 | 1.44 | 1.55E-03 |
| | | | NM_194271 | | | |
| ALCAM | 201951_at | BF242905 | NM_001627 | 214 | 1.44 | 1.25E-03 |
| FGD6 | 1555137_a_at | BC013319 | NM_018351 | 55785 | 1.43 | 8.61E-03 |
| AI821477 | 235985_at | AI821477 | --- | --- | 1.43 | 8.29E-04 |
| SRPK2 | 239811_at | BF954306 | NM_182691 | 6733 | 1.43 | 2.17E-03 |
| | | | NM_182692 | | | |
| CNTN1 | 227209_at | AI091445 | NM_001843 | 1272 | 1.43 | 6.48E-03 |
| | | | NM_175038 | | | |
| PDLIM5 | 213684_s_at | BF671400 | NM_00101151 3 | 10611 | 1.43 | 4.72E-05 |
| | | | NM_00101151 4 | | | |
| | | | NM_00101151 5 | | | |
| | | | NM_00101151 6 | | | |
| | | | NM_006457 | | | |
| FLJ11710 | 244646_at | AW972881 | --- | 79904 | 1.43 | 6.04E-03 |
| MAPRE2 | 1564733_at | BC013931 | NM_014268 | 10982 | 1.43 | 4.15E-03 |
| DST | 1558019_at | BC020911 | NM_001723 | 667 | 1.42 | 5.60E-03 |
| | | | NM_015548 | | | |
| | | | NM_020388 | | | |
| | | | NM_183380 | | | |
| SCC-112 | 241798_at | AI339930 | NM_015200 | 23244 | 1.42 | 4.68E-03 |
| SPON1 | 209436_at | AB018305 | NM_006108 | 10418 | 1.41 | 1.28E-03 |
| PPP3R1 | 241786_at | A1380514 | NM_000945 | 5534 | 1.41 | 8.09E-03 |
| ARID1B | 1556818_at | BE671639 | NM_017519 | 57492 | 1.40 | 1.56E-03 |
| | | | NM_020732 | | | |
| | | | NM_175863 | | | |
| ZA20D1 | 238994_at | AW022496 | NM_020205 | 56957 | 1.40 | 1.24E-03 |
| HIAT1 | 242374_at | AA747563 | NM_033055 | 64645 | 1.40 | 9.78E-04 |
| ZNF503 | 227195_at | AA603467 | NM_032772 | 84858 | 1.40 | 1.14E-04 |
| SMARCC2 | 1561973_at | AL833124 | NM_003075 | 6601 | 1.39 | 4.39E-03 |
| | | | NM_139067 | | | |
| ZNF215 | 220214_at | NM_013250 | NM_013250 | 7762 | 1.39 | 5.26E-03 |
| KIAA0318 | 238817_at | BF063412 | NM_015347 | 23504 | 1.39 | 3.46E-03 |
| RAP80 | 233596_at | AF113675 | NM_016290 | 51720 | 1.38 | 8.59E-03 |
| ACY1L2 | 225421_at | AI654133 | NM_001010853 | 135293 | 1.37 | 9.04E-04 |
| UBE1C | 1556338_at | BM353142 | NM_003968 | 9039 | 1.37 | 4.28E-03 |
| | | | NM_198195 | | | |
| | | | NM_198197 | | | |
| RASEF | 1553186_x_at | NM_152573 | NM_152573 | 158158 | 1.37 | 6.41E-03 |
| MDN1 | 1569484_s_at | AL603301 | NM_014611 | 23195 | 1.37 | 1.48E-03 |
| E2-230K | 1554814_at | BC007822 | NM_022066 | 63893 | 1.36 | 5.24E-03 |
| ZFOC1 | 1558816_at | BC037793 | NM_152437 | 144348 | 1.35 | 1.76E-03 |
| CAPZA2 | 240146_at | AW418562 | NM_006136 | 830 | 1.35 | 1.22E-03 |
| DLEU2 | 215629_s_at | AA905286 | NM_006021 | 8847 | 1.35 | 3.10E-03 |
| CLASP2 | 1558759_s_at | AA310888 | NM_015097 | 23122 | 1.34 | 2.92E-03 |
| KIAA1545 | 1557539_at | BC008052 | XM_495939 | 57666 | 1.34 | 1.63E-03 |
| FLJ45645 | 243038_at | AW292769 | NM_198557 | 375287 | 1.34 | 4.56E-03 |
| MGC34032 | 1552782_at | AK091400 | NM_152697 | 204962 | 1.34 | 1.91E-03 |
| ELOVL7 | 227180_at | AW138767 | XM_371740 | 79993 | 1.33 | 4.90E-04 |
| FLJ12666 | 215029_at | AL117451 | NM_024595 | 79647 | 1.32 | 3.06E-03 |
| AA156721 | 201952_at | AA156721 | --- | --- | 1.31 | 9.34E-05 |
| IREB2 | 1555476_at | BC017880 | NM_004136 | 3658 | 1.31 | 1.01E-03 |
| SCML1 | 235652_at | AI431345 | NM_006746 | 6322 | 1.31 | 1.08E-03 |
| BTBD3 | 243462_s_at | BF590303 | NM_014962 | 22903 | 1.31 | 1.87E-03 |
| | | | NM_181443 | | | |
| LRRTM1 | 243330_at | AW304000 | NM_178839 | 347730 | 1.30 | 7.44E-03 |
| RNF159 | 241956_at | AI521883 | NM_032373 | 84333 | 1.30 | 2.94E-03 |
| L3MBTL | 242637_at | C14069 | NM_015478 | 26013 | 1.30 | 2.01E-03 |
| | | | NM_032107 | | | |
| LRRFIP2 | 232704_s_at | AK025207 | NM_006309 | 9209 | 1.29 | 1.87E-03 |
| | | | NM_017724 | | | |
| FLJ40773 | 1563933_a_at | AK091691 | NM_152666 | 200150 | 1.29 | 6.62E-03 |
| N46436 | 1556568_a_at | N46436 | --- | --- | 1.29 | 3.59E-03 |
| TRIM33 | 239716_at | AA908970 | NM_015906 | 51592 | 1.29 | 6.38E-03 |
| | | | NM_033020 | | | |
| RIP | 1556088_at | AK098491 | NM_032308 | 84268 | 1.29 | 7.36E-04 |
| AK098258 | 1564175_at | AK098258 | --- | --- | 1.29 | 9.36E-03 |
| WWOX | 1556595_at | BQ025203 | NM_016373 | 51741 | 1.29 | 9.57E-03 |
| | | | NM_018560 | | | |
| | | | NM_130788 | | | |
| | | | NM_130790 | | | |
| | | | NM_130791 | | | |
| | | | NM_130792 | | | |
| | | | NM_130844 | | | |
| AW874669 | 239834_at | AW874669 | --- | --- | 1.29 | 1.39E-03 |
| SYNCRIP | 1555427_s_at | AY034482 | NM_006372 | 10492 | 1.28 | 1.39E-03 |
| ZNF198 | 1569091_at | BC039458 | NM_003453 | 7750 | 1.28 | 9.19E-03 |
| | | | NM_197968 | | | |
| NOL7 | 244474_at | BE549780 | NM_016167 | 51406 | 1.28 | 9.53E-03 |
| C20orf23 | 232083_at | AB046810 | NM_024704 | 55614 | 1.28 | 6.84E-03 |
| THRAP1 | 236978_at | N51961 | NM_005121 | 9969 | 1.28 | 5.36E-04 |
| KIAA0217 | 241965_at | BF589232 | NM_015155 | 23185 | 1.28 | 7.35E-03 |
| ASXL1 | 242439_s_at | AI819150 | NM_015338 | 171023 | 1.27 | 6.77E-03 |
| ELOVL5 | 1566079_at | AL833001 | NM_021814 | 60481 | 1.27 | 1.08E-03 |
| XPO7 | 240168_at | AA679589 | NM_015024 | 23039 | 1.26 | 3.43E-03 |
| IREB2 | 1563129_at | AL109710 | NM_004136 | 3658 | 1.26 | 2.34E-03 |
| MMD | 244523_at | AW104453 | NM_012329 | 23531 | 1.26 | 1.24E-06 |
| RASEF | 1553185_at | NM_152573 | NM_152573 | 158158 | 1.26 | 6.94E-03 |
| ZA20D3 | 238812_at | AA741296 | NM_019006 | 54469 | 1.26 | 5.79E-03 |
| ARFGEF1 | 236742_at | AA132172 | NM_006421 | 10565 | 1.25 | 6.55E-03 |
| CUL3 | 242362_at | AI797788 | NM_003590 | 8452 | 1.25 | 5.38E-03 |
| THOC4 | 226319_s_at | AF047002 | NM_005782 | 10189 | 1.25 | 5.54E-04 |
| KLHL13 | 227875_at | AB037730 | NM_033495 | 90293 | 1.25 | 1.10E-05 |
| TAF15 | 241885_at | BF431050 | NM_003487 | 8148 | 1.25 | 3.79E-03 |
| | | | NM_139215 | | | |
| C18965 | 242853_at | C18965 | XM_379243 | 401106 | 1.24 | 2.00E-03 |
| EZH2 | 203358_s_at | NM_004456 | NM_004456 | 2146 | 1.24 | 4.13E-03 |
| | | | NM_152998 | | | |
| DLEU2 | 216870_x_at | AF264787 | NM_006021 | 8847 | 1.24 | 3.31E-04 |
| CABIN1 | 1557581_x_at | BC027347 | NM_012295 | 23523 | 1.23 | 4.73E-03 |
| HOXD4 | 205522_at | NM_014621 | NM_014621 | 3233 | 1.23 | 7.69E-03 |
| AW157450 | 235716_at | AW157450 | --- | --- | 1.23 | 1.18E-03 |
| TPARL | 1560622_at | AK000203 | NM_018475 | 55858 | 1.23 | 3.89E-03 |
| KDELC2 | 225128_at | AL548941 | NM_153705 | 143888 | 1.23 | 6.61E-04 |
| PTPRK | 203038_at | NM_002844 | NM_002844 | 5796 | 1.23 | 1.53E-03 |
| SFRS2IP | 1570507_at | BC020736 | NM_004719 | 9169 | 1.23 | 3.26E-03 |
| ZNF326 | 227680_at | AI057121 | NM_181781 | 284695 | 1.23 | 1.88E-03 |
| | | | NM_182975 | | | |
| | | | NM_182976 | | | |
| NM_000575 | 208200_at | NM_000575 | --- | --- | 1.23 | 1.37E-03 |
| GPI | 232002_at | AW843302 | NM_000175 | 2821 | 1.23 | 2.05E-03 |
| CAPN1 | 232012_at | AV691296 | NM_005186 | 823 | 1.22 | 3.25E-05 |
| XPO7 | 235796_at | AI927957 | NM_015024 | 23039 | 1.22 | 8.08E-03 |
| PITPNB | 239978_at | BF109370 | NM_012399 | 23760 | 1.22 | 8.12E-03 |
| DENR | 238982_at | AW665791 | NM_003677 | 8562 | 1.22 | 1.47E-05 |
| PFKFB3 | 202464_s_at | NM_004566 | NM_004566 | 5209 | 1.22 | 3.62E-04 |
| AA343057 | 234997_x_at | AA343057 | XM_496451 | 388760 | 1.21 | 9.45E-03 |
| RB1CC1 | 237626_at | AI801666 | NM_014781 | 9821 | 1.21 | 5.81E-03 |
| NR2C1 | 210530_s_at | M21985 | NM_003297 | 7181 | 1.21 | 1.06E-06 |
| PLXNA2 | 213030_s_at | AI688418 | NM_025179 | 5362 | 1.21 | 7.50E-03 |
| AI857788 | 227126_at | AI857788 | --- | --- | 1.20 | 3.82E-05 |
| MYO6 | 210480_s_at | U90236 | NM_004999 | 4646 | 1.20 | 3.13E-03 |
| TH1L | 225865_x_at | AJ238374 | NM_016397 | 51497 | 1.20 | 3.35E-05 |
| | | | NM_198976 | | | |
| AL044078 | 221860_at | AL044078 | --- | --- | 1.19 | 1.28E-05 |
| ZGPAT | 221848_at | AL121845 | NM_032527 | 84619 | 1.19 | 7.13E-04 |
| | | | NM_181484 | | | |
| | | | NM_181485 | | | |
| HGF | 209960_at | X16323 | NM_000601 | 3082 | 1.18 | 7.95E-03 |
| | | | NM_00101093 1 | | | |
| | | | NM_00101093 2 | | | |
| | | | NM_00101093 3 | | | |
| | | | NM_00101093 4 | | | |
| MGC47869 | 243056_at | AI301111 | NM_175874 | 144608 | 1.18 | 5.95E-03 |
| NKTR | 1557737_s_at | AI880383 | NM_005385 | 4820 | 1.18 | 1.36E-03 |
| TRA2A | 229574_at | AI268231 | NM_013293 | 29896 | 1.18 | 1.72E-03 |
| ITGB5 | 201125_s_at | NM_002213 | NM_002213 | 3693 | 1.18 | 5.48E-04 |
| ARIH1 | 1558710_at | BI791845 | NM_005744 | 25820 | 1.18 | 7.13E-03 |
| GOLGA4 | 215203_at | AW438464 | NM_002078 | 2803 | 1.18 | 3.99E-03 |
| BASP1 | 202391_at | NM_006317 | NM_006317 | 10409 | 1.18 | 3.05E-03 |
| TM4SF14 | 223314_at | BF025955 | NM_030927 | 81619 | 1.18 | 1.52E-03 |
| SYNCRIP | 209025_s_at | AF037448 | NM_006372 | 10492 | 1.17 | 3.35E-03 |
| TH1L | 220607_x_at | NM_016397 | NM_016397 | 51497 | 1.15 | 2.89E-04 |
| | | | NM_198976 | | | |
| COBRA1 | 1556434_at | BQ777552 | NM_015456 | 25920 | 1.15 | 3.42E-03 |
| SPATS2 | 1570130_at | BC032441 | NM_023071 | 65244 | 1.15 | 6.79E-03 |
| GATA2 | 210358_x_at | BC002557 | NM_032638 | 2624 | 1.15 | 2.50E-03 |
| MYST3 | 242480_at | AA868356 | NM_006766 | 7994 | 1.15 | 9.06E-03 |
| TH1L | 225261_x_at | AJ238376 | NM_016397 | 51497 | 1.15 | 2.80E-05 |
| | | | NM_198976 | | | |
| PTK2 | 241387_at | AW276701 | NM_005607 | 5747 | 1.14 | 7.90E-03 |
| | | | NM_153831 | | | |
| C9orf39 | 1559005_s_at | AK098502 | NM_017738 | 54875 | 1.14 | 4.61E-03 |
| SFRS8 | 240078_at | BG434474 | NM_004592 | 6433 | 1.14 | 2.74E-03 |
| | | | NM_152235 | | | |
| TBC1D5 | 215612_at | AU147983 | NM_014744 | 9779 | 1.14 | 2.73E-03 |
| TCTEL1 | 242109_at | AI038577 | NM_006519 | 6993 | 1.14 | 1.48E-06 |
| SMC5L1 | 1562948_at | BC035661 | NM_015110 | 23137 | 1.14 | 9.50E-03 |
| PLS3 | 201215_at | NM_005032 | NM_005032 | 5358 | 1.13 | 6.23E-03 |
| TM4SF14 | 221002_s_at | NM_030927 | NM_030927 | 81619 | 1.13 | 1.20E-03 |
| SSB4 | 229929_at | AL563476 | NM_080862 | 92369 | 1.13 | 8.19E-03 |
| THOC4 | 226320_at | AF047002 | NM_005782 | 10189 | 1.13 | 6.76E-04 |
| EB-1 | 227440_at | AW005572 | NM_020140 | 56899 | 1.13 | 5.86E-03 |
| | | | NM_152788 | | | |
| | | | NM_181670 | | | |
| PCBP2 | 213517_at | AW103422 | NM_005016 | 5094 | 1.13 | 2.39E-05 |
| | | | NM_031989 | | | |
| C9orf100 | 230522_s_at | BG028209 | NM_032818 | 84904 | 1.12 | 5.16E-03 |
| BNC2 | 235723_at | AA843242 | NM_017637 | 54796 | 1.12 | 8.45E-03 |
| UBE1C | 1556339_a_at | BM353142 | NM_003968 | 9039 | 1.12 | 2.60E-03 |
| | | | NM_198195 | | | |
| | | | NM_198197 | | | |
| C20orf58 | 228017_s_at | BF593263 | NM_152864 | 128414 | 1.12 | 4.82E-03 |
| AW978811 | 242080_at | AW978811 | --- | --- | 1.12 | 5.08E-03 |
| EXT1 | 1564378_a_at | AK025101 | NM_000127 | 2131 | 1.12 | 4.78E-03 |
| KIAA1423 | 225127_at | BF217531 | XM_376550 | 57583 | 1.11 | 7.59E-05 |
| CCAR1 | 236566_at | AI291189 | NM_018237 | 55749 | 1.11 | 5.91E-03 |
| SMG1 | 244766_at | BG180003 | NM_014006 | 23049 | 1.11 | 2.54E-03 |
| KIAA0220 | | | NM_015092 | 283846 | | |
| LOC440345 | | | XM_496125 | 440345 | | |
| TMP21 | 239851_at | AW629289 | NM_006827 | 10972 | 1.11 | 9.85E-03 |
| NOS1 | 207309_at | NM_000620 | NM_000620 | 4842 | 1.11 | 4.13E-03 |
| EIF4ENIF1 | 242983_at | AI806626 | NM_019843 | 56478 | 1.11 | 2.41E-03 |
| ANAPC5 | 239651_at | BE671583 | NM_016237 | 51433 | 1.11 | 8.65E-03 |
| FLJ20186 | 219646_at | NM_017702 | NM_017702 | 54849 | 1.11 | 7.86E-03 |
| | | | NM_207514 | | | |
| BF196475 | 241529_at | BF196475 | --- | --- | 1.11 | 6.33E-03 |
| FRMD4A | 208476_s_at | NM_018027 | NM_018027 | 55691 | 1.11 | 5.78E-03 |
| SDHA | 230077_at | W90764 | NM_004168 | 255812 | 1.10 | 2.82E-03 |
| SDHAL2 | | | XM_171032 | 6389 | | |
| CHCHD3 | 216170_at | AK025271 | NM_017812 | 54927 | 1.10 | 4.90E-03 |
| SLC30A9 | 237051_at | AI890591 | NM_006345 | 10463 | 1.10 | 3.32E-03 |
| ANP32A | 235954_at | AA972597 | NM_006305 | 8125 | 1.10 | 6.96E-04 |
| AV752058 | 244574_at | AV752058 | --- | --- | 1.09 | 1.04E-03 |
| CTGLF1 | 1565620_at | BG621044 | NM_133446 | 119016 | 1.09 | 8.36E-03 |
| ZCCHC11 | 230713_at | BF115786 | NM_00100988 1 | 23318 | 1.09 | 6.14E-03 |
| | | | NM_00100988 2 | | | |
| | | | NM_015269 | | | |
| KIAA1219 | 242233_at | AI739332 | NM_020336 | 57148 | 1.09 | 6.47E-03 |
| PDGFD | 219304_s_at | NM_025208 | NM_025208 | 80310 | 1.09 | 1.44E-03 |
| | | | NM_033135 | | | |
| AA743413 | 236985_at | AA743413 | --- | --- | 1.08 | 9.66E-03 |
| RNF10 | 237062_at | BE222109 | NM_014868 | 9921 | 1.08 | 5.28E-03 |
| ORC6L | 219105_x_at | NM_014321 | NM_014321 | 23594 | 1.08 | 3.86E-03 |
| TH1L | 225006_x_at | AJ238379 | NM_016397 | 51497 | 1.08 | 3.76E-05 |
| | | | NM_198976 | | | |
| KIAA2024 | 1566166_at | AL833423 | NM_172070 | 130507 | 1.07 | 9.88E-03 |
| LOC283871 | 222622_at | BG284709 | XM_208887 | 283871 | 1.06 | 3.90E-03 |
| BE856960 | 242712_x_at | BE856960 | --- | --- | 1.06 | 1.22E-03 |
| RPUSD3 | 1566603_s_at | AK094587 | NM_173659 | 285367 | 1.05 | 1.31E-03 |
| LOC284244 | 214162_at | AF070541 | --- | 284244 | 1.05 | 6.69E-03 |
| AI817960 | 239649_at | AI817960 | --- | --- | 1.05 | 8.05E-03 |
| TRA2A | 213593_s_at | AW978896 | NM_013293 | 29896 | 1.05 | 1.05E-03 |
| SBNO1 | 216161_at | AK024128 | NM_018183 | 55206 | 1.05 | 2.06E-03 |
| SIRT2 | 1558331_at | BG722779 | NM_012237 | 22933 | 1.05 | 4.53E-04 |
| | | | NM_030593 | | | |
| AF090944 | 233995_at | AF090944 | --- | --- | 1.05 | 9.19E-03 |
| C20orf58 | 230668_at | AI758937 | NM_152864 | 128414 | 1.04 | 8.75E-03 |
| HM13 | 1560297_at | BG928538 | NM_030789 | 81502 | 1.04 | 7.20E-03 |
| | | | NM_178580 | | | |
| | | | NM_178581 | | | |
| | | | NM_178582 | | | |
| SLCO3A1 | 227367_at | AW976431 | NM_013272 | 28232 | 1.04 | 1.11E-03 |
| PAN3 | 239393_at | AW510927 | NM_175854 | 255967 | 1.04 | 2.63E-03 |
| CNTN4 | 229084_at | R42166 | NM_175607 | 152330 | 1.04 | 7.84E-03 |
| | | | NM_175612 | | | |
| | | | NM_175613 | | | |
| LOC51760 | 205613_at | NM_016524 | NM_016524 | 51760 | 1.04 | 6.90E-03 |
| APC | 216933_x_at | S67788 | NM_000038 | 324 | 1.04 | 6.77E-03 |
| H43040 | 231108_at | H43040 | --- | --- | 1.03 | 6.01E-04 |
| C9orf10 | 241242_at | BE503118 | NM_014612 | 23196 | 1.03 | 9.13E-03 |
| C13orf25 | 232291_at | AA256157 | NM_213723 | 407975 | 1.03 | 4.81E-03 |
| | | | NM_213724 | | | |
| HAN11 | 233782_at | AU147133 | NM_00100372 5 | 10238 | 1.03 | 2.43E-04 |
| | | | NM_005828 | | | |
| FLJ43654 | 235376_at | BF125564 | NM_198562 | 375341 | 1.03 | 1.61E-03 |
| RTN4 | 1556049_at | CA428769 | NM_007008 | 57142 | 1.02 | 3.35E-03 |
| | | | NM_020532 | | | |
| | | | NM_153828 | | | |
| | | | NM_207520 | | | |
| | | | NM_207521 | | | |
| CSE1L | 210765_at | AF053640 | NM_001316 | 1434 | 1.02 | 6.67E-05 |
| | | | NM_177436 | | | |
| CBLB | 209682_at | U26710 | NM_170662 | 868 | 1.02 | 1.33E-06 |
| FLJ12750 | 236889_at | AI911084 | NM_024667 | 79720 | 1.02 | 9.90E-03 |
| PEG10 | 212094_at | AL582836 | XM_496907 | 23089 | 1.02 | 4.05E-03 |
| | | | XM_499343 | | | |
| T40928 | 241913_at | T40928 | XM_498972 | 441028 | 1.02 | 9.84E-03 |
| FLJ11783 | 220546_at | NM_024891 | NM_024891 | 79951 | 1.02 | 9.99E-04 |
| NM_018627 | 220410_s_at | NM_018627 | --- | --- | 1.01 | 5.37E-03 |
| CSPG6 | 1556925_at | AL360194 | NM_005445 | 9126 | 1.01 | 1.34E-03 |
| DERA | 218102_at | NM_015954 | NM_015954 | 51071 | 1.01 | 3.80E-03 |
| DLL3 | 219537_x_at | NM_016941 | NM_016941 | 10683 | 1.01 | 2.97E-04 |
| | | | NM_203486 | | | |
| CNOT7 | 233019_at | AU145061 | NM_013354 | 29883 | 1.01 | 2.14E-03 |
| | | | NM_054026 | | | |
| HIVEP2 | 243254_at | T08739 | NM_006734 | 3097 | 1.01 | 2.67E-03 |
| MGC33926 | 229302_at | AA058832 | NM_152390 | 130733 | 1.01 | 3.60E-03 |
| LRRN5 | 216167_at | AK024867 | NM_006338 | 10446 | 1.01 | 6.96E-03 |
| | | | NM_201630 | | | |
| API5 | 214960_at | AF229253 | NM_006595 | 8539 | 1.00 | 5.16E-04 |
| EAF2 | 219551_at | NM_018456 | NM_018456 | 55840 | 1.00 | 5.97E-05 |
| C2orf23 | 204364_s_at | BE535746 | NM_022912 | 65055 | 1.00 | 9.51E-04 |
| EVL | 227232_at | T58044 | NM_016337 | 51466 | 1.00 | 6.72E-04 |
| MAP4K3 | 242448_at | AI800895 | NM_003618 | 8491 | 1.00 | 4.16E-03 |
| GLS | 223080_at | AK025021 | NM_014905 | 2744 | 1.00 | 5.50E-03 |
| ST8SIA1 | 210073_at | L32867 | NM_003034 | 6489 | 1.00 | 2.41E-03 |
| SNAG1 | 226683_at | AU146771 | NM_052870 | 112574 | 0.99 | 1.15E-03 |
| TDG | 203742_s_at | BF674842 | NM_00100841 1 | 6996 | 0.99 | 1.56E-03 |
| | | | NM_003211 | | | |
| GALNT7 | 216756_at | AK024995 | NM_017423 | 51809 | 0.99 | 7.57E-03 |
| DCP2 | 244777_at | AA504595 | NM_152624 | 167227 | 0.99 | 7.31E-03 |
| HIPK2 | 224016_at | AF111850 | NM_022740 | 28996 | 0.99 | 3.36E-03 |
| BE467787 | 243835_at | BE467787 | --- | --- | 0.99 | 6.62E-03 |
| C9orf39 | 239989_at | AW170610 | NM_017738 | 54875 | 0.99 | 7.75E-05 |
| AI655229 | 243497_at | AI655229 | --- | --- | 0.99 | 6.76E-03 |
| WSB1 | 227501_at | A1377135 | NM_015626 | 26118 | 0.99 | 6.53E-03 |
| | | | NM_134264 | | | |
| | | | NM_134265 | | | |
| NM_025120 | 220728_at | NM_025120 | --- | --- | 0.98 | 1.00E-03 |
| PCBP2 | 229467_at | AA504356 | NM_005016 | 5094 | 0.98 | 8.91E-03 |
| | | | NM_031989 | | | |
| EIF4A1 | 214805_at | U79273 | NM_001416 | 1973 | 0.98 | 7.43E-03 |
| MGEA5 | 214972_at | AU144791 | NM_012215 | 10724 | 0.97 | 7.74E-03 |
| FLJ10579 | 230843_at | AA482037 | NM_018145 | 55177 | 0.97 | 2.54E-03 |
| ZCWPW2 | 243863_at | BF575233 | XM_087384 | 152098 | 0.97 | 4.74E-03 |
| RNF111 | 241413_at | W80457 | NM_017610 | 54778 | 0.97 | 1.29E-03 |
| PLEKHA6 | 205093_at | NM_014935 | NM_014935 | 22874 | 0.97 | 1.50E-04 |
| SEZ6L | 207873_x_at | NM_021115 | NM_021115 | 23544 | 0.97 | 2.35E-04 |
| FLJ10213 | 219906_at | NM_018029 | NM_018029 | 55096 | 0.97 | 6.80E-03 |
| WTAP | 1558783_at | AK024805 | NM_004906 | 9589 | 0.97 | 2.62E-04 |
| | | | NM_152857 | | | |
| | | | NM_152858 | | | |
| CTNNB1 | 242558_at | AW362945 | NM_001904 | 1499 | 0.96 | 7.55E-03 |
| AA830545 | 235730_at | AA830545 | --- | --- | 0.96 | 3.70E-05 |
| MGC33846 | 231430_at | AW205640 | NM_175885 | 220382 | 0.95 | 1.36E-03 |
| ZNF238 | 207164_s_at | NM_006352 | NM_006352 | 10472 | 0.95 | 2.86E-03 |
| | | | NM_205768 | | | |
| AF5Q31 | 232864_s_at | N59653 | NM_014423 | 27125 | 0.95 | 4.59E-04 |
| SFRS2 | 200753_x_at | BE866585 | NM_003016 | 6427 | 0.95 | 2.39E-04 |
| H40696 | 236122_at | H40696 | XM_370722 | 387921 | 0.95 | 4.70E-03 |
| AW971254 | 222369_at | AW971254 | --- | --- | 0.94 | 4.58E-03 |
| DKFZp547A023 | 214731_at | AB037854 | NM_018704 | 55917 | 0.94 | 7.37E-03 |
| AK022388 | 233017_x_at | AK022388 | --- | --- | 0.94 | 7.06E-03 |
| PRDX2 | 215067_x_at | AU147942 | NM_005809 | 7001 | 0.94 | 2.97E-03 |
| | | | NM_181737 | | | |
| | | | NM_181738 | | | |
| RET | 215771_x_at | X15786 | NM_020630 | 5979 | 0.94 | 8.55E-03 |
| | | | NM_020975 | | | |
| ACTR6 | 239679_at | AI871160 | NM_022496 | 64431 | 0.94 | 4.88E-03 |
| HNRPA1 | 222040_at | AI144007 | NM_002136 | 3178 | 0.93 | 4.24E-03 |
| | | | NM_031157 | | | |
| TMEM4 | 209797 _at | BC001027 | NM_014255 | 10330 | 0.93 | 5.35E-04 |
| CIAPIN1 | 230838_s_at | AW771492 | NM_020313 | 57019 | 0.93 | 9.30E-04 |
| PRPF4B | 202126_at | AA156948 | NM_003913 | 8899 | 0.93 | 2.94E-03 |
| | | | NM_176800 | | | |
| ETV3 | 1552423_at | NM_005240 | NM_005240 | 2117 | 0.92 | 5.12E-03 |
| KCNQ1OT1 | 243435_at | AI023707 | --- | 10984 | 0.92 | 5.64E-03 |
| HNRPA2B1 | 205292_s_at | NM_002137 | NM_002137 | 3181 | 0.92 | 7.56E-04 |
| | | | NM_031243 | | | |
| AW138794 | 227283_at | AW138794 | --- | --- | 0.92 | 1.97E-03 |
| HNRPU | 236244_at | AI458297 | NM_004501 | 3192 | 0.92 | 8.40E-04 |
| | | | NM_031844 | | | |
| TUBB6 | 209191_at | BC002654 | NM_032525 | 84617 | 0.91 | 2.73E-03 |
| SEZ6L | 211894_x_at | AB041736 | NM_021115 | 23544 | 0.91 | 9.29E-04 |
| C2orf23 | 204365_s_at | NM_022912 | NM_022912 | 65055 | 0.91 | 3.53E-04 |
| HNRPM | 238610_s_at | AI906424 | NM_005968 | 4670 | 0.91 | 6.28E-03 |
| | | | NM_031203 | | | |
| NUP210 | 220035_at | NM_024923 | NM_024923 | 23225 | 0.91 | 8.57E-03 |
| RNF130 | 236292_at | AW196696 | NM_018434 | 55819 | 0.91 | 5.09E-03 |
| STMN3 | 222557_at | AL353715 | NM_015894 | 50861 | 0.91 | 1.21E-04 |
| KIAA0907 | 230028_at | BF508843 | NM_014949 | 22889 | 0.91 | 1.57E-04 |
| AI346432 | 229132_at | AI346432 | --- | --- | 0.91 | 6.45E-03 |
| DLL3 | 222898_s_at | BE350882 | NM_016941 | 10683 | 0.90 | 1.05E-03 |
| | | | NM_203486 | | | |
| CYB561 | 209164_s_at | BC002976 | NM_001915 | 1534 | 0.90 | 8.80E-03 |
| CBWD1 | 226193_x_at | AV709992 | NM_018491 | 150472 | 0.90 | 5.40E-03 |
| CBWD2 | | | NM_172003 | 220869 | | |
| LOC220869 | | | NM_201453 | 389760 | | |
| DC36 | | | | 55871 | | |
| SFRS2 | 214882_s_at | BG254869 | NM_003016 | 6427 | 0.90 | 2.75E-04 |
| AK024901 | 234326_at | AK024901 | --- | --- | 0.90 | 3.19E-03 |
| RAB12 | 238714_at | AA504249 | XM_113967 | 201475 | 0.89 | 3.77E-03 |
| BC001743 | 209446_s_at | BC001743 | --- | --- | 0.89 | 2.43E-05 |
| CDK2AP1 | 201938_at | NM_004642 | NM_004642 | 8099 | 0.89 | 4.50E-03 |
| AF5Q31 | 1555436_a_at | BC025700 | NM_014423 | 27125 | 0.89 | 2.72E-03 |
| LOC144997 | 227289_at | AU119437 | --- | 144997 | 0.88 | 6.49E-03 |
| C10orf58 | 224435_at | BC005871 | NM_032333 | 84293 | 0.88 | 3.29E-03 |
| MAP3K13 | 211083_s_at | Z25428 | NM_004721 | 9175 | 0.88 | 1.85E-03 |
| CP110 | 1569353_at | BC030223 | NM_014711 | 9738 | 0.88 | 1.17E-03 |
| NDUFS1 | 1559691_at | BC032767 | NM_005006 | 4719 | 0.88 | 5.87E-03 |
| NUDT9 | 241489_at | BE671532 | NM_024047 | 53343 | 0.88 | 3.25E-03 |
| | | | NM_198038 | | | |
| | | | NM_198039 | | | |
| NFATC2IP | 212808_at | AI884627 | NM_032815 | 84901 | 0.88 | 1.28E-03 |
| AW444985 | 243690_at | AW444985 | --- | --- | 0.87 | 3.30E-03 |
| SYT11 | 232677_at | AU146128 | NM_152280 | 23208 | 0.87 | 7.93E-03 |
| PTPN1 | 202716_at | NM_002827 | NM_002827 | 5770 | 0.87 | 7.56E-03 |
| FLJ45983 | 240827_at | AA846824 | NM_207423 | 399717 | 0.87 | 6.25E-03 |
| BM041211 | 1562062_at | BM041211 | --- | --- | 0.87 | 8.38E-03 |
| UQCRC2 | 241755_at | AI961429 | NM_003366 | 7385 | 0.87 | 1.07E-03 |
| WHSC2 | 1557300_s_at | AI741292 | NM_005663 | 7469 | 0.87 | 7.80E-03 |
| PDE7A | 224046_s_at | U67932 | NM_002603 | 5150 | 0.87 | 9.76E-03 |
| | | | NM_002604 | | | |
| PHF20 | 235389_at | BG168139 | NM_016436 | 51230 | 0.87 | 1.49E-03 |
| AW973410 | 244310_at | AW973410 | XM_498613 | 440283 | 0.87 | 4.14E-03 |
| FLJ10120 | 220220_at | NM_018001 | --- | 55073 | 0.86 | 4.44E-03 |
| ILF3 | 208931_s_at | AF147209 | NM_004516 | 3609 | 0.86 | 4.15E-04 |
| | | | NM_012218 | | | |
| | | | NM_153464 | | | |
| MARCKS | 201670_s_at | M68956 | NM_002356 | 4082 | 0.86 | 1.21E-03 |
| U06715 | 217200_x_at | U06715 | --- | --- | 0.86 | 6.65E-03 |
| 182-FIP | 224956_at | AI743689 | NM_020772 | 57532 | 0.85 | 8.46E-03 |
| FLJ13855 | 236107_at | AA147920 | NM_023079 | 65264 | 0.85 | 4.95E-03 |
| BI496583 | 1557675_at | BI496583 | --- | --- | 0.85 | 1.81E-03 |
| C10orf58 | 228155_at | BF512388 | NM_032333 | 84293 | 0.84 | 4.87E-03 |
| SEZ6L | 216047_x_at | AL050253 | NM_021115 | 23544 | 0.84 | 6.17E-04 |
| LOC254057 | 232370_at | AI635756 | --- | 254057 | 0.84 | 3.35E-03 |
| TCF7L2 | 216511_s_at | AJ270770 | NM_030756 | 6934 | 0.84 | 7.95E-03 |
| OKFZP564K196 | 223170_at | AF132000 | NM_015544 | 26022 | 0.84 | 3.64E-03 |
| SFRS2 | 200754_x_at | NM_003016 | NM_003016 | 6427 | 0.83 | 7.20E-04 |
| AA447714 | 244047_at | AA447714 | --- | --- | 0.83 | 8.79E-03 |
| MARCKS | 201668_x_at | AW163148 | NM_002356 | 4082 | 0.83 | 5.34E-03 |
| AF222691 | 216187_x_at | AF222691 | --- | --- | 0.3 | 8.37E-03 |
| DCL-1 | 232371_at | AU155401 | NM_014880 | 9936 | 0.83 | 8.82E-04 |
| RPS24 | 1555878_at | AK094613 | NM_001026 | 6229 | 0.83 | 7.05E-03 |
| | | | NM_033022 | | | |
| ZNF238 | 212774_at | AJ223321 | NM_006352 | 10472 | 0.82 | 1.69E-03 |
| | | | NM_205768 | | | |
| NKTR | 202379_s_at | AI361805 | NM_005385 | 4820 | 0.82 | 2.20E-03 |
| KIAA0485 | 242824_at | AW191647 | --- | 57235 | 0.82 | 2.73E-03 |
| CSE1L | 201111_at | AF053641 | NM_001316 | 1434 | 0.82 | 9.40E-03 |
| | | | NM_177436 | | | |
| NKTR | 1557736_at | AI880383 | NM_005385 | 4820 | 0.82 | 2.47E-03 |
| MBD2 | 214397_at | AI827820 | NM_003927 | 8932 | 0.82 | 8.51E-03 |
| | | | NM_015832 | | | |
| MCCC1 | 242223_at | AA505323 | NM_020166 | 56922 | 0.82 | 5.18E-04 |
| CREBBP | 237239_at | AW183655 | NM_004380 | 1387 | 0.82 | 1.36E-03 |
| LOC129285 | 1554145_a_at | BC040721 | NM_152994 | 129285 | 0.81 | 7.02E-03 |
| SEZ6L | 213609_s_at | AB023144 | NM_021115 | 23544 | 0.81 | 8.59E-03 |
| HNRPM | 238611_at | AI906424 | NM_005968 | 4670 | 0.81 | 5.61E-03 |
| | | | NM_031203 | | | |
| MYLIP | 223129_x_at | T63512 | NM_013262 | 29116 | 0.81 | 1.14E-03 |
| AI476542 | 243032_at | AI476542 | --- | --- | 0.81 | 4.19E-03 |
| HYPB | 241458_at | AI868267 | NM_012271 | 29072 | 0.81 | 8.29E-03 |
| | | | NM_014159 | | | |
| SNRPB2 | 1561965_at | BE466925 | NM_003092 | 6629 | 0.81 | 8.93E-04 |
| | | | NM_198220 | | | |
| SMARCA4 | 217656_at | AW128846 | NM_003072 | 6597 | 0.80 | 2.21E-03 |
| MRPL4 | 223742_at | BC000756 | NM_015956 | 51073 | 0.80 | 7.99E-05 |
| | | | NM_146387 | | | |
| | | | NM_146388 | | | |
| HS6ST2 | 1552767_a_at | NM_147174 | NM_147174 | 90161 | 0.80 | 2.64E-03 |
| | | | NM_147175 | | | |
| NEURL | 243853_at | BF516539 | NM_004210 | 9148 | 0.80 | 5.90E-04 |
| SMARCD1 | 203183_s_at | NM_003076 | NM_003076 | 6602 | 0.80 | 2.99E-03 |
| | | | NM_139071 | | | |
| HECTD1 | 242349_at | AW275658 | NM_015382 | 25831 | 0.80 | 7.69E-04 |
| GGA2 | 213772_s_at | BF196572 | NM_015044 | 23062 | 0.80 | 3.58E-03 |
| | | | NM_138640 | | | |
| AA056145 | 227633_at | AA056145 | --- | --- | 0.79 | 9.47E-04 |
| MAN2A1 | 235103_at | AA029155 | NM_002372 | 4124 | 0.79 | 7.09E-03 |
| SENP3 | 215114_at | AK000923 | NM_015670 | 26168 | 0.79 | 2.65E-09 |
| GULP1 | 223837_at | BC001103 | NM_016315 | 51454 | 0.78 | 2.45E-03 |
| JARID2 | 203297_s_at | BG029530 | NM_004973 | 3720 | 0.78 | 9.88E-04 |
| DNAPTP6 | 222154_s_at | AK002064 | NM_015535 | 26010 | 0.78 | 5.12E-03 |
| VPS29 | 223026_s_at | AF201946 | NM_016226 | 51699 | 0.78 | 9.91E-03 |
| | | | NM_057180 | | | |
| AF279774 | 216967_at | AF279774 | --- | --- | 0.78 | 1.36E-03 |
| COBLL1 | 203642_s_at | NM_014900 | NM_014900 | 22837 | 0.78 | 1.12E-03 |
| AI041522 | 228030_at | A1041522 | --- | --- | 0.78 | 4.80E-03 |
| FLJ11029 | 219392_x_at | NM_018304 | NM_018304 | 55771 | 0.78 | 4.83E-03 |
| RNF34 | 219035_s_at | NM_025126 | NM_025126 | 80196 | 0.77 | 2.35E-03 |
| | | | NM_194271 | | | |
| BF056892 | 229024_at | BF056892 | --- | --- | 0.77 | 3.47E-06 |
| DKFZP564C18C | 1559139_at | BC009786 | NM_015658 | 26155 | 0.77 | 3.20E-03 |
| SFRS1 | 227164_at | AL521786 | NM_006924 | 6426 | 0.77 | 1.00E-03 |
| MR-1 | 225298_at | AA074597 | NM_015488 | 25953 | 0.76 | 4.65E-03 |
| | | | NM_022572 | | | |
| ATP6V0A2 | 217603_at | AW444520 | NM_012463 | 23545 | 0.76 | 2.94E-03 |
| RECQL | 210568_s_at | BC001052 | NM_002907 | 5965 | 0.76 | 4.23E-03 |
| | | | NM_032941 | | | |
| YWHAB | 208743_s_at | BC001359 | NM_003404 | 7529 | 0.76 | 4.79E-03 |
| | | | NM_139323 | | | |
| ANKHD1 | 229457_at | AU148042 | NM_017747 | 54882 | 0.76 | 4.26E-03 |
| | | | NM_017978 | | | |
| | | | NM_024668 | | | |
| STRBP | 233251_at | AK024960 | NM_018387 | 55342 | 0.76 | 3.48E-03 |
| AF147390 | 1562230_at | AF147390 | --- | --- | 0.76 | 2.73E-09 |
| HIC2 | 212966_at | AL043112 | NM_015094 | 23119 | 0.76 | 4.03E-03 |
| LUC7L2 | 226758_at | AA043552 | NM_016019 | 51631 | 0.76 | 5.54E-03 |
| GNG2 | 224965_at | AU118419 | NM_053064 | 54331 | 0.76 | 4.33E-03 |
| USP7 | 230761_at | AI972599 | NM_003470 | 7874 | 0.75 | 2.83E-03 |
| SH3MD1 | 213252_at | AI739005 | NM_014631 | 9644 | 0.75 | 1.10E-03 |
| TLE4 | 204872_at | NM_007005 | NM_007005 | 7091 | 0.75 | 4.50E-03 |
| AA167167 | 243684_at | AA167167 | --- | --- | 0.75 | 4.31E-03 |
| SLC25A13 | 229061_s_at | AW268880 | NM_014251 | 10165 | 0.75 | 6.06E-03 |
| NSD1 | 235760_at | AI421972 | NM_022455 | 64324 | 0.75 | 9.51E-03 |
| | | | NM_172349 | | | |
| BTBD14A | 243431_at | BF000597 | NM_144653 | 138151 | 0.75 | 9.59E-03 |
| AKR1B1 | 201272_at | NM_001628 | NM_001628 | 231 | 0.74 | 2.19E-03 |
| SDS3 | 233841_s_at | AK026749 | NM_022491 | 64426 | 0.74 | 1.60E-04 |
| ANAPC7 | 225554_s_at | AA131793 | NM_016238 | 51434 | 0.74 | 4.31E-03 |
| EPOR | 215054_at | H16758 | NM_000121 | 2057 | 0.74 | 9.06E-04 |
| KHDRBS3 | 209781_s_at | AF069681 | NM_006558 | 10656 | 0.74 | 6.64E-04 |
| C6orf115 | 223361_at | AF116682 | XM_371848 | 58527 | 0.73 | 1.49E-03 |
| LOC157627 | 214839_at | AF052108 | --- | 157627 | 0.73 | 8.43E-03 |
| HS6ST2 | 230030_at | A1767756 | NM_147174 | 90161 | 0.73 | 3.77E-03 |
| | | | NM_147175 | | | |
| DHX35 | 218579_s_at | NM_021931 | NM_021931 | 60625 | 0.73 | 6.34E-06 |
| AI700633 | 212812_at | AI700633 | --- | --- | 0.73 | 7.29E-03 |
| ZNF281 | 222619_at | AU150752 | NM_012482 | 23528 | 0.73 | 9.28E-04 |
| NARG1 | 219158_s_at | NM_025085 | NM_057175 | 80155 | 0.73 | 4.16E-03 |
| C5orf13 | 201309_x_at | U36189 | NM_004772 | 9315 | 0.73 | 6.38E-03 |
| PBP | 205353_s_at | NM_002567 | NM_002567 | 5037 | 0.73 | 5.93E-04 |
| KIAA0220 | 211996_s_at | BG256504 | --- | 283846 | 0.72 | 1.81E-03 |
| MYH10 | 212372_at | AK026977 | NM_005964 | 4628 | 0.72 | 1.71E-03 |
| AU147166 | 215212_at | AU147166 | --- | --- | 0.71 | 8.93E-03 |
| BTBD3 | 202946_s_at | NM_014962 | NM_014962 | 22903 | 0.71 | 8.99E-04 |
| | | | NM_181443 | | | |
| DNAJC9 | 213088_s_at | BE551340 | NM_015190 | 23234 | 0.71 | 9.31E-03 |
| MAN1A1 | 208116_s_at | NM_005907 | NM_005907 | 4121 | 0.71 | 5.70E-03 |
| ATP5G1 | 208972_s_at | AL080089 | NM_00100202 7 | 516 | 0.71 | 3.03E-03 |
| | | | NM_005175 | | | |
| PAICS | 201013_s_at | AA902652 | NM_006452 | 10606 | 0.71 | 2.75E-03 |
| AI378500 | 229821_at | AI378500 | --- | --- | 0.71 | 3.49E-03 |
| KLHL5 | 226001_at | AK002174 | NM_00100707 5 | 51088 | 0.71 | 2.63E-03 |
| | | | NM_199039 | | | |
| SFRS9 | 201698_s_at | NM_003769 | NM_003769 | 8683 | 0.71 | 5.14E-04 |
| PABPN1 | 213046_at | AI130920 | NM_004643 | 8106 | 0.71 | 3.81E-04 |
| GARNL1 | 234923_at | AK022988 | NM_014990 | 253959 | 0.70 | 5.70E-03 |
| | | | NM_194301 | | | |
| MAN1A1 | 221760_at | BG287153 | NM_005907 | 4121 | 0.70 | 7.22E-03 |
| C5orf13 | 201310_s_at | NM_004772 | NM_004772 | 9315 | 0.70 | 6.72E-03 |
| NM_031221 | 208120_x_at | NM_031221 | --- | --- | 0.70 | 4.42E-04 |
| CYHR1 | 213681_at | AW512817 | NM_032687 | 50626 | 0.70 | 4.56E-03 |
| BF437591 | 222026_at | BF437591 | --- | --- | 0.70 | 7.17E-03 |
| SEZ6L | 231650_s_at | BE672217 | NM_021115 | 23544 | 0.70 | 9.10E-03 |
| UFD1L | 209103_s_at | BC001049 | NM_005659 | 7353 | 0.70 | 1.25E-03 |
| H37943 | 242311_x_at | H37943 | --- | --- | 0.70 | 2.95E-03 |
| ACY1L2 | 225431_x_at | BE779764 | NM_001010852 | 135293 | 0.70 | 3.20E-03 |
| SCC-112 | 213983_s_at | AW991219 | NM_015200 | 23244 | 0.70 | 8.02E-04 |
| RPUSD3 | 1555916_at | BM670238 | NM_173659 | 285367 | 0.70 | 1.32E-05 |
| CCAR1 | 224736_at | BE617899 | NM_018237 | 55749 | 0.69 | 8.03E-03 |
| SNX1 | 1559154_at | AK098001 | NM_003099 | 6642 | 0.69 | 5.03E-03 |
| | | | NM_148955 | | | |
| | | | NM_152826 | | | |
| MGC52282 | 231638_at | AA400057 | NM_178453 | 124221 | 0.69 | 4.60E-03 |
| MAPKAPK3 | 202788_at | NM_004635 | NM_004635 | 7867 | 0.69 | 3.76E-03 |
| HNRPA3 | 211931_s_at | BG505670 | NM_194247 | 220988 | 0.69 | 2.94E-04 |
| AK024881 | 234723_x_at | AK024881 | --- | --- | 0.69 | 7.45E-03 |
| PCIA1 | 218260_at | NM_024050 | NM_024050 | 79016 | 0.69 | 9.77E-03 |
| ANKRD36 | 238464_at | AA463221 | XM_371540 | 375248 | 0.69 | 2.40E-03 |
| N4BP2 | 231996_at | AB037834 | NM_018177 | 55728 | 0.69 | 1.75E-03 |
| EMID2 | 229080_at | BE741432 | NM_133457 | 136227 | 0.68 | 1.65E-03 |
| FLJ22490 | 242041_at | AI933671 | NM_024790 | 79848 | 0.68 | 7.80E-03 |
| IDH3B | 210418_s_at | AF023265 | NM_006899 | 3420 | 0.68 | 2.50E-03 |
| | | | NM_174855 | | | |
| | | | NM_174856 | | | |
| MAN2A1 | 205105 _ at | NM_002372 | NM_002372 | 4124 | 0.68 | 1.85E-03 |
| BF055201 | 228242_at | BF055201 | --- | --- | 0.68 | 9.05E-03 |
| IDH3B | 210014_x_at | AF023266 | NM_006899 | 3420 | 0.68 | 2.17E-03 |
| | | | NM_174855 | | | |
| | | | NM_174856 | | | |
| GALNT4 | 231832_at | AI890347 | NM_003774 | 8693 | 0.67 | 7.13E-03 |
| OCIAD1 | 232644_x_at | AK024302 | NM_017830 | 54940 | 0.67 | 5.83E-03 |
| GPR51 | 211679_x_at | AF095784 | NM_005458 | 9568 | 0.67 | 6.98E-03 |
| SRC | 213324_at | AK024281 | NM_005417 | 6714 | 0.67 | 1.56E-03 |
| | | | NM_198291 | | | |
| PBP | 210825_s_at | AF130103 | NM_002567 | 5037 | 0.67 | 4.20E-03 |
| THRAP4 | 213043_s_at | AI023317 | NM_014815 | 9862 | 0.67 | 6.87E-04 |
| ATAD3A | 1552641_s_at | NM_031921 | NM_018188 | 55210 | 0.66 | 7.21E-03 |
| ATAD3B | | | NM_031921 | 83858 | | |
| FLJ20364 | 1559222_at | AA781731 | NM_017785 | 54908 | 0.66 | 1.64E-03 |
| TA-PP2C | 225204_at | AA521311 | NM_139283 | 160760 | 0.66 | 2.71E-03 |
| EIF5A | 201123_s_at | NM_001970 | NM_001970 | 1984 | 0.66 | 8.31E-04 |
| RECQL | 212917_x_at | AI814728 | NM_002907 | 5965 | 0.66 | 3.26E-03 |
| | | | NM_032941 | | | |
| ZC3HDC5 | 1562434_at | AL832312 | XM_036115 | 85451 | 0.66 | 7.52E-03 |
| BCR | 202315_s_at | NM_004327 | NM_004327 | 613 | 0.66 | 3.87E-03 |
| | | | NM_021574 | | | |
| C14orf2 | 242858_at | BF507638 | NM_004894 | 9556 | 0.66 | 1.48E-03 |
| MAP3K13 | 206249_at | NM_004721 | NM_004721 | 9175 | 0.66 | 3.65E-03 |
| SULT1A3 | 222094_at | AI580112 | NM_003166 | 6818 | 0.66 | 3.16E-04 |
| | | | NM_177552 | | | |
| ZNF207 | 244153_at | AA521438 | NM_003457 | 7756 | 0.65 | 8.57E-03 |
| PEX26 | 219180_s_at | AI817074 | NM_017929 | 55670 | 0.65 | 4.16E-03 |
| RAN | 200750_s_at | AF054183 | NM_006325 | 5901 | 0.65 | 6.43E-03 |
| EIF5A | 213753_x_at | BF541557 | NM_001970 | 1984 | 0.65 | 1.96E-05 |
| C13orf6 | 235348_at | AA404347 | NM_032859 | 84945 | 0.65 | 2.02E-03 |
| EWSR1 | 210012_s_at | BC000527 | NM_005243 | 2130 | 0.65 | 4.34E-03 |
| | | | NM_013986 | | | |
| SPG7 | 230885_at | BE670386 | NM_003119 | 6687 | 0.65 | 7.60E-03 |
| | | | NM_199367 | | | |
| SCLY | 59705_at | AA911739 | NM_016510 | 51540 | 0.65 | 9.03E-03 |
| KCTD10 | 223208_at | AF113208 | NM_031954 | 83892 | 0.64 | 1.07E-04 |
| FLJ13149 | 219016_at | NM_021826 | NM_021826 | 60493 | 0.64 | 7.47E-04 |
| ZNF217 | 203739_at | NM_006526 | NM_006526 | 7764 | 0.64 | 2.22E-03 |
| GGA2 | 208913_at | AA868560 | NM_015044 | 23062 | 0.64 | 4.26E-03 |
| | | | NM_138640 | | | |
| DST | 1560296_at | AV702787 | NM_001723 | 667 | 0.64 | 8.16E-03 |
| | | | NM_015548 | | | |
| | | | NM_020388 | | | |
| | | | NM_183380 | | | |
| UBQLN1 | 222989_s_at | NM_013438 | NM_013438 | 29979 | 0.64 | 9.49E-03 |
| | | | NM_053067 | | | |
| TOMM40 | 202264_s_at | NM_006114 | NM_006114 | 10452 | 0.63 | 5.40E-03 |
| VTI1A | 235034_at | AW194969 | NM_145206 | 143187 | 0.63 | 9.95E-03 |
| TMEM33 | 222642_s_at | BC000948 | NM_018126 | 55161 | 0.63 | 6.62E-03 |
| KIAA1219 | 221738_at | BG236163 | NM_020336 | 57148 | 0.63 | 1.93E-03 |
| RPL23 | 215224_at | AK025200 | NM_000978 | 9349 | 0.63 | 7.04E-04 |
| ZNF43 | 222136_x_at | AK022905 | NM_003423 | 7594 | 0.63 | 4.57E-03 |
| BE671123 | 235798_at | BE671123 | --- | --- | 0.63 | 8.79E-03 |
| WDR17 | 1552430_at | NM_170710 | NM_170710 | 116966 | 0.63 | 5.45E-04 |
| | | | NM_181265 | | | |
| FUBP1 | 212847_at | AL036840 | NM_003902 | 8880 | 0.63 | 6.95E-03 |
| MBTD1 | 207115_x_at | NM_017643 | NM_017643 | 54799 | 0.63 | 7.84E-03 |
| PHC2 | 1557852_at | AW418842 | NM_004427 | 1912 | 0.62 | 8.02E-03 |
| | | | NM_198040 | | | |
| AP1S2 | 228415_at | AA205444 | NM_003916 | 8905 | 0.62 | 8.44E-03 |
| GPR125 | 210473_s_at | M37712 | NM_145290 | 166647 | 0.62 | 6.47E-03 |
| SFRS6 | 208804_s_at | AL031681 | NM_006275 | 6431 | 0.62 | 3.30E-03 |
| AI216567 | 236754_at | AI216567 | --- | --- | 0.62 | 4.60E-03 |
| C15orf25 | 220071_x_at | NM_018097 | NM_018097 | 55142 | 0.62 | 9.64E-03 |
| CDH2 | 203441_s_at | NM_001792 | NM_001792 | 1000 | 0.62 | 5.11E-03 |
| MRPL35 | 222775_s_at | AK026220 | NM_016622 | 51318 | 0.62 | 1.89E-03 |
| | | | NM_145644 | | | |
| CNP | 208912_s_at | BC001362 | NM_033133 | 1267 | 0.62 | 3.84E-03 |
| FLJ20331 | 218577_at | NM_017768 | NM_017768 | 55631 | 0.62 | 6.26E-03 |
| FLJ21415 | 218867_s_at | NM_024738 | NM_024738 | 79794 | 0.62 | 3.36E-03 |
| AF336882 | 224288_x_at | AF336882 | --- | --- | 0.62 | 3.59E-03 |
| SSBP3 | 223635_s_at | BC003605 | NM_00100995 5 | 23648 | 0.62 | 2.27E-03 |
| | | | NM_018070 | | | |
| | | | NM_145716 | | | |
| ATF7IP | 231825_x_at | AK025060 | NM_018179 | 55729 | 0.62 | 7.81E-03 |
| TOP1 | 208901_s_at | J03250 | NM_003286 | 7150 | 0.61 | 6.28E-04 |
| SYNCRIP | 209024_s_at | AI472757 | NM_006372 | 10492 | 0.61 | 1.15E-03 |
| DHX30 | 239772_x_at | AI368527 | NM_014966 | 22907 | 0.61 | 1.59E-04 |
| | | | NM_138614 | | | |
| | | | NM_138615 | | | |
| BE878277 | 212829_at | BE878277 | --- | --- | 0.61 | 1.63E-03 |
| FBXL10 | 226215_s_at | AI989996 | NM_00100536 6 | 84678 | 0.61 | 7.38E-03 |
| | | | NM_032590 | | | |
| KIAA0251 | 1555347_at | BC033748 | NM_015027 | 23042 | 0.61 | 2.52E-03 |
| RBM14 | 204178_s_at | NM_006328 | NM_006328 | 10432 | 0.61 | 3.59E-05 |
| EXOSC1 | 1559044_at | BC012538 | NM_016046 | 51013 | 0.61 | 7.00E-04 |
| KIAA0992 | 234135_x_at | AK021652 | NM_016081 | 23022 | 0.60 | 8.94E-03 |
| EIF5A | 201122_x_at | BC000751 | NM_001970 | 1984 | 0.60 | 1.14E-03 |
| R26456 | 243135_x_at | R26456 | --- | --- | 0.60 | 5.74E-03 |
| TMEM33 | 218465_at | NM_018126 | NM_018126 | 55161 | 0.60 | 8.99E-03 |
| SFRS1 | 201741_x_at | M69040 | NM_006924 | 6426 | 0.60 | 1.36E-03 |
| RAB11FIP3 | 214999_s_at | AW243089 | NM_014700 | 9727 | 0.60 | 1.50E-04 |
| DDX46 | 228039_at | AI765169 | NM_014829 | 9879 | 0.60 | 2.91E-03 |
| HNRPU | 200593_s_at | BC003621 | NM_004501 | 3192 | 0.60 | 1.26E-05 |
| | | | NM_031844 | | | |
| HNRPU | 200594_x_at | NM_004501 | NM_004501 | 3192 | 0.59 | 1.74E-04 |
| | | | NM_031844 | | | |
| GNG12 | 212294_at | BG111761 | NM_018841 | 55970 | 0.59 | 6.29E-03 |
| ARS2 | 201680_x_at | NM_015908 | NM_015908 | 51593 | 0.59 | 2.20E-04 |
| | | | NM_182800 | | | |
| FLJ37078 | 235880_at | BF593867 | NM_153043 | 222183 | 0.59 | 7.82E-05 |
| GPR51 | 209990_s_at | AF056085 | NM_005458 | 9568 | 0.59 | 7.76E-03 |
| MARCKS | 201669_s_at | NM_002356 | NM_002356 | 4082 | 0.59 | 4.74E-05 |
| MAN2A1 | 226538_at | AV700323 | NM_002372 | 4124 | 0.59 | 4.65E-03 |
| HCFC1 | 202474_s_at | NM_005334 | NM_005334 | 3054 | 0.59 | 2.12E-03 |
| FLJ35709 | 242540_at | AI911330 | NM_173589 | 283294 | 0.58 | 5.90E-03 |
| MYO1B | 212365_at | BF215996 | NM_012223 | 4430 | 0.58 | 6.28E-04 |
| PPP2CA | 208652_at | BC000400 | NM_002715 | 5515 | 0.58 | 3.88E-03 |
| CDC5L | 209055_s_at | AW268817 | NM_001253 | 988 | 0.58 | 2.61E-04 |

**Table 12. Decreased Gene Expression in Cell Lines H1 and BB10 vs.Cell Line SiMa in Differentiated State Using Microarray Analysis**

| **Gene Symbol** | **Probe Set ID** | **Representative Public ID** | **RefSeq Transcript ID** | **Entrez Gene** | **Log2 Ratio** | **pvalue** |
|---|---|---|---|---|---|---|
| CYTL1 | 219837_s_at | NM_018659 | NM_018659 | 54360 | -6.94 | 6.41E-06 |
| GNAS | 217057_s_at | AF107846 | NM_000516 | 2778 | -5.47 | 4.18E-03 |
| | | | NM_016592 | | | |
| | | | NM_080425 | | | |
| | | | NM_080426 | | | |
| CYGB | 226632_at | AL513673 | NM_134268 | 114757 | -4.81 | 3.60E-03 |
| TNFRSF10D | 227345_at | AI738556 | NM_003840 | 8793 | -4.68 | 2.31E-06 |
| ECEL1 | 219914_at | NM_004826 | NM_004826 | 9427 | -4.67 | 3.83E-05 |
| SYT13 | 226086_at | AB037848 | NM_020826 | 57586 | -4.06 | 1.83E-04 |
| TMIE | 1553601_a_at | NM_147196 | NM_147196 | 259236 | -3.86 | 6.07E-05 |
| NNAT | 204239_s_at | NM_005386 | NM_005386 | 4826 | -3.83 | 5.67E-07 |
| | | | NM_181689 | | | |
| CNGA3 | 207261_at | NM_001298 | NM_001298 | 1261 | -3.76 | 4.84E-04 |
| AU157716 | 227051_at | AU157716 | --- | --- | -3.75 | 4.31E-03 |
| AF150317 | 243111_at | AF150317 | --- | --- | -3.69 | 9.83E-04 |
| ASAM | 226834_at | BG112263 | NM_024769 | 79827 | -3.61 | 5.78E-03 |
| LOC254128 | 1557059_at | AK098264 | --- | 254128 | -3.51 | 1.95E-07 |
| LOC339290 | 228160_at | AI433706 | XM_375527 | 339290 | -3.50 | 6.83E-05 |
| FUCA2 | 223120_at | BC003060 | NM_032020 | 2519 | -3.47 | 8.50E-03 |
| STX3A | 209238_at | BE966922 | NM_004177 | 6809 | -3.45 | 8.83E-03 |
| CYGB | 1553572_a_at | NM_134268 | NM_134268 | 114757 | -3.43 | 4.93E-06 |
| EDG7 | 231192_at | AW274018 | NM_012152 | 23566 | -3.39 | 1.39E-03 |
| HTATIP2 | 207180_s_at | NM_006410 | NM_006410 | 10553 | -3.38 | 4.96E-04 |
| HTATIP2 | 209448_at | BC002439 | NM_006410 | 10553 | -3.38 | 3.60E-04 |
| LMCD1 | 218574_s_at | NM_014583 | NM_014583 | 29995 | -3.34 | 6.84E-03 |
| BACE2 | 217867_x_at | NM_012105 | NM_012105 | 25825 | -3.33 | 5.05E-04 |
| | | | NM_138991 | | | |
| | | | NM_138992 | | | |
| FLJ45880 | 228538_at | BE671164 | NM_207404 | 389114 | -3.28 | 4.82E-06 |
| EDG7 | 220816_at | NM_012152 | NM_012152 | 23566 | -3.28 | 2.18E-03 |
| CDC42EP1 | 204693_at | NM_007061 | NM_007061 | 11135 | -3.25 | 9.63E-03 |
| | | | NM_152243 | | | |
| TCEAL2 | 211276_at | AF063606 | NM_080390 | 140597 | -3.25 | 3.45E-04 |
| MEOX2 | 206201_s_at | NM_005924 | NM_005924 | 4223 | -3.23 | 2.59E-06 |
| FGF13 | 205110_s_at | NM_004114 | NM_004114 | 2258 | -3.22 | 4.26E-05 |
| | | | NM_033642 | | | |
| IGFBP7 | 201163_s_at | NM_001553 | NM_001553 | 3490 | -3.17 | 3.25E-03 |
| BACE2 | 222446_s_at | AF178532 | NM_012105 | 25825 | -3.16 | 1.05E-04 |
| | | | NM_138991 | | | |
| | | | NM_138992 | | | |
| SLC35F3 | 229065_at | BF968270 | NM_173508 | 148641 | -3.14 | 1.47E-06 |
| TncRNA | 224565_at | BE675516 | --- | 283131 | -3.11 | 3.26E-03 |
| LGALS3 | 208949_s_at | BC001120 | NM_002306 | 3958 | -3.08 | 1.25E-03 |
| GALIG | | | NM_194327 | 81625 | | |
| HSPA1A | 200799_at | NM_005345 | NM_005345 | 3303 | -3.07 | 6.70E-03 |
| AW136654 | 240802_at | AW136654 | --- | --- | -3.06 | 1.56E-03 |
| LOC56901 | 218484_at | NM_020142 | NM_020142 | 56901 | -3.02 | 3.26E-03 |
| MMP14 | 202827_s_at | AU149305 | NM_004995 | 4323 | -3.01 | 1.34E-05 |
| FLJ20701 | 219093_at | NM_017933 | NM_017933 | 55022 | -2.99 | 6.11E-03 |
| CAP2 | 212554_at | N90755 | NM_006366 | 10486 | -2.95 | 3.40E-03 |
| BF941609 | 240218_at | BF941609 | --- | --- | -2.91 | 1.65E-08 |
| SPINT2 | 210715_s_at | AF027205 | NM_021102 | 10653 | -2.90 | 6.36E-06 |
| BG325630 | 234973_at | BG325630 | --- | --- | -2.90 | 2.55E-03 |
| )KFZp313N062 | 1555905_a_at | AI147556 | NM_173826 | 285343 | -2.87 | 1.28E-04 |
| NPY | 206001_at | NM_000905 | NM_000905 | 4852 | -2.85 | 5.26E-03 |
| CYP2E1 | 1431_at | J02843 | NM_000773 | 1571 | -2.75 | 3.27E-03 |
| HHLA3 | 220387_s_at | NM_007071 | NM_007071 | 11147 | -2.72 | 6.75E-04 |
| IL13RA2 | 206172_at | NM_000640 | NM_000640 | 3598 | -2.66 | 6.71E-05 |
| FLJ31295 | 226509_at | AV700302 | NM_152320 | 121274 | -2.65 | 7.10E-04 |
| TncRNA | 224566_at | AI042152 | --- | 283131 | -2.65 | 1.05E-04 |
| DKFZP564I0422 | 223588_at | AL136607 | NM_031435 | 83591 | -2.64 | 1.28E-05 |
| IGSF4D | 1552754_a_at | AA640422 | NM_153184 | 253559 | -2.64 | 1.95E-03 |
| PRKACB | 235780_at | BE622723 | NM_002731 | 5567 | -2.63 | 2.35E-03 |
| | | | NM_182948 | | | |
| | | | NM_207578 | | | |
| GNAS | 214157_at | AA401492 | NM_000516 | 2778 | -2.61 | 1.23E-04 |
| | | | NM_016592 | | | |
| | | | NM_080425 | | | |
| | | | NM_080426 | | | |
| AW770245 | 236513_at | AW770245 | --- | --- | -2.60 | 8.47E-05 |
| LGI1 | 206349_at | NM_005097 | NM_005097 | 9211 | -2.60 | 8.58E-04 |
| AI810266 | 235456_at | AI810266 | --- | --- | -2.58 | 5.24E-03 |
| HIST1H2BD | 209911_x_at | BC002842 | NM_021063 | 3017 | -2.55 | 2.10E-03 |
| | | | NM_138720 | | | |
| AL353759 | 215071_s_at | AL353759 | --- | --- | -2.51 | 9.79E-04 |
| LOC339290 | 215283_at | U79248 | XM_375527 | 339290 | -2.51 | 3.84E-04 |
| SLC16A3 | 202856_s_at | NM_004207 | NM_004207 | 9123 | -2.51 | 8.97E-03 |
| LGALS1 | 201105_at | NM_002305 | NM_002305 | 3956 | -2.49 | 4.79E-03 |
| N92599 | 241421_at | N92599 | --- | --- | -2.47 | 1.03E-04 |
| ECHDC3 | 219298_at | NM_024693 | NM_024693 | 79746 | -2.47 | 7.27E-04 |
| NEBL | 203961_at | AL157398 | NM_006393 | 10529 | -2.43 | 4.07E-04 |
| | | | NM_213569 | | | |
| RAC2 | 207419_s_at | NM_002872 | NM_002872 | 5880 | -2.42 | 2.93E-03 |
| BF672306 | 229189_s_at | BF672306 | --- | --- | -2.42 | 1.43E-03 |
| CYP2E1 | 209975_at | AF182276 | NM_000773 | 1571 | -2.41 | 4.83E-03 |
| NEBL | 203962_s_at | NM_006393 | NM_006393 | 10529 | -2.40 | 9.78E-04 |
| | | | NM_213569 | | | |
| BE045392 | 243028_x_at | BE045392 | --- | --- | -2.40 | 3.93E-03 |
| LOC339290 | 217506_at | H49382 | XM_375527 | 339290 | -2.38 | 1.30E-05 |
| PPAPDC1 | 236044_at | BF130943 | XM_113641 | 196051 | -2.35 | 1.18E-04 |
| CYP2E1 | 209976_s_at | AF182276 | NM_000773 | 1571 | -2.34 | 7.08E-03 |
| ASAM | 228082_at | BF056275 | NM_024769 | 79827 | -2.32 | 6.80E-04 |
| ICAM2 | 204683_at | NM_000873 | NM_000873 | 3384 | -2.32 | 7.89E-03 |
| MEGF11 | 1552439_s_at | NM_032445 | NM_032445 | 84465 | -2.31 | 2.69E-03 |
| CDS1 | 226185_at | AK026697 | NM_001263 | 1040 | -2.31 | 4.59E-05 |
| ADARB1 | 203865_s_at | NM_015833 | NM_001112 | 104 | -2.30 | 2.34E-03 |
| | | | NM_015833 | | | |
| | | | NM_015834 | | | |
| ELMO1 | 204513_s_at | NM_014800 | NM_014800 | 9844 | -2.30 | 9.13E-03 |
| | | | NM_130442 | | | |
| BC039553 | 1559544_s_at | BC039553 | --- | --- | -2.29 | 2.32E-03 |
| IGFBP5 | 211959_at | AW007532 | NM_000599 | 3488 | -2.27 | 8.27E-03 |
| C6orf198 | 227834_at | AL589605 | XM_371849 | 167838 | -2.22 | 5.80E-03 |
| AI826125 | 229866_at | AI826125 | --- | --- | -2.20 | 1.50E-04 |
| NLF1 | 241031_at | BE218239 | NM_207322 | 145741 | -2.19 | 3.17E-03 |
| C6orf154 | 235935_at | AI539370 | XM_168060 | 221424 | -2.18 | 8.19E-04 |
| GAL | 214240_at | AL556409 | NM_015973 | 51083 | -2.17 | 3.60E-03 |
| CFC1 | 223753_s_at | AF312769 | NM_032545 | 55997 | -2.17 | 1.16E-04 |
| LOC344405 | 237291_at | AI695007 | XM_293034 | 344405 | -2.16 | 1.83E-03 |
| PTN | 211737_x_at | BC005916 | NM_002825 | 5764 | -2.13 | 4.30E-04 |
| PTGER2 | 206631_at | NM_000956 | NM_000956 | 5732 | -2.12 | 3.45E-05 |
| DKFZp761O011 | 207797_s_at | NM_018409 | NM_018409 | 55805 | -2.12 | 1.51E-03 |
| C1GALT1C1 | 238989_at | BF749723 | NM_001011551 | 29071 | -2.11 | 6.52E-04 |
| | | | NM_152692 | | | |
| AA757630 | 236724_at | AA757630 | --- | --- | -2.08 | 7.95E-06 |
| PDLIM3 | 209621_s_at | AF002280 | NM_014476 | 27295 | -2.08 | 9.67E-03 |
| KCNMA1 | 221584_s_at | U11058 | NM_002247 | 3778 | -2.08 | 1.62E-04 |
| TSAP6 | 218424_s_at | NM_018234 | NM_00100841 0 | 55240 | -2.07 | 6.98E-03 |
| | | | NM_018234 | | | |
| | | | NM_182915 | | | |
| ST3GAL3 | 1555702_a_at | AF425866 | NM_006279 | 6487 | -2.03 | 6.54E-03 |
| | | | NM_174963 | | | |
| | | | NM_174964 | | | |
| | | | NM_174965 | | | |
| | | | NM_174966 | | | |
| | | | NM_174967 | | | |
| | | | NM_174968 | | | |
| | | | NM_174969 | | | |
| | | | NM_174970 | | | |
| | | | NM_174971 | | | |
| | | | NM_174972 | | | |
| ZNF346 | 232753_at | AU147613 | NM_012279 | 23567 | -2.03 | 2.80E-04 |
| SYT5 | 206161_s_at | AI659957 | NM_003180 | 6861 | -2.02 | 8.93E-03 |
| HEBP2 | 203430_at | NM_014320 | NM_014320 | 23593 | -2.02 | 4.86E-05 |
| C9orf19 | 225604_s_at | AA284532 | NM_022343 | 152007 | -2.02 | 1.60E-03 |
| AKAP12 | 227529_s_at | BF511276 | NM_005100 | 9590 | -2.02 | 6.85E-05 |
| | | | NM_144497 | | | |
| FLJ20152 | 218532_s_at | NM_019000 | NM_019000 | 54463 | -2.01 | 2.04E-03 |
| SYNPR | 230303_at | H11380 | NM_144642 | 132204 | -1.99 | 7.36E-03 |
| UBE2D3 | 230171_at | AW662789 | NM_003340 | 7323 | -1.97 | 7.18E-06 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| STAC2 | 228888_at | AI821472 | NM_198993 | 342667 | -1.96 | 4.27E-04 |
| BE894882 | 235681_at | BE894882 | --- | --- | -1.95 | 5.54E-03 |
| AKAP12 | 210517_s_at | AB003476 | NM_005100 | 9590 | -1.94 | 6.61E-05 |
| | | | | | | |
| ZNF91 | 238466_at | R43486 | NM_003430 | 7644 | -1.93 | 2.26E-05 |
| HIST1H2BK | 209806_at | BC000893 | NM_080593 | 85236 | -1.92 | 1.28E-04 |
| BF432875 | 235782_at | BF432875 | XM_497668 | 388620 | -1.91 | 5.20E-03 |
| TMEM4 | 202857_at | NM_014255 | NM_014255 | 10330 | -1.91 | 5.19E-03 |
| FCRH1 | 243968_x_at | AI572979 | NM_052938 | 115350 | -1.90 | 4.72E-04 |
| AKAP12 | 227530_at | BF511276 | NM_005100 | 9590 | -1.89 | 1.05E-04 |
| | | | NM_144497 | | | |
| COL16A1 | 204345_at | NM_001856 | NM_001856 | 1307 | -1.89 | 4.40E-03 |
| FLJ32752 | 229631_at | AL040892 | NM_144666 | 144132 | -1.89 | 3.14E-03 |
| GPX7 | 213170_at | AA406605 | NM_015696 | 2882 | -1.89 | 3.82E-04 |
| BF000047 | 235736_at | BF000047 | --- | --- | -1.88 | 9.64E-03 |
| FLJ25952 | 243786_at | AA454190 | NM_153251 | 253832 | -1.87 | 9.52E-05 |
| ARTS-1 | 214012_at | BE551138 | NM_016442 | 51752 | -1.87 | 5.76E-03 |
| RAC2 | 213603_s_at | BE138888 | NM_002872 | 5880 | -1.86 | 2.55E-03 |
| IL7 | 206693_at | NM_000880 | NM_000880 | 3574 | -1.86 | 7.63E-03 |
| B3GNT5 | 225612_s_at | BE672260 | NM_032047 | 84002 | -1.86 | 1.01E-03 |
| BCL2 | 203685_at | NM_000633 | NM_000633 | 596 | -1.85 | 3.00E-03 |
| | | | NM_000657 | | | |
| ZAP128 | 202982_s_at | NM_006821 | NM_006821 | 10965 | -1.84 | 2.07E-03 |
| AF085995 | 1561759_at | AF085995 | --- | --- | -1.84 | 4.44E-04 |
| UCN | 206072_at | NM_003353 | NM_003353 | 7349 | -1.84 | 1.79E-04 |
| CDH10 | 220115_s_at | NM_006727 | NM_006727 | 1008 | -1.83 | 1.73E-03 |
| BF114967 | 231067_s_at | BF114967 | --- | --- | -1.83 | 6.70E-06 |
| ADAMTS1 | 222162_s_at | AK023795 | NM_006988 | 9510 | -1.82 | 1.09E-04 |
| PPFIA4 | 214978_s_at | AK023365 | NM_015053 | 8497 | -1.82 | 6.27E-03 |
| BE674309 | 230913_at | BE674309 | --- | --- | -1.81 | 1.25E-03 |
| AL355688 | 232081_at | AL355688 | --- | --- | -1.80 | 1.15E-03 |
| AI698684 | 1558626_at | AI698684 | --- | --- | -1.78 | 3.33E-03 |
| PRPH | 213847_at | NM_006262 | NM_006262 | 5630 | -1.78 | 3.01E-03 |
| PTGES | 210367_s_at | AF010316 | NM_004878 | 9536 | -1.78 | 4.71E-06 |
| | | | NM_198797 | | | |
| HPCAL1 | 205462_s_at | NM_002149 | NM_002149 | 3241 | -1.77 | 3.68E-03 |
| | | | NM_134421 | | | |
| INSIG1 | 229370_at | BF507344 | NM_005542 | 3638 | -1.77 | 3.55E-04 |
| | | | NM_198336 | | | |
| | | | NM_198337 | | | |
| RAB6B | 221792_at | AW118072 | NM_016577 | 51560 | -1.77 | 1.12E-04 |
| AI733281 | 239994_at | AI733281 | --- | --- | -1.75 | 1.40E-03 |
| NDFIP2 | 1568807_a_at | AI301081 | NM_019080 | 54602 | -1.75 | 2.04E-04 |
| KIAA1598 | 221802_s_at | AU157109 | NM_018330 | 57698 | -1.74 | 1.10E-03 |
| CAP2 | 212551_at | NM_006366 | NM_006366 | 10486 | -1.74 | 7.36E-03 |
| TICAM2 | 228234_at | AI423165 | NM_021649 | 353376 | -1.73 | 1.46E-03 |
| DSCAM | 237268_at | BE503065 | NM_001389 | 1826 | -1.73 | 5.66E-04 |
| | | | NM_206887 | | | |
| MATN3 | 230390_at | AI809899 | NM_002381 | 4148 | -1.72 | 2.82E-04 |
| AL833383 | 1563776_at | AL833383 | --- | --- | -1.71 | 6.57E-06 |
| C21orf109 | 1553608_a_at | NM_138968 | --- | 193629 | -1.70 | 9.95E-03 |
| FLJ35390 | 1569089_a_at | BC024303 | --- | 255031 | -1.70 | 2.94E-03 |
| AF052119 | 214821_at | AF052119 | --- | --- | -1.69 | 6.58E-04 |
| M160 | 223655_at | AF264014 | NM_174941 | 283316 | -1.69 | 9.95E-03 |
| AI588962 | 230477_at | AI588962 | --- | --- | -1.69 | 8.76E-05 |
| DKFZp313N062 | 1555906_s_at | AI147556 | NM_173826 | 285343 | -1.69 | 2.73E-04 |
| DRD1IP | 219896_at | NM_015722 | NM_015722 | 50632 | -1.69 | 4.68E-03 |
| DKFZp313N062 | 226688_at | AW003508 | NM_173826 | 285343 | -1.68 | 8.18E-03 |
| AW450420 | 236373_at | AW450420 | --- | --- | -1.66 | 8.61E-03 |
| LOC133308 | 1564746_at | BC009732 | NM_178833 | 133308 | -1.65 | 1.14E-04 |
| AL037414 | 230055_at | AL037414 | --- | --- | -1.63 | 9.73E-05 |
| RGAG4 | 227823_at | BE348679 | XM_291322 | 340526 | -1.63 | 4.87E-05 |
| APOBEC3G | 204205_at | NM_021822 | NM_021822 | 60489 | -1.62 | 9.31E-03 |
| BTN3A3 | 38241_at | U90548 | NM_006994 | 10384 | -1.62 | 2.24E-03 |
| | | | NM_197974 | | | |
| LOC133308 | 229491_at | BF433180 | NM_178833 | 133308 | -1.61 | 8.27E-04 |
| C21orf66 | 239407_at | AI793248 | NM_013329 | 94104 | -1.61 | 6.82E-03 |
| | | | NM_016631 | | | |
| | | | NM_058191 | | | |
| | | | NM_145328 | | | |
| DKFZp761N111 | 229254_at | BE550027 | NM_181644 | 148808 | -1.60 | 3.14E-03 |
| DSCAM | 211484_s_at | AF023450 | NM_001389 | 1826 | -1.60 | 1.94E-04 |
| | | | NM_206887 | | | |
| ADAMTS1 | 222486_s_at | AF060152 | NM_006988 | 9510 | -1.59 | 4.21E-03 |
| LOC286052 | 241370_at | AA278233 | --- | 286052 | -1.59 | 8.30E-05 |
| MGC40157 | 239754_at | BE671886 | NM_152350 | 125144 | -1.59 | 9.45E-03 |
| ZNF571 | 206648_at | NM_016536 | NM_016536 | 51276 | -1.59 | 1.41E-03 |
| LIPT1 | 242053_at | AW665279 | NM_015929 | 51601 | -1.59 | 3.26E-04 |
| | | | NM_145196 | | | |
| | | | NM_145197 | | | |
| | | | NM_145198 | | | |
| | | | NM_145199 | | | |
| FBI4 | 238081_at | AI694300 | NM_205857 | 404201 | -1.58 | 1.29E-03 |
| FLJ11286 | 53720_at | AI862559 | NM_018381 | 55337 | -1.58 | 1.79E-03 |
| FLJ30294 | 1553301_a_at | NM_144632 | NM_144632 | 130827 | -1.57 | 1.18E-04 |
| HUMRGE/M100UMRGE/M1009-HUMRGE/M100 | | | --- | --- | -1.57 | 8.64E-03 |
| HADHA | 232590_at | AK025919 | NM_000182 | 3030 | -1.57 | 4.01E-03 |
| NEDL1 | 243743_at | AI373512 | NM_015052 | 23072 | -1.56 | 9.50E-04 |
| BF114745 | 235459_at | BF114745 | --- | --- | -1.56 | 1.20E-03 |
| FLJ13614 | 226521_s_at | AK023676 | NM_139076 | 84142 | -1.55 | 2.64E-03 |
| AI276956 | 230766_at | AI276956 | --- | --- | -1.54 | 4.30E-03 |
| AI765942 | 243914_at | AI765942 | --- | --- | -1.54 | 6.05E-03 |
| TBC1D10 | 226613_at | AI742029 | NM_031937 | 83874 | -1.53 | 5.51E-03 |
| IL10RB | 209575_at | BC001903 | NM_000628 | 3588 | -1.53 | 1.40E-05 |
| GLT25D2 | 209883_at | AF288389 | NM_015101 | 23127 | -1.52 | 3.20E-05 |
| AI971535 | 229626_at | AI971535 | XM_370691 | 387856 | -1.52 | 8.91E-03 |
| FLJ13614 | 233543_s_at | AK021582 | NM_139076 | 84142 | -1.51 | 9.66E-03 |
| BF432855 | 237364_at | BF432855 | --- | --- | -1.51 | 2.88E-05 |
| AI590198 | 243418_at | AI590198 | --- | --- | -1.50 | 5.83E-03 |
| B7 | 206076_at | NM_006992 | NM_006992 | 10233 | -1.50 | 3.23E-03 |
| | | | NM_201650 | | | |
| PDGFRB | 202273_at | NM_002609 | NM_002609 | 5159 | -1.49 | 1.88E-03 |
| MCTP1 | 220122_at | NM_024717 | NM_00100279 6 | 79772 | -1.49 | 5.73E-04 |
| | | | NM_024717 | | | |
| AA747287 | 242273_at | AA747287 | --- | --- | -1.48 | 9.43E-03 |
| GPR123 | 239221_at | AI884909 | NM_032422 | 84435 | -1.48 | 1.25E-03 |
| C10orf57 | 222545_s_at | AA029276 | NM_025125 | 80195 | -1.48 | 3.36E-03 |
| HPCAL1 | 212552_at | BE617588 | NM_002149 | 3241 | -1.48 | 9.71E-04 |
| | | | NM_134421 | | | |
| LOC284361 | 229289_at | AL517395 | NM_175063 | 284361 | -1.48 | 2.00E-03 |
| | | | NM_206538 | | | |
| PDE4DIP | 213388_at | H15535 | NM_00100281 0 | 9659 | -1.47 | 1.48E-03 |
| | | | NM_00100281 1 | | | |
| | | | NM_00100281 2 | | | |
| | | | NM_014644 | | | |
| | | | NM_022359 | | | |
| LOC441422 | 236453_at | AW243154 | XR_000264 | 441422 | -1.47 | 2.36E-05 |
| AA976778 | 230679_at | AA976778 | --- | --- | -1.46 | 1.46E-05 |
| FBI4 | 241401_at | BG496631 | NM_205857 | 404201 | -1.46 | 1.53E-03 |
| C14orf149 | 227699_at | BF511003 | NM_144581 | 112849 | -1.45 | 1.61E-03 |
| FBI4 | 1562953_s_at | BC019264 | NM_205857 | 404201 | -1.45 | 5.64E-03 |
| AI758408 | 227235_at | AI758408 | --- | --- | -1.45 | 1.51E-03 |
| TMEM5 | 204808_s_at | NM_014254 | NM_014254 | 10329 | -1.44 | 3.94E-03 |
| BF111884 | 230491_at | BF111884 | --- | --- | -1.44 | 3.82E-04 |
| COL27A1 | 225293_at | AK021957 | NM_032888 | 85301 | -1.44 | 6.78E-03 |
| AA705933 | 239866_at | AA705933 | --- | --- | -1.44 | 5.37E-03 |
| PTN | 209466_x_at | M57399 | NM_002825 | 5764 | -1.43 | 9.60E-04 |
| COL6A1 | 213428_s_at | AA292373 | NM_001848 | 1291 | -1.43 | 7.05E-03 |
| DKFZp313N062 | 241666_at | BG493222 | NM_173826 | 285343 | -1.43 | 8.80E-04 |
| BF241692 | 238491_at | BF241692 | --- | --- | -1.43 | 8.44E-04 |
| NR1H3 | 203920_at | NM_005693 | NM_005693 | 10062 | -1.42 | 8.44E-03 |
| DKFZP434I071 | 231954_at | AL137273 | --- | 54553 | -1.42 | 2.11E-03 |
| AI927692 | 228528_at | AI927692 | --- | --- | -1.41 | 9.58E-04 |
| SH3GL2 | 205751_at | NM_003026 | NM_003026 | 6456 | -1.41 | 1.80E-05 |
| SNF1LK | 208078_s_at | NM_030751 | NM_173354 | 150094 | -1.40 | 9.31E-04 |
| LGALS3BP | 200923_at | NM_005567 | NM_005567 | 3959 | -1.40 | 1.13E-05 |
| AI796536 | 229810_at | AI796536 | --- | --- | -1.40 | 2.02E-03 |
| DUSP22 | 226440_at | BE222746 | NM_020185 | 56940 | -1.40 | 2.32E-03 |
| DUSP16 | 224336_s_at | AB052156 | NM_030640 | 80824 | -1.39 | 1.91E-03 |
| BF591692 | 231532_at | BF591692 | --- | --- | -1.39 | 3.15E-04 |
| CAMK2D | 228555_at | AA029441 | NM_001221 | 817 | -1.39 | 2.14E-04 |
| | | | NM_172115 | | | |
| | | | NM_172127 | | | |
| | | | NM_172128 | | | |
| HRMT1L1 | 202098_s_at | NM_001535 | NM_001535 | 3275 | -1.39 | 4.67E-06 |
| | | | NM_206962 | | | |
| DISP1 | 228184_at | AK023679 | NM_032890 | 84976 | -1.39 | 2.54E-05 |
| MAP3K5 | 203836_s_at | D84476 | NM_005923 | 4217 | -1.39 | 1.24E-03 |
| H46176 | 237083_at | H46176 | --- | --- | -1.38 | 8.44E-03 |
| RPL37 | 224767 _ at | AL044126 | NM_000997 | 6167 | -1.37 | 1.60E-03 |
| TIMP1 | 201666_at | NM_003254 | NM_003254 | 7076 | -1.37 | 7.35E-03 |
| PTGES | 207388_s_at | NM_004878 | NM_004878 | 9536 | -1.37 | 6.78E-03 |
| | | | NM_198797 | | | |
| SCRG1 | 205475_at | NM_007281 | NM_007281 | 11341 | -1.37 | 1.72E-03 |
| LOC153682 | 232794_at | AL137383 | --- | 153682 | -1.36 | 2.78E-04 |
| LOC146174 | 230721_at | BF436957 | NM_173501 | 146174 | -1.36 | 1.19E-04 |
| DUSP22 | 218845_at | NM_020185 | NM_020185 | 56940 | -1.36 | 3.43E-06 |
| PH-4 | 222125_s_at | BC000580 | NM_017732 | 54681 | -1.35 | 5.67E-04 |
| | | | NM_177938 | | | |
| | | | NM_177939 | | | |
| DJ462O23.2 | 225876_at | T84558 | NM_020448 | 57185 | -1.35 | 1.26E-03 |
| CDKN2A | 209644_x_at | U38945 | NM_000077 | 1029 | -1.35 | 1.67E-04 |
| | | | NM_058195 | | | |
| | | | NM_058197 | | | |
| DNER | 226281_at | BF059512 | NM_139072 | 92737 | -1.35 | 1.29E-04 |
| ANKH | 220076_at | NM_019847 | NM_054027 | 56172 | -1.34 | 4.18E-03 |
| TBC1D10 | 233528_s_at | AC004997 | NM_031937 | 83874 | -1.34 | 1.10E-03 |
| C1orf21 | 221272_s_at | NM_030806 | NM_030806 | 81563 | -1.34 | 5.15E-04 |
| NMB | 205204_at | NM_021077 | NM_021077 | 4828 | -1.33 | 1.42E-04 |
| | | | NM_205858 | | | |
| SLC16A1 | 1560579_s_at | BC023568 | NM_003051 | 6566 | -1.33 | 8.18E-03 |
| BE672499 | 230962_at | BE672499 | --- | --- | -1.32 | 7.98E-03 |
| C21orf57 | 227421_at | BF026326 | NM_00100611 4 | 54059 | -1.31 | 2.23E-06 |
| | | | NM_058181 | | | |
| C1orf21 | 223127_s_at | AW003850 | NM_030806 | 81563 | -1.31 | 2.69E-03 |
| LOC91526 | 228471_at | AA744636 | NM_153697 | 91526 | -1.31 | 6.93E-03 |
| FHL2 | 202949_s_at | NM_001450 | NM_001450 | 2274 | -1.31 | 1.03E-03 |
| | | | NM_201555 | | | |
| | | | NM_201556 | | | |
| | | | NM_201557 | | | |
| NM_024599 | 219202_at | NM_024599 | --- | --- | -1.31 | 6.35E-03 |
| C10orf57 | 218174_s_at | NM_025125 | NM_025125 | 80195 | -1.31 | 7.96E-03 |
| LOC196394 | 226258_at | BG031897 | NM_207337 | 196394 | -1.30 | 8.09E-04 |
| AK001829 | 232594_at | AK001829 | XM_498704 | 440498 | -1.30 | 2.12E-03 |
| BE551142 | 241091_at | BE551142 | XM_497668 | 388620 | -1.29 | 2.90E-03 |
| ZFP28 | 234300_s_at | AF226995 | NM_020828 | 140612 | -1.29 | 6.22E-04 |
| BE046461 | 215303_at | BE046461 | --- | --- | -1.29 | 9.65E-03 |
| PCDHAC2 | 224539_s_at | AF152474 | | 56134 | -1.28 | 4.06E-03 |
| | | | NM_018899 | | | |
| | | | NM_031883 | | | |
| RPL37 | 224763_at | BF724210 | NM_000997 | 6167 | -1.28 | 6.11E-03 |
| AI631072 | 228638_at | AI631072 | --- | --- | -1.28 | 1.12E-03 |
| MGC39821 | 1555363_s_at | BC034236 | XM_498465 | 284440 | -1.28 | 1.45E-03 |
| | | | XM_499595 | | | |
| ATP6V1E2 | 1552286_at | NM_080653 | NM_080653 | 90423 | -1.28 | 2.19E-03 |
| DTX3L | 225415_at | AA577672 | NM_138287 | 151636 | -1.26 | 6.35E-03 |
| ZNF258 | 213698_at | AI805560 | NM_007167 | 9204 | -1.26 | 4.04E-03 |
| | | | NM_145310 | | | |
| RHOU | 223168_at | AL096776 | NM_021205 | 58480 | -1.26 | 2.56E-04 |
| RFX2 | 226872_at | AK024288 | NM_000635 | 5990 | -1.25 | 5.59E-03 |
| | | | NM_134433 | | | |
| LOC402524 | 242206_at | AW590588 | XM_379850 | 402524 | -1.25 | 2.69E-04 |
| NEFH | 33767_at | X15306 | NM_021076 | 4744 | -1.25 | 4.29E-03 |
| HRMT1L1 | 228722_at | AI928367 | NM_001535 | 3275 | -1.25 | 1.86E-03 |
| | | | NM_206962 | | | |
| AI963605 | 230269_at | AI963605 | --- | --- | -1.24 | 2.72E-03 |
| NDUFV3 | 226616_s_at | NM_021075 | NM_00100150 3 | 4731 | -1.24 | 6.22E-04 |
| | | | NM_021075 | | | |
| NOTCH4 | 205247_at | AI743713 | NM_004557 | 4855 | -1.24 | 2.38E-03 |
| LOC401321 | 1560371_at | BI823265 | XM_379483 | 401321 | -1.24 | 3.08E-03 |
| | | | XM_380099 | | | |
| SMPD1 | 209420_s_at | M59916 | NM_000543 | 6609 | -1.24 | 2.86E-03 |
| | | | NM_00100759 3 | | | |
| RAB6B | 225259_at | AI805050 | NM_016577 | 51560 | -1.24 | 5.78E-03 |
| BRUNOL6 | 227775_at | BE467313 | NM_052840 | 60677 | -1.24 | 2.38E-04 |
| MCTP1 | 235740_at | BG250585 | NM_00100279 6 | 79772 | -1.24 | 3.07E-04 |
| | | | NM_024717 | | | |
| PHLDB3 | 1557948_at | BC007947 | NM_198850 | 284345 | -1.23 | 5.46E-04 |
| PLRG1 | 227246_at | BF438014 | NM_002669 | 5356 | -1.23 | 4.56E-03 |
| C9orf19 | 225602_at | H92988 | NM_022343 | 152007 | -1.23 | 4.21E-03 |
| MGC16037 | 229050_s_at | AL533103 | --- | 84973 | -1.23 | 9.30E-03 |
| PLP2 | 201136_at | NM_002668 | NM_002668 | 5355 | -1.21 | 2.30E-03 |
| LOC91526 | 226641_at | AU157224 | NM_153697 | 91526 | -1.21 | 9.94E-03 |
| ZNF404 | 239043_at | AA084273 | XM_292765 | 342908 | -1.21 | 8.85E-04 |
| CNN1 | 203951_at | NM_001299 | NM_001299 | 1264 | -1.20 | 5.29E-03 |
| AI862542 | 235696_at | AI862542 | --- | --- | -1.19 | 2.30E-04 |
| FHL3 | 218818_at | NM_004468 | NM_004468 | 2275 | -1.19 | 5.70E-03 |
| LOC90246 | 233835_at | AK023635 | --- | 90246 | -1.19 | 1.62E-04 |
| ARMCX5 | 1555902_at | BC022066 | NM_022838 | 64860 | -1.19 | 1.49E-04 |
| LOC388227 | 229107_at | AW629461 | XM_373670 | 388227 | -1.19 | 5.59E-03 |
| C6orf52 | 236504_x_at | BE670432 | XM_371798 | 347744 | -1.19 | 9.06E-03 |
| ZNF606 | 219635_at | NM_025027 | NM_025027 | 80095 | -1.19 | 2.38E-03 |
| C10orf39 | 233076_at | AL137551 | NM_194303 | 282973 | -1.19 | 6.97E-04 |
| FVT1 | 1558279_a_at | BF666293 | NM_002035 | 2531 | -1.19 | 3.09E-03 |
| TAF12 | 209463_s_at | D50544 | NM_005644 | 6883 | -1.18 | 3.00E-04 |
| AI929792 | 236081_at | AI929792 | --- | --- | -1.18 | 2.41E-05 |
| PDCD2 | 228420_at | AW590850 | NM_002598 | 5134 | -1.18 | 9.07E-03 |
| | | | NM_144781 | | | |
| ETFDH | 33494_at | S69232 | NM_004453 | 2110 | -1.18 | 6.70E-03 |
| CD302 | 203799_at | NM_014880 | NM_014880 | 9936 | -1.17 | 9.28E-03 |
| N30152 | 229850_at | N30152 | --- | --- | -1.17 | 5.16E-03 |
| MGC11332 | 213403_at | BF223370 | NM_032718 | 84804 | -1.17 | 1.04E-03 |
| SPIN3 | 1554099_a_at | BC032490 | NM_001010862 | 169981 | -1.17 | 1.85E-05 |
| PHKA1 | 229876_at | BE503584 | NM_002637 | 5255 | -1.17 | 2.72E-04 |
| PCNXL2 | 1554256_a_at | BC008300 | NM_014801 | 80003 | -1.17 | 8.67E-05 |
| | | | NM_024938 | | | |
| LOC284409 | 239995_at | AI692774 | XM_211447 | 284409 | -1.17 | 1.43E-04 |
| AV704017 | 222376_at | AV704017 | --- | --- | -1.16 | 4.90E-03 |
| AI767751 | 228084_at | AI767751 | --- | --- | -1.16 | 6.90E-05 |
| FLJ30294 | 238578_at | BF574691 | NM_144632 | 130827 | -1.16 | 2.44E-06 |
| BTN3A1 | 209770_at | U90552 | NM_007048 | 11119 | -1.16 | 4.37E-03 |
| | | | NM_194441 | | | |
| ZC3HDC6 | 241372_at | R34135 | NM_198581 | 376940 | -1.16 | 4.27E-05 |
| LOC285550 | 236321_at | AW439843 | XM_209656 | 285550 | -1.16 | 6.99E-03 |
| MGC11332 | 213393_at | AI767210 | NM_032718 | 84804 | -1.15 | 7.85E-03 |
| CYP2U1 | 226402_at | AK026498 | NM_183075 | 113612 | -1.15 | 2.05E-03 |
| OPTN | 202074_s_at | NM_021980 | NM_001008211 | 10133 | -1.15 | 7.05E-03 |
| | | | NM_001008212 | | | |
| | | | NM_001008213 | | | |
| | | | NM_021980 | | | |
| D4S234E | 209569_x_at | NM_014392 | NM_014392 | 27065 | -1.15 | 1.26E-03 |
| GUCY1A3 | 221942_s_at | AI719730 | NM_000856 | 2982 | -1.15 | 4.01E-04 |
| C18orf18 | 229829_at | AA429735 | --- | 147525 | -1.14 | 7.95E-03 |
| DUSP16 | 224832_at | AB051487 | NM_030640 | 80824 | -1.14 | 2.08E-04 |
| C21orf25 | 212875_s_at | AP001745 | NM_199050 | 25966 | -1.13 | 2.32E-03 |
| DKFZP434B172 | 232124_at | AL117530 | XM_046264 | 26172 | -1.13 | 1.47E-03 |
| AKT2 | 236664_at | AA448167 | NM_001626 | 208 | -1.13 | 5.51E-03 |
| FGF7 | 1554741_s_at | AF523265 | NM_002009 | 2252 | -1.13 | 2.06E-03 |
| FLJ30435 | | | NM_174950 | 387628 | | |
| SPOCK2 | 202524_s_at | NM_014767 | NM_014767 | 9806 | -1.12 | 8.32E-03 |
| ULK2 | 204063_s_at | NM_014683 | NM_014683 | 9706 | -1.12 | 5.36E-03 |
| DCTD | 201571_s_at | AI656493 | NM_001921 | 1635 | -1.12 | 9.86E-05 |
| BACE1 | 217904_s_at | NM_012104 | NM_012104 | 23621 | -1.12 | 1.17E-03 |
| | | | NM_138971 | | | |
| | | | NM_138972 | | | |
| | | | NM_138973 | | | |
| BC006438 | 224519_at | BC006438 | XR_000264 | 441422 | -1.12 | 9.10E-04 |
| AW192692 | 227917_at | AW192692 | --- | --- | -1.12 | 3.83E-06 |
| AGA | 216064_s_at | W27131 | NM_000027 | 175 | -1.12 | 8.70E-03 |
| AI887898 | 228423_at | AI887898 | XM_374094 | 389237 | -1.12 | 4.40E-03 |
| AV714268 | 242323_at | AV714268 | --- | --- | -1.11 | 2.68E-04 |
| CXorf50 | 242292_at | H12084 | NM_152693 | 203429 | -1.11 | 4.02E-04 |
| BC040287 | 1558605_at | BC040287 | --- | --- | -1.11 | 7.41E-03 |
| RSHL2 | 223713_at | AF353618 | NM_031924 | 83861 | -1.11 | 4.32E-05 |
| AI042344 | 240121_x_at | AI042344 | --- | --- | -1.11 | 1.04E-03 |
| GTDC1 | 239390_at | BE327650 | NM_001006636 | 79712 | -1.11 | 5.93E-03 |
| | | | NM_024659 | | | |
| MGC14425 | 1552665_at | NM_032903 | --- | 84989 | -1.11 | 1.79E-03 |
| ERBB2 | 216836_s_at | X03363 | NM_001005862 | 2064 | -1.11 | 1.73E-05 |
| | | | NM_004448 | | | |
| C21orf51 | 228239_at | AA148789 | NM_058182 | 54065 | -1.11 | 7.04E-06 |
| FLJ35220 | 238401_at | AI311040 | NM_173627 | 284131 | -1.11 | 2.38E-08 |
| SAMD4 | 212845_at | AB028976 | NM_015589 | 23034 | -1.10 | 5.48E-03 |
| CLCN3 | 201734_at | AI760629 | NM_001829 | 1182 | -1.10 | 9.57E-03 |
| | | | NM_173872 | | | |
| FLJ31265 | 238515_at | BF687577 | NM_152395 | 131870 | -1.10 | 7.24E-04 |
| ITGB1BP1 | 203337_x_at | NM_004763 | NM_004763 | 9270 | -1.10 | 2.94E-03 |
| | | | NM_022334 | | | |
| AW467480 | 236202_at | AW467480 | --- | --- | -1.09 | 3.18E-03 |
| LIFR | 225575_at | AI680541 | NM_002310 | 3977 | -1.09 | 6.39E-03 |
| PCDHB16 | 232099_at | AB046841 | | 57717 | -1.09 | 5.69E-03 |
| | | | NM_020957 | | | |
| AI304870 | 236574_at | AI304870 | --- | --- | -1.09 | 4.77E-03 |
| LOC157378 | 241342_at | BG288115 | NM_194291 | 157378 | -1.09 | 7.42E-03 |
| M19720 | 214058_at | M19720 | --- | --- | -1.09 | 6.67E-03 |
| LOC90693 | 228087_at | AK026684 | NM_138771 | 90693 | -1.08 | 6.62E-06 |
| C10orf104 | 229145_at | AA541762 | NM_173473 | 119504 | -1.08 | 3.73E-03 |
| KIAA1324 | 221874_at | AB037745 | NM_020775 | 57535 | -1.07 | 1.16E-04 |
| FGF19 | 223761_at | AF110400 | NM_005117 | 9965 | -1.07 | 8.40E-03 |
| AI768374 | 228661_s_at | AI768374 | --- | --- | -1.07 | 9.53E-04 |
| PLRG1 | 225194_at | NM_002669 | NM_002669 | 5356 | -1.07 | 8.76E-05 |
| AI821995 | 240201_at | AI821995 | --- | --- | -1.07 | 5.19E-03 |
| LOC400924 | 226956_at | AW025389 | XM_376003 | 400924 | -1.07 | 6.57E-04 |
| SLC22A5 | 205074_at | NM_003060 | NM_003060 | 6584 | -1.07 | 4.71E-03 |
| DALRD3 | 221934_s_at | BF941492 | NM_001009996 | 55152 | -1.07 | 8.88E-03 |
| | | | NM_018114 | | | |
| AL122040 | 232429_at | AL122040 | --- | --- | -1.07 | 1.70E-06 |
| FLJ10490 | 220151_at | NM_018111 | NM_018111 | 55150 | -1.07 | 4.45E-04 |
| ABTB1 | 229164_s_at | AI337369 | NM_032548 | 80325 | -1.07 | 5.29E-03 |
| | | | NM_172027 | | | |
| | | | NM_172028 | | | |
| AI569997 | 242610_x_at | AI569997 | --- | --- | -1.06 | 7.58E-03 |
| ZNF285 | 236328_at | AW513227 | NM_152354 | 26974 | -1.06 | 2.76E-04 |
| FSTL1 | 208782_at | BC000055 | NM_007085 | 11167 | -1.06 | 9.38E-03 |
| TAF1B | 235673_at | AI436217 | NM_005680 | 9014 | -1.06 | 2.05E-03 |
| BF002625 | 229530_at | BF002625 | --- | --- | -1.06 | 6.62E-04 |
| PEX6 | 204545_at | NM_000287 | NM_000287 | 5190 | -1.06 | 4.66E-05 |
| ZNF258 | 219924_s_at | NM_007167 | NM_007167 | 9204 | -1.06 | 5.59E-03 |
| | | | NM_145310 | | | |
| BE467383 | 228932_at | BE467383 | XM_497668 | 388620 | -1.06 | 3.42E-03 |
| TIGD4 | 232998_at | AK024235 | NM_145720 | 201798 | -1.06 | 7.64E-04 |
| HCG8 | 215985_at | X92110 | --- | 80869 | -1.05 | 5.10E-03 |
| PTPRD | 214043_at | BF062299 | NM_002839 | 5789 | -1.05 | 2.37E-03 |
| | | | NM_130391 | | | |
| | | | NM_130392 | | | |
| | | | NM_130393 | | | |
| MAN2A2 | 219999_at | NM_018621 | NM_006122 | 4122 | -1.05 | 3.41E-03 |
| CHC1 | 206499_s_at | NM_001269 | NM_001269 | 1104 | -1.05 | 3.22E-03 |
| DUSP16 | 1558739_at | R30807 | NM_030640 | 80824 | -1.04 | 6.86E-04 |
| PLEKHA2 | 238013_at | BF347859 | XM_496973 | 59339 | -1.04 | 7.63E-03 |
| RODH | 37512_at | U89281 | NM_003725 | 8630 | -1.04 | 2.71E-03 |
| TMEM42 | 226361_at | BF056095 | NM_144638 | 131616 | -1.04 | 2.56E-03 |
| MGC4655 | 226058_at | AI651445 | NM_033309 | 84752 | -1.04 | 4.77E-04 |
| LMNA | 203411_s_at | NM_005572 | NM_005572 | 4000 | -1.04 | 6.43E-03 |
| | | | NM_170707 | | | |
| | | | NM_170708 | | | |
| AL552727 | 244080_at | AL552727 | --- | --- | -1.04 | 1.69E-03 |
| ALLC | 220365_at | NM_018436 | NM_018436 | 55821 | -1.04 | 3.61E-03 |
| | | | NM_199232 | | | |
| KIAA0543 | 213444_at | AB011115 | XM_376720 | 23145 | -1.04 | 3.24E-07 |
| | | | XM_379967 | | | |
| TRIM61 | 238990_x_at | AI763262 | XM_373038 | 391712 | -1.04 | 3.91E-03 |
| AA156605 | 226798_at | AA156605 | --- | --- | -1.04 | 1.65E-03 |
| STX12 | 212111_at | AA628051 | NM_177424 | 23673 | -1.03 | 9.08E-07 |
| PHF17 | 225816_at | AW138134 | NM_024900 | 79960 | -1.03 | 9.73E-03 |
| | | | NM_199320 | | | |
| AL042817 | 225553_at | AL042817 | --- | --- | -1.03 | 2.32E-04 |
| HEL308 | 228736_at | AW084661 | NM_133636 | 113510 | -1.03 | 9.31E-04 |
| LOC440388 | 228855_at | AI927964 | XM_496170 | 440388 | -1.03 | 2.15E-03 |
| ANKRA2 | 218769_s_at | NM_023039 | NM_023039 | 57763 | -1.03 | 3.79E-04 |
| SH3KBP1 | 223082_at | AF230904 | NM_031892 | 30011 | -1.03 | 5.39E-04 |
| MAP7 | 202890_at | AW242297 | NM_003980 | 9053 | -1.03 | 9.43E-03 |
| AI263044 | 214376_at | AI263044 | --- | --- | -1.03 | 4.37E-03 |
| D4S234E | 209570_s_at | BC001745 | NM_014392 | 27065 | -1.03 | 5.33E-03 |
| BF510563 | 228561_at | BF510563 | --- | --- | -1.03 | 7.28E-03 |
| LOC157378 | 238045_at | BG167675 | NM_194291 | 157378 | -1.02 | 9.58E-03 |
| BF510762 | 230262_at | BF510762 | --- | --- | -1.02 | 3.17E-03 |
| SLC2A11 | 1558540_s_at | AK055523 | NM_030807 | 66035 | -1.01 | 5.49E-03 |
| INPP5A | 1554757_a_at | AF273055 | NM_005539 | 3632 | -1.01 | 3.78E-03 |
| CARD10 | 210026_s_at | AY028896 | NM_014550 | 29775 | -1.01 | 4.13E-03 |
| EFNB3 | 205031_at | NM_001406 | NM_001406 | 1949 | -1.01 | 2.94E-03 |
| SVH | 1554480_a_at | AY150851 | NM_031905 | 83787 | -1.01 | 4.33E-05 |
| PP1057 | 220968_s_at | NM_031285 | --- | 83441 | -1.01 | 3.02E-04 |
| LOC401320 | 238540_at | AA933883 | XM_379482 | 401320 | -1.00 | 1.29E-03 |
| | | | XM_380098 | | | |
| GNB5 | 211871_x_at | AF300650 | NM_006578 | 10681 | -1.00 | 2.54E-03 |
| | | | NM_016194 | | | |
| BE549742 | 230724_s_at | BE549742 | --- | --- | -1.00 | 3.40E-03 |
| MGC34648 | 228604_at | AI805069 | NM_152660 | 199870 | -1.00 | 2.70E-03 |
| AL390170 | 213904_at | AL390170 | --- | --- | -1.00 | 4.30E-03 |
| LOC400713 | 232315_at | AU149712 | XR_000217 | 400713 | -1.00 | 1.91E-03 |
| DKFZP434H132 | 204494_s_at | AW516789 | NM_015492 | 56905 | -1.00 | 2.50E-04 |
| DBT | 231919_at | AK024946 | NM_001918 | 1629 | -1.00 | 3.13E-04 |
| AI459157 | 225066_at | AI459157 | --- | --- | -1.00 | 1.73E-03 |
| WASL | 227010_at | BF438330 | NM_003941 | 8976 | -1.00 | 3.51E-04 |
| SH3KBP1 | 1554168_a_at | AF542051 | NM_031892 | 30011 | -0.99 | 9.41E-04 |
| LOC339803 | 227941_at | AW327931 | --- | 339803 | -0.99 | 7.41E-04 |
| NM_017923 | 219574_at | NM_017923 | --- | --- | -0.99 | 1.05E-04 |
| HMGCLL1 | 232305_at | AF131827 | NM_019036 | 54511 | -0.99 | 7.25E-06 |
| MGC29898 | 235259_at | AA398590 | NM_145048 | 133015 | -0.99 | 2.85E-03 |
| LIN7B | 219760_at | NM_022165 | NM_022165 | 64130 | -0.99 | 6.61E-06 |
| AI161338 | 65472_at | AI161338 | XM_371506 | 388969 | -0.99 | 7.88E-03 |
| PIGH | 209625_at | BC004100 | NM_004569 | 5283 | -0.99 | 6.18E-04 |
| RRN3 | 216902_s_at | AF001549 | NM_018427 | 54700 | -0.98 | 1.48E-05 |
| BE646451 | 235893_at | BE646451 | --- | --- | -0.98 | 1.01E-03 |
| KIAA1223 | 225735_at | AL110131 | XM_048747 | 57182 | -0.98 | 9.14E-04 |
| SLC18A2 | 1553328_a_at | NM_003054 | NM_003054 | 6571 | -0.98 | 5.03E-03 |
| C19orf4 | 219005_at | NM_012109 | NM_012109 | 25789 | -0.98 | 2.75E-03 |
| D4S234E | 213533_at | M98528 | NM_014392 | 27065 | -0.98 | 2.97E-04 |
| CAPN2 | 208683_at | M23254 | NM_001748 | 824 | -0.98 | 7.76E-03 |
| TMEM23 | 212989_at | AI377497 | NM_147156 | 259230 | -0.98 | 5.42E-03 |
| NEFH | 204412_s_at | NM_021076 | NM_021076 | 4744 | -0.98 | 9.35E-03 |
| ANXA5 | 200782_at | NM_001154 | NM_001154 | 308 | -0.98 | 5.17E-03 |
| AI955713 | 227193_at | AI955713 | --- | --- | -0.97 | 5.36E-08 |
| LOC284422 | 231982_at | AF161441 | XM_209196 | 284422 | -0.97 | 7.92E-03 |
| KCTD18 | 226493_at | AI627249 | NM_152387 | 130535 | -0.97 | 3.04E-05 |
| LOC90024 | 228832_at | AI884670 | XM_028217 | 90024 | -0.97 | 7.03E-03 |
| CA442342 | 1556097_at | CA442342 | --- | --- | -0.97 | 3.17E-03 |
| LOC90639 | 235533_at | AW021673 | --- | 90639 | -0.97 | 4.53E-03 |
| HDAC4 | 228813_at | AW206037 | NM_006037 | 9759 | -0.96 | 3.30E-03 |
| BC040901 | 1559535_s_at | BC040901 | --- | --- | -0.96 | 6.19E-03 |
| BBS7 | 235007_at | AI683802 | NM_018190 | 55212 | -0.96 | 1.86E-03 |
| | | | NM_176824 | | | |
| MGC48625 | 1569039_s_at | BC029855 | NM_182609 | 342926 | -0.96 | 7.88E-05 |
| MBNL1 | 201151_s_at | BF512200 | NM_021038 | 4154 | -0.96 | 4.33E-03 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| CIB1 | 201953_at | NM_006384 | NM_006384 | 10519 | -0.96 | 2.33E-04 |
| RPP21 | 218836_at | NM_024839 | NM_024839 | 79897 | -0.96 | 2.49E-03 |
| CHC1 | 215747_s_at | X06130 | NM_001269 | 1104 | -0.96 | 4.49E-03 |
| ACSL1 | 201963_at | NM_021122 | NM_001995 | 2180 | -0.95 | 5.71E-04 |
| RHOU | 223169_s_at | AB051826 | NM_021205 | 58480 | -0.95 | 1.29E-03 |
| KIAA0773 | 205368_at | NM_014690 | NM_014690 | 9715 | -0.95 | 1.39E-03 |
| PDPK1 | 32029_at | AC005591 | NM_002613 | 5170 | -0.95 | 4.56E-04 |
| | | | NM_031268 | | | |
| GNG8 | 234284_at | AF188179 | NM_033258 | 94235 | -0.95 | 5.33E-04 |
| C14orf133 | 233140_s_at | AF111168 | NM_022067 | 63894 | -0.95 | 4.26E-04 |
| ITR | 231871_at | AU160685 | NM_180989 | 160897 | -0.95 | 5.11E-03 |
| BC015852 | 1560570_a_at | BC015852 | --- | --- | -0.95 | 6.20E-03 |
| SNF1LK2 | 223430_at | AL136764 | NM_015191 | 23235 | -0.95 | 2.99E-03 |
| CKLFSF1 | 235286_at | BG533580 | NM_052999 | 113540 | -0.95 | 5.78E-03 |
| | | | NM_181268 | | | |
| | | | NM_181269 | | | |
| | | | NM_181270 | | | |
| | | | NM_181271 | | | |
| | | | NM_181272 | | | |
| | | | NM_181283 | | | |
| | | | NM_181285 | | | |
| | | | NM_181286 | | | |
| | | | NM_181287 | | | |
| | | | NM_181288 | | | |
| | | | NM_181289 | | | |
| | | | NM_181290 | | | |
| | | | NM_181292 | | | |
| | | | NM_181293 | | | |
| | | | NM_181294 | | | |
| | | | NM_181295 | | | |
| | | | NM_181296 | | | |
| | | | NM_181297 | | | |
| | | | NM_181298 | | | |
| | | | NM_181299 | | | |
| | | | NM_181300 | | | |
| | | | NM_181301 | | | |
| BTN3A3 | 204821_at | NM_006994 | NM_006994 | 10384 | -0.95 | 7.79E-05 |
| | | | NM_197974 | | | |
| PLEKHC1 | 209210_s_at | Z24725 | NM_006832 | 10979 | -0.94 | 2.90E-03 |
| RNF41 | 201962_s_at | NM_005785 | NM_005785 | 10193 | -0.94 | 3.16E-03 |
| | | | NM_194358 | | | |
| | | | NM_194359 | | | |
| NAP1L3 | 204749_at | NM_004538 | NM_004538 | 4675 | -0.94 | 2.43E-03 |
| ZNF251 | 226754_at | W93231 | XM_291262 | 90987 | -0.94 | 1.04E-03 |
| PLEKHA2 | 217677_at | BF542055 | XM_496973 | 59339 | -0.94 | 7.55E-03 |
| SIDT2 | 56256_at | AA150165 | NM_015996 | 51092 | -0.94 | 3.96E-04 |
| STX12 | 212112_s_at | AI816243 | NM_177424 | 23673 | -0.94 | 7.35E-05 |
| STX17 | 228091_at | AI800609 | NM_017919 | 55014 | -0.94 | 6.04E-03 |
| C2orf17 | 221983_at | AL040896 | NM_024293 | 79137 | -0.94 | 4.85E-03 |
| ZFP28 | 234937_x_at | AC007228 | NM_020828 | 140612 | -0.94 | 3.30E-03 |
| HLA-G | 210514_x_at | AF226990 | NM_002127 | 3135 | -0.94 | 1.71E-04 |
| MGC14801 | 224443_at | BC005997 | NM_032705 | 84791 | -0.93 | 9.68E-03 |
| N79601 | 242981_at | N79601 | --- | --- | -0.93 | 4.16E-03 |
| LOC284926 | 1556064_at | BQ575161 | --- | 284926 | -0.93 | 5.64E-03 |
| WDR9 | 225446_at | AI638279 | NM_00100724 6 | 54014 | -0.93 | 3.04E-04 |
| | | | NM_018963 | | | |
| | | | NM_033656 | | | |
| AI652546 | 228853_at | AI652546 | --- | --- | -0.93 | 4.31E-03 |
| EIF4E3 | 238461_at | AA228031 | --- | 317649 | -0.92 | 5.07E-03 |
| AU151944 | 236916_at | AU151944 | --- | --- | -0.92 | 1.50E-03 |
| ZNF610 | 235953_at | AA776810 | NM_173530 | 162963 | -0.92 | 3.79E-04 |
| RGS14 | 38290_at | AF037195 | NM_006480 | 10636 | -0.92 | 1.22E-03 |
| WDR24 | 223293_at | AL136863 | NM_032259 | 84219 | -0.92 | 5.54E-04 |
| T87730 | 242500_at | T87730 | --- | --- | -0.92 | 7.27E-03 |
| AW615274 | 229423_at | AW615274 | --- | --- | -0.92 | 8.89E-03 |
| SIRT5 | 219185_at | NM_012241 | NM_012241 | 23408 | -0.91 | 1.64E-03 |
| | | | NM_031244 | | | |
| MGC54289 | 225230_at | A1735261 | NM_178454 | 128338 | -0.91 | 9.98E-03 |
| LMNA | 212089_at | M13452 | NM_005572 | 4000 | -0.91 | 7.00E-03 |
| | | | NM_170707 | | | |
| | | | NM_170708 | | | |
| NINJ1 | 203045_at | NM 004148 | NM_004148 | 4814 | -0.91 | 3.51E-04 |
| C9orf127 | 229729_at | BF507870 | NM_016446 | 51754 | -0.91 | 1.20E-04 |
| LAF4 | 227198_at | AW085505 | NM_002285 | 3899 | -0.91 | 4.25E-05 |
| ADPRHL2 | 223097_at | AF212236 | NM_017825 | 54936 | -0.91 | 1.91E-04 |
| N21364 | 214202_at | N21364 | --- | --- | -0.90 | 6.59E-06 |
| LOC388727 | 231233_at | AI382195 | XM_373881 | 388727 | -0.90 | 1.97E-03 |
| ALU Sequence, | FFX-hum_alu_a | AFFX-hum_alu | NM_000021 | --- | -0.90 | 2.52E-03 |
| | | | | | | |
| | | | NM_000136 | | | |
| | | | NM_000141 | | | |
| | | | NM_000144 | | | |
| | | | NM_000202 | | | |
| | | | NM_00022 | | | |
| | | | NM_000230 | | | |
| | | | NM_000294 | | | |
| | | | NM_000297 | | | |
| | | | NM_000314 | | | |
| | | | NM_000356 | | | |
| | | | NM_000364 | | | |
| | | | NM_000382 | | | |
| | | | NM_000441 | | | |
| | | | NM_000484 | | | |
| | | | NM_000527 | | | |
| | | | NM_000554 | | | |
| ACYP2 | 206833_s_at | NM_001108 | NM_138448 | 98 | -0.90 | 4.12E-03 |
| EXOC7 | 212026_s_at | BE646386 | NM_015219 | 23265 | -0.90 | 1.03E-03 |
| RPUSD3 | 225743_at | AI125996 | NM_173659 | 285367 | -0.90 | 3.62E-03 |
| R15431 | 236440_at | R15431 | --- | --- | -0.90 | 3.46E-05 |
| AA194266 | 238728_at | AA194266 | --- | --- | -0.90 | 8.94E-03 |
| GNB5 | 207124_s_at | NM_006578 | NM_006578 | 10681 | -0.90 | 2.52E-04 |
| | | | NM_016194 | | | |
| TEX264 | 233350_s_at | AU158474 | NM_015926 | 51368 | -0.89 | 3.96E-04 |
| TRIM32 | 1557315_a_at | AL134420 | NM_012210 | 22954 | -0.89 | 4.37E-03 |
| BF342524 | 235074_at | BF342524 | --- | --- | -0.89 | 2.65E-05 |
| HADHA | 236120_at | AW955973 | NM_000182 | 3030 | -0.89 | 8.56E-03 |
| C21orf119 | 227424_x_at | AI800837 | NM_032910 | 84996 | -0.88 | 6.71E-03 |
| CLN5 | 204085_s_at | NM_006493 | NM_006493 | 1203 | -0.88 | 6.71E-03 |
| SAV1 | 218276_s_at | NM_021818 | NM_021818 | 60485 | -0.88 | 4.58E-03 |
| HDHD3 | 221256_s_at | NM_031219 | NM_031219 | 81932 | -0.88 | 4.72E-03 |
| EMID1 | 213779_at | AL031186 | NM_133455 | 129080 | -0.88 | 4.61E-05 |
| GAL3ST3 | 1553257_at | AY026481 | NM_033036 | 89792 | -0.88 | 5.62E-03 |
| USP44 | 224048_at | AL136825 | NM_032147 | 84101 | -0.88 | 1.11E-03 |
| ACSL1 | 207275_s_at | NM_001995 | NM_001995 | 2180 | -0.87 | 9.24E-03 |
| AK024681 | 231972_at | AK024681 | --- | --- | -0.87 | 6.32E-03 |
| AL527331 | 238716_at | AL527331 | --- | --- | -0.87 | 3.42E-03 |
| KIAA0711 | 204301_at | NM_014867 | NM_014867 | 9920 | -0.87 | 5.63E-07 |
| NOL3 | 221566_s_at | AF043244 | NM_003946 | 8996 | -0.87 | 4.45E-03 |
| AW236954 | 1557117_at | AW236954 | --- | --- | -0.87 | 1.02E-03 |
| MRPS33 | 218654_s_at | NM_016071 | NM_016071 | 51650 | -0.87 | 5.05E-03 |
| | | | NM_053035 | | | |
| SLC19A1 | 229489_at | AI681043 | NM_003056 | 6573 | -0.87 | 4.84E-04 |
| | | | NM_194255 | | | |
| ATP2C1 | 209935_at | AF225981 | NM_00100148 5 | 27032 | -0.87 | 1.03E-03 |
| | | | NM_00100148 6 | | | |
| | | | NM_00100148 7 | | | |
| | | | NM_014382 | | | |
| MBNL1 | 201153_s_at | NM_021038 | NM_021038 | 4154 | -0.87 | 2.49E-04 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| MGC52110 | 226039_at | AW006441 | NM_00100821E | 493753 | -0.86 | 1.48E-03 |
| OTUB2 | 219369_s_at | NM_023112 | NM_023112 | 78990 | -0.86 | 4.20E-03 |
| ZNF558 | 227810_at | AW119060 | NM_144693 | 148156 | -0.86 | 1.86E-04 |
| ANKH | 229176_at | A1672354 | NM_054027 | 56172 | -0.86 | 8.12E-03 |
| SYNE1 | 209447_at | AF043290 | NM_015293 | 23345 | -0.86 | 3.81E-03 |
| | | | NM_033071 | | | |
| | | | NM_133650 | | | |
| | | | NM_182961 | | | |
| MGC35097 | 225755_at | AI160540 | NM_173546 | 200942 | -0.86 | 6.77E-03 |
| AI471969 | 239278_at | AI471969 | --- | --- | -0.86 | 2.25E-03 |
| OR6M1 | 227112_at | AW270037 | NM_00100532E | 390261 | -0.86 | 2.99E-04 |
| DSCR3 | 217309_s_at | AJ001867 | NM_006052 | 10311 | -0.86 | 1.52E-04 |
| VGLL4 | 212399_s_at | D50911 | NM_014667 | 9686 | -0.86 | 1.90E-03 |
| RNH | 206050_s_at | NM_002939 | NM_002939 | 6050 | -0.85 | 1.17E-03 |
| | | | NM_203383 | | | |
| | | | NM_203384 | | | |
| | | | NM_203385 | | | |
| | | | NM_203386 | | | |
| | | | NM_203387 | | | |
| | | | NM_203388 | | | |
| | | | NM_203389 | | | |
| EIF3S2 | 208756_at | U36764 | NM_003757 | 8668 | -0.85 | 3.95E-04 |
| CHCHD5 | 235825_at | AI057238 | NM_032309 | 84269 | -0.85 | 2.48E-03 |
| FLJ25715 | 231952_at | AU157271 | NM_182570 | 284241 | -0.85 | 4.49E-03 |
| H28915 | 244359_s_at | H28915 | XM_496692 | 441014 | -0.85 | 2.81E-03 |
| SVH | 223328_at | BC003586 | NM_031905 | 83787 | -0.85 | 2.28E-03 |
| BRD4 | 238612_at | AW298070 | NM_014299 | 23476 | -0.85 | 4.92E-04 |
| | | | NM_058243 | | | |
| PLEKHC1 | 209209_s_at | AW469573 | NM_006832 | 10979 | -0.85 | 9.05E-03 |
| LOC220930 | 229090_at | AK026657 | --- | 220930 | -0.85 | 5.83E-04 |
| AW665538 | 244532_x_at | AW665538 | XM_496692 | 441014 | -0.85 | 3.20E-03 |
| DKFZp727A071 | 227348_at | AW409848 | NM_152268 | 25973 | -0.85 | 4.70E-05 |
| STK19 | 36019_at | L26260 | | 8859 | -0.85 | 1.10E-03 |
| | | | NM_004197 | | | |
| | | | NM_032454 | | | |
| DCTD | 210137_s_at | BC001286 | NM_001921 | 1635 | -0.84 | 7.33E-03 |
| AMT | 204294_at | NM_000481 | NM_000481 | 275 | -0.84 | 1.21E-03 |
| ERBB4 | 206794_at | NM_005235 | NM_005235 | 2066 | -0.84 | 7.70E-03 |
| AI003508 | 244114_x_at | AI003508 | XM_496690 | 401131 | -0.84 | 2.43E-03 |
| CNOT6L | 227119_at | BF103856 | NM_144571 | 246175 | -0.84 | 1.58E-04 |
| LRRC14 | 32062_at | D25216 | NM_014665 | 9684 | -0.84 | 1.95E-03 |
| AI796858 | 236164_at | AI796858 | --- | --- | -0.84 | 8.09E-03 |
| SUMF2 | 225002_s_at | BE349022 | NM_015411 | 25870 | -0.84 | 1.85E-03 |
| CLN8 | 229958_at | W93695 | NM_018941 | 2055 | -0.84 | 1.34E-03 |
| TDRKH | 221052_at | NM_006862 | NM_006862 | 11022 | -0.84 | 1.92E-03 |
| BF340290 | 225220_at | BF340290 | --- | --- | -0.84 | 8.97E-03 |
| LNX | 227293_at | AI264003 | NM_032622 | 84708 | -0.84 | 2.20E-03 |
| ADCK1 | 227482_at | AI097656 | NM_020421 | 57143 | -0.83 | 4.63E-03 |
| FLJ31340 | 235301_at | AI797353 | NM_152748 | 222223 | -0.83 | 9.27E-04 |
| FLJ12716 | 218179_s_at | NM_021942 | NM_021942 | 60684 | -0.83 | 3.97E-04 |
| | | | NM_199053 | | | |
| ACP6 | 218795_at | NM_016361 | NM_016361 | 51205 | -0.83 | 7.19E-04 |
| KIAA0543 | 226808_at | AW771007 | XM_376720 | 23145 | -0.83 | 2.42E-04 |
| | | | XM_379967 | | | |
| PAK6 | 1555310_a_at | BC035596 | NM_020168 | 56924 | -0.83 | 2.78E-03 |
| TXNDC4 | 208958_at | AI827677 | NM_015051 | 23071 | -0.83 | 1.35E-03 |
| PAM | 202336_s_at | NM_000919 | NM_000919 | 5066 | -0.83 | 4.55E-03 |
| | | | NM_138766 | | | |
| | | | NM_138821 | | | |
| | | | NM_138822 | | | |
| LOC57149 | 203897_at | BE963444 | NM_020424 | 57149 | -0.82 | 1.05E-04 |
| DCP1B | 226093_at | AW204088 | NM_152640 | 196513 | -0.82 | 6.19E-04 |
| FLJ12716 | 233558_s_at | AK023390 | NM_021942 | 60684 | -0.82 | 8.71E-04 |
| | | | NM_199053 | | | |
| TP53TG3 | 220167_s_at | NM_015369 | NM_015369 | 24150 | -0.82 | 3.88E-04 |
| | | | NM_016212 | | | |
| OCIAD2 | 225314_at | BG291649 | NM_152398 | 132299 | -0.82 | 8.74E-03 |
| C14orf133 | 218431_at | NM_022067 | NM_022067 | 63894 | -0.82 | 9.75E-04 |
| FLJ38991 | 227442_at | BG283902 | NM_173827 | 285521 | -0.82 | 2.25E-03 |
| FLJ31340 | 244317_at | BF035563 | NM_152748 | 222223 | -0.82 | 5.35E-03 |
| DSCR3 | 203635_at | NM_006052 | NM_006052 | 10311 | -0.82 | 8.42E-04 |
| PDCD2 | 204025_s_at | NM_002598 | NM_002598 | 5134 | -0.81 | 5.01E-04 |
| | | | NM_144781 | | | |
| DKFZP564D166 | 224952_at | BF115054 | XM_371074 | 26115 | -0.81 | 3.50E-04 |
| FLJ13231 | 219381_at | NM_023073 | NM_023073 | 65250 | -0.81 | 9.78E-03 |
| CDC37L1 | 235787_at | T65394 | NM_017913 | 55664 | -0.81 | 7.07E-04 |
| WDTC1 | 215497_s_at | AK023778 | NM_015023 | 23038 | -0.81 | 5.43E-04 |
| LOC94431 | 216908_x_at | AF001549 | NM_145237 | 94431 | -0.81 | 3.74E-06 |
| SEC31L1 | 215009_s_at | U92014 | NM_014933 | 22872 | -0.81 | 2.06E-03 |
| | | | NM_016211 | | | |
| ZNF148 | 230821_at | AW594167 | NM_021964 | 7707 | -0.81 | 5.30E-03 |
| DHRSX | 225503_at | AL547782 | NM_145177 | 207063 | -0.81 | 8.16E-06 |
| LYPLA3 | 204458_at | AL110209 | NM_012320 | 23659 | -0.81 | 3.93E-03 |
| DCTD | 201572_x_at | NM_001921 | NM_001921 | 1635 | -0.81 | 5.30E-04 |
| FLJ31265 | 228341_at | AI809108 | NM_152395 | 131870 | -0.81 | 9.80E-04 |
| TBC1D12 | 221858_at | N34407 | XM_051081 | 23232 | -0.81 | 2.80E-03 |
| FLJ22795 | 223327_x_at | AF316855 | NM_025084 | 80154 | -0.81 | 6.81E-06 |
| ZNF274 | 204937_s_at | NM_016325 | NM_016324 | 10782 | -0.81 | 2.65E-03 |
| | | | NM_016325 | | | |
| | | | NM_133502 | | | |
| CSTB | 201201_at | NM_000100 | NM_000100 | 1476 | -0.80 | 6.53E-04 |
| LOC151146 | 227604_at | BG292251 | --- | 151146 | -0.80 | 2.89E-03 |
| SLC18A2 | 205857_at | AI269290 | NM_003054 | 6571 | -0.80 | 3.73E-03 |
| MANSC1 | 220945_x_at | NM_018050 | NM_018050 | 54682 | -0.80 | 2.89E-03 |
| R44603 | 244113_at | R44603 | --- | --- | -0.80 | 2.19E-03 |
| PAK1 | 226507_at | AU154408 | NM_002576 | 5058 | -0.80 | 4.33E-03 |
| KIRREL3 | 240402_at | H05918 | NM_032531 | 84623 | -0.80 | 2.61E-03 |
| AW516665 | 227571_at | AW516665 | --- | --- | -0.80 | 4.02E-03 |
| FBXL5 | 209005_at | AF157323 | NM_012161 | 26234 | -0.80 | 2.18E-04 |
| | | | NM_033535 | | | |
| TM4SF2 | 202242_at | NM_004615 | NM_004615 | 7102 | -0.80 | 4.39E-03 |
| EHD2 | 45297_at | AI417917 | NM_014601 | 30846 | -0.80 | 2.06E-05 |
| UBE2D3 | 200667_at | BF448062 | NM_003340 | 7323 | -0.79 | 7.56E-06 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| AA868896 | 226203_at | AA868896 | --- | --- | -0.79 | 2.92E-03 |
| DUSP24 | 233982_x_at | AF188204 | NM_016086 | 51657 | -0.79 | 9.14E-03 |
| AU157441 | 226511_at | AU157441 | --- | --- | -0.79 | 1.27E-03 |
| AK2 | 205996_s_at | NM_013411 | NM_001625 | 204 | -0.79 | 2.76E-03 |
| | | | NM_013411 | | | |
| | | | NM_172199 | | | |
| AF086443 | 1560982_at | AF086443 | --- | --- | -0.79 | 4.61E-03 |
| C9orf9 | 220050_at | NM_018956 | NM_018956 | 11092 | -0.79 | 1.17E-03 |
| ZNF23 | 213934_s_at | AL567808 | NM_145911 | 7571 | -0.79 | 3.42E-03 |
| HLA-G | 211529_x_at | M90684 | NM_002127 | 3135 | -0.79 | 1.15E-03 |
| XPR1 | 226615_at | BE439489 | NM_004736 | 9213 | -0.79 | 8.43E-04 |
| THAP9 | 230185_at | AW301218 | NM_024672 | 79725 | -0.79 | 4.10E-03 |
| CCNDBP1 | 223084_s_at | AF246144 | NM_012142 | 23582 | -0.79 | 1.05E-04 |
| | | | NM_037370 | | | |
| AP3B2 | 205678_at | NM_004644 | NM_004644 | 8120 | -0.78 | 3.03E-03 |
| CTHRC1 | 225681_at | AA584310 | NM_138455 | 115908 | -0.78 | 2.80E-03 |
| LOC93622 | 225391_at | AL562398 | NM_138699 | 93622 | -0.78 | 8.43E-03 |
| JAG2 | 209784_s_at | AF029778 | NM_002226 | 3714 | -0.78 | 7.94E-03 |
| | | | NM_145159 | | | |
| ZNF585A | 229794_at | BE550717 | NM_152655 | 199704 | -0.78 | 4.45E-03 |
| | | | NM_199126 | | | |
| MGC2650 | 204985_s_at | NM_024108 | NM_024108 | 79090 | -0.78 | 4.43E-03 |
| APRT | 203219_s_at | NM_000485 | NM_000485 | 353 | -0.78 | 5.08E-04 |
| USP36 | 220370_s_at | NM_025090 | NM_025090 | 57602 | -0.78 | 3.64E-03 |
| INPP5A | 203006_at | NM_005539 | NM_005539 | 3632 | -0.78 | 8.67E-03 |
| ZNF197 | 205855_at | NM_006991 | NM_006991 | 10168 | -0.78 | 6.14E-03 |
| PLEKHA2 | 225136_at | BF968578 | XM_496973 | 59339 | -0.78 | 4.19E-04 |
| ARHGEF4 | 205109_s_at | NM_015320 | NM_015320 | 50649 | -0.77 | 7.05E-03 |
| | | | NM_032995 | | | |
| NETO1 | 1552736_a_at | NM_138966 | NM_138966 | 81832 | -0.77 | 2.29E-04 |
| | | | NM_138999 | | | |
| | | | NM_153181 | | | |
| LRPAP1 | 201186_at | NM_002337 | NM_002337 | 4043 | -0.77 | 7.07E-03 |
| MYST1 | 221820_s_at | AK024102 | NM_032188 | 84148 | -0.77 | 7.03E-03 |
| FLJ12998 | 244734_at | W45568 | NM_022764 | 64779 | -0.77 | 1.04E-04 |
| CLTC | 238646_at | AA747756 | NM_004859 | 1213 | -0.77 | 8.00E-03 |
| SLC30A5 | 1555334_s_at | AF439324 | NM_022902 | 64924 | -0.77 | 3.91E-03 |
| | | | NM_024055 | | | |
| PRKAG1 | 201805_at | NM_002733 | NM_002733 | 5571 | -0.77 | 2.24E-03 |
| | | | NM_212461 | | | |
| GPRASP1 | 204793_at | NM_014710 | NM_014710 | 9737 | -0.77 | 1.73E-03 |
| NETO1 | 1562713_a_at | AL834354 | NM_138966 | 81832 | -0.77 | 2.38E-03 |
| | | | NM_138999 | | | |
| | | | NM_153181 | | | |
| LOC89894 | 227172_at | BC000282 | NM_138341 | 89894 | -0.77 | 3.71E-04 |
| STK19 | 204090_at | NM_004197 | | 8859 | -0.77 | 2.21E-03 |
| | | | NM_004197 | | | |
| | | | NM_032454 | | | |
| LIPT1 | 205571_at | NM_015929 | NM_015929 | 51601 | -0.77 | 1.12E-03 |
| | | | NM_145196 | | | |
| | | | NM_145197 | | | |
| | | | NM_145198 | | | |
| | | | NM_145199 | | | |
| ZCWCC3 | 213000_at | AP000693 | NM_015358 | 23515 | -0.77 | 1.90E-03 |
| CLCN3 | 201735_s_at | NM_001829 | NM_001829 | 1182 | -0.77 | 8.06E-03 |
| | | | NM_173872 | | | |
| FLJ32370 | 226483_at | AI890761 | NM_152417 | 137695 | -0.76 | 2.24E-03 |
| FYCO1 | 218204_s_at | NM_024513 | NM_024513 | 79443 | -0.76 | 1.16E-07 |
| NEIL2 | 226585_at | BE466620 | NM_145043 | 252969 | -0.76 | 5.49E-04 |
| KCNH2 | 205262_at | NM_000238 | NM_000238 | 3757 | -0.76 | 9.14E-06 |
| | | | NM_172056 | | | |
| | | | NM_172057 | | | |
| BF966015 | 226787 _at | BF966015 | --- | --- | -0.76 | 3.80E-06 |
| BBP | 213882_at | AA012917 | NM_032027 | 83941 | -0.76 | 1.89E-03 |
| GSTZ1 | 209531_at | BC001453 | NM_001513 | 2954 | -0.76 | 3.56E-03 |
| | | | NM_145870 | | | |
| | | | NM_145871 | | | |
| EIF2C1 | 228120_at | AW136032 | NM_012199 | 26523 | -0.76 | 6.17E-03 |
| PCTK2 | 206474_at | NM_002595 | NM_002595 | 5128 | -0.76 | 4.76E-03 |
| C13orf21 | 243094_at | BF509416 | NM_001010897 | 387923 | -0.76 | 5.57E-03 |
| MAPRE3 | 214270_s_at | AI885178 | NM_012326 | 22924 | -0.76 | 3.88E-03 |
| BC017239 | 1558045_a_at | BC017239 | --- | --- | -0.75 | 3.87E-03 |
| COL27A1 | 225288_at | AI949136 | NM_032888 | 85301 | -0.75 | 9.38E-04 |
| AI672159 | 226550_at | AI672159 | --- | --- | -0.75 | 1.09E-04 |
| MBNL1 | 201152_s_at | N31913 | NM_021038 | 4154 | -0.75 | 1.55E-04 |
| | | | NM_207292 | | | |
| | | | NM_207293 | | | |
| | | | NM_207294 | | | |
| | | | NM_207295 | | | |
| | | | NM_207296 | | | |
| | | | NM_207297 | | | |
| TBN | 1556178_x_at | BU618741 | NM_138572 | 129685 | -0.75 | 9.97E-03 |
| TMAP1 | 227326_at | BE966768 | NM_00100852 8 | 439921 | -0.75 | 4.09E-04 |
| | | | NM_00100852 9 | | | |
| | | | NM_198530 | | | |
| TNFRSF21 | 218856_at | NM_016629 | NM_014452 | 27242 | -0.75 | 6.87E-03 |
| CHMP1.5 | 218177_at | AA293502 | NM_020412 | 57132 | -0.75 | 5.52E-05 |
| SHFM3 | 221519_at | AF281859 | NM_022039 | 6468 | -0.75 | 1.33E-03 |
| AU151635 | 232111_at | AU151635 | --- | --- | -0.75 | 1.40E-07 |
| PTK9L | 202009_at | NM_007284 | NM_007284 | 11344 | -0.75 | 8.11E-03 |
| PAM | 214620_x_at | BF038548 | NM_000919 | 5066 | -0.75 | 3.40E-03 |
| | | | NM_138766 | | | |
| | | | NM_138821 | | | |
| | | | NM_138822 | | | |
| WDFY3 | 212606_at | AL536319 | NM_014991 | 23001 | -0.75 | 1.05E-03 |
| | | | NM_178583 | | | |
| | | | NM_178585 | | | |
| KIAA1277 | 221885_at | AI131051 | NM_015689 | 27147 | -0.75 | 5.98E-03 |
| DIRAS2 | 219619_at | NM_017594 | NM_017594 | 54769 | -0.74 | 6.42E-03 |
| EXT2 | 202012_s_at | AA196245 | NM_000401 | 2132 | -0.74 | 5.88E-04 |
| | | | NM_207122 | | | |
| C18orf17 | 238480_at | AI871745 | NM_153211 | 125488 | -0.74 | 4.89E-04 |
| SLC9A1 | 209453_at | M81768 | NM_003047 | 6548 | -0.74 | 5.21E-06 |
| PCBD1 | 203557_s_at | NM_000281 | NM_000281 | 5092 | -0.74 | 2.72E-04 |
| | | | NM_001001939 | | | |
| C6orf83 | 225850_at | BF975327 | NM_145169 | 113402 | -0.74 | 6.67E-03 |
| PIGV | 219238_at | NM_017837 | NM_017837 | 55650 | -0.74 | 4.79E-04 |
| KIAA0276 | 243691_at | AA150455 | NM_015115 | 23142 | -0.74 | 7.85E-03 |
| PAM | 212958_x_at | AI022882 | NM_000919 | 5066 | -0.74 | 9.54E-03 |
| | | | NM_138766 | | | |
| | | | NM_138821 | | | |
| | | | NM_138822 | | | |
| NM_152909 | 1553719_s_at | NM_152909 | --- | --- | -0.74 | 1.13E-03 |
| PKD1 | 216949_s_at | L39891 | NM_000296 | 5310 | -0.74 | 1.43E-03 |
| | | | NM_001009944 | | | |
| AI768895 | 231069_at | AI768895 | --- | --- | -0.74 | 7.25E-03 |
| MTMR7 | 228024_at | AW028100 | NM_004686 | 9108 | -0.74 | 3.76E-05 |
| BRUNOL4 | 223653_x_at | AW593887 | NM_020180 | 56853 | -0.74 | 9.74E-03 |
| LOH12CR1 | 227723_at | BF439234 | NM_058169 | 118426 | -0.74 | 7.19E-03 |
| PTN | 209465_x_at | AL565812 | NM_002825 | 5764 | -0.73 | 7.19E-03 |
| RPL4 | 226556_at | BF431260 | NM_000968 | 6124 | -0.73 | 4.08E-03 |
| MTRF1 | 219822_at | NM_004294 | NM_004294 | 9617 | -0.73 | 1.42E-04 |
| PCCA | 203860_at | NM_000282 | NM_000282 | 5095 | -0.73 | 9.56E-03 |
| RPS4Y1 | 201909_at | NM_001008 | NM_001008 | 6192 | -0.73 | 2.03E-03 |
| FLJ11273 | 222787_s_at | AV705186 | NM_018374 | 54664 | -0.73 | 7.27E-04 |
| CSE-C | 224391_s_at | AF303378 | NM_170601 | 54414 | -0.73 | 3.37E-03 |
| KIAA0323 | 212356_at | AB002321 | NM_015299 | 23351 | -0.73 | 8.63E-03 |
| A1061377 | 235232_at | A1061377 | --- | --- | -0.73 | 9.74E-03 |
| SH3YL1 | 204019_s_at | NM_015677 | NM_015677 | 26751 | -0.73 | 2.48E-03 |
| LOC255458 | 1556180_at | BE646146 | --- | 255458 | -0.73 | 6.08E-05 |
| JAGN1 | 223104_at | AF212230 | NM_032492 | 84522 | -0.73 | 2.17E-05 |
| FLJ10038 | 205511_at | NM_017976 | --- | 55056 | -0.73 | 5.36E-03 |
| FLJ13984 | 220007_at | NM_024770 | NM_024770 | 79828 | -0.73 | 6.13E-03 |
| TMEM34 | 219074_at | NM_018241 | NM_018241 | 55751 | -0.73 | 8.00E-03 |
| ARRDC1 | 226405_s_at | AK001822 | NM_152285 | 92714 | -0.72 | 2.87E-03 |
| BF056746 | 228425_at | BF056746 | --- | --- | -0.72 | 4.99E-04 |
| P15RS | 222559_s_at | T79568 | NM_018170 | 55197 | -0.72 | 1.36E-05 |
| FLJ23560 | 219487_at | NM_024685 | NM_024685 | 79738 | -0.72 | 9.23E-03 |
| ANKH | 223093_at | T99215 | NM_054027 | 56172 | -0.72 | 3.35E-03 |
| VAPA | 225198_at | AL571942 | NM_003574 | 9218 | -0.72 | 2.38E-03 |
| | | | NM_194434 | | | |
| CCDC2 | 219174_at | NM_025103 | NM_025103 | 80173 | -0.72 | 2.41E-03 |
| AA709148 | 243751_at | AA709148 | --- | --- | -0.72 | 2.28E-04 |
| GART | 217445_s_at | AF008655 | NM_000819 | 2618 | -0.72 | 1.27E-03 |
| | | | NM_175085 | | | |
| LOC389906 | 1558046_x_at | BC017239 | XM_372273 | 389906 | -0.72 | 1.71E-03 |
| LOC389910 | | | XM_374343 | 389910 | | |
| LOC401625 | | | XM_377076 | 401625 | | |
| SAV1 | 222573_s_at | AI679398 | NM_021818 | 60485 | -0.72 | 5.04E-04 |
| BBS2 | 223227_at | AF342736 | NM_031885 | 583 | -0.72 | 2.39E-03 |
| FLJ10726 | 218314_s_at | NM_018195 | NM_018195 | 55216 | -0.72 | 9.03E-04 |
| AK055769 | 1558801_at | AK055769 | --- | --- | -0.71 | 5.90E-03 |
| R53065 | 240430_at | R53065 | --- | --- | -0.71 | 6.12E-03 |
| WDFY3 | 212602_at | AI806395 | NM_014991 | 23001 | -0.71 | 1.21E-03 |
| | | | NM_178583 | | | |
| | | | NM_178585 | | | |
| CSNK2A2 | 203575_at | NM_001896 | NM_001896 | 1459 | -0.71 | 5.96E-03 |
| KIAA0992 | 200907_s_at | AU157932 | NM_016081 | 23022 | -0.71 | 2.15E-03 |
| LMNA | 212086_x_at | AK026584 | NM_005572 | 4000 | -0.71 | 6.61E-03 |
| | | | NM_170707 | | | |
| | | | NM_170708 | | | |
| BE502930 | 235124_at | BE502930 | --- | --- | -0.71 | 3.16E-04 |
| AI765014 | 224649_x_at | AI765014 | --- | --- | -0.71 | 2.78E-03 |
| TTC13 | 219481_at | NM_024525 | NM_024525 | 79573 | -0.71 | 1.48E-03 |
| GAS5 | 224841_x_at | BF316352 | --- | 60674 | -0.71 | 6.06E-03 |
| AI652899 | 228812_at | AI652899 | --- | --- | -0.71 | 5.25E-05 |
| PLCB1 | 213222_at | AL049593 | NM_015192 | 23236 | -0.71 | 7.21E-04 |
| | | | NM_182734 | | | |
| DET1 | 219641_at | NM_017996 | NM_017996 | 55070 | -0.71 | 1.63E-03 |
| ACSL3 | 201660_at | AL525798 | NM_004457 | 2181 | -0.71 | 3.90E-04 |
| | | | NM_203372 | | | |
| MGC2749 | 200076_s_at | BC006479 | NM_024069 | 79036 | -0.71 | 6.49E-03 |
| QPCT | 205174_s_at | NM_012413 | NM_012413 | 25797 | -0.71 | 6.07E-03 |
| PML | 209640_at | M79462 | NM_002675 | 5371 | -0.70 | 3.55E-03 |
| | | | NM_033238 | | | |
| | | | NM_033239 | | | |
| | | | NM_033240 | | | |
| | | | NM_033242 | | | |
| | | | NM_033244 | | | |
| | | | NM_033245 | | | |
| | | | NM_033246 | | | |
| | | | NM_033247 | | | |
| | | | NM_033249 | | | |
| | | | NM_033250 | | | |
| LRRC27 | 1558483_at | AK098652 | NM_030626 | 80313 | -0.70 | 3.75E-03 |
| STK16 | 209622_at | AB020739 | NM_001008910 | 8576 | -0.70 | 2.64E-04 |
| | | | NM_003691 | | | |
| CRYZL1 | 219767_s_at | NM_005111 | NM_005111 | 9946 | -0.70 | 2.94E-03 |
| | | | NM_145311 | | | |
| | | | NM_145858 | | | |
| TMEM43 | 217795_s_at | W74580 | NM_024334 | 79188 | -0.70 | 3.09E-03 |
| DFFA | 203277_at | NM_004401 | NM_004401 | 1676 | -0.70 | 4.51E-03 |
| | | | NM_213566 | | | |
| BF062875 | 230072_at | BF062875 | --- | --- | -0.70 | 7.79E-04 |
| AW974666 | 222365_at | AW974666 | --- | --- | -0.70 | 3.83E-03 |
| AA827878 | 223577_x_at | AA827878 | --- | --- | -0.70 | 8.67E-03 |
| TMEM43 | 222418_s_at | AA115485 | NM_024334 | 79188 | -0.70 | 3.97E-03 |
| C14orf118 | 219720_s_at | NM_017972 | NM_017926 | 55668 | -0.70 | 1.92E-03 |
| | | | NM_017972 | | | |
| LOC285550 | 227270_at | BF593037 | XM_209656 | 285550 | -0.70 | 1.40E-03 |
| LRRC28 | 227423_at | AI655972 | NM_144598 | 123355 | -0.70 | 6.98E-04 |
| PFN2 | 237116_at | AI262277 | NM_002628 | 5217 | -0.70 | 2.39E-03 |
| | | | NM_053024 | | | |
| JMY | 226352_at | BF447037 | NM_152405 | 133746 | -0.70 | 9.37E-04 |
| TLOC1 | 225352_at | AI763287 | NM_003262 | 7095 | -0.69 | 2.60E-04 |
| PPID | 204186_s_at | AI014573 | NM_005038 | 5481 | -0.69 | 1.10E-03 |
| FLJ38968 | 226383_at | AI652452 | NM_152316 | 120534 | -0.69 | 1.37E-03 |
| KIAA0992 | 200897 _s_at | NM_016081 | NM_016081 | 23022 | -0.69 | 1.00E-03 |
| SLC30A5 | 232432_s_at | AK022818 | NM_022902 | 64924 | -0.69 | 2.34E-03 |
| | | | NM_024055 | | | |
| AIG1 | 230520_at | T78402 | NM_016108 | 51390 | -0.69 | 7.09E-05 |
| FKBP9 | 212169_at | AL050187 | NM_007270 | 11328 | -0.69 | 3.13E-03 |
| T1 | 56829_at | H61826 | NM_031466 | 83696 | -0.69 | 4.28E-03 |
| AA813998 | 235837_at | AA813998 | --- | --- | -0.69 | 7.10E-03 |
| SNAP91 | 204953_at | NM_014841 | NM_014841 | 9892 | -0.69 | 8.76E-03 |
| PLD3 | 201050_at | NM_012268 | NM_012268 | 23646 | -0.69 | 2.09E-03 |
| CGI-41 | 218914_at | NM_015997 | NM_015997 | 51093 | -0.69 | 6.72E-03 |
| RARA | 228037_at | AA404273 | NM_000964 | 5914 | -0.69 | 9.78E-04 |
| MYO9A | 219027_s_at | NM_006901 | NM_006901 | 4649 | -0.68 | 3.06E-03 |
| RDH13 | 1559190_s_at | AL833150 | NM_138412 | 112724 | -0.68 | 6.77E-03 |
| RGS14 | 204280_at | NM_006480 | NM_006480 | 10636 | -0.68 | 1.43E-04 |
| FLJ34222 | 235717_at | AA180985 | XM_371175 | 284350 | -0.68 | 3.45E-05 |
| BF434383 | 235473_at | BF434383 | --- | --- | -0.68 | 8.03E-04 |
| CDKL3 | 219831_at | NM_016508 | NM_016508 | 51265 | -0.68 | 4.12E-05 |
| FRBZ1 | 226962_at | AW299558 | NM_194314 | 360023 | -0.68 | 3.75E-03 |
| FLJ37953 | 241825_at | AI265967 | NM_152382 | 129450 | -0.68 | 8.57E-04 |
| TRIM4 | 223384_s_at | BE501464 | | 89122 | -0.68 | 2.58E-03 |
| | | | NM_033017 | | | |
| | | | NM_033091 | | | |
| SF3B3 | 200688_at | D13642 | NM_012426 | 23450 | -0.68 | 4.67E-03 |
| PHF10 | 219126_at | NM_018288 | NM_018288 | 55274 | -0.68 | 2.92E-03 |
| | | | NM_133325 | | | |
| IL17RB | 235531_at | AA928257 | NM_018725 | 55540 | -0.68 | 2.10E-03 |
| | | | NM_172234 | | | |
| BCL2L13 | 224035_s_at | AF183411 | NM_015367 | 23786 | -0.68 | 7.08E-04 |
| MAN1A2 | 217922_at | AL157902 | NM_006699 | 10905 | -0.68 | 3.21E-03 |
| ZNF232 | 236075_s_at | AI693543 | NM_014519 | 7775 | -0.68 | 6.78E-03 |
| BC024246 | 1556301_at | BC024246 | --- | --- | -0.68 | 2.06E-04 |
| ZRANB1 | 225130_at | AW003734 | NM_017580 | 54764 | -0.67 | 1.84E-04 |
| PP1665 | 32502_at | AL041124 | NM_030792 | 81544 | -0.67 | 9.49E-04 |
| CAB39 | 224311_s_at | AF151824 | NM_016289 | 51719 | -0.67 | 2.14E-03 |
| KRTAP4-7 | 225225_at | BF791544 | NM_033061 | 85287 | -0.67 | 8.80E-03 |
| SLC30A5 | 218989_x_at | NM_022902 | NM_022902 | 64924 | -0.67 | 6.75E-03 |
| | | | NM_024055 | | | |
| RNF41 | 201961_s_at | AL583171 | NM_005785 | 10193 | -0.67 | 8.95E-03 |
| | | | NM_194358 | | | |
| | | | NM_194359 | | | |
| C6orf168 | 232067_at | BC004869 | NM_032511 | 84553 | -0.67 | 2.42E-03 |
| TNIP2 | 232160_s_at | AL137262 | NM_024309 | 79155 | -0.67 | 1.56E-03 |
| DKFZP564D172 | 212936_at | AI927701 | NM_032042 | 83989 | -0.67 | 5.18E-04 |
| LOC439962 | 1560047_s_at | AA127813 | XM_498479 | 439962 | -0.67 | 3.91E-03 |
| MCM3APAS | 220459_at | NM_018118 | --- | 114044 | -0.67 | 2.26E-03 |
| SLC35E1 | 229410_at | AI659219 | NM_024881 | 79939 | -0.67 | 5.75E-03 |
| FBXW7 | 229419_at | BF222826 | NM_018315 | 55294 | -0.67 | 9.92E-04 |
| | | | NM_033632 | | | |
| C10orf22 | 212500_at | AL049319 | NM_032804 | 84890 | -0.67 | 3.49E-04 |
| SURF2 | 205224_at | NM_017503 | | 6835 | -0.67 | 3.01E-04 |
| | | | NM_017503 | | | |
| MGC12966 | 224452_s_at | BC006110 | NM_032706 | 84792 | -0.67 | 4.02E-04 |
| FLJ20195 | 223833_at | BC002482 | NM_017706 | 54853 | -0.67 | 5.69E-03 |
| FLJ20542 | 233625_x_at | AK021939 | NM_017871 | 54973 | -0.67 | 8.46E-04 |
| | | | NM_032179 | | | |
| LRCH2 | 227688_at | AK022128 | NM_020871 | 57631 | -0.67 | 2.21E-04 |
| SLC22A17 | 218675_at | NM_020372 | NM_016609 | 51310 | -0.67 | 3.50E-03 |
| | | | NM_020372 | | | |
| ARNT2 | 202986_at | NM 014862 | NM_ 014862 | 9915 | -0.67 | 1.18E-03 |
| LOC440118 | FX-M27830_M | AFFX-M27830_M | XM _498554 | 440118 | -0.67 | 5.77E-03 |
| FLJ23518 | 223300_s_at | AI286012 | NM_024725 | 79780 | -0.66 | 7.90E-04 |
| PROSC | 209384_at | AA176833 | NM_007198 | 11212 | -0.66 | 1.14E-03 |
| P15RS | 222558_at | T79568 | NM_018170 | 55197 | -0.66 | 1.67E-04 |
| PPP2R5C | 201877_s_at | NM_002719 | NM_002719 | 5527 | -0.66 | 3.07E-06 |
| | | | NM_178586 | | | |
| | | | NM_178587 | | | |
| | | | NM_178588 | | | |
| SGNE1 | 203889_at | NM_003020 | NM_003020 | 6447 | -0.66 | 1.31E-04 |
| CNOT6L | 1553267_a_at | NM_144571 | NM_144571 | 246175 | -0.66 | 5.11E-03 |
| TTLL1 | 205652_s_at | NM_012263 | NM_001008572 | 25809 | -0.66 | 3.38E-04 |
| | | | NM_012263 | | | |
| AD023 | 223411_at | AF225422 | NM_020679 | 57409 | -0.66 | 7.26E-06 |
| KIF3C | 203389_at | AF035621 | NM_002254 | 3797 | -0.66 | 2.70E-04 |
| AI598222 | 235679_at | AI598222 | --- | --- | -0.65 | 7.93E-03 |
| PPP3CA | 202457 _s_at | AA911231 | NM_000944 | 5530 | -0.65 | 9.97E-03 |
| RPIP8 | 213439_x_at | AI197870 | NM_006695 | 10900 | -0.65 | 1.89E-03 |
| NSMCE1 | 224666_at | AF161451 | NM_145080 | 197370 | -0.65 | 3.16E-04 |
| BACE1 | 222462_s_at | AI653425 | NM_012104 | 23621 | -0.65 | 3.25E-03 |
| | | | NM_138971 | | | |
| | | | NM_138972 | | | |
| | | | NM_138973 | | | |
| MPI | 202472_at | NM_002435 | NM_002435 | 4351 | -0.65 | 8.59E-04 |
| FBXW7 | 222729_at | BE551877 | NM_018315 | 55294 | -0.65 | 2.52E-03 |
| | | | NM_033632 | | | |
| NELF | 221214_s_at | NM_015537 | NM_015537 | 26012 | -0.65 | 7.98E-04 |
| HLA-A | 215313_x_at | AA573862 | NM_002116 | 3105 | -0.65 | 7.23E-03 |
| FGF2 | 204422_s_at | NM_002006 | NM_002006 | 2247 | -0.65 | 4.86E-07 |
| P15RS | 225953_at | AI684281 | NM_018170 | 55197 | -0.65 | 3.76E-04 |
| GAS5 | 224741_x_at | BG329175 | --- | 60674 | -0.65 | 8.18E-03 |
| IPO8 | 205701_at | NM_006390 | NM_006390 | 10526 | -0.65 | 7.25E-04 |
| ECE2 | 230706_s_at | A1084921 | NM_014693 | 9718 | -0.65 | 4.71E-04 |
| ZCSL3 | 213853_at | AL050199 | NM_181706 | 120526 | -0.65 | 6.76E-03 |
| PSEN1 | 226577_at | N49844 | NM_000021 | 5663 | -0.65 | 3.44E-03 |
| | | | NM_007318 | | | |
| | | | NM_007319 | | | |
| LOC284018 | 226901_at | AI214996 | NM_181655 | 284018 | -0.64 | 5.00E-04 |
| | | | NM_181656 | | | |
| FLJ32384 | 238547_at | AI972367 | NM_144608 | 124790 | -0.64 | 3.77E-03 |
| LOC284106 | 230727_at | BE348515 | XM_375449 | 284106 | -0.64 | 7.65E-03 |
| WDR32 | 219001_s_at | NM_024345 | NM_024345 | 79269 | -0.64 | 1.25E-04 |
| RNF13 | 201779_s_at | AF070558 | NM_007282 | 11342 | -0.64 | 1.26E-04 |
| | | | NM_183381 | | | |
| | | | NM_183382 | | | |
| | | | NM_183383 | | | |
| | | | NM_183384 | | | |
| VPS33B | 218415_at | NM_018668 | NM_018668 | 26276 | -0.64 | 7.92E-03 |
| SPTBN2 | 231311_at | AA725246 | NM_006946 | 6712 | -0.64 | 1.71E-03 |
| LOC389906 | 1569629_x_at | BC017231 | XM_372273 | 389906 | -0.64 | 4.97E-04 |
| C22orf9 | 227144_at | AA476916 | NM_00100988 0 | 23313 | -0.64 | 3.58E-03 |
| | | | NM_015264 | | | |
| PHKA1 | 205450_at | NM_002637 | NM_002637 | 5255 | -0.64 | 3.01E-03 |
| COPS7A | 209029_at | AF193844 | NM_016319 | 50813 | -0.64 | 6.55E-05 |
| EIF2S3 | 205321_at | NM_001415 | NM_001415 | 1968 | -0.64 | 8.39E-03 |
| TM6SF1 | 1558102_at | AK055438 | NM_023003 | 53346 | -0.64 | 2.18E-04 |
| ATCAY | 227365_at | AL566774 | NM_033064 | 85300 | -0.64 | 8.51E-03 |
| CGI-119 | 223892_s_at | AF182414 | NM_016056 | 51643 | -0.64 | 1.66E-04 |
| APG7L | 224025_s_at | BC000091 | NM_006395 | 10533 | -0.64 | 4.74E-03 |
| CIRBP | 230142_s_at | AW088030 | NM_001280 | 1153 | -0.64 | 2.64E-03 |
| C21orf127 | 220311_at | NM_013240 | NM_013240 | 29104 | -0.64 | 2.93E-03 |
| | | | NM_182749 | | | |
| DKFZP566D134 | 238597_at | BG028770 | NM_030816 | 81573 | -0.63 | 9.05E-03 |
| WNT3 | 229103_at | AA463626 | NM_030753 | 7473 | -0.63 | 1.82E-03 |
| C10orf88 | 219240_s_at | NM_024942 | NM_024942 | 80007 | -0.63 | 2.53E-03 |
| KCNMB3 | 235980_at | AA767763 | NM_014407 | 27094 | -0.63 | 9.48E-04 |
| | | | NM_171828 | | | |
| | | | NM_171829 | | | |
| | | | NM_171830 | | | |
| LOC440360 | 236838_at | H11609 | XM_498641 | 440360 | -0.63 | 4.34E-03 |
| AA868380 | 230569_at | AA868380 | --- | --- | -0.63 | 7.44E-03 |
| C3orf6 | 235051_at | BF696931 | NM_174908 | 152137 | -0.63 | 9.04E-03 |
| | | | NM_178335 | | | |
| BLP2 | 1552426_a_at | NM_078474 | NM_025141 | 80213 | -0.63 | 9.66E-03 |
| | | | NM_078474 | | | |
| PPID | 204185_x_at | NM_005038 | NM_005038 | 5481 | -0.63 | 3.34E-03 |
| CXorf12 | 204340_at | NM_003492 | NM_003492 | 8269 | -0.63 | 4.03E-03 |
| PLCB1 | 211925_s_at | AY004175 | NM_015192 | 23236 | -0.63 | 8.12E-05 |
| | | | NM_182734 | | | |
| MYO7A | 208189_s_at | NM_000260 | NM_000260 | 4647 | -0.63 | 1.17E-04 |
| UBE2D3 | 229355_at | AU150386 | NM_003340 | 7323 | -0.63 | 6.20E-03 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| CYP2U1 | 226393_at | AW055161 | NM_183075 | 113612 | -0.63 | 7.35E-03 |
| PFDN5 | 207132_x_at | NM_002624 | NM_002624 | 5204 | -0.63 | 9.53E-04 |
| | | | NM_145896 | | | |
| | | | NM_145897 | | | |
| WSB1 | 201294_s_at | N24643 | NM_015626 | 26118 | -0.63 | 2.39E-03 |
| | | | NM_134264 | | | |
| | | | NM_134265 | | | |
| MGC45386 | 227008_at | AI393309 | NM_198527 | 374659 | -0.63 | 9.40E-03 |
| PROSC | 214545_s_at | NM_007198 | NM_007198 | 11212 | -0.62 | 3.15E-04 |
| SELM | 226051_at | BF973568 | NM_080430 | 140606 | -0.62 | 4.03E-03 |
| FLJ21919 | 202809_s_at | NM_023015 | NM_023015 | 65123 | -0.62 | 7.33E-03 |
| MGC5356 | 220426_at | NM_024059 | NM_024059 | 79025 | -0.62 | 6.04E-03 |
| KIAA1671 | 225525_at | AB051458 | XM_371461 | 85379 | -0.62 | 1.04E-03 |
| SNX6 | 217789_at | NM_021249 | NM_021249 | 58533 | -0.62 | 4.75E-03 |
| | | | NM_152233 | | | |
| MPPE1 | 213727_x_at | AI743654 | NM_023075 | 65258 | -0.62 | 7.16E-04 |
| | | | NM_138608 | | | |
| ST6GALNAC6 | 222571_at | AK023900 | NM_013443 | 30815 | -0.62 | 2.17E-03 |
| MGC5391 | 226639_at | AI304320 | NM_032740 | 84826 | -0.62 | 3.40E-03 |
| MAP7 | 215471_s_at | AJ242502 | NM_003980 | 9053 | -0.62 | 3.97E-04 |
| FLJ11273 | 226529_at | BF513060 | NM_018374 | 54664 | -0.62 | 7.53E-03 |
| ADCK2 | 44120_at | AI879381 | NM_052853 | 90956 | -0.62 | 3.67E-03 |
| TST | 209605_at | D87292 | NM_003312 | 7263 | -0.62 | 8.01E-03 |
| NUDT2 | 218609_s_at | NM_001161 | NM_001161 | 318 | -0.62 | 7.23E-03 |
| | | | NM_147172 | | | |
| | | | NM_147173 | | | |
| AI632010 | 244687_at | AI632010 | --- | --- | -0.62 | 3.57E-04 |
| PHC1 | 225958_at | AI554106 | NM_004426 | 1911 | -0.62 | 8.62E-05 |
| PIK3CB | 212688_at | BC003393 | NM_006219 | 5291 | -0.62 | 6.77E-04 |
| LOC401466 | 1555241_at | BC032347 | XM_376783 | 401466 | -0.61 | 2.14E-03 |
| KIAA1458 | 224843_at | AB040891 | XM_044434 | 57606 | -0.61 | 4.73E-04 |
| AV756131 | 224915_x_at | AV756131 | XM_497769 | 441951 | -0.61 | 3.59E-03 |
| APRT | 213892_s_at | AA927724 | NM_000485 | 353 | -0.61 | 6.05E-04 |
| WDR41 | 218055_s_at | NM_018268 | NM_018268 | 55255 | -0.61 | 5.04E-03 |
| LOC143458 | 234985_at | AW043782 | NM_174902 | 143458 | -0.61 | 1.09E-04 |
| MGC15429 | 224821_at | AL520200 | NM_032750 | 84836 | -0.61 | 1.73E-03 |
| LOC387593 | 239715_at | AI374756 | --- | 387593 | -0.61 | 7.41E-03 |
| MGC2494 | 219709_x_at | NM_023933 | NM_023933 | 65990 | -0.61 | 7.21E-03 |
| SBF2 | 226169_at | AW276572 | NM_030962 | 81846 | -0.61 | 2.63E-03 |
| CGI-119 | 222845_x_at | AF161526 | NM_016056 | 51643 | -0.61 | 6.18E-04 |
| UBE2D3 | 200669_s_at | NM_003340 | NM_003340 | 7323 | -0.61 | 7.57E-04 |
| | | | NM_181886 | | | |
| | | | NM_181887 | | | |
| | | | NM_181888 | | | |
| | | | NM_181889 | | | |
| | | | NM_181890 | | | |
| | | | NM_181891 | | | |
| | | | NM_181892 | | | |
| | | | NM_181893 | | | |
| NPC1L1 | 220106_at | NM_013389 | NM_013389 | 29881 | -0.61 | 9.81E-04 |
| RNF13 | 201780_s_at | NM_007282 | NM_007282 | 11342 | -0.61 | 9.87E-04 |
| | | | NM_183381 | | | |
| | | | NM_183382 | | | |
| | | | NM_183383 | | | |
| | | | NM_183384 | | | |
| ARFIP1 | 214483_s_at | AF124489 | NM_014447 | 27236 | -0.60 | 3.32E-05 |
| AI741365 | 229757_at | AI741365 | --- | --- | -0.60 | 4.00E-03 |
| FAM14A | 223626_x_at | AF208232 | NM_032036 | 83982 | -0.60 | 4.30E-04 |
| DBT | 205369_x_at | J03208 | NM_001918 | 1629 | -0.60 | 2.60E-03 |
| MRPL1 | 223154_at | AF212225 | NM_020236 | 65008 | -0.60 | 2.59E-03 |
| KIAA1223 | 225731_at | BF196876 | XM_048747 | 57182 | -0.60 | 4.63E-03 |
| NFATC4 | 213345_at | AI624015 | NM_004554 | 4776 | -0.60 | 1.80E-03 |
| BF185165 | 226227_x_at | BF185165 | XM_497769 | 441951 | -0.59 | 3.75E-03 |
| GAL | 207466_at | NM_015973 | NM_015973 | 51083 | -0.59 | 6.79E-03 |
| LOC92017 | 225608_at | H15273 | NM_001009607 | 92017 | -0.59 | 1.54E-05 |
| WDR9 | 214820_at | AJ002572 | NM_00100724 6 | 54014 | -0.59 | 4.03E-04 |
| | | | NM_018963 | | | |
| | | | NM_033656 | | | |
| PLEKHA3 | 223370_at | AF286162 | NM_019091 | 65977 | -0.59 | 4.77E-03 |
| PPM1K | 235061_at | AV706522 | NM_152542 | 152926 | -0.59 | 2.94E-03 |
| MTL5 | 241466_at | AI275776 | NM_004923 | 9633 | -0.59 | 5.56E-04 |
| DHX32 | 218198_at | NM_018180 | NM_018180 | 55760 | -0.59 | 7.21E-03 |
| DKFZp451M211 | 231540_at | BF511274 | NM_182585 | 285023 | -0.59 | 1.28E-03 |
| MTM1 | 36920_at | U46024 | NM_000252 | 4534 | -0.59 | 4.62E-03 |
| ALKBH | 236721_at | AI922200 | NM_006020 | 8846 | -0.59 | 7.70E-03 |
| BC019017 | 1558128_at | BC019017 | --- | --- | -0.59 | 1.03E-03 |
| CDC42EP3 | 209288_s_at | AL136842 | NM_006449 | 10602 | -0.59 | 8.78E-04 |
| FLJ20277 | 233638_s_at | AK026430 | NM_017739 | 55624 | -0.59 | 4.86E-03 |
| AI766065 | 224647_at | AI766065 | --- | --- | -0.59 | 1.11E-03 |
| SETDB2 | 235339_at | W65369 | NM_031915 | 83852 | -0.59 | 5.43E-03 |
| BE326214 | 229287_at | BE326214 | --- | --- | -0.59 | 8.90E-04 |
| HS2ST1 | 203285_s_at | NM_012262 | NM_012262 | 9653 | -0.58 | 4.84E-04 |
| N51708 | 213930_at | N51708 | XM_499012 | 441104 | -0.58 | 1.86E-03 |
| GPR56 | 212070_at | AL554008 | NM_005682 | 9289 | -0.58 | 2.25E-03 |
| | | | NM_201524 | | | |
| | | | NM_201525 | | | |
| LOC119710 | 228249_at | AA535128 | NM_138787 | 119710 | -0.58 | 7.12E-03 |
| FLJ13576 | 234726_s_at | AK025482 | NM_022484 | 64418 | -0.58 | 5.50E-03 |
| PFDN5 | 210908_s_at | AB055804 | NM_002624 | 5204 | -0.58 | 1.71E-03 |
| | | | NM_145896 | | | |
| | | | NM_145897 | | | |
| SIDT2 | 218765_at | NM_015996 | NM_015996 | 51092 | -0.58 | 4.83E-03 |
| FLJ21168 | 204666_s_at | NM_025073 | NM_025073 | 80143 | -0.58 | 9.92E-04 |
| KIAA0241 | 212475_at | AI797458 | NM_015060 | 23080 | -0.58 | 4.82E-05 |
| CHIC1 | 228345_at | AI745136 | XR_000216 | 53344 | -0.58 | 6.80E-03 |
| BE645776 | 227288_at | BE645776 | XM_068632 | 133993 | -0.58 | 4.35E-04 |
| CTBS | 218923_at | AW304174 | NM_004388 | 1486 | -0.58 | 8.95E-03 |
| TMF1 | 235566_at | AW591660 | NM_007114 | 7110 | -0.58 | 7.30E-03 |
| PKNOX1 | 221883_at | AA133342 | NM_004571 | 5316 | -0.58 | 1.27E-03 |
| | | | NM_197976 | | | |
| ACTR1B | 202135_s_at | NM_005735 | NM_005735 | 10120 | -0.58 | 2.50E-03 |
| TNFRSF21 | 214581_x_at | BE568134 | NM_014452 | 27242 | -0.58 | 8.02E-03 |
| PEX7 | 211033_s_at | BC006268 | NM_000288 | 5191 | -0.58 | 8.93E-03 |
| EIF4E3 | 225941_at | AI935522 | --- | 317649 | -0.58 | 5.78E-03 |

## Claims

1. Cells from an immortal cell line selected from a parental SiMA cell line DSMZ ACC 164 wherein the cells are susceptible to BoNT/A intoxication and wherein the cells exhibit an EC₅₀ for BoNT/A activity of 2.2 pM or less.

## Patentansprüche

1. Zellen aus einer immortalen Zelllinie, ausgewählt aus einer parentalen SiMA-Zelllinie DSMZ ACC 164, wobei die Zellen für BoNT/A-Intoxikation empfänglich sind und wobei die Zellen einen EC₅₀ für BoNT/A-Aktivität von 2,2 pM oder kleiner aufweisen.

## Revendications

1. Cellules provenant d'une lignée cellulaire immortelle sélectionnée à partir d'une lignée cellulaire SiMA parentale DSMZ ACC 164 dans lesquelles les cellules sont sensibles à une intoxication aux BoNT/A et dans lesquelles les cellules présentent une EC₅₀ pour l'activité de BoNT/A égale ou inférieure à 2,2 pM.


SEQUENCE LISTING
<110> Birgitte Jacky
   Wang, Joanne
   Zhu, Hong
   Hodges, D. Dianne
   Fernandez-Salas, Ester
<120> Cells Useful for Immuno-Based Botulinum
   Toxin Serotype A Activity Assays
<130> 18495 (BOT)
<150> US 61/160,199
   <151> 2009-03-13
<160> 152
<170> FastSEQ for Windows Version 4.0
<210> 1
   <211> 1296
   <212> PRT
   <213> Clostridium botulinum
<400> 1
   Met Pro Phe Val Asn Lys Gln Phe Asn Tyr Lys Asp Pro Val Asn Gly
    1               5                  10                  15
   Val Asp Ile Ala Tyr Ile Lys Ile Pro Asn Ala Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile His Ala Gly His Arg Leu Tyr Gly Ile Ala Ile Asn Pro Asn
   225                 230                 235                 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
       290                 295                 300
   Gly Thr Thr Ala Ser Leu Gln Tyr Met Lys Asn Val Phe Lys Glu Lys
   305 310 315 320
   Tyr Leu Leu Ser Glu Asp Thr Ser Gly Lys Phe Ser Val Asp Lys Leu
   325 330 335
   Lys Phe Asp Lys Leu Tyr Lys Met Leu Thr Glu Ile Tyr Thr Glu Asp
   340 345 350
   Asn Phe Val Lys Phe Phe Lys Val Leu Asn Arg Lys Thr Tyr Leu Asn
   355 360 365
   Phe Asp Lys Ala Val Phe Lys Ile Asn Ile Val Pro Lys Val Asn Tyr
   370 375 380
   Thr Ile Tyr Asp Gly Phe Asn Leu Arg Asn Thr Asn Leu Ala Ala Asn
   385 390 395 400
   Phe Asn Gly Gln Asn Thr Glu Ile Asn Asn Met Asn Phe Thr Lys Leu
   405 410 415
   Lys Asn Phe Thr Gly Leu Phe Glu Phe Tyr Lys Leu Leu Cys Val Arg
   420 425 430
   Gly Ile Ile Thr Ser Lys Thr Lys Ser Leu Asp Lys Gly Tyr Asn Lys
   435 440 445
   Ala Leu Asn Asp Leu Cys Ile Lys Val Asn Asn Trp Asp Leu Phe Phe
   450 455 460
   Ser Pro Ser Glu Asp Asn Phe Thr Asn Asp Leu Asn Lys Gly Glu Glu
   465 470 475 480
   Ile Thr Ser Asp Thr Asn Ile Glu Ala Ala Glu Glu Asn Ile Ser Leu
   485 490 495
   Asp Leu Ile Gln Gln Tyr Tyr Leu Thr Phe Asn Phe Asp Asn Glu Pro
   500 505 510
   Glu Asn Ile Ser Ile Glu Asn Leu Ser Ser Asp Ile Ile Gly Gln Leu
   515 520 525
   Glu Leu Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Lys Ser Arg Ile Ala Leu Thr Asn Ser Val Asn Glu Ala Leu
   565 570 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
           835                 840                 845
   Ile Pro Phe Gln Leu Ser Lys Tyr Val Asp Asn Gln Arg Leu Leu Ser
       850                 855                 860
   Thr Phe Thr Glu Tyr Ile Lys Asn Ile Ile Asn Thr Ser Ile Leu Asn
   865                 870                 875                 880
   Leu Arg Tyr Glu Ser Asn His Leu Ile Asp Leu Ser Arg Tyr Ala Ser
                   885                 890                 895
   Lys Ile Asn Ile Gly Ser Lys Val Asn Phe Asp Pro Ile Asp Lys Asn
               900                 905                 910
   Gln Ile Gln Leu Phe Asn Leu Glu Ser Ser Lys Ile Glu Val Ile Leu
           915                 920                 925
   Lys Asn Ala Ile Val Tyr Asn Ser Met Tyr Glu Asn Phe Ser Thr Ser
       930                 935                 940
   Phe Trp Ile Arg Ile Pro Lys Tyr Phe Asn Ser Ile Ser Leu Asn Asn
   945                 950                 955                 960
   Glu Tyr Thr Ile Ile Asn Cys Met Glu Asn Asn Ser Gly Trp Lys Val
                   965                 970                 975
   Ser Leu Asn Tyr Gly Glu Ile Ile Trp Thr Leu Gln Asp Thr Gln Glu
               980                 985                 990
   Ile Lys Gln Arg Val Val Phe Lys Tyr Ser Gln Met Ile Asn Ile Ser
           995                 1000                1005
   Asp Tyr Ile Asn Arg Trp Ile Phe Val Thr Ile Thr Asn Asn Arg Leu
       1010                1015                1020
   Asn Asn Ser Lys Ile Tyr Ile Asn Gly Arg Leu Ile Asp Gln Lys Pro
   1025                1030                1035                1040
   Ile Ser Asn Leu Gly Asn Ile His Ala Ser Asn Asn Ile Met Phe Lys
                   1045                1050                1055
   Leu Asp Gly Cys Arg Asp Thr His Arg Tyr Ile Trp Ile Lys Tyr Phe
               1060                1065                1070
   Asn Leu Phe Asp Lys Glu Leu Asn Glu Lys Glu Ile Lys Asp Leu Tyr
           1075                1080                1085
   Asp Asn Gln Ser Asn Ser Gly Ile Leu Lys Asp Phe Trp Gly Asp Tyr
       1090                1095                1100
   Leu Gln Tyr Asp Lys Pro Tyr Tyr Met Leu Asn Leu Tyr Asp Pro Asn
   1105                1110                1115                1120
   Lys Tyr Val Asp Val Asn Asn Val Gly Ile Arg Gly Tyr Met Tyr Leu
                   1125                1130                1135
   Lys Gly Pro Arg Gly Ser Val Met Thr Thr Asn Ile Tyr Leu Asn Ser
               1140                1145                1150
   Ser Leu Tyr Arg Gly Thr Lys Phe Ile Ile Lys Lys Tyr Ala Ser Gly
           1155                1160                1165
   Asn Lys Asp Asn Ile Val Arg Asn Asn Asp Arg Val Tyr Ile Asn Val
       1170                1175                1180
   Val Val Lys Asn Lys Glu Tyr Arg Leu Ala Thr Asn Ala Ser Gln Ala
   1185                1190                1195                1200
   Gly Val Glu Lys Ile Leu Ser Ala Leu Glu Ile Pro Asp Val Gly Asn
                   1205                1210                1215
   Leu Ser Gln Val Val Val Met Lys Ser Lys Asn Asp Gln Gly Ile Thr
               1220                1225                1230
   Asn Lys Cys Lys Met Asn Leu Gln Asp Asn Asn Gly Asn Asp Ile Gly
           1235                1240                1245
   Phe Ile Gly Phe His Gln Phe Asn Asn Ile Ala Lys Leu Val Ala Ser
       1250                1255                1260
   Asn Trp Tyr Asn Arg Gln Ile Glu Arg Ser Ser Arg Thr Leu Gly Cys
   1265                1270                1275                1280
   Ser Trp Glu Phe Ile Pro Val Asp Asp Gly Trp Gly Glu Arg Pro Leu
                   1285                1290                1295
<210> 2
   <211> 1296
   <212> PRT
   <213> Clostridium botulinum
<400> 2
   Met Pro Phe Val Asn Lys Gln Phe Asn Tyr Lys Asp Pro Val Asn Gly
   1 5 10 15
   Val Asp Ile Ala Tyr Ile Lys Ile Pro Asn Ala Gly Gln Met Gln Pro
   20 25 30
   Val Lys Ala Phe Lys Ile His Asn Lys Ile Trp Val Ile Pro Glu Arg
   35 40 45
   Asp Thr Phe Thr Asn Pro Glu Glu Gly Asp Leu Asn Pro Pro Pro Glu
   50 55 60
   Ala Lys Gln Val Pro Val Ser Tyr Tyr Asp Ser Thr Tyr Leu Ser Thr
   65 70 75 80
   Asp Asn Glu Lys Asp Asn Tyr Leu Lys Gly Val Thr Lys Leu Phe Glu
   85 90 95
   Arg Ile Tyr Ser Thr Asp Leu Gly Arg Met Leu Leu Thr Ser Ile Val
   100 105 110
   Arg Gly Ile Pro Phe Trp Gly Gly Ser Thr Ile Asp Thr Glu Leu Lys
   115 120 125
   Val Ile Asp Thr Asn Cys Ile Asn Val Ile Gln Pro Asp Gly Ser Tyr
   130 135 140
   Arg Ser Glu Glu Leu Asn Leu Val Ile Ile Gly Pro Ser Ala Asp Ile
   145 150 155 160
   Ile Gln Phe Glu Cys Lys Ser Phe Gly His Asp Val Leu Asn Leu Thr
   165 170 175
   Arg Asn Gly Tyr Gly Ser Thr Gln Tyr Ile Arg Phe Ser Pro Asp Phe
   180 185 190
   Thr Phe Gly Phe Glu Glu Ser Leu Glu Val Asp Thr Asn Pro Leu Leu
   195 200 205
   Gly Ala Gly Lys Phe Ala Thr Asp Pro Ala Val Thr Leu Ala His Glu
   210 215 220
   Leu Ile His Ala Glu His Arg Leu Tyr Gly Ile Ala Ile Asn Pro Asn
   225 230 235 240
   Arg Val Phe Lys Val Asn Thr Asn Ala Tyr Tyr Glu Met Ser Gly Leu
   245 250 255
   Glu Val Ser Phe Glu Glu Leu Arg Thr Phe Gly Gly His Asp Ala Lys
   260 265 270
   Phe Ile Asp Ser Leu Gln Glu Asn Glu Phe Arg Leu Tyr Tyr Tyr Asn
   275 280 285
   Lys Phe Lys Asp Val Ala Ser Thr Leu Asn Lys Ala Lys Ser Ile Ile
   290 295 300
   Gly Thr Thr Ala Ser Leu Gln Tyr Met Lys Asn Val Phe Lys Glu Lys
   305 310 315 320
   Tyr Leu Leu Ser Glu Asp Thr Ser Gly Lys Phe Ser Val Asp Lys Leu
   325 330 335
   Lys Phe Asp Lys Leu Tyr Lys Met Leu Thr Glu Ile Tyr Thr Glu Asp
   340 345 350
   Asn Phe Val Asn Phe Phe Lys Val Ile Asn Arg Lys Thr Tyr Leu Asn
   355 360 365
   Phe Asp Lys Ala Val Phe Arg Ile Asn Ile Val Pro Asp Glu Asn Tyr
   370 375 380
   Thr Ile Lys Asp Gly Phe Asn Leu Lys Gly Ala Asn Leu Ser Thr Asn
   385 390 395 400
   Phe Asn Gly Gln Asn Thr Glu Ile Asn Ser Arg Asn Phe Thr Arg Leu
   405 410 415
   Lys Asn Phe Thr Gly Leu Phe Glu Phe Tyr Lys Leu Leu Cys Val Arg
   420 425 430
   Gly Ile Ile Pro Phe Lys Thr Lys Ser Leu Asp Glu Gly Tyr Asn Lys
   435 440 445
   Ala Leu Asn Asp Leu Cys Ile Lys Val Asn Asn Trp Asp Leu Phe Phe
   450 455 460
   Ser Pro Ser Glu Asp Asn Phe Thr Asn Asp Leu Asp Lys Val Glu Glu
   465 470 475 480
   Ile Thr Ala Asp Thr Asn Ile Glu Ala Ala Glu Glu Asn Ile Ser Leu
   485 490 495
   Asp Leu Ile Gln Gln Tyr Tyr Leu Thr Phe Asp Phe Asp Asn Glu Pro
   500 505 510
   Glu Asn Ile Ser Ile Glu Asn Leu Ser Ser Asp Ile Ile Gly Gln Leu
   515 520 525
   Glu Pro Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Asp Ser Arg Ile Ile Leu Thr Asn Ser Ala Glu Glu Ala Leu
   565 570 575
   Leu Lys Pro Asn Val Ala Tyr Thr Phe Phe Ser Ser Lys Tyr Val Lys
   580 585 590
   Lys Ile Asn Lys Ala Val Glu Ala Phe Met Phe Leu Asn Trp Ala Glu
   595 600 605
   Glu Leu Val Tyr Asp Phe Thr Asp Glu Thr Asn Glu Val Thr Thr Met
   610 615 620
   Asp Lys Ile Ala Asp Ile Thr Ile Ile Val Pro Tyr Ile Gly Pro Ala
   625 630 635 640
   Leu Asn Ile Gly Asn Met Leu Ser Lys Gly Glu Phe Val Glu Ala Ile
   645 650 655
   Ile Phe Thr Gly Val Val Ala Met Leu Glu Phe Ile Pro Glu Tyr Ala
   660 665 670
   Leu Pro Val Phe Gly Thr Phe Ala Ile Val Ser Tyr Ile Ala Asn Lys
   675 680 685
   Val Leu Thr Val Gln Thr Ile Asn Asn Ala Leu Ser Lys Arg Asn Glu
   690 695 700
   Lys Trp Asp Glu Val Tyr Lys Tyr Thr Val Thr Asn Trp Leu Ala Lys
   705 710 715 720
   Val Asn Thr Gln Ile Asp Leu Ile Arg Glu Lys Met Lys Lys Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Ser Ala Met Ile Asn Ile
   770 775 780
   Asn Lys Phe Leu Asp Gln Cys Ser Val Ser Tyr Leu Met Asn Ser Met
   785 790 795 800
   Ile Pro Tyr Ala Val Lys Arg Leu Lys Asp Phe Asp Ala Ser Val Arg
   805 810 815
   Asp Val Leu Leu Lys Tyr Ile Tyr Asp Asn Arg Gly Thr Leu Val Leu
   820 825 830
   Gln Val Asp Arg Leu Lys Asp Glu Val Asn Asn Thr Leu Ser Ala Asp
   835 840 845
   Ile Pro Phe Gln Leu Ser Lys Tyr Val Asp Asn Lys Lys Leu Leu Ser
   850 855 860
   Thr Phe Thr Glu Tyr Ile Lys Asn Ile Val Asn Thr Ser Ile Leu Ser
   865 870 875 880
   Ile Val Tyr Lys Lys Asp Asp Leu Ile Asp Leu Ser Arg Tyr Gly Ala
   885 890 895
   Lys Ile Asn Ile Gly Asp Arg Val Tyr Tyr Asp Ser Ile Asp Lys Asn
   900 905 910
   Gln Ile Lys Leu Ile Asn Leu Glu Ser Ser Thr Ile Glu Val Ile Leu
   915 920 925
   Lys Asn Ala Ile Val Tyr Asn Ser Met Tyr Glu Asn Phe Ser Thr Ser
   930 935 940
   Phe Trp Ile Lys Ile Pro Lys Tyr Phe Ser Lys Ile Asn Leu Asn Asn
   945 950 955 960
   Glu Tyr Thr Ile Ile Asn Cys Ile Glu Asn Asn Ser Gly Trp Lys Val
   965 970 975
   Ser Leu Asn Tyr Gly Glu Ile Ile Trp Thr Leu Gln Asp Asn Lys Gln
   980 985 990
   Asn Ile Gln Arg Val Val Phe Lys Tyr Ser Gln Met Val Asn Ile Ser
   995 1000 1005
   Asp Tyr Ile Asn Arg Trp Ile Phe Val Thr Ile Thr Asn Asn Arg Leu
   1010 1015 1020
   Thr Lys Ser Lys Ile Tyr Ile Asn Gly Arg Leu Ile Asp Gln Lys Pro
   1025                1030                1035                1040
   Ile Ser Asn Leu Gly Asn Ile His Ala Ser Asn Lys Ile Met Phe Lys
                   1045                1050                1055
   Leu Asp Gly Cys Arg Asp Pro Arg Arg Tyr Ile Met Ile Lys Tyr Phe
               1060                1065                1070
   Asn Leu Phe Asp Lys Glu Leu Asn Glu Lys Glu Ile Lys Asp Leu Tyr
           1075                1080                1085
   Asp Ser Gln Ser Asn Ser Gly Ile Leu Lys Asp Phe Trp Gly Asn Tyr
       1090                1095                1100
   Leu Gln Tyr Asp Lys Pro Tyr Tyr Met Leu Asn Leu Phe Asp Pro Asn
   1105                1110                1115                1120
   Lys Tyr Val Asp Val Asn Asn Ile Gly Ile Arg Gly Tyr Met Tyr Leu
                   1125                1130                1135
   Lys Gly Pro Arg Gly Ser Val Val Thr Thr Asn Ile Tyr Leu Asn Ser
               1140                1145                1150
   Thr Leu Tyr Glu Gly Thr Lys Phe Ile Ile Lys Lys Tyr Ala Ser Gly
           1155                1160                1165
   Asn Glu Asp Asn Ile Val Arg Asn Asn Asp Arg Val Tyr Ile Asn Val
       1170                1175                1180
   Val Val Lys Asn Lys Glu Tyr Arg Leu Ala Thr Asn Ala Ser Gln Ala
   1185                1190                1195                1200
   Gly Val Glu Lys Ile Leu Ser Ala Leu Glu Ile Pro Asp Val Gly Asn
                   1205                1210                1215
   Leu Ser Gln Val Val Val Met Lys Ser Lys Asp Asp Gln Gly Ile Arg
               1220                1225                1230
   Asn Lys Cys Lys Met Asn Leu Gln Asp Asn Asn Gly Asn Asp Ile Gly
           1235                1240                1245
   Phe Ile Gly Phe His Leu Tyr Asp Asn Ile Ala Lys Leu Val Ala Ser
       1250                1255                1260
   Asn Trp Tyr Asn Arg Gln Val Gly Lys Ala Ser Arg Thr Phe Gly Cys
   1265                1270                1275                1280
   Ser Trp Glu Phe Ile Pro Val Asp Asp Gly Trp Gly Glu Ser Ser Leu
                   1285                1290                1295
<210> 3
   <211> 1292
   <212> PRT
   <213> Clostridium botulinum
<400> 3
   Met Pro Phe Val Asn Lys Pro Phe Asn Tyr Arg Asp Pro Gly Asn Gly
    1               5                  10                  15
   Val Asp Ile Ala Tyr Ile Lys Ile Pro Asn Ala Gly Gln Met Gln Pro
               20                  25                  30
   Val Lys Ala Phe Lys Ile His Glu Gly Val Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Ile Lys Leu Phe Asp
                   85                  90                  95
   Arg Ile Tyr Ser Thr Gly Leu Gly Arg Met Leu Leu Ser Phe Ile Val
               100                 105                 110
   Lys Gly Ile Pro Phe Trp Gly Gly Ser Thr Ile Asp Thr Glu Leu Lys
           115                 120                 125
   Val Ile Asp Thr Asn Cys Ile Asn Val Ile Glu Pro Gly Gly Ser Tyr
       130                 135                 140
   Arg Ser Glu Glu Leu Asn Leu Val Ile Thr Gly Pro Ser Ala Asp Ile
   145                 150                 155                 160
   Ile Gln Phe Glu Cys Lys Ser Phe Gly His Asp Val Phe Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
   195 200 205
   Gly Ala Gly Thr Phe Ala Thr Asp Pro Ala Val Thr Leu Ala His Glu
   210 215 220
   Leu Ile His Ala Ala His Arg Leu Tyr Gly Ile Ala Ile Asn Pro Asn
   225 230 235 240
   Arg Val Leu Lys Val Lys Thr Asn Ala Tyr Tyr Glu Met Ser Gly Leu
   245 250 255
   Glu Val Ser Phe Glu Glu Leu Arg Thr Phe Gly Gly Asn Asp Thr Asn
   260 265 270
   Phe Ile Asp Ser Leu Trp Gln Lys Lys Phe Ser Arg Asp Ala Tyr Asp
   275 280 285
   Asn Leu Gln Asn Ile Ala Arg Ile Leu Asn Glu Ala Lys Thr Ile Val
   290 295 300
   Gly Thr Thr Thr Pro Leu Gln Tyr Met Lys Asn Ile Phe Ile Arg Lys
   305 310 315 320
   Tyr Phe Leu Ser Glu Asp Ala Ser Gly Lys Ile Ser Val Asn Lys Ala
   325 330 335
   Ala Phe Lys Glu Phe Tyr Arg Val Leu Thr Arg Gly Phe Thr Glu Leu
   340 345 350
   Glu Phe Val Asn Pro Phe Lys Val Ile Asn Arg Lys Thr Tyr Leu Asn
   355 360 365
   Phe Asp Lys Ala Val Phe Arg Ile Asn Ile Val Pro Asp Glu Asn Tyr
   370 375 380
   Thr Ile Asn Glu Gly Phe Asn Leu Glu Gly Ala Asn Ser Asn Gly Gln
   385 390 395 400
   Asn Thr Glu Ile Asn Ser Arg Asn Phe Thr Arg Leu Lys Asn Phe Thr
   405 410 415
   Gly Leu Phe Glu Phe Tyr Lys Leu Leu Cys Val Arg Gly Ile Ile Pro
   420 425 430
   Phe Lys Thr Lys Ser Leu Asp Glu Gly Tyr Asn Lys Ala Leu Asn Tyr
   435 440 445
   Leu Cys Ile Lys Val Asn Asn Trp Asp Leu Phe Phe Ser Pro Ser Glu
   450 455 460
   Asp Asn Phe Thr Asn Asp Leu Asp Lys Val Glu Glu Ile Thr Ala Asp
   465 470 475 480
   Thr Asn Ile Glu Ala Ala Glu Glu Asn Ile Ser Ser Asp Leu Ile Gln
   485 490 495
   Gln Tyr Tyr Leu Thr Phe Asp Phe Asp Asn Glu Pro Glu Asn Ile Ser
   500 505 510
   Ile Glu Asn Leu Ser Ser Asp Ile Ile Gly Gln Leu Glu Pro Met Pro
   515 520 525
   Asn Ile Glu Arg Phe Pro Asn Gly Lys Lys Tyr Glu Leu Asp Lys Tyr
   530 535 540
   Thr Met Phe His Tyr Leu Arg Ala Gln Glu Phe Glu His Gly Asp Ser
   545 550 555 560
   Arg Ile Ile Leu Thr Asn Ser Ala Glu Glu Ala Leu Leu Lys Pro Asn
   565 570 575
   Val Ala Tyr Thr Phe Phe Ser Ser Lys Tyr Val Lys Lys Ile Asn Lys
   580 585 590
   Ala Val Glu Ala Val Ile Phe Leu Ser Trp Ala Glu Glu Leu Val Tyr
   595 600 605
   Asp Phe Thr Asp Glu Thr Asn Glu Val Thr Thr Met Asp Lys Ile Ala
   610 615 620
   Asp Ile Thr Ile Ile Val Pro Tyr Ile Gly Pro Ala Leu Asn Ile Gly
   625 630 635 640
   Asn Met Val Ser Lys Gly Glu Phe Val Glu Ala Ile Leu Phe Thr Gly
   645 650 655
   Val Val Ala Leu Leu Glu Phe Ile Pro Glu Tyr Ser Leu Pro Val Phe
   660 665 670
   Gly Thr Phe Ala Ile Val Ser Tyr Ile Ala Asn Lys Val Leu Thr Val
   675 680 685
   Gln Thr Ile Asn Asn Ala Leu Ser Lys Arg Asn Glu Lys Trp Asp Glu
   690 695 700
   Val Tyr Lys Tyr Thr Val Thr Asn Trp Leu Ala Lys Val Asn Thr Gln
   705 710 715 720
   Ile Asp Leu Ile Arg Glu Lys Met Lys Lys Ala Leu Glu Asn Gln Ala
   725 730 735
   Glu Ala Thr Arg Ala Ile Ile Asn Tyr Gln Tyr Asn Gln Tyr Thr Glu
   740 745 750
   Glu Glu Lys Asn Asn Ile Asn Phe Asn Ile Asp Asp Leu Ser Ser Lys
   755 760 765
   Leu Asn Arg Ser Ile Asn Arg Ala Met Ile Asn Ile Asn Lys Phe Leu
   770 775 780
   Asp Gln Cys Ser Val Ser Tyr Leu Met Asn Ser Met Ile Pro Tyr Ala
   785 790 795 800
   Val Lys Arg Leu Lys Asp Phe Asp Ala Ser Val Arg Asp Val Leu Leu
   805 810 815
   Lys Tyr Ile Tyr Asp Asn Arg Gly Thr Leu Ile Leu Gln Val Asp Arg
   820 825 830
   Leu Lys Asp Glu Val Asn Asn Thr Leu Ser Ala Asp Ile Pro Phe Gln
   835 840 845
   Leu Ser Lys Tyr Val Asn Asp Lys Lys Leu Leu Ser Thr Phe Thr Glu
   850 855 860
   Tyr Ile Lys Asn Ile Val Asn Thr Ser Ile Leu Ser Ile Val Tyr Lys
   865 870 875 880
   Lys Asp Asp Leu Ile Asp Leu Ser Arg Tyr Gly Ala Lys Ile Asn Ile
   885 890 895
   Gly Asp Arg Val Tyr Tyr Asp Ser Ile Asp Lys Asn Gln Ile Lys Leu
   900 905 910
   Ile Asn Leu Glu Ser Ser Thr Ile Glu Val Ile Leu Lys Asn Ala Ile
   915 920 925
   Val Tyr Asn Ser Met Tyr Glu Asn Phe Ser Thr Ser Phe Trp Ile Lys
   930 935 940
   Ile Pro Lys Tyr Phe Ser Lys Ile Asn Leu Asn Asn Glu Tyr Thr Ile
   945 950 955 960
   Ile Asn Cys Ile Glu Asn Asn Ser Gly Trp Lys Val Ser Leu Asn Tyr
   965 970 975
   Gly Glu Ile Ile Trp Thr Leu Gln Asp Asn Lys Gln Asn Ile Gln Arg
   980 985 990
   Val Val Phe Lys Tyr Ser Gln Met Val Asn Ile Ser Asp Tyr Ile Asn
   995 1000 1005
   Arg Trp Met Phe Val Thr Ile Thr Asn Asn Arg Leu Thr Lys Ser Lys
   1010 1015 1020
   Ile Tyr Ile Asn Gly Arg Leu Ile Asp Gln Lys Pro Ile Ser Asn Leu
   1025 1030 1035 1040
   Gly Asn Ile His Ala Ser Asn Lys Ile Met Phe Lys Leu Asp Gly Cys
   1045 1050 1055
   Arg Asp Pro Arg Arg Tyr Ile Met Ile Lys Tyr Phe Asn Leu Phe Asp
   1060 1065 1070
   Lys Glu Leu Asn Glu Lys Glu Ile Lys Asp Leu Tyr Asp Ser Gln Ser
   1075 1080 1085
   Asn Pro Gly Ile Leu Lys Asp Phe Trp Gly Asn Tyr Leu Gln Tyr Asp
   1090 1095 1100
   Lys Pro Tyr Tyr Met Leu Asn Leu Phe Asp Pro Asn Lys Tyr Val Asp
   1105 1110 1115 1120
   Val Asn Asn Ile Gly Ile Arg Gly Tyr Met Tyr Leu Lys Gly Pro Arg
   1125 1130 1135
   Gly Ser Val Met Thr Thr Asn Ile Tyr Leu Asn Ser Thr Leu Tyr Met
   1140 1145 1150
   Gly Thr Lys Phe Ile Ile Lys Lys Tyr Ala Ser Gly Asn Glu Asp Asn
   1155 1160 1165
   Ile Val Arg Asn Asn Asp Arg Val Tyr Ile Asn Val Val Val Lys Asn
   1170 1175 1180
   Lys Glu Tyr Arg Leu Ala Thr Asn Ala Ser Gln Ala Gly Val Glu Lys
   1185 1190 1195 1200
   Ile Leu Ser Ala Leu Glu Ile Pro Asp Val Gly Asn Leu Ser Gln Val
   1205 1210 1215
   Val Val Met Lys Ser Lys Asp Asp Gln Gly Ile Arg Asn Lys Cys Lys
   1220 1225 1230
   Met Asn Leu Gln Asp Asn Asn Gly Asn Asp Ile Gly Phe Val Gly Phe
           1235                1240                1245
   His Leu Tyr Asp Asn Ile Ala Lys Leu Val Ala Ser Asn Trp Tyr Asn
       1250                1255                1260
   Arg Gln Val Gly Lys Ala Ser Arg Thr Phe Gly Cys Ser Trp Glu Phe
   1265                1270                1275                1280
   Ile Pro Val Asp Asp Gly Trp Gly Glu Ser Ser Leu
                   1285                1290
<210> 4
   <211> 1296
   <212> PRT
   <213> Clostridium botulinum
<400> 4
   Met Pro Leu Val Asn Gln Gln Ile Asn Tyr Tyr Asp Pro Val Asn Gly
   1 5 10 15
   Val Asp Ile Ala Tyr Ile Lys Ile Pro Asn Ala Gly Lys Met Gln Pro
   20 25 30
   Val Lys Ala Phe Lys Ile His Asn Lys Val Trp Val Ile Pro Glu Arg
   35 40 45
   Asp Ile Phe Thr Asn Pro Glu Glu Val Asp Leu Asn Pro Pro Pro Glu
   50 55 60
   Ala Lys Gln Val Pro Ile Ser Tyr Tyr Asp Ser Ala Tyr Leu Ser Thr
   65 70 75 80
   Asp Asn Glu Lys Asp Asn Tyr Leu Lys Gly Val Ile Lys Leu Phe Glu
   85 90 95
   Arg Ile Tyr Ser Thr Asp Leu Gly Arg Met Leu Leu Ile Ser Ile Val
   100 105 110
   Arg Gly Ile Pro Phe Trp Gly Gly Gly Lys Ile Asp Thr Glu Leu Lys
   115 120 125
   Val Ile Asp Thr Asn Cys Ile Asn Ile Ile Gln Leu Asp Asp Ser Tyr
   130 135 140
   Arg Ser Glu Glu Leu Asn Leu Ala Ile Ile Gly Pro Ser Ala Asn Ile
   145 150 155 160
   Ile Glu Ser Gln Cys Ser Ser Phe Arg Asp Asp Val Leu Asn Leu Thr
   165 170 175
   Arg Asn Gly Tyr Gly Ser Thr Gln Tyr Ile Arg Phe Ser Pro Asp Phe
   180 185 190
   Thr Val Gly Phe Glu Glu Ser Leu Glu Val Asp Thr Asn Pro Leu Leu
   195 200 205
   Gly Ala Gly Lys Phe Ala Gln Asp Pro Ala Val Ala Leu Ala His Glu
   210 215 220
   Leu Ile His Ala Glu His Arg Leu Tyr Gly Ile Ala Ile Asn Thr Asn
   225 230 235 240
   Arg Val Phe Lys Val Asn Thr Asn Ala Tyr Tyr Glu Met Ala Gly Leu
   245 250 255
   Glu Val Ser Leu Glu Glu Leu Ile Thr Phe Gly Gly Asn Asp Ala Lys
   260 265 270
   Phe Ile Asp Ser Leu Gln Lys Lys Glu Phe Ser Leu Tyr Tyr Tyr Asn
   275 280 285
   Lys Phe Lys Asp Ile Ala Ser Thr Leu Asn Lys Ala Lys Ser Ile Val
   290 295 300
   Gly Thr Thr Ala Ser Leu Gln Tyr Met Lys Asn Val Phe Lys Glu Lys
   305 310 315 320
   Tyr Leu Leu Ser Glu Asp Ala Thr Gly Lys Phe Leu Val Asp Arg Leu
   325 330 335
   Lys Phe Asp Glu Leu Tyr Lys Leu Leu Thr Glu Ile Tyr Thr Glu Asp
   340 345 350
   Asn Phe Val Lys Phe Phe Lys Val Leu Asn Arg Lys Thr Tyr Leu Asn
   355 360 365
   Phe Asp Lys Ala Val Phe Lys Ile Asn Ile Val Pro Asp Val Asn Tyr
   370 375 380
   Thr Ile His Asp Gly Phe Asn Leu Arg Asn Thr Asn Leu Ala Ala Asn
   385 390 395 400
   Phe Asn Gly Gln Asn Ile Glu Ile Asn Asn Lys Asn Phe Asp Lys Leu
   405 410 415
   Lys Asn Phe Thr Gly Leu Phe Glu Phe Tyr Lys Leu Leu Cys Val Arg
   420 425 430
   Gly Ile Ile Thr Ser Lys Thr Lys Ser Leu Asp Glu Gly Tyr Asn Lys
   435 440 445
   Ala Leu Asn Glu Leu Cys Ile Lys Val Asn Asn Trp Asp Leu Phe Phe
   450 455 460
   Ser Pro Ser Glu Asp Asn Phe Thr Asn Asp Leu Asp Lys Val Glu Glu
   465 470 475 480
   Ile Thr Ser Asp Thr Asn Ile Glu Ala Ala Glu Glu Asn Ile Ser Leu
   485 490 495
   Asp Leu Ile Gln Gln Tyr Tyr Leu Asn Phe Asn Phe Asp Asn Glu Pro
   500 505 510
   Glu Asn Thr Ser Ile Glu Asn Leu Ser Ser Asp Ile Ile Gly Gln Leu
   515 520 525
   Glu Pro Met Pro Asn Ile Glu Arg Phe Pro Asn Gly Lys Lys Tyr Glu
   530 535 540
   Leu Asn Lys Tyr Thr Met Phe His Tyr Leu Arg Ala Gln Glu Phe Lys
   545 550 555 560
   His Ser Asn Ser Arg Ile Ile Leu Thr Asn Ser Ala Lys Glu Ala Leu
   565 570 575
   Leu Lys Pro Asn Ile Val Tyr Thr Phe Phe Ser Ser Lys Tyr Ile Lys
   580 585 590
   Ala Ile Asn Lys Ala Val Glu Ala Val Thr Phe Val Asn Trp Ile Glu
   595 600 605
   Asn Leu Val Tyr Asp Phe Thr Asp Glu Thr Asn Glu Val Ser Thr Met
   610 615 620
   Asp Lys Ile Ala Asp Ile Thr Ile Val Ile Pro Tyr Ile Gly Pro Ala
   625 630 635 640
   Leu Asn Ile Gly Asn Met Ile Tyr Lys Gly Glu Phe Val Glu Ala Ile
   645 650 655
   Ile Phe Ser Gly Ala Val Ile Leu Leu Glu Ile Val Pro Glu Ile Ala
   660 665 670
   Leu Pro Val Leu Gly Thr Phe Ala Leu Val Ser Tyr Val Ser Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Ile
   705 710 715 720
   Val Asn Thr Gln Ile Asn Leu Ile Arg Glu Lys Met Lys Lys Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Ser Ala Met Ile Asn Ile
   770 775 780
   Asn Lys Phe Leu Asp Gln Cys Ser Val Ser Tyr Leu Met Asn Ser Met
   785 790 795 800
   Ile Pro Tyr Ala Val Lys Arg Leu Lys Asp Phe Asp Ala Ser Val Arg
   805 810 815
   Asp Val Leu Leu Lys Tyr Ile Tyr Asp Asn Arg Gly Thr Leu Ile Gly
   820 825 830
   Gln Val Asn Arg Leu Lys Asp Lys Val Asn Asn Thr Leu Ser Ala Asp
   835 840 845
   Ile Pro Phe Gln Leu Ser Lys Tyr Val Asp Asn Lys Lys Leu Leu Ser
   850 855 860
   Thr Phe Thr Glu Tyr Ile Lys Asn Ile Thr Asn Ala Ser Ile Leu Ser
   865 870 875 880
   Ile Val Tyr Lys Asp Asp Asp Leu Ile Asp Leu Ser Arg Tyr Gly Ala
   885 890 895
   Glu Ile Tyr Asn Gly Asp Lys Val Tyr Tyr Asn Ser Ile Asp Lys Asn
   900 905 910
   Gln Ile Arg Leu Ile Asn Leu Glu Ser Ser Thr Ile Glu Val Ile Leu
           915                 920                 925
   Lys Lys Ala Ile Val Tyr Asn Ser Met Tyr Glu Asn Phe Ser Thr Ser
       930                 935                 940
   Phe Trp Ile Arg Ile Pro Lys Tyr Phe Asn Ser Ile Ser Leu Asn Asn
   945                 950                 955                 960
   Glu Tyr Thr Ile Ile Asn Cys Met Glu Asn Asn Ser Gly Trp Lys Val
                   965                 970                 975
   Ser Leu Asn Tyr Gly Glu Ile Ile Trp Thr Phe Gln Asp Thr Gln Glu
               980                 985                 990
   Ile Lys Gln Arg Val Val Phe Lys Tyr Ser Gln Met Ile Asn Ile Ser
           995                 1000                1005
   Asp Tyr Ile Asn Arg Trp Ile Phe Val Thr Ile Thr Asn Asn Arg Ile
       1010                1015                1020
   Thr Lys Ser Lys Ile Tyr Ile Asn Gly Arg Leu Ile Asp Gln Lys Pro
   1025                1030                1035                1040
   Ile Ser Asn Leu Gly Asn Ile His Ala Ser Asn Lys Ile Met Phe Lys
                   1045                1050                1055
   Leu Asp Gly Cys Arg Asp Pro His Arg Tyr Ile Val Ile Lys Tyr Phe
               1060                1065                1070
   Asn Leu Phe Asp Lys Glu Leu Ser Glu Lys Glu Ile Lys Asp Leu Tyr
           1075                1080                1085
   Asp Asn Gln Ser Asn Ser Gly Ile Leu Lys Asp Phe Trp Gly Asp Tyr
       1090                1095                1100
   Leu Gln Tyr Asp Lys Ser Tyr Tyr Met Leu Asn Leu Tyr Asp Pro Asn
   1105                1110                1115                1120
   Lys Tyr Val Asp Val Asn Asn Val Gly Ile Arg Gly Tyr Met Tyr Leu
                   1125                1130                1135
   Lys Gly Pro Arg Asp Asn Val Met Thr Thr Asn Ile Tyr Leu Asn Ser
               1140                1145                1150
   Ser Leu Tyr Met Gly Thr Lys Phe Ile Ile Lys Lys Tyr Ala Ser Gly
           1155                1160                1165
   Asn Lys Asp Asn Ile Val Arg Asn Asn Asp Arg Val Tyr Ile Asn Val
       1170                1175                1180
   Val Val Lys Asn Lys Glu Tyr Arg Leu Ala Thr Asn Ala Ser Gln Ala
   1185                1190                1195                1200
   Gly Val Glu Lys Ile Leu Ser Ala Leu Glu Ile Pro Asp Val Gly Asn
                   1205                1210                1215
   Leu Ser Gln Val Val Val Met Lys Ser Lys Asn Asp Gln Gly Ile Thr
               1220                1225                1230
   Asn Lys Cys Lys Met Asn Leu Gln Asp Asn Asn Gly Asn Asp Ile Gly
           1235                1240                1245
   Phe Ile Gly Phe His Gln Phe Asn Asn Ile Ala Lys Leu Val Ala Ser
       1250                1255                1260
   Asn Trp Tyr Asn Arg Gln Ile Glu Arg Ser Ser Arg Thr Leu Gly Cys
   1265                1270                1275                1280
   Ser Trp Glu Phe Ile Pro Val Asp Asp Gly Trp Arg Glu Arg Pro Leu
                   1285                1290                1295
<210> 5
   <211> 206
   <212> PRT
   <213> Homo sapiens
<400> 5
   Met Ala Glu Asp Ala Asp Met Arg Asn Glu Leu Glu Glu Met Gln Arg
    1               5                  10                  15
   Arg Ala Asp Gln Leu Ala Asp Glu Ser Leu Glu Ser Thr Arg Arg Met
               20                  25                  30
   Leu Gln Leu Val Glu Glu Ser Lys Asp Ala Gly Ile Arg Thr Leu Val
           35                  40                  45
   Met Leu Asp Glu Gln Gly Glu Gln Leu Asp Arg Val Glu Glu Gly Met
       50                  55                  60
   Asn His Ile Asn Gln Asp Met Lys Glu Ala Glu Lys Asn Leu Lys Asp
   65                  70                  75                  80
   Leu Gly Lys Cys Cys Gly Leu Phe Ile Cys Pro Cys Asn Lys Leu Lys
                   85                  90                  95
   Ser Ser Asp Ala Tyr Lys Lys Ala Trp Gly Asn Asn Gln Asp Gly Val
               100                 105                 110
   Val Ala Ser Gln Pro Ala Arg Val Val Asp Glu Arg Glu Gln Met Ala
           115                 120                 125
   Ile Ser Gly Gly Phe Ile Arg Arg Val Thr Asn Asp Ala Arg Glu Asn
       130                 135                 140
   Glu Met Asp Glu Asn Leu Glu Gln Val Ser Gly Ile Ile Gly Asn Leu
   145                 150                 155                 160
   Arg His Met Ala Leu Asp Met Gly Asn Glu Ile Asp Thr Gln Asn Arg
                   165                 170                 175
   Gln Ile Asp Arg Ile Met Glu Lys Ala Asp Ser Asn Lys Thr Arg Ile
               180                 185                 190
   Asp Glu Ala Asn Gln Arg Ala Thr Lys Met Leu Gly Ser Gly
           195                 200                 205
<210> 6
   <211> 206
   <212> PRT
   <213> Homo sapiens
<400> 6
<210> 7
   <211> 206
   <212> PRT
   <213> Macaca mulatta
<400> 7
<210> 8
   <211> 206
   <212> PRT
   <213> Rattus norvegicus
<400> 8
   Met Ala Glu Asp Ala Asp Met Arg Asn Glu Leu Glu Glu Met Gln Arg
    1               5                  10                  15
   Arg Ala Asp Gln Leu Ala Asp Glu Ser Leu Glu Ser Thr Arg Arg Met
               20                  25                  30
   Leu Gln Leu Val Glu Glu Ser Lys Asp Ala Gly Ile Arg Thr Leu Val
           35                  40                  45
   Met Leu Asp Glu Gln Gly Glu Gln Leu Asp Arg Val Glu Glu Gly Met
       50                  55                  60
   Asn His Ile Asn Gln Asp Met Lys Glu Ala Glu Lys Asn Leu Lys Asp
   65                  70                  75                  80
   Leu Gly Lys Cys Cys Gly Leu Phe Ile Cys Pro Cys Asn Lys Leu Lys
                   85                  90                  95
   Ser Ser Asp Ala Tyr Lys Lys Ala Trp Gly Asn Asn Gln Asp Gly Val
               100                 105                 110
   Val Ala Ser Gln Pro Ala Arg Val Val Asp Glu Arg Glu Gln Met Ala
           115                 120                 125
   Ile Ser Gly Gly Phe Ile Arg Arg Val Thr Asn Asp Ala Arg Glu Asn
       130                 135                 140
   Glu Met Asp Glu Asn Leu Glu Gln Val Ser Gly Ile Ile Gly Asn Leu
   145                 150                 155                 160
   Arg His Met Ala Leu Asp Met Gly Asn Glu Ile Asp Thr Gln Asn Arg
                   165                 170                 175
   Gln Ile Asp Arg Ile Met Glu Lys Ala Asp Ser Asn Lys Thr Arg Ile
               180                 185                 190
   Asp Glu Ala Asn Gln Arg Ala Thr Lys Met Leu Gly Ser Gly
           195                 200                 205
<210> 9
   <211> 206
   <212> PRT
   <213> Rattus norvegicus
<400> 9
<210> 10
   <211> 206
   <212> PRT
   <213> Mus musculus
<400> 10
<210> 11
   <211> 206
   <212> PRT
   <213> Gallus gallus
<400> 11
<210> 12
   <211> 204
   <212> PRT
   <213> Carassius auratus
<400> 12
   Met Ala Glu Asp Ala Asp Met Arg Asn Glu Leu Ser Asp Met Gln Gln
    1              5                   10                  15
   Arg Ala Asp Gln Leu Ala Asp Glu Ser Leu Glu Ser Thr Arg Arg Met
               20                  25                  30
   Leu Gln Leu Val Glu Glu Ser Lys Asp Ala Gly Ile Arg Thr Leu Val
           35                  40                  45
   Met Leu Asp Glu Gln Gly Glu Gln Leu Glu Arg Ile Glu Glu Gly Met
       50                  55                  60
   Asp Gln Ile Asn Lys Asp Met Lys Asp Ala Glu Lys Asn Leu Asn Asp
   65                  70                  75                  80
   Leu Gly Lys Phe Cys Gly Leu Cys Ser Cys Pro Cys Asn Lys Met Lys
                   85                  90                  95
   Ser Gly Gly Ser Lys Ala Trp Gly Asn Asn Gln Asp Gly Val Val Ala
               100                 105                 110
   Ser Gln Pro Ala Arg Val Val Asp Glu Arg Glu Gln Met Ala Ile Ser
           115                 120                 125
   Gly Gly Phe Ile Arg Arg Val Thr Asp Asp Ala Arg Glu Asn Glu Met
       130                 135                 140
   Asp Glu Asn Leu Glu Gln Val Gly Gly Ile Ile Gly Asn Leu Arg His
   145                 150                 155                 160
   Met Ala Leu Asp Met Gly Asn Glu Ile Asp Thr Gln Asn Arg Gln Ile
                   165                 170                 175
   Asp Arg Ile Met Glu Lys Ala Asp Ser Asn Lys Thr Arg Ile Asp Glu
               180                 185                 190
   Ala Asn Gln Arg Ala Thr Lys Met Leu Gly Ser Gly
           195                 200
<210> 13
   <211> 203
   <212> PRT
   <213> Carassius auratus
<400> 13
   Met Ala Asp Glu Ala Asp Met Arg Asn Glu Leu Thr Asp Met Gln Ala
    1               5                  10                  15
   Arg Ala Asp Gln Leu Gly Asp Glu Ser Leu Glu Ser Thr Arg Arg Met
               20                  25                  30
   Leu Gln Leu Val Glu Glu Ser Lys Asp Ala Gly Ile Arg Thr Leu Val
           35                  40                  45
   Met Leu Asp Glu Gln Gly Glu Gln Leu Glu Arg Ile Glu Glu Gly Met
       50                  55                  60
   Asp Gln Ile Asn Lys Asp Met Lys Glu Ala Glu Lys Asn Leu Thr Asp
   65                  70                  75                  80
   Leu Gly Asn Leu Cys Gly Leu Cys Pro Cys Pro Cys Asn Lys Leu Lys
                   85                  90                  95
   Gly Gly Gly Gln Ser Trp Gly Asn Asn Gln Asp Gly Val Val Ser Ser
               100                 105                 110
   Gln Pro Ala Arg Val Val Asp Glu Arg Glu Gln Met Ala Ile Ser Gly
           115                 120                 125
   Gly Phe Ile Arg Arg Val Thr Asn Asp Ala Arg Glu Asn Glu Met Asp
       130                 135                 140
   Glu Asn Leu Glu Gln Val Gly Ser Ile Ile Gly Asn Leu Arg His Met
   145                 150                 155                 160
   Ala Leu Asp Met Gly Asn Glu Ile Asp Thr Gln Asn Arg Gln Ile Asp
                   165                 170                 175
   Arg Ile Met Asp Met Ala Asp Ser Asn Lys Thr Arg Ile Asp Glu Ala
               180                 185                 190
   Asn Gln Arg Ala Thr Lys Met Leu Gly Ser Gly
           195                 200
<210> 14
   <211> 204
   <212> PRT
   <213> Danio rerio
<400> 14
   Met Ala Glu Asp Ser Asp Met Arg Asn Glu Leu Ala Asp Met Gln Gln
    1               5                  10                  15
   Arg Ala Asp Gln Leu Ala Asp Glu Ser Leu Glu Ser Thr Arg Arg Met
               20                  25                  30
   Leu Gln Leu Val Glu Glu Ser Lys Asp Ala Gly Ile Arg Thr Leu Val
           35                  40                  45
   Met Leu Asp Glu Gln Gly Glu Gln Leu Glu Arg Ile Glu Glu Gly Met
       50                  55                  60
   Asp Gln Ile Asn Lys Asp Met Lys Asp Ala Glu Lys Asn Leu Asn Asp
   65                  70                  75                  80
   Leu Gly Lys Phe Cys Gly Leu Cys Ser Cys Pro Cys Asn Lys Met Lys
                   85                  90                  95
   Ser Gly Ala Ser Lys Ala Trp Gly Asn Asn Gln Asp Gly Val Val Ala
               100                 105                 110
   Ser Gln Pro Ala Arg Val Val Asp Glu Arg Glu Gln Met Ala Ile Ser
           115                 120                 125
   Gly Gly Phe Ile Arg Arg Val Thr Asp Asp Ala Arg Glu Asn Glu Met
       130                 135                 140
   Asp Glu Asn Leu Glu Gln Val Gly Gly Ile Ile Gly Asn Leu Arg His
   145                 150                 155                 160
   Met Ala Leu Asp Met Gly Asn Glu Ile Asp Thr Gln Asn Arg Gln Ile
                   165                 170                 175
   Asp Arg Ile Met Glu Lys Ala Asp Ser Asn Lys Thr Arg Ile Asp Glu
               180                 185                 190
   Ala Asn Gln Arg Ala Thr Lys Met Leu Gly Ser Gly
           195                 200
<210> 15
   <211> 203
   <212> PRT
   <213> Danio rerio
<400> 15
   Met Ala Asp Glu Ser Asp Met Arg Asn Glu Leu Asn Asp Met Gln Ala
    1               5                  10                  15
   Arg Ala Asp Gln Leu Gly Asp Glu Ser Leu Glu Ser Thr Arg Arg Met
               20                  25                  30
   Leu Gln Leu Val Glu Glu Ser Lys Asp Ala Gly Ile Arg Thr Leu Val
           35                  40                  45
   Met Leu Asp Glu Gln Gly Glu Gln Leu Glu Arg Ile Glu Glu Gly Met
       50                  55                  60
   Asp Gln Ile Asn Lys Asp Met Lys Glu Ala Glu Lys Asn Leu Thr Asp
   65                  70                  75                  80
   Leu Gly Asn Leu Cys Gly Leu Cys Pro Cys Pro Cys Asn Lys Leu Lys
                   85                  90                  95
   Gly Gly Gly Gln Ser Trp Gly Asn Asn Gln Asp Gly Val Val Ser Ser
               100                 105                 110
   Gln Pro Ala Arg Val Val Asp Glu Arg Glu Gln Met Ala Ile Ser Gly
           115                 120                 125
   Gly Phe Ile Arg Arg Val Thr Asn Asp Ala Arg Glu Asn Glu Met Asp
       130                 135                 140
   Glu Asn Leu Glu Gln Val Gly Ser Ile Ile Gly Asn Leu Arg His Met
   145                 150                 155                 160
   Ala Leu Asp Met Gly Asn Glu Ile Asp Thr Gln Asn Arg Gln Ile Asp
                   165                 170                 175
   Arg Ile Met Asp Met Ala Asp Ser Asn Lys Thr Arg Ile Asp Glu Ala
               180                 185                 190
   Asn Gln Arg Ala Thr Lys Met Leu Gly Ser Gly
           195                 200
<210> 16
   <211> 210
   <212> PRT
   <213> Torpedo marmorata
<400> 16
   Met Glu Asn Ser Val Glu Asn Ser Met Asp Pro Arg Ser Glu Gln Glu
    1               5                  10                  15
   Glu Met Gln Arg Cys Ala Asp Gln Ile Thr Asp Glu Ser Leu Glu Ser
               20                  25                  30
   Thr Arg Arg Met Leu Gln Leu Val Glu Glu Ser Lys Asp Ala Gly Ile
           35                  40                  45
   Arg Thr Leu Val Met Leu Asp Glu Gln Gly Glu Gln Leu Glu Arg Ile
       50                  55                  60
   Glu Glu Gly Met Asp Gln Ile Asn Lys Asp Met Lys Glu Ala Glu Lys
   65                  70                  75                  80
   Asn Leu Ser Asp Leu Gly Lys Cys Cys Gly Leu Cys Ser Cys Pro Cys
                   85                  90                  95
   Asn Lys Leu Lys Asn Phe Glu Ala Gly Gly Ala Tyr Lys Lys Val Trp
               100                 105                 110
   Gly Asn Asn Gln Asp Gly Val Val Ala Ser Gln Pro Ala Arg Val Met
           115                 120                 125
   Asp Asp Arg Glu Gln Met Ala Met Ser Gly Gly Tyr Ile Arg Arg Ile
       130                 135                 140
   Thr Asp Asp Ala Arg Glu Asn Glu Met Glu Glu Asn Leu Asp Gln Val
   145                 150                 155                 160
   Gly Ser Ile Ile Gly Asn Leu Arg His Met Ala Leu Asp Met Ser Asn
                   165                 170                 175
   Glu Ile Gly Ser Gln Asn Ala Gln Ile Asp Arg Ile Val Val Lys Gly
               180                 185                 190
   Asp Met Asn Lys Ala Arg Ile Asp Glu Ala Asn Lys His Ala Thr Lys
                 195                 200                 205
   Met Leu
       210
<210> 17
   <211> 206
   <212> PRT
   <213> Xenopus laevis
<400> 17
   Met Ala Asp Asp Ala Asp Met Arg Asn Glu Leu Glu Glu Met Gln Arg
    1               5                  10                  15
   Arg Ala Asp Gln Leu Ala Asp Glu Ser Leu Glu Ser Thr Arg Arg Met
               20                  25                  30
   Leu Gln Tyr Val Glu Gly Ser Lys Asp Ala Gly Ile Arg Thr Leu Val
           35                  40                  45
   Met Leu Asp Glu Gln Gly Glu Gln Leu Asp Arg Val Glu Glu Gly Met
       50                  55                  60
   Asn His Ile Asn Gln Asp Met Lys Glu Ala Glu Lys Asn Leu Lys Asp
   65                  70                  75                  80
   Leu Gly Lys Cys Cys Gly Leu Phe Ile Cys Pro Cys Asn Lys Leu Lys
                   85                  90                  95
   Ser Ser Gly Ala Tyr Asn Lys Ala Trp Gly Asn Asn Gln Asp Gly Val
               100                 105                 110
   Val Ala Ser Gln Pro Ala Arg Val Val Asp Glu Arg Glu Gln Met Ala
           115                 120                 125
   Ile Ser Gly Gly Phe Val Arg Arg Val Thr Asn Asp Ala Arg Glu Thr
       130                 135                 140
   Glu Met Asp Glu Asn Leu Glu Gln Val Gly Gly Ile Ile Gly Asn Leu
   145                 150                 155                 160
   Arg His Met Ala Leu Asp Met Gly Asn Glu Ile Asp Thr Gln Asn Arg
                   165                 170                 175
   Gln Ile Asp Arg Ile Met Glu Lys Ala Asp Ser Asn Lys Ala Arg Ile
               180                 185                 190
   Asp Glu Ala Asn Lys His Ala Thr Lys Met Leu Gly Ser Gly
           195                 200                 205
<210> 18
   <211> 206
   <212> PRT
   <213> Xenopus laevis
<400> 18
   Met Ala Asp Asp Ala Asp Met Arg Asn Glu Leu Glu Glu Met Gln Arg
    1               5                  10                  15
   Arg Ala Asp Gln Leu Ala Asp Glu Ser Leu Glu Ser Thr Arg Arg Met
               20                  25                  30
   Leu Gln Tyr Val Glu Gly Ser Lys Asp Ala Gly Ile Arg Thr Leu Val
           35                  40                  45
   Met Leu Asp Glu Gln Gly Glu Gln Leu Glu Arg Ile Glu Glu Gly Met
       50                  55                  60
   Glu Gln Ile Asn Lys Asp Met Lys Glu Ala Glu Lys Asn Leu Thr Asp
   65                  70                  75                  80
   Leu Gly Lys Phe Cys Gly Leu Cys Val Cys Pro Cys Asn Lys Leu Lys
                   85                  90                  95
   Ser Ser Asp Ala Tyr Lys Lys Ala Trp Gly Asn Asn Gln Asp Gly Val
               100                 105                 110
   Val Ala Ser Gln Pro Ala Arg Val Val Asp Glu Arg Glu Gln Met Ala
           115                 120                 125
   Ile Ser Gly Gly Phe Val Arg Arg Val Thr Asn Asp Ala Arg Glu Thr
       130                 135                 140
   Glu Met Asp Glu Asn Leu Glu Gln Val Gly Gly Ile Ile Gly Asn Leu
   145                 150                 155                 160
   Arg His Met Ala Leu Asp Met Gly Asn Glu Ile Asp Thr Gln Asn Arg
                   165                 170                 175
   Gln Ile Asp Arg Ile Met Glu Lys Ala Asp Ser Asn Lys Ala Arg Ile
               180                 185                 190
   Asp Glu Ala Asn Lys His Ala Thr Lys Met Leu Gly Ser Gly
           195                 200                 205
<210> 19
   <211> 212
   <212> PRT
   <213> Strongylocentrotus purpuratus
<400> 19
   Met Glu Asp Gln Asn Asp Met Asn Met Arg Ser Glu Leu Glu Glu Ile
    1               5                  10                  15
   Gln Met Gln Ser Asn Met Gln Thr Asp Glu Ser Leu Glu Ser Thr Arg
               20                  25                  30
   Arg Met Leu Gln Met Ala Glu Glu Ser Gln Asp Met Gly Ile Lys Thr
           35                  40                  45
   Leu Val Met Leu Asp Glu Gln Gly Glu Gln Leu Asp Arg Ile Glu Glu
       50                  55                  60
   Gly Met Asp Gln Ile Asn Thr Asp Met Arg Glu Ala Glu Lys Asn Leu
   65                  70                  75                  80
   Thr Gly Leu Glu Lys Cys Cys Gly Ile Cys Val Cys Pro Trp Lys Lys
                   85                  90                  95
   Leu Gly Asn Phe Glu Lys Gly Asp Asp Tyr Lys Lys Thr Trp Lys Gly
               100                 105                 110
   Asn Asp Asp Gly Lys Val Asn Ser His Gln Pro Met Arg Met Glu Asp
           115                 120                 125
   Asp Arg Asp Gly Cys Gly Gly Asn Ala Ser Met Ile Thr Arg Ile Thr
       130                 135                 140
   Asn Asp Ala Arg Glu Asp Glu Met Asp Glu Asn Leu Thr Gln Val Ser
   145                 150                 155                 160
   Ser Ile Val Gly Asn Leu Arg His Met Ala Ile Asp Met Gln Ser Glu
                   165                 170                 175
   Ile Gly Ala Gln Asn Ser Gln Val Gly Arg Ile Thr Ser Lys Ala Glu
               180                 185                 190
   Ser Asn Glu Gly Arg Ile Asn Ser Ala Asp Lys Arg Ala Lys Asn Ile
           195                 200                 205
   Leu Arg Asn Lys
       210
<210> 20
   <211> 212
   <212> PRT
   <213> Drosophila melanogaster
<400> 20
   Met Pro Ala Asp Pro Ser Glu Glu Val Ala Pro Gln Val Pro Lys Thr
    1               5                  10                  15
   Glu Leu Glu Glu Leu Gln Ile Asn Ala Gln Gly Val Ala Asp Glu Ser
               20                  25                  30
   Leu Glu Ser Thr Arg Arg Met Leu Ala Leu Cys Glu Glu Ser Lys Glu
           35                  40                  45
   Ala Gly Ile Arg Thr Leu Val Ala Leu Asp Asp Gln Gly Glu Gln Leu
       50                  55                  60
   Asp Arg Ile Glu Glu Gly Met Asp Gln Ile Asn Ala Asp Met Arg Glu
   65                  70                  75                  80
   Ala Glu Lys Asn Leu Ser Gly Met Glu Lys Cys Cys Gly Ile Cys Val
                   85                  90                  95
   Leu Pro Cys Asn Lys Ser Gln Ser Phe Lys Glu Asp Asp Gly Thr Trp
               100                 105                 110
   Lys Gly Asn Asp Asp Gly Lys Val Val Asn Asn Gln Pro Gln Arg Val
           115                 120                 125
   Met Asp Asp Arg Asn Gly Met Met Ala Gln Ala Gly Tyr Ile Gly Arg
       130                 135                 140
   Ile Thr Asn Asp Ala Arg Glu Asp Glu Met Glu Glu Asn Met Gly Gln
   145                 150                 155                 160
   Val Asn Thr Met Ile Gly Asn Leu Arg Asn Met Ala Leu Asp Met Gly
                   165                 170                 175
   Ser Glu Leu Glu Asn Gln Asn Arg Gln Ile Asp Arg Ile Asn Arg Lys
               180                 185                 190
   Gly Glu Ser Asn Glu Ala Arg Ile Ala Val Ala Asn Gln Arg Ala His
           195                 200                 205
   Gln Leu Leu Lys
       210
<210> 21
   <211> 212
   <212> PRT
   <213> Hirudo medicinalis
<400> 21
   Met Ala Lys Asp Ile Lys Pro Lys Pro Ala Asn Gly Arg Asp Ser Pro
    1               5                  10                  15
   Thr Asp Leu Gln Glu Ile Gln Leu Gln Met Asn Ala Ile Thr Asp Asp
               20                  25                  30
   Ser Leu Glu Ser Thr Arg Arg Met Leu Ala Met Cys Glu Glu Ser Lys
           35                  40                  45
   Asp Ala Gly Ile Arg Thr Leu Val Met Leu Asp Glu Gln Gly Glu Gln
       50                  55                  60
   Leu Asp Arg Ile Glu Glu Gly Met Asp Gln Ile Asn Gln Asp Met Arg
   65                  70                  75                  80
   Asp Ala Glu Lys Asn Leu Glu Gly Met Glu Lys Cys Cys Gly Leu Cys
                   85                  90                  95
   Ile Leu Pro Trp Lys Arg Thr Lys Asn Phe Asp Lys Gly Ala Glu Trp
               100                 105                 110
   Asn Lys Gly Asp Glu Gly Lys Val Asn Thr Asp Gly Pro Arg Leu Val
           115                 120                 125
   Val Gly Asp Gly Asn Met Gly Pro Ser Gly Gly Phe Ile Thr Lys Ile
       130                 135                 140
   Thr Asn Asp Ala Arg Glu Glu Glu Met Glu Gln Asn Met Gly Glu Val
   145                 150                 155                 160
   Ser Asn Met Ile Ser Asn Leu Arg Asn Met Ala Val Asp Met Gly Ser
                   165                 170                 175
   Glu Ile Asp Ser Gln Asn Arg Gln Val Asp Arg Ile Asn Asn Lys Met
               180                 185                 190
   Thr Ser Asn Gln Leu Arg Ile Ser Asp Ala Asn Lys Arg Ala Ser Lys
           195                 200                 205
   Leu Leu Lys Glu
       210
<210> 22
   <211> 212
   <212> PRT
   <213> Loligo pealei
<400> 22
   Met Ser Ala Asn Gly Glu Val Glu Val Pro Lys Thr Glu Leu Glu Glu
    1               5                  10                  15
   Ile Gln Gln Gln Cys Asn Gln Val Thr Asp Asp Ser Leu Glu Ser Thr
               20                  25                  30
   Arg Arg Met Leu Asn Met Cys Glu Glu Ser Lys Glu Ala Gly Ile Arg
           35                  40                  45
   Thr Leu Val Met Leu Asp Glu Gln Gly Glu Gln Leu Asp Arg Ile Glu
       50                  55                  60
   Glu Gly Leu Asp Gln Ile Asn Gln Asp Met Lys Asp Ala Glu Lys Asn
   65                  70                  75                  80
   Leu Glu Gly Met Glu Lys Cys Cys Gly Leu Cys Val Leu Pro Trp Lys
                   85                  90                  95
   Arg Gly Lys Ser Phe Glu Lys Ser Gly Asp Tyr Ala Asn Thr Trp Lys
               100                 105                 110
   Lys Asp Asp Asp Gly Pro Thr Asn Thr Asn Gly Pro Arg Val Thr Val
           115                 120                 125
   Gly Asp Gln Asn Gly Met Gly Pro Ser Ser Gly Tyr Val Thr Arg Ile
       130                 135                 140
   Thr Asn Asp Ala Arg Glu Asp Asp Met Glu Asn Asn Met Lys Glu Val
   145                 150                 155                 160
   Ser Ser Met Ile Gly Asn Leu Arg Asn Met Ala Ile Asp Met Gly Asn
                   165                 170                 175
   Glu Ile Gly Ser Gln Asn Arg Gln Val Asp Arg Ile Gln Gln Lys Ala
               180                 185                 190
   Glu Ser Asn Glu Ser Arg Ile Asp Glu Ala Asn Lys Lys Ala Thr Lys
           195                 200                 205
   Leu Leu Lys Asn
       210
<210> 23
   <211> 220
   <212> PRT
   <213> Lymnaea stagnalis
<400> 23
   Met Thr Thr Asn Gly Glu Ile Leu Pro Val Gly Glu Glu Glu Glu Glu
    1               5                  10                  15
   Glu Leu Gly Glu Asp Ala Leu Leu Arg Lys Gln Ile Asp Cys Asn Thr
               20                  25                  30
   Asn Glu Ser Leu Glu Ser Thr Arg Arg Met Leu Ser Leu Cys Glu Glu
           35                  40                  45
   Ser Lys Glu Ala Gly Ile Lys Thr Leu Val Met Leu Asp Glu Gln Gly
       50                  55                  60
   Glu Gln Leu Asp Arg Ile Glu Glu Gly Met Gly Gln Ile Asn Gln Asp
   65                  70                  75                  80
   Met Arg Asp Ala Glu Lys Asn Leu Glu Gly Leu Glu Lys Cys Cys Gly
                   85                  90                  95
   Leu Cys Val Leu Pro Trp Lys Arg Ser Lys Asn Phe Glu Lys Gly Ser
               100                 105                 110
   Asp Tyr Asn Lys Thr Trp Lys Ala Ser Glu Asp Gly Lys Ile Asn Thr
           115                 120                 125
   Asn Gly Pro Arg Leu Val Val Asp Gln Gly Asn Gly Ser Gly Pro Thr
       130                 135                 140
   Gly Gly Tyr Ile Thr Arg Ile Thr Asn Asp Ala Arg Glu Asp Glu Met
   145                 150                 155                 160
   Glu Gln Asn Ile Gly Glu Val Ala Gly Met Val Ser Asn Leu Arg Asn
                   165                 170                 175
   Met Ala Val Asp Met Gly Asn Glu Ile Glu Ser Gln Asn Lys Gln Leu
               180                 185                 190
   Asp Arg Ile Asn Gln Lys Gly Gly Ser Leu Asn Val Arg Val Asp Glu
           195                 200                 205
   Ala Asn Lys Arg Ala Asn Arg Ile Leu Arg Lys Gln
       210                 215                 220
<210> 24
   <211> 207
   <212> PRT
   <213> Caenorhabditis elegans
<400> 24
   Met Ser Gly Asp Asp Asp Ile Pro Glu Gly Leu Glu Ala Ile Asn Leu
   1               5                   10                  15
   Lys Met Asn Ala Thr Thr Asp Asp Ser Leu Glu Ser Thr Arg Arg Met
               20                  25                  30
   Leu Ala Leu Cys Glu Glu Ser Lys Glu Ala Gly Ile Lys Thr Leu Val
           35                  40                  45
   Met Leu Asp Asp Gln Gly Glu Gln Leu Glu Arg Cys Glu Gly Ala Leu
       50                  55                  60
   Asp Thr Ile Asn Gln Asp Met Lys Glu Ala Glu Asp His Leu Lys Gly
   65                  70                  75                  80
   Met Glu Lys Cys Cys Gly Leu Cys Val Leu Pro Trp Asn Lys Thr Asp
                   85                  90                  95
   Asp Phe Glu Lys Thr Glu Phe Ala Lys Ala Trp Lys Lys Asp Asp Asp
               100                 105                 110
   Gly Gly Val Ile Ser Asp Gln Pro Arg Ile Thr Val Gly Asp Ser Ser
           115                 120                 125
   Met Gly Pro Gln Gly Gly Tyr Ile Thr Lys Ile Thr Asn Asp Ala Arg
       130                 135                 140
   Glu Asp Glu Met Asp Glu Asn Val Gln Gln Val Ser Thr Met Val Gly
   145                 150                 155                 160
   Asn Leu Arg Asn Met Ala Ile Asp Met Ser Thr Glu Val Ser Asn Gln
                   165                 170                 175
   Asn Arg Gln Leu Asp Arg Ile His Asp Lys Ala Gln Ser Asn Glu Val
               180                 185                 190
   Arg Val Glu Ser Ala Asn Lys Arg Ala Lys Asn Leu Ile Thr Lys
           195                 200                 205
<210> 25
   <211> 808
   <212> PRT
   <213> Homo sapiens
<400> 25
<210> 26
   <211> 806
   <212> PRT
   <213> Homo sapiens
<400> 26
   Met Gly Ala Pro Ala Cys Ala Leu Ala Leu Cys Val Ala Val Ala Ile
    1               5                  10                  15
   Val Ala Gly Ala Ser Ser Glu Ser Leu Gly Thr Glu Gln Arg Val Val
               20                  25                  30
   Gly Arg Ala Ala Glu Val Pro Gly Pro Glu Pro Gly Gln Gln Glu Gln
           35                  40                  45
   Leu Val Phe Gly Ser Gly Asp Ala Val Glu Leu Ser Cys Pro Pro Pro
       50                  55                  60
   Gly Gly Gly Pro Met Gly Pro Thr Val Trp Val Lys Asp Gly Thr Gly
   65                  70                  75                  80
   Leu Val Pro Ser Glu Arg Val Leu Val Gly Pro Gln Arg Leu Gln Val
                   85                  90                  95
   Leu Asn Ala Ser His Glu Asp Ser Gly Ala Tyr Ser Cys Arg Gln Arg
               100                 105                 110
   Leu Thr Gln Arg Val Leu Cys His Phe Ser Val Arg Val Thr Asp Ala
           115                 120                 125
   Pro Ser Ser Gly Asp Asp Glu Asp Gly Glu Asp Glu Ala Glu Asp Thr
       130                 135                 140
   Gly Val Asp Thr Gly Ala Pro Tyr Trp Thr Arg Pro Glu Arg Met Asp
   145                 150                 155                 160
   Lys Lys Leu Leu Ala Val Pro Ala Ala Asn Thr Val Arg Phe Arg Cys
                   165                 170                 175
   Pro Ala Ala Gly Asn Pro Thr Pro Ser Ile Ser Trp Leu Lys Asn Gly
               180                 185                 190
   Arg Glu Phe Arg Gly Glu His Arg Ile Gly Gly Ile Lys Leu Arg His
           195                 200                 205
   Gln Gln Trp Ser Leu Val Met Glu Ser Val Val Pro Ser Asp Arg Gly
       210                 215                 220
   Asn Tyr Thr Cys Val Val Glu Asn Lys Phe Gly Ser Ile Arg Gln Thr
   225                 230                 235                 240
   Tyr Thr Leu Asp Val Leu Glu Arg Ser Pro His Arg Pro Ile Leu Gln
                   245                 250                 255
   Ala Gly Leu Pro Ala Asn Gln Thr Ala Val Leu Gly Ser Asp Val Glu
               260                 265                 270
   Phe His Cys Lys Val Tyr Ser Asp Ala Gln Pro His Ile Gln Trp Leu
           275                 280                 285
   Lys His Val Glu Val Asn Gly Ser Lys Val Gly Pro Asp Gly Thr Pro
       290                 295                 300
   Tyr Val Thr Val Leu Lys Thr Ala Gly Ala Asn Thr Thr Asp Lys Glu
   305                 310                 315                 320
   Leu Glu Val Leu Ser Leu His Asn Val Thr Phe Glu Asp Ala Gly Glu
                   325                 330                 335
   Tyr Thr Cys Leu Ala Gly Asn Ser Ile Gly Phe Ser His His Ser Ala
               340                 345                 350
   Trp Leu Val Val Leu Pro Ala Glu Glu Glu Leu Val Glu Ala Asp Glu
           355                 360                 365
   Ala Gly Ser Val Tyr Ala Gly Ile Leu Ser Tyr Gly Val Gly Phe Phe
       370                 375                 380
   Leu Phe Ile Leu Val Val Ala Ala Val Thr Leu Cys Arg Leu Arg Ser
   385                 390                 395                 400
   Pro Pro Lys Lys Gly Leu Gly Ser Pro Thr Val His Lys Ile Ser Arg
                   405                 410                 415
   Phe Pro Leu Lys Arg Gln Val Ser Leu Glu Ser Asn Ala Ser Met Ser
               420                 425                 430
   Ser Asn Thr Pro Leu Val Arg Ile Ala Arg Leu Ser Ser Gly Glu Gly
           435                 440                 445
   Pro Thr Leu Ala Asn Val Ser Glu Leu Glu Leu Pro Ala Asp Pro Lys
       450                 455                 460
   Trp Glu Leu Ser Arg Ala Arg Leu Thr Leu Gly Lys Pro Leu Gly Glu
   465                 470                 475                 480
   Gly Cys Phe Gly Gln Val Val Met Ala Glu Ala Ile Gly Ile Asp Lys
                   485                 490                 495
   Asp Arg Ala Ala Lys Pro Val Thr Val Ala Val Lys Met Leu Lys Asp
               500                 505                 510
   Asp Ala Thr Asp Lys Asp Leu Ser Asp Leu Val Ser Glu Met Glu Met
           515                 520                 525
   Met Lys Met Ile Gly Lys His Lys Asn Ile Ile Asn Leu Leu Gly Ala
       530                 535                 540
   Cys Thr Gln Gly Gly Pro Leu Tyr Val Leu Val Glu Tyr Ala Ala Lys
   545                 550                 555                 560
   Gly Asn Leu Arg Glu Phe Leu Arg Ala Arg Arg Pro Pro Gly Leu Asp
                   565                 570                 575
   Tyr Ser Phe Asp Thr Cys Lys Pro Pro Glu Glu Gln Leu Thr Phe Lys
               580                 585                 590
   Asp Leu Val Ser Cys Ala Tyr Gln Val Ala Arg Gly Met Glu Tyr Leu
           595                 600                 605
   Ala Ser Gln Lys Cys Ile His Arg Asp Leu Ala Ala Arg Asn Val Leu
       610                 615                 620
   Val Thr Glu Asp Asn Val Met Lys Ile Ala Asp Phe Gly Leu Ala Arg
   625                 630                 635                 640
   Asp Val His Asn Leu Asp Tyr Tyr Lys Lys Thr Thr Asn Gly Arg Leu
                   645                 650                 655
   Pro Val Lys Trp Met Ala Pro Glu Ala Leu Phe Asp Arg Val Tyr Thr
               660                 665                 670
   His Gln Ser Asp Val Trp Ser Phe Gly Val Leu Leu Trp Glu Ile Phe
           675                 680                 685
   Thr Leu Gly Gly Ser Pro Tyr Pro Gly Ile Pro Val Glu Glu Leu Phe
       690                 695                 700
   Lys Leu Leu Lys Glu Gly His Arg Met Asp Lys Pro Ala Asn Cys Thr
   705                 710                 715                 720
   His Asp Leu Tyr Met Ile Met Arg Glu Cys Trp His Ala Ala Pro Ser
                   725                 730                 735
   Gln Arg Pro Thr Phe Lys Gln Leu Val Glu Asp Leu Asp Arg Val Leu
               740                 745                 750
   Thr Val Thr Ser Thr Asp Glu Tyr Leu Asp Leu Ser Ala Pro Phe Glu
           755                 760                 765
   Gln Tyr Ser Pro Gly Gly Gln Asp Thr Pro Ser Ser Ser Ser Ser Gly
       770                 775                 780
   Asp Asp Ser Val Phe Ala His Asp Leu Leu Pro Pro Ala Pro Pro Ser
   785                 790                 795                 800
   Ser Gly Gly Ser Arg Thr
                   805
<210> 27
   <211> 694
   <212> PRT
   <213> Homo sapiens
<400> 27
   Met Gly Ala Pro Ala Cys Ala Leu Ala Leu Cys Val Ala Val Ala Ile
    1               5                  10                  15
   Val Ala Gly Ala Ser Ser Glu Ser Leu Gly Thr Glu Gln Arg Val Val
               20                  25                  30
   Gly Arg Ala Ala Glu Val Pro Gly Pro Glu Pro Gly Gln Gln Glu Gln
           35                  40                  45
   Leu Val Phe Gly Ser Gly Asp Ala Val Glu Leu Ser Cys Pro Pro Pro
       50                  55                  60
   Gly Gly Gly Pro Met Gly Pro Thr Val Trp Val Lys Asp Gly Thr Gly
   65                  70                  75                  80
   Leu Val Pro Ser Glu Arg Val Leu Val Gly Pro Gln Arg Leu Gln Val
                   85                  90                  95
   Leu Asn Ala Ser His Glu Asp Ser Gly Ala Tyr Ser Cys Arg Gln Arg
               100                 105                 110
   Leu Thr Gln Arg Val Leu Cys His Phe Ser Val Arg Val Thr Asp Ala
           115                 120                 125
   Pro Ser Ser Gly Asp Asp Glu Asp Gly Glu Asp Glu Ala Glu Asp Thr
       130                 135                 140
   Gly Val Asp Thr Gly Ala Pro Tyr Trp Thr Arg Pro Glu Arg Met Asp
   145                 150                 155                 160
   Lys Lys Leu Leu Ala Val Pro Ala Ala Asn Thr Val Arg Phe Arg Cys
                   165                 170                 175
   Pro Ala Ala Gly Asn Pro Thr Pro Ser Ile Ser Trp Leu Lys Asn Gly
               180                 185                 190
   Arg Glu Phe Arg Gly Glu His Arg Ile Gly Gly Ile Lys Leu Arg His
           195                 200                 205
   Gln Gln Trp Ser Leu Val Met Glu Ser Val Val Pro Ser Asp Arg Gly
       210                 215                 220
   Asn Tyr Thr Cys Val Val Glu Asn Lys Phe Gly Ser Ile Arg Gln Thr
   225                 230                 235                 240
   Tyr Thr Leu Asp Val Leu Glu Arg Ser Pro His Arg Pro Ile Leu Gln
                   245                 250                 255
   Ala Gly Leu Pro Ala Asn Gln Thr Ala Val Leu Gly Ser Asp Val Glu
               260                 265                 270
   Phe His Cys Lys Val Tyr Ser Asp Ala Gln Pro His Ile Gln Trp Leu
           275                 280                 285
   Lys His Val Glu Val Asn Gly Ser Lys Val Gly Pro Asp Gly Thr Pro
       290                 295                 300
   Tyr Val Thr Val Leu Lys Val Ser Leu Glu Ser Asn Ala Ser Met Ser
   305                 310                 315                 320
   Ser Asn Thr Pro Leu Val Arg Ile Ala Arg Leu Ser Ser Gly Glu Gly
                   325                 330                 335
   Pro Thr Leu Ala Asn Val Ser Glu Leu Glu Leu Pro Ala Asp Pro Lys
               340                 345                 350
   Trp Glu Leu Ser Arg Ala Arg Leu Thr Leu Gly Lys Pro Leu Gly Glu
           355                 360                 365
   Gly Cys Phe Gly Gln Val Val Met Ala Glu Ala Ile Gly Ile Asp Lys
       370                 375                 380
   Asp Arg Ala Ala Lys Pro Val Thr Val Ala Val Lys Met Leu Lys Asp
   385                 390                 395                 400
   Asp Ala Thr Asp Lys Asp Leu Ser Asp Leu Val Ser Glu Met Glu Met
                   405                 410                 415
   Met Lys Met Ile Gly Lys His Lys Asn Ile Ile Asn Leu Leu Gly Ala
               420                 425                 430
   Cys Thr Gln Gly Gly Pro Leu Tyr Val Leu Val Glu Tyr Ala Ala Lys
           435                 440                 445
   Gly Asn Leu Arg Glu Phe Leu Arg Ala Arg Arg Pro Pro Gly Leu Asp
       450                 455                 460
   Tyr Ser Phe Asp Thr Cys Lys Pro Pro Glu Glu Gln Leu Thr Phe Lys
   465                 470                 475                 480
   Asp Leu Val Ser Cys Ala Tyr Gln Val Ala Arg Gly Met Glu Tyr Leu
                   485                 490                 495
   Ala Ser Gln Lys Cys Ile His Arg Asp Leu Ala Ala Arg Asn Val Leu
               500                 505                 510
   Val Thr Glu Asp Asn Val Met Lys Ile Ala Asp Phe Gly Leu Ala Arg
           515                 520                 525
   Asp Val His Asn Leu Asp Tyr Tyr Lys Lys Thr Thr Asn Gly Arg Leu
       530                 535                 540
   Pro Val Lys Trp Met Ala Pro Glu Ala Leu Phe Asp Arg Val Tyr Thr
   545                 550                 555                 560
   His Gln Ser Asp Val Trp Ser Phe Gly Val Leu Leu Trp Glu Ile Phe
                   565                 570                 575
   Thr Leu Gly Gly Ser Pro Tyr Pro Gly Ile Pro Val Glu Glu Leu Phe
               580                 585                 590
   Lys Leu Leu Lys Glu Gly His Arg Met Asp Lys Pro Ala Asn Cys Thr
           595                 600                 605
   His Asp Leu Tyr Met Ile Met Arg Glu Cys Trp His Ala Ala Pro Ser
       610                 615                 620
   Gln Arg Pro Thr Phe Lys Gln Leu Val Glu Asp Leu Asp Arg Val Leu
   625                 630                 635                 640
   Thr Val Thr Ser Thr Asp Glu Tyr Leu Asp Leu Ser Ala Pro Phe Glu
                   645                 650                 655
   Gln Tyr Ser Pro Gly Gly Gln Asp Thr Pro Ser Ser Ser Ser Ser Gly
               660                 665                 670
   Asp Asp Ser Val Phe Ala His Asp Leu Leu Pro Pro Ala Pro Pro Ser
           675                 680                 685
   Ser Gly Gly Ser Arg Thr
       690
<210> 28
   <211> 604
   <212> PRT
   <213> Homo sapiens
<400> 28
   Ala Gln Arg Arg Lys Glu Arg Glu Glu Leu Ala Gln Gln Tyr Glu Ala
   1               5                   10                  15
   Ile Leu Arg Glu Cys Gly His Gly Arg Phe Gln Trp Thr Leu Tyr Phe
               20                  25                  30
   Val Leu Gly Leu Ala Leu Met Ala Asp Gly Val Glu Val Phe Val Val
           35                  40                  45
   Gly Phe Val Leu Pro Ser Ala Glu Lys Asp Met Cys Leu Ser Asp Ser
       50                  55                  60
   Asn Lys Gly Met Leu Gly Leu Ile Val Tyr Leu Gly Met Met Val Gly
   65                  70                  75                  80
   Ala Phe Leu Trp Gly Gly Leu Ala Asp Arg Leu Gly Arg Arg Gln Cys
                   85                  90                  95
   Leu Leu Ile Ser Leu Ser Val Asn Ser Val Phe Ala Phe Phe Ser Ser
               100                 105                 110
   Phe Val Gln Gly Tyr Gly Thr Phe Leu Phe Cys Arg Leu Leu Ser Gly
           115                 120                 125
   Val Gly Ile Gly Gly Ser Ile Pro Ile Val Phe Ser Tyr Phe Ser Glu
       130                 135                 140
   Phe Leu Ala Gln Glu Lys Arg Gly Glu His Leu Ser Trp Leu Cys Met
   145                 150                 155                 160
   Phe Trp Met Ile Gly Gly Val Tyr Ala Ala Ala Met Ala Trp Ala Ile
                   165                 170                 175
   Ile Pro His Tyr Gly Trp Ser Phe Gln Met Gly Ser Ala Tyr Gln Phe
               180                 185                 190
   His Ser Trp Arg Val Phe Val Leu Val Cys Ala Phe Pro Ser Val Phe
           195                 200                 205
   Ala Ile Gly Ala Leu Thr Thr Gln Pro Glu Ser Pro Arg Phe Phe Leu
       210                 215                 220
   Glu Asn Gly Lys His Asp Glu Ala Trp Met Val Leu Lys Gln Val His
   225                 230                 235                 240
   Asp Thr Asn Met Arg Ala Lys Gly His Pro Glu Arg Val Phe Ser Val
                   245                 250                 255
   Thr His Ile Lys Thr Ile His Gln Glu Asp Glu Leu Ile Glu Ile Gln
               260                 265                 270
   Ser Asp Thr Gly Thr Trp Tyr Gln Arg Trp Gly Val Arg Ala Leu Ser
           275                 280                 285
   Leu Gly Gly Gln Val Trp Gly Asn Phe Leu Ser Cys Phe Gly Pro Glu
       290                 295                 300
   Tyr Arg Arg Ile Thr Leu Met Met Met Gly Val Trp Phe Thr Met Ser
   305                 310                 315                 320
   Phe Ser Tyr Tyr Gly Leu Thr Val Trp Phe Pro Asp Met Ile Arg His
                       325                 330                 335
   Leu Gln Ala Val Asp Tyr Ala Ser Arg Thr Lys Val Phe Pro Gly Glu
                   340                 345                 350
   Arg Val Glu His Val Thr Phe Asn Phe Thr Leu Glu Asn Gln Ile His
               355                 360                 365
   Arg Gly Gly Gln Tyr Phe Asn Asp Lys Phe Ile Gly Leu Arg Leu Lys
       370                 375                 380
   Ser Val Ser Phe Glu Asp Ser Leu Phe Glu Glu Cys Tyr Phe Glu Asp
   385                 390                 395                 400
   Val Thr Ser Ser Asn Thr Phe Phe Arg Asn Cys Thr Phe Ile Asn Thr
                       405                 410                 415
   Val Phe Tyr Asn Thr Asp Leu Phe Glu Tyr Lys Phe Val Asn Ser Arg
                   420                 425                 430
   Leu Ile Asn Ser Thr Phe Leu His Asn Lys Glu Gly Cys Pro Leu Asp
               435                 440                 445
   Val Thr Gly Thr Gly Glu Gly Ala Tyr Met Val Tyr Phe Val Ser Phe
       450                 455                 460
   Leu Gly Thr Leu Ala Val Leu Pro Gly Asn Ile Val Ser Ala Leu Leu
   465                 470                 475                 480
   Met Asp Lys Ile Gly Arg Leu Arg Met Leu Ala Gly Ser Ser Val Met
                       485                 490                 495
   Ser Cys Val Ser Cys Phe Phe Leu Ser Phe Gly Asn Ser Glu Ser Ala
                   500                 505                 510
   Met Ile Ala Leu Leu Cys Leu Phe Gly Gly Val Ser Ile Ala Ser Trp
               515                 520                 525
   Asn Ala Leu Asp Val Leu Thr Val Glu Leu Tyr Pro Ser Asp Lys Arg
       530                 535                 540
   Thr Thr Ala Phe Gly Phe Leu Asn Ala Leu Cys Lys Leu Ala Ala Val
   545                 550                 555                 560
   Leu Gly Ile Ser Ile Phe Thr Ser Phe Val Gly Ile Thr Lys Ala Ala
                       565                 570                 575
   Pro Ile Leu Phe Ala Ser Ala Ala Leu Ala Leu Gly Ser Ser Leu Ala
                   580                 585                 590
   Leu Lys Leu Pro Glu Thr Arg Gly Gln Val Leu Gln
               595                 600
<210> 29
   <211> 683
   <212> PRT
   <213> Homo sapiens
<400> 29
<210> 30
   <211> 727
   <212> PRT
   <213> Homo sapiens
<400> 30
   Met Glu Asp Ser Tyr Lys Asp Arg Thr Ser Leu Met Lys Gly Ala Lys
    1               5                  10                  15
   Asp Ile Ala Arg Glu Val Lys Lys Gln Thr Val Lys Lys Val Asn Gln
               20                  25                  30
   Ala Val Asp Arg Ala Gln Asp Glu Tyr Thr Gln Arg Ser Tyr Ser Arg
           35                  40                  45
   Phe Gln Asp Glu Glu Asp Asp Asp Asp Tyr Tyr Pro Ala Gly Glu Thr
       50                  55                  60
   Tyr Asn Gly Glu Ala Asn Asp Asp Glu Gly Ser Ser Glu Ala Thr Glu
   65                  70                  75                  80
   Gly His Asp Glu Asp Asp Glu Ile Tyr Glu Gly Glu Tyr Gln Gly Ile
                   85                  90                  95
   Pro Ser Met Asn Gln Ala Lys Asp Ser Ile Val Ser Val Gly Gln Pro
               100                 105                 110
   Lys Gly Asp Glu Tyr Lys Asp Arg Arg Glu Leu Glu Ser Glu Arg Arg
           115                 120                 125
   Ala Asp Glu Glu Glu Leu Ala Gln Gln Tyr Glu Leu Ile Ile Gln Glu
       130                 135                 140
   Cys Gly His Gly Arg Phe Gln Trp Ala Leu Phe Phe Val Leu Gly Met
   145                 150                 155                 160
   Ala Leu Met Ala Asp Gly Val Glu Val Phe Val Val Gly Phe Val Leu
                   165                 170                 175
   Pro Ser Ala Glu Thr Asp Leu Cys Ile Pro Asn Ser Gly Ser Gly Trp
               180                 185                 190
   Leu Gly Ser Ile Val Tyr Leu Gly Met Met Val Gly Ala Phe Phe Trp
           195                 200                 205
   Gly Gly Leu Ala Asp Lys Val Gly Arg Lys Gln Ser Leu Leu Ile Cys
       210                 215                 220
   Met Ser Val Asn Gly Phe Phe Ala Phe Leu Ser Ser Phe Val Gln Gly
   225                 230                 235                 240
   Tyr Gly Phe Phe Leu Phe Cys Arg Leu Leu Ser Gly Phe Gly Ile Gly
                   245                 250                 255
   Gly Ala Ile Pro Thr Val Phe Ser Tyr Phe Ala Glu Val Leu Ala Arg
               260                 265                 270
   Glu Lys Arg Gly Glu His Leu Ser Trp Leu Cys Met Phe Trp Met Ile
           275                 280                 285
   Gly Gly Ile Tyr Ala Ser Ala Met Ala Trp Ala Ile Ile Pro His Tyr
       290                 295                 300
   Gly Trp Ser Phe Ser Met Gly Ser Ala Tyr Gln Phe His Ser Trp Arg
   305                 310                 315                 320
   Val Phe Val Ile Val Cys Ala Leu Pro Cys Val Ser Ser Val Val Ala
                   325                 330                 335
   Leu Thr Phe Met Pro Glu Ser Pro Arg Phe Leu Leu Glu Val Gly Lys
               340                 345                 350
   His Asp Glu Ala Trp Met Ile Leu Lys Leu Ile His Asp Thr Asn Met
           355                 360                 365
   Arg Ala Arg Gly Gln Pro Glu Lys Val Phe Thr Val Asn Lys Ile Lys
       370                 375                 380
   Thr Pro Lys Gln Ile Asp Glu Leu Ile Glu Ile Glu Ser Asp Thr Gly
   385                 390                 395                 400
   Thr Trp Tyr Arg Arg Cys Phe Val Arg Ile Arg Thr Glu Leu Tyr Gly
                   405                 410                 415
   Ile Trp Leu Thr Phe Met Arg Cys Phe Asn Tyr Pro Val Arg Asp Asn
               420                 425                 430
   Thr Ile Lys Leu Thr Ile Val Trp Phe Thr Leu Ser Phe Gly Tyr Tyr
           435                 440                 445
   Gly Leu Ser Val Trp Phe Pro Asp Val Ile Lys Pro Leu Gln Ser Asp
       450                 455                 460
   Glu Tyr Ala Leu Leu Thr Arg Asn Val Glu Arg Asp Lys Tyr Ala Asn
   465                 470                 475                 480
   Phe Thr Ile Asn Phe Thr Met Glu Asn Gln Ile His Thr Gly Met Glu
                   485                 490                 495
   Tyr Asp Asn Gly Arg Phe Ile Gly Val Lys Phe Lys Ser Val Thr Phe
               500                 505                 510
   Lys Asp Ser Val Phe Lys Ser Cys Thr Phe Glu Asp Val Thr Ser Val
           515                 520                 525
   Asn Thr Tyr Phe Lys Asn Cys Thr Phe Ile Asp Thr Val Phe Asp Asn
       530                 535                 540
   Thr Asp Phe Glu Pro Tyr Lys Phe Ile Asp Ser Glu Phe Lys Asn Cys
   545                 550                 555                 560
   Ser Phe Phe His Asn Lys Thr Gly Cys Gln Ile Thr Phe Asp Asp Asp
                   565                 570                 575
   Tyr Ser Ala Tyr Trp Ile Tyr Phe Val Asn Phe Leu Gly Thr Leu Ala
               580                 585                 590
   Val Leu Pro Gly Asn Ile Val Ser Ala Leu Leu Met Asp Arg Ile Gly
           595                 600                 605
   Arg Leu Thr Met Leu Gly Gly Ser Met Val Leu Ser Gly Ile Ser Cys
       610                 615                 620
   Phe Phe Leu Trp Phe Gly Thr Ser Glu Ser Met Met Ile Gly Met Leu
   625                 630                 635                 640
   Cys Leu Tyr Asn Gly Leu Thr Ile Ser Ala Trp Asn Ser Leu Asp Val
                   645                 650                 655
   Val Thr Val Glu Leu Tyr Pro Thr Asp Arg Arg Ala Thr Gly Phe Gly
               660                 665                 670
   Phe Leu Asn Ala Leu Cys Lys Ala Ala Ala Val Leu Gly Asn Leu Ile
           675                 680                 685
   Phe Gly Ser Leu Val Ser Ile Thr Lys Ser Ile Pro Ile Leu Leu Ala
       690                 695                 700
   Ser Thr Val Leu Val Cys Gly Gly Leu Val Gly Leu Cys Leu Pro Asp
   705                 710                 715                 720
   Thr Arg Thr Gln Val Leu Met
                   725
<210> 31
   <211> 742
   <212> PRT
   <213> Homo sapiens
<400> 31
   Met Glu Glu Gly Phe Arg Asp Arg Ala Ala Phe Ile Arg Gly Ala Lys
    1               5                  10                  15
   Asp Ile Ala Lys Glu Val Lys Lys His Ala Ala Lys Lys Val Val Lys
               20                  25                  30
   Gly Leu Asp Arg Val Gln Asp Glu Tyr Ser Arg Arg Ser Tyr Ser Arg
           35                  40                  45
   Phe Glu Glu Glu Asp Asp Asp Asp Asp Phe Pro Ala Pro Ser Asp Gly
       50                  55                  60
   Tyr Tyr Arg Gly Glu Gly Thr Gln Asp Glu Glu Glu Gly Gly Ala Ser
   65                  70                  75                  80
   Ser Asp Ala Thr Glu Gly His Asp Glu Asp Asp Glu Ile Tyr Glu Gly
                   85                  90                  95
   Glu Tyr Gln Asp Ile Pro Arg Ala Glu Ser Gly Gly Lys Gly Glu Arg
               100                 105                 110
   Met Ala Asp Gly Ala Pro Leu Ala Gly Val Arg Gly Gly Leu Ser Asp
           115                 120                 125
   Gly Glu Gly Pro Pro Gly Gly Arg Gly Glu Ala Gln Arg Arg Lys Glu
       130                 135                 140
   Arg Glu Glu Leu Ala Gln Gln Tyr Glu Ala Ile Leu Arg Glu Cys Gly
   145                 150                 155                 160
   His Gly Arg Phe Gln Trp Thr Leu Tyr Phe Val Leu Gly Leu Ala Leu
                   165                 170                 175
   Met Ala Asp Gly Val Glu Val Phe Val Val Gly Phe Val Leu Pro Ser
                   180                 185                 190
   Ala Glu Lys Asp Met Cys Leu Ser Asp Ser Asn Lys Gly Met Leu Gly
               195                 200                 205
   Leu Ile Val Tyr Leu Gly Met Met Val Gly Ala Phe Leu Trp Gly Gly
       210                 215                 220
   Leu Ala Asp Arg Leu Gly Arg Arg Gln Cys Leu Leu Ile Ser Leu Ser
   225                 230                 235                 240
   Val Asn Ser Val Phe Ala Phe Phe Ser Ser Phe Val Gln Gly Tyr Gly
                   245                 250                 255
   Thr Phe Leu Phe Cys Arg Leu Leu Ser Gly Val Gly Ile Gly Gly Ser
               260                 265                 270
   Ile Pro Ile Val Phe Ser Tyr Phe Ser Glu Phe Leu Ala Gln Glu Lys
           275                 280                 285
   Arg Gly Glu His Leu Ser Trp Leu Cys Met Phe Trp Met Ile Gly Gly
       290                 295                 300
   Val Tyr Ala Ala Ala Met Ala Trp Ala Ile Ile Pro His Tyr Gly Trp
   305                 310                 315                 320
   Ser Phe Gln Met Gly Ser Ala Tyr Gln Phe His Ser Trp Arg Val Phe
                   325                 330                 335
   Val Leu Val Cys Ala Phe Pro Ser Val Phe Ala Ile Gly Ala Leu Thr
               340                 345                 350
   Thr Gln Pro Glu Ser Pro Arg Phe Phe Leu Glu Asn Gly Lys His Asp
           355                 360                 365
   Glu Ala Trp Met Val Leu Lys Gln Val His Asp Thr Asn Met Arg Ala
       370                 375                 380
   Lys Gly His Pro Glu Arg Val Phe Ser Val Thr His Ile Lys Thr Ile
   385                 390                 395                 400
   His Gln Glu Asp Glu Leu Ile Glu Ile Gln Ser Asp Thr Gly Thr Trp
                   405                 410                 415
   Tyr Gln Arg Trp Gly Val Arg Ala Leu Ser Leu Gly Gly Gln Val Trp
               420                 425                 430
   Gly Asn Phe Leu Ser Cys Phe Gly Pro Glu Tyr Arg Arg Ile Thr Leu
           435                 440                 445
   Met Met Met Gly Val Trp Phe Thr Met Ser Phe Ser Tyr Tyr Gly Leu
       450                 455                 460
   Thr Val Trp Phe Pro Asp Met Ile Arg His Leu Gln Ala Val Asp Tyr
   465                 470                 475                 480
   Ala Ser Arg Thr Lys Val Phe Pro Gly Glu Arg Val Gly His Val Thr
                   485                 490                 495
   Phe Asn Phe Thr Leu Glu Asn Gln Ile His Arg Gly Gly Gln Tyr Phe
               500                 505                 510
   Asn Asp Lys Phe Ile Gly Leu Arg Leu Lys Ser Val Ser Phe Glu Asp
           515                 520                 525
   Ser Leu Phe Glu Glu Cys Tyr Phe Glu Asp Val Thr Ser Ser Asn Thr
       530                 535                 540
   Phe Phe Arg Asn Cys Thr Phe Ile Asn Thr Val Phe Tyr Asn Thr Asp
   545                 550                 555                 560
   Leu Phe Glu Tyr Lys Phe Val Asn Ser Arg Leu Ile Asn Ser Thr Phe
                   565                 570                 575
   Leu His Asn Lys Glu Gly Cys Pro Leu Asp Val Thr Gly Thr Gly Glu
               580                 585                 590
   Gly Ala Tyr Met Val Tyr Phe Val Ser Phe Leu Gly Thr Leu Ala Val
           595                 600                 605
   Leu Pro Gly Asn Ile Val Ser Ala Leu Leu Met Asp Lys Ile Gly Arg
       610                 615                 620
   Leu Arg Met Leu Ala Gly Ser Ser Val Met Ser Cys Val Ser Cys Phe
   625                 630                 635                 640
   Phe Leu Ser Phe Gly Asn Ser Glu Ser Ala Met Ile Ala Leu Leu Cys
                   645                 650                 655
   Leu Phe Gly Gly Val Ser Ile Ala Ser Trp Asn Ala Leu Asp Val Leu
               660                 665                 670
   Thr Val Glu Leu Tyr Pro Ser Asp Lys Arg Thr Thr Ala Phe Gly Phe
           675                 680                 685
   Leu Asn Ala Leu Cys Lys Leu Ala Ala Val Leu Gly Ile Ser Ile Phe
       690                 695                 700
   Thr Ser Phe Val Gly Ile Thr Lys Ala Ala Pro Ile Leu Phe Ala Ser
   705 710 715 720
   Ala Ala Leu Ala Leu Gly Ser Ser Leu Ala Leu Lys Leu Pro Glu Thr
   725 730 735
   Arg Gly Gln Val Leu Gln
   740
<210> 32
   <211> 7
   <212> PRT
   <213> Artificial Sequence
<220>
   <223> SNAP-25 antigen having a free carboxyl-terminus at the P1 residue of the scissile bond of the BoNT/A cleavage site
<400> 32
   Arg Ile Asp Glu Ala Asn Gln
   1 5
<210> 33
   <211> 8
   <212> PRT
   <213> Artificial Sequence
<220>
   <223> SNAP-25 antigen having a free carboxyl-terminus at the P1 residue of the scissile bond of the BoNT/A cleavage site
<400> 33
   Thr Arg Ile Asp Glu Ala Asn Gln
   1 5
<210> 34
   <211> 9
   <212> PRT
   <213> Artificial Sequence
<220>
   <223> SNAP-25 antigen having a free carboxyl-terminus at the P1 residue of the scissile bond of the BoNT/A cleavage site
<400> 34
   Lys Thr Arg Ile Asp Glu Ala Asn Gln
   1 5
<210> 35
   <211> 10
   <212> PRT
   <213> Artificial Sequence
<220>
   <223> SNAP-25 antigen having a free carboxyl-terminus at the P1 residue of the scissile bond of the BoNT/A cleavage site
<400> 35
   Asn Lys Thr Arg Ile Asp Glu Ala Asn Gln
   1 5 10
<210> 36
   <211> 11
   <212> PRT
   <213> Artificial Sequence
<220>
   <223> SNAP-25 antigen having a free carboxyl-terminus at the P1 residue of the scissile bond of the BoNT/A cleavage site
<400> 36
   Ser Asn Lys Thr Arg Ile Asp Glu Ala Asn Gln
   1 5 10
<210> 37
   <211> 12
   <212> PRT
   <213> Artificial Sequence
<220>
   <223> SNAP-25 antigen having a free carboxyl-terminus at the P1 residue of the scissile bond of the BoNT/A cleavage site
<400> 37
   Asp Ser Asn Lys Thr Arg Ile Asp Glu Ala Asn Gln
   1 5 10
<210> 38
   <211> 13
   <212> PRT
   <213> Artificial Sequence
<220>
   <223> SNAP-25 antigen having a free carboxylated carboxyl-terminus at the P1 residue of the scissile bond of the BoNT/A cleavage site
<221> SITE
   <222> (13) ... (13)
   <223> carboxylated glutamine
<400> 38
   Cys Asp Ser Asn Lys Thr Arg Ile Asp Glu Ala Asn Gln
   1 5 10
<210> 39
   <211> 7
   <212> PRT
   <213> Artificial Sequence
<220>
   <223> SNAP-25 antigen having a free carboxyl-terminus at the P1 residue of the scissile bond of the BoNT/A cleavage site
<400> 39
   Arg Ile Asp Glu Ala Asn Lys
   1 5
<210> 40
   <211> 8
   <212> PRT
   <213> Artificial Sequence
<220>
   <223> SNAP-25 antigen having a free carboxyl-terminus at the P1 residue of the scissile bond of the BoNT/A cleavage site
<400> 40
   Ala Arg Ile Asp Glu Ala Asn Lys
   1 5
<210> 41
   <211> 9
   <212> PRT
   <213> Artificial Sequence
<220>
   <223> SNAP-25 antigen having a free carboxyl-terminus at the P1 residue of the scissile bond of the BoNT/A cleavage site
<400> 41
   Lys Ala Arg Ile Asp Glu Ala Asn Lys
   1 5
<210> 42
   <211> 10
   <212> PRT
   <213> Artificial Sequence
<220>
   <223> SNAP-25 antigen having a free carboxyl-terminus at the P1 residue of the scissile bond of the BoNT/A cleavage site
<400> 42
   Asn Lys Ala Arg Ile Asp Glu Ala Asn Lys
   1 5 10
<210> 43
   <211> 11
   <212> PRT
   <213> Artificial Sequence
<220>
   <223> SNAP-25 antigen having a free carboxyl-terminus at the P1 residue of the scissile bond of the BoNT/A cleavage site
<400> 43
   Met Asn Lys Ala Arg Ile Asp Glu Ala Asn Lys
   1 5 10
<210> 44
   <211> 12
   <212> PRT
   <213> Artificial Sequence
<220>
   <223> SNAP-25 antigen having a free carboxyl-terminus at the P1 residue of the scissile bond of the BoNT/A cleavage site
<400> 44
   Asp Met Asn Lys Ala Arg Ile Asp Glu Ala Asn Lys
   1 5 10
<210> 45
   <211> 13
   <212> PRT
   <213> Artificial Sequence
<220>
   <223> SNAP-25 antigen having a free carboxylated carboxyl-terminus at the P1 residue of the scissile bond of the BoNT/A cleavage site
<221> SITE
   <222> (13) ... (13)
   <223> Carboxylated lysine
<400> 45
   Cys Asp Met Asn Lys Ala Arg Ile Asp Glu Ala Asn Lys
   1 5 10
<210> 46
   <211> 11
   <212> PRT
   <213> Artificial Sequence
<220>
   <223> SNAP-25 antigen
<400> 46
   Cys Gly Gly Gly Arg Ile Asp Glu Ala Asn Gln
   1 5 10
<210> 47
   <211> 11
   <212> PRT
   <213> Artificial Sequence
<220>
   <223> SNAP-25 antigen
<400> 47
   Cys Gly Gly Gly Arg Ile Asp Glu Ala Asn Lys
    1               5                   10
<210> 48
   <211> 88
   <212> PRT
   <213> Artificial Sequence
<220>
   <223> BirA-HisTag?-SNAP-25-134-197
<400> 48
   Met Gly Gly Gly Leu Asn Asp Ile Phe Glu Ala Gln Lys Ile Glu Trp
    1               5                   10                  15
   His His His His His His His His Ile Arg Arg Val Thr Asn Asp Ala
               20                  25                  30
   Arg Glu Asn Glu Met Asp Glu Asn Leu Glu Gln Val Ser Gly Ile Ile
           35                  40                  45
   Gly Asn Leu Arg His Met Ala Leu Asp Met Gly Asn Glu Ile Asp Thr
       50                  55                  60
   Gln Asn Arg Gln Ile Asp Arg Ile Met Glu Lys Ala Asp Ser Asn Lys
   65                  70                  75                  80
   Thr Arg Ile Asp Glu Ala Asn Gln
                   85
<210> 49
   <211> 97
   <212> PRT
   <213> Artificial Sequence
<220>
   <223> BirA-HisTag?-SNAP-25-134-206
<400> 49
   Met Gly Gly Gly Leu Asn Asp Ile Phe Glu Ala Gln Lys Ile Glu Trp
    1               5                   10                  15
   His His His His His His His His Ile Arg Arg Val Thr Asn Asp Ala
               20                  25                  30
   Arg Glu Asn Glu Met Asp Glu Asn Leu Glu Gln Val Ser Gly Ile Ile
           35                  40                  45
   Gly Asn Leu Arg His Met Ala Leu Asp Met Gly Asn Glu Ile Asp Thr
       50                  55                  60
   Gln Asn Arg Gln Ile Asp Arg Ile Met Glu Lys Ala Asp Ser Asn Lys
   65                  70                  75                  80
   Thr Arg Ile Asp Glu Ala Asn Gln Arg Ala Thr Lys Met Leu Gly Ser
                   85                  90                  95
   Gly
<210> 50
   <211> 16
   <212> PRT
   <213> Artificial Sequence
<220>
   <223> BirA peptide
<400> 50
   Gly Gly Gly Leu Asn Asp Ile Phe Glu Ala Gln Lys Ile Glu Trp His
    1               5                  10                  15
<210> 51
   <211> 7570
   <212> DNA
   <213> Artificial Sequence
<220>
   <223> pQBI-25/GFP-BoNT/A-LC expression construct.
<400> 51
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcc tcgaggcctg gccattgcat acgttgtatc 240
   catatcataa tatgtacatt tatattggct catgtccaac attaccgcca tgttgacatt 300
   gattattgac tagttattaa tagtaatcaa ttacggggtc attagttcat agcccatata 360
   tggagttccg cgttacataa cttacggtaa atggcccgcc tggctgaccg cccaacgacc 420
   cccgcccatt gacgtcaata atgacgtatg ttcccatagt aacgccaata gggactttcc 480
   attgacgtca atgggtggag tatttacggt aaactgccca cttggcagta catcaagtgt 540
   atcatatgcc aagtacgccc cctattgacg tcaatgacgg taaatggccc gcctggcatt 600
   atgcccagta catgacctta tgggactttc ctacttggca gtacatctac gtattagtca 660
   tcgctattac catggtgatg cggttttggc agtacatcaa tgggcgtgga tagcggtttg 720
   actcacgggg atttccaagt ctccacccca ttgacgtcaa tgggagtttg ttttggcacc 780
   aaaatcaacg ggactttcca aaatgtcgta acaactccgc cccattgacg caaatgggcg 840
   gtaggcgtgt acggtgggag gtctatataa gcagagctcg tttagtgaac cgtcagatcg 900
   cctggagacg ccatccacgc tgttttgacc tccatagaag acaccgggac cgatccagcc 960
   tccgcgggcc accatggagg gcccggttac cggtaccgga tccagatatc tgggcggccg 1020
   ctcagcaagc ttcgcgaatt cgggaggcgg aggtggagct agcaaaggag aagaactctt 1080
   cactggagtt gtcccaattc ttgttgaatt agatggtgat gttaacggcc acaagttctc 1140
   tgtcagtgga gagggtgaag gtgatgcaac atacggaaaa cttaccctga agttcatctg 1200
   cactactggc aaactgcctg ttccatggcc aacactagtc actactctgt gctatggtgt 1260
   tcaatgcttt tcaagatacc cggatcatat gaaacggcat gactttttca agagtgccat 1320
   gcccgaaggt tatgtacagg aaaggaccat cttcttcaaa gatgacggca actacaagac 1380
   acgtgctgaa gtcaagtttg aaggtgatac ccttgttaat agaatcgagt taaaaggtat 1440
   tgacttcaag gaagatggca acattctggg acacaaattg gaatacaact ataactcaca 1500
   caatgtatac atcatggcag acaaacaaaa gaatggaatc aaagtgaact tcaagacccg 1560
   ccacaacatt gaagatggaa gcgttcaact agcagaccat tatcaacaaa atactccaat 1620
   tggcgatggc cctgtccttt taccagacaa ccattacctg tccacacaat ctgccctttc 1680
   gaaagatccc aacgaaaaga gagaccacat ggtccttctt gagtttgtaa cagctgctgg 1740
   gattacacat ggcatggatg aactgtacaa catcgatgga ggcggaggtg gaccttttgt 1800
   taataaacaa tttaattata aagatcctgt aaatggtgtt gatattgctt atataaaaat 1860
   tccaaatgca ggacaaatgc aaccagtaaa agcttttaaa attcataata aaatatgggt 1920
   tattccagaa agagatacat ttacaaatcc tgaagaagga gatttaaatc caccaccaga 1980
   agcaaaacaa gttccagttt catattatga ttcaacatat ttaagtacag ataatgaaaa 2040
   agataattat ttaaagggag ttacaaaatt atttgagaga atttattcaa ctgatcttgg 2100
   aagaatgttg ttaacatcaa tagtaagggg aataccattt tggggtggaa gtacaataga 2160
   tacagaatta aaagttattg atactaattg tattaatgtg atacaaccag atggtagtta 2220
   tagatcagaa gaacttaatc tagtaataat aggaccctca gctgatatta tacagtttga 2280
   atgtaaaagc tttggacatg aagttttgaa tcttacgcga aatggttatg gctctactca 2340
   atacattaga tttagcccag attttacatt tggttttgag gagtcacttg aagttgatac 2400
   aaatcctctt ttaggtgcag gcaaatttgc tacagatcca gcagtaacat tagcacatga 2460
   acttatacat gctggacata gattatatgg aatagcaatt aatccaaata gggtttttaa 2520
   agtaaatact aatgcctatt atgaaatgag tgggttagaa gtaagctttg aggaacttag 2580
   aacatttggg ggacatgatg caaagtttat agatagttta caggaaaacg aatttcgtct 2640
   atattattat aataagttta aagatatagc aagtacactt aataaagcta aatcaatagt 2700
   aggtactact gcttcattac agtatatgaa aaatgttttt aaagagaaat atctcctatc 2760
   tgaagataca tctggaaaat tttcggtaga taaattaaaa tttgataagt tatacaaaat 2820
   gttaacagag atttacacag aggataattt tgttaagttt tttaaagtac ttaacagaaa 2880
   aacatatttg aattttgata aagccgtatt taagataaat atagtaccta aggtaaatta 2940
   cacaatatat gatggattta atttaagaaa tacaaattta gcagcaaact ttaatggtca 3000
   aaatacagaa attaataata tgaattttac taaactaaaa aattttactg gattgtttga 3060
   attttataag ttgctatgtg taagagggat aatcacttcg aaatgaacgc gttggcccta 3120
   ttctatagtg tcacctaaat gctagagctc gctgatcagc ctcgactgtg ccttctagtt 3180
   gccagccatc tgttgtttgc ccctcccccg tgccttcctt gaccctggaa ggtgccactc 3240
   ccactgtcct ttcctaataa aatgaggaaa ttgcatcgca ttgtctgagt aggtgtcatt 3300
   ctattctggg gggtggggtg gggcaggaca gcaaggggga ggattgggaa gacaatagca 3360
   ggcatgctgg ggatgcggtg ggctctatgg cttctgaggc ggaaagaacc agctggggct 3420
   ctagggggta tccccacgcg ccctgtagcg gcgcattaag cgcggcgggt gtggtggtta 3480
   cgcgcagcgt gaccgctaca cttgccagcg ccctagcgcc cgctcctttc gctttcttcc 3540
   cttcctttct cgccacgttc gccggctttc cccgtcaagc tctaaatcgg ggcatccctt 3600
   tagggttccg atttagtgct ttacggcacc tcgaccccaa aaaacttgat tagggtgatg 3660
   gttcacgtag tgggccatcg ccctgataga cggtttttcg ccctttgacg ttggagtcca 3720
   cgttctttaa tagtggactc ttgttccaaa ctggaacaac actcaaccct atctcggtct 3780
   attcttttga tttataaggg attttgggga tttcggccta ttggttaaaa aatgagctga 3840
   tttaacaaaa atttaacgcg aattaattct gtggaatgtg tgtcagttag ggtgtggaaa 3900
   gtccccaggc tccccaggca ggcagaagta tgcaaagcat gcatctcaat tagtcagcaa 3960
   ccaggtgtgg aaagtcccca ggctccccag caggcagaag tatgcaaagc atgcatctca 4020
   attagtcagc aaccatagtc ccgcccctaa ctccgcccat cccgccccta actccgccca 4080
   gttccgccca ttctccgccc catggctgac taattttttt tatttatgca gaggccgagg 4140
   ccgcctctgc ctctgagcta ttccagaagt agtgaggagg cttttttgga ggcctaggct 4200
   tttgcaaaaa gctcccggga gcttgtatat ccattttcgg atctgatcaa gagacaggat 4260
   gaggatcgtt tcgcatgatt gaacaagatg gattgcacgc aggttctccg gccgcttggg 4320
   tggagaggct attcggctat gactgggcac aacagacaat cggctgctct gatgccgccg 4380
   tgttccggct gtcagcgcag gggcgcccgg ttctttttgt caagaccgac ctgtccggtg 4440
   ccctgaatga actgcaggac gaggcagcgc ggctatcgtg gctggccacg acgggcgttc 4500
   cttgcgcagc tgtgctcgac gttgtcactg aagcgggaag ggactggctg ctattgggcg 4560
   aagtgccggg gcaggatctc ctgtcatctc accttgctcc tgccgagaaa gtatccatca 4620
   tggctgatgc aatgcggcgg ctgcatacgc ttgatccggc tacctgccca ttcgaccacc 4680
   aagcgaaaca tcgcatcgag cgagcacgta ctcggatgga agccggtctt gtcgatcagg 4740
   atgatctgga cgaagagcat caggggctcg cgccagccga actgttcgcc aggctcaagg 4800
   cgcgcatgcc cgacggcgag gatctcgtcg tgacccatgg cgatgcctgc ttgccgaata 4860
   tcatggtgga aaatggccgc ttttctggat tcatcgactg tggccggctg ggtgtggcgg 4920
   accgctatca ggacatagcg ttggctaccc gtgatattgc tgaagagctt ggcggcgaat 4980
   gggctgaccg cttcctcgtg ctttacggta tcgccgctcc cgattcgcag cgcatcgcct 5040
   tctatcgcct tcttgacgag ttcttctgag cgggactctg gggttcgaaa tgaccgacca 5100
   agcgacgccc aacctgccat cacgagattt cgattccacc gccgccttct atgaaaggtt 5160
   gggcttcgga atcgttttcc gggacgccgg ctggatgatc ctccagcgcg gggatctcat 5220
   gctggagttc ttcgcccacc ccaacttgtt tattgcagct tataatggtt acaaataaag 5280
   caatagcatc acaaatttca caaataaagc atttttttca ctgcattcta gttgtggttt 5340
   gtccaaactc atcaatgtat cttatcatgt ctgtataccg tcgacctcta gctagagctt 5400
   ggcgtaatca tggtcatagc tgtttcctgt gtgaaattgt tatccgctca caattccaca 5460
   caacatacga gccggaagca taaagtgtaa agcctggggt gcctaatgag tgagctaact 5520
   cacattaatt gcgttgcgct cactgcccgc tttccagtcg ggaaacctgt cgtgccagct 5580
   gcattaatga atcggccaac gcgcggggag aggcggtttg cgtattgggc gctcttccgc 5640
   ttcctcgctc actgactcgc tgcgctcggt cgttcggctg cggcgagcgg tatcagctca 5700
   ctcaaaggcg gtaatacggt tatccacaga atcaggggat aacgcaggaa agaacatgtg 5760
   agcaaaaggc cagcaaaagg ccaggaaccg taaaaaggcc gcgttgctgg cgtttttcca 5820
   taggctccgc ccccctgacg agcatcacaa aaatcgacgc tcaagtcaga ggtggcgaaa 5880
   cccgacagga ctataaagat accaggcgtt tccccctgga agctccctcg tgcgctctcc 5940
   tgttccgacc ctgccgctta ccggatacct gtccgccttt ctcccttcgg gaagcgtggc 6000
   gctttctcaa tgctcacgct gtaggtatct cagttcggtg taggtcgttc gctccaagct 6060
   gggctgtgtg cacgaacccc ccgttcagcc cgaccgctgc gccttatccg gtaactatcg 6120
   tcttgagtcc aacccggtaa gacacgactt atcgccactg gcagcagcca ctggtaacag 6180
   gattagcaga gcgaggtatg taggcggtgc tacagagttc ttgaagtggt ggcctaacta 6240
   cggctacact agaaggacag tatttggtat ctgcgctctg ctgaagccag ttaccttcgg 6300
   aaaaagagtt ggtagctctt gatccggcaa acaaaccacc gctggtagcg gtggtttttt 6360
   tgtttgcaag cagcagatta cgcgcagaaa aaaaggatct caagaagatc ctttgatctt 6420
   ttctacgggg tctgacgctc agtggaacga aaactcacgt taagggattt tggtcatgag 6480
   attatcaaaa aggatcttca cctagatcct tttaaattaa aaatgaagtt ttaaatcaat 6540
   ctaaagtata tatgagtaaa cttggtctga cagttaccaa tgcttaatca gtgaggcacc 6600
   tatctcagcg atctgtctat ttcgttcatc catagttgcc tgactccccg tcgtgtagat 6660
   aactacgata cgggagggct taccatctgg ccccagtgct gcaatgatac cgcgagaccc 6720
   acgctcaccg gctccagatt tatcagcaat aaaccagcca gccggaaggg ccgagcgcag 6780
   aagtggtcct gcaactttat ccgcctccat ccagtctatt aattgttgcc gggaagctag 6840
   agtaagtagt tcgccagtta atagtttgcg caacgttgtt gccattgcta caggcatcgt 6900
   ggtgtcacgc tcgtcgtttg gtatggcttc attcagctcc ggttcccaac gatcaaggcg 6960
   agttacatga tcccccatgt tgtgcaaaaa agcggttagc tccttcggtc ctccgatcgt 7020
   tgtcagaagt aagttggccg cagtgttatc actcatggtt atggcagcac tgcataattc 7080
   tcttactgtc atgccatccg taagatgctt ttctgtgact ggtgagtact caaccaagtc 7140
   attctgagaa tagtgtatgc ggcgaccgag ttgctcttgc ccggcgtcaa tacgggataa 7200
   taccgcgcca catagcagaa ctttaaaagt gctcatcatt ggaaaacgtt cttcggggcg 7260
   aaaactctca aggatcttac cgctgttgag atccagttcg atgtaaccca ctcgtgcacc 7320
   caactgatct tcagcatctt ttactttcac cagcgtttct gggtgagcaa aaacaggaag 7380
   gcaaaatgcc gcaaaaaagg gaataagggc gacacggaaa tgttgaatac tcatactctt 7440
   cctttttcaa tattattgaa gcatttatca gggttattgt ctcatgagcg gatacatatt 7500
   tgaatgtatt tagaaaaata aacaaatagg ggttccgcgc acatttcccc gaaaagtgcc 7560
   acctgacgtc 7570
<210> 52
   <211> 682
   <212> PRT
   <213> Artificial Sequence
<220>
   <223> GFP-BoNT/A light chain amino acid sequence.
<400> 52
   Ala Ser Lys Gly Glu Glu Leu Phe Thr Gly Val Val Pro Ile Leu Val
   1               5                   10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Leu
       50                  55                  60
   Cys Tyr Gly Val Gln Cys Phe Ser Arg Tyr Pro Asp His Met Lys Arg
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Thr Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Asn Ile Asp
   225                 230                 235                 240
   Gly Gly Gly Gly Gly Pro Phe Val Asn Lys Gln Phe Asn Tyr Lys Asp
                   245                 250                 255
   Pro Val Asn Gly Val Asp Ile Ala Tyr Ile Lys Ile Pro Asn Ala Gly
               260                 265                 270
   Gln Met Gln Pro Val Lys Ala Phe Lys Ile His Asn Lys Ile Trp Val
           275                 280                 285
   Ile Pro Glu Arg Asp Thr Phe Thr Asn Pro Glu Glu Gly Asp Leu Asn
       290                 295                 300
   Pro Pro Pro Glu Ala Lys Gln Val Pro Val Ser Tyr Tyr Asp Ser Thr
   305                 310                 315                 320
   Tyr Leu Ser Thr Asp Asn Glu Lys Asp Asn Tyr Leu Lys Gly Val Thr
                   325                 330                 335
   Lys Leu Phe Glu Arg Ile Tyr Ser Thr Asp Leu Gly Arg Met Leu Leu
               340                 345                 350
   Thr Ser Ile Val Arg Gly Ile Pro Phe Trp Gly Gly Ser Thr Ile Asp
           355                 360                 365
   Thr Glu Leu Lys Val Ile Asp Thr Asn Cys Ile Asn Val Ile Gln Pro
       370                 375                 380
   Asp Gly Ser Tyr Arg Ser Glu Glu Leu Asn Leu Val Ile Ile Gly Pro
   385                 390                 395                 400
   Ser Ala Asp Ile Ile Gln Phe Glu Cys Lys Ser Phe Gly His Glu Val
                   405                 410                 415
   Leu Asn Leu Thr Arg Asn Gly Tyr Gly Ser Thr Gln Tyr Ile Arg Phe
               420                 425                 430
   Ser Pro Asp Phe Thr Phe Gly Phe Glu Glu Ser Leu Glu Val Asp Thr
           435                 440                 445
   Asn Pro Leu Leu Gly Ala Gly Lys Phe Ala Thr Asp Pro Ala Val Thr
       450                 455                 460
   Leu Ala His Glu Leu Ile His Ala Gly His Arg Leu Tyr Gly Ile Ala
   465                 470                 475                 480
   Ile Asn Pro Asn Arg Val Phe Lys Val Asn Thr Asn Ala Tyr Tyr Glu
                   485                 490                 495
   Met Ser Gly Leu Glu Val Ser Phe Glu Glu Leu Arg Thr Phe Gly Gly
               500                 505                 510
   His Asp Ala Lys Phe Ile Asp Ser Leu Gln Glu Asn Glu Phe Arg Leu
           515                 520                 525
   Tyr Tyr Tyr Asn Lys Phe Lys Asp Ile Ala Ser Thr Leu Asn Lys Ala
       530                 535                 540
   Lys Ser Ile Val Gly Thr Thr Ala Ser Leu Gln Tyr Met Lys Asn Val
   545                 550                 555                 560
   Phe Lys Glu Lys Tyr Leu Leu Ser Glu Asp Thr Ser Gly Lys Phe Ser
                   565                 570                 575
   Val Asp Lys Leu Lys Phe Asp Lys Leu Tyr Lys Met Leu Thr Glu Ile
               580                 585                 590
   Tyr Thr Glu Asp Asn Phe Val Lys Phe Phe Lys Val Leu Asn Arg Lys
           595                 600                 605
   Thr Tyr Leu Asn Phe Asp Lys Ala Val Phe Lys Ile Asn Ile Val Pro
       610                 615                 620
   Lys Val Asn Tyr Thr Ile Tyr Asp Gly Phe Asn Leu Arg Asn Thr Asn
   625                 630                 635                 640
   Leu Ala Ala Asn Phe Asn Gly Gln Asn Thr Glu Ile Asn Asn Met Asn
                   645                 650                 655
   Phe Thr Lys Leu Lys Asn Phe Thr Gly Leu Phe Glu Phe Tyr Lys Leu
               660                 665                 670
   Leu Cys Val Arg Gly Ile Ile Thr Ser Lys
           675                 680
<210> 53
   <211> 6259
   <212> DNA
   <213> Artificial Sequence
<220>
   <223> pQBI-25/GFP expression construct.
<400> 53
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcc tcgaggcctg gccattgcat acgttgtatc 240
   catatcataa tatgtacatt tatattggct catgtccaac attaccgcca tgttgacatt 300
   gattattgac tagttattaa tagtaatcaa ttacggggtc attagttcat agcccatata 360
   tggagttccg cgttacataa cttacggtaa atggcccgcc tggctgaccg cccaacgacc 420
   cccgcccatt gacgtcaata atgacgtatg ttcccatagt aacgccaata gggactttcc 480
   attgacgtca atgggtggag tatttacggt aaactgccca cttggcagta catcaagtgt 540
   atcatatgcc aagtacgccc cctattgacg tcaatgacgg taaatggccc gcctggcatt 600
   atgcccagta catgacctta tgggactttc ctacttggca gtacatctac gtattagtca 660
   tcgctattac catggtgatg cggttttggc agtacatcaa tgggcgtgga tagcggtttg 720
   actcacgggg atttccaagt ctccacccca ttgacgtcaa tgggagtttg ttttggcacc 780
   aaaatcaacg ggactttcca aaatgtcgta acaactccgc cccattgacg caaatgggcg 840
   gtaggcgtgt acggtgggag gtctatataa gcagagctcg tttagtgaac cgtcagatcg 900
   cctggagacg ccatccacgc tgttttgacc tccatagaag acaccgggac cgatccagcc 960
   tccgcgggcc accatggagg gcccggttac cggtaccgga tccagatatc tgggcggccg 1020
   ctcagcaagc ttcgcgaatt cgggaggcgg aggtggagct agcaaaggag aagaactctt 1080
   cactggagtt gtcccaattc ttgttgaatt agatggtgat gttaacggcc acaagttctc 1140
   tgtcagtgga gagggtgaag gtgatgcaac atacggaaaa cttaccctga agttcatctg 1200
   cactactggc aaactgcctg ttccatggcc aacactagtc actactctgt gctatggtgt 1260
   tcaatgcttt tcaagatacc cggatcatat gaaacggcat gactttttca agagtgccat 1320
   gcccgaaggt tatgtacagg aaaggaccat cttcttcaaa gatgacggca actacaagac 1380
   acgtgctgaa gtcaagtttg aaggtgatac ccttgttaat agaatcgagt taaaaggtat 1440
   tgacttcaag gaagatggca acattctggg acacaaattg gaatacaact ataactcaca 1500
   caatgtatac atcatggcag acaaacaaaa gaatggaatc aaagtgaact tcaagacccg 1560
   ccacaacatt gaagatggaa gcgttcaact agcagaccat tatcaacaaa atactccaat 1620
   tggcgatggc cctgtccttt taccagacaa ccattacctg tccacacaat ctgccctttc 1680
   gaaagatccc aacgaaaaga gagaccacat ggtccttctt gagtttgtaa cagctgctgg 1740
   gattacacat ggcatggatg aactgtacaa catcgatgga ggcggaggtg gatgaacgcg 1800
   ttggccctat tctatagtgt cacctaaatg ctagagctcg ctgatcagcc tcgactgtgc 1860
   cttctagttg ccagccatct gttgtttgcc cctcccccgt gccttccttg accctggaag 1920
   gtgccactcc cactgtcctt tcctaataaa atgaggaaat tgcatcgcat tgtctgagta 1980
   ggtgtcattc tattctgggg ggtggggtgg ggcaggacag caagggggag gattgggaag 2040
   acaatagcag gcatgctggg gatgcggtgg gctctatggc ttctgaggcg gaaagaacca 2100
   gctggggctc tagggggtat ccccacgcgc cctgtagcgg cgcattaagc gcggcgggtg 2160
   tggtggttac gcgcagcgtg accgctacac ttgccagcgc cctagcgccc gctcctttcg 2220
   ctttcttccc ttcctttctc gccacgttcg ccggctttcc ccgtcaagct ctaaatcggg 2280
   gcatcccttt agggttccga tttagtgctt tacggcacct cgaccccaaa aaacttgatt 2340
   agggtgatgg ttcacgtagt gggccatcgc cctgatagac ggtttttcgc cctttgacgt 2400
   tggagtccac gttctttaat agtggactct tgttccaaac tggaacaaca ctcaacccta 2460
   tctcggtcta ttcttttgat ttataaggga ttttggggat ttcggcctat tggttaaaaa 2520
   atgagctgat ttaacaaaaa tttaacgcga attaattctg tggaatgtgt gtcagttagg 2580
   gtgtggaaag tccccaggct ccccaggcag gcagaagtat gcaaagcatg catctcaatt 2640
   agtcagcaac caggtgtgga aagtccccag gctccccagc aggcagaagt atgcaaagca 2700
   tgcatctcaa ttagtcagca accatagtcc cgcccctaac tccgcccatc ccgcccctaa 2760
   ctccgcccag ttccgcccat tctccgcccc atggctgact aatttttttt atttatgcag 2820
   aggccgaggc cgcctctgcc tctgagctat tccagaagta gtgaggaggc ttttttggag 2880
   gcctaggctt ttgcaaaaag ctcccgggag cttgtatatc cattttcgga tctgatcaag 2940
   agacaggatg aggatcgttt cgcatgattg aacaagatgg attgcacgca ggttctccgg 3000
   ccgcttgggt ggagaggcta ttcggctatg actgggcaca acagacaatc ggctgctctg 3060
   atgccgccgt gttccggctg tcagcgcagg ggcgcccggt tctttttgtc aagaccgacc 3120
   tgtccggtgc cctgaatgaa ctgcaggacg aggcagcgcg gctatcgtgg ctggccacga 3180
   cgggcgttcc ttgcgcagct gtgctcgacg ttgtcactga agcgggaagg gactggctgc 3240
   tattgggcga agtgccgggg caggatctcc tgtcatctca ccttgctcct gccgagaaag 3300
   tatccatcat ggctgatgca atgcggcggc tgcatacgct tgatccggct acctgcccat 3360
   tcgaccacca agcgaaacat cgcatcgagc gagcacgtac tcggatggaa gccggtcttg 3420
   tcgatcagga tgatctggac gaagagcatc aggggctcgc gccagccgaa ctgttcgcca 3480
   ggctcaaggc gcgcatgccc gacggcgagg atctcgtcgt gacccatggc gatgcctgct 3540
   tgccgaatat catggtggaa aatggccgct tttctggatt catcgactgt ggccggctgg 3600
   gtgtggcgga ccgctatcag gacatagcgt tggctacccg tgatattgct gaagagcttg 3660
   gcggcgaatg ggctgaccgc ttcctcgtgc tttacggtat cgccgctccc gattcgcagc 3720
   gcatcgcctt ctatcgcctt cttgacgagt tcttctgagc gggactctgg ggttcgaaat 3780
   gaccgaccaa gcgacgccca acctgccatc acgagatttc gattccaccg ccgccttcta 3840
   tgaaaggttg ggcttcggaa tcgttttccg ggacgccggc tggatgatcc tccagcgcgg 3900
   ggatctcatg ctggagttct tcgcccaccc caacttgttt attgcagctt ataatggtta 3960
   caaataaagc aatagcatca caaatttcac aaataaagca tttttttcac tgcattctag 4020
   ttgtggtttg tccaaactca tcaatgtatc ttatcatgtc tgtataccgt cgacctctag 4080
   ctagagcttg gcgtaatcat ggtcatagct gtttcctgtg tgaaattgtt atccgctcac 4140
   aattccacac aacatacgag ccggaagcat aaagtgtaaa gcctggggtg cctaatgagt 4200
   gagctaactc acattaattg cgttgcgctc actgcccgct ttccagtcgg gaaacctgtc 4260
   gtgccagctg cattaatgaa tcggccaacg cgcggggaga ggcggtttgc gtattgggcg 4320
   ctcttccgct tcctcgctca ctgactcgct gcgctcggtc gttcggctgc ggcgagcggt 4380
   atcagctcac tcaaaggcgg taatacggtt atccacagaa tcaggggata acgcaggaaa 4440
   gaacatgtga gcaaaaggcc agcaaaaggc caggaaccgt aaaaaggccg cgttgctggc 4500
   gtttttccat aggctccgcc cccctgacga gcatcacaaa aatcgacgct caagtcagag 4560
   gtggcgaaac ccgacaggac tataaagata ccaggcgttt ccccctggaa gctccctcgt 4620
   gcgctctcct gttccgaccc tgccgcttac cggatacctg tccgcctttc tcccttcggg 4680
   aagcgtggcg ctttctcaat gctcacgctg taggtatctc agttcggtgt aggtcgttcg 4740
   ctccaagctg ggctgtgtgc acgaaccccc cgttcagccc gaccgctgcg ccttatccgg 4800
   taactatcgt cttgagtcca acccggtaag acacgactta tcgccactgg cagcagccac 4860
   tggtaacagg attagcagag cgaggtatgt aggcggtgct acagagttct tgaagtggtg 4920
   gcctaactac ggctacacta gaaggacagt atttggtatc tgcgctctgc tgaagccagt 4980
   taccttcgga aaaagagttg gtagctcttg atccggcaaa caaaccaccg ctggtagcgg 5040
   tggttttttt gtttgcaagc agcagattac gcgcagaaaa aaaggatctc aagaagatcc 5100
   tttgatcttt tctacggggt ctgacgctca gtggaacgaa aactcacgtt aagggatttt 5160
   ggtcatgaga ttatcaaaaa ggatcttcac ctagatcctt ttaaattaaa aatgaagttt 5220
   taaatcaatc taaagtatat atgagtaaac ttggtctgac agttaccaat gcttaatcag 5280
   tgaggcacct atctcagcga tctgtctatt tcgttcatcc atagttgcct gactccccgt 5340
   cgtgtagata actacgatac gggagggctt accatctggc cccagtgctg caatgatacc 5400
   gcgagaccca cgctcaccgg ctccagattt atcagcaata aaccagccag ccggaagggc 5460
   cgagcgcaga agtggtcctg caactttatc cgcctccatc cagtctatta attgttgccg 5520
   ggaagctaga gtaagtagtt cgccagttaa tagtttgcgc aacgttgttg ccattgctac 5580
   aggcatcgtg gtgtcacgct cgtcgtttgg tatggcttca ttcagctccg gttcccaacg 5640
   atcaaggcga gttacatgat cccccatgtt gtgcaaaaaa gcggttagct ccttcggtcc 5700
   tccgatcgtt gtcagaagta agttggccgc agtgttatca ctcatggtta tggcagcact 5760
   gcataattct cttactgtca tgccatccgt aagatgcttt tctgtgactg gtgagtactc 5820
   aaccaagtca ttctgagaat agtgtatgcg gcgaccgagt tgctcttgcc cggcgtcaat 5880
   acgggataat accgcgccac atagcagaac tttaaaagtg ctcatcattg gaaaacgttc 5940
   ttcggggcga aaactctcaa ggatcttacc gctgttgaga tccagttcga tgtaacccac 6000
   tcgtgcaccc aactgatctt cagcatcttt tactttcacc agcgtttctg ggtgagcaaa 6060
   aacaggaagg caaaatgccg caaaaaaggg aataagggcg acacggaaat gttgaatact 6120
   catactcttc ctttttcaat attattgaag catttatcag ggttattgtc tcatgagcgg 6180
   atacatattt gaatgtattt agaaaaataa acaaataggg gttccgcgca catttccccg 6240
   aaaagtgcca cctgacgtc                                               6259
<210> 54
   <211> 245
   <212> PRT
   <213> Artificial Sequence
<220>
   <223> GFP amino acid sequence.
<400> 54
   Ala Ser Lys Gly Glu Glu Leu Phe Thr Gly Val Val Pro Ile Leu Val
    1               5                  10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Leu
       50                  55                  60
   Cys Tyr Gly Val Gln Cys Phe Ser Arg Tyr Pro Asp His Met Lys Arg
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Thr Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Asn Ile Asp
   225                 230                 235                 240
   Gly Gly Gly Gly Gly
                   245
<210> 55
   <211> 4
   <212> PRT
   <213> Artificial Sequence
<220>
   <223> G-spacer flexible spacer
<400> 55
   Gly Gly Gly Gly
   1
<210> 56
   <211> 5
   <212> PRT
   <213> Artificial Sequence
<220>
   <223> G-spacer flexible spacer
<400> 56
   Gly Gly Gly Gly Ser
   1 5
<210> 57
   <211> 4
   <212> PRT
   <213> Artificial Sequence
<220>
   <223> A-spacer flexible spacer
<400> 57
   Ala Ala Ala Ala
   1
<210> 58
   <211> 5
   <212> PRT
   <213> Artificial Sequence
<220>
   <223> A-spacer flexible spacer
<400> 58
   Ala Ala Ala Ala Val
   1 5
<210> 59
   <211> 821
   <212> PRT
   <213> Homo sapiens
<400> 59
<210> 60
   <211> 822
   <212> PRT
   <213> Homo sapiens
<400> 60
   Met Val Ser Trp Gly Arg Phe Ile Cys Leu Val Val Val Thr Met Ala
   1               5                   10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Glu Arg Ser Pro His Arg Pro
   245 250 255
   Ile Leu Gln Ala Gly Leu Pro Ala Asn Ala Ser Thr Val Val Gly Gly
   260 265 270
   Asp Val Glu Phe Val Cys Lys Val Tyr Ser Asp Ala Gln Pro His Ile
   275 280 285
   Gln Trp Ile Lys His Val Glu Lys Asn Gly Ser Lys Tyr Gly Pro Asp
   290 295 300
   Gly Leu Pro Tyr Leu Lys Val Leu Lys His Ser Gly Ile Asn Ser Ser
   305 310 315 320
   Asn Ala Glu Val Leu Ala Leu Phe Asn Val Thr Glu Ala Asp Ala Gly
   325 330 335
   Glu Tyr Ile Cys Lys Val Ser Asn Tyr Ile Gly Gln Ala Asn Gln Ser
   340 345 350
   Ala Trp Leu Thr Val Leu Pro Lys Gln Gln Ala Pro Gly Arg Glu Lys
   355 360 365
   Glu Ile Thr Ala Ser Pro Asp Tyr Leu Glu Ile Ala Ile Tyr Cys Ile
   370 375 380
   Gly Val Phe Leu Ile Ala Cys Met Val Val Thr Val Ile Leu Cys Arg
   385 390 395 400
   Met Lys Asn Thr Thr Lys Lys Pro Asp Phe Ser Ser Gln Pro Ala Val
   405 410 415
   His Lys Leu Thr Lys Arg Ile Pro Leu Arg Arg Gln Val Thr Val Ser
   420 425 430
   Ala Glu Ser Ser Ser Ser Met Asn Ser Asn Thr Pro Leu Val Arg Ile
   435 440 445
   Thr Thr Arg Leu Ser Ser Thr Ala Asp Thr Pro Met Leu Ala Gly Val
   450 455 460
   Ser Glu Tyr Glu Leu Pro Glu Asp Pro Lys Trp Glu Phe Pro Arg Asp
   465 470 475 480
   Lys Leu Thr Leu Gly Lys Pro Leu Gly Glu Gly Cys Phe Gly Gln Val
   485 490 495
   Val Met Ala Glu Ala Val Gly Ile Asp Lys Asp Lys Pro Lys Glu Ala
   500 505 510
   Val Thr Val Ala Val Lys Met Leu Lys Asp Asp Ala Thr Glu Lys Asp
   515 520 525
   Leu Ser Asp Leu Val Ser Glu Met Glu Met Met Lys Met Ile Gly Lys
   530 535 540
   His Lys Asn Ile Ile Asn Leu Leu Gly Ala Cys Thr Gln Asp Gly Pro
   545 550 555 560
   Leu Tyr Val Ile Val Glu Tyr Ala Ser Lys Gly Asn Leu Arg Glu Tyr
   565 570 575
   Leu Arg Ala Arg Arg Pro Pro Gly Met Glu Tyr Ser Tyr Asp Ile Asn
   580 585 590
   Arg Val Pro Glu Glu Gln Met Thr Phe Lys Asp Leu Val Ser Cys Thr
   595 600 605
   Tyr Gln Leu Ala Arg Gly Met Glu Tyr Leu Ala Ser Gln Lys Cys Ile
   610 615 620
   His Arg Asp Leu Ala Ala Arg Asn Val Leu Val Thr Glu Asn Asn Val
   625 630 635 640
   Met Lys Ile Ala Asp Phe Gly Leu Ala Arg Asp Ile Asn Asn Ile Asp
   645 650 655
   Tyr Tyr Lys Lys Thr Thr Asn Gly Arg Leu Pro Val Lys Trp Met Ala
   660 665 670
   Pro Glu Ala Leu Phe Asp Arg Val Tyr Thr His Gln Ser Asp Val Trp
   675 680 685
   Ser Phe Gly Val Leu Met Trp Glu Ile Phe Thr Leu Gly Gly Ser Pro
   690 695 700
   Tyr Pro Gly Ile Pro Val Glu Glu Leu Phe Lys Leu Leu Lys Glu Gly
   705 710 715 720
   His Arg Met Asp Lys Pro Ala Asn Cys Thr Asn Glu Leu Tyr Met Met
   725 730 735
   Met Arg Asp Cys Trp His Ala Val Pro Ser Gln Arg Pro Thr Phe Lys
   740 745 750
   Gln Leu Val Glu Asp Leu Asp Arg Ile Leu Thr Leu Thr Thr Asn Glu
   755 760 765
   Glu Tyr Leu Asp Leu Ser Gln Pro Leu Glu Gln Tyr Ser Pro Ser Tyr
       770                 775                 780
   Pro Asp Thr Arg Ser Ser Cys Ser Ser Gly Asp Asp Ser Val Phe Ser
   785                 790                 795                 800
   Pro Asp Pro Met Pro Tyr Glu Pro Cys Leu Pro Gln Tyr Pro His Ile
                   805                 810                 815
   Asn Gly Ser Val Lys Thr
               820
<210> 61
   <211> 769
   <212> PRT
   <213> Homo sapiens
<400> 61
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Glu Arg Ser Pro His Arg Pro
                   245                 250                 255
   Ile Leu Gln Ala Gly Leu Pro Ala Asn Ala Ser Thr Val Val Gly Gly
               260                 265                 270
   Asp Val Glu Phe Val Cys Lys Val Tyr Ser Asp Ala Gln Pro His Ile
           275                 280                 285
   Gln Trp Ile Lys His Val Glu Lys Asn Gly Ser Lys Tyr Gly Pro Asp
       290                 295                 300
   Gly Leu Pro Tyr Leu Lys Val Leu Lys His Ser Gly Ile Asn Ser Ser
   305                 310                 315                 320
   Asn Ala Glu Val Leu Ala Leu Phe Asn Val Thr Glu Ala Asp Ala Gly
                   325                 330                 335
   Glu Tyr Ile Cys Lys Val Ser Asn Tyr Ile Gly Gln Ala Asn Gln Ser
               340                 345                 350
   Ala Trp Leu Thr Val Leu Pro Lys Gln Gln Ala Pro Gly Arg Glu Lys
           355                 360                 365
   Glu Ile Thr Ala Ser Pro Asp Tyr Leu Glu Ile Ala Ile Tyr Cys Ile
       370                 375                 380
   Gly Val Phe Leu Ile Ala Cys Met Val Val Thr Val Ile Leu Cys Arg
   385                 390                 395                 400
   Met Lys Asn Thr Thr Lys Lys Pro Asp Phe Ser Ser Gln Pro Ala Val
                   405                 410                 415
   His Lys Leu Thr Lys Arg Ile Pro Leu Arg Arg Gln Val Thr Val Ser
               420                 425                 430
   Ala Glu Ser Ser Ser Ser Met Asn Ser Asn Thr Pro Leu Val Arg Ile
           435                 440                 445
   Thr Thr Arg Leu Ser Ser Thr Ala Asp Thr Pro Met Leu Ala Gly Val
       450                 455                 460
   Ser Glu Tyr Glu Leu Pro Glu Asp Pro Lys Trp Glu Phe Pro Arg Asp
   465                 470                 475                 480
   Lys Leu Thr Leu Gly Lys Pro Leu Gly Glu Gly Cys Phe Gly Gln Val
                   485                 490                 495
   Val Met Ala Glu Ala Val Gly Ile Asp Lys Asp Lys Pro Lys Glu Ala
               500                 505                 510
   Val Thr Val Ala Val Lys Met Leu Lys Asp Asp Ala Thr Glu Lys Asp
           515                 520                 525
   Leu Ser Asp Leu Val Ser Glu Met Glu Met Met Lys Met Ile Gly Lys
       530                 535                 540
   His Lys Asn Ile Ile Asn Leu Leu Gly Ala Cys Thr Gln Asp Gly Pro
   545                 550                 555                 560
   Leu Tyr Val Ile Val Glu Tyr Ala Ser Lys Gly Asn Leu Arg Glu Tyr
                   565                 570                 575
   Leu Arg Ala Arg Arg Pro Pro Gly Met Glu Tyr Ser Tyr Asp Ile Asn
               580                 585                 590
   Arg Val Pro Glu Glu Gln Met Thr Phe Lys Asp Leu Val Ser Cys Thr
           595                 600                 605
   Tyr Gln Leu Ala Arg Gly Met Glu Tyr Leu Ala Ser Gln Lys Cys Ile
       610                 615                 620
   His Arg Asp Leu Ala Ala Arg Asn Val Leu Val Thr Glu Asn Asn Val
   625                 630                 635                 640
   Met Lys Ile Ala Asp Phe Gly Leu Ala Arg Asp Ile Asn Asn Ile Asp
                   645                 650                 655
   Tyr Tyr Lys Lys Thr Thr Asn Gly Arg Leu Pro Val Lys Trp Met Ala
               660                 665                 670
   Pro Glu Ala Leu Phe Asp Arg Val Tyr Thr His Gln Ser Asp Val Trp
           675                 680                 685
   Ser Phe Gly Val Leu Met Trp Glu Ile Phe Thr Leu Gly Gly Ser Pro
       690                 695                 700
   Tyr Pro Gly Ile Pro Val Glu Glu Leu Phe Lys Leu Leu Lys Glu Gly
   705                 710                 715                 720
   His Arg Met Asp Lys Pro Ala Asn Cys Thr Asn Glu Leu Tyr Met Met
                   725                 730                 735
   Met Arg Asp Cys Trp His Ala Val Pro Ser Gln Arg Pro Thr Phe Lys
               740                 745                 750
   Gln Leu Val Glu Asp Leu Asp Arg Ile Leu Thr Leu Thr Thr Asn Glu
           755                 760                 765
   Ile
<210> 62
   <211> 709
   <212> PRT
   <213> Homo sapiens
<400> 62
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Ala Pro Gly Arg Glu Lys Glu
                   245                 250                 255
   Ile Thr Ala Ser Pro Asp Tyr Leu Glu Ile Ala Ile Tyr Cys Ile Gly
               260                 265                 270
   Val Phe Leu Ile Ala Cys Met Val Val Thr Val Ile Leu Cys Arg Met
           275                 280                 285
   Lys Asn Thr Thr Lys Lys Pro Asp Phe Ser Ser Gln Pro Ala Val His
       290                 295                 300
   Lys Leu Thr Lys Arg Ile Pro Leu Arg Arg Gln Val Thr Val Ser Ala
   305                 310                 315                 320
   Glu Ser Ser Ser Ser Met Asn Ser Asn Thr Pro Leu Val Arg Ile Thr
                   325                 330                 335
   Thr Arg Leu Ser Ser Thr Ala Asp Thr Pro Met Leu Ala Gly Val Ser
               340                 345                 350
   Glu Tyr Glu Leu Pro Glu Asp Pro Lys Trp Glu Phe Pro Arg Asp Lys
           355                 360                 365
   Leu Thr Leu Gly Lys Pro Leu Gly Glu Gly Cys Phe Gly Gln Val Val
       370                 375                 380
   Met Ala Glu Ala Val Gly Ile Asp Lys Asp Lys Pro Lys Glu Ala Val
   385                 390                 395                 400
   Thr Val Ala Val Lys Met Leu Lys Asp Asp Ala Thr Glu Lys Asp Leu
                   405                 410                 415
   Ser Asp Leu Val Ser Glu Met Glu Met Met Lys Met Ile Gly Lys His
               420                 425                 430
   Lys Asn Ile Ile Asn Leu Leu Gly Ala Cys Thr Gln Asp Gly Pro Leu
           435                 440                 445
   Tyr Val Ile Val Glu Tyr Ala Ser Lys Gly Asn Leu Arg Glu Tyr Leu
       450                 455                 460
   Arg Ala Arg Arg Pro Pro Gly Met Glu Tyr Ser Tyr Asp Ile Asn Arg
   465                 470                 475                 480
   Val Pro Glu Glu Gln Met Thr Phe Lys Asp Leu Val Ser Cys Thr Tyr
                   485                 490                 495
   Gln Leu Ala Arg Gly Met Glu Tyr Leu Ala Ser Gln Lys Cys Ile His
               500                 505                 510
   Arg Asp Leu Ala Ala Arg Asn Val Leu Val Thr Glu Asn Asn Val Met
           515                 520                 525
   Lys Ile Ala Asp Phe Gly Leu Ala Arg Asp Ile Asn Asn Ile Asp Tyr
       530                 535                 540
   Tyr Lys Lys Thr Thr Asn Gly Arg Leu Pro Val Lys Trp Met Ala Pro
   545                 550                 555                 560
   Glu Ala Leu Phe Asp Arg Val Tyr Thr His Gln Ser Asp Val Trp Ser
                   565                 570                 575
   Phe Gly Val Leu Met Trp Glu Ile Phe Thr Leu Gly Gly Ser Pro Tyr
                   580                 585                 590
   Pro Gly Ile Pro Val Glu Glu Leu Phe Lys Leu Leu Lys Glu Gly His
               595                 600                 605
   Arg Met Asp Lys Pro Ala Asn Cys Thr Asn Glu Leu Tyr Met Met Met
           610                 615                 620
   Arg Asp Cys Trp His Ala Val Pro Ser Gln Arg Pro Thr Phe Lys Gln
   625                 630                 635                 640
   Leu Val Glu Asp Leu Asp Arg Ile Leu Thr Leu Thr Thr Asn Glu Glu
                   645                 650                 655
   Tyr Leu Asp Leu Ser Gln Pro Leu Glu Gln Tyr Ser Pro Ser Tyr Pro
               660                 665                 670
   Asp Thr Arg Ser Ser Cys Ser Ser Gly Asp Asp Ser Val Phe Ser Pro
           675                 680                 685
   Asp Pro Met Pro Tyr Glu Pro Cys Leu Pro Gln Tyr Pro His Ile Asn
       690                 695                 700
   Gly Ser Val Lys Thr
   705
<210> 63
   <211> 707
   <212> PRT
   <213> Homo sapiens
<400> 63
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Asp Ala Ile Ser Ser Gly Asp Asp Glu Asp Asp Thr
           35                  40                  45
   Asp Gly Ala Glu Asp Phe Val Ser Glu Asn Ser Asn Asn Lys Arg Ala
       50                  55                  60
   Pro Tyr Trp Thr Asn Thr Glu Lys Met Glu Lys Arg Leu His Ala Val
   65                  70                  75                  80
   Pro Ala Ala Asn Thr Val Lys Phe Arg Cys Pro Ala Gly Gly Asn Pro
                   85                  90                  95
   Met Pro Thr Met Arg Trp Leu Lys Asn Gly Lys Glu Phe Lys Gln Glu
               100                 105                 110
   His Arg Ile Gly Gly Tyr Lys Val Arg Asn Gln His Trp Ser Leu Ile
           115                 120                 125
   Met Glu Ser Val Val Pro Ser Asp Lys Gly Asn Tyr Thr Cys Val Val
       130                 135                 140
   Glu Asn Glu Tyr Gly Ser Ile Asn His Thr Tyr His Leu Asp Val Val
   145                 150                 155                 160
   Glu Arg Ser Pro His Arg Pro Ile Leu Gln Ala Gly Leu Pro Ala Asn
                   165                 170                 175
   Ala Ser Thr Val Val Gly Gly Asp Val Glu Phe Val Cys Lys Val Tyr
               180                 185                 190
   Ser Asp Ala Gln Pro His Ile Gln Trp Ile Lys His Val Glu Lys Asn
           195                 200                 205
   Gly Ser Lys Tyr Gly Pro Asp Gly Leu Pro Tyr Leu Lys Val Leu Lys
       210                 215                 220
   Ala Ala Gly Val Asn Thr Thr Asp Lys Glu Ile Glu Val Leu Tyr Ile
   225                 230                 235                 240
   Arg Asn Val Thr Phe Glu Asp Ala Gly Glu Tyr Thr Cys Leu Ala Gly
                   245                 250                 255
   Asn Ser Ile Gly Ile Ser Phe His Ser Ala Trp Leu Thr Val Leu Pro
               260                 265                 270
   Ala Pro Gly Arg Glu Lys Glu Ile Thr Ala Ser Pro Asp Tyr Leu Glu
           275                 280                 285
   Ile Ala Ile Tyr Cys Ile Gly Val Phe Leu Ile Ala Cys Met Val Val
       290                 295                 300
   Thr Val Ile Leu Cys Arg Met Lys Asn Thr Thr Lys Lys Pro Asp Phe
   305                 310                 315                 320
   Ser Ser Gln Pro Ala Val His Lys Leu Thr Lys Arg Ile Pro Leu Arg
   325 330 335
   Arg Gln Val Thr Val Ser Ala Glu Ser Ser Ser Ser Met Asn Ser Asn
   340 345 350
   Thr Pro Leu Val Arg Ile Thr Thr Arg Leu Ser Ser Thr Ala Asp Thr
   355 360 365
   Pro Met Leu Ala Gly Val Ser Glu Tyr Glu Leu Pro Glu Asp Pro Lys
   370 375 380
   Trp Glu Phe Pro Arg Asp Lys Leu Thr Leu Gly Lys Pro Leu Gly Glu
   385 390 395 400
   Gly Cys Phe Gly Gln Val Val Met Ala Glu Ala Val Gly Ile Asp Lys
   405 410 415
   Asp Lys Pro Lys Glu Ala Val Thr Val Ala Val Lys Met Leu Lys Asp
   420 425 430
   Asp Ala Thr Glu Lys Asp Leu Ser Asp Leu Val Ser Glu Met Glu Met
   435 440 445
   Met Lys Met Ile Gly Lys His Lys Asn Ile Ile Asn Leu Leu Gly Ala
   450 455 460
   Cys Thr Gln Asp Gly Pro Leu Tyr Val Ile Val Glu Tyr Ala Ser Lys
   465 470 475 480
   Gly Asn Leu Arg Glu Tyr Leu Arg Ala Arg Arg Pro Pro Gly Met Glu
   485 490 495
   Tyr Ser Tyr Asp Ile Asn Arg Val Pro Glu Glu Gln Met Thr Phe Lys
   500 505 510
   Asp Leu Val Ser Cys Thr Tyr Gln Leu Ala Arg Gly Met Glu Tyr Leu
   515 520 525
   Ala Ser Gln Lys Cys Ile His Arg Asp Leu Ala Ala Arg Asn Val Leu
   530 535 540
   Val Thr Glu Asn Asn Val Met Lys Ile Ala Asp Phe Gly Leu Ala Arg
   545 550 555 560
   Asp Ile Asn Asn Ile Asp Tyr Tyr Lys Lys Thr Thr Asn Gly Arg Leu
   565 570 575
   Pro Val Lys Trp Met Ala Pro Glu Ala Leu Phe Asp Arg Val Tyr Thr
   580 585 590
   His Gln Ser Asp Val Trp Ser Phe Gly Val Leu Met Trp Glu Ile Phe
   595 600 605
   Thr Leu Gly Gly Ser Pro Tyr Pro Gly Ile Pro Val Glu Glu Leu Phe
   610 615 620
   Lys Leu Leu Lys Glu Gly His Arg Met Asp Lys Pro Ala Asn Cys Thr
   625 630 635 640
   Asn Glu Leu Tyr Met Met Met Arg Asp Cys Trp His Ala Val Pro Ser
   645 650 655
   Gln Arg Pro Thr Phe Lys Gln Leu Val Glu Asp Leu Asp Arg Ile Leu
   660 665 670
   Thr Leu Thr Thr Asn Glu Glu Glu Lys Lys Val Ser Gly Ala Val Asp
   675 680 685
   Cys His Lys Pro Pro Cys Asn Pro Ser His Leu Pro Cys Val Leu Ala
   690 695 700
   Val Asp Gln
   705
<210> 64
   <211> 706
   <212> PRT
   <213> Homo sapiens
<400> 64
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Gly Ala Pro Tyr Trp Thr Asn Thr Glu Lys Met Glu
           35                  40                  45
   Lys Arg Leu His Ala Val Pro Ala Ala Asn Thr Val Lys Phe Arg Cys
       50                  55                  60
   Pro Ala Gly Gly Asn Pro Met Pro Thr Met Arg Trp Leu Lys Asn Gly
   65                  70                  75                  80
   Lys Glu Phe Lys Gln Glu His Arg Ile Gly Gly Tyr Lys Val Arg Asn
                   85                  90                  95
   Gln His Trp Ser Leu Ile Met Glu Ser Val Val Pro Ser Asp Lys Gly
               100                 105                 110
   Asn Tyr Thr Cys Val Val Glu Asn Glu Tyr Gly Ser Ile Asn His Thr
           115                 120                 125
   Tyr His Leu Asp Val Val Glu Arg Ser Pro His Arg Pro Ile Leu Gln
       130                 135                 140
   Ala Gly Leu Pro Ala Asn Ala Ser Thr Val Val Gly Gly Asp Val Glu
   145                 150                 155                 160
   Phe Val Cys Lys Val Tyr Ser Asp Ala Gln Pro His Ile Gln Trp Ile
                   165                 170                 175
   Lys His Val Glu Lys Asn Gly Ser Lys Tyr Gly Pro Asp Gly Leu Pro
               180                 185                 190
   Tyr Leu Lys Val Leu Lys Ala Ala Gly Val Asn Thr Thr Asp Lys Glu
           195                 200                 205
   Ile Glu Val Leu Tyr Ile Arg Asn Val Thr Phe Glu Asp Ala Gly Glu
       210                 215                 220
   Tyr Thr Cys Leu Ala Gly Asn Ser Ile Gly Ile Ser Phe His Ser Ala
   225                 230                 235                 240
   Trp Leu Thr Val Leu Pro Ala Pro Gly Arg Glu Lys Glu Ile Thr Ala
                   245                 250                 255
   Ser Pro Asp Tyr Leu Glu Ile Ala Ile Tyr Cys Ile Gly Val Phe Leu
               260                 265                 270
   Ile Ala Cys Met Val Val Thr Val Ile Leu Cys Arg Met Lys Asn Thr
           275                 280                 285
   Thr Lys Lys Pro Asp Phe Ser Ser Gln Pro Ala Val His Lys Leu Thr
       290                 295                 300
   Lys Arg Ile Pro Leu Arg Arg Gln Val Thr Val Ser Ala Glu Ser Ser
   305                 310                 315                 320
   Ser Ser Met Asn Ser Asn Thr Pro Leu Val Arg Ile Thr Thr Arg Leu
                   325                 330                 335
   Ser Ser Thr Ala Asp Thr Pro Met Leu Ala Gly Val Ser Glu Tyr Glu
               340                 345                 350
   Leu Pro Glu Asp Pro Lys Trp Glu Phe Pro Arg Asp Lys Leu Thr Leu
           355                 360                 365
   Gly Lys Pro Leu Gly Glu Gly Cys Phe Gly Gln Val Val Met Ala Glu
       370                 375                 380
   Ala Val Gly Ile Asp Lys Asp Lys Pro Lys Glu Ala Val Thr Val Ala
   385                 390                 395                 400
   Val Lys Met Leu Lys Asp Asp Ala Thr Glu Lys Asp Leu Ser Asp Leu
                   405                 410                 415
   Val Ser Glu Met Glu Met Met Lys Met Ile Gly Lys His Lys Asn Ile
               420                 425                 430
   Ile Asn Leu Leu Gly Ala Cys Thr Gln Asp Gly Pro Leu Tyr Val Ile
           435                 440                 445
   Val Glu Tyr Ala Ser Lys Gly Asn Leu Arg Glu Tyr Leu Arg Ala Arg
       450                 455                 460
   Arg Pro Pro Gly Met Glu Tyr Ser Tyr Asp Ile Asn Arg Val Pro Glu
   465                 470                 475                 480
   Glu Gln Met Thr Phe Lys Asp Leu Val Ser Cys Thr Tyr Gln Leu Ala
                   485                 490                 495
   Arg Gly Met Glu Tyr Leu Ala Ser Gln Lys Cys Ile His Arg Asp Leu
               500                 505                 510
   Ala Ala Arg Asn Val Leu Val Thr Glu Asn Asn Val Met Lys Ile Ala
           515                 520                 525
   Asp Phe Gly Leu Ala Arg Asp Ile Asn Asn Ile Asp Tyr Tyr Lys Lys
       530                 535                 540
   Thr Thr Asn Gly Arg Leu Pro Val Lys Trp Met Ala Pro Glu Ala Leu
   545                 550                 555                 560
   Phe Asp Arg Val Tyr Thr His Gln Ser Asp Val Trp Ser Phe Gly Val
                   565                 570                 575
   Leu Met Trp Glu Ile Phe Thr Leu Gly Gly Ser Pro Tyr Pro Gly Ile
               580                 585                 590
   Pro Val Glu Glu Leu Phe Lys Leu Leu Lys Glu Gly His Arg Met Asp
           595                 600                 605
   Lys Pro Ala Asn Cys Thr Asn Glu Leu Tyr Met Met Met Arg Asp Cys
       610                 615                 620
   Trp His Ala Val Pro Ser Gln Arg Pro Thr Phe Lys Gln Leu Val Glu
   625                 630                 635                 640
   Asp Leu Asp Arg Ile Leu Thr Leu Thr Thr Asn Glu Glu Tyr Leu Asp
                   645                 650                 655
   Leu Ser Gln Pro Leu Glu Gln Tyr Ser Pro Ser Tyr Pro Asp Thr Arg
               660                 665                 670
   Ser Ser Cys Ser Ser Gly Asp Asp Ser Val Phe Ser Pro Asp Pro Met
           675                 680                 685
   Pro Tyr Glu Pro Cys Leu Pro Gln Tyr Pro His Ile Asn Gly Ser Val
       690                 695                 700
   Lys Thr
   705
<210> 65
   <211> 705
   <212> PRT
   <213> Homo sapiens
<400> 65
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Glu Arg Ser Pro His Arg Pro
                   245                 250                 255
   Ile Leu Gln Ala Gly Leu Pro Ala Asn Ala Ser Thr Val Val Gly Gly
               260                 265                 270
   Asp Val Glu Phe Val Cys Lys Val Tyr Ser Asp Ala Gln Pro His Ile
           275                 280                 285
   Gln Trp Ile Lys His Val Glu Lys Asn Gly Ser Lys Tyr Gly Pro Asp
       290                 295                 300
   Gly Leu Pro Tyr Leu Lys Val Leu Lys Val Ser Ala Glu Ser Ser Ser
   305                 310                 315                 320
   Ser Met Asn Ser Asn Thr Pro Leu Val Arg Ile Thr Thr Arg Leu Ser
                   325                 330                 335
   Ser Thr Ala Asp Thr Pro Met Leu Ala Gly Val Ser Glu Tyr Glu Leu
               340                 345                 350
   Pro Glu Asp Pro Lys Trp Glu Phe Pro Arg Asp Lys Leu Thr Leu Gly
           355                 360                 365
   Lys Pro Leu Gly Glu Gly Cys Phe Gly Gln Val Val Met Ala Glu Ala
       370                 375                 380
   Val Gly Ile Asp Lys Asp Lys Pro Lys Glu Ala Val Thr Val Ala Val
   385                 390                 395                 400
   Lys Met Leu Lys Asp Asp Ala Thr Glu Lys Asp Leu Ser Asp Leu Val
                   405                 410                 415
   Ser Glu Met Glu Met Met Lys Met Ile Gly Lys His Lys Asn Ile Ile
               420                 425                 430
   Asn Leu Leu Gly Ala Cys Thr Gln Asp Gly Pro Leu Tyr Val Ile Val
           435                 440                 445
   Glu Tyr Ala Ser Lys Gly Asn Leu Arg Glu Tyr Leu Arg Ala Arg Arg
       450                 455                 460
   Pro Pro Gly Met Glu Tyr Ser Tyr Asp Ile Asn Arg Val Pro Glu Glu
   465                 470                 475                 480
   Gln Met Thr Phe Lys Asp Leu Val Ser Cys Thr Tyr Gln Leu Ala Arg
                   485                 490                 495
   Gly Met Glu Tyr Leu Ala Ser Gln Lys Cys Ile His Arg Asp Leu Ala
               500                 505                 510
   Ala Arg Asn Val Leu Val Thr Glu Asn Asn Val Met Lys Ile Ala Asp
           515                 520                 525
   Phe Gly Leu Ala Arg Asp Ile Asn Asn Ile Asp Tyr Tyr Lys Lys Thr
       530                 535                 540
   Thr Asn Gly Arg Leu Pro Val Lys Trp Met Ala Pro Glu Ala Leu Phe
   545                 550                 555                 560
   Asp Arg Val Tyr Thr His Gln Ser Asp Val Trp Ser Phe Gly Val Leu
                   565                 570                 575
   Met Trp Glu Ile Phe Thr Leu Gly Gly Ser Pro Tyr Pro Gly Ile Pro
               580                 585                 590
   Val Glu Glu Leu Phe Lys Leu Leu Lys Glu Gly His Arg Met Asp Lys
           595                 600                 605
   Pro Ala Asn Cys Thr Asn Glu Leu Tyr Met Met Met Arg Asp Cys Trp
       610                 615                 620
   His Ala Val Pro Ser Gln Arg Pro Thr Phe Lys Gln Leu Val Glu Asp
   625                 630                 635                 640
   Leu Asp Arg Ile Leu Thr Leu Thr Thr Asn Glu Glu Tyr Leu Asp Leu
                   645                 650                 655
   Ser Gln Pro Leu Glu Gln Tyr Ser Pro Ser Tyr Pro Asp Thr Arg Ser
               660                 665                 670
   Ser Cys Ser Ser Gly Asp Asp Ser Val Phe Ser Pro Asp Pro Met Pro
           675                 680                 685
   Tyr Glu Pro Cys Leu Pro Gln Tyr Pro His Ile Asn Gly Ser Val Lys
       690                 695                 700
   Thr
   705
<210> 66
   <211> 704
   <212> PRT
   <213> Homo sapiens
<400> 66
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Gly Ala Pro Tyr Trp Thr Asn Thr Glu Lys Met Glu
           35                  40                  45
   Lys Arg Leu His Ala Val Pro Ala Ala Asn Thr Val Lys Phe Arg Cys
       50                  55                  60
   Pro Ala Gly Gly Asn Pro Met Pro Thr Met Arg Trp Leu Lys Asn Gly
   65                  70                  75                  80
   Lys Glu Phe Lys Gln Glu His Arg Ile Gly Gly Tyr Lys Val Arg Asn
                   85                  90                  95
   Gln His Trp Ser Leu Ile Met Glu Ser Val Val Pro Ser Asp Lys Gly
               100                 105                 110
   Asn Tyr Thr Cys Val Val Glu Asn Glu Tyr Gly Ser Ile Asn His Thr
           115                 120                 125
   Tyr His Leu Asp Val Val Glu Arg Ser Pro His Arg Pro Ile Leu Gln
       130                 135                 140
   Ala Gly Leu Pro Ala Asn Ala Ser Thr Val Val Gly Gly Asp Val Glu
   145                 150                 155                 160
   Phe Val Cys Lys Val Tyr Ser Asp Ala Gln Pro His Ile Gln Trp Ile
                   165                 170                 175
   Lys His Val Glu Lys Asn Gly Ser Lys Tyr Gly Pro Asp Gly Leu Pro
               180                 185                 190
   Tyr Leu Lys Val Leu Lys Ala Ala Gly Val Asn Thr Thr Asp Lys Glu
           195                 200                 205
   Ile Glu Val Leu Tyr Ile Arg Asn Val Thr Phe Glu Asp Ala Gly Glu
       210                 215                 220
   Tyr Thr Cys Leu Ala Gly Asn Ser Ile Gly Ile Ser Phe His Ser Ala
   225                 230                 235                 240
   Trp Leu Thr Val Leu Pro Ala Pro Gly Arg Glu Lys Glu Ile Thr Ala
                   245                 250                 255
   Ser Pro Asp Tyr Leu Glu Ile Ala Ile Tyr Cys Ile Gly Val Phe Leu
               260                 265                 270
   Ile Ala Cys Met Val Val Thr Val Ile Leu Cys Arg Met Lys Asn Thr
           275                 280                 285
   Thr Lys Lys Pro Asp Phe Ser Ser Gln Pro Ala Val His Lys Leu Thr
       290                 295                 300
   Lys Arg Ile Pro Leu Arg Arg Gln Val Ser Ala Glu Ser Ser Ser Ser
   305                 310                 315                 320
   Met Asn Ser Asn Thr Pro Leu Val Arg Ile Thr Thr Arg Leu Ser Ser
                   325                 330                 335
   Thr Ala Asp Thr Pro Met Leu Ala Gly Val Ser Glu Tyr Glu Leu Pro
               340                 345                 350
   Glu Asp Pro Lys Trp Glu Phe Pro Arg Asp Lys Leu Thr Leu Gly Lys
           355                 360                 365
   Pro Leu Gly Glu Gly Cys Phe Gly Gln Val Val Met Ala Glu Ala Val
       370                 375                 380
   Gly Ile Asp Lys Asp Lys Pro Lys Glu Ala Val Thr Val Ala Val Lys
   385                 390                 395                 400
   Met Leu Lys Asp Asp Ala Thr Glu Lys Asp Leu Ser Asp Leu Val Ser
                   405                 410                 415
   Glu Met Glu Met Met Lys Met Ile Gly Lys His Lys Asn Ile Ile Asn
               420                 425                 430
   Leu Leu Gly Ala Cys Thr Gln Asp Gly Pro Leu Tyr Val Ile Val Glu
           435                 440                 445
   Tyr Ala Ser Lys Gly Asn Leu Arg Glu Tyr Leu Arg Ala Arg Arg Pro
       450                 455                 460
   Pro Gly Met Glu Tyr Ser Tyr Asp Ile Asn Arg Val Pro Glu Glu Gln
   465                 470                 475                 480
   Met Thr Phe Lys Asp Leu Val Ser Cys Thr Tyr Gln Leu Ala Arg Gly
                   485                 490                 495
   Met Glu Tyr Leu Ala Ser Gln Lys Cys Ile His Arg Asp Leu Ala Ala
               500                 505                 510
   Arg Asn Val Leu Val Thr Glu Asn Asn Val Met Lys Ile Ala Asp Phe
           515                 520                 525
   Gly Leu Ala Arg Asp Ile Asn Asn Ile Asp Tyr Tyr Lys Lys Thr Thr
       530                 535                 540
   Asn Gly Arg Leu Pro Val Lys Trp Met Ala Pro Glu Ala Leu Phe Asp
   545                 550                 555                 560
   Arg Val Tyr Thr His Gln Ser Asp Val Trp Ser Phe Gly Val Leu Met
                       565                     570                     575
   Trp Glu Ile Phe Thr Leu Gly Gly Ser Pro Tyr Pro Gly Ile Pro Val
                   580                     585                     590
   Glu Glu Leu Phe Lys Leu Leu Lys Glu Gly His Arg Met Asp Lys Pro
               595                     600                     605
   Ala Asn Cys Thr Asn Glu Leu Tyr Met Met Met Arg Asp Cys Trp His
       610                     615                     620
   Ala Val Pro Ser Gln Arg Pro Thr Phe Lys Gln Leu Val Glu Asp Leu
   625                     630                     635                     640
   Asp Arg Ile Leu Thr Leu Thr Thr Asn Glu Glu Tyr Leu Asp Leu Ser
                       645                     650                     655
   Gln Pro Leu Glu Gln Tyr Ser Pro Ser Tyr Pro Asp Thr Arg Ser Ser
                   660                     665                     670
   Cys Ser Ser Gly Asp Asp Ser Val Phe Ser Pro Asp Pro Met Pro Tyr
               675                     680                     685
   Glu Pro Cys Leu Pro Gln Tyr Pro His Ile Asn Gly Ser Val Lys Thr
       690                     695                     700
<210> 67
   <211> 680
   <212> PRT
   <213> Homo sapiens
<400> 67
   Met Val Ser Trp Gly Arg Phe Ile Cys Leu Val Val Val Thr Met Ala
   1 5 10 15
   Thr Leu Ser Leu Ala Arg Pro Ser Phe Ser Leu Val Glu Asp Thr Thr
   20 25 30
   Leu Glu Pro Glu Asp Ala Ile Ser Ser Gly Asp Asp Glu Asp Asp Thr
   35 40 45
   Asp Gly Ala Glu Asp Phe Val Ser Glu Asn Ser Asn Asn Lys Arg Ala
   50 55 60
   Pro Tyr Trp Thr Asn Thr Glu Lys Met Glu Lys Arg Leu His Ala Val
   65 70 75 80
   Pro Ala Ala Asn Thr Val Lys Phe Arg Cys Pro Ala Gly Gly Asn Pro
   85 90 95
   Met Pro Thr Met Arg Trp Leu Lys Asn Gly Lys Glu Phe Lys Gln Glu
   100 105 110
   His Arg Ile Gly Gly Tyr Lys Val Arg Asn Gln His Trp Ser Leu Ile
   115 120 125
   Met Glu Ser Val Val Pro Ser Asp Lys Gly Asn Tyr Thr Cys Val Val
   130 135 140
   Glu Asn Glu Tyr Gly Ser Ile Asn His Thr Tyr His Leu Asp Val Val
   145 150 155 160
   Glu Arg Ser Pro His Arg Pro Ile Leu Gln Ala Gly Leu Pro Ala Asn
   165 170 175
   Ala Ser Thr Val Val Gly Gly Asp Val Glu Phe Val Cys Lys Val Tyr
   180 185 190
   Ser Asp Ala Gln Pro His Ile Gln Trp Ile Lys His Val Glu Lys Asn
   195 200 205
   Gly Ser Lys Tyr Gly Pro Asp Gly Leu Pro Tyr Leu Lys Val Leu Lys
   210 215 220
   His Ser Gly Ile Asn Ser Ser Asn Ala Glu Val Leu Ala Leu Phe Asn
   225 230 235 240
   Val Thr Glu Ala Asp Ala Gly Glu Tyr Ile Cys Lys Val Ser Asn Tyr
   245 250 255
   Ile Gly Gln Ala Asn Gln Ser Ala Trp Leu Thr Val Leu Pro Lys Gln
   260 265 270
   Gln Ala Pro Gly Arg Glu Lys Glu Ile Thr Ala Ser Pro Asp Tyr Leu
   275 280 285
   Glu Ile Ala Ile Tyr Cys Ile Gly Val Phe Leu Ile Ala Cys Met Val
   290 295 300
   Val Thr Val Ile Leu Cys Arg Met Lys Asn Thr Thr Lys Lys Pro Asp
   305 310 315 320
   Phe Ser Ser Gln Pro Ala Val His Lys Leu Thr Lys Arg Ile Pro Leu
                   325                 330                 335
   Arg Arg Gln Val Thr Val Ser Ala Glu Ser Ser Ser Ser Met Asn Ser
               340                 345                 350
   Asn Thr Pro Leu Val Arg Ile Thr Thr Arg Leu Ser Ser Thr Ala Asp
           355                 360                 365
   Thr Pro Met Leu Ala Gly Val Ser Glu Tyr Glu Leu Pro Glu Asp Pro
       370                 375                 380
   Lys Trp Glu Phe Pro Arg Asp Lys Leu Thr Leu Gly Lys Pro Leu Gly
   385                 390                 395                 400
   Glu Gly Cys Phe Gly Gln Val Val Met Ala Glu Ala Val Gly Ile Asp
                   405                 410                 415
   Lys Asp Lys Pro Lys Glu Ala Val Thr Val Ala Val Lys Met Leu Lys
               420                 425                 430
   Asp Asp Ala Thr Glu Lys Asp Leu Ser Asp Leu Val Ser Glu Met Glu
           435                 440                 445
   Met Met Lys Met Ile Gly Lys His Lys Asn Ile Ile Asn Leu Leu Gly
       450                 455                 460
   Ala Cys Thr Gln Asp Gly Pro Leu Tyr Val Ile Val Glu Tyr Ala Ser
   465                 470                 475                 480
   Lys Gly Asn Leu Arg Glu Tyr Leu Arg Ala Arg Arg Pro Pro Gly Met
                   485                 490                 495
   Glu Tyr Ser Tyr Asp Ile Asn Arg Val Pro Glu Glu Gln Met Thr Phe
               500                 505                 510
   Lys Asp Leu Val Ser Cys Thr Tyr Gln Leu Ala Arg Gly Met Glu Tyr
           515                 520                 525
   Leu Ala Ser Gln Lys Cys Ile His Arg Asp Leu Ala Ala Arg Asn Val
       530                 535                 540
   Leu Val Thr Glu Asn Asn Val Met Lys Ile Ala Asp Phe Gly Leu Ala
   545                 550                 555                 560
   Arg Asp Ile Asn Asn Ile Asp Tyr Tyr Lys Lys Thr Thr Asn Gly Arg
                   565                 570                 575
   Leu Pro Val Lys Trp Met Ala Pro Glu Ala Leu Phe Asp Arg Val Tyr
               580                 585                 590
   Thr His Gln Ser Asp Val Trp Ser Phe Gly Val Leu Met Trp Glu Ile
           595                 600                 605
   Phe Thr Leu Gly Gly Ser Pro Tyr Pro Gly Ile Pro Val Glu Glu Leu
       610                 615                 620
   Phe Lys Leu Leu Lys Glu Gly His Arg Met Asp Lys Pro Ala Asn Cys
   625                 630                 635                 640
   Thr Asn Glu Leu Tyr Met Met Met Arg Asp Cys Trp His Ala Val Pro
                   645                 650                 655
   Ser Gln Arg Pro Thr Phe Lys Gln Leu Val Glu Asp Leu Asp Arg Ile
               660                 665                 670
   Leu Thr Leu Thr Thr Asn Glu Ile
           675                 680
<210> 68
   <211> 396
   <212> PRT
   <213> Homo sapiens
<400> 68
<210> 69
   <211> 317
   <212> PRT
   <213> Homo sapiens
<400> 69
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Glu Arg Ser Pro His Arg Pro
                   245                 250                 255
   Ile Leu Gln Ala Gly Leu Pro Ala Asn Ala Ser Thr Val Val Gly Gly
               260                 265                 270
   Asp Val Glu Phe Val Cys Lys Val Tyr Ser Asp Ala Gln Pro His Ile
           275                 280                 285
   Gln Trp Ile Lys His Val Glu Lys Asn Gly Ser Lys Tyr Gly Pro Asp
       290                 295                 300
   Gly Leu Pro Tyr Leu Lys Val Leu Lys Val Arg Thr Phe
   305                 310                 315
<210> 70
   <211> 266
   <212> PRT
   <213> Homo sapiens
<400> 70
<210> 71
   <211> 336
   <212> DNA
   <213> Mus musculus
<400> 71
   caggtgaagc tgcaggagtc tggcgctgag ttggtgaaac ctggggcttc agtgaagata 60
   tcctgcaagg cttctggcta catcttcact gaccatgctc ttcactgggt gaggcagaag 120
   cctgaacagg gcctggaatg gattgggtat atttttcccg gaaatggtaa tattgagtac 180
   aatgagaagt tcaagggcaa ggccacactg actgcagaca aatcctccag tactgcctac 240
   atgcagctca acagcctgac atctggagat tctgcaatgt atttctgtaa aaagatggac 300
   tactggggcc aagggaccac ggtcaccgtc tcctca                           336
<210> 72
   <211> 112
   <212> PRT
   <213> Mus musculus
<400> 72
   Gln Val Lys Leu Gln Glu Ser Gly Ala Glu Leu Val Lys Pro Gly Ala
   1 5 10 15
   Ser Val Lys Ile Ser Cys Lys Ala Ser Gly Tyr Ile Phe Thr Asp His
   20 25 30
   Ala Leu His Trp Val Arg Gln Lys Pro Glu Gln Gly Leu Glu Trp Ile
   35 40 45
   Gly Tyr Ile Phe Pro Gly Asn Gly Asn Ile Glu Tyr Asn Glu Lys Phe
   50 55 60
   Lys Gly Lys Ala Thr Leu Thr Ala Asp Lys Ser Ser Ser Thr Ala Tyr
   65 70 75 80
   Met Gln Leu Asn Ser Leu Thr Ser Gly Asp Ser Ala Met Tyr Phe Cys
   85 90 95
   Lys Lys Met Asp Tyr Trp Gly Gln Gly Thr Thr Val Thr Val Ser Ser
   100 105 110
<210> 73
   <211> 336
   <212> DNA
   <213> Mus musculus
<400> 73
   caggtgaagc tgcaggagtc tggcgctgag ttggtgaaac ctggggcttc agtgaagatc 60
   tcctgcaagg cttctggtta caccttcact gaccattcta ttcactgggt gaagcagaag 120
   cctggacagg gcctagaatg gattggatat ctttttcccg gaaatggtaa ttttgaatat 180
   aatgagaaat tcaagggcaa ggccacactg actgcagaca aatcctccag cactgcctac 240
   atgcacctca acagcctgac atctgaggat tctgcagtgt atttctgtaa aaagatggac 300
   tactggggcc aagggaccac ggtcaccgtc tcctca 336
<210> 74
   <211> 112
   <212> PRT
   <213> Mus musculus
<400> 74
   Gln Val Lys Leu Gln Glu Ser Gly Ala Glu Leu Val Lys Pro Gly Ala
   1 5 10 15
   Ser Val Lys Ile Ser Cys Lys Ala Ser Gly Tyr Thr Phe Thr Asp His
   20 25 30
   Ser Ile His Trp Val Lys Gln Lys Pro Gly Gln Gly Leu Glu Trp Ile
   35 40 45
   Gly Tyr Leu Phe Pro Gly Asn Gly Asn Phe Glu Tyr Asn Glu Lys Phe
   50 55 60
   Lys Gly Lys Ala Thr Leu Thr Ala Asp Lys Ser Ser Ser Thr Ala Tyr
   65 70 75 80
   Met His Leu Asn Ser Leu Thr Ser Glu Asp Ser Ala Val Tyr Phe Cys
   85 90 95
   Lys Lys Met Asp Tyr Trp Gly Gln Gly Thr Thr Val Thr Val Ser Ser
   100 105 110
<210> 75
   <211> 336
   <212> DNA
   <213> Mus musculus
<400> 75
   caggttcagc tgcagcagtc cgacgctgag ttggtgaaac ctggggcttc agtgaagata 60
   tcctgcaggg cttctggcta caccttcact gaccattcta ttcactgggt gaagcagcag 120
   cctggccagg gcctggaatg gatcggatat atttttcccg gaaatggaaa tattgaatac 180
   aatgacaaat tcaagggcaa ggccacactg actgcagaca aatcctccgg cactgcctac 240
   atgcagctca acagcctgac atctgaggat tctgcagtgt atttctgtaa aaggatgggg 300
   tactggggtc aaggaacctc agtcaccgtc tcctca                           336
<210> 76
   <211> 112
   <212> PRT
   <213> Mus musculus
<400> 76
   Gln Val Gln Leu Gln Gln Ser Asp Ala Glu Leu Val Lys Pro Gly Ala
    1               5                   10                  15
   Ser Val Lys Ile Ser Cys Arg Ala Ser Gly Tyr Thr Phe Thr Asp His
               20                  25                  30
   Ser Ile His Trp Val Lys Gln Gln Pro Gly Gln Gly Leu Glu Trp Ile
           35                  40                  45
   Gly Tyr Ile Phe Pro Gly Asn Gly Asn Ile Glu Tyr Asn Asp Lys Phe
       50                  55                  60
   Lys Gly Lys Ala Thr Leu Thr Ala Asp Lys Ser Ser Gly Thr Ala Tyr
   65                  70                  75                  80
   Met Gln Leu Asn Ser Leu Thr Ser Glu Asp Ser Ala Val Tyr Phe Cys
                   85                  90                  95
   Lys Arg Met Gly Tyr Trp Gly Gln Gly Thr Ser Val Thr Val Ser Ser
               100                 105                 110
<210> 77
   <211> 336
   <212> DNA
   <213> Mus musculus
<400> 77
   caggtcaagc tgcaggagtc tggcgctgag ttggtgaaac ctggggcttc agtgaagatc 60
   tcctgcaagg cttctggcta caccttcact gaccattcta ttcactgggt gaagcagaag 120
   cctggacagg gcctagaatg gattggatat ctttttcccg gaaatggtaa ttttgagtac 180
   aatgaaaaat tcaagggcaa ggccacactg actgcagaca aatcctccag cactgtctac 240
   atgtacctca acagcctgac atctgaggat tctgcagtgt atttctgtaa aaggatgggg 300
   tactggggcc aagggaccac ggtcaccgtc tcctca                           336
<210> 78
   <211> 112
   <212> PRT
   <213> Mus musculus
<400> 78
   Gln Val Lys Leu Gln Glu Ser Gly Ala Glu Leu Val Lys Pro Gly Ala
    1               5                   10                  15
   Ser Val Lys Ile Ser Cys Lys Ala Ser Gly Tyr Thr Phe Thr Asp His
               20                  25                  30
   Ser Ile His Trp Val Lys Gln Lys Pro Gly Gln Gly Leu Glu Trp Ile
           35                  40                  45
   Gly Tyr Leu Phe Pro Gly Asn Gly Asn Phe Glu Tyr Asn Glu Lys Phe
       50                  55                  60
   Lys Gly Lys Ala Thr Leu Thr Ala Asp Lys Ser Ser Ser Thr Val Tyr
   65                  70                  75                  80
   Met Tyr Leu Asn Ser Leu Thr Ser Glu Asp Ser Ala Val Tyr Phe Cys
                   85                  90                  95
   Lys Arg Met Gly Tyr Trp Gly Gln Gly Thr Thr Val Thr Val Ser Ser
               100                 105                 110
<210> 79
   <211> 336
   <212> DNA
   <213> Mus musculus
<400> 79
   caggtcaagc tgcaggagtc tggacctgaa ctggtaaagc ctggggcttc agtgaagatg 60
   tcctgcaagg cttctggata cacattcact aactatgtta tacactgggt gaagcaaaag 120
   cctgggcagg gccttgagtg gattggatat attaatcctt acaatgatgg ctctaagtac 180
   aatgagaagt tcaaaggcaa ggcctcactg acttcagaca aatcctccag cacagcctac 240
   atggagctca gcagcctgac ctctgaggac tctgcggtct attactgtgc aagaatggac 300
   tactggggcc aagggaccac ggtcaccgtc tcctca                           336
<210> 80
   <211> 112
   <212> PRT
   <213> Mus musculus
<400> 80
   Gln Val Gln Leu Gln Glu Ser Gly Pro Glu Leu Val Lys Pro Gly Ala
    1               5                  10                  15
   Ser Val Lys Met Ser Cys Lys Ala Ser Gly Tyr Thr Phe Thr Asn Tyr
               20                  25                  30
   Val Ile His Trp Val Lys Gln Lys Pro Gly Gln Gly Leu Glu Trp Ile
           35                  40                  45
   Gly Tyr Ile Asn Pro Tyr Asn Asp Gly Ser Lys Tyr Asn Glu Lys Phe
       50                  55                  60
   Lys Gly Lys Ala Ser Leu Thr Ser Asp Lys Ser Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Met Asp Tyr Trp Gly Gln Gly Thr Thr Leu Thr Val Ser Ser
               100                 105                 110
<210> 81
   <211> 336
   <212> DNA
   <213> Mus musculus
<400> 81
   caggtcaagc tgcaggagtc tggacctgaa ctggtaaagc ctggggcttc agtgaagatg 60
   tcctgcaagg cttctggata cacattcact aactatgtta tacactgggt gaagcaaaag 120
   cctgggcagg gccttgagtg gattggatat attaatcctt acaatgatgg ctctaagtac 180
   aatgagaagt tcaaaggcaa ggcctcactg acttcagaca aatcctccag cacagcctac 240
   atggagctca gcagcctgac ctctgaggac tctgcggtct attactgtgc aagaatgggg 300
   tactggggcc aagggaccac ggtcaccgtc tcctca                           336
<210> 82
   <211> 112
   <212> PRT
   <213> Mus musculus
<400> 82
   Gln Val Gln Leu Gln Glu Ser Gly Pro Glu Leu Val Lys Pro Gly Ala
    1               5                  10                  15
   Ser Val Lys Met Ser Cys Lys Ala Ser Gly Tyr Thr Phe Thr Asn Tyr
               20                  25                  30
   Val Ile His Trp Val Lys Gln Lys Pro Gly Gln Gly Leu Glu Trp Ile
           35                  40                  45
   Gly Tyr Ile Asn Pro Tyr Asn Asp Gly Ser Lys Tyr Asn Glu Lys Phe
       50                  55                  60
   Lys Gly Lys Ala Ser Leu Thr Ser Asp Lys Ser Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Met Gly Tyr Trp Gly Gln Gly Thr Thr Leu Thr Val Ser Ser
               100                 105                 110
<210> 83
   <211> 342
   <212> DNA
   <213> Mus musculus
<400> 83
   gatgttttga tgacccaaac tccactctcc ctgcctgtca gtcttggaga tcaagcctcc 60
   atctcttgca gatctagtca gagcattgta catagtaatg gaaacaccta tttagaatgg 120
   tacctgcaga aaccaggcca gtctccaaag ctcctgatct acaaagtttc caaccgattt 180
   tctggggtcc cagacaggtt cagtggcagt ggatcaggga cagatttcac actcaagatc 240
   agcagagtgg aggctgagga tctgggagtt tattactgct ttcaaggttc acatgttcct 300
   cctacgttcg gtgctgggac caagctggag ctgaaacggg ct                     342
<210> 84
   <211> 113
   <212> PRT
   <213> Mus musculus
<400> 84
   Asp Val Leu Met Thr Gln Thr Pro Leu Ser Leu Pro Val Ser Leu Gly
    1               5                  10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Pro Thr Phe Gly Ala Gly Thr Lys Leu Glu Leu Lys
               100                 105                 110
   Arg
<210> 85
   <211> 324
   <212> DNA
   <213> Mus musculus
<400> 85
   gacatccaga tgactcagtc tccagcctcc ctatctgcat ctgtgggaga aactgtcacc 60
   atcacatgtc gaacaactga aaatatttac agttattttg tatggtctca gcagagacag 120
   ggaaaatctc ctcagctccg ggtctataat gcaaaatcct tagcagaagg tgtgccatca 180
   agtttcaatg tcagtgtatc aggcacacag ttttctctga agatcaatag cctgcagcct 240
   gaagattttg ggacttatca ctgtcaacac cattatggta ctccgtacac gttcggaggg 300
   gggaccaggc tggaaataag acgg                                        324
<210> 86
   <211> 108
   <212> PRT
   <213> Mus musculus
<400> 86
   Asp Ile Gln Met Thr Gln Ser Pro Ala Ser Leu Ser Ala Ser Val Gly
    1               5                  10                  15
   Glu Thr Val Thr Ile Thr Cys Arg Thr Thr Glu Asn Ile Tyr Ser Tyr
               20                  25                  30
   Phe Val Trp Ser Gln Gln Arg Gln Gly Lys Ser Pro Gln Leu Arg Val
           35                  40                  45
   Tyr Asn Ala Lys Ser Leu Ala Glu Gly Val Pro Ser Ser Phe Asn Val
       50                  55                  60
   Ser Val Ser Gly Thr Gln Phe Ser Leu Lys Ile Asn Ser Leu Gln Pro
   65                  70                  75                  80
   Glu Asp Phe Gly Thr Tyr His Cys Gln His His Tyr Gly Thr Pro Tyr
                   85                  90                  95
   Thr Phe Gly Gly Gly Thr Arg Leu Glu Ile Arg Arg
               100                 105
<210> 87
   <211> 339
   <212> DNA
   <213> Mus musculus
<400> 87
<210> 88
   <211> 113
   <212> PRT
   <213> Mus musculus
<400> 88
   Asp Val Leu Met Thr Gln Thr Pro Leu Thr Leu Ser Val Thr Ile Gly
    1               5                  10                  15
   Gln Pro Ala Ser Ile Ser Cys Lys Ser Ser Gln Ser Leu Leu Tyr Thr
               20                  25                  30
   Asn Gly Lys Thr Tyr Leu Thr Trp Leu Phe Gln Arg Pro Gly Gln Ser
           35                  40                  45
   Pro Lys Arg Leu Ile Tyr Leu Val Ser Glu Leu Asp Ser Gly Val Pro
       50                  55                  60
   Asp Arg Phe Ser Gly Ser Gly Ser Gly Thr Asp Phe Thr Leu Glu Ile
   65                  70                  75                  80
   Thr Arg Val Glu Ala Glu Asp Leu Gly Val Tyr Tyr Cys Leu Gln Ser
                   85                  90                  95
   Ala His Phe Pro Phe Thr Phe Gly Ser Gly Thr Lys Leu Glu Ile Lys
               100                 105                 110
   Arg
<210> 89
   <211> 327
   <212> DNA
   <213> Mus musculus
<400> 89
<210> 90
   <211> 113
   <212> PRT
   <213> Mus musculus
<400> 90
   Asp Val Val Met Thr Gln Thr Pro Leu Thr Leu Ser Val Thr Ile Gly
    1               5                  10                  15
   Gln Pro Ala Ser Ile Ser Cys Lys Ser Ser Gln Ser Leu Leu Asn Thr
               20                  25                  30
   Asn Gly Lys Thr Tyr Leu Thr Trp Leu Ile Gln Arg Pro Gly Gln Ser
           35                  40                  45
   Pro Gln Arg Leu Ile Tyr Leu Val Ser Lys Leu Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Leu Gln Ser
                   85                  90                  95
   Ser His Phe Pro Phe Thr Phe Gly Ser Gly Thr Lys Leu Glu Ile Lys
               100                 105                 110
   Arg
<210> 91
   <211> 327
   <212> DNA
   <213> Mus musculus
<400> 91
   gatgttgtgc taactcagtc tcctgccacc ctgtctgtga ctccaggaga tagagtcagt 60
   ctttcctgca gggccagcca aaatattggc aactacctac actggtatca acagaaatca 120
   catgagtctc caaggcttct catcaagtat gcttcccagt ccatctctgg gatcccctcc 180
   aggttcagtg gcagtggatc agtcacagat ttcactctca atatcaacag tgtggagact 240
   gaagattttg gaatgtattt ctgtcaacag agtgacacct ggcctctcac gttcggtgct 300
   gggaccaagc tggagctgaa acgggct                                     327
<210> 92
   <211> 108
   <212> PRT
   <213> Mus musculus
<400> 92
   Asp Val Val Leu Thr Gln Ser Pro Ala Thr Leu Ser Val Thr Pro Gly
    1               5                  10                  15
   Asp Arg Val Ser Leu Ser Cys Arg Ala Ser Gln Asn Ile Gly Asn Tyr
               20                  25                  30
   Leu His Trp Tyr Gln Gln Lys Ser His Glu Ser Pro Arg Leu Leu Ile
           35                  40                  45
   Lys Tyr Ala Ser Gln Ser Ile Ser Gly Ile Pro Ser Arg Phe Ser Gly
       50                  55                  60
   Ser Gly Ser Val Thr Asp Phe Thr Leu Asn Ile Asn Ser Val Glu Thr
   65                  70                  75                  80
   Glu Asp Phe Gly Met Tyr Phe Cys Gln Gln Ser Asp Thr Trp Pro Leu
                   85                  90                  95
   Thr Phe Gly Ala Gly Thr Lys Leu Glu Leu Lys Arg
               100                 105
<210> 93
   <211> 8
   <212> PRT
   <213> Mus musculus
<400> 93
   Thr Phe Thr Asp His Ser Ile His
   1 5
<210> 94
   <211> 8
   <212> PRT
   <213> Mus musculus
<400> 94
   Thr Phe Thr Asn Tyr Val Ile His
   1 5
<210> 95
   <211> 8
   <212> PRT
   <213> Mus musculus
<400> 95
   Ile Phe Thr Asp His Ala Leu His
   1 5
<210> 96
   <211> 17
   <212> PRT
   <213> Mus musculus
<400> 96
   Tyr Ile Phe Pro Gly Asn Gly Asn Ile Glu Tyr Asn Asp Lys Phe Lys
   1 5 10 15
   Gly
<210> 97
   <211> 17
   <212> PRT
   <213> Mus musculus
<400> 97
   Tyr Leu Phe Pro Gly Asn Gly Asn Phe Glu Tyr Asn Glu Lys Phe Lys
   1 5 10 15
   Gly
<210> 98
   <211> 17
   <212> PRT
   <213> Mus musculus
<400> 98
   Tyr Ile Asn Pro Tyr Asn Asp Gly Ser Lys Tyr Asn Glu Lys Phe Lys
   1 5 10 15
   Gly
<210> 99
   <211> 17
   <212> PRT
   <213> Mus musculus
<400> 99
   Tyr Ile Phe Pro Gly Asn Gly Asn Ile Glu Tyr Asn Glu Lys Phe Lys
   1 5 10 15
   Gly
<210> 100
   <211> 5
   <212> PRT
   <213> Mus musculus
<400> 100
   Lys Arg Met Gly Tyr
   1 5
<210> 101
   <211> 5
   <212> PRT
   <213> Mus musculus
<400> 101
   Lys Lys Met Asp Tyr
   1 5
<210> 102
   <211> 13
   <212> PRT
   <213> Mus musculus
<400> 102
<210> 103
   <211> 16
   <212> PRT
   <213> Mus musculus
<400> 103
   Arg Ser Ser Gln Ser Ile Val His Ser Asn Gly Asn Thr Tyr Leu Glu
   1 5 10 15
<210> 104
   <211> 11
   <212> PRT
   <213> Mus musculus
<400> 104
   Arg Thr Thr Glu Asn Ile Tyr Ser Tyr Phe Val
   1 5 10
<210> 105
   <211> 16
   <212> PRT
   <213> Mus musculus
<400> 105
   Lys Ser Ser Gln Ser Leu Leu Tyr Thr Asn Gly Lys Thr Tyr Leu Thr
   1 5 10 15
<210> 106
   <211> 16
   <212> PRT
   <213> Mus musculus
<400> 106
   Lys Ser Ser Gln Ser Leu Leu Asn Thr Asn Gly Lys Thr Tyr Leu Thr
   1 5 10 15
<210> 107
   <211> 11
   <212> PRT
   <213> Mus musculus
<400> 107
   Arg Ala Ser Gln Asn Ile Gly Asn Tyr Leu His
   1 5 10
<210> 108
   <211> 7
   <212> PRT
   <213> Mus musculus
<400> 108
   Lys Val Ser Asn Arg Phe Ser
   1 5
<210> 109
   <211> 7
   <212> PRT
   <213> Mus musculus
<400> 109
   Asn Ala Lys Ser Leu Ala Glu
   1 5
<210> 110
   <211> 7
   <212> PRT
   <213> Mus musculus
<400> 110
   Leu Val Ser Glu Leu Asp Ser
   1 5
<210> 111
   <211> 7
   <212> PRT
   <213> Mus musculus
<400> 111
   Leu Val Ser Lys Leu Asp Ser
   1 5
<210> 112
   <211> 7
   <212> PRT
   <213> Mus musculus
<400> 112
   Tyr Ala Ser Gln Ser Ile Ser
   1 5
<210> 113
   <211> 9
   <212> PRT
   <213> Mus musculus
<400> 113
   Phe Gln Gly Ser His Val Pro Pro Thr
   1 5
<210> 114
   <211> 9
   <212> PRT
   <213> Mus musculus
<400> 114
   Gln His His Tyr Gly Thr Pro Tyr Thr
   1 5
<210> 115
   <211> 9
   <212> PRT
   <213> Mus musculus
<400> 115
   Leu Gln Ser Ala His Phe Pro Phe Thr
   1 5
<210> 116
   <211> 9
   <212> PRT
   <213> Mus musculus
<400> 116
   Leu Gln Ser Ser His Phe Pro Phe Thr
   1 5
<210> 117
   <211> 9
   <212> PRT
   <213> Mus musculus
<400> 117
   Gln Gln Ser Asp Thr Trp Pro Leu Thr
   1 5
<210> 118
   <211> 5
   <212> PRT
   <213> Mus musculus
<400> 118
   Asp His Ala Leu His
   1 5
<210> 119
   <211> 5
   <212> PRT
   <213> Mus musculus
<400> 119
   Asp His Ser Ile His
   1 5
<210> 120
   <211> 5
   <212> PRT
   <213> Mus musculus
<400> 120
   Asn Tyr Val Ile His
   1 5
<210> 121
   <211> 9
   <212> PRT
   <213> Mus musculus
<400> 121
   Ile Phe Pro Gly Asn Gly Asn Ile Glu
   1 5
<210> 122
   <211> 9
   <212> PRT
   <213> Mus musculus
<400> 122
   Leu Phe Pro Gly Asn Gly Asn Phe Glu
   1 5
<210> 123
   <211> 9
   <212> PRT
   <213> Mus musculus
<400> 123
   Ile Asn Pro Tyr Asn Asp Gly Ser Lys
   1 5
<210> 124
   <211> 11
   <212> PRT
   <213> Mus musculus
<400> 124
   His Leu Ala Asn Thr Tyr Tyr Tyr Phe Asp Tyr
   1 5 10
<210> 125
   <211> 5
   <212> PRT
   <213> Mus musculus
<400> 125
   Ser Asn Gly Asn Thr
   1 5
<210> 126
   <211> 5
   <212> PRT
   <213> Mus musculus
<400> 126
   Glu Asn Ile Tyr Ser
   1 5
<210> 127
   <211> 5
   <212> PRT
   <213> Mus musculus
<400> 127
   Thr Ser Gly Tyr Ser
   1 5
<210> 128
   <211> 5
   <212> PRT
   <213> Mus musculus
<400> 128
   Gln Asp Ile Lys Ser
   1 5
<210> 129
   <211> 5
   <212> PRT
   <213> Mus musculus
<400> 129
   Gln Asn Ile Gly Asn
   1 5
<210> 130
   <211> 4654
   <212> DNA
   <213> Homo sapiens
<400> 130
   ggcggcggct ggaggagagc gcggtggaga gccgagcggg cgggcggcgg gtgcggagcg 60
   ggcgagggag cgcgcgcggc cgccacaaag ctcgggcgcc gcggggctgc atgcggcgta 120
   cctggcccgg cgcggcgact gctctccggg ctggcggggg ccggccgcga gccccggggg 180
   ccccgaggcc gcagcttgcc tgcgcgctct gagccttcgc aactcgcgag caaagtttgg 240
   tggaggcaac gccaagcctg agtcctttct tcctctcgtt ccccaaatcc gagggcagcc 300
   cgcgggcgtc atgcccgcgc tcctccgcag cctggggtac gcgtgaagcc cgggaggctt 360
   ggcgccggcg aagacccaag gaccactctt ctgcgtttgg agttgctccc cgcaaccccg 420
   ggctcgtcgc tttctccatc ccgacccacg cggggcgcgg ggacaacaca ggtcgcggag 480
   gagcgttgcc attcaagtga ctgcagcagc agcggcagcg cctcggttcc tgagcccacc 540
   gcaggctgaa ggcattgcgc gtagtccatg cccgtagagg aagtgtgcag atgggattaa 600
   cgtccacatg gagatatgga agaggaccgg ggattggtac cgtaaccatg gtcagctggg 660
   gtcgtttcat ctgcctggtc gtggtcacca tggcaacctt gtccctggcc cggccctcct 720
   tcagtttagt tgaggatacc acattagagc cagaagagcc accaaccaaa taccaaatct 780
   ctcaaccaga agtgtacgtg gctgcgccag gggagtcgct agaggtgcgc tgcctgttga 840
   aagatgccgc cgtgatcagt tggactaagg atggggtgca cttggggccc aacaatagga 900
   cagtgcttat tggggagtac ttgcagataa agggcgccac gcctagagac tccggcctct 960
   atgcttgtac tgccagtagg actgtagaca gtgaaacttg gtacttcatg gtgaatgtca 1020
   cagatgccat ctcatccgga gatgatgagg atgacaccga tggtgcggaa gattttgtca 1080
   gtgagaacag taacaacaag agagcaccat actggaccaa cacagaaaag atggaaaagc 1140
   ggctccatgc tgtgcctgcg gccaacactg tcaagtttcg ctgcccagcc ggggggaacc 1200
   caatgccaac catgcggtgg ctgaaaaacg ggaaggagtt taagcaggag catcgcattg 1260
   gaggctacaa ggtacgaaac cagcactgga gcctcattat ggaaagtgtg gtcccatctg 1320
   acaagggaaa ttatacctgt gtagtggaga atgaatacgg gtccatcaat cacacgtacc 1380
   acctggatgt tgtggagcga tcgcctcacc ggcccatcct ccaagccgga ctgccggcaa 1440
   atgcctccac agtggtcgga ggagacgtag agtttgtctg caaggtttac agtgatgccc 1500
   agccccacat ccagtggatc aagcacgtgg aaaagaacgg cagtaaatac gggcccgacg 1560
   ggctgcccta cctcaaggtt ctcaaggccg ccggtgttaa caccacggac aaagagattg 1620
   aggttctcta tattcggaat gtaacttttg aggacgctgg ggaatatacg tgcttggcgg 1680
   gtaattctat tgggatatcc tttcactctg catggttgac agttctgcca gcgcctggaa 1740
   gagaaaagga gattacagct tccccagact acctggagat agccatttac tgcatagggg 1800
   tcttcttaat cgcctgtatg gtggtaacag tcatcctgtg ccgaatgaag aacacgacca 1860
   agaagccaga cttcagcagc cagccggctg tgcacaagct gaccaaacgt atccccctgc 1920
   ggagacaggt aacagtttcg gctgagtcca gctcctccat gaactccaac accccgctgg 1980
   tgaggataac aacacgcctc tcttcaacgg cagacacccc catgctggca ggggtctccg 2040
   agtatgaact tccagaggac ccaaaatggg agtttccaag agataagctg acactgggca 2100
   agcccctggg agaaggttgc tttgggcaag tggtcatggc ggaagcagtg ggaattgaca 2160
   aagacaagcc caaggaggcg gtcaccgtgg ccgtgaagat gttgaaagat gatgccacag 2220
   agaaagacct ttctgatctg gtgtcagaga tggagatgat gaagatgatt gggaaacaca 2280
   agaatatcat aaatcttctt ggagcctgca cacaggatgg gcctctctat gtcatagttg 2340
   agtatgcctc taaaggcaac ctccgagaat acctccgagc ccggaggcca cccgggatgg 2400
   agtactccta tgacattaac cgtgttcctg aggagcagat gaccttcaag gacttggtgt 2460
   catgcaccta ccagctggcc agaggcatgg agtacttggc ttcccaaaaa tgtattcatc 2520
   gagatttagc agccagaaat gttttggtaa cagaaaacaa tgtgatgaaa atagcagact 2580
   ttggactcgc cagagatatc aacaatatag actattacaa aaagaccacc aatgggcggc 2640
   ttccagtcaa gtggatggct ccagaagccc tgtttgatag agtatacact catcagagtg 2700
   atgtctggtc cttcggggtg ttaatgtggg agatcttcac tttagggggc tcgccctacc 2760
   cagggattcc cgtggaggaa ctttttaagc tgctgaagga aggacacaga atggataagc 2820
   cagccaactg caccaacgaa ctgtacatga tgatgaggga ctgttggcat gcagtgccct 2880
   cccagagacc aacgttcaag cagttggtag aagacttgga tcgaattctc actctcacaa 2940
   ccaatgagga atacttggac ctcagccaac ctctcgaaca gtattcacct agttaccctg 3000
   acacaagaag ttcttgttct tcaggagatg attctgtttt ttctccagac cccatgcctt 3060
   acgaaccatg ccttcctcag tatccacaca taaacggcag tgttaaaaca tgaatgactg 3120
   tgtctgcctg tccccaaaca ggacagcact gggaacctag ctacactgag cagggagacc 3180
   atgcctccca gagcttgttg tctccacttg tatatatgga tcagaggagt aaataattgg 3240
   aaaagtaatc agcatatgtg taaagattta tacagttgaa aacttgtaat cttccccagg 3300
   aggagaagaa ggtttctgga gcagtggact gccacaagcc accatgtaac ccctctcacc 3360
   tgccgtgcgt actggctgtg gaccagtagg actcaaggtg gacgtgcgtt ctgccttcct 3420
   tgttaatttt gtaataattg gagaagattt atgtcagcac acacttacag agcacaaatg 3480
   cagtatatag gtgctggatg tatgtaaata tattcaaatt atgtataaat atatattata 3540
   tatttacaag gagttatttt ttgtattgat tttaaatgga tgtcccaatg cacctagaaa 3600
   attggtctct ctttttttaa tagctatttg ctaaatgctg ttcttacaca taatttctta 3660
   attttcaccg agcagaggtg gaaaaatact tttgctttca gggaaaatgg tataacgtta 3720
   atttattaat aaattggtaa tatacaaaac aattaatcat ttatagtttt ttttgtaatt 3780
   taagtggcat ttctatgcag gcagcacagc agactagtta atctattgct tggacttaac 3840
   tagttatcag atcctttgaa aagagaatat ttacaatata tgactaattt ggggaaaatg 3900
   aagttttgat ttatttgtgt ttaaatgctg ctgtcagacg attgttctta gacctcctaa 3960
   atgccccata ttaaaagaac tcattcatag gaaggtgttt cattttggtg tgcaaccctg 4020
   tcattacgtc aacgcaacgt ctaactggac ttcccaagat aaatggtacc agcgtcctct 4080
   taaaagatgc cttaatccat tccttgagga cagaccttag ttgaaatgat agcagaatgt 4140
   gcttctctct ggcagctggc cttctgcttc tgagttgcac attaatcaga ttagcctgta 4200
   ttctcttcag tgaattttga taatggcttc cagactcttt ggcgttggag acgcctgtta 4260
   ggatcttcaa gtcccatcat agaaaattga aacacagagt tgttctgctg atagttttgg 4320
   ggatacgtcc atctttttaa gggattgctt tcatctaatt ctggcaggac ctcaccaaaa 4380
   gatccagcct catacctaca tcagacaaaa tatcgccgtt gttccttctg tactaaagta 4440
   ttgtgttttg ctttggaaac acccactcac tttgcaatag ccgtgcaaga tgaatgcaga 4500
   ttacactgat cttatgtgtt acaaaattgg agaaagtatt taataaaacc tgttaatttt 4560
   tatactgaca ataaaaatgt ttctacagat attaatgtta acaagacaaa ataaatgtca 4620
   cgcaacttat ttttttaata aaaaaaaaaa aaaa                              4654
<210> 131
   <211> 4657
   <212> DNA
   <213> Homo sapiens
<400> 131
   ggcggcggct ggaggagagc gcggtggaga gccgagcggg cgggcggcgg gtgcggagcg 60
   ggcgagggag cgcgcgcggc cgccacaaag ctcgggcgcc gcggggctgc atgcggcgta 120
   cctggcccgg cgcggcgact gctctccggg ctggcggggg ccggccgcga gccccggggg 180
   ccccgaggcc gcagcttgcc tgcgcgctct gagccttcgc aactcgcgag caaagtttgg 240
   tggaggcaac gccaagcctg agtcctttct tcctctcgtt ccccaaatcc gagggcagcc 300
   cgcgggcgtc atgcccgcgc tcctccgcag cctggggtac gcgtgaagcc cgggaggctt 360
   ggcgccggcg aagacccaag gaccactctt ctgcgtttgg agttgctccc cgcaaccccg 420
   ggctcgtcgc tttctccatc ccgacccacg cggggcgcgg ggacaacaca ggtcgcggag 480
   gagcgttgcc attcaagtga ctgcagcagc agcggcagcg cctcggttcc tgagcccacc 540
   gcaggctgaa ggcattgcgc gtagtccatg cccgtagagg aagtgtgcag atgggattaa 600
   cgtccacatg gagatatgga agaggaccgg ggattggtac cgtaaccatg gtcagctggg 660
   gtcgtttcat ctgcctggtc gtggtcacca tggcaacctt gtccctggcc cggccctcct 720
   tcagtttagt tgaggatacc acattagagc cagaagagcc accaaccaaa taccaaatct 780
   ctcaaccaga agtgtacgtg gctgcgccag gggagtcgct agaggtgcgc tgcctgttga 840
   aagatgccgc cgtgatcagt tggactaagg atggggtgca cttggggccc aacaatagga 900
   cagtgcttat tggggagtac ttgcagataa agggcgccac gcctagagac tccggcctct 960
   atgcttgtac tgccagtagg actgtagaca gtgaaacttg gtacttcatg gtgaatgtca 1020
   cagatgccat ctcatccgga gatgatgagg atgacaccga tggtgcggaa gattttgtca 1080
   gtgagaacag taacaacaag agagcaccat actggaccaa cacagaaaag atggaaaagc 1140
   ggctccatgc tgtgcctgcg gccaacactg tcaagtttcg ctgcccagcc ggggggaacc 1200
   caatgccaac catgcggtgg ctgaaaaacg ggaaggagtt taagcaggag catcgcattg 1260
   gaggctacaa ggtacgaaac cagcactgga gcctcattat ggaaagtgtg gtcccatctg 1320
   acaagggaaa ttatacctgt gtagtggaga atgaatacgg gtccatcaat cacacgtacc 1380
   acctggatgt tgtggagcga tcgcctcacc ggcccatcct ccaagccgga ctgccggcaa 1440
   atgcctccac agtggtcgga ggagacgtag agtttgtctg caaggtttac agtgatgccc 1500
   agccccacat ccagtggatc aagcacgtgg aaaagaacgg cagtaaatac gggcccgacg 1560
   ggctgcccta cctcaaggtt ctcaagcact cggggataaa tagttccaat gcagaagtgc 1620
   tggctctgtt caatgtgacc gaggcggatg ctggggaata tatatgtaag gtctccaatt 1680
   atatagggca ggccaaccag tctgcctggc tcactgtcct gccaaaacag caagcgcctg 1740
   gaagagaaaa ggagattaca gcttccccag actacctgga gatagccatt tactgcatag 1800
   gggtcttctt aatcgcctgt atggtggtaa cagtcatcct gtgccgaatg aagaacacga 1860
   ccaagaagcc agacttcagc agccagccgg ctgtgcacaa gctgaccaaa cgtatccccc 1920
   tgcggagaca ggtaacagtt tcggctgagt ccagctcctc catgaactcc aacaccccgc 1980
   tggtgaggat aacaacacgc ctctcttcaa cggcagacac ccccatgctg gcaggggtct 2040
   ccgagtatga acttccagag gacccaaaat gggagtttcc aagagataag ctgacactgg 2100
   gcaagcccct gggagaaggt tgctttgggc aagtggtcat ggcggaagca gtgggaattg 2160
   acaaagacaa gcccaaggag gcggtcaccg tggccgtgaa gatgttgaaa gatgatgcca 2220
   cagagaaaga cctttctgat ctggtgtcag agatggagat gatgaagatg attgggaaac 2280
   acaagaatat cataaatctt cttggagcct gcacacagga tgggcctctc tatgtcatag 2340
   ttgagtatgc ctctaaaggc aacctccgag aatacctccg agcccggagg ccacccggga 2400
   tggagtactc ctatgacatt aaccgtgttc ctgaggagca gatgaccttc aaggacttgg 2460
   tgtcatgcac ctaccagctg gccagaggca tggagtactt ggcttcccaa aaatgtattc 2520
   atcgagattt agcagccaga aatgttttgg taacagaaaa caatgtgatg aaaatagcag 2580
   actttggact cgccagagat atcaacaata tagactatta caaaaagacc accaatgggc 2640
   ggcttccagt caagtggatg gctccagaag ccctgtttga tagagtatac actcatcaga 2700
   gtgatgtctg gtccttcggg gtgttaatgt gggagatctt cactttaggg ggctcgccct 2760
   acccagggat tcccgtggag gaacttttta agctgctgaa ggaaggacac agaatggata 2820
   agccagccaa ctgcaccaac gaactgtaca tgatgatgag ggactgttgg catgcagtgc 2880
   cctcccagag accaacgttc aagcagttgg tagaagactt ggatcgaatt ctcactctca 2940
   caaccaatga ggaatacttg gacctcagcc aacctctcga acagtattca cctagttacc 3000
   ctgacacaag aagttcttgt tcttcaggag atgattctgt tttttctcca gaccccatgc 3060
   cttacgaacc atgccttcct cagtatccac acataaacgg cagtgttaaa acatgaatga 3120
   ctgtgtctgc ctgtccccaa acaggacagc actgggaacc tagctacact gagcagggag 3180
   accatgcctc ccagagcttg ttgtctccac ttgtatatat ggatcagagg agtaaataat 3240
   tggaaaagta atcagcatat gtgtaaagat ttatacagtt gaaaacttgt aatcttcccc 3300
   aggaggagaa gaaggtttct ggagcagtgg actgccacaa gccaccatgt aacccctctc 3360
   acctgccgtg cgtactggct gtggaccagt aggactcaag gtggacgtgc gttctgcctt 3420
   ccttgttaat tttgtaataa ttggagaaga tttatgtcag cacacactta cagagcacaa 3480
   atgcagtata taggtgctgg atgtatgtaa atatattcaa attatgtata aatatatatt 3540
   atatatttac aaggagttat tttttgtatt gattttaaat ggatgtccca atgcacctag 3600
   aaaattggtc tctctttttt taatagctat ttgctaaatg ctgttcttac acataatttc 3660
   ttaattttca ccgagcagag gtggaaaaat acttttgctt tcagggaaaa tggtataacg 3720
   ttaatttatt aataaattgg taatatacaa aacaattaat catttatagt tttttttgta 3780
   atttaagtgg catttctatg caggcagcac agcagactag ttaatctatt gcttggactt 3840
   aactagttat cagatccttt gaaaagagaa tatttacaat atatgactaa tttggggaaa 3900
   atgaagtttt gatttatttg tgtttaaatg ctgctgtcag acgattgttc ttagacctcc 3960
   taaatgcccc atattaaaag aactcattca taggaaggtg tttcattttg gtgtgcaacc 4020
   ctgtcattac gtcaacgcaa cgtctaactg gacttcccaa gataaatggt accagcgtcc 4080
   tcttaaaaga tgccttaatc cattccttga ggacagacct tagttgaaat gatagcagaa 4140
   tgtgcttctc tctggcagct ggccttctgc ttctgagttg cacattaatc agattagcct 4200
   gtattctctt cagtgaattt tgataatggc ttccagactc tttggcgttg gagacgcctg 4260
   ttaggatctt caagtcccat catagaaaat tgaaacacag agttgttctg ctgatagttt 4320
   tggggatacg tccatctttt taagggattg ctttcatcta attctggcag gacctcacca 4380
   aaagatccag cctcatacct acatcagaca aaatatcgcc gttgttcctt ctgtactaaa 4440
   gtattgtgtt ttgctttgga aacacccact cactttgcaa tagccgtgca agatgaatgc 4500
   agattacact gatcttatgt gttacaaaat tggagaaagt atttaataaa acctgttaat 4560
   ttttatactg acaataaaaa tgtttctaca gatattaatg ttaacaagac aaaataaatg 4620
   tcacgcaact tattttttta ataaaaaaaa aaaaaaa 4657
<210> 132
   <211> 2781
   <212> DNA
   <213> Homo sapiens
<400> 132
   tgactgcagc agcagcggca gcgcctcggt tcctgagccc accgcaggct gaaggcattg 60
   cgcgtagtcc atgcccgtag aggaagtgtg cagatgggat taacgtccac atggagatat 120
   ggaagaggac cggggattgg taccgtaacc atggtcagct ggggtcgttt catctgcctg 180
   gtcgtggtca ccatggcaac cttgtccctg gcccggccct ccttcagttt agttgaggat 240
   accacattag agccagaaga gccaccaacc aaataccaaa tctctcaacc agaagtgtac 300
   gtggctgcgc caggggagtc gctagaggtg cgctgcctgt tgaaagatgc cgccgtgatc 360
   agttggacta aggatggggt gcacttgggg cccaacaata ggacagtgct tattggggag 420
   tacttgcaga taaagggcgc cacgcctaga gactccggcc tctatgcttg tactgccagt 480
   aggactgtag acagtgaaac ttggtacttc atggtgaatg tcacagatgc catctcatcc 540
   ggagatgatg aggatgacac cgatggtgcg gaagattttg tcagtgagaa cagtaacaac 600
   aagagagcac catactggac caacacagaa aagatggaaa agcggctcca tgctgtgcct 660
   gcggccaaca ctgtcaagtt tcgctgccca gccgggggga acccaatgcc aaccatgcgg 720
   tggctgaaaa acgggaagga gtttaagcag gagcatcgca ttggaggcta caaggtacga 780
   aaccagcact ggagcctcat tatggaaagt gtggtcccat ctgacaaggg aaattatacc 840
   tgtgtagtgg agaatgaata cgggtccatc aatcacacgt accacctgga tgttgtggag 900
   cgatcgcctc accggcccat cctccaagcc ggactgccgg caaatgcctc cacagtggtc 960
   ggaggagacg tagagtttgt ctgcaaggtt tacagtgatg cccagcccca catccagtgg 1020
   atcaagcacg tggaaaagaa cggcagtaaa tacgggcccg acgggctgcc ctacctcaag 1080
   gttctcaagc actcggggat aaatagttcc aatgcagaag tgctggctct gttcaatgtg 1140
   accgaggcgg atgctgggga atatatatgt aaggtctcca attatatagg gcaggccaac 1200
   cagtctgcct ggctcactgt cctgccaaaa cagcaagcgc ctggaagaga aaaggagatt 1260
   acagcttccc cagactacct ggagatagcc atttactgca taggggtctt cttaatcgcc 1320
   tgtatggtgg taacagtcat cctgtgccga atgaagaaca cgaccaagaa gccagacttc 1380
   agcagccagc cggctgtgca caagctgacc aaacgtatcc ccctgcggag acaggtaaca 1440
   gtttcggctg agtccagctc ctccatgaac tccaacaccc cgctggtgag gataacaaca 1500
   cgcctctctt caacggcaga cacccccatg ctggcagggg tctccgagta tgaacttcca 1560
   gaggacccaa aatgggagtt tccaagagat aagctgacac tgggcaagcc cctgggagaa 1620
   ggttgctttg ggcaagtggt catggcggaa gcagtgggaa ttgacaaaga caagcccaag 1680
   gaggcggtca ccgtggccgt gaagatgttg aaagatgatg ccacagagaa agacctttct 1740
   gatctggtgt cagagatgga gatgatgaag atgattggga aacacaagaa tatcataaat 1800
   cttcttggag cctgcacaca ggatgggcct ctctatgtca tagttgagta tgcctctaaa 1860
   ggcaacctcc gagaatacct ccgagcccgg aggccacccg ggatggagta ctcctatgac 1920
   attaaccgtg ttcctgagga gcagatgacc ttcaaggact tggtgtcatg cacctaccag 1980
   ctggccagag gcatggagta cttggcttcc caaaaatgta ttcatcgaga tttagcagcc 2040
   agaaatgttt tggtaacaga aaacaatgtg atgaaaatag cagactttgg actcgccaga 2100
   gatatcaaca atatagacta ttacaaaaag accaccaatg ggcggcttcc agtcaagtgg 2160
   atggctccag aagccctgtt tgatagagta tacactcatc agagtgatgt ctggtccttc 2220
   ggggtgttaa tgtgggagat cttcacttta gggggctcgc cctacccagg gattcccgtg 2280
   gaggaacttt ttaagctgct gaaggaagga cacagaatgg ataagccagc caactgcacc 2340
   aacgaactgt acatgatgat gagggactgt tggcatgcag tgccctccca gagaccaacg 2400
   ttcaagcagt tggtagaaga cttggatcga attctcactc tcacaaccaa tgagatctga 2460
   aagtttatgg cttcattgag aaactgggaa aagttggtca ggcgcagtgg ctcatgcctg 2520
   taatcccagc actttgggag gccgaggcag gcggatcatg aggtcaggag ttccagacca 2580
   gcctggccaa catggtgaaa ccctgtctct actaaagata caaaaaatta gccgggcgtg 2640
   ttggtgtgca cctgtaatcc cagctactcc gggaggctga ggcaggagag tcacttgaac 2700
   cggggaggcg gaggttgcag tgagccgaga tcatgccatt gcattccagc cttggcgaca 2760
   gagcgagact ccgtctcaaa a                                          2781
<210> 133
   <211> 3821
   <212> DNA
   <213> Homo sapiens
<400> 133
   tgactgcagc agcagcggca gcgcctcggt tcctgagccc accgcaggct gaaggcattg 60
   cgcgtagtcc atgcccgtag aggaagtgtg cagatgggat taacgtccac atggagatat 120
   ggaagaggac cggggattgg taccgtaacc atggtcagct ggggtcgttt catctgcctg 180
   gtcgtggtca ccatggcaac cttgtccctg gcccggccct ccttcagttt agttgaggat 240
   accacattag agccagaaga gccaccaacc aaataccaaa tctctcaacc agaagtgtac 300
   gtggctgcgc caggggagtc gctagaggtg cgctgcctgt tgaaagatgc cgccgtgatc 360
   agttggacta aggatggggt gcacttgggg cccaacaata ggacagtgct tattggggag 420
   tacttgcaga taaagggcgc cacgcctaga gactccggcc tctatgcttg tactgccagt 480
   aggactgtag acagtgaaac ttggtacttc atggtgaatg tcacagatgc catctcatcc 540
   ggagatgatg aggatgacac cgatggtgcg gaagattttg tcagtgagaa cagtaacaac 600
   aagagagcac catactggac caacacagaa aagatggaaa agcggctcca tgctgtgcct 660
   gcggccaaca ctgtcaagtt tcgctgccca gccgggggga acccaatgcc aaccatgcgg 720
   tggctgaaaa acgggaagga gtttaagcag gagcatcgca ttggaggcta caaggtacga 780
   aaccagcact ggagcctcat tatggaaagt gtggtcccat ctgacaaggg aaattatacc 840
   tgtgtagtgg agaatgaata cgggtccatc aatcacacgt accacctgga tgttgtggcg 900
   cctggaagag aaaaggagat tacagcttcc ccagactacc tggagatagc catttactgc 960
   ataggggtct tcttaatcgc ctgtatggtg gtaacagtca tcctgtgccg aatgaagaac 1020
   acgaccaaga agccagactt cagcagccag ccggctgtgc acaagctgac caaacgtatc 1080
   cccctgcgga gacaggtaac agtttcggct gagtccagct cctccatgaa ctccaacacc 1140
   ccgctggtga ggataacaac acgcctctct tcaacggcag acacccccat gctggcaggg 1200
   gtctccgagt atgaacttcc agaggaccca aaatgggagt ttccaagaga taagctgaca 1260
   ctgggcaagc cctgggagaa aggttgcttt gggcaagtgg tcatggcgga agcagtggga 1320
   attgacaaag acaagcccaa ggaggcggtc accgtggccg tgaagatgtt gaaagatgat 1380
   gccacagaga aagacctttc tgatctggtg tcagagatgg agatgatgaa gatgattggg 1440
   aaacacaaga atatcataaa tcttcttgga gcctgcacac aggatgggcc tctctatgtc 1500
   atagttgagt atgcctctaa aggcaacctc cgagaatacc tccgagcccg gaggccaccc 1560
   gggatggagt actcctatga cattaaccgt gttcctgagg agcagatgac cttcaaggac 1620
   ttggtgtcat gcacctacca gctggccaga ggcatggagt acttggcttc ccaaaaatgt 1680
   attcatcgag atttagcagc cagaaatgtt ttggtaacag aaaacaatgt gatgaaaata 1740
   gcagactttg gactcgccag agatatcaac aatatagact attacaaaaa gaccaccaat 1800
   gggcggcttc cagtcaagtg gatggctcca gaagccctgt ttgatagagt atacactcat 1860
   cagagtgatg tctggtcctt cggggtgtta atgtgggaga tcttcacttt agggggctcg 1920
   ccctacccag ggattcccgt ggaggaactt tttaagctgc tgaaggaagg acacagaatg 1980
   gataagccag ccaactgcac caacgaactg tacatgatga tgagggactg ttggcatgca 2040
   gtgccctccc agagaccaac gttcaagcag ttggtagaag acttggatcg aattctcact 2100
   ctcacaacca atgaggaata cttggacctc agccaacctc tcgaacagta ttcacctagt 2160
   taccctgaca caagaagttc ttgttcttca ggagatgatt ctgttttttc tccagacccc 2220
   atgccttacg aaccatgcct tcctcagtat ccacacataa acggcagtgt taaaacatga 2280
   atgactgtgt ctgcctgtcc ccaaacagga cagcactggg aacctagcta cactgagcag 2340
   ggagaccatg cctcccagag cttgttgtct ccacttgtat atatggatca gaggagtaaa 2400
   taattggaaa agtaatcagc atatgtgtaa agatttatac agttgaaaac ttgtaatctt 2460
   ccccaggagg agaagaaggt ttctggagca gtggactgcc acaagccacc atgtaacccc 2520
   tctcacctgc cgtgcgtact ggctgtggac cagtaggact caaggtggac gtgcgttctg 2580
   ccttccttgt taattttgta ataattggag aagatttatg tcagcacaca cttacagagc 2640
   acaaatgcag tatataggtg ctggatgtat gtaaatatat tcaaattatg tataaatata 2700
   tattatatat ttacaaggag ttattttttg tattgatttt aaatggatgt cccaatgcac 2760
   ctagaaaatt ggtctctctt tttttaatag ctatttgcta aatgctgttc ttacacataa 2820
   tttcttaatt ttcaccgagc agaggtggaa aaatactttt gctttcaggg aaaatggtat 2880
   aacgttaatt tattaataaa ttggtaatat acaaaacaat taatcattta tagttttttt 2940
   tgtaatttaa gtggcatttc tatgcaggca gcacagcaga ctagttaatc tattgcttgg 3000
   acttaactag ttatcagatc ctttgaaaag agaatattta caatatatga ctaatttggg 3060
   gaaaatgaag ttttgattta tttgtgttta aatgctgctg tcagacgatt gttcttagac 3120
   ctcctaaatg ccccatatta aaagaactca ttcataggaa ggtgtttcat tttggtgtgc 3180
   aaccctgtca ttacgtcaac gcaacgtcta actggacttc ccaagataaa tggtaccagc 3240
   gtcctcttaa aagatgcctt aatccattcc ttgaggacag accttagttg aaatgatagc 3300
   agaatgtgct tctctctggc agctggcctt ctgcttctga gttgcacatt aatcagatta 3360
   gcctgtattc tcttcagtga attttgataa tggcttccag actctttggc gttggagacg 3420
   cctgttagga tcttcaagtc ccatcataga aaattgaaac acagagttgt tctgctgata 3480
   gttttgggga tacgtccatc tttttaaggg attgctttca tctaattctg gcaggacctc 3540
   accaaaagat ccagcctcat acctacatca gacaaaatat cgccgttgtt ccttctgtac 3600
   taaagtattg tgttttgctt tggaaacacc cactcacttt gcaatagccg tgcaagatga 3660
   atgcagatta cactgatctt atgtgttaca aaattggaga aagtatttaa taaaacctgt 3720
   taatttttat actgacaata aaaatgtttc tacagatatt aatgttaaca agacaaaata 3780
   aatgtcacgc aacttatttt tttaataaaa aaaaaaaaaa a                     3821
<210> 134
   <211> 3708
   <212> DNA
   <213> Homo sapiens
<400> 134
   aatttgttga ggaatttccc cctagccttg accccttgac agctcccgct cctactcagt 60
   gctggggaga agtagggagg ccttaagcga agagatgggt ctgcactttg gaggagccgg 120
   acactgttga ctttcctgat gtgaaatcta cccaggaaca aaacaccagt gactgcagca 180
   gcagcggcag cgcctcggtt cctgagccca ccgcaggctg aaggcattgc gcgtagtcca 240
   tgcccgtaga ggaagtgtgc agatgggatt aacgtccaca tggagatatg gaagaggacc 300
   ggggattggt accgtaacca tggtcagctg gggtcgtttc atctgcctgg tcgtggtcac 360
   catggcaacc ttgtccctgg cccggccctc cttcagttta gttgaggata ccacattaga 420
   gccagaagat gccatctcat ccggagatga tgaggatgac accgatggtg cggaagattt 480
   tgtcagtgag aacagtaaca acaagagagc accatactgg accaacacag aaaagatgga 540
   aaagcggctc catgctgtgc ctgcggccaa cactgtcaag tttcgctgcc cagccggggg 600
   gaacccaatg ccaaccatgc ggtggctgaa aaacgggaag gagtttaagc aggagcatcg 660
   cattggaggc tacaaggtac gaaaccagca ctggagcctc attatggaaa gtgtggtccc 720
   atctgacaag ggaaattata cctgtgtagt ggagaatgaa tacgggtcca tcaatcacac 780
   gtaccacctg gatgttgtgg agcgatcgcc tcaccggccc atcctccaag ccggactgcc 840
   ggcaaatgcc tccacagtgg tcggaggaga cgtagagttt gtctgcaagg tttacagtga 900
   tgcccagccc cacatccagt ggatcaagca cgtggaaaag aacggcagta aatacgggcc 960
   cgacgggctg ccctacctca aggttctcaa ggccgccggt gttaacacca cggacaaaga 1020
   gattgaggtt ctctatattc ggaatgtaac ttttgaggac gctggggaat atacgtgctt 1080
   ggcgggtaat tctattggga tatcctttca ctctgcatgg ttgacagttc tgccagcgcc 1140
   tggaagagaa aaggagatta cagcttcccc agactacctg gagatagcca tttactgcat 1200
   aggggtcttc ttaatcgcct gtatggtggt aacagtcatc ctgtgccgaa tgaagaacac 1260
   gaccaagaag ccagacttca gcagccagcc ggctgtgcac aagctgacca aacgtatccc 1320
   cctgcggaga caggtaacag tttcggctga gtccagctcc tccatgaact ccaacacccc 1380
   gctggtgagg ataacaacac gcctctcttc aacggcagac acccccatgc tggcaggggt 1440
   ctccgagtat gaacttccag aggacccaaa atgggagttt ccaagagata agctgacact 1500
   gggcaagccc ctgggagaag gttgctttgg gcaagtggtc atggcggaag cagtgggaat 1560
   tgacaaagac aagcccaagg aggcggtcac cgtggccgtg aagatgttga aagatgatgc 1620
   cacagagaaa gacctttctg atctggtgtc agagatggag atgatgaaga tgattgggaa 1680
   acacaagaat atcataaatc ttcttggagc ctgcacacag gatgggcctc tctatgtcat 1740
   agttgagtat gcctctaaag gcaacctccg agaatacctc cgagcccgga ggccacccgg 1800
   gatggagtac tcctatgaca ttaaccgtgt tcctgaggag cagatgacct tcaaggactt 1860
   ggtgtcatgc acctaccagc tggccagagg catggagtac ttggcttccc aaaaatgtat 1920
   tcatcgagat ttagcagcca gaaatgtttt ggtaacagaa aacaatgtga tgaaaatagc 1980
   agactttgga ctcgccagag atatcaacaa tatagactat tacaaaaaga ccaccaatgg 2040
   gcggcttcca gtcaagtgga tggctccaga agccctgttt gatagagtat acactcatca 2100
   gagtgatgtc tggtccttcg gggtgttaat gtgggagatc ttcactttag ggggctcgcc 2160
   ctacccaggg attcccgtgg aggaactttt taagctgctg aaggaaggac acagaatgga 2220
   taagccagcc aactgcacca acgaactgta catgatgatg agggactgtt ggcatgcagt 2280
   gccctcccag agaccaacgt tcaagcagtt ggtagaagac ttggatcgaa ttctcactct 2340
   cacaaccaat gaggaggaga agaaggtttc tggagcagtg gactgccaca agccaccatg 2400
   taacccctct cacctgccgt gcgtactggc tgtggaccag taggactcaa ggtggacgtg 2460
   cgttctgcct tccttgttaa ttttgtaata attggagaag atttatgtca gcacacactt 2520
   acagagcaca aatgcagtat ataggtgctg gatgtatgta aatatattca aattatgtat 2580
   aaatatatat tatatattta caaggagtta ttttttgtat tgattttaaa tggatgtccc 2640
   aatgcaccta gaaaattggt ctctcttttt ttaatagcta tttgctaaat gctgttctta 2700
   cacataattt cttaatttcc accgagcaga ggtggaaaaa tacttttgct ttcagggaaa 2760
   atggtataac gttaatttat taataaattg gtaatataca aaacaattaa tcatttatag 2820
   ttttttttgt aatttaagtg gcatttctat gcaggcagca cagcagacta gttaatctat 2880
   tgcttggact taactagtta tcagatcctt tgaaaagaga atatttacaa tatatgacta 2940
   atttggggaa aatgaagttt tgatttattt gtgtttaaat gctgctgtca gacgattgtt 3000
   cttagacctc ctaaatgccc catattaaaa gaactcattc ataggaaggt gtttcatttt 3060
   ggtgtgcaac cctgtcatta cgtcaacgca acgtctaact ggacttccca agataaatgg 3120
   taccagcgtc ctcttaaaag atgccttaat ccattccttg aggacagacc ttagttgaaa 3180
   tgatagcaga atgtgcttct ctctggcagc tggccttctg cttctgagtt gcacattaat 3240
   cagattagcc tgtattctct tcagtgaatt ttgataatgg cttccagact ctttggcgtt 3300
   ggagacgcct gttaggatct tcaagtccca tcatagaaaa ttgaaacaca gagttgttct 3360
   gctgatagtt ttggggatac gtccatcttt ttaagggatt gctttcatct aattctggca 3420
   ggacctcacc aaaagatcca gcctcatacc tacatcagac aaaatatcgc cgttgttcct 3480
   tctgtactaa agtattgtgt tttgctttgg aaacacccac tcactttgca atagccgtgc 3540
   aagatgaatg cagattacac tgatcttatg tgttacaaaa ttggagaaag tatttaataa 3600
   aacctgttaa tttttatact gacaataaaa atgtttctac agatattaat gttaacaaga 3660
   caaaataaat gtcacgcaac ttattttttt aataaaaaaa aaaaaaaa                3708
<210> 135
   <211> 4103
   <212> DNA
   <213> Homo sapiens
<400> 135
   gagcacacat tgcctcactg aagtggctgc acgtatctga gtcctgtagc tactgtttta 60
   tctctgtttc ttaaaagtat gcttttaaaa agattagcct cacacatttc tgtggaccgg 120
   tctggtggta tcacctggga ctctgaggtg aggatggaag gatttagcag ataatgaaaa 180
   agaactctgt ttgcgcacat ttgagaggct gaaaaatggt tttatcccac ttgggctgga 240
   gtgatttggc attggggaag attccctgac tcgccaatct ctttccttta gtgactgcag 300
   cagcagcggc agcgcctcgg ttcctgagcc caccgcaggc tgaaggcatt gcgcgtagtc 360
   catgcccgta gaggaagtgt gcagatggga ttaacgtcca catggagata tggaagagga 420
   ccggggattg gtaccgtaac catggtcagc tggggtcgtt tcatctgcct ggtcgtggtc 480
   accatggcaa ccttgtccct ggcccggccc tccttcagtt tagttgagga taccacatta 540
   gagccagaag gagcaccata ctggaccaac acagaaaaga tggaaaagcg gctccatgct 600
   gtgcctgcgg ccaacactgt caagtttcgc tgcccagccg gggggaaccc aatgccaacc 660
   atgcggtggc tgaaaaacgg gaaggagttt aagcaggagc atcgcattgg aggctacaag 720
   gtacgaaacc agcactggag cctcattatg gaaagtgtgg tcccatctga caagggaaat 780
   tatacctgtg tagtggagaa tgaatacggg tccatcaatc acacgtacca cctggatgtt 840
   gtggagcgat cgcctcaccg gcccatcctc caagccggac tgccggcaaa tgcctccaca 900
   gtggtcggag gagacgtaga gtttgtctgc aaggtttaca gtgatgccca gccccacatc 960
   cagtggatca agcacgtgga aaagaacggc agtaaatacg ggcccgacgg gctgccctac 1020
   ctcaaggttc tcaaggccgc cggtgttaac accacggaca aagagattga ggttctctat 1080
   attcggaatg taacttttga ggacgctggg gaatatacgt gcttggcggg taattctatt 1140
   gggatatcct ttcactctgc atggttgaca gttctgccag cgcctggaag agaaaaggag 1200
   attacagctt ccccagacta cctggagata gccatttact gcataggggt cttcttaatc 1260
   gcctgtatgg tggtaacagt catcctgtgc cgaatgaaga acacgaccaa gaagccagac 1320
   ttcagcagcc agccggctgt gcacaagctg accaaacgta tccccctgcg gagacaggta 1380
   acagtttcgg ctgagtccag ctcctccatg aactccaaca ccccgctggt gaggataaca 1440
   acacgcctct cttcaacggc agacaccccc atgctggcag gggtctccga gtatgaactt 1500
   ccagaggacc caaaatggga gtttccaaga gataagctga cactgggcaa gcccctggga 1560
   gaaggttgct ttgggcaagt ggtcatggcg gaagcagtgg gaattgacaa agacaagccc 1620
   aaggaggcgg tcaccgtggc cgtgaagatg ttgaaagatg atgccacaga gaaagacctt 1680
   tctgatctgg tgtcagagat ggagatgatg aagatgattg ggaaacacaa gaatatcata 1740
   aatcttcttg gagcctgcac acaggatggg cctctctatg tcatagttga gtatgcctct 1800
   aaaggcaacc tccgagaata cctccgagcc cggaggccac ccgggatgga gtactcctat 1860
   gacattaacc gtgttcctga ggagcagatg accttcaagg acttggtgtc atgcacctac 1920
   cagctggcca gaggcatgga gtacttggct tcccaaaaat gtattcatcg agatttagca 1980
   gccagaaatg ttttggtaac agaaaacaat gtgatgaaaa tagcagactt tggactcgcc 2040
   agagatatca acaatataga ctattacaaa aagaccacca atgggcggct tccagtcaag 2100
   tggatggctc cagaagccct gtttgataga gtatacactc atcagagtga tgtctggtcc 2160
   ttcggggtgt taatgtggga gatcttcact ttagggggct cgccctaccc agggattccc 2220
   gtggaggaac tttttaagct gctgaaggaa ggacacagaa tggataagcc agccaactgc 2280
   accaacgaac tgtacatgat gatgagggac tgttggcatg cagtgccctc ccagagacca 2340
   acgttcaagc agttggtaga agacttggat cgaattctca ctctcacaac caatgaggaa 2400
   tacttggacc tcagccaacc tctcgaacag tattcaccta gttaccctga cacaagaagt 2460
   tcttgttctt caggagatga ttctgttttt tctccagacc ccatgcctta cgaaccatgc 2520
   cttcctcagt atccacacat aaacggcagt gttaaaacat gaatgactgt gtctgcctgt 2580
   ccccaaacag gacagcactg ggaacctagc tacactgagc agggagacca tgcctcccag 2640
   agcttgttgt ctccacttgt atatatggat cagaggagta aataattgga aaagtaatca 2700
   gcatatgtgt aaagatttat acagttgaaa acttgtaatc ttccccagga ggagaagaag 2760
   gtttctggag cagtggactg ccacaagcca ccatgtaacc cctctcacct gccgtgcgta 2820
   ctggctgtgg accagtagga ctcaaggtgg acgtgcgttc tgccttcctt gttaattttg 2880
   taataattgg agaagattta tgtcagcaca cacttacaga gcacaaatgc agtatatagg 2940
   tgctggatgt atgtaaatat attcaaatta tgtataaata tatattatat atttacaagg 3000
   agttattttt tgtattgatt ttaaatggat gtcccaatgc acctagaaaa ttggtctctc 3060
   tttttttaat agctatttgc taaatgctgt tcttacacat aatttcttaa ttttcaccga 3120
   gcagaggtgg aaaaatactt ttgctttcag ggaaaatggt ataacgttaa tttattaata 3180
   aattggtaat atacaaaaca attaatcatt tatagttttt tttgtaattt aagtggcatt 3240
   tctatgcagg cagcacagca gactagttaa tctattgctt ggacttaact agttatcaga 3300
   tcctttgaaa agagaatatt tacaatatat gactaatttg ggaaaatgaa agttttgatt 3360
   tatttgtgtt taaatgctgc tgtcagacga ttgttcttag acctcctaaa tgccccatat 3420
   taaaagaact cattcatagg aaggtgtttc attttggtgt gcaaccctgt cattacgtca 3480
   acgcaacgtc taactggact tcccaagata aatggtacca gcgtcctctt aaaagatgcc 3540
   ttaatccatt ccttgaggac agaccttagt tgaaatgata gcagaatgtg cttctctctg 3600
   gcagctggcc ttctgcttct gagttgcaca ttaatcagat tagcctgtat tctcttcagt 3660
   gaattttgat aatggcttcc agactctttg gcgttggaga cgcctgttag gatcttcaag 3720
   tcccatcata gaaaattgaa acacagagtt gttctgctga tagttttggg gatacgtcca 3780
   tctttttaag ggattgcttt catctaattc tggcaggacc tcaccaaaag atccagcctc 3840
   atacctacat cagacaaaat atcgccgttg ttccttctgt actaaagtat tgtgttttgc 3900
   tttggaaaca cccactcact ttgcaatagc cgtgcaagat gaatgcagat tacactgatc 3960
   ttatgtgtta caaaattgga gaaagtattt aataaaacct gttaattttt atactgacaa 4020
   taaaaatgtt tctacagata ttaatgttaa caagacaaaa taaatgtcac gcaacttatt 4080
   tttttaataa aaaaaaaaaa aaa 4103
<210> 136
   <211> 4306
   <212> DNA
   <213> Homo sapiens
<400> 136
   ggcggcggct ggaggagagc gcggtggaga gccgagcggg cgggcggcgg gtgcggagcg 60
   ggcgagggag cgcgcgcggc cgccacaaag ctcgggcgcc gcggggctgc atgcggcgta 120
   cctggcccgg cgcggcgact gctctccggg ctggcggggg ccggccgcga gccccggggg 180
   ccccgaggcc gcagcttgcc tgcgcgctct gagccttcgc aactcgcgag caaagtttgg 240
   tggaggcaac gccaagcctg agtcctttct tcctctcgtt ccccaaatcc gagggcagcc 300
   cgcgggcgtc atgcccgcgc tcctccgcag cctggggtac gcgtgaagcc cgggaggctt 360
   ggcgccggcg aagacccaag gaccactctt ctgcgtttgg agttgctccc cgcaaccccg 420
   ggctcgtcgc tttctccatc ccgacccacg cggggcgcgg ggacaacaca ggtcgcggag 480
   gagcgttgcc attcaagtga ctgcagcagc agcggcagcg cctcggttcc tgagcccacc 540
   gcaggctgaa ggcattgcgc gtagtccatg cccgtagagg aagtgtgcag atgggattaa 600
   cgtccacatg gagatatgga agaggaccgg ggattggtac cgtaaccatg gtcagctggg 660
   gtcgtttcat ctgcctggtc gtggtcacca tggcaacctt gtccctggcc cggccctcct 720
   tcagtttagt tgaggatacc acattagagc cagaagagcc accaaccaaa taccaaatct 780
   ctcaaccaga agtgtacgtg gctgcgccag gggagtcgct agaggtgcgc tgcctgttga 840
   aagatgccgc cgtgatcagt tggactaagg atggggtgca cttggggccc aacaatagga 900
   cagtgcttat tggggagtac ttgcagataa agggcgccac gcctagagac tccggcctct 960
   atgcttgtac tgccagtagg actgtagaca gtgaaacttg gtacttcatg gtgaatgtca 1020
   cagatgccat ctcatccgga gatgatgagg atgacaccga tggtgcggaa gattttgtca 1080
   gtgagaacag taacaacaag agagcaccat actggaccaa cacagaaaag atggaaaagc 1140
   ggctccatgc tgtgcctgcg gccaacactg tcaagtttcg ctgcccagcc ggggggaacc 1200
   caatgccaac catgcggtgg ctgaaaaacg ggaaggagtt taagcaggag catcgcattg 1260
   gaggctacaa ggtacgaaac cagcactgga gcctcattat ggaaagtgtg gtcccatctg 1320
   acaagggaaa ttatacctgt gtagtggaga atgaatacgg gtccatcaat cacacgtacc 1380
   acctggatgt tgtggagcga tcgcctcacc ggcccatcct ccaagccgga ctgccggcaa 1440
   atgcctccac agtggtcgga ggagacgtag agtttgtctg caaggtttac agtgatgccc 1500
   agccccacat ccagtggatc aagcacgtgg aaaagaacgg cagtaaatac gggcccgacg 1560
   ggctgcccta cctcaaggtt ctcaaggttt cggctgagtc cagctcctcc atgaactcca 1620
   acaccccgct ggtgaggata acaacacgcc tctcttcaac ggcagacacc cccatgctgg 1680
   caggggtctc cgagtatgaa cttccagagg acccaaaatg ggagtttcca agagataagc 1740
   tgacactggg caagcccctg ggagaaggtt gctttgggca agtggtcatg gcggaagcag 1800
   tgggaattga caaagacaag cccaaggagg cggtcaccgt ggccgtgaag atgttgaaag 1860
   atgatgccac agagaaagac ctttctgatc tggtgtcaga gatggagatg atgaagatga 1920
   ttgggaaaca caagaatatc ataaatcttc ttggagcctg cacacaggat gggcctctct 1980
   atgtcatagt tgagtatgcc tctaaaggca acctccgaga atacctccga gcccggaggc 2040
   cacccgggat ggagtactcc tatgacatta accgtgttcc tgaggagcag atgaccttca 2100
   aggacttggt gtcatgcacc taccagctgg ccagaggcat ggagtacttg gcttcccaaa 2160
   aatgtattca tcgagattta gcagccagaa atgttttggt aacagaaaac aatgtgatga 2220
   aaatagcaga ctttggactc gccagagata tcaacaatat agactattac aaaaagacca 2280
   ccaatgggcg gcttccagtc aagtggatgg ctccagaagc cctgtttgat agagtataca 2340
   ctcatcagag tgatgtctgg tccttcgggg tgttaatgtg ggagatcttc actttagggg 2400
   gctcgcccta cccagggatt cccgtggagg aactttttaa gctgctgaag gaaggacaca 2460
   gaatggataa gccagccaac tgcaccaacg aactgtacat gatgatgagg gactgttggc 2520
   atgcagtgcc ctcccagaga ccaacgttca agcagttggt agaagacttg gatcgaattc 2580
   tcactctcac aaccaatgag gaatacttgg acctcagcca acctctcgaa cagtattcac 2640
   ctagttaccc tgacacaaga agttcttgtt cttcaggaga tgattctgtt ttttctccag 2700
   accccatgcc ttacgaacca tgccttcctc agtatccaca cataaacggc agtgttaaaa 2760
   catgaatgac tgtgtctgcc tgtccccaaa caggacagca ctgggaacct agctacactg 2820
   agcagggaga ccatgcctcc cagagcttgt tgtctccact tgtatatatg gatcagagga 2880
   gtaaataatt ggaaaagtaa tcagcatatg tgtaaagatt tatacagttg aaaacttgta 2940
   atcttcccca ggaggagaag aaggtttctg gagcagtgga ctgccacaag ccaccatgta 3000
   acccctctca cctgccgtgc gtactggctg tggaccagta ggactcaagg tggacgtgcg 3060
   ttctgccttc cttgttaatt ttgtaataat tggagaagat ttatgtcagc acacacttac 3120
   agagcacaaa tgcagtatat aggtgctgga tgtatgtaaa tatattcaaa ttatgtataa 3180
   atatatatta tatatttaca aggagttatt ttttgtattg attttaaatg gatgtcccaa 3240
   tgcacctaga aaattggtct ctcttttttt aatagctatt tgctaaatgc tgttcttaca 3300
   cataatttct taattttcac cgagcagagg tggaaaaata cttttgcttt cagggaaaat 3360
   ggtataacgt taatttatta ataaattggt aatatacaaa acaattaatc atttatagtt 3420
   ttttttgtaa tttaagtggc atttctatgc aggcagcaca gcagactagt taatctattg 3480
   cttggactta actagttatc agatcctttg aaaagagaat atttacaata tatgactaat 3540
   ttggggaaaa tgaagttttg atttatttgt gtttaaatgc tgctgtcaga cgattgttct 3600
   tagacctcct aaatgcccca tattaaaaga actcattcat aggaaggtgt ttcattttgg 3660
   tgtgcaaccc tgtcattacg tcaacgcaac gtctaactgg acttcccaag ataaatggta 3720
   ccagcgtcct cttaaaagat gccttaatcc attccttgag gacagacctt agttgaaatg 3780
   atagcagaat gtgcttctct ctggcagctg gccttctgct tctgagttgc acattaatca 3840
   gattagcctg tattctcttc agtgaatttt gataatggct tccagactct ttggcgttgg 3900
   agacgcctgt taggatcttc aagtcccatc atagaaaatt gaaacacaga gttgttctgc 3960
   tgatagtttt ggggatacgt ccatcttttt aagggattgc tttcatctaa ttctggcagg 4020
   acctcaccaa aagatccagc ctcataccta catcagacaa aatatcgccg ttgttccttc 4080
   tgtactaaag tattgtgttt tgctttggaa acacccactc actttgcaat agccgtgcaa 4140
   gatgaatgca gattacactg atcttatgtg ttacaaaatt ggagaaagta tttaataaaa 4200
   cctgttaatt tttatactga caataaaaat gtttctacag atattaatgt taacaagaca 4260
   aaataaatgt cacgcaactt atttttttaa taaaaaaaaa aaaaaa 4306
<210> 137
   <211> 4303
   <212> DNA
   <213> Homo sapiens
<400> 137
   ggcggcggct ggaggagagc gcggtggaga gccgagcggg cgggcggcgg gtgcggagcg 60
   ggcgagggag cgcgcgcggc cgccacaaag ctcgggcgcc gcggggctgc atgcggcgta 120
   cctggcccgg cgcggcgact gctctccggg ctggcggggg ccggccgcga gccccggggg 180
   ccccgaggcc gcagcttgcc tgcgcgctct gagccttcgc aactcgcgag caaagtttgg 240
   tggaggcaac gccaagcctg agtcctttct tcctctcgtt ccccaaatcc gagggcagcc 300
   cgcgggcgtc atgcccgcgc tcctccgcag cctggggtac gcgtgaagcc cgggaggctt 360
   ggcgccggcg aagacccaag gaccactctt ctgcgtttgg agttgctccc cgcaaccccg 420
   ggctcgtcgc tttctccatc ccgacccacg cggggcgcgg ggacaacaca ggtcgcggag 480
   gagcgttgcc attcaagtga ctgcagcagc agcggcagcg cctcggttcc tgagcccacc 540
   gcaggctgaa ggcattgcgc gtagtccatg cccgtagagg aagtgtgcag atgggattaa 600
   cgtccacatg gagatatgga agaggaccgg ggattggtac cgtaaccatg gtcagctggg 660
   gtcgtttcat ctgcctggtc gtggtcacca tggcaacctt gtccctggcc cggccctcct 720
   tcagtttagt tgaggatacc acattagagc cagaaggagc accatactgg accaacacag 780
   aaaagatgga aaagcggctc catgctgtgc ctgcggccaa cactgtcaag tttcgctgcc 840
   cagccggggg gaacccaatg ccaaccatgc ggtggctgaa aaacgggaag gagtttaagc 900
   aggagcatcg cattggaggc tacaaggtac gaaaccagca ctggagcctc attatggaaa 960
   gtgtggtccc atctgacaag ggaaattata cctgtgtagt ggagaatgaa tacgggtcca 1020
   tcaatcacac gtaccacctg gatgttgtgg agcgatcgcc tcaccggccc atcctccaag 1080
   ccggactgcc ggcaaatgcc tccacagtgg tcggaggaga cgtagagttt gtctgcaagg 1140
   tttacagtga tgcccagccc cacatccagt ggatcaagca cgtggaaaag aacggcagta 1200
   aatacgggcc cgacgggctg ccctacctca aggttctcaa ggccgccggt gttaacacca 1260
   cggacaaaga gattgaggtt ctctatattc ggaatgtaac ttttgaggac gctggggaat 1320
   atacgtgctt ggcgggtaat tctattggga tatcctttca ctctgcatgg ttgacagttc 1380
   tgccagcgcc tggaagagaa aaggagatta cagcttcccc agactacctg gagatagcca 1440
   tttactgcat aggggtcttc ttaatcgcct gtatggtggt aacagtcatc ctgtgccgaa 1500
   tgaagaacac gaccaagaag ccagacttca gcagccagcc ggctgtgcac aagctgacca 1560
   aacgtatccc cctgcggaga caggtttcgg ctgagtccag ctcctccatg aactccaaca 1620
   ccccgctggt gaggataaca acacgcctct cttcaacggc agacaccccc atgctggcag 1680
   gggtctccga gtatgaactt ccagaggacc caaaatggga gtttccaaga gataagctga 1740
   cactgggcaa gcccctggga gaaggttgct ttgggcaagt ggtcatggcg gaagcagtgg 1800
   gaattgacaa agacaagccc aaggaggcgg tcaccgtggc cgtgaagatg ttgaaagatg 1860
   atgccacaga gaaagacctt tctgatctgg tgtcagagat ggagatgatg aagatgattg 1920
   ggaaacacaa gaatatcata aatcttcttg gagcctgcac acaggatggg cctctctatg 1980
   tcatagttga gtatgcctct aaaggcaacc tccgagaata cctccgagcc cggaggccac 2040
   ccgggatgga gtactcctat gacattaacc gtgttcctga ggagcagatg accttcaagg 2100
   acttggtgtc atgcacctac cagctggcca gaggcatgga gtacttggct tcccaaaaat 2160
   gtattcatcg agatttagca gccagaaatg ttttggtaac agaaaacaat gtgatgaaaa 2220
   tagcagactt tggactcgcc agagatatca acaatataga ctattacaaa aagaccacca 2280
   atgggcggct tccagtcaag tggatggctc cagaagccct gtttgataga gtatacactc 2340
   atcagagtga tgtctggtcc ttcggggtgt taatgtggga gatcttcact ttagggggct 2400
   cgccctaccc agggattccc gtggaggaac tttttaagct gctgaaggaa ggacacagaa 2460
   tggataagcc agccaactgc accaacgaac tgtacatgat gatgagggac tgttggcatg 2520
   cagtgccctc ccagagacca acgttcaagc agttggtaga agacttggat cgaattctca 2580
   ctctcacaac caatgaggaa tacttggacc tcagccaacc tctcgaacag tattcaccta 2640
   gttaccctga cacaagaagt tcttgttctt caggagatga ttctgttttt tctccagacc 2700
   ccatgcctta cgaaccatgc cttcctcagt atccacacat aaacggcagt gttaaaacat 2760
   gaatgactgt gtctgcctgt ccccaaacag gacagcactg ggaacctagc tacactgagc 2820
   agggagacca tgcctcccag agcttgttgt ctccacttgt atatatggat cagaggagta 2880
   aataattgga aaagtaatca gcatatgtgt aaagatttat acagttgaaa acttgtaatc 2940
   ttccccagga ggagaagaag gtttctggag cagtggactg ccacaagcca ccatgtaacc 3000
   cctctcacct gccgtgcgta ctggctgtgg accagtagga ctcaaggtgg acgtgcgttc 3060
   tgccttcctt gttaattttg taataattgg agaagattta tgtcagcaca cacttacaga 3120
   gcacaaatgc agtatatagg tgctggatgt atgtaaatat attcaaatta tgtataaata 3180
   tatattatat atttacaagg agttattttt tgtattgatt ttaaatggat gtcccaatgc 3240
   acctagaaaa ttggtctctc tttttttaat agctatttgc taaatgctgt tcttacacat 3300
   aatttcttaa ttttcaccga gcagaggtgg aaaaatactt ttgctttcag ggaaaatggt 3360
   ataacgttaa tttattaata aattggtaat atacaaaaca attaatcatt tatagttttt 3420
   tttgtaattt aagtggcatt tctatgcagg cagcacagca gactagttaa tctattgctt 3480
   ggacttaact agttatcaga tcctttgaaa agagaatatt tacaatatat gactaatttg 3540
   gggaaaatga agttttgatt tatttgtgtt taaatgctgc tgtcagacga ttgttcttag 3600
   acctcctaaa tgccccatat taaaagaact cattcatagg aaggtgtttc attttggtgt 3660
   gcaaccctgt cattacgtca acgcaacgtc taactggact tcccaagata aatggtacca 3720
   gcgtcctctt aaaagatgcc ttaatccatt ccttgaggac agaccttagt tgaaatgata 3780
   gcagaatgtg cttctctctg gcagctggcc ttctgcttct gagttgcaca ttaatcagat 3840
   tagcctgtat tctcttcagt gaattttgat aatggcttcc agactctttg gcgttggaga 3900
   cgcctgttag gatcttcaag tcccatcata gaaaattgaa acacagagtt gttctgctga 3960
   tagttttggg gatacgtcca tctttttaag ggattgcttt catctaattc tggcaggacc 4020
   tcaccaaaag atccagcctc atacctacat cagacaaaat atcgccgttg ttccttctgt 4080
   actaaagtat tgtgttttgc tttggaaaca cccactcact ttgcaatagc cgtgcaagat 4140
   gaatgcagat tacactgatc ttatgtgtta caaaattgga gaaagtattt aataaaacct 4200
   gttaattttt atactgacaa taaaaatgtt tctacagata ttaatgttaa caagacaaaa 4260
   taaatgtcac gcaacttatt tttttaataa aaaaaaaaaa aaa                   4303
<210> 138
   <211> 3011
   <212> DNA
   <213> Homo sapiens
<400> 138
   ggcggcggct ggaggagagc gcggtggaga gccgagcggg cgggcggcgg gtgcggagcg 60
   ggcgagggag cgcgcgcggc cgccacaaag ctcgggcgcc gcggggctgc atgcggcgta 120
   cctggcccgg cgcggcgact gctctccggg ctggcggggg ccggccgcga gccccggggg 180
   ccccgaggcc gcagcttgcc tgcgcgctct gagccttcgc aactcgcgag caaagtttgg 240
   tggaggcaac gccaagcctg agtcctttct tcctctcgtt ccccaaatcc gagggcagcc 300
   cgcgggcgtc atgcccgcgc tcctccgcag cctggggtac gcgtgaagcc cgggaggctt 360
   ggcgccggcg aagacccaag gaccactctt ctgcgtttgg agttgctccc cgcaaccccg 420
   ggctcgtcgc tttctccatc ccgacccacg cggggcgcgg ggacaacaca ggtcgcggag 480
   gagcgttgcc attcaagtga ctgcagcagc agcggcagcg cctcggttcc tgagcccacc 540
   gcaggctgaa ggcattgcgc gtagtccatg cccgtagagg aagtgtgcag atgggattaa 600
   cgtccacatg gagatatgga agaggaccgg ggattggtac cgtaaccatg gtcagctggg 660
   gtcgtttcat ctgcctggtc gtggtcacca tggcaacctt gtccctggcc cggccctcct 720
   tcagtttagt tgaggatacc acattagagc cagaagatgc catctcatcc ggagatgatg 780
   aggatgacac cgatggtgcg gaagattttg tcagtgagaa cagtaacaac aagagagcac 840
   catactggac caacacagaa aagatggaaa agcggctcca tgctgtgcct gcggccaaca 900
   ctgtcaagtt tcgctgccca gccgggggga acccaatgcc aaccatgcgg tggctgaaaa 960
   acgggaagga gtttaagcag gagcatcgca ttggaggcta caaggtacga aaccagcact 1020
   ggagcctcat tatggaaagt gtggtcccat ctgacaaggg aaattatacc tgtgtagtgg 1080
   agaatgaata cgggtccatc aatcacacgt accacctgga tgttgtggag cgatcgcctc 1140
   accggcccat cctccaagcc ggactgccgg caaatgcctc cacagtggtc ggaggagacg 1200
   tagagtttgt ctgcaaggtt tacagtgatg cccagcccca catccagtgg atcaagcacg 1260
   tggaaaagaa cggcagtaaa tacgggcccg acgggctgcc ctacctcaag gttctcaagc 1320
   actcggggat aaatagttcc aatgcagaag tgctggctct gttcaatgtg accgaggcgg 1380
   atgctgggga atatatatgt aaggtctcca attatatagg gcaggccaac cagtctgcct 1440
   ggctcactgt cctgccaaaa cagcaagcgc ctggaagaga aaaggagatt acagcttccc 1500
   cagactacct ggagatagcc atttactgca taggggtctt cttaatcgcc tgtatggtgg 1560
   taacagtcat cctgtgccga atgaagaaca cgaccaagaa gccagacttc agcagccagc 1620
   cggctgtgca caagctgacc aaacgtatcc ccctgcggag acaggtaaca gtttcggctg 1680
   agtccagctc ctccatgaac tccaacaccc cgctggtgag gataacaaca cgcctctctt 1740
   caacggcaga cacccccatg ctggcagggg tctccgagta tgaacttcca gaggacccaa 1800
   aatgggagtt tccaagagat aagctgacac tgggcaagcc cctgggagaa ggttgctttg 1860
   ggcaagtggt catggcggaa gcagtgggaa ttgacaaaga caagcccaag gaggcggtca 1920
   ccgtggccgt gaagatgttg aaagatgatg ccacagagaa agacctttct gatctggtgt 1980
   cagagatgga gatgatgaag atgattggga aacacaagaa tatcataaat cttcttggag 2040
   cctgcacaca ggatgggcct ctctatgtca tagttgagta tgcctctaaa ggcaacctcc 2100
   gagaatacct ccgagcccgg aggccacccg ggatggagta ctcctatgac attaaccgtg 2160
   ttcctgagga gcagatgacc ttcaaggact tggtgtcatg cacctaccag ctggccagag 2220
   gcatggagta cttggcttcc caaaaatgta ttcatcgaga tttagcagcc agaaatgttt 2280
   tggtaacaga aaacaatgtg atgaaaatag cagactttgg actcgccaga gatatcaaca 2340
   atatagacta ttacaaaaag accaccaatg ggcggcttcc agtcaagtgg atggctccag 2400
   aagccctgtt tgatagagta tacactcatc agagtgatgt ctggtccttc ggggtgttaa 2460
   tgtgggagat cttcacttta gggggctcgc cctacccagg gattcccgtg gaggaacttt 2520
   ttaagctgct gaaggaagga cacagaatgg ataagccagc caactgcacc aacgaactgt 2580
   acatgatgat gagggactgt tggcatgcag tgccctccca gagaccaacg ttcaagcagt 2640
   tggtagaaga cttggatcga attctcactc tcacaaccaa tgagatctga aagtttatgg 2700
   cttcattgag aaactgggaa aagttggtca ggcgcagtgg ctcatgcctg taatcccagc 2760
   actttgggag gccgaggcag gcggatcatg aggtcaggag ttccagacca gcctggccaa 2820
   catggtgaaa ccctgtctct actaaagata caaaaaatta gccgggcgtg ttggtgtgca 2880
   cctgtaatcc cagctactcc gggaggctga ggcaggagag tcacttgaac cggggaggcg 2940
   gaggttgcag tgagccgaga tcatgccatt gcattccagc cttggcgaca gagcgagact 3000
   ccgtctcaaa a                                                      3011
<210> 139
   <211> 4286
   <212> DNA
   <213> Homo sapiens
<400> 139
<210> 140
   <211> 3950
   <212> DNA
   <213> Homo sapiens
<400> 140
   gtcgcgggca gctggcgccg cgcggtcctg ctctgccggt cgcacggacg caccggcggg 60
   ccgccggccg gagggacggg gcgggagctg ggcccgcgga cagcgagccg gagcgggagc 120
   cgcgcgtagc gagccgggct ccggcgctcg ccagtctccc gagcggcgcc cgcctcccgc 180
   cggtgcccgc gccgggccgt ggggggcagc atgcccgcgc gcgctgcctg aggacgccgc 240
   ggcccccgcc cccgccatgg gcgcccctgc ctgcgccctc gcgctctgcg tggccgtggc 300
   catcgtggcc ggcgcctcct cggagtcctt ggggacggag cagcgcgtcg tggggcgagc 360
   ggcagaagtc ccgggcccag agcccggcca gcaggagcag ttggtcttcg gcagcgggga 420
   tgctgtggag ctgagctgtc ccccgcccgg gggtggtccc atggggccca ctgtctgggt 480
   caaggatggc acagggctgg tgccctcgga gcgtgtcctg gtggggcccc agcggctgca 540
   ggtgctgaat gcctcccacg aggactccgg ggcctacagc tgccggcagc ggctcacgca 600
   gcgcgtactg tgccacttca gtgtgcgggt gacagacgct ccatcctcgg gagatgacga 660
   agacggggag gacgaggctg aggacacagg tgtggacaca ggggcccctt actggacacg 720
   gcccgagcgg atggacaaga agctgctggc cgtgccggcc gccaacaccg tccgcttccg 780
   ctgcccagcc gctggcaacc ccactccctc catctcctgg ctgaagaacg gcagggagtt 840
   ccgcggcgag caccgcattg gaggcatcaa gctgcggcat cagcagtgga gcctggtcat 900
   ggaaagcgtg gtgccctcgg accgcggcaa ctacacctgc gtcgtggaga acaagtttgg 960
   cagcatccgg cagacgtaca cgctggacgt gctggagcgc tccccgcacc ggcccatcct 1020
   gcaggcgggg ctgccggcca accagacggc ggtgctgggc agcgacgtgg agttccactg 1080
   caaggtgtac agtgacgcac agccccacat ccagtggctc aagcacgtgg aggtgaatgg 1140
   cagcaaggtg ggcccggacg gcacacccta cgttaccgtg ctcaaggtgt ccctggagtc 1200
   caacgcgtcc atgagctcca acacaccact ggtgcgcatc gcaaggctgt cctcagggga 1260
   gggccccacg ctggccaatg tctccgagct cgagctgcct gccgacccca aatgggagct 1320
   gtctcgggcc cggctgaccc tgggcaagcc ccttggggag ggctgcttcg gccaggtggt 1380
   catggcggag gccatcggca ttgacaagga ccgggccgcc aagcctgtca ccgtagccgt 1440
   gaagatgctg aaagacgatg ccactgacaa ggacctgtcg gacctggtgt ctgagatgga 1500
   gatgatgaag atgatcggga aacacaaaaa catcatcaac ctgctgggcg cctgcacgca 1560
   gggcgggccc ctgtacgtgc tggtggagta cgcggccaag ggtaacctgc gggagtttct 1620
   gcgggcgcgg cggcccccgg gcctggacta ctccttcgac acctgcaagc cgcccgagga 1680
   gcagctcacc ttcaaggacc tggtgtcctg tgcctaccag gtggcccggg gcatggagta 1740
   cttggcctcc cagaagtgca tccacaggga cctggctgcc cgcaatgtgc tggtgaccga 1800
   ggacaacgtg atgaagatcg cagacttcgg gctggcccgg gacgtgcaca acctcgacta 1860
   ctacaagaag acgaccaacg gccggctgcc cgtgaagtgg atggcgcctg aggccttgtt 1920
   tgaccgagtc tacactcacc agagtgacgt ctggtccttt ggggtcctgc tctgggagat 1980
   cttcacgctg gggggctccc cgtaccccgg catccctgtg gaggagctct tcaagctgct 2040
   gaaggagggc caccgcatgg acaagcccgc caactgcaca cacgacctgt acatgatcat 2100
   gcgggagtgc tggcatgccg cgccctccca gaggcccacc ttcaagcagc tggtggagga 2160
   cctggaccgt gtccttaccg tgacgtccac cgacgagtac ctggacctgt cggcgccttt 2220
   cgagcagtac tccccgggtg gccaggacac ccccagctcc agctcctcag gggacgactc 2280
   cgtgtttgcc cacgacctgc tgcccccggc cccacccagc agtgggggct cgcggacgtg 2340
   aagggccact ggtccccaac aatgtgaggg gtccctagca gcccaccctg ctgctggtgc 2400
   acagccactc cccggcatga gactcagtgc agatggagag acagctacac agagctttgg 2460
   tctgtgtgtg tgtgtgtgcg tgtgtgtgtg tgtgtgtgca catccgcgtg tgcctgtgtg 2520
   cgtgcgcatc ttgcctccag gtgcagaggt accctgggtg tccccgctgc tgtgcaacgg 2580
   tctcctgact ggtgctgcag caccgagggg cctttgttct ggggggaccc agtgcagaat 2640
   gtaagtgggc ccacccggtg ggacccccgt ggggcaggga gctgggcccg acatggctcc 2700
   ggcctctgcc tttgcaccac gggacatcac agggtgggcc tcggcccctc ccacacccaa 2760
   agctgagcct gcagggaagc cccacatgtc cagcaccttg tgcctggggt gttagtggca 2820
   ccgcctcccc acctccaggc tttcccactt cccaccctgc ccctcagaga ctgaaattac 2880
   gggtacctga agatgggagc ctttaccttt tatgcaaaag gtttattccg gaaactagtg 2940
   tacatttcta taaatagatg ctgtgtatat ggtatatata catatatata tataacatat 3000
   atggaagagg aaaaggctgg tacaacggag gcctgcgacc ctgggggcac aggaggcagg 3060
   catggccctg ggcggggcgt gggggggcgt ggagggaggc cccagggggt ctcacccatg 3120
   caagcagagg accagggcct tttctggcac cgcagttttg ttttaaaact ggacctgtat 3180
   atttgtaaag ctatttatgg gcccctggca ctcttgttcc cacaccccaa cacttccagc 3240
   atttagctgg ccacatggcg gagagtttta atttttaact tattgacaac cgagaaggtt 3300
   tatcccgccg atagagggac ggccaagaat gtacgtccag cctgccccgg agctggagga 3360
   tcccctccaa gcctaaaagg ttgttaatag ttggaggtga ttccagtgaa gatattttat 3420
   ttcctttgtc ctttttcagg agaattagat ttctatagga tttttcttta ggagatttat 3480
   tttttggact tcaaagcaag ctggtatttt catacaaatt cttctaattg ctgtgtgtcc 3540
   caggcaggga gacggtttcc agggaggggc cggccctgtg tgcaggttcc gatgttatta 3600
   gatgttacaa gtttatatat atctatatat ataatttatt gagtttttac aagatgtatt 3660
   tgttgtagac ttaacacttc ttacgcaatg cttctagagt tttatagcct ggactgctac 3720
   ctttcaaagc ttggagggaa gccgtgaatt cagttggttc gttctgtact gttactgggc 3780
   cctgagtctg ggcagctgtc ccttgcttgc ctgcagggcc atggctcagg gtggtctctt 3840
   cttggggccc agtgcatggt ggccagaggt gtcacccaaa ccggcaggtg cgattttgtt 3900
   aacccagcga cgaactttcc gaaaaataaa gacacctggt tgctaacctg            3950
<210> 141
   <211> 2085
   <212> DNA
   <213> Homo sapiens
<400> 141
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag gtgtccctgg agtccaacgc gtccatgagc 960
   tccaacacac cactggtgcg catcgcaagg ctgtcctcag ggagggcccc cacgctggcc 1020
   aatgtctccg agctcgagct gcctgccgac cccaaatggg agctgtctcg ggcccggctg 1080
   accctgggca agccccttgg ggagggctgc ttcggccagg tggtcatggc ggaggccatc 1140
   ggcattgaca aggaccgggc cgccaagcct gtcaccgtag ccgtgaagat gctgaaagac 1200
   gatgccactg acaaggacct gtcggacctg gtgtctgaga tggagatgat gaagatgatc 1260
   gggaaacaca aaaacatcat caacctgctg ggcgcctgca cgcagggcgg gcccctgtac 1320
   gtgctggtgg agtacgcggc caagggtaac ctgcgggagt ttctgcgggc gcggcggccc 1380
   ccgggcctgg actactcctt cgacacctgc aagccgcccg aggagcagct caccttcaag 1440
   gacctggtgt cctgtgccta ccaggtggcc cggggcatgg agtacttggc ctcccagaag 1500
   tgcatccaca gggacctggc tgcccgcaat gtgctggtga ccgaggacaa cgtgatgaag 1560
   atcgcagact tcgggctggc ccgggacgtg cacaacctcg actactacaa gaagacaacc 1620
   aacggccggc tgcccgtgaa gtggatggcg cctgaggcct tgtttgaccg agtctacact 1680
   caccagagtg acgtctggtc ctttggggtc ctgctctggg agatcttcac gctggggggc 1740
   tccccgtacc ccggcatccc tgtggaggag ctcttcaagc tgctgaagga gggccaccgc 1800
   atggacaagc ccgccaactg cacacacgac ctgtacatga tcatgcggga gtgctggcat 1860
   gccgcgccct cccagaggcc caccttcaag cagctggtgg aggacctgga ccgtgtcctt 1920
   accgtgacgt ccaccgacga gtacctggac ctgtcggcgc ctttcgagca gtactccccg 1980
   ggtggccagg acacccccag ctccagctcc tcaggggacg actccgtgtt tgcccacgac 2040
   ctgctgcccc cggccccacc cagcagtggg ggctcgcgga cgtga                 2085
<210> 142
   <211> 4431
   <212> DNA
   <213> Homo sapiens
<400> 142
<210> 143
   <211> 5311
   <212> DNA
   <213> Homo sapiens
<400> 143
   agcataacct tcggtggcag gacaaatcag gccagcacgc agtctgccaa gtcctgctcg 60
   ctccctgtca agaaaaacag ctggatccat ttctaatcaa cacttcccaa cgcaacactt 120
   ctgagtctct gaaggagacc agagcttgaa actttccaga cttccaacag acatcgagtg 180
   caaaaggata tttaggttgt ctttgcacaa atctggttga tttgagagat aaaggggggg 240
   ggaaccagtg tgactttcac ctaagaagtc acatgaacat atttcacatt tgaactacat 300
   aatgaatgat ggttattgaa atagcccaaa cctctaccac agagcgaggg atatagctca 360
   aggggcaacc aggcagtcgc agaaccaagg aatggatgac tacaagtatc aggacaatta 420
   tgggggctat gctcccagtg atggctatta ccgcggcaat gagtccaacc cagaagaaga 480
   tgcacagagt gatgtcaccg aaggccatga tgaggaagac gagatctatg agggcgagta 540
   ccagggtatc cctcacccag atgatgtcaa ggccaagcag gccaagatgg cgccctccag 600
   aatggacagc cttcggggcc agacagacct gatggctgag aggctggaag atgaggagca 660
   gttggcccac cagtacgaga ccatcatgga tgagtgtggc catggccgct tccagtggat 720
   cctctttttc gtcttgggtt tggccctgat ggccgatggg gtggaagtgt tcgtggtgag 780
   ttttgccctg cccagtgcag agaaggacat gtgtctgtcc agttccaaaa aaggaatgct 840
   agggatgata gtctacttgg gaatgatggc gggcgccttc atcctgggag gcctggctga 900
   taagctggga aggaagcgag tcctcagcat gtctctggcc gtcaatgcct ccttcgcctc 960
   cctctcttcc ttcgtgcagg gatatggagc cttcctcttc tgccgactca tctcaggcat 1020
   cggtattggg ggtgctctac cgattgtttt tgcctatttt tctgaattct tgtctcggga 1080
   gaagcgagga gaacacctca gttggctggg catcttctgg atgactgggg gcctgtacgc 1140
   atctgccatg gcctggagca tcatcccaca ctatggctgg ggcttcagca tggggaccaa 1200
   ttaccacttc catagctgga gagtgtttgt catcgtctgt gctctgccct gcaccgtgtc 1260
   catggtggcc ctgaagttca tgccagagag cccaaggttt ctgctagaga tgggcaaaca 1320
   tgatgaagcc tggatgattc tcaagcaagt ccatgacacc aacatgagag ctaaggggac 1380
   cccagagaaa gtgttcacgg tttccaacat caaaactccc aagcaaatgg atgaattcat 1440
   tgagatccaa agttcaacag gaacctggta ccagcgctgg ctggtcagat tcaagaccat 1500
   tttcaagcag gtctgggata atgccctgta ctgtgtgatg ggccctacag gaatgaatac 1560
   actgattctg gccgtggttt ggtttgccat ggcattcagt tactatggac tgacagtttg 1620
   gtttcctgat atgatccgct attttcaaga tgaagaatac aagtctaaaa tgaaggtgtt 1680
   ttttggtgag catgtgtacg gcgccacaat caacttcacg atggaaaatc agatccacca 1740
   acatgggaaa cttgtgaatg ataagttcac aagaatgtac tttaaacatg tactctttga 1800
   ggacacattc tttgacgagt gctattttga agacgtaaca tcaacagata cctacttcaa 1860
   aaattgtacc attgaatcaa ccatctttta caacacagac ctctacgagc acaagttcat 1920
   caactgtcgg tttatcaact ccaccttcct ggagcagaag gagggctgcc acatggactt 1980
   ggagcaagat aatgacttcc tgatttacct cgtcagcttc ctgggcagcc tgtctgtctt 2040
   acccgggaac atcatttctg ccctgctcat ggatagaatt ggaaggctca agatgattgg 2100
   tggctccatg ctaatctctg cagtctgctg cttcttcctg ttttttggca acagtgagtc 2160
   tgcaatgatc ggctggcagt gcctgttctg tgggacaagc attgcagcct ggaatgctct 2220
   ggatgtgatc acagtggagc tgtatcccac caaccagaga gcaacagcct tcggcattct 2280
   caatggatta tgcaaatttg gcgccatcct gggaaacacc atctttgctt cttttgttgg 2340
   gataaccaaa gtggtcccca tccttctggc tgctgcttct ctggttgggg gtggcctgat 2400
   tgcccttcga ctgccagaga ctcgagaaca ggtcctgatg tgaacaacct atgggaaaag 2460
   gaaaggtcga gagaatcttg tccaggacac tgaaatgcat ccacacttcc tgcctatcac 2520
   ggtccggagg acaccttgga tagcacggga ggagaagttg actttgtgac ccctagttta 2580
   ggacccactt cagctgtcaa tatgtttgta actcaggtga ctgatttggg ggtgccctga 2640
   gccaccctta gaatcacaga gctgcgtgtt taacttcaag tcttcccagt ccaaggcagg 2700
   gagaggattc tccagtgagt gcacacacta tgcgaggagc aagcatttct ctaagtcaag 2760
   tgcaaggact taacttgcgt ttgaaaagga attagagggt cagaaacacc caggttcctc 2820
   cagaaagctc cttggagccc aacaacttaa caaatcaact tggctggaag ttagagtcat 2880
   tatatgaaga ttgggcttga agtatatatt tttgcattta aaagtatcac ctatcatatt 2940
   ttccactcga aaattgacat agtagcattg aggatactct gatctagaaa gccaagtatt 3000
   tgagcaacat ctatagagat ctacttttct cctatgtctc ctaggctttc catgataatt 3060
   aggtaataca tttaagaagg atatttattt ctgttttgct ctattcaaag aaacggaatg 3120
   ggatagttat tctgtaaact aagtttgtat ataactttat ttgggtttaa tttccacaac 3180
   tggtatctgc aaatattgcc agcattttag ccatattttg ggagaacttg gtgtttgagg 3240
   tcccaggaaa tgaggtctga tcaaatgaaa tgcaagcaca atttcttaca gccatttaac 3300
   tttctgttgg gaggatgaat taacaaactc acattgtgca gtctgcttaa tccaggcact 3360
   tttctttgtg caggtgtagt gagtagttac ttctctccct tacacagatg acttgtgaaa 3420
   ctcaagctca ccatcttcag tgctggcatt ttactttgcc actacccaaa aacaatgtga 3480
   gatgtgttca gtggcctctg gtactctttg caggcaagaa tcaaacaaca tggggactga 3540
   gggaaggatg gggaagtgta gccacagttc ttccaaatgt aaatactttt tgtttgttct 3600
   agtggtaaaa tgcaaatgca atccatattt gttaggatgg tcaggtctca tgagaaatct 3660
   atgctatgtg tccagagctt ttgaaacaga gtccattgga gtgggagtta gggagtgtag 3720
   tggatgccaa atatgttttt cttcagtgct taagagaact gtttcctgaa gtccagcttt 3780
   gaacataaac aggggtgtgg gttgggggag gagcttagga caaacctctc tgatgaaggt 3840
   cagcaataga ctgaagtctt gactgcatgg aagaggaaaa acatcagaac tgtctgacaa 3900
   tggaggggac agtgagctac gcacaactgc cagcggaggt gaacttgcac ctgcccaggc 3960
   cggatgaaca tcagcctgca agaactagtt gtttgagttg atttgcagtg ctctcaatgg 4020
   gcaagtgcca cattttccct ggcagagatc tccaaaaatt taaaacagaa taataatggc 4080
   tatatcgagt gttttctcag tattggagaa atgcttaggt cctatgatag cttcgggaca 4140
   tctttctgta attttcctca attaacgggt tggtaggggt aaatcttatg acacctttcc 4200
   accgtcgatt tgagatcagt tttaatggtt aaaatgttta ctctccttct gtcaaccctc 4260
   acctttttat ttacacccct cccttttttt ctgtacaggg agagaagaca tattgactct 4320
   gactggacac cctgattcct ccaaatatat ataccactgt gtattaatct ttctctcagt 4380
   gttttatagg agtactaaca tttattgctc tgtcaataat gaaaggctcg atgtaatata 4440
   gctgtaattt actttccata tgaatacagt ggctaggttc ataaaagaga attgtgtgag 4500
   tctgggatta ccacatctaa aacattattc tttaatggga taatacaatt cattgagcag 4560
   ctaccactta aaaaacttgc aggacagtta gagcctgcat ttctagttaa gatggatctt 4620
   gtaaatttaa aattggatta acattggagt gctggggtgg ctgcaataat ttgggggcta 4680
   actccatttg gtttccaaga tctcacttct gcattatctt tatggctctt taaaccagcc 4740
   acctagccaa tcaagggcaa ttcccatctc atccatcact caggtctttg taaagggtgc 4800
   agccaagctc tgcagacttt tgcaggattg tctagcctga gtaccgggct acttcttaaa 4860
   tgccgtcact cctgctgaga taaatgcgtc tttaaaaata gtctctgtgg caggtcactg 4920
   ggggacaatg tacagcattc tggccatcca cttctttttc acttcatgtt ctaccccaag 4980
   agactcccga tgtcggctgt ggagggttaa agggatgagg ctttcctttg tttagcaaat 5040
   ctgttcacag ttcttgatga tgtattttat gatgcccagc ttggaaatag ttgctttcca 5100
   tagtctcaac tgtattgtgt catctcctga tgctgatttt tgatcttttg ttttattaaa 5160
   aataattagt gaaagaggtg tgcctatctg tgaagtttgt agtacatcat cctgaggtca 5220
   tgtaacaagt aaaccccaac ccagcgttcc ctcctacgtt gtgttagttc attaaaacta 5280
   aataataaaa ataactgtaa gaaaacctta a                                5311
<210> 144
   <211> 2362
   <212> DNA
   <213> Homo sapiens
<400> 144
   cactcagggc aagggtgtcc gacggctgga gcgttctgtt ttgaacccaa agtggatgat 60
   gctgtcagag ctgaactact gaaaggaggc tgtgaaaatt tcccatcttc tcattggcca 120
   tcagttgaga taagatggaa gactcttaca aggataggac ttcactgatg aagggtgcca 180
   aggacattgc cagagaggtg aagaaacaaa cagtaaagaa ggtgaatcaa gctgtggacc 240
   gagcccagga tgaatacacc cagaggtcct acagtcggtt ccaagatgaa gaagatgatg 300
   atgactacta cccggctgga gaaacctata atggtgaggc caacgatgac gaaggctcaa 360
   gtgaagccac tgaggggcat gatgaagatg atgagatcta tgagggggag tatcagggca 420
   tccccagtat gaaccaagcg aaggacagca tcgtgtcagt ggggcagccc aagggcgatg 480
   agtacaagga ccgacgggag ctggaatcag aaaggagagc tgacgaggaa gagttagccc 540
   agcagtatga gctgataatc caagaatgcg gtcatggtcg ttttcagtgg gcccttttct 600
   tcgtcctggg catggctctt atggcagacg gtgtagaggt gtttgtcgtt ggcttcgtgt 660
   tacccagtgc tgagacagac ctctgcatcc caaattcagg atctggatgg ctaggcagca 720
   tagtgtacct cgggatgatg gtgggggcgt tcttctgggg aggactggca gacaaagtgg 780
   gaaggaaaca gtctcttctg atttgcatgt ctgtcaacgg attctttgcc ttcctttctt 840
   catttgtcca aggttatggc ttctttctct tctgtcgctt actttctgga ttcgggattg 900
   gaggagccat acccactgtg ttctcgtact ttgctgaagt cctggcccgg gaaaagcggg 960
   gcgaacactt gagctggctc tgcatgttct ggatgatcgg tggcatctac gcctctgcca 1020
   tggcctgggc catcatcccg cactacgggt ggagcttcag catgggatcg gcctaccagt 1080
   ttcacagttg gcgtgtgttt gtcatcgtct gtgcactccc ctgtgtctcc tccgtggtgg 1140
   ccctcacatt catgcctgaa agcccacgat tcttgttgga ggttggaaaa catgatgaag 1200
   cttggatgat tctgaagtta attcatgaca ccaacatgag agcccggggt cagcctgaga 1260
   aggtcttcac ggtaaacaaa ataaaaactc ctaaacaaat agatgagctg attgaaattg 1320
   agagtgacac aggaacatgg tataggaggt gttttgttcg gatccgcacc gagctgtacg 1380
   gaatttggtt gacttttatg agatgtttca actacccagt cagggataat acaataaagc 1440
   ttacaattgt ttggttcacc ctgtcctttg ggtactatgg attatccgtt tggttccctg 1500
   atgtcattaa acctctgcag tccgatgaat atgcattgct aaccagaaat gtggagagag 1560
   ataaatatgc aaatttcact attaacttta caatggaaaa tcagattcat actggaatgg 1620
   aatacgacaa tggcagattc ataggggtca agttcaaatc tgtaactttc aaagactctg 1680
   tttttaagtc ctgcaccttt gaggatgtaa cttcagtgaa cacctacttc aagaactgca 1740
   catttattga cactgttttt gacaacacag attttgagcc atataaattc attgacagtg 1800
   aatttaaaaa ctgctcgttt tttcacaaca agacgggatg tcagattacc tttgatgatg 1860
   actatagtgc ctactggatt tattttgtca actttctggg gacattggca gtattgccag 1920
   ggaacattgt gtctgctctg ctgatggaca gaattgggcg cttaacaatg ctaggtggct 1980
   ctatggtgct ttcggggatc agctgtttct tcctttggtt cggcaccagt gaatccatga 2040
   tgataggcat gctgtgtctg tacaatggat tgaccatctc agcctggaac tctcttgacg 2100
   tggtcactgt ggaactgtac cccacagacc ggagggcaac aggctttggc ttcttaaatg 2160
   cgctatgcaa ggcagcagcc gtcctgggaa acttaatatt tggctctctg gtcagcatca 2220
   ccaaatcaat ccccatcctg ctggcttcta ctgtgctcgt gtgtggagga ctcgttgggc 2280
   tgtgcctgcc tgacacacga acccaggttc tgatgtaatg ggaaaaaaag ccatccttcc 2340
   tgcgtttctt cctcctgccc tg                                          2362
<210> 145
   <211> 2053
   <212> DNA
   <213> Homo sapiens
<400> 145
   catctttgat gagggcagag ctcacgttgc attgaagacg aaacctcggg gaggtcaggc 60
   gctgtctttc cttccctccc tgctcggcgg ctccaccaca gttgcaacct gcagaggccc 120
   ggagaacaca accctcccga gaagcccagg tccagagcca aacccgtcac tgacccccca 180
   gcccaggcgc ccagccactc cccaccgcta ccatggccga agacgcagac atgcgcaatg 240
   agctggagga gatgcagcga agggctgacc agttggctga tgagtcgctg gaaagcaccc 300
   gtcgtatgct gcaactggtt gaagagagta aagatgctgg tatcaggact ttggttatgt 360
   tggatgaaca aggagaacaa ctcgatcgtg tcgaagaagg catgaaccat atcaaccaag 420
   acatgaagga ggctgagaaa aatttaaaag atttagggaa atgctgtggc cttttcatat 480
   gtccttgtaa caagcttaaa tcaagtgatg cttacaaaaa agcctggggc aataatcagg 540
   acggagtggt ggccagccag cctgctcgtg tagtggacga acgggagcag atggccatca 600
   gtggcggctt catccgcagg gtaacaaatg atgcccgaga aaatgaaatg gatgaaaacc 660
   tagagcaggt gagcggcatc atcgggaacc tccgtcacat ggccctggat atgggcaatg 720
   agatcgatac acagaatcgc cagatcgaca ggatcatgga gaaggctgat tccaacaaaa 780
   ccagaattga tgaggccaac caacgtgcaa caaagatgct gggaagtggt taagtgtgcc 840
   cacccgtgtt ctcctccaaa tgctgtcggg caagatagct ccttcatgct tttctcatgg 900
   tattatctag taggtctgca cacataacac acatcagtcc acccccattg tgaatgttgt 960
   cctgtgtcat ctgtcagctt cccaacaata ctttgtgtct tttgttctct cttggtctct 1020
   ttctttccaa aggttgtaca tagtggtcat ttggtggctc taactccttg atgtcttgag 1080
   tttcattttt cattttctct cctcggtggc atttgctgaa taacaacaat ttaggaatgc 1140
   tcaatgtgct gttgattctt tcaatccaca gtattgttct tgtaaaactg tgacattcca 1200
   cagagttact gccacggtcc tttgagtgtc aggctctgaa tctctcaaaa tgtgccgtct 1260
   ttggttcctc atggctgtta tctgtcttta tgatttcatg attagacaat gtggaattac 1320
   ataacaggca ttgcactaaa agtgatgtga tttatgcatt tatgcatgag aactaaatag 1380
   atttttagat tcctacttaa acaaaaactt tccatgacag tagcatactg atgagacaac 1440
   acacacacac acaaaacaac agcaacaaca acagaacaac aacaaagcat gctcagtatt 1500
   gagacactgt caagattaag ttataccagc aaaagtgcag tagtgtcact tttttcctgt 1560
   caatatatag agacttctaa atcataatca tcctttttta aaaaaaagaa ttttaaaaaa 1620
   gatggatttg acacactcac catttaatca tttccagcaa aatatatgtt tggctgaaat 1680
   tatgtcaaat ggatgtaata tagggtttgt ttgctgcttt tgatggctac gttttggaga 1740
   gagcaatctt gctgtgaaac agtgtggatg taaattttat aaggctgact cttactaacc 1800
   accatttccc ctgtggtttg ttatcagtac aattctttgt tgcttaatct agagctatgc 1860
   acaccaaatt gctgagatgt ttagtagctg ataaagaaac cttttaaaaa aataatataa 1920
   atgaatgaaa tataaactgt gagataaata tcattatagc atgtaatatt aaattcctcc 1980
   tgtctcctct gtcagtttgt gaagtgattg acattttgta gctagtttaa aattattaaa 2040
   aattatagac tcc                                                    2053
<210> 146
   <211> 2053
   <212> DNA
   <213> Homo sapiens
<400> 146
   catctttgat gagggcagag ctcacgttgc attgaagacg aaacctcggg gaggtcaggc 60
   gctgtctttc cttccctccc tgctcggcgg ctccaccaca gttgcaacct gcagaggccc 120
   ggagaacaca accctcccga gaagcccagg tccagagcca aacccgtcac tgacccccca 180
   gcccaggcgc ccagccactc cccaccgcta ccatggccga agacgcagac atgcgcaatg 240
   agctggagga gatgcagcga agggctgacc agttggctga tgagtcgctg gaaagcaccc 300
   gtcgtatgct gcaactggtt gaagagagta aagatgctgg tatcaggact ttggttatgt 360
   tggatgaaca aggagaacaa ctggaacgca ttgaggaagg gatggaccaa atcaataagg 420
   acatgaaaga agcagaaaag aatttgacgg acctaggaaa attctgcggg ctttgtgtgt 480
   gtccctgtaa caagcttaaa tcaagtgatg cttacaaaaa agcctggggc aataatcagg 540
   acggagtggt ggccagccag cctgctcgtg tagtggacga acgggagcag atggccatca 600
   gtggcggctt catccgcagg gtaacaaatg atgcccgaga aaatgaaatg gatgaaaacc 660
   tagagcaggt gagcggcatc atcgggaacc tccgtcacat ggccctggat atgggcaatg 720
   agatcgatac acagaatcgc cagatcgaca ggatcatgga gaaggctgat tccaacaaaa 780
   ccagaattga tgaggccaac caacgtgcaa caaagatgct gggaagtggt taagtgtgcc 840
   cacccgtgtt ctcctccaaa tgctgtcggg caagatagct ccttcatgct tttctcatgg 900
   tattatctag taggtctgca cacataacac acatcagtcc acccccattg tgaatgttgt 960
   cctgtgtcat ctgtcagctt cccaacaata ctttgtgtct tttgttctct cttggtctct 1020
   ttctttccaa aggttgtaca tagtggtcat ttggtggctc taactccttg atgtcttgag 1080
   tttcattttt cattttctct cctcggtggc atttgctgaa taacaacaat ttaggaatgc 1140
   tcaatgtgct gttgattctt tcaatccaca gtattgttct tgtaaaactg tgacattcca 1200
   cagagttact gccacggtcc tttgagtgtc aggctctgaa tctctcaaaa tgtgccgtct 1260
   ttggttcctc atggctgtta tctgtcttta tgatttcatg attagacaat gtggaattac 1320
   ataacaggca ttgcactaaa agtgatgtga tttatgcatt tatgcatgag aactaaatag 1380
   atttttagat tcctacttaa acaaaaactt tccatgacag tagcatactg atgagacaac 1440
   acacacacac acaaaacaac agcaacaaca acagaacaac aacaaagcat gctcagtatt 1500
   gagacactgt caagattaag ttataccagc aaaagtgcag tagtgtcact tttttcctgt 1560
   caatatatag agacttctaa atcataatca tcctttttta aaaaaaagaa ttttaaaaaa 1620
   gatggatttg acacactcac catttaatca tttccagcaa aatatatgtt tggctgaaat 1680
   tatgtcaaat ggatgtaata tagggtttgt ttgctgcttt tgatggctac gttttggaga 1740
   gagcaatctt gctgtgaaac agtgtggatg taaattttat aaggctgact cttactaacc 1800
   accatttccc ctgtggtttg ttatcagtac aattctttgt tgcttaatct agagctatgc 1860
   acaccaaatt gctgagatgt ttagtagctg ataaagaaac cttttaaaaa aataatataa 1920
   atgaatgaaa tataaactgt gagataaata tcattatagc atgtaatatt aaattcctcc 1980
   tgtctcctct gtcagtttgt gaagtgattg acattttgta gctagtttaa aattattaaa 2040
   aattatagac tcc                                                    2053
<210> 147
   <211> 5
   <212> PRT
   <213> Artificial Sequence
<220>
   <223> SNAP-25 antigen having a free carboxyl-terminus at the P1 residue of the scissile bond of the BoNT/A cleavage site
<400> 147
   Asp Glu Ala Asn Gln
   1               5
<210> 148
   <211> 6
   <212> PRT
   <213> Artificial Sequence
<220>
   <223> SNAP-25 antigen having a free carboxyl-terminus at the P1 residue of the scissile bond of the BoNT/A cleavage site
<400> 148
   Ile Asp Glu Ala Asn Gln
   1 5
<210> 149
   <211> 357
   <212> DNA
   <213> Mus musculus
<400> 149
<210> 150
   <211> 120
   <212> PRT
   <213> Mus musculus
<400> 150
   Gln Val Gln Leu Gln Glu Ser Gly Pro Glu Leu Val Lys Pro Gly Ala
   1 5 10 15
   Ser Val Lys Met Ser Cys Lys Ala Ser Gly Tyr Thr Phe Thr Asn Tyr
   20 25 30
   Val Ile His Trp Val Lys Gln Lys Pro Gly Gln Gly Leu Glu Trp Ile
   35 40 45
   Gly Tyr Ile Asn Pro Tyr Asn Asp Gly Ser Lys Tyr Asn Glu Lys Phe
   50 55 60
   Lys Gly Lys Ala Ser Leu Thr Ser Asp Lys Ser Ser Ser Thr Ala Tyr
   65 70 75 80
   Met Glu Leu Ser Ser Leu Thr Ser Glu Asp Ser Ala Val Tyr Tyr Cys
   85 90 95
   Ala Arg His Leu Ala Asn Thr Tyr Tyr Tyr Phe Asp Tyr Trp Gly Gln
   100 105 110
   Gly Thr Thr Leu Thr Val Ser Ser
   115 120
<210> 151
   <211> 5
   <212> PRT
   <213> Mus musculus
<400> 151
   Ala Arg Met Gly Tyr
   1 5
<210> 152
   <211> 13
   <212> PRT
   <213> Mus musculus
<400> 152
   Ala Arg His Leu Ala Asn Thr Tyr Tyr Tyr Phe Asp Tyr
    1               5                   10